# EUROPEAN PATENT SPECIFICATION

(11) **EP 3 418 379 B1**
(45) Date of publication and mention of the grant of the patent: **09.12.2020**
(21) Application number: 18174860.9
(22) Date of filing: 18.09.2014
(51) Int. Cl.: C12N 5/10, C12N 15/10, C12N 15/85, C12N 15/90

(54) **METHODS, CELLS & ORGANISMS**
VERFAHREN, ZELLEN UND ORGANISMEN
PROCÉDÉS, CELLULES ET ORGANISMES

(30) Priority: 18.09.2013 GB 201316560; 02.12.2013 GB 201321210
(43) Date of publication of application: 26.12.2018
(62) Divisional of application: 14772198.9
(73) Proprietor: Kymab Limited, Cambridge CB22 3AT (GB)
(72) Inventor: BRADLEY, Allan, Cambridge, Cambridgeshire CB22 3AT (GB); ALI, Hanif, Cambridge, Cambridgeshire CB22 3AT (GB); LEE, E-Chiang, Cambridge, Cambridgeshire CB22 3AT (GB)
(74) Representative: CMS Cameron McKenna Nabarro Olswang LLP

(56) References cited:
- WO-A1-2013/061078
- WO-A1-2013/176772
- WO-A2-2013/041846
- WOONG Y HWANG ET AL: "Efficient genome editing in zebrafish using a CRISPR-Cas system", NATURE BIOTECHNOLOGY, vol. 31, no. 3, 29 January 2013 (2013-01-29), pages 227-229, XP055086625, ISSN: 1087-0156, DOI: 10.1038/nbt.2501
- WOONG Y HWANG ET AL: "Efficient genome editing using RNA-guided Nucleases", HHS PUBLIC ACCESS AUTHOR MANUSCRIPT, 1 March 2013 (2013-03-01), pages 1-12, XP055401624, DOI: 10.1038/nbt.2501
- L. CONG ET AL: "Multiplex Genome Engineering Using CRISPR/Cas Systems", SCIENCE, vol. 339, no. 6121, 15 February 2013 (2013-02-15), pages 819-823, XP055067741, ISSN: 0036-8075, DOI: 10.1126/science.1231143 & CONG L ET AL: "Supplementary Material to : Multiplex Genome Engineering Using CRISPR/Cas Systems", SCIENCE, AMERICAN ASSOCIATION FOR THE ADVANCEMENT OF SCIENCE, US, vol. 339, no. 6121, 3 January 2013 (2013-01-03), pages 819-823, XP002730884, ISSN: 0036-8075, DOI: 10.1126/SCIENCE.1231143
- PATRICK D HSU ET AL: "DNA targeting specificity of RNA-guided Cas9 nucleases", NATURE BIOTECHNOLOGY, NATURE PUBLISHING GROUP, UNITED STATES, vol. 31, no. 9, 1 September 2013 (2013-09-01), pages 827-832, XP002718604, ISSN: 1546-1696, DOI: 10.1038/NBT.2647 [retrieved on 2013-07-21] & PATRICK D HSU ET AL: "Supplementary Information: DNA targeting specificity of RNA-guided Cas9 nuclease, Supplementary Methods", NATURE BIOTECHNOLOGY, NATURE PUBLISHING GROUP, UNITED STATES, vol. 31, no. 9, 1 September 2013 (2013-09-01), pages 1-26, XP002718605, ISSN: 1546-1696, DOI: 10.1038/NBT.2647 [retrieved on 2013-07-21]
- HAGEN RICHTER ET AL: "Exploiting CRISPR/Cas: Interference Mechanisms and Applications", INTERNATIONAL JOURNAL OF MOLECULAR SCIENCES, vol. 14, no. 7, 12 July 2013 (2013-07-12), pages 14518-14531, XP055153313, DOI: 10.3390/ijms140714518
- GAJ THOMAS ET AL: "ZFN, TALEN, and CRISPR/Cas-based methods for genome engineering", TRENDS IN BIOTECHNOLOGY, ELSEVIER PUBLICATIONS, CAMBRIDGE, GB, vol. 31, no. 7, 9 May 2013 (2013-05-09), pages 397-405, XP028571313, ISSN: 0167-7799, DOI: 10.1016/J.TIBTECH.2013.04.004
- ANN RAN F ET AL: "Double nicking by RNA-guided CRISPR Cas9 for enhanced genome editing specificity", CELL, CELL PRESS, US, vol. 154, no. 6, 12 September 2013 (2013-09-12), pages 1380-1389, XP002718603, ISSN: 0092-8674, DOI: 10.1016/J.CELL.2013.08.021 [retrieved on 2013-08-29]
- S. Kondo ET AL: "Highly Improved Gene Targeting by Germline-Specific Cas9 Expression in Drosophila", GENETICS, vol. 195, no. 3, 3 September 2013 (2013-09-03), pages 715-721, XP055248203, US ISSN: 0016-6731, DOI: 10.1534/genetics.113.156737
- W. Fujii ET AL: "Efficient generation of large-scale genome-modified mice using gRNA and CAS9 endonuclease", Nucleic Acids Research, vol. 41, no. 20, 30 August 2013 (2013-08-30), pages 1-9, XP055270998, ISSN: 0305-1048, DOI: 10.1093/nar/gkt772
- You-Tzung Chen ET AL: "PiggyBac Transposon-Mediated, Reversible Gene Transfer in Human Embryonic Stem Cells", Stem Cells and Development, vol. 19, no. 6, 1 June 2010 (2010-06-01), pages 763-771, XP055248208, US ISSN: 1547-3287, DOI: 10.1089/scd.2009.0118
- Daniel J Dickinson ET AL: "Engineering the Caenorhabditis elegans genome using Cas9-triggered homologous recombination", Nature Methods, vol. 10, no. 10, 1 September 2013 (2013-09-01), pages 1028-1034, XP055340980, New York ISSN: 1548-7091, DOI: 10.1038/nmeth.2641 & Daniel J Dickinson ET AL: "Engineering the Caenorhabditis elegans genome using Cas9-triggered homologous recombination", Nature Methods, vol. 10, no. 10, 1 September 2013 (2013-09-01), pages 1028-1034, XP055340984, New York ISSN: 1548-7091, DOI: 10.1038/nmeth.2641

### SEQUENCE LISTING

<110> Kymab Inc.
<120> Methods, Cells and Organisms
<130> K000016-2 PCT
<150> EP14772198.9
   <151> 2014-09-18
<160> 208
<170> FastSEQ for Windows Version 4.0
<210> 1
   <211> 11
   <212> RNA
   <213> Artificial Sequence
<220>
   <223> an example of part of gRNA before linker sequence
<400> 1
   uuuuagagcu a 11
<210> 2
   <211> 13
   <212> RNA
   <213> Artificial Sequence
<220>
   <223> an example of part of gRNA following linker sequence
<400> 2
   uagcaaguua aaa 13
<210> 3
   <211> 12
   <212> RNA
   <213> Artificial Sequence
<220>
   <223> exemplary portion of gRNA (crRNA)
<220>
   <221> misc_feature
   <222> 1, 7, 8
   <223> n = A,U,C or G
<400> 3
   nuuuuanngc ua 12
<210> 4
   <211> 12
   <212> RNA
   <213> Artificial Sequence
<220>
   <223> exemplary tracrRNA which comprises a portion of a gRNA
<220>
   <221> misc_feature
   <222> 5
   <223> n = spacer sequence
<220>
   <221> misc_feature
   <222> 12
   <223> n = A,U,C or G
<400> 4
   uagcnuuaaa an 12
<210> 5
   <211> 26
   <212> RNA
   <213> Artificial Sequence
<220>
   <223> an example of a tracrRNA
<400> 5
   uagcaaguua aaauaaggcu aguccg 26
<210> 6
   <211> 76
   <212> RNA
   <213> Artificial Sequence
<220>
   <223> an example of a gRNA
<400> 6
   guuuuagagc uagaaauagc aaguuaaaau aaggcuaguc cguuaucaac uugaaaaagu 60
   ggcaccgagu cggugc                                                 76
<210> 7
   <211> 1340
   <212> DNA
   <213> Artificial Sequence
<220>
   <223> Fragment 1A (1340 bp)
<400> 7
   ggtaccgggc cccccctcga ggtcgacggt atcgataagc ttgatgaggg cctatttccc 60
   atgattcctt catatttgca tatacgatac aaggctgtta gagagataat tggaattaat 120
   ttgactgtaa acacaaagat attagtacaa aatacgtgac gtagaaagta ataatttctt 180
   gggtagtttg cagttttaaa attatgtttt aaaatggact atcatatgct taccgtaact 240
   tgaaagtatt tcgatttctt ggctttatat atcttgtgga aaggacgaaa caccgggtct 300
   tcgagaagac ctgttttaga gctagaaata gcaagttaaa ataaggctag tccgttatca 360
   acttgaaaaa gtggcaccga gtcggtgctt ttttgtttta gagctagaaa tagcaagtta 420
   aaataaggct agtccgtttt tagcgcgtgc gccaattctg cagacaaatg gctctagagg 480
   tacccgttac ataacttacg gtaaatggcc cgcctggctg accgcccaac gacccccgcc 540
   cattgacgtc aatagtaacg ccaataggga ctttccattg acgtcaatgg gtggagtatt 600
   tacggtaaac tgcccacttg gcagtacatc aagtgtatca tatgccaagt acgcccccta 660
   ttgacgtcaa tgacggtaaa tggcccgcct ggcattgtgc ccagtacatg accttatggg 720
   actttcctac ttggcagtac atctacgtat tagtcatcgc tattaccatg gtcgaggtga 780
   gccccacgtt ctgcttcact ctccccatct cccccccctc cccaccccca attttgtatt 840
   tatttatttt ttaattattt tgtgcagcga tgggggcggg gggggggggg gggcgcgcgc 900
   caggcggggc ggggcggggc gaggggcggg gcggggcgag gcggagaggt gcggcggcag 960
   ccaatcagag cggcgcgctc cgaaagtttc cttttatggc gaggcggcgg cggcggcggc 1020
   cctataaaaa gcgaagcgcg cggcgggcgg gagtcgctgc gacgctgcct tcgccccgtg 1080
   ccccgctccg ccgccgcctc gcgccgcccg ccccggctct gactgaccgc gttactccca 1140
   caggtgagcg ggcgggacgg cccttctcct ccgggctgta attagctgag caagaggtaa 1200
   gggtttaagg gatggttggt tggtggggta ttaatgttta attacctgga gcacctgcct 1260
   gaaatcactt tttttcaggt tggaccggtg ccaccatgga ctataaggac cacgacggag 1320
   actacaagga tcatgatatt                                             1340
<210> 8
   <211> 852
   <212> DNA
   <213> Artificial Sequence
<220>
   <223> Fragment 2 (852 bp)
<400> 8
   atggactata aggaccacga cggagactac aaggatcatg atattgatta caaagacgat 60
   gacgataaga tggccccaaa gaagaagcgg aaggtcggta tccacggagt cccagcagcc 120
   gacaagaagt acagcatcgg cctggacatc ggcaccaact ctgtgggctg ggccgtgatc 180
   accgacgagt acaaggtgcc cagcaagaaa ttcaaggtgc tgggcaacac cgaccggcac 240
   agcatcaaga agaacctgat cggagccctg ctgttcgaca gcggcgaaac agccgaggcc 300
   acccggctga agagaaccgc cagaagaaga tacaccagac ggaagaaccg gatctgctat 360
   ctgcaagaga tcttcagcaa cgagatggcc aaggtggacg acagcttctt ccacagactg 420
   gaagagtcct tcctggtgga agaggataag aagcacgagc ggcaccccat cttcggcaac 480
   atcgtggacg aggtggccta ccacgagaag taccccacca tctaccacct gagaaagaaa 540
   ctggtggaca gcaccgacaa ggccgacctg cggctgatct atctggccct ggcccacatg 600
   atcaagttcc ggggccactt cctgatcgag ggcgacctga accccgacaa cagcgacgtg 660
   gacaagctgt tcatccagct ggtgcagacc tacaaccagc tgttcgagga aaaccccatc 720
   aacgccagcg gcgtggacgc caaggccatc ctgtctgcca gactgagcaa gagcagacgg 780
   ctggaaaatc tgatcgccca gctgcccggc gagaagaaga atggcctgtt cggaaacctg 840
   attgccctga gc                                                    852
<210> 9
   <211> 920
   <212> DNA
   <213> Artificial Sequence
<220>
   <223> Fragment 3 (920 bp)
<400> 9
   ggcgagaaga agaatggcct gttcggaaac ctgattgccc tgagcctggg cctgaccccc 60
   aacttcaaga gcaacttcga cctggccgag gatgccaaac tgcagctgag caaggacacc 120
   tacgacgacg acctggacaa cctgctggcc cagatcggcg accagtacgc cgacctgttt 180
   ctggccgcca agaacctgtc cgacgccatc ctgctgagcg acatcctgag agtgaacacc 240
   gagatcacca aggcccccct gagcgcctct atgatcaaga gatacgacga gcaccaccag 300
   gacctgaccc tgctgaaagc tctcgtgcgg cagcagctgc ctgagaagta caaagagatt 360
   ttcttcgacc agagcaagaa cggctacgcc ggctacattg acggcggagc cagccaggaa 420
   gagttctaca agttcatcaa gcccatcctg gaaaagatgg acggcaccga ggaactgctc 480
   gtgaagctga acagagagga cctgctgcgg aagcagcgga ccttcgacaa cggcagcatc 540
   ccccaccaga tccacctggg agagctgcac gccattctgc ggcggcagga agatttttac 600
   ccattcctga aggacaaccg ggaaaagatc gagaagatcc tgaccttccg catcccctac 660
   tacgtgggcc ctctggccag gggaaacagc agattcgcct ggatgaccag aaagagcgag 720
   gaaaccatca ccccctggaa cttcgaggaa gtggtggaca agggcgcttc cgcccagagc 780
   ttcatcgagc ggatgaccaa cttcgataag aacctgccca acgagaaggt gctgcccaag 840
   cacagcctgc tgtacgagta cttcaccgtg tataacgagc tgaccaaagt gaaatacgtg 900
   accgagggaa tgagaaagcc                                            920
<210> 10
   <211> 920
   <212> DNA
   <213> Artificial Sequence
<220>
   <223> Fragment 4 (920 bp)
<400> 10
   cgagctgacc aaagtgaaat acgtgaccga gggaatgaga aagcccgcct tcctgagcgg 60
   cgagcagaaa aaggccatcg tggacctgct gttcaagacc aaccggaaag tgaccgtgaa 120
   gcagctgaaa gaggactact tcaagaaaat cgagtgcttc gactccgtgg aaatctccgg 180
   cgtggaagat cggttcaacg cctccctggg cacataccac gatctgctga aaattatcaa 240
   ggacaaggac ttcctggaca atgaggaaaa cgaggacatt ctggaagata tcgtgctgac 300
   cctgacactg tttgaggaca gagagatgat cgaggaacgg ctgaaaacct atgcccacct 360
   gttcgacgac aaagtgatga agcagctgaa gcggcggaga tacaccggct ggggcaggct 420
   gagccggaag ctgatcaacg gcatccggga caagcagtcc ggcaagacaa tcctggattt 480
   cctgaagtcc gacggcttcg ccaacagaaa cttcatgcag ctgatccacg acgacagcct 540
   gacctttaaa gaggacatcc agaaagccca ggtgtccggc cagggcgata gcctgcacga 600
   gcacattgcc aatctggccg gcagccccgc cattaagaag ggcatcctgc agacagtgaa 660
   ggtggtggac gagctcgtga aagtgatggg ccggcacaag cccgagaaca tcgtgatcga 720
   aatggccaga gagaaccaga ccacccagaa gggacagaag aacagccgcg agagaatgaa 780
   gcggatcgaa gagggcatca aagagctggg cagccagatc ctgaaagaac accccgtgga 840
   aaacacccag ctgcagaacg agaagctgta cctgtactac ctgcagaatg ggcgggatat 900
   gtacgtggac caggaactgg                                             920
<210> 11
   <211> 920
   <212> DNA
   <213> Artificial Sequence
<220>
   <223> Fragment 5 (920 bp)
<400> 11
   actacctgca gaatgggcgg gatatgtacg tggaccagga actggacatc aaccggctgt 60
   ccgactacga tgtggaccat atcgtgcctc agagctttct gaaggacgac tccatcgaca 120
   acaaggtgct gaccagaagc gacaagaacc ggggcaagag cgacaacgtg ccctccgaag 180
   aggtcgtgaa gaagatgaag aactactggc ggcagctgct gaacgccaag ctgattaccc 240
   agagaaagtt cgacaatctg accaaggccg agagaggcgg cctgagcgaa ctggataagg 300
   ccggcttcat caagagacag ctggtggaaa cccggcagat cacaaagcac gtggcacaga 360
   tcctggactc ccggatgaac actaagtacg acgagaatga caagctgatc cgggaagtga 420
   aagtgatcac cctgaagtcc aagctggtgt ccgatttccg gaaggatttc cagttttaca 480
   aagtgcgcga gatcaacaac taccaccacg cccacgacgc ctacctgaac gccgtcgtgg 540
   gaaccgccct gatcaaaaag taccctaagc tggaaagcga gttcgtgtac ggcgactaca 600
   aggtgtacga cgtgcggaag atgatcgcca agagcgagca ggaaatcggc aaggctaccg 660
   ccaagtactt cttctacagc aacatcatga actttttcaa gaccgagatt accctggcca 720
   acggcgagat ccggaagcgg cctctgatcg agacaaacgg cgaaaccggg gagatcgtgt 780
   gggataaggg ccgggatttt gccaccgtgc ggaaagtgct gagcatgccc caagtgaata 840
   tcgtgaaaaa gaccgaggtg cagacaggcg gcttcagcaa agagtctatc ctgcccaaga 900
   ggaacagcga taagctgatc                                             920
<210> 12
   <211> 789
   <212> DNA
   <213> Artificial Sequence
<220>
   <223> Fragment 6 (789 bp)
<400> 12
   agcaaagagt ctatcctgcc caagaggaac agcgataagc tgatcgccag aaagaaggac 60
   tgggacccta agaagtacgg cggcttcgac agccccaccg tggcctattc tgtgctggtg 120
   gtggccaaag tggaaaaggg caagtccaag aaactgaaga gtgtgaaaga gctgctgggg 180
   atcaccatca tggaaagaag cagcttcgag aagaatccca tcgactttct ggaagccaag 240
   ggctacaaag aagtgaaaaa ggacctgatc atcaagctgc ctaagtactc cctgttcgag 300
   ctggaaaacg gccggaagag aatgctggcc tctgccggcg aactgcagaa gggaaacgaa 360
   ctggccctgc cctccaaata tgtgaacttc ctgtacctgg ccagccacta tgagaagctg 420
   aagggctccc ccgaggataa tgagcagaaa cagctgtttg tggaacagca caagcactac 480
   ctggacgaga tcatcgagca gatcagcgag ttctccaaga gagtgatcct ggccgacgct 540
   aatctggaca aagtgctgtc cgcctacaac aagcaccggg ataagcccat cagagagcag 600
   gccgagaata tcatccacct gtttaccctg accaatctgg gagcccctgc cgccttcaag 660
   tactttgaca ccaccatcga ccggaagagg tacaccagca ccaaagaggt gctggacgcc 720
   accctgatcc accagagcat caccggcctg tacgagacac ggatcgacct gtctcagctg 780
   ggaggcgac                                                         789
<210> 13
   <211> 535
   <212> DNA
   <213> Artificial Sequence
<220>
   <223> Fragment 7 (535 bp)
<400> 13
   ggcctgtacg agacacggat cgacctgtct cagctgggag gcgacaaaag gccggcggcc 60
   acgaaaaagg ccggccaggc aaaaaagaaa aagtaagaat tcctagagct cgctgatcag 120
   cctcgactgt gccttctagt tgccagccat ctgttgtttg cccctccccc gtgccttcct 180
   tgaccctgga aggtgccact cccactgtcc tttcctaata aaatgaggaa attgcatcgc 240
   attgtctgag taggtgtcat tctattctgg ggggtggggt ggggcaggac agcaaggggg 300
   aggattggga agagaatagc aggcatgctg gggagcggcc gcaggaaccc ctagtgatgg 360
   agttggccac tccctctctg cgcgctcgct cgctcactga ggccgggcga ccaaaggtcg 420
   cccgacgccc gggctttgcc cgggcggcct cagtgagcga gcgagcgcgc agctgcctgc 480
   aggggcgcct atcgaattcc tgcagcccgg gggatccact agttctagag cggcc      535
<210> 14
   <211> 852
   <212> DNA
   <213> Artificial Sequence
<220>
   <223> Fragment 2A (852 bp)
<400> 14
   atggactata aggaccacga cggagactac aaggatcatg atattgatta caaagacgat 60
   gacgataaga tggccccaaa gaagaagcgg aaggtcggta tccacggagt cccagcagcc 120
   gacaagaagt acagcatcgg cctggccatc ggcaccaact ctgtgggctg ggccgtgatc 180
   accgacgagt acaaggtgcc cagcaagaaa ttcaaggtgc tgggcaacac cgaccggcac 240
   agcatcaaga agaacctgat cggagccctg ctgttcgaca gcggcgaaac agccgaggcc 300
   acccggctga agagaaccgc cagaagaaga tacaccagac ggaagaaccg gatctgctat 360
   ctgcaagaga tcttcagcaa cgagatggcc aaggtggacg acagcttctt ccacagactg 420
   gaagagtcct tcctggtgga agaggataag aagcacgagc ggcaccccat cttcggcaac 480
   atcgtggacg aggtggccta ccacgagaag taccccacca tctaccacct gagaaagaaa 540
   ctggtggaca gcaccgacaa ggccgacctg cggctgatct atctggccct ggcccacatg 600
   atcaagttcc ggggccactt cctgatcgag ggcgacctga accccgacaa cagcgacgtg 660
   gacaagctgt tcatccagct ggtgcagacc tacaaccagc tgttcgagga aaaccccatc 720
   aacgccagcg gcgtggacgc caaggccatc ctgtctgcca gactgagcaa gagcagacgg 780
   ctggaaaatc tgatcgccca gctgcccggc gagaagaaga atggcctgtt cggaaacctg 840
   attgccctga gc                                                      852
<210> 15
   <211> 24
   <212> DNA
   <213> Artificial Sequence
<220>
   <223> an example oligo
<220>
   <221> misc_feature
   <222> 6, 7, 8, 9, 10, 11, 12, 13, 14, 15, 16, 17, 18, 19, 20, 21, 22, 23, 24
   <223> n = A,T,C or G
<400> 15
   caccgnnnnn nnnnnnnnnn nnnn 24
<210> 16
   <211> 24
   <212> DNA
   <213> Artificial Sequence
<220>
   <223> an example oligo
<220>
   <221> misc_feature
   <222> 5, 6, 7, 8, 9, 10, 11, 12, 13, 14, 15, 16, 17, 18, 19, 20, 21, 22, 23
   <223> n = A,T,C or G
<400> 16
   aaacnnnnnn nnnnnnnnnn nnnc 24
<210> 17
   <211> 122
   <212> DNA
   <213> Artificial Sequence
<220>
   <223> Example T7-gRNA Sequence
<220>
   <221> misc_feature
   <222> 21, 22, 23, 24, 25, 26, 27, 28, 29, 30, 31, 32, 33, 34, 35, 36, 37, 38, 39, 40
   <223> n = A,T,C or G
<400> 17
   ttaatacgac tcactatagg nnnnnnnnnn nnnnnnnnnn gttttagagc tagaaatagc 60
   aagttaaaat aaggctagtc cgttatcaac ttgaaaaagt ggcaccgagt cggtgctttt 120
   tt                                                                122
<210> 18
   <211> 112
   <212> DNA
   <213> Artificial Sequence
<220>
   <223> Fragment 1 (111 bp)
<400> 18
   ggtaccgggc cccccctcga ggtcgacggt atcgataagc ttgataatac gactcactat 60
   agggagaatg gactataagg accacgacgg agactacaag gatcatgata tt         112
<210> 19
   <211> 20
   <212> DNA
   <213> Artificial Sequence
<220>
   <223> primer Cas9-F
<400> 19
   ttaatacgac tcactatagg 20
<210> 20
   <211> 21
   <212> DNA
   <213> Artificial Sequence
<220>
   <223> primer Cas9-R used for PCR amplification
<400> 20
   gcgagctcta ggaattctta c 21
<210> 21
   <211> 20
   <212> DNA
   <213> Artificial Sequence
<220>
   <223> primer CgRNA-F
<400> 21
   ttaatacgac tcactatagg 20
<210> 22
   <211> 24
   <212> DNA
   <213> Artificial Sequence
<220>
   <223> primer CgRNA-R
<400> 22
   aaaaaagcac cgactcggtg ccac 24
<210> 23
   <211> 3108
   <212> DNA
   <213> Artificial Sequence
<220>
   <223> Sequence of Rosa26 5-prime homology arm
<400> 23
   cacatttggt cctgcttgaa cattgccatg gctcttaaag tcttaattaa gaatattaat 60
   tgtgtaatta ttgtttttcc tcctttagat cattccttga ggacaggaca gtgcttgttt 120
   aaggctatat ttctgctgtc tgagcagcaa caggtcttcg agatcaacat gatgttcata 180
   atcccaagat gttgccattt atgttctcag aagcaagcag aggcatgatg gtcagtgaca 240
   gtaatgtcac tgtgttaaat gttgctatgc agtttggatt tttctaatgt agtgtaggta 300
   gaacatatgt gttctgtatg aattaaactc ttaagttaca ccttgtataa tccatgcaat 360
   gtgttatgca attaccattt taagtattgt agctttcttt gtatgtgagg ataaaggtgt 420
   ttgtcataaa atgttttgaa catttcccca aagttccaaa ttataaaacc acaacgttag 480
   aacttattta tgaacaatgg ttgtagtttc atgcttttaa aatgcttaat tattcaatta 540
   acaccgtttg tgttataata tatataaaac tgacatgtag aagtgtttgt ccagaacatt 600
   tcttaaatgt atactgtctt tagagagttt aatatagcat gtcttttgca acatactaac 660
   ttttgtgttg gtgcgagcaa tattgtgtag tcattttgaa aggagtcatt tcaatgagtg 720
   tcagattgtt ttgaatgtta ttgaacattt taaatgcaga cttgttcgtg ttttagaaag 780
   caaaactgtc agaagctttg aactagaaat taaaaagctg aagtatttca gaagggaaat 840
   aagctacttg ctgtattagt tgaaggaaag tgtaatagct tagaaaattt aaaaccatat 900
   agttgtcatt gctgaatatc tggcagatga aaagaaatac tcagtggttc ttttgagcaa 960
   tataacagct tgttatatta aaaattttcc ccacagatat aaactctaat ctataactca 1020
   taaatgttac aaatggatga agcttacaaa tgtggcttga cttgtcactg tgcttgtttt 1080
   agttatgtga aagtttggca ataaacctat gtcctaaata gtcaaactgt ggaatgactt 1140
   tttaatctat tggtttgtct agaacagtta tgttgccatt tgccctaatg gtgaaagaaa 1200
   aagtggggag tgccttggca ctgttcattt gtggtgtgaa ccaaagaggg gggcatgcac 1260
   ttacacttca aacatccttt tgaaagactg acaagtttgg gtcttcacag ttggaattgg 1320
   gcatcccttt tgtcagggag ggagggaggg agggaggctg gcttgttatg ctgacaagtg 1380
   tgattaaatt caaactttga ggtaagttgg aggaacttgt acattgttag gagtgtgaca 1440
   atttggactc ttaatgattt ggtcatacaa aatgaaccta gaccaacttc tggaagatgt 1500
   atataataac tccatgttac attgatttca cctgactaat acttatccct tatcaattaa 1560
   atacagaaga tgccagccat ctgggccttt taacccagaa atttagtttc aaactcctag 1620
   gttagtgttc tcactgagct acatcctgat ctagtcctga aaataggacc accatcaccc 1680
   ccaaaaaaat ctcaaataag atttatgcta gtgtttcaaa attttaggaa taggtaagat 1740
   tagaaagttt taaattttga gaaatggctt ctctagaaag atgtacatag tgaacactga 1800
   atggctccta aagagcctag aaaactggta ctgagcacac aggactgaga ggtctttctt 1860
   gaaaagcatg tattgcttta cgtgggtcac agaaggcagg caggaagaac ttgggctgaa 1920
   actggtgtct taagtggcta acatcttcac aactgatgag caagaacttt atcctgatgc 1980
   aaaaaccatc caaacaaact aagtgaaagg tggcaatgga tcccaggctg ctctagagga 2040
   ggacttgact tctcatccca tcacccacac cagatagctc atagactgcc aattaacacc 2100
   agcttctagc ctccacaggc acctgcactg gtacacataa tttcacacaa acacagtaag 2160
   aagccttcca cctggcatgg tattgcttat ctttagttcc caacacttgg gaggcagagg 2220
   ccagccaggg ctatgtgaca aaaaccttgt ctagaggaga aacttcatag cttatttcct 2280
   attcacgtaa ccaggttagc aaaatttacc agccagagat gaagctaaca gtgtccacta 2340
   tatttgtagt gttttaagtc aattttttaa atatacttaa tagaattaaa gctatggtga 2400
   accaagtaca aacctggtgt attaacttga gaacttagca taaaaagtag ttcatttgtt 2460
   cagtaaatat taaatgctta ctggcaaaga ttatgtcagg aacttggtaa atggtgatga 2520
   aacaatcata gttgtacatc ttggttctgt gatcaccttg gtttgaggta aaagtggttc 2580
   ctttgatcaa ggatggaatt ttaagtttat attcaatcaa taatgtatta ttttgtgatt 2640
   gcaaaattgc ctatctaggg tataaaacct ttaaaaattt cataatacca gttcattctc 2700
   cagttactaa ttccaaaaag ccactgacta tggtgccaat gtggattctg ttctcaaagg 2760
   aaggattgtc tgtgcccttt attctaatag aaacatcaca ctgaaaatct aagctgaaag 2820
   aagccagact ttcctaaata aataactttc cataaagctc aaacaaggat tacttttagg 2880
   aggcactgtt aaggaactga taagtaatga ggttacttat ataatgatag tcccacaaga 2940
   ctatctgagg aaaaatcagt acaactcgaa aacagaacaa ccagctaggc aggaataaca 3000
   gggctcccaa gtcaggaggt ctatccaaca cccttttctg ttgagggccc cagacctaca 3060
   tattgtatac aaacagggag gtgggtgatt ttaactctcc tgaggtac            3108
<210> 24
   <211> 3102
   <212> DNA
   <213> Artificial Sequence
<220>
   <223> Sequence of Rosa26 3-prime homology arm
<400> 24
   cttggtaaat ctttgtcctg agtaagcagt acagtgtaca gtttacattt tcatttaaag 60
   atacattagc tccctctacc ccctaagact gacaggcact ttgggggtgg ggagggcttt 120
   ggaaaataac gcttccatac actaaaagag aaatttcttt aattaggctt gttggttcca 180
   tacatctact ggtgtttcta ctacttagta atattataat agtcacacaa gcatctttgc 240
   tctgtttagg ttgtatattt attttaaggc agatgataaa actgtagatc ttaagggatg 300
   cttctgcttc tgagatgata caaagaattt agaccataaa acagtaggtt gcacaagcaa 360
   tagaatatgg cctaaagtgt tctgacactt agaagccaag cagtgtaggc ttcttaagaa 420
   ataccattac aatcaccttg ctagaaatca agcattctgg agtggtcaag cagtgtaacc 480
   tgtactgtaa gttacttttc tgctattttt ctcccaaagc aagttcttta tgctgatatt 540
   tccagtgtta ggaactacaa atattaataa gttgtcttca ctcttttctt taccaaggag 600
   ggtctcttcc ttcatcttga tctgaaggat gaacaaaggc ttgagcagtg cgctttagaa 660
   gataaactgc agcatgaagg cccccgatgt tcacccagac tacatggacc tttcgccaca 720
   catgtcccat tccagataag gcctggcaca cacaaaaaac ataagtcatt aggctaccag 780
   tctgattcta aaacaaccta aaatcttccc acttaaatgc tatgggtggt gggttggaaa 840
   gttgactcag aaaatcactt gctgttttta gagaggatct gggttcagtt tctgatacat 900
   tgtggcttac aactataact ccagttctag ggggtccatc caacatcctc ttctgttgag 960
   ggcaccaaat aaatgtattg tgtacaaaca gggaggtgag tgatttaact ctcgtgtata 1020
   gtaccttggt aaaacatttc ttgtcctgag taagcagtac agctctgcct gtccctggtc 1080
   tacagacacg gctcatttcc cgaaggcaag ctggatagag attccaattt ctcttcttgg 1140
   atcccatcct ataaaagaag gtcaagttta atctattgca aaaggtaaat aggtagtttc 1200
   ttacatgaga caagaacaaa tcttaggtgt gaagcagtca tcttttacag gccagagcct 1260
   ctattctatg ccaatgaagg aaactgttag tccagtgtta tagagttagt ccagtgtata 1320
   gttttctatc agaacacttt ttttttaaac aactgcaact tagcttattg aagacaaacc 1380
   acgagtagaa atctgtccaa gaagcaagtg cttctcagcc tacaatgtgg aataggacca 1440
   tgtaatggta cagtgagtga aatgaattat ggcatgtttt tctgactgag aagacagtac 1500
   aataaaaggt aaactcatgg tatttattta aaaagaatcc aatttctacc tttttccaaa 1560
   tggcatatct gttacaataa tatccacaga agcagttctc agtgggaggt tgcagatatc 1620
   ccactgaaca gcatcaatgg gcaaacccca ggttgttttt ctgtggagac aaaggtaaga 1680
   tatttcaata tattttccca agctaatgag atggctcagc aaataatggt actggccatt 1740
   aagtctcatg acctgagctt gatcctcagg gaccatgtgg tacaaggaga gacctaaatc 1800
   cttcagttgg acttcaatct tctaccctca tgtccacaca caaataaata caataaaaaa 1860
   cattctgcag tctgaatttc taaaggttgt ttttctaaaa agaaatgtta aagtaacata 1920
   ggaagaaata tgtccataac tgaaatacaa gttttttaaa tggttaagac tggttttcaa 1980
   aggatgtatg gttaagaaaa taccagggaa aatgagctta catgtaaaaa agtgtctaaa 2040
   aggccagaga aatgacccag ctggcaaagg tgtctgccct aagccagaca aaaggaattt 2100
   gattcacagg aagaagagac ccaactctca ctagttatcc tctgacttcc acaccatgac 2160
   acagctccat ggcactctca ggcccccaca catatacaga tataaacaga aacctaatcc 2220
   accagccttc agaagcaaag caattggagg atttaaacag gccatggcta ctaatagaga 2280
   taactggtag tttaaaagtt atggtaatga ctttcatgct tctttcaact catattgttc 2340
   taaataatta atttggtttt tcaaggcagg gtttctctgt gtagttctgg ctgtcctgga 2400
   actcactctg tagaccaggc tggccttgaa ctcagatcca tctgcctctg gaataagggc 2460
   acgtgcgtgc cttttctaca taacaaaacc tatactataa caaaacctat accatactgt 2520
   accgttttgg gaaaagacaa aaaataatga acaaaaaagg agaaataaca ttccaataaa 2580
   gtatggaaat ggtagttaaa ttaattacaa atgtttttca gtaaattaga tgtgacttct 2640
   catactgttc atttggctat aatgatacca caaagcactg ggggtgaata ataattccaa 2700
   gtcagtaggg agagagactt gaaaagatgc aatgcaatca ttgaagttaa acttacccat 2760
   ctttaatctg gctcttagtc aatagagatg agatgttatt tgctgctctg ttcactgcca 2820
   gtgggttatt gtccccagca atatggtaac agtgagacca ctcagtagcc ccctatgaga 2880
   caggagtgtt ggttaaacat gccacaagag aaaagggaaa agtcactatg gccaactctc 2940
   agtaacatgg caatccgtgc cattcatttc cttgccagaa atgtcttccc tgttcttctg 3000
   cctactgaac tttcacccac tagaaatgtg gctccaatgt catccactat gacatcaatg 3060
   tcagcgctag aagcactttg cacacctctg ttgctgactt ag                   3102
<210> 25
   <211> 30
   <212> DNA
   <213> Artificial Sequence
<220>
   <223> CRISPR consensus sequence of Genome Mth and PaM NGG
<400> 25
   atttcaatcc cattttggtc tgattttaac 30
<210> 26
   <211> 29
   <212> DNA
   <213> Artificial Sequence
<220>
   <223> CRISPR consensus sequence of Genome Lmo and PaM WGG
<400> 26
   atttacattt cahaataagt arytaaaac 29
<210> 27
   <211> 29
   <212> DNA
   <213> Artificial Sequence
<220>
   <223> CRISPR consensus sequence of Genome Eco and PaM CWT
<400> 27
   cggtttatcc ccgctggcgc ggggaacwc 29
<210> 28
   <211> 29
   <212> DNA
   <213> Artificial Sequence
<220>
   <223> CRISPR consensus sequence of Genome Pae and PaM CTT
<400> 28
   cggttcatcc ccacrcmygt ggggaacac 29
<210> 29
   <211> 32
   <212> DNA
   <213> Artificial Sequence
<220>
   <223> CRISPR consensus sequence of Genome Spy and PaM GAA
<400> 29
   atttcaatcc actcacccat gaagggtgag ac 32
<210> 30
   <211> 31
   <212> DNA
   <213> Artificial Sequence
<220>
   <223> CRISPR consensus sequence of Genome Xan and PaM WGG
<400> 30
   gtttcaatcc acgcgcccgt gaggrcgcga c 31
<210> 31
   <211> 28
   <212> DNA
   <213> Artificial Sequence
<220>
   <223> CRISPR consensus sequence of Genome She and PaM GG
<400> 31
   tttctaagcc gcctgtgcgg cggtgaac 28
<210> 32
   <211> 28
   <212> DNA
   <213> Artificial Sequence
<220>
   <223> CRISPR consensus sequence of Genome Pae and PaM GG
<400> 32
   tttcttagct gcctatacgg cagtgaac 28
<210> 33
   <211> 28
   <212> DNA
   <213> Artificial Sequence
<220>
   <223> CRISPR consensus sequence of Genome Ype and PaM GG
<400> 33
   tttctaagct gcctgtgcgg cagtgaac 28
<210> 34
   <211> 24
   <212> DNA
   <213> Artificial Sequence
<220>
   <223> CRISPR consensus sequence of Genome Sso and PaM NGG
<400> 34
   ctttcaattc tataagagat tatc 24
<210> 35
   <211> 24
   <212> DNA
   <213> Artificial Sequence
<220>
   <223> CRISPR consensus sequence of Genome Mse and PaM NGG
<400> 35
   ctttcaactc tataggagat taac 24
<210> 36
   <211> 36
   <212> DNA
   <213> Artificial Sequence
<220>
   <223> CRISPR consensus sequence of Genome Str and PaM NGG
<400> 36
   gttttagagc tatgctgttt tgaatggtcc caaaac 36
<210> 37
   <211> 36
   <212> DNA
   <213> Artificial Sequence
<220>
   <223> CRISPR consensus sequence of Genome Lis and PaM NGG
<400> 37
   gttttagagc tatgttattt tgaatgctam caaaac 36
<210> 38
   <211> 10
   <212> DNA
   <213> Artificial Sequence
<220>
   <223> Leader
<400> 38
   agggcggatt 10
<210> 39
   <211> 10
   <212> DNA
   <213> Artificial Sequence
<220>
   <223> Leader
<400> 39
   atggccaatt 10
<210> 40
   <211> 10
   <212> DNA
   <213> Artificial Sequence
<220>
   <223> Leader
<400> 40
   ccactaactt 10
<210> 41
   <211> 10
   <212> DNA
   <213> Artificial Sequence
<220>
   <223> Leader
<400> 41
   ccgctctatt 10
<210> 42
   <211> 10
   <212> DNA
   <213> Artificial Sequence
<220>
   <223> Leader
<400> 42
   tctaaacata 10
<210> 43
   <211> 10
   <212> DNA
   <213> Artificial Sequence
<220>
   <223> Leader
<400> 43
   tctaaaagta 10
<210> 44
   <211> 10
   <212> DNA
   <213> Artificial Sequence
<220>
   <223> Leader
<400> 44
   acttaccgta 10
<210> 45
   <211> 10
   <212> DNA
   <213> Artificial Sequence
<220>
   <223> Leader
<400> 45
   ccttaccgta 10
<210> 46
   <211> 10
   <212> DNA
   <213> Artificial Sequence
<220>
   <223> Leader
<400> 46
   tgcgccaaat 10
<210> 47
   <211> 10
   <212> DNA
   <213> Artificial Sequence
<220>
   <223> Leader
<400> 47
   ccccccttag 10
<210> 48
   <211> 10
   <212> DNA
   <213> Artificial Sequence
<220>
   <223> Leader
<400> 48
   gccgccagca 10
<210> 49
   <211> 10
   <212> DNA
   <213> Artificial Sequence
<220>
   <223> Leader
<400> 49
   aatagcttat 10
<210> 50
   <211> 10
   <212> DNA
   <213> Artificial Sequence
<220>
   <223> Leader
<400> 50
   tgtagaataa 10
<210> 51
   <211> 10
   <212> DNA
   <213> Artificial Sequence
<220>
   <223> Leader
<400> 51
   tagctccgaa 10
<210> 52
   <211> 10
   <212> DNA
   <213> Artificial Sequence
<220>
   <223> Leader
<400> 52
   tagaccaaaa 10
<210> 53
   <211> 10
   <212> DNA
   <213> Artificial Sequence
<220>
   <223> Leader
<400> 53
   gtaagataat 10
<210> 54
   <211> 10
   <212> DNA
   <213> Artificial Sequence
<220>
   <223> Leader
<400> 54
   tgagggttta 10
<210> 55
   <211> 10
   <212> DNA
   <213> Artificial Sequence
<220>
   <223> Leader
<400> 55
   tgataccttt 10
<210> 56
   <211> 10
   <212> DNA
   <213> Artificial Sequence
<220>
   <223> Leader
<400> 56
   tgaaactttt 10
<210> 57
   <211> 10
   <212> DNA
   <213> Artificial Sequence
<220>
   <223> Leader
<400> 57
   tgacactctt 10
<210> 58
   <211> 10
   <212> DNA
   <213> Artificial Sequence
<220>
   <223> Leader
<400> 58
   ctcgtagact 10
<210> 59
   <211> 10
   <212> DNA
   <213> Artificial Sequence
<220>
   <223> Leader
<400> 59
   ctcgtagaaa 10
<210> 60
   <211> 10
   <212> DNA
   <213> Artificial Sequence
<220>
   <223> Leader
<400> 60
   ctcgcagaat 10
<210> 61
   <211> 10
   <212> DNA
   <213> Artificial Sequence
<220>
   <223> Leader
<400> 61
   ctcgtagaat 10
<210> 62
   <211> 1037
   <212> PRT
   <213> Parvibaculum lavamentivorans
<220>
   <223> CRISPR-associated endonuclease
<308> YP_001411379
   <309> 2013-06-10
<400> 62
   Met Glu Arg Ile Phe Gly Phe Asp Ile Gly Thr Thr Ser Ile Gly Phe
    1               5                  10                  15
   Ser Val Ile Asp Tyr Ser Ser Thr Gln Ser Ala Gly Asn Ile Gln Arg
               20                  25                  30
   Leu Gly Val Arg Ile Phe Pro Glu Ala Arg Asp Pro Asp Gly Thr Pro
           35                  40                  45
   Leu Asn Gln Gln Arg Arg Gln Lys Arg Met Met Arg Arg Gln Leu Arg
       50                  55                  60
   Arg Arg Arg Ile Arg Arg Lys Ala Leu Asn Glu Thr Leu His Glu Ala
   65                  70                  75                  80
   Gly Phe Leu Pro Ala Tyr Gly Ser Ala Asp Trp Pro Val Val Met Ala
                   85                  90                  95
   Asp Glu Pro Tyr Glu Leu Arg Arg Arg Gly Leu Glu Glu Gly Leu Ser
               100                 105                 110
   Ala Tyr Glu Phe Gly Arg Ala Ile Tyr His Leu Ala Gln His Arg His
           115                 120                 125
   Phe Lys Gly Arg Glu Leu Glu Glu Ser Asp Thr Pro Asp Pro Asp Val
       130                 135                 140
   Asp Asp Glu Lys Glu Ala Ala Asn Glu Arg Ala Ala Thr Leu Lys Ala
   145                 150                 155                 160
   Leu Lys Asn Glu Gln Thr Thr Leu Gly Ala Trp Leu Ala Arg Arg Pro
                   165                 170                 175
   Pro Ser Asp Arg Lys Arg Gly Ile His Ala His Arg Asn Val Val Ala
               180                 185                 190
   Glu Glu Phe Glu Arg Leu Trp Glu Val Gln Ser Lys Phe His Pro Ala
           195                 200                 205
   Leu Lys Ser Glu Glu Met Arg Ala Arg Ile Ser Asp Thr Ile Phe Ala
       210                 215                 220
   Gln Arg Pro Val Phe Trp Arg Lys Asn Thr Leu Gly Glu Cys Arg Phe
   225                 230                 235                 240
   Met Pro Gly Glu Pro Leu Cys Pro Lys Gly Ser Trp Leu Ser Gln Gln
                   245                 250                 255
   Arg Arg Met Leu Glu Lys Leu Asn Asn Leu Ala Ile Ala Gly Gly Asn
               260                 265                 270
   Ala Arg Pro Leu Asp Ala Glu Glu Arg Asp Ala Ile Leu Ser Lys Leu
           275                 280                 285
   Gln Gln Gln Ala Ser Met Ser Trp Pro Gly Val Arg Ser Ala Leu Lys
       290                 295                 300
   Ala Leu Tyr Lys Gln Arg Gly Glu Pro Gly Ala Glu Lys Ser Leu Lys
   305                 310                 315                 320
   Phe Asn Leu Glu Leu Gly Gly Glu Ser Lys Leu Leu Gly Asn Ala Leu
                   325                 330                 335
   Glu Ala Lys Leu Ala Asp Met Phe Gly Pro Asp Trp Pro Ala His Pro
               340                 345                 350
   Arg Lys Gln Glu Ile Arg His Ala Val His Glu Arg Leu Trp Ala Ala
           355                 360                 365
   Asp Tyr Gly Glu Thr Pro Asp Lys Lys Arg Val Ile Ile Leu Ser Glu
       370                 375                 380
   Lys Asp Arg Lys Ala His Arg Glu Ala Ala Ala Asn Ser Phe Val Ala
   385                 390                 395                 400
   Asp Phe Gly Ile Thr Gly Glu Gln Ala Ala Gln Leu Gln Ala Leu Lys
                   405                 410                 415
   Leu Pro Thr Gly Trp Glu Pro Tyr Ser Ile Pro Ala Leu Asn Leu Phe
               420                 425                 430
   Leu Ala Glu Leu Glu Lys Gly Glu Arg Phe Gly Ala Leu Val Asn Gly
           435                 440                 445
   Pro Asp Trp Glu Gly Trp Arg Arg Thr Asn Phe Pro His Arg Asn Gln
       450                 455                 460
   Pro Thr Gly Glu Ile Leu Asp Lys Leu Pro Ser Pro Ala Ser Lys Glu
   465                 470                 475                 480
   Glu Arg Glu Arg Ile Ser Gln Leu Arg Asn Pro Thr Val Val Arg Thr
                   485                 490                 495
   Gln Asn Glu Leu Arg Lys Val Val Asn Asn Leu Ile Gly Leu Tyr Gly
               500                 505                 510
   Lys Pro Asp Arg Ile Arg Ile Glu Val Gly Arg Asp Val Gly Lys Ser
           515                 520                 525
   Lys Arg Glu Arg Glu Glu Ile Gln Ser Gly Ile Arg Arg Asn Glu Lys
       530                 535                 540
   Gln Arg Lys Lys Ala Thr Glu Asp Leu Ile Lys Asn Gly Ile Ala Asn
   545                 550                 555                 560
   Pro Ser Arg Asp Asp Val Glu Lys Trp Ile Leu Trp Lys Glu Gly Gln
                   565                 570                 575
   Glu Arg Cys Pro Tyr Thr Gly Asp Gln Ile Gly Phe Asn Ala Leu Phe
               580                 585                 590
   Arg Glu Gly Arg Tyr Glu Val Glu His Ile Trp Pro Arg Ser Arg Ser
           595                 600                 605
   Phe Asp Asn Ser Pro Arg Asn Lys Thr Leu Cys Arg Lys Asp Val Asn
       610                 615                 620
   Ile Glu Lys Gly Asn Arg Met Pro Phe Glu Ala Phe Gly His Asp Glu
   625                 630                 635                 640
   Asp Arg Trp Ser Ala Ile Gln Ile Arg Leu Gln Gly Met Val Ser Ala
                   645                 650                 655
   Lys Gly Gly Thr Gly Met Ser Pro Gly Lys Val Lys Arg Phe Leu Ala
               660                 665                 670
   Lys Thr Met Pro Glu Asp Phe Ala Ala Arg Gln Leu Asn Asp Thr Arg
           675                 680                 685
   Tyr Ala Ala Lys Gln Ile Leu Ala Gln Leu Lys Arg Leu Trp Pro Asp
       690                 695                 700
   Met Gly Pro Glu Ala Pro Val Lys Val Glu Ala Val Thr Gly Gln Val
   705                 710                 715                 720
   Thr Ala Gln Leu Arg Lys Leu Trp Thr Leu Asn Asn Ile Leu Ala Asp
                   725                 730                 735
   Asp Gly Glu Lys Thr Arg Ala Asp His Arg His His Ala Ile Asp Ala
               740                 745                 750
   Leu Thr Val Ala Cys Thr His Pro Gly Met Thr Asn Lys Leu Ser Arg
           755                 760                 765
   Tyr Trp Gln Leu Arg Asp Asp Pro Arg Ala Glu Lys Pro Ala Leu Thr
       770                 775                 780
   Pro Pro Trp Asp Thr Ile Arg Ala Asp Ala Glu Lys Ala Val Ser Glu
   785                 790                 795                 800
   Ile Val Val Ser His Arg Val Arg Lys Lys Val Ser Gly Pro Leu His
                   805                 810                 815
   Lys Glu Thr Thr Tyr Gly Asp Thr Gly Thr Asp Ile Lys Thr Lys Ser
               820                 825                 830
   Gly Thr Tyr Arg Gln Phe Val Thr Arg Lys Lys Ile Glu Ser Leu Ser
           835                 840                 845
   Lys Gly Glu Leu Asp Glu Ile Arg Asp Pro Arg Ile Lys Glu Ile Val
       850                 855                 860
   Ala Ala His Val Ala Gly Arg Gly Gly Asp Pro Lys Lys Ala Phe Pro
   865                 870                 875                 880
   Pro Tyr Pro Cys Val Ser Pro Gly Gly Pro Glu Ile Arg Lys Val Arg
                   885                 890                 895
   Leu Thr Ser Lys Gln Gln Leu Asn Leu Met Ala Gln Thr Gly Asn Gly
               900                 905                 910
   Tyr Ala Asp Leu Gly Ser Asn His His Ile Ala Ile Tyr Arg Leu Pro
           915                 920                 925
   Asp Gly Lys Ala Asp Phe Glu Ile Val Ser Leu Phe Asp Ala Ser Arg
       930                 935                 940
   Arg Leu Ala Gln Arg Asn Pro Ile Val Gln Arg Thr Arg Ala Asp Gly
   945                 950                 955                 960
   Ala Ser Phe Val Met Ser Leu Ala Ala Gly Glu Ala Ile Met Ile Pro
                   965                 970                 975
   Glu Gly Ser Lys Lys Gly Ile Trp Ile Val Gln Gly Val Trp Ala Ser
               980                 985                 990
   Gly Gln Val Val Leu Glu Arg Asp Thr Asp Ala Asp His Ser Thr Thr
           995                 1000                1005
   Thr Arg Pro Met Pro Asn Pro Ile Leu Lys Asp Asp Ala Lys Lys Val
       1010                1015                1020
   Ser Ile Asp Pro Ile Gly Arg Val Arg Pro Ser Asn Asp
   1025                1030                1035
<210> 63
   <211> 1629
   <212> PRT
   <213> Francisella novicida
<220>
   <223> CRISPR-associated endonuclease
<308> YP_898402
   <309> 2013-06-27
<400> 63
   Met Asn Phe Lys Ile Leu Pro Ile Ala Ile Asp Leu Gly Val Lys Asn
    1               5                  10                  15
   Thr Gly Val Phe Ser Ala Phe Tyr Gln Lys Gly Thr Ser Leu Glu Arg
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Arg Arg His Gln Arg Arg Gly
       50                  55                  60
   Ile Asp Arg Lys Gln Leu Val Lys Arg Leu Phe Lys Leu Ile Trp Thr
   65                  70                  75                  80
   Glu Gln Leu Asn Leu Glu Trp Asp Lys Asp Thr Gln Gln Ala Ile Ser
                   85                  90                  95
   Phe Leu Phe Asn Arg Arg Gly Phe Ser Phe Ile Thr Asp Gly Tyr Ser
               100                 105                 110
   Pro Glu Tyr Leu Asn Ile Val Pro Glu Gln Val Lys Ala Ile Leu Met
           115                 120                 125
   Asp Ile Phe Asp Asp Tyr Asn Gly Glu Asp Asp Leu Asp Ser Tyr Leu
       130                 135                 140
   Lys Leu Ala Thr Glu Gln Glu Ser Lys Ile Ser Glu Ile Tyr Asn Lys
   145                 150                 155                 160
   Leu Met Gln Lys Ile Leu Glu Phe Lys Leu Met Lys Leu Cys Thr Asp
                   165                 170                 175
   Ile Lys Asp Asp Lys Val Ser Thr Lys Thr Leu Lys Glu Ile Thr Ser
               180                 185                 190
   Tyr Glu Phe Glu Leu Leu Ala Asp Tyr Leu Ala Asn Tyr Ser Glu Ser
           195                 200                 205
   Leu Lys Thr Gln Lys Phe Ser Tyr Thr Asp Lys Gln Gly Asn Leu Lys
       210                 215                 220
   Glu Leu Ser Tyr Tyr His His Asp Lys Tyr Asn Ile Gln Glu Phe Leu
   225                 230                 235                 240
   Lys Arg His Ala Thr Ile Asn Asp Arg Ile Leu Asp Thr Leu Leu Thr
                   245                 250                 255
   Asp Asp Leu Asp Ile Trp Asn Phe Asn Phe Glu Lys Phe Asp Phe Asp
               260                 265                 270
   Lys Asn Glu Glu Lys Leu Gln Asn Gln Glu Asp Lys Asp His Ile Gln
           275                 280                 285
   Ala His Leu His His Phe Val Phe Ala Val Asn Lys Ile Lys Ser Glu
       290                 295                 300
   Met Ala Ser Gly Gly Arg His Arg Ser Gln Tyr Phe Gln Glu Ile Thr
   305                 310                 315                 320
   Asn Val Leu Asp Glu Asn Asn His Gln Glu Gly Tyr Leu Lys Asn Phe
                   325                 330                 335
   Cys Glu Asn Leu His Asn Lys Lys Tyr Ser Asn Leu Ser Val Lys Asn
               340                 345                 350
   Leu Val Asn Leu Ile Gly Asn Leu Ser Asn Leu Glu Leu Lys Pro Leu
           355                 360                 365
   Arg Lys Tyr Phe Asn Asp Lys Ile His Ala Lys Ala Asp His Trp Asp
       370                 375                 380
   Glu Gln Lys Phe Thr Glu Thr Tyr Cys His Trp Ile Leu Gly Glu Trp
   385                 390                 395                 400
   Arg Val Gly Val Lys Asp Gln Asp Lys Lys Asp Gly Ala Lys Tyr Ser
                   405                 410                 415
   Tyr Lys Asp Leu Cys Asn Glu Leu Lys Gln Lys Val Thr Lys Ala Gly
               420                 425                 430
   Leu Val Asp Phe Leu Leu Glu Leu Asp Pro Cys Arg Thr Ile Pro Pro
           435                 440                 445
   Tyr Leu Asp Asn Asn Asn Arg Lys Pro Pro Lys Cys Gln Ser Leu Ile
       450                 455                 460
   Leu Asn Pro Lys Phe Leu Asp Asn Gln Tyr Pro Asn Trp Gln Gln Tyr
   465                 470                 475                 480
   Leu Gln Glu Leu Lys Lys Leu Gln Ser Ile Gln Asn Tyr Leu Asp Ser
                   485                 490                 495
   Phe Glu Thr Asp Leu Lys Val Leu Lys Ser Ser Lys Asp Gln Pro Tyr
               500                 505                 510
   Phe Val Glu Tyr Lys Ser Ser Asn Gln Gln Ile Ala Ser Gly Gln Arg
           515                 520                 525
   Asp Tyr Lys Asp Leu Asp Ala Arg Ile Leu Gln Phe Ile Phe Asp Arg
       530                 535                 540
   Val Lys Ala Ser Asp Glu Leu Leu Leu Asn Glu Ile Tyr Phe Gln Ala
   545                 550                 555                 560
   Lys Lys Leu Lys Gln Lys Ala Ser Ser Glu Leu Glu Lys Leu Glu Ser
                   565                 570                 575
   Ser Lys Lys Leu Asp Glu Val Ile Ala Asn Ser Gln Leu Ser Gln Ile
               580                 585                 590
   Leu Lys Ser Gln His Thr Asn Gly Ile Phe Glu Gln Gly Thr Phe Leu
           595                 600                 605
   His Leu Val Cys Lys Tyr Tyr Lys Gln Arg Gln Arg Ala Arg Asp Ser
       610                 615                 620
   Arg Leu Tyr Ile Met Pro Glu Tyr Arg Tyr Asp Lys Lys Leu His Lys
   625                 630                 635                 640
   Tyr Asn Asn Thr Gly Arg Phe Asp Asp Asp Asn Gln Leu Leu Thr Tyr
                   645                 650                 655
   Cys Asn His Lys Pro Arg Gln Lys Arg Tyr Gln Leu Leu Asn Asp Leu
               660                 665                 670
   Ala Gly Val Leu Gln Val Ser Pro Asn Phe Leu Lys Asp Lys Ile Gly
           675                 680                 685
   Ser Asp Asp Asp Leu Phe Ile Ser Lys Trp Leu Val Glu His Ile Arg
       690                 695                 700
   Gly Phe Lys Lys Ala Cys Glu Asp Ser Leu Lys Ile Gln Lys Asp Asn
   705                 710                 715                 720
   Arg Gly Leu Leu Asn His Lys Ile Asn Ile Ala Arg Asn Thr Lys Gly
                   725                 730                 735
   Lys Cys Glu Lys Glu Ile Phe Asn Leu Ile Cys Lys Ile Glu Gly Ser
               740                 745                 750
   Glu Asp Lys Lys Gly Asn Tyr Lys His Gly Leu Ala Tyr Glu Leu Gly
           755                 760                 765
   Val Leu Leu Phe Gly Glu Pro Asn Glu Ala Ser Lys Pro Glu Phe Asp
       770                 775                 780
   Arg Lys Ile Lys Lys Phe Asn Ser Ile Tyr Ser Phe Ala Gln Ile Gln
   785 790 795 800
   Gln Ile Ala Phe Ala Glu Arg Lys Gly Asn Ala Asn Thr Cys Ala Val
   805 810 815
   Cys Ser Ala Asp Asn Ala His Arg Met Gln Gln Ile Lys Ile Thr Glu
   820 825 830
   Pro Val Glu Asp Asn Lys Asp Lys Ile Ile Leu Ser Ala Lys Ala Gln
   835 840 845
   Arg Leu Pro Ala Ile Pro Thr Arg Ile Val Asp Gly Ala Val Lys Lys
   850 855 860
   Met Ala Thr Ile Leu Ala Lys Asn Ile Val Asp Asp Asn Trp Gln Asn
   865 870 875 880
   Ile Lys Gln Val Leu Ser Ala Lys His Gln Leu His Ile Pro Ile Ile
   885 890 895
   Thr Glu Ser Asn Ala Phe Glu Phe Glu Pro Ala Leu Ala Asp Val Lys
   900 905 910
   Gly Lys Ser Leu Lys Asp Arg Arg Lys Lys Ala Leu Glu Arg Ile Ser
   915 920 925
   Pro Glu Asn Ile Phe Lys Asp Lys Asn Asn Arg Ile Lys Glu Phe Ala
   930 935 940
   Lys Gly Ile Ser Ala Tyr Ser Gly Ala Asn Leu Thr Asp Gly Asp Phe
   945 950 955 960
   Asp Gly Ala Lys Glu Glu Leu Asp His Ile Ile Pro Arg Ser His Lys
   965 970 975
   Lys Tyr Gly Thr Leu Asn Asp Glu Ala Asn Leu Ile Cys Val Thr Arg
   980 985 990
   Gly Asp Asn Lys Asn Lys Gly Asn Arg Ile Phe Cys Leu Arg Asp Leu
   995 1000 1005
   Ala Asp Asn Tyr Lys Leu Lys Gln Phe Glu Thr Thr Asp Asp Leu Glu
   1010 1015 1020
   Ile Glu Lys Lys Ile Ala Asp Thr Ile Trp Asp Ala Asn Lys Lys Asp
   1025 1030 1035 1040
   Phe Lys Phe Gly Asn Tyr Arg Ser Phe Ile Asn Leu Thr Pro Gln Glu
   1045 1050 1055
   Gln Lys Ala Phe Arg His Ala Leu Phe Leu Ala Asp Glu Asn Pro Ile
   1060 1065 1070
   Lys Gln Ala Val Ile Arg Ala Ile Asn Asn Arg Asn Arg Thr Phe Val
   1075 1080 1085
   Asn Gly Thr Gln Arg Tyr Phe Ala Glu Val Leu Ala Asn Asn Ile Tyr
   1090 1095 1100
   Leu Arg Ala Lys Lys Glu Asn Leu Asn Thr Asp Lys Ile Ser Phe Asp
   1105 1110 1115 1120
   Tyr Phe Gly Ile Pro Thr Ile Gly Asn Gly Arg Gly Ile Ala Glu Ile
   1125 1130 1135
   Arg Gln Leu Tyr Glu Lys Val Asp Ser Asp Ile Gln Ala Tyr Ala Lys
   1140 1145 1150
   Gly Asp Lys Pro Gln Ala Ser Tyr Ser His Leu Ile Asp Ala Met Leu
   1155 1160 1165
   Ala Phe Cys Ile Ala Ala Asp Glu His Arg Asn Asp Gly Ser Ile Gly
   1170 1175 1180
   Leu Glu Ile Asp Lys Asn Tyr Ser Leu Tyr Pro Leu Asp Lys Asn Thr
   1185 1190 1195 1200
   Gly Glu Val Phe Thr Lys Asp Ile Phe Ser Gln Ile Lys Ile Thr Asp
   1205 1210 1215
   Asn Glu Phe Ser Asp Lys Lys Leu Val Arg Lys Lys Ala Ile Glu Gly
   1220 1225 1230
   Phe Asn Thr His Arg Gln Met Thr Arg Asp Gly Ile Tyr Ala Glu Asn
   1235 1240 1245
   Tyr Leu Pro Ile Leu Ile His Lys Glu Leu Asn Glu Val Arg Lys Gly
   1250 1255 1260
   Tyr Thr Trp Lys Asn Ser Glu Glu Ile Lys Ile Phe Lys Gly Lys Lys
   1265 1270 1275 1280
   Tyr Asp Ile Gln Gln Leu Asn Asn Leu Val Tyr Cys Leu Lys Phe Val
                   1285                1290                1295
   Asp Lys Pro Ile Ser Ile Asp Ile Gln Ile Ser Thr Leu Glu Glu Leu
               1300                1305                1310
   Arg Asn Ile Leu Thr Thr Asn Asn Ile Ala Ala Thr Ala Glu Tyr Tyr
           1315                1320                1325
   Tyr Ile Asn Leu Lys Thr Gln Lys Leu His Glu Tyr Tyr Ile Glu Asn
       1330                1335                1340
   Tyr Asn Thr Ala Leu Gly Tyr Lys Lys Tyr Ser Lys Glu Met Glu Phe
   1345                1350                1355                1360
   Leu Arg Ser Leu Ala Tyr Arg Ser Glu Arg Val Lys Ile Lys Ser Ile
                   1365                1370                1375
   Asp Asp Val Lys Gln Val Leu Asp Lys Asp Ser Asn Phe Ile Ile Gly
               1380                1385                1390
   Lys Ile Thr Leu Pro Phe Lys Lys Glu Trp Gln Arg Leu Tyr Arg Glu
           1395                1400                1405
   Trp Gln Asn Thr Thr Ile Lys Asp Asp Tyr Glu Phe Leu Lys Ser Phe
       1410                1415                1420
   Phe Asn Val Lys Ser Ile Thr Lys Leu His Lys Lys Val Arg Lys Asp
   1425                1430                1435                1440
   Phe Ser Leu Pro Ile Ser Thr Asn Glu Gly Lys Phe Leu Val Lys Arg
                   1445                1450                1455
   Lys Thr Trp Asp Asn Asn Phe Ile Tyr Gln Ile Leu Asn Asp Ser Asp
               1460                1465                1470
   Ser Arg Ala Asp Gly Thr Lys Pro Phe Ile Pro Ala Phe Asp Ile Ser
           1475                1480                1485
   Lys Asn Glu Ile Val Glu Ala Ile Ile Asp Ser Phe Thr Ser Lys Asn
       1490                1495                1500
   Ile Phe Trp Leu Pro Lys Asn Ile Glu Leu Gln Lys Val Asp Asn Lys
   1505                1510                1515                1520
   Asn Ile Phe Ala Ile Asp Thr Ser Lys Trp Phe Glu Val Glu Thr Pro
                   1525                1530                1535
   Ser Asp Leu Arg Asp Ile Gly Ile Ala Thr Ile Gln Tyr Lys Ile Asp
               1540                1545                1550
   Asn Asn Ser Arg Pro Lys Val Arg Val Lys Leu Asp Tyr Val Ile Asp
           1555                1560                1565
   Asp Asp Ser Lys Ile Asn Tyr Phe Met Asn His Ser Leu Leu Lys Ser
       1570                1575                1580
   Arg Tyr Pro Asp Lys Val Leu Glu Ile Leu Lys Gln Ser Thr Ile Ile
   1585                1590                1595                1600
   Glu Phe Glu Ser Ser Gly Phe Asn Lys Thr Ile Lys Glu Met Leu Gly
                   1605                1610                1615
   Met Lys Leu Ala Gly Ile Tyr Asn Glu Thr Ser Asn Asn
               1620                1625
<210> 64
   <211> 984
   <212> PRT
   <213> Campylobacter jejuni
<220>
   <223> CRISPR-associated endonuclease
<308> YP_002344900
   <309> 2014-07-11
<400> 64
   Met Ala Arg Ile Leu Ala Phe Asp Ile Gly Ile Ser Ser Ile Gly Trp
    1               5                  10                  15
   Ala Phe Ser Glu Asn Asp Glu Leu Lys Asp Cys Gly Val Arg Ile Phe
               20                  25                  30
   Thr Lys Val Glu Asn Pro Lys Thr Gly Glu Ser Leu Ala Leu Pro Arg
           35                  40                  45
   Arg Leu Ala Arg Ser Ala Arg Lys Arg Leu Ala Arg Arg Lys Ala Arg
       50                  55                  60
   Leu Asn His Leu Lys His Leu Ile Ala Asn Glu Phe Lys Leu Asn Tyr
   65                  70                  75                  80
   Glu Asp Tyr Gln Ser Phe Asp Glu Ser Leu Ala Lys Ala Tyr Lys Gly
                   85                  90                  95
   Ser Leu Ile Ser Pro Tyr Glu Leu Arg Phe Arg Ala Leu Asn Glu Leu
               100                 105                 110
   Leu Ser Lys Gln Asp Phe Ala Arg Val Ile Leu His Ile Ala Lys Arg
           115                 120                 125
   Arg Gly Tyr Asp Asp Ile Lys Asn Ser Asp Asp Lys Glu Lys Gly Ala
       130                 135                 140
   Ile Leu Lys Ala Ile Lys Gln Asn Glu Glu Lys Leu Ala Asn Tyr Gln
   145                 150                 155                 160
   Ser Val Gly Glu Tyr Leu Tyr Lys Glu Tyr Phe Gln Lys Phe Lys Glu
                   165                 170                 175
   Asn Ser Lys Glu Phe Thr Asn Val Arg Asn Lys Lys Glu Ser Tyr Glu
               180                 185                 190
   Arg Cys Ile Ala Gln Ser Phe Leu Lys Asp Glu Leu Lys Leu Ile Phe
           195                 200                 205
   Lys Lys Gln Arg Glu Phe Gly Phe Ser Phe Ser Lys Lys Phe Glu Glu
       210                 215                 220
   Glu Val Leu Ser Val Ala Phe Tyr Lys Arg Ala Leu Lys Asp Phe Ser
   225                 230                 235                 240
   His Leu Val Gly Asn Cys Ser Phe Phe Thr Asp Glu Lys Arg Ala Pro
                   245                 250                 255
   Lys Asn Ser Pro Leu Ala Phe Met Phe Val Ala Leu Thr Arg Ile Ile
               260                 265                 270
   Asn Leu Leu Asn Asn Leu Lys Asn Thr Glu Gly Ile Leu Tyr Thr Lys
           275                 280                 285
   Asp Asp Leu Asn Ala Leu Leu Asn Glu Val Leu Lys Asn Gly Thr Leu
       290                 295                 300
   Thr Tyr Lys Gln Thr Lys Lys Leu Leu Gly Leu Ser Asp Asp Tyr Glu
   305                 310                 315                 320
   Phe Lys Gly Glu Lys Gly Thr Tyr Phe Ile Glu Phe Lys Lys Tyr Lys
                   325                 330                 335
   Glu Phe Ile Lys Ala Leu Gly Glu His Asn Leu Ser Gln Asp Asp Leu
               340                 345                 350
   Asn Glu Ile Ala Lys Asp Ile Thr Leu Ile Lys Asp Glu Ile Lys Leu
           355                 360                 365
   Lys Lys Ala Leu Ala Lys Tyr Asp Leu Asn Gln Asn Gln Ile Asp Ser
       370                 375                 380
   Leu Ser Lys Leu Glu Phe Lys Asp His Leu Asn Ile Ser Phe Lys Ala
   385                 390                 395                 400
   Leu Lys Leu Val Thr Pro Leu Met Leu Glu Gly Lys Lys Tyr Asp Glu
                   405                 410                 415
   Ala Cys Asn Glu Leu Asn Leu Lys Val Ala Ile Asn Glu Asp Lys Lys
               420                 425                 430
   Asp Phe Leu Pro Ala Phe Asn Glu Thr Tyr Tyr Lys Asp Glu Val Thr
           435                 440                 445
   Asn Pro Val Val Leu Arg Ala Ile Lys Glu Tyr Arg Lys Val Leu Asn
       450                 455                 460
   Ala Leu Leu Lys Lys Tyr Gly Lys Val His Lys Ile Asn Ile Glu Leu
   465                 470                 475                 480
   Ala Arg Glu Val Gly Lys Asn His Ser Gln Arg Ala Lys Ile Glu Lys
                   485                 490                 495
   Glu Gln Asn Glu Asn Tyr Lys Ala Lys Lys Asp Ala Glu Leu Glu Cys
               500                 505                 510
   Glu Lys Leu Gly Leu Lys Ile Asn Ser Lys Asn Ile Leu Lys Leu Arg
           515                 520                 525
   Leu Phe Lys Glu Gln Lys Glu Phe Cys Ala Tyr Ser Gly Glu Lys Ile
       530                 535                 540
   Lys Ile Ser Asp Leu Gln Asp Glu Lys Met Leu Glu Ile Asp His Ile
   545 550 555 560
   Tyr Pro Tyr Ser Arg Ser Phe Asp Asp Ser Tyr Met Asn Lys Val Leu
   565 570 575
   Val Phe Thr Lys Gln Asn Gln Glu Lys Leu Asn Gln Thr Pro Phe Glu
   580 585 590
   Ala Phe Gly Asn Asp Ser Ala Lys Trp Gln Lys Ile Glu Val Leu Ala
   595 600 605
   Lys Asn Leu Pro Thr Lys Lys Gln Lys Arg Ile Leu Asp Lys Asn Tyr
   610 615 620
   Lys Asp Lys Glu Gln Lys Asn Phe Lys Asp Arg Asn Leu Asn Asp Thr
   625 630 635 640
   Arg Tyr Ile Ala Arg Leu Val Leu Asn Tyr Thr Lys Asp Tyr Leu Asp
   645 650 655
   Phe Leu Pro Leu Ser Asp Asp Glu Asn Thr Lys Leu Asn Asp Thr Gln
   660 665 670
   Lys Gly Ser Lys Val His Val Glu Ala Lys Ser Gly Met Leu Thr Ser
   675 680 685
   Ala Leu Arg His Thr Trp Gly Phe Ser Ala Lys Asp Arg Asn Asn His
   690 695 700
   Leu His His Ala Ile Asp Ala Val Ile Ile Ala Tyr Ala Asn Asn Ser
   705 710 715 720
   Ile Val Lys Ala Phe Ser Asp Phe Lys Lys Glu Gln Glu Ser Asn Ser
   725 730 735
   Ala Glu Leu Tyr Ala Lys Lys Ile Ser Glu Leu Asp Tyr Lys Asn Lys
   740 745 750
   Arg Lys Phe Phe Glu Pro Phe Ser Gly Phe Arg Gln Lys Val Leu Asp
   755 760 765
   Lys Ile Asp Glu Ile Phe Val Ser Lys Pro Glu Arg Lys Lys Pro Ser
   770 775 780
   Gly Ala Leu His Glu Glu Thr Phe Arg Lys Glu Glu Glu Phe Tyr Gln
   785 790 795 800
   Ser Tyr Gly Gly Lys Glu Gly Val Leu Lys Ala Leu Glu Leu Gly Lys
   805 810 815
   Ile Arg Lys Val Asn Gly Lys Ile Val Lys Asn Gly Asp Met Phe Arg
   820 825 830
   Val Asp Ile Phe Lys His Lys Lys Thr Asn Lys Phe Tyr Ala Val Pro
   835 840 845
   Ile Tyr Thr Met Asp Phe Ala Leu Lys Val Leu Pro Asn Lys Ala Val
   850 855 860
   Ala Arg Ser Lys Lys Gly Glu Ile Lys Asp Trp Ile Leu Met Asp Glu
   865 870 875 880
   Asn Tyr Glu Phe Cys Phe Ser Leu Tyr Lys Asp Ser Leu Ile Leu Ile
   885 890 895
   Gln Thr Lys Asp Met Gln Glu Pro Glu Phe Val Tyr Tyr Asn Ala Phe
   900 905 910
   Thr Ser Ser Thr Val Ser Leu Ile Val Ser Lys His Asp Asn Lys Phe
   915 920 925
   Glu Thr Leu Ser Lys Asn Gln Lys Ile Leu Phe Lys Asn Ala Asn Glu
   930 935 940
   Lys Glu Val Ile Ala Lys Ser Ile Gly Ile Gln Asn Leu Lys Val Phe
   945 950 955 960
   Glu Lys Tyr Ile Val Ser Ala Leu Gly Glu Val Thr Lys Ala Glu Phe
   965 970 975
   Arg Gln Arg Glu Asp Phe Lys Lys
   980
<210> 65
   <211> 1187
   <212> PRT
   <213> Bifidobacterium longum
<220>
   <223> CRISPR-associated endonuclease
<308> YP_001955845
   <309> 2013-08-26
<400> 65
   Met Leu Ser Arg Gln Leu Leu Gly Ala Ser His Leu Ala Arg Pro Val
   1               5                   10                  15
   Ser Tyr Ser Tyr Asn Val Gln Asp Asn Asp Val His Cys Ser Tyr Gly
               20                  25                  30
   Glu Arg Cys Phe Met Arg Gly Lys Arg Tyr Arg Ile Gly Ile Asp Val
           35                  40                  45
   Gly Leu Asn Ser Val Gly Leu Ala Ala Val Glu Val Ser Asp Glu Asn
       50                  55                  60
   Ser Pro Val Arg Leu Leu Asn Ala Gln Ser Val Ile His Asp Gly Gly
   65                  70                  75                  80
   Val Asp Pro Gln Lys Asn Lys Glu Ala Ile Thr Arg Lys Asn Met Ser
                   85                  90                  95
   Gly Val Ala Arg Arg Thr Arg Arg Met Arg Arg Arg Lys Arg Glu Arg
               100                 105                 110
   Leu His Lys Leu Asp Met Leu Leu Gly Lys Phe Gly Tyr Pro Val Ile
           115                 120                 125
   Glu Pro Glu Ser Leu Asp Lys Pro Phe Glu Glu Trp His Val Arg Ala
       130                 135                 140
   Glu Leu Ala Thr Arg Tyr Ile Glu Asp Asp Glu Leu Arg Arg Glu Ser
   145                 150                 155                 160
   Ile Ser Ile Ala Leu Arg His Met Ala Arg His Arg Gly Trp Arg Asn
                   165                 170                 175
   Pro Tyr Arg Gln Val Asp Ser Leu Ile Ser Asp Asn Pro Tyr Ser Lys
               180                 185                 190
   Gln Tyr Gly Glu Leu Lys Glu Lys Ala Lys Ala Tyr Asn Asp Asp Ala
           195                 200                 205
   Thr Ala Ala Glu Glu Glu Ser Thr Pro Ala Gln Leu Val Val Ala Met
       210                 215                 220
   Leu Asp Ala Gly Tyr Ala Glu Ala Pro Arg Leu Arg Trp Arg Thr Gly
   225                 230                 235                 240
   Ser Lys Lys Pro Asp Ala Glu Gly Tyr Leu Pro Val Arg Leu Met Gln
                   245                 250                 255
   Glu Asp Asn Ala Asn Glu Leu Lys Gln Ile Phe Arg Val Gln Arg Val
               260                 265                 270
   Pro Ala Asp Glu Trp Lys Pro Leu Phe Arg Ser Val Phe Tyr Ala Val
           275                 280                 285
   Ser Pro Lys Gly Ser Ala Glu Gln Arg Val Gly Gln Asp Pro Leu Ala
       290                 295                 300
   Pro Glu Gln Ala Arg Ala Leu Lys Ala Ser Leu Ala Phe Gln Glu Tyr
   305                 310                 315                 320
   Arg Ile Ala Asn Val Ile Thr Asn Leu Arg Ile Lys Asp Ala Ser Ala
                   325                 330                 335
   Glu Leu Arg Lys Leu Thr Val Asp Glu Lys Gln Ser Ile Tyr Asp Gln
               340                 345                 350
   Leu Val Ser Pro Ser Ser Glu Asp Ile Thr Trp Ser Asp Leu Cys Asp
           355                 360                 365
   Phe Leu Gly Phe Lys Arg Ser Gln Leu Lys Gly Val Gly Ser Leu Thr
       370                 375                 380
   Glu Asp Gly Glu Glu Arg Ile Ser Ser Arg Pro Pro Arg Leu Thr Ser
   385                 390                 395                 400
   Val Gln Arg Ile Tyr Glu Ser Asp Asn Lys Ile Arg Lys Pro Leu Val
                   405                 410                 415
   Ala Trp Trp Lys Ser Ala Ser Asp Asn Glu His Glu Ala Met Ile Arg
               420                 425                 430
   Leu Leu Ser Asn Thr Val Asp Ile Asp Lys Val Arg Glu Asp Val Ala
           435                 440                 445
   Tyr Ala Ser Ala Ile Glu Phe Ile Asp Gly Leu Asp Asp Asp Ala Leu
       450                 455                 460
   Thr Lys Leu Asp Ser Val Asp Leu Pro Ser Gly Arg Ala Ala Tyr Ser
   465                 470                 475                 480
   Val Glu Thr Leu Gln Lys Leu Thr Arg Gln Met Leu Thr Thr Asp Asp
                   485                 490                 495
   Asp Leu His Glu Ala Arg Lys Thr Leu Phe Asn Val Thr Asp Ser Trp
               500                 505                 510
   Arg Pro Pro Ala Asp Pro Ile Gly Glu Pro Leu Gly Asn Pro Ser Val
           515                 520                 525
   Asp Arg Val Leu Lys Asn Val Asn Arg Tyr Leu Met Asn Cys Gln Gln
       530                 535                 540
   Arg Trp Gly Asn Pro Val Ser Val Asn Ile Glu His Val Arg Ser Ser
   545                 550                 555                 560
   Phe Ser Ser Val Ala Phe Ala Arg Lys Asp Lys Arg Glu Tyr Glu Lys
                   565                 570                 575
   Asn Asn Glu Lys Arg Ser Ile Phe Arg Ser Ser Leu Ser Glu Gln Leu
               580                 585                 590
   Arg Ala Asp Glu Gln Met Glu Lys Val Arg Glu Ser Asp Leu Arg Arg
           595                 600                 605
   Leu Glu Ala Ile Gln Arg Gln Asn Gly Gln Cys Leu Tyr Cys Gly Arg
       610                 615                 620
   Thr Ile Thr Phe Arg Thr Cys Glu Met Asp His Ile Val Pro Arg Lys
   625                 630                 635                 640
   Gly Val Gly Ser Thr Asn Thr Arg Thr Asn Phe Ala Ala Val Cys Ala
                   645                 650                 655
   Glu Cys Asn Arg Met Lys Ser Asn Thr Pro Phe Ala Ile Trp Ala Arg
               660                 665                 670
   Ser Glu Asp Ala Gln Thr Arg Gly Val Ser Leu Ala Glu Ala Lys Lys
           675                 680                 685
   Arg Val Thr Met Phe Thr Phe Asn Pro Lys Ser Tyr Ala Pro Arg Glu
       690                 695                 700
   Val Lys Ala Phe Lys Gln Ala Val Ile Ala Arg Leu Gln Gln Thr Glu
   705                 710                 715                 720
   Asp Asp Ala Ala Ile Asp Asn Arg Ser Ile Glu Ser Val Ala Trp Met
                   725                 730                 735
   Ala Asp Glu Leu His Arg Arg Ile Asp Trp Tyr Phe Asn Ala Lys Gln
               740                 745                 750
   Tyr Val Asn Ser Ala Ser Ile Asp Asp Ala Glu Ala Glu Thr Met Lys
           755                 760                 765
   Thr Thr Val Ser Val Phe Gln Gly Arg Val Thr Ala Ser Ala Arg Arg
       770                 775                 780
   Ala Ala Gly Ile Glu Gly Lys Ile His Phe Ile Gly Gln Gln Ser Lys
   785                 790                 795                 800
   Thr Arg Leu Asp Arg Arg His His Ala Val Asp Ala Ser Val Ile Ala
                   805                 810                 815
   Met Met Asn Thr Ala Ala Ala Gln Thr Leu Met Glu Arg Glu Ser Leu
               820                 825                 830
   Arg Glu Ser Gln Arg Leu Ile Gly Leu Met Pro Gly Glu Arg Ser Trp
           835                 840                 845
   Lys Glu Tyr Pro Tyr Glu Gly Thr Ser Arg Tyr Glu Ser Phe His Leu
       850                 855                 860
   Trp Leu Asp Asn Met Asp Val Leu Leu Glu Leu Leu Asn Asp Ala Leu
   865                 870                 875                 880
   Asp Asn Asp Arg Ile Ala Val Met Gln Ser Gln Arg Tyr Val Leu Gly
                   885                 890                 895
   Asn Ser Ile Ala His Asp Ala Thr Ile His Pro Leu Glu Lys Val Pro
               900                 905                 910
   Leu Gly Ser Ala Met Ser Ala Asp Leu Ile Arg Arg Ala Ser Thr Pro
           915                 920                 925
   Ala Leu Trp Cys Ala Leu Thr Arg Leu Pro Asp Tyr Asp Glu Lys Glu
       930                 935                 940
   Gly Leu Pro Glu Asp Ser His Arg Glu Ile Arg Val His Asp Thr Arg
   945                 950                 955                 960
   Tyr Ser Ala Asp Asp Glu Met Gly Phe Phe Ala Ser Gln Ala Ala Gln
                   965                 970                 975
   Ile Ala Val Gln Glu Gly Ser Ala Asp Ile Gly Ser Ala Ile His His
               980                 985                 990
   Ala Arg Val Tyr Arg Cys Trp Lys Thr Asn Ala Lys Gly Val Arg Lys
           995                 1000                1005
   Tyr Phe Tyr Gly Met Ile Arg Val Phe Gln Thr Asp Leu Leu Arg Ala
       1010                1015                1020
   Cys His Asp Asp Leu Phe Thr Val Pro Leu Pro Pro Gln Ser Ile Ser
   1025                1030                1035                1040
   Met Arg Tyr Gly Glu Pro Arg Val Val Gln Ala Leu Gln Ser Gly Asn
                   1045                1050                1055
   Ala Gln Tyr Leu Gly Ser Leu Val Val Gly Asp Glu Ile Glu Met Asp
               1060                1065                1070
   Phe Ser Ser Leu Asp Val Asp Gly Gln Ile Gly Glu Tyr Leu Gln Phe
           1075                1080                1085
   Phe Ser Gln Phe Ser Gly Gly Asn Leu Ala Trp Lys His Trp Val Val
       1090                1095                1100
   Asp Gly Phe Phe Asn Gln Thr Gln Leu Arg Ile Arg Pro Arg Tyr Leu
   1105                1110                1115                1120
   Ala Ala Glu Gly Leu Ala Lys Ala Phe Ser Asp Asp Val Val Pro Asp
                   1125                1130                1135
   Gly Val Gln Lys Ile Val Thr Lys Gln Gly Trp Leu Pro Pro Val Asn
               1140                1145                1150
   Thr Ala Ser Lys Thr Ala Val Arg Ile Val Arg Arg Asn Ala Phe Gly
           1155                1160                1165
   Glu Pro Arg Leu Ser Ser Ala His His Met Pro Cys Ser Trp Gln Trp
       1170                1175                1180
   Arg His Glu
   1185
<210> 66
   <211> 1270
   <212> PRT
   <213> Mycoplasma gallisepticum
<220>
   <223> CRISPR-associated endonuclease
<308> NP_853456
   <309> 2013-06-27
<400> 66
   Met Asn Asn Ser Ile Lys Ser Lys Pro Glu Val Thr Ile Gly Leu Asp
    1               5                  10                  15
   Leu Gly Val Gly Ser Val Gly Trp Ala Ile Val Asp Asn Glu Thr Asn
               20                  25                  30
   Ile Ile His His Leu Gly Ser Arg Leu Phe Ser Gln Ala Lys Thr Ala
           35                  40                  45
   Glu Asp Arg Arg Ser Phe Arg Gly Val Arg Arg Leu Ile Arg Arg Arg
       50                  55                  60
   Lys Tyr Lys Leu Lys Arg Phe Val Asn Leu Ile Trp Lys Tyr Asn Ser
   65                  70                  75                  80
   Tyr Phe Gly Phe Lys Asn Lys Glu Asp Ile Leu Asn Asn Tyr Gln Glu
                   85                  90                  95
   Gln Gln Lys Leu His Asn Thr Val Leu Asn Leu Lys Leu Glu Ala Leu
               100                 105                 110
   Asn Ala Lys Ile Asp Pro Lys Ala Leu Ser Trp Ile Leu His Asp Tyr
           115                 120                 125
   Leu Lys Asn Arg Gly His Phe Tyr Glu Asp Asn Arg Asp Phe Asn Val
       130                 135                 140
   Tyr Pro Thr Glu Glu Leu Ala Asn Tyr Phe Asp Glu Phe Gly Tyr Tyr
   145                 150                 155                 160
   Lys Gly Ile Ile Asp Ser Lys Asn Asp Asp Asp Asp Lys Leu Glu Glu
                   165                 170                 175
   Gly Leu Thr Lys Tyr Lys Phe Ser Asn Gln His Trp Leu Glu Glu Val
               180                 185                 190
   Lys Lys Val Leu Ser Asn Gln Thr Gly Leu Pro Glu Lys Phe Lys Glu
           195                 200                 205
   Glu Tyr Glu Ser Leu Phe Ser Tyr Val Arg Asn Tyr Ser Glu Gly Pro
       210                 215                 220
   Gly Ser Ile Asn Ser Val Ser Pro Tyr Gly Ile Tyr His Leu Asp Glu
   225                 230                 235                 240
   Lys Glu Gly Lys Val Val Gln Lys Tyr Asn Asn Ile Trp Asp Lys Thr
                   245                 250                 255
   Ile Gly Lys Cys Ser Ile Phe Pro Asp Glu Tyr Arg Ala Pro Lys Asn
               260                 265                 270
   Ser Pro Ile Ala Met Ile Phe Asn Glu Ile Asn Glu Leu Ser Thr Ile
           275                 280                 285
   Arg Ser Tyr Ser Ile Tyr Leu Thr Gly Trp Phe Ile Asn Gln Glu Phe
       290                 295                 300
   Lys Lys Ala Tyr Leu Asn Lys Leu Leu Asp Leu Leu Ile Lys Thr Asn
   305                 310                 315                 320
   Ser Glu Lys Pro Ile Asp Ala Arg Gln Phe Lys Lys Leu Arg Glu Glu
                   325                 330                 335
   Thr Ile Ala Glu Ser Ile Gly Lys Glu Thr Leu Lys Asp Val Glu Ser
               340                 345                 350
   Glu Glu Lys Leu Glu Lys Asp Asp His Lys Trp Lys Leu Lys Gly Leu
           355                 360                 365
   Lys Leu Asn Thr Asn Gly Lys Ile Gln Tyr Asn Asp Leu Ser Ser Leu
       370                 375                 380
   Ala Lys Phe Val His Lys Leu Lys Gln His Leu Lys Leu Asp Phe Leu
   385                 390                 395                 400
   Leu Glu Asp Gln Tyr Thr Pro Leu Asp Lys Ile Asn Phe Leu Gln Ser
                   405                 410                 415
   Leu Tyr Val Tyr Leu Gly Lys His Leu Arg Tyr Ser Asn Arg Val Asp
               420                 425                 430
   Ser Ala Asn Leu Lys Glu Phe Ser Asp Ser Ser Arg Leu Phe Glu Arg
           435                 440                 445
   Val Leu Gln Glu Gln Lys Asp Gly Leu Phe Lys Leu Phe Glu Gln Thr
       450                 455                 460
   Asp Lys Asp Asp Glu Lys Ile Leu Thr Gln Thr His Ser Leu Ser Thr
   465                 470                 475                 480
   Lys Ala Met Leu Leu Ala Ile Thr Arg Met Thr Asn Leu Asp Asn Asp
                   485                 490                 495
   Glu Asp Asn Gln Lys Asn Asn Asp Lys Gly Trp Asn Phe Glu Ala Ile
               500                 505                 510
   Lys Asn Phe Asp Gln Lys Phe Ile Asp Ile Thr Lys Thr Asn Asn Asn
           515                 520                 525
   Leu Ser Leu Lys Gln Asp Lys Arg Tyr Leu Asp Asp Gln Phe Ile Asn
       530                 535                 540
   Asp Ala Ile Leu Ser Pro Gly Val Lys Arg Ile Leu Arg Glu Ala Thr
   545                 550                 555                 560
   Lys Val Phe Asn Ala Ile Leu Lys Gln Phe Ser Glu Glu Tyr Asp Val
                   565                 570                 575
   Thr Lys Val Val Ile Glu Leu Ala Arg Glu Leu Ser Glu Glu Lys Glu
               580                 585                 590
   Leu Glu Asn Thr Lys Asn Tyr Lys Lys Leu Ile Lys Lys Asn Gly Asp
           595                 600                 605
   Lys Ile Ser Glu Gly Leu Lys Ala Leu Gly Ile Ala Glu Asp Lys Ile
       610                 615                 620
   Glu Glu Ile Leu Lys Ser Pro Thr Lys Ser Tyr Lys Val Leu Leu Trp
   625                 630                 635                 640
   Leu Gln Gln Asp His Ile Asp Pro Tyr Ser Gln Lys Glu Ile Ala Phe
                   645                 650                 655
   Asp Asp Ile Leu Thr Lys Thr Glu Lys Thr Glu Ile Asp His Ile Ile
   660 665 670
   Pro Tyr Ser Ile Ser Phe Asp Asp Ser Ser Ser Asn Lys Leu Leu Val
   675 680 685
   Leu Ala Glu Ser Asn Gln Ala Lys Ser Asn Gln Thr Pro Tyr Glu Phe
   690 695 700
   Ile Asn Ser Gly Lys Ala Glu Ile Thr Trp Glu Val Tyr Glu Ala Tyr
   705 710 715 720
   Cys His Lys Phe Lys Asn Gly Asp Ser Ser Leu Leu Asp Ser Thr Gln
   725 730 735
   Arg Ser Lys Lys Phe Ala Lys Met Met Lys Thr Asp Thr Ser Ser Lys
   740 745 750
   Tyr Asp Ile Gly Phe Leu Ala Arg Asn Leu Asn Asp Thr Arg Tyr Ala
   755 760 765
   Thr Ile Val Phe Arg Asp Ala Leu Lys Asp Tyr Ala Asn Asn His Leu
   770 775 780
   Val Glu Asp Lys Pro Met Phe Lys Val Val Cys Ile Asn Gly Gly Val
   785 790 795 800
   Thr Ser Phe Leu Arg Lys Asn Phe Asp Pro Lys Ser Trp Tyr Ala Lys
   805 810 815
   Lys Asp Arg Asp Lys Asn Ile His His Ala Val Asp Ala Ser Ile Ile
   820 825 830
   Ser Ile Phe Ser Asn Glu Thr Lys Thr Leu Phe Asn Gln Leu Thr Lys
   835 840 845
   Phe Ala Asp Tyr Lys Leu Phe Lys Asn Thr Asp Gly Ser Trp Lys Lys
   850 855 860
   Ile Asp Pro Lys Thr Gly Val Val Ser Glu Val Thr Asp Glu Asn Trp
   865 870 875 880
   Lys Gln Ile Arg Val Arg Asn Gln Val Ser Glu Ile Ala Lys Val Ile
   885 890 895
   Asp Lys Tyr Ile Gln Asp Ser Asn Ile Glu Arg Lys Ala Arg Tyr Ser
   900 905 910
   Arg Lys Ile Glu Asn Lys Thr Asn Ile Ser Leu Phe Asn Asp Thr Val
   915 920 925
   Tyr Ser Ala Lys Lys Val Gly Tyr Glu Asp Gln Ile Lys Arg Lys Asn
   930 935 940
   Leu Lys Thr Leu Asp Ile His Glu Ser Ala Glu Glu Asn Lys Asn Ser
   945 950 955 960
   Lys Val Lys Lys Gln Phe Val Tyr Arg Lys Leu Val Asn Val Ser Leu
   965 970 975
   Leu Asn Asn Asp Lys Leu Ala Asp Leu Phe Ala Glu Lys Glu Asp Ile
   980 985 990
   Leu Met Tyr Arg Ala Asn Pro Trp Val Ile Asn Leu Ala Glu Gln Ile
   995 1000 1005
   Phe Asn Glu Tyr Thr Glu Asn Lys Lys Ile Lys Ser Gln Asn Val Phe
   1010 1015 1020
   Glu Lys Tyr Met Leu Asp Leu Thr Lys Glu Phe Pro Glu Lys Phe Ser
   1025 1030 1035 1040
   Glu Ala Phe Val Lys Ser Met Ile Arg Asn Lys Thr Ala Ile Ile Tyr
   1045 1050 1055
   Asn Val Glu Lys Asp Val Val His Arg Ile Lys Arg Leu Lys Ile Leu
   1060 1065 1070
   Ser Ser Glu Leu Lys Glu Asn Lys Trp Ser Asn Val Ile Ile Arg Ser
   1075 1080 1085
   Lys Asn Glu Ser Gly Thr Lys Leu Ser Tyr Gln Asp Thr Ile Asn Ser
   1090 1095 1100
   Ile Ala Leu Met Ile Met Arg Ser Ile Asp Pro Thr Ala Lys Lys Gln
   1105 1110 1115 1120
   Tyr Ile Arg Val Pro Leu Asn Thr Leu Asn Leu His Leu Gly Asp Gln
   1125 1130 1135
   Asp Phe Asp Leu His Asn Ile Asp Ala Tyr Leu Lys Lys Pro Lys Phe
   1140 1145 1150
   Val Lys Tyr Leu Lys Ala Asn Glu Ile Gly Asp Glu Tyr Lys Pro Trp
           1155                1160                1165
   Arg Val Leu Thr Ser Gly Thr Leu Leu Ile His Lys Lys Asp Lys Lys
       1170                1175                1180
   Leu Met Tyr Ile Ser Ser Phe Gln Asn Leu Asn Asp Leu Ile Glu Ile
   1185                1190                1195                1200
   Lys Asn Leu Ile Glu Thr Glu Tyr Lys Glu Asn Val Asp Ser Asp Pro
                   1205                1210                1215
   Lys Lys Lys Lys Lys Ala Ser Gln Ile Leu Arg Ser Leu Ser Val Ile
               1220                1225                1230
   Leu Asn Asp Tyr Ile Leu Leu Asp Ala Lys Tyr Asn Phe Asp Ile Leu
           1235                1240                1245
   Gly Leu Ser Lys Asn Lys Ile Asp Glu Ile Leu Asn Ser Lys Leu Asp
       1250                1255                1260
   Leu Asp Lys Ile Ala Lys
   1265                1270
<210> 67
   <211> 1195
   <212> PRT
   <213> Elusimicrobium minutum
<220>
   <223> CRISPR-associated endonuclease
<308> YP_001875142
   <309> 2013-06-10
<400> 67
   Met Gln Lys Asn Ile Asn Thr Lys Gln Asn His Ile Tyr Ile Lys Gln
    1               5                  10                  15
   Ala Gln Lys Ile Lys Glu Lys Leu Gly Asp Lys Pro Tyr Arg Ile Gly
               20                  25                  30
   Leu Asp Leu Gly Val Gly Ser Ile Gly Phe Ala Ile Val Ser Met Glu
           35                  40                  45
   Glu Asn Asp Gly Asn Val Leu Leu Pro Lys Glu Ile Ile Met Val Gly
       50                  55                  60
   Ser Arg Ile Phe Lys Ala Ser Ala Gly Ala Ala Asp Arg Lys Leu Ser
   65                  70                  75                  80
   Arg Gly Gln Arg Asn Asn His Arg His Thr Arg Glu Arg Met Arg Tyr
                   85                  90                  95
   Leu Trp Lys Val Leu Ala Glu Gln Lys Leu Ala Leu Pro Val Pro Ala
               100                 105                 110
   Asp Leu Asp Arg Lys Glu Asn Ser Ser Glu Gly Glu Thr Ser Ala Lys
           115                 120                 125
   Arg Phe Leu Gly Asp Val Leu Gln Lys Asp Ile Tyr Glu Leu Arg Val
       130                 135                 140
   Lys Ser Leu Asp Glu Arg Leu Ser Leu Gln Glu Leu Gly Tyr Val Leu
   145                 150                 155                 160
   Tyr His Ile Ala Gly His Arg Gly Ser Ser Ala Ile Arg Thr Phe Glu
                   165                 170                 175
   Asn Asp Ser Glu Glu Ala Gln Lys Glu Asn Thr Glu Asn Lys Lys Ile
               180                 185                 190
   Ala Gly Asn Ile Lys Arg Leu Met Ala Lys Lys Asn Tyr Arg Thr Tyr
           195                 200                 205
   Gly Glu Tyr Leu Tyr Lys Glu Phe Phe Glu Asn Lys Glu Lys His Lys
       210                 215                 220
   Arg Glu Lys Ile Ser Asn Ala Ala Asn Asn His Lys Phe Ser Pro Thr
   225                 230                 235                 240
   Arg Asp Leu Val Ile Lys Glu Ala Glu Ala Ile Leu Lys Lys Gln Ala
                   245                 250                 255
   Gly Lys Asp Gly Phe His Lys Glu Leu Thr Glu Glu Tyr Ile Glu Lys
               260                 265                 270
   Leu Thr Lys Ala Ile Gly Tyr Glu Ser Glu Lys Leu Ile Pro Glu Ser
               275                 280                 285
   Gly Phe Cys Pro Tyr Leu Lys Asp Glu Lys Arg Leu Pro Ala Ser His
       290                 295                 300
   Lys Leu Asn Glu Glu Arg Arg Leu Trp Glu Thr Leu Asn Asn Ala Arg
   305                 310                 315                 320
   Tyr Ser Asp Pro Ile Val Asp Ile Val Thr Gly Glu Ile Thr Gly Tyr
                   325                 330                 335
   Tyr Glu Lys Gln Phe Thr Lys Glu Gln Lys Gln Lys Leu Phe Asp Tyr
               340                 345                 350
   Leu Leu Thr Gly Ser Glu Leu Thr Pro Ala Gln Thr Lys Lys Leu Leu
           355                 360                 365
   Gly Leu Lys Asn Thr Asn Phe Glu Asp Ile Ile Leu Gln Gly Arg Asp
       370                 375                 380
   Lys Lys Ala Gln Lys Ile Lys Gly Tyr Lys Leu Ile Lys Leu Glu Ser
   385                 390                 395                 400
   Met Pro Phe Trp Ala Arg Leu Ser Glu Ala Gln Gln Asp Ser Phe Leu
                   405                 410                 415
   Tyr Asp Trp Asn Ser Cys Pro Asp Glu Lys Leu Leu Thr Glu Lys Leu
               420                 425                 430
   Ser Asn Glu Tyr His Leu Thr Glu Glu Glu Ile Asp Asn Ala Phe Asn
           435                 440                 445
   Glu Ile Val Leu Ser Ser Ser Tyr Ala Pro Leu Gly Lys Ser Ala Met
       450                 455                 460
   Leu Ile Ile Leu Glu Lys Ile Lys Asn Asp Leu Ser Tyr Thr Glu Ala
   465                 470                 475                 480
   Val Glu Glu Ala Leu Lys Glu Gly Lys Leu Thr Lys Glu Lys Gln Ala
                   485                 490                 495
   Ile Lys Asp Arg Leu Pro Tyr Tyr Gly Ala Val Leu Gln Glu Ser Thr
               500                 505                 510
   Gln Lys Ile Ile Ala Lys Gly Phe Ser Pro Gln Phe Lys Asp Lys Gly
           515                 520                 525
   Tyr Lys Thr Pro His Thr Asn Lys Tyr Glu Leu Glu Tyr Gly Arg Ile
       530                 535                 540
   Ala Asn Pro Val Val His Gln Thr Leu Asn Glu Leu Arg Lys Leu Val
   545                 550                 555                 560
   Asn Glu Ile Ile Asp Ile Leu Gly Lys Lys Pro Cys Glu Ile Gly Leu
                   565                 570                 575
   Glu Thr Ala Arg Glu Leu Lys Lys Ser Ala Glu Asp Arg Ser Lys Leu
               580                 585                 590
   Ser Arg Glu Gln Asn Asp Asn Glu Ser Asn Arg Asn Arg Ile Tyr Glu
           595                 600                 605
   Ile Tyr Ile Arg Pro Gln Gln Gln Val Ile Ile Thr Arg Arg Glu Asn
       610                 615                 620
   Pro Arg Asn Tyr Ile Leu Lys Phe Glu Leu Leu Glu Glu Gln Lys Ser
   625                 630                 635                 640
   Gln Cys Pro Phe Cys Gly Gly Gln Ile Ser Pro Asn Asp Ile Ile Asn
                   645                 650                 655
   Asn Gln Ala Asp Ile Glu His Leu Phe Pro Ile Ala Glu Ser Glu Asp
               660                 665                 670
   Asn Gly Arg Asn Asn Leu Val Ile Ser His Ser Ala Cys Asn Ala Asp
           675                 680                 685
   Lys Ala Lys Arg Ser Pro Trp Ala Ala Phe Ala Ser Ala Ala Lys Asp
       690                 695                 700
   Ser Lys Tyr Asp Tyr Asn Arg Ile Leu Ser Asn Val Lys Glu Asn Ile
   705                 710                 715                 720
   Pro His Lys Ala Trp Arg Phe Asn Gln Gly Ala Phe Glu Lys Phe Ile
                   725                 730                 735
   Glu Asn Lys Pro Met Ala Ala Arg Phe Lys Thr Asp Asn Ser Tyr Ile
               740                 745                 750
   Ser Lys Val Ala His Lys Tyr Leu Ala Cys Leu Phe Glu Lys Pro Asn
           755                 760                 765
   Ile Ile Cys Val Lys Gly Ser Leu Thr Ala Gln Leu Arg Met Ala Trp
       770                 775                 780
   Gly Leu Gln Gly Leu Met Ile Pro Phe Ala Lys Gln Leu Ile Thr Glu
   785                 790                 795                 800
   Lys Glu Ser Glu Ser Phe Asn Lys Asp Val Asn Ser Asn Lys Lys Ile
                   805                 810                 815
   Arg Leu Asp Asn Arg His His Ala Leu Asp Ala Ile Val Ile Ala Tyr
               820                 825                 830
   Ala Ser Arg Gly Tyr Gly Asn Leu Leu Asn Lys Met Ala Gly Lys Asp
           835                 840                 845
   Tyr Lys Ile Asn Tyr Ser Glu Arg Asn Trp Leu Ser Lys Ile Leu Leu
       850                 855                 860
   Pro Pro Asn Asn Ile Val Trp Glu Asn Ile Asp Ala Asp Leu Glu Ser
   865                 870                 875                 880
   Phe Glu Ser Ser Val Lys Thr Ala Leu Lys Asn Ala Phe Ile Ser Val
                   885                 890                 895
   Lys His Asp His Ser Asp Asn Gly Glu Leu Val Lys Gly Thr Met Tyr
               900                 905                 910
   Lys Ile Phe Tyr Ser Glu Arg Gly Tyr Thr Leu Thr Thr Tyr Lys Lys
           915                 920                 925
   Leu Ser Ala Leu Lys Leu Thr Asp Pro Gln Lys Lys Lys Thr Pro Lys
       930                 935                 940
   Asp Phe Leu Glu Thr Ala Leu Leu Lys Phe Lys Gly Arg Glu Ser Glu
   945                 950                 955                 960
   Met Lys Asn Glu Lys Ile Lys Ser Ala Ile Glu Asn Asn Lys Arg Leu
                   965                 970                 975
   Phe Asp Val Ile Gln Asp Asn Leu Glu Lys Ala Lys Lys Leu Leu Glu
               980                 985                 990
   Glu Glu Asn Glu Lys Ser Lys Ala Glu Gly Lys Lys Glu Lys Asn Ile
           995                 1000                1005
   Asn Asp Ala Ser Ile Tyr Gln Lys Ala Ile Ser Leu Ser Gly Asp Lys
       1010                1015                1020
   Tyr Val Gln Leu Ser Lys Lys Glu Pro Gly Lys Phe Phe Ala Ile Ser
   1025                1030                1035                1040
   Lys Pro Thr Pro Thr Thr Thr Gly Tyr Gly Tyr Asp Thr Gly Asp Ser
                   1045                1050                1055
   Leu Cys Val Asp Leu Tyr Tyr Asp Asn Lys Gly Lys Leu Cys Gly Glu
               1060                1065                1070
   Ile Ile Arg Lys Ile Asp Ala Gln Gln Lys Asn Pro Leu Lys Tyr Lys
           1075                1080                1085
   Glu Gln Gly Phe Thr Leu Phe Glu Arg Ile Tyr Gly Gly Asp Ile Leu
       1090                1095                1100
   Glu Val Asp Phe Asp Ile His Ser Asp Lys Asn Ser Phe Arg Asn Asn
   1105                1110                1115                1120
   Thr Gly Ser Ala Pro Glu Asn Arg Val Phe Ile Lys Val Gly Thr Phe
                   1125                1130                1135
   Thr Glu Ile Thr Asn Asn Asn Ile Gln Ile Trp Phe Gly Asn Ile Ile
               1140                1145                1150
   Lys Ser Thr Gly Gly Gln Asp Asp Ser Phe Thr Ile Asn Ser Met Gln
           1155                1160                1165
   Gln Tyr Asn Pro Arg Lys Leu Ile Leu Ser Ser Cys Gly Phe Ile Lys
       1170                1175                1180
   Tyr Arg Ser Pro Ile Leu Lys Asn Lys Glu Gly
   1185                1190                1195
<210> 68
   <211> 1125
   <212> PRT
   <213> Francisella tularensis
<220>
   <223> CRISPR-associated endonuclease
<308> YP_001122287
   <309> 2013-06-10
<400> 68
   Met Asn Val Lys Ile Leu Pro Ile Ala Ile Asp Leu Asp Val Lys Asn
    1               5                  10                  15
   Thr Gly Val Phe Ser Ala Phe Tyr Gln Lys Gly Thr Ser Leu Glu Lys
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Arg Arg His Gln Arg Arg Gly
       50                  55                  60
   Ile Asp Arg Lys Gln Leu Val Lys Arg Leu Phe Lys Leu Val Trp Thr
   65                  70                  75                  80
   Glu Gln Leu Asn Leu Glu Trp Asp Lys Asp Thr Gln Gln Ala Ile Ser
                   85                  90                  95
   Phe Leu Phe Asn Arg Arg Gly Phe Ser Phe Ile Thr Asp Gly Tyr Ser
               100                 105                 110
   Thr Glu Tyr Leu Asn Ile Val Pro Glu Gln Val Lys Ala Ile Leu Met
           115                 120                 125
   Asp Ile Phe Asp Asp Tyr Asn Gly Glu Asp Asp Leu Asp Ser Tyr Leu
       130                 135                 140
   Lys Leu Ala Thr Glu Gln Glu Ser Lys Ile Ser Glu Ile Tyr Asn Lys
   145                 150                 155                 160
   Leu Met Gln Lys Ile Leu Glu Phe Lys Leu Arg Lys Leu Cys Thr Asp
                   165                 170                 175
   Ile Lys Asp Asp Lys Val Ser Thr Lys Thr Leu Lys Glu Ile Thr Ser
               180                 185                 190
   Tyr Glu Phe Glu Leu Leu Ala Asp Tyr Leu Ala Asn Tyr Ser Glu Ser
           195                 200                 205
   Leu Lys Ile Gln Lys Phe Ser Tyr Thr Asp Lys Gln Gly Asn Leu Lys
       210                 215                 220
   Glu Leu Ser Tyr Tyr His His Asp Lys Tyr Asn Ile Gln Glu Phe Leu
   225                 230                 235                 240
   Lys Arg His Ala Thr Ile Asn Asp Glu Ile Leu Asp Thr Leu Leu Thr
                   245                 250                 255
   Asp Asp Phe Asp Ile Trp Asn Phe Asn Phe Glu Lys Phe Asp Phe Asp
               260                 265                 270
   Lys Asn Glu Glu Lys Leu Gln Asn Gln Glu Asp Lys Asp His Thr Gln
           275                 280                 285
   Ala His Leu His His Phe Val Phe Ala Val Asn Lys Ile Lys Ser Glu
       290                 295                 300
   Met Ala Ser Gly Gly Arg His Arg Ser Gln Tyr Phe Gln Glu Ile Thr
   305                 310                 315                 320
   Asn Val Leu Asp Glu Asn Asn His Gln Glu Gly Tyr Leu Lys Asn Phe
                   325                 330                 335
   Cys Glu Asn Leu His Asn Lys Lys Tyr Ser Asn Leu Ser Val Lys Asn
               340                 345                 350
   Leu Val Asn Leu Val Gly Asn Leu Ser Asn Leu Glu Leu Lys Pro Leu
           355                 360                 365
   Arg Lys Tyr Phe Asn Asp Lys Ile His Ala Lys Ala Asp His Trp Asp
       370                 375                 380
   Glu Gln Lys Phe Thr Glu Thr Tyr Cys His Trp Ile Leu Gly Glu Trp
   385                 390                 395                 400
   Arg Val Gly Val Lys Asp Gln Asp Lys Lys Asp Gly Ala Lys Tyr Ser
                   405                 410                 415
   Tyr Lys Asp Leu Cys Asn Glu Leu Lys Gln Lys Val Thr Lys Ala Gly
               420                 425                 430
   Leu Ile Asp Phe Leu Leu Glu Leu Asp Pro Cys Arg Thr Ile Pro Pro
           435                 440                 445
   Tyr Leu Asp Asn Asn Asn Arg Lys Pro Pro Lys Cys Gln Ser Leu Ile
       450                 455                 460
   Leu Asn Pro Lys Phe Leu Asp Asn Gln Tyr Pro Asn Trp Gln Gln Tyr
   465 470 475 480
   Leu Gln Glu Leu Lys Lys Leu Gln Ser Ile Gln Asp Tyr Leu Asp Ser
   485 490 495
   Phe Glu Thr Asp Leu Lys Asp Leu Lys Ser Ser Lys Asp Gln Pro Tyr
   500 505 510
   Phe Val Glu Tyr Lys Ser Ser Asn Gln Gln Met Ala Ser Gly Gln Arg
   515 520 525
   Asp Tyr Lys Asp Leu Asp Ala Arg Ile Leu Gln Phe Ile Phe Asp Arg
   530 535 540
   Val Lys Ala Ser Asp Glu Leu Leu Leu Asn Glu Ile Tyr Phe Gln Ala
   545 550 555 560
   Lys Lys Leu Lys Gln Lys Ala Ser Ser Glu Leu Glu Lys Leu Glu Ser
   565 570 575
   Ser Lys Lys Leu Asp Glu Val Ile Ala Asn Ser Gln Leu Ser Gln Ile
   580 585 590
   Leu Lys Ser Gln His Thr Asn Gly Ile Phe Glu Gln Gly Thr Phe Leu
   595 600 605
   His Leu Val Cys Lys Tyr Tyr Lys Gln Arg Gln Arg Ala Arg Asp Ser
   610 615 620
   Arg Leu Tyr Ile Met Pro Glu Tyr Arg Tyr Asp Lys Lys Leu Asp Lys
   625 630 635 640
   Tyr Asn Asn Thr Gly Arg Phe Asp Asp Asn Asn Gln Leu Leu Thr Tyr
   645 650 655
   Cys Asn His Lys Pro Arg Gln Lys Arg Tyr Gln Leu Leu Asn Asp Leu
   660 665 670
   Ala Gly Val Leu Gln Val Ser Arg Asn Gln Leu Leu Ser Ser Val Glu
   675 680 685
   Glu Trp Phe Gln Gln Ala Gln Arg Val Gly Glu Ile Ser Lys Ser Gln
   690 695 700
   Asp Glu Gln Ile Phe Glu Trp Leu Lys Ser Phe Lys Ile Ala Ser Tyr
   705 710 715 720
   Cys Lys Ala Ala Val Glu Met Gln Lys Gln Tyr Arg Gly Thr Leu Lys
   725 730 735
   Asn Ala Ile Gln Thr Ala Ile Phe Arg Gln Ser Glu Asn Ile Asn Lys
   740 745 750
   Asn Lys Asn Lys Asn Thr Gly Asn Gln Gln Gln Ala Leu Ser Glu Asn
   755 760 765
   Ser Lys Asp Val Lys Ser Leu Thr Ala Asp Glu Lys Lys Leu Leu Lys
   770 775 780
   Leu Ile Glu Asn Ile Ala Lys Ala Ser Gln Lys Ile Gly Glu Ser Leu
   785 790 795 800
   Gly Leu Asn Asp Lys Gln Ile Lys Lys Phe Asn Ser Ile Tyr Ser Phe
   805 810 815
   Ala Gln Ile Gln Gln Ile Ala Phe Ala Glu Arg Lys Gly Asn Ala Asn
   820 825 830
   Thr Cys Ala Val Cys Ser Ala Asp Asn Ala His Arg Met Gln Gln Ile
   835 840 845
   Lys Ile Thr Glu Leu Val Glu Asp Asn Lys Asp Asn Ile Ile Leu Ser
   850 855 860
   Ala Lys Ala Gln Arg Leu Pro Ala Ile Pro Thr Arg Ile Val Asp Gly
   865 870 875 880
   Ala Val Lys Lys Met Ala Thr Ile Leu Ala Lys Asn Ile Val Asp Asp
   885 890 895
   Asn Trp Gln Asn Ile Lys Gln Val Leu Ser Ala Lys His Gln Leu His
   900 905 910
   Ile Pro Ile Ile Thr Glu Ser Asn Ala Phe Glu Phe Glu Pro Ala Leu
   915 920 925
   Ala Asp Val Lys Gly Lys Ser Leu Lys Asp Arg Arg Lys Lys Ala Leu
   930 935 940
   Glu Arg Ile Ser Pro Glu Asn Ile Phe Lys Asp Lys Asn Asn Arg Ile
   945 950 955 960
   Lys Glu Phe Ala Lys Gly Ile Ser Ala Tyr Ser Gly Ala Asn Leu Thr
   965 970 975
   Asp Gly Asp Phe Asp Gly Ala Lys Glu Glu Leu Asp His Ile Ile Pro
               980                 985                 990
   Arg Ser His Lys Lys Tyr Gly Thr Leu Asn Asp Glu Ala Asn Leu Ile
           995                 1000                1005
   Cys Val Thr Arg Asp Asp Asn Lys Asn Ile Phe Ala Ile Asp Thr Ser
       1010                1015                1020
   Lys Trp Phe Glu Ile Glu Thr Pro Ser Asp Leu Arg Asp Ile Gly Val
   1025                1030                1035                1040
   Ala Thr Ile Gln Tyr Lys Ile Asp Asn Asn Ser Arg Pro Lys Val Arg
                   1045                1050                1055
   Val Lys Leu Asp Tyr Val Ile Asp Asp Asp Ser Lys Ile Asn Tyr Phe
               1060                1065                1070
   Met Asn His Ser Leu Leu Lys Ser Arg Tyr Pro Asp Lys Val Leu Glu
           1075                1080                1085
   Ile Leu Lys Gln Ser Thr Ile Ile Glu Phe Glu Ser Ser Gly Phe Asn
       1090                1095                1100
   Lys Thr Ile Lys Glu Met Leu Gly Met Thr Leu Ala Gly Ile Tyr Asn
   1105                1110                1115                1120
   Glu Thr Ser Asn Asn
                   1125
<210> 69
   <211> 1130
   <212> PRT
   <213> Streptococcus macedonicus
<220>
   <223> CRISPR-associated endonuclease
<308> YP_005095062
   <309> 2013-06-11
<400> 69
   Met Thr Asn Gly Lys Ile Leu Gly Leu Asp Ile Gly Ile Ala Ser Val
    1               5                  10                  15
   Gly Val Gly Ile Ile Glu Ala Lys Thr Gly Lys Val Val His Ala Asn
               20                  25                  30
   Ser Arg Leu Phe Ser Ala Ala Asn Ala Glu Asn Asn Ala Glu Arg Arg
           35                  40                  45
   Gly Phe Arg Gly Ser Arg Arg Leu Asn Arg Arg Lys Lys His Arg Val
       50                  55                  60
   Lys Arg Val Arg Asp Leu Phe Glu Lys Tyr Gly Ile Val Thr Asp Phe
   65                  70                  75                  80
   Arg Asn Leu Asn Leu Asn Pro Tyr Glu Leu Arg Val Lys Gly Leu Thr
                   85                  90                  95
   Glu Gln Leu Thr Asn Glu Glu Leu Phe Ala Ala Leu Arg Thr Ile Ser
               100                 105                 110
   Lys Arg Arg Gly Ile Ser Tyr Leu Asp Asp Ala Glu Asp Asp Ser Thr
           115                 120                 125
   Gly Ser Thr Asp Tyr Ala Lys Ser Ile Asp Glu Asn Arg Arg Leu Leu
       130                 135                 140
   Lys Thr Lys Thr Pro Gly Gln Ile Gln Leu Glu Arg Leu Glu Lys Tyr
   145                 150                 155                 160
   Gly Gln Leu Arg Gly Asn Phe Thr Val Tyr Asp Glu Asn Gly Glu Ala
                   165                 170                 175
   His Arg Leu Ile Asn Val Phe Ser Thr Ser Asp Tyr Glu Lys Glu Ala
               180                 185                 190
   Arg Lys Ile Leu Glu Thr Gln Ala Asp Tyr Asn Lys Lys Ile Thr Ala
           195                 200                 205
   Glu Phe Ile Asp Asp Tyr Val Glu Ile Leu Thr Gln Lys Arg Lys Tyr
       210                 215                 220
   Tyr His Gly Pro Gly Asn Glu Lys Ser Arg Thr Asp Tyr Gly Arg Phe
   225 230 235 240
   Arg Thr Asp Gly Thr Thr Leu Glu Asn Ile Phe Gly Ile Leu Ile Gly
   245 250 255
   Lys Cys Asn Phe Tyr Pro Asp Glu Tyr Arg Ala Ser Lys Ala Ser Tyr
   260 265 270
   Thr Ala Gln Glu Tyr Asn Phe Leu Asn Asp Leu Asn Asn Leu Lys Val
   275 280 285
   Pro Thr Glu Thr Gly Lys Leu Ser Thr Glu Gln Lys Glu Ser Leu Val
   290 295 300
   Glu Phe Ala Lys Asn Thr Ala Ile Leu Gly Pro Ala Lys Leu Leu Lys
   305 310 315 320
   Glu Ile Ala Lys Ile Leu Asp Cys Lys Val Asp Glu Ile Lys Gly Tyr
   325 330 335
   Arg Glu Asp Asp Lys Gly Lys Pro Asp Leu His Thr Phe Glu Pro Tyr
   340 345 350
   Arg Lys Leu Lys Phe Asn Leu Asp Ser Val Asn Ile Asp Tyr Leu Ser
   355 360 365
   Arg Glu Val Leu Asp Lys Leu Ala Asp Ile Leu Thr Leu Asn Thr Glu
   370 375 380
   Arg Glu Gly Ile Glu Asp Ala Ile Lys Arg Asn Leu Pro Asn Gln Phe
   385 390 395 400
   Thr Glu Glu Gln Ile Ser Glu Ile Ile Asn Val Arg Lys Ser Gln Ser
   405 410 415
   Thr Ala Phe Asn Lys Gly Trp His Ser Phe Ser Ala Lys Leu Met Asn
   420 425 430
   Glu Leu Ile Pro Glu Leu Tyr Ala Thr Ser Asp Glu Gln Met Thr Ile
   435 440 445
   Leu Thr Arg Leu Glu Lys Phe Lys Val Lys Lys Lys Ser Ser Lys Asn
   450 455 460
   Ile Lys Thr Ile Asp Glu Lys Glu Ile Thr Asp Glu Ile Tyr Asn Pro
   465 470 475 480
   Val Val Ala Lys Ser Val Arg Gln Thr Ile Lys Ile Ile Asn Ala Ala
   485 490 495
   Val Lys Lys Tyr Gly Asp Phe Asp Lys Ile Val Ile Glu Met Pro Arg
   500 505 510
   Asp Lys Asn Ala Asp Asp Glu Lys Lys Phe Ile Asn Lys Arg Asn Lys
   515 520 525
   Glu Asn Lys Lys Glu Lys Asp Asp Ala Leu Lys Arg Ala Ala Tyr Leu
   530 535 540
   Tyr Asn Gly Thr Asp Lys Leu Pro Asp Glu Val Phe His Gly Asn Lys
   545 550 555 560
   Gln Leu Glu Thr Lys Ile Arg Leu Trp Tyr Gln Gln Gly Glu Arg Cys
   565 570 575
   Leu Tyr Ser Gly Lys Pro Ile Pro Ile Gln Glu Leu Val His Asn Ser
   580 585 590
   Asn Asn Phe Glu Ile Asp His Ile Leu Pro Leu Ser Leu Ser Phe Asp
   595 600 605
   Asp Ser Leu Ala Asn Lys Val Leu Val Tyr Ser Trp Thr Asn Gln Glu
   610 615 620
   Lys Gly Gln Lys Thr Pro Tyr Gln Val Ile Asp Ser Met Asp Ala Ala
   625 630 635 640
   Trp Ser Phe Arg Glu Met Lys Glu Tyr Val Leu Lys Gln Lys Gly Leu
   645 650 655
   Gly Lys Lys Lys Arg Asp Tyr Leu Leu Thr Thr Glu Asn Ile Asp Lys
   660 665 670
   Ile Glu Val Lys Lys Lys Phe Ile Glu Arg Asn Leu Val Asp Thr Arg
   675 680 685
   Tyr Ala Ser Arg Val Val Leu Asn Ser Leu Gln Ser Ala Leu Arg Glu
   690 695 700
   Leu Gly Lys Asp Thr Lys Val Ser Val Val Arg Gly Gln Phe Thr Ser
   705 710 715 720
   Gln Leu Arg Arg Lys Trp Lys Ile Asp Lys Ser Arg Glu Thr Tyr His
   725 730 735
   His His Ala Val Asp Ala Leu Ile Ile Ala Ala Ser Ser Gln Leu Lys
   740 745 750
   Leu Trp Glu Lys Gln Asp Asn Pro Met Phe Val Asp Tyr Gly Asn Asn
   755 760 765
   Gln Val Val Asp Lys Gln Thr Gly Glu Ile Leu Ser Val Ser Asp Asn
   770 775 780
   Glu Tyr Lys Glu Leu Val Phe Gln Pro Pro Tyr Gln Gly Phe Val Asn
   785 790 795 800
   Thr Ile Ser Ser Lys Gly Phe Glu Asp Glu Ile Leu Phe Ser Tyr Gln
   805 810 815
   Val Asp Ser Lys Tyr Asn Arg Lys Val Ser Asp Ala Thr Ile Tyr Ser
   820 825 830
   Thr Arg Lys Ala Lys Ile Gly Lys Asp Lys Lys Glu Glu Thr Tyr Val
   835 840 845
   Leu Gly Lys Ile Lys Asp Ile Tyr Ser Gln Asn Gly Phe Asp Thr Phe
   850 855 860
   Val Lys Lys Tyr Asn Lys Asp Lys Thr Gln Phe Leu Met Tyr Gln Lys
   865 870 875 880
   Asp Pro Leu Thr Trp Glu Asn Val Ile Glu Val Ile Leu Gly Asp Tyr
   885 890 895
   Pro Thr Thr Lys Lys Ser Glu Asp Gly Lys Asn Asp Val Lys Cys Asn
   900 905 910
   Pro Phe Glu Glu Tyr Arg Arg Glu Asn Gly Leu Ile Cys Lys Tyr Ser
   915 920 925
   Lys Lys Gly Lys Gly Thr Pro Ile Lys Ser Leu Lys Tyr Tyr Asp Lys
   930 935 940
   Lys Leu Gly Asn Cys Ile Asp Ile Thr Pro Glu Glu Ser Arg Asn Lys
   945 950 955 960
   Val Ile Leu Gln Ser Ile Asn Pro Trp Arg Ala Asp Val Tyr Phe Asn
   965 970 975
   Pro Glu Thr Leu Lys Tyr Glu Leu Met Gly Leu Lys Tyr Ser Asp Leu
   980 985 990
   Ser Phe Glu Lys Gly Thr Gly Asn Tyr His Ile Ser Gln Glu Lys Tyr
   995 1000 1005
   Asp Ala Ile Lys Glu Lys Glu Gly Ile Gly Lys Lys Ser Glu Phe Lys
   1010 1015 1020
   Phe Thr Leu Tyr Arg Asn Asp Leu Ile Leu Ile Lys Asp Ile Ala Ser
   1025 1030 1035 1040
   Gly Glu Gln Glu Ile Tyr Arg Phe Leu Ser Arg Thr Met Pro Asn Val
   1045 1050 1055
   Asn His Tyr Val Glu Leu Lys Pro Tyr Asp Lys Glu Lys Phe Asp Asn
   1060 1065 1070
   Val Gln Glu Leu Val Glu Ala Leu Gly Glu Ala Asp Lys Val Gly Arg
   1075 1080 1085
   Cys Ile Lys Ser Leu Asn Lys Pro Asn Ile Ser Ile Tyr Lys Val Arg
   1090 1095 1100
   Thr Asp Val Leu Gly Asn Lys Tyr Phe Val Lys Lys Glu Gly Asp Lys
   1105 1110 1115 1120
   Pro Lys Leu Asp Phe Lys Asn Asn Lys Lys
   1125 1130
<210> 70
   <211> 1122
   <212> PRT
   <213> Streptococcus suis
<220>
   <223> CRISPR-associated endonuclease
<308> YP_004401929
   <309> 2013-07-06
<400> 70
   Met Ser Asn Gly Lys Ile Leu Gly Leu Asp Ile Gly Ile Ala Ser Val
    1               5                  10                  15
   Gly Val Gly Val Ile Asp Ala Gln Thr Gly Glu Ile Ile His Ala Ser
               20                  25                  30
   Ser Arg Ile Phe Pro Ser Ala Asn Ala Ala Asn Asn Ala Glu Arg Arg
           35                  40                  45
   Thr Phe Arg Gly Ser Arg Arg Leu Ile Arg Arg Lys Lys His Arg Ile
       50                  55                  60
   Lys Arg Leu Asp Asp Leu Phe Asn Asp Phe His Ile Asn Leu Asp Gly
   65                  70                  75                  80
   Glu Met Ser Thr Asp Asn Pro Tyr Val Leu Arg Val Lys Gly Leu Ser
                   85                  90                  95
   Gln Lys Leu Thr Val Glu Glu Leu Tyr Ile Ser Ile Lys Asn Ile Met
               100                 105                 110
   Lys Arg Arg Gly Ile Ser Tyr Leu Asp Asp Ala Glu Ser Asp Asn Glu
           115                 120                 125
   Ala Gly Arg Ser Asp Tyr Ala Lys Ala Ile Glu Arg Asn Arg Gln Leu
       130                 135                 140
   Leu Thr Ser Lys Thr Pro Gly Glu Ile Gln Leu Glu Arg Leu Glu Lys
   145                 150                 155                 160
   Tyr Gly Gln Leu Arg Gly Asn Phe Thr Ile Ile Asp Glu Glu Gly Gln
                   165                 170                 175
   Ser Gln Gln Ile Ile Asn Val Phe Ser Thr Ser Asp Tyr Val Lys Glu
               180                 185                 190
   Val Glu Lys Ile Leu Asp Cys Gln Lys Met Tyr His Lys Phe Ile Ser
           195                 200                 205
   Asp Glu Phe Cys Asp Lys Leu Ile Glu Leu Leu Arg Glu Lys Arg Lys
       210                 215                 220
   Tyr Tyr Val Gly Pro Gly Asn Glu Lys Ser Arg Thr Asp Tyr Gly Ile
   225                 230                 235                 240
   Tyr Arg Thr Asp Gly Thr Thr Leu Glu Asn Leu Phe Gly Ile Leu Ile
                   245                 250                 255
   Gly Lys Cys Thr Phe Tyr Pro Asp Gln Tyr Arg Ser Ser Arg Ala Ser
               260                 265                 270
   Tyr Thr Ala Gln Glu Phe Asn Phe Leu Asn Asp Leu Asn Asn Leu Thr
           275                 280                 285
   Val Pro Thr Glu Thr Lys Lys Leu Ser Gln Glu Gln Lys Glu Phe Leu
       290                 295                 300
   Val Asn Tyr Ala Lys Glu Thr Ser Val Leu Gly Ala Gly Lys Ile Leu
   305                 310                 315                 320
   Gln Gln Ile Ala Lys Leu Ala Asp Cys Lys Val Glu Asp Ile Arg Gly
                   325                 330                 335
   Tyr Arg Leu Asp Asn Lys Asp Lys Pro Glu Leu His Thr Phe Glu Thr
               340                 345                 350
   Tyr Arg Ala Met Lys Gly Leu Val Pro Leu Val Asp Ile Gly Val Leu
           355                 360                 365
   Ser Arg Glu Gln Leu Asp Ile Leu Ala Asp Ile Leu Thr Leu Asn Thr
       370                 375                 380
   Asp Phe Glu Gly Ile Arg Glu Ala Leu Lys Lys Gln Leu Pro Asn Val
   385                 390                 395                 400
   Phe Asp Glu Lys Gln Val Lys Gly Leu Ala Ser Phe Arg Lys Ser Lys
                   405                 410                 415
   Ser Gln Leu Phe Ala Lys Gly Trp His Asn Leu Ser Gln Lys Ile Met
               420                 425                 430
   Leu Glu Val Ile Pro Glu Leu Tyr Ala Thr Ser Asp Glu Gln Met Thr
           435                 440                 445
   Ile Leu Thr Arg Leu Gly Lys Phe Glu Lys Ser Ser Val Ala Glu Tyr
       450                 455                 460
   Pro Ser Ser Ile Asn Val Asp Glu Ile Thr Asp Glu Ile Tyr Asn Pro
   465                 470                 475                 480
   Val Val Ala Lys Ser Ile Arg Gln Thr Ile Lys Ile Ile Asn Ala Ser
                   485                 490                 495
   Ile Lys Lys Trp Gly Glu Phe Asp Gln Ile Val Ile Glu Met Pro Arg
   500 505 510
   Asp Arg Asn Glu Asp Glu Glu Lys Lys Arg Ile Ala Asp Gly Gln Lys
   515 520 525
   Ala Asn Ala Lys Glu Lys Ala Asp Ser Ile Leu Arg Ala Ala Glu Leu
   530 535 540
   Tyr Cys Ala Gly Lys Val Leu Pro Asp Tyr Val Tyr Asn Gly His Asn
   545 550 555 560
   Gln Leu Ala Thr Lys Ile Arg Leu Trp Tyr Gln Gln Gly Glu Arg Cys
   565 570 575
   Ile Tyr Thr Gly Gln Pro Ile Ser Ile His Asp Leu Ile His Asn Gln
   580 585 590
   Asn Gln Tyr Glu Ile Asp His Ile Leu Pro Leu Ser Leu Thr Phe Asp
   595 600 605
   Asp Ser Leu Ser Asn Lys Val Leu Val Leu Ala Thr Ala Asn Gln Glu
   610 615 620
   Lys Ala Gln Arg Thr Pro Tyr Asn Tyr Leu Lys Ser Ala Thr Ser Ala
   625 630 635 640
   Trp Ser Tyr Arg Glu Phe Lys Asp Tyr Val Thr Lys Arg Lys Gly Ile
   645 650 655
   Gly Lys Lys Lys Cys Glu Tyr Leu Thr Phe Glu Glu Asp Ile Asn Gly
   660 665 670
   Phe Glu Val Arg Ser Lys Phe Ile Gln Arg Asn Leu Val Asp Thr Arg
   675 680 685
   Tyr Ala Ser Lys Val Ile Leu Asn Ala Leu Gln Asp Tyr Phe Lys Ile
   690 695 700
   Ser Gly Ile Gln Thr Lys Val Ser Val Val Arg Gly Gln Phe Thr Ser
   705 710 715 720
   Gln Leu Arg His Lys Trp Gly Ile Glu Lys Thr Arg Glu Thr Tyr His
   725 730 735
   His His Ala Val Asp Ala Leu Ile Ile Ala Ala Ser Ser Gln Leu Arg
   740 745 750
   Leu Trp Lys Lys Gln Glu Ser Pro Leu Val Val Asp Tyr Gln Glu Gly
   755 760 765
   Arg Gln Val Asp Leu Glu Thr Gly Glu Ile Leu Glu Leu Thr Asp Glu
   770 775 780
   Gln Tyr Lys Glu Leu Val Tyr Gln Pro Pro Tyr Gln Gly Phe Val Asn
   785 790 795 800
   Thr Ile Ser Ser Ser Ala Phe Asp Asn Glu Ile Leu Phe Ser Tyr Gln
   805 810 815
   Val Asp Ser Lys Val Asn Arg Lys Ile Ser Asp Ala Thr Ile Tyr Ala
   820 825 830
   Thr Arg Asn Ala Gln Leu Gly Lys Asp Lys Thr Glu Gly Ile Tyr Val
   835 840 845
   Leu Gly Lys Ile Lys Asp Ile Tyr Thr Gln Ala Gly Tyr Glu Ala Phe
   850 855 860
   Leu Lys Arg Tyr Thr Lys Asp Lys Thr Ser Phe Leu Met Tyr His Lys
   865 870 875 880
   Asp Leu Asp Thr Trp Glu Lys Val Ile Glu Ile Ile Leu Arg Asp Tyr
   885 890 895
   Arg Glu Tyr Asp Glu Lys Gly Lys Glu Ile Gly Asn Pro Phe Glu Arg
   900 905 910
   Tyr Arg Arg Glu Asn Gly Tyr Val Lys Lys Tyr Ser Arg Lys Gly Asn
   915 920 925
   Gly Thr Ala Ile Lys Ser Leu Lys Tyr Tyr Asp Asn Lys Leu Gly Asn
   930 935 940
   His Ile Asp Ile Thr Pro Glu Asn Ser Arg Asn Ala Val Val Leu Gln
   945 950 955 960
   Ser Leu Lys Pro Trp Arg Thr Asp Val Tyr Phe Asn Lys Glu Thr Gly
   965 970 975
   Lys Tyr Glu Phe Leu Gly Ile Lys Tyr Ser Asp Leu Ser Phe Glu Lys
   980 985 990
   Gly Thr Gly Glu Tyr Gly Ile Ser Gln Glu Lys Tyr Asp Ser Ile Lys
               995                 1000                1005
   Ile Ala Glu Gly Val Ala Lys Lys Ser Ile Phe Lys Phe Thr Leu Tyr
           1010                1015                1020
   Lys Gln Asp Leu Leu Phe Ile Lys Asp Ile Glu Asn Asn Phe Gly Lys
   1025                1030                1035                1040
   Leu Leu Arg Phe Thr Ser Lys Asn Asp Thr Ser Lys His Tyr Val Glu
                   1045                1050                1055
   Leu Lys Pro Tyr Asp Lys Asn Lys Phe Gly Thr Glu Glu Pro Leu Leu
               1060                1065                1070
   Pro Val Leu Gly Asn Val Ala Lys Ser Gly Gln Cys Ile Lys Gly Leu
           1075                1080                1085
   Asn Lys Ser Asn Ile Ser Ile Tyr Lys Val Arg Thr Asp Ile Leu Gly
       1090                1095                1100
   Tyr Arg His Phe Ile Lys Gln Glu Gly Glu His Pro Gln Leu Lys Phe
   1105                1110                1115                1120
   Lys Lys
<210> 71
   <211> 1130
   <212> PRT
   <213> Streptococcus gallolyticus
<220>
   <223> CRISPR-associated endonuclease
<308> YP_003430854
   <309> 2013-06-10
<400> 71
   Met Thr Asn Gly Lys Ile Leu Gly Leu Asp Ile Gly Ile Ala Ser Val
    1               5                  10                  15
   Gly Val Gly Ile Ile Glu Ala Lys Thr Gly Lys Val Val His Ala Asn
               20                  25                  30
   Ser Arg Leu Phe Ser Ala Ala Asn Ala Glu Asn Asn Ala Glu Arg Arg
           35                  40                  45
   Gly Phe Arg Gly Ser Arg Arg Leu Asn Arg Arg Lys Lys His Arg Val
       50                  55                  60
   Lys Arg Val Arg Asp Leu Phe Glu Lys Tyr Glu Ile Val Thr Asp Phe
   65                  70                  75                  80
   Arg Asn Leu Asn Leu Asn Pro Tyr Glu Leu Arg Val Lys Gly Leu Thr
                   85                  90                  95
   Glu Gln Leu Thr Asn Glu Glu Leu Phe Ala Ala Leu Arg Thr Ile Ser
               100                 105                 110
   Lys Arg Arg Gly Ile Ser Tyr Leu Asp Asp Ala Glu Asp Asp Ser Thr
           115                 120                 125
   Gly Ser Thr Asp Tyr Ala Lys Ser Ile Asp Glu Asn Arg Arg Leu Leu
       130                 135                 140
   Lys Thr Lys Thr Pro Gly Gln Ile Gln Leu Glu Arg Leu Glu Lys Tyr
   145                 150                 155                 160
   Gly Gln Leu Arg Gly Asn Phe Thr Val Tyr Asp Glu Asn Gly Glu Ala
                   165                 170                 175
   His Arg Leu Ile Asn Val Phe Ser Thr Ser Asp Tyr Glu Lys Glu Ala
               180                 185                 190
   Arg Lys Ile Leu Glu Thr Gln Ala Asp Tyr Asn Lys Lys Ile Thr Ala
           195                 200                 205
   Glu Phe Ile Asp Asp Tyr Val Glu Ile Leu Thr Gln Lys Arg Lys Tyr
       210                 215                 220
   Tyr His Gly Pro Gly Asn Glu Lys Ser Arg Thr Asp Tyr Gly Arg Phe
   225                 230                 235                 240
   Arg Thr Asp Gly Thr Thr Leu Glu Asn Ile Phe Gly Ile Leu Ile Gly
                   245                 250                 255
   Lys Cys Ser Phe Tyr Pro Asp Glu Tyr Arg Ala Ser Lys Ala Ser Tyr
               260                 265                 270
   Thr Ala Gln Glu Tyr Asn Phe Leu Asn Asp Leu Asn Asn Leu Lys Val
           275                 280                 285
   Pro Thr Glu Thr Gly Lys Leu Ser Thr Glu Gln Lys Glu Ala Leu Val
       290                 295                 300
   Glu Phe Ala Lys Ser Thr Ala Thr Leu Gly Pro Ala Lys Leu Leu Lys
   305                 310                 315                 320
   Glu Ile Ala Lys Ile Leu Asp Cys Lys Val Asp Glu Ile Lys Gly Tyr
                   325                 330                 335
   Arg Glu Asp Asp Lys Gly Lys Pro Asp Leu His Thr Phe Glu Pro Tyr
               340                 345                 350
   Arg Lys Leu Lys Phe Asn Leu Asp Ser Val Asn Ile Asp Asp Leu Ser
           355                 360                 365
   Arg Glu Val Leu Asp Lys Leu Ala Asp Ile Leu Thr Leu Asn Thr Glu
       370                 375                 380
   Arg Glu Gly Ile Glu Asp Ala Ile Arg His Asn Leu Pro Asn Gln Phe
   385                 390                 395                 400
   Thr Glu Gly Gln Ile Ser Glu Ile Ile Lys Val Arg Lys Ser Gln Ser
                   405                 410                 415
   Thr Ala Phe Asn Lys Gly Trp His Ser Phe Ser Ala Lys Leu Met Asn
               420                 425                 430
   Glu Leu Ile Pro Glu Leu Tyr Ala Thr Ser Asp Glu Gln Met Thr Ile
           435                 440                 445
   Leu Thr Arg Leu Glu Lys Phe Lys Val Asn Lys Lys Ser Ser Lys Asn
       450                 455                 460
   Thr Lys Thr Ile Asp Glu Lys Glu Val Thr Asp Glu Ile Tyr Asn Pro
   465                 470                 475                 480
   Val Val Ala Lys Ser Val Arg Gln Thr Ile Lys Ile Ile Asn Ala Ala
                   485                 490                 495
   Val Lys Lys Tyr Gly Asp Phe Asp Lys Ile Val Ile Glu Met Pro Arg
               500                 505                 510
   Asp Lys Asn Ala Asp Asp Glu Lys Lys Phe Ile Asp Lys Arg Asn Lys
           515                 520                 525
   Glu Asn Lys Lys Glu Lys Asp Asp Ala Leu Lys Arg Ala Ala Tyr Leu
       530                 535                 540
   Tyr Asn Gly Thr Asp Lys Leu Pro Asp Glu Val Phe His Gly Asn Lys
   545                 550                 555                 560
   Gln Leu Glu Thr Lys Ile Arg Leu Trp Tyr Gln Gln Gly Glu Arg Cys
                   565                 570                 575
   Leu Tyr Ser Gly Lys Pro Ile Pro Ile Gln Glu Leu Val His Asn Ser
               580                 585                 590
   Asn Asn Phe Glu Ile Asp His Ile Leu Pro Leu Ser Leu Ser Phe Asp
           595                 600                 605
   Asp Ser Leu Ala Asn Lys Val Leu Val Tyr Ala Trp Thr Asn Gln Glu
       610                 615                 620
   Lys Gly Gln Lys Thr Pro Tyr Gln Val Ile Asp Ser Met Asp Ala Ala
   625                 630                 635                 640
   Trp Ser Phe Arg Glu Met Lys Asp Tyr Val Leu Lys Gln Lys Gly Leu
                   645                 650                 655
   Gly Lys Lys Lys Arg Asp Tyr Leu Leu Thr Thr Glu Asn Ile Asp Lys
               660                 665                 670
   Ile Glu Val Lys Lys Lys Phe Ile Glu Arg Asn Leu Val Asp Thr Arg
           675                 680                 685
   Tyr Ala Ser Arg Val Val Leu Asn Ser Leu Gln Ser Ala Leu Arg Glu
       690                 695                 700
   Leu Gly Lys Asp Thr Lys Ile Ser Val Ile Arg Gly Gln Phe Thr Ser
   705                 710                 715                 720
   Gln Leu Arg Arg Lys Trp Lys Ile Asp Lys Ser Arg Glu Thr Tyr His
                   725                 730                 735
   His His Ala Val Asp Ala Leu Ile Ile Ala Ala Ser Ser Gln Leu Lys
               740                 745                 750
   Leu Trp Glu Lys Gln Asp Asn Pro Met Phe Val Asp Tyr Gly Asn Asn
   755 760 765
   Gln Val Val Asp Lys Gln Thr Gly Glu Ile Leu Ser Val Ser Asp Asp
   770 775 780
   Glu Tyr Lys Glu Leu Val Phe Gln Pro Pro Tyr Gln Gly Phe Val Asn
   785 790 795 800
   Met Ile Ser Ser Lys Gly Phe Glu Asp Glu Ile Leu Phe Ser Tyr Gln
   805 810 815
   Val Asp Ser Lys Tyr Asn Arg Lys Val Ser Asp Ala Thr Ile Tyr Ser
   820 825 830
   Thr Arg Lys Ala Lys Ile Gly Lys Asp Lys Lys Glu Glu Thr Tyr Val
   835 840 845
   Leu Gly Lys Ile Lys Asp Ile Tyr Ser Gln Asn Gly Phe Asp Thr Phe
   850 855 860
   Ile Lys Lys Tyr Asn Lys Asp Lys Thr Gln Phe Leu Met Tyr Gln Lys
   865 870 875 880
   Asp Pro Leu Thr Trp Glu Asn Val Ile Glu Val Ile Leu Arg Asp Tyr
   885 890 895
   Pro Thr Thr Lys Lys Ser Glu Asp Gly Lys Asn Asp Val Lys Cys Asn
   900 905 910
   Pro Phe Glu Glu Tyr Arg Arg Glu Asn Gly Leu Val Cys Lys Tyr Ser
   915 920 925
   Lys Lys Gly Lys Gly Thr Pro Ile Lys Ser Leu Lys Tyr Tyr Asp Lys
   930 935 940
   Lys Leu Gly Asn Cys Ile Asp Ile Thr Pro Glu Gly Ser Lys Asn Glu
   945 950 955 960
   Val Val Leu Gln Ser Leu Asn Pro Trp Arg Ala Asp Val Tyr Phe Asn
   965 970 975
   Pro Glu Thr Leu Lys Tyr Glu Leu Leu Gly Leu Lys Tyr Ser Asp Leu
   980 985 990
   Ser Phe Glu Lys Gly Thr Gly Lys Tyr His Ile Ser Gln Glu Lys Tyr
   995 1000 1005
   Asp Val Ile Lys Glu Lys Glu Gly Ile Gly Lys Lys Ser Glu Phe Lys
   1010 1015 1020
   Phe Thr Leu Tyr Arg Asn Asp Leu Ile Leu Ile Lys Asp Thr Ala Ser
   1025 1030 1035 1040
   Gly Glu Gln Glu Ile Tyr Arg Phe Leu Ser Arg Thr Met Pro Asn Val
   1045 1050 1055
   Lys His Tyr Ala Glu Leu Lys Pro Tyr Asp Lys Glu Lys Phe Asp Asn
   1060 1065 1070
   Val Gln Glu Leu Val Glu Ala Leu Gly Glu Ala Asp Lys Val Gly Arg
   1075 1080 1085
   Cys Ile Lys Gly Leu Asn Lys Ser Asn Leu Ser Ile Tyr Lys Val Arg
   1090 1095 1100
   Thr Asp Val Leu Gly Asn Lys Tyr Phe Val Lys Lys Glu Gly Asp Lys
   1105 1110 1115 1120
   Pro Lys Leu Asp Phe Lys Asn Asn Lys Lys
   1125 1130
<210> 72
   <211> 1371
   <212> PRT
   <213> Streptococcus gallolyticus
<220>
   <223> CRISPR-associated endonuclease
<308> YP_003430861
   <309> 2013-06-10
<400> 72
   Met Thr Glu Lys Asn Tyr Ser Ile Gly Leu Asp Ile Gly Thr Asn Ser
   1               5                   10                  15
   Val Gly Trp Ala Val Ile Thr Asp Asp Tyr Lys Val Pro Ala Lys Lys
   20 25 30
   Met Lys Val Leu Gly Asn Thr Asp Lys Lys Tyr Ile Lys Lys Asn Leu
   35 40 45
   Leu Gly Ala Leu Leu Phe Asp Asn Gly Glu Thr Ala Glu Ala Thr Arg
   50 55 60
   Leu Lys Arg Thr Ala Arg Arg Arg Tyr Thr Arg Arg Lys Asn Arg Leu
   65 70 75 80
   Arg Tyr Leu Gln Glu Ile Phe Ala Glu Glu Met Ala Lys Val Asp Glu
   85 90 95
   Ser Phe Phe Tyr Arg Leu Asp Glu Ser Phe Leu Thr Thr Asp Asp Lys
   100 105 110
   Asp Phe Glu Arg His Pro Ile Phe Gly Asn Lys Ala Asp Glu Ile Lys
   115 120 125
   Tyr His Gln Glu Phe Pro Thr Ile Tyr His Leu Arg Lys His Leu Ala
   130 135 140
   Asp Ser His Glu Lys Ala Asp Leu Arg Leu Ile Tyr Leu Ala Leu Ala
   145 150 155 160
   His Met Ile Lys Phe Arg Gly His Phe Leu Ile Glu Gly Glu Leu Asn
   165 170 175
   Ala Glu Asn Thr Asp Val Gln Lys Leu Phe Glu Ala Phe Val Glu Val
   180 185 190
   Tyr Asp Arg Thr Phe Asp Asp Ser Asn Leu Ser Glu Ile Thr Val Asp
   195 200 205
   Ala Ser Ser Ile Leu Thr Glu Lys Phe Ser Lys Ser Arg Arg Leu Glu
   210 215 220
   Asn Leu Ile Lys His Tyr Pro Thr Glu Lys Lys Asn Thr Leu Phe Gly
   225 230 235 240
   Asn Leu Val Ala Leu Ala Leu Gly Leu Gln Pro Asn Phe Lys Thr Ser
   245 250 255
   Phe Lys Leu Ser Glu Asp Ala Lys Leu Gln Phe Ser Lys Asp Thr Tyr
   260 265 270
   Glu Glu Asp Leu Glu Glu Leu Ile Gly Lys Ile Gly Asp Glu Tyr Ala
   275 280 285
   Asp Leu Phe Thr Ser Ala Lys Asn Leu Tyr Asp Ala Ile Leu Leu Ser
   290 295 300
   Gly Ile Leu Thr Val Ala Asp Asn Thr Thr Lys Ala Pro Leu Ser Ala
   305 310 315 320
   Ser Met Ile Lys Arg Tyr Asn Glu His Gln Val Asp Leu Lys Lys Leu
   325 330 335
   Lys Glu Phe Ile Lys Asn Asn Ala Ser Asp Lys Tyr Asp Glu Ile Phe
   340 345 350
   Asn Asp Lys Asp Lys Asn Gly Tyr Ala Gly Tyr Ile Glu Asn Gly Val
   355 360 365
   Lys Gln Asp Glu Phe Tyr Lys Tyr Leu Lys Thr Thr Leu Ser Lys Ile
   370 375 380
   Asp Gly Ser Asp Tyr Phe Leu Asp Lys Ile Glu Arg Glu Asp Phe Leu
   385 390 395 400
   Arg Lys Gln Arg Thr Phe Asp Asn Gly Ser Ile Pro His Gln Ile His
   405 410 415
   Leu Gln Glu Met His Ala Ile Leu Arg Arg Gln Gly Glu His Tyr Ala
   420 425 430
   Phe Leu Lys Glu Asn Gln Ala Lys Ile Glu Lys Ile Leu Thr Phe Arg
   435 440 445
   Ile Pro Tyr Tyr Val Gly Pro Leu Ala Arg Lys Asn Ser Arg Phe Ala
   450 455 460
   Trp Ala Glu Tyr His Ser Asp Glu Lys Ile Thr Pro Trp Asn Phe Asp
   465 470 475 480
   Glu Ile Ile Asp Lys Glu Lys Ser Ala Glu Lys Phe Ile Thr Arg Met
   485 490 495
   Thr Leu Asn Asp Leu Tyr Leu Pro Glu Glu Lys Val Leu Pro Lys His
   500 505 510
   Ser Leu Val Tyr Glu Thr Tyr Thr Val Tyr Asn Glu Leu Thr Lys Val
   515 520 525
   Lys Tyr Val Asn Glu Gln Gly Lys Ser Asn Phe Phe Asp Ala Asn Met
   530 535 540
   Lys Gln Glu Ile Phe Glu His Val Phe Lys Glu Asn Arg Lys Val Thr
   545 550 555 560
   Lys Asp Lys Phe Leu Asn Tyr Leu Asn Lys Glu Phe Pro Glu Tyr Arg
   565 570 575
   Ile Gln Asp Leu Ile Gly Leu Asp Lys Glu Asn Lys Ser Phe Asn Ala
   580 585 590
   Ser Leu Gly Thr Tyr His Asp Leu Lys Lys Ile Leu Asp Lys Ser Phe
   595 600 605
   Leu Asp Asp Lys Thr Asn Glu Thr Ile Ile Glu Asp Ile Ile Gln Thr
   610 615 620
   Leu Thr Leu Phe Glu Asp Arg Asp Met Ile Arg Gln Arg Leu Gln Lys
   625 630 635 640
   Tyr Ser Asp Ile Phe Thr Pro Gln Gln Leu Lys Lys Leu Glu Arg Arg
   645 650 655
   His Tyr Thr Gly Trp Gly Arg Leu Ser Tyr Lys Leu Ile Asn Gly Ile
   660 665 670
   Arg Asn Lys Glu Asn Gly Lys Ser Ile Leu Asp Tyr Leu Ile Asp Asp
   675 680 685
   Gly Tyr Ala Asn Arg Asn Phe Met Gln Leu Ile Ser Asp Asp Thr Leu
   690 695 700
   Pro Phe Lys Gln Ile Ile Lys Asp Ala Gln Ile Ile Gly Asp Ile Asp
   705 710 715 720
   Asp Val Thr Ser Val Val Arg Glu Leu Pro Gly Ser Pro Ala Ile Lys
   725 730 735
   Lys Gly Ile Leu Gln Ser Val Lys Ile Val Asp Glu Leu Val Lys Val
   740 745 750
   Met Gly His Asn Pro Asp Asn Ile Val Ile Glu Met Ala Arg Glu Asn
   755 760 765
   Gln Thr Thr Asn Arg Gly Arg Asn Gln Ser Gln Gln Arg Leu Lys Lys
   770 775 780
   Leu Gln Asp Ser Leu Lys Glu Leu Gly Ser Asn Ile Leu Asn Glu Glu
   785 790 795 800
   Lys Pro Ser Tyr Ile Glu Gly Lys Val Glu Asn Asn His Leu Gln Asp
   805 810 815
   Asp Arg Leu Phe Leu Tyr Tyr Ile Gln Asn Gly Lys Asp Met Tyr Thr
   820 825 830
   Gly Asp Glu Leu Asp Ile Asp His Leu Ser Asp Tyr Asp Ile Asp His
   835 840 845
   Ile Ile Pro Gln Ala Phe Ile Lys Asp Asp Ser Ile Asp Asn Arg Val
   850 855 860
   Leu Thr Ser Ser Ala Lys Asn Arg Gly Lys Ser Asp Asp Val Pro Ser
   865 870 875 880
   Leu Asp Ile Val His Asp Arg Lys Ala Asp Trp Ile Arg Leu Tyr Lys
   885 890 895
   Ser Gly Leu Ile Ser Lys Arg Lys Phe Asp Asn Leu Thr Lys Ala Glu
   900 905 910
   Arg Gly Gly Leu Thr Glu Asn Asp Lys Ala Gly Phe Ile Lys Arg Gln
   915 920 925
   Leu Val Glu Thr Arg Gln Ile Thr Lys His Val Ala Gln Ile Leu Asp
   930 935 940
   Ser Arg Phe Asn Thr Glu Arg Asp Glu Asn Asp Lys Val Ile Arg Asn
   945 950 955 960
   Val Lys Val Ile Thr Leu Lys Ser Asn Leu Val Ser Gln Phe Arg Lys
   965 970 975
   Asp Phe Lys Phe Tyr Lys Val Arg Glu Ile Asn Asp Tyr His His Ala
   980 985 990
   His Asp Ala Tyr Leu Asn Ala Val Val Gly Thr Ala Leu Leu Lys Lys
   995 1000 1005
   Tyr Pro Lys Leu Ala Pro Glu Phe Val Tyr Gly Glu Tyr Lys Lys Tyr
   1010 1015 1020
   Asp Ile Arg Lys Phe Ile Thr Ser Ser Gly Asp Lys Ala Thr Ala Lys
   1025 1030 1035 1040
   Tyr Phe Phe Tyr Ser Asn Leu Met Asn Phe Phe Lys Arg Val Ile Arg
   1045 1050 1055
   Tyr Ser Asn Gly Lys Val Val Val Arg Pro Val Ile Glu Cys Ser Lys
   1060 1065 1070
   Asp Thr Gly Glu Ile Ala Trp Asn Lys Gln Thr Asp Phe Glu Lys Val
   1075 1080 1085
   Arg Arg Val Leu Ser Tyr Pro Gln Val Asn Ile Val Lys Lys Val Glu
   1090 1095 1100
   Thr Gln Thr Gly Gly Phe Ser Lys Glu Ser Ile Leu Pro Lys Gly Asn
   1105 1110 1115 1120
   Ser Asp Lys Leu Ile Pro Arg Lys Thr Lys Lys Phe Arg Trp Asp Thr
   1125 1130 1135
   Pro Lys Tyr Gly Gly Phe Asp Ser Pro Asn Ile Ala Tyr Ser Val Phe
   1140 1145 1150
   Val Ile Ala Asp Val Glu Lys Gly Lys Ala Lys Lys Leu Lys Thr Val
   1155 1160 1165
   Lys Glu Leu Val Gly Ile Ser Ile Met Glu Arg Ser Ser Phe Glu Glu
   1170 1175 1180
   Asn Pro Val Val Phe Leu Glu Lys Lys Gly Tyr Gln Asn Val Gln Glu
   1185 1190 1195 1200
   Asp Asn Leu Ile Lys Leu Pro Lys Tyr Ser Leu Phe Glu Phe Glu Gly
   1205 1210 1215
   Gly Arg Arg Arg Leu Leu Ala Ser Ala Ser Glu Leu Gln Lys Gly Asn
   1220 1225 1230
   Glu Val Val Leu Ser Arg His Leu Val Glu Leu Leu Tyr His Ala His
   1235 1240 1245
   Arg Val Asn Ser Phe Asn Asn Ser Glu His Leu Lys Tyr Val Ser Glu
   1250 1255 1260
   His Lys Lys Glu Phe Gly Glu Val Leu Ser Cys Val Glu Asn Phe Ala
   1265 1270 1275 1280
   Lys Ser Tyr Val Asp Val Glu Lys Asn Leu Gly Lys Ile Arg Ala Val
   1285 1290 1295
   Ala Asp Lys Ile Asp Thr Phe Ser Ile Glu Asp Ile Ser Ile Ser Phe
   1300 1305 1310
   Val Asn Leu Leu Thr Leu Thr Ala Leu Gly Ala Pro Ala Asp Phe Asn
   1315 1320 1325
   Phe Leu Gly Glu Lys Ile Pro Arg Lys Arg Tyr Thr Ser Thr Lys Glu
   1330 1335 1340
   Cys Leu Asn Ala Thr Leu Ile His Gln Ser Ile Thr Gly Leu Tyr Glu
   1345 1350 1355 1360
   Thr Arg Ile Asp Leu Ser Lys Leu Gly Glu Glu
   1365 1370
<210> 73
   <211> 1138
   <212> PRT
   <213> Bifidobacterium dentium
<220>
   <223> CRISPR-associated endonuclease
<308> YP_003360699
   <309> 2013-08-27
<400> 73
   Met Leu Cys Arg Cys His Arg Arg Tyr Arg Ile Gly Ile Asp Val Gly
    1              5                   10                  15
   Leu Met Ser Val Gly Leu Ala Ala Ile Glu Val Asp Glu Thr Gly Phe
               20                  25                  30
   Pro Ile Arg Ile Leu Asn Ile Gln Ser Val Ile His Asp Gly Gly Ile
               35                  40                  45
   Asp Pro Gln Leu Gly Lys Lys Lys Ser Ile Ser Arg Lys Ala Ile Ser
       50                  55                  60
   Gly Val Ala Arg Arg Thr Arg Arg Met Arg Arg Arg Lys Val Val Arg
   65                  70                  75                  80
   Leu Arg Gln Leu Asp Ala Thr Leu Glu Ser Leu Gly Phe Pro Ile Val
                   85                  90                  95
   Glu Pro Asp Ser Leu Asp Thr Trp Glu Ile Trp Leu Thr Arg Ala Lys
               100                 105                 110
   Leu Ala Ser Ser Tyr Ile Gln Asp Asp Glu Thr Arg Lys Glu Asp Val
           115                 120                 125
   Ser Ile Ala Leu Arg His Ile Ala Arg His Arg Gly Trp Arg Asn Pro
       130                 135                 140
   Tyr Ser Asn Val Glu Ser Leu Phe Val Asp Val Ala Pro Ser Glu Gln
   145                 150                 155                 160
   Tyr Glu Glu Leu Arg Arg Lys Ala Glu Glu Lys Leu Gly Thr Ser Leu
                   165                 170                 175
   Ser Gly Asn Pro Thr Ser Ala Glu Ile Val Glu Cys Ile Leu Lys Arg
               180                 185                 190
   Gln Val Gly Glu Val Gln Leu Arg Thr Arg Ser Arg Lys Gly Val Asn
           195                 200                 205
   Gly Asn Ala Val Glu Lys Glu Gly Leu Leu Pro Thr Lys Leu Met Gln
       210                 215                 220
   Ser Asp Asn Ala Ala Glu Ile Arg Arg Ile Phe Lys Val Gln Arg Ile
   225                 230                 235                 240
   Asp Asp Glu Thr Gly Arg Lys Leu Leu Arg Ala Val Phe Lys Ala Cys
                   245                 250                 255
   Ser Pro Lys Gly Ser Val Glu Arg Arg Val Gly Arg Asp Ala Leu Ala
               260                 265                 270
   Pro Gly Leu Pro Arg Ala Leu Lys Ala Ser Leu Ala Phe Gln Glu Tyr
           275                 280                 285
   Arg Ile Ala Asn Val Leu Thr Asn Leu Val Ile Ser Glu Asp Gly Thr
       290                 295                 300
   Lys Arg Lys Leu Thr Ile Ala Glu Lys Gln Arg Val Phe Gly Phe Leu
   305                 310                 315                 320
   Ser Ser Asn Glu Thr Asp Asp Ile Thr Trp Ile Asp Val Ala Glu Lys
                   325                 330                 335
   Leu Gly Leu Glu Arg Arg Gln Ile Gln Gly Val Gly Cys Leu Thr Ala
               340                 345                 350
   Asp Gly Glu Glu Arg Ile Ser Ser Thr Pro Pro Arg Asn Ile Thr Ala
           355                 360                 365
   Leu His Ile His Ser Ala Lys Pro Val Ile Arg Glu Lys Leu Val Ala
       370                 375                 380
   Trp Trp Lys Ala Ala Ser Pro Thr Val Arg Glu Thr Met Val Glu Leu
   385                 390                 395                 400
   Leu Ser Asn Thr Val Asp Ile Asp Arg Val Arg Glu Asp Met Asp Tyr
                   405                 410                 415
   Ser Ser Ala Val Ala Phe Ile Asp Ser Leu Gly Asp Glu Glu Leu Thr
               420                 425                 430
   Ala Leu Asp Ser Val Asp Leu Leu Ser Gly Arg Ala Ala Tyr Ser Arg
           435                 440                 445
   Gln Thr Leu Lys Gln Leu Thr Glu Arg Met Leu Asn Thr Asp Asp Asp
       450                 455                 460
   Leu His Glu Ala Arg Lys Ala Val Phe Gly Val Asp Asp Asn Trp Lys
   465                 470                 475                 480
   Pro Pro Val Asp Ala Ile Ser Thr Ser Val Gly Asn Pro Ala Val Asp
                   485                 490                 495
   Arg Val Leu Lys Ile Val Asn Arg Tyr Leu Leu Ala Cys Gln Lys Arg
               500                 505                 510
   Trp Arg Asn Pro Gln Ser Val Gln Ile Glu His Val Arg Glu Ser Phe
           515                 520                 525
   Ser Ser Val Ala Lys Ala Lys Glu Thr Glu Arg Val Gln Gly Glu Arg
       530                 535                 540
   Ala Arg Tyr Arg Asn Asp Leu Arg Arg Glu Leu Cys Glu Gln Glu Gly
   545 550 555 560
   Leu Asn Lys Ile Arg Glu Ser Asp Ile Arg Arg Trp Glu Ser Val Gln
   565 570 575
   Arg Gln Asn Gly Thr Cys Leu Tyr Cys Gly Ser Pro Ile Thr Phe Arg
   580 585 590
   Thr Cys Glu Met Asp His Ile Val Pro Arg Lys Gly Val Gly Ser Thr
   595 600 605
   Asn Thr Arg Val Asn Leu Ala Ala Ala Cys Ala Ala Cys Asn Lys Leu
   610 615 620
   Lys Ser Asn Thr Ala Phe Ser Val Trp Ala Arg Thr Glu Gly Ala Ala
   625 630 635 640
   Asn Arg Lys Val Ser Gln Arg Asp Ala Ile Glu Arg Val Lys Thr Phe
   645 650 655
   Ile Phe Asn Lys Ser Val Pro Pro Arg Glu Gln Arg Ala Phe Lys Gln
   660 665 670
   Ala Val Ile Ser Arg Leu Arg Gln Ala Glu Ser Asp Pro Ala Ile Asp
   675 680 685
   Asn Arg Ser Ile Glu Ser Val Ala Trp Met Ala Asp Glu Leu His Arg
   690 695 700
   Arg Ile Glu Ala Tyr Phe Asn Gln Asp Ala Asp Val Ser Gly Asn Thr
   705 710 715 720
   Pro Ala Thr Gln Val Trp Val Phe Gln Gly Arg Val Thr Ala Leu Ala
   725 730 735
   Arg Lys Ala Ser Gly Ile Glu Gly Arg Ile His Phe Ala Gly Glu Trp
   740 745 750
   Lys Lys Thr Arg Leu Asp Arg Arg His His Ala Val Asp Ala Ala Val
   755 760 765
   Ile Ala Met Met Asn His Ser Ala Ala Gln Thr Leu Val Glu Arg Asp
   770 775 780
   Ser Leu Arg Leu Ser Gln Trp Ile Thr Arg Lys Pro Glu Thr Trp Lys
   785 790 795 800
   Asn Tyr Pro Phe Ser Glu Gly Gln Lys Lys Thr Arg Phe Lys Glu Trp
   805 810 815
   Leu Asp Cys Met Asn Ala Leu Leu Asp Leu Leu Asn Asp Ala Leu Asp
   820 825 830
   Ser Asp Arg Val Pro Val Thr Arg Cys Gln Arg Met Ser Leu Gly Asn
   835 840 845
   Ser Gln Ala His Ala Ala Thr Ile Arg Lys Met Lys Arg Val Glu Leu
   850 855 860
   Phe Ser Pro Met Asp Ala Asp Leu Ile Arg Arg Ala Ser Thr Pro Ala
   865 870 875 880
   Leu Tyr Cys Ala Leu Thr Arg Leu Pro Glu Tyr Ser Pro Glu Lys Gly
   885 890 895
   Leu Pro Glu Asn Arg Gly Arg Glu Ile Val Leu Asn Gly Arg His Ile
   900 905 910
   Arg Ser Thr Glu Thr Val Asp Phe Phe Ala Ser Gly Ala Ala Gln Leu
   915 920 925
   Met Val Arg Asn Gly Ser Ala Glu Ile Gly Asp Ser Ile His His Met
   930 935 940
   Arg Ile Tyr Arg Tyr Arg Lys Thr Gly Ala Lys Ser Gln Glu Tyr Val
   945 950 955 960
   Tyr Gly Met Ile Arg Val Phe Gln Ile Asp Leu Leu His Ala Ser Ser
   965 970 975
   Glu Asn Leu Phe Ala Val Gln Leu Pro Ser Gln Ser Val Ser Val Arg
   980 985 990
   Tyr Gly Asp Lys Asn Val Val Gln Ala Ile Gln Glu Gly Arg Ala Glu
   995 1000 1005
   Tyr Leu Gly His Ile Val Pro Asn Asp Glu Ile Leu Ile Ser Tyr Glu
   1010 1015 1020
   Gly Val Arg Met Lys Asp Ser Glu Lys Ile Gly Ala Tyr Ala Asn Asp
   1025 1030 1035 1040
   Phe Ser Gly Thr Ala Ser Glu Leu Ala Gln Arg Arg His Trp Val Val
                   1045                1050                1055
   Val Gly Ser Asp Val Ser Lys Leu Ala Leu Arg Pro Ser Tyr Leu Ser
               1060                1065                1070
   Glu Glu Gly Leu Glu Asn Ser Gly Ser Glu Ser Glu Arg Leu Ser Lys
           1075                1080                1085
   Gln Thr Arg Lys Val Ile Glu Gly Lys Gly Trp Val Val Ala Val Asn
       1090                1095                1100
   Ile Leu Ala Lys Tyr His Pro Val Val Ile Arg Arg Asn Ala Leu Gly
   1105                1110                1115                1120
   Glu Pro Arg Phe Arg Thr Asn Ser Gly Met Pro Val Ser Trp Arg Trp
                   1125                1130                1135
   Asp Arg
<210> 74
   <211> 1082
   <212> PRT
   <213> Neisseria meningitidis
<220>
   <223> CRISPR-associated endonuclease
<308> YP_003082577
   <309> 2013-06-10
<400> 74
   Met Ala Ala Phe Lys Pro Asn Pro Ile Asn Tyr Ile Leu Gly Leu Asp
    1               5                  10                  15
   Ile Gly Ile Ala Ser Val Gly Trp Ala Met Val Glu Ile Asp Glu Glu
               20                  25                  30
   Glu Asn Pro Ile Arg Leu Ile Asp Leu Gly Val Arg Val Phe Glu Arg
           35                  40                  45
   Ala Glu Val Pro Lys Thr Gly Asp Ser Leu Ala Met Val Arg Arg Leu
       50                  55                  60
   Ala Arg Ser Val Arg Arg Leu Thr Arg Arg Arg Ala His Arg Leu Leu
   65                  70                  75                  80
   Arg Ala Arg Arg Leu Leu Lys Arg Glu Gly Val Leu Gln Ala Ala Asp
                   85                  90                  95
   Phe Asp Glu Asn Gly Leu Ile Lys Ser Leu Pro Asn Thr Pro Trp Gln
               100                 105                 110
   Leu Arg Ala Ala Ala Leu Asp Arg Lys Leu Thr Pro Leu Glu Trp Ser
           115                 120                 125
   Ala Val Leu Leu His Leu Ile Lys His Arg Gly Tyr Leu Ser Gln Arg
       130                 135                 140
   Lys Asn Glu Gly Glu Thr Ala Asp Lys Glu Leu Gly Ala Leu Leu Lys
   145                 150                 155                 160
   Gly Val Ala Asp Asn Ala His Ala Leu Gln Thr Gly Asp Phe Arg Thr
                   165                 170                 175
   Pro Ala Glu Leu Ala Leu Asn Lys Phe Glu Lys Glu Ser Gly His Ile
               180                 185                 190
   Arg Asn Gln Arg Gly Asp Tyr Ser His Thr Phe Ser Arg Lys Asp Leu
           195                 200                 205
   Gln Ala Glu Leu Ile Leu Leu Phe Glu Lys Gln Lys Glu Phe Gly Asn
       210                 215                 220
   Pro His Ile Ser Gly Gly Leu Lys Glu Gly Ile Glu Thr Leu Leu Met
   225                 230                 235                 240
   Thr Gln Arg Pro Ala Leu Ser Gly Asp Ala Val Gln Lys Met Leu Gly
                   245                 250                 255
   His Cys Thr Phe Glu Pro Ala Glu Pro Lys Ala Ala Lys Asn Thr Tyr
               260                 265                 270
   Thr Ala Glu Arg Phe Ile Trp Leu Thr Lys Leu Asn Asn Leu Arg Ile
           275                 280                 285
   Leu Glu Gln Gly Ser Glu Arg Pro Leu Thr Asp Thr Glu Arg Ala Thr
   290 295 300
   Leu Met Asp Glu Pro Tyr Arg Lys Ser Lys Leu Thr Tyr Ala Gln Ala
   305 310 315 320
   Arg Lys Leu Leu Gly Leu Glu Asp Thr Ala Phe Phe Lys Gly Leu Arg
   325 330 335
   Tyr Gly Lys Asp Asn Ala Glu Ala Ser Thr Leu Met Glu Met Lys Ala
   340 345 350
   Tyr His Ala Ile Ser Arg Ala Leu Glu Lys Glu Gly Leu Lys Asp Lys
   355 360 365
   Lys Ser Pro Leu Asn Leu Ser Pro Glu Leu Gln Asp Glu Ile Gly Thr
   370 375 380
   Ala Phe Ser Leu Phe Lys Thr Asp Glu Asp Ile Thr Gly Arg Leu Lys
   385 390 395 400
   Asp Arg Ile Gln Pro Glu Ile Leu Glu Ala Leu Leu Lys His Ile Ser
   405 410 415
   Phe Asp Lys Phe Val Gln Ile Ser Leu Lys Ala Leu Arg Arg Ile Val
   420 425 430
   Pro Leu Met Glu Gln Gly Lys Arg Tyr Asp Glu Ala Cys Ala Glu Ile
   435 440 445
   Tyr Gly Asp His Tyr Gly Lys Lys Asn Thr Glu Glu Lys Ile Tyr Leu
   450 455 460
   Pro Pro Ile Pro Ala Asp Glu Ile Arg Asn Pro Val Val Leu Arg Ala
   465 470 475 480
   Leu Ser Gln Ala Arg Lys Val Ile Asn Gly Val Val Arg Arg Tyr Gly
   485 490 495
   Ser Pro Ala Arg Ile His Ile Glu Thr Ala Arg Glu Val Gly Lys Ser
   500 505 510
   Phe Lys Asp Arg Lys Glu Ile Glu Lys Arg Gln Glu Glu Asn Arg Lys
   515 520 525
   Asp Arg Glu Lys Ala Ala Ala Lys Phe Arg Glu Tyr Phe Pro Asn Phe
   530 535 540
   Val Gly Glu Pro Lys Ser Lys Asp Ile Leu Lys Leu Arg Leu Tyr Glu
   545 550 555 560
   Gln Gln His Gly Lys Cys Leu Tyr Ser Gly Lys Glu Ile Asn Leu Gly
   565 570 575
   Arg Leu Asn Glu Lys Gly Tyr Val Glu Ile Asp His Ala Leu Pro Phe
   580 585 590
   Ser Arg Thr Trp Asp Asp Ser Phe Asn Asn Lys Val Leu Val Leu Gly
   595 600 605
   Ser Glu Asn Gln Asn Lys Gly Asn Gln Thr Pro Tyr Glu Tyr Phe Asn
   610 615 620
   Gly Lys Asp Asn Ser Arg Glu Trp Gln Glu Phe Lys Ala Arg Val Glu
   625 630 635 640
   Thr Ser Arg Phe Pro Arg Ser Lys Lys Gln Arg Ile Leu Leu Gln Lys
   645 650 655
   Phe Asp Glu Asp Gly Phe Lys Glu Arg Asn Leu Asn Asp Thr Arg Tyr
   660 665 670
   Val Asn Arg Phe Leu Cys Gln Phe Val Ala Asp Arg Met Arg Leu Thr
   675 680 685
   Gly Lys Gly Lys Lys Arg Val Phe Ala Ser Asn Gly Gln Ile Thr Asn
   690 695 700
   Leu Leu Arg Gly Phe Trp Gly Leu Arg Lys Val Arg Ala Glu Asn Asp
   705 710 715 720
   Arg His His Ala Leu Asp Ala Val Val Val Ala Cys Ser Thr Val Ala
   725 730 735
   Met Gln Gln Lys Ile Thr Arg Phe Val Arg Tyr Lys Glu Met Asn Ala
   740 745 750
   Phe Asp Gly Lys Thr Ile Asp Lys Glu Thr Gly Glu Val Leu His Gln
   755 760 765
   Lys Thr His Phe Pro Gln Pro Trp Glu Phe Phe Ala Gln Glu Val Met
   770 775 780
   Ile Arg Val Phe Gly Lys Pro Asp Gly Lys Pro Glu Phe Glu Glu Ala
   785                 790                 795                 800
   Asp Thr Pro Glu Lys Leu Arg Thr Leu Leu Ala Glu Lys Leu Ser Ser
                   805                 810                 815
   Arg Pro Glu Ala Val His Glu Tyr Val Thr Pro Leu Phe Val Ser Arg
               820                 825                 830
   Ala Pro Asn Arg Lys Met Ser Gly Gln Gly His Met Glu Thr Val Lys
           835                 840                 845
   Ser Ala Lys Arg Leu Asp Glu Gly Val Ser Val Leu Arg Val Pro Leu
       850                 855                 860
   Thr Gln Leu Lys Leu Lys Asp Leu Glu Lys Met Val Asn Arg Glu Arg
   865                 870                 875                 880
   Glu Pro Lys Leu Tyr Glu Ala Leu Lys Ala Arg Leu Glu Ala His Lys
                   885                 890                 895
   Asp Asp Pro Ala Lys Ala Phe Ala Glu Pro Phe Tyr Lys Tyr Asp Lys
               900                 905                 910
   Ala Gly Asn Arg Thr Gln Gln Val Lys Ala Val Arg Val Glu Gln Val
           915                 920                 925
   Gln Lys Thr Gly Val Trp Val Arg Asn His Asn Gly Ile Ala Asp Asn
       930                 935                 940
   Ala Thr Met Val Arg Val Asp Val Phe Glu Lys Gly Asp Lys Tyr Tyr
   945                 950                 955                 960
   Leu Val Pro Ile Tyr Ser Trp Gln Val Ala Lys Gly Ile Leu Pro Asp
                   965                 970                 975
   Arg Ala Val Val Gln Gly Lys Asp Glu Glu Asp Trp Gln Leu Ile Asp
               980                 985                 990
   Asp Ser Phe Asn Phe Lys Phe Ser Leu His Pro Asn Asp Leu Val Glu
           995                 1000                1005
   Val Ile Thr Lys Lys Ala Arg Met Phe Gly Tyr Phe Ala Ser Cys His
       1010                1015                1020
   Arg Gly Thr Gly Asn Ile Asn Ile Arg Ile His Asp Leu Asp His Lys
   1025                1030                1035                1040
   Ile Gly Lys Asn Gly Ile Leu Glu Gly Ile Gly Val Lys Thr Ala Leu
                   1045                1050                1055
   Ser Phe Gln Lys Tyr Gln Ile Asp Glu Leu Gly Lys Glu Ile Arg Pro
               1060                1065                1070
   Cys Arg Leu Lys Lys Arg Pro Pro Val Arg
           1075                1080
<210> 75
   <211> 1348
   <212> PRT
   <213> Streptococcus equi
<220>
   <223> CRISPR-associated endonuclease
<308> YP_002123679
   <309> 2013-06-27
<400> 75
   Met Lys Lys Pro Tyr Thr Ile Ala Leu Asp Ile Gly Thr Asn Ser Val
    1               5                  10                  15
   Gly Trp Val Val Val Thr Asp Asp Tyr Arg Val Pro Thr Lys Lys Met
               20                  25                  30
   Lys Val Leu Gly Asn Thr Glu Arg Lys Thr Ile Lys Lys Asn Leu Ile
           35                  40                  45
   Gly Ala Leu Leu Phe Asp Ser Gly Asp Thr Ala Glu Gly Thr Arg Leu
       50                  55                  60
   Lys Arg Thr Ala Arg Arg Arg Tyr Thr Arg Arg Lys Asn Arg Leu Arg
   65                  70                  75                  80
   Tyr Leu Lys Glu Ile Phe Thr Glu Glu Met Ala Lys Val Asp Asp Gly
                   85                  90                  95
   Phe Phe Gln Arg Leu Glu Asp Ser Phe Tyr Val Leu Glu Asp Lys Glu
               100                 105                 110
   Gly Asn Lys His Pro Ile Phe Ala Asn Leu Ala Asp Glu Val Ala Tyr
           115                 120                 125
   His Lys Lys Tyr Pro Thr Ile Tyr His Leu Arg Lys Glu Leu Val Asp
       130                 135                 140
   Asn Pro Gln Lys Ala Asp Leu Arg Leu Ile Tyr Leu Ala Val Ala His
   145                 150                 155                 160
   Ile Ile Lys Phe Arg Gly His Phe Leu Ile Glu Gly Thr Leu Ser Ser
                   165                 170                 175
   Lys Asn Asn Asn Leu Gln Lys Ser Phe Asp His Leu Val Asp Thr Tyr
               180                 185                 190
   Asn Leu Leu Phe Glu Glu Gln Arg Leu Leu Thr Glu Gly Ile Asn Ala
           195                 200                 205
   Lys Glu Leu Leu Ser Ala Ala Leu Ser Lys Ser Lys Arg Leu Glu Asn
       210                 215                 220
   Leu Ile Ser Leu Ile Pro Gly Gln Lys Lys Thr Gly Ile Phe Gly Asn
   225                 230                 235                 240
   Ile Ile Ala Leu Ser Leu Gly Leu Thr Pro Asn Phe Lys Ala Asn Phe
                   245                 250                 255
   Gly Leu Ser Lys Asp Val Lys Leu Gln Leu Ala Lys Asp Thr Tyr Ala
               260                 265                 270
   Asp Asp Leu Asp Ser Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Thr Glu Ser Asp Glu Ile Thr Arg Ala Pro Leu Ser Ala Ser
   305                 310                 315                 320
   Met Val Lys Arg Tyr Arg Glu His His Lys Asp Leu Val Thr Leu Lys
                   325                 330                 335
   Thr Leu Ile Lys Asp Gln Leu Pro Glu Lys Tyr Gln Glu Ile Phe Leu
               340                 345                 350
   Asp Lys Thr Lys Asn Gly Tyr Ala Gly Tyr Ile Glu Gly Gln Val Ser
           355                 360                 365
   Gln Glu Glu Phe Tyr Lys Tyr Leu Lys Pro Ile Leu Ala Arg Leu Asp
       370                 375                 380
   Gly Ser Glu Pro Leu Leu Leu Lys Ile Asp Arg Glu Asp Phe Leu Arg
   385                 390                 395                 400
   Lys Gln Arg Thr Phe Asp Asn Gly Ser Ile Pro His Gln Ile His Leu
                   405                 410                 415
   Glu Glu Leu His Ala Ile Leu Arg Arg Gln Glu Val Phe Tyr Pro Phe
               420                 425                 430
   Leu Lys Asp Asn Arg Lys Lys Ile Glu Ser Leu Leu Thr Phe Arg Ile
           435                 440                 445
   Pro Tyr Tyr Val Gly Pro Leu Ala Arg Gly His Ser Arg Phe Ala Trp
       450                 455                 460
   Val Lys Arg Lys Phe Asp Gly Ala Ile Arg Pro Trp Asn Phe Glu Glu
   465                 470                 475                 480
   Ile Val Asp Glu Glu Ala Ser Ala Gln Ile Phe Ile Glu Lys Met Thr
                   485                 490                 495
   Lys Asn Asp Leu Tyr Leu Pro Asn Glu Lys Val Leu Pro Lys His Ser
               500                 505                 510
   Leu Leu Tyr Glu Thr Phe Thr Val Tyr Asn Glu Leu Thr Lys Val Lys
           515                 520                 525
   Tyr Ala Thr Glu Gly Met Thr Arg Pro Gln Phe Leu Ser Ala Asp Gln
       530                 535                 540
   Lys Gln Ala Ile Val Asp Leu Leu Phe Lys Thr Asn Arg Lys Val Thr
   545                 550                 555                 560
   Val Lys Gln Leu Lys Glu Asn Tyr Phe Lys Lys Ile Glu Cys Trp Asp
                   565                 570                 575
   Ser Val Glu Ile Thr Gly Val Glu Asp Ser Phe Asn Ala Ser Leu Gly
               580                 585                 590
   Thr Tyr His Asp Leu Leu Lys Ile Ile Gln Asp Lys Asp Phe Leu Asp
           595                 600                 605
   Asn Pro Asp Asn Gln Lys Ile Ile Glu Asp Ile Ile Leu Thr Leu Thr
       610                 615                 620
   Leu Phe Glu Asp Lys Lys Met Ile Ser Lys Arg Leu Asp Gln Tyr Ala
   625                 630                 635                 640
   His Leu Phe Asp Lys Val Val Leu Asn Lys Leu Glu Arg His His Tyr
                   645                 650                 655
   Thr Gly Trp Gly Arg Leu Ser Gly Lys Leu Ile Asn Gly Ile Arg Asp
               660                 665                 670
   Lys Gln Ser Gly Lys Thr Ile Leu Asp Phe Leu Lys Ala Asp Gly Phe
           675                 680                 685
   Ala Asn Arg Asn Phe Met Gln Leu Ile His Asp Ser Glu Leu Ser Phe
       690                 695                 700
   Ile Asp Glu Ile Ala Lys Ala Gln Val Ile Gly Lys Thr Glu Tyr Ser
   705                 710                 715                 720
   Lys Asp Leu Val Gly Asn Leu Ala Gly Ser Pro Ala Ile Lys Lys Gly
                   725                 730                 735
   Ile Ser Gln Thr Ile Lys Ile Val Asp Glu Leu Val Lys Ile Met Gly
               740                 745                 750
   Tyr Leu Pro Gln Gln Ile Val Ile Glu Met Ala Arg Glu Asn Gln Thr
           755                 760                 765
   Thr Ala Gln Gly Ile Lys Asn Ala Arg Gln Arg Met Arg Lys Leu Glu
       770                 775                 780
   Glu Thr Ala Lys Lys Leu Gly Ser Asn Ile Leu Lys Glu His Pro Val
   785                 790                 795                 800
   Asp Asn Ser Gln Leu Gln Asn Asp Lys Arg Tyr Leu Tyr Tyr Leu Gln
                   805                 810                 815
   Asn Gly Lys Asp Met Tyr Thr Gly Asp Asp Leu Asp Ile Asp Tyr Leu
               820                 825                 830
   Ser Ser Tyr Asp Ile Asp His Ile Ile Pro Gln Ser Phe Ile Lys Asn
           835                 840                 845
   Asn Ser Ile Asp Asn Lys Val Leu Thr Ser Gln Gly Ala Asn Arg Gly
       850                 855                 860
   Lys Leu Asp Asn Val Pro Ser Glu Ala Ile Val Arg Lys Met Lys Gly
   865                 870                 875                 880
   Tyr Trp Gln Ser Leu Leu Arg Ala Gly Ala Ile Ser Lys Gln Lys Phe
                   885                 890                 895
   Asp Asn Leu Thr Lys Ala Glu Arg Gly Gly Leu Thr Gln Val Asp Lys
               900                 905                 910
   Ala Gly Phe Ile Gln Arg Gln Leu Val Glu Thr Arg Gln Ile Thr Lys
           915                 920                 925
   His Val Ala Gln Ile Leu Asp Ser Arg Phe Asn Thr Glu Phe Asp Asp
       930                 935                 940
   His Asn Lys Arg Ile Arg Lys Val His Ile Ile Thr Leu Lys Ser Lys
   945                 950                 955                 960
   Leu Val Ser Asp Phe Arg Lys Glu Phe Gly Leu Tyr Lys Ile Arg Asp
                   965                 970                 975
   Ile Asn His Tyr His His Ala His Asp Ala Tyr Leu Asn Ala Val Val
               980                 985                 990
   Ala Lys Ala Ile Leu Gly Lys Tyr Pro Gln Leu Ala Pro Glu Phe Val
           995                 1000                1005
   Tyr Gly Asp Tyr Pro Lys Tyr Asn Ser Phe Lys Glu Arg Gln Lys Ala
       1010                1015                1020
   Thr Gln Lys Met Leu Phe Tyr Ser Asn Ile Leu Lys Phe Phe Lys Asp
   1025                1030                1035                1040
   Gln Glu Ser Leu His Val Asn Ser Asp Gly Glu Glu Ile Trp Asn Ala
                   1045                1050                1055
   Asn Lys His Leu Pro Ile Ile Lys Asn Val Leu Ser Ile Pro Gln Val
               1060                1065                1070
   Asn Ile Val Lys Lys Thr Glu Val Gln Thr Gly Gly Phe Tyr Lys Glu
           1075                1080                1085
   Ser Ile Leu Ser Lys Gly Asn Ser Asp Lys Leu Ile Pro Arg Lys Asn
       1090                1095                1100
   Asn Trp Asp Thr Arg Lys Tyr Gly Gly Phe Asp Ser Pro Thr Val Ala
   1105                1110                1115                1120
   Tyr Ser Val Leu Val Ile Ala Lys Met Glu Lys Gly Lys Ala Lys Val
                   1125                1130                1135
   Leu Lys Pro Val Lys Glu Met Val Gly Ile Thr Ile Met Glu Arg Thr
               1140                1145                1150
   Ala Phe Glu Glu Asn Pro Val Val Phe Leu Glu Ala Arg Gly Tyr Arg
           1155                1160                1165
   Glu Ile Gln Glu His Leu Ile Ile Lys Leu Pro Lys Tyr Ser Leu Phe
       1170                1175                1180
   Glu Leu Glu Asn Gly Arg Arg Arg Leu Leu Ala Ser Ala Ser Glu Leu
   1185                1190                1195                1200
   Gln Lys Gly Asn Glu Leu Phe Leu Pro Val Asp Tyr Met Thr Phe Leu
                   1205                1210                1215
   Tyr Leu Ala Ala His Tyr His Glu Leu Thr Gly Ser Ser Glu Asp Val
               1220                1225                1230
   Leu Arg Lys Lys Tyr Phe Val Asp Arg His Leu His Tyr Phe Asp Asp
           1235                1240                1245
   Ile Ile Gln Met Ile Asn Asp Phe Ala Glu Arg His Ile Leu Ala Ser
       1250                1255                1260
   Ser Asn Leu Glu Lys Ile Asn His Thr Tyr His Asn Asn Ser Asp Leu
   1265                1270                1275                1280
   Pro Val Asn Glu Arg Ala Glu Asn Ile Ile Asn Val Phe Thr Phe Val
                   1285                1290                1295
   Ala Leu Gly Ala Pro Ala Ala Phe Lys Phe Phe Asp Ala Thr Ile Asp
               1300                1305                1310
   Arg Lys Arg Tyr Thr Ser Thr Lys Glu Val Leu Asn Ala Thr Leu Ile
           1315                1320                1325
   His Gln Ser Val Thr Gly Leu Tyr Glu Thr Arg Ile Asp Leu Ser Gln
       1330                1335                1340
   Leu Gly Glu Asn
   1345
<210> 76
   <211> 1136
   <212> PRT
   <213> Streptococcus gordonii
<220>
   <223> CRISPR-associated endonuclease
<308> YP_001450662
   <309> 2013-06-10
<400> 76
   Met Asn Gly Leu Val Leu Gly Leu Asp Ile Gly Ile Ala Ser Val Gly
    1               5                  10                  15
   Val Gly Ile Leu Glu Lys Asp Thr Gly Lys Ile Ile His Ala Ser Ser
               20                  25                  30
   Arg Leu Phe Pro Ala Ala Thr Ala Asp Asn Asn Val Glu Arg Arg Ser
           35                  40                  45
   Asn Arg Gln Gly Arg Arg Leu Asn Arg Arg Lys Lys His Arg Ser Val
       50                  55                  60
   Arg Leu Gln Asp Leu Phe Glu Gly Tyr Gly Leu Leu Thr Asp Phe Ser
   65                  70                  75                  80
   Lys Val Ser Met Asn Leu Asn Pro Tyr Gln Leu Arg Val Gln Gly Met
                   85                  90                  95
   Glu Asn Gln Leu Thr Asn Glu Glu Leu Phe Val Ala Leu Lys Asn Ile
               100                 105                 110
   Val Lys Arg Arg Gly Ile Ser Tyr Leu Asp Asp Ala Ser Glu Asp Gly
           115                 120                 125
   Gly Thr Val Ser Ser Asp Tyr Gly Lys Ala Val Glu Glu Asn Arg Lys
           130                 135                 140
   Leu Leu Ala Glu Lys Thr Pro Gly Gln Ile Gln Leu Glu Arg Phe Glu
   145                 150                 155                 160
   Lys Tyr Gly Gln Leu Arg Gly Asp Phe Thr Val Glu Glu Asn Gly Glu
                   165                 170                 175
   Lys His Arg Leu Ile Asn Val Phe Ser Thr Ser Ala Tyr Arg Lys Glu
               180                 185                 190
   Ala Glu Arg Ile Leu Arg Lys Gln Gln Glu Phe Asn Ser Lys Ile Thr
           195                 200                 205
   Asp Glu Phe Ile Glu Asp Tyr Leu Ile Ile Leu Thr Gly Lys Arg Lys
       210                 215                 220
   Tyr Tyr His Gly Pro Gly Asn Glu Lys Ser Arg Thr Asp Tyr Gly Arg
   225                 230                 235                 240
   Phe Arg Thr Asp Gly Thr Thr Leu Asp Asn Ile Phe Gly Ile Leu Ile
                   245                 250                 255
   Gly Lys Cys Thr Phe Tyr Thr Glu Glu Tyr Arg Ala Ser Lys Ala Ser
               260                 265                 270
   Tyr Thr Ala Gln Glu Phe Asn Leu Leu Asn Asp Leu Asn Asn Leu Thr
           275                 280                 285
   Val Pro Thr Glu Thr Lys Lys Leu Ser Glu Glu Gln Lys Lys Leu Ile
       290                 295                 300
   Ile Glu Tyr Ala Lys Ser Ala Lys Thr Leu Gly Ala Ser Thr Leu Leu
   305                 310                 315                 320
   Lys Tyr Ile Ala Lys Met Ile Asp Ala Ser Val Asp Gln Ile Arg Gly
                   325                 330                 335
   Tyr Arg Val Asp Val Asn Asn Lys Pro Glu Met His Thr Phe Glu Val
               340                 345                 350
   Tyr Arg Lys Met Gln Ser Leu Glu Thr Ile Lys Val Glu Glu Leu Pro
           355                 360                 365
   Arg Lys Val Leu Asp Glu Leu Ala His Ile Leu Thr Leu Asn Thr Glu
       370                 375                 380
   Arg Glu Gly Ile Glu Glu Ala Ile Asn Ser Lys Leu Lys Asp Ile Phe
   385                 390                 395                 400
   Asn Arg Asp Gln Val Leu Glu Leu Val Gln Phe Arg Lys Asn Asn Ser
                   405                 410                 415
   Ser Leu Phe Ser Lys Gly Trp His Asn Phe Ser Ile Lys Leu Met Met
               420                 425                 430
   Glu Leu Ile Pro Glu Leu Tyr Glu Thr Ser Glu Glu Gln Met Thr Ile
           435                 440                 445
   Leu Thr Arg Leu Gly Lys Gln Arg Ser Lys Glu Thr Ser Lys Arg Thr
       450                 455                 460
   Lys Tyr Ile Asp Glu Lys Glu Leu Thr Glu Glu Ile Tyr Asn Pro Val
   465                 470                 475                 480
   Val Ala Lys Ser Val Arg Gln Ala Ile Lys Ile Ile Asn Glu Ala Thr
                   485                 490                 495
   Lys Lys Tyr Gly Ile Phe Asp Asn Ile Val Ile Glu Met Ala Arg Glu
               500                 505                 510
   Asn Asn Glu Glu Asp Ala Lys Lys Asp Tyr Ile Lys Arg Gln Lys Ala
           515                 520                 525
   Asn Gln Asp Glu Lys Asn Ala Ala Met Glu Lys Ala Ala Phe Gln Tyr
       530                 535                 540
   Asn Gly Lys Lys Glu Leu Pro Asp Asn Ile Phe His Gly His Lys Glu
   545                 550                 555                 560
   Leu Thr Thr Lys Ile Arg Leu Trp His Gln Gln Gly Glu Lys Cys Leu
                   565                 570                 575
   Tyr Thr Gly Lys Asn Ile Pro Ile Ser Asp Leu Ile His Asn Gln Tyr
               580                 585                 590
   Lys Tyr Glu Ile Asp His Ile Leu Pro Leu Ser Leu Ser Phe Asp Asp
           595                 600                 605
   Ser Leu Ser Asn Lys Val Leu Val Leu Ala Thr Ala Asn Gln Glu Lys
       610                 615                 620
   Gly Gln Arg Thr Pro Phe Gln Ala Leu Asp Ser Met Asp Asp Ala Trp
   625                 630                 635                 640
   Ser Tyr Arg Glu Phe Lys Ser Tyr Val Lys Asp Ser Lys Leu Leu Ser
   645 650 655
   Asn Lys Lys Lys Asp Tyr Leu Leu Thr Glu Glu Asp Ile Ser Lys Ile
   660 665 670
   Glu Val Lys Gln Lys Phe Ile Glu Arg Asn Leu Val Asp Thr Arg Tyr
   675 680 685
   Ser Ser Arg Val Val Leu Asn Ala Leu Gln Asp Phe Tyr Lys Ser His
   690 695 700
   Gln Leu Asp Thr Thr Ile Ser Val Val Arg Gly Gln Phe Thr Ser Gln
   705 710 715 720
   Leu Arg Arg Lys Trp Gly Ile Glu Lys Ser Arg Glu Thr Tyr His His
   725 730 735
   His Ala Val Asp Ala Leu Ile Ile Ala Ala Ser Ser Gln Leu Arg Leu
   740 745 750
   Trp Lys Lys His Ser Asn Pro Leu Ile Ala Tyr Lys Glu Gly Gln Phe
   755 760 765
   Val Asp Ser Glu Thr Gly Glu Ile Val Ser Leu Ser Asp Glu Glu Tyr
   770 775 780
   Lys Glu Leu Val Phe Lys Ala Pro Tyr Asp His Phe Val Asp Thr Leu
   785 790 795 800
   Arg Ser Lys Lys Phe Glu Asp Ser Ile Leu Phe Ser Tyr Gln Val Asp
   805 810 815
   Ser Lys Tyr Asn Arg Lys Ile Ser Asp Ala Thr Ile Tyr Ala Thr Arg
   820 825 830
   Lys Ala Lys Leu Asp Lys Glu Lys Lys Glu Tyr Thr Tyr Thr Leu Gly
   835 840 845
   Lys Ile Lys Asp Ile Tyr Ala Leu Gly Thr Lys Thr Pro Ser Lys Thr
   850 855 860
   Gly Phe Tyr Lys Phe Leu Asp Leu Tyr Lys Thr Asp Lys Ser Gln Phe
   865 870 875 880
   Leu Met Tyr Gln Lys Asp Arg Lys Thr Trp Asp Glu Val Ile Glu Lys
   885 890 895
   Ile Ile Glu Gln Tyr Arg Pro Phe Lys Glu Tyr Asp Lys Asn Gly Lys
   900 905 910
   Glu Val Asp Phe Asn Pro Phe Glu Lys Tyr Arg Ile Gly Asn Gly Pro
   915 920 925
   Ile Arg Lys Tyr Ser Lys Lys Gly Asn Gly Pro Glu Ile Lys Ser Leu
   930 935 940
   Lys Tyr Tyr Asp Ile Leu Leu Gly Lys His Lys Asn Ile Thr Pro Asp
   945 950 955 960
   Gly Ser Arg Asn Thr Val Ala Leu Leu Ser Leu Asn Pro Trp Arg Thr
   965 970 975
   Asp Val Tyr Tyr Asn Ser Glu Thr Lys Lys Tyr Glu Phe Leu Gly Leu
   980 985 990
   Lys Tyr Ala Asp Leu Cys Phe Glu Glu Gly Gly Ala Tyr Gly Ile Ser
   995 1000 1005
   Glu Val Lys Tyr Lys Lys Ile Arg Glu Lys Glu Gly Ile Gly Lys Asn
   1010 1015 1020
   Ser Glu Phe Lys Phe Thr Leu Tyr Lys Asn Asp Leu Ile Leu Ile Lys
   1025 1030 1035 1040
   Asp Thr Glu Thr Asn Cys Gln Gln Phe Phe Arg Phe Trp Ser Arg Thr
   1045 1050 1055
   Gly Lys Asp Asn Pro Lys Ser Phe Glu Lys His Lys Ile Glu Leu Lys
   1060 1065 1070
   Pro Tyr Glu Lys Ala Lys Phe Glu Lys Gly Glu Glu Leu Lys Val Leu
   1075 1080 1085
   Gly Lys Val Pro Pro Ser Ser Asn Gln Phe Gln Lys Asn Met Gln Ile
   1090 1095 1100
   Glu Asn Leu Ser Ile Tyr Lys Val Lys Thr Asp Ile Leu Gly Asn Lys
   1105 1110 1115 1120
   His Phe Ile Lys Lys Glu Gly Asp Glu Pro Lys Leu Lys Phe Lys Lys
   1125 1130 1135
<210> 77
   <211> 1368
   <212> PRT
   <213> Streptococcus pyogenes
<220>
   <223> CRISPR-associated endonuclease
<308> YP_280216
   <309> 2013-08-28
<400> 77
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Leu Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Ala Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Gln Ile Leu Lys Glu His Pro
   785                 790                 795                 800
   Val Glu Asn Thr Gln Leu Gln Asn Glu Lys Leu Tyr Leu Tyr Tyr Leu
                   805                 810                 815
   Gln Asn Gly Arg Asp Met Tyr Val Asp Gln Glu Leu Asp Ile Asn Arg
               820                 825                 830
   Leu Ser Asp Tyr Asp Val Asp His Ile Val Pro Gln Ser Phe Ile Lys
           835                 840                 845
   Asp Asp Ser Ile Asp Asn Lys Val Leu Thr Arg Ser Asp Lys Asn Arg
       850                 855                 860
   Gly Lys Ser Asn Asn Val Pro Ser Glu Glu Val Val Lys Lys Met Lys
   865                 870                 875                 880
   Asn Tyr Trp Arg Gln Leu Leu Asn Ala Lys Leu Ile Thr Gln Arg Lys
                   885                 890                 895
   Phe Asp Asn Leu Thr Lys Ala Glu Arg Gly Gly Leu Ser Glu Leu Asp
                   900                 905                 910
   Lys Ala Gly Phe Ile Lys Arg Gln Leu Val Glu Thr Arg Gln Ile Thr
               915                 920                 925
   Lys His Val Ala Gln Ile Leu Asp Ser Arg Met Asn Thr Lys Tyr Asp
       930                 935                 940
   Glu Asn Asp Lys Leu Ile Arg Glu Val Lys Val Ile Thr Leu Lys Ser
   945                 950                 955                 960
   Lys Leu Val Ser Asp Phe Arg Lys Asp Phe Gln Phe Tyr Lys Val Arg
                   965                 970                 975
   Glu Ile Asn Asn Tyr His His Ala His Asp Ala Tyr Leu Asn Ala Val
               980                 985                 990
   Val Gly Thr Ala Leu Ile Lys Lys Tyr Pro Lys Leu Glu Ser Glu Phe
           995                 1000                1005
   Val Tyr Gly Asp Tyr Lys Val Tyr Asp Val Arg Lys Met Ile Ala Lys
       1010                1015                1020
   Ser Glu Gln Glu Ile Gly Lys Ala Thr Ala Lys Tyr Phe Phe Tyr Ser
   1025                1030                1035                1040
   Asn Ile Met Asn Phe Phe Lys Thr Glu Ile Thr Leu Ala Asn Gly Glu
                   1045                1050                1055
   Ile Arg Lys Arg Pro Leu Ile Glu Thr Asn Gly Glu Thr Gly Glu Ile
               1060                1065                1070
   Val Trp Asp Lys Gly Arg Asp Phe Ala Thr Val Arg Lys Val Leu Ser
           1075                1080                1085
   Met Pro Gln Val Asn Ile Val Lys Lys Thr Glu Val Gln Thr Gly Gly
       1090                1095                1100
   Phe Ser Lys Glu Ser Ile Leu Pro Lys Arg Asn Ser Asp Lys Leu Ile
   1105                1110                1115                1120
   Ala Arg Lys Lys Asp Trp Asp Pro Lys Lys Tyr Gly Gly Phe Asp Ser
                   1125                1130                1135
   Pro Thr Val Ala Tyr Ser Val Leu Val Val Ala Lys Val Glu Lys Gly
               1140                1145                1150
   Lys Ser Lys Lys Leu Lys Ser Val Lys Glu Leu Leu Gly Ile Thr Ile
           1155                1160                1165
   Met Glu Arg Ser Ser Phe Glu Lys Asn Pro Ile Asp Phe Leu Glu Ala
       1170                1175                1180
   Lys Gly Tyr Lys Glu Val Arg Lys Asp Leu Ile Val Lys Leu Pro Lys
   1185                1190                1195                1200
   Tyr Ser Leu Phe Glu Leu Glu Asn Gly Arg Lys Arg Met Leu Ala Ser
                   1205                1210                1215
   Ala Gly Glu Leu Gln Lys Gly Asn Glu Leu Ala Leu Pro Ser Lys Tyr
               1220                1225                1230
   Val Asn Phe Leu Tyr Leu Ala Ser His Tyr Glu Lys Leu Lys Gly Ser
           1235                1240                1245
   Pro Glu Asp Asn Glu Gln Lys Gln Leu Phe Val Glu Gln His Lys His
       1250                1255                1260
   Tyr Leu Asp Glu Ile Ile Glu Gln Ile Ser Glu Phe Ser Lys Arg Val
   1265                1270                1275                1280
   Ile Leu Ala Asp Ala Asn Leu Asp Lys Val Leu Ser Ala Tyr Asn Lys
                   1285                1290                1295
   His Arg Asp Lys Pro Ile Arg Glu Gln Ala Glu Asn Ile Ile His Leu
               1300                1305                1310
   Phe Thr Leu Thr Asn Leu Gly Ala Pro Ala Ala Phe Lys Tyr Phe Asp
           1315                1320                1325
   Thr Thr Ile Asp Arg Lys Arg Tyr Thr Ser Thr Lys Glu Val Leu Asp
       1330                1335                1340
   Ala Thr Phe Ile His Gln Ser Ile Thr Gly Leu Tyr Glu Thr Arg Ile
   1345                1350                1355                1360
   Asp Leu Ser Gln Leu Gly Gly Asp
                   1365
<210> 78
   <211> 1370
   <212> PRT
   <213> Streptococcus gallolyticus
<220>
   <223> CRISPR-associated endonuclease
<308> YP_004288385
   <309> 2013-07-06
<400> 78
   Met Thr Lys Lys Asn Tyr Ser Ile Gly Leu Asp Ile Gly Thr Asn Ser
   1 5 10 15
   Val Gly Trp Ala Val Ile Thr Asp Asp Tyr Lys Val Pro Ala Lys Lys
   20 25 30
   Met Lys Val Leu Gly Asn Thr Asp Lys Lys Tyr Ile Lys Lys Asn Leu
   35 40 45
   Leu Gly Ala Leu Leu Phe Asp Ser Gly Glu Thr Ala Glu Ala Thr Arg
   50 55 60
   Leu Lys Arg Thr Ala Arg Arg Arg Tyr Thr Arg Arg Lys Asn Arg Leu
   65 70 75 80
   Arg Tyr Leu Gln Glu Ile Phe Ala Glu Glu Met Thr Lys Val Asp Glu
   85 90 95
   Ser Phe Phe Tyr Arg Leu Asp Glu Ser Phe Leu Thr Thr Asp Glu Lys
   100 105 110
   Asp Phe Glu Arg His Pro Ile Phe Gly Asn Lys Ala Glu Glu Asp Ala
   115 120 125
   Tyr His Gln Lys Phe Pro Thr Ile Tyr His Leu Arg Asn Tyr Leu Ala
   130 135 140
   Asp Ser Ser Glu Lys Ala Asp Leu Arg Leu Val Tyr Leu Ala Leu Ala
   145 150 155 160
   His Met Ile Lys Tyr Arg Gly His Phe Leu Ile Glu Gly Lys Leu Asn
   165 170 175
   Ala Glu Asn Thr Asp Val Gln Lys Leu Phe Thr Asp Phe Val Gly Val
   180 185 190
   Tyr Asp Arg Thr Phe Asp Asp Ser His Leu Ser Glu Ile Thr Val Asp
   195 200 205
   Val Ala Ser Thr Leu Thr Glu Lys Ile Ser Lys Ser Arg Arg Leu Glu
   210 215 220
   Asn Leu Ile Lys Tyr Tyr Pro Thr Glu Lys Lys Asn Thr Leu Phe Gly
   225 230 235 240
   Asn Leu Ile Ala Leu Ala Leu Gly Leu Gln Pro Asn Phe Lys Met Asn
   245 250 255
   Phe Lys Leu Ser Glu Asp Ala Lys Leu Gln Phe Ser Lys Asp Thr Tyr
   260 265 270
   Glu Glu Asp Leu Glu Glu Leu Leu Gly Lys Ile Gly Asp Asp Tyr Ala
   275 280 285
   Asp Leu Phe Thr Ser Ala Lys Asn Leu Tyr Asp Ala Ile Leu Leu Ser
   290 295 300
   Gly Ile Leu Thr Val Asp Asp Asn Ser Thr Lys Ala Pro Leu Ser Ala
   305 310 315 320
   Ser Met Ile Lys Arg Tyr Val Glu His His Glu Asp Leu Glu Lys Leu
   325 330 335
   Lys Glu Phe Ile Lys Ala Asn Lys Ser Glu Leu Tyr His Asp Ile Phe
   340 345 350
   Lys Asp Lys Asn Lys Asn Gly Tyr Ala Gly Tyr Ile Glu Asn Gly Val
   355 360 365
   Lys Gln Asp Glu Phe Tyr Lys Tyr Leu Lys Asn Thr Leu Ser Lys Ile
   370 375 380
   Asp Gly Ser Asp Tyr Phe Leu Asp Lys Ile Glu Arg Glu Asp Phe Leu
   385 390 395 400
   Arg Lys Gln Arg Thr Phe Asp Asn Gly Ser Ile Pro His Gln Ile His
   405 410 415
   Leu Gln Glu Met His Ala Ile Leu Arg Arg Gln Gly Asp Tyr Tyr Pro
   420 425 430
   Phe Leu Lys Glu Lys Gln Asp Arg Ile Glu Lys Ile Leu Thr Phe Arg
   435 440 445
   Ile Pro Tyr Tyr Val Gly Pro Leu Val Arg Lys Asp Ser Arg Phe Ala
   450 455 460
   Trp Ala Glu Tyr Arg Ser Asp Glu Lys Ile Thr Pro Trp Asn Phe Asp
   465 470 475 480
   Lys Val Ile Asp Lys Glu Lys Ser Ala Glu Lys Phe Ile Thr Arg Met
   485 490 495
   Thr Leu Asn Asp Leu Tyr Leu Pro Glu Glu Lys Val Leu Pro Lys His
   500 505 510
   Ser His Val Tyr Glu Thr Tyr Ala Val Tyr Asn Glu Leu Thr Lys Ile
   515 520 525
   Lys Tyr Val Asn Glu Gln Gly Lys Glu Ser Phe Phe Asp Ser Asn Met
   530 535 540
   Lys Gln Glu Ile Phe Asp His Val Phe Lys Glu Asn Arg Lys Val Thr
   545 550 555 560
   Lys Glu Lys Leu Leu Asn Tyr Leu Asn Lys Glu Phe Pro Glu Tyr Arg
   565 570 575
   Ile Lys Asp Leu Ile Gly Leu Asp Lys Glu Asn Lys Ser Phe Asn Ala
   580 585 590
   Ser Leu Gly Thr Tyr His Asp Leu Lys Lys Ile Leu Asp Lys Ala Phe
   595 600 605
   Leu Asp Asp Lys Val Asn Glu Glu Val Ile Glu Asp Ile Ile Lys Thr
   610 615 620
   Leu Thr Leu Phe Glu Asp Lys Asp Met Ile His Glu Arg Leu Gln Lys
   625 630 635 640
   Tyr Ser Asp Ile Phe Thr Ala Asn Gln Leu Lys Lys Leu Glu Arg Arg
   645 650 655
   His Tyr Thr Gly Trp Gly Arg Leu Ser Tyr Lys Leu Ile Asn Gly Ile
   660 665 670
   Arg Asn Lys Glu Asn Asn Lys Thr Ile Leu Asp Tyr Leu Ile Asp Asp
   675 680 685
   Gly Ser Ala Asn Arg Asn Phe Met Gln Leu Ile Asn Asp Asp Thr Leu
   690 695 700
   Pro Phe Lys Gln Ile Ile Gln Lys Ser Gln Val Val Gly Asp Val Asp
   705 710 715 720
   Asp Ile Glu Ala Val Val His Asp Leu Pro Gly Ser Pro Ala Ile Lys
   725 730 735
   Lys Gly Ile Leu Gln Ser Val Lys Ile Val Asp Glu Leu Val Lys Val
   740 745 750
   Met Gly Asp Asn Pro Asp Asn Ile Val Ile Glu Met Ala Arg Glu Asn
   755 760 765
   Gln Thr Thr Asn Arg Gly Arg Ser Gln Ser Gln Gln Arg Leu Lys Lys
   770 775 780
   Leu Gln Ser Ser Leu Lys Glu Leu Gly Ser Asn Ile Leu Asn Glu Glu
   785 790 795 800
   Lys Pro Ser Tyr Ile Glu Asp Lys Val Glu Asn Ser His Leu Gln Asn
   805 810 815
   Asp Gln Leu Phe Leu Tyr Tyr Ile Gln Asn Gly Lys Asp Met Tyr Thr
   820 825 830
   Gly Asp Glu Leu Asp Ile Asp His Leu Ser Asp Tyr Asp Ile Asp His
   835 840 845
   Ile Ile Pro Gln Ala Phe Ile Lys Asp Asp Ser Ile Asp Asn Arg Val
   850 855 860
   Leu Thr Ser Ser Ala Lys Asn Arg Gly Lys Ser Asp Asp Val Pro Ser
   865 870 875 880
   Leu Asp Ile Val Arg Ala Arg Lys Ala Glu Trp Val Arg Leu Tyr Lys
   885 890 895
   Ser Gly Leu Ile Ser Lys Arg Lys Phe Asp Asn Leu Thr Lys Ala Glu
   900 905 910
   Arg Gly Gly Leu Thr Glu Ala Asp Lys Ala Gly Phe Ile Lys Arg Gln
   915 920 925
   Leu Val Glu Thr Arg Gln Ile Thr Lys His Val Ala Gln Ile Leu Asp
   930 935 940
   Ala Arg Phe Asn Thr Glu His Asp Glu Asn Asp Lys Val Ile Arg Asp
   945 950 955 960
   Val Lys Val Ile Thr Leu Lys Ser Asn Leu Val Ser Gln Phe Arg Lys
   965 970 975
   Asp Phe Glu Phe Tyr Lys Val Arg Glu Ile Asn Asp Tyr His His Ala
   980 985 990
   His Asp Ala Tyr Leu Asn Ala Val Val Gly Thr Ala Leu Leu Lys Lys
   995 1000 1005
   Tyr Pro Lys Leu Ala Ser Glu Phe Val Tyr Gly Glu Tyr Lys Lys Tyr
   1010 1015 1020
   Asp Ile Arg Lys Phe Ile Thr Asn Ser Ser Asp Lys Ala Thr Ala Lys
   1025 1030 1035 1040
   Tyr Phe Phe Tyr Ser Asn Leu Met Asn Phe Phe Lys Thr Lys Val Lys
   1045 1050 1055
   Tyr Ala Asp Gly Thr Val Phe Glu Arg Pro Ile Ile Glu Thr Asn Ala
   1060 1065 1070
   Asp Gly Glu Ile Ala Trp Asn Lys Gln Ile Asp Phe Glu Lys Val Arg
   1075 1080 1085
   Lys Val Leu Ser Tyr Pro Gln Val Asn Ile Val Lys Lys Val Glu Thr
   1090 1095 1100
   Gln Thr Gly Gly Phe Ser Lys Glu Ser Ile Leu Pro Lys Gly Asp Ser
   1105 1110 1115 1120
   Asp Lys Leu Ile Pro Arg Lys Thr Lys Lys Val Tyr Trp Asp Thr Lys
   1125 1130 1135
   Lys Tyr Gly Gly Phe Asp Ser Pro Thr Val Ala Tyr Ser Val Phe Val
   1140 1145 1150
   Val Ala Asp Val Glu Lys Gly Lys Ala Lys Lys Leu Lys Thr Val Lys
   1155 1160 1165
   Glu Leu Val Gly Ile Ser Ile Met Glu Arg Ser Phe Phe Glu Glu Asn
   1170 1175 1180
   Pro Val Glu Phe Leu Glu Asn Lys Gly Tyr His Asn Ile Arg Glu Asp
   1185 1190 1195 1200
   Lys Leu Ile Lys Leu Pro Lys Tyr Ser Leu Phe Glu Phe Glu Gly Gly
   1205 1210 1215
   Arg Arg Arg Leu Leu Ala Ser Ala Ser Glu Leu Gln Lys Gly Asn Glu
   1220 1225 1230
   Met Val Leu Pro Gly Tyr Leu Val Glu Leu Leu Tyr His Ala His Arg
   1235 1240 1245
   Ala Asp Asn Phe Asn Ser Thr Glu Tyr Leu Asn Tyr Val Ser Glu His
   1250 1255 1260
   Lys Lys Glu Phe Glu Lys Val Leu Ser Cys Val Glu Asp Phe Ala Asn
   1265 1270 1275 1280
   Leu Tyr Val Asp Val Glu Lys Asn Leu Ser Lys Ile Arg Ala Val Ala
   1285 1290 1295
   Asp Ser Met Asp Asn Phe Ser Ile Glu Glu Ile Ser Asn Ser Phe Ile
   1300 1305 1310
   Asn Leu Leu Thr Leu Thr Ala Leu Gly Ala Pro Ala Asp Phe Asn Phe
   1315 1320 1325
   Leu Gly Glu Lys Ile Pro Arg Lys Arg Tyr Thr Ser Thr Lys Glu Cys
   1330 1335 1340
   Leu Thr Ala Thr Leu Ile His Gln Ser Ile Thr Gly Leu Tyr Glu Thr
   1345 1350 1355 1360
   Arg Ile Asp Leu Ser Lys Leu Gly Glu Glu
   1365 1370
<210> 79
   <211> 1035
   <212> PRT
   <213> Candidatus Puniceispirillum marinum
<220>
   <223> CRISPR-associated endonuclease
<308> YP_003552871
   <309> 2013-06-10
<400> 79
   Met Arg Arg Leu Gly Leu Asp Leu Gly Thr Asn Ser Ile Gly Trp Cys
   1               5                   10                  15
   Leu Leu Asp Leu Gly Asp Asp Gly Glu Pro Val Ser Ile Phe Arg Thr
               20                  25                  30
   Gly Ala Arg Ile Phe Ser Asp Gly Arg Asp Pro Lys Ser Leu Gly Ser
           35                  40                  45
   Leu Lys Ala Thr Arg Arg Glu Ala Arg Leu Thr Arg Arg Arg Arg Asp
       50                  55                  60
   Arg Phe Ile Gln Arg Gln Lys Asn Leu Ile Asn Ala Leu Val Lys Tyr
   65                  70                  75                  80
   Gly Leu Met Pro Ala Asp Glu Ile Gln Arg Gln Ala Leu Ala Tyr Lys
                   85                  90                  95
   Asp Pro Tyr Pro Ile Arg Lys Lys Ala Leu Asp Glu Ala Ile Asp Pro
               100                 105                 110
   Tyr Glu Met Gly Arg Ala Ile Phe His Ile Asn Gln Arg Arg Gly Phe
           115                 120                 125
   Lys Ser Asn Arg Lys Ser Ala Asp Asn Glu Ala Gly Val Val Lys Gln
       130                 135                 140
   Ser Ile Ala Asp Leu Glu Met Lys Leu Gly Glu Ala Gly Ala Arg Thr
   145                 150                 155                 160
   Ile Gly Glu Phe Leu Ala Asp Arg Gln Ala Thr Asn Asp Thr Val Arg
                   165                 170                 175
   Ala Arg Arg Leu Ser Gly Thr Asn Ala Leu Tyr Glu Phe Tyr Pro Asp
               180                 185                 190
   Arg Tyr Met Leu Glu Gln Glu Phe Asp Thr Leu Trp Ala Lys Gln Ala
           195                 200                 205
   Ala Phe Asn Pro Ser Leu Tyr Ile Glu Ala Ala Arg Glu Arg Leu Lys
       210                 215                 220
   Glu Ile Val Phe Phe Gln Arg Lys Leu Lys Pro Gln Glu Val Gly Arg
   225                 230                 235                 240
   Cys Ile Phe Leu Ser Asp Glu Asp Arg Ile Ser Lys Ala Leu Pro Ser
                   245                 250                 255
   Phe Gln Arg Phe Arg Ile Tyr Gln Glu Leu Ser Asn Leu Ala Trp Ile
               260                 265                 270
   Asp His Asp Gly Val Ala His Arg Ile Thr Ala Ser Leu Ala Leu Arg
           275                 280                 285
   Asp His Leu Phe Asp Glu Leu Glu His Lys Lys Lys Leu Thr Phe Lys
       290                 295                 300
   Ala Met Arg Ala Ile Leu Arg Lys Gln Gly Val Val Asp Tyr Pro Val
   305                 310                 315                 320
   Gly Phe Asn Leu Glu Ser Asp Asn Arg Asp His Leu Ile Gly Asn Leu
                   325                 330                 335
   Thr Ser Cys Ile Met Arg Asp Ala Lys Lys Met Ile Gly Ser Ala Trp
               340                 345                 350
   Asp Arg Leu Asp Glu Glu Glu Gln Asp Ser Phe Ile Leu Met Leu Gln
           355                 360                 365
   Asp Asp Gln Lys Gly Asp Asp Glu Val Arg Ser Ile Leu Thr Gln Gln
       370                 375                 380
   Tyr Gly Leu Ser Asp Asp Val Ala Glu Asp Cys Leu Asp Val Arg Leu
   385                 390                 395                 400
   Pro Asp Gly His Gly Ser Leu Ser Lys Lys Ala Ile Asp Arg Ile Leu
                   405                 410                 415
   Pro Val Leu Arg Asp Gln Gly Leu Ile Tyr Tyr Asp Ala Val Lys Glu
               420                 425                 430
   Ala Gly Leu Gly Glu Ala Asn Leu Tyr Asp Pro Tyr Ala Ala Leu Ser
           435                 440                 445
   Asp Lys Leu Asp Tyr Tyr Gly Lys Ala Leu Ala Gly His Val Met Gly
       450                 455                 460
   Ala Ser Gly Lys Phe Glu Asp Ser Asp Glu Lys Arg Tyr Gly Thr Ile
   465                 470                 475                 480
   Ser Asn Pro Thr Val His Ile Ala Leu Asn Gln Val Arg Ala Val Val
                   485                 490                 495
   Asn Glu Leu Ile Arg Leu His Gly Lys Pro Asp Glu Val Val Ile Glu
               500                 505                 510
   Ile Gly Arg Asp Leu Pro Met Gly Ala Asp Gly Lys Arg Glu Leu Glu
           515                 520                 525
   Arg Phe Gln Lys Glu Gly Arg Ala Lys Asn Glu Arg Ala Arg Asp Glu
       530                 535                 540
   Leu Lys Lys Leu Gly His Ile Asp Ser Arg Glu Ser Arg Gln Lys Phe
   545                 550                 555                 560
   Gln Leu Trp Glu Gln Leu Ala Lys Glu Pro Val Asp Arg Cys Cys Pro
                   565                 570                 575
   Phe Thr Gly Lys Met Met Ser Ile Ser Asp Leu Phe Ser Asp Lys Val
               580                 585                 590
   Glu Ile Glu His Leu Leu Pro Phe Ser Leu Thr Leu Asp Asp Ser Met
           595                 600                 605
   Ala Asn Lys Thr Val Cys Phe Arg Gln Ala Asn Arg Asp Lys Gly Asn
       610                 615                 620
   Arg Ala Pro Phe Asp Ala Phe Gly Asn Ser Pro Ala Gly Tyr Asp Trp
   625                 630                 635                 640
   Gln Glu Ile Leu Gly Arg Ser Gln Asn Leu Pro Tyr Ala Lys Arg Trp
                   645                 650                 655
   Arg Phe Leu Pro Asp Ala Met Lys Arg Phe Glu Ala Asp Gly Gly Phe
               660                 665                 670
   Leu Glu Arg Gln Leu Asn Asp Thr Arg Tyr Ile Ser Arg Tyr Thr Thr
           675                 680                 685
   Glu Tyr Ile Ser Thr Ile Ile Pro Lys Asn Lys Ile Trp Val Val Thr
       690                 695                 700
   Gly Arg Leu Thr Ser Leu Leu Arg Gly Phe Trp Gly Leu Asn Ser Ile
   705                 710                 715                 720
   Leu Arg Gly His Asn Thr Asp Asp Gly Thr Pro Ala Lys Lys Ser Arg
                   725                 730                 735
   Asp Asp His Arg His His Ala Ile Asp Ala Ile Val Val Gly Met Thr
               740                 745                 750
   Ser Arg Gly Leu Leu Gln Lys Val Ser Lys Ala Ala Arg Arg Ser Glu
           755                 760                 765
   Asp Leu Asp Leu Thr Arg Leu Phe Glu Gly Arg Ile Asp Pro Trp Asp
       770                 775                 780
   Gly Phe Arg Asp Glu Val Lys Lys His Ile Asp Ala Ile Ile Val Ser
   785                 790                 795                 800
   His Arg Pro Arg Lys Lys Ser Gln Gly Ala Leu His Asn Asp Thr Ala
                   805                 810                 815
   Tyr Gly Ile Val Glu His Ala Glu Asn Gly Ala Ser Thr Val Val His
               820                 825                 830
   Arg Val Pro Ile Thr Ser Leu Gly Lys Gln Ser Asp Ile Glu Lys Val
           835                 840                 845
   Arg Asp Pro Leu Ile Lys Ser Ala Leu Leu Asn Glu Thr Ala Gly Leu
       850                 855                 860
   Ser Gly Lys Ser Phe Glu Asn Ala Val Gln Lys Trp Cys Ala Asp Asn
   865                 870                 875                 880
   Ser Ile Lys Ser Leu Arg Ile Val Glu Thr Val Ser Ile Ile Pro Ile
                   885                 890                 895
   Thr Asp Lys Glu Gly Val Ala Tyr Lys Gly Tyr Lys Gly Asp Gly Asn
               900                 905                 910
   Ala Tyr Met Asp Ile Tyr Gln Asp Pro Thr Ser Ser Lys Trp Lys Gly
           915                 920                 925
   Glu Ile Val Ser Arg Phe Asp Ala Asn Gln Lys Gly Phe Ile Pro Ser
       930                 935                 940
   Trp Gln Ser Gln Phe Pro Thr Ala Arg Leu Ile Met Arg Leu Arg Ile
   945                 950                 955                 960
   Asn Asp Leu Leu Lys Leu Gln Asp Gly Glu Ile Glu Glu Ile Tyr Arg
                   965                 970                 975
   Val Gln Arg Leu Ser Gly Ser Lys Ile Leu Met Ala Pro His Thr Glu
               980                 985                 990
   Ala Asn Val Asp Ala Arg Asp Arg Asp Lys Asn Asp Thr Phe Lys Leu
           995                 1000                1005
   Thr Ser Lys Ser Pro Gly Lys Leu Gln Ser Ala Ser Ala Arg Lys Val
       1010                1015                1020
   His Ile Ser Pro Thr Gly Leu Ile Arg Glu Gly
   1025                1030                1035
<210> 80
   <211> 1395
   <212> PRT
   <213> Treponema denticola
<220>
   <223> CRISPR-associated endonuclease
<308> NP_970941
   <309> 2013-06-10
<400> 80
   Met Lys Lys Glu Ile Lys Asp Tyr Phe Leu Gly Leu Asp Val Gly Thr
   1 5 10 15
   Gly Ser Val Gly Trp Ala Val Thr Asp Thr Asp Tyr Lys Leu Leu Lys
   20 25 30
   Ala Asn Arg Lys Asp Leu Trp Gly Met Arg Cys Phe Glu Thr Ala Glu
   35 40 45
   Thr Ala Glu Val Arg Arg Leu His Arg Gly Ala Arg Arg Arg Ile Glu
   50 55 60
   Arg Arg Lys Lys Arg Ile Lys Leu Leu Gln Glu Leu Phe Ser Gln Glu
   65 70 75 80
   Ile Ala Lys Thr Asp Glu Gly Phe Phe Gln Arg Met Lys Glu Ser Pro
   85 90 95
   Phe Tyr Ala Glu Asp Lys Thr Ile Leu Gln Glu Asn Thr Leu Phe Asn
   100 105 110
   Asp Lys Asp Phe Ala Asp Lys Thr Tyr His Lys Ala Tyr Pro Thr Ile
   115 120 125
   Asn His Leu Ile Lys Ala Trp Ile Glu Asn Lys Val Lys Pro Asp Pro
   130 135 140
   Arg Leu Leu Tyr Leu Ala Cys His Asn Ile Ile Lys Lys Arg Gly His
   145 150 155 160
   Phe Leu Phe Glu Gly Asp Phe Asp Ser Glu Asn Gln Phe Asp Thr Ser
   165 170 175
   Ile Gln Ala Leu Phe Glu Tyr Leu Arg Glu Asp Met Glu Val Asp Ile
   180 185 190
   Asp Ala Asp Ser Gln Lys Val Lys Glu Ile Leu Lys Asp Ser Ser Leu
   195 200 205
   Lys Asn Ser Glu Lys Gln Ser Arg Leu Asn Lys Ile Leu Gly Leu Lys
   210 215 220
   Pro Ser Asp Lys Gln Lys Lys Ala Ile Thr Asn Leu Ile Ser Gly Asn
   225 230 235 240
   Lys Ile Asn Phe Ala Asp Leu Tyr Asp Asn Pro Asp Leu Lys Asp Ala
   245 250 255
   Glu Lys Asn Ser Ile Ser Phe Ser Lys Asp Asp Phe Asp Ala Leu Ser
   260 265 270
   Asp Asp Leu Ala Ser Ile Leu Gly Asp Ser Phe Glu Leu Leu Leu Lys
   275 280 285
   Ala Lys Ala Val Tyr Asn Cys Ser Val Leu Ser Lys Val Ile Gly Asp
   290 295 300
   Glu Gln Tyr Leu Ser Phe Ala Lys Val Lys Ile Tyr Glu Lys His Lys
   305                 310                 315                 320
   Thr Asp Leu Thr Lys Leu Lys Asn Val Ile Lys Lys His Phe Pro Lys
                   325                 330                 335
   Asp Tyr Lys Lys Val Phe Gly Tyr Asn Lys Asn Glu Lys Asn Asn Asn
               340                 345                 350
   Asn Tyr Ser Gly Tyr Val Gly Val Cys Lys Thr Lys Ser Lys Lys Leu
           355                 360                 365
   Ile Ile Asn Asn Ser Val Asn Gln Glu Asp Phe Tyr Lys Phe Leu Lys
       370                 375                 380
   Thr Ile Leu Ser Ala Lys Ser Glu Ile Lys Glu Val Asn Asp Ile Leu
   385                 390                 395                 400
   Thr Glu Ile Glu Thr Gly Thr Phe Leu Pro Lys Gln Ile Ser Lys Ser
                   405                 410                 415
   Asn Ala Glu Ile Pro Tyr Gln Leu Arg Lys Met Glu Leu Glu Lys Ile
               420                 425                 430
   Leu Ser Asn Ala Glu Lys His Phe Ser Phe Leu Lys Gln Lys Asp Glu
           435                 440                 445
   Lys Gly Leu Ser His Ser Glu Lys Ile Ile Met Leu Leu Thr Phe Lys
       450                 455                 460
   Ile Pro Tyr Tyr Ile Gly Pro Ile Asn Asp Asn His Lys Lys Phe Phe
   465                 470                 475                 480
   Pro Asp Arg Cys Trp Val Val Lys Lys Glu Lys Ser Pro Ser Gly Lys
                   485                 490                 495
   Thr Thr Pro Trp Asn Phe Phe Asp His Ile Asp Lys Glu Lys Thr Ala
               500                 505                 510
   Glu Ala Phe Ile Thr Ser Arg Thr Asn Phe Cys Thr Tyr Leu Val Gly
           515                 520                 525
   Glu Ser Val Leu Pro Lys Ser Ser Leu Leu Tyr Ser Glu Tyr Thr Val
       530                 535                 540
   Leu Asn Glu Ile Asn Asn Leu Gln Ile Ile Ile Asp Gly Lys Asn Ile
   545                 550                 555                 560
   Cys Asp Ile Lys Leu Lys Gln Lys Ile Tyr Glu Asp Leu Phe Lys Lys
                   565                 570                 575
   Tyr Lys Lys Ile Thr Gln Lys Gln Ile Ser Thr Phe Ile Lys His Glu
               580                 585                 590
   Gly Ile Cys Asn Lys Thr Asp Glu Val Ile Ile Leu Gly Ile Asp Lys
           595                 600                 605
   Glu Cys Thr Ser Ser Leu Lys Ser Tyr Ile Glu Leu Lys Asn Ile Phe
       610                 615                 620
   Gly Lys Gln Val Asp Glu Ile Ser Thr Lys Asn Met Leu Glu Glu Ile
   625                 630                 635                 640
   Ile Arg Trp Ala Thr Ile Tyr Asp Glu Gly Glu Gly Lys Thr Ile Leu
                   645                 650                 655
   Lys Thr Lys Ile Lys Ala Glu Tyr Gly Lys Tyr Cys Ser Asp Glu Gln
               660                 665                 670
   Ile Lys Lys Ile Leu Asn Leu Lys Phe Ser Gly Trp Gly Arg Leu Ser
           675                 680                 685
   Arg Lys Phe Leu Glu Thr Val Thr Ser Glu Met Pro Gly Phe Ser Glu
       690                 695                 700
   Pro Val Asn Ile Ile Thr Ala Met Arg Glu Thr Gln Asn Asn Leu Met
   705                 710                 715                 720
   Glu Leu Leu Ser Ser Glu Phe Thr Phe Thr Glu Asn Ile Lys Lys Ile
                   725                 730                 735
   Asn Ser Gly Phe Glu Asp Ala Glu Lys Gln Phe Ser Tyr Asp Gly Leu
               740                 745                 750
   Val Lys Pro Leu Phe Leu Ser Pro Ser Val Lys Lys Met Leu Trp Gln
           755                 760                 765
   Thr Leu Lys Leu Val Lys Glu Ile Ser His Ile Thr Gln Ala Pro Pro
       770                 775                 780
   Lys Lys Ile Phe Ile Glu Met Ala Lys Gly Ala Glu Leu Glu Pro Ala
   785                 790                 795                 800
   Arg Thr Lys Thr Arg Leu Lys Ile Leu Gln Asp Leu Tyr Asn Asn Cys
                   805                 810                 815
   Lys Asn Asp Ala Asp Ala Phe Ser Ser Glu Ile Lys Asp Leu Ser Gly
               820                 825                 830
   Lys Ile Glu Asn Glu Asp Asn Leu Arg Leu Arg Ser Asp Lys Leu Tyr
           835                 840                 845
   Leu Tyr Tyr Thr Gln Leu Gly Lys Cys Met Tyr Cys Gly Lys Pro Ile
       850                 855                 860
   Glu Ile Gly His Val Phe Asp Thr Ser Asn Tyr Asp Ile Asp His Ile
   865                 870                 875                 880
   Tyr Pro Gln Ser Lys Ile Lys Asp Asp Ser Ile Ser Asn Arg Val Leu
                   885                 890                 895
   Val Cys Ser Ser Cys Asn Lys Asn Lys Glu Asp Lys Tyr Pro Leu Lys
               900                 905                 910
   Ser Glu Ile Gln Ser Lys Gln Arg Gly Phe Trp Asn Phe Leu Gln Arg
           915                 920                 925
   Asn Asn Phe Ile Ser Leu Glu Lys Leu Asn Arg Leu Thr Arg Ala Thr
       930                 935                 940
   Pro Ile Ser Asp Asp Glu Thr Ala Lys Phe Ile Ala Arg Gln Leu Val
   945                 950                 955                 960
   Glu Thr Arg Gln Ala Thr Lys Val Ala Ala Lys Val Leu Glu Lys Met
                   965                 970                 975
   Phe Pro Glu Thr Lys Ile Val Tyr Ser Lys Ala Glu Thr Val Ser Met
               980                 985                 990
   Phe Arg Asn Lys Phe Asp Ile Val Lys Cys Arg Glu Ile Asn Asp Phe
           995                 1000                1005
   His His Ala His Asp Ala Tyr Leu Asn Ile Val Val Gly Asn Val Tyr
       1010                1015                1020
   Asn Thr Lys Phe Thr Asn Asn Pro Trp Asn Phe Ile Lys Glu Lys Arg
   1025                1030                1035                1040
   Asp Asn Pro Lys Ile Ala Asp Thr Tyr Asn Tyr Tyr Lys Val Phe Asp
                   1045                1050                1055
   Tyr Asp Val Lys Arg Asn Asn Ile Thr Ala Trp Glu Lys Gly Lys Thr
               1060                1065                1070
   Ile Ile Thr Val Lys Asp Met Leu Lys Arg Asn Thr Pro Ile Tyr Thr
           1075                1080                1085
   Arg Gln Ala Ala Cys Lys Lys Gly Glu Leu Phe Asn Gln Thr Ile Met
       1090                1095                1100
   Lys Lys Gly Leu Gly Gln His Pro Leu Lys Lys Glu Gly Pro Phe Ser
   1105                1110                1115                1120
   Asn Ile Ser Lys Tyr Gly Gly Tyr Asn Lys Val Ser Ala Ala Tyr Tyr
                   1125                1130                1135
   Thr Leu Ile Glu Tyr Glu Glu Lys Gly Asn Lys Ile Arg Ser Leu Glu
               1140                1145                1150
   Thr Ile Pro Leu Tyr Leu Val Lys Asp Ile Gln Lys Asp Gln Asp Val
           1155                1160                1165
   Leu Lys Ser Tyr Leu Thr Asp Leu Leu Gly Lys Lys Glu Phe Lys Ile
       1170                1175                1180
   Leu Val Pro Lys Ile Lys Ile Asn Ser Leu Leu Lys Ile Asn Gly Phe
   1185                1190                1195                1200
   Pro Cys His Ile Thr Gly Lys Thr Asn Asp Ser Phe Leu Leu Arg Pro
                   1205                1210                1215
   Ala Val Gln Phe Cys Cys Ser Asn Asn Glu Val Leu Tyr Phe Lys Lys
               1220                1225                1230
   Ile Ile Arg Phe Ser Glu Ile Arg Ser Gln Arg Glu Lys Ile Gly Lys
           1235                1240                1245
   Thr Ile Ser Pro Tyr Glu Asp Leu Ser Phe Arg Ser Tyr Ile Lys Glu
       1250                1255                1260
   Asn Leu Trp Lys Lys Thr Lys Asn Asp Glu Ile Gly Glu Lys Glu Phe
   1265                1270                1275                1280
   Tyr Asp Leu Leu Gln Lys Lys Asn Leu Glu Ile Tyr Asp Met Leu Leu
                   1285                1290                1295
   Thr Lys His Lys Asp Thr Ile Tyr Lys Lys Arg Pro Asn Ser Ala Thr
               1300                1305                1310
   Ile Asp Ile Leu Val Lys Gly Lys Glu Lys Phe Lys Ser Leu Ile Ile
           1315                1320                1325
   Glu Asn Gln Phe Glu Val Ile Leu Glu Ile Leu Lys Leu Phe Ser Ala
       1330                1335                1340
   Thr Arg Asn Val Ser Asp Leu Gln His Ile Gly Gly Ser Lys Tyr Ser
   1345                1350                1355                1360
   Gly Val Ala Lys Ile Gly Asn Lys Ile Ser Ser Leu Asp Asn Cys Ile
                   1365                1370                1375
   Leu Ile Tyr Gln Ser Ile Thr Gly Ile Phe Glu Lys Arg Ile Asp Leu
               1380                1385                1390
   Leu Lys Val
           1395
<210> 81
   <211> 1130
   <212> PRT
   <213> Streptococcus pasteurianus
<220>
   <223> CRISPR-associated endonuclease
<308> YP_004559470
   <309> 2013-07-06
<400> 81
   Met Thr Asn Gly Lys Ile Leu Gly Leu Asp Ile Gly Ile Ala Ser Val
    1               5                  10                  15
   Gly Val Gly Ile Ile Glu Ala Lys Thr Gly Lys Val Val His Ala Asn
               20                  25                  30
   Ser Arg Leu Phe Ser Ala Ala Asn Ala Glu Asn Asn Ala Glu Arg Arg
           35                  40                  45
   Gly Phe Arg Gly Ser Arg Arg Leu Asn Arg Arg Lys Lys His Arg Val
       50                  55                  60
   Lys Arg Val Arg Asp Leu Phe Glu Lys Tyr Gly Ile Val Thr Asp Phe
   65                  70                  75                  80
   Arg Asn Leu Asn Leu Asn Pro Tyr Glu Leu Arg Val Lys Gly Leu Thr
                   85                  90                  95
   Glu Gln Leu Lys Asn Glu Glu Leu Phe Ala Ala Leu Arg Thr Ile Ser
               100                 105                 110
   Lys Arg Arg Gly Ile Ser Tyr Leu Asp Asp Ala Glu Asp Asp Ser Thr
           115                 120                 125
   Gly Ser Thr Asp Tyr Ala Lys Ser Ile Asp Glu Asn Arg Arg Leu Leu
       130                 135                 140
   Lys Asn Lys Thr Pro Gly Gln Ile Gln Leu Glu Arg Leu Glu Lys Tyr
   145                 150                 155                 160
   Gly Gln Leu Arg Gly Asn Phe Thr Val Tyr Asp Glu Asn Gly Glu Ala
                   165                 170                 175
   His Arg Leu Ile Asn Val Phe Ser Thr Ser Asp Tyr Glu Lys Glu Ala
               180                 185                 190
   Arg Lys Ile Leu Glu Thr Gln Ala Asp Tyr Asn Lys Lys Ile Thr Ala
           195                 200                 205
   Glu Phe Ile Asp Asp Tyr Val Glu Ile Leu Thr Gln Lys Arg Lys Tyr
       210                 215                 220
   Tyr His Gly Pro Gly Asn Glu Lys Ser Arg Thr Asp Tyr Gly Arg Phe
   225                 230                 235                 240
   Arg Thr Asp Gly Thr Thr Leu Glu Asn Ile Phe Gly Ile Leu Ile Gly
                   245                 250                 255
   Lys Cys Asn Phe Tyr Pro Asp Glu Tyr Arg Ala Ser Lys Ala Ser Tyr
               260                 265                 270
   Thr Ala Gln Glu Tyr Asn Phe Leu Asn Asp Leu Asn Asn Leu Lys Val
           275                 280                 285
   Ser Thr Glu Thr Gly Lys Leu Ser Thr Glu Gln Lys Glu Ser Leu Val
   290 295 300
   Glu Phe Ala Lys Asn Thr Ala Thr Leu Gly Pro Ala Lys Leu Leu Lys
   305 310 315 320
   Glu Ile Ala Lys Ile Leu Asp Cys Lys Val Asp Glu Ile Lys Gly Tyr
   325 330 335
   Arg Glu Asp Asp Lys Gly Lys Pro Asp Leu His Thr Phe Glu Pro Tyr
   340 345 350
   Arg Lys Leu Lys Phe Asn Leu Glu Ser Ile Asn Ile Asp Asp Leu Ser
   355 360 365
   Arg Glu Val Ile Asp Lys Leu Ala Asp Ile Leu Thr Leu Asn Thr Glu
   370 375 380
   Arg Glu Gly Ile Glu Asp Ala Ile Lys Arg Asn Leu Pro Asn Gln Phe
   385 390 395 400
   Thr Glu Glu Gln Ile Ser Glu Ile Ile Lys Val Arg Lys Ser Gln Ser
   405 410 415
   Thr Ala Phe Asn Lys Gly Trp His Ser Phe Ser Ala Lys Leu Met Asn
   420 425 430
   Glu Leu Ile Pro Glu Leu Tyr Ala Thr Ser Asp Glu Gln Met Thr Ile
   435 440 445
   Leu Thr Arg Leu Glu Lys Phe Lys Val Asn Lys Lys Ser Ser Lys Asn
   450 455 460
   Thr Lys Thr Ile Asp Glu Lys Glu Val Thr Asp Glu Ile Tyr Asn Pro
   465 470 475 480
   Val Val Ala Lys Ser Val Arg Gln Thr Ile Lys Ile Ile Asn Ala Ala
   485 490 495
   Val Lys Lys Tyr Gly Asp Phe Asp Lys Ile Val Ile Glu Met Pro Arg
   500 505 510
   Asp Lys Asn Ala Asp Asp Glu Lys Lys Phe Ile Asp Lys Arg Asn Lys
   515 520 525
   Glu Asn Lys Lys Glu Lys Asp Asp Ala Leu Lys Arg Ala Ala Tyr Leu
   530 535 540
   Tyr Asn Ser Ser Asp Lys Leu Pro Asp Glu Val Phe His Gly Asn Lys
   545 550 555 560
   Gln Leu Glu Thr Lys Ile Arg Leu Trp Tyr Gln Gln Gly Glu Arg Cys
   565 570 575
   Leu Tyr Ser Gly Lys Pro Ile Ser Ile Gln Glu Leu Val His Asn Ser
   580 585 590
   Asn Asn Phe Glu Ile Asp His Ile Leu Pro Leu Ser Leu Ser Phe Asp
   595 600 605
   Asp Ser Leu Ala Asn Lys Val Leu Val Tyr Ala Trp Thr Asn Gln Glu
   610 615 620
   Lys Gly Gln Lys Thr Pro Tyr Gln Val Ile Asp Ser Met Asp Ala Ala
   625 630 635 640
   Trp Ser Phe Arg Glu Met Lys Asp Tyr Val Leu Lys Gln Lys Gly Leu
   645 650 655
   Gly Lys Lys Lys Arg Asp Tyr Leu Leu Thr Thr Glu Asn Ile Asp Lys
   660 665 670
   Ile Glu Val Lys Lys Lys Phe Ile Glu Arg Asn Leu Val Asp Thr Arg
   675 680 685
   Tyr Ala Ser Arg Val Val Leu Asn Ser Leu Gln Ser Ala Leu Arg Glu
   690 695 700
   Leu Gly Lys Asp Thr Lys Val Ser Val Val Arg Gly Gln Phe Thr Ser
   705 710 715 720
   Gln Leu Arg Arg Lys Trp Lys Ile Asp Lys Ser Arg Glu Thr Tyr His
   725 730 735
   His His Ala Val Asp Ala Leu Ile Ile Ala Ala Ser Ser Gln Leu Lys
   740 745 750
   Leu Trp Glu Lys Gln Asp Asn Pro Met Phe Val Asp Tyr Gly Lys Asn
   755 760 765
   Gln Val Val Asp Lys Gln Thr Gly Glu Ile Leu Ser Val Ser Asp Asp
   770 775 780
   Glu Tyr Lys Glu Leu Val Phe Gln Pro Pro Tyr Gln Gly Phe Val Asn
   785 790 795 800
   Thr Ile Ser Ser Lys Gly Phe Glu Asp Glu Ile Leu Phe Ser Tyr Gln
   805 810 815
   Val Asp Ser Lys Tyr Asn Arg Lys Val Ser Asp Ala Thr Ile Tyr Ser
   820 825 830
   Thr Arg Lys Ala Lys Ile Gly Lys Asp Lys Lys Glu Glu Thr Tyr Val
   835 840 845
   Leu Gly Lys Ile Lys Asp Ile Tyr Ser Gln Asn Gly Phe Asp Thr Phe
   850 855 860
   Ile Lys Lys Tyr Asn Lys Asp Lys Thr Gln Phe Leu Met Tyr Gln Lys
   865 870 875 880
   Asp Ser Leu Thr Trp Glu Asn Val Ile Glu Val Ile Leu Arg Asp Tyr
   885 890 895
   Pro Thr Thr Lys Lys Ser Glu Asp Gly Lys Asn Asp Val Lys Cys Asn
   900 905 910
   Pro Phe Glu Glu Tyr Arg Arg Glu Asn Gly Leu Ile Cys Lys Tyr Ser
   915 920 925
   Lys Lys Gly Lys Gly Thr Pro Ile Lys Ser Leu Lys Tyr Tyr Asp Lys
   930 935 940
   Lys Leu Gly Asn Cys Ile Asp Ile Thr Pro Glu Glu Ser Arg Asn Lys
   945 950 955 960
   Val Ile Leu Gln Ser Ile Asn Pro Trp Arg Ala Asp Val Tyr Phe Asn
   965 970 975
   Pro Glu Thr Leu Lys Tyr Glu Leu Met Gly Leu Lys Tyr Ser Asp Leu
   980 985 990
   Ser Phe Glu Lys Gly Thr Gly Asn Tyr His Ile Ser Gln Glu Lys Tyr
   995 1000 1005
   Asp Ala Ile Lys Glu Lys Glu Gly Ile Gly Lys Lys Ser Glu Phe Lys
   1010 1015 1020
   Phe Thr Leu Tyr Arg Asn Asp Leu Ile Leu Ile Lys Asp Ile Ala Ser
   1025 1030 1035 1040
   Gly Glu Gln Glu Ile Tyr Arg Phe Leu Ser Arg Thr Met Pro Asn Val
   1045 1050 1055
   Asn His Tyr Val Glu Leu Lys Pro Tyr Asp Lys Glu Lys Phe Asp Asn
   1060 1065 1070
   Val Gln Glu Leu Val Glu Ala Leu Gly Glu Ala Asp Lys Val Gly Arg
   1075 1080 1085
   Cys Ile Lys Gly Leu Asn Lys Pro Asn Ile Ser Ile Tyr Lys Val Arg
   1090 1095 1100
   Thr Asp Val Leu Gly Asn Lys Tyr Phe Val Lys Lys Lys Gly Asp Lys
   1105 1110 1115 1120
   Pro Lys Leu Asp Phe Lys Asn Asn Lys Lys
   1125 1130
<210> 82
   <211> 897
   <212> PRT
   <213> Corynebacterium ulcerans
<220>
   <223> CRISPR-associated endonuclease
<308> YP_004628671
   <309> 2013-07-06
<400> 82
   Met Thr Asn Ala Val Ala Asn His His Val Leu Trp Ala Lys Phe Asp
    1               5                  10                  15
   Asn Val Ser Glu Pro Tyr Pro Leu Leu Ala His Leu Leu Asp Thr Ala
               20                  25                  30
   Thr Ala Ala Thr Cys Leu Phe Asn His Trp Leu Arg Lys Gly Leu Arg
           35                  40                  45
   Asp Arg Leu Ser Thr Glu Leu Gly Pro Asp Ala Glu Lys Ile Leu Gly
           50                  55                  60
   Phe Val Ala Gly Ile His Asp Leu Gly Lys Ala Asn Pro Tyr Phe Gln
   65                  70                  75                  80
   Ala Gln Arg Arg Asn Lys Lys Glu Glu Trp Ile Thr Leu Arg Asp Ala
                   85                  90                  95
   Ile Gln Lys Ala Gly Phe Pro Leu Ser Asn Gly Thr Ser Ala Leu Phe
               100                 105                 110
   Glu Glu Thr Lys Glu Lys Arg Arg His Glu Asn Ile Thr Leu Ser Ile
           115                 120                 125
   Leu Gly Trp Glu Ile Thr Lys Phe Leu Gln Val Lys Asp Val Trp Pro
       130                 135                 140
   Gln Leu Ala Ile Ile Gly His His Gly Asn Phe Ser Ala Pro Gly Phe
   145                 150                 155                 160
   Leu Ser Asp Glu Asp Asp Leu Glu Asp Ile Glu Asp Ile Phe Asp Asp
                   165                 170                 175
   Asn Gly Trp Ser Pro Thr His Glu Leu Leu Val Ser Ser Leu Leu Gln
               180                 185                 190
   Ala Val Gly Leu Glu Lys Gln Pro Glu Ile Lys His Ile Ser Pro Ala
           195                 200                 205
   Ser Ala Ile Leu Ile Ser Gly Leu Val Val Leu Ala Asp Arg Ile Ala
       210                 215                 220
   Ser Gln Ser Glu Met Ala Ser Asp Gly Leu Gln Ala Leu Gln Lys Glu
   225                 230                 235                 240
   Glu Leu Phe Phe His Gln Pro Glu Lys Trp Ile Ala Asn Arg Lys Ala
                   245                 250                 255
   Phe Cys Arg Glu Ile Ile Glu Asn Thr Val Gly Thr Tyr His Pro Trp
               260                 265                 270
   Glu Ser Glu Ala Ala Gly Ile Arg Ala Val Leu Gly Asp Tyr Glu Pro
           275                 280                 285
   Arg Phe Thr Gln Lys Ala Ala Leu Asn Ala Gly Asp Gly Leu Phe Asn
       290                 295                 300
   Val Met Glu Thr Thr Gly Ala Gly Lys Thr Glu Ala Ala Leu Leu Arg
   305                 310                 315                 320
   His Val Lys Arg Lys Glu Arg Leu Leu Phe Phe Leu Pro Thr Gln Ala
                   325                 330                 335
   Thr Thr Asn Ala Ile Met Asp Arg Ile Gly Lys Ile Phe Asp Gly Thr
               340                 345                 350
   Pro Asn Val Ala Ser Leu Ala His Gly Leu Ala Val Thr Glu Asp Phe
           355                 360                 365
   Tyr Ala His Pro Ile Leu Pro Val Gln Gly Ser Ser Asp Asp Ala Asn
       370                 375                 380
   Tyr Lys Asp Asn Gly Gly Leu Tyr Pro Thr Glu Phe Val Arg Ser Ala
   385                 390                 395                 400
   Gly Thr Pro Arg Leu Leu Ala Pro Val Cys Val Gly Thr Ile Asp Gln
                   405                 410                 415
   Ala Leu Met Gly Ala Leu Pro Ser Lys Phe Asn His Leu Arg Leu Leu
               420                 425                 430
   Ala Leu Ala Asn Ala His Val Val Val Asp Glu Val His Thr Met Asp
           435                 440                 445
   Gln Tyr Gln Ser Glu Leu Met Ser Gly Leu Leu Glu Trp Trp Ser Ala
       450                 455                 460
   Thr Asp Thr Pro Val Thr Leu Leu Thr Ala Thr Met Pro Ala Trp Gln
   465                 470                 475                 480
   Arg Glu Lys Phe His Leu Ser Tyr Thr Gly Lys Asp Pro His Phe Lys
                   485                 490                 495
   Gly Val Phe Pro Ser Leu Glu Asp Trp Ser Thr Pro Ser Lys Asn Thr
               500                 505                 510
   Glu Thr Ser Gln Glu Asn Ile Pro Thr Glu Ala Phe Thr Ile Pro Ile
           515                 520                 525
   Asn Ile Asp Lys Ile Ala His Asn Glu Ile Val Asp Ser His Val Gln
       530                 535                 540
   Trp Val Ile Glu Gln Arg Lys Leu Phe Pro Gln Ala Arg Ile Gly Ile
   545                 550                 555                 560
   Ile Cys Asn Thr Val Gly Arg Ala Gln Ser Ile Ala Glu Ala Leu Ala
                   565                 570                 575
   His Glu Ser Pro Ile Val Leu His Ser Arg Met Thr Ala Gly His Arg
               580                 585                 590
   Lys Glu Ala Ala Thr Lys Leu Glu Gln Ala Ile Gly Lys Lys Gly Thr
           595                 600                 605
   Ala Asn Ala Thr Leu Val Ile Gly Thr Gln Ala Ile Glu Ala Ser Leu
       610                 615                 620
   Asp Ile Asp Leu Asp Leu Leu Arg Thr Glu Leu Cys Pro Ala Pro Ser
   625                 630                 635                 640
   Leu Ile Gln Arg Ala Gly Arg Leu Trp Arg Arg Leu Asp Pro Gln Arg
                   645                 650                 655
   Glu Val Arg Val Pro Gly Met Val Gly Lys Lys Leu Thr Ile Ala Val
               660                 665                 670
   Val Asp Ser Pro Ser Thr Gly Gln Thr Leu Pro Tyr Leu Arg Ser Gln
           675                 680                 685
   Leu Tyr Arg Val Glu Ser Trp Leu Lys Gln Arg Asp Arg Ile Glu Phe
       690                 695                 700
   Pro Ala Asp Ile Gln Asp Phe Ile Asp Ala Thr Thr Pro Gly Leu Gln
   705                 710                 715                 720
   Glu Leu Phe Gln Lys Val Ser Leu Pro Glu Asp Cys Gly Ser Ala Glu
                   725                 730                 735
   Glu Arg Glu Ala Leu Ala Asp Asp Tyr Leu Asn Glu Val Ala Ser Trp
               740                 745                 750
   Val Thr Lys Gln Arg Gln Ala Gly Thr Ser Arg Ile Asp Phe Ala Lys
           755                 760                 765
   His Gly Lys Pro Arg Gln Val Leu Ala Ser Asp Cys Val Val Glu Asp
       770                 775                 780
   Phe Leu Gln Ile Thr Ser Ala Asn Asn Leu Glu Glu Ser Ala Thr Arg
   785                 790                 795                 800
   Leu Ile Asp Tyr Pro Ser Ile Ser Ala Ile Leu Cys Asp Pro Thr Gly
                   805                 810                 815
   Thr Ile Pro Gly Ala Trp Thr Asp Ser Val Glu Lys Leu Ile Ala Ile
               820                 825                 830
   Ser Ala Lys Asp Ser Glu Ser Leu Arg Arg Ala Leu Arg Ala Ser Ile
           835                 840                 845
   Ser Ile Pro His Ser Lys Lys Phe Leu Pro Ile Thr Ser Arg Glu Ile
       850                 855                 860
   Pro Leu Ser Glu Ala Lys Thr Leu Leu Ser Gly Tyr Ser Ala Val His
   865                 870                 875                 880
   Ile Gln Pro Asp Glu Tyr Asp Leu Gln Ser Gly Leu Lys Gly Pro Gln
                   885                 890                 895
   Lys
<210> 83
   <211> 1368
   <212> PRT
   <213> Streptococcus pyogenes
<220>
   <223> CRISPR-associated endonuclease
<308> YP_600439
   <309> 2013-08-28
<400> 83
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
               35                  40                  45
   Gly Ala Leu Leu Phe Asp Ser Gly Glu Ile Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Leu Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Ala Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Thr Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
   610 615 620
   Leu Phe Glu Asp Arg Glu Met Ile Glu Glu Arg Leu Lys Thr Tyr Ala
   625 630 635 640
   His Leu Phe Asp Asp Lys Val Met Lys Gln Leu Lys Arg Arg Arg Tyr
   645 650 655
   Thr Gly Trp Gly Arg Leu Ser Arg Lys Leu Ile Asn Gly Ile Arg Asp
   660 665 670
   Lys Gln Ser Gly Lys Thr Ile Leu Asp Phe Leu Lys Ser Asp Gly Phe
   675 680 685
   Ala Asn Arg Asn Phe Met Gln Leu Ile His Asp Asp Ser Leu Thr Phe
   690 695 700
   Lys Glu Asp Ile Gln Lys Ala Gln Val Ser Gly Gln Gly Asp Ser Leu
   705 710 715 720
   His Glu His Ile Ala Asn Leu Ala Gly Ser Pro Ala Ile Lys Lys Gly
   725 730 735
   Ile Leu Gln Thr Val Lys Val Val Asp Glu Leu Val Lys Val Met Gly
   740 745 750
   Arg His Lys Pro Glu Asn Ile Val Ile Glu Met Ala Arg Glu Asn Gln
   755 760 765
   Thr Thr Gln Lys Gly Gln Lys Asn Ser Arg Glu Arg Met Lys Arg Ile
   770 775 780
   Glu Glu Gly Ile Lys Glu Leu Gly Ser Gln Ile Leu Lys Glu His Pro
   785 790 795 800
   Val Glu Asn Thr Gln Leu Gln Asn Glu Lys Leu Tyr Leu Tyr Tyr Leu
   805 810 815
   Gln Asn Gly Arg Asp Met Tyr Val Asp Gln Glu Leu Asp Ile Asn Arg
   820 825 830
   Leu Ser Asp Tyr Asp Val Asp His Ile Val Pro Gln Ser Phe Ile Lys
   835 840 845
   Asp Asp Ser Ile Asp Asn Lys Val Leu Thr Arg Ser Asp Lys Asn Arg
   850 855 860
   Gly Lys Ser Asp Asn Val Pro Ser Glu Glu Val Val Lys Lys Met Lys
   865 870 875 880
   Asn Tyr Trp Arg Gln Leu Leu Asn Ala Lys Leu Ile Thr Gln Arg Lys
   885 890 895
   Phe Asp Asn Leu Thr Lys Ala Glu Arg Gly Gly Leu Ser Glu Leu Asp
   900 905 910
   Lys Ala Gly Phe Ile Lys Arg Gln Leu Val Glu Thr Arg Gln Ile Thr
   915 920 925
   Lys His Val Ala Gln Ile Leu Asp Ser Arg Met Asn Thr Lys Tyr Asp
   930 935 940
   Glu Asn Asp Lys Leu Ile Arg Glu Val Lys Val Ile Thr Leu Lys Ser
   945 950 955 960
   Lys Leu Val Ser Asp Phe Arg Lys Asp Phe Gln Phe Tyr Lys Val Arg
   965 970 975
   Glu Ile Asn Asn Tyr His His Ala His Asp Ala Tyr Leu Asn Ala Val
   980 985 990
   Val Gly Thr Ala Leu Ile Lys Lys Tyr Pro Lys Leu Glu Ser Glu Phe
   995 1000 1005
   Val Tyr Gly Asp Tyr Lys Val Tyr Asp Val Arg Lys Met Leu Ala Lys
   1010 1015 1020
   Ser Glu Gln Glu Ile Gly Lys Ala Thr Ala Lys Tyr Phe Phe Tyr Ser
   1025 1030 1035 1040
   Asn Ile Met Asn Phe Phe Lys Thr Glu Ile Thr Leu Ala Asn Gly Glu
                   1045                1050                1055
   Ile Arg Lys Arg Pro Leu Ile Glu Thr Asn Gly Glu Thr Gly Glu Ile
               1060                1065                1070
   Val Trp Asp Lys Gly Arg Asp Phe Ala Thr Val Arg Lys Val Leu Ser
           1075                1080                1085
   Met Pro Gln Val Asn Ile Val Lys Lys Thr Glu Val Gln Thr Gly Gly
       1090                1095                1100
   Phe Ser Lys Glu Ser Ile Leu Pro Lys Arg Asn Ser Asp Lys Leu Ile
   1105                1110                1115                1120
   Ala Arg Lys Lys Asp Trp Asp Pro Lys Lys Tyr Gly Gly Phe Asp Ser
                   1125                1130                1135
   Pro Thr Val Ala Tyr Ser Val Leu Val Val Ala Lys Val Glu Lys Gly
               1140                1145                1150
   Lys Ser Lys Lys Leu Lys Ser Val Lys Glu Leu Leu Gly Ile Thr Ile
           1155                1160                1165
   Met Glu Arg Ser Ser Phe Glu Lys Asn Pro Ile Asp Phe Leu Glu Ala
       1170                1175                1180
   Lys Gly Tyr Lys Glu Val Arg Lys Asp Leu Ile Ile Lys Leu Pro Lys
   1185                1190                1195                1200
   Tyr Ser Leu Phe Glu Leu Glu Asn Gly Arg Lys Arg Met Leu Ala Ser
                   1205                1210                1215
   Ala Gly Glu Leu Gln Lys Gly Asn Glu Leu Ala Leu Pro Ser Lys Tyr
               1220                1225                1230
   Val Asn Phe Leu Tyr Leu Ala Ser His Tyr Glu Lys Leu Lys Gly Ser
           1235                1240                1245
   Pro Glu Asp Asn Glu Gln Lys Gln Leu Phe Val Glu Gln His Lys His
       1250                1255                1260
   Tyr Leu Asp Glu Ile Ile Glu Gln Ile Ser Glu Phe Ser Lys Arg Val
   1265                1270                1275                1280
   Ile Leu Ala Asp Ala Asn Leu Asp Lys Val Leu Ser Ala Tyr Asn Lys
                   1285                1290                1295
   His Arg Asp Lys Pro Ile Arg Glu Gln Ala Glu Asn Ile Ile His Leu
               1300                1305                1310
   Phe Thr Leu Thr Asn Leu Gly Ala Pro Thr Ala Phe Lys Tyr Phe Asp
           1315                1320                1325
   Thr Thr Ile Asp Arg Lys Arg Tyr Thr Ser Thr Lys Glu Val Leu Asp
       1330                1335                1340
   Ala Thr Phe Ile His Gln Ser Ile Thr Gly Leu Tyr Glu Thr Arg Ile
   1345                1350                1355                1360
   Asp Leu Ser Gln Leu Gly Gly Asp
                   1365
<210> 84
   <211> 1368
   <212> PRT
   <213> Streptococcus pyogenes
<220>
   <223> CRISPR-associated endonuclease
<308> YP_596617
   <309> 2013-08-28
<400> 84
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Ile Ala Glu Ala Thr Arg Leu
       50                  55                  60
   Lys Arg Thr Ala Arg Arg Arg Tyr Thr Arg Arg Lys Asn Arg Ile Cys
   65 70 75 80
   Tyr Leu Gln Glu Ile Phe Ser Asn Glu Met Ala Lys Val Asp Asp Ser
   85 90 95
   Phe Phe His Arg Leu Glu Glu Ser Phe Leu Val Glu Glu Asp Lys Lys
   100 105 110
   His Glu Arg His Pro Ile Phe Gly Asn Ile Val Asp Glu Val Ala Tyr
   115 120 125
   His Glu Lys Tyr Pro Thr Ile Tyr His Leu Arg Lys Lys Leu Val Asp
   130 135 140
   Ser Thr Asp Lys Ala Asp Leu Arg Leu Ile Tyr Leu Ala Leu Ala His
   145 150 155 160
   Met Ile Lys Phe Arg Gly His Phe Leu Ile Glu Gly Asp Leu Asn Pro
   165 170 175
   Asp Asn Ser Asp Val Asp Lys Leu Phe Ile Gln Leu Val Gln Thr Tyr
   180 185 190
   Asn Gln Leu Phe Glu Glu Asn Pro Ile Asn Ala Ser Gly Val Asp Ala
   195 200 205
   Lys Ala Ile Leu Ser Ala Arg Leu Ser Lys Ser Arg Arg Leu Glu Asn
   210 215 220
   Leu Ile Ala Gln Leu Pro Gly Glu Lys Lys Asn Gly Leu Phe Gly Asn
   225 230 235 240
   Leu Ile Ala Leu Ser Leu Gly Leu Thr Pro Asn Phe Lys Ser Asn Phe
   245 250 255
   Asp Leu Ala Glu Asp Ala Lys Leu Gln Leu Ser Lys Asp Thr Tyr Asp
   260 265 270
   Asp Asp Leu Asp Asn Leu Leu Ala Gln Ile Gly Asp Gln Tyr Ala Asp
   275 280 285
   Leu Phe Leu Ala Ala Lys Asn Leu Ser Asp Ala Ile Leu Leu Ser Asp
   290 295 300
   Ile Leu Arg Leu Asn Ser Glu Ile Thr Lys Ala Pro Leu Ser Ala Ser
   305 310 315 320
   Met Ile Lys Arg Tyr Asp Glu His His Gln Asp Leu Thr Leu Leu Lys
   325 330 335
   Ala Leu Val Arg Gln Gln Leu Pro Glu Lys Tyr Lys Glu Ile Phe Phe
   340 345 350
   Asp Gln Ser Lys Asn Gly Tyr Ala Gly Tyr Ile Asp Gly Gly Ala Ser
   355 360 365
   Gln Glu Glu Phe Tyr Lys Phe Ile Lys Pro Ile Leu Glu Lys Met Asp
   370 375 380
   Gly Thr Glu Glu Leu Leu Ala Lys Leu Asn Arg Glu Asp Leu Leu Arg
   385 390 395 400
   Lys Gln Arg Thr Phe Asp Asn Gly Ser Ile Pro His Gln Ile His Leu
   405 410 415
   Gly Glu Leu His Ala Ile Leu Arg Arg Gln Glu Asp Phe Tyr Pro Phe
   420 425 430
   Leu Lys Asp Asn Arg Glu Lys Ile Glu Lys Ile Leu Thr Phe Arg Ile
   435 440 445
   Pro Tyr Tyr Val Gly Pro Leu Ala Arg Gly Asn Ser Arg Phe Ala Trp
   450 455 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
   530 535 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Val Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Gln Ile Leu Lys Glu His Pro
   785                 790                 795                 800
   Val Glu Asn Thr Gln Leu Gln Asn Glu Lys Leu Tyr Leu Tyr Tyr Leu
                   805                 810                 815
   Gln Asn Gly Arg Asp Met Tyr Val Asp Gln Glu Leu Asp Ile Asn Arg
               820                 825                 830
   Leu Ser Asp Tyr Asp Val Asp His Ile Val Pro Gln Ser Phe Ile Lys
           835                 840                 845
   Asp Asp Ser Ile Asp Asn Lys Val Leu Thr Arg Ser Asp Lys Asn Arg
       850                 855                 860
   Gly Lys Ser Asp Asn Val Pro Ser Glu Glu Val Val Lys Lys Met Lys
   865                 870                 875                 880
   Asn Tyr Trp Arg Gln Leu Leu Asn Ala Lys Leu Ile Thr Gln Arg Lys
                   885                 890                 895
   Phe Asp Asn Leu Thr Lys Ala Glu Arg Gly Gly Leu Ser Glu Leu Asp
               900                 905                 910
   Lys Ala Gly Phe Ile Lys Arg Gln Leu Val Glu Thr Arg Gln Ile Thr
           915                 920                 925
   Lys His Val Ala Gln Ile Leu Asp Ser Arg Met Asn Thr Lys Tyr Asp
       930                 935                 940
   Glu Asn Asp Lys Leu Ile Arg Glu Val Lys Val Ile Thr Leu Lys Ser
   945                 950                 955                 960
   Lys Leu Val Ser Asp Phe Arg Lys Asp Phe Gln Phe Tyr Lys Val Arg
                   965                 970                 975
   Glu Ile Asn Asn Tyr His His Ala His Asp Ala Tyr Leu Asn Ala Val
               980                 985                 990
   Val Gly Thr Ala Leu Ile Lys Lys Tyr Pro Lys Leu Glu Ser Glu Phe
           995                 1000                1005
   Val Tyr Gly Asp Tyr Lys Val Tyr Asp Val Arg Lys Met Leu Ala Lys
       1010                1015                1020
   Ser Glu Gln Glu Ile Gly Lys Ala Thr Ala Lys Tyr Phe Phe Tyr Ser
   1025                1030                1035                1040
   Asn Ile Met Asn Phe Phe Lys Thr Glu Ile Thr Leu Ala Asn Gly Glu
                   1045                1050                1055
   Ile Arg Lys Arg Pro Leu Ile Glu Thr Asn Gly Glu Thr Gly Glu Ile
               1060                1065                1070
   Val Trp Asp Lys Gly Arg Asp Phe Ala Thr Val Arg Lys Val Leu Ser
   1075 1080 1085
   Met Pro Gln Val Asn Ile Val Lys Lys Thr Glu Val Gln Thr Gly Gly
   1090 1095 1100
   Phe Ser Lys Glu Ser Ile Leu Pro Lys Arg Asn Ser Asp Lys Leu Ile
   1105 1110 1115 1120
   Ala Arg Lys Lys Asp Trp Asp Pro Lys Lys Tyr Gly Gly Phe Asp Ser
   1125 1130 1135
   Pro Thr Val Ala Tyr Ser Val Leu Val Val Ala Lys Val Glu Lys Gly
   1140 1145 1150
   Lys Ser Lys Lys Leu Lys Ser Val Lys Glu Leu Leu Gly Ile Thr Ile
   1155 1160 1165
   Met Glu Arg Ser Ser Phe Glu Lys Asn Pro Ile Asp Phe Leu Glu Ala
   1170 1175 1180
   Lys Gly Tyr Lys Glu Val Arg Lys Asp Leu Ile Ile Lys Leu Pro Lys
   1185 1190 1195 1200
   Tyr Ser Leu Phe Glu Leu Glu Asn Gly Arg Lys Arg Met Leu Ala Ser
   1205 1210 1215
   Ala Gly Glu Leu Gln Lys Gly Asn Glu Leu Ala Leu Pro Ser Lys Tyr
   1220 1225 1230
   Val Asn Phe Leu Tyr Leu Ala Ser His Tyr Glu Lys Leu Lys Gly Ser
   1235 1240 1245
   Pro Glu Asp Asn Glu Gln Lys Gln Leu Phe Val Glu Gln His Lys His
   1250 1255 1260
   Tyr Leu Asp Glu Ile Ile Glu Gln Ile Ser Glu Phe Ser Lys Arg Val
   1265 1270 1275 1280
   Ile Leu Ala Asp Ala Asn Leu Asp Lys Val Leu Ser Ala Tyr Asn Lys
   1285 1290 1295
   His Arg Asp Lys Pro Ile Arg Glu Gln Ala Glu Asn Ile Ile His Leu
   1300 1305 1310
   Phe Thr Leu Thr Asn Leu Gly Ala Pro Thr Ala Phe Lys Tyr Phe Asp
   1315 1320 1325
   Thr Thr Ile Asp Arg Lys Arg Tyr Thr Ser Thr Lys Glu Val Leu Asp
   1330 1335 1340
   Ala Thr Phe Ile His Gln Ser Ile Thr Gly Leu Tyr Glu Thr Arg Ile
   1345 1350 1355 1360
   Asp Leu Ser Gln Leu Gly Gly Asp
   1365
<210> 85
   <211> 1168
   <212> PRT
   <213> Azospirillum sp.
<220>
   <223> CRISPR-associated endonuclease
<308> YP_003448082
   <309> 2013-06-10
<400> 85
   Met Ala Arg Pro Ala Phe Arg Ala Pro Arg Arg Glu His Val Asn Gly
    1               5                  10                  15
   Trp Thr Pro Asp Pro His Arg Ile Ser Lys Pro Phe Phe Ile Leu Val
               20                  25                  30
   Ser Trp His Leu Leu Ser Arg Val Val Ile Asp Ser Ser Ser Gly Cys
           35                  40                  45
   Phe Pro Gly Thr Ser Arg Asp His Thr Asp Lys Phe Ala Glu Trp Glu
       50                  55                  60
   Cys Ala Val Gln Pro Tyr Arg Leu Ser Phe Asp Leu Gly Thr Asn Ser
   65                  70                  75                  80
   Ile Gly Trp Gly Leu Leu Asn Leu Asp Arg Gln Gly Lys Pro Arg Glu
                       85                  90                  95
   Ile Arg Ala Leu Gly Ser Arg Ile Phe Ser Asp Gly Arg Asp Pro Gln
                   100                 105                 110
   Asp Lys Ala Ser Leu Ala Val Ala Arg Arg Leu Ala Arg Gln Met Arg
               115                 120                 125
   Arg Arg Arg Asp Arg Tyr Leu Thr Arg Arg Thr Arg Leu Met Gly Ala
           130                 135                 140
   Leu Val Arg Phe Gly Leu Met Pro Ala Asp Pro Ala Ala Arg Lys Arg
   145                 150                 155                 160
   Leu Glu Val Ala Val Asp Pro Tyr Leu Ala Arg Glu Arg Ala Thr Arg
                   165                 170                 175
   Glu Arg Leu Glu Pro Phe Glu Ile Gly Arg Ala Leu Phe His Leu Asn
               180                 185                 190
   Gln Arg Arg Gly Tyr Lys Pro Val Arg Thr Ala Thr Lys Pro Asp Glu
           195                 200                 205
   Glu Ala Gly Lys Val Lys Glu Ala Val Glu Arg Leu Glu Ala Ala Ile
       210                 215                 220
   Ala Ala Ala Gly Ala Pro Thr Leu Gly Ala Trp Phe Ala Trp Arg Lys
   225                 230                 235                 240
   Thr Arg Gly Glu Thr Leu Arg Ala Arg Leu Ala Gly Lys Gly Lys Glu
                   245                 250                 255
   Ala Ala Tyr Pro Phe Tyr Pro Ala Arg Arg Met Leu Glu Ala Glu Phe
               260                 265                 270
   Asp Thr Leu Trp Ala Glu Gln Ala Arg His His Pro Asp Leu Leu Thr
           275                 280                 285
   Ala Glu Ala Arg Glu Ile Leu Arg His Arg Ile Phe His Gln Arg Pro
       290                 295                 300
   Leu Lys Pro Pro Pro Val Gly Arg Cys Thr Leu Tyr Pro Asp Asp Gly
   305                 310                 315                 320
   Arg Ala Pro Arg Ala Leu Pro Ser Ala Gln Arg Leu Arg Leu Phe Gln
                   325                 330                 335
   Glu Leu Ala Ser Leu Arg Val Ile His Leu Asp Leu Ser Glu Arg Pro
               340                 345                 350
   Leu Thr Pro Ala Glu Arg Asp Arg Ile Val Ala Phe Val Gln Gly Arg
           355                 360                 365
   Pro Pro Lys Ala Gly Arg Lys Pro Gly Lys Val Gln Lys Ser Val Pro
       370                 375                 380
   Phe Glu Lys Leu Arg Gly Leu Leu Glu Leu Pro Pro Gly Thr Gly Phe
   385                 390                 395                 400
   Ser Leu Glu Ser Asp Lys Arg Pro Glu Leu Leu Gly Asp Glu Thr Gly
                   405                 410                 415
   Ala Arg Ile Ala Pro Ala Phe Gly Pro Gly Trp Thr Ala Leu Pro Leu
               420                 425                 430
   Glu Glu Gln Asp Ala Leu Val Glu Leu Leu Leu Thr Glu Ala Glu Pro
           435                 440                 445
   Glu Arg Ala Ile Ala Ala Leu Thr Ala Arg Trp Ala Leu Asp Glu Ala
       450                 455                 460
   Thr Ala Ala Lys Leu Ala Gly Ala Thr Leu Pro Asp Phe His Gly Arg
   465                 470                 475                 480
   Tyr Gly Arg Arg Ala Val Ala Glu Leu Leu Pro Val Leu Glu Arg Glu
                   485                 490                 495
   Thr Arg Gly Asp Pro Asp Gly Arg Val Arg Pro Ile Arg Leu Asp Glu
               500                 505                 510
   Ala Val Lys Leu Leu Arg Gly Gly Lys Asp His Ser Asp Phe Ser Arg
           515                 520                 525
   Glu Gly Ala Leu Leu Asp Ala Leu Pro Tyr Tyr Gly Ala Val Leu Glu
       530                 535                 540
   Arg His Val Ala Phe Gly Thr Gly Asn Pro Ala Asp Pro Glu Glu Lys
   545                 550                 555                 560
   Arg Val Gly Arg Val Ala Asn Pro Thr Val His Ile Ala Leu Asn Gln
                   565                 570                 575
   Leu Arg His Leu Val Asn Ala Ile Leu Ala Arg His Gly Arg Pro Glu
               580                 585                 590
   Glu Ile Val Ile Glu Leu Ala Arg Asp Leu Lys Arg Ser Ala Glu Asp
   595 600 605
   Arg Arg Arg Glu Asp Lys Arg Gln Ala Asp Asn Gln Lys Arg Asn Glu
   610 615 620
   Glu Arg Lys Arg Leu Ile Leu Ser Leu Gly Glu Arg Pro Thr Pro Arg
   625 630 635 640
   Asn Leu Leu Lys Leu Arg Leu Trp Glu Glu Gln Gly Pro Val Glu Asn
   645 650 655
   Arg Arg Cys Pro Tyr Ser Gly Glu Thr Ile Ser Met Arg Met Leu Leu
   660 665 670
   Ser Glu Gln Val Asp Ile Asp His Ile Leu Pro Phe Ser Val Ser Leu
   675 680 685
   Asp Asp Ser Ala Ala Asn Lys Val Val Cys Leu Arg Glu Ala Asn Arg
   690 695 700
   Ile Lys Arg Asn Arg Ser Pro Trp Glu Ala Phe Gly His Asp Ser Glu
   705 710 715 720
   Arg Trp Ala Gly Ile Leu Ala Arg Ala Glu Ala Leu Pro Lys Asn Lys
   725 730 735
   Arg Trp Arg Phe Ala Pro Asp Ala Leu Glu Lys Leu Glu Gly Glu Gly
   740 745 750
   Gly Leu Arg Ala Arg His Leu Asn Asp Thr Arg His Leu Ser Arg Leu
   755 760 765
   Ala Val Glu Tyr Leu Arg Cys Val Cys Pro Lys Val Arg Val Ser Pro
   770 775 780
   Gly Arg Leu Thr Ala Leu Leu Arg Arg Arg Trp Gly Ile Asp Ala Ile
   785 790 795 800
   Leu Ala Glu Ala Asp Gly Pro Pro Pro Glu Val Pro Ala Glu Thr Leu
   805 810 815
   Asp Pro Ser Pro Ala Glu Lys Asn Arg Ala Asp His Arg His His Ala
   820 825 830
   Leu Asp Ala Val Val Ile Gly Cys Ile Asp Arg Ser Met Val Gln Arg
   835 840 845
   Val Gln Leu Ala Ala Ala Ser Ala Glu Arg Glu Ala Ala Ala Arg Glu
   850 855 860
   Asp Asn Ile Arg Arg Val Leu Glu Gly Phe Lys Glu Glu Pro Trp Asp
   865 870 875 880
   Gly Phe Arg Ala Glu Leu Glu Arg Arg Ala Arg Thr Ile Val Val Ser
   885 890 895
   His Arg Pro Glu His Gly Ile Gly Gly Ala Leu His Lys Glu Thr Ala
   900 905 910
   Tyr Gly Pro Val Asp Pro Pro Glu Glu Gly Phe Asn Leu Val Val Arg
   915 920 925
   Lys Pro Ile Asp Gly Leu Ser Lys Asp Glu Ile Asn Ser Val Arg Asp
   930 935 940
   Pro Arg Leu Arg Arg Ala Leu Ile Asp Arg Leu Ala Ile Arg Arg Arg
   945 950 955 960
   Asp Ala Asn Asp Pro Ala Thr Ala Leu Ala Lys Ala Ala Glu Asp Leu
   965 970 975
   Ala Ala Gln Pro Ala Ser Arg Gly Ile Arg Arg Val Arg Val Leu Lys
   980 985 990
   Lys Glu Ser Asn Pro Ile Arg Val Glu His Gly Gly Asn Pro Ser Gly
   995 1000 1005
   Pro Arg Ser Gly Gly Pro Phe His Lys Leu Leu Leu Ala Gly Glu Val
   1010 1015 1020
   His His Val Asp Val Ala Leu Arg Ala Asp Gly Arg Arg Trp Val Gly
   1025 1030 1035 1040
   His Trp Val Thr Leu Phe Glu Ala His Gly Gly Arg Gly Ala Asp Gly
   1045 1050 1055
   Ala Ala Ala Pro Pro Arg Leu Gly Asp Gly Glu Arg Phe Leu Met Arg
   1060 1065 1070
   Leu His Lys Gly Asp Cys Leu Lys Leu Glu His Lys Gly Arg Val Arg
   1075 1080 1085
   Val Met Gln Val Val Lys Leu Glu Pro Ser Ser Asn Ser Val Val Val
       1090                1095                1100
   Val Glu Pro His Gln Val Lys Thr Asp Arg Ser Lys His Val Lys Ile
   1105                1110                1115                1120
   Ser Cys Asp Gln Leu Arg Ala Arg Gly Ala Arg Arg Val Thr Val Asp
                   1125                1130                1135
   Pro Leu Gly Arg Val Arg Val His Ala Pro Gly Ala Arg Val Gly Ile
               1140                1145                1150
   Gly Gly Asp Ala Gly Arg Thr Ala Met Glu Pro Ala Glu Asp Ile Ser
           1155                1160                1165
<210> 86
   <211> 1114
   <212> PRT
   <213> Eubacterium rectale
<220>
   <223> CRISPR-associated endonuclease
<308> YP_002937591
   <309> 2013-06-27
<400> 86
   Met Asn Tyr Thr Glu Lys Glu Lys Leu Phe Met Lys Tyr Ile Leu Ala
   1               5                   10                  15
   Leu Asp Ile Gly Ile Ala Ser Val Gly Trp Ala Ile Leu Asp Lys Glu
               20                  25                  30
   Ser Glu Thr Val Ile Glu Ala Gly Ser Asn Ile Phe Pro Glu Ala Ser
           35                  40                  45
   Ala Ala Asp Asn Gln Leu Arg Arg Asp Met Arg Gly Ala Lys Arg Asn
       50                  55                  60
   Asn Arg Arg Leu Lys Thr Arg Ile Asn Asp Phe Ile Lys Leu Trp Glu
   65                  70                  75                  80
   Asn Asn Asn Leu Ser Ile Pro Gln Phe Lys Ser Thr Glu Ile Val Gly
                   85                  90                  95
   Leu Lys Val Arg Ala Ile Thr Glu Glu Ile Thr Leu Asp Glu Leu Tyr
               100                 105                 110
   Leu Ile Leu Tyr Ser Tyr Leu Lys His Arg Gly Ile Ser Tyr Leu Glu
           115                 120                 125
   Asp Ala Leu Asp Asp Thr Val Ser Gly Ser Ser Ala Tyr Ala Asn Gly
       130                 135                 140
   Leu Lys Leu Asn Ala Lys Glu Leu Glu Thr His Tyr Pro Cys Glu Ile
   145                 150                 155                 160
   Gln Gln Glu Arg Leu Asn Thr Ile Gly Lys Tyr Arg Gly Gln Ser Gln
                   165                 170                 175
   Ile Ile Asn Glu Asn Gly Glu Val Leu Asp Leu Ser Asn Val Phe Thr
               180                 185                 190
   Ile Gly Ala Tyr Arg Lys Glu Ile Gln Arg Val Phe Glu Ile Gln Lys
           195                 200                 205
   Lys Tyr His Pro Glu Leu Thr Asp Glu Phe Cys Asp Gly Tyr Met Leu
       210                 215                 220
   Ile Phe Asn Arg Lys Arg Lys Tyr Tyr Glu Gly Pro Gly Asn Glu Lys
   225                 230                 235                 240
   Ser Arg Thr Asp Tyr Gly Arg Phe Thr Thr Lys Leu Asp Ala Asn Gly
                   245                 250                 255
   Asn Tyr Ile Thr Glu Asp Asn Ile Phe Glu Lys Leu Ile Gly Lys Cys
               260                 265                 270
   Ser Val Tyr Pro Asp Glu Leu Arg Ala Ala Ala Ala Ser Tyr Thr Ala
           275                 280                 285
   Gln Glu Tyr Asn Val Leu Asn Asp Leu Asn Asn Leu Thr Ile Asn Gly
       290                 295                 300
   Arg Lys Leu Glu Glu Asn Glu Lys His Glu Ile Val Glu Arg Ile Lys
   305                 310                 315                 320
   Ser Ser Asn Thr Ile Asn Met Arg Lys Ile Ile Ser Asp Cys Met Gly
                   325                 330                 335
   Glu Asn Ile Asp Asp Phe Ala Gly Ala Arg Ile Asp Lys Ser Gly Lys
               340                 345                 350
   Glu Ile Phe His Lys Phe Glu Val Tyr Asn Lys Met Arg Lys Ala Leu
           355                 360                 365
   Leu Glu Ile Gly Ile Asp Ile Ser Asn Tyr Ser Arg Glu Glu Leu Asp
       370                 375                 380
   Glu Ile Gly Tyr Ile Met Thr Ile Asn Thr Asp Lys Glu Ala Met Met
   385                 390                 395                 400
   Glu Ala Phe Gln Lys Ser Trp Ile Asp Leu Ser Asp Asp Val Lys Gln
                   405                 410                 415
   Cys Leu Ile Asn Met Arg Lys Thr Asn Gly Ala Leu Phe Asn Lys Trp
               420                 425                 430
   Gln Ser Phe Ser Leu Lys Ile Met Asn Glu Leu Ile Pro Glu Met Tyr
           435                 440                 445
   Ala Gln Pro Lys Glu Gln Met Thr Leu Leu Thr Glu Met Gly Val Thr
       450                 455                 460
   Lys Gly Thr Gln Glu Glu Phe Ala Gly Leu Lys Tyr Ile Pro Val Asp
   465                 470                 475                 480
   Val Val Ser Glu Asp Ile Phe Asn Pro Val Val Arg Arg Ser Val Arg
                   485                 490                 495
   Ile Ser Phe Lys Ile Leu Asn Ala Val Leu Lys Lys Tyr Lys Ala Leu
               500                 505                 510
   Asp Thr Ile Val Ile Glu Met Pro Arg Asp Arg Asn Ser Glu Glu Gln
           515                 520                 525
   Lys Lys Arg Ile Asn Asp Ser Gln Lys Leu Asn Glu Lys Glu Met Glu
       530                 535                 540
   Tyr Ile Glu Lys Lys Leu Ala Val Thr Tyr Gly Ile Lys Leu Ser Pro
   545                 550                 555                 560
   Ser Asp Phe Ser Ser Gln Lys Gln Leu Ser Leu Lys Leu Lys Leu Trp
                   565                 570                 575
   Asn Glu Gln Asp Gly Ile Cys Leu Tyr Ser Gly Lys Thr Ile Asp Pro
               580                 585                 590
   Asn Asp Ile Ile Asn Asn Pro Gln Leu Phe Glu Ile Asp His Ile Ile
           595                 600                 605
   Pro Arg Ser Ile Ser Phe Asp Asp Ala Arg Ser Asn Lys Val Leu Val
       610                 615                 620
   Tyr Arg Ser Glu Asn Gln Lys Lys Gly Asn Gln Thr Pro Tyr Tyr Tyr
   625                 630                 635                 640
   Leu Thr His Ser His Ser Glu Trp Ser Phe Glu Gln Tyr Lys Ala Thr
                   645                 650                 655
   Val Met Asn Leu Ser Lys Lys Lys Glu Tyr Ala Ile Ser Arg Lys Lys
               660                 665                 670
   Ile Gln Asn Leu Leu Tyr Ser Glu Asp Ile Thr Lys Met Asp Val Leu
           675                 680                 685
   Lys Gly Phe Ile Asn Arg Asn Ile Asn Asp Thr Ser Tyr Ala Ser Arg
       690                 695                 700
   Leu Val Leu Asn Thr Ile Gln Asn Phe Phe Met Ala Asn Glu Ala Asp
   705                 710                 715                 720
   Thr Lys Val Lys Val Ile Lys Gly Ser Tyr Thr His Gln Met Arg Cys
                   725                 730                 735
   Asn Leu Lys Leu Asp Lys Asn Arg Asp Glu Ser Tyr Ser His His Ala
               740                 745                 750
   Val Asp Ala Met Leu Ile Gly Tyr Ser Glu Leu Gly Tyr Glu Ala Tyr
           755                 760                 765
   His Lys Leu Gln Gly Glu Phe Ile Asp Phe Glu Thr Gly Glu Ile Leu
       770                 775                 780
   Arg Lys Asp Met Trp Asp Glu Asn Met Ser Asp Glu Val Tyr Ala Asp
   785                 790                 795                 800
   Tyr Leu Tyr Gly Lys Lys Trp Ala Asn Ile Arg Asn Glu Val Val Lys
                   805                 810                 815
   Ala Glu Lys Asn Val Lys Tyr Trp His Tyr Val Met Arg Lys Ser Asn
                   820                 825                 830
   Arg Gly Leu Cys Asn Gln Thr Ile Arg Gly Thr Arg Glu Tyr Asp Gly
               835                 840                 845
   Lys Gln Tyr Lys Ile Asn Lys Leu Asp Ile Arg Thr Lys Glu Gly Ile
       850                 855                 860
   Lys Val Phe Ala Lys Leu Ala Phe Ser Lys Lys Asp Ser Asp Arg Glu
   865                 870                 875                 880
   Arg Leu Leu Val Tyr Leu Asn Asp Arg Arg Thr Phe Asp Asp Leu Cys
                   885                 890                 895
   Lys Ile Tyr Glu Asp Tyr Ser Asp Ala Ala Asn Pro Phe Val Gln Tyr
               900                 905                 910
   Glu Lys Glu Thr Gly Asp Ile Ile Arg Lys Tyr Ser Lys Lys His Asn
           915                 920                 925
   Gly Pro Arg Ile Asp Lys Leu Lys Tyr Lys Asp Gly Glu Val Gly Ala
       930                 935                 940
   Cys Ile Asp Ile Ser His Lys Tyr Gly Phe Glu Lys Gly Ser Lys Lys
   945                 950                 955                 960
   Val Ile Leu Glu Ser Leu Val Pro Tyr Arg Met Asp Val Tyr Tyr Lys
                   965                 970                 975
   Glu Glu Asn His Ser Tyr Tyr Leu Val Gly Val Lys Gln Ser Asp Ile
               980                 985                 990
   Lys Phe Glu Lys Gly Arg Asn Val Ile Asp Glu Glu Ala Tyr Ala Arg
           995                 1000                1005
   Ile Leu Val Asn Glu Lys Met Ile Gln Pro Gly Gln Ser Arg Ala Asp
       1010                1015                1020
   Leu Glu Asn Leu Gly Phe Lys Phe Lys Leu Ser Phe Tyr Lys Asn Asp
   1025                1030                1035                1040
   Ile Ile Glu Tyr Glu Lys Asp Gly Lys Ile Tyr Thr Glu Arg Leu Val
                   1045                1050                1055
   Ser Arg Thr Met Pro Lys Gln Arg Asn Tyr Ile Glu Thr Lys Pro Ile
               1060                1065                1070
   Asp Lys Ala Lys Phe Glu Lys Gln Asn Leu Val Gly Leu Gly Lys Thr
           1075                1080                1085
   Lys Phe Ile Lys Lys Tyr Arg Tyr Asp Ile Leu Gly Asn Lys Tyr Ser
       1090                1095                1100
   Cys Ser Glu Glu Lys Phe Thr Ser Phe Cys
   1105                1110
<210> 87
   <211> 1029
   <212> PRT
   <213> Alicycliphilus denitrificans
<220>
   <223> CRISPR-associated endonuclease
<308> YP_004386148
   <309> 2013-07-06
<400> 87
   Met Arg Ser Leu Arg Tyr Arg Leu Ala Leu Asp Leu Gly Ser Thr Ser
    1               5                  10                  15
   Leu Gly Trp Ala Leu Phe Arg Leu Asp Ala Cys Asn Arg Pro Thr Ala
               20                  25                  30
   Val Ile Lys Ala Gly Val Arg Ile Phe Ser Asp Gly Arg Asn Pro Lys
           35                  40                  45
   Asp Gly Ser Ser Leu Ala Val Thr Arg Arg Ala Ala Arg Ala Met Arg
       50                  55                  60
   Arg Arg Arg Asp Arg Leu Leu Lys Arg Lys Thr Arg Met Gln Ala Lys
   65                  70                  75                  80
   Leu Val Glu His Gly Phe Phe Pro Ala Asp Ala Gly Lys Arg Lys Ala
                   85                  90                  95
   Leu Glu Gln Leu Asn Pro Tyr Ala Leu Arg Ala Lys Gly Leu Gln Glu
   100 105 110
   Ala Leu Leu Pro Gly Glu Phe Ala Arg Ala Leu Phe His Ile Asn Gln
   115 120 125
   Arg Arg Gly Phe Lys Ser Asn Arg Lys Thr Asp Lys Lys Asp Asn Asp
   130 135 140
   Ser Gly Val Leu Lys Lys Ala Ile Gly Gln Leu Arg Gln Gln Met Ala
   145 150 155 160
   Glu Gln Gly Ser Arg Thr Val Gly Glu Tyr Leu Trp Thr Arg Leu Gln
   165 170 175
   Gln Gly Gln Gly Val Arg Ala Arg Tyr Arg Glu Lys Pro Tyr Thr Thr
   180 185 190
   Glu Glu Gly Lys Lys Arg Ile Asp Lys Ser Tyr Asp Leu Tyr Ile Asp
   195 200 205
   Arg Ala Met Ile Glu Gln Glu Phe Asp Ala Leu Trp Ala Ala Gln Ala
   210 215 220
   Ala Phe Asn Pro Thr Leu Phe His Glu Ala Ala Arg Ala Asp Leu Lys
   225 230 235 240
   Asp Thr Leu Leu His Gln Arg Pro Leu Arg Pro Val Lys Pro Gly Arg
   245 250 255
   Cys Thr Leu Leu Pro Glu Glu Glu Arg Ala Pro Leu Ala Leu Pro Ser
   260 265 270
   Thr Gln Arg Phe Arg Ile His Gln Glu Val Asn His Leu Arg Leu Leu
   275 280 285
   Asp Glu Asn Leu Arg Glu Val Ala Leu Thr Leu Ala Gln Arg Asp Ala
   290 295 300
   Val Val Thr Ala Leu Glu Thr Lys Ala Lys Leu Ser Phe Glu Gln Ile
   305 310 315 320
   Arg Lys Leu Leu Lys Leu Ser Gly Ser Val Gln Phe Asn Leu Glu Asp
   325 330 335
   Ala Lys Arg Thr Glu Leu Lys Gly Asn Ala Thr Ser Ala Ala Leu Ala
   340 345 350
   Arg Lys Glu Leu Phe Gly Ala Ala Trp Ser Gly Phe Asp Glu Ala Leu
   355 360 365
   Gln Asp Glu Ile Val Trp Gln Leu Val Thr Glu Glu Gly Glu Gly Ala
   370 375 380
   Leu Ile Ala Trp Leu Gln Thr His Thr Gly Val Asp Glu Ala Arg Ala
   385 390 395 400
   Gln Ala Ile Val Asp Val Ser Leu Pro Glu Gly Tyr Gly Asn Leu Ser
   405 410 415
   Arg Lys Ala Leu Ala Arg Ile Val Pro Ala Leu Arg Ala Ala Val Ile
   420 425 430
   Thr Tyr Asp Lys Ala Val Gln Ala Ala Gly Phe Asp His His Ser Gln
   435 440 445
   Leu Gly Phe Glu Tyr Asp Ala Ser Glu Val Glu Asp Leu Val His Pro
   450 455 460
   Glu Thr Gly Glu Ile Arg Ser Val Phe Lys Gln Leu Pro Tyr Tyr Gly
   465 470 475 480
   Lys Ala Leu Gln Arg His Val Ala Phe Gly Ser Gly Lys Pro Glu Asp
   485 490 495
   Pro Asp Glu Lys Arg Tyr Gly Lys Ile Ala Asn Pro Thr Val His Ile
   500 505 510
   Gly Leu Asn Gln Val Arg Met Val Val Asn Ala Leu Ile Arg Arg Tyr
   515 520 525
   Gly Arg Pro Thr Glu Val Val Ile Glu Leu Ala Arg Asp Leu Lys Gln
   530 535 540
   Ser Arg Glu Gln Lys Val Glu Ala Gln Arg Arg Gln Ala Asp Asn Gln
   545 550 555 560
   Arg Arg Asn Ala Arg Ile Arg Arg Ser Ile Ala Glu Val Leu Gly Ile
   565 570 575
   Gly Glu Glu Arg Val Arg Gly Ser Asp Ile Gln Lys Trp Ile Cys Trp
   580 585 590
   Glu Glu Leu Ser Phe Asp Ala Ala Asp Arg Arg Cys Pro Tyr Ser Gly
           595                 600                 605
   Val Gln Ile Ser Ala Ala Met Leu Leu Ser Asp Glu Val Glu Val Glu
       610                 615                 620
   His Ile Leu Pro Phe Ser Lys Thr Leu Asp Asp Ser Leu Asn Asn Arg
   625                 630                 635                 640
   Thr Val Ala Met Arg Gln Ala Asn Arg Ile Lys Arg Asn Arg Thr Pro
                   645                 650                 655
   Trp Asp Ala Arg Ala Glu Phe Glu Ala Gln Gly Trp Ser Tyr Glu Asp
               660                 665                 670
   Ile Leu Gln Arg Ala Glu Arg Met Pro Leu Arg Lys Arg Tyr Arg Phe
           675                 680                 685
   Ala Pro Asp Gly Tyr Glu Arg Trp Leu Gly Asp Asp Lys Asp Phe Leu
       690                 695                 700
   Ala Arg Ala Leu Asn Asp Thr Arg Tyr Leu Ser Arg Val Ala Ala Glu
   705                 710                 715                 720
   Tyr Leu Arg Leu Val Cys Pro Gly Thr Arg Val Ile Pro Gly Gln Leu
                   725                 730                 735
   Thr Ala Leu Leu Arg Gly Lys Phe Gly Leu Asn Asp Val Leu Gly Leu
               740                 745                 750
   Asp Gly Glu Lys Asn Arg Asn Asp His Arg His His Ala Val Asp Ala
           755                 760                 765
   Cys Val Ile Gly Val Thr Asp Gln Gly Leu Met Gln Arg Phe Ala Thr
       770                 775                 780
   Ala Ser Ala Gln Ala Arg Gly Asp Gly Leu Thr Arg Leu Val Asp Gly
   785                 790                 795                 800
   Met Pro Met Pro Trp Pro Thr Tyr Arg Asp His Val Glu Arg Ala Val
                   805                 810                 815
   Arg His Ile Trp Val Ser His Arg Pro Asp His Gly Phe Glu Gly Ala
               820                 825                 830
   Met Met Glu Glu Thr Ser Tyr Gly Ile Arg Lys Asp Gly Ser Ile Lys
           835                 840                 845
   Gln Arg Arg Lys Ala Asp Gly Ser Ala Gly Arg Glu Ile Ser Asn Leu
       850                 855                 860
   Ile Arg Ile His Glu Ala Thr Gln Pro Leu Arg His Gly Val Ser Ala
   865                 870                 875                 880
   Asp Gly Gln Pro Leu Ala Tyr Lys Gly Tyr Val Gly Gly Ser Asn Tyr
                   885                 890                 895
   Cys Ile Glu Ile Thr Val Asn Asp Lys Gly Lys Trp Glu Gly Glu Val
               900                 905                 910
   Ile Ser Thr Phe Arg Ala Tyr Gly Val Val Arg Ala Gly Gly Met Gly
           915                 920                 925
   Arg Leu Arg Asn Pro His Glu Gly Gln Asn Gly Arg Lys Leu Ile Met
       930                 935                 940
   Arg Leu Val Ile Gly Asp Ser Val Arg Leu Glu Val Asp Gly Ala Glu
   945                 950                 955                 960
   Arg Thr Met Arg Ile Val Lys Ile Ser Gly Ser Asn Gly Gln Ile Phe
                   965                 970                 975
   Met Ala Pro Ile His Glu Ala Asn Val Asp Ala Arg Asn Thr Asp Lys
               980                 985                 990
   Gln Asp Ala Phe Thr Tyr Thr Ser Lys Tyr Ala Gly Ser Leu Gln Lys
           995                 1000                1005
   Ala Lys Thr Arg Arg Val Thr Ile Ser Pro Ile Gly Glu Val Arg Asp
       1010                1015                1020
   Pro Gly Phe Lys Gly
   1025
<210> 88
   <211> 1029
   <212> PRT
   <213> Alicycliphilus denitrificans
<220>
   <223> CRISPR-associated endonuclease
<308> YP_004124877
   <309> 2013-07-06
<400> 88
   Met Arg Ser Leu Arg Tyr Arg Leu Ala Leu Asp Leu Gly Ser Thr Ser
   1               5                   10                  15
   Leu Gly Trp Ala Leu Phe Arg Leu Asp Ala Cys Asn Arg Pro Thr Ala
               20                  25                  30
   Val Ile Lys Ala Gly Val Arg Ile Phe Ser Asp Gly Arg Asn Pro Lys
           35                  40                  45
   Asp Gly Ser Ser Leu Ala Val Thr Arg Arg Ala Ala Arg Ala Met Arg
       50                  55                  60
   Arg Arg Arg Asp Arg Leu Leu Lys Arg Lys Thr Arg Met Gln Ala Lys
   65                  70                  75                  80
   Leu Val Glu His Gly Phe Phe Pro Ala Asp Ala Gly Lys Arg Lys Ala
                   85                  90                  95
   Leu Glu Gln Leu Asn Pro Tyr Ala Leu Arg Ala Lys Gly Leu Gln Glu
               100                 105                 110
   Ala Leu Leu Pro Gly Glu Phe Ala Arg Ala Leu Phe His Ile Asn Gln
           115                 120                 125
   Arg Arg Gly Phe Lys Ser Asn Arg Lys Thr Asp Lys Lys Asp Asn Asp
       130                 135                 140
   Ser Gly Val Leu Lys Lys Ala Ile Gly Gln Leu Arg Gln Gln Met Ala
   145                 150                 155                 160
   Glu Gln Gly Ser Arg Thr Val Gly Glu Tyr Leu Trp Thr Arg Leu Gln
                   165                 170                 175
   Gln Gly Gln Gly Val Arg Ala Arg Tyr Arg Glu Lys Pro Tyr Thr Thr
               180                 185                 190
   Glu Glu Gly Lys Lys Arg Ile Asp Lys Ser Tyr Asp Leu Tyr Ile Asp
           195                 200                 205
   Arg Ala Met Ile Glu Gln Glu Phe Asp Ala Leu Trp Ala Ala Gln Ala
       210                 215                 220
   Ala Phe Asn Pro Thr Leu Phe His Glu Ala Ala Arg Ala Asp Leu Lys
   225                 230                 235                 240
   Asp Thr Leu Leu His Gln Arg Pro Leu Arg Pro Val Lys Pro Gly Arg
                   245                 250                 255
   Cys Thr Leu Leu Pro Glu Glu Glu Arg Ala Pro Leu Ala Leu Pro Ser
               260                 265                 270
   Thr Gln Arg Phe Arg Ile His Gln Glu Val Asn His Leu Arg Leu Leu
           275                 280                 285
   Asp Glu Asn Leu Arg Glu Val Ala Leu Thr Leu Ala Gln Arg Asp Ala
       290                 295                 300
   Val Val Thr Ala Leu Glu Thr Lys Ala Lys Leu Ser Phe Glu Gln Ile
   305                 310                 315                 320
   Arg Lys Leu Leu Lys Leu Ser Gly Ser Val Gln Phe Asn Leu Glu Asp
                   325                 330                 335
   Ala Lys Arg Thr Glu Leu Lys Gly Asn Ala Thr Ser Ala Ala Leu Ala
               340                 345                 350
   Arg Lys Glu Leu Phe Gly Ala Ala Trp Ser Gly Phe Asp Glu Ala Leu
           355                 360                 365
   Gln Asp Glu Ile Val Trp Gln Leu Val Thr Glu Glu Gly Glu Gly Ala
       370                 375                 380
   Leu Ile Ala Trp Leu Gln Thr His Thr Gly Val Asp Glu Ala Arg Ala
   385                 390                 395                 400
   Gln Ala Ile Val Asp Val Ser Leu Pro Glu Gly Tyr Gly Asn Leu Ser
                   405                 410                 415
   Arg Lys Ala Leu Ala Arg Ile Val Pro Ala Leu Arg Ala Ala Val Ile
               420                 425                 430
   Thr Tyr Asp Lys Ala Val Gln Ala Ala Gly Phe Asp His His Ser Gln
           435                 440                 445
   Leu Gly Phe Glu Tyr Asp Ala Ser Glu Val Glu Asp Leu Val His Pro
   450 455 460
   Glu Thr Gly Glu Ile Arg Ser Val Phe Lys Gln Leu Pro Tyr Tyr Gly
   465 470 475 480
   Lys Ala Leu Gln Arg His Val Ala Phe Gly Ser Gly Lys Pro Glu Asp
   485 490 495
   Pro Asp Glu Lys Arg Tyr Gly Lys Ile Ala Asn Pro Thr Val His Ile
   500 505 510
   Gly Leu Asn Gln Val Arg Met Val Val Asn Ala Leu Ile Arg Arg Tyr
   515 520 525
   Gly Arg Pro Thr Glu Val Val Ile Glu Leu Ala Arg Asp Leu Lys Gln
   530 535 540
   Ser Arg Glu Gln Lys Val Glu Ala Gln Arg Arg Gln Ala Asp Asn Gln
   545 550 555 560
   Arg Arg Asn Ala Arg Ile Arg Arg Ser Ile Ala Glu Val Leu Gly Ile
   565 570 575
   Gly Glu Glu Arg Val Arg Gly Ser Asp Ile Gln Lys Trp Ile Cys Trp
   580 585 590
   Glu Glu Leu Ser Phe Asp Ala Ala Asp Arg Arg Cys Pro Tyr Ser Gly
   595 600 605
   Val Gln Ile Ser Ala Ala Met Leu Leu Ser Asp Glu Val Glu Val Glu
   610 615 620
   His Ile Leu Pro Phe Ser Lys Thr Leu Asp Asp Ser Leu Asn Asn Arg
   625 630 635 640
   Thr Val Ala Met Arg Gln Ala Asn Arg Ile Lys Arg Asn Arg Thr Pro
   645 650 655
   Trp Asp Ala Arg Ala Glu Phe Glu Ala Gln Gly Trp Ser Tyr Glu Asp
   660 665 670
   Ile Leu Gln Arg Ala Glu Arg Met Pro Leu Arg Lys Arg Tyr Arg Phe
   675 680 685
   Ala Pro Asp Gly Tyr Glu Arg Trp Leu Gly Asp Asp Lys Asp Phe Leu
   690 695 700
   Ala Arg Ala Leu Asn Asp Thr Arg Tyr Leu Ser Arg Val Ala Ala Glu
   705 710 715 720
   Tyr Leu Arg Leu Val Cys Pro Gly Thr Arg Val Ile Pro Gly Gln Leu
   725 730 735
   Thr Ala Leu Leu Arg Gly Lys Phe Gly Leu Asn Asp Val Leu Gly Leu
   740 745 750
   Asp Gly Glu Lys Asn Arg Asn Asp His Arg His His Ala Val Asp Ala
   755 760 765
   Cys Val Ile Gly Val Thr Asp Gln Gly Leu Met Gln Arg Phe Ala Thr
   770 775 780
   Ala Ser Ala Gln Ala Arg Gly Asp Gly Leu Thr Arg Leu Val Asp Gly
   785 790 795 800
   Met Pro Met Pro Trp Pro Thr Tyr Arg Asp His Val Glu Arg Ala Val
   805 810 815
   Arg His Ile Trp Val Ser His Arg Pro Asp His Gly Phe Glu Gly Ala
   820 825 830
   Met Met Glu Glu Thr Ser Tyr Gly Ile Arg Lys Asp Gly Ser Ile Lys
   835 840 845
   Gln Arg Arg Lys Ala Asp Gly Ser Ala Gly Arg Glu Ile Ser Asn Leu
   850 855 860
   Ile Arg Ile His Glu Ala Thr Gln Pro Leu Arg His Gly Val Ser Ala
   865 870 875 880
   Asp Gly Gln Pro Leu Ala Tyr Lys Gly Tyr Val Gly Gly Ser Asn Tyr
   885 890 895
   Cys Ile Glu Ile Thr Val Asn Asp Lys Gly Lys Trp Glu Gly Glu Val
   900 905 910
   Ile Ser Thr Phe Arg Ala Tyr Gly Val Val Arg Ala Gly Gly Met Gly
   915 920 925
   Arg Leu Arg Asn Pro His Glu Gly Gln Asn Gly Arg Lys Leu Ile Met
   930 935 940
   Arg Leu Val Ile Gly Asp Ser Val Arg Leu Glu Val Asp Gly Ala Glu
   945 950 955 960
   Arg Thr Met Arg Ile Val Lys Ile Ser Gly Ser Asn Gly Gln Ile Phe
                   965                 970                 975
   Met Ala Pro Ile His Glu Ala Asn Val Asp Ala Arg Asn Thr Asp Lys
               980                 985                 990
   Gln Asp Ala Phe Thr Tyr Thr Ser Lys Tyr Ala Gly Ser Leu Gln Lys
           995                 1000                1005
   Ala Lys Thr Arg Arg Val Thr Ile Ser Pro Ile Gly Glu Val Arg Asp
       1010                1015                1020
   Pro Gly Phe Lys Gly
   1025
<210> 89
   <211> 1388
   <212> PRT
   <213> Streptococcus thermophilus
<220>
   <223> CRISPR-associated endonuclease
<308> YP_820832
   <309> 2013-08-27
<400> 89
   Met Thr Lys Pro Tyr Ser Ile Gly Leu Asp Ile Gly Thr Asn Ser Val
   1               5                   10                  15
   Gly Trp Ala Val Thr Thr Asp Asn Tyr Lys Val Pro Ser Lys Lys Met
               20                  25                  30
   Lys Val Leu Gly Asn Thr Ser Lys Lys Tyr Ile Lys Lys Asn Leu Leu
           35                  40                  45
   Gly Val Leu Leu Phe Asp Ser Gly Ile Thr Ala Glu Gly Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ser Thr Glu Met Ala Thr Leu Asp Asp Ala
                   85                  90                  95
   Phe Phe Gln Arg Leu Asp Asp Ser Phe Leu Val Pro Asp Asp Lys Arg
               100                 105                 110
   Asp Ser Lys Tyr Pro Ile Phe Gly Asn Leu Val Glu Glu Lys Ala Tyr
           115                 120                 125
   His Asp Glu Phe Pro Thr Ile Tyr His Leu Arg Lys Tyr Leu Ala Asp
       130                 135                 140
   Ser Thr Lys Lys Ala Asp Leu Arg Leu Val Tyr Leu Ala Leu Ala His
   145                 150                 155                 160
   Met Ile Lys Tyr Arg Gly His Phe Leu Ile Glu Gly Glu Phe Asn Ser
                   165                 170                 175
   Lys Asn Asn Asp Ile Gln Lys Asn Phe Gln Asp Phe Leu Asp Thr Tyr
               180                 185                 190
   Asn Ala Ile Phe Glu Ser Asp Leu Ser Leu Glu Asn Ser Lys Gln Leu
           195                 200                 205
   Glu Glu Ile Val Lys Asp Lys Ile Ser Lys Leu Glu Lys Lys Asp Arg
       210                 215                 220
   Ile Leu Lys Leu Phe Pro Gly Glu Lys Asn Ser Gly Ile Phe Ser Glu
   225                 230                 235                 240
   Phe Leu Lys Leu Ile Val Gly Asn Gln Ala Asp Phe Arg Lys Cys Phe
                   245                 250                 255
   Asn Leu Asp Glu Lys Ala Ser Leu His Phe Ser Lys Glu Ser Tyr Asp
               260                 265                 270
   Glu Asp Leu Glu Thr Leu Leu Gly Tyr Ile Gly Asp Asp Tyr Ser Asp
           275                 280                 285
   Val Phe Leu Lys Ala Lys Lys Leu Tyr Asp Ala Ile Leu Leu Ser Gly
       290                 295                 300
   Phe Leu Thr Val Thr Asp Asn Glu Thr Glu Ala Pro Leu Ser Ser Ala
   305                 310                 315                 320
   Met Ile Lys Arg Tyr Asn Glu His Lys Glu Asp Leu Ala Leu Leu Lys
                   325                 330                 335
   Glu Tyr Ile Arg Asn Ile Ser Leu Lys Thr Tyr Asn Glu Val Phe Lys
               340                 345                 350
   Asp Asp Thr Lys Asn Gly Tyr Ala Gly Tyr Ile Asp Gly Lys Thr Asn
           355                 360                 365
   Gln Glu Asp Phe Tyr Val Tyr Leu Lys Lys Leu Leu Ala Glu Phe Glu
       370                 375                 380
   Gly Ala Asp Tyr Phe Leu Glu Lys Ile Asp Arg Glu Asp Phe Leu Arg
   385                 390                 395                 400
   Lys Gln Arg Thr Phe Asp Asn Gly Ser Ile Pro Tyr Gln Ile His Leu
                   405                 410                 415
   Gln Glu Met Arg Ala Ile Leu Asp Lys Gln Ala Lys Phe Tyr Pro Phe
               420                 425                 430
   Leu Ala Lys Asn Lys Glu Arg Ile Glu Lys Ile Leu Thr Phe Arg Ile
           435                 440                 445
   Pro Tyr Tyr Val Gly Pro Leu Ala Arg Gly Asn Ser Asp Phe Ala Trp
       450                 455                 460
   Ser Ile Arg Lys Arg Asn Glu Lys Ile Thr Pro Trp Asn Phe Glu Asp
   465                 470                 475                 480
   Val Ile Asp Lys Glu Ser Ser Ala Glu Ala Phe Ile Asn Arg Met Thr
                   485                 490                 495
   Ser Phe Asp Leu Tyr Leu Pro Glu Glu Lys Val Leu Pro Lys His Ser
               500                 505                 510
   Leu Leu Tyr Glu Thr Phe Asn Val Tyr Asn Glu Leu Thr Lys Val Arg
           515                 520                 525
   Phe Ile Ala Glu Ser Met Arg Asp Tyr Gln Phe Leu Asp Ser Lys Gln
       530                 535                 540
   Lys Lys Asp Ile Val Arg Leu Tyr Phe Lys Asp Lys Arg Lys Val Thr
   545                 550                 555                 560
   Asp Lys Asp Ile Ile Glu Tyr Leu His Ala Ile Tyr Gly Tyr Asp Gly
                   565                 570                 575
   Ile Glu Leu Lys Gly Ile Glu Lys Gln Phe Asn Ser Ser Leu Ser Thr
               580                 585                 590
   Tyr His Asp Leu Leu Asn Ile Ile Asn Asp Lys Glu Phe Leu Asp Asp
           595                 600                 605
   Ser Ser Asn Glu Ala Ile Ile Glu Glu Ile Ile His Thr Leu Thr Ile
       610                 615                 620
   Phe Glu Asp Arg Glu Met Ile Lys Gln Arg Leu Ser Lys Phe Glu Asn
   625                 630                 635                 640
   Ile Phe Asp Lys Ser Val Leu Lys Lys Leu Ser Arg Arg His Tyr Thr
                   645                 650                 655
   Gly Trp Gly Lys Leu Ser Ala Lys Leu Ile Asn Gly Ile Arg Asp Glu
               660                 665                 670
   Lys Ser Gly Asn Thr Ile Leu Asp Tyr Leu Ile Asp Asp Gly Ile Ser
           675                 680                 685
   Asn Arg Asn Phe Met Gln Leu Ile His Asp Asp Ala Leu Ser Phe Lys
       690                 695                 700
   Lys Lys Ile Gln Lys Ala Gln Ile Ile Gly Asp Glu Asp Lys Gly Asn
   705                 710                 715                 720
   Ile Lys Glu Val Val Lys Ser Leu Pro Gly Ser Pro Ala Ile Lys Lys
                   725                 730                 735
   Gly Ile Leu Gln Ser Ile Lys Ile Val Asp Glu Leu Val Lys Val Met
               740                 745                 750
   Gly Gly Arg Lys Pro Glu Ser Ile Val Val Glu Met Ala Arg Glu Asn
           755                 760                 765
   Gln Tyr Thr Asn Gln Gly Lys Ser Asn Ser Gln Gln Arg Leu Lys Arg
       770                 775                 780
   Leu Glu Lys Ser Leu Lys Glu Leu Gly Ser Lys Ile Leu Lys Glu Asn
   785                 790                 795                 800
   Ile Pro Ala Lys Leu Ser Lys Ile Asp Asn Asn Ala Leu Gln Asn Asp
                   805                 810                 815
   Arg Leu Tyr Leu Tyr Tyr Leu Gln Asn Gly Lys Asp Met Tyr Thr Gly
   820 825 830
   Asp Asp Leu Asp Ile Asp Arg Leu Ser Asn Tyr Asp Ile Asp His Ile
   835 840 845
   Ile Pro Gln Ala Phe Leu Lys Asp Asn Ser Ile Asp Asn Lys Val Leu
   850 855 860
   Val Ser Ser Ala Ser Asn Arg Gly Lys Ser Asp Asp Val Pro Ser Leu
   865 870 875 880
   Glu Val Val Lys Lys Arg Lys Thr Phe Trp Tyr Gln Leu Leu Lys Ser
   885 890 895
   Lys Leu Ile Ser Gln Arg Lys Phe Asp Asn Leu Thr Lys Ala Glu Arg
   900 905 910
   Gly Gly Leu Ser Pro Glu Asp Lys Ala Gly Phe Ile Gln Arg Gln Leu
   915 920 925
   Val Glu Thr Arg Gln Ile Thr Lys His Val Ala Arg Leu Leu Asp Glu
   930 935 940
   Lys Phe Asn Asn Lys Lys Asp Glu Asn Asn Arg Ala Val Arg Thr Val
   945 950 955 960
   Lys Ile Ile Thr Leu Lys Ser Thr Leu Val Ser Gln Phe Arg Lys Asp
   965 970 975
   Phe Glu Leu Tyr Lys Val Arg Glu Ile Asn Asp Phe His His Ala His
   980 985 990
   Asp Ala Tyr Leu Asn Ala Val Val Ala Ser Ala Leu Leu Lys Lys Tyr
   995 1000 1005
   Pro Lys Leu Glu Pro Glu Phe Val Tyr Gly Asp Tyr Pro Lys Tyr Asn
   1010 1015 1020
   Ser Phe Arg Glu Arg Lys Ser Ala Thr Glu Lys Val Tyr Phe Tyr Ser
   1025 1030 1035 1040
   Asn Ile Met Asn Ile Phe Lys Lys Ser Ile Ser Leu Ala Asp Gly Arg
   1045 1050 1055
   Val Ile Glu Arg Pro Leu Ile Glu Val Asn Glu Glu Thr Gly Glu Ser
   1060 1065 1070
   Val Trp Asn Lys Glu Ser Asp Leu Ala Thr Val Arg Arg Val Leu Ser
   1075 1080 1085
   Tyr Pro Gln Val Asn Val Val Lys Lys Val Glu Glu Gln Asn His Gly
   1090 1095 1100
   Leu Asp Arg Gly Lys Pro Lys Gly Leu Phe Asn Ala Asn Leu Ser Ser
   1105 1110 1115 1120
   Lys Pro Lys Pro Asn Ser Asn Glu Asn Leu Val Gly Ala Lys Glu Tyr
   1125 1130 1135
   Leu Asp Pro Lys Lys Tyr Gly Gly Tyr Ala Gly Ile Ser Asn Ser Phe
   1140 1145 1150
   Thr Val Leu Val Lys Gly Thr Ile Glu Lys Gly Ala Lys Lys Lys Ile
   1155 1160 1165
   Thr Asn Val Leu Glu Phe Gln Gly Ile Ser Ile Leu Asp Arg Ile Asn
   1170 1175 1180
   Tyr Arg Lys Asp Lys Leu Asn Phe Leu Leu Glu Lys Gly Tyr Lys Asp
   1185 1190 1195 1200
   Ile Glu Leu Ile Ile Glu Leu Pro Lys Tyr Ser Leu Phe Glu Leu Ser
   1205 1210 1215
   Asp Gly Ser Arg Arg Met Leu Ala Ser Ile Leu Ser Thr Asn Asn Lys
   1220 1225 1230
   Arg Gly Glu Ile His Lys Gly Asn Gln Ile Phe Leu Ser Gln Lys Phe
   1235 1240 1245
   Val Lys Leu Leu Tyr His Ala Lys Arg Ile Ser Asn Thr Ile Asn Glu
   1250 1255 1260
   Asn His Arg Lys Tyr Val Glu Asn His Lys Lys Glu Phe Glu Glu Leu
   1265 1270 1275 1280
   Phe Tyr Tyr Ile Leu Glu Phe Asn Glu Asn Tyr Val Gly Ala Lys Lys
   1285 1290 1295
   Asn Gly Lys Leu Leu Asn Ser Ala Phe Gln Ser Trp Gln Asn His Ser
   1300 1305 1310
   Ile Asp Glu Leu Cys Ser Ser Phe Ile Gly Pro Thr Gly Ser Glu Arg
           1315                1320                1325
   Lys Gly Leu Phe Glu Leu Thr Ser Arg Gly Ser Ala Ala Asp Phe Glu
       1330                1335                1340
   Phe Leu Gly Val Lys Ile Pro Arg Tyr Arg Asp Tyr Thr Pro Ser Ser
   1345                1350                1355                1360
   Leu Leu Lys Asp Ala Thr Leu Ile His Gln Ser Val Thr Gly Leu Tyr
                   1365                1370                1375
   Glu Thr Arg Ile Asp Leu Ala Lys Leu Gly Glu Gly
               1380                1385
<210> 90
   <211> 1121
   <212> PRT
   <213> Streptococcus thermophilus
<220>
   <223> CRISPR-associated endonuclease
<308> YP_820161
   <309> 2013-08-27
<400> 90
   Met Ser Asp Leu Val Leu Gly Leu Asp Ile Gly Ile Gly Ser Val Gly
   1 5 10 15
   Val Gly Ile Leu Asn Lys Val Thr Gly Glu Ile Ile His Lys Asn Ser
   20 25 30
   Arg Ile Phe Pro Ala Ala Gln Ala Glu Asn Asn Leu Val Arg Arg Thr
   35 40 45
   Asn Arg Gln Gly Arg Arg Leu Ala Arg Arg Lys Lys His Arg Arg Val
   50 55 60
   Arg Leu Asn Arg Leu Phe Glu Glu Ser Gly Leu Ile Thr Asp Phe Thr
   65 70 75 80
   Lys Ile Ser Ile Asn Leu Asn Pro Tyr Gln Leu Arg Val Lys Gly Leu
   85 90 95
   Thr Asp Glu Leu Ser Asn Glu Glu Leu Phe Ile Ala Leu Lys Asn Met
   100 105 110
   Val Lys His Arg Gly Ile Ser Tyr Leu Asp Asp Ala Ser Asp Asp Gly
   115 120 125
   Asn Ser Ser Val Gly Asp Tyr Ala Gln Ile Val Lys Glu Asn Ser Lys
   130 135 140
   Gln Leu Glu Thr Lys Thr Pro Gly Gln Ile Gln Leu Glu Arg Tyr Gln
   145 150 155 160
   Thr Tyr Gly Gln Leu Arg Gly Asp Phe Thr Val Glu Lys Asp Gly Lys
   165 170 175
   Lys His Arg Leu Ile Asn Val Phe Pro Thr Ser Ala Tyr Arg Ser Glu
   180 185 190
   Ala Leu Arg Ile Leu Gln Thr Gln Gln Glu Phe Asn Pro Gln Ile Thr
   195 200 205
   Asp Glu Phe Ile Asn Arg Tyr Leu Glu Ile Leu Thr Gly Lys Arg Lys
   210 215 220
   Tyr Tyr His Gly Pro Gly Asn Glu Lys Ser Arg Thr Asp Tyr Gly Arg
   225 230 235 240
   Tyr Arg Thr Ser Gly Glu Thr Leu Asp Asn Ile Phe Gly Ile Leu Ile
   245 250 255
   Gly Lys Cys Thr Phe Tyr Pro Asp Glu Phe Arg Ala Ala Lys Ala Ser
   260 265 270
   Tyr Thr Ala Gln Glu Phe Asn Leu Leu Asn Asp Leu Asn Asn Leu Thr
   275 280 285
   Val Pro Thr Glu Thr Lys Lys Leu Ser Lys Glu Gln Lys Asn Gln Ile
   290 295 300
   Ile Asn Tyr Val Lys Asn Glu Lys Ala Met Gly Pro Ala Lys Leu Phe
   305 310 315 320
   Lys Tyr Ile Ala Lys Leu Leu Ser Cys Asp Val Ala Asp Ile Lys Gly
   325 330 335
   Tyr Arg Ile Asp Lys Ser Gly Lys Ala Glu Ile His Thr Phe Glu Ala
   340 345 350
   Tyr Arg Lys Met Lys Thr Leu Glu Thr Leu Asp Ile Glu Gln Met Asp
   355 360 365
   Arg Glu Thr Leu Asp Lys Leu Ala Tyr Val Leu Thr Leu Asn Thr Glu
   370 375 380
   Arg Glu Gly Ile Gln Glu Ala Leu Glu His Glu Phe Ala Asp Gly Ser
   385 390 395 400
   Phe Ser Gln Lys Gln Val Asp Glu Leu Val Gln Phe Arg Lys Ala Asn
   405 410 415
   Ser Ser Ile Phe Gly Lys Gly Trp His Asn Phe Ser Val Lys Leu Met
   420 425 430
   Met Glu Leu Ile Pro Glu Leu Tyr Glu Thr Ser Glu Glu Gln Met Thr
   435 440 445
   Ile Leu Thr Arg Leu Gly Lys Gln Lys Thr Thr Ser Ser Ser Asn Lys
   450 455 460
   Thr Lys Tyr Ile Asp Glu Lys Leu Leu Thr Glu Glu Ile Tyr Asn Pro
   465 470 475 480
   Val Val Ala Lys Ser Val Arg Gln Ala Ile Lys Ile Val Asn Ala Ala
   485 490 495
   Ile Lys Glu Tyr Gly Asp Phe Asp Asn Ile Val Ile Glu Met Ala Arg
   500 505 510
   Glu Thr Asn Glu Asp Asp Glu Lys Lys Ala Ile Gln Lys Ile Gln Lys
   515 520 525
   Ala Asn Lys Asp Glu Lys Asp Ala Ala Met Leu Lys Ala Ala Asn Gln
   530 535 540
   Tyr Asn Gly Lys Ala Glu Leu Pro His Ser Val Phe His Gly His Lys
   545 550 555 560
   Gln Leu Ala Thr Lys Ile Arg Leu Trp His Gln Gln Gly Glu Arg Cys
   565 570 575
   Leu Tyr Thr Gly Lys Thr Ile Ser Ile His Asp Leu Ile Asn Asn Ser
   580 585 590
   Asn Gln Phe Glu Val Asp His Ile Leu Pro Leu Ser Ile Thr Phe Asp
   595 600 605
   Asp Ser Leu Ala Asn Lys Val Leu Val Tyr Ala Thr Ala Asn Gln Glu
   610 615 620
   Lys Gly Gln Arg Thr Pro Tyr Gln Ala Leu Asp Ser Met Asp Asp Ala
   625 630 635 640
   Trp Ser Phe Arg Glu Leu Lys Ala Phe Val Arg Glu Ser Lys Thr Leu
   645 650 655
   Ser Asn Lys Lys Lys Glu Tyr Leu Leu Thr Glu Glu Asp Ile Ser Lys
   660 665 670
   Phe Asp Val Arg Lys Lys Phe Ile Glu Arg Asn Leu Val Asp Thr Arg
   675 680 685
   Tyr Ala Ser Arg Val Val Leu Asn Ala Leu Gln Glu His Phe Arg Ala
   690 695 700
   His Lys Ile Asp Thr Lys Val Ser Val Val Arg Gly Gln Phe Thr Ser
   705 710 715 720
   Gln Leu Arg Arg His Trp Gly Ile Glu Lys Thr Arg Asp Thr Tyr His
   725 730 735
   His His Ala Val Asp Ala Leu Ile Ile Ala Ala Ser Ser Gln Leu Asn
   740 745 750
   Leu Trp Lys Lys Gln Lys Asn Thr Leu Val Ser Tyr Ser Glu Asp Gln
   755 760 765
   Leu Leu Asp Ile Glu Thr Gly Glu Leu Ile Ser Asp Asp Glu Tyr Lys
   770 775 780
   Glu Ser Val Phe Lys Ala Pro Tyr Gln His Phe Val Asp Thr Leu Lys
   785 790 795 800
   Ser Lys Glu Phe Glu Asp Ser Ile Leu Phe Ser Tyr Gln Val Asp Ser
   805 810 815
   Lys Phe Asn Arg Lys Ile Ser Asp Ala Thr Ile Tyr Ala Thr Arg Gln
                   820                 825                 830
   Ala Lys Val Gly Lys Asp Lys Ala Asp Glu Thr Tyr Val Leu Gly Lys
               835                 840                 845
   Ile Lys Asp Ile Tyr Thr Gln Asp Gly Tyr Asp Ala Phe Met Lys Ile
       850                 855                 860
   Tyr Lys Lys Asp Lys Ser Lys Phe Leu Met Tyr Arg His Asp Pro Gln
   865                 870                 875                 880
   Thr Phe Glu Lys Val Ile Glu Pro Ile Leu Glu Asn Tyr Pro Asn Lys
                   885                 890                 895
   Gln Ile Asn Glu Lys Gly Lys Glu Val Pro Cys Asn Pro Phe Leu Lys
               900                 905                 910
   Tyr Lys Glu Glu His Gly Tyr Ile Arg Lys Tyr Ser Lys Lys Gly Asn
           915                 920                 925
   Gly Pro Glu Ile Lys Ser Leu Lys Tyr Tyr Asp Ser Lys Leu Gly Asn
       930                 935                 940
   His Ile Asp Ile Thr Pro Lys Asp Ser Asn Asn Lys Val Val Leu Gln
   945                 950                 955                 960
   Ser Val Ser Pro Trp Arg Ala Asp Val Tyr Phe Asn Lys Thr Thr Gly
                   965                 970                 975
   Lys Tyr Glu Ile Leu Gly Leu Lys Tyr Ala Asp Leu Gln Phe Glu Lys
               980                 985                 990
   Gly Thr Gly Thr Tyr Lys Ile Ser Gln Glu Lys Tyr Asn Asp Ile Lys
           995                 1000                1005
   Lys Lys Glu Gly Val Asp Ser Asp Ser Glu Phe Lys Phe Thr Leu Tyr
       1010                1015                1020
   Lys Asn Asp Leu Leu Leu Val Lys Asp Thr Glu Thr Lys Glu Gln Gln
   1025                1030                1035                1040
   Leu Phe Arg Phe Leu Ser Arg Thr Met Pro Lys Gln Lys His Tyr Val
                   1045                1050                1055
   Glu Leu Lys Pro Tyr Asp Lys Gln Lys Phe Glu Gly Gly Glu Ala Leu
               1060                1065                1070
   Ile Lys Val Leu Gly Asn Val Ala Asn Ser Gly Gln Cys Lys Lys Gly
           1075                1080                1085
   Leu Gly Lys Ser Asn Ile Ser Ile Tyr Lys Val Arg Thr Asp Val Leu
       1090                1095                1100
   Gly Asn Gln His Ile Ile Lys Asn Glu Gly Asp Lys Pro Lys Leu Asp
   1105                1110                1115                1120
   Phe
<210> 91
   <211> 876
   <212> PRT
   <213> Corynebacterium diphtheriae
<220>
   <223> CRISPR-associated endonuclease
<308> YP_005126356
   <309> 2013-06-11
<400> 91
   Met Asn Pro His Glu Glu Leu Trp Ala Lys Gln Lys Gly Leu Ala Lys
    1               5                  10                  15
   Pro Tyr Pro Leu Leu Ala His Leu Leu Asp Ser Ala Ala Val Ala Gly
               20                  25                  30
   Ala Leu Trp Asp His Trp Leu Arg Gln Asp Leu Arg Gln Met Phe Ile
           35                  40                  45
   Glu Glu Leu Gly Ser Asn Ala Arg Glu Ile Ile Gln Phe Val Val Gly
       50                  55                  60
   Ser His Asp Ile Gly Lys Ala Thr Pro Leu Phe Gln Tyr Gln Lys Ala
   65                  70                  75                  80
   Gln Lys Gly Glu Val Trp Asp Ser Ile Arg Tyr Ala Ile Asp Arg Thr
   85 90 95
   Gly Arg Tyr Gln Lys Pro Leu Pro Ser Ser Tyr Leu Val Lys Lys Thr
   100 105 110
   Ser Gly Gly Pro Asn Arg His Glu Gln Trp Ser Ser Phe Ala Ser Lys
   115 120 125
   Asn Glu Tyr Leu Lys Pro Ser Ala Ala Ala Lys Glu Asn Trp Ile Gly
   130 135 140
   Leu Ala Ile Gly Gly His His Gly Arg Phe Glu Pro Val Gly Tyr Gly
   145 150 155 160
   Arg His Gln Arg Lys Ala Ala Glu Asp Leu Ala Lys Ser Gly Trp Ser
   165 170 175
   Ala Ala Gln Gln Asp Leu Leu Arg Ala Leu Glu Lys Ala Ser Gly Ile
   180 185 190
   Thr Arg Ala Ser Leu Pro Ser Glu Leu Ser Pro Glu Leu Thr Leu Val
   195 200 205
   Leu Ser Gly Leu Thr Ile Leu Ala Asp Arg Ile Ser Ser Thr Glu Ser
   210 215 220
   Phe Val Ile Thr Gly Ala Arg Met Ile Asp Asp Gly Thr Leu His Leu
   225 230 235 240
   Ala Thr Pro Ile Asp Trp Leu Lys Thr Arg Lys Leu Asp Ser Glu Lys
   245 250 255
   His Val Ala Lys Thr Val Gly Ile Tyr His Gly Trp Asn Asn His Glu
   260 265 270
   Ser Ala Ile His Ser Ile Leu Lys Gly Tyr Asp Pro Arg Pro Leu Gln
   275 280 285
   Thr Ile Ala Leu Gln Asn Gln Val Gly Leu Leu Asn Leu Met Ala Pro
   290 295 300
   Thr Gly Asn Gly Lys Thr Glu Ala Ala Ile Leu Arg His Ser Leu Lys
   305 310 315 320
   Glu Asn Asp Arg Leu Ile Phe Leu Leu Pro Thr Gln Ala Thr Ser Asn
   325 330 335
   Ala Ile Met Arg Arg Val Gln Gly Ile Tyr Ser Asp Thr Pro Asn Ala
   340 345 350
   Ala Ala Leu Ala His Ser Leu Ala Ser Val Glu Asp Phe Tyr Gln Thr
   355 360 365
   Pro Leu Ser Val Phe Asp Asp His Tyr Asp Pro Ser Lys Glu Gln Phe
   370 375 380
   Glu Ser Ser Met Ser Gly Gly Leu Tyr Pro Ser Ser Phe Val Cys Ser
   385 390 395 400
   Gly Ala Ala Arg Leu Leu Ala Pro Ile Cys Ile Gly Thr Val Asp Gln
   405 410 415
   Ala Leu Ala Thr Ala Leu Pro Gly Lys Trp Ile His Leu Arg Ile Leu
   420 425 430
   Ala Leu Ala Asn Ala His Ile Val Ile Asp Glu Val His Thr Leu Asp
   435 440 445
   His Tyr Gln Thr Ala Leu Leu Glu Asn Ile Leu Pro Ile Leu Ala Lys
   450 455 460
   Leu Lys Thr Lys Ile Thr Phe Leu Thr Ala Thr Met Pro Ser Trp Gln
   465 470 475 480
   Arg Thr Lys Leu Leu Thr Ala Tyr Gly Gly Glu Asp Leu Gln Ile Pro
   485 490 495
   Pro Thr Val Phe Pro Ala Ala Glu Thr Val Leu Pro Gly Gln Phe Asn
   500 505 510
   Arg Thr Leu Ile Asp Ser Asp Ser Thr Thr Ile Asp Phe Thr Met Glu
   515 520 525
   Glu Thr Ser Tyr Asp His Leu Val Glu Ser His Val Lys Trp His Gln
   530 535 540
   Thr Thr Arg Leu Asn Ala Pro His Ala Arg Ile Gly Leu Ile Cys Asn
   545 550 555 560
   Thr Val Lys Arg Ala Gln Glu Ile Ala Ala Ala Leu Glu Lys Thr Asn
   565 570 575
   Asp Arg Ile Val Leu Leu His Ser Arg Met Thr Thr Glu His Arg Arg
                   580                 585                 590
   Arg Ser Ala Glu Leu Leu Glu Ser Leu Leu Gly Pro Asn Gly Asn Arg
           595                 600                 605
   Lys Thr Ile Thr Val Val Gly Thr Gln Ala Ile Glu Ala Ser Leu Asp
       610                 615                 620
   Ile Asp Leu Asp Ile Leu Arg Thr Glu Leu Cys Pro Ala Pro Ser Leu
   625                 630                 635                 640
   Val Gln Arg Ala Gly Arg Val Trp Arg Arg Asn Asp Pro Tyr Arg Ser
                   645                 650                 655
   Ser Arg Ile Thr Ala Asp His Lys Pro Ile Ser Val Val Phe Ile Ala
               660                 665                 670
   Glu Ala Lys Asp Trp Gln Val Leu Pro Tyr Leu Arg Ala Glu Thr Ser
           675                 680                 685
   Arg Thr Gln Arg Trp Leu Glu Lys His Asn Gln Met Phe Leu Pro Gln
       690                 695                 700
   Met Ala Gln Glu Phe Ile Asp Ala Ala Thr Val Asp Leu Asp Thr Ala
   705                 710                 715                 720
   Thr Ser Glu Met Asp Leu Asp Ala Leu Ala Leu Met Gly Ile His Leu
                   725                 730                 735
   Met Lys Ala Asp Gly Ala Lys Ala Arg Ile Gln Asp Val Leu Asn Ser
               740                 745                 750
   Asp Ser Lys Val Ser Asp Phe Ala Leu Leu Thr Ser Lys Asn Glu Ile
           755                 760                 765
   Asp Glu Ala Gln Thr Arg Leu Ile Glu Glu Gly Thr His Leu Arg Ile
       770                 775                 780
   Ile Leu Gly Asp Glu Asn Glu Ser Ile Pro Gly Gly Trp Lys His Gly
   785                 790                 795                 800
   Leu Ser Ser Leu Leu Lys Leu Lys Ala Ser Asp Arg Glu Ser Leu Arg
                   805                 810                 815
   Thr Ala Leu Leu Ala Ser Ile Pro Leu Leu Val Ser Glu Lys Gln Lys
               820                 825                 830
   Gln Leu Leu Tyr Gln His Asn Leu Val Pro Leu Ser Ser Ser Lys Thr
           835                 840                 845
   Val Leu Ala Gly Phe Tyr Phe Leu Pro Lys Ala Gln Asn Phe Tyr Ser
       850                 855                 860
   Lys Asn Leu Gly Phe Ile Trp Pro Glu Glu Lys Asp
   865                 870                 875
<210> 92
   <211> 1084
   <212> PRT
   <213> Corynebacterium diphtheriae
<220>
   <223> CRISPR-associated endonuclease
<308> YP_005124303
   <309> 2013-06-11
<400> 92
   Met Lys Tyr His Val Gly Ile Asp Val Gly Thr Phe Ser Val Gly Leu
    1               5                  10                  15
   Ala Ala Ile Glu Val Asp Asp Ala Gly Met Pro Ile Lys Thr Leu Ser
               20                  25                  30
   Leu Val Ser His Ile His Asp Ser Gly Leu Asp Pro Asp Lys Ile Lys
           35                  40                  45
   Ser Ala Val Thr Arg Leu Ala Ser Ser Gly Ile Ala Arg Arg Thr Arg
       50                  55                  60
   Arg Leu Tyr Arg Arg Lys Arg Arg Arg Leu Gln Gln Leu Asp Lys Phe
   65                  70                  75                  80
   Ile Gln Arg Gln Gly Trp Pro Val Ile Glu Leu Glu Asp Tyr Ser Asp
                   85                  90                  95
   Pro Leu Tyr Pro Trp Lys Val Arg Ala Glu Leu Ala Ala Ser Tyr Ile
   100 105 110
   Ala Asp Glu Lys Glu Arg Gly Glu Lys Leu Ser Val Ala Leu Arg His
   115 120 125
   Ile Ala Arg His Arg Gly Trp Arg Asn Pro Tyr Ala Lys Val Ser Ser
   130 135 140
   Leu Tyr Leu Pro Asp Glu Pro Ser Asp Ala Phe Lys Ala Ile Arg Glu
   145 150 155 160
   Glu Ile Lys Arg Ala Ser Gly Gln Pro Val Pro Glu Thr Ala Thr Val
   165 170 175
   Gly Gln Met Val Thr Leu Cys Glu Leu Gly Thr Leu Lys Leu Arg Gly
   180 185 190
   Glu Gly Gly Val Leu Ser Ala Arg Leu Gln Gln Ser Asp His Ala Arg
   195 200 205
   Glu Ile Gln Glu Ile Cys Arg Met Gln Glu Ile Gly Gln Glu Leu Tyr
   210 215 220
   Arg Lys Ile Ile Asp Val Val Phe Ala Ala Glu Ser Pro Lys Gly Ser
   225 230 235 240
   Ala Ser Ser Arg Val Gly Lys Asp Pro Leu Gln Pro Gly Lys Asn Arg
   245 250 255
   Ala Leu Lys Ala Ser Asp Ala Phe Gln Arg Tyr Arg Ile Ala Ala Leu
   260 265 270
   Ile Gly Asn Leu Arg Val Arg Val Asp Gly Glu Lys Arg Ile Leu Ser
   275 280 285
   Val Glu Glu Lys Asn Leu Val Phe Asp His Leu Val Asn Leu Ala Pro
   290 295 300
   Lys Lys Glu Pro Glu Trp Val Thr Ile Ala Glu Ile Leu Gly Ile Asp
   305 310 315 320
   Arg Gly Gln Leu Ile Gly Thr Ala Thr Met Thr Asp Asp Gly Glu Arg
   325 330 335
   Ala Gly Ala Arg Pro Pro Thr His Asp Thr Asn Arg Ser Ile Val Asn
   340 345 350
   Ser Arg Ile Ala Pro Leu Val Asp Trp Trp Lys Thr Ala Ser Ala Leu
   355 360 365
   Glu Gln His Ala Met Val Lys Ala Leu Ser Asn Ala Glu Val Asp Asp
   370 375 380
   Phe Asp Ser Pro Glu Gly Ala Lys Val Gln Ala Phe Phe Ala Asp Leu
   385 390 395 400
   Asp Asp Asp Val His Ala Lys Leu Asp Ser Leu His Leu Pro Val Gly
   405 410 415
   Arg Ala Ala Tyr Ser Glu Asp Thr Leu Val Arg Leu Thr Arg Arg Met
   420 425 430
   Leu Ala Asp Gly Val Asp Leu Tyr Thr Ala Arg Leu Gln Glu Phe Gly
   435 440 445
   Ile Glu Pro Ser Trp Thr Pro Pro Ala Pro Arg Ile Gly Glu Pro Val
   450 455 460
   Gly Asn Pro Ala Val Asp Arg Val Leu Lys Thr Val Ser Arg Trp Leu
   465 470 475 480
   Lys Ser Ala Thr Lys Thr Trp Gly Ala Pro Glu Arg Val Ile Ile Glu
   485 490 495
   His Val Arg Glu Gly Phe Val Thr Glu Lys Arg Ala Arg Glu Met Asp
   500 505 510
   Gly Asp Met Arg Arg Arg Ala Ala Arg Asn Ala Lys Leu Phe Gln Glu
   515 520 525
   Met Gln Glu Lys Leu Asn Val Gln Gly Lys Pro Ser Arg Ala Asp Leu
   530 535 540
   Trp Arg Tyr Gln Ser Val Gln Arg Gln Asn Cys Gln Cys Ala Tyr Cys
   545 550 555 560
   Gly Ser Pro Ile Thr Phe Ser Asn Ser Glu Met Asp His Ile Val Pro
   565 570 575
   Arg Ala Gly Gln Gly Ser Thr Asn Thr Arg Glu Asn Leu Val Ala Val
   580 585 590
   Cys His Arg Cys Asn Gln Ser Lys Gly Asn Thr Pro Phe Ala Ile Trp
           595                 600                 605
   Ala Lys Asn Thr Ser Ile Glu Gly Val Ser Val Lys Glu Ala Val Glu
       610                 615                 620
   Arg Thr Arg His Trp Val Thr Asp Thr Gly Met Arg Ser Thr Asp Phe
   625                 630                 635                 640
   Lys Lys Phe Thr Lys Ala Val Val Glu Arg Phe Gln Arg Ala Thr Met
                   645                 650                 655
   Asp Glu Glu Ile Asp Ala Arg Ser Met Glu Ser Val Ala Trp Met Ala
               660                 665                 670
   Asn Glu Leu Arg Ser Arg Val Ala Gln His Phe Ala Ser His Gly Thr
           675                 680                 685
   Thr Val Arg Val Tyr Arg Gly Ser Leu Thr Ala Glu Ala Arg Arg Ala
       690                 695                 700
   Ser Gly Ile Ser Gly Lys Leu Glu Phe Leu Asp Gly Val Gly Lys Ser
   705                 710                 715                 720
   Arg Leu Asp Arg Arg His His Ala Ile Asp Ala Ala Val Ile Ala Phe
                   725                 730                 735
   Thr Ser Asp Tyr Val Ala Glu Thr Leu Ala Val Arg Ser Asn Leu Lys
               740                 745                 750
   Gln Ser Gln Ala His Arg Gln Glu Ala Pro Gln Trp Arg Glu Phe Thr
           755                 760                 765
   Gly Lys Asp Ala Glu His Arg Ala Ala Trp Arg Val Trp Cys Gln Lys
       770                 775                 780
   Met Glu Lys Leu Ser Ala Leu Leu Thr Glu Asp Leu Arg Asp Asp Arg
   785                 790                 795                 800
   Val Val Val Met Ser Asn Val Arg Leu Arg Leu Gly Asn Gly Ser Ala
                   805                 810                 815
   His Glu Glu Thr Ile Gly Lys Leu Ser Lys Val Lys Leu Gly Ser Gln
               820                 825                 830
   Leu Ser Val Ser Asp Ile Asp Lys Ala Ser Ser Glu Ala Leu Trp Cys
           835                 840                 845
   Ala Leu Thr Arg Glu Pro Asp Phe Asp Pro Lys Asp Gly Leu Pro Ala
       850                 855                 860
   Asn Pro Glu Arg His Ile Arg Val Asn Gly Thr His Val Tyr Ala Gly
   865                 870                 875                 880
   Asp Asn Ile Gly Leu Phe Pro Val Ser Ala Gly Ser Ile Ala Leu Arg
                   885                 890                 895
   Gly Gly Tyr Ala Glu Leu Gly Ser Ser Phe His His Ala Arg Val Tyr
               900                 905                 910
   Lys Ile Thr Ser Gly Lys Lys Pro Ala Phe Ala Met Leu Arg Val Tyr
           915                 920                 925
   Thr Ile Asp Leu Leu Pro Tyr Arg Asn Gln Asp Leu Phe Ser Val Glu
       930                 935                 940
   Leu Lys Pro Gln Thr Met Ser Met Arg Gln Ala Glu Lys Lys Leu Arg
   945                 950                 955                 960
   Asp Ala Leu Ala Thr Gly Asn Ala Glu Tyr Leu Gly Trp Leu Val Val
                   965                 970                 975
   Asp Asp Glu Leu Val Val Asp Thr Ser Lys Ile Ala Thr Asp Gln Val
               980                 985                 990
   Lys Ala Val Glu Ala Glu Leu Gly Thr Ile Arg Arg Trp Arg Val Asp
           995                 1000                1005
   Gly Phe Phe Gly Asp Thr Arg Leu Arg Leu Arg Pro Leu Gln Met Ser
       1010                1015                1020
   Lys Glu Gly Ile Lys Lys Glu Ser Ala Pro Glu Leu Ser Lys Ile Ile
   1025                1030                1035                1040
   Asp Arg Pro Gly Trp Leu Pro Ala Val Asn Lys Leu Phe Ser Glu Gly
                   1045                1050                1055
   Asn Val Thr Val Val Arg Arg Asp Ser Leu Gly Arg Val Arg Leu Glu
               1060                1065                1070
   Ser Thr Ala His Leu Pro Val Thr Trp Lys Val Gln
           1075                1080
<210> 93
   <211> 1384
   <212> PRT
   <213> Coriobacterium glomerans
<220>
   <223> CRISPR-associated endonuclease
<308> YP_004373648
   <309> 2013-07-06
<400> 93
   Met Lys Leu Arg Gly Ile Glu Asp Asp Tyr Ser Ile Gly Leu Asp Met
   1               5                   10                  15
   Gly Thr Ser Ser Val Gly Trp Ala Val Thr Asp Glu Arg Gly Thr Leu
               20                  25                  30
   Ala His Phe Lys Arg Lys Pro Thr Trp Gly Ser Arg Leu Phe Arg Glu
           35                  40                  45
   Ala Gln Thr Ala Ala Val Ala Arg Met Pro Arg Gly Gln Arg Arg Arg
       50                  55                  60
   Tyr Val Arg Arg Arg Trp Arg Leu Asp Leu Leu Gln Lys Leu Phe Glu
   65                  70                  75                  80
   Gln Gln Met Glu Gln Ala Asp Pro Asp Phe Phe Ile Arg Leu Arg Gln
                   85                  90                  95
   Ser Arg Leu Leu Arg Asp Asp Arg Ala Glu Glu His Ala Asp Tyr Arg
               100                 105                 110
   Trp Pro Leu Phe Asn Asp Cys Lys Phe Thr Glu Arg Asp Tyr Tyr Gln
           115                 120                 125
   Arg Phe Pro Thr Ile Tyr His Val Arg Ser Trp Leu Met Glu Thr Asp
       130                 135                 140
   Glu Gln Ala Asp Ile Arg Leu Ile Tyr Leu Ala Leu His Asn Ile Val
   145                 150                 155                 160
   Lys His Arg Gly Asn Phe Leu Arg Glu Gly Gln Ser Leu Ser Ala Lys
                   165                 170                 175
   Ser Ala Arg Pro Asp Glu Ala Leu Asn His Leu Arg Glu Thr Leu Arg
               180                 185                 190
   Val Trp Ser Ser Glu Arg Gly Phe Glu Cys Ser Ile Ala Asp Asn Gly
           195                 200                 205
   Ser Ile Leu Ala Met Leu Thr His Pro Asp Leu Ser Pro Ser Asp Arg
       210                 215                 220
   Arg Lys Lys Ile Ala Pro Leu Phe Asp Val Lys Ser Asp Asp Ala Ala
   225                 230                 235                 240
   Ala Asp Lys Lys Leu Gly Ile Ala Leu Ala Gly Ala Val Ile Gly Leu
                   245                 250                 255
   Lys Thr Glu Phe Lys Asn Ile Phe Gly Asp Phe Pro Cys Glu Asp Ser
               260                 265                 270
   Ser Ile Tyr Leu Ser Asn Asp Glu Ala Val Asp Ala Val Arg Ser Ala
           275                 280                 285
   Cys Pro Asp Asp Cys Ala Glu Leu Phe Asp Arg Leu Cys Glu Val Tyr
       290                 295                 300
   Ser Ala Tyr Val Leu Gln Gly Leu Leu Ser Tyr Ala Pro Gly Gln Thr
   305                 310                 315                 320
   Ile Ser Ala Asn Met Val Glu Lys Tyr Arg Arg Tyr Gly Glu Asp Leu
                   325                 330                 335
   Ala Leu Leu Lys Lys Leu Val Lys Ile Tyr Ala Pro Asp Gln Tyr Arg
               340                 345                 350
   Met Phe Phe Ser Gly Ala Thr Tyr Pro Gly Thr Gly Ile Tyr Asp Ala
           355                 360                 365
   Ala Gln Ala Arg Gly Tyr Thr Lys Tyr Asn Leu Gly Pro Lys Lys Ser
       370                 375                 380
   Glu Tyr Lys Pro Ser Glu Ser Met Gln Tyr Asp Asp Phe Arg Lys Ala
   385                 390                 395                 400
   Val Glu Lys Leu Phe Ala Lys Thr Asp Ala Arg Ala Asp Glu Arg Tyr
                   405                 410                 415
   Arg Met Met Met Asp Arg Phe Asp Lys Gln Gln Phe Leu Arg Arg Leu
               420                 425                 430
   Lys Thr Ser Asp Asn Gly Ser Ile Tyr His Gln Leu His Leu Glu Glu
           435                 440                 445
   Leu Lys Ala Ile Val Glu Asn Gln Gly Arg Phe Tyr Pro Phe Leu Lys
       450                 455                 460
   Arg Asp Ala Asp Lys Leu Val Ser Leu Val Ser Phe Arg Ile Pro Tyr
   465                 470                 475                 480
   Tyr Val Gly Pro Leu Ser Thr Arg Asn Ala Arg Thr Asp Gln His Gly
                   485                 490                 495
   Glu Asn Arg Phe Ala Trp Ser Glu Arg Lys Pro Gly Met Gln Asp Glu
               500                 505                 510
   Pro Ile Phe Pro Trp Asn Trp Glu Ser Ile Ile Asp Arg Ser Lys Ser
           515                 520                 525
   Ala Glu Lys Phe Ile Leu Arg Met Thr Gly Met Cys Thr Tyr Leu Gln
       530                 535                 540
   Gln Glu Pro Val Leu Pro Lys Ser Ser Leu Leu Tyr Glu Glu Phe Cys
   545                 550                 555                 560
   Val Leu Asn Glu Leu Asn Gly Ala His Trp Ser Ile Asp Gly Asp Asp
                   565                 570                 575
   Glu His Arg Phe Asp Ala Ala Asp Arg Glu Gly Ile Ile Glu Glu Leu
               580                 585                 590
   Phe Arg Arg Lys Arg Thr Val Ser Tyr Gly Asp Val Ala Gly Trp Met
           595                 600                 605
   Glu Arg Glu Arg Asn Gln Ile Gly Ala His Val Cys Gly Gly Gln Gly
       610                 615                 620
   Glu Lys Gly Phe Glu Ser Lys Leu Gly Ser Tyr Ile Phe Phe Cys Lys
   625                 630                 635                 640
   Asp Val Phe Lys Val Glu Arg Leu Glu Gln Ser Asp Tyr Pro Met Ile
                   645                 650                 655
   Glu Arg Ile Ile Leu Trp Asn Thr Leu Phe Glu Asp Arg Lys Ile Leu
               660                 665                 670
   Ser Gln Arg Leu Lys Glu Glu Tyr Gly Ser Arg Leu Ser Ala Glu Gln
           675                 680                 685
   Ile Lys Thr Ile Cys Lys Lys Arg Phe Thr Gly Trp Gly Arg Leu Ser
       690                 695                 700
   Glu Lys Phe Leu Thr Gly Ile Thr Val Gln Val Asp Glu Asp Ser Val
   705                 710                 715                 720
   Ser Ile Met Asp Val Leu Arg Glu Gly Cys Pro Val Ser Gly Lys Arg
                   725                 730                 735
   Gly Arg Ala Met Val Met Met Glu Ile Leu Arg Asp Glu Glu Leu Gly
               740                 745                 750
   Phe Gln Lys Lys Val Asp Asp Phe Asn Arg Ala Phe Phe Ala Glu Asn
           755                 760                 765
   Ala Gln Ala Leu Gly Val Asn Glu Leu Pro Gly Ser Pro Ala Val Arg
       770                 775                 780
   Arg Ser Leu Asn Gln Ser Ile Arg Ile Val Asp Glu Ile Ala Ser Ile
   785                 790                 795                 800
   Ala Gly Lys Ala Pro Ala Asn Ile Phe Ile Glu Val Thr Arg Asp Glu
                   805                 810                 815
   Asp Pro Lys Lys Lys Gly Arg Arg Thr Lys Arg Arg Tyr Asn Asp Leu
               820                 825                 830
   Lys Asp Ala Leu Glu Ala Phe Lys Lys Glu Asp Pro Glu Leu Trp Arg
           835                 840                 845
   Glu Leu Cys Glu Thr Ala Pro Asn Asp Met Asp Glu Arg Leu Ser Leu
       850                 855                 860
   Tyr Phe Met Gln Arg Gly Lys Cys Leu Tyr Ser Gly Arg Ala Ile Asp
   865                 870                 875                 880
   Ile His Gln Leu Ser Asn Ala Gly Ile Tyr Glu Val Asp His Ile Ile
                   885                 890                 895
   Pro Arg Thr Tyr Val Lys Asp Asp Ser Leu Glu Asn Lys Ala Leu Val
               900                 905                 910
   Tyr Arg Glu Glu Asn Gln Arg Lys Thr Asp Met Leu Leu Ile Asp Pro
   915 920 925
   Glu Ile Arg Arg Arg Met Ser Gly Tyr Trp Arg Met Leu His Glu Ala
   930 935 940
   Lys Leu Ile Gly Asp Lys Lys Phe Arg Asn Leu Leu Arg Ser Arg Ile
   945 950 955 960
   Asp Asp Lys Ala Leu Lys Gly Phe Ile Ala Arg Gln Leu Val Glu Thr
   965 970 975
   Gly Gln Met Val Lys Leu Val Arg Ser Leu Leu Glu Ala Arg Tyr Pro
   980 985 990
   Glu Thr Asn Ile Ile Ser Val Lys Ala Ser Ile Ser His Asp Leu Arg
   995 1000 1005
   Thr Ala Ala Glu Leu Val Lys Cys Arg Glu Ala Asn Asp Phe His His
   1010 1015 1020
   Ala His Asp Ala Phe Leu Ala Cys Arg Val Gly Leu Phe Ile Gln Lys
   1025 1030 1035 1040
   Arg His Pro Cys Val Tyr Glu Asn Pro Ile Gly Leu Ser Gln Val Val
   1045 1050 1055
   Arg Asn Tyr Val Arg Gln Gln Ala Asp Ile Phe Lys Arg Cys Arg Thr
   1060 1065 1070
   Ile Pro Gly Ser Ser Gly Phe Ile Val Asn Ser Phe Met Thr Ser Gly
   1075 1080 1085
   Phe Asp Lys Glu Thr Gly Glu Ile Phe Lys Asp Asp Trp Asp Ala Glu
   1090 1095 1100
   Ala Glu Val Glu Gly Ile Arg Arg Ser Leu Asn Phe Arg Gln Cys Phe
   1105 1110 1115 1120
   Ile Ser Arg Met Pro Phe Glu Asp His Gly Val Phe Trp Asp Ala Thr
   1125 1130 1135
   Ile Tyr Ser Pro Arg Ala Lys Lys Thr Ala Ala Leu Pro Leu Lys Gln
   1140 1145 1150
   Gly Leu Asn Pro Ser Arg Tyr Gly Ser Phe Ser Arg Glu Gln Phe Ala
   1155 1160 1165
   Tyr Phe Phe Ile Tyr Lys Ala Arg Asn Pro Arg Lys Glu Gln Thr Leu
   1170 1175 1180
   Phe Glu Phe Ala Gln Val Pro Val Arg Leu Ser Ala Gln Ile Arg Gln
   1185 1190 1195 1200
   Asp Glu Asn Ala Leu Glu Arg Tyr Ala Arg Glu Leu Ala Lys Asp Gln
   1205 1210 1215
   Gly Leu Glu Phe Ile Arg Ile Glu Arg Ser Lys Ile Leu Lys Asn Gln
   1220 1225 1230
   Leu Ile Glu Ile Asp Gly Asp Arg Leu Cys Ile Thr Gly Lys Glu Glu
   1235 1240 1245
   Val Arg Asn Ala Cys Glu Leu Ala Phe Ala Gln Asp Glu Met Arg Val
   1250 1255 1260
   Ile Arg Met Leu Val Ser Glu Lys Pro Val Ser Arg Glu Cys Val Ile
   1265 1270 1275 1280
   Ser Leu Phe Asn Arg Ile Leu Leu His Gly Asp Gln Ala Ser Arg Arg
   1285 1290 1295
   Leu Ser Lys Gln Leu Lys Leu Ala Leu Leu Ser Glu Ala Phe Ser Glu
   1300 1305 1310
   Ala Ser Asp Asn Val Gln Arg Asn Val Val Leu Gly Leu Ile Ala Ile
   1315 1320 1325
   Phe Asn Gly Ser Thr Asn Met Val Asn Leu Ser Asp Ile Gly Gly Ser
   1330 1335 1340
   Lys Phe Ala Gly Asn Val Arg Ile Lys Tyr Lys Lys Glu Leu Ala Ser
   1345 1350 1355 1360
   Pro Lys Val Asn Val His Leu Ile Asp Gln Ser Val Thr Gly Met Phe
   1365 1370 1375
   Glu Arg Arg Thr Lys Ile Gly Leu
   1380
<210> 94
   <211> 1458
   <212> PRT
   <213> Fluviicola taffensis
<220>
   <223> CRISPR-associated endonuclease
<308> YP_004345959
   <309> 2013-07-06
<400> 94
   Met Thr Lys Thr Ile Leu Gly Leu Asp Leu Gly Thr Asn Ser Ile Gly
    1               5                  10                  15
   Trp Ala Leu Ile Asn Gln Asp Phe Asp Asn Lys Lys Gly Glu Ile Leu
               20                  25                  30
   Gly Met Gly Ser Arg Ile Ile Pro Met Thr Gln Asp Ile Lys Asp Glu
           35                  40                  45
   Phe Gly Lys Gly Asn Ser Ile Ser Gln Thr Ala Glu Arg Thr Arg Leu
       50                  55                  60
   Arg Gly Val Arg Arg Leu Ile His Arg Thr Leu Leu Arg Arg Glu Arg
   65                  70                  75                  80
   Leu His Arg Val Leu Asn Ile Ile Gly Phe Leu Pro Glu His Tyr Ala
                   85                  90                  95
   Asn Gln Ile Asp Phe Glu Lys Arg Phe Gly Lys Phe Lys Pro Glu Ser
               100                 105                 110
   Glu Pro Lys Ile Ser Phe Asp Gly Asn Asp Val Phe Leu Phe Glu Lys
           115                 120                 125
   Ser Tyr Gln Glu Met Leu Val Asp Phe Lys Ile His Gln Pro Gln Leu
       130                 135                 140
   Val Ser Asn Gly Lys Lys Ile Pro His Asp Trp Thr Ile Tyr Tyr Leu
   145                 150                 155                 160
   Arg Lys Lys Ala Leu Ser Lys Lys Ile Glu Lys Glu Glu Leu Ala Trp
                   165                 170                 175
   Ile Leu Leu Asn Phe Asn Gln Lys Arg Gly Tyr Tyr Gln Leu Arg Gly
               180                 185                 190
   Glu Glu Glu Glu Glu Thr Pro Asn Lys Leu Val Glu Phe His Ser Leu
           195                 200                 205
   Lys Ile Val Glu Val Asn Ala Asp Glu Pro Gln Lys Gly Lys Pro Glu
       210                 215                 220
   Ile Trp Tyr Ser Leu Val Leu Glu Asn Gly Trp Val Tyr Arg Arg Ala
   225                 230                 235                 240
   Ser Lys Thr Pro Leu Phe Asp Trp Lys Asp Lys Ile Arg Glu Phe Ile
                   245                 250                 255
   Val Thr Thr Glu Ile Asn Asn Asp Gly Thr Val Lys Thr Asp Lys Glu
               260                 265                 270
   Gly Thr Glu Lys Arg Ser Phe Arg Ala Pro Lys Pro Glu Asp Trp Thr
           275                 280                 285
   Leu Gln Lys Lys Lys Thr Glu Phe Glu Leu Ser Lys Ser Gly Asn Glu
       290                 295                 300
   Val Gly Ala Phe Ile Tyr Glu Ser Ile Leu Gln Lys Pro Asn Gln Lys
   305                 310                 315                 320
   Ile Arg Gly Lys Leu Ile Ser Thr Ile Glu Arg Lys Phe Tyr Lys Glu
                   325                 330                 335
   Glu Leu Lys Thr Ile Leu Lys Thr Gln Leu Phe Phe His Lys Glu Leu
               340                 345                 350
   Lys Asp Glu Lys Leu Tyr Asn Ala Cys Ile Glu Glu Leu Tyr Lys Asn
           355                 360                 365
   Asn Glu Ala His Arg Ser Leu Leu Ser Asn Lys Gly Phe Glu His Leu
       370                 375                 380
   Phe Ile Asn Asp Ile Leu Phe Tyr Gln Arg Pro Leu Arg Ser Lys Lys
   385                 390                 395                 400
   Ser Gln Ile Ser Asn Cys Pro Leu Glu Lys Arg Thr Tyr Lys Lys Glu
                   405                 410                 415
   Gly Ile Glu Ile Thr Glu Gly Ile Lys Val Ile Ser Lys Ser Asn Pro
               420                 425                 430
   Ile Tyr Gln Glu Phe Arg Leu Trp Gln Trp Ile Ser Asn Leu Ser Leu
           435                 440                 445
   Tyr Cys Ile Glu Pro Thr Glu Thr Asn Val Thr Ser Thr Phe Leu Asn
       450                 455                 460
   Ser Ile Glu Asp Tyr Glu Asn Leu Phe Glu Phe Leu Asn Asn Arg Lys
   465                 470                 475                 480
   Glu Ile Glu Gln Lys His Leu Leu Lys Tyr Leu Leu Glu Asn Gln Gly
                   485                 490                 495
   Phe Lys Gly Lys Leu Leu Thr Asn Glu Leu Glu Lys Phe Arg Trp Asn
               500                 505                 510
   Phe Val Ala Asp Lys Lys Tyr Pro Cys Asn Glu Thr Gly Ser Leu Leu
           515                 520                 525
   His Thr Arg Leu Ser Lys Val Lys Asn Ile Ser Pro Asp Phe Leu Thr
       530                 535                 540
   Lys Glu Ile Glu His Gln Leu Trp His Ile Ile Tyr Ser Val Thr Asp
   545                 550                 555                 560
   Lys Ile Glu Tyr Glu Gln Ala Leu Lys Thr Phe Ala Arg Lys Asn Asn
                   565                 570                 575
   Leu Asp Val Asp Ser Phe Phe Glu His Phe Lys Lys Ile Pro Pro Phe
               580                 585                 590
   Glu Ser Thr Tyr Gly Ala Tyr Ser Glu Lys Ala Ile Lys Lys Leu Leu
           595                 600                 605
   Pro Leu Ile Arg Leu Gly Lys Tyr Trp Asn Trp Glu Ala Ile Asp Ser
       610                 615                 620
   Ile Ser Lys Asp Arg Ile Ser Lys Ile Leu Ser Gly Glu Tyr Asp Glu
   625                 630                 635                 640
   Asn Ile Lys Asn Arg Val Arg Glu Lys Ala Val His Leu Thr Ser Glu
                   645                 650                 655
   Asn Asn Phe Gln Gly Leu Gln Glu Trp Leu Ala Lys Tyr Ile Val Tyr
               660                 665                 670
   Asp Arg His Ser Glu Gly Asn Asp Leu Gly Lys Trp Thr Ser Val Ser
           675                 680                 685
   Asp Leu Glu Thr Tyr Leu Lys Glu Phe Lys Gln His Ser Leu Arg Asn
       690                 695                 700
   Pro Ile Val Glu Gln Val Ile Thr Glu Thr Leu Arg Val Val Lys Asp
   705                 710                 715                 720
   Ile Trp Ile Lys His Gly Lys Gly Thr Glu Asn Phe Phe Asp Glu Ile
                   725                 730                 735
   His Val Glu Leu Gly Arg Glu Met Lys Asn Asn Ser Glu Asp Arg Lys
               740                 745                 750
   Arg Leu Thr Asn Thr Ile Thr Glu Asn Glu Asn Thr Asn Leu Arg Ile
           755                 760                 765
   Lys Ala Leu Leu Met Glu Met Met Asn Asp Asn Asp Val Glu Asn Val
       770                 775                 780
   Arg Pro Tyr Ser Pro Ser Gln Gln Glu Ile Leu Lys Ile Tyr Glu Asp
   785                 790                 795                 800
   Gly Ala Leu Asn Ser Asn Ile Glu Leu Asp Asp Glu Ile Val Lys Ile
                   805                 810                 815
   Ser Lys Lys Ala Glu Pro Thr Lys Ser Glu Leu Gln Arg Tyr Lys Leu
               820                 825                 830
   Trp Leu Glu Gln Lys Tyr Arg Ser Pro Tyr Thr Gly Gln Val Ile Pro
           835                 840                 845
   Leu Asn Lys Leu Phe Thr Ser Glu Tyr Glu Ile Glu His Val Val Pro
       850                 855                 860
   Gln Ser Arg Phe Phe Asp Asp Ser Phe Ser Asn Lys Val Ile Cys Glu
   865                 870                 875                 880
   Ser Ala Val Asn Lys Arg Lys Asp Asn Gln Leu Gly Leu Gln Phe Ile
                   885                 890                 895
   Lys Asn His Ser Gly Glu Lys Val Glu Leu Gly Phe Gly Lys Val Val
               900                 905                 910
   Gln Val Phe Thr Glu Glu Gln Tyr Leu Asp Phe Val Lys Glu His Tyr
   915 920 925
   Ser Lys Asn Arg Ser Lys His Asn Lys Leu Leu Leu Glu Glu Ile Pro
   930 935 940
   Glu Lys Met Ile Glu Arg Gln Leu Asn Asp Thr Arg Tyr Ile Ser Lys
   945 950 955 960
   Phe Val Ser Ser Ile Leu Ser Asn Ile Val Arg Ser Glu Lys Asp Asp
   965 970 975
   Asp Gly Leu Asn Ser Lys Asn Ile Leu Pro Gly Asn Gly Lys Ile Thr
   980 985 990
   Thr Glu Leu Lys Arg Asp Trp Gly Leu Asn Asp Val Trp Asn Asp Leu
   995 1000 1005
   Ile Leu Pro Arg Phe Glu Arg Met Asn Thr Ile Thr Asn Ser Asp Leu
   1010 1015 1020
   Phe Thr Thr Tyr Asn Asp Lys Tyr Gln Lys His Leu Pro Thr Val Pro
   1025 1030 1035 1040
   Phe Glu Tyr Ser Lys Gly Phe Gln Lys Lys Arg Ile Asp His Arg His
   1045 1050 1055
   His Ala Met Asp Ala Leu Val Ile Ala Cys Ala Thr Arg Asp His Leu
   1060 1065 1070
   Asn Leu Met Asn Asn Gln Ser Ala Lys Ser Glu Leu Lys Arg Tyr Asp
   1075 1080 1085
   Leu Gln Asn Lys Leu Arg Lys Lys Glu Pro Tyr Phe Asn Gln Lys Glu
   1090 1095 1100
   Asn Lys Gln Lys Glu Ala Phe Lys Asp Phe Ile Lys Pro Trp Gly Thr
   1105 1110 1115 1120
   Phe Thr Glu Asp Ser Lys Asn Ala Leu Glu Lys Ile Ile Ile Ser Phe
   1125 1130 1135
   Lys Gln Asn Leu Arg Val Ile Asn Lys Ala Thr Asn Ser Tyr Glu Ser
   1140 1145 1150
   Tyr Lys Asp Glu Asp Gly Asn Leu Arg Ile Gly Lys Asp Gly Lys Pro
   1155 1160 1165
   Glu Lys Gly Leu Ile Lys Gln Lys Gly Leu Asn Trp Ala Ile Arg Lys
   1170 1175 1180
   Pro Leu His Lys Asp Thr Val Ser Gly Gln Ile Asn Leu Ser Arg Ile
   1185 1190 1195 1200
   Lys Leu Pro Asn Gly Lys Ile Leu Thr Ala Thr Arg Lys Asn Leu Asp
   1205 1210 1215
   Thr Ser Phe Asp Leu Lys Thr Ile Glu Asn Ser Ile Thr Asp Thr Gly
   1220 1225 1230
   Ile Gln Lys Ile Leu Lys Asn Tyr Leu Leu Ser Lys Glu Ser Pro Glu
   1235 1240 1245
   Leu Ala Phe Ser Pro Glu Gly Leu Glu Glu Met Asn Asn Glu Ile Glu
   1250 1255 1260
   Lys Tyr Asn Asn Gly Lys Phe His His Pro Ile Asn Lys Ala Arg Ile
   1265 1270 1275 1280
   Tyr Glu Leu Gly Ser Lys Phe Asn Val Gly Gln Thr Gly Asn Arg Arg
   1285 1290 1295
   Asp Lys Phe Val Glu Ala Ala Lys Gly Thr Asn Leu Phe Phe Ala Ile
   1300 1305 1310
   Tyr Gln Asp Glu Asn Lys Asn Arg Ser Tyr Glu Thr Ile Pro Leu Asn
   1315 1320 1325
   Glu Val Ile Glu His Gln Lys Trp Arg Ala Ser Leu Pro Lys Glu Glu
   1330 1335 1340
   Gln Glu Lys Ile Pro Leu Val Pro Val Asn Lys Leu Lys Gly Thr Phe
   1345 1350 1355 1360
   Ile Phe Ser Leu Ser Pro Asn Asp Leu Val Tyr Val Pro Ser Asn Asp
   1365 1370 1375
   Glu Leu Glu Arg Ser Ala Ser Ile Asp Phe Ser Lys Leu Lys Lys Glu
   1380 1385 1390
   Gln Ile Asn Arg Leu Tyr Lys Met Val Ser Ser Ser Gly Ser Gln Cys
   1395 1400 1405
   Phe Phe Val Lys Ser Glu Val Ala Thr Ser Val Val Asn Lys Met Glu
   1410 1415 1420
   Tyr Ser Ser Leu Asn Lys Met Glu Lys Ser Ile Asp Asn Leu Met Val
   1425                1430                1435                1440
   Lys Glu Ile Cys Ile Lys Leu Lys Ile Asp Arg Leu Gly Asn Ile Ser
                   1445                1450                1455
   Lys Ala
<210> 95
   <211> 1045
   <212> PRT
   <213> Acidovorax avenae
<220>
   <223> CRISPR-associated endonuclease
<308> YP_004232757
   <309> 2013-07-06
<400> 95
   Met Gln Lys Glu Lys Pro Leu Ala Tyr Arg Leu Ala Leu Asp Leu Gly
    1               5                   10                  15
   Ser Thr Ser Leu Gly Trp Ala Val Leu Arg Leu Ser Ala Pro Gly Glu
               20                  25                  30
   Pro Ser Ala Val Ile Arg Ala Gly Ser Arg Ile Phe Ser Asp Gly Arg
           35                  40                  45
   Asn Pro Lys Asp Gly Ser Ser Leu Ala Val Thr Arg Arg Ala Ala Arg
       50                  55                  60
   Ala Met Arg Arg Arg Arg Asp Arg Leu Leu Lys Arg Lys Ala Arg Met
   65                  70                  75                  80
   Leu Ala Ala Leu Glu Arg His Gly Phe Phe Pro Gln Asp Glu Ala Ala
                   85                  90                  95
   Arg Lys Ala Leu Glu Lys Leu Asn Pro Tyr Gly Leu Arg Ala Arg Gly
               100                 105                 110
   Leu Glu Ser Ala Leu Lys Pro Glu Glu Phe Gly Arg Ala Leu Phe His
           115                 120                 125
   Leu Asn Gln Arg Arg Gly Phe Gln Ser Asn Arg Lys Thr Asp Arg Lys
       130                 135                 140
   Asp Ser Asp Ser Gly Ala Leu Lys Gln Ala Ile Gly Thr Leu Arg Ala
   145                 150                 155                 160
   Gln Met His Glu Ser Gly Cys Arg Thr Val Gly Glu Trp Phe Trp Lys
                   165                 170                 175
   Val Arg Met Glu Arg Lys Pro Glu Gly Val Lys Gly Gln Gly Val Arg
               180                 185                 190
   Ala Arg Tyr Arg Glu Thr Pro Phe Thr Thr Asp Glu Gly Lys Lys Arg
           195                 200                 205
   Ile Asp Lys Arg Tyr Asp Leu Tyr Val Asp Arg Ala Met Val Ala Gln
       210                 215                 220
   Glu Phe Asp Ala Leu Trp Ala Ala Gln Ala Thr Phe Ala Pro Ala Leu
   225                 230                 235                 240
   Phe Asn Glu Ala Ala Arg Ala Glu Leu Arg Asp Ile Leu Leu His Gln
                   245                 250                 255
   Arg Lys Leu Arg Pro Val Gln Pro Gly Arg Cys Thr Leu Val Pro Glu
               260                 265                 270
   Glu Pro Arg Ala Leu Leu Ala Leu Pro Ser Thr Gln Arg Phe Arg Ile
           275                 280                 285
   Tyr Gln Glu Val Asn His Leu Arg Val Leu His Asp Glu Leu Arg Glu
       290                 295                 300
   Glu Ala Leu Thr Leu Ala Gln Arg Asp Gln Ile Val Ala Leu Leu Glu
   305                 310                 315                 320
   Arg Gln Ala Lys Val Thr Phe Thr Ala Met Arg Lys Ala Leu Gly Phe
                   325                 330                 335
   Ser Ala Leu Phe Asn Leu Glu Asp Ala Lys Arg Asp Gly Leu Lys Gly
               340                 345                 350
   Asn Ala Thr Thr Ala Ala Leu Gly Lys Lys Asp Leu Phe Gly Pro Leu
           355                 360                 365
   Trp Ala Gly Phe Asp Thr Ala Leu Gln Asp Glu Ile Val Thr Gln Leu
       370                 375                 380
   Met Thr Glu Glu Ser Glu Pro Arg Leu Ile Ala Trp Leu Gln Glu His
   385                 390                 395                 400
   Thr Gly Val Asp Glu Ala Arg Ala Gln Ala Ile Ala Asn Ala Gly Leu
                   405                 410                 415
   Pro Glu Gly Tyr Gly Ser Leu Gly Arg Thr Ala Leu Ala Arg Ile Val
               420                 425                 430
   Pro Glu Leu Gln Arg Asp Val Ile Thr Tyr Asp Lys Ala Val Gln Ala
           435                 440                 445
   Ala Gly Phe Ala His His Ser Asp Leu Gly Phe Ser Phe Glu His Asp
       450                 455                 460
   Ala Asp Glu Val Glu Arg Ile Gly Glu Arg Thr Ile Ala Ser Thr Gly
   465                 470                 475                 480
   Glu Ile Lys Pro Val Phe Ala Phe Lys Gln Leu Pro Tyr Tyr Gly Arg
                   485                 490                 495
   Ala Leu Gln Arg His Val Ala Phe Ala Lys Glu Asn Pro Arg Asn Asp
               500                 505                 510
   Glu Glu Arg Tyr Gly Lys Ile Ala Asn Pro Thr Val His Ile Gly Leu
           515                 520                 525
   Asn Gln Val Arg Val Val Val Asn Ala Leu Met Cys Arg Tyr Gly Arg
       530                 535                 540
   Pro Thr Glu Val Val Ile Glu Leu Ala Arg Asp Leu Lys Gln Ser Arg
   545                 550                 555                 560
   Glu Gln Lys Leu Glu Ala Gln Gln Arg Gln Ala Asp Asn Gln Lys Arg
                   565                 570                 575
   Asn Ala Arg Ile Arg Arg Ser Ile Ala Glu Val Leu Gly Ile Ser Glu
               580                 585                 590
   Glu Arg Val Arg Thr Leu Asp Ile Gln Lys Trp Ile Leu Trp Glu Glu
           595                 600                 605
   Leu Ser His Asp Ala Ala Asp Arg Arg Cys Pro Tyr Ser Gly Val Gln
       610                 615                 620
   Ile Ser Ala Ser Met Leu Leu Gly Glu Arg Val Glu Ile Glu His Ile
   625                 630                 635                 640
   Leu Pro Phe Ser Met Thr Leu Asp Asp Ser Leu Asn Asn Arg Thr Val
                   645                 650                 655
   Ala Leu Arg Glu Ala Asn Arg Ile Lys Arg Asn Arg Thr Pro Trp Asp
               660                 665                 670
   Ala Arg Thr Asp Phe Glu Ala His Gly Trp Ser Tyr Asp Gly Ile Leu
           675                 680                 685
   Gln Arg Ala Glu Arg Met Pro Gln Arg Lys Arg His Arg Phe Ala Pro
       690                 695                 700
   Asp Gly Tyr Glu Arg Trp Leu Gly Glu Asp Lys Asp Phe Leu Ala Arg
   705                 710                 715                 720
   Ala Leu Asn Asp Thr Arg Tyr Leu Ser Arg Val Ala Ala Glu Tyr Leu
                   725                 730                 735
   Arg Leu Val Cys Pro Gly Ser Ala Thr Arg Val Ile Pro Gly Gln Met
               740                 745                 750
   Thr Ala Leu Leu Arg Gly Lys Phe Gly Leu Asn Asp Val Leu Gly Leu
           755                 760                 765
   Asp Gly Glu Lys Asn Arg Asn Asp His Arg His His Ala Val Asp Ala
       770                 775                 780
   Cys Val Ile Gly Val Thr Asp Gln Arg Met Met Gln Arg Phe Ala Gln
   785                 790                 795                 800
   Ala Asn Ala Gln Ala Arg Glu Asp Gly Leu Thr Arg Leu Val Glu Asp
                   805                 810                 815
   Met Pro Leu Pro Trp Pro Thr Tyr Arg Ala His Val Glu Arg Ala Val
               820                 825                 830
   Arg His Ile Trp Val Ser His Lys Pro Asp His Gly Phe Glu Gly Ala
           835                 840                 845
   Met Met Glu Glu Thr Ser Tyr Gly Ile Leu Arg Asp Gly Thr Ile Lys
       850                 855                 860
   Gln Arg Arg Lys Ala Asp Gly Ser Ala Gly Arg Glu Ile Ser Arg Leu
   865                 870                 875                 880
   Ile Arg Ile Thr Glu Pro Ser Gln Pro Gln Arg His Gly Val Asp Ala
                   885                 890                 895
   Glu Gly Arg Pro Leu Pro Tyr Lys Gly Tyr Val Gly Gly Ser Asn Tyr
               900                 905                 910
   Cys Leu Glu Ile Thr Val Asn Asp Lys Gly Lys Trp Glu Gly Gln Val
           915                 920                 925
   Ile Ser Thr Phe Asp Ala Tyr Arg Ile Val Arg Glu Gly Gly Leu Arg
       930                 935                 940
   Gln Leu Arg His Ser Thr Gln Gly Gln Asn Gly Gln Ala Leu Ala Met
   945                 950                 955                 960
   Arg Leu Val Ile Gly Asp Ser Val Arg Leu Glu Val Asp Glu Gln Glu
                   965                 970                 975
   Arg Val Met Arg Ile Val Lys Ile Asn Gly Asn Asn Gly Gln Val Phe
               980                 985                 990
   Met Ala Pro Val His Glu Ala Asn Val Asp Ala Arg Asn Ser Asp Lys
           995                 1000                1005
   Asn Glu Ala Phe Ala Tyr Thr Ser Lys Ile Ala Gly Ser Phe Gln Lys
       1010                1015                1020
   Thr Lys Ala Arg Gln Val Thr Ile Ser Pro Ile Gly Glu Leu Arg Asp
   1025                1030                1035                1040
   Pro Gly Phe Lys Gly
                   1045
<210> 96
   <211> 1044
   <212> PRT
   <213> Nitrosomonas sp.
<220>
   <223> CRISPR-associated endonuclease
<308> YP_004295898
   <309> 2013-07-06
<400> 96
   Met Leu His Lys Met Arg Tyr Arg Leu Ala Leu Asp Leu Gly Ser Thr
    1               5                  10                  15
   Ser Leu Gly Trp Ala Met Val Arg Leu Asn Ala Asn Gln Gln Pro Cys
               20                  25                  30
   Ala Val Ile Lys Ala Gly Val Arg Ile Phe Ser Asn Gly Arg Asn Pro
           35                  40                  45
   Lys Asp Gly Ser Ser Leu Ala Val Thr Arg Arg Glu Ala Arg Ser Met
       50                  55                  60
   Arg Arg Arg Arg Asp Arg Leu Leu Lys Arg Lys Ala Arg Met Met Arg
   65                  70                  75                  80
   Thr Leu Ile Glu Tyr Gly Phe Phe Pro Ala Asn Glu Thr Gln Arg Lys
                   85                  90                  95
   Ala Leu Glu Asn Leu Asn Pro Tyr Ala Leu Arg Ala Lys Gly Leu Asp
               100                 105                 110
   Glu Ala Leu Thr Pro Ser Glu Phe Ala Arg Ala Leu Phe His Ile Asn
           115                 120                 125
   Gln Arg Arg Gly Phe Lys Ser Asn Arg Lys Thr Asp Lys Lys Asp Ser
       130                 135                 140
   Asp Ser Gly Ala Leu Lys Thr Ala Ile Lys Gln Leu His Ser Val Leu
   145                 150                 155                 160
   Asp Pro Gln Gly Asn Asp Gly Lys Pro Arg Thr Val Gly Glu Leu Leu
                   165                 170                 175
   Tyr Lys Arg Phe Thr Asp Leu Ser Lys Leu Pro Lys Asp Arg Thr Val
               180                 185                 190
   Arg Ala Arg Tyr Arg Gln Asp Lys Ile Val Lys Asp Asp Gly Lys Thr
           195                 200                 205
   Lys Ile Asp Lys Tyr Tyr Asp Leu Tyr Ile Asp Arg Ala Met Ile Glu
       210                 215                 220
   Gln Glu Phe Asp Ala Leu Trp Lys Lys Gln Ser Glu Leu Asn Pro Ile
   225                 230                 235                 240
   Leu Phe Thr Glu Asn Ala Arg Ala Asp Leu Lys Asp Val Leu Leu Tyr
                   245                 250                 255
   Gln Arg Ser Leu Lys Pro Val Lys Pro Gly Arg Cys Thr Phe Met Ser
               260                 265                 270
   Glu Glu Glu Arg Ala Pro Leu Ala Leu Pro Ser Thr Gln Arg Phe Arg
           275                 280                 285
   Met Tyr Gln Glu Val Asn Asn Leu Arg Ile Leu Arg Glu Gly Leu Lys
       290                 295                 300
   Glu Glu Ser Leu Thr Leu Gln Gln Arg Asp Asp Leu Ile Asn Ala Leu
   305                 310                 315                 320
   Glu Lys Asn Asn Arg Arg Thr Phe Thr Gln Ile Lys Lys Leu Leu Gly
                   325                 330                 335
   Val Gly Gly Ser Val Gln Phe Asn Phe Glu Asp Pro Lys Arg Glu Glu
               340                 345                 350
   Leu Lys Gly Asn Thr Thr Ser Ala Ile Leu Gly Lys Ser Glu His Phe
           355                 360                 365
   Gly Glu Ala Trp Phe Ala Phe Asn Glu Ala Lys Gln Asp Ala Ile Val
       370                 375                 380
   Leu Gln Leu Ile Lys Glu Glu Asn Glu Ala Lys Leu Val Arg Trp Leu
   385                 390                 395                 400
   Gln Asp Glu Thr Gly Ile Asp Glu Lys Arg Ala Glu Val Ile Ala Asn
                   405                 410                 415
   Ala Gly Leu Pro Glu Gly Tyr Gly Ser Leu Cys Ile Glu Ala Leu Ala
               420                 425                 430
   Arg Ile Leu Pro Glu Leu Arg Arg Asp Val Val Thr Tyr Asp Lys Ala
           435                 440                 445
   Val Gln Ala Ala Gly Phe Glu His His Ser Lys Leu Asn Arg Asn Glu
       450                 455                 460
   Glu Ile Pro Asp Ile Thr Phe Lys Ile Glu Ser Ile Asp Arg Asn Ser
   465                 470                 475                 480
   Gly Glu Ile Lys Gln Phe His Leu His Lys Glu Leu Pro Tyr Tyr Gly
                   485                 490                 495
   Glu Tyr Leu Gln Arg His Val Gly Phe Gly Ser Gly Lys Pro Glu Asp
               500                 505                 510
   Ser Ile Glu Lys Arg Tyr Gly Lys Ile Ala Asn Pro Thr Val His Ile
           515                 520                 525
   Gly Leu Asn Gln Val Arg Val Val Val Asn Ala Leu Ile Lys Arg Tyr
       530                 535                 540
   Gly His Pro Ser Glu Ile Ile Val Glu Val Ala Arg Asp Leu Lys Gln
   545                 550                 555                 560
   Ser Lys Asp Arg Arg Asp Glu Glu Asn Lys Arg Gln Ala Glu Asn Gln
                   565                 570                 575
   Lys Arg Asn Glu Arg Leu Arg Lys Asp Ile Ala Asp Ile Leu Gly Ile
               580                 585                 590
   Ser Glu Glu Arg Val Arg Arg Asp Asp Ile Glu Lys Met Ile Leu Trp
           595                 600                 605
   Glu Glu Leu Ser Phe Asp Pro Ala Asp Arg Arg Cys Pro Tyr Ser Gly
       610                 615                 620
   Val Gln Val Ser Thr Val Met Leu Leu Ser Asp Glu Val Glu Val Glu
   625                 630                 635                 640
   His Ile Leu Pro Phe Ser Gln Thr Leu Asp Asp Ser Leu Asn Asn Lys
                   645                 650                 655
   Thr Val Ala Leu Arg Gln Ala Asn Arg Ile Lys Gly Asn Arg Thr Pro
               660                 665                 670
   Trp Glu Ala Phe Gly Thr Ser Asn Ile Ser Gly Phe Asp Tyr Ser Gly
           675                 680                 685
   Ile Leu Thr Arg Ala Glu Leu Met Pro Lys Ala Lys Arg Tyr Arg Phe
   690 695 700
   Ala Glu Asp Gly Tyr Gln Arg Trp Leu Lys Asp Asp Ala Gly Phe Leu
   705 710 715 720
   Ala Arg Ala Leu Asn Asp Thr Arg His Leu Ser Lys Ile Ala Arg Glu
   725 730 735
   Tyr Met Ser Leu Ile Cys Pro Asn Thr Arg Val Ile Pro Gly Gln Met
   740 745 750
   Thr Ala Met Leu Arg Arg Asn Phe Gly Leu Asn Asp Val Leu Gly Leu
   755 760 765
   Asn Gly Glu Lys Asn Arg Asn Asp His Arg His His Ala Val Asp Ala
   770 775 780
   Cys Val Ile Ala Val Thr Asp Gln Gly Leu Leu Gln Arg Phe Ala Ala
   785 790 795 800
   Ala Ser Ala Ser Ala Arg Glu Arg Gln Leu Asn Arg Leu Val Glu Asn
   805 810 815
   Met Pro Leu Pro Trp Glu Ser Tyr Arg Glu His Val Arg Arg Ala Ile
   820 825 830
   Asp Gly Ile Trp Val Ser His Arg Pro Asp His Ser His Glu Gly Ala
   835 840 845
   Met His Asn Asp Thr Ala Tyr Gly Leu Arg Gly Asn Gly Arg Val Ser
   850 855 860
   Phe His Lys Met Val Glu Gly Lys Arg Glu Tyr Ile Glu Asp Asn Leu
   865 870 875 880
   Lys Val Ile Glu Ile Thr Asp Thr Lys Ala Ile Asp Arg His Gly Leu
   885 890 895
   Leu Pro Thr Gly Lys Pro Arg Pro Tyr Lys Gly Tyr Lys Gly Asp Ser
   900 905 910
   Asn Tyr Cys Ile Glu Ile Val Arg Asn Glu Lys Gly Arg Trp Glu Gly
   915 920 925
   Glu Ile Ile Ser Thr Phe Asp Ala Tyr Gln Leu Val Arg Lys Tyr Gly
   930 935 940
   Ile Val Gln Leu Arg Tyr Pro Val Leu Ser Val Ser Gly Lys Pro Leu
   945 950 955 960
   Val Met Arg Leu Ile Arg Asn Asp Phe Val Arg Leu Glu His Glu Gly
   965 970 975
   Ile Thr Arg Thr Leu Arg Val Cys Lys Met Arg Glu Asp Gly Gln Val
   980 985 990
   Ala Leu Ala Asp Val Thr Glu Ala Asn Val Asp Ala Arg Thr Arg Thr
   995 1000 1005
   Lys Glu Ile Cys Tyr Val Phe Lys Thr Gly Gly Ser Leu Gln Lys Ala
   1010 1015 1020
   Lys Ala Arg Arg Ile Thr Ile Ser Pro Ile Gly Glu Leu Arg Asp Pro
   1025 1030 1035 1040
   Gly Phe Lys Glu
<210> 97
   <211> 1179
   <212> PRT
   <213> Sphaerochaeta globosa
<220>
   <223> CRISPR-associated endonuclease
<308> YP_004248194
   <309> 2013-07-06
<400> 97
   Met Ser Lys Lys Val Ser Arg Arg Tyr Glu Glu Gln Ala Gln Glu Ile
    1               5                  10                  15
   Cys Gln Arg Leu Gly Ser Arg Pro Tyr Ser Ile Gly Leu Asp Leu Gly
               20                  25                  30
   Val Gly Ser Ile Gly Val Ala Val Ala Ala Tyr Asp Pro Ile Lys Lys
   35 40 45
   Gln Pro Ser Asp Leu Val Phe Val Ser Ser Arg Ile Phe Ile Pro Ser
   50 55 60
   Thr Gly Ala Ala Glu Arg Arg Gln Lys Arg Gly Gln Arg Asn Ser Leu
   65 70 75 80
   Arg His Arg Ala Asn Arg Leu Lys Phe Leu Trp Lys Leu Leu Ala Glu
   85 90 95
   Arg Asn Leu Met Leu Ser Tyr Ser Glu Gln Asp Val Pro Asp Pro Ala
   100 105 110
   Arg Leu Arg Phe Glu Asp Ala Val Val Arg Ala Asn Pro Tyr Glu Leu
   115 120 125
   Arg Leu Lys Gly Leu Asn Glu Gln Leu Thr Leu Ser Glu Leu Gly Tyr
   130 135 140
   Ala Leu Tyr His Ile Ala Asn His Arg Gly Ser Ser Ser Val Arg Thr
   145 150 155 160
   Phe Leu Asp Glu Glu Lys Ser Ser Asp Asp Lys Lys Leu Glu Glu Gln
   165 170 175
   Gln Ala Met Thr Glu Gln Leu Ala Lys Glu Lys Gly Ile Ser Thr Phe
   180 185 190
   Ile Glu Val Leu Thr Ala Phe Asn Thr Asn Gly Leu Ile Gly Tyr Arg
   195 200 205
   Asn Ser Glu Ser Val Lys Ser Lys Gly Val Pro Val Pro Thr Arg Asp
   210 215 220
   Ile Ile Ser Asn Glu Ile Asp Val Leu Leu Gln Thr Gln Lys Gln Phe
   225 230 235 240
   Tyr Gln Glu Ile Leu Ser Asp Glu Tyr Cys Asp Arg Ile Val Ser Ala
   245 250 255
   Ile Leu Phe Glu Asn Glu Lys Ile Val Pro Glu Ala Gly Cys Cys Pro
   260 265 270
   Tyr Phe Pro Asp Glu Lys Lys Leu Pro Arg Cys His Phe Leu Asn Glu
   275 280 285
   Glu Arg Arg Leu Trp Glu Ala Ile Asn Asn Ala Arg Ile Lys Met Pro
   290 295 300
   Met Gln Glu Gly Ala Ala Lys Arg Tyr Gln Ser Ala Ser Phe Ser Asp
   305 310 315 320
   Glu Gln Arg His Ile Leu Phe His Ile Ala Arg Ser Gly Thr Asp Ile
   325 330 335
   Thr Pro Lys Leu Val Gln Lys Glu Phe Pro Ala Leu Lys Thr Ser Ile
   340 345 350
   Ile Val Leu Gln Gly Lys Glu Lys Ala Ile Gln Lys Ile Ala Gly Phe
   355 360 365
   Arg Phe Arg Arg Leu Glu Glu Lys Ser Phe Trp Lys Arg Leu Ser Glu
   370 375 380
   Glu Gln Lys Asp Asp Phe Phe Ser Ala Trp Thr Asn Thr Pro Asp Asp
   385 390 395 400
   Lys Arg Leu Ser Lys Tyr Leu Met Lys His Leu Leu Leu Thr Glu Asn
   405 410 415
   Glu Val Val Asp Ala Leu Lys Thr Val Ser Leu Ile Gly Asp Tyr Gly
   420 425 430
   Pro Ile Gly Lys Thr Ala Thr Gln Leu Leu Met Lys His Leu Glu Asp
   435 440 445
   Gly Leu Thr Tyr Thr Glu Ala Leu Glu Arg Gly Met Glu Thr Gly Glu
   450 455 460
   Phe Gln Glu Leu Ser Val Trp Glu Gln Gln Ser Leu Leu Pro Tyr Tyr
   465 470 475 480
   Gly Gln Ile Leu Thr Gly Ser Thr Gln Ala Leu Met Gly Lys Tyr Trp
   485 490 495
   His Ser Ala Phe Lys Glu Lys Arg Asp Ser Glu Gly Phe Phe Lys Pro
   500 505 510
   Asn Thr Asn Ser Asp Glu Glu Lys Tyr Gly Arg Ile Ala Asn Pro Val
   515 520 525
   Val His Gln Thr Leu Asn Glu Leu Arg Lys Leu Met Asn Glu Leu Ile
       530                 535                 540
   Thr Ile Leu Gly Ala Lys Pro Gln Glu Ile Thr Val Glu Leu Ala Arg
   545                 550                 555                 560
   Glu Leu Lys Val Gly Ala Glu Lys Arg Glu Asp Ile Ile Lys Gln Gln
                   565                 570                 575
   Thr Lys Gln Glu Lys Glu Ala Val Leu Ala Tyr Ser Lys Tyr Cys Glu
               580                 585                 590
   Pro Asn Asn Leu Asp Lys Arg Tyr Ile Glu Arg Phe Arg Leu Leu Glu
           595                 600                 605
   Asp Gln Ala Phe Val Cys Pro Tyr Cys Leu Glu His Ile Ser Val Ala
       610                 615                 620
   Asp Ile Ala Ala Gly Arg Ala Asp Val Asp His Ile Phe Pro Arg Asp
   625                 630                 635                 640
   Asp Thr Ala Asp Asn Ser Tyr Gly Asn Lys Val Val Ala His Arg Gln
                   645                 650                 655
   Cys Asn Asp Ile Lys Gly Lys Arg Thr Pro Tyr Ala Ala Phe Ser Asn
               660                 665                 670
   Thr Ser Ala Trp Gly Pro Ile Met His Tyr Leu Asp Glu Thr Pro Gly
           675                 680                 685
   Met Trp Arg Lys Arg Arg Lys Phe Glu Thr Asn Glu Glu Glu Tyr Ala
       690                 695                 700
   Lys Tyr Leu Gln Ser Lys Gly Phe Val Ser Arg Phe Glu Ser Asp Asn
   705                 710                 715                 720
   Ser Tyr Ile Ala Lys Ala Ala Lys Glu Tyr Leu Arg Cys Leu Phe Asn
                   725                 730                 735
   Pro Asn Asn Val Thr Ala Val Gly Ser Leu Lys Gly Met Glu Thr Ser
               740                 745                 750
   Ile Leu Arg Lys Ala Trp Asn Leu Gln Gly Ile Asp Asp Leu Leu Gly
           755                 760                 765
   Ser Arg His Trp Ser Lys Asp Ala Asp Thr Ser Pro Thr Met Arg Lys
       770                 775                 780
   Asn Arg Asp Asp Asn Arg His His Gly Leu Asp Ala Ile Val Ala Leu
   785                 790                 795                 800
   Tyr Cys Ser Arg Ser Leu Val Gln Met Ile Asn Thr Met Ser Glu Gln
                   805                 810                 815
   Gly Lys Arg Ala Val Glu Ile Glu Ala Met Ile Pro Ile Pro Gly Tyr
               820                 825                 830
   Ala Ser Glu Pro Asn Leu Ser Phe Glu Ala Gln Arg Glu Leu Phe Arg
           835                 840                 845
   Lys Lys Ile Leu Glu Phe Met Asp Leu His Ala Phe Val Ser Met Lys
       850                 855                 860
   Thr Asp Asn Asp Ala Asn Gly Ala Leu Leu Lys Asp Thr Val Tyr Ser
   865                 870                 875                 880
   Ile Leu Gly Ala Asp Thr Gln Gly Glu Asp Leu Val Phe Val Val Lys
                   885                 890                 895
   Lys Lys Ile Lys Asp Ile Gly Val Lys Ile Gly Asp Tyr Glu Glu Val
               900                 905                 910
   Ala Ser Ala Ile Arg Gly Arg Ile Thr Asp Lys Gln Pro Lys Trp Tyr
           915                 920                 925
   Pro Met Glu Met Lys Asp Lys Ile Glu Gln Leu Gln Ser Lys Asn Glu
       930                 935                 940
   Ala Ala Leu Gln Lys Tyr Lys Glu Ser Leu Val Gln Ala Ala Ala Val
   945                 950                 955                 960
   Leu Glu Glu Ser Asn Arg Lys Leu Ile Glu Ser Gly Lys Lys Pro Ile
                   965                 970                 975
   Gln Leu Ser Glu Lys Thr Ile Ser Lys Lys Ala Leu Glu Leu Val Gly
               980                 985                 990
   Gly Tyr Tyr Tyr Leu Ile Ser Asn Asn Lys Arg Thr Lys Thr Phe Val
           995                 1000                1005
   Val Lys Glu Pro Ser Asn Glu Val Lys Gly Phe Ala Phe Asp Thr Gly
       1010                1015                1020
   Ser Asn Leu Cys Leu Asp Phe Tyr His Asp Ala Gln Gly Lys Leu Cys
   1025                1030                1035                1040
   Gly Glu Ile Ile Arg Lys Ile Gln Ala Met Asn Pro Ser Tyr Lys Pro
                   1045                1050                1055
   Ala Tyr Met Lys Gln Gly Tyr Ser Leu Tyr Val Arg Leu Tyr Gln Gly
               1060                1065                1070
   Asp Val Cys Glu Leu Arg Ala Ser Asp Leu Thr Glu Ala Glu Ser Asn
           1075                1080                1085
   Leu Ala Lys Thr Thr His Val Arg Leu Pro Asn Ala Lys Pro Gly Arg
       1090                1095                1100
   Thr Phe Val Ile Ile Ile Thr Phe Thr Glu Met Gly Ser Gly Tyr Gln
   1105                1110                1115                1120
   Ile Tyr Phe Ser Asn Leu Ala Lys Ser Lys Lys Gly Gln Asp Thr Ser
                   1125                1130                1135
   Phe Thr Leu Thr Thr Ile Lys Asn Tyr Asp Val Arg Lys Val Gln Leu
               1140                1145                1150
   Ser Ser Ala Gly Leu Val Arg Tyr Val Ser Pro Leu Leu Val Asp Lys
           1155                1160                1165
   Ile Glu Lys Asp Glu Val Ala Leu Cys Gly Glu
       1170                1175
<210> 98
   <211> 315
   <212> PRT
   <213> Thauera sp.
<220>
   <223> CRISPR-associated endonuclease
<308> YP_002364238
   <309> 2013-06-07
<400> 98
   Met Ala Arg Ser Thr Tyr Lys Arg Thr Glu Glu Glu Lys Arg Asn Leu
   1               5                   10                  15
   Lys Val Asp Leu Ser Arg Ile Pro Glu Trp Glu Ser Glu Ile Glu Ser
               20                  25                  30
   Ile Arg Ser Arg Phe Arg Arg Phe Ser Leu Pro Lys Ile Ser Ser Ala
           35                  40                  45
   Ala Ser Leu Val His Glu Met Arg Lys Lys Leu Tyr Ile Gly Arg His
       50                  55                  60
   Ser Asp Arg Glu Arg Asp Ala Glu Ala Leu Glu Asn Trp Lys Arg Ser
   65                  70                  75                  80
   Leu Ser Ile Asp Ser Leu Ile Arg Met Ser Asp Asp Tyr Ser Ala Pro
                   85                  90                  95
   Thr Phe Leu Arg Ser Gly Val Glu Glu Asn Ile Ile Asp Lys Ile Arg
               100                 105                 110
   Ser Thr Leu Glu Gln Thr Glu Arg Gln Leu Ala Glu Tyr Arg Thr Arg
           115                 120                 125
   Gly Val Thr Asp Ser Phe Trp Val Ser Val Phe Thr Ser Lys Val Asp
       130                 135                 140
   Ser Asp Thr Val Lys Arg Phe Glu Gln Gln Lys Thr Asp Tyr Leu Ala
   145                 150                 155                 160
   Leu Ser Arg Glu Ile Pro Arg Val Ala Glu Ala Ile Arg Leu Leu Asp
                   165                 170                 175
   Glu Val Arg Ser Glu Ser Arg Glu Phe Ala Asn Arg Val Ser Lys Ile
               180                 185                 190
   Lys Glu Arg Leu Trp Leu Ala Glu Glu Lys Met Asp Ala Ile Glu Arg
           195                 200                 205
   Phe Glu Ala Lys His Gly Arg Val Phe Gly Lys Ala Ser Ala Val Asp
       210                 215                 220
   Asn Lys Thr Arg Glu Arg Ala Ser Ser Leu Lys Arg Leu Val Lys Lys
   225                 230                 235                 240
   Thr Lys Asp Cys Pro Tyr Cys Gly Ser Glu Leu Gly Val Asp Pro His
                   245                 250                 255
   Leu Asp His Ile Tyr Pro Val Ser Lys Gly Gly Leu Ser Val Ile Glu
               260                 265                 270
   Asn Leu Val Trp Cys Cys Ser Ala Cys Asn Thr Val Lys Ser Asp Lys
           275                 280                 285
   Gly Leu Met Gln Phe Leu Lys Glu Arg Gly Phe Ser Ile Asp Arg Thr
       290                 295                 300
   Leu Ser Arg Leu His Ala Leu Gly Lys His Val
   305                 310                 315
<210> 99
   <211> 1003
   <212> PRT
   <213> Gluconacetobacter diazotrophicus
<220>
   <223> CRISPR-associated endonuclease
<308> YP_002274753
   <309> 2013-06-10
<400> 99
   Met Ile Asp Glu Ser Leu Thr Phe Gly Ile Asp Leu Gly Ile Gly Ser
   1               5                   10                  15
   Cys Gly Trp Ala Val Leu Arg Arg Pro Ser Ala Phe Gly Arg Lys Gly
               20                  25                  30
   Val Ile Glu Gly Met Gly Ser Trp Cys Phe Asp Val Pro Glu Thr Ser
           35                  40                  45
   Lys Glu Arg Thr Pro Thr Asn Gln Ile Arg Arg Ser Asn Arg Leu Leu
       50                  55                  60
   Arg Arg Val Ile Arg Arg Arg Arg Asn Arg Met Ala Ala Ile Arg Arg
   65                  70                  75                  80
   Leu Leu His Ala Ala Gly Leu Leu Pro Ser Thr Asp Ser Asp Ala Leu
                   85                  90                  95
   Lys Arg Pro Gly His Asp Pro Trp Glu Leu Arg Ala Arg Gly Leu Asp
               100                 105                 110
   Lys Pro Leu Lys Pro Val Glu Phe Ala Val Val Leu Gly His Ile Ala
           115                 120                 125
   Lys Arg Arg Gly Phe Lys Ser Ala Ala Lys Arg Lys Ala Thr Asn Ile
       130                 135                 140
   Ser Ser Asp Asp Lys Lys Met Leu Thr Ala Leu Glu Ala Thr Arg Glu
   145                 150                 155                 160
   Arg Leu Gly Arg Tyr Arg Thr Val Gly Glu Met Phe Ala Arg Asp Pro
                   165                 170                 175
   Asp Phe Ala Ser Arg Arg Arg Asn Arg Glu Gly Lys Tyr Asp Arg Thr
               180                 185                 190
   Thr Ala Arg Asp Asp Leu Glu His Glu Val His Ala Leu Phe Ala Ala
           195                 200                 205
   Gln Arg Arg Leu Gly Gln Gly Phe Ala Ser Pro Glu Leu Glu Glu Ala
       210                 215                 220
   Phe Thr Ala Ser Ala Phe His Gln Arg Pro Met Gln Asp Ser Glu Arg
   225                 230                 235                 240
   Leu Val Gly Phe Cys Pro Phe Glu Arg Thr Glu Lys Arg Ala Ala Lys
                   245                 250                 255
   Leu Thr Pro Ser Phe Glu Arg Phe Arg Leu Leu Ala Arg Leu Leu Asn
               260                 265                 270
   Leu Arg Ile Thr Thr Pro Asp Gly Glu Arg Pro Leu Thr Val Asp Glu
           275                 280                 285
   Ile Ala Leu Val Thr Arg Asp Leu Gly Lys Thr Ala Lys Leu Ser Ile
       290                 295                 300
   Lys Arg Val Arg Thr Leu Ile Gly Leu Glu Asp Asn Gln Arg Phe Thr
   305                 310                 315                 320
   Thr Ile Arg Pro Glu Asp Glu Asp Arg Asp Ile Val Ala Arg Thr Gly
                   325                 330                 335
   Gly Ala Met Thr Gly Thr Ala Thr Leu Arg Lys Ala Leu Gly Glu Ala
               340                 345                 350
   Leu Trp Thr Asp Met Gln Glu Arg Pro Glu Gln Leu Asp Ala Ile Val
           355                 360                 365
   Gln Val Leu Ser Phe Phe Glu Ala Asn Glu Thr Ile Thr Glu Lys Leu
       370                 375                 380
   Arg Glu Ile Gly Leu Thr Leu Ala Val Leu Asp Val Leu Leu Thr Ala
   385                 390                 395                 400
   Leu Asp Ala Gly Val Phe Ala Lys Phe Lys Gly Ala Ala His Ile Ser
                   405                 410                 415
   Thr Lys Ala Ala Arg Asn Leu Leu Pro His Leu Glu Gln Gly Arg Arg
               420                 425                 430
   Tyr Asp Glu Ala Cys Thr Met Ala Gly Tyr Asp His Ala Ala Ser Arg
           435                 440                 445
   Leu Ser His His Gly Gln Ile Val Ala Lys Thr Gln Phe Asn Ala Leu
       450                 455                 460
   Val Thr Glu Ile Gly Glu Ser Ile Ala Asn Pro Ile Ala Arg Lys Ala
   465                 470                 475                 480
   Leu Ile Glu Gly Leu Lys Gln Ile Trp Ala Met Arg Asn His Trp Gly
                   485                 490                 495
   Leu Pro Gly Ser Ile His Val Glu Leu Ala Arg Asp Val Gly Asn Ser
               500                 505                 510
   Ile Glu Lys Arg Arg Glu Ile Glu Lys His Ile Glu Lys Asn Thr Ala
           515                 520                 525
   Leu Arg Ala Arg Glu Arg Arg Glu Val His Asp Leu Leu Asp Leu Glu
       530                 535                 540
   Asp Val Asn Gly Asp Thr Leu Leu Arg Tyr Arg Leu Trp Lys Glu Gln
   545                 550                 555                 560
   Gly Gly Lys Cys Leu Tyr Thr Gly Lys Ala Ile His Ile Arg Gln Ile
                   565                 570                 575
   Ala Ala Thr Asp Asn Ser Val Gln Val Asp His Ile Leu Pro Trp Ser
               580                 585                 590
   Arg Phe Gly Asp Asp Ser Phe Asn Asn Lys Thr Leu Cys Leu Ala Ser
           595                 600                 605
   Ala Asn Gln Gln Lys Lys Arg Ser Thr Pro Tyr Glu Trp Leu Ser Gly
       610                 615                 620
   Gln Thr Gly Asp Ala Trp Asn Ala Phe Val Gln Arg Ile Glu Thr Asn
   625                 630                 635                 640
   Lys Glu Leu Arg Gly Phe Lys Lys Arg Asn Tyr Leu Leu Lys Asn Ala
                   645                 650                 655
   Lys Glu Ala Glu Glu Lys Phe Arg Ser Arg Asn Leu Asn Asp Thr Arg
               660                 665                 670
   Tyr Ala Ala Arg Leu Phe Ala Glu Ala Val Lys Leu Leu Tyr Ala Phe
           675                 680                 685
   Gly Glu Arg Gln Glu Lys Gly Gly Asn Arg Arg Val Phe Thr Arg Pro
       690                 695                 700
   Gly Ala Leu Thr Ala Ala Leu Arg Gln Ala Trp Gly Val Glu Ser Leu
   705                 710                 715                 720
   Lys Lys Gln Asp Gly Lys Arg Ile Asn Asp Asp Arg His His Ala Leu
                   725                 730                 735
   Asp Ala Leu Thr Val Ala Ala Val Asp Glu Ala Glu Ile Gln Arg Leu
               740                 745                 750
   Thr Lys Ser Phe His Glu Trp Glu Gln Gln Gly Leu Gly Arg Pro Leu
           755                 760                 765
   Arg Arg Val Glu Pro Pro Trp Glu Ser Phe Arg Ala Asp Val Glu Ala
       770                 775                 780
   Thr Tyr Pro Glu Val Phe Val Ala Arg Pro Glu Arg Arg Arg Ala Arg
   785                 790                 795                 800
   Gly Glu Gly His Ala Ala Thr Ile Arg Gln Val Lys Glu Arg Glu Cys
                   805                 810                 815
   Thr Pro Ile Val Phe Glu Arg Lys Ala Val Ser Ser Leu Lys Glu Ala
                   820                 825                 830
   Asp Leu Glu Arg Ile Lys Asp Gly Glu Arg Asn Glu Ala Ile Val Glu
               835                 840                 845
   Ala Ile Arg Ser Trp Ile Ala Thr Gly Arg Pro Ala Asp Ala Pro Pro
           850                 855                 860
   Arg Ser Pro Arg Gly Asp Ile Ile Thr Lys Ile Arg Leu Ala Thr Thr
   865                 870                 875                 880
   Ile Lys Ala Ala Val Pro Val Arg Gly Gly Thr Ala Gly Arg Gly Glu
                   885                 890                 895
   Met Val Arg Ala Asp Val Phe Ser Lys Pro Asn Arg Arg Gly Lys Asp
               900                 905                 910
   Glu Trp Tyr Leu Val Pro Val Tyr Pro His Gln Ile Met Asn Arg Lys
           915                 920                 925
   Ala Trp Pro Lys Pro Pro Met Arg Ser Ile Val Ala Asn Lys Asp Glu
       930                 935                 940
   Asp Glu Trp Thr Glu Val Gly Pro Glu His Gln Phe Arg Phe Ser Leu
   945                 950                 955                 960
   Tyr Pro Arg Ser Asn Ile Glu Ile Ile Arg Pro Ser Gly Glu Val Ile
                   965                 970                 975
   Glu Gly Tyr Phe Val Gly Leu His Arg Asn Thr Gly Ala Leu Ile Pro
               980                 985                 990
   Thr Pro Val Gly Pro Asp Ser Tyr Val Ile Ala
           995                 1000
<210> 100
   <211> 984
   <212> PRT
   <213> Campylobacter jejuni
<220>
   <223> CRISPR-associated endonuclease
<308> YP_001398821
   <309> 2013-06-10
<400> 100
   Met Ala Lys Ile Leu Ala Phe Asp Ile Gly Ile Ser Ser Ile Gly Trp
    1               5                  10                  15
   Ala Phe Ser Glu Asn Asp Glu Leu Lys Asp Cys Gly Val Arg Ile Phe
               20                  25                  30
   Thr Lys Ala Glu Asn Pro Lys Thr Gly Glu Ser Leu Ala Leu Pro Arg
           35                  40                  45
   Arg Ile Ala Arg Ser Ala Arg Lys Arg Leu Ala Arg Arg Lys Ala Arg
       50                  55                  60
   Leu Asn His Leu Lys His Leu Ile Ala Asn Glu Phe Lys Leu Asn Tyr
   65                  70                  75                  80
   Glu Asp Tyr Gln Ser Phe Asp Glu Ile Leu Ala Lys Ala Tyr Lys Gly
                   85                  90                  95
   Ser Leu Ile Ser Pro Tyr Glu Leu Arg Phe Arg Ala Leu Asn Glu Leu
               100                 105                 110
   Leu Ser Lys Gln Asp Phe Ala Arg Val Ile Leu His Ile Ala Lys Arg
           115                 120                 125
   Arg Gly Tyr Asp Asp Ile Lys Asn Asn Gly Asp Glu Glu Lys Gly Lys
       130                 135                 140
   Ile Leu Lys Ala Ile Lys Gln Asn Glu Glu Lys Leu Ala Asn Tyr Gln
   145                 150                 155                 160
   Ser Val Gly Glu Tyr Leu Tyr Lys Glu Tyr Phe Gln Lys Phe Lys Glu
                   165                 170                 175
   Asn Ser Lys Glu Phe Ile Asn Val Arg Asn Lys Lys Glu Ser Tyr Glu
               180                 185                 190
   Arg Cys Ile Ala Gln Ser Phe Leu Lys Asp Glu Leu Lys Leu Ile Phe
           195                 200                 205
   Lys Lys Gln Arg Glu Phe Gly Phe Ser Phe Ser Lys Lys Phe Glu Glu
       210                 215                 220
   Glu Val Leu Ser Val Ala Phe Tyr Lys Arg Ala Leu Lys Asp Phe Ser
   225                 230                 235                 240
   His Leu Val Gly Asn Cys Ser Phe Phe Thr Asp Glu Lys Arg Ala Pro
                   245                 250                 255
   Lys Asn Ser Pro Leu Ala Phe Met Phe Val Ala Leu Thr Arg Ile Ile
               260                 265                 270
   Asn Leu Leu Asn Asn Leu Lys Asn Thr Leu Tyr Thr Lys Asp Asp Leu
           275                 280                 285
   Asn Ala Leu Leu Asn Glu Val Leu Lys Asn Gly Thr Leu Thr Tyr Lys
       290                 295                 300
   Gln Thr Lys Lys Leu Leu Gly Leu Ser Asp Asp Tyr Glu Phe Lys Gly
   305                 310                 315                 320
   Glu Lys Gly Asn Tyr Phe Ile Glu Phe Lys Lys Tyr Lys Glu Phe Ile
                   325                 330                 335
   Lys Ala Leu Gly Glu His Ser Leu Ser Gln Asp Asp Leu Asn Glu Ile
               340                 345                 350
   Ala Lys Asp Ile Thr Leu Ile Lys Asp Glu Ile Lys Leu Lys Lys Ala
           355                 360                 365
   Leu Ala Lys Tyr Asp Leu Asn Glu Asn Gln Ile Asp Asn Leu Ser Lys
       370                 375                 380
   Leu Glu Phe Lys Asp His Leu Asn Ile Ser Phe Lys Ala Leu Lys Leu
   385                 390                 395                 400
   Ile Thr Pro Leu Met Leu Glu Gly Lys Lys Tyr Asp Glu Ala Cys Asn
                   405                 410                 415
   Glu Leu Thr Leu Lys Val Ala Ile Asn Glu Asp Lys Lys Asp Phe Leu
               420                 425                 430
   Pro Ala Phe Asn Glu Thr Tyr Tyr Lys Asp Glu Val Thr Asn Pro Val
           435                 440                 445
   Val Leu Arg Ala Ile Lys Glu Tyr Arg Lys Val Leu Asn Ala Leu Leu
       450                 455                 460
   Lys Lys Tyr Gly Lys Val His Lys Ile Asn Ile Glu Leu Ala Arg Glu
   465                 470                 475                 480
   Val Gly Lys Asn His Ser Gln Arg Ala Lys Ile Glu Lys Glu Gln Asn
                   485                 490                 495
   Glu Asn Tyr Lys Ala Lys Lys Asp Ala Glu Leu Glu Cys Glu Lys Leu
               500                 505                 510
   Gly Leu Lys Ile Asn Ser Lys Asn Ile Leu Lys Leu Arg Leu Phe Lys
           515                 520                 525
   Glu Gln Lys Glu Phe Cys Val Tyr Ser Gly Glu Lys Ile Lys Leu Ser
       530                 535                 540
   Asp Leu Gln Asp Glu Lys Met Leu Glu Ile Asp His Ile Tyr Pro Tyr
   545                 550                 555                 560
   Ser Arg Ser Phe Asp Asp Ser Tyr Met Asn Lys Val Leu Val Phe Thr
                   565                 570                 575
   Lys Gln Asn Gln Glu Lys Leu Asn Gln Thr Pro Phe Glu Ala Phe Gly
               580                 585                 590
   Asn Asp Ser Ala Lys Trp Gln Lys Ile Glu Val Leu Ala Lys Asn Leu
           595                 600                 605
   Pro Thr Lys Lys Gln Lys Arg Ile Leu Asp Lys Asn Tyr Lys Asp Lys
       610                 615                 620
   Glu Gln Lys Asp Phe Lys Asp Arg Asn Leu Asn Asp Thr Arg Tyr Ile
   625                 630                 635                 640
   Ala Arg Leu Val Leu Asn Tyr Thr Lys Asp Tyr Leu Asp Phe Leu Pro
                   645                 650                 655
   Leu Ser Asp Asp Glu Asn Thr Lys Leu Asn Asp Thr Gln Lys Gly Ser
               660                 665                 670
   Lys Val His Val Glu Ala Lys Ser Gly Met Leu Thr Ser Ala Leu Arg
           675                 680                 685
   His Thr Trp Gly Phe Ser Thr Lys Asp Arg Asn Asn His Leu His His
       690                 695                 700
   Ala Ile Asp Ala Val Ile Ile Ala Tyr Ala Asn Asn Ser Ile Val Lys
   705                 710                 715                 720
   Ala Phe Ser Asp Phe Lys Lys Glu Gln Glu Ser Asn Thr Ala Glu Leu
                   725                 730                 735
   Tyr Ala Lys Lys Ile Ser Glu Leu Asp Tyr Lys Asn Lys Arg Lys Phe
               740                 745                 750
   Phe Glu Pro Phe Ser Gly Phe Arg Gln Lys Val Leu Asp Lys Ile Asp
           755                 760                 765
   Glu Ile Phe Val Ser Lys Pro Glu Arg Lys Lys Pro Ser Gly Ala Leu
       770                 775                 780
   His Glu Glu Thr Phe Arg Lys Glu Glu Glu Phe Tyr Gln Ser Tyr Gly
   785                 790                 795                 800
   Gly Lys Glu Gly Val Leu Lys Ala Leu Glu Leu Gly Lys Ile Arg Lys
                   805                 810                 815
   Val Asn Gly Lys Ile Val Lys Asn Gly Asp Met Phe Arg Val Asp Ile
               820                 825                 830
   Phe Lys His Lys Lys Thr Asn Lys Phe Tyr Ala Val Pro Ile Tyr Thr
           835                 840                 845
   Met Asp Phe Ala Leu Lys Val Leu Pro Asn Lys Ala Val Ala Arg Ser
       850                 855                 860
   Lys Lys Gly Glu Ile Lys Asp Trp Ile Leu Met Asp Glu Asn Tyr Glu
   865                 870                 875                 880
   Phe Cys Phe Ser Leu Tyr Lys Asp Ser Leu Ile Leu Ile Gln Thr Lys
                   885                 890                 895
   Asp Met Gln Glu Pro Glu Phe Val Tyr Tyr Asn Ala Phe Thr Ser Ser
               900                 905                 910
   Thr Val Ser Leu Ile Val Ser Lys His Asp Asn Lys Phe Glu Thr Leu
           915                 920                 925
   Ser Lys Asn Gln Lys Ile Leu Phe Lys Asn Ala Asn Glu Lys Glu Val
       930                 935                 940
   Ile Ala Lys Ser Ile Gly Ile Gln Asn Leu Lys Val Phe Glu Lys Tyr
   945                 950                 955                 960
   Ile Val Ser Ala Leu Gly Glu Val Thr Lys Ala Glu Phe Arg Gln Arg
                   965                 970                 975
   Glu Glu Phe Lys Asn Glu Leu Arg
               980
<210> 101
   <211> 1062
   <212> PRT
   <213> Actinobacillus succinogenes
<220>
   <223> CRISPR-associated endonuclease
<308> YP_001343689
   <309> 2013-06-10
<400> 101
   Met Asn Leu Asn Tyr Ile Leu Gly Leu Asp Leu Gly Ile Ala Ser Val
    1               5                  10                  15
   Gly Trp Ala Val Val Glu Ile Asp Glu Gln Glu Asn Pro Gln Arg Leu
               20                  25                  30
   Ile Asp Val Gly Val Arg Thr Phe Asp Arg Ala Glu Val Leu Lys Thr
           35                  40                  45
   Gly Glu Ser Leu Ala Leu Ala Arg Arg Leu Ala Arg Ser Ser Arg Arg
       50                  55                  60
   Leu Val Lys Arg Arg Ala Glu Arg Ile Lys Lys Ala Lys Arg Leu Leu
   65                  70                  75                  80
   Lys Ala Glu Asn Leu Leu Leu Ser Ala Asp Glu Ile Leu Pro Asn Glu
                   85                  90                  95
   Ala Trp Gln Leu Arg Val Lys Gly Leu Asp Gln Lys Leu Glu Arg Gln
               100                 105                 110
   Glu Trp Ala Ala Val Leu Leu His Leu Leu Lys His Arg Gly Tyr Leu
           115                 120                 125
   Ser Gln Arg Lys Asn Glu Ser Lys Ser Glu Asn Lys Glu Leu Gly Ala
       130                 135                 140
   Leu Leu Ser Gly Val Ala Ile Asn His Gln Ile Leu Gln Ser Ala Glu
   145                 150                 155                 160
   Tyr Arg Thr Pro Ala Glu Ile Ala Val Asn Lys Phe Gln Ala Glu Glu
                   165                 170                 175
   Gly His Ile Arg Asn Gln Arg Gly Ala Tyr Thr His Thr Phe Ser Arg
               180                 185                 190
   Leu Asp Leu Leu Ala Glu Met Glu Leu Leu Phe Gln Arg Gln Ala Thr
           195                 200                 205
   Leu Gly Asn Pro His Thr Ser Thr Lys Leu Leu Glu Asn Leu Thr Ala
       210                 215                 220
   Leu Leu Met Trp Gln Lys Pro Ala Leu Ala Gly Glu Ala Ile Leu Lys
   225                 230                 235                 240
   Met Leu Gly Lys Cys Thr Phe Glu Pro Thr Glu Tyr Lys Ala Ala Lys
                   245                 250                 255
   Asn Ser Tyr Ser Ala Glu Arg Phe Val Trp Leu Thr Lys Leu Asn Asn
               260                 265                 270
   Leu Arg Ile Leu Glu Gln Gly Ala Glu Arg Ala Leu Thr Asp Asn Glu
           275                 280                 285
   Arg Phe Ala Leu Leu Asp Gln Pro Tyr Glu Lys Ala Lys Leu Thr Tyr
       290                 295                 300
   Ala Gln Val Arg Thr Leu Leu Gly Leu Ser Asp Glu Ala Ile Phe Lys
   305                 310                 315                 320
   Gly Leu Arg Tyr Gln Gly Glu Asp Lys Lys Ala Val Glu Thr Lys Thr
                   325                 330                 335
   Thr Leu Met Glu Val Lys Ala Tyr His Gln Ile Arg Lys Ala Leu Glu
               340                 345                 350
   Gly Leu Asp Leu Lys Ala Glu Trp Asn Glu Leu Lys Asn Asn Pro Ala
           355                 360                 365
   Leu Leu Asp Glu Ile Gly Thr Ala Phe Ser Leu Tyr Lys Thr Asp Asp
       370                 375                 380
   Asp Ile His Arg Tyr Leu Asp Gly Lys Leu Ser Glu Ser Val Leu Asn
   385                 390                 395                 400
   Ala Leu Leu Glu Asn Leu Asn Phe Asp Lys Phe Ile Gln Leu Ser Leu
                   405                 410                 415
   Lys Ala Leu Gln Gln Ile Leu Pro Leu Met Leu Gln Gly Gln Arg Tyr
               420                 425                 430
   Asp Glu Ala Val Ser Ala Ile Tyr Gly Asp His Tyr Gly Lys Lys Ser
           435                 440                 445
   Asp Glu Ile Asp Arg Leu Leu Pro Asn Ile Pro Thr Asp Glu Ile Arg
       450                 455                 460
   Asn Pro Val Val Leu Arg Thr Leu Thr Gln Ala Arg Lys Val Ile Asn
   465                 470                 475                 480
   Ala Val Val Arg Leu Tyr Gly Ser Pro Ala Arg Ile His Ile Glu Thr
                   485                 490                 495
   Gly Arg Glu Val Gly Lys Ser Tyr Gln Asp Arg Lys Lys Leu Glu Lys
               500                 505                 510
   Gln Gln Glu Glu Asn Arg Lys Gln Arg Glu Asn Ala Val Lys Lys Phe
           515                 520                 525
   Lys Glu Tyr Phe Pro Asn Phe Val Gly Glu Pro Lys Gly Lys Asp Ile
       530                 535                 540
   Leu Lys Met Arg Leu Tyr Glu Leu Gln Gln Ala Lys Cys Leu Tyr Ser
   545                 550                 555                 560
   Gly Lys Ser Leu Glu Leu Tyr Arg Leu Leu Glu Lys Gly Tyr Val Glu
                   565                 570                 575
   Val Asp His Ala Leu Pro Phe Ser Arg Thr Trp Asp Asp Ser Phe Asn
               580                 585                 590
   Asn Lys Val Leu Val Leu Ala Asn Glu Asn Gln Asn Lys Gly Asn Ala
           595                 600                 605
   Thr Pro Tyr Glu Trp Leu Asp Gly Lys Asn Asn Ser Glu Gly Trp Gln
       610                 615                 620
   His Phe Val Ala Arg Val Gln Thr Ser Gly Phe Ser Tyr Ala Lys Lys
   625                 630                 635                 640
   Gln Arg Val Leu Ile Gln Glu Leu Asp Glu Lys Ser Phe Ile Glu Arg
                   645                 650                 655
   Asn Leu Asn Asp Thr Arg Tyr Val Ala Arg Phe Leu Cys Asn His Ile
               660                 665                 670
   Ala Asp Asn Met Leu Leu Thr Gly Lys Gly Lys Arg Lys Val Phe Ala
           675                 680                 685
   Ser Asn Gly Gln Ile Thr Ala Leu Leu Arg Gly Cys Trp Gly Leu Gln
       690                 695                 700
   Lys Val Arg Glu Asn Asn Asp Arg His His Ala Leu Asp Ala Val Val
   705                 710                 715                 720
   Val Ala Cys Ser Thr Lys Ser Met Gln Asp Lys Ile Thr Lys Phe Val
                   725                 730                 735
   Lys Tyr Lys Glu Gly Arg Leu Phe Lys Asp Lys Lys Asp Gly Val Tyr
               740                 745                 750
   Lys Gly Glu Arg Ile Asn Arg Glu Thr Gly Glu Ile Ile Ser Leu His
           755                 760                 765
   Phe Pro Thr Pro Trp Lys Phe Phe Lys Glu Asn Val Glu Ile Arg Ile
       770                 775                 780
   Phe Ser Glu Asn Pro Lys Leu Glu Leu Glu Asn Arg Leu Pro Asp Tyr
   785                 790                 795                 800
   Pro Gln Tyr Asn His Glu Phe Val Gln Pro Leu Phe Val Ser Arg Met
                   805                 810                 815
   Pro Thr Arg Lys Met Thr Gly Gln Gly His Met Glu Thr Val Lys Ser
               820                 825                 830
   Ala Lys Arg Leu Asp Lys Gly Leu Ser Val Leu Lys Val Pro Leu Thr
           835                 840                 845
   Gln Leu Lys Leu Ser Asp Leu Glu Arg Met Val Asn Arg Glu Arg Glu
       850                 855                 860
   Met Ala Leu Tyr Glu Ala Leu Lys Glu Cys Leu Glu Gln Phe Gly Asn
   865                 870                 875                 880
   Asp Pro Ala Lys Ala Phe Ala Glu Pro Phe Tyr Lys Lys Gly Gly Ala
                   885                 890                 895
   Leu Val Lys Ala Val Arg Val Glu Gln Thr Gln Lys Ser Gly Val Leu
               900                 905                 910
   Val Arg Asn Gly Asn Gly Val Ala Asp Asn Ala Thr Gly Ser Ile Val
           915                 920                 925
   Arg Val Asp Val Phe Thr Lys Gly Gly Lys Tyr Phe Leu Val Pro Ile
       930                 935                 940
   Tyr Thr Trp Gln Val Ala Lys Gly Ile Leu Pro Asn Lys Ala Val Ile
   945                 950                 955                 960
   Gln Gly Lys Asp Glu Ser Glu Trp Glu Glu Met Asp Glu Asn Ala Val
                   965                 970                 975
   Phe Gln Phe Ser Met His Lys Asn Asp Leu Ile Lys Val Lys Thr Lys
               980                 985                 990
   Lys Gly Glu Ile Phe Gly Tyr Phe Asn Gly Leu Asp Arg Ala Thr Gly
           995                 1000                1005
   Ser Ile Glu Ile Glu Glu His Asp Leu Glu Lys Ser Lys Gly Lys Asn
       1010                1015                1020
   Gly Ser His Arg Gly Leu Gly Val Lys Thr Ala Phe Phe Leu Glu Lys
   1025                1030                1035                1040
   Tyr Gln Val Asp Glu Leu Gly Lys Asn Ile Arg Pro Cys Arg Pro Thr
                   1045                1050                1055
   Lys Arg Gln His Val Arg
               1060
<210> 102
   <211> 1068
   <212> PRT
   <213> Verminephrobacter eiseniae
<220>
   <223> CRISPR-associated endonuclease
<308> YP_996018
   <309> 2013-06-10
<400> 102
   Met Asn Lys Ala Val Ala Tyr Arg Leu Ala Leu Asp Leu Gly Ser Thr
   1               5                   10                  15
   Ser Leu Gly Trp Ala Ile Phe Arg Leu Asn Glu Ala Arg Glu Pro Thr
               20                  25                  30
   Ala Ile Ile Arg Ala Gly Val Arg Ile Phe Ser Asp Gly Arg Asn Ala
           35                  40                  45
   Asn Ser Glu Pro Leu Ala Val His Arg Arg Val Ala Arg Ala Met Arg
       50                  55                  60
   Arg Arg Arg Asp Arg Leu Leu Lys Arg Lys Lys Arg Met His Asp Gln
   65                  70                  75                  80
   Leu Val Gln His Gly Phe Phe Pro Ala Glu Val Ala Glu Arg Lys Glu
                   85                  90                  95
   Leu Glu Arg Leu Asn Pro Tyr Gln Leu Arg Ala Lys Gly Leu His Glu
               100                 105                 110
   Ala Leu Thr Pro Gly Glu Phe Ala Arg Ala Leu Phe His Ile Asn Gln
           115                 120                 125
   Arg Arg Gly Phe Lys Ser Asn Arg Lys Thr Asp Arg Lys Asp Asn Asp
       130                 135                 140
   Ser Gly Ala Leu Lys Gln Ala Ile Ser Glu Leu Arg Lys Arg Ile Gln
   145                 150                 155                 160
   Asp Ser Asp Cys Ala Thr Ala Gly Glu Trp Phe Trp Lys Glu Arg Met
                   165                 170                 175
   Gln Gln Lys Pro Glu Gly Val Arg Gly Gln Trp Val Arg Ala Arg Tyr
               180                 185                 190
   Arg Lys Thr Pro Ser Thr Thr Asp Glu Gly Lys Lys Arg Ile Gly Tyr
           195                 200                 205
   Asp Leu Tyr Val Asp Arg Ala Met Val Glu Gln Glu Phe Asp Ala Leu
       210                 215                 220
   Trp Ala Ala Gln Ala Ala Leu Gln Pro Gly Leu Phe Thr Glu Ala Ala
   225                 230                 235                 240
   Arg Ala Glu Leu Lys Asp Thr Leu Leu Tyr Gln Arg Asp Leu Arg Pro
                   245                 250                 255
   Val Lys Pro Gly Arg Cys Thr Leu Leu Pro Ala Glu Glu Arg Ala Pro
               260                 265                 270
   Leu Ala Leu Pro Ser Thr Gln Arg Phe Arg Ile Leu Gln Glu Val Asn
           275                 280                 285
   Asn Leu Arg Leu Leu Asp Glu Ala Leu Arg Glu Val Pro Leu Asn Leu
       290                 295                 300
   Ala Gln Arg Asp Ala Val Val Ser Ala Leu Glu Gly Lys Lys Glu Leu
   305                 310                 315                 320
   Ser Phe Ala Ala Ile Arg Lys Leu Leu Thr Leu Ser Gly Lys Phe Asn
                   325                 330                 335
   Leu Glu Asp Glu Lys Arg Ala Glu Leu Lys Gly Asn Ala Thr Ser Val
               340                 345                 350
   Ile Leu Ala Arg Lys Asp Leu Phe Gly Asp Ala Trp Ala Gly Phe Asp
           355                 360                 365
   Ala Ala Val Gln Asp Glu Ile Val Trp Arg Leu Val Ser Gln Glu Ser
       370                 375                 380
   Glu Gly Ala Leu Ile Ala Trp Leu Gln Gln His Thr Gly Val Asp Gly
   385                 390                 395                 400
   Val Cys Ala Glu Ala Ile Val Asn Thr Arg Leu Pro Asp Gly Tyr Gly
                   405                 410                 415
   Arg Leu Ser Arg Lys Ala Leu Glu Arg Ile Val Pro Ala Leu Gln Arg
               420                 425                 430
   Glu Val Cys Thr Tyr Asp Lys Ala Val Gln Ala Ala Gly Phe Ala His
               435                 440                 445
   His Ser Asp Leu Gly Phe Asp Phe Asp Tyr Ala Glu Asp Glu Val Gln
           450                 455                 460
   Gln Val Gly Glu His Thr Ile Ala Ser Thr Gly Glu Val Gln Ala Gln
   465                 470                 475                 480
   Tyr Ala Phe Lys Gln Leu Pro Tyr Tyr Gly Lys Ala Leu Gln Arg His
                   485                 490                 495
   Val Ala Phe Gly Ser Gly Asp Ala Lys Asp His Glu Glu Lys Cys Tyr
               500                 505                 510
   Gly Lys Ile Ala Asn Pro Thr Val His Ile Gly Leu Asn Gln Val Arg
           515                 520                 525
   Thr Val Val Asn Ala Leu Ile Arg Arg Tyr Gly His Pro Thr Glu Val
       530                 535                 540
   Val Val Glu Leu Ala Arg Asp Leu Lys Gln Ser Arg Glu Gln Lys Gln
   545                 550                 555                 560
   Gln Thr Gln Arg Glu Gln Ala Asp Asn Gln Lys Arg Asn Glu Asp Ile
                   565                 570                 575
   Arg Lys Arg Ile Ala Pro Ile Leu Glu Thr Ser Pro Glu Arg Val Arg
               580                 585                 590
   Asp Arg Asp Ile Lys Lys Trp Ile Leu Trp Glu Glu Leu Asn Lys Lys
           595                 600                 605
   Asp Ile Ala Asp Arg His Cys Pro Tyr Ser Gly Glu Arg Ile Ser Ala
       610                 615                 620
   Thr Met Leu Leu Ser Glu Ala Val Glu Ile Glu His Ile Leu Pro Phe
   625                 630                 635                 640
   Ser Arg Thr Leu Asp Asp Ser Leu Asn Asn Arg Thr Val Ala Met Arg
                   645                 650                 655
   Arg Ala Asn Arg Ile Lys Gly Asp Arg Thr Pro Trp Glu Ala Arg Ala
               660                 665                 670
   Asp Phe Glu Ala Gln Gly Trp Arg Tyr Glu Ala Ile Leu Gln Arg Ala
           675                 680                 685
   Glu Gly Met Pro Pro Arg Lys Arg Tyr Arg Phe Ala Glu Asp Gly Tyr
       690                 695                 700
   Gln Arg Trp Leu Gly Lys Asp Arg Asn Phe Leu Ala Arg Ala Leu Asn
   705                 710                 715                 720
   Asp Thr Ser Tyr Leu Ser Arg Leu Ala Ala Asn Tyr Leu Arg Leu Val
                   725                 730                 735
   Cys Pro Gln Gly Val Arg Val Ile Pro Gly Gln Met Thr Asp Lys Leu
               740                 745                 750
   Arg Gly Lys Phe Gly Leu Asn Ser Val Leu Gly Leu Asp Gly Lys Lys
           755                 760                 765
   Asn Arg Asn Asp His Arg His His Ala Val Asp Ala Cys Val Ile Gly
       770                 775                 780
   Val Thr Asp Gln Gly Leu Met Gln Arg Phe Ala Asn Ala Ser Lys Gln
   785                 790                 795                 800
   Ala Arg Glu Asn Gly Leu Thr Arg Leu Val Gln Asp Met Leu Leu Pro
                   805                 810                 815
   Trp Trp Pro Ser Tyr Tyr Asp His Val Glu Arg Ala Val Arg His Ile
               820                 825                 830
   Arg Val Ser His Arg Pro Asp His Gly Phe Glu Gly Ala Met Met Lys
           835                 840                 845
   Gly Thr Ala His Gly Ile Arg Glu Asp Gly Ile Arg Glu Asp Gly Arg
       850                 855                 860
   Ile Lys Gln Arg Pro Lys Ala Lys Gly Ser Ala Asp His Lys Thr Ile
   865                 870                 875                 880
   Thr Leu Ile Pro Ile Asp Glu Pro Arg Gln Leu Ala Arg His Gly Val
                   885                 890                 895
   Asp Ala Glu Gly Lys Pro Leu Pro Tyr Lys Gly Tyr Ala Ser Gly Ser
               900                 905                 910
   Asn Tyr Cys Ile Glu Ile Thr Lys Asn Gly Lys Gly Lys Trp Glu Gly
           915                 920                 925
   Gln Val Ile Ser Thr Phe Asp Ala Tyr Arg Ile Lys Ala Ala Ala Asp
       930                 935                 940
   Ala Ala Ala Arg Ala Gly Gln Val Ile Ser Thr Val Glu Ala Asp Ser
   945                 950                 955                 960
   Ile Val Arg Lys Ser Gly Trp Glu Arg Leu Arg Gly Ala Gln Ser Gln
                   965                 970                 975
   Asn Gly Gln Pro Leu Val Met Arg Leu Val Ile Gly Asp Ser Val Arg
               980                 985                 990
   Met Glu Val Asp Gly Arg Asp Glu Val Met Arg Val Val Lys Met Ser
           995                 1000                1005
   Gly Lys Glu Met Val Phe Ala Pro Val Arg Glu Ala Asn Val Asp Lys
       1010                1015                1020
   Arg Asn Asn Met Pro Asp Glu Gln Asp Pro Phe Thr Tyr Thr Tyr Lys
   1025                1030                1035                1040
   Arg Ala Asp Gln Leu Arg Lys Ala Lys Ala Arg Gln Val Thr Ile Ser
                   1045                1050                1055
   Pro Ile Gly Glu Leu Arg Asp Pro Gly Phe Lys Gly
               1060                1065
<210> 103
   <211> 1368
   <212> PRT
   <213> Streptococcus pyogenes
<220>
   <223> CRISPR-associated endonuclease
<308> YP_598495
   <309> 2013-09-06
<400> 103
   Met Asp Lys Lys Tyr Ser Ile Gly Leu Asp Ile Gly Thr Asn Ser Val
   1               5                   10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
                   260                 265                 270
   Asp Asp Leu Asp Asn Leu Leu Ala Gln Ile Gly Asp Gln Tyr Ala Asp
               275                 280                 285
   Leu Phe Leu Ala Ala Lys Asn Leu Ser Asp Ala Ile Leu Leu Ser Asp
       290                 295                 300
   Ile Leu Arg Leu Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Ala Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
   770 775 780
   Glu Glu Gly Ile Lys Glu Leu Gly Ser Gln Ile Leu Lys Glu His Pro
   785 790 795 800
   Val Glu Asn Thr Gln Leu Gln Asn Glu Lys Leu Tyr Leu Tyr Tyr Leu
   805 810 815
   Gln Asn Gly Arg Asp Met Tyr Val Asp Gln Glu Leu Asp Ile Asn Arg
   820 825 830
   Leu Ser Asp Tyr Asp Val Asp His Ile Val Pro Gln Ser Phe Ile Lys
   835 840 845
   Asp Asp Ser Ile Asp Asn Lys Val Leu Thr Arg Ser Asp Lys Asn Arg
   850 855 860
   Gly Lys Ser Asp Asn Val Pro Ser Glu Glu Val Val Lys Lys Met Lys
   865 870 875 880
   Asn Tyr Trp Arg Gln Leu Leu Asn Ala Lys Leu Ile Thr Gln Arg Lys
   885 890 895
   Phe Asp Asn Leu Thr Lys Ala Glu Arg Gly Gly Leu Ser Glu Leu Asp
   900 905 910
   Lys Ala Gly Phe Ile Lys Arg Gln Leu Val Glu Thr Arg Gln Ile Thr
   915 920 925
   Lys His Val Ala Gln Ile Leu Asp Ser Arg Met Asn Thr Lys Tyr Asp
   930 935 940
   Glu Asn Asp Lys Leu Ile Arg Glu Val Lys Val Ile Thr Leu Lys Ser
   945 950 955 960
   Lys Leu Val Ser Asp Phe Arg Lys Asp Phe Gln Phe Tyr Lys Val Arg
   965 970 975
   Glu Ile Asn Asn Tyr His His Ala His Asp Ala Tyr Leu Asn Ala Val
   980 985 990
   Val Gly Thr Ala Leu Ile Lys Lys Tyr Pro Lys Leu Glu Ser Glu Phe
   995 1000 1005
   Val Tyr Gly Asp Tyr Lys Val Tyr Asp Val Arg Lys Met Ile Ala Lys
   1010 1015 1020
   Ser Glu Gln Glu Ile Gly Lys Ala Thr Ala Lys Tyr Phe Phe Tyr Ser
   1025 1030 1035 1040
   Asn Ile Met Asn Phe Phe Lys Thr Glu Ile Thr Leu Ala Asn Gly Glu
   1045 1050 1055
   Ile Arg Lys Arg Pro Leu Ile Glu Thr Asn Gly Glu Thr Gly Glu Ile
   1060 1065 1070
   Val Trp Asp Lys Gly Arg Asp Phe Ala Thr Val Arg Lys Val Leu Ser
   1075 1080 1085
   Met Pro Gln Val Asn Ile Val Lys Lys Thr Glu Val Gln Thr Gly Gly
   1090 1095 1100
   Phe Ser Lys Glu Ser Ile Leu Pro Lys Arg Asn Ser Asp Lys Leu Ile
   1105 1110 1115 1120
   Ala Arg Lys Lys Asp Trp Asp Pro Lys Lys Tyr Gly Gly Phe Asp Ser
   1125 1130 1135
   Pro Thr Val Ala Tyr Ser Val Leu Val Val Ala Lys Val Glu Lys Gly
   1140 1145 1150
   Lys Ser Lys Lys Leu Lys Ser Val Lys Glu Leu Leu Gly Leu Thr Ile
   1155 1160 1165
   Met Glu Arg Ser Ser Phe Glu Lys Asn Pro Ile Asp Phe Leu Glu Ala
   1170 1175 1180
   Lys Gly Tyr Lys Glu Val Lys Lys Asp Leu Ile Ile Lys Leu Pro Lys
   1185 1190 1195 1200
   Tyr Ser Leu Phe Glu Leu Glu Asn Gly Arg Lys Arg Met Leu Ala Ser
   1205 1210 1215
   Ala Gly Glu Leu Gln Lys Gly Asn Glu Leu Ala Leu Pro Ser Lys Tyr
   1220 1225 1230
   Val Asn Phe Leu Tyr Leu Ala Ser His Tyr Glu Lys Leu Lys Gly Ser
   1235 1240 1245
   Pro Glu Asp Asn Glu Gln Lys Gln Leu Phe Val Glu Gln His Lys His
   1250 1255 1260
   Tyr Leu Asp Glu Ile Ile Glu Gln Ile Ser Glu Phe Ser Lys Arg Val
   1265                1270                1275                1280
   Ile Leu Ala Asp Ala Asn Leu Asp Lys Val Leu Ser Ala Tyr Asn Lys
                   1285                1290                1295
   His Arg Asp Lys Pro Ile Arg Glu Gln Ala Glu Asn Ile Ile His Leu
               1300                1305                1310
   Phe Thr Leu Thr Asn Leu Gly Ala Pro Ala Ala Phe Lys Tyr Phe Asp
           1315                1320                1325
   Thr Thr Ile Asp Arg Lys Arg Tyr Thr Ser Thr Lys Glu Val Leu Asp
       1330                1335                1340
   Ala Thr Leu Ile His Gln Ser Ile Thr Gly Leu Tyr Glu Thr Arg Ile
   1345                1350                1355                1360
   Asp Leu Ser Gln Leu Gly Gly Asp
                   1365
<210> 104
   <211> 1377
   <212> PRT
   <213> Streptococcus agalactiae
<220>
   <223> CRISPR-associated endonuclease
<308> NP_735360
   <309> 2013-06-27
<400> 104
   Met Asn Lys Pro Tyr Ser Ile Gly Leu Asp Ile Gly Thr Asn Ser Val
    1               5                  10                  15
   Gly Trp Ser Ile Ile Thr Asp Asp Tyr Lys Val Pro Ala Lys Lys Met
               20                  25                  30
   Arg Val Leu Gly Asn Thr Asp Lys Glu Tyr Ile Lys Lys Asn Leu Ile
           35                  40                  45
   Gly Ala Leu Leu Phe Asp Gly Gly Asn Thr Ala Ala Asp Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ala Glu Glu Met Ser Lys Val Asp Asp Ser
                   85                  90                  95
   Phe Phe His Arg Leu Glu Asp Ser Phe Leu Val Glu Glu Asp Lys Arg
               100                 105                 110
   Gly Ser Lys Tyr Pro Ile Phe Ala Thr Met Gln Glu Glu Lys Tyr Tyr
           115                 120                 125
   His Glu Lys Phe Pro Thr Ile Tyr His Leu Arg Lys Glu Leu Ala Asp
       130                 135                 140
   Lys Lys Glu Lys Ala Asp Leu Arg Leu Val Tyr Leu Ala Leu Ala His
   145                 150                 155                 160
   Ile Ile Lys Phe Arg Gly His Phe Leu Ile Glu Asp Asp Arg Phe Asp
                   165                 170                 175
   Val Arg Asn Thr Asp Ile Gln Lys Gln Tyr Gln Ala Phe Leu Glu Ile
               180                 185                 190
   Phe Asp Thr Thr Phe Glu Asn Asn His Leu Leu Ser Gln Asn Val Asp
           195                 200                 205
   Val Glu Ala Ile Leu Thr Asp Lys Ile Ser Lys Ser Ala Lys Lys Asp
       210                 215                 220
   Arg Ile Leu Ala Gln Tyr Pro Asn Gln Lys Ser Thr Gly Ile Phe Ala
   225                 230                 235                 240
   Glu Phe Leu Lys Leu Ile Val Gly Asn Gln Ala Asp Phe Lys Lys His
                   245                 250                 255
   Phe Asn Leu Glu Asp Lys Thr Pro Leu Gln Phe Ala Lys Asp Ser Tyr
               260                 265                 270
   Asp Glu Asp Leu Glu Asn Leu Leu Gly Gln Ile Gly Asp Glu Phe Ala
           275                 280                 285
   Asp Leu Phe Ser Val Ala Lys Lys Leu Tyr Asp Ser Val Leu Leu Ser
       290                 295                 300
   Gly Ile Leu Thr Val Thr Asp Leu Ser Thr Lys Ala Pro Leu Ser Ala
   305                 310                 315                 320
   Ser Met Ile Gln Arg Tyr Asp Glu His His Glu Asp Leu Lys His Leu
                   325                 330                 335
   Lys Gln Phe Val Lys Ala Ser Leu Pro Glu Asn Tyr Arg Glu Val Phe
               340                 345                 350
   Ala Asp Ser Ser Lys Asp Gly Tyr Ala Gly Tyr Ile Glu Gly Lys Thr
           355                 360                 365
   Asn Gln Glu Ala Phe Tyr Lys Tyr Leu Leu Lys Leu Leu Thr Lys Gln
       370                 375                 380
   Glu Gly Ser Glu Tyr Phe Leu Glu Lys Ile Lys Asn Glu Asp Phe Leu
   385                 390                 395                 400
   Arg Lys Gln Arg Thr Phe Asp Asn Gly Ser Ile Pro His Gln Val His
                   405                 410                 415
   Leu Thr Glu Leu Arg Ala Ile Ile Arg Arg Gln Ser Glu Tyr Tyr Pro
               420                 425                 430
   Phe Leu Lys Glu Asn Gln Asp Arg Ile Glu Lys Ile Leu Thr Phe Arg
           435                 440                 445
   Ile Pro Tyr Tyr Val Gly Pro Leu Ala Arg Glu Lys Ser Asp Phe Ala
       450                 455                 460
   Trp Met Thr Arg Lys Thr Asp Asp Ser Ile Arg Pro Trp Asn Phe Glu
   465                 470                 475                 480
   Asp Leu Val Asp Lys Glu Lys Ser Ala Glu Ala Phe Ile His Arg Met
                   485                 490                 495
   Thr Asn Asn Asp Leu Tyr Leu Pro Glu Glu Lys Val Leu Pro Lys His
               500                 505                 510
   Ser Leu Ile Tyr Glu Lys Phe Thr Val Tyr Asn Glu Leu Thr Lys Val
           515                 520                 525
   Arg Phe Leu Ala Glu Gly Phe Lys Asp Phe Gln Phe Leu Asn Arg Lys
       530                 535                 540
   Gln Lys Glu Thr Ile Phe Asn Ser Leu Phe Lys Glu Lys Arg Lys Val
   545                 550                 555                 560
   Thr Glu Lys Asp Ile Ile Ser Phe Leu Asn Lys Val Asp Gly Tyr Glu
                   565                 570                 575
   Gly Ile Ala Ile Lys Gly Ile Glu Lys Gln Phe Asn Ala Ser Leu Ser
               580                 585                 590
   Thr Tyr His Asp Leu Lys Lys Ile Leu Gly Lys Asp Phe Leu Asp Asn
           595                 600                 605
   Thr Asp Asn Glu Leu Ile Leu Glu Asp Ile Val Gln Thr Leu Thr Leu
       610                 615                 620
   Phe Glu Asp Arg Glu Met Ile Lys Lys Cys Leu Asp Ile Tyr Lys Asp
   625                 630                 635                 640
   Phe Phe Thr Glu Ser Gln Leu Lys Lys Leu Tyr Arg Arg His Tyr Thr
                   645                 650                 655
   Gly Trp Gly Arg Leu Ser Ala Lys Leu Ile Asn Gly Ile Arg Asn Lys
               660                 665                 670
   Glu Asn Gln Lys Thr Ile Leu Asp Tyr Leu Ile Asp Asp Gly Ser Ala
           675                 680                 685
   Asn Arg Asn Phe Met Gln Leu Ile Asn Asp Asp Asp Leu Ser Phe Lys
       690                 695                 700
   Pro Ile Ile Asp Lys Ala Arg Thr Gly Ser His Ser Asp Asn Leu Lys
   705                 710                 715                 720
   Glu Val Val Gly Glu Leu Ala Gly Ser Pro Ala Ile Lys Lys Gly Ile
                   725                 730                 735
   Leu Gln Ser Leu Lys Ile Val Asp Glu Leu Val Lys Val Met Gly Tyr
               740                 745                 750
   Glu Pro Glu Gln Ile Val Val Glu Met Ala Arg Glu Asn Gln Thr Thr
           755                 760                 765
   Ala Lys Gly Leu Ser Arg Ser Arg Gln Arg Leu Thr Thr Leu Arg Glu
       770                 775                 780
   Ser Leu Ala Asn Leu Lys Ser Asn Ile Leu Glu Glu Lys Lys Pro Lys
   785 790 795 800
   Tyr Val Lys Asp Gln Val Glu Asn His His Leu Ser Asp Asp Arg Leu
   805 810 815
   Phe Leu Tyr Tyr Leu Gln Asn Gly Arg Asp Met Tyr Thr Lys Lys Ala
   820 825 830
   Leu Asp Ile Asp Asn Leu Ser Gln Tyr Asp Ile Asp His Ile Ile Pro
   835 840 845
   Gln Ala Phe Ile Lys Asp Asp Ser Ile Asp Asn Arg Val Leu Val Ser
   850 855 860
   Ser Ala Lys Asn Arg Gly Lys Ser Asp Asp Val Pro Ser Ile Glu Ile
   865 870 875 880
   Val Lys Ala Arg Lys Met Phe Trp Lys Asn Leu Leu Asp Ala Lys Leu
   885 890 895
   Met Ser Gln Arg Lys Tyr Asp Asn Leu Thr Lys Ala Glu Arg Gly Gly
   900 905 910
   Leu Thr Ser Asp Asp Lys Ala Arg Phe Ile Gln Arg Gln Leu Val Glu
   915 920 925
   Thr Arg Gln Ile Thr Lys His Val Ala Arg Ile Leu Asp Glu Arg Phe
   930 935 940
   Asn Asn Glu Val Asp Asn Gly Lys Lys Ile Cys Lys Val Lys Ile Val
   945 950 955 960
   Thr Leu Lys Ser Asn Leu Val Ser Asn Phe Arg Lys Glu Phe Gly Phe
   965 970 975
   Tyr Lys Ile Arg Glu Val Asn Asp Tyr His His Ala His Asp Ala Tyr
   980 985 990
   Leu Asn Ala Val Val Ala Lys Ala Ile Leu Thr Lys Tyr Pro Gln Leu
   995 1000 1005
   Glu Pro Glu Phe Val Tyr Gly Met Tyr Arg Gln Lys Lys Leu Ser Lys
   1010 1015 1020
   Ile Val His Glu Asp Lys Glu Glu Lys Tyr Ser Glu Ala Thr Arg Lys
   1025 1030 1035 1040
   Met Phe Phe Tyr Ser Asn Leu Met Asn Met Phe Lys Arg Val Val Arg
   1045 1050 1055
   Leu Ala Asp Gly Ser Ile Val Val Arg Pro Val Ile Glu Thr Gly Arg
   1060 1065 1070
   Tyr Met Arg Lys Thr Ala Trp Asp Lys Lys Lys His Phe Ala Thr Val
   1075 1080 1085
   Arg Lys Val Leu Ser Tyr Pro Gln Asn Asn Ile Val Lys Lys Thr Glu
   1090 1095 1100
   Ile Gln Thr Gly Gly Phe Ser Lys Glu Ser Ile Leu Ala His Gly Asn
   1105 1110 1115 1120
   Ser Asp Lys Leu Ile Pro Arg Lys Thr Lys Asp Ile Tyr Leu Asp Pro
   1125 1130 1135
   Lys Lys Tyr Gly Gly Phe Asp Ser Pro Ile Val Ala Tyr Ser Val Leu
   1140 1145 1150
   Val Val Ala Asp Ile Lys Lys Gly Lys Ala Gln Lys Leu Lys Thr Val
   1155 1160 1165
   Thr Glu Leu Leu Gly Ile Thr Ile Met Glu Arg Ser Arg Phe Glu Lys
   1170 1175 1180
   Asn Pro Ser Ala Phe Leu Glu Ser Lys Gly Tyr Leu Asn Ile Arg Asp
   1185 1190 1195 1200
   Asp Lys Leu Met Ile Leu Pro Lys Tyr Ser Leu Phe Glu Leu Glu Asn
   1205 1210 1215
   Gly Arg Arg Arg Leu Leu Ala Ser Ala Gly Glu Leu Gln Lys Gly Asn
   1220 1225 1230
   Glu Leu Ala Leu Pro Thr Gln Phe Met Lys Phe Leu Tyr Leu Ala Ser
   1235 1240 1245
   Arg Tyr Asn Glu Ser Lys Gly Lys Pro Glu Glu Ile Glu Lys Lys Gln
   1250 1255 1260
   Glu Phe Val Asn Gln His Val Ser Tyr Phe Asp Asp Ile Leu Gln Leu
   1265 1270 1275 1280
   Ile Asn Asp Phe Ser Lys Arg Val Ile Leu Ala Asp Ala Asn Leu Glu
   1285 1290 1295
   Lys Ile Asn Lys Leu Tyr Gln Asp Asn Lys Glu Asn Ile Pro Val Asp
                   1300                1305                1310
   Glu Leu Ala Asn Asn Ile Ile Asn Leu Phe Thr Phe Thr Ser Leu Gly
               1315                1320                1325
   Ala Pro Ala Ala Phe Lys Phe Phe Asp Lys Ile Val Asp Arg Lys Arg
       1330                1335                1340
   Tyr Thr Ser Thr Lys Glu Val Leu Asn Ser Thr Leu Ile His Gln Ser
   1345                1350                1355                1360
   Ile Thr Gly Leu Tyr Glu Thr Arg Ile Asp Leu Gly Lys Leu Gly Glu
                   1365                1370                1375
   Asp
<210> 105
   <211> 1082
   <212> PRT
   <213> Neisseria meningitidis
<220>
   <223> CRISPR-associated endonuclease
<308> YP_002342100
   <309> 2013-06-10
<400> 105
   Met Ala Ala Phe Lys Pro Asn Pro Ile Asn Tyr Ile Leu Gly Leu Asp
    1               5                  10                  15
   Ile Gly Ile Ala Ser Val Gly Trp Ala Met Val Glu Ile Asp Glu Asp
               20                  25                  30
   Glu Asn Pro Ile Cys Leu Ile Asp Leu Gly Val Arg Val Phe Glu Arg
           35                  40                  45
   Ala Glu Val Pro Lys Thr Gly Asp Ser Leu Ala Met Ala Arg Arg Leu
       50                  55                  60
   Ala Arg Ser Val Arg Arg Leu Thr Arg Arg Arg Ala His Arg Leu Leu
   65                  70                  75                  80
   Arg Ala Arg Arg Leu Leu Lys Arg Glu Gly Val Leu Gln Ala Ala Asp
                   85                  90                  95
   Phe Asp Glu Asn Gly Leu Ile Lys Ser Leu Pro Asn Thr Pro Trp Gln
               100                 105                 110
   Leu Arg Ala Ala Ala Leu Asp Arg Lys Leu Thr Pro Leu Glu Trp Ser
           115                 120                 125
   Ala Val Leu Leu His Leu Ile Lys His Arg Gly Tyr Leu Ser Gln Arg
       130                 135                 140
   Lys Asn Glu Gly Glu Thr Ala Asp Lys Glu Leu Gly Ala Leu Leu Lys
   145                 150                 155                 160
   Gly Val Ala Asp Asn Ala His Ala Leu Gln Thr Gly Asp Phe Arg Thr
                   165                 170                 175
   Pro Ala Glu Leu Ala Leu Asn Lys Phe Glu Lys Glu Ser Gly His Ile
               180                 185                 190
   Arg Asn Gln Arg Gly Asp Tyr Ser His Thr Phe Ser Arg Lys Asp Leu
           195                 200                 205
   Gln Ala Glu Leu Ile Leu Leu Phe Glu Lys Gln Lys Glu Phe Gly Asn
       210                 215                 220
   Pro His Val Ser Gly Gly Leu Lys Glu Gly Ile Glu Thr Leu Leu Met
   225                 230                 235                 240
   Thr Gln Arg Pro Ala Leu Ser Gly Asp Ala Val Gln Lys Met Leu Gly
                   245                 250                 255
   His Cys Thr Phe Glu Pro Ala Glu Pro Lys Ala Ala Lys Asn Thr Tyr
               260                 265                 270
   Thr Ala Glu Arg Phe Ile Trp Leu Thr Lys Leu Asn Asn Leu Arg Ile
           275                 280                 285
   Leu Glu Gln Gly Ser Glu Arg Pro Leu Thr Asp Thr Glu Arg Ala Thr
       290                 295                 300
   Leu Met Asp Glu Pro Tyr Arg Lys Ser Lys Leu Thr Tyr Ala Gln Ala
   305                 310                 315                 320
   Arg Lys Leu Leu Gly Leu Glu Asp Thr Ala Phe Phe Lys Gly Leu Arg
                   325                 330                 335
   Tyr Gly Lys Asp Asn Ala Glu Ala Ser Thr Leu Met Glu Met Lys Ala
               340                 345                 350
   Tyr His Ala Ile Ser Arg Ala Leu Glu Lys Glu Gly Leu Lys Asp Lys
           355                 360                 365
   Lys Ser Pro Leu Asn Leu Ser Pro Glu Leu Gln Asp Glu Ile Gly Thr
       370                 375                 380
   Ala Phe Ser Leu Phe Lys Thr Asp Glu Asp Ile Thr Gly Arg Leu Lys
   385                 390                 395                 400
   Asp Arg Ile Gln Pro Glu Ile Leu Glu Ala Leu Leu Lys His Ile Ser
                   405                 410                 415
   Phe Asp Lys Phe Val Gln Ile Ser Leu Lys Ala Leu Arg Arg Ile Val
               420                 425                 430
   Pro Leu Met Glu Gln Gly Lys Arg Tyr Asp Glu Ala Cys Ala Glu Ile
           435                 440                 445
   Tyr Gly Asp His Tyr Gly Lys Lys Asn Thr Glu Glu Lys Ile Tyr Leu
       450                 455                 460
   Pro Pro Ile Pro Ala Asp Glu Ile Arg Asn Pro Val Val Leu Arg Ala
   465                 470                 475                 480
   Leu Ser Gln Ala Arg Lys Val Ile Asn Gly Val Val Arg Arg Tyr Gly
                   485                 490                 495
   Ser Pro Ala Arg Ile His Ile Glu Thr Ala Arg Glu Val Gly Lys Ser
               500                 505                 510
   Phe Lys Asp Arg Lys Glu Ile Glu Lys Arg Gln Glu Glu Asn Arg Lys
           515                 520                 525
   Asp Arg Glu Lys Ala Ala Ala Lys Phe Arg Glu Tyr Phe Pro Asn Phe
       530                 535                 540
   Val Gly Glu Pro Lys Ser Lys Asp Ile Leu Lys Leu Arg Leu Tyr Glu
   545                 550                 555                 560
   Gln Gln His Gly Lys Cys Leu Tyr Ser Gly Lys Glu Ile Asn Leu Gly
                   565                 570                 575
   Arg Leu Asn Glu Lys Gly Tyr Val Glu Ile Asp His Ala Leu Pro Phe
               580                 585                 590
   Ser Arg Thr Trp Asp Asp Ser Phe Asn Asn Lys Val Leu Val Leu Gly
           595                 600                 605
   Ser Glu Asn Gln Asn Lys Gly Asn Gln Thr Pro Tyr Glu Tyr Phe Asn
       610                 615                 620
   Gly Lys Asp Asn Ser Arg Glu Trp Gln Glu Phe Lys Ala Arg Val Glu
   625                 630                 635                 640
   Thr Ser Arg Phe Pro Arg Ser Lys Lys Gln Arg Ile Leu Leu Gln Lys
                   645                 650                 655
   Phe Asp Glu Asp Gly Phe Lys Glu Arg Asn Leu Asn Asp Thr Arg Tyr
               660                 665                 670
   Val Asn Arg Phe Leu Cys Gln Phe Val Ala Asp Arg Met Arg Leu Thr
           675                 680                 685
   Gly Lys Gly Lys Lys Arg Val Phe Ala Ser Asn Gly Gln Ile Thr Asn
       690                 695                 700
   Leu Leu Arg Gly Phe Trp Gly Leu Arg Lys Val Arg Ala Glu Asn Asp
   705                 710                 715                 720
   Arg His His Ala Leu Asp Ala Val Val Val Ala Cys Ser Thr Val Ala
                   725                 730                 735
   Met Gln Gln Lys Ile Thr Arg Phe Val Arg Tyr Lys Glu Met Asn Ala
               740                 745                 750
   Phe Asp Gly Lys Thr Ile Asp Lys Glu Thr Gly Glu Val Leu His Gln
           755                 760                 765
   Lys Thr His Phe Pro Gln Pro Trp Glu Phe Phe Ala Gln Glu Val Met
       770                 775                 780
   Ile Arg Val Phe Gly Lys Pro Asp Gly Lys Pro Glu Phe Glu Glu Ala
   785                 790                 795                 800
   Asp Thr Pro Glu Lys Leu Arg Thr Leu Leu Ala Glu Lys Leu Ser Ser
                   805                 810                 815
   Arg Pro Glu Ala Val His Glu Tyr Val Thr Pro Leu Phe Val Ser Arg
               820                 825                 830
   Ala Pro Asn Arg Lys Met Ser Gly Gln Gly His Met Glu Thr Val Lys
           835                 840                 845
   Ser Ala Lys Arg Leu Asp Glu Gly Val Ser Val Leu Arg Val Pro Leu
       850                 855                 860
   Thr Gln Leu Lys Leu Lys Asp Leu Glu Lys Met Val Asn Arg Glu Arg
   865                 870                 875                 880
   Glu Pro Lys Leu Tyr Glu Ala Leu Lys Ala Arg Leu Glu Ala His Lys
                   885                 890                 895
   Asp Asp Pro Ala Lys Ala Phe Ala Glu Pro Phe Tyr Lys Tyr Asp Lys
               900                 905                 910
   Ala Gly Asn Arg Thr Gln Gln Val Lys Ala Val Arg Val Glu Gln Val
           915                 920                 925
   Gln Lys Thr Gly Val Trp Val Arg Asn His Asn Gly Ile Ala Asp Asn
       930                 935                 940
   Ala Thr Met Val Arg Val Asp Val Phe Glu Lys Gly Asp Lys Tyr Tyr
   945                 950                 955                 960
   Leu Val Pro Ile Tyr Ser Trp Gln Val Ala Lys Gly Ile Leu Pro Asp
                   965                 970                 975
   Arg Ala Val Val Gln Gly Lys Asp Glu Glu Asp Trp Gln Leu Ile Asp
               980                 985                 990
   Asp Ser Phe Asn Phe Lys Phe Ser Leu His Pro Asn Asp Leu Val Glu
           995                 1000                1005
   Val Ile Thr Lys Lys Ala Arg Met Phe Gly Tyr Phe Ala Ser Cys His
       1010                1015                1020
   Arg Gly Thr Gly Asn Ile Asn Ile Arg Ile His Asp Leu Asp His Lys
   1025                1030                1035                1040
   Ile Gly Lys Asn Gly Ile Leu Glu Gly Ile Gly Val Lys Thr Ala Leu
                   1045                1050                1055
   Ser Phe Gln Lys Tyr Gln Ile Asp Glu Leu Gly Lys Glu Ile Arg Pro
               1060                1065                1070
   Cys Arg Leu Lys Lys Arg Pro Pro Val Arg
           1075                1080
<210> 106
   <211> 1430
   <212> PRT
   <213> Capnocytophaga canimorsus
<220>
   <223> CRISPR-associated endonuclease
<308> YP_004740688
   <309> 2013-06-11
<400> 106
   Met Lys His Ile Leu Gly Leu Asp Leu Gly Thr Asn Ser Ile Gly Trp
    1               5                  10                  15
   Ala Val Val Lys Glu Ala Gln Asn Asp Asn Glu Gln Ser Glu Ile Val
               20                  25                  30
   Asp Val Gly Val Arg Val Asn Pro Leu Thr Thr Asp Glu Lys Thr Asn
           35                  40                  45
   Phe Glu Lys Gly Lys Pro Ile Ser Thr Asn Ala Asp Arg Thr Leu Lys
       50                  55                  60
   Arg Ser Ala Arg Arg Asn Leu Gln Arg Tyr Lys Leu Arg Arg Lys Glu
   65                  70                  75                  80
   Leu Ile Glu Leu Leu Lys Glu His Gln Leu Ile Ser Thr Asp Thr Ile
                   85                  90                  95
   Leu Thr Glu Asn Gly Lys Gly Thr Thr Phe Gln Thr Leu Glu Leu Arg
                   100                 105                 110
   Ala Lys Ser Ala Lys Glu Lys Ile Ser Leu Glu Glu Leu Ala Arg Val
               115                 120                 125
   Phe Leu Thr Ile Asn Lys Lys Arg Gly Tyr Lys Ser Ser Arg Lys Ala
       130                 135                 140
   Lys Asn Glu Asp Glu Gly Gln Leu Ile Asp Gly Met Glu Ile Ala Lys
   145                 150                 155                 160
   Arg Leu Tyr Glu Glu Asn Leu Thr Thr Gly Gln Leu Ser Leu Gln Leu
                   165                 170                 175
   Leu Lys Glu Gly Lys Lys Ser Leu Pro Asp Tyr Tyr Arg Ser Asp Leu
               180                 185                 190
   Gln Phe Glu Leu Asp Lys Val Trp Asn Phe Gln Lys Gln Phe His Ser
           195                 200                 205
   His Ile Leu Thr Asp Glu Phe Tyr Ala Gln Leu Lys Gly Lys Gly Gln
       210                 215                 220
   Arg Ala Thr Ser Ala Leu Phe Leu Ala Lys Tyr Gln Ile Tyr Thr Ala
   225                 230                 235                 240
   Glu Asn Lys Gly Ser Arg Glu Asp Lys Lys Leu Gln Ala Tyr Gln Trp
                   245                 250                 255
   Arg Val Asp Ala Leu Ser Lys Glu Leu Thr Arg Glu Gln Leu Ala Phe
               260                 265                 270
   Val Ile Thr Glu Ile Asn Asn Asn Leu Asn Asn Ser Ser Gly Tyr Leu
           275                 280                 285
   Gly Ala Ile Ser Asp Arg Ser Lys Glu Leu Tyr Phe Asn Asn Glu Thr
       290                 295                 300
   Val Gly Glu Tyr Leu Trp Lys Gln Ile Ala Ala Asn Pro His Thr Ser
   305                 310                 315                 320
   Leu Lys Asn Gln Val Phe Tyr Arg Gln Asp Tyr Leu Asp Glu Phe Glu
                   325                 330                 335
   Gln Ile Trp Lys Thr Gln Ser Ala Phe Tyr Pro Glu Ile Leu Thr Asp
               340                 345                 350
   Glu Leu Lys Lys Glu Leu Arg Asp Val Val Ile Phe Tyr Gln Arg Lys
           355                 360                 365
   Leu Lys Ser Gln Lys Ser Leu Val Ser Phe Cys Glu Phe Glu Asn Lys
       370                 375                 380
   Glu Ile Glu Ile Lys Lys Glu Asp Gly Thr Ser Thr Lys Lys Val Ile
   385                 390                 395                 400
   Gly Leu Lys Val Ala Pro Lys Ser Ser Pro Leu Phe Gln Glu Phe Lys
                   405                 410                 415
   Ile Trp Gln Asn Leu Asn Asn Val Leu Ile Arg Lys Lys Gly Ser Lys
               420                 425                 430
   Thr Lys Lys Val Asp Lys Asn Gln Gln Ala Val Leu Phe Glu Glu Glu
           435                 440                 445
   Lys Thr Ile Phe Glu Leu Asp Leu Glu Ala Lys Gln Ala Leu Phe Asn
       450                 455                 460
   Glu Leu Asn Leu Lys Gly Asn Leu Lys Ala Tyr Tyr Cys Leu Glu Ile
   465                 470                 475                 480
   Leu Gly Tyr Ser Pro Lys Glu Trp Glu Met Asn Tyr Ser Val Leu Glu
                   485                 490                 495
   Gly Asn Arg Thr Asn Lys Ala Leu Tyr Glu Ala Tyr Leu Lys Ile Thr
               500                 505                 510
   Asp Leu Glu Gly Tyr Asp Ala Lys Asn His Leu Lys Val Lys Leu Asn
           515                 520                 525
   Lys Asp Glu Val Glu Leu Asp Asp Leu Lys Val Pro Ala Ser Glu Ile
       530                 535                 540
   Lys Asp Met Val Gln Ala Ile Phe Lys Glu Ala Gly Ile Asn Thr Asp
   545                 550                 555                 560
   Ile Leu His Phe Asn Ala Glu Leu Asp Gly Lys Asp Phe Glu Asn Gln
                   565                 570                 575
   Ala Ser Tyr Gln Leu Trp His Leu Leu Tyr Ser Tyr Glu Gly Asp Asp
               580                 585                 590
   Ser Lys Ser Gly Asn Glu Leu Leu Tyr Lys Leu Leu Glu Thr Lys Phe
           595                 600                 605
   Gly Phe Lys Lys Glu Tyr Ala Gln Ile Leu Ala Asn Val Ser Leu Ser
       610                 615                 620
   Asp Asp Tyr Gly Ser Leu Ser Thr Lys Ala Met Arg Lys Ile His Pro
   625                 630                 635                 640
   Tyr Ile Lys Glu Asn Lys Phe Ser Thr Ala Cys Glu Leu Ala Gly Tyr
                   645                 650                 655
   Arg His Ser Lys His Ser Leu Thr Lys Glu Glu Asn Asp Asn Arg Val
               660                 665                 670
   Leu Lys Asp Ser Leu Glu Val Leu Lys Lys Asn Ser Leu Arg Gln Pro
           675                 680                 685
   Val Val Glu Lys Ile Leu Asn Gln Met Ile Asn Leu Val Asn Ala Leu
       690                 695                 700
   Leu Lys Lys Tyr Arg Thr Glu Asn Pro Asn Phe Lys Phe Asp Glu Ile
   705                 710                 715                 720
   Arg Ile Glu Leu Ala Arg Glu Leu Lys Lys Asn Ala Glu Glu Arg Ala
                   725                 730                 735
   Asn Met Thr Val Asn Ile Asn Lys Ala Lys Thr Glu His Glu Lys Ile
               740                 745                 750
   Ile Lys Ile Leu Lys Asn Glu Phe Gly Leu Pro Asn Pro Thr Arg Asn
           755                 760                 765
   Asp Ile Ile Arg Tyr Arg Leu Tyr Glu Glu Leu Lys Ala Asn Gly Tyr
       770                 775                 780
   Lys Asp Leu Tyr Thr Asn Glu Tyr Ile Glu Lys Gln Asp Leu Phe Thr
   785                 790                 795                 800
   Lys Asn Ile Asp Ile Glu His Ile Ile Pro Lys Ser Arg Val Phe Asp
                   805                 810                 815
   Asp Ser Phe Ser Asn Lys Thr Leu Ser Phe Arg Lys Thr Asn Leu Asp
               820                 825                 830
   Lys Gly Glu Arg Thr Ala Phe Asp Tyr Ile Glu Ser Lys Phe Gly Ala
           835                 840                 845
   Asp Lys Leu Glu Glu Tyr Val Ala Arg Val Glu Asp Leu Tyr Lys Asn
       850                 855                 860
   Asn Ile Ile Ser Lys Ala Lys Tyr Gln Lys Leu Leu Lys Lys Glu Ser
   865                 870                 875                 880
   Glu Ile Gly Asp Gly Phe Val Glu Arg Asp Leu Arg Glu Thr Gln Tyr
                   885                 890                 895
   Ile Ala Lys Lys Ala Lys Glu Ile Leu Leu Gln Ile Thr Pro Asn Val
               900                 905                 910
   Leu Ser Thr Ser Gly Gly Ile Thr Asp Arg Leu Arg Glu Asp Trp Asp
           915                 920                 925
   Leu Ile Asn Val Met Lys Glu Leu Asn Leu Pro Lys Tyr Arg Ala Leu
       930                 935                 940
   Gly Leu Thr Gln Met Glu Asp Arg Lys Tyr Gly Gln Gln Val Glu Val
   945                 950                 955                 960
   Ile Ile Asp Trp Thr Lys Arg Asn Asp His Arg His His Ala Met Asp
                   965                 970                 975
   Ala Leu Thr Val Ala Phe Thr Lys His Asn His Ile Gln Tyr Leu Asn
               980                 985                 990
   Tyr Leu Asn Ala Arg Lys Asn Glu Lys His Arg Glu His Ser Asn Ile
           995                 1000                1005
   Met Gly Ile Gln Gln Leu Glu Thr Ile Lys Val Thr Asp Lys Asn Gly
       1010                1015                1020
   Asn Glu Lys Arg Val Phe Lys Ala Pro Met Pro Asn Phe Arg Gln Val
   1025                1030                1035                1040
   Thr Lys Ala Phe Leu Glu Asn Val Leu Ile Ser His Lys Ala Lys Asn
                   1045                1050                1055
   Lys Val Val Thr Lys Asn Lys Asn Lys Val Ala Gly Ser Asn Lys Val
               1060                1065                1070
   Gln Glu Val Leu Thr Pro Arg Gly Gln Leu His Lys Glu Thr Val Tyr
           1075                1080                1085
   Gly Lys Tyr Gln Tyr Tyr Val Gln Lys Glu Glu Lys Ile Gly Gly Lys
       1090                1095                1100
   Phe Asp Leu Glu Thr Ile Lys Lys Val Ala Asn Pro Leu Tyr Gln Ser
   1105                1110                1115                1120
   Leu Leu Lys Lys Arg Leu Glu Glu Asn Gly Gly Asp Pro Lys Lys Ala
                   1125                1130                1135
   Phe Thr Gly Lys Asn Ser Leu Ala Lys Asn Pro Ile Tyr Leu Asp Asp
               1140                1145                1150
   Ala Gln Thr Glu Gln Leu Pro Glu Lys Val Lys Leu Val Trp Leu Glu
           1155                1160                1165
   Asn Ser Phe Ser Ile Arg Lys Asp Ile Thr Pro Glu Asn Phe Lys Asp
       1170                1175                1180
   Glu Lys Leu Ile Asp Lys Ile Leu Asp Glu Gly Val Lys Arg Ile Leu
   1185                1190                1195                1200
   Lys Glu Arg Leu Gln Ser Phe Gly Asn Asp Ala Lys Lys Ala Phe Ser
                   1205                1210                1215
   Asp Leu Asp Lys Asn Pro Ile Trp Leu Asn Lys Glu Lys Gly Ile Ala
               1220                1225                1230
   Ile Lys Arg Val Ala Ile Ser Gly Val Ala Asn Ala Glu Pro Leu His
           1235                1240                1245
   Thr Lys Lys Asp His Leu Gly Lys Glu Ile Leu Asp Lys Glu Gly Lys
       1250                1255                1260
   Ser Ile Pro Val Asp Phe Ile Ser Thr Gly Asn Asn His His Val Ala
   1265                1270                1275                1280
   Val Tyr Arg Asp Glu Lys Gly Asn Leu Gln Glu Lys Val Val Ser Val
                   1285                1290                1295
   Phe Glu Ala Val Val Arg Ala Asn Gln Gly Leu Pro Ile Ile Asp Lys
               1300                1305                1310
   Thr Tyr Asn Gln His Leu Gly Trp Glu Phe Leu Phe Thr Met Lys Gln
           1315                1320                1325
   Asn Glu Met Phe Val Phe Ser Asn Glu Ser Thr Gly Phe Asn Pro Asn
       1330                1335                1340
   Asp Ile Asp Leu Leu Asp Pro Lys Asn Lys Lys Ile Ile Ser Pro Asn
   1345                1350                1355                1360
   Leu Phe Arg Val Gln Lys Phe Ser Lys Leu Gln Tyr Gly Asn Ser Phe
                   1365                1370                1375
   Val Arg Glu Phe Val Phe Arg His His Leu Glu Thr Thr Val Glu Asp
               1380                1385                1390
   Arg Lys Glu Leu Gln Gly Ile Thr Tyr Lys Gln Met Lys Asn Ser Ala
           1395                1400                1405
   Phe Phe Asn Asp Ile Ile Lys Val Arg Ile Asn His Leu Gly Gly Ile
       1410                1415                1420
   Val Ser Val Gly Glu Tyr
   1425                1430
<210> 107
   <211> 1372
   <212> PRT
   <213> Legionella pneumophila
<220>
   <223> CRISPR-associated endonuclease
<308> YP_122507
   <309> 2013-06-10
<400> 107
   Met Glu Ser Ser Gln Ile Leu Ser Pro Ile Gly Ile Asp Leu Gly Gly
    1              5                   10                  15
   Lys Phe Thr Gly Val Cys Leu Ser His Leu Glu Ala Phe Ala Glu Leu
               20                  25                  30
   Pro Asn His Ala Asn Thr Lys Tyr Ser Val Ile Leu Ile Asp His Asn
           35                  40                  45
   Asn Phe Gln Leu Ser Gln Ala Gln Arg Arg Ala Thr Arg His Arg Val
       50                  55                  60
   Arg Asn Lys Lys Arg Asn Gln Phe Val Lys Arg Val Ala Leu Gln Leu
   65 70 75 80
   Phe Gln His Ile Leu Ser Arg Asp Leu Asn Ala Lys Glu Glu Thr Ala
   85 90 95
   Leu Cys His Tyr Leu Asn Asn Arg Gly Tyr Thr Tyr Val Asp Thr Asp
   100 105 110
   Leu Asp Glu Tyr Ile Lys Asp Glu Thr Thr Ile Asn Leu Leu Lys Glu
   115 120 125
   Leu Leu Pro Ser Glu Ser Glu His Asn Phe Ile Asp Trp Phe Leu Gln
   130 135 140
   Lys Met Gln Ser Ser Glu Phe Arg Lys Ile Leu Val Ser Lys Val Glu
   145 150 155 160
   Glu Lys Lys Asp Asp Lys Glu Leu Lys Asn Ala Val Lys Asn Ile Lys
   165 170 175
   Asn Phe Ile Thr Gly Phe Glu Lys Asn Ser Val Glu Gly His Arg His
   180 185 190
   Arg Lys Val Tyr Phe Glu Asn Ile Lys Ser Asp Ile Thr Lys Asp Asn
   195 200 205
   Gln Leu Asp Ser Ile Lys Lys Lys Ile Pro Ser Val Cys Leu Ser Asn
   210 215 220
   Leu Leu Gly His Leu Ser Asn Leu Gln Trp Lys Asn Leu His Arg Tyr
   225 230 235 240
   Leu Ala Lys Asn Pro Lys Gln Phe Asp Glu Gln Thr Phe Gly Asn Glu
   245 250 255
   Phe Leu Arg Met Leu Lys Asn Phe Arg His Leu Lys Gly Ser Gln Glu
   260 265 270
   Ser Leu Ala Val Arg Asn Leu Ile Gln Gln Leu Glu Gln Ser Gln Asp
   275 280 285
   Tyr Ile Ser Ile Leu Glu Lys Thr Pro Pro Glu Ile Thr Ile Pro Pro
   290 295 300
   Tyr Glu Ala Arg Thr Asn Thr Gly Met Glu Lys Asp Gln Ser Leu Leu
   305 310 315 320
   Leu Asn Pro Glu Lys Leu Asn Asn Leu Tyr Pro Asn Trp Arg Asn Leu
   325 330 335
   Ile Pro Gly Ile Ile Asp Ala His Pro Phe Leu Glu Lys Asp Leu Glu
   340 345 350
   His Thr Lys Leu Arg Asp Arg Lys Arg Ile Ile Ser Pro Ser Lys Gln
   355 360 365
   Asp Glu Lys Arg Asp Ser Tyr Ile Leu Gln Arg Tyr Leu Asp Leu Asn
   370 375 380
   Lys Lys Ile Asp Lys Phe Lys Ile Lys Lys Gln Leu Ser Phe Leu Gly
   385 390 395 400
   Gln Gly Lys Gln Leu Pro Ala Asn Leu Ile Glu Thr Gln Lys Glu Met
   405 410 415
   Glu Thr His Phe Asn Ser Ser Leu Val Ser Val Leu Ile Gln Ile Ala
   420 425 430
   Ser Ala Tyr Asn Lys Glu Arg Glu Asp Ala Ala Gln Gly Ile Trp Phe
   435 440 445
   Asp Asn Ala Phe Ser Leu Cys Glu Leu Ser Asn Ile Asn Pro Pro Arg
   450 455 460
   Lys Gln Lys Ile Leu Pro Leu Leu Val Gly Ala Ile Leu Ser Glu Asp
   465 470 475 480
   Phe Ile Asn Asn Lys Asp Lys Trp Ala Lys Phe Lys Ile Phe Trp Asn
   485 490 495
   Thr His Lys Ile Gly Arg Thr Ser Leu Lys Ser Lys Cys Lys Glu Ile
   500 505 510
   Glu Glu Ala Arg Lys Asn Ser Gly Asn Ala Phe Lys Ile Asp Tyr Glu
   515 520 525
   Glu Ala Leu Asn His Pro Glu His Ser Asn Asn Lys Ala Leu Ile Lys
   530 535 540
   Ile Ile Gln Thr Ile Pro Asp Ile Ile Gln Ala Ile Gln Ser His Leu
   545 550 555 560
   Gly His Asn Asp Ser Gln Ala Leu Ile Tyr His Asn Pro Phe Ser Leu
                   565                 570                 575
   Ser Gln Leu Tyr Thr Ile Leu Glu Thr Lys Arg Asp Gly Phe His Lys
               580                 585                 590
   Asn Cys Val Ala Val Thr Cys Glu Asn Tyr Trp Arg Ser Gln Lys Thr
           595                 600                 605
   Glu Ile Asp Pro Glu Ile Ser Tyr Ala Ser Arg Leu Pro Ala Asp Ser
       610                 615                 620
   Val Arg Pro Phe Asp Gly Val Leu Ala Arg Met Met Gln Arg Leu Ala
   625                 630                 635                 640
   Tyr Glu Ile Ala Met Ala Lys Trp Glu Gln Ile Lys His Ile Pro Asp
                   645                 650                 655
   Asn Ser Ser Leu Leu Ile Pro Ile Tyr Leu Glu Gln Asn Arg Phe Glu
               660                 665                 670
   Phe Glu Glu Ser Phe Lys Lys Ile Lys Gly Ser Ser Ser Asp Lys Thr
           675                 680                 685
   Leu Glu Gln Ala Ile Glu Lys Gln Asn Ile Gln Trp Glu Glu Lys Phe
       690                 695                 700
   Gln Arg Ile Ile Asn Ala Ser Met Asn Ile Cys Pro Tyr Lys Gly Ala
   705                 710                 715                 720
   Ser Ile Gly Gly Gln Gly Glu Ile Asp His Ile Tyr Pro Arg Ser Leu
                   725                 730                 735
   Ser Lys Lys His Phe Gly Val Ile Phe Asn Ser Glu Val Asn Leu Ile
               740                 745                 750
   Tyr Cys Ser Ser Gln Gly Asn Arg Glu Lys Lys Glu Glu His Tyr Leu
           755                 760                 765
   Leu Glu His Leu Ser Pro Leu Tyr Leu Lys His Gln Phe Gly Thr Asp
       770                 775                 780
   Asn Val Ser Asp Ile Lys Asn Phe Ile Ser Gln Asn Val Ala Asn Ile
   785                 790                 795                 800
   Lys Lys Tyr Ile Ser Phe His Leu Leu Thr Pro Glu Gln Gln Lys Ala
                   805                 810                 815
   Ala Arg His Ala Leu Phe Leu Asp Tyr Asp Asp Glu Ala Phe Lys Thr
               820                 825                 830
   Ile Thr Lys Phe Leu Met Ser Gln Gln Lys Ala Arg Val Asn Gly Thr
           835                 840                 845
   Gln Lys Phe Leu Gly Lys Gln Ile Met Glu Phe Leu Ser Thr Leu Ala
       850                 855                 860
   Asp Ser Lys Gln Leu Gln Leu Glu Phe Ser Ile Lys Gln Ile Thr Ala
   865                 870                 875                 880
   Glu Glu Val His Asp His Arg Glu Leu Leu Ser Lys Gln Glu Pro Lys
                   885                 890                 895
   Leu Val Lys Ser Arg Gln Gln Ser Phe Pro Ser His Ala Ile Asp Ala
               900                 905                 910
   Thr Leu Thr Met Ser Ile Gly Leu Lys Glu Phe Pro Gln Phe Ser Gln
           915                 920                 925
   Glu Leu Asp Asn Ser Trp Phe Ile Asn His Leu Met Pro Asp Glu Val
       930                 935                 940
   His Leu Asn Pro Val Arg Ser Lys Glu Lys Tyr Asn Lys Pro Asn Ile
   945                 950                 955                 960
   Ser Ser Thr Pro Leu Phe Lys Asp Ser Leu Tyr Ala Glu Arg Phe Ile
                   965                 970                 975
   Pro Val Trp Val Lys Gly Glu Thr Phe Ala Ile Gly Phe Ser Glu Lys
               980                 985                 990
   Asp Leu Phe Glu Ile Lys Pro Ser Asn Lys Glu Lys Leu Phe Thr Leu
           995                 1000                1005
   Leu Lys Thr Tyr Ser Thr Lys Asn Pro Gly Glu Ser Leu Gln Glu Leu
       1010                1015                1020
   Gln Ala Lys Ser Lys Ala Lys Trp Leu Tyr Phe Pro Ile Asn Lys Thr
   1025                1030                1035                1040
   Leu Ala Leu Glu Phe Leu His His Tyr Phe His Lys Glu Ile Val Thr
                   1045                1050                1055
   Pro Asp Asp Thr Thr Val Cys His Phe Ile Asn Ser Leu Arg Tyr Tyr
               1060                1065                1070
   Thr Lys Lys Glu Ser Ile Thr Val Lys Ile Leu Lys Glu Pro Met Pro
           1075                1080                1085
   Val Leu Ser Val Lys Phe Glu Ser Ser Lys Lys Asn Val Leu Gly Ser
       1090                1095                1100
   Phe Lys His Thr Ile Ala Leu Pro Ala Thr Lys Asp Trp Glu Arg Leu
   1105                1110                1115                1120
   Phe Asn His Pro Asn Phe Leu Ala Leu Lys Ala Asn Pro Ala Pro Asn
                   1125                1130                1135
   Pro Lys Glu Phe Asn Glu Phe Ile Arg Lys Tyr Phe Leu Ser Asp Asn
               1140                1145                1150
   Asn Pro Asn Ser Asp Ile Pro Asn Asn Gly His Asn Ile Lys Pro Gln
           1155                1160                1165
   Lys His Lys Ala Val Arg Lys Val Phe Ser Leu Pro Val Ile Pro Gly
       1170                1175                1180
   Asn Ala Gly Thr Met Met Arg Ile Arg Arg Lys Asp Asn Lys Gly Gln
   1185                1190                1195                1200
   Pro Leu Tyr Gln Leu Gln Thr Ile Asp Asp Thr Pro Ser Met Gly Ile
                   1205                1210                1215
   Gln Ile Asn Glu Asp Arg Leu Val Lys Gln Glu Val Leu Met Asp Ala
               1220                1225                1230
   Tyr Lys Thr Arg Asn Leu Ser Thr Ile Asp Gly Ile Asn Asn Ser Glu
           1235                1240                1245
   Gly Gln Ala Tyr Ala Thr Phe Asp Asn Trp Leu Thr Leu Pro Val Ser
       1250                1255                1260
   Thr Phe Lys Pro Glu Ile Ile Lys Leu Glu Met Lys Pro His Ser Lys
   1265                1270                1275                1280
   Thr Arg Arg Tyr Ile Arg Ile Thr Gln Ser Leu Ala Asp Phe Ile Lys
                   1285                1290                1295
   Thr Ile Asp Glu Ala Leu Met Ile Lys Pro Ser Asp Ser Ile Asp Asp
               1300                1305                1310
   Pro Leu Asn Met Pro Asn Glu Ile Val Cys Lys Asn Lys Leu Phe Gly
           1315                1320                1325
   Asn Glu Leu Lys Pro Arg Asp Gly Lys Met Lys Ile Val Ser Thr Gly
       1330                1335                1340
   Lys Ile Val Thr Tyr Glu Phe Glu Ser Asp Ser Thr Pro Gln Trp Ile
   1345                1350                1355                1360
   Gln Thr Leu Tyr Val Thr Gln Leu Lys Lys Gln Pro
                   1365                1370
<210> 108
   <211> 346
   <212> PRT
   <213> Clostridium beijerinckii
<220>
   <223> CRISPR-associated endonuclease
<308> YP_001309203
   <309> 2013-06-10
<400> 108
   Met Tyr Thr Ile Glu Glu Ile Leu Glu Met Lys Tyr Pro Tyr Glu Leu
    1               5                  10                  15
   Phe Thr Gly Asn Lys Glu Thr Ala Lys Lys Glu His Ile Asp Leu Leu
               20                  25                  30
   Lys Ile Phe His Pro Asp Leu His Arg Asp Ser Glu Lys Tyr Lys Glu
           35                  40                  45
   Val Thr Ser Lys Ile Asn Glu Leu Tyr Ser Glu Thr Val Lys Leu Leu
       50                  55                  60
   Asp Asn Gly Asn Trp Ile Glu Asp Gly Leu Ile Lys Ile Thr Ser Ser
   65                  70                  75                  80
   Asp Gly Glu Lys Tyr Ser Met Lys Tyr Asn Val Glu Tyr Ser Phe Glu
                   85                  90                  95
   Leu Gly Ser Tyr Phe Ile Gly Asn Arg Ser Ile Leu Tyr Leu Ile Asp
               100                 105                 110
   Lys Lys His Lys Asp Phe Val Glu Asn Ala Leu Asp Arg Ile Lys Asn
           115                 120                 125
   Leu Lys Tyr Glu Asn Asn Asn Met Lys Ser Glu Phe Glu Lys Tyr Phe
       130                 135                 140
   Pro Lys Val Leu Lys Arg Phe Glu Thr Met Asp Lys Lys Ile Gly Leu
   145                 150                 155                 160
   Leu Ile Glu Lys Pro Glu Asp Ala Tyr Ser Leu Lys Asp Ile Leu Ser
                   165                 170                 175
   Tyr Tyr Ser Ser Lys Val Pro Pro Arg His Val Met Trp Ile Leu Ser
               180                 185                 190
   Ser Leu Tyr Asn Ile Thr Cys Phe Leu Tyr Tyr Asn Asn Leu Ser His
           195                 200                 205
   Asn Gly Ile Asn Ile Asp Asn Tyr Phe Ile Ser Pro Ser Tyr His Ser
       210                 215                 220
   Gly Phe Leu Leu Gly Gly Trp Trp Tyr Thr Val Pro Ile Asp Gly Lys
   225                 230                 235                 240
   Met Leu Gly Ile Pro Ser Glu Ile Tyr Ser Ile Met Ser Pro Glu Met
                   245                 250                 255
   Lys Ser Ser Lys Thr Gly Thr Phe Leu Leu Asp Leu Glu Ser Ile Arg
               260                 265                 270
   Leu Ile Gly Arg Thr Leu Leu Gly Asp Lys Asn Gly Met Ser Phe Ile
           275                 280                 285
   Glu Asp Ser Asp Ile Pro Lys Pro Leu Ile Asp Trp Ala Arg Gly Val
       290                 295                 300
   Ser Val Asn Asn Pro Met Glu Glu Tyr Lys Leu Trp Glu Ser Val Ile
   305                 310                 315                 320
   Lys Gln Ser Tyr Gly Glu Arg Lys Phe Ile Glu Met Asp Ile Asp Ile
                   325                 330                 335
   Thr Lys Leu Tyr Ala Gly Lys Ile Asn Lys
               340                 345
<210> 109
   <211> 1236
   <212> PRT
   <213> Mycoplasma mobile
<220>
   <223> CRISPR-associated endonuclease
<308> YP_015730
   <309> 2013-06-10
<400> 109
   Met Tyr Phe Tyr Lys Asn Lys Glu Asn Lys Leu Asn Lys Lys Val Val
    1               5                  10                  15
   Leu Gly Leu Asp Leu Gly Ile Ala Ser Val Gly Trp Cys Leu Thr Asp
               20                  25                  30
   Ile Ser Gln Lys Glu Asp Asn Lys Phe Pro Ile Ile Leu His Gly Val
           35                  40                  45
   Arg Leu Phe Glu Thr Val Asp Asp Ser Asp Asp Lys Leu Leu Asn Glu
       50                  55                  60
   Thr Arg Arg Lys Lys Arg Gly Gln Arg Arg Arg Asn Arg Arg Leu Phe
   65                  70                  75                  80
   Thr Arg Lys Arg Asp Phe Ile Lys Tyr Leu Ile Asp Asn Asn Ile Ile
                   85                  90                  95
   Glu Leu Glu Phe Asp Lys Asn Pro Lys Ile Leu Val Arg Asn Phe Ile
               100                 105                 110
   Glu Lys Tyr Ile Asn Pro Phe Ser Lys Asn Leu Glu Leu Lys Tyr Lys
           115                 120                 125
   Ser Val Thr Asn Leu Pro Ile Gly Phe His Asn Leu Arg Lys Ala Ala
       130                 135                 140
   Ile Asn Glu Lys Tyr Lys Leu Asp Lys Ser Glu Leu Ile Val Leu Leu
   145                 150                 155                 160
   Tyr Phe Tyr Leu Ser Leu Arg Gly Ala Phe Phe Asp Asn Pro Glu Asp
                   165                 170                 175
   Thr Lys Ser Lys Glu Met Asn Lys Asn Glu Ile Glu Ile Phe Asp Lys
               180                 185                 190
   Asn Glu Ser Ile Lys Asn Ala Glu Phe Pro Ile Asp Lys Ile Ile Glu
           195                 200                 205
   Phe Tyr Lys Ile Ser Gly Lys Ile Arg Ser Thr Ile Asn Leu Lys Phe
       210                 215                 220
   Gly His Gln Asp Tyr Leu Lys Glu Ile Lys Gln Val Phe Glu Lys Gln
   225                 230                 235                 240
   Asn Ile Asp Phe Met Asn Tyr Glu Lys Phe Ala Met Glu Glu Lys Ser
                   245                 250                 255
   Phe Phe Ser Arg Ile Arg Asn Tyr Ser Glu Gly Pro Gly Asn Glu Lys
               260                 265                 270
   Ser Phe Ser Lys Tyr Gly Leu Tyr Ala Asn Glu Asn Gly Asn Pro Glu
           275                 280                 285
   Leu Ile Ile Asn Glu Lys Gly Gln Lys Ile Tyr Thr Lys Ile Phe Lys
       290                 295                 300
   Thr Leu Trp Glu Ser Lys Ile Gly Lys Cys Ser Tyr Asp Lys Lys Leu
   305                 310                 315                 320
   Tyr Arg Ala Pro Lys Asn Ser Phe Ser Ala Lys Val Phe Asp Ile Thr
                   325                 330                 335
   Asn Lys Leu Thr Asp Trp Lys His Lys Asn Glu Tyr Ile Ser Glu Arg
               340                 345                 350
   Leu Lys Arg Lys Ile Leu Leu Ser Arg Phe Leu Asn Lys Asp Ser Lys
           355                 360                 365
   Ser Ala Val Glu Lys Ile Leu Lys Glu Glu Asn Ile Lys Phe Glu Asn
       370                 375                 380
   Leu Ser Glu Ile Ala Tyr Asn Lys Asp Asp Asn Lys Ile Asn Leu Pro
   385                 390                 395                 400
   Ile Ile Asn Ala Tyr His Ser Leu Thr Thr Ile Phe Lys Lys His Leu
                   405                 410                 415
   Ile Asn Phe Glu Asn Tyr Leu Ile Ser Asn Glu Asn Asp Leu Ser Lys
               420                 425                 430
   Leu Met Ser Phe Tyr Lys Gln Gln Ser Glu Lys Leu Phe Val Pro Asn
           435                 440                 445
   Glu Lys Gly Ser Tyr Glu Ile Asn Gln Asn Asn Asn Val Leu His Ile
       450                 455                 460
   Phe Asp Ala Ile Ser Asn Ile Leu Asn Lys Phe Ser Thr Ile Gln Asp
   465                 470                 475                 480
   Arg Ile Arg Ile Leu Glu Gly Tyr Phe Glu Phe Ser Asn Leu Lys Lys
                   485                 490                 495
   Asp Val Lys Ser Ser Glu Ile Tyr Ser Glu Ile Ala Lys Leu Arg Glu
               500                 505                 510
   Phe Ser Gly Thr Ser Ser Leu Ser Phe Gly Ala Tyr Tyr Lys Phe Ile
           515                 520                 525
   Pro Asn Leu Ile Ser Glu Gly Ser Lys Asn Tyr Ser Thr Ile Ser Tyr
       530                 535                 540
   Glu Glu Lys Ala Leu Gln Asn Gln Lys Asn Asn Phe Ser His Ser Asn
   545                 550                 555                 560
   Leu Phe Glu Lys Thr Trp Val Glu Asp Leu Ile Ala Ser Pro Thr Val
                   565                 570                 575
   Lys Arg Ser Leu Arg Gln Thr Met Asn Leu Leu Lys Glu Ile Phe Lys
               580                 585                 590
   Tyr Ser Glu Lys Asn Asn Leu Glu Ile Glu Lys Ile Val Val Glu Val
           595                 600                 605
   Thr Arg Ser Ser Asn Asn Lys His Glu Arg Lys Lys Ile Glu Gly Ile
       610                 615                 620
   Asn Lys Tyr Arg Lys Glu Lys Tyr Glu Glu Leu Lys Lys Val Tyr Asp
   625 630 635 640
   Leu Pro Asn Glu Asn Thr Thr Leu Leu Lys Lys Leu Trp Leu Leu Arg
   645 650 655
   Gln Gln Gln Gly Tyr Asp Ala Tyr Ser Leu Arg Lys Ile Glu Ala Asn
   660 665 670
   Asp Val Ile Asn Lys Pro Trp Asn Tyr Asp Ile Asp His Ile Val Pro
   675 680 685
   Arg Ser Ile Ser Phe Asp Asp Ser Phe Ser Asn Leu Val Ile Val Asn
   690 695 700
   Lys Leu Asp Asn Ala Lys Lys Ser Asn Asp Leu Ser Ala Lys Gln Phe
   705 710 715 720
   Ile Glu Lys Ile Tyr Gly Ile Glu Lys Leu Lys Glu Ala Lys Glu Asn
   725 730 735
   Trp Gly Asn Trp Tyr Leu Arg Asn Ala Asn Gly Lys Ala Phe Asn Asp
   740 745 750
   Lys Gly Lys Phe Ile Lys Leu Tyr Thr Ile Asp Asn Leu Asp Glu Phe
   755 760 765
   Asp Asn Ser Asp Phe Ile Asn Arg Asn Leu Ser Asp Thr Ser Tyr Ile
   770 775 780
   Thr Asn Ala Leu Val Asn His Leu Thr Phe Ser Asn Ser Lys Tyr Lys
   785 790 795 800
   Tyr Ser Val Val Ser Val Asn Gly Lys Gln Thr Ser Asn Leu Arg Asn
   805 810 815
   Gln Ile Ala Phe Val Gly Ile Lys Asn Asn Lys Glu Thr Glu Arg Glu
   820 825 830
   Trp Lys Arg Pro Glu Gly Phe Lys Ser Ile Asn Ser Asn Asp Phe Leu
   835 840 845
   Ile Arg Glu Glu Gly Lys Asn Asp Val Lys Asp Asp Val Leu Ile Lys
   850 855 860
   Asp Arg Ser Phe Asn Gly His His Ala Glu Asp Ala Tyr Phe Ile Thr
   865 870 875 880
   Ile Ile Ser Gln Tyr Phe Arg Ser Phe Lys Arg Ile Glu Arg Leu Asn
   885 890 895
   Val Asn Tyr Arg Lys Glu Thr Arg Glu Leu Asp Asp Leu Glu Lys Asn
   900 905 910
   Asn Ile Lys Phe Lys Glu Lys Ala Ser Phe Asp Asn Phe Leu Leu Ile
   915 920 925
   Asn Ala Leu Asp Glu Leu Asn Glu Lys Leu Asn Gln Met Arg Phe Ser
   930 935 940
   Arg Met Val Ile Thr Lys Lys Asn Thr Gln Leu Phe Asn Glu Thr Leu
   945 950 955 960
   Tyr Ser Gly Lys Tyr Asp Lys Gly Lys Asn Thr Ile Lys Lys Val Glu
   965 970 975
   Lys Leu Asn Leu Leu Asp Asn Arg Thr Asp Lys Ile Lys Lys Ile Glu
   980 985 990
   Glu Phe Phe Asp Glu Asp Lys Leu Lys Glu Asn Glu Leu Thr Lys Leu
   995 1000 1005
   His Ile Phe Asn His Asp Lys Asn Leu Tyr Glu Thr Leu Lys Ile Ile
   1010 1015 1020
   Trp Asn Glu Val Lys Ile Glu Ile Lys Asn Lys Asn Leu Asn Glu Lys
   1025 1030 1035 1040
   Asn Tyr Phe Lys Tyr Phe Val Asn Lys Lys Leu Gln Glu Gly Lys Ile
   1045 1050 1055
   Ser Phe Asn Glu Trp Val Pro Ile Leu Asp Asn Asp Phe Lys Ile Ile
   1060 1065 1070
   Arg Lys Ile Arg Tyr Ile Lys Phe Ser Ser Glu Glu Lys Glu Thr Asp
   1075 1080 1085
   Glu Ile Ile Phe Ser Gln Ser Asn Phe Leu Lys Ile Asp Gln Arg Gln
   1090 1095 1100
   Asn Phe Ser Phe His Asn Thr Leu Tyr Trp Val Gln Ile Trp Val Tyr
   1105 1110 1115 1120
   Lys Asn Gln Lys Asp Gln Tyr Cys Phe Ile Ser Ile Asp Ala Arg Asn
   1125 1130 1135
   Ser Lys Phe Glu Lys Asp Glu Ile Lys Ile Asn Tyr Glu Lys Leu Lys
               1140                1145                1150
   Thr Gln Lys Glu Lys Leu Gln Ile Ile Asn Glu Glu Pro Ile Leu Lys
           1155                1160                1165
   Ile Asn Lys Gly Asp Leu Phe Glu Asn Glu Glu Lys Glu Leu Phe Tyr
       1170                1175                1180
   Ile Val Gly Arg Asp Glu Lys Pro Gln Lys Leu Glu Ile Lys Tyr Ile
   1185                1190                1195                1200
   Leu Gly Lys Lys Ile Lys Asp Gln Lys Gln Ile Gln Lys Pro Val Lys
                   1205                1210                1215
   Lys Tyr Phe Pro Asn Trp Lys Lys Val Asn Leu Thr Tyr Met Gly Glu
               1220                1225                1230
   Ile Phe Lys Lys
           1235
<210> 110
   <211> 1269
   <212> PRT
   <213> Mycoplasma gallisepticum
<220>
   <223> CRISPR-associated endonuclease
<308> YP_005880991
   <309> 2013-06-11
<400> 110
   Met Asn Asn Ser Ile Lys Ser Lys Pro Glu Val Thr Ile Gly Leu Asp
    1               5                  10                  15
   Leu Gly Val Gly Ser Val Gly Trp Ala Ile Val Asp Asn Glu Thr Asn
               20                  25                  30
   Ile Ile His His Leu Gly Ser Arg Leu Phe Ser Gln Ala Lys Thr Ala
           35                  40                  45
   Glu Asp Arg Arg Ser Phe Arg Gly Val Arg Arg Leu Ile Arg Arg Arg
       50                  55                  60
   Lys Tyr Lys Leu Lys Arg Phe Val Asn Leu Ile Trp Lys Tyr Asn Ser
   65                  70                  75                  80
   Tyr Phe Gly Phe Lys Asn Lys Glu Asp Ile Leu Asn Asn Tyr Gln Glu
                   85                  90                  95
   Gln Gln Lys Leu His Asn Thr Val Leu Asn Leu Lys Ser Glu Ala Leu
               100                 105                 110
   Asn Ala Lys Ile Asp Pro Lys Ala Leu Ser Trp Ile Leu His Asp Tyr
           115                 120                 125
   Leu Lys Asn Arg Gly His Phe Tyr Glu Asp Asn Arg Asp Phe Asn Val
       130                 135                 140
   Tyr Pro Thr Lys Glu Leu Ala Lys Tyr Phe Asp Lys Tyr Gly Tyr Tyr
   145                 150                 155                 160
   Lys Gly Ile Ile Asp Ser Lys Glu Asp Asn Asp Asn Lys Leu Glu Glu
                   165                 170                 175
   Glu Leu Thr Lys Tyr Lys Phe Ser Asn Lys His Trp Leu Glu Glu Val
               180                 185                 190
   Lys Lys Val Leu Ser Asn Gln Thr Gly Leu Pro Glu Lys Phe Lys Glu
           195                 200                 205
   Glu Tyr Glu Ser Leu Phe Ser Tyr Val Arg Asn Tyr Ser Glu Gly Pro
       210                 215                 220
   Gly Ser Ile Asn Ser Val Ser Pro Tyr Gly Ile Tyr His Leu Asp Glu
   225                 230                 235                 240
   Lys Glu Gly Lys Val Val Gln Lys Tyr Asn Asn Ile Trp Asp Lys Thr
                   245                 250                 255
   Ile Gly Lys Cys Asn Ile Phe Pro Asp Glu Tyr Arg Ala Pro Lys Asn
               260                 265                 270
   Ser Pro Ile Ala Met Ile Phe Asn Glu Ile Asn Glu Leu Ser Thr Ile
               275                 280                 285
   Arg Ser Tyr Ser Ile Tyr Leu Thr Gly Trp Phe Ile Asn Gln Glu Phe
           290                 295                 300
   Lys Lys Ala Tyr Leu Asn Lys Leu Leu Asp Leu Leu Ile Lys Thr Asn
   305                 310                 315                 320
   Gly Glu Lys Pro Ile Asp Ala Arg Gln Phe Lys Lys Leu Arg Glu Glu
                   325                 330                 335
   Thr Ile Ala Glu Ser Ile Gly Lys Glu Thr Leu Lys Asp Val Glu Asn
               340                 345                 350
   Glu Glu Lys Leu Glu Lys Glu Asp His Lys Trp Lys Leu Lys Gly Leu
           355                 360                 365
   Lys Leu Asn Thr Asn Gly Lys Ile Gln Tyr Asn Asp Leu Ser Ser Leu
       370                 375                 380
   Ala Lys Phe Val His Lys Leu Lys Gln His Leu Lys Leu Asp Phe Leu
   385                 390                 395                 400
   Leu Glu Asp Gln Tyr Ala Thr Leu Asp Lys Ile Asn Phe Leu Gln Ser
                   405                 410                 415
   Leu Phe Val Tyr Leu Gly Lys His Leu Arg Tyr Ser Asn Arg Val Asp
               420                 425                 430
   Ser Ala Asn Leu Lys Glu Phe Ser Asp Ser Asn Lys Leu Phe Glu Arg
           435                 440                 445
   Ile Leu Gln Lys Gln Lys Asp Gly Leu Phe Lys Leu Phe Glu Gln Thr
       450                 455                 460
   Asp Lys Asp Asp Glu Lys Ile Leu Ala Gln Thr His Ser Leu Ser Thr
   465                 470                 475                 480
   Lys Ala Met Leu Leu Ala Ile Thr Arg Met Thr Asn Leu Asp Asn Asp
                   485                 490                 495
   Glu Asp Asn Gln Lys Asn Asn Asp Lys Gly Trp Asn Phe Glu Ala Ile
               500                 505                 510
   Lys Asn Phe Asp Gln Lys Phe Ile Asp Ile Thr Lys Lys Asn Asn Asn
           515                 520                 525
   Leu Ser Leu Lys Gln Asn Lys Arg Tyr Leu Asp Asp Arg Phe Ile Asn
       530                 535                 540
   Asp Ala Ile Leu Ser Pro Gly Val Lys Arg Ile Leu Arg Glu Ala Thr
   545                 550                 555                 560
   Lys Val Phe Asn Ala Ile Leu Lys Gln Phe Ser Glu Glu Tyr Asp Val
                   565                 570                 575
   Thr Lys Val Val Ile Glu Leu Ala Arg Glu Leu Ser Glu Glu Lys Glu
               580                 585                 590
   Leu Glu Asn Thr Lys Asn Tyr Lys Lys Leu Ile Lys Lys Asn Gly Asp
           595                 600                 605
   Lys Ile Ser Glu Gly Leu Lys Ala Leu Gly Ile Ser Glu Asp Glu Ile
       610                 615                 620
   Lys Asp Ile Leu Lys Ser Pro Thr Lys Ser Tyr Lys Phe Leu Leu Trp
   625                 630                 635                 640
   Leu Gln Gln Asp His Ile Asp Pro Tyr Ser Leu Lys Glu Ile Ala Phe
                   645                 650                 655
   Asp Asp Ile Phe Thr Lys Thr Glu Lys Phe Glu Ile Asp His Ile Ile
               660                 665                 670
   Pro Tyr Ser Ile Ser Phe Asp Asp Ser Ser Ser Asn Lys Leu Leu Val
           675                 680                 685
   Leu Ala Glu Ser Asn Gln Ala Lys Ser Asn Gln Thr Pro Tyr Glu Phe
       690                 695                 700
   Ile Ser Ser Gly Asn Ala Gly Ile Lys Trp Glu Asp Tyr Glu Ala Tyr
   705                 710                 715                 720
   Cys Arg Lys Phe Lys Asp Gly Asp Ser Ser Leu Leu Asp Ser Thr Gln
                   725                 730                 735
   Arg Ser Lys Lys Phe Ala Lys Met Met Lys Thr Asp Thr Ser Ser Lys
               740                 745                 750
   Tyr Asp Ile Gly Phe Leu Ala Arg Asn Leu Asn Asp Thr Arg Tyr Ala
           755                 760                 765
   Thr Ile Val Phe Arg Asp Ala Leu Glu Asp Tyr Ala Asn Asn His Leu
       770                 775                 780
   Val Glu Asp Lys Pro Met Phe Lys Val Val Cys Ile Asn Gly Ser Val
   785 790 795 800
   Thr Ser Phe Leu Arg Lys Asn Phe Asp Asp Ser Ser Tyr Ala Lys Lys
   805 810 815
   Asp Arg Asp Lys Asn Ile His His Ala Val Asp Ala Ser Ile Ile Ser
   820 825 830
   Ile Phe Ser Asn Glu Thr Lys Thr Leu Phe Asn Gln Leu Thr Gln Phe
   835 840 845
   Ala Asp Tyr Lys Leu Phe Lys Asn Thr Asp Gly Ser Trp Lys Lys Ile
   850 855 860
   Asp Pro Lys Thr Gly Val Val Thr Glu Val Thr Asp Glu Asn Trp Lys
   865 870 875 880
   Gln Ile Arg Val Arg Asn Gln Val Ser Glu Ile Ala Lys Val Ile Glu
   885 890 895
   Lys Tyr Ile Gln Asp Ser Asn Ile Glu Arg Lys Ala Arg Tyr Ser Arg
   900 905 910
   Lys Ile Glu Asn Lys Thr Asn Ile Ser Leu Phe Asn Asp Thr Val Tyr
   915 920 925
   Ser Ala Lys Lys Val Gly Tyr Glu Asp Gln Ile Lys Arg Lys Asn Leu
   930 935 940
   Lys Thr Leu Asp Ile His Glu Ser Ala Lys Glu Asn Lys Asn Ser Lys
   945 950 955 960
   Val Lys Arg Gln Phe Val Tyr Arg Lys Leu Val Asn Val Ser Leu Leu
   965 970 975
   Asn Asn Asp Lys Leu Ala Asp Leu Phe Ala Glu Lys Glu Asp Ile Leu
   980 985 990
   Met Tyr Arg Ala Asn Pro Trp Val Ile Asn Leu Ala Glu Gln Ile Phe
   995 1000 1005
   Asn Glu Tyr Thr Glu Asn Lys Lys Ile Lys Ser Gln Asn Val Phe Glu
   1010 1015 1020
   Lys Tyr Met Leu Asp Leu Thr Lys Glu Phe Pro Glu Lys Phe Ser Glu
   1025 1030 1035 1040
   Phe Leu Val Lys Ser Met Leu Arg Asn Lys Thr Ala Ile Ile Tyr Asp
   1045 1050 1055
   Asp Lys Lys Asn Ile Val His Arg Ile Lys Arg Leu Lys Met Leu Ser
   1060 1065 1070
   Ser Glu Leu Lys Glu Asn Lys Leu Ser Asn Val Ile Ile Arg Ser Lys
   1075 1080 1085
   Asn Gln Ser Gly Thr Lys Leu Ser Tyr Gln Asp Thr Ile Asn Ser Leu
   1090 1095 1100
   Ala Leu Met Ile Met Arg Ser Ile Asp Pro Thr Ala Lys Lys Gln Tyr
   1105 1110 1115 1120
   Ile Arg Val Pro Leu Asn Thr Leu Asn Leu His Leu Gly Asp His Asp
   1125 1130 1135
   Phe Asp Leu His Asn Met Asp Ala Tyr Leu Lys Lys Pro Lys Phe Val
   1140 1145 1150
   Lys Tyr Leu Lys Ala Asn Glu Ile Gly Asp Glu Tyr Lys Pro Trp Arg
   1155 1160 1165
   Val Leu Thr Ser Gly Thr Leu Leu Ile His Lys Lys Asp Lys Lys Leu
   1170 1175 1180
   Met Tyr Ile Ser Ser Phe Gln Asn Leu Asn Asp Val Ile Glu Ile Lys
   1185 1190 1195 1200
   Asn Leu Ile Glu Thr Glu Tyr Lys Glu Asn Asp Asp Ser Asp Ser Lys
   1205 1210 1215
   Lys Lys Lys Lys Ala Asn Arg Phe Leu Met Thr Leu Ser Thr Ile Leu
   1220 1225 1230
   Asn Asp Tyr Ile Leu Leu Asp Ala Lys Asp Asn Phe Asp Ile Leu Gly
   1235 1240 1245
   Leu Ser Lys Asn Arg Ile Asp Glu Ile Leu Asn Ser Lys Leu Gly Leu
   1250 1255 1260
   Asp Lys Ile Val Lys
   1265
<210> 111
   <211> 1270
   <212> PRT
   <213> Mycoplasma gallisepticum
<220>
   <223> CRISPR-associated endonuclease
<308> YP_005880236
   <309> 2013-06-11
<400> 111
   Met Asn Asn Ser Ile Lys Ser Lys Pro Glu Val Thr Ile Gly Leu Asp
   1               5                   10                  15
   Leu Gly Val Gly Ser Val Gly Trp Ala Ile Val Asp Asn Glu Thr Asn
               20                  25                  30
   Ile Ile His His Leu Gly Ser Arg Leu Phe Ser Gln Ala Lys Thr Ala
           35                  40                  45
   Glu Asp Arg Arg Ser Phe Arg Gly Val Arg Arg Leu Ile Arg Arg Arg
       50                  55                  60
   Lys Tyr Lys Leu Lys Arg Phe Val Asn Leu Ile Trp Lys Tyr Asn Ser
   65                  70                  75                  80
   Tyr Phe Gly Phe Lys Asn Lys Glu Asp Ile Leu Asn Asn Tyr Gln Glu
                   85                  90                  95
   Gln Gln Lys Leu His Asn Thr Val Leu Asn Leu Lys Leu Glu Ala Leu
               100                 105                 110
   Asn Ala Lys Ile Asp Pro Lys Ala Leu Ser Trp Ile Leu His Asp Tyr
           115                 120                 125
   Leu Lys Asn Arg Gly His Phe Tyr Glu Asp Asn Arg Asp Phe Asn Val
       130                 135                 140
   Tyr Pro Thr Glu Glu Leu Ala Asn Tyr Phe Asp Glu Phe Gly Tyr Tyr
   145                 150                 155                 160
   Lys Gly Ile Ile Asp Ser Lys Asn Asp Asp Asp Asp Lys Leu Glu Glu
                   165                 170                 175
   Gly Leu Thr Lys Tyr Lys Phe Ser Asn Gln His Trp Leu Glu Glu Val
               180                 185                 190
   Lys Lys Val Leu Ser Asn Gln Thr Gly Leu Pro Glu Lys Phe Lys Glu
           195                 200                 205
   Glu Tyr Glu Ser Leu Phe Ser Tyr Val Arg Asn Tyr Ser Glu Gly Pro
       210                 215                 220
   Gly Ser Ile Asn Ser Val Ser Pro Tyr Gly Ile Tyr His Leu Asp Glu
   225                 230                 235                 240
   Lys Glu Gly Lys Val Val Gln Lys Tyr Asn Asn Ile Trp Asp Lys Thr
                   245                 250                 255
   Ile Gly Lys Cys Ser Ile Phe Pro Asp Glu Tyr Arg Ala Pro Lys Asn
               260                 265                 270
   Ser Pro Ile Ala Met Ile Phe Asn Glu Ile Asn Glu Leu Ser Thr Ile
           275                 280                 285
   Arg Ser Tyr Ser Ile Tyr Leu Thr Gly Trp Phe Ile Asn Gln Glu Phe
       290                 295                 300
   Lys Lys Ala Tyr Leu Asn Lys Leu Leu Asp Leu Leu Ile Lys Thr Asn
   305                 310                 315                 320
   Ser Glu Lys Pro Ile Asp Ala Arg Gln Phe Lys Lys Leu Arg Glu Glu
                   325                 330                 335
   Thr Ile Ala Glu Ser Ile Gly Lys Glu Thr Leu Lys Asp Val Glu Ser
               340                 345                 350
   Glu Glu Lys Leu Glu Lys Asp Asp His Lys Trp Lys Leu Lys Gly Leu
           355                 360                 365
   Lys Leu Asn Thr Asn Gly Lys Ile Gln Tyr Asn Asp Leu Ser Ser Leu
       370                 375                 380
   Ala Lys Phe Val His Lys Leu Lys Gln His Leu Lys Leu Asp Phe Leu
   385                 390                 395                 400
   Leu Glu Asp Gln Tyr Thr Pro Leu Asp Lys Ile Asn Phe Leu Gln Ser
   405 410 415
   Leu Tyr Val Tyr Leu Gly Lys His Leu Arg Tyr Ser Asn Arg Val Asp
   420 425 430
   Ser Ala Asn Leu Lys Glu Phe Ser Asp Ser Ser Arg Leu Phe Glu Arg
   435 440 445
   Val Leu Gln Glu Gln Lys Asp Gly Leu Phe Lys Leu Phe Glu Gln Thr
   450 455 460
   Asp Lys Asp Asp Glu Lys Ile Leu Thr Gln Thr His Ser Leu Ser Thr
   465 470 475 480
   Lys Ala Met Leu Leu Ala Ile Thr Arg Met Thr Asn Leu Asp Asn Asp
   485 490 495
   Glu Asp Asn Gln Lys Asn Asn Asp Lys Gly Trp Asn Phe Glu Ala Ile
   500 505 510
   Lys Asn Phe Asp Gln Lys Phe Ile Asp Ile Thr Lys Thr Asn Asn Asn
   515 520 525
   Leu Ser Leu Lys Gln Asp Lys Arg Tyr Leu Asp Asp Gln Phe Ile Asn
   530 535 540
   Asp Ala Ile Leu Ser Pro Gly Val Lys Arg Ile Leu Arg Glu Ala Thr
   545 550 555 560
   Lys Val Phe Asn Ala Ile Leu Lys Gln Phe Ser Glu Glu Tyr Asp Val
   565 570 575
   Thr Lys Val Val Ile Glu Leu Ala Arg Glu Leu Ser Glu Glu Lys Glu
   580 585 590
   Leu Glu Asn Thr Lys Asn Tyr Lys Lys Leu Ile Lys Lys Asn Gly Asp
   595 600 605
   Lys Ile Ser Glu Gly Leu Lys Ala Leu Gly Ile Ala Glu Asp Lys Ile
   610 615 620
   Glu Glu Ile Leu Lys Ser Pro Thr Lys Ser Tyr Lys Val Leu Leu Trp
   625 630 635 640
   Leu Gln Gln Asp His Ile Asp Pro Tyr Ser Gln Lys Glu Ile Ala Phe
   645 650 655
   Asp Asp Ile Leu Thr Lys Thr Glu Lys Thr Glu Ile Asp His Ile Ile
   660 665 670
   Pro Tyr Ser Ile Ser Phe Asp Asp Ser Ser Ser Asn Lys Leu Leu Val
   675 680 685
   Leu Ala Glu Ser Asn Gln Ala Lys Ser Asn Gln Thr Pro Tyr Glu Phe
   690 695 700
   Ile Asn Ser Gly Lys Ala Glu Ile Thr Trp Glu Val Tyr Glu Ala Tyr
   705 710 715 720
   Cys His Lys Phe Lys Asn Gly Asp Ser Ser Leu Leu Asp Ser Thr Gln
   725 730 735
   Arg Ser Lys Lys Phe Ala Lys Met Met Lys Thr Asp Thr Ser Ser Lys
   740 745 750
   Tyr Asp Ile Gly Phe Leu Ala Arg Asn Leu Asn Asp Thr Arg Tyr Ala
   755 760 765
   Thr Ile Val Phe Arg Asp Ala Leu Lys Asp Tyr Ala Asn Asn His Leu
   770 775 780
   Val Glu Asp Lys Pro Met Phe Lys Val Val Cys Ile Asn Gly Gly Val
   785 790 795 800
   Thr Ser Phe Leu Arg Lys Asn Phe Asp Pro Lys Ser Trp Tyr Ala Lys
   805 810 815
   Lys Asp Arg Asp Lys Asn Ile His His Ala Val Asp Ala Ser Ile Ile
   820 825 830
   Ser Ile Phe Ser Asn Glu Thr Lys Thr Leu Phe Asn Gln Leu Thr Lys
   835 840 845
   Phe Ala Asp Tyr Lys Leu Phe Lys Asn Thr Asp Gly Ser Trp Lys Lys
   850 855 860
   Ile Asp Pro Lys Thr Gly Val Val Ser Glu Val Thr Asp Glu Asn Trp
   865 870 875 880
   Lys Gln Ile Arg Val Arg Asn Gln Val Ser Glu Ile Ala Lys Val Ile
   885 890 895
   Asp Lys Tyr Ile Gln Asp Ser Asn Ile Glu Arg Lys Ala Arg Tyr Ser
               900                 905                 910
   Arg Lys Ile Glu Asn Lys Thr Asn Ile Ser Leu Phe Asn Asp Thr Val
           915                 920                 925
   Tyr Ser Ala Lys Lys Val Gly Tyr Glu Asp Gln Ile Lys Arg Lys Asn
       930                 935                 940
   Leu Lys Thr Leu Asp Ile His Glu Ser Ala Glu Glu Asn Lys Asn Ser
   945                 950                 955                 960
   Lys Val Lys Lys Gln Phe Val Tyr Arg Lys Leu Val Asn Val Ser Leu
                   965                 970                 975
   Leu Asn Asn Asp Lys Leu Ala Asp Leu Phe Ala Glu Lys Glu Asp Ile
               980                 985                 990
   Leu Met Tyr Arg Ala Asn Pro Trp Val Ile Asn Leu Ala Glu Gln Ile
           995                 1000                1005
   Phe Asn Glu Tyr Thr Glu Asn Lys Lys Ile Lys Ser Gln Asn Val Phe
       1010                1015                1020
   Glu Lys Tyr Met Leu Asp Leu Thr Lys Glu Phe Pro Glu Lys Phe Ser
   1025                1030                1035                1040
   Glu Ala Phe Val Lys Ser Met Ile Arg Asn Lys Thr Ala Ile Ile Tyr
                   1045                1050                1055
   Asn Val Glu Lys Asp Val Val His Arg Ile Lys Arg Leu Lys Ile Leu
               1060                1065                1070
   Ser Ser Glu Leu Lys Glu Asn Lys Trp Ser Asn Val Ile Ile Arg Ser
           1075                1080                1085
   Lys Asn Glu Ser Gly Thr Lys Leu Ser Tyr Gln Asp Thr Ile Asn Ser
       1090                1095                1100
   Ile Ala Leu Met Ile Met Arg Ser Ile Asp Pro Thr Ala Lys Lys Gln
   1105                1110                1115                1120
   Tyr Ile Arg Val Pro Leu Asn Thr Leu Asn Leu His Leu Gly Asp Gln
                   1125                1130                1135
   Asp Phe Asp Leu His Asn Ile Asp Ala Tyr Leu Lys Lys Pro Lys Phe
               1140                1145                1150
   Val Lys Tyr Leu Lys Ala Asn Glu Ile Gly Asp Glu Tyr Lys Pro Trp
           1155                1160                1165
   Arg Val Leu Thr Ser Gly Thr Leu Leu Ile His Lys Lys Asp Lys Lys
       1170                1175                1180
   Leu Met Tyr Ile Ser Ser Phe Gln Asn Leu Asn Asp Leu Ile Glu Ile
   1185                1190                1195                1200
   Lys Asn Leu Ile Glu Thr Glu Tyr Lys Glu Asn Val Asp Ser Asp Pro
                   1205                1210                1215
   Lys Lys Lys Lys Lys Ala Ser Gln Ile Leu Arg Ser Leu Ser Val Ile
               1220                1225                1230
   Leu Asn Asp Tyr Ile Leu Leu Asp Ala Lys Tyr Asn Phe Asp Ile Leu
           1235                1240                1245
   Gly Leu Ser Lys Asn Lys Ile Asp Glu Ile Leu Asn Ser Lys Leu Asp
       1250                1255                1260
   Leu Asp Lys Ile Ala Lys
   1265                1270
<210> 112
   <211> 1259
   <212> PRT
   <213> Streptobacillus moniliformis
<220>
   <223> CRISPR-associated endonuclease
<308> YP_003306403
   <309> 2013-06-10
<400> 112
   Met Lys Tyr Ile Leu Gly Leu Asp Leu Gly Val Ala Ser Ile Gly Trp
   1               5                   10                  15
   Ala Val Thr Glu Ile Asn Glu Lys Gly Glu Pro Val Gly Leu Ile Asp
   20 25 30
   Ala Asn Ser Val Ile Phe Lys Pro Leu Asp Asn Asp Lys Gly Lys Leu
   35 40 45
   Tyr Asn Val Glu Arg Arg Asp Lys Arg Gly Ser Arg Arg Ile Leu Arg
   50 55 60
   Arg Lys Lys His Arg Val Glu Arg Thr Lys Met Leu Leu Val Asn Ser
   65 70 75 80
   Ser Phe Leu Thr Asn Glu Glu Ile Asp Asn Leu Tyr Val Gly Lys Leu
   85 90 95
   Asp Asn Ile Tyr Glu Val Arg Leu Lys Gly Leu Lys Glu Gln Leu Ser
   100 105 110
   Lys Asn Glu Ile Ala Arg Leu Met Ile Tyr Tyr Cys Lys Asn Arg Gly
   115 120 125
   Phe Lys Ser Asn Arg Lys Thr Glu Asp Leu Glu Ile Leu Lys Ser Leu
   130 135 140
   Gly Glu Lys Ile Ser Glu Lys Asp Ser Asp Glu Lys Lys Leu Lys Pro
   145 150 155 160
   Ile Ile Ala Lys Asn Thr Glu Leu Ile Glu Ser Lys Gly Leu Thr Pro
   165 170 175
   Ile Glu Val Ile Tyr Met Ile Arg Glu Asn Asp Asn Ser Ile Leu Gly
   180 185 190
   Phe Lys Asn Lys Glu Gly Asn Tyr Lys Phe Gly Phe Lys Arg Asn Gln
   195 200 205
   Val Ile Asp Glu Val Arg Lys Ile Leu Gly Thr Gln Gln Ile Leu Ser
   210 215 220
   Lys Glu Ile Val Asp Glu Tyr Ile Asp Ile Leu Ser Ser Gln Arg Asp
   225 230 235 240
   Phe Ser Asp Gly Pro Gly Gly Asp Ser Lys Tyr Lys Ile Asp Tyr Thr
   245 250 255
   Lys Leu Ala Gly Lys Cys Lys Tyr Thr Gly Glu Val Arg Ala Val Lys
   260 265 270
   Ser Ala Pro Ser Tyr Glu Ile Phe Thr Met Leu Gln Lys Leu Asn Asp
   275 280 285
   Ile Arg Tyr Val Lys Phe Thr Ser Glu Asp Lys Ile Glu Lys Lys Lys
   290 295 300
   Leu Ser Lys Glu Val Ile His Lys Leu Tyr Asp Leu Val Val Glu Lys
   305 310 315 320
   Asn Lys Thr Leu Thr Tyr Asp Leu Ile Glu Lys Ser Ile Asp Glu Glu
   325 330 335
   Asn Ile Lys Leu Leu Asn Ile Pro Lys Leu Ser Lys Ser Lys Tyr Val
   340 345 350
   Glu Leu Arg Lys Ser Tyr Phe Lys Glu Arg Asn Asp Asn Glu Asn Leu
   355 360 365
   Thr Glu Glu Glu Ile Asn Ala Phe Asn Glu Lys Leu Asn Asn Glu Arg
   370 375 380
   Met Lys Gln Glu Leu Ile Arg Asn Asn Leu Lys Ala Tyr Lys Glu Leu
   385 390 395 400
   Lys Ser Lys Phe Lys Lys Asn Asn Ile Asp Glu Asn Arg Ile Lys Glu
   405 410 415
   Leu Gly Gly Ile Tyr Phe Leu Asp Leu Val Ala Glu Leu Leu Thr Tyr
   420 425 430
   Ser Lys Thr Asp Glu Lys Leu Glu Tyr Phe Val Glu Asn Val Glu Arg
   435 440 445
   Tyr Ser Leu Phe Lys Glu Gln Arg Asp Ile Val Glu Ile Ile Lys Thr
   450 455 460
   Phe Pro Asn Tyr Thr Lys Asn Gly Asn Leu Ser Leu Ser Leu Val Arg
   465 470 475 480
   Glu Leu Asn Lys Leu Leu Met Glu Gly His Asp Tyr Glu Ser Ser Leu
   485 490 495
   Ser Ser Leu Asn Tyr Tyr Ile Asp Thr Asp Val Asp Trp Glu Lys Phe
   500 505 510
   Pro Thr Ile Ser Glu Ile Glu Asp Ser Leu Ser Thr Lys Ile Thr Asn
           515                 520                 525
   Pro Asn Val Lys His Ile Leu Val Ile Leu Arg Lys Leu Tyr Asn Thr
       530                 535                 540
   Leu Leu Phe Lys Tyr Gly Arg Pro Glu Lys Val His Leu Glu Leu Ser
   545                 550                 555                 560
   Arg Asp Phe Ser Asn Asp Phe Ser Thr Arg Asn Lys Ile Gln Lys Glu
                   565                 570                 575
   Gln Leu Glu Asn Lys Val Arg Arg Glu Val Ala Ala Phe Glu Met Tyr
               580                 585                 590
   Gly Ala Asn Lys Asp Ile Val Ala Gly Lys Asp Arg Leu Ser Asn Asp
           595                 600                 605
   Asp Phe Val Arg Ile Lys Leu Trp Glu Glu Gln Asn Lys Val Cys Met
       610                 615                 620
   Tyr Ser Gly Arg Thr Ile Glu Lys Tyr Gln Leu Thr Ser Ala Glu Val
   625                 630                 635                 640
   Gln Ile Asp His Ile Leu Pro Tyr Ser Lys Ser Phe Asp Asn Ser Tyr
                   645                 650                 655
   Ser Asn Lys Val Leu Val Phe Ser Asn Glu Asn Gln Asp Lys Lys Glu
               660                 665                 670
   Arg Thr Pro Tyr Gln Trp Leu Lys Gly Thr Glu Lys Trp Asn Glu Phe
           675                 680                 685
   Lys Gln Arg Val Arg Leu Asn Leu Asn Ile Ser Asn Lys Lys Lys Glu
       690                 695                 700
   Asn Leu Leu Phe Glu Asp Glu Val Val Asn Asn Glu Phe Leu Glu Arg
   705                 710                 715                 720
   Glu Leu His Ala Thr Ser Tyr Ser Ser Arg Leu Ala Leu Asn Ile Phe
                   725                 730                 735
   Gln Arg Leu Ile Pro Val Ser Asp Glu Asp Lys Tyr Asp Glu His Gly
               740                 745                 750
   Asn Glu Lys Val Lys Tyr Leu Tyr Asn Arg Asn Val Ile Ala Phe Gln
           755                 760                 765
   Gly Lys Met Thr Ser Met Leu Arg Asn Leu Tyr Ser Leu Asn Lys Tyr
       770                 775                 780
   Thr His Ser Phe Glu Ser Asp Thr Leu Asp Ile Asp Asn Lys Ala Phe
   785                 790                 795                 800
   Ile Leu Lys Glu Phe Glu Ile Asn Lys Asp Asn Leu Lys Met Thr Ala
                   805                 810                 815
   Tyr Asn Val Asn Thr Gly Leu Glu Ile Thr Ser Glu Thr Lys Val Glu
               820                 825                 830
   Lys Asn Lys Ser Gly Glu Phe Lys Thr Ile Lys Asp Glu Leu Leu Lys
           835                 840                 845
   Lys Glu Leu Asp Lys Lys Glu Asn Leu Ile Glu Ile Ser Asp Tyr Phe
       850                 855                 860
   Lys Asp Lys Val Leu Phe Asp Leu Lys Ile Val Asp Phe Glu Glu Ile
   865                 870                 875                 880
   Asp Thr Val Glu Pro Glu Ile Ile Ser Ile Leu Tyr Lys Met Ile Thr
                   885                 890                 895
   Ala Leu Lys Glu Glu Val Tyr Ser Lys Asn Arg Glu Asn His Leu His
               900                 905                 910
   His Ser Leu Asp Ala Phe Leu Leu Thr Ile Met Asn Arg Ser Met Gln
           915                 920                 925
   Met Lys Leu Ile Lys Tyr Asn Gln Leu Ile Ser Val Leu Lys Asn Lys
       930                 935                 940
   Glu Ile Glu Ile Phe Asn Glu Glu Lys Gly Glu Tyr Ile Asp Ser Lys
   945                 950                 955                 960
   Glu Phe Ala Glu Glu Leu Gln Lys Gln Lys Ile Ile Asp Ile Asp Gly
                   965                 970                 975
   Asn Glu Arg Gly Ile Lys Phe Asn Val Asn Gly Lys Thr His Arg Leu
               980                 985                 990
   Asn Leu Val Lys Pro Tyr Glu Asn Phe Leu Asp Asp Ile Lys Asn Lys
           995                 1000                1005
   Ile Phe Asp Lys Arg Glu Asn Asn Lys Leu Pro Tyr His Val Val Lys
       1010                1015                1020
   Ser Lys Val Ser Gly Ala Leu His Ala Glu Thr Ile Leu Gly Glu Ser
   1025                1030                1035                1040
   Lys Gly Glu Ile Thr Lys Arg Ile Ser Val Phe Asp Ile Asn Tyr Lys
                   1045                1050                1055
   Asn Leu Gly Lys Ile Phe Asp Lys Asp Gly Ser Gln Lys Glu Ile Tyr
               1060                1065                1070
   Glu Thr Leu Val Lys Trp Leu Glu Ala Lys Ser Lys Asp Tyr Pro Lys
           1075                1080                1085
   Leu Lys Asn Gly Asn Ile Ile Lys Lys Val Lys Ile Val Asp Gly Asn
       1090                1095                1100
   Lys Asp Lys Leu Ile Lys Leu Gly Asn Lys Arg Tyr Val Glu Met Gly
   1105                1110                1115                1120
   Arg Thr Thr Val Lys Ile Leu Val Leu Lys Lys Glu Ala Glu Glu Gly
                   1125                1130                1135
   Leu Lys Phe Ala Ser Ile Gly Arg Tyr Lys Tyr Asn Leu Leu Lys Ser
               1140                1145                1150
   Glu Lys Asp Val Asn Ile Ser Ile Trp Lys Asp Ala Ile His Phe Cys
           1155                1160                1165
   Lys Val Arg Tyr Asn Lys Leu Lys Glu Asn Gly Tyr Arg Leu Ile His
       1170                1175                1180
   Glu Leu Ile Pro Gly Glu Thr Ile Glu Leu Glu Leu Asn Lys Gly Asn
   1185                1190                1195                1200
   Ile Ser Lys Cys Leu Val Val Gly Phe Ser Gly Gly Lys Ile Glu Ile
                   1205                1210                1215
   Ser Ser Val Ile Gly Asp Ala Leu Asp Leu Ile Asn Asp Lys Ile Ser
               1220                1225                1230
   Thr Arg Ile Lys Glu Arg Tyr Tyr Ile Thr Val Ser Thr Ile Lys Ser
           1235                1240                1245
   Ile Lys Lys Ile Asn Lys Asn Ile Leu Gly Asp
       1250                1255
<210> 113
   <211> 1368
   <212> PRT
   <213> Streptococcus pyogenes
<220>
   <223> CRISPR-associated endonuclease
<308> YP_002285828
   <309> 2013-06-10
<400> 113
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Ile Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Ala Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Arg Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Arg Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Ala Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
   660 665 670
   Lys Gln Ser Gly Lys Thr Ile Leu Asp Phe Leu Lys Ser Asp Gly Phe
   675 680 685
   Ala Asn Arg Asn Phe Met Gln Leu Ile His Asp Asp Ser Leu Thr Phe
   690 695 700
   Lys Glu Asp Leu Gln Lys Ala Gln Val Ser Gly Gln Gly Asp Ser Leu
   705 710 715 720
   His Glu His Ile Ala Asn Leu Ala Gly Ser Pro Ala Ile Lys Lys Gly
   725 730 735
   Ile Leu Gln Thr Val Lys Val Val Asp Glu Leu Val Lys Val Met Gly
   740 745 750
   Arg His Lys Pro Glu Asn Ile Val Ile Glu Met Ala Arg Glu Asn Gln
   755 760 765
   Thr Thr Gln Lys Gly Gln Lys Asn Ser Arg Glu Arg Met Lys Arg Ile
   770 775 780
   Glu Glu Gly Ile Lys Glu Leu Gly Ser Asp Ile Leu Lys Glu Tyr Pro
   785 790 795 800
   Val Glu Thr Thr Gln Leu Gln Asn Glu Lys Leu Tyr Leu Tyr Tyr Leu
   805 810 815
   Gln Asn Gly Arg Asp Met Tyr Val Asp Gln Glu Leu Asp Ile Asn Arg
   820 825 830
   Leu Ser Asp Tyr Asp Val Asp His Ile Val Pro Gln Ser Phe Leu Lys
   835 840 845
   Asp Asp Ser Ile Asp Asn Lys Val Leu Thr Arg Ser Asp Lys Asn Arg
   850 855 860
   Gly Lys Ser Asp Asn Val Pro Ser Glu Glu Val Val Lys Lys Met Lys
   865 870 875 880
   Asn Tyr Trp Lys Gln Leu Leu Asn Ala Lys Leu Ile Thr Gln Arg Lys
   885 890 895
   Phe Asp Asn Leu Thr Lys Ala Glu Arg Gly Gly Leu Ser Glu Leu Asp
   900 905 910
   Lys Ala Gly Phe Ile Lys Arg Gln Leu Val Glu Thr Arg Gln Ile Thr
   915 920 925
   Lys His Val Ala Gln Ile Leu Asp Ser Arg Met Asn Thr Lys Tyr Asp
   930 935 940
   Glu Asn Asp Lys Leu Ile Arg Glu Val Arg Val Ile Thr Leu Lys Ser
   945 950 955 960
   Lys Leu Val Ser Asp Phe Arg Lys Asp Phe Gln Phe Tyr Lys Val Arg
   965 970 975
   Glu Ile Asn Asn Tyr His His Ala His Asp Ala Tyr Leu Asn Ala Val
   980 985 990
   Val Gly Thr Ala Leu Ile Lys Lys Tyr Pro Lys Leu Glu Ser Glu Phe
   995 1000 1005
   Val Tyr Gly Asp Tyr Lys Val Tyr Asp Ile Arg Lys Met Ile Ala Lys
   1010 1015 1020
   Ser Glu Gln Glu Ile Gly Lys Ala Thr Ala Lys Tyr Phe Phe Tyr Ser
   1025 1030 1035 1040
   Asn Ile Met Asn Phe Phe Lys Thr Glu Ile Thr Leu Ala Asn Gly Glu
   1045 1050 1055
   Ile Arg Lys Arg Pro Leu Ile Glu Thr Asn Gly Glu Thr Gly Glu Ile
   1060 1065 1070
   Val Trp Asp Lys Gly Arg Asp Phe Ala Thr Val Arg Lys Val Leu Ser
   1075 1080 1085
   Met Pro Gln Val Asn Ile Val Lys Lys Thr Glu Val Gln Thr Gly Gly
   1090 1095 1100
   Phe Ser Lys Glu Ser Ile Leu Pro Lys Arg Asn Ser Asp Lys Leu Ile
   1105 1110 1115 1120
   Ala Arg Lys Lys Asp Trp Asp Pro Lys Lys Tyr Gly Gly Phe Asp Ser
   1125 1130 1135
   Pro Thr Val Ala Tyr Ser Val Leu Val Val Ala Lys Val Glu Lys Gly
   1140 1145 1150
   Lys Ser Lys Lys Leu Lys Ser Val Lys Glu Leu Val Gly Ile Thr Ile
           1155                1160                1165
   Met Glu Arg Ser Ser Phe Glu Lys Asp Pro Val Asp Phe Leu Glu Ala
       1170                1175                1180
   Lys Gly Tyr Lys Glu Val Arg Lys Asp Leu Ile Ile Lys Leu Pro Lys
   1185                1190                1195                1200
   Tyr Ser Leu Phe Glu Leu Glu Asn Gly Arg Lys Arg Met Leu Ala Ser
                   1205                1210                1215
   Ala Gly Glu Leu Gln Lys Gly Asn Glu Leu Ala Leu Pro Ser Lys Tyr
               1220                1225                1230
   Val Asn Phe Leu Tyr Leu Ala Ser His Tyr Glu Lys Leu Lys Gly Ser
           1235                1240                1245
   Pro Glu Asp Asn Glu Gln Lys Gln Leu Phe Val Glu Gln His Lys His
       1250                1255                1260
   Tyr Leu Asp Glu Ile Ile Glu Gln Ile Ser Glu Phe Ser Lys Arg Val
   1265                1270                1275                1280
   Ile Leu Ala Asp Ala Asn Leu Asp Lys Val Leu Ser Ala Tyr Asn Lys
                   1285                1290                1295
   His Arg Asp Lys Pro Ile Arg Glu Gln Ala Glu Asn Ile Ile His Leu
               1300                1305                1310
   Phe Thr Leu Thr Asn Leu Gly Ala Pro Ala Ala Phe Lys Cys Phe Asp
           1315                1320                1325
   Thr Thr Ile Gly Arg Asn Arg Tyr Lys Ser Ile Lys Glu Val Leu Asp
       1330                1335                1340
   Ala Thr Leu Ile His Gln Ser Ile Thr Gly Leu Tyr Glu Thr Arg Ile
   1345                1350                1355                1360
   Asp Leu Ser Gln Leu Gly Gly Asp
                   1365
<210> 114
   <211> 984
   <212> PRT
   <213> Campylobacter jejuni
<220>
   <223> CRISPR-associated endonuclease
<308> YP_001483000
   <309> 2013-06-10
<400> 114
   Met Ala Arg Ile Leu Ala Phe Asp Ile Gly Ile Ser Ser Ile Gly Trp
    1               5                  10                  15
   Ala Phe Ser Glu Asn Asp Glu Leu Lys Asp Cys Gly Val Arg Ile Phe
               20                  25                  30
   Thr Lys Ala Glu Asn Pro Lys Thr Gly Glu Ser Leu Ala Leu Pro Arg
           35                  40                  45
   Arg Leu Ala Arg Ser Ala Arg Lys Arg Leu Ala Arg Arg Lys Ala Arg
       50                  55                  60
   Leu Asn His Leu Lys His Leu Ile Ala Asn Glu Phe Lys Leu Asn Tyr
   65                  70                  75                  80
   Glu Asp Tyr Gln Ser Phe Asp Glu Ser Leu Ala Lys Ala Tyr Lys Gly
                   85                  90                  95
   Ser Leu Ile Ser Pro Tyr Glu Leu Arg Phe Arg Ala Leu Asn Glu Leu
               100                 105                 110
   Leu Ser Lys Gln Asp Phe Ala Arg Val Ile Leu His Ile Ala Lys Arg
           115                 120                 125
   Arg Gly Tyr Asp Asp Ile Lys Asn Asn Gly Asp Glu Glu Lys Ser Glu
       130                 135                 140
   Ile Leu Lys Ala Ile Lys Gln Asn Glu Glu Lys Leu Val Asn Tyr Gln
   145                 150                 155                 160
   Ser Val Gly Glu Tyr Leu Tyr Lys Glu Tyr Phe Gln Lys Phe Lys Glu
                   165                 170                 175
   Asn Ser Lys Glu Phe Ile Asn Val Arg Asn Lys Lys Glu Ser Tyr Glu
               180                 185                 190
   Arg Cys Ile Thr Gln Ser Phe Leu Lys Asp Glu Leu Lys Leu Ile Phe
           195                 200                 205
   Gln Lys Gln Arg Glu Phe Gly Phe Ser Phe Ser Lys Lys Phe Glu Glu
       210                 215                 220
   Glu Val Leu Ser Val Ala Phe Tyr Lys Arg Ala Leu Lys Asp Phe Ser
   225                 230                 235                 240
   His Leu Val Gly Asn Cys Ser Phe Phe Thr Asp Glu Lys Arg Ala Pro
                   245                 250                 255
   Lys Asn Ser Pro Leu Ala Phe Met Phe Val Ala Leu Thr Arg Ile Ile
               260                 265                 270
   Asn Ile Leu Asn Asn Leu Lys Asn Thr Glu Gly Ile Leu Tyr Thr Lys
           275                 280                 285
   Asp Asp Leu Asn Ala Leu Leu Asn Glu Val Leu Lys Asn Gly Thr Leu
       290                 295                 300
   Thr Tyr Lys Gln Thr Lys Lys Leu Leu Gly Leu Ser Asp Asp Tyr Glu
   305                 310                 315                 320
   Phe Lys Gly Glu Lys Gly Thr Tyr Phe Ile Glu Phe Lys Lys Tyr Lys
                   325                 330                 335
   Glu Phe Ile Lys Ala Leu Gly Glu His Asn Leu Ser Gln Asp Asp Leu
               340                 345                 350
   Asn Glu Ile Ala Lys Asp Ile Thr Leu Ile Lys Asp Glu Ile Lys Leu
           355                 360                 365
   Lys Lys Ala Leu Ala Lys Tyr Asp Leu Asn Gln Asn Gln Ile Asp Ser
       370                 375                 380
   Leu Ser Lys Leu Glu Phe Lys Asp His Leu Asn Ile Ser Phe Lys Ala
   385                 390                 395                 400
   Leu Lys Leu Ile Thr Pro Leu Met Leu Glu Gly Lys Lys Tyr Asp Glu
                   405                 410                 415
   Ala Cys Asn Glu Leu Asn Leu Lys Val Ala Ile Asn Glu Asp Lys Lys
               420                 425                 430
   Asp Phe Leu Pro Ala Phe Asn Glu Thr Tyr Tyr Lys Asp Glu Val Thr
           435                 440                 445
   Asn Pro Val Val Leu Arg Ala Ile Lys Glu Tyr Arg Lys Ile Leu Asn
       450                 455                 460
   Ala Leu Leu Lys Lys Tyr Gly Lys Val His Lys Ile Asn Ile Glu Leu
   465                 470                 475                 480
   Ala Arg Glu Val Gly Lys Asn His Ser Gln Arg Ala Lys Ile Glu Lys
                   485                 490                 495
   Glu Gln Asn Glu Asn Tyr Lys Ala Lys Lys Asp Ala Glu Ile Glu Cys
               500                 505                 510
   Glu Lys Leu Gly Leu Lys Ile Asn Ser Lys Asn Ile Leu Lys Leu Arg
           515                 520                 525
   Leu Phe Lys Glu Gln Lys Glu Phe Cys Ala Tyr Ser Gly Glu Lys Ile
       530                 535                 540
   Lys Leu Ser Asp Leu Gln Asp Glu Lys Met Leu Glu Ile Asp His Ile
   545                 550                 555                 560
   Tyr Pro Tyr Ser Arg Ser Phe Asp Asp Ser Tyr Met Asn Lys Val Leu
                   565                 570                 575
   Val Phe Thr Lys Gln Asn Gln Glu Lys Leu Asn Lys Thr Pro Phe Glu
               580                 585                 590
   Ala Phe Gly Asn Asp Ser Thr Lys Trp Gln Lys Ile Glu Val Leu Ala
           595                 600                 605
   Lys Asn Leu Pro Glu Lys Lys Gln Lys Arg Ile Leu Asp Lys Asn Tyr
       610                 615                 620
   Lys Asp Lys Glu Gln Lys Asp Phe Lys Asp Arg Asn Leu Asn Asp Thr
   625                 630                 635                 640
   Arg Tyr Ile Ala Arg Leu Val Leu Asn Tyr Thr Lys Asp Tyr Leu Asp
                   645                 650                 655
   Phe Leu Pro Leu Ser Asp Asp Glu Asn Thr Lys Leu Asn Asp Thr Gln
               660                 665                 670
   Lys Gly Ser Lys Val His Val Glu Ala Lys Ser Gly Met Leu Thr Ser
               675                 680                 685
   Ala Leu Arg His Thr Trp Gly Phe Ser Ala Lys Asp Arg Asn Asn His
           690                 695                 700
   Leu His His Ala Ile Asp Ala Ala Ile Ile Ala Tyr Ala Asn Asn Ser
   705                 710                 715                 720
   Ile Val Lys Ala Phe Ser Asp Phe Lys Lys Glu Gln Glu Ser Asn Ser
                   725                 730                 735
   Val Glu Leu Tyr Ala Lys Lys Ile Ser Glu Leu Asp Tyr Lys Asn Lys
               740                 745                 750
   Arg Lys Phe Phe Glu Pro Phe Ser Gly Phe Arg Gln Lys Val Leu Asp
           755                 760                 765
   Lys Ile Asp Glu Ile Phe Val Ser Lys Pro Glu Arg Lys Lys Pro Ser
       770                 775                 780
   Gly Ala Leu His Glu Glu Thr Phe Arg Lys Glu Glu Glu Phe Tyr Gln
   785                 790                 795                 800
   Ser Tyr Gly Gly Lys Glu Gly Val Leu Lys Ala Leu Glu Leu Gly Lys
                   805                 810                 815
   Ile Arg Lys Val Asn Gly Lys Ile Val Lys Asn Gly Asp Met Phe Arg
               820                 825                 830
   Val Asp Ile Phe Lys His Lys Lys Thr Asn Lys Phe Tyr Ala Val Pro
           835                 840                 845
   Ile Tyr Thr Met Asp Phe Ala Leu Lys Val Leu Pro Asn Lys Ala Val
       850                 855                 860
   Ala Arg Ser Lys Lys Gly Glu Ile Lys Asp Trp Ile Leu Met Asp Glu
   865                 870                 875                 880
   Asn Tyr Glu Phe Cys Phe Ser Leu Tyr Lys Asp Ser Leu Ile Leu Ile
                   885                 890                 895
   Gln Thr Lys Asp Met Gln Glu Pro Glu Leu Val Tyr Phe Asn Ala Phe
               900                 905                 910
   Thr Ser Ser Thr Val Ser Leu Ile Val Ser Lys His Asp Asn Lys Phe
           915                 920                 925
   Glu Thr Leu Ser Lys Asn Gln Lys Ile Leu Phe Lys Asn Ala Asn Glu
       930                 935                 940
   Lys Glu Val Ile Ala Lys Ser Ile Gly Ile Gln Asn Leu Lys Val Phe
   945                 950                 955                 960
   Glu Lys Tyr Ile Val Ser Ala Leu Gly Glu Val Thr Lys Ala Glu Phe
                   965                 970                 975
   Arg Gln Arg Glu Asp Phe Lys Lys
               980
<210> 115
   <211> 1123
   <212> PRT
   <213> Francisella tularensis
<220>
   <223> CRISPR-associated endonuclease
<308> YP_666740
   <309> 2013-06-10
<400> 115
   Met Asn Val Lys Ile Leu Pro Ile Ala Ile Asp Leu Asp Val Lys Asn
    1               5                  10                  15
   Thr Gly Val Phe Ser Ala Phe Tyr Gln Lys Gly Thr Ser Leu Glu Lys
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Arg Arg His Lys Arg Arg Gly
       50                  55                  60
   Ile Asp Arg Lys Gln Leu Val Lys Arg Leu Phe Lys Leu Val Trp Thr
   65                  70                  75                  80
   Glu Gln Leu Asn Leu Glu Trp Asp Lys Asp Thr Gln Gln Ala Ile Ser
   85 90 95
   Phe Leu Phe Asn Arg Arg Gly Phe Ser Phe Ile Thr Asp Gly Tyr Ser
   100 105 110
   Thr Glu Tyr Leu Asn Ile Val Pro Glu Gln Val Lys Ala Ile Leu Met
   115 120 125
   Asp Ile Phe Asp Asp Tyr Asn Gly Glu Asp Asp Leu Asp Ser Tyr Leu
   130 135 140
   Lys Leu Ala Thr Glu Gln Glu Ser Lys Ile Ser Glu Ile Tyr Asn Lys
   145 150 155 160
   Leu Met Gln Lys Ile Leu Glu Phe Lys Leu Arg Lys Leu Cys Thr Asp
   165 170 175
   Ile Lys Asp Asp Lys Val Ser Thr Lys Thr Leu Lys Glu Ile Thr Ser
   180 185 190
   Tyr Glu Phe Glu Leu Leu Ala Asp Tyr Leu Ala Asn Tyr Ser Glu Ser
   195 200 205
   Leu Lys Thr Gln Lys Phe Ser Tyr Thr Asp Lys Gln Gly Asn Leu Lys
   210 215 220
   Glu Leu Ser Tyr Tyr His His Asp Lys Tyr Asn Ile Gln Glu Phe Leu
   225 230 235 240
   Lys Arg His Ala Thr Ile Asn Asp Glu Ile Leu Gly Thr Leu Leu Thr
   245 250 255
   Asp Asp Phe Asp Ile Trp Asn Phe Asn Phe Glu Lys Phe Asp Phe Asp
   260 265 270
   Lys Asn Glu Glu Lys Leu Gln Asn Gln Glu Asp Lys Asp His Thr Gln
   275 280 285
   Ala His Leu His His Phe Val Phe Val Val Asn Lys Ile Lys Ser Glu
   290 295 300
   Met Ala Ser Gly Gly Arg His Arg Ser Gln Tyr Phe Gln Glu Ile Thr
   305 310 315 320
   Asn Val Leu Asp Glu Asn Asn His Gln Glu Gly Tyr Leu Lys Asn Phe
   325 330 335
   Cys Glu Asn Leu His Asn Lys Lys Tyr Ser Asn Leu Ser Val Lys Asn
   340 345 350
   Leu Val Asn Leu Val Gly Asn Leu Ser Asn Leu Glu Leu Lys Pro Leu
   355 360 365
   Arg Lys Tyr Phe Asn Asp Lys Ile His Ala Lys Ala Asp His Trp Asp
   370 375 380
   Glu Gln Lys Phe Thr Glu Thr Tyr Cys His Trp Ile Leu Gly Glu Trp
   385 390 395 400
   Arg Val Gly Val Lys Asp Gln Asp Lys Lys Asp Gly Ala Lys Tyr Ser
   405 410 415
   Tyr Lys Asp Leu Cys Asn Glu Leu Lys Gln Lys Val Thr Lys Ala Gly
   420 425 430
   Leu Ile Asp Phe Leu Leu Glu Leu Asp Pro Cys Arg Thr Ile Pro Pro
   435 440 445
   Tyr Leu Asp Asn Asn Asn Arg Lys Pro Pro Lys Cys Gln Ser Leu Ile
   450 455 460
   Leu Asn Pro Lys Phe Leu Asp Asn Gln Tyr Pro Asn Trp Gln Gln Tyr
   465 470 475 480
   Leu Gln Glu Leu Lys Lys Leu Gln Ser Ile Gln Asp Tyr Leu Asp Ser
   485 490 495
   Phe Glu Thr Asp Leu Lys Asp Leu Lys Ser Ser Lys Asp Gln Pro Tyr
   500 505 510
   Phe Val Glu Tyr Lys Ser Ser Asn Gln Gln Met Ala Ser Gly Gln Arg
   515 520 525
   Asp Tyr Lys Asp Leu Asp Ala Arg Ile Leu Gln Phe Ile Phe Asp Arg
   530 535 540
   Val Lys Ala Ser Asp Glu Leu Leu Leu Asn Glu Ile Tyr Phe Gln Ala
   545 550 555 560
   Lys Lys Leu Lys Gln Lys Ala Ser Ser Glu Leu Glu Lys Leu Glu Ser
   565 570 575
   Ser Lys Lys Leu Asp Glu Val Ile Ala Asn Ser Gln Leu Ser Gln Ile
   580 585 590
   Leu Lys Ser Gln His Thr Asn Gly Ile Phe Glu Gln Gly Thr Phe Leu
   595 600 605
   His Leu Val Cys Lys Tyr Tyr Lys Gln Arg Gln Arg Ala Arg Asp Ser
   610 615 620
   Arg Leu Tyr Ile Met Pro Glu Tyr Arg Tyr Asp Lys Lys Leu Asp Lys
   625 630 635 640
   Tyr Asn Asn Thr Gly Arg Phe Asp Asp Asn Asn Gln Leu Leu Thr Tyr
   645 650 655
   Cys Asn His Lys Pro Arg Gln Lys Arg Tyr Gln Leu Leu Asn Asp Leu
   660 665 670
   Ala Gly Val Leu Gln Val Ser Arg Asn Gln Leu Leu Ser Ser Val Glu
   675 680 685
   Glu Trp Phe Gln Gln Ala Gln Arg Val Gly Glu Ile Ser Lys Ser Gln
   690 695 700
   Asp Glu Gln Ile Phe Glu Trp Leu Lys Ser Phe Lys Ile Ala Ser Tyr
   705 710 715 720
   Cys Lys Ala Ala Val Glu Met Gln Lys Gln Tyr Arg Gly Thr Leu Lys
   725 730 735
   Asn Ala Ile Gln Thr Ala Ile Phe Arg Gln Ser Glu Asn Ile Asn Lys
   740 745 750
   Asn Lys Asn Thr Gly Asn Gln Gln Gln Ala Leu Ser Glu Asn Ser Lys
   755 760 765
   Asp Val Lys Ser Leu Thr Ala Asp Glu Lys Lys Leu Leu Lys Leu Ile
   770 775 780
   Glu Asn Ile Ala Lys Ala Ser Gln Lys Ile Gly Glu Ser Leu Gly Leu
   785 790 795 800
   Asn Asp Lys Gln Ile Lys Lys Phe Asn Ser Ile Tyr Ser Phe Ala Gln
   805 810 815
   Ile Gln Gln Ile Ala Phe Ala Lys Arg Lys Gly Asn Ala Asn Thr Cys
   820 825 830
   Ala Val Cys Ser Ala Asp Asn Ala His Arg Met Gln Gln Ile Lys Ile
   835 840 845
   Thr Glu Leu Val Glu Asp Asn Lys Asp Asn Ile Ile Leu Ser Ala Lys
   850 855 860
   Ala Gln Arg Leu Pro Ala Ile Pro Thr Arg Ile Val Asp Gly Ala Val
   865 870 875 880
   Lys Lys Met Ala Thr Ile Leu Ala Lys Asn Ile Val Asp Asp Asn Trp
   885 890 895
   Gln Asn Ile Lys Gln Val Leu Ser Ala Lys His Gln Leu His Ile Pro
   900 905 910
   Ile Ile Thr Glu Ser Asn Ala Phe Glu Phe Glu Pro Ala Leu Ala Asp
   915 920 925
   Val Lys Gly Lys Ser Leu Lys Asp Arg Arg Lys Lys Ala Leu Glu Arg
   930 935 940
   Ile Ser Pro Glu Asn Ile Phe Lys Asp Lys Asn Asn Arg Ile Lys Glu
   945 950 955 960
   Phe Ala Lys Gly Ile Ser Ala Tyr Ser Gly Ala Asn Leu Thr Asp Gly
   965 970 975
   Asp Phe Asp Gly Ala Lys Glu Glu Leu Asp His Ile Ile Pro Arg Ser
   980 985 990
   His Lys Lys Tyr Gly Thr Leu Asn Asp Glu Ala Asn Leu Ile Cys Val
   995 1000 1005
   Thr Arg Asp Asp Asn Lys Asn Ile Phe Ala Ile Asp Thr Ser Lys Trp
   1010 1015 1020
   Phe Glu Ile Glu Thr Pro Ser Asp Leu Arg Asp Ile Gly Val Ala Thr
   1025 1030 1035 1040
   Ile Gln Tyr Lys Ile Asp Asn Asn Ser Arg Pro Lys Val Arg Val Lys
   1045 1050 1055
   Leu Asp Tyr Val Ile Asp Asp Asp Ser Lys Ile Asn Tyr Phe Met Asn
   1060 1065 1070
   His Ser Leu Leu Lys Ser Arg Tyr Pro Asp Lys Val Leu Glu Ile Leu
   1075 1080 1085
   Lys Gln Ser Thr Ile Ile Glu Phe Glu Ser Ser Gly Phe Asn Lys Thr
       1090                1095                1100
   Ile Lys Glu Met Leu Gly Met Thr Leu Ala Gly Ile Tyr Asn Glu Thr
   1105                1110                1115                1120
   Ser Asn Asn
<210> 116
   <211> 1123
   <212> PRT
   <213> Francisella tularensis
<220>
   <223> CRISPR-associated endonuclease
<308> YP_169608
   <309> 2014-05-16
<400> 116
   Met Asn Val Lys Ile Leu Pro Ile Ala Ile Asp Leu Asp Val Lys Asn
    1               5                  10                  15
   Thr Gly Val Phe Ser Ala Phe Tyr Gln Lys Gly Thr Ser Leu Glu Lys
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Arg Arg His Lys Arg Arg Gly
       50                  55                  60
   Ile Asp Arg Lys Gln Leu Val Lys Arg Leu Phe Lys Leu Val Trp Thr
   65                  70                  75                  80
   Glu Gln Leu Asn Leu Glu Trp Asp Lys Asp Thr Gln Gln Ala Ile Ser
                   85                  90                  95
   Phe Leu Phe Asn Arg Arg Gly Phe Ser Phe Ile Thr Asp Gly Tyr Ser
               100                 105                 110
   Thr Glu Tyr Leu Asn Ile Val Pro Glu Gln Val Lys Ala Ile Leu Met
           115                 120                 125
   Asp Ile Phe Asp Asp Tyr Asn Gly Glu Asp Asp Leu Asp Ser Tyr Leu
       130                 135                 140
   Lys Leu Ala Thr Glu Gln Glu Ser Lys Ile Ser Glu Ile Tyr Asn Lys
   145                 150                 155                 160
   Leu Met Gln Lys Ile Leu Glu Phe Lys Leu Arg Lys Leu Cys Thr Asp
                   165                 170                 175
   Ile Lys Asp Asp Lys Val Ser Thr Lys Thr Leu Lys Glu Ile Thr Ser
               180                 185                 190
   Tyr Glu Phe Glu Leu Leu Ala Asp Tyr Leu Ala Asn Tyr Ser Glu Ser
           195                 200                 205
   Leu Lys Thr Gln Lys Phe Ser Tyr Thr Asp Lys Gln Gly Asn Leu Lys
       210                 215                 220
   Glu Leu Ser Tyr Tyr His His Asp Lys Tyr Asn Ile Gln Glu Phe Leu
   225                 230                 235                 240
   Lys Arg His Ala Thr Ile Asn Asp Glu Ile Leu Gly Thr Leu Leu Thr
                   245                 250                 255
   Asp Asp Phe Asp Ile Trp Asn Phe Asn Phe Glu Lys Phe Asp Phe Asp
               260                 265                 270
   Lys Asn Glu Glu Lys Leu Gln Asn Gln Glu Asp Lys Asp His Thr Gln
           275                 280                 285
   Ala His Leu His His Phe Val Phe Val Val Asn Lys Ile Lys Ser Glu
       290                 295                 300
   Met Ala Ser Gly Gly Arg His Arg Ser Gln Tyr Phe Gln Glu Ile Thr
   305                 310                 315                 320
   Asn Val Leu Asp Glu Asn Asn His Gln Glu Gly Tyr Leu Lys Asn Phe
                   325                 330                 335
   Cys Glu Asn Leu His Asn Lys Lys Tyr Ser Asn Leu Ser Val Lys Asn
   340 345 350
   Leu Val Asn Leu Val Gly Asn Leu Ser Asn Leu Glu Leu Lys Pro Leu
   355 360 365
   Arg Lys Tyr Phe Asn Asp Lys Ile His Ala Lys Ala Asp His Trp Asp
   370 375 380
   Glu Gln Lys Phe Thr Glu Thr Tyr Cys His Trp Ile Leu Gly Glu Trp
   385 390 395 400
   Arg Val Gly Val Lys Asp Gln Asp Lys Lys Asp Gly Ala Lys Tyr Ser
   405 410 415
   Tyr Lys Asp Leu Cys Asn Glu Leu Lys Gln Lys Val Thr Lys Ala Gly
   420 425 430
   Leu Ile Asp Phe Leu Leu Glu Leu Asp Pro Cys Arg Thr Ile Pro Pro
   435 440 445
   Tyr Leu Asp Asn Asn Asn Arg Lys Pro Pro Lys Cys Gln Ser Leu Ile
   450 455 460
   Leu Asn Pro Lys Phe Leu Asp Asn Gln Tyr Pro Asn Trp Gln Gln Tyr
   465 470 475 480
   Leu Gln Glu Leu Lys Lys Leu Gln Ser Ile Gln Asp Tyr Leu Asp Ser
   485 490 495
   Phe Glu Thr Asp Leu Lys Asp Leu Lys Ser Ser Lys Asp Gln Pro Tyr
   500 505 510
   Phe Val Glu Tyr Lys Ser Ser Asn Gln Gln Met Ala Ser Gly Gln Arg
   515 520 525
   Asp Tyr Lys Asp Leu Asp Ala Arg Ile Leu Gln Phe Ile Phe Asp Arg
   530 535 540
   Val Lys Ala Ser Asp Glu Leu Leu Leu Asn Glu Ile Tyr Phe Gln Ala
   545 550 555 560
   Lys Lys Leu Lys Gln Lys Ala Ser Ser Glu Leu Glu Lys Leu Glu Ser
   565 570 575
   Ser Lys Lys Leu Asp Glu Val Ile Ala Asn Ser Gln Leu Ser Gln Ile
   580 585 590
   Leu Lys Ser Gln His Thr Asn Gly Ile Phe Glu Gln Gly Thr Phe Leu
   595 600 605
   His Leu Val Cys Lys Tyr Tyr Lys Gln Arg Gln Arg Ala Arg Asp Ser
   610 615 620
   Arg Leu Tyr Ile Met Pro Glu Tyr Arg Tyr Asp Lys Lys Leu Asp Lys
   625 630 635 640
   Tyr Asn Asn Thr Gly Arg Phe Asp Asp Asn Asn Gln Leu Leu Thr Tyr
   645 650 655
   Cys Asn His Lys Pro Arg Gln Lys Arg Tyr Gln Leu Leu Asn Asp Leu
   660 665 670
   Ala Gly Val Leu Gln Val Ser Arg Asn Gln Leu Leu Ser Ser Val Glu
   675 680 685
   Glu Trp Phe Gln Gln Ala Gln Arg Val Gly Glu Ile Ser Lys Ser Gln
   690 695 700
   Asp Glu Gln Ile Phe Glu Trp Leu Lys Ser Phe Lys Ile Ala Ser Tyr
   705 710 715 720
   Cys Lys Ala Ala Val Glu Met Gln Lys Gln Tyr Arg Gly Thr Leu Lys
   725 730 735
   Asn Ala Ile Gln Thr Ala Ile Phe Arg Gln Ser Glu Asn Ile Asn Lys
   740 745 750
   Asn Lys Asn Thr Gly Asn Gln Gln Gln Ala Leu Ser Glu Asn Ser Lys
   755 760 765
   Asp Val Lys Ser Leu Thr Ala Asp Glu Lys Lys Leu Leu Lys Leu Ile
   770 775 780
   Glu Asn Ile Ala Lys Ala Ser Gln Lys Ile Gly Glu Ser Leu Gly Leu
   785 790 795 800
   Asn Asp Lys Gln Ile Lys Lys Phe Asn Ser Ile Tyr Ser Phe Ala Gln
   805 810 815
   Ile Gln Gln Ile Ala Phe Ala Lys Arg Lys Gly Asn Ala Asn Thr Cys
   820 825 830
   Ala Val Cys Ser Ala Asp Asn Ala His Arg Met Gln Gln Ile Lys Ile
   835 840 845
   Thr Glu Leu Val Glu Asp Asn Lys Asp Asn Ile Ile Leu Ser Ala Lys
       850                 855                 860
   Ala Gln Arg Leu Pro Ala Ile Pro Thr Arg Ile Val Asp Gly Ala Val
   865                 870                 875                 880
   Lys Lys Met Ala Thr Ile Leu Ala Lys Asn Ile Val Asp Asp Asn Trp
                   885                 890                 895
   Gln Asn Ile Lys Gln Val Leu Ser Ala Lys His Gln Leu His Ile Pro
               900                 905                 910
   Ile Ile Thr Glu Ser Asn Ala Phe Glu Phe Glu Pro Ala Leu Ala Asp
           915                 920                 925
   Val Lys Gly Lys Ser Leu Lys Asp Arg Arg Lys Lys Ala Leu Glu Arg
       930                 935                 940
   Ile Ser Pro Glu Asn Ile Phe Lys Asp Lys Asn Asn Arg Ile Lys Glu
   945                 950                 955                 960
   Phe Ala Lys Gly Ile Ser Ala Tyr Ser Gly Ala Asn Leu Thr Asp Gly
                   965                 970                 975
   Asp Phe Asp Gly Ala Lys Glu Glu Leu Asp His Ile Ile Pro Arg Ser
               980                 985                 990
   His Lys Lys Tyr Gly Thr Leu Asn Asp Glu Ala Asn Leu Ile Cys Val
           995                 1000                1005
   Thr Arg Asp Asp Asn Lys Asn Ile Phe Ala Ile Asp Thr Ser Lys Trp
       1010                1015                1020
   Phe Glu Ile Glu Thr Pro Ser Asp Leu Arg Asp Ile Gly Val Ala Thr
   1025                1030                1035                1040
   Ile Gln Tyr Lys Ile Asp Asn Asn Ser Arg Pro Lys Val Arg Val Lys
                   1045                1050                1055
   Leu Asp Tyr Val Ile Asp Asp Asp Ser Lys Ile Asn Tyr Phe Met Asn
               1060                1065                1070
   His Ser Leu Leu Lys Ser Arg Tyr Pro Asp Lys Val Leu Glu Ile Leu
           1075                1080                1085
   Lys Gln Ser Thr Ile Ile Glu Phe Glu Ser Ser Gly Phe Asn Lys Thr
       1090                1095                1100
   Ile Lys Glu Met Leu Gly Met Thr Leu Ala Gly Ile Tyr Asn Glu Thr
   1105                1110                1115                1120
   Ser Asn Asn
<210> 117
   <211> 1371
   <212> PRT
   <213> Streptococcus dysgalactiae
<220>
   <223> CRISPR-associated endonuclease
<308> YP_006859751
   <309> 2013-08-27
<400> 117
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Arg
   65                  70                  75                  80
   Tyr Leu Gln Glu Ile Phe Ser Ser Glu Met Ser Lys Val Asp Asp Ser
                   85                  90                  95
   Phe Phe His Arg Leu Glu Glu Ser Phe Leu Val Glu Glu Asp Lys Lys
                   100                 105                 110
   His Glu Arg His Pro Ile Phe Gly Asn Ile Val Asp Glu Val Ala Tyr
               115                 120                 125
   His Glu Lys Tyr Pro Thr Ile Tyr His Leu Arg Lys Lys Leu Ala Asp
           130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Met Asp Lys Leu Phe Ile Gln Leu Val Gln Thr Tyr
               180                 185                 190
   Asn Gln Leu Phe Glu Glu Asn Pro Ile Asn Ala Ser Arg Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Arg Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Ala Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Glu Phe Leu Ser Gly Lys Gln
       530                 535                 540
   Lys Glu Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Lys Glu Met Ile Glu Glu Arg Leu Lys Thr Tyr Ala
   625                 630                 635                 640
   His Leu Phe Asp Asp Lys Val Met Lys Gln Leu Lys Arg Arg His Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Ile Gln Leu Ile His Asp Asp Ser Leu Thr Phe
       690                 695                 700
   Lys Glu Ala Ile Gln Lys Ala Gln Val Ser Gly Gln Gly His Ser Leu
   705                 710                 715                 720
   His Glu Gln Ile Ala Asn Leu Ala Gly Ser Pro Ala Ile Lys Lys Gly
                   725                 730                 735
   Ile Leu Gln Ser Val Lys Val Val Asp Glu Leu Val Lys Val Met Gly
               740                 745                 750
   His Lys Pro Glu Asn Ile Val Ile Glu Met Ala Arg Glu Asn Gln Thr
           755                 760                 765
   Thr Gln Lys Gly Gln Lys Asn Ser Arg Glu Arg Met Lys Arg Ile Glu
       770                 775                 780
   Glu Gly Ile Lys Glu Leu Gly Ser Gln Ile Leu Lys Glu His Pro Val
   785                 790                 795                 800
   Glu Asn Thr Gln Leu Gln Asn Glu Lys Leu Tyr Leu Tyr Tyr Leu Gln
                   805                 810                 815
   Asn Gly Arg Asp Met Tyr Val Asp Gln Glu Leu Asp Ile Asn Arg Leu
               820                 825                 830
   Ser Asp Tyr Asp Val Asp His Ile Val Pro Gln Ser Phe Ile Lys Asp
           835                 840                 845
   Asp Ser Ile Asp Asn Lys Val Leu Thr Arg Ser Asp Lys Asn Arg Gly
       850                 855                 860
   Lys Ser Asp Asp Val Pro Ser Glu Glu Val Val Lys Lys Met Lys Asn
   865                 870                 875                 880
   Tyr Trp Arg Gln Leu Leu Asn Ala Lys Leu Ile Thr Gln Arg Lys Phe
                   885                 890                 895
   Asp Asn Leu Thr Lys Ala Glu Arg Gly Gly Leu Ser Glu Leu Asp Lys
               900                 905                 910
   Ala Gly Phe Ile Lys Arg Gln Leu Val Glu Thr Arg Gln Ile Thr Lys
           915                 920                 925
   His Val Ala Gln Ile Leu Asp Ser Arg Met Asn Thr Lys Tyr Asp Glu
       930                 935                 940
   Asn Asp Lys Leu Ile Arg Glu Val Lys Val Ile Thr Leu Lys Ser Lys
   945                 950                 955                 960
   Leu Val Ser Asp Phe Arg Lys Asp Phe Gln Phe Tyr Lys Val Arg Glu
                   965                 970                 975
   Ile Asn Asn Tyr His His Ala His Asp Ala Tyr Leu Asn Ala Val Val
               980                 985                 990
   Gly Thr Ala Leu Ile Lys Lys Tyr Pro Lys Leu Glu Ser Glu Phe Val
           995                 1000                1005
   Tyr Gly Asp Tyr Lys Val Tyr Asp Val Arg Lys Met Ile Ala Lys Ser
       1010                1015                1020
   Glu Gln Glu Ile Gly Lys Ala Thr Ala Lys Arg Phe Phe Tyr Ser Asn
   1025                1030                1035                1040
   Ile Met Asn Phe Phe Lys Thr Glu Ile Thr Leu Ala Asn Gly Glu Ile
                   1045                1050                1055
   Arg Lys Arg Pro Leu Ile Glu Thr Asn Glu Glu Thr Gly Glu Ile Val
               1060                1065                1070
   Trp Asn Lys Gly Arg Asp Phe Ala Thr Val Arg Lys Val Leu Ser Met
           1075                1080                1085
   Pro Gln Val Asn Ile Val Lys Lys Thr Glu Val Gln Thr Gly Ala Leu
       1090                1095                1100
   Thr Asn Glu Ser Ile Tyr Ala Arg Gly Ser Phe Asp Lys Leu Ile Ser
   1105                1110                1115                1120
   Arg Lys His Arg Phe Glu Ser Ser Lys Tyr Gly Gly Phe Gly Ser Pro
                   1125                1130                1135
   Thr Val Thr Tyr Ser Val Leu Val Val Ala Lys Ser Lys Val Gln Asp
               1140                1145                1150
   Gly Lys Val Lys Lys Ile Lys Thr Gly Lys Glu Leu Ile Gly Ile Thr
           1155                1160                1165
   Leu Leu Asp Lys Leu Val Phe Glu Lys Asn Pro Leu Lys Phe Ile Glu
       1170                1175                1180
   Asp Lys Gly Tyr Gly Asn Val Gln Ile Asp Lys Cys Ile Lys Leu Pro
   1185                1190                1195                1200
   Lys Tyr Ser Leu Phe Glu Phe Glu Asn Gly Thr Arg Arg Met Leu Ala
                   1205                1210                1215
   Ser Val Met Ala Asn Asn Asn Ser Arg Gly Asp Leu Gln Lys Ala Asn
               1220                1225                1230
   Glu Met Phe Leu Pro Ala Lys Leu Val Thr Leu Leu Tyr His Ala His
           1235                1240                1245
   Lys Ile Glu Ser Ser Lys Glu Leu Glu His Glu Ala Tyr Ile Leu Asp
       1250                1255                1260
   His Tyr Asn Asp Leu Tyr Gln Leu Leu Ser Tyr Ile Glu Arg Phe Ala
   1265                1270                1275                1280
   Ser Leu Tyr Val Asp Val Glu Lys Asn Ile Ser Lys Val Lys Glu Leu
                   1285                1290                1295
   Phe Ser Asn Ile Glu Ser Tyr Ser Ile Ser Glu Ile Cys Ser Ser Val
               1300                1305                1310
   Ile Asn Leu Leu Thr Leu Thr Ala Ser Gly Ala Pro Ala Asp Phe Lys
           1315                1320                1325
   Phe Leu Gly Thr Thr Ile Pro Arg Lys Arg Tyr Gly Ser Pro Gln Ser
       1330                1335                1340
   Ile Leu Ser Ser Thr Leu Ile His Gln Ser Ile Thr Gly Leu Tyr Glu
   1345                1350                1355                1360
   Thr Arg Ile Asp Leu Ser Gln Leu Gly Gly Asp
                   1365                1370
<210> 118
   <211> 1345
   <212> PRT
   <213> Streptococcus mutans
<220>
   <223> CRISPR-associated endonuclease
<308> YP_006490549
   <309> 2013-06-11
<400> 118
   Met Lys Lys Pro Tyr Ser Ile Gly Leu Asp Ile Gly Thr Asn Ser Val
   1               5                   10                  15
   Gly Trp Ala Val Val Thr Asp Asp Tyr Lys Val Pro Ala Lys Lys Met
               20                  25                  30
   Lys Val Leu Gly Asn Thr Asp Lys Ser His Ile Lys Lys Asn Leu Leu
           35                  40                  45
   Gly Ala Leu Leu Phe Asp Ser Gly Asn Thr Ala Glu Asp Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ser Glu Glu Met Gly Lys Val Asp Asp Ser
                   85                  90                  95
   Phe Phe His Arg Leu Asp Glu Ser Phe Leu Thr Asp Asp Asp Lys Asn
               100                 105                 110
   Phe Asp Ser His Pro Ile Phe Gly Asn Lys Ala Glu Glu Asp Ala Tyr
           115                 120                 125
   His Gln Lys Phe Pro Thr Ile Tyr His Leu Arg Lys His Leu Ala Asp
   130 135 140
   Ser Thr Glu Lys Ala Asp Leu Arg Leu Val Tyr Leu Ala Leu Ala His
   145 150 155 160
   Met Ile Lys Phe Arg Gly His Phe Leu Ile Glu Gly Glu Leu Asn Ala
   165 170 175
   Glu Asn Thr Asp Val Gln Lys Leu Phe Ala Asp Phe Val Gly Val Tyr
   180 185 190
   Asp Arg Thr Phe Asp Asp Ser His Leu Ser Glu Ile Thr Val Asp Ala
   195 200 205
   Ser Ser Ile Leu Thr Glu Lys Ile Ser Lys Ser Arg Arg Leu Glu Lys
   210 215 220
   Leu Ile Asn Asn Tyr Pro Lys Glu Lys Lys Asn Thr Leu Phe Gly Asn
   225 230 235 240
   Leu Ile Ala Leu Ser Leu Gly Leu Gln Pro Asn Phe Lys Thr Asn Phe
   245 250 255
   Lys Leu Ser Glu Asp Ala Lys Leu Gln Phe Ser Lys Asp Thr Tyr Glu
   260 265 270
   Glu Glu Leu Glu Val Leu Leu Ala Gln Ile Gly Asp Asn Tyr Ala Glu
   275 280 285
   Leu Phe Leu Ser Ala Lys Lys Leu Tyr Asp Ser Ile Leu Leu Ser Gly
   290 295 300
   Ile Leu Thr Val Thr Asp Val Ser Thr Lys Ala Pro Leu Ser Ala Ser
   305 310 315 320
   Met Ile Gln Arg Tyr Asn Glu His Gln Met Asp Leu Ala Gln Leu Lys
   325 330 335
   Gln Phe Ile Arg Gln Lys Leu Ser Asp Lys Tyr Asn Glu Val Phe Ser
   340 345 350
   Asp Val Ser Lys Asp Gly Tyr Ala Gly Tyr Ile Asp Gly Lys Thr Asn
   355 360 365
   Gln Glu Ala Phe Tyr Lys Tyr Leu Lys Gly Leu Leu Asn Lys Ile Glu
   370 375 380
   Gly Ser Gly Tyr Phe Leu Asp Lys Ile Glu Arg Glu Asp Phe Leu Arg
   385 390 395 400
   Lys Gln Arg Thr Phe Asp Asn Gly Ser Ile Pro His Gln Ile His Leu
   405 410 415
   Gln Glu Met Arg Ala Ile Ile Arg Arg Gln Ala Glu Phe Tyr Pro Phe
   420 425 430
   Leu Ala Asp Asn Gln Asp Arg Ile Glu Lys Ile Leu Thr Phe Arg Ile
   435 440 445
   Pro Tyr Tyr Val Gly Pro Leu Ala Arg Gly Lys Ser Asp Phe Ala Trp
   450 455 460
   Leu Ser Arg Lys Ser Ala Asp Lys Ile Thr Pro Trp Asn Phe Asp Glu
   465 470 475 480
   Ile Val Asp Lys Glu Ser Ser Val Glu Ala Phe Ile Asn Arg Met Thr
   485 490 495
   Asn Tyr Asp Leu Tyr Leu Pro Asn Gln Lys Val Leu Pro Lys His Ser
   500 505 510
   Leu Leu Tyr Glu Lys Phe Thr Val Tyr Asn Glu Leu Thr Lys Val Lys
   515 520 525
   Tyr Lys Thr Glu Gln Gly Lys Thr Ala Phe Phe Asp Ala Asn Met Lys
   530 535 540
   Gln Glu Ile Phe Asp Gly Val Phe Lys Val Tyr Arg Lys Val Thr Lys
   545 550 555 560
   Asp Lys Leu Met Asp Phe Leu Glu Lys Glu Phe Asp Glu Phe Arg Ile
   565 570 575
   Val Asp Leu Thr Gly Leu Asp Lys Glu Asn Lys Ala Phe Asn Ala Ser
   580 585 590
   Tyr Gly Thr Tyr His Asp Leu Arg Lys Ile Leu Asp Lys Asp Phe Leu
   595 600 605
   Asp Asn Ser Lys Asn Glu Lys Ile Leu Glu Asp Ile Val Leu Thr Leu
   610 615 620
   Thr Leu Phe Glu Asp Arg Glu Met Ile Arg Lys Arg Leu Lys Asn Tyr
   625 630 635 640
   Ser Asp Leu Leu Thr Lys Glu Gln Leu Lys Lys Leu Glu Arg Arg His
   645 650 655
   Tyr Thr Gly Trp Gly Arg Leu Ser Ala Glu Leu Ile His Gly Ile Arg
   660 665 670
   Asn Lys Glu Ser Arg Lys Thr Ile Leu Asp Tyr Leu Ile Asp Asp Gly
   675 680 685
   Asn Ser Asn Arg Asn Phe Met Gln Leu Ile Asn Asp Asp Ala Leu Ser
   690 695 700
   Phe Lys Glu Glu Ile Ala Lys Ala Gln Val Ile Gly Glu Thr Asp Asn
   705 710 715 720
   Leu Asn Gln Val Val Ser Asp Ile Ala Gly Ser Pro Ala Ile Lys Lys
   725 730 735
   Gly Ile Leu Gln Ser Leu Lys Ile Val Asp Glu Leu Val Lys Ile Met
   740 745 750
   Gly His Gln Pro Glu Asn Ile Val Val Glu Met Ala Arg Glu Asn Gln
   755 760 765
   Phe Thr Asn Gln Gly Arg Arg Asn Ser Gln Gln Arg Leu Lys Gly Leu
   770 775 780
   Thr Asp Ser Ile Lys Glu Phe Gly Ser Gln Ile Leu Lys Glu His Pro
   785 790 795 800
   Val Glu Asn Ser Gln Leu Gln Asn Asp Arg Leu Phe Leu Tyr Tyr Leu
   805 810 815
   Gln Asn Gly Arg Asp Met Tyr Thr Gly Glu Glu Leu Asp Ile Asp Tyr
   820 825 830
   Leu Ser Gln Tyr Asp Ile Asp His Ile Ile Pro Gln Ala Phe Ile Lys
   835 840 845
   Asp Asn Ser Ile Asp Asn Arg Val Leu Thr Ser Ser Lys Glu Asn Arg
   850 855 860
   Gly Lys Ser Asp Asp Val Pro Ser Lys Asp Val Val Arg Lys Met Lys
   865 870 875 880
   Ser Tyr Trp Ser Lys Leu Leu Ser Ala Lys Leu Ile Thr Gln Arg Lys
   885 890 895
   Phe Asp Asn Leu Thr Lys Gly Glu Arg Gly Gly Leu Thr Asp Asp Asp
   900 905 910
   Lys Ala Gly Phe Ile Lys Arg Gln Leu Val Glu Thr Arg Gln Ile Thr
   915 920 925
   Lys His Val Ala Arg Ile Leu Asp Glu Arg Phe Asn Thr Glu Thr Asp
   930 935 940
   Glu Asn Asn Lys Lys Ile Arg Gln Val Lys Ile Val Thr Leu Lys Ser
   945 950 955 960
   Asn Leu Val Ser Asn Phe Arg Lys Glu Phe Glu Leu Tyr Lys Val Arg
   965 970 975
   Glu Ile Asn Asp Tyr His His Ala His Asp Ala Tyr Leu Asn Ala Val
   980 985 990
   Ile Gly Lys Ala Leu Leu Gly Val Tyr Pro Gln Leu Glu Pro Glu Phe
   995 1000 1005
   Val Tyr Gly Asp Tyr Pro His Phe His Gly His Lys Glu Asn Lys Ala
   1010 1015 1020
   Thr Ala Lys Lys Phe Phe Tyr Ser Asn Ile Met Asn Phe Phe Lys Lys
   1025 1030 1035 1040
   Asp Asp Val Arg Thr Asp Lys Asn Gly Glu Ile Ile Trp Lys Lys Asp
   1045 1050 1055
   Glu Tyr Ile Ser Asn Ile Lys Lys Val Leu Ser Tyr Pro Gln Val Asn
   1060 1065 1070
   Ile Val Lys Lys Val Glu Glu Gln Thr Gly Gly Phe Ser Lys Glu Ser
   1075 1080 1085
   Ile Leu Pro Lys Gly Asp Ser Asp Lys Leu Ile Pro Arg Lys Thr Lys
   1090 1095 1100
   Lys Phe Tyr Trp Asp Thr Lys Lys Tyr Gly Gly Phe Asp Ser Pro Ile
   1105 1110 1115 1120
   Val Ala Tyr Ser Ile Leu Val Ile Ala Asp Ile Glu Lys Gly Lys Ser
   1125 1130 1135
   Lys Lys Leu Lys Thr Val Lys Ala Leu Val Gly Val Thr Ile Met Glu
               1140                1145                1150
   Lys Met Thr Phe Glu Arg Asp Pro Val Ala Phe Leu Glu Arg Lys Gly
           1155                1160                1165
   Tyr Arg Asn Val Gln Glu Glu Asn Ile Ile Lys Leu Pro Lys Tyr Ser
       1170                1175                1180
   Leu Phe Lys Leu Glu Asn Gly Arg Lys Arg Leu Leu Ala Ser Ala Arg
   1185                1190                1195                1200
   Glu Leu Gln Lys Gly Asn Glu Ile Val Leu Pro Asn His Leu Gly Thr
                   1205                1210                1215
   Leu Leu Tyr His Ala Lys Asn Ile His Lys Val Asp Glu Pro Lys His
               1220                1225                1230
   Leu Asp Tyr Val Asp Lys His Lys Asp Glu Phe Lys Glu Leu Leu Asp
           1235                1240                1245
   Val Val Ser Asn Phe Ser Lys Lys Tyr Thr Leu Ala Glu Gly Asn Leu
       1250                1255                1260
   Glu Lys Ile Lys Glu Leu Tyr Ala Gln Asn Asn Gly Glu Asp Leu Lys
   1265                1270                1275                1280
   Glu Leu Ala Ser Ser Phe Ile Asn Leu Leu Thr Phe Thr Ala Ile Gly
                   1285                1290                1295
   Ala Pro Ala Thr Phe Lys Phe Phe Asp Lys Asn Ile Asp Arg Lys Arg
               1300                1305                1310
   Tyr Thr Ser Thr Thr Glu Ile Leu Asn Ala Thr Leu Ile His Gln Ser
           1315                1320                1325
   Ile Thr Gly Leu Tyr Glu Thr Arg Ile Asp Leu Ser Lys Leu Gly Gly
       1330                1335                1340
   Asp
   1345
<210> 119
   <211> 1388
   <212> PRT
   <213> Streptococcus thermophilus
<220>
   <223> CRISPR-associated endonuclease
<308> YP_006340526
   <309> 2013-06-11
<400> 119
   Met Thr Lys Pro Tyr Ser Ile Gly Leu Asp Ile Gly Thr Asn Ser Val
    1               5                  10                  15
   Gly Trp Ala Val Ile Thr Asp Asn Tyr Lys Val Pro Ser Lys Lys Met
               20                  25                  30
   Lys Val Leu Gly Asn Thr Ser Lys Lys Tyr Ile Lys Lys Asn Leu Leu
           35                  40                  45
   Gly Val Leu Leu Phe Asp Ser Gly Ile Thr Ala Glu Gly Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ser Thr Glu Met Ala Thr Leu Asp Asp Ala
                   85                  90                  95
   Phe Phe Gln Arg Leu Asp Asp Ser Phe Leu Val Pro Asp Asp Lys Arg
               100                 105                 110
   Asp Ser Lys Tyr Pro Ile Phe Gly Asn Leu Val Glu Glu Lys Ala Tyr
           115                 120                 125
   His Asp Glu Phe Pro Thr Ile Tyr His Leu Arg Lys Tyr Leu Ala Asp
       130                 135                 140
   Ser Thr Lys Lys Ala Asp Leu Arg Leu Val Tyr Leu Ala Leu Ala His
   145                 150                 155                 160
   Met Ile Lys Tyr Arg Gly His Phe Leu Ile Glu Gly Glu Phe Asn Ser
                       165                 170                 175
   Lys Asn Asn Asp Ile Gln Lys Asn Phe Gln Asp Phe Leu Asp Thr Tyr
                   180                 185                 190
   Asn Ala Ile Phe Glu Ser Asp Leu Ser Leu Glu Asn Ser Lys Gln Leu
               195                 200                 205
   Glu Glu Ile Val Lys Asp Lys Ile Ser Lys Leu Glu Lys Lys Asp Arg
       210                 215                 220
   Ile Leu Lys Leu Phe Pro Gly Glu Lys Asn Ser Gly Ile Phe Ser Glu
   225                 230                 235                 240
   Phe Leu Lys Leu Ile Val Gly Asn Gln Ala Asp Phe Arg Lys Cys Phe
                   245                 250                 255
   Asn Leu Asp Glu Lys Ala Ser Leu His Phe Ser Lys Glu Ser Tyr Asp
               260                 265                 270
   Glu Asp Leu Glu Thr Leu Leu Gly Tyr Ile Gly Asp Asp Tyr Ser Asp
           275                 280                 285
   Val Phe Leu Lys Ala Lys Lys Leu Tyr Asp Ala Ile Leu Leu Ser Gly
       290                 295                 300
   Phe Leu Thr Val Thr Asp Asn Glu Thr Glu Ala Pro Leu Ser Ser Ala
   305                 310                 315                 320
   Met Ile Lys Arg Tyr Asn Glu His Lys Glu Asp Leu Ala Leu Leu Lys
                   325                 330                 335
   Glu Tyr Ile Arg Asn Ile Ser Leu Lys Thr Tyr Asn Glu Val Phe Lys
               340                 345                 350
   Asp Asp Thr Lys Asn Gly Tyr Ala Gly Tyr Ile Asp Gly Lys Thr Asn
           355                 360                 365
   Gln Glu Asp Phe Tyr Val Tyr Leu Lys Asn Leu Leu Ala Glu Phe Glu
       370                 375                 380
   Gly Ala Asp Tyr Phe Leu Glu Lys Ile Asp Arg Glu Asp Phe Leu Arg
   385                 390                 395                 400
   Lys Gln Arg Thr Phe Asp Asn Gly Ser Ile Pro Tyr Gln Ile His Leu
                   405                 410                 415
   Gln Glu Met Arg Ala Ile Leu Asp Lys Gln Ala Lys Phe Tyr Pro Phe
               420                 425                 430
   Leu Ala Lys Asn Lys Glu Arg Ile Glu Lys Ile Leu Thr Phe Arg Ile
           435                 440                 445
   Pro Tyr Tyr Val Gly Pro Leu Ala Arg Gly Asn Ser Asp Phe Ala Trp
       450                 455                 460
   Ser Ile Arg Lys Arg Asn Glu Lys Ile Thr Pro Trp Asn Phe Glu Asp
   465                 470                 475                 480
   Val Ile Asp Lys Glu Ser Ser Ala Glu Ala Phe Ile Asn Arg Met Thr
                   485                 490                 495
   Ser Phe Asp Leu Tyr Leu Pro Glu Glu Lys Val Leu Pro Lys His Ser
               500                 505                 510
   Leu Leu Tyr Glu Thr Phe Asn Val Tyr Asn Glu Leu Thr Lys Val Arg
           515                 520                 525
   Phe Ile Ala Glu Ser Met Arg Asp Tyr Gln Phe Leu Asp Ser Lys Gln
       530                 535                 540
   Lys Lys Asp Ile Val Arg Leu Tyr Phe Lys Asp Lys Arg Lys Val Thr
   545                 550                 555                 560
   Asp Lys Asp Ile Ile Glu Tyr Leu His Ala Ile Tyr Gly Tyr Asp Gly
                   565                 570                 575
   Ile Glu Leu Lys Gly Ile Glu Lys Gln Phe Asn Ser Ser Leu Ser Thr
               580                 585                 590
   Tyr His Asp Leu Leu Asn Ile Ile Asn Asp Lys Glu Phe Leu Asp Asp
           595                 600                 605
   Ser Ser Asn Glu Ala Ile Ile Glu Glu Ile Ile His Thr Leu Thr Ile
       610                 615                 620
   Phe Glu Asp Arg Glu Met Ile Lys Gln Arg Leu Ser Lys Phe Glu Asn
   625                 630                 635                 640
   Ile Phe Asp Lys Ser Val Leu Lys Lys Leu Ser Arg Arg His Tyr Thr
                   645                 650                 655
   Gly Trp Gly Lys Leu Ser Ala Lys Leu Ile Asn Gly Ile Arg Asp Glu
               660                 665                 670
   Lys Ser Gly Asn Thr Ile Leu Asp Tyr Leu Ile Asp Asp Gly Ile Ser
           675                 680                 685
   Asn Arg Asn Phe Met Gln Leu Ile His Asp Asp Ala Leu Ser Phe Lys
       690                 695                 700
   Lys Lys Ile Gln Lys Ala Gln Ile Ile Gly Asp Glu Asp Lys Gly Asn
   705                 710                 715                 720
   Ile Lys Glu Val Val Lys Ser Leu Pro Gly Ser Pro Ala Ile Lys Lys
                   725                 730                 735
   Gly Ile Leu Gln Ser Ile Lys Ile Val Asp Glu Leu Val Lys Val Met
               740                 745                 750
   Gly Gly Arg Lys Pro Glu Ser Ile Val Val Glu Met Ala Arg Glu Asn
           755                 760                 765
   Gln Tyr Thr Asn Gln Gly Lys Ser Asn Ser Gln Gln Arg Leu Lys Arg
       770                 775                 780
   Leu Glu Lys Ser Leu Lys Glu Leu Gly Ser Lys Ile Leu Lys Glu Asn
   785                 790                 795                 800
   Ile Pro Ala Lys Leu Ser Lys Ile Asp Asn Asn Ala Leu Gln Asn Asp
                   805                 810                 815
   Arg Leu Tyr Leu Tyr Tyr Leu Gln Asn Gly Lys Asp Met Tyr Thr Gly
               820                 825                 830
   Asp Asp Leu Asp Ile Asp Arg Leu Ser Asn Tyr Asp Ile Asp His Ile
           835                 840                 845
   Ile Pro Gln Ala Phe Leu Lys Asp Asn Ser Ile Asp Asn Lys Val Leu
       850                 855                 860
   Val Ser Ser Ala Ser Asn Arg Gly Lys Ser Asp Asp Phe Pro Ser Leu
   865                 870                 875                 880
   Glu Val Val Lys Lys Arg Lys Thr Phe Trp Tyr Gln Leu Leu Lys Ser
                   885                 890                 895
   Lys Leu Ile Ser Gln Arg Lys Phe Asp Asn Leu Thr Lys Ala Glu Arg
               900                 905                 910
   Gly Gly Leu Leu Pro Glu Asp Lys Ala Gly Phe Ile Gln Arg Gln Leu
           915                 920                 925
   Val Glu Thr Arg Gln Ile Thr Lys His Val Ala Arg Leu Leu Asp Glu
       930                 935                 940
   Lys Phe Asn Asn Lys Lys Asp Glu Asn Asn Arg Ala Val Arg Thr Val
   945                 950                 955                 960
   Lys Ile Ile Thr Leu Lys Ser Thr Leu Val Ser Gln Phe Arg Lys Asp
                   965                 970                 975
   Phe Glu Leu Tyr Lys Val Arg Glu Ile Asn Asp Phe His His Ala His
               980                 985                 990
   Asp Ala Tyr Leu Asn Ala Val Ile Ala Ser Ala Leu Leu Lys Lys Tyr
           995                 1000                1005
   Pro Lys Leu Glu Pro Glu Phe Val Tyr Gly Asp Tyr Pro Lys Tyr Asn
       1010                1015                1020
   Ser Phe Arg Glu Arg Lys Ser Ala Thr Glu Lys Val Tyr Phe Tyr Ser
   1025                1030                1035                1040
   Asn Ile Met Asn Ile Phe Lys Lys Ser Ile Ser Leu Ala Asp Gly Arg
                   1045                1050                1055
   Val Ile Glu Arg Pro Leu Ile Glu Val Asn Glu Glu Thr Gly Glu Ser
               1060                1065                1070
   Val Trp Asn Lys Glu Ser Asp Leu Ala Thr Val Arg Arg Val Leu Ser
           1075                1080                1085
   Tyr Pro Gln Val Asn Val Val Lys Lys Val Glu Glu Gln Asn His Gly
       1090                1095                1100
   Leu Asp Arg Gly Lys Pro Lys Gly Leu Phe Asn Ala Asn Leu Ser Ser
   1105                1110                1115                1120
   Lys Pro Lys Pro Asn Ser Asn Glu Asn Leu Val Gly Ala Lys Glu Tyr
                   1125                1130                1135
   Leu Asp Pro Lys Lys Tyr Gly Gly Tyr Ala Gly Ile Ser Asn Ser Phe
               1140                1145                1150
   Ala Val Leu Val Lys Gly Thr Ile Glu Lys Gly Ala Lys Lys Lys Ile
           1155                1160                1165
   Thr Asn Val Leu Glu Phe Gln Gly Ile Ser Ile Leu Asp Arg Ile Asn
       1170                1175                1180
   Tyr Arg Lys Asp Lys Leu Asn Phe Leu Leu Glu Lys Gly Tyr Lys Asp
   1185                1190                1195                1200
   Ile Glu Leu Ile Ile Glu Leu Pro Lys Tyr Ser Leu Phe Glu Leu Ser
                   1205                1210                1215
   Asp Gly Ser Arg Arg Met Leu Ala Ser Ile Leu Ser Thr Asn Asn Lys
               1220                1225                1230
   Arg Gly Glu Ile His Lys Gly Asn Gln Ile Phe Leu Ser Gln Lys Phe
           1235                1240                1245
   Val Lys Leu Leu Tyr His Ala Lys Arg Ile Ser Asn Thr Ile Asn Glu
       1250                1255                1260
   Asn His Arg Lys Tyr Val Glu Asn His Lys Lys Glu Phe Glu Glu Leu
   1265                1270                1275                1280
   Phe Tyr Tyr Ile Leu Glu Phe Asn Glu Asn Tyr Val Gly Ala Lys Lys
                   1285                1290                1295
   Asn Gly Lys Leu Leu Asn Ser Ala Phe Gln Ser Trp Gln Asn His Ser
               1300                1305                1310
   Ile Asp Glu Leu Cys Ser Ser Phe Ile Gly Pro Thr Gly Ser Glu Arg
           1315                1320                1325
   Lys Gly Leu Phe Glu Leu Thr Ser Arg Gly Ser Ala Ala Asp Phe Glu
       1330                1335                1340
   Phe Leu Gly Val Lys Ile Pro Arg Tyr Arg Asp Tyr Thr Pro Ser Ser
   1345                1350                1355                1360
   Leu Leu Lys Asp Ala Thr Leu Ile His Gln Ser Val Thr Gly Leu Tyr
                   1365                1370                1375
   Glu Thr Arg Ile Asp Leu Ala Lys Leu Gly Glu Gly
               1380                1385
<210> 120
   <211> 1121
   <212> PRT
   <213> Streptococcus thermophilus
<220>
   <223> CRISPR-associated endonuclease
<308> YP_006339747
   <309> 2013-06-11
<400> 120
   Met Ser Asp Leu Val Leu Gly Leu Asp Ile Gly Ile Gly Ser Val Gly
   1               5                   10                  15
   Val Gly Ile Leu Asn Lys Val Thr Gly Glu Ile Ile His Lys Asn Ser
               20                  25                  30
   Arg Ile Phe Pro Ala Ala Gln Ala Glu Asn Asn Leu Val Arg Arg Thr
           35                  40                  45
   Asn Arg Gln Gly Arg Arg Leu Thr Arg Arg Lys Lys His Arg Arg Val
       50                  55                  60
   Arg Leu Asn Arg Leu Phe Glu Glu Ser Gly Leu Ile Thr Asp Phe Thr
   65                  70                  75                  80
   Lys Ile Ser Ile Asn Leu Asn Pro Tyr Gln Leu Arg Val Lys Gly Leu
                   85                  90                  95
   Thr Asp Glu Leu Ser Asn Glu Glu Leu Phe Ile Ala Leu Lys Asn Met
               100                 105                 110
   Val Lys His Arg Gly Ile Ser Tyr Leu Asp Asp Ala Ser Asp Asp Gly
           115                 120                 125
   Asn Ser Ser Val Gly Asp Tyr Ala Gln Ile Val Lys Glu Asn Ser Lys
       130                 135                 140
   Gln Leu Glu Thr Lys Thr Pro Gly Gln Ile Gln Leu Glu Arg Tyr Gln
   145                 150                 155                 160
   Thr Tyr Gly Gln Leu Arg Gly Asp Phe Thr Val Glu Lys Asp Gly Lys
                   165                 170                 175
   Lys His Arg Leu Ile Asn Val Phe Pro Thr Ser Ala Tyr Arg Ser Glu
               180                 185                 190
   Ala Leu Arg Ile Leu Gln Thr Gln Gln Glu Phe Asn Pro Gln Ile Thr
           195                 200                 205
   Asp Glu Phe Val Asn Arg Tyr Leu Glu Ile Leu Thr Gly Lys Arg Lys
       210                 215                 220
   Tyr Tyr His Gly Pro Gly Asn Glu Lys Ser Arg Thr Asp Tyr Gly Arg
   225                 230                 235                 240
   Tyr Arg Thr Asn Gly Glu Thr Leu Asp Asn Ile Phe Gly Ile Leu Ile
                   245                 250                 255
   Gly Lys Cys Thr Phe Tyr Pro Glu Glu Phe Arg Ala Ala Lys Ala Ser
               260                 265                 270
   Tyr Thr Ala Gln Glu Phe Asn Leu Leu Asn Asp Leu Asn Asn Leu Thr
           275                 280                 285
   Val Pro Thr Glu Thr Lys Lys Leu Ser Lys Glu Gln Lys Asn Gln Ile
       290                 295                 300
   Ile Asn Tyr Val Lys Asn Glu Lys Ala Met Gly Pro Ala Lys Leu Phe
   305                 310                 315                 320
   Lys Tyr Ile Ala Lys Leu Leu Ser Cys Asp Val Ala Asp Ile Lys Gly
                   325                 330                 335
   Tyr Arg Ile Asp Lys Ser Gly Lys Ala Glu Ile His Thr Phe Glu Ala
               340                 345                 350
   Tyr Arg Lys Met Lys Thr Leu Glu Thr Leu Asp Ile Glu Gln Met Asp
           355                 360                 365
   Arg Glu Thr Leu Asp Lys Leu Ala Tyr Val Leu Thr Leu Asn Thr Glu
       370                 375                 380
   Arg Glu Gly Ile Gln Glu Ala Leu Glu His Glu Phe Ala Asp Gly Ser
   385                 390                 395                 400
   Phe Ser Gln Lys Gln Val Asp Glu Leu Val Gln Phe Arg Lys Ala Asn
                   405                 410                 415
   Ser Ser Ile Phe Gly Lys Gly Trp His Asn Phe Ser Val Lys Leu Met
               420                 425                 430
   Met Glu Leu Ile Pro Glu Leu Tyr Glu Thr Ser Glu Glu Gln Met Thr
           435                 440                 445
   Ile Leu Lys Arg Leu Gly Lys Gln Lys Thr Thr Ser Ser Ser Asn Lys
       450                 455                 460
   Thr Lys Tyr Ile Asp Glu Lys Leu Leu Thr Glu Glu Ile Tyr Asn Pro
   465                 470                 475                 480
   Val Val Ala Lys Ser Ile Arg Gln Ala Ile Lys Ile Val Asn Ala Ala
                   485                 490                 495
   Ile Lys Glu Tyr Gly Asp Phe Asp Asn Ile Val Ile Glu Met Ala Arg
               500                 505                 510
   Glu Thr Asn Glu Asp Asp Glu Lys Lys Ala Ile Gln Lys Ile Gln Lys
           515                 520                 525
   Ala Asn Lys Asp Glu Lys Asp Ala Ala Met Leu Lys Ala Ala Asn Gln
       530                 535                 540
   Tyr Asn Gly Lys Ala Glu Leu Pro His Ser Val Phe His Gly His Lys
   545                 550                 555                 560
   Gln Leu Ala Thr Lys Ile Arg Leu Trp His Gln Gln Gly Glu Arg Cys
                   565                 570                 575
   Leu Tyr Thr Gly Lys Thr Ile Ser Ile His Asp Leu Ile Asn Asn Pro
               580                 585                 590
   Asn Gln Phe Glu Val Asp His Ile Leu Pro Leu Ser Ile Thr Phe Asp
           595                 600                 605
   Asp Ser Leu Ala Asn Lys Val Leu Val Tyr Ala Thr Ala Asn Gln Glu
       610                 615                 620
   Lys Gly Gln Arg Thr Pro Tyr Gln Ala Leu Asp Ser Met Asp Asp Ala
   625                 630                 635                 640
   Trp Ser Phe Arg Glu Leu Lys Ala Phe Val Arg Glu Ser Lys Thr Leu
                   645                 650                 655
   Ser Asn Lys Lys Lys Glu Tyr Leu Leu Thr Glu Glu Asp Ile Ser Lys
               660                 665                 670
   Phe Asp Val Arg Lys Lys Phe Ile Glu Arg Asn Leu Val Asp Thr Arg
           675                 680                 685
   Tyr Ala Ser Arg Val Val Leu Asn Ala Leu Gln Glu His Phe Arg Ala
       690                 695                 700
   His Lys Ile Asp Thr Lys Val Ser Val Val Arg Gly Gln Phe Thr Ser
   705                 710                 715                 720
   Gln Leu Arg Arg His Trp Gly Ile Glu Lys Thr Arg Asp Thr Tyr His
                   725                 730                 735
   His His Ala Val Asp Ala Leu Ile Ile Ala Ala Ser Ser Gln Leu Asn
               740                 745                 750
   Leu Trp Lys Lys Gln Lys Asn Thr Leu Val Ser Tyr Ser Glu Asp Gln
           755                 760                 765
   Leu Leu Asp Ile Glu Thr Gly Glu Leu Ile Ser Asp Asp Glu Tyr Lys
       770                 775                 780
   Glu Ser Val Phe Lys Ala Pro Tyr Gln His Phe Val Asp Thr Leu Lys
   785                 790                 795                 800
   Ser Lys Glu Phe Glu Asp Ser Ile Leu Phe Ser Tyr Gln Val Asp Ser
                   805                 810                 815
   Lys Phe Asn Arg Lys Ile Ser Asp Ala Thr Ile Tyr Ala Thr Arg Gln
               820                 825                 830
   Ala Lys Val Gly Lys Asp Lys Lys Asp Glu Thr Tyr Val Leu Gly Lys
           835                 840                 845
   Ile Lys Asp Ile Tyr Thr Gln Asp Gly Tyr Asp Ala Phe Met Lys Ile
       850                 855                 860
   Tyr Lys Lys Asp Lys Ser Lys Phe Leu Met Tyr Arg His Asp Pro Gln
   865                 870                 875                 880
   Thr Phe Glu Lys Val Ile Glu Pro Ile Leu Glu Asn Tyr Pro Asn Lys
                   885                 890                 895
   Glu Met Asn Glu Lys Gly Lys Glu Val Pro Cys Asn Pro Phe Leu Lys
               900                 905                 910
   Tyr Lys Glu Glu His Gly Tyr Ile Arg Lys Tyr Ser Lys Lys Gly Asn
           915                 920                 925
   Asp Pro Glu Ile Lys Ser Leu Lys Tyr Tyr Asp Ser Lys Leu Gly Asn
       930                 935                 940
   His Ile Asp Ile Thr Pro Lys Asp Ser Asn Asn Lys Val Val Leu Gln
   945                 950                 955                 960
   Ser Val Ser Pro Trp Arg Ala Asp Val Tyr Phe Asn Lys Thr Thr Gly
                   965                 970                 975
   Lys Tyr Glu Ile Leu Gly Leu Lys Tyr Ala Asp Leu Gln Phe Glu Lys
               980                 985                 990
   Gly Thr Gly Thr Tyr Lys Ile Ser Gln Glu Lys Tyr Asn Asp Ile Lys
           995                 1000                1005
   Lys Lys Glu Gly Val Asp Ser Asp Ser Glu Phe Lys Phe Thr Leu Tyr
       1010                1015                1020
   Lys Asn Asp Leu Leu Leu Val Lys Asp Thr Glu Thr Lys Glu Gln Gln
   1025                1030                1035                1040
   Leu Phe Arg Phe Leu Ser Arg Thr Met Pro Lys Gln Lys His Tyr Val
                   1045                1050                1055
   Glu Leu Lys Pro Tyr Asp Lys Gln Lys Phe Glu Gly Gly Glu Ala Leu
               1060                1065                1070
   Ile Lys Val Leu Gly Asn Val Ala Asn Ser Gly Gln Cys Lys Lys Gly
           1075                1080                1085
   Leu Gly Lys Ser Asn Ile Ser Ile Tyr Lys Val Arg Thr Asp Val Leu
       1090                1095                1100
   Gly Asn Gln His Ile Ile Lys Asn Glu Gly Asp Lys Pro Lys Leu Asp
   1105                1110                1115                1120
   Phe
<210> 121
   <211> 1127
   <212> PRT
   <213> Streptococcus salivarius
<220>
   <223> CRISPR-associated endonuclease
<308> YP_006069875
   <309> 2013-06-11
<400> 121
   Met Ser Asp Leu Val Leu Gly Leu Asp Ile Gly Ile Gly Ser Val Gly
   1               5                   10                  15
   Val Gly Ile Leu Asn Lys Val Thr Gly Glu Ile Ile His Lys Asn Ser
               20                  25                  30
   Arg Ile Phe Pro Ala Ala Gln Ala Glu Asn Asn Val Glu Arg Arg Ile
           35                  40                  45
   Asn Arg Gln Gly Arg Arg Leu Thr Arg Arg Lys Lys His Arg Arg Val
       50                  55                  60
   Arg Leu Asn His Leu Phe Glu Glu Ser Gly Leu Ile Thr Asp Phe Thr
   65                  70                  75                  80
   Asn Val Ser Ile Asn Leu Asn Pro Tyr Gln Leu Arg Val Lys Gly Leu
                   85                  90                  95
   Thr Asp Glu Leu Ser Asn Glu Glu Leu Phe Ile Ala Leu Lys Asn Met
               100                 105                 110
   Val Lys His Arg Gly Ile Ser Tyr Leu Asp Asp Ala Ser Asp Asp Gly
           115                 120                 125
   Asn Ser Ser Val Gly Asp Tyr Ala Gln Ile Val Lys Glu Asn Ser Lys
       130                 135                 140
   Gln Leu Glu Thr Lys Thr Pro Gly Gln Ile Gln Leu Glu Arg Tyr Gln
   145                 150                 155                 160
   Lys Tyr Gly Gln Leu Arg Gly Asp Phe Thr Val Glu Glu Asp Gly Lys
                   165                 170                 175
   Lys His Arg Leu Ile Asn Val Phe Pro Thr Ser Ala Tyr Arg Ala Glu
               180                 185                 190
   Ala Leu Arg Ile Leu Gln Thr Gln Gln Glu Phe Asn Pro Gln Ile Thr
           195                 200                 205
   Asp Glu Phe Ile Asn Ser Tyr Leu Gln Ile Leu Thr Gly Lys Arg Lys
       210                 215                 220
   Tyr Tyr His Gly Pro Gly Asn Glu Lys Ser Arg Thr Asp Tyr Gly Arg
   225                 230                 235                 240
   Tyr Thr Thr Lys Lys Asp Ser Glu Asp Glu Tyr Ile Thr Leu Asp Asn
                   245                 250                 255
   Ile Phe Gly Ile Leu Ile Gly Lys Cys Thr Phe Tyr Pro Glu Glu Tyr
               260                 265                 270
   Arg Ala Ala Lys Ala Ser Tyr Thr Ala Gln Glu Phe Asn Leu Leu Asn
           275                 280                 285
   Asp Leu Asn Asn Leu Thr Val Pro Thr Glu Thr Lys Lys Leu Ser Glu
       290                 295                 300
   Glu Gln Lys Asn Gln Ile Ile Thr Asn Val Lys Asn Glu Lys Ala Met
   305                 310                 315                 320
   Gly Pro Ala Lys Leu Phe Lys Tyr Ile Ala Lys Leu Leu Ser Cys Asp
                   325                 330                 335
   Val Ala Asp Ile Lys Gly Tyr Arg Ile Asp Lys Ser Asp Lys Ala Glu
               340                 345                 350
   Ile His Thr Phe Glu Ala Tyr Arg Lys Met Lys Thr Leu Ala Thr Ile
           355                 360                 365
   Asp Ile Glu Gln Met Glu Arg Glu Asn Leu Asp Lys Leu Ala Tyr Val
       370                 375                 380
   Leu Thr Leu Asn Thr Glu Lys Glu Gly Ile Gln Glu Ala Leu Glu His
   385                 390                 395                 400
   Glu Phe Ala Asp Gly Thr Phe Ser Gln Glu Gln Ile Asp Glu Leu Val
                   405                 410                 415
   Gln Phe Arg Lys Ala Asn Ser Ser Ile Phe Gly Lys Gly Trp His Ser
               420                 425                 430
   Phe Ser Val Lys Leu Met Met Glu Leu Ile Pro Glu Leu Tyr Ala Thr
               435                 440                 445
   Ser Glu Glu Gln Met Thr Ile Leu Thr Arg Leu Gly Lys Gln Lys Thr
           450                 455                 460
   Thr Ser Ser Ser Asn Lys Thr Lys Tyr Ile Asp Glu Lys Gln Leu Thr
   465                 470                 475                 480
   Glu Glu Ile Tyr Asn Pro Val Ala Ala Lys Ser Val Arg Gln Ala Ile
                   485                 490                 495
   Lys Ile Val Asn Ala Ala Ile Lys Glu Tyr Gly Asp Phe Asp Asn Ile
               500                 505                 510
   Val Ile Glu Met Ala Arg Glu Thr Asn Glu Asp Asp Glu Lys Lys Ala
           515                 520                 525
   Ile Gln Lys Ile Gln Lys Ala Asn Lys Asp Glu Lys Asp Ala Ala Met
       530                 535                 540
   Leu Lys Ala Ala Asn Gln Tyr Asn Gly Lys Ala Glu Leu Pro His Ser
   545                 550                 555                 560
   Val Phe His Gly His Lys Gln Leu Ala Thr Lys Ile Arg Leu Trp His
                   565                 570                 575
   Gln Gln Gly Glu Arg Cys Leu Tyr Thr Gly Lys Thr Ile Ser Ile His
               580                 585                 590
   Asp Leu Ile Asn Asn Ser Asn Gln Phe Glu Ile Asp His Ile Leu Pro
           595                 600                 605
   Leu Ser Ile Thr Phe Asp Asp Ser Leu Ala Asn Lys Val Leu Val Tyr
       610                 615                 620
   Ala Thr Ala Asn Gln Glu Lys Gly Gln Arg Thr Pro Tyr Gln Ala Leu
   625                 630                 635                 640
   Asp Ser Met Asp Asp Ala Trp Ser Phe Arg Glu Leu Lys Ala Phe Val
                   645                 650                 655
   Arg Asp Ser Lys Ala Leu Ser Asn Lys Lys Lys Glu Tyr Leu Leu Thr
               660                 665                 670
   Glu Glu Asp Ile Ser Lys Phe Asp Val Arg Lys Lys Phe Ile Glu Arg
           675                 680                 685
   Asn Leu Val Asp Thr Arg Tyr Ala Ser Arg Val Val Leu Asn Ala Leu
       690                 695                 700
   Gln Glu His Phe Arg Val His Lys Thr Asp Thr Lys Val Ser Val Val
   705                 710                 715                 720
   Arg Gly Gln Phe Thr Ser Gln Leu Arg Arg His Trp Gly Ile Glu Lys
                   725                 730                 735
   Thr Arg Asp Thr Tyr His His His Ala Val Asp Ala Leu Ile Ile Ala
               740                 745                 750
   Ala Ser Ser Gln Leu Asn Leu Trp Lys Lys Gln Lys Asn Thr Leu Val
           755                 760                 765
   Ser Tyr Ser Glu Asp Gln Leu Leu Asp Ile Glu Thr Gly Glu Leu Ile
       770                 775                 780
   Ser Asp Asp Glu Tyr Lys Glu Ser Val Phe Lys Ala Pro Tyr Gln His
   785                 790                 795                 800
   Phe Val Asp Thr Leu Lys Ser Lys Glu Phe Glu Asp Ser Ile Leu Phe
                   805                 810                 815
   Ser Tyr Gln Val Asp Ser Lys Phe Asn Arg Lys Ile Ser Asp Ala Thr
               820                 825                 830
   Ile Tyr Ala Thr Arg Gln Ala Lys Val Gly Lys Asp Lys Lys Asp Glu
           835                 840                 845
   Thr Tyr Val Leu Gly Lys Ile Lys Asp Ile Tyr Ser Gln Thr Gly Tyr
       850                 855                 860
   Asp Ala Phe Ile Lys Ile Tyr Lys Lys Asp Lys Ser Lys Phe Leu Met
   865                 870                 875                 880
   Tyr Arg His Asp Pro Gln Thr Phe Glu Lys Val Ile Glu Pro Ile Leu
                   885                 890                 895
   Glu Asn Tyr Pro Asn Lys Glu Leu Asn Glu Lys Gly Lys Glu Val Pro
               900                 905                 910
   Cys Asn Pro Phe Leu Lys Tyr Lys Glu Asp His Gly Tyr Ile Arg Lys
           915                 920                 925
   Tyr Ser Lys Lys Gly Asn Gly Pro Glu Ile Lys Ser Leu Lys Tyr Tyr
       930                 935                 940
   Asp Ser Lys Leu Gly Asn His Ile Asp Ile Thr Pro Lys Asn Ser Asn
   945                 950                 955                 960
   Asn Lys Val Val Leu Gln Ser Val Ser Pro Trp Arg Ala Asp Val Tyr
                   965                 970                 975
   Phe Asn Lys Thr Thr Gly Lys Tyr Glu Ile Leu Gly Leu Lys Tyr Ala
               980                 985                 990
   Asp Leu Lys Phe Glu Lys Gly Thr Gly Thr Tyr Lys Ile Ser Glu Glu
           995                 1000                1005
   Lys Tyr Asn Asp Ile Lys Ile Lys Glu Gly Val Asp Ser Asp Ser Glu
       1010                1015                1020
   Phe Lys Phe Thr Leu Tyr Lys Asn Asp Leu Leu Leu Ile Lys Asp Thr
   1025                1030                1035                1040
   Glu Thr Lys Glu Gln Gln Leu Phe Arg Phe Leu Ser Arg Thr Met Pro
                   1045                1050                1055
   Asn Val Lys His Tyr Val Glu Leu Lys Pro Tyr Asp Lys Gln Lys Phe
               1060                1065                1070
   Asp Asp Asn Glu Glu Leu Ile Lys Ile Leu Gly Ile Val Ala Lys Gly
           1075                1080                1085
   Gly Gln Cys Lys Lys Gly Val Ser Lys Pro Asn Ile Ser Ile Tyr Lys
       1090                1095                1100
   Ile Arg Thr Asp Val Leu Gly Asn Gln His Ile Ile Lys Asn Glu Gly
   1105                1110                1115                1120
   Asp Lys Pro Lys Leu Asp Phe
                   1125
<210> 122
   <211> 1345
   <212> PRT
   <213> Streptococcus mutans
<220>
   <223> CRISPR-associated endonuclease
<308> YP_006250978
   <309> 2013-06-11
<400> 122
   Met Lys Lys Pro Tyr Ser Ile Gly Leu Asp Ile Gly Thr Asn Ser Val
   1               5                   10                  15
   Gly Trp Ala Val Val Thr Asp Asp Tyr Lys Val Pro Asp Lys Lys Met
               20                  25                  30
   Lys Val Leu Gly Asn Thr Asp Lys Ser His Ile Lys Lys Asn Leu Leu
           35                  40                  45
   Gly Ala Leu Leu Phe Asp Ser Gly Asn Thr Ala Glu Asp Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ser Glu Glu Met Gly Lys Val Asp Asp Ser
                   85                  90                  95
   Phe Phe His Arg Leu Glu Asp Ser Phe Leu Val Thr Glu Asp Lys Arg
               100                 105                 110
   Gly Glu Arg His Pro Ile Phe Gly Asn Leu Glu Glu Glu Val Lys Tyr
           115                 120                 125
   His Glu Asn Phe Pro Thr Ile Tyr His Leu Arg Gln Tyr Leu Ala Asp
       130                 135                 140
   Asn Pro Glu Lys Thr Asp Leu Arg Leu Val Tyr Leu Ala Leu Ala His
   145                 150                 155                 160
   Ile Ile Lys Phe Arg Gly His Phe Leu Ile Glu Gly Lys Phe Asp Thr
                   165                 170                 175
   Arg Asn Asn Asp Val Gln Arg Leu Phe Gln Glu Phe Leu Ala Val Tyr
               180                 185                 190
   Asp Asn Thr Phe Glu Asn Ser Ser Leu Gln Glu Gln Asn Val Gln Val
               195                 200                 205
   Glu Glu Ile Leu Thr Asp Lys Ile Ser Lys Ser Ala Lys Lys Asp Arg
       210                 215                 220
   Val Leu Lys Leu Phe Pro Asn Glu Lys Ser Asn Gly Arg Phe Ala Glu
   225                 230                 235                 240
   Phe Leu Lys Leu Ile Val Gly Asn Gln Ala Asp Phe Lys Lys His Phe
                   245                 250                 255
   Glu Leu Glu Glu Lys Ala Pro Leu Gln Phe Ser Lys Asp Ile Tyr Glu
               260                 265                 270
   Glu Glu Leu Glu Val Leu Leu Ala Gln Ile Gly Asp Asn Tyr Ala Glu
           275                 280                 285
   Leu Phe Leu Ser Ala Lys Lys Leu Tyr Asp Ser Ile Leu Leu Ser Gly
       290                 295                 300
   Ile Leu Thr Val Thr Asp Val Ser Thr Lys Ala Pro Leu Ser Ala Ser
   305                 310                 315                 320
   Met Ile Gln Arg Tyr Asn Glu His Gln Met Asp Leu Ala Gln Leu Lys
                   325                 330                 335
   Gln Phe Ile Arg Gln Lys Leu Ser Asp Lys Tyr Asn Glu Val Phe Ser
               340                 345                 350
   Asp Val Ser Lys Asp Gly Tyr Ala Gly Tyr Ile Asp Gly Lys Thr Asn
           355                 360                 365
   Gln Glu Ala Phe Tyr Lys Tyr Leu Lys Gly Leu Leu Asn Lys Ile Glu
       370                 375                 380
   Gly Ser Gly Tyr Phe Leu Asp Lys Ile Glu Arg Glu Asp Phe Leu Arg
   385                 390                 395                 400
   Lys Gln Arg Thr Phe Asp Asn Gly Ser Ile Pro His Gln Ile His Leu
                   405                 410                 415
   Gln Glu Met Arg Ala Ile Ile Arg Arg Gln Ala Glu Phe Tyr Pro Phe
               420                 425                 430
   Leu Ala Asp Asn Gln Asp Arg Ile Glu Lys Ile Leu Thr Phe Arg Ile
           435                 440                 445
   Pro Tyr Tyr Val Gly Pro Leu Ala Arg Gly Lys Ser Asp Phe Ala Trp
       450                 455                 460
   Leu Ser Arg Lys Ser Ala Asp Lys Ile Thr Pro Trp Asn Phe Asp Glu
   465                 470                 475                 480
   Ile Val Asp Lys Glu Ser Ser Val Glu Ala Phe Ile Asn Arg Met Thr
                   485                 490                 495
   Asn Tyr Asp Leu Tyr Leu Pro Asn Gln Lys Val Leu Pro Lys His Ser
               500                 505                 510
   Leu Leu Tyr Glu Lys Phe Thr Val Tyr Asn Glu Leu Thr Lys Val Lys
           515                 520                 525
   Tyr Lys Thr Glu Gln Gly Lys Thr Ala Phe Phe Asp Ala Asn Met Lys
       530                 535                 540
   Gln Glu Ile Phe Asp Gly Val Phe Lys Val Tyr Arg Lys Val Thr Lys
   545                 550                 555                 560
   Asp Lys Leu Met Asp Phe Leu Glu Lys Glu Phe Asp Glu Phe Arg Ile
                   565                 570                 575
   Val Asp Leu Thr Gly Leu Asp Lys Glu Asn Lys Ala Phe Asn Ala Ser
               580                 585                 590
   Tyr Gly Thr Tyr His Asp Leu Arg Lys Ile Leu Asp Lys Asp Phe Leu
           595                 600                 605
   Asp Asn Ser Lys Asn Glu Lys Ile Leu Glu Asp Ile Val Leu Thr Leu
       610                 615                 620
   Thr Leu Phe Glu Asp Arg Glu Met Ile Arg Lys Arg Leu Lys Asn Tyr
   625                 630                 635                 640
   Ser Asp Leu Leu Thr Lys Glu Gln Leu Lys Lys Leu Glu Arg Arg His
                   645                 650                 655
   Tyr Thr Gly Trp Gly Arg Leu Ser Ala Glu Leu Ile His Gly Ile Arg
               660                 665                 670
   Asn Lys Glu Ser Arg Lys Thr Ile Leu Asp Tyr Leu Ile Asp Asp Gly
           675                 680                 685
   Asn Ser Asn Arg Asn Phe Met Gln Leu Ile Asn Asp Asp Ala Leu Ser
       690                 695                 700
   Phe Lys Glu Glu Ile Ala Lys Ala Gln Val Ile Gly Glu Thr Asp Asn
   705 710 715 720
   Leu Asn Gln Val Val Ser Asp Ile Ala Gly Ser Pro Ala Ile Lys Lys
   725 730 735
   Gly Ile Leu Gln Ser Leu Lys Ile Val Asp Glu Leu Val Lys Ile Met
   740 745 750
   Gly His Gln Pro Glu Asn Ile Val Val Glu Met Ala Arg Glu Asn Gln
   755 760 765
   Phe Thr Asn Gln Gly Arg Arg Asn Ser Gln Gln Arg Leu Lys Gly Leu
   770 775 780
   Thr Asp Ser Ile Lys Glu Phe Gly Ser Gln Ile Leu Lys Glu His Pro
   785 790 795 800
   Val Glu Asn Ser Gln Leu Gln Asn Asp Arg Leu Phe Leu Tyr Tyr Leu
   805 810 815
   Gln Asn Gly Arg Asp Met Tyr Thr Gly Glu Glu Leu Asp Ile Asp Tyr
   820 825 830
   Leu Ser Gln Tyr Asp Ile Asp His Ile Ile Pro Gln Ala Phe Ile Lys
   835 840 845
   Asp Asn Ser Ile Asp Asn Arg Val Leu Thr Ser Ser Lys Glu Asn Arg
   850 855 860
   Gly Lys Ser Asp Asp Val Pro Ser Lys Asp Val Val Arg Lys Met Lys
   865 870 875 880
   Ser Tyr Trp Ser Lys Leu Leu Ser Ala Lys Leu Ile Thr Gln Arg Lys
   885 890 895
   Phe Asp Asn Leu Thr Lys Ala Glu Arg Gly Gly Leu Thr Asp Asp Asp
   900 905 910
   Lys Ala Gly Phe Ile Lys Arg Gln Leu Val Glu Thr Arg Gln Ile Thr
   915 920 925
   Lys His Val Ala Arg Ile Leu Asp Glu Arg Phe Tyr Thr Glu Thr Asp
   930 935 940
   Glu Asn Asn Lys Lys Ile Arg Gln Val Lys Ile Val Thr Leu Lys Ser
   945 950 955 960
   Asn Leu Val Ser Asn Phe Arg Lys Glu Phe Glu Leu Tyr Lys Val Arg
   965 970 975
   Glu Ile Asn Asp Tyr His His Ala His Asp Ala Tyr Leu Asn Ala Val
   980 985 990
   Ile Gly Lys Ala Leu Leu Gly Val Tyr Pro Gln Leu Glu Pro Glu Phe
   995 1000 1005
   Val Tyr Gly Asp Tyr Pro His Phe His Gly His Lys Glu Asn Lys Ala
   1010 1015 1020
   Thr Ala Lys Lys Phe Phe Tyr Ser Asn Ile Met Asn Phe Phe Lys Lys
   1025 1030 1035 1040
   Asp Asp Val Arg Thr Asp Lys Asn Gly Glu Ile Ile Trp Lys Lys Asp
   1045 1050 1055
   Glu His Ile Ser Asn Ile Lys Lys Val Leu Ser Tyr Pro Gln Val Asn
   1060 1065 1070
   Ile Val Lys Lys Val Glu Glu Gln Thr Gly Gly Phe Ser Lys Glu Ser
   1075 1080 1085
   Ile Leu Pro Lys Gly Asn Ser Asp Lys Leu Ile Pro Arg Lys Thr Lys
   1090 1095 1100
   Lys Phe Tyr Trp Asp Thr Lys Lys Tyr Gly Gly Phe Asp Ser Pro Ile
   1105 1110 1115 1120
   Val Ala Tyr Ser Ile Leu Val Ile Ala Asp Ile Glu Lys Gly Lys Ser
   1125 1130 1135
   Lys Lys Leu Lys Thr Val Lys Ala Leu Val Gly Val Thr Ile Met Glu
   1140 1145 1150
   Lys Met Thr Phe Glu Arg Asp Pro Val Ala Phe Leu Glu Arg Lys Gly
   1155 1160 1165
   Tyr Arg Asn Val Gln Glu Glu Asn Ile Ile Lys Leu Pro Lys Tyr Ser
   1170 1175 1180
   Leu Phe Lys Leu Glu Asn Gly Arg Lys Arg Leu Leu Ala Ser Ala Arg
   1185 1190 1195 1200
   Glu Leu Gln Lys Gly Asn Glu Ile Val Leu Pro Asn His Leu Gly Thr
                   1205                1210                1215
   Leu Leu Tyr His Ala Lys Asn Ile His Lys Val Asp Glu Pro Lys His
               1220                1225                1230
   Leu Asp Tyr Val Asp Lys His Lys Asp Glu Phe Lys Glu Leu Leu Asp
           1235                1240                1245
   Val Val Ser Asn Phe Ser Lys Lys Tyr Thr Leu Ala Glu Gly Asn Leu
       1250                1255                1260
   Glu Lys Ile Lys Glu Leu Tyr Ala Gln Asn Asn Gly Glu Asp Leu Lys
   1265                1270                1275                1280
   Glu Leu Ala Ser Ser Phe Ile Asn Leu Leu Thr Phe Thr Ala Ile Gly
                   1285                1290                1295
   Ala Pro Ala Thr Phe Lys Phe Phe Asp Lys Asn Ile Asp Arg Lys Arg
               1300                1305                1310
   Tyr Thr Ser Thr Thr Glu Ile Leu Asn Ala Thr Leu Ile His Gln Ser
           1315                1320                1325
   Ile Thr Gly Leu Tyr Glu Thr Arg Ile Asp Leu Ser Lys Leu Gly Gly
       1330                1335                1340
   Asp
   1345
<210> 123
   <211> 1400
   <212> PRT
   <213> Riemerella anatipestifer
<220>
   <223> CRISPR-associated endonuclease
<308> YP_006017889
   <309> 2013-06-11
<400> 123
   Met Lys Thr Ile Leu Gly Leu Asp Leu Gly Thr Asn Ser Ile Gly Trp
    1               5                  10                  15
   Ala Leu Val Lys Glu Thr Glu Asn Ser Asn Glu Lys Ser Glu Ile Ile
               20                  25                  30
   Lys Leu Gly Val Arg Val Asn Pro Leu Thr Val Asp Glu Lys Thr Asn
           35                  40                  45
   Phe Glu Ala Gly Arg Pro Leu Ser Thr Asn Ala Asp Arg Thr Ala Lys
       50                  55                  60
   Arg Ser Ala Arg Arg Asn Leu Gln Arg Tyr Lys Leu Arg Arg Lys Asn
   65                  70                  75                  80
   Leu Ile Asp Leu Leu Ile Lys His Arg Leu Ile Asp Lys Asp Thr Pro
                   85                  90                  95
   Leu Thr Glu Ile Gly Lys Asn Thr Thr His Gln Thr Leu Glu Leu Arg
               100                 105                 110
   Ala Lys Ala Ala Arg Glu Arg Ile Glu Leu Glu Asp Leu Ala Arg Val
           115                 120                 125
   Phe Leu Ala Ile Asn Lys Lys Arg Gly Tyr Arg Ser Ser Arg Lys Val
       130                 135                 140
   Asn Asn Glu Glu Glu Gly Gln Val Val Asp Gly Met Ala Val Ala Lys
   145                 150                 155                 160
   Lys Leu Tyr Asp Glu Asn Leu Thr Pro Gly Gln Tyr Ala Tyr Glu Leu
                   165                 170                 175
   Leu Ser Lys Gly Lys Lys Tyr Val Pro Asp Phe Tyr Arg Ser Asp Leu
               180                 185                 190
   Gln Ala Glu Phe Asp Ser Ile Trp Glu Tyr Gln Lys Gln Phe Tyr Ala
           195                 200                 205
   Asp Ile Leu Asp Asp Glu Leu Tyr Asn Ala Leu Lys Gly Gln Gly Gln
       210                 215                 220
   Gln Asn Ser Arg Lys Arg Phe Leu Ala Ile Lys Gly Val Tyr Thr Ala
   225                 230                 235                 240
   Glu Asn Lys Gly Lys Arg Asp Glu Val Lys Leu Gln His Tyr Lys Trp
   245 250 255
   Arg Ser Glu Ala Ile Thr Gln Lys Leu Ser Ile Glu Glu Val Ala Tyr
   260 265 270
   Val Leu Val Glu Ile Asn Asn Asp Leu Asn Lys Ser Ser Gly Tyr Leu
   275 280 285
   Gly Ala Ile Ser Asp Arg Ser Lys Glu Leu Tyr Phe Asn Lys Glu Thr
   290 295 300
   Val Gly Glu Asn Leu Trp Lys Gln Ile Gln Lys Asn Pro His Thr Ser
   305 310 315 320
   Leu Lys Asn Gln Val Phe Tyr Arg Gln Asp Tyr Leu Asp Glu Phe Glu
   325 330 335
   Gln Ile Trp Glu Thr Gln Ala Gln Phe His Pro Gln Leu Thr Leu Ala
   340 345 350
   Leu Lys Glu Gln Ile Arg Asp Val Val Ile Phe Tyr Gln Arg Lys Leu
   355 360 365
   Lys Ser Gln Lys Gly Leu Leu Ser Phe Cys Gln Phe Glu Ser Trp Glu
   370 375 380
   Ile Glu Arg Lys Asp Glu Asn Gly Asn Val Ile Leu Asn Lys Thr Thr
   385 390 395 400
   Gln Leu Pro Lys Arg Gln Thr Val Gly Arg Arg Val Ala Pro Lys Ser
   405 410 415
   Ser Pro Leu Phe Gln Glu Phe Lys Ile Trp Gln Asn Ile Asn Asn Leu
   420 425 430
   Glu Ile Ala Lys Ile Ser Asp Gly Asn Ser Lys Asn Lys Lys Glu Thr
   435 440 445
   Glu Ala Leu Ser Asp Asp Glu Arg Lys Leu Leu Phe Glu Glu Leu Asn
   450 455 460
   Leu Arg Gly Asn Leu Ser Gln Lys Glu Val Leu Gln Ile Leu Gly Leu
   465 470 475 480
   Lys Asp Lys Glu Tyr Lys Thr Asn Phe Pro Glu Gly Leu Glu Gly Asn
   485 490 495
   Arg Thr Asn Ala Ala Leu Phe Asn Ile Tyr Gln Gln Ile Ala Glu Asn
   500 505 510
   Glu Gly Tyr Gly Asp Trp Thr Lys Lys Ser Ala Gln Glu Ile Lys Glu
   515 520 525
   Glu Leu Lys Ala Val Phe Pro Gln Ile Gly Ile Gln Ala Asp Ile Leu
   530 535 540
   Asp Phe Asn Ala Glu Leu Asp Gly Lys Glu Phe Glu Asn Gln Ala Ser
   545 550 555 560
   Tyr Gln Leu Trp His Leu Leu Tyr Ser Ala Glu Glu Asp Asp Lys Ile
   565 570 575
   Asn Glu Glu Asp Gln Ile Ile Tyr Gly Asn Ser Ala Val Ser Leu Lys
   580 585 590
   Lys Lys Leu Cys Glu Lys Phe Gly Phe Thr Pro Glu Tyr Ala Lys Trp
   595 600 605
   Ile Ala Asn Val Ser Leu Gln Asp Asp Tyr Gly Asn Leu Ser Thr Lys
   610 615 620
   Ala Met Arg Lys Ile Ile Pro Tyr Leu Ile Asp Gly Asn Asp Tyr Ser
   625 630 635 640
   Glu Ala Cys Ala Leu Ala Gly Tyr Asn His Ser Asn Ser Leu Thr Lys
   645 650 655
   Glu Glu Asn Asp Asn Arg Glu Arg Leu Asn Lys Leu Glu Leu Leu Pro
   660 665 670
   Lys Asn Ser Leu Arg Asn Pro Val Val Glu Lys Ile Leu Asn Gln Met
   675 680 685
   Val Asn Val Val Asn Gln Val Ile Glu Thr Tyr Gly Lys Pro Asp Glu
   690 695 700
   Val Arg Ile Glu Leu Ala Arg Glu Leu Lys Lys Ser Ala Glu Glu Arg
   705 710 715 720
   Ala Glu Met Thr Lys Gly Ile Asn Glu Ala Thr Arg Arg Asn Glu Asp
   725 730 735
   Ile Lys Lys Leu Ile Thr Lys Asn Phe Gly Ile Pro Asn Pro Thr Lys
   740 745 750
   Asn Asp Val Val Arg Tyr Arg Leu Trp Glu Glu Leu Ala Pro Leu Ala
   755 760 765
   Tyr Lys Asp Val Phe Thr Gly Arg Gln Ile Lys Lys Glu Asp Leu Phe
   770 775 780
   Ser Ser Lys Ile Asp Ile Glu His Ile Ile Pro Lys Ala Leu Leu Phe
   785 790 795 800
   Asp Asp Ser Phe Ser Asn Lys Thr Leu Ala Phe Arg Glu Thr Asn Leu
   805 810 815
   Lys Lys Ala Asp Arg Thr Ala Tyr Gly Phe Ile Glu Ser Asp Tyr Asn
   820 825 830
   Ala Thr Leu Asp Asp Tyr Ile Gln Arg Val Glu Thr Leu Tyr Asn Asn
   835 840 845
   Ala Lys Gly Thr Ile Ser Lys Gly Lys Arg Asn Lys Leu Leu Met Ala
   850 855 860
   Gln Lys Asn Leu Pro Asp Gly Phe Ile Glu Arg Asp Leu Arg Asn Ser
   865 870 875 880
   Gln Tyr Ile Ala Lys Lys Ala Lys Ser Met Ile Glu Lys Val Phe Asn
   885 890 895
   Asp Val Asn Val Thr Ser Gly Ser Ile Thr Asp Arg Leu Arg Glu Asp
   900 905 910
   Trp Asp Leu Ile Asn Val Met Lys Glu Leu Asn Phe Pro Lys Tyr Lys
   915 920 925
   Ala Leu Gly Leu Thr Glu Ile Glu Glu Arg Tyr Asp Ile Gly Gln Glu
   930 935 940
   Lys Thr Lys Lys Val Glu Val Ile Thr Asp Trp Thr Lys Arg Asn Asp
   945 950 955 960
   His Arg His His Ala Met Asp Ala Leu Thr Val Ala Phe Thr Thr Lys
   965 970 975
   Ser His Val Gln Tyr Leu Asn Asn Leu Asn Thr Ile His Ser Leu Lys
   980 985 990
   Gly Asp Glu Val Asp Leu Glu Leu Ser Lys Leu Tyr Gly Leu Lys Asn
   995 1000 1005
   Thr Ile Thr Glu Val Val Asn Lys Lys Arg Lys Phe Ile Pro Pro Met
   1010 1015 1020
   Pro Asn Phe Arg Glu Glu Ala Lys Lys His Ile Glu Thr Ile Leu Val
   1025 1030 1035 1040
   Ser Ile Lys Asn Lys Asn Lys Val Tyr Thr Lys Asn Ile Asn Lys Thr
   1045 1050 1055
   Lys Lys Glu Ser Gly Tyr His Thr Lys Glu Gln Leu Thr Pro Arg Gly
   1060 1065 1070
   Gln Leu His Lys Glu Thr Val Tyr Gly Lys Ser Lys Arg Pro Met Asn
   1075 1080 1085
   Lys Pro Thr Lys Ile Asn Lys Asn Phe Ser Leu Glu Gln Ala Gln Leu
   1090 1095 1100
   Ile Ile Asn Val Gln Glu Lys Glu Leu Val Leu Arg His Leu Ala Gln
   1105 1110 1115 1120
   Phe Asp Asn Asn Pro Ala Val Ala Phe Asp Thr Lys Thr Leu Lys Lys
   1125 1130 1135
   Met Pro Leu Leu Lys Asn Gly Glu Pro Leu Lys Glu Val Leu Cys Phe
   1140 1145 1150
   Glu Glu Ile Phe Thr Ile Arg Lys Asp Ile Thr Pro Asp Leu Lys Ile
   1155 1160 1165
   Glu Lys Val Val Asp Glu Lys Ile Lys Ala Ile Leu Glu Gln Arg Lys
   1170 1175 1180
   Lys Asp Phe Gly Gly Asn Ala Lys Glu Ala Phe Ser Asp Leu Asp Lys
   1185 1190 1195 1200
   Asn Pro Ile Trp Leu Asn Lys Glu Lys Gly Ile Cys Ile Lys Arg Val
   1205 1210 1215
   Thr Ile Thr Gly Val Ser Asn Ala Glu Ala Leu His His Gln Lys Asp
   1220 1225 1230
   His Leu Gly Arg Glu Ile Leu Asp Asp Asn Gly Asn Pro Ile Pro Ala
   1235 1240 1245
   Asp Phe Val Ser Thr Gly Asn Asn His His Val Ala Ile Tyr Glu Asp
       1250                1255                1260
   Glu Lys Gly Lys Leu Gln Glu Lys Val Val Ser Leu Tyr Glu Ala Val
   1265                1270                1275                1280
   Ala Arg Ala Asn Gln Gly Leu Pro Val Val Asp Lys Gly Leu Asn Glu
                   1285                1290                1295
   Asp Leu Gly Trp Lys Phe Leu Phe Thr Met Lys Gln Asn Glu Met Phe
               1300                1305                1310
   Val Phe Pro Asn Asp Asp Phe Asp Val Asn Asp Ile Asp Leu Leu Asp
           1315                1320                1325
   Glu Asn Asn Ala Lys Ile Ile Ser Gln His Leu Phe Arg Val Gln Lys
       1330                1335                1340
   Leu Ala Thr Lys Asn Tyr Met Phe Arg His His Leu Glu Thr Asn Val
   1345                1350                1355                1360
   Gln Glu Val Lys Glu Leu Asn Asp Ile Ala Tyr Lys Ser Ile Arg Ser
                   1365                1370                1375
   Thr Glu Pro Leu Lys Asn Ile Lys Lys Val Arg Ile Asn His Ile Gly
               1380                1385                1390
   Lys Ile Val Gln Val Gly Glu Tyr
           1395                1400
<210> 124
   <211> 1121
   <212> PRT
   <213> Streptococcus thermophilus
<220>
   <223> CRISPR-associated endonuclease
<308> YP_006002222
   <309> 2013-06-11
<400> 124
   Met Ser Asp Leu Val Leu Gly Leu Asp Ile Gly Ile Gly Ser Val Gly
    1               5                  10                  15
   Val Gly Ile Leu Asn Lys Val Thr Gly Glu Ile Ile His Lys Asn Ser
               20                  25                  30
   Arg Ile Phe Pro Ala Ala Gln Ala Glu Asn Asn Leu Val Arg Arg Thr
           35                  40                  45
   Asn Arg Gln Gly Arg Arg Leu Thr Arg Arg Lys Lys His Arg Ile Val
       50                  55                  60
   Arg Leu Asn Arg Leu Phe Glu Glu Ser Gly Leu Ile Thr Asp Phe Thr
   65                  70                  75                  80
   Lys Ile Ser Ile Asn Leu Asn Pro Tyr Gln Leu Arg Val Lys Gly Leu
                   85                  90                  95
   Thr Asp Glu Leu Ser Asn Glu Glu Leu Phe Ile Ala Leu Lys Asn Met
               100                 105                 110
   Val Lys His Arg Gly Ile Ser Tyr Leu Asp Asp Ala Ser Asp Asp Gly
           115                 120                 125
   Asn Ser Ser Val Gly Asp Tyr Ala Gln Ile Val Lys Glu Asn Ser Lys
       130                 135                 140
   Gln Leu Glu Thr Lys Thr Pro Gly Gln Ile Gln Leu Glu Arg Tyr Gln
   145                 150                 155                 160
   Thr Tyr Gly Gln Leu Arg Gly Asp Phe Thr Val Glu Lys Asp Gly Lys
                   165                 170                 175
   Lys His Arg Leu Ile Asn Val Phe Pro Thr Ser Ala Tyr Arg Ser Glu
               180                 185                 190
   Ala Leu Arg Ile Leu Gln Thr Gln Gln Glu Phe Asn Pro Gln Ile Thr
           195                 200                 205
   Asp Glu Phe Ile Asn Arg Tyr Leu Glu Ile Leu Thr Gly Lys Arg Lys
       210                 215                 220
   Tyr Tyr His Gly Pro Gly Asn Glu Lys Ser Arg Thr Asp Tyr Gly Arg
   225                 230                 235                 240
   Tyr Arg Thr Ser Gly Glu Thr Leu Asp Asn Ile Phe Gly Ile Leu Ile
                   245                 250                 255
   Gly Lys Cys Thr Phe Tyr Pro Asp Glu Phe Arg Ala Ala Lys Ala Ser
               260                 265                 270
   Tyr Thr Ala Gln Glu Phe Asn Leu Leu Asn Asp Leu Asn Asn Leu Thr
           275                 280                 285
   Val Pro Thr Glu Thr Lys Lys Leu Ser Lys Glu Gln Lys Asn Gln Ile
       290                 295                 300
   Ile Asn Tyr Val Lys Asn Glu Lys Ala Met Gly Pro Ala Lys Leu Phe
   305                 310                 315                 320
   Lys Tyr Ile Ala Lys Leu Leu Ser Cys Asp Val Ala Asp Ile Lys Gly
                   325                 330                 335
   Tyr Arg Ile Asp Lys Ser Gly Lys Ala Glu Ile His Thr Phe Glu Ala
               340                 345                 350
   Tyr Arg Lys Met Lys Thr Leu Glu Thr Leu Asp Ile Glu Gln Met Asp
           355                 360                 365
   Arg Glu Thr Leu Asp Lys Leu Ala Tyr Val Leu Thr Leu Asn Thr Glu
       370                 375                 380
   Arg Glu Gly Ile Gln Glu Ala Leu Glu His Glu Phe Ala Asp Gly Ser
   385                 390                 395                 400
   Phe Ser Gln Lys Gln Val Asp Glu Leu Val Gln Phe Arg Lys Ala Asn
                   405                 410                 415
   Ser Ser Ile Phe Gly Lys Gly Trp His Asn Phe Ser Val Lys Leu Met
               420                 425                 430
   Met Glu Leu Ile Pro Glu Leu Tyr Glu Thr Ser Glu Glu Gln Met Thr
           435                 440                 445
   Ile Leu Thr Arg Leu Gly Lys Gln Lys Thr Thr Ser Ser Ser Asn Lys
       450                 455                 460
   Thr Lys Tyr Ile Asp Glu Lys Leu Leu Thr Glu Glu Ile Tyr Asn Pro
   465                 470                 475                 480
   Val Val Ala Lys Ser Val Arg Gln Ala Ile Lys Ile Val Asn Ala Ala
                   485                 490                 495
   Ile Lys Glu Tyr Gly Asp Phe Asp Asn Ile Val Ile Glu Met Ala Arg
               500                 505                 510
   Glu Thr Asn Glu Asp Asp Glu Lys Lys Ala Ile Gln Lys Ile Gln Lys
           515                 520                 525
   Ala Asn Lys Asp Glu Lys Asp Ala Ala Met Leu Lys Ala Ala Asn Gln
       530                 535                 540
   Tyr Asn Gly Lys Ala Glu Leu Pro His Ser Val Phe His Gly His Lys
   545                 550                 555                 560
   Gln Leu Ala Thr Lys Ile Arg Leu Trp His Gln Gln Gly Glu Arg Cys
                   565                 570                 575
   Leu Tyr Thr Gly Lys Thr Ile Ser Ile His Asp Leu Ile Asn Asn Ser
               580                 585                 590
   Asn Gln Phe Glu Val Asp His Ile Leu Pro Leu Ser Ile Thr Phe Asp
           595                 600                 605
   Asp Ser Leu Ala Asn Lys Val Leu Val Tyr Ala Thr Ala Asn Gln Glu
       610                 615                 620
   Lys Gly Gln Arg Thr Pro Tyr Gln Ala Leu Asp Ser Met Asp Asp Ala
   625                 630                 635                 640
   Trp Ser Phe Arg Glu Leu Lys Ala Phe Val Arg Glu Ser Lys Thr Leu
                   645                 650                 655
   Ser Asn Lys Lys Lys Glu Tyr Leu Leu Thr Glu Glu Asp Ile Ser Lys
               660                 665                 670
   Phe Asp Val Arg Lys Lys Phe Ile Glu Arg Asn Leu Val Asp Thr Arg
           675                 680                 685
   Tyr Ala Ser Arg Val Val Leu Asn Ala Leu Gln Glu His Phe Arg Ala
       690                 695                 700
   His Lys Ile Asp Thr Lys Val Ser Val Val Arg Gly Gln Phe Thr Ser
   705                 710                 715                 720
   Gln Leu Arg Arg His Trp Gly Ile Glu Lys Thr Arg Asp Thr Tyr His
                   725                 730                 735
   His His Ala Val Asp Ala Leu Ile Ile Ala Ala Ser Ser Gln Leu Asn
                   740                 745                 750
   Leu Trp Lys Lys Gln Lys Asn Thr Leu Val Ser Tyr Ser Glu Asp Gln
               755                 760                 765
   Leu Leu Asp Ile Glu Thr Gly Glu Leu Ile Ser Asp Asp Glu Tyr Lys
       770                 775                 780
   Glu Ser Val Phe Lys Ala Pro Tyr Gln His Phe Val Asp Thr Leu Lys
   785                 790                 795                 800
   Ser Lys Glu Phe Glu Asp Ser Ile Leu Phe Ser Tyr Gln Val Asp Ser
                       805                 810                 815
   Lys Phe Asn Arg Lys Ile Ser Asp Ala Thr Ile Tyr Ala Thr Arg Gln
                   820                 825                 830
   Ala Lys Val Gly Lys Asp Lys Ala Asp Glu Thr Tyr Val Leu Gly Lys
               835                 840                 845
   Ile Lys Asp Ile Tyr Thr Gln Asp Gly Tyr Asp Ala Phe Met Lys Ile
       850                 855                 860
   Tyr Lys Lys Asp Lys Ser Lys Phe Leu Met Tyr Arg His Asp Pro Gln
   865                 870                 875                 880
   Thr Phe Glu Lys Val Ile Glu Pro Ile Leu Glu Asn Tyr Pro Asn Lys
                       885                 890                 895
   Gln Met Asn Glu Lys Gly Lys Glu Val Pro Cys Asn Pro Phe Leu Lys
                   900                 905                 910
   Tyr Lys Glu Glu His Gly Tyr Ile Arg Lys Tyr Ser Lys Lys Gly Asn
               915                 920                 925
   Gly Pro Glu Ile Lys Ser Leu Lys Tyr Tyr Asp Ser Lys Leu Gly Asn
       930                 935                 940
   His Ile Asp Ile Thr Pro Lys Asp Ser Asn Asn Lys Val Val Leu Gln
   945                 950                 955                 960
   Ser Val Ser Pro Trp Arg Ala Asp Ile Tyr Phe Asn Asn Thr Thr Gly
                       965                 970                 975
   Lys Tyr Glu Ile Leu Gly Leu Lys Tyr Ala Asp Leu Gln Phe Glu Lys
                   980                 985                 990
   Gly Thr Gly Thr Tyr Lys Ile Ser Gln Glu Lys Tyr Asn Asp Ile Lys
               995                 1000                1005
   Lys Lys Glu Gly Val Asp Ser Asp Ser Glu Phe Lys Phe Thr Leu Tyr
       1010                1015                1020
   Lys Asn Asp Leu Leu Leu Val Lys Glu Thr Glu Thr Lys Glu Gln Gln
   1025                1030                1035                1040
   Leu Phe Arg Phe Leu Ser Arg Thr Met Pro Lys Gln Lys His Tyr Val
                       1045                1050                1055
   Glu Leu Lys Pro Tyr Asp Lys Gln Lys Phe Glu Gly Gly Glu Ala Leu
                   1060                1065                1070
   Ile Lys Val Leu Gly Asn Val Ala Asn Gly Gly Gln Cys Ile Lys Gly
               1075                1080                1085
   Leu Gly Lys Ser Asn Ile Ser Ile Tyr Lys Val Arg Thr Asp Val Leu
       1090                1095                1100
   Gly Asn Gln His Ile Ile Lys Asn Glu Gly Asp Lys Pro Lys Leu Asp
   1105                1110                1115                1120
   Phe
<210> 125
   <211> 1145
   <212> PRT
   <213> Riemerella anatipestifer
<220>
   <223> CRISPR-associated endonuclease
<308> YP_005394506
   <309> 2013-07-06
<400> 125
   Met Lys Thr Ile Leu Gly Leu Asp Leu Gly Thr Asn Ser Ile Gly Trp
   1               5                   10                  15
   Ala Leu Val Lys Glu Thr Glu Asn Ser Asn Glu Lys Ser Glu Ile Ile
               20                  25                  30
   Lys Leu Gly Val Arg Val Asn Pro Leu Thr Val Asp Glu Lys Thr Asn
           35                  40                  45
   Phe Glu Ala Gly Arg Pro Leu Ser Thr Asn Ala Asp Arg Thr Ala Lys
       50                  55                  60
   Arg Ser Ala Arg Arg Asn Leu Gln Arg Tyr Lys Leu Arg Arg Lys Asn
   65                  70                  75                  80
   Leu Ile Asp Leu Leu Ile Lys His Arg Leu Ile Asp Lys Asp Thr Pro
                   85                  90                  95
   Leu Thr Glu Ile Gly Lys Asn Thr Thr His Gln Thr Leu Glu Leu Arg
               100                 105                 110
   Ala Lys Ala Ala Arg Glu Arg Ile Glu Leu Glu Asp Leu Ala Arg Val
           115                 120                 125
   Phe Leu Ala Ile Asn Lys Lys Arg Gly Tyr Arg Ser Ser Arg Lys Val
       130                 135                 140
   Asn Asn Glu Glu Glu Gly Gln Val Val Asp Gly Met Ala Val Ala Lys
   145                 150                 155                 160
   Lys Leu Tyr Asp Glu Asn Leu Thr Pro Gly Gln Tyr Ala Tyr Glu Leu
                   165                 170                 175
   Leu Ser Lys Gly Lys Lys Tyr Val Pro Asp Phe Tyr Arg Ser Asp Leu
               180                 185                 190
   Gln Ala Glu Phe Asp Ser Ile Trp Glu Tyr Gln Lys Gln Phe Tyr Ala
           195                 200                 205
   Asp Ile Leu Asp Asp Glu Leu Tyr Asn Ala Leu Lys Gly Gln Gly Gln
       210                 215                 220
   Gln Asn Ser Arg Lys Arg Phe Leu Ala Ile Lys Gly Val Tyr Thr Ala
   225                 230                 235                 240
   Glu Asn Lys Gly Lys Arg Asp Glu Val Lys Leu Gln His Tyr Lys Trp
                   245                 250                 255
   Arg Ser Glu Ala Ile Thr Gln Lys Leu Ser Ile Glu Glu Val Ala Tyr
               260                 265                 270
   Val Leu Val Glu Ile Asn Asn Asp Leu Asn Lys Ser Ser Gly Tyr Leu
           275                 280                 285
   Gly Ala Ile Ser Asp Arg Ser Lys Glu Leu Tyr Phe Asn Lys Glu Thr
       290                 295                 300
   Val Gly Glu Asn Leu Trp Lys Gln Ile Gln Lys Asn Pro His Thr Ser
   305                 310                 315                 320
   Leu Lys Asn Gln Val Phe Tyr Arg Gln Asp Tyr Leu Asp Glu Phe Glu
                   325                 330                 335
   Gln Ile Trp Glu Thr Gln Ala Gln Phe His Pro Gln Leu Thr Leu Ala
               340                 345                 350
   Leu Lys Glu Gln Ile Arg Asp Val Val Ile Phe Tyr Gln Arg Lys Leu
           355                 360                 365
   Lys Ser Gln Lys Gly Leu Leu Ser Phe Cys Gln Phe Glu Ser Trp Glu
       370                 375                 380
   Ile Glu Arg Lys Asp Glu Asn Gly Asn Val Ile Leu Asn Lys Thr Thr
   385                 390                 395                 400
   Gln Leu Pro Lys Arg Gln Thr Val Gly Arg Arg Val Ala Pro Lys Ser
                   405                 410                 415
   Ser Pro Leu Phe Gln Glu Phe Lys Ile Trp Gln Asn Ile Asn Asn Leu
               420                 425                 430
   Glu Ile Ala Lys Ile Ser Asp Gly Asn Ser Lys Asn Lys Lys Glu Thr
           435                 440                 445
   Glu Ala Leu Ser Asp Asp Glu Arg Lys Leu Leu Phe Glu Glu Leu Asn
       450                 455                 460
   Leu Arg Gly Asn Leu Ser Gln Lys Glu Val Leu Gln Ile Leu Gly Leu
   465                 470                 475                 480
   Lys Asp Lys Glu Tyr Lys Thr Asn Phe Pro Glu Gly Leu Glu Gly Asn
                   485                 490                 495
   Arg Thr Asn Ala Ala Leu Phe Asn Ile Tyr Gln Gln Ile Ala Glu Asn
   500 505 510
   Glu Gly Tyr Gly Asp Trp Thr Lys Lys Ser Ala Gln Glu Ile Lys Glu
   515 520 525
   Glu Leu Lys Ala Val Phe Pro Gln Ile Gly Ile Gln Ala Asp Ile Leu
   530 535 540
   Asp Phe Asn Ala Glu Leu Asp Gly Lys Glu Phe Glu Asn Gln Ala Ser
   545 550 555 560
   Tyr Gln Leu Trp His Leu Leu Tyr Ser Ala Glu Glu Asp Asp Lys Ile
   565 570 575
   Asn Glu Glu Asp Gln Ile Ile Tyr Gly Asn Ser Ala Val Ser Leu Lys
   580 585 590
   Lys Lys Leu Cys Glu Lys Phe Gly Phe Thr Pro Glu Tyr Ala Lys Trp
   595 600 605
   Ile Ala Asn Val Ser Leu Gln Asp Asp Tyr Gly Asn Leu Ser Thr Lys
   610 615 620
   Ala Met Arg Lys Ile Ile Pro Tyr Leu Ile Asp Gly Asn Asp Tyr Ser
   625 630 635 640
   Glu Ala Cys Ala Leu Ala Gly Tyr Asn His Ser Asn Ser Leu Thr Lys
   645 650 655
   Glu Glu Asn Asp Asn Arg Glu Arg Leu Asn Lys Leu Glu Leu Leu Pro
   660 665 670
   Lys Asn Ser Leu Arg Asn Pro Val Val Glu Lys Ile Leu Asn Gln Met
   675 680 685
   Val Asn Val Val Asn Gln Val Ile Glu Thr Tyr Gly Lys Pro Asp Glu
   690 695 700
   Val Arg Ile Glu Leu Ala Arg Glu Leu Lys Lys Ser Ala Glu Glu Arg
   705 710 715 720
   Ala Glu Met Thr Lys Gly Ile Asn Glu Ala Thr Arg Arg Asn Glu Asp
   725 730 735
   Ile Lys Lys Leu Ile Thr Lys Asn Phe Gly Ile Pro Asn Pro Thr Lys
   740 745 750
   Asn Asp Val Val Arg Tyr Arg Leu Trp Glu Glu Leu Ala Pro Leu Ala
   755 760 765
   Tyr Lys Asp Val Phe Thr Gly Arg Gln Ile Lys Lys Glu Asp Leu Phe
   770 775 780
   Ser Ser Lys Ile Asp Ile Glu His Ile Ile Pro Lys Ala Leu Leu Phe
   785 790 795 800
   Asp Asp Ser Phe Ser Asn Lys Thr Leu Ala Phe Arg Glu Thr Asn Leu
   805 810 815
   Lys Lys Ala Asp Arg Thr Ala Tyr Gly Phe Ile Glu Ser Asp Tyr Asn
   820 825 830
   Ala Thr Leu Asp Asp Tyr Ile Gln Arg Val Glu Thr Leu Tyr Asn Asn
   835 840 845
   Ala Lys Gly Thr Ile Ser Lys Gly Lys Arg Asn Lys Leu Leu Met Ala
   850 855 860
   Gln Lys Asn Leu Pro Asp Gly Phe Ile Glu Arg Asp Leu Arg Asn Ser
   865 870 875 880
   Gln Tyr Ile Ala Lys Lys Ala Lys Ser Met Ile Glu Lys Val Phe Asn
   885 890 895
   Asp Val Asn Val Thr Ser Gly Ser Ile Thr Asp Arg Leu Arg Glu Asp
   900 905 910
   Trp Asp Leu Ile Asn Val Met Lys Glu Leu Asn Phe Pro Lys Tyr Lys
   915 920 925
   Ala Leu Gly Leu Thr Glu Ile Glu Glu Arg Tyr Asp Ile Gly Gln Glu
   930 935 940
   Lys Thr Lys Lys Val Glu Val Ile Thr Asp Trp Thr Lys Arg Asn Asp
   945 950 955 960
   His Arg His His Ala Met Asp Ala Leu Thr Val Ala Phe Thr Thr Lys
   965 970 975
   Ser His Val Gln Tyr Leu Asn Asn Leu Asn Thr Ile His Ser Leu Lys
   980 985 990
   Gly Asp Glu Val Asp Leu Glu Leu Ser Lys Leu Tyr Gly Leu Lys Asn
           995                 1000                1005
   Thr Ile Thr Glu Val Val Asn Lys Lys Arg Lys Phe Ile Pro Pro Met
       1010                1015                1020
   Pro Asn Phe Arg Glu Glu Ala Lys Lys His Ile Glu Thr Ile Leu Val
   1025                1030                1035                1040
   Ser Ile Lys Asn Lys Asn Lys Val Tyr Thr Lys Asn Ile Asn Lys Thr
                   1045                1050                1055
   Lys Lys Glu Ser Gly Tyr His Thr Lys Glu Gln Leu Thr Pro Arg Gly
               1060                1065                1070
   Gln Leu His Lys Glu Thr Val Tyr Gly Lys Ser Lys Arg Pro Met Asn
           1075                1080                1085
   Lys Pro Thr Lys Ile Asn Lys Asn Phe Ser Leu Glu Gln Ala Gln Leu
       1090                1095                1100
   Ile Ile Asn Val Gln Glu Lys Glu Leu Val Leu Arg His Leu Ala Gln
   1105                1110                1115                1120
   Phe Asp Asn Asn Pro Ala Val Ala Phe Asp Thr Lys Thr Leu Lys Lys
                   1125                1130                1135
   Cys His Tyr Leu Lys Met Val Asn Leu
               1140                1145
<210> 126
   <211> 1375
   <212> PRT
   <213> Streptococcus infantarius
<220>
   <223> CRISPR-associated endonuclease
<308> YP_005204039
   <309> 2013-06-11
<400> 126
   Met Glu Lys Ser Tyr Ser Ile Gly Leu Asp Ile Gly Thr Asn Ser Val
   1               5                   10                  15
   Gly Trp Ala Val Ile Thr Asp Asp Tyr Lys Val Pro Ala Lys Lys Met
               20                  25                  30
   Lys Val Leu Gly Asn Thr Asp Lys Lys Tyr Ile Lys Lys Asn Leu Leu
           35                  40                  45
   Gly Ala Leu Leu Phe Asp Ser Gly Glu Thr Ala Glu Val Thr Arg Leu
       50                  55                  60
   Lys Arg Thr Ala Arg Arg Arg Tyr Thr Arg Arg Lys Asn Arg Leu Arg
   65                  70                  75                  80
   Tyr Leu Gln Glu Ile Phe Ala Lys Glu Met Thr Lys Val Asp Glu Ser
                   85                  90                  95
   Phe Phe Gln Arg Leu Glu Glu Ser Phe Leu Thr Asp Asp Asp Lys Thr
               100                 105                 110
   Phe Asp Ser His Pro Ile Phe Gly Asn Lys Ala Glu Glu Asp Ala Tyr
           115                 120                 125
   His Gln Lys Phe Pro Thr Ile Tyr His Leu Arg Lys Tyr Leu Ala Asp
       130                 135                 140
   Ser Gln Glu Lys Ala Asp Leu Arg Leu Val Tyr Leu Ala Leu Ala His
   145                 150                 155                 160
   Met Ile Lys Tyr Arg Gly His Phe Leu Ile Glu Gly Glu Leu Asn Ala
                   165                 170                 175
   Glu Asn Thr Asp Val Gln Lys Leu Phe Asn Val Phe Val Glu Thr Tyr
               180                 185                 190
   Asp Lys Ile Val Asp Glu Ser His Leu Ser Glu Ile Glu Val Asp Ala
           195                 200                 205
   Ser Ser Ile Leu Thr Glu Lys Val Ser Lys Ser Arg Arg Leu Glu Asn
       210                 215                 220
   Leu Ile Lys Gln Tyr Pro Thr Glu Lys Lys Asn Thr Leu Phe Gly Asn
   225                 230                 235                 240
   Leu Ile Ala Leu Ala Leu Gly Leu Gln Pro Asn Phe Lys Thr Asn Phe
   245 250 255
   Lys Leu Ser Glu Asp Ala Lys Leu Gln Phe Ser Lys Asp Thr Tyr Glu
   260 265 270
   Glu Asp Leu Glu Glu Leu Leu Gly Lys Val Gly Asp Asp Tyr Ala Asp
   275 280 285
   Leu Phe Ile Ser Ala Lys Asn Leu Tyr Asp Ala Ile Leu Leu Ser Gly
   290 295 300
   Ile Leu Thr Val Asp Asp Asn Ser Thr Lys Ala Pro Leu Ser Ala Ser
   305 310 315 320
   Met Ile Lys Arg Tyr Val Glu His His Glu Asp Leu Glu Lys Leu Lys
   325 330 335
   Glu Phe Ile Lys Ile Asn Lys Leu Lys Leu Tyr His Asp Ile Phe Lys
   340 345 350
   Asp Lys Thr Lys Asn Gly Tyr Ala Gly Tyr Ile Asp Asn Gly Val Lys
   355 360 365
   Gln Asp Glu Phe Tyr Lys Tyr Leu Lys Thr Ile Leu Thr Lys Ile Asp
   370 375 380
   Asp Ser Asp Tyr Phe Leu Asp Lys Ile Glu Arg Asp Asp Phe Leu Arg
   385 390 395 400
   Lys Gln Arg Thr Phe Asp Asn Gly Ser Ile Pro His Gln Ile His Leu
   405 410 415
   Gln Glu Met His Ser Ile Leu Arg Arg Gln Gly Asp Tyr Tyr Pro Phe
   420 425 430
   Leu Lys Glu Asn Gln Ala Lys Ile Glu Lys Ile Leu Thr Phe Arg Ile
   435 440 445
   Pro Tyr Tyr Val Gly Pro Leu Ala Arg Lys Asp Ser Arg Phe Ala Trp
   450 455 460
   Ala Asn Tyr His Ser Asp Glu Pro Ile Thr Pro Trp Asn Phe Asp Glu
   465 470 475 480
   Val Val Asp Lys Glu Lys Ser Ala Glu Lys Phe Ile Thr Arg Met Thr
   485 490 495
   Leu Asn Asp Leu Tyr Leu Pro Glu Glu Lys Val Leu Pro Lys His Ser
   500 505 510
   His Val Tyr Glu Thr Phe Thr Val Tyr Asn Glu Leu Thr Lys Ile Lys
   515 520 525
   Tyr Val Asn Glu Gln Gly Glu Ser Phe Phe Phe Asp Ala Asn Met Lys
   530 535 540
   Gln Glu Ile Phe Asp His Val Phe Lys Glu Asn Arg Lys Val Thr Lys
   545 550 555 560
   Ala Lys Leu Leu Ser Tyr Leu Asn Asn Glu Phe Glu Glu Phe Arg Ile
   565 570 575
   Asn Asp Leu Ile Gly Leu Asp Lys Asp Ser Lys Ser Phe Asn Ala Ser
   580 585 590
   Leu Gly Thr Tyr His Asp Leu Lys Lys Ile Leu Asp Lys Ser Phe Leu
   595 600 605
   Asp Asp Lys Thr Asn Gly Gln Ile Ile Glu Asp Ile Val Leu Thr Leu
   610 615 620
   Thr Leu Phe Glu Asp Arg Asp Met Ile His Glu Arg Leu Gln Lys Tyr
   625 630 635 640
   Ser Asp Phe Phe Thr Ser Gln Gln Leu Lys Lys Leu Glu Arg Arg His
   645 650 655
   Tyr Thr Gly Trp Gly Arg Leu Ser Tyr Lys Leu Ile Asn Gly Ile Arg
   660 665 670
   Asn Lys Glu Asn Asn Lys Thr Ile Leu Asp Phe Leu Ile Asp Asp Gly
   675 680 685
   His Ala Asn Arg Asn Phe Met Gln Leu Ile Asn Asp Glu Ser Leu Ser
   690 695 700
   Phe Lys Thr Ile Ile Gln Glu Ala Gln Val Val Gly Asp Val Asp Asp
   705 710 715 720
   Ile Glu Ala Val Val His Asp Leu Pro Gly Ser Pro Ala Ile Lys Lys
   725 730 735
   Gly Ile Leu Gln Ser Val Lys Ile Val Asp Glu Leu Val Lys Val Met
               740                 745                 750
   Gly Asp Asn Pro Asp Asn Ile Val Ile Glu Met Ala Arg Glu Asn Gln
           755                 760                 765
   Thr Thr Gly Tyr Gly Arg Asn Lys Ser Asn Gln Arg Leu Lys Arg Leu
       770                 775                 780
   Gln Asp Ser Leu Lys Glu Phe Gly Ser Asp Ile Leu Ser Lys Lys Lys
   785                 790                 795                 800
   Pro Ser Tyr Val Asp Ser Lys Val Glu Asn Ser His Leu Gln Asn Asp
                   805                 810                 815
   Arg Leu Phe Leu Tyr Tyr Ile Gln Asn Gly Lys Asp Met Tyr Thr Gly
               820                 825                 830
   Glu Glu Leu Asp Ile Asp Arg Leu Ser Asp Tyr Asp Ile Asp His Ile
           835                 840                 845
   Ile Pro Gln Ala Phe Ile Lys Asp Asn Ser Ile Asp Asn Lys Val Leu
       850                 855                 860
   Thr Ser Ser Ala Lys Asn Arg Gly Lys Ser Asp Asp Val Pro Ser Ile
   865                 870                 875                 880
   Glu Ile Val Arg Asn Arg Arg Ser Tyr Trp Tyr Lys Leu Tyr Lys Ser
                   885                 890                 895
   Gly Leu Ile Ser Lys Arg Lys Phe Asp Asn Leu Thr Lys Ala Glu Arg
               900                 905                 910
   Gly Gly Leu Thr Glu Ala Asp Lys Ala Gly Phe Ile Lys Arg Gln Leu
           915                 920                 925
   Val Glu Thr Arg Gln Ile Thr Lys His Val Ala Gln Ile Leu Asp Ala
       930                 935                 940
   Arg Phe Asn Thr Lys Arg Asp Glu Asn Asp Lys Val Ile Arg Asp Val
   945                 950                 955                 960
   Lys Val Ile Thr Leu Lys Ser Asn Leu Val Ser Gln Phe Arg Lys Glu
                   965                 970                 975
   Phe Lys Phe Tyr Lys Val Arg Glu Ile Asn Asp Tyr His His Ala His
               980                 985                 990
   Asp Ala Tyr Leu Asn Ala Val Val Gly Thr Ala Leu Leu Lys Lys Tyr
           995                 1000                1005
   Pro Lys Leu Thr Pro Glu Phe Val Tyr Gly Glu Tyr Lys Lys Tyr Asp
       1010                1015                1020
   Val Arg Lys Leu Ile Ala Lys Ser Ser Asp Asp Tyr Ser Glu Met Gly
   1025                1030                1035                1040
   Lys Ala Thr Ala Lys Tyr Phe Phe Tyr Ser Asn Leu Met Asn Phe Phe
                   1045                1050                1055
   Lys Thr Glu Val Lys Tyr Ala Asp Gly Arg Val Phe Glu Arg Pro Asp
               1060                1065                1070
   Ile Glu Thr Asn Ala Asp Gly Glu Val Val Trp Asn Lys Gln Lys Asp
           1075                1080                1085
   Phe Asp Ile Val Arg Lys Val Leu Ser Tyr Pro Gln Val Asn Ile Val
       1090                1095                1100
   Lys Lys Val Glu Ala Gln Thr Gly Gly Phe Ser Lys Glu Ser Ile Leu
   1105                1110                1115                1120
   Ser Lys Gly Asp Ser Asp Lys Leu Ile Pro Arg Lys Thr Lys Lys Val
                   1125                1130                1135
   Tyr Trp Asn Thr Lys Lys Tyr Gly Gly Phe Asp Ser Pro Thr Val Ala
               1140                1145                1150
   Tyr Ser Val Leu Val Val Ala Asp Ile Glu Lys Gly Lys Ala Lys Lys
           1155                1160                1165
   Leu Lys Thr Val Lys Glu Leu Val Gly Ile Ser Ile Met Glu Arg Ser
       1170                1175                1180
   Phe Phe Glu Glu Asn Pro Val Ser Phe Leu Glu Lys Lys Gly Tyr His
   1185                1190                1195                1200
   Asn Val Gln Glu Asp Lys Leu Ile Lys Leu Pro Lys Tyr Ser Leu Phe
                   1205                1210                1215
   Glu Phe Glu Gly Gly Arg Arg Arg Leu Leu Ala Ser Ala Thr Glu Leu
               1220                1225                1230
   Gln Lys Gly Asn Glu Val Met Leu Pro Ala His Leu Val Glu Leu Leu
           1235                1240                1245
   Tyr His Ala His Arg Ile Asp Ser Phe Asn Ser Thr Glu His Leu Lys
   1250 1255 1260
   Tyr Val Ser Glu His Lys Lys Glu Phe Glu Lys Val Leu Ser Cys Val
   1265 1270 1275 1280
   Glu Asn Phe Ser Asn Leu Tyr Val Asp Val Glu Lys Asn Leu Ser Lys
   1285 1290 1295
   Val Arg Ala Ala Ala Glu Ser Met Thr Asn Phe Ser Leu Glu Glu Ile
   1300 1305 1310
   Ser Ala Ser Phe Ile Asn Leu Leu Thr Leu Thr Ala Leu Gly Ala Pro
   1315 1320 1325
   Ala Asp Phe Asn Phe Leu Gly Glu Lys Ile Pro Arg Lys Arg Tyr Thr
   1330 1335 1340
   Ser Thr Lys Glu Cys Leu Ser Ala Thr Leu Ile His Gln Ser Val Thr
   1345 1350 1355 1360
   Gly Leu Tyr Glu Thr Arg Ile Asp Leu Ser Lys Leu Gly Glu Glu
   1365 1370 1375
<210> 127
   <211> 1132
   <212> PRT
   <213> Nitratifractor salsuginis
<220>
   <223> CRISPR-associated endonuclease
<308> YP_004168469
   <309> 2013-07-06
<400> 127
   Met Lys Lys Ile Leu Gly Val Asp Leu Gly Ile Thr Ser Phe Gly Tyr
    1               5                  10                  15
   Ala Ile Leu Gln Glu Thr Gly Lys Asp Leu Tyr Arg Cys Leu Asp Asn
               20                  25                  30
   Ser Val Val Met Arg Asn Asn Pro Tyr Asp Glu Lys Ser Gly Glu Ser
           35                  40                  45
   Ser Gln Ser Ile Arg Ser Thr Gln Lys Ser Met Arg Arg Leu Ile Glu
       50                  55                  60
   Lys Arg Lys Lys Arg Ile Arg Cys Val Ala Gln Thr Met Glu Arg Tyr
   65                  70                  75                  80
   Gly Ile Leu Asp Tyr Ser Glu Thr Met Lys Ile Asn Asp Pro Lys Asn
                   85                  90                  95
   Asn Pro Ile Lys Asn Arg Trp Gln Leu Arg Ala Val Asp Ala Trp Lys
               100                 105                 110
   Arg Pro Leu Ser Pro Gln Glu Leu Phe Ala Ile Phe Ala His Met Ala
           115                 120                 125
   Lys His Arg Gly Tyr Lys Ser Ile Ala Thr Glu Asp Leu Ile Tyr Glu
       130                 135                 140
   Leu Glu Leu Glu Leu Gly Leu Asn Asp Pro Glu Lys Glu Ser Glu Lys
   145                 150                 155                 160
   Lys Ala Asp Glu Arg Arg Gln Val Tyr Asn Ala Leu Arg His Leu Glu
                   165                 170                 175
   Glu Leu Arg Lys Lys Tyr Gly Gly Glu Thr Ile Ala Gln Thr Ile His
               180                 185                 190
   Arg Ala Val Glu Ala Gly Asp Leu Arg Ser Tyr Arg Asn His Asp Asp
           195                 200                 205
   Tyr Glu Lys Met Ile Arg Arg Glu Asp Ile Glu Glu Glu Ile Glu Lys
       210                 215                 220
   Val Leu Leu Arg Gln Ala Glu Leu Gly Ala Leu Gly Leu Pro Glu Glu
   225                 230                 235                 240
   Gln Val Ser Glu Leu Ile Asp Glu Leu Lys Ala Cys Ile Thr Asp Gln
                   245                 250                 255
   Glu Met Pro Thr Ile Asp Glu Ser Leu Phe Gly Lys Cys Thr Phe Tyr
           260                 265                 270
   Lys Asp Glu Leu Ala Ala Pro Ala Tyr Ser Tyr Leu Tyr Asp Leu Tyr
               275                 280                 285
   Arg Leu Tyr Lys Lys Leu Ala Asp Leu Asn Ile Asp Gly Tyr Glu Val
       290                 295                 300
   Thr Gln Glu Asp Arg Glu Lys Val Ile Glu Trp Val Glu Lys Lys Ile
   305                 310                 315                 320
   Ala Gln Gly Lys Asn Leu Lys Lys Ile Thr His Lys Asp Leu Arg Lys
                   325                 330                 335
   Ile Leu Gly Leu Ala Pro Glu Gln Lys Ile Phe Gly Val Glu Asp Glu
               340                 345                 350
   Arg Ile Val Lys Gly Lys Lys Glu Pro Arg Thr Phe Val Pro Phe Phe
           355                 360                 365
   Phe Leu Ala Asp Ile Ala Lys Phe Lys Glu Leu Phe Ala Ser Ile Gln
       370                 375                 380
   Lys His Pro Asp Ala Leu Gln Ile Phe Arg Glu Leu Ala Glu Ile Leu
   385                 390                 395                 400
   Gln Arg Ser Lys Thr Pro Gln Glu Ala Leu Asp Arg Leu Arg Ala Leu
                   405                 410                 415
   Met Ala Gly Lys Gly Ile Asp Thr Asp Asp Arg Glu Leu Leu Glu Leu
               420                 425                 430
   Phe Lys Asn Lys Arg Ser Gly Thr Arg Glu Leu Ser His Arg Tyr Ile
           435                 440                 445
   Leu Glu Ala Leu Pro Leu Phe Leu Glu Gly Tyr Asp Glu Lys Glu Val
       450                 455                 460
   Gln Arg Ile Leu Gly Phe Asp Asp Arg Glu Asp Tyr Ser Arg Tyr Pro
   465                 470                 475                 480
   Lys Ser Leu Arg His Leu His Leu Arg Glu Gly Asn Leu Phe Glu Lys
                   485                 490                 495
   Glu Glu Asn Pro Ile Asn Asn His Ala Val Lys Ser Leu Ala Ser Trp
               500                 505                 510
   Ala Leu Gly Leu Ile Ala Asp Leu Ser Trp Arg Tyr Gly Pro Phe Asp
           515                 520                 525
   Glu Ile Ile Leu Glu Thr Thr Arg Asp Ala Leu Pro Glu Lys Ile Arg
       530                 535                 540
   Lys Glu Ile Asp Lys Ala Met Arg Glu Arg Glu Lys Ala Leu Asp Lys
   545                 550                 555                 560
   Ile Ile Gly Lys Tyr Lys Lys Glu Phe Pro Ser Ile Asp Lys Arg Leu
                   565                 570                 575
   Ala Arg Lys Ile Gln Leu Trp Glu Arg Gln Lys Gly Leu Asp Leu Tyr
               580                 585                 590
   Ser Gly Lys Val Ile Asn Leu Ser Gln Leu Leu Asp Gly Ser Ala Asp
           595                 600                 605
   Ile Glu His Ile Val Pro Gln Ser Leu Gly Gly Leu Ser Thr Asp Tyr
       610                 615                 620
   Asn Thr Ile Val Thr Leu Lys Ser Val Asn Ala Ala Lys Gly Asn Arg
   625                 630                 635                 640
   Leu Pro Gly Asp Trp Leu Ala Gly Asn Pro Asp Tyr Arg Glu Arg Ile
                   645                 650                 655
   Gly Met Leu Ser Glu Lys Gly Leu Ile Asp Trp Lys Lys Arg Lys Asn
               660                 665                 670
   Leu Leu Ala Gln Ser Leu Asp Glu Ile Tyr Thr Glu Asn Thr His Ser
           675                 680                 685
   Lys Gly Ile Arg Ala Thr Ser Tyr Leu Glu Ala Leu Val Ala Gln Val
       690                 695                 700
   Leu Lys Arg Tyr Tyr Pro Phe Pro Asp Pro Glu Leu Arg Lys Asn Gly
   705                 710                 715                 720
   Ile Gly Val Arg Met Ile Pro Gly Lys Val Thr Ser Lys Thr Arg Ser
                   725                 730                 735
   Leu Leu Gly Ile Lys Ser Lys Ser Arg Glu Thr Asn Phe His His Ala
               740                 745                 750
   Glu Asp Ala Leu Ile Leu Ser Thr Leu Thr Arg Gly Trp Gln Asn Arg
           755                 760                 765
   Leu His Arg Met Leu Arg Asp Asn Tyr Gly Lys Ser Glu Ala Glu Leu
       770                 775                 780
   Lys Glu Leu Trp Lys Lys Tyr Met Pro His Ile Glu Gly Leu Thr Leu
   785                 790                 795                 800
   Ala Asp Tyr Ile Asp Glu Ala Phe Arg Arg Phe Met Ser Lys Gly Glu
                   805                 810                 815
   Glu Ser Leu Phe Tyr Arg Asp Met Phe Asp Thr Ile Arg Ser Ile Ser
               820                 825                 830
   Tyr Trp Val Asp Lys Lys Pro Leu Ser Ala Ser Ser His Lys Glu Thr
           835                 840                 845
   Val Tyr Ser Ser Arg His Glu Val Pro Thr Leu Arg Lys Asn Ile Leu
       850                 855                 860
   Glu Ala Phe Asp Ser Leu Asn Val Ile Lys Asp Arg His Lys Leu Thr
   865                 870                 875                 880
   Thr Glu Glu Phe Met Lys Arg Tyr Asp Lys Glu Ile Arg Gln Lys Leu
                   885                 890                 895
   Trp Leu His Arg Ile Gly Asn Thr Asn Asp Glu Ser Tyr Arg Ala Val
               900                 905                 910
   Glu Glu Arg Ala Thr Gln Ile Ala Gln Ile Leu Thr Arg Tyr Gln Leu
           915                 920                 925
   Met Asp Ala Gln Asn Asp Lys Glu Ile Asp Glu Lys Phe Gln Gln Ala
       930                 935                 940
   Leu Lys Glu Leu Ile Thr Ser Pro Ile Glu Val Thr Gly Lys Leu Leu
   945                 950                 955                 960
   Arg Lys Met Arg Phe Val Tyr Asp Lys Leu Asn Ala Met Gln Ile Asp
                   965                 970                 975
   Arg Gly Leu Val Glu Thr Asp Lys Asn Met Leu Gly Ile His Ile Ser
               980                 985                 990
   Lys Gly Pro Asn Glu Lys Leu Ile Phe Arg Arg Met Asp Val Asn Asn
           995                 1000                1005
   Ala His Glu Leu Gln Lys Glu Arg Ser Gly Ile Leu Cys Tyr Leu Asn
       1010                1015                1020
   Glu Met Leu Phe Ile Phe Asn Lys Lys Gly Leu Ile His Tyr Gly Cys
   1025                1030                1035                1040
   Leu Arg Ser Tyr Leu Glu Lys Gly Gln Gly Ser Lys Tyr Ile Ala Leu
                   1045                1050                1055
   Phe Asn Pro Arg Phe Pro Ala Asn Pro Lys Ala Gln Pro Ser Lys Phe
               1060                1065                1070
   Thr Ser Asp Ser Lys Ile Lys Gln Val Gly Ile Gly Ser Ala Thr Gly
           1075                1080                1085
   Ile Ile Lys Ala His Leu Asp Leu Asp Gly His Val Arg Ser Tyr Glu
       1090                1095                1100
   Val Phe Gly Thr Leu Pro Glu Gly Ser Ile Glu Trp Phe Lys Glu Glu
   1105                1110                1115                1120
   Ser Gly Tyr Gly Arg Val Glu Asp Asp Pro His His
                   1125                1130
<210> 128
   <211> 1082
   <212> PRT
   <213> Neisseria lactamica
<220>
   <223> CRISPR-associated endonuclease
<308> YP_004049328
   <309> 2013-07-06
<400> 128
   Met Ala Ala Phe Lys Pro Asn Pro Met Asn Tyr Ile Leu Gly Leu Asp
   1               5                   10                  15
   Ile Gly Ile Ala Ser Val Gly Trp Ala Met Val Glu Ile Asp Lys Glu
               20                  25                  30
   Glu Asn Pro Ile Arg Leu Ile Asp Leu Gly Val Arg Val Phe Glu Arg
           35                  40                  45
   Ala Glu Val Pro Lys Thr Gly Asp Ser Leu Ala Met Ala Arg Arg Leu
       50                  55                  60
   Ala Arg Ser Val Arg Arg Leu Thr Arg Arg Arg Ala His Arg Leu Leu
   65                  70                  75                  80
   Arg Ala Arg Arg Leu Leu Lys Arg Glu Gly Val Leu Gln Asp Ala Asp
                   85                  90                  95
   Phe Asp Glu Asn Gly Leu Val Lys Ser Leu Pro Asn Thr Pro Trp Gln
               100                 105                 110
   Leu Arg Ala Ala Ala Leu Asp Arg Lys Leu Thr Cys Leu Glu Trp Ser
           115                 120                 125
   Ala Val Leu Leu His Leu Ile Lys His Arg Gly Tyr Leu Ser Gln Arg
       130                 135                 140
   Lys Asn Glu Gly Glu Thr Ala Asp Lys Glu Leu Gly Ala Leu Leu Lys
   145                 150                 155                 160
   Gly Val Ala Asp Asn Ala His Ala Leu Gln Thr Gly Asp Phe Arg Thr
                   165                 170                 175
   Pro Ala Glu Leu Ala Leu Asn Lys Phe Glu Lys Glu Ser Gly His Ile
               180                 185                 190
   Arg Asn Gln Arg Gly Asp Tyr Ser His Thr Phe Ser Arg Lys Asp Leu
           195                 200                 205
   Gln Ala Glu Leu Asn Leu Leu Phe Glu Lys Gln Lys Glu Phe Ser Asn
       210                 215                 220
   Pro His Val Ser Asp Ser Leu Lys Glu Gly Ile Glu Thr Leu Leu Met
   225                 230                 235                 240
   Ala Gln Arg Pro Ala Leu Ser Gly Asp Ala Val Gln Lys Met Leu Gly
                   245                 250                 255
   His Cys Thr Phe Glu Pro Ala Glu Pro Lys Ala Ala Lys Asn Thr Tyr
               260                 265                 270
   Thr Ala Glu Arg Phe Ile Trp Leu Thr Lys Leu Asn Asn Leu Arg Ile
           275                 280                 285
   Leu Glu Gln Gly Ser Glu Arg Pro Leu Thr Asp Thr Glu Arg Ala Thr
       290                 295                 300
   Leu Met Asp Glu Pro Tyr Arg Lys Ser Lys Leu Thr Tyr Ala Gln Ala
   305                 310                 315                 320
   Arg Lys Leu Leu Gly Leu Glu Asp Thr Ala Phe Phe Lys Gly Leu Arg
                   325                 330                 335
   Tyr Gly Lys Asp Asn Ala Glu Ala Pro Thr Leu Met Glu Met Lys Ala
               340                 345                 350
   Tyr His Ala Ile Ser Arg Ala Leu Glu Lys Glu Gly Leu Lys Asp Lys
           355                 360                 365
   Lys Ser Pro Leu Asn Leu Ser Thr Glu Leu Gln Asp Glu Ile Gly Thr
       370                 375                 380
   Ala Phe Ser Leu Phe Lys Thr Asp Lys Asp Ile Thr Gly Arg Leu Lys
   385                 390                 395                 400
   Asp Arg Val Gln Pro Glu Ile Leu Glu Ala Leu Leu Lys His Ile Ser
                   405                 410                 415
   Phe Asp Lys Phe Val Gln Ile Ser Leu Lys Ala Leu Arg Arg Ile Val
               420                 425                 430
   Pro Leu Met Glu Gln Gly Lys Arg Tyr Asp Glu Ala Cys Ala Glu Ile
           435                 440                 445
   Tyr Gly Asp His Tyr Cys Lys Lys Asn Ala Glu Glu Lys Ile Tyr Leu
       450                 455                 460
   Pro Pro Ile Pro Ala Asp Glu Ile Arg Asn Pro Val Val Leu Arg Ala
   465                 470                 475                 480
   Leu Ser Gln Ala Arg Lys Val Ile Asn Cys Val Val Arg Arg Tyr Gly
                   485                 490                 495
   Ser Pro Ala Arg Ile His Ile Glu Thr Ala Arg Glu Val Gly Lys Ser
               500                 505                 510
   Phe Lys Asp Arg Lys Glu Ile Glu Lys Arg Gln Glu Glu Asn Arg Lys
           515                 520                 525
   Asp Arg Glu Lys Ala Ala Ala Lys Phe Arg Glu Tyr Phe Pro Asn Phe
       530                 535                 540
   Val Gly Glu Pro Lys Ser Lys Asp Ile Leu Lys Leu Arg Leu Tyr Glu
   545                 550                 555                 560
   Gln Gln His Gly Lys Cys Leu Tyr Ser Gly Lys Glu Ile Asn Leu Val
                   565                 570                 575
   Arg Leu Asn Glu Lys Gly Tyr Val Glu Ile Asp His Ala Leu Pro Phe
               580                 585                 590
   Ser Arg Thr Trp Asp Asp Ser Phe Asn Asn Lys Val Leu Val Leu Gly
           595                 600                 605
   Ser Glu Asn Gln Asn Lys Gly Asn Gln Thr Pro Tyr Glu Tyr Phe Asn
       610                 615                 620
   Gly Lys Asp Asn Ser Arg Glu Trp Gln Glu Phe Lys Ala Arg Val Glu
   625                 630                 635                 640
   Thr Ser Arg Phe Pro Arg Ser Lys Lys Gln Arg Ile Leu Leu Gln Lys
                   645                 650                 655
   Phe Asp Glu Glu Gly Phe Lys Glu Arg Asn Leu Asn Asp Thr Arg Tyr
               660                 665                 670
   Val Asn Arg Phe Leu Cys Gln Phe Val Ala Asp His Ile Leu Leu Thr
           675                 680                 685
   Gly Lys Gly Lys Arg Arg Val Phe Ala Ser Asn Gly Gln Ile Thr Asn
       690                 695                 700
   Leu Leu Arg Gly Phe Trp Gly Leu Arg Lys Val Arg Ala Glu Asn Asp
   705                 710                 715                 720
   Arg His His Ala Leu Asp Ala Val Val Val Ala Cys Ser Thr Val Ala
                   725                 730                 735
   Met Gln Gln Lys Ile Thr Arg Phe Val Arg Tyr Lys Glu Met Asn Ala
               740                 745                 750
   Phe Asp Gly Lys Thr Ile Asp Lys Glu Thr Gly Glu Val Leu His Gln
           755                 760                 765
   Lys Ala His Phe Pro Gln Pro Trp Glu Phe Phe Ala Gln Glu Val Met
       770                 775                 780
   Ile Arg Val Phe Gly Lys Pro Asp Gly Lys Pro Glu Phe Glu Glu Ala
   785                 790                 795                 800
   Asp Thr Pro Glu Lys Leu Arg Thr Leu Leu Ala Glu Lys Leu Ser Ser
                   805                 810                 815
   Arg Pro Glu Ala Val His Glu Tyr Val Thr Pro Leu Phe Val Ser Arg
               820                 825                 830
   Ala Pro Asn Arg Lys Met Ser Gly Gln Gly His Met Glu Thr Val Lys
           835                 840                 845
   Ser Ala Lys Arg Leu Asp Glu Gly Ile Ser Val Leu Arg Val Pro Leu
       850                 855                 860
   Thr Gln Leu Lys Leu Lys Gly Leu Glu Lys Met Val Asn Arg Glu Arg
   865                 870                 875                 880
   Glu Pro Lys Leu Tyr Asp Ala Leu Lys Ala Gln Leu Glu Thr His Lys
                   885                 890                 895
   Asp Asn Pro Ala Lys Ala Phe Ala Glu Pro Phe Tyr Lys Tyr Asp Lys
               900                 905                 910
   Ala Gly Asn Arg Thr Gln Gln Val Lys Ala Val Arg Ile Glu Gln Val
           915                 920                 925
   Gln Lys Thr Gly Val Trp Val Arg Asn His Asn Gly Ile Ala Asp Asn
       930                 935                 940
   Ala Thr Met Val Arg Val Asp Val Phe Glu Lys Gly Gly Lys Tyr Tyr
   945                 950                 955                 960
   Leu Val Pro Ile Tyr Ser Trp Gln Val Ala Lys Gly Ile Leu Pro Asp
                   965                 970                 975
   Arg Ala Val Val Ala Phe Lys Asp Glu Glu Asp Trp Thr Val Met Asp
               980                 985                 990
   Asp Ser Phe Glu Phe Arg Phe Val Leu Tyr Ala Asn Asp Leu Ile Lys
           995                 1000                1005
   Leu Thr Ala Lys Lys Asn Glu Phe Leu Gly Tyr Phe Val Ser Leu Asn
       1010                1015                1020
   Arg Ala Thr Gly Ala Ile Asp Ile Arg Thr His Asp Thr Asp Ser Thr
   1025                1030                1035                1040
   Lys Gly Lys Asn Gly Ile Phe Gln Ser Val Gly Val Lys Thr Ala Leu
                   1045                1050                1055
   Ser Phe Gln Lys Asn Gln Ile Asp Glu Leu Gly Lys Glu Ile Arg Pro
               1060                1065                1070
   Cys Arg Leu Lys Lys Arg Pro Pro Val Arg
           1075                1080
<210> 129
   <211> 1345
   <212> PRT
   <213> Streptococcus mutans
<220>
   <223> CRISPR-associated endonuclease
<308> YP_003484612
   <309> 2013-08-27
<400> 129
   Met Lys Lys Pro Tyr Ser Ile Gly Leu Asp Ile Gly Thr Asn Ser Val
    1               5                  10                  15
   Gly Trp Ala Val Val Thr Asp Asp Tyr Lys Val Pro Ala Lys Lys Met
               20                  25                  30
   Lys Val Leu Gly Asn Thr Asp Lys Ser His Ile Lys Lys Asn Leu Leu
           35                  40                  45
   Gly Ala Leu Leu Phe Asp Ser Gly Asn Thr Ala Ala Asp Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ala Glu Glu Met Ser Lys Val Asp Asp Ser
                   85                  90                  95
   Phe Phe His Arg Leu Glu Asp Ser Phe Leu Val Thr Glu Asp Lys Arg
               100                 105                 110
   Gly Glu Arg His Pro Ile Phe Gly Asn Leu Glu Glu Glu Val Lys Tyr
           115                 120                 125
   His Glu Asn Phe Pro Thr Ile Tyr His Leu Arg Gln Tyr Leu Ala Asp
       130                 135                 140
   Asn Pro Glu Lys Val Asp Leu Arg Leu Val Tyr Leu Ala Leu Ala His
   145                 150                 155                 160
   Ile Ile Lys Phe Arg Gly His Phe Leu Ile Glu Gly Lys Phe Asp Thr
                   165                 170                 175
   Arg Asn Asn Asp Val Gln Arg Leu Phe Gln Glu Phe Leu Ala Val Tyr
               180                 185                 190
   Asp Asn Thr Phe Glu Asn Ser Ser Leu Gln Glu Gln Asn Val Gln Val
           195                 200                 205
   Glu Glu Ile Leu Thr Asp Lys Ile Ser Lys Ser Ala Lys Lys Asp Arg
       210                 215                 220
   Val Leu Lys Leu Phe Pro Asn Glu Lys Ser Asn Gly Arg Phe Ala Glu
   225                 230                 235                 240
   Phe Leu Lys Leu Ile Val Gly Asn Gln Ala Asp Phe Lys Lys His Phe
                   245                 250                 255
   Glu Leu Glu Glu Lys Ala Pro Leu Gln Phe Ser Lys Asp Thr Tyr Glu
               260                 265                 270
   Glu Glu Leu Glu Val Leu Leu Ala Gln Ile Gly Asp Asn Tyr Ala Glu
           275                 280                 285
   Leu Phe Leu Ser Ala Lys Lys Leu Tyr Asp Ser Ile Leu Leu Ser Gly
       290                 295                 300
   Ile Leu Thr Val Thr Asp Val Ser Thr Lys Ala Pro Leu Ser Ala Ser
   305                 310                 315                 320
   Met Ile Gln Arg Tyr Asn Glu His Gln Met Asp Leu Ala Gln Leu Lys
                   325                 330                 335
   Gln Phe Ile Arg Gln Lys Leu Ser Asp Lys Tyr Asn Glu Val Phe Ser
   340 345 350
   Asp Val Ser Lys Asp Gly Tyr Ala Gly Tyr Ile Asp Gly Lys Thr Asn
   355 360 365
   Gln Glu Ala Phe Tyr Lys Tyr Leu Lys Gly Leu Leu Asn Lys Ile Glu
   370 375 380
   Gly Ser Gly Tyr Phe Leu Asp Lys Ile Glu Arg Glu Asp Phe Leu Arg
   385 390 395 400
   Lys Gln Arg Thr Phe Asp Asn Gly Ser Ile Pro His Gln Ile His Leu
   405 410 415
   Gln Glu Met Arg Ala Ile Ile Arg Arg Gln Ala Glu Phe Tyr Pro Phe
   420 425 430
   Leu Ala Asp Asn Gln Asp Arg Ile Glu Lys Ile Leu Thr Phe Arg Ile
   435 440 445
   Pro Tyr Tyr Val Gly Pro Leu Ala Arg Gly Lys Ser Asp Phe Ala Trp
   450 455 460
   Leu Ser Arg Lys Ser Ala Asp Lys Ile Thr Pro Trp Asn Phe Asp Glu
   465 470 475 480
   Ile Val Asp Lys Glu Ser Ser Ala Glu Ala Phe Ile Asn Arg Met Thr
   485 490 495
   Asn Tyr Asp Leu Tyr Leu Pro Asn Gln Lys Val Leu Pro Lys His Ser
   500 505 510
   Leu Leu Tyr Glu Lys Phe Thr Val Tyr Asn Glu Leu Thr Lys Val Lys
   515 520 525
   Tyr Lys Thr Glu Gln Gly Lys Thr Ala Phe Phe Asp Ala Asn Met Lys
   530 535 540
   Gln Glu Ile Phe Asp Gly Val Phe Lys Val Tyr Arg Lys Val Thr Lys
   545 550 555 560
   Asp Lys Leu Met Asp Phe Leu Glu Lys Glu Phe Asp Glu Phe Arg Ile
   565 570 575
   Val Asp Leu Thr Gly Leu Asp Lys Glu Asn Lys Ala Phe Asn Ala Ser
   580 585 590
   Tyr Gly Thr Tyr His Asp Leu Arg Lys Ile Leu Asp Lys Asp Phe Leu
   595 600 605
   Asp Asn Ser Lys Asn Glu Lys Ile Leu Glu Asp Ile Val Leu Thr Leu
   610 615 620
   Thr Leu Phe Glu Asp Arg Glu Met Ile Arg Lys Arg Leu Glu Asn Tyr
   625 630 635 640
   Ser Asp Leu Leu Thr Lys Glu Gln Val Lys Lys Leu Glu Arg Arg His
   645 650 655
   Tyr Thr Gly Trp Gly Arg Leu Ser Ala Glu Leu Ile His Gly Ile Arg
   660 665 670
   Asn Lys Glu Ser Arg Lys Thr Ile Leu Asp Tyr Leu Ile Asp Asp Gly
   675 680 685
   Asn Ser Asn Arg Asn Phe Met Gln Leu Ile Asn Asp Asp Ala Leu Ser
   690 695 700
   Phe Lys Glu Glu Ile Ala Lys Ala Gln Val Ile Gly Glu Thr Asp Asn
   705 710 715 720
   Leu Asn Gln Val Val Ser Asp Ile Ala Gly Ser Pro Ala Ile Lys Lys
   725 730 735
   Gly Ile Leu Gln Ser Leu Lys Ile Val Asp Glu Leu Val Lys Ile Met
   740 745 750
   Gly His Gln Pro Glu Asn Ile Val Val Glu Met Ala Arg Glu Asn Gln
   755 760 765
   Phe Thr Asn Gln Gly Arg Arg Asn Ser Gln Gln Arg Leu Lys Gly Leu
   770 775 780
   Thr Asp Ser Ile Lys Glu Phe Gly Ser Gln Ile Leu Lys Glu His Pro
   785 790 795 800
   Val Lys His Ser Gln Leu Gln Asn Asp Arg Leu Phe Leu Tyr Tyr Leu
   805 810 815
   Gln Asn Gly Arg Asp Met Tyr Thr Gly Glu Glu Leu Asp Ile Asp Tyr
   820 825 830
   Leu Ser Gln Tyr Asp Ile Asp His Ile Ile Pro Gln Ala Phe Ile Lys
   835 840 845
   Asp Asn Ser Ile Asp Asn Arg Val Leu Thr Ser Ser Lys Glu Asn Arg
   850 855 860
   Gly Lys Ser Asp Asp Val Pro Ser Lys Asn Val Val Arg Lys Met Lys
   865 870 875 880
   Ser Tyr Trp Ser Lys Leu Leu Ser Ala Lys Leu Ile Thr Gln Arg Lys
   885 890 895
   Phe Asp Asn Leu Thr Lys Ala Glu Arg Gly Gly Leu Thr Asp Asp Asp
   900 905 910
   Lys Ala Gly Phe Ile Lys Arg Gln Leu Val Glu Thr Arg Gln Ile Thr
   915 920 925
   Lys His Val Ala Arg Ile Leu Asp Glu Arg Phe His Thr Glu Thr Asp
   930 935 940
   Glu Asn Asn Lys Lys Ile Arg Gln Val Lys Ile Val Thr Leu Lys Ser
   945 950 955 960
   Asn Leu Val Ser Asn Phe Arg Lys Glu Phe Glu Leu Tyr Lys Val Arg
   965 970 975
   Glu Ile Asn Asp Tyr His His Ala His Asp Ala Tyr Leu Asn Ala Val
   980 985 990
   Ile Gly Lys Ala Leu Leu Gly Val Tyr Pro Gln Leu Glu Pro Glu Phe
   995 1000 1005
   Val Tyr Gly Asp Tyr Pro His Phe His Gly His Lys Glu Asn Lys Ala
   1010 1015 1020
   Thr Ala Lys Lys Phe Phe Tyr Ser Asn Ile Met Asn Phe Phe Lys Lys
   1025 1030 1035 1040
   Asp Asp Val Arg Thr Asp Lys Asn Gly Glu Ile Ile Trp Lys Lys Asp
   1045 1050 1055
   Glu His Ile Ser Asn Ile Lys Lys Val Leu Ser Tyr Pro Gln Val Asn
   1060 1065 1070
   Ile Val Lys Lys Val Glu Glu Gln Thr Gly Gly Phe Ser Lys Glu Ser
   1075 1080 1085
   Ile Leu Pro Lys Gly Asn Ser Asp Lys Leu Ile Pro Arg Lys Thr Lys
   1090 1095 1100
   Lys Phe Tyr Trp Asp Thr Lys Lys Tyr Gly Gly Phe Asp Ser Pro Ile
   1105 1110 1115 1120
   Val Ala Tyr Ser Ile Leu Val Ile Ala Asp Ile Glu Lys Gly Lys Ser
   1125 1130 1135
   Lys Lys Leu Lys Thr Val Lys Ala Leu Val Gly Val Thr Ile Met Glu
   1140 1145 1150
   Lys Met Thr Phe Glu Arg Asp Pro Val Ala Phe Leu Glu Arg Lys Gly
   1155 1160 1165
   Tyr Arg Asn Val Gln Glu Glu Asn Ile Ile Lys Leu Pro Lys Tyr Ser
   1170 1175 1180
   Leu Phe Lys Leu Glu Asn Gly Arg Lys Arg Leu Leu Ala Ser Ala Arg
   1185 1190 1195 1200
   Glu Leu Gln Lys Gly Asn Glu Ile Val Leu Pro Asn His Leu Gly Thr
   1205 1210 1215
   Leu Leu Tyr His Ala Lys Asn Ile His Lys Val Asp Glu Pro Lys His
   1220 1225 1230
   Leu Asp Tyr Val Asp Lys His Lys Asp Glu Phe Lys Glu Leu Leu Asp
   1235 1240 1245
   Val Val Ser Asn Phe Ser Lys Lys Tyr Thr Leu Ala Glu Gly Asn Leu
   1250 1255 1260
   Glu Lys Ile Lys Glu Leu Tyr Ala Gln Asn Asn Gly Glu Asp Leu Lys
   1265 1270 1275 1280
   Glu Leu Ala Ser Ser Phe Ile Asn Leu Leu Thr Phe Thr Ala Ile Gly
   1285 1290 1295
   Ala Pro Ala Thr Phe Lys Phe Phe Asp Lys Asn Ile Asp Arg Lys Arg
   1300 1305 1310
   Tyr Thr Ser Thr Thr Glu Ile Leu Asn Ala Thr Leu Ile His Gln Ser
   1315 1320 1325
   Ile Thr Gly Leu Tyr Glu Thr Arg Ile Asp Leu Ser Lys Leu Gly Gly
   1330 1335 1340
   Asp
   1345
<210> 130
   <211> 1371
   <212> PRT
   <213> Streptococcus dysgalactiae
<220>
   <223> CRISPR-associated endonuclease
<308> YP_002996940
   <309> 2013-06-27
<400> 130
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Arg
   65                  70                  75                  80
   Tyr Leu Gln Glu Ile Phe Ser Ser Glu Met Ser Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Ala Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Met Asp Lys Leu Phe Ile Gln Leu Val Gln Thr Tyr
               180                 185                 190
   Asn Gln Leu Phe Glu Glu Asn Pro Ile Asn Ala Ser Arg Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Arg Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
   370 375 380
   Gly Thr Glu Glu Leu Leu Ala Lys Leu Asn Arg Glu Asp Leu Leu Arg
   385 390 395 400
   Lys Gln Arg Thr Phe Asp Asn Gly Ser Ile Pro His Gln Ile His Leu
   405 410 415
   Gly Glu Leu His Ala Ile Leu Arg Arg Gln Glu Asp Phe Tyr Pro Phe
   420 425 430
   Leu Lys Asp Asn Arg Glu Lys Ile Glu Lys Ile Leu Thr Phe Arg Ile
   435 440 445
   Pro Tyr Tyr Val Gly Pro Leu Ala Arg Gly Asn Ser Arg Phe Ala Trp
   450 455 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Glu Phe Leu Ser Gly Lys Gln
   530 535 540
   Lys Glu Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Thr Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
   610 615 620
   Leu Phe Glu Asp Lys Glu Met Ile Glu Glu Arg Leu Lys Lys Tyr Ala
   625 630 635 640
   Asn Leu Phe Asp Asp Lys Val Met Lys Gln Leu Lys Arg Arg His Tyr
   645 650 655
   Thr Gly Trp Gly Arg Leu Ser Arg Lys Leu Ile Asn Gly Ile Arg Asp
   660 665 670
   Lys Gln Ser Gly Lys Thr Ile Leu Asp Phe Leu Lys Ser Asp Gly Phe
   675 680 685
   Ala Asn Arg Asn Phe Met Gln Leu Ile Asn Asp Asp Ser Leu Thr Phe
   690 695 700
   Lys Glu Ala Ile Gln Lys Ala Gln Val Ser Gly Gln Gly His Ser Leu
   705 710 715 720
   His Glu Gln Ile Ala Asn Leu Ala Gly Ser Pro Ala Ile Lys Lys Gly
   725 730 735
   Ile Leu Gln Ser Val Lys Val Val Asp Glu Leu Val Lys Val Met Gly
   740 745 750
   His Lys Pro Glu Asn Ile Val Ile Glu Met Ala Arg Glu Asn Gln Thr
   755 760 765
   Thr Gln Lys Gly Gln Lys Asn Ser Arg Glu Arg Met Lys Arg Ile Glu
   770 775 780
   Glu Gly Ile Lys Glu Leu Gly Ser Gln Ile Leu Lys Glu His Pro Val
   785 790 795 800
   Glu Asn Thr Gln Leu Gln Asn Glu Lys Leu Tyr Leu Tyr Tyr Leu Gln
   805 810 815
   Asn Gly Arg Asp Met Tyr Val Asp Gln Glu Leu Asp Ile Asn Arg Leu
   820 825 830
   Ser Asp Tyr Asp Val Asp His Ile Val Pro Gln Ser Phe Ile Lys Asp
   835 840 845
   Asp Ser Ile Asp Asn Lys Ile Leu Thr Arg Ser Asp Lys Asn Arg Gly
   850 855 860
   Lys Ser Asp Asn Val Pro Ser Glu Glu Val Val Lys Lys Met Lys Asn
   865 870 875 880
   Tyr Trp Arg Gln Leu Leu Asn Ala Lys Leu Ile Thr Gln Arg Lys Phe
   885 890 895
   Asp Asn Leu Thr Lys Ala Glu Arg Gly Gly Leu Ser Glu Leu Asp Lys
   900 905 910
   Ala Gly Phe Ile Lys Arg Gln Leu Val Glu Thr Arg Gln Ile Thr Lys
   915 920 925
   His Val Ala Gln Ile Leu Asp Ser Arg Met Asn Thr Lys Tyr Asp Glu
   930 935 940
   Asn Asp Lys Leu Ile Arg Glu Val Lys Val Ile Thr Leu Lys Ser Lys
   945 950 955 960
   Leu Val Ser Asp Phe Arg Lys Asp Phe Gln Phe Tyr Lys Val Arg Glu
   965 970 975
   Ile Asn Asn Tyr His His Ala His Asp Ala Tyr Leu Asn Ala Val Val
   980 985 990
   Gly Thr Ala Leu Ile Lys Lys Tyr Thr Lys Leu Glu Ser Glu Phe Val
   995 1000 1005
   Tyr Gly Asp Tyr Lys Val Tyr Asp Val Arg Lys Met Ile Ala Lys Ser
   1010 1015 1020
   Glu Gln Glu Ile Gly Lys Ala Thr Ala Lys Arg Phe Phe Tyr Ser Asn
   1025 1030 1035 1040
   Ile Met Asn Phe Phe Lys Thr Glu Ile Thr Leu Ala Asn Gly Glu Ile
   1045 1050 1055
   Arg Lys Arg Pro Leu Ile Glu Thr Asn Glu Glu Thr Gly Glu Ile Val
   1060 1065 1070
   Trp Asp Lys Gly Arg Asp Phe Ala Thr Val Arg Lys Val Leu Ser Met
   1075 1080 1085
   Pro Gln Val Asn Ile Val Lys Lys Thr Glu Val Gln Thr Gly Ala Leu
   1090 1095 1100
   Thr Asn Glu Ser Ile Tyr Ala Arg Gly Ser Phe Asp Lys Leu Ile Ser
   1105 1110 1115 1120
   Arg Lys His Arg Phe Glu Ser Ser Lys Tyr Gly Gly Phe Gly Ser Pro
   1125 1130 1135
   Thr Val Thr Tyr Ser Val Leu Val Val Ala Lys Ser Lys Val Gln Asp
   1140 1145 1150
   Gly Lys Val Lys Lys Ile Lys Thr Gly Lys Glu Leu Ile Gly Met Thr
   1155 1160 1165
   Leu Leu Asp Lys Leu Val Phe Glu Lys Asn Pro Leu Lys Phe Ile Glu
   1170 1175 1180
   Asp Lys Gly Tyr Gly Asn Val Gln Ile Asp Lys Cys Ile Lys Leu Pro
   1185 1190 1195 1200
   Lys Tyr Ser Leu Phe Glu Phe Glu Asn Gly Thr Arg Arg Met Leu Ala
   1205 1210 1215
   Ser Val Met Ala Asn Asn Asn Ser Arg Gly Asp Leu Gln Lys Ala Asn
   1220 1225 1230
   Glu Met Phe Leu Pro Ala Lys Leu Val Thr Leu Leu Tyr His Ala His
   1235 1240 1245
   Lys Ile Glu Ser Ser Lys Glu Leu Glu His Glu Ala Tyr Ile Leu Asp
   1250 1255 1260
   His Tyr Asn Asp Leu Tyr Gln Leu Leu Ser Tyr Ile Glu Arg Phe Ala
   1265 1270 1275 1280
   Ser Leu Tyr Val Asp Val Glu Lys Asn Ile Ser Lys Val Lys Glu Leu
   1285 1290 1295
   Phe Ser Asn Ile Glu Ser Tyr Ser Ile Ser Glu Ile Cys Ser Ser Val
   1300 1305 1310
   Ile Asn Leu Leu Thr Leu Thr Ala Ser Gly Ala Pro Ala Asp Phe Lys
   1315 1320 1325
   Phe Leu Gly Thr Thr Ile Pro Arg Lys Arg Tyr Gly Ser Pro Gln Ser
   1330 1335 1340
   Ile Leu Ser Ser Thr Leu Ile His Gln Ser Ile Thr Gly Leu Tyr Glu
   1345 1350 1355 1360
   Thr Arg Ile Asp Leu Ser Gln Leu Gly Gly Asp
   1365 1370
<210> 131
   <211> 1082
   <212> PRT
   <213> Neisseria meningitidis
<220>
   <223> CRISPR-associated endonuclease
<308> YP_001598557
   <309> 2013-06-10
<400> 131
   Met Ala Ala Phe Lys Pro Asn Pro Ile Asn Tyr Ile Leu Gly Leu Asp
    1               5                  10                  15
   Ile Gly Ile Ala Ser Val Gly Trp Ala Met Val Glu Ile Asp Glu Asp
               20                  25                  30
   Glu Asn Pro Ile Cys Leu Ile Asp Leu Gly Val Arg Val Phe Glu Arg
           35                  40                  45
   Ala Glu Val Pro Lys Thr Gly Asp Ser Leu Ala Met Ala Arg Arg Leu
       50                  55                  60
   Ala Arg Ser Val Arg Arg Leu Thr Arg Arg Arg Ala His Arg Leu Leu
   65                  70                  75                  80
   Arg Ala Arg Arg Leu Leu Lys Arg Glu Gly Val Leu Gln Ala Ala Asp
                   85                  90                  95
   Phe Asp Glu Asn Gly Leu Ile Lys Ser Leu Pro Asn Thr Pro Trp Gln
               100                 105                 110
   Leu Arg Ala Ala Ala Leu Asp Arg Lys Leu Thr Pro Leu Glu Trp Ser
           115                 120                 125
   Ala Val Leu Leu His Leu Ile Lys His Arg Gly Tyr Leu Ser Gln Arg
       130                 135                 140
   Lys Asn Glu Gly Glu Thr Ala Asp Lys Glu Leu Gly Ala Leu Leu Lys
   145                 150                 155                 160
   Gly Val Ala Asp Asn Ala His Ala Leu Gln Thr Gly Asp Phe Arg Thr
                   165                 170                 175
   Pro Ala Glu Leu Ala Leu Asn Lys Phe Glu Lys Glu Ser Gly His Ile
               180                 185                 190
   Arg Asn Gln Arg Gly Asp Tyr Ser His Thr Phe Ser Arg Lys Asp Leu
           195                 200                 205
   Gln Ala Glu Leu Asn Leu Leu Phe Glu Lys Gln Lys Glu Phe Gly Asn
       210                 215                 220
   Pro His Ile Ser Asp Gly Leu Lys Glu Gly Ile Glu Thr Leu Leu Met
   225                 230                 235                 240
   Thr Gln Arg Pro Ala Leu Ser Gly Asp Ala Val Gln Lys Met Leu Gly
                   245                 250                 255
   His Cys Thr Phe Glu Pro Ala Glu Pro Lys Ala Ala Lys Asn Thr Tyr
               260                 265                 270
   Thr Ala Glu Arg Phe Ile Trp Leu Thr Lys Leu Asn Asn Leu Arg Ile
           275                 280                 285
   Leu Glu Gln Gly Ser Glu Arg Pro Leu Thr Asp Thr Glu Arg Thr Thr
       290                 295                 300
   Leu Ile Asp Glu Pro Tyr Arg Lys Ser Lys Leu Thr Tyr Ala Gln Ala
   305                 310                 315                 320
   Arg Lys Leu Leu Glu Leu Asp Asn Thr Ala Phe Phe Lys Gly Leu Arg
                   325                 330                 335
   Tyr Gly Lys Asp Asn Ala Glu Ala Ser Thr Leu Met Glu Met Lys Ala
               340                 345                 350
   Tyr His Ala Ile Ser Arg Ala Leu Glu Lys Glu Gly Leu Lys Asp Lys
           355                 360                 365
   Lys Ser Pro Leu Asn Leu Ser Pro Glu Leu Gln Asp Glu Ile Gly Thr
       370                 375                 380
   Ala Phe Ser Leu Phe Lys Thr Asp Glu Asp Ile Thr Gly Arg Leu Lys
   385                 390                 395                 400
   Asp Arg Val Gln Pro Glu Ile Leu Glu Ala Leu Leu Lys His Ile Ser
   405 410 415
   Phe Asp Lys Phe Val Gln Ile Ser Leu Lys Ala Leu Arg Arg Ile Val
   420 425 430
   Pro Leu Met Glu Gln Gly Lys Arg Tyr Asp Glu Ala Cys Ala Glu Ile
   435 440 445
   Tyr Gly Asp His Tyr Gly Lys Lys Asn Thr Glu Glu Lys Ile Asp Leu
   450 455 460
   Pro Pro Ile Pro Ala Asp Glu Ile Arg Asn Pro Val Val Leu Arg Ala
   465 470 475 480
   Leu Ser Gln Ala Arg Lys Val Ile Asn Gly Val Val Arg Arg Tyr Gly
   485 490 495
   Ser Pro Ala Arg Ile His Ile Glu Thr Ala Arg Glu Val Gly Lys Ser
   500 505 510
   Phe Lys Asp Arg Lys Glu Ile Glu Lys Arg Gln Glu Glu Asn Arg Lys
   515 520 525
   Asp Arg Glu Lys Ala Ala Thr Lys Phe Arg Glu Tyr Phe Pro Asn Phe
   530 535 540
   Val Gly Glu Pro Lys Ser Lys Asp Ile Leu Lys Leu Arg Leu Tyr Glu
   545 550 555 560
   Gln Gln His Gly Lys Cys Leu Tyr Ser Gly Lys Glu Ile Asn Leu Gly
   565 570 575
   Arg Leu Asn Glu Lys Gly Tyr Val Glu Ile Asp His Ala Leu Pro Phe
   580 585 590
   Ser Arg Thr Trp Asp Asp Ser Phe Asn Asn Lys Val Leu Val Leu Gly
   595 600 605
   Ser Glu Asn Gln Asn Lys Gly Asn Gln Thr Pro Tyr Glu Tyr Phe Asn
   610 615 620
   Gly Lys Asp Asn Ser Arg Glu Trp Gln Glu Phe Lys Ala Arg Val Glu
   625 630 635 640
   Thr Ser Arg Phe Pro Arg Ser Lys Lys Gln Arg Ile Leu Leu Gln Lys
   645 650 655
   Phe Asp Glu Glu Gly Phe Lys Glu Arg Asn Leu Asn Asp Thr Arg Tyr
   660 665 670
   Val Asn Arg Phe Leu Cys Gln Phe Val Ala Asp His Met Leu Leu Thr
   675 680 685
   Gly Lys Gly Lys Arg Arg Val Phe Ala Ser Asn Gly Gln Ile Thr Asn
   690 695 700
   Leu Leu Arg Gly Phe Trp Gly Leu Arg Lys Val Arg Ala Glu Asn Asp
   705 710 715 720
   Arg His His Ala Leu Asp Ala Val Val Val Ala Cys Ser Thr Val Ala
   725 730 735
   Met Gln Gln Lys Ile Thr Arg Phe Val Arg Tyr Lys Glu Met Asn Ala
   740 745 750
   Phe Asp Gly Lys Thr Ile Asp Lys Glu Thr Gly Glu Val Leu His Gln
   755 760 765
   Lys Thr His Phe Pro Gln Pro Trp Glu Phe Phe Ala Gln Glu Val Met
   770 775 780
   Ile Arg Val Phe Gly Lys Pro Asp Gly Lys Pro Glu Phe Glu Glu Ala
   785 790 795 800
   Asp Thr Pro Glu Lys Leu Arg Thr Leu Leu Ala Glu Lys Leu Ser Ser
   805 810 815
   Arg Pro Glu Ala Val His Glu Tyr Val Thr Pro Leu Phe Val Ser Arg
   820 825 830
   Ala Pro Asn Arg Lys Met Ser Gly Gln Gly His Met Glu Thr Val Lys
   835 840 845
   Ser Ala Lys Arg Leu Asp Glu Gly Val Ser Val Leu Arg Val Pro Leu
   850 855 860
   Thr Gln Leu Lys Leu Lys Asp Leu Glu Lys Met Val Asn Arg Glu Arg
   865 870 875 880
   Glu Pro Lys Leu Tyr Glu Ala Leu Lys Ala Arg Leu Glu Ala His Lys
   885 890 895
   Asp Asp Pro Ala Lys Ala Phe Ala Glu Pro Phe Tyr Lys Tyr Asp Lys
                   900                 905                 910
   Ala Gly Asn Arg Thr Gln Gln Val Lys Ala Val Arg Val Glu Gln Val
               915                 920                 925
   Gln Lys Thr Gly Val Trp Val Arg Asn His Asn Gly Ile Ala Asp Asn
           930                 935                 940
   Ala Thr Met Val Arg Val Asp Val Phe Glu Lys Gly Asp Lys Tyr Tyr
   945                 950                 955                 960
   Leu Val Pro Ile Tyr Ser Trp Gln Val Ala Lys Gly Ile Leu Pro Asp
                   965                 970                 975
   Arg Ala Val Val Gln Gly Lys Asp Glu Glu Asp Trp Gln Leu Ile Asp
               980                 985                 990
   Asp Ser Phe Asn Phe Lys Phe Ser Leu His Pro Asn Asp Leu Val Glu
           995                 1000                1005
   Val Ile Thr Lys Lys Ala Arg Met Phe Gly Tyr Phe Ala Ser Cys His
       1010                1015                1020
   Arg Gly Thr Gly Asn Ile Asn Ile Arg Ile His Asp Leu Asp His Lys
   1025                1030                1035                1040
   Ile Gly Lys Asn Gly Ile Leu Glu Gly Ile Gly Val Lys Thr Ala Leu
                   1045                1050                1055
   Ser Phe Gln Lys Tyr Gln Ile Asp Glu Leu Gly Lys Glu Ile Arg Pro
               1060                1065                1070
   Cys Arg Leu Lys Lys Arg Pro Pro Val Arg
           1075                1080
<210> 132
   <211> 1370
   <212> PRT
   <213> Streptococcus agalactiae
<220>
   <223> CRISPR-associated endonuclease
<308> YP_329639
   <309> 2013-06-10
<400> 132
   Met Asn Lys Pro Tyr Ser Ile Gly Leu Asp Ile Gly Thr Asn Ser Val
    1               5                  10                  15
   Gly Trp Ser Ile Ile Thr Asp Asp Tyr Lys Val Pro Ala Lys Lys Met
               20                  25                  30
   Arg Val Leu Gly Asn Thr Asp Lys Glu Tyr Ile Lys Lys Asn Leu Ile
           35                  40                  45
   Gly Ala Leu Leu Phe Asp Gly Gly Asn Thr Ala Ala Asp Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ala Glu Glu Met Ser Lys Val Asp Asp Ser
                   85                  90                  95
   Phe Phe His Arg Leu Glu Asp Ser Phe Leu Val Glu Glu Asp Lys Arg
               100                 105                 110
   Gly Ser Lys Tyr Pro Ile Phe Ala Thr Leu Gln Glu Glu Lys Asp Tyr
           115                 120                 125
   His Glu Lys Phe Ser Thr Ile Tyr His Leu Arg Lys Glu Leu Ala Asp
       130                 135                 140
   Lys Lys Glu Lys Ala Asp Leu Arg Leu Ile Tyr Ile Ala Leu Ala His
   145                 150                 155                 160
   Ile Ile Lys Phe Arg Gly His Phe Leu Ile Glu Asp Asp Ser Phe Asp
                   165                 170                 175
   Val Arg Asn Thr Asp Ile Ser Lys Gln Tyr Gln Asp Phe Leu Glu Ile
               180                 185                 190
   Phe Asn Thr Thr Phe Glu Asn Asn Asp Leu Leu Ser Gln Asn Val Asp
           195                 200                 205
   Val Glu Ala Ile Leu Thr Asp Lys Ile Ser Lys Ser Ala Lys Lys Asp
       210                 215                 220
   Arg Ile Leu Ala Gln Tyr Pro Asn Gln Lys Ser Thr Gly Ile Phe Ala
   225                 230                 235                 240
   Glu Phe Leu Lys Leu Ile Val Gly Asn Gln Ala Asp Phe Lys Lys Tyr
                   245                 250                 255
   Phe Asn Leu Glu Asp Lys Thr Pro Leu Gln Phe Ala Lys Asp Ser Tyr
               260                 265                 270
   Asp Glu Asp Leu Glu Asn Leu Leu Gly Gln Ile Gly Asp Glu Phe Ala
           275                 280                 285
   Asp Leu Phe Ser Ala Ala Lys Lys Leu Tyr Asp Ser Val Leu Leu Ser
       290                 295                 300
   Gly Ile Leu Thr Val Ile Asp Leu Ser Thr Lys Ala Pro Leu Ser Ala
   305                 310                 315                 320
   Ser Met Ile Gln Arg Tyr Asp Glu His Arg Glu Asp Leu Lys Gln Leu
                   325                 330                 335
   Lys Gln Phe Val Lys Ala Ser Leu Pro Glu Lys Tyr Gln Glu Ile Phe
               340                 345                 350
   Ala Asp Ser Ser Lys Asp Gly Tyr Ala Gly Tyr Ile Glu Gly Lys Thr
           355                 360                 365
   Asn Gln Glu Ala Phe Tyr Lys Tyr Leu Ser Lys Leu Leu Thr Lys Gln
       370                 375                 380
   Glu Asp Ser Glu Asn Phe Leu Glu Lys Ile Lys Asn Glu Asp Phe Leu
   385                 390                 395                 400
   Arg Lys Gln Arg Thr Phe Asp Asn Gly Ser Ile Pro His Gln Val His
                   405                 410                 415
   Leu Thr Glu Leu Lys Ala Ile Ile Arg Arg Gln Ser Glu Tyr Tyr Pro
               420                 425                 430
   Phe Leu Lys Glu Asn Gln Asp Arg Ile Glu Lys Ile Leu Thr Phe Arg
           435                 440                 445
   Ile Pro Tyr Tyr Ile Gly Pro Leu Ala Arg Glu Lys Ser Asp Phe Ala
       450                 455                 460
   Trp Met Thr Arg Lys Thr Asp Asp Ser Ile Arg Pro Trp Asn Phe Glu
   465                 470                 475                 480
   Asp Leu Val Asp Lys Glu Lys Ser Ala Glu Ala Phe Ile His Arg Met
                   485                 490                 495
   Thr Asn Asn Asp Phe Tyr Leu Pro Glu Glu Lys Val Leu Pro Lys His
               500                 505                 510
   Ser Leu Ile Tyr Glu Lys Phe Thr Val Tyr Asn Glu Leu Thr Lys Val
           515                 520                 525
   Arg Tyr Lys Asn Glu Gln Gly Glu Thr Tyr Phe Phe Asp Ser Asn Ile
       530                 535                 540
   Lys Gln Glu Ile Phe Asp Gly Val Phe Lys Glu His Arg Lys Val Ser
   545                 550                 555                 560
   Lys Lys Lys Leu Leu Asp Phe Leu Ala Lys Glu Tyr Glu Glu Phe Arg
                   565                 570                 575
   Ile Val Asp Val Ile Gly Leu Asp Lys Glu Asn Lys Ala Phe Asn Ala
               580                 585                 590
   Ser Leu Gly Thr Tyr His Asp Leu Glu Lys Ile Leu Asp Lys Asp Phe
           595                 600                 605
   Leu Asp Asn Pro Asp Asn Glu Ser Ile Leu Glu Asp Ile Val Gln Thr
       610                 615                 620
   Leu Thr Leu Phe Glu Asp Arg Glu Met Ile Lys Lys Arg Leu Glu Asn
   625                 630                 635                 640
   Tyr Lys Asp Leu Phe Thr Glu Ser Gln Leu Lys Lys Leu Tyr Arg Arg
                   645                 650                 655
   His Tyr Thr Gly Trp Gly Arg Leu Ser Ala Lys Leu Ile Asn Gly Ile
               660                 665                 670
   Arg Asp Lys Glu Ser Gln Lys Thr Ile Leu Asp Tyr Leu Ile Asp Asp
           675                 680                 685
   Gly Arg Ser Asn Arg Asn Phe Met Gln Leu Ile Asn Asp Asp Gly Leu
       690                 695                 700
   Ser Phe Lys Ser Ile Ile Ser Lys Ala Gln Ala Gly Ser His Ser Asp
   705 710 715 720
   Asn Leu Lys Glu Val Val Gly Glu Leu Ala Gly Ser Pro Ala Ile Lys
   725 730 735
   Lys Gly Ile Leu Gln Ser Leu Lys Ile Val Asp Glu Leu Val Lys Val
   740 745 750
   Met Gly Tyr Glu Pro Glu Gln Ile Val Val Glu Met Ala Arg Glu Asn
   755 760 765
   Gln Thr Thr Asn Gln Gly Arg Arg Asn Ser Arg Gln Arg Tyr Lys Leu
   770 775 780
   Leu Asp Asp Gly Val Lys Asn Leu Ala Ser Asp Leu Asn Gly Asn Ile
   785 790 795 800
   Leu Lys Glu Tyr Pro Thr Asp Asn Gln Ala Leu Gln Asn Glu Arg Leu
   805 810 815
   Phe Leu Tyr Tyr Leu Gln Asn Gly Arg Asp Met Tyr Thr Gly Glu Ala
   820 825 830
   Leu Asp Ile Asp Asn Leu Ser Gln Tyr Asp Ile Asp His Ile Ile Pro
   835 840 845
   Gln Ala Phe Ile Lys Asp Asp Ser Ile Asp Asn Arg Val Leu Val Ser
   850 855 860
   Ser Ala Lys Asn Arg Gly Lys Ser Asp Asp Val Pro Ser Leu Glu Ile
   865 870 875 880
   Val Lys Asp Cys Lys Val Phe Trp Lys Lys Leu Leu Asp Ala Lys Leu
   885 890 895
   Met Ser Gln Arg Lys Tyr Asp Asn Leu Thr Lys Ala Glu Arg Gly Gly
   900 905 910
   Leu Thr Ser Asp Asp Lys Ala Arg Phe Ile Gln Arg Gln Leu Val Glu
   915 920 925
   Thr Arg Gln Ile Thr Lys His Val Ala Arg Ile Leu Asp Glu Arg Phe
   930 935 940
   Asn Asn Glu Leu Asp Ser Lys Gly Arg Arg Ile Arg Lys Val Lys Ile
   945 950 955 960
   Val Thr Leu Lys Ser Asn Leu Val Ser Asn Phe Arg Lys Glu Phe Gly
   965 970 975
   Phe Tyr Lys Ile Arg Glu Val Asn Asn Tyr His His Ala His Asp Ala
   980 985 990
   Tyr Leu Asn Ala Val Val Ala Lys Ala Ile Leu Thr Lys Tyr Pro Gln
   995 1000 1005
   Leu Glu Pro Glu Phe Val Tyr Gly Asp Tyr Pro Lys Tyr Asn Ser Tyr
   1010 1015 1020
   Lys Thr Arg Lys Ser Ala Thr Glu Lys Leu Phe Phe Tyr Ser Asn Ile
   1025 1030 1035 1040
   Met Asn Phe Phe Lys Thr Lys Val Thr Leu Ala Asp Gly Thr Val Val
   1045 1050 1055
   Val Lys Asp Asp Ile Glu Val Asn Asn Asp Thr Gly Glu Ile Val Trp
   1060 1065 1070
   Asp Lys Lys Lys His Phe Ala Thr Val Arg Lys Val Leu Ser Tyr Pro
   1075 1080 1085
   Gln Asn Asn Ile Val Lys Lys Thr Glu Ile Gln Thr Gly Gly Phe Ser
   1090 1095 1100
   Lys Glu Ser Ile Leu Ala His Gly Asn Ser Asp Lys Leu Ile Pro Arg
   1105 1110 1115 1120
   Lys Thr Lys Asp Ile Tyr Leu Asp Pro Lys Lys Tyr Gly Gly Phe Asp
   1125 1130 1135
   Ser Pro Ile Val Ala Tyr Ser Val Leu Val Val Ala Asp Ile Lys Lys
   1140 1145 1150
   Gly Lys Ala Gln Lys Leu Lys Thr Val Thr Glu Leu Leu Gly Ile Thr
   1155 1160 1165
   Ile Met Glu Arg Ser Arg Phe Glu Lys Asn Pro Ser Ala Phe Leu Glu
   1170 1175 1180
   Ser Lys Gly Tyr Leu Asn Ile Arg Ala Asp Lys Leu Ile Ile Leu Pro
   1185 1190 1195 1200
   Lys Tyr Ser Leu Phe Glu Leu Glu Asn Gly Arg Arg Arg Leu Leu Ala
   1205 1210 1215
   Ser Ala Gly Glu Leu Gln Lys Gly Asn Glu Leu Ala Leu Pro Thr Gln
               1220                1225                1230
   Phe Met Lys Phe Leu Tyr Leu Ala Ser Arg Tyr Asn Glu Ser Lys Gly
           1235                1240                1245
   Lys Pro Glu Glu Ile Glu Lys Lys Gln Glu Phe Val Asn Gln His Val
       1250                1255                1260
   Ser Tyr Phe Asp Asp Ile Leu Gln Leu Ile Asn Asp Phe Ser Lys Arg
   1265                1270                1275                1280
   Val Ile Leu Ala Asp Ala Asn Leu Glu Lys Ile Asn Lys Leu Tyr Gln
                   1285                1290                1295
   Asp Asn Lys Glu Asn Ile Ser Val Asp Glu Leu Ala Asn Asn Ile Ile
               1300                1305                1310
   Asn Leu Phe Thr Phe Thr Ser Leu Gly Ala Pro Ala Ala Phe Lys Phe
           1315                1320                1325
   Phe Asp Lys Ile Val Asp Arg Lys Arg Tyr Thr Ser Thr Lys Glu Val
       1330                1335                1340
   Leu Asn Ser Thr Leu Ile His Gln Ser Ile Thr Gly Leu Tyr Glu Thr
   1345                1350                1355                1360
   Arg Ile Asp Leu Gly Lys Leu Gly Glu Asp
                   1365                1370
<210> 133
   <211> 1368
   <212> PRT
   <213> Streptococcus pyogenes
<220>
   <223> CRISPR-associated endonuclease
<308> YP_282132
   <309> 2013-08-28
<400> 133
   Met Asp Lys Lys Tyr Ser Ile Gly Leu Asp Ile Gly Thr Asn Ser Val
   1               5                   10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Gln Ile Leu Lys Glu His Pro
   785                 790                 795                 800
   Val Glu Asn Thr Gln Leu Gln Asn Glu Lys Leu Tyr Leu Tyr Tyr Leu
                   805                 810                 815
   Gln Asn Gly Arg Asp Met Tyr Val Asp Gln Glu Leu Asp Ile Asn Arg
               820                 825                 830
   Leu Ser Asp Tyr Asp Val Asp His Ile Val Pro Gln Ser Phe Leu Lys
           835                 840                 845
   Asp Asp Ser Ile Asp Asn Lys Val Leu Thr Arg Ser Asp Lys Asn Arg
       850                 855                 860
   Gly Lys Ser Asp Asn Val Pro Ser Glu Glu Val Val Lys Lys Met Lys
   865                 870                 875                 880
   Asn Tyr Trp Arg Gln Leu Leu Asn Ala Lys Leu Ile Thr Gln Arg Lys
                   885                 890                 895
   Phe Asp Asn Leu Thr Lys Ala Glu Arg Gly Gly Leu Ser Glu Leu Asp
               900                 905                 910
   Lys Ala Gly Phe Ile Lys Arg Gln Leu Val Glu Thr Arg Gln Ile Thr
           915                 920                 925
   Lys His Val Ala Gln Ile Leu Asp Ser Arg Met Asn Thr Lys Tyr Asp
       930                 935                 940
   Glu Asn Asp Lys Leu Ile Arg Glu Val Lys Val Ile Thr Leu Lys Ser
   945                 950                 955                 960
   Lys Leu Val Ser Asp Phe Arg Lys Asp Phe Gln Phe Tyr Lys Val Arg
                   965                 970                 975
   Glu Ile Asn Asn Tyr His His Ala His Asp Ala Tyr Leu Asn Ala Val
               980                 985                 990
   Val Gly Thr Ala Leu Ile Lys Lys Tyr Pro Lys Leu Glu Ser Glu Phe
           995                 1000                1005
   Val Tyr Gly Asp Tyr Lys Val Tyr Asp Val Arg Lys Met Ile Ala Lys
       1010                1015                1020
   Ser Glu Gln Glu Ile Gly Lys Ala Thr Ala Lys Tyr Phe Phe Tyr Ser
   1025                1030                1035                1040
   Asn Ile Met Asn Phe Phe Lys Thr Glu Ile Thr Leu Ala Asn Gly Glu
                   1045                1050                1055
   Ile Arg Lys Arg Pro Leu Ile Glu Thr Asn Gly Glu Thr Gly Glu Ile
               1060                1065                1070
   Val Trp Asp Lys Gly Arg Asp Phe Ala Thr Val Arg Lys Val Leu Ser
           1075                1080                1085
   Met Pro Gln Val Asn Ile Val Lys Lys Thr Glu Val Gln Thr Gly Gly
       1090                1095                1100
   Phe Ser Lys Glu Ser Ile Leu Pro Lys Arg Asn Ser Asp Lys Leu Ile
   1105                1110                1115                1120
   Ala Arg Lys Lys Asp Trp Asp Pro Lys Lys Tyr Gly Gly Phe Asp Ser
                   1125                1130                1135
   Pro Thr Val Ala Tyr Ser Val Leu Val Val Ala Lys Val Glu Lys Gly
               1140                1145                1150
   Lys Ser Lys Lys Leu Lys Ser Val Lys Glu Leu Leu Gly Ile Thr Ile
           1155                1160                1165
   Met Glu Arg Ser Ser Phe Glu Lys Asn Pro Ile Asp Phe Leu Glu Ala
       1170                1175                1180
   Lys Gly Tyr Lys Glu Val Lys Lys Asp Leu Ile Ile Lys Leu Pro Lys
   1185                1190                1195                1200
   Tyr Ser Leu Phe Glu Leu Glu Asn Gly Arg Lys Arg Met Leu Ala Ser
                   1205                1210                1215
   Ala Gly Glu Leu Gln Lys Gly Asn Glu Leu Ala Leu Pro Ser Lys Tyr
               1220                1225                1230
   Val Asn Phe Leu Tyr Leu Ala Ser His Tyr Glu Lys Leu Lys Gly Ser
           1235                1240                1245
   Pro Glu Asp Asn Glu Gln Lys Gln Leu Phe Val Glu Gln His Lys His
       1250                1255                1260
   Tyr Leu Asp Glu Ile Ile Glu Gln Ile Ser Glu Phe Ser Lys Arg Val
   1265                1270                1275                1280
   Ile Leu Ala Asp Ala Asn Leu Asp Lys Val Leu Ser Ala Tyr Asn Lys
                   1285                1290                1295
   His Arg Asp Lys Pro Ile Arg Glu Gln Ala Glu Asn Ile Ile His Leu
               1300                1305                1310
   Phe Thr Leu Thr Asn Leu Gly Ala Pro Ala Ala Phe Lys Tyr Phe Asp
           1315                1320                1325
   Thr Thr Ile Asp Arg Lys Arg Tyr Thr Ser Thr Lys Glu Val Leu Asp
       1330                1335                1340
   Ala Thr Leu Ile His Gln Ser Ile Thr Gly Leu Tyr Glu Thr Arg Ile
   1345                1350                1355                1360
   Asp Leu Ser Gln Leu Gly Gly Asp
                   1365
<210> 134
   <211> 1314
   <212> PRT
   <213> Mycoplasma synoviae
<220>
   <223> CRISPR-associated endonuclease
<308> YP_278700
   <309> 2013-06-10
<400> 134
   Met Leu Arg Leu Tyr Cys Ala Asn Asn Leu Val Leu Asn Asn Val Gln
    1               5                  10                  15
   Asn Leu Trp Lys Tyr Leu Leu Leu Leu Ile Phe Asp Lys Lys Ile Ile
               20                  25                  30
   Phe Leu Phe Lys Ile Lys Val Ile Leu Ile Arg Arg Tyr Met Glu Asn
           35                  40                  45
   Asn Asn Lys Glu Lys Ile Val Ile Gly Phe Asp Leu Gly Val Ala Ser
       50                  55                  60
   Val Gly Trp Ser Ile Val Asn Ala Glu Thr Lys Glu Val Ile Asp Leu
   65                  70                  75                  80
   Gly Val Arg Leu Phe Ser Glu Pro Glu Lys Ala Asp Tyr Arg Arg Ala
                   85                  90                  95
   Lys Arg Thr Thr Arg Arg Leu Leu Arg Arg Lys Lys Phe Lys Arg Glu
               100                 105                 110
   Lys Phe His Lys Leu Ile Leu Lys Asn Ala Glu Ile Phe Gly Leu Gln
           115                 120                 125
   Ser Arg Asn Glu Ile Leu Asn Val Tyr Lys Asp Gln Ser Ser Lys Tyr
       130                 135                 140
   Arg Asn Ile Leu Lys Leu Lys Ile Asn Ala Leu Lys Glu Glu Ile Lys
   145                 150                 155                 160
   Pro Ser Glu Leu Val Trp Ile Leu Arg Asp Tyr Leu Gln Asn Arg Gly
                   165                 170                 175
   Tyr Phe Tyr Lys Asn Glu Lys Leu Thr Asp Glu Phe Val Ser Asn Ser
               180                 185                 190
   Phe Pro Ser Lys Lys Leu His Glu His Tyr Glu Lys Tyr Gly Phe Phe
           195                 200                 205
   Arg Gly Ser Val Lys Leu Asp Asn Lys Leu Asp Asn Lys Lys Asp Lys
       210                 215                 220
   Ala Lys Glu Lys Asp Glu Glu Glu Glu Ser Asp Ala Lys Lys Glu Ser
   225                 230                 235                 240
   Glu Glu Leu Ile Phe Ser Asn Lys Gln Trp Ile Asn Glu Ile Val Lys
                   245                 250                 255
   Val Phe Glu Asn Gln Ser Tyr Leu Thr Glu Ser Phe Lys Glu Glu Tyr
   260 265 270
   Leu Lys Leu Phe Asn Tyr Val Arg Pro Phe Asn Lys Gly Pro Gly Ser
   275 280 285
   Lys Asn Ser Arg Thr Ala Tyr Gly Val Phe Ser Thr Asp Ile Asp Pro
   290 295 300
   Glu Thr Asn Lys Phe Lys Asp Tyr Ser Asn Ile Trp Asp Lys Thr Ile
   305 310 315 320
   Gly Lys Cys Ser Leu Phe Glu Glu Glu Ile Arg Ala Pro Lys Asn Leu
   325 330 335
   Pro Ser Ala Leu Ile Phe Asn Leu Gln Asn Glu Ile Cys Thr Ile Lys
   340 345 350
   Asn Glu Phe Thr Glu Phe Lys Asn Trp Trp Leu Asn Ala Glu Gln Lys
   355 360 365
   Ser Glu Ile Leu Lys Phe Val Phe Thr Glu Leu Phe Asn Trp Lys Asp
   370 375 380
   Lys Lys Tyr Ser Asp Lys Lys Phe Asn Lys Asn Leu Gln Asp Lys Ile
   385 390 395 400
   Lys Lys Tyr Leu Leu Asn Phe Ala Leu Glu Asn Phe Asn Leu Asn Glu
   405 410 415
   Glu Ile Leu Lys Asn Arg Asp Leu Glu Asn Asp Thr Val Leu Gly Leu
   420 425 430
   Lys Gly Val Lys Tyr Tyr Glu Lys Ser Asn Ala Thr Ala Asp Ala Ala
   435 440 445
   Leu Glu Phe Ser Ser Leu Lys Pro Leu Tyr Val Phe Ile Lys Phe Leu
   450 455 460
   Lys Glu Lys Lys Leu Asp Leu Asn Tyr Leu Leu Gly Leu Glu Asn Thr
   465 470 475 480
   Glu Ile Leu Tyr Phe Leu Asp Ser Ile Tyr Leu Ala Ile Ser Tyr Ser
   485 490 495
   Ser Asp Leu Lys Glu Arg Asn Glu Trp Phe Lys Lys Leu Leu Lys Glu
   500 505 510
   Leu Tyr Pro Lys Ile Lys Asn Asn Asn Leu Glu Ile Ile Glu Asn Val
   515 520 525
   Glu Asp Ile Phe Glu Ile Thr Asp Gln Glu Lys Phe Glu Ser Phe Ser
   530 535 540
   Lys Thr His Ser Leu Ser Arg Glu Ala Phe Asn His Ile Ile Pro Leu
   545 550 555 560
   Leu Leu Ser Asn Asn Glu Gly Lys Asn Tyr Glu Ser Leu Lys His Ser
   565 570 575
   Asn Glu Glu Leu Lys Lys Arg Thr Glu Lys Ala Glu Leu Lys Ala Gln
   580 585 590
   Gln Asn Gln Lys Tyr Leu Lys Asp Asn Phe Leu Lys Glu Ala Leu Val
   595 600 605
   Pro Leu Ser Val Lys Thr Ser Val Leu Gln Ala Ile Lys Ile Phe Asn
   610 615 620
   Gln Ile Ile Lys Asn Phe Gly Lys Lys Tyr Glu Ile Ser Gln Val Val
   625 630 635 640
   Ile Glu Met Ala Arg Glu Leu Thr Lys Pro Asn Leu Glu Lys Leu Leu
   645 650 655
   Asn Asn Ala Thr Asn Ser Asn Ile Lys Ile Leu Lys Glu Lys Leu Asp
   660 665 670
   Gln Thr Glu Lys Phe Asp Asp Phe Thr Lys Lys Lys Phe Ile Asp Lys
   675 680 685
   Ile Glu Asn Ser Val Val Phe Arg Asn Lys Leu Phe Leu Trp Phe Glu
   690 695 700
   Gln Asp Arg Lys Asp Pro Tyr Thr Gln Leu Asp Ile Lys Ile Asn Glu
   705 710 715 720
   Ile Glu Asp Glu Thr Glu Ile Asp His Val Ile Pro Tyr Ser Lys Ser
   725 730 735
   Ala Asp Asp Ser Trp Phe Asn Lys Leu Leu Val Lys Lys Ser Thr Asn
   740 745 750
   Gln Leu Lys Lys Asn Lys Thr Val Trp Glu Tyr Tyr Gln Asn Glu Ser
   755 760 765
   Asp Pro Glu Ala Lys Trp Asn Lys Phe Val Ala Trp Ala Lys Arg Ile
   770 775 780
   Tyr Leu Val Gln Lys Ser Asp Lys Glu Ser Lys Asp Asn Ser Glu Lys
   785 790 795 800
   Asn Ser Ile Phe Lys Asn Lys Lys Pro Asn Leu Lys Phe Lys Asn Ile
   805 810 815
   Thr Lys Lys Leu Phe Asp Pro Tyr Lys Asp Leu Gly Phe Leu Ala Arg
   820 825 830
   Asn Leu Asn Asp Thr Arg Tyr Ala Thr Lys Val Phe Arg Asp Gln Leu
   835 840 845
   Asn Asn Tyr Ser Lys His His Ser Lys Asp Asp Glu Asn Lys Leu Phe
   850 855 860
   Lys Val Val Cys Met Asn Gly Ser Ile Thr Ser Phe Leu Arg Lys Ser
   865 870 875 880
   Met Trp Arg Lys Asn Glu Glu Gln Val Tyr Arg Phe Asn Phe Trp Lys
   885 890 895
   Lys Asp Arg Asp Gln Phe Phe His His Ala Val Asp Ala Ser Ile Ile
   900 905 910
   Ala Ile Phe Ser Leu Leu Thr Lys Thr Leu Tyr Asn Lys Leu Arg Val
   915 920 925
   Tyr Glu Ser Tyr Asp Val Gln Arg Arg Glu Asp Gly Val Tyr Leu Ile
   930 935 940
   Asn Lys Glu Thr Gly Glu Val Lys Lys Ala Asp Lys Asp Tyr Trp Lys
   945 950 955 960
   Asp Gln His Asn Phe Leu Lys Ile Arg Glu Asn Ala Ile Glu Ile Lys
   965 970 975
   Asn Val Leu Asn Asn Val Asp Phe Gln Asn Gln Val Arg Tyr Ser Arg
   980 985 990
   Lys Ala Asn Thr Lys Leu Asn Thr Gln Leu Phe Asn Glu Thr Leu Tyr
   995 1000 1005
   Gly Val Lys Glu Phe Glu Asn Asn Phe Tyr Lys Leu Glu Lys Val Asn
   1010 1015 1020
   Leu Phe Ser Arg Lys Asp Leu Arg Lys Phe Ile Leu Glu Asp Leu Asn
   1025 1030 1035 1040
   Glu Glu Ser Glu Lys Asn Lys Lys Asn Glu Asn Gly Ser Arg Lys Arg
   1045 1050 1055
   Ile Leu Thr Glu Lys Tyr Ile Val Asp Glu Ile Leu Gln Ile Leu Glu
   1060 1065 1070
   Asn Glu Glu Phe Lys Asp Ser Lys Ser Asp Ile Asn Ala Leu Asn Lys
   1075 1080 1085
   Tyr Met Asp Ser Leu Pro Ser Lys Phe Ser Glu Phe Phe Ser Gln Asp
   1090 1095 1100
   Phe Ile Asn Lys Cys Lys Lys Glu Asn Ser Leu Ile Leu Thr Phe Asp
   1105 1110 1115 1120
   Ala Ile Lys His Asn Asp Pro Lys Lys Val Ile Lys Ile Lys Asn Leu
   1125 1130 1135
   Lys Phe Phe Arg Glu Asp Ala Thr Leu Lys Asn Lys Gln Ala Val His
   1140 1145 1150
   Lys Asp Ser Lys Asn Gln Ile Lys Ser Phe Tyr Glu Ser Tyr Lys Cys
   1155 1160 1165
   Val Gly Phe Ile Trp Leu Lys Asn Lys Asn Asp Leu Glu Glu Ser Ile
   1170 1175 1180
   Phe Val Pro Ile Asn Ser Arg Val Ile His Phe Gly Asp Lys Asp Lys
   1185 1190 1195 1200
   Asp Ile Phe Asp Phe Asp Ser Tyr Asn Lys Glu Lys Leu Leu Asn Glu
   1205 1210 1215
   Ile Asn Leu Lys Arg Pro Glu Asn Lys Lys Phe Asn Ser Ile Asn Glu
   1220 1225 1230
   Ile Glu Phe Val Lys Phe Val Lys Pro Gly Ala Leu Leu Leu Asn Phe
   1235 1240 1245
   Glu Asn Gln Gln Ile Tyr Tyr Ile Ser Thr Leu Glu Ser Ser Ser Leu
   1250 1255 1260
   Arg Ala Lys Ile Lys Leu Leu Asn Lys Met Asp Lys Gly Lys Ala Val
   1265                1270                1275                1280
   Ser Met Lys Lys Ile Thr Asn Pro Asp Glu Tyr Lys Ile Ile Glu His
                   1285                1290                1295
   Val Asn Pro Leu Gly Ile Asn Leu Asn Trp Thr Lys Lys Leu Glu Asn
               1300                1305                1310
   Asn Asn
<210> 135
   <211> 1084
   <212> PRT
   <213> Corynebacterium diphtheriae
<220>
   <223> CRISPR-associated endonuclease
<308> NP_938445
   <309> 2013-06-10
<400> 135
   Met Lys Tyr His Val Gly Ile Asp Val Gly Thr Phe Ser Val Gly Leu
   1               5                   10                  15
   Ala Ala Ile Glu Val Asp Asp Ala Gly Met Pro Ile Lys Thr Leu Ser
               20                  25                  30
   Leu Val Ser His Ile His Asp Ser Gly Leu Asp Pro Asp Glu Ile Lys
           35                  40                  45
   Ser Ala Val Thr Arg Leu Ala Ser Ser Gly Ile Ala Arg Arg Thr Arg
       50                  55                  60
   Arg Leu Tyr Arg Arg Lys Arg Arg Arg Leu Gln Gln Leu Asp Lys Phe
   65                  70                  75                  80
   Ile Gln Arg Gln Gly Trp Pro Val Ile Glu Leu Glu Asp Tyr Ser Asp
                   85                  90                  95
   Pro Leu Tyr Pro Trp Lys Val Arg Ala Glu Leu Ala Ala Ser Tyr Ile
               100                 105                 110
   Ala Asp Glu Lys Glu Arg Gly Glu Lys Leu Ser Val Ala Leu Arg His
           115                 120                 125
   Ile Ala Arg His Arg Gly Trp Arg Asn Pro Tyr Ala Lys Val Ser Ser
       130                 135                 140
   Leu Tyr Leu Pro Asp Gly Pro Ser Asp Ala Phe Lys Ala Ile Arg Glu
   145                 150                 155                 160
   Glu Ile Lys Arg Ala Ser Gly Gln Pro Val Pro Glu Thr Ala Thr Val
                   165                 170                 175
   Gly Gln Met Val Thr Leu Cys Glu Leu Gly Thr Leu Lys Leu Arg Gly
               180                 185                 190
   Glu Gly Gly Val Leu Ser Ala Arg Leu Gln Gln Ser Asp Tyr Ala Arg
           195                 200                 205
   Glu Ile Gln Glu Ile Cys Arg Met Gln Glu Ile Gly Gln Glu Leu Tyr
       210                 215                 220
   Arg Lys Ile Ile Asp Val Val Phe Ala Ala Glu Ser Pro Lys Gly Ser
   225                 230                 235                 240
   Ala Ser Ser Arg Val Gly Lys Asp Pro Leu Gln Pro Gly Lys Asn Arg
                   245                 250                 255
   Ala Leu Lys Ala Ser Asp Ala Phe Gln Arg Tyr Arg Ile Ala Ala Leu
               260                 265                 270
   Ile Gly Asn Leu Arg Val Arg Val Asp Gly Glu Lys Arg Ile Leu Ser
           275                 280                 285
   Val Glu Glu Lys Asn Leu Val Phe Asp His Leu Val Asn Leu Thr Pro
       290                 295                 300
   Lys Lys Glu Pro Glu Trp Val Thr Ile Ala Glu Ile Leu Gly Ile Asp
   305                 310                 315                 320
   Arg Gly Gln Leu Ile Gly Thr Ala Thr Met Thr Asp Asp Gly Glu Arg
                   325                 330                 335
   Ala Gly Ala Arg Pro Pro Thr His Asp Thr Asn Arg Ser Ile Val Asn
               340                 345                 350
   Ser Arg Ile Ala Pro Leu Val Asp Trp Trp Lys Thr Ala Ser Ala Leu
           355                 360                 365
   Glu Gln His Ala Met Val Lys Ala Leu Ser Asn Ala Glu Val Asp Asp
       370                 375                 380
   Phe Asp Ser Pro Glu Gly Ala Lys Val Gln Ala Phe Phe Ala Asp Leu
   385                 390                 395                 400
   Asp Asp Asp Val His Ala Lys Leu Asp Ser Leu His Leu Pro Val Gly
                   405                 410                 415
   Arg Ala Ala Tyr Ser Glu Asp Thr Leu Val Arg Leu Thr Arg Arg Met
               420                 425                 430
   Leu Ser Asp Gly Val Asp Leu Tyr Thr Ala Arg Leu Gln Glu Phe Gly
           435                 440                 445
   Ile Glu Pro Ser Trp Thr Pro Pro Thr Pro Arg Ile Gly Glu Pro Val
       450                 455                 460
   Gly Asn Pro Ala Val Asp Arg Val Leu Lys Thr Val Ser Arg Trp Leu
   465                 470                 475                 480
   Glu Ser Ala Thr Lys Thr Trp Gly Ala Pro Glu Arg Val Ile Ile Glu
                   485                 490                 495
   His Val Arg Glu Gly Phe Val Thr Glu Lys Arg Ala Arg Glu Met Asp
               500                 505                 510
   Gly Asp Met Arg Arg Arg Ala Ala Arg Asn Ala Lys Leu Phe Gln Glu
           515                 520                 525
   Met Gln Glu Lys Leu Asn Val Gln Gly Lys Pro Ser Arg Ala Asp Leu
       530                 535                 540
   Trp Arg Tyr Gln Ser Val Gln Arg Gln Asn Cys Gln Cys Ala Tyr Cys
   545                 550                 555                 560
   Gly Ser Pro Ile Thr Phe Ser Asn Ser Glu Met Asp His Ile Val Pro
                   565                 570                 575
   Arg Ala Gly Gln Gly Ser Thr Asn Thr Arg Glu Asn Leu Val Ala Val
               580                 585                 590
   Cys His Arg Cys Asn Gln Ser Lys Gly Asn Thr Pro Phe Ala Ile Trp
           595                 600                 605
   Ala Lys Asn Thr Ser Ile Glu Gly Val Ser Val Lys Glu Ala Val Glu
       610                 615                 620
   Arg Thr Arg His Trp Val Thr Asp Thr Gly Met Arg Ser Thr Asp Phe
   625                 630                 635                 640
   Lys Lys Phe Thr Lys Ala Val Val Glu Arg Phe Gln Arg Ala Thr Met
                   645                 650                 655
   Asp Glu Glu Ile Asp Ala Arg Ser Met Glu Ser Val Ala Trp Met Ala
               660                 665                 670
   Asn Glu Leu Arg Ser Arg Val Ala Gln His Phe Ala Ser His Gly Thr
           675                 680                 685
   Thr Val Arg Val Tyr Arg Gly Ser Leu Thr Ala Glu Ala Arg Arg Ala
       690                 695                 700
   Ser Gly Ile Ser Gly Lys Leu Lys Phe Phe Asp Gly Val Gly Lys Ser
   705                 710                 715                 720
   Arg Leu Asp Arg Arg His His Ala Ile Asp Ala Ala Val Ile Ala Phe
                   725                 730                 735
   Thr Ser Asp Tyr Val Ala Glu Thr Leu Ala Val Arg Ser Asn Leu Lys
               740                 745                 750
   Gln Ser Gln Ala His Arg Gln Glu Ala Pro Gln Trp Arg Glu Phe Thr
           755                 760                 765
   Gly Lys Asp Ala Glu His Arg Ala Ala Trp Arg Val Trp Cys Gln Lys
       770                 775                 780
   Met Glu Lys Leu Ser Ala Leu Leu Thr Glu Asp Leu Arg Asp Asp Arg
   785                 790                 795                 800
   Val Val Val Met Ser Asn Val Arg Leu Arg Leu Gly Asn Gly Ser Ala
                   805                 810                 815
   His Lys Glu Thr Ile Gly Lys Leu Ser Lys Val Lys Leu Ser Ser Gln
               820                 825                 830
   Leu Ser Val Ser Asp Ile Asp Lys Ala Ser Ser Glu Ala Leu Trp Cys
           835                 840                 845
   Ala Leu Thr Arg Glu Pro Gly Phe Asp Pro Lys Glu Gly Leu Pro Ala
       850                 855                 860
   Asn Pro Glu Arg His Ile Arg Val Asn Gly Thr His Val Tyr Ala Gly
   865                 870                 875                 880
   Asp Asn Ile Gly Leu Phe Pro Val Ser Ala Gly Ser Ile Ala Leu Arg
                   885                 890                 895
   Gly Gly Tyr Ala Glu Leu Gly Ser Ser Phe His His Ala Arg Val Tyr
               900                 905                 910
   Lys Ile Thr Ser Gly Lys Lys Pro Ala Phe Ala Met Leu Arg Val Tyr
           915                 920                 925
   Thr Ile Asp Leu Leu Pro Tyr Arg Asn Gln Asp Leu Phe Ser Val Glu
       930                 935                 940
   Leu Lys Pro Gln Thr Met Ser Met Arg Gln Ala Glu Lys Lys Leu Arg
   945                 950                 955                 960
   Asp Ala Leu Ala Thr Gly Asn Ala Glu Tyr Leu Gly Trp Leu Val Val
                   965                 970                 975
   Asp Asp Glu Leu Val Val Asp Thr Ser Lys Ile Ala Thr Asp Gln Val
               980                 985                 990
   Lys Ala Val Glu Ala Glu Leu Gly Thr Ile Arg Arg Trp Arg Val Asp
           995                 1000                1005
   Gly Phe Phe Ser Pro Ser Lys Leu Arg Leu Arg Pro Leu Gln Met Ser
       1010                1015                1020
   Lys Glu Gly Ile Lys Lys Glu Ser Ala Pro Glu Leu Ser Lys Ile Ile
   1025                1030                1035                1040
   Asp Arg Pro Gly Trp Leu Pro Ala Val Asn Lys Leu Phe Ser Asp Gly
                   1045                1050                1055
   Asn Val Thr Val Val Arg Arg Asp Ser Leu Gly Arg Val Arg Leu Glu
               1060                1065                1070
   Ser Thr Ala His Leu Pro Val Thr Trp Lys Val Gln
           1075                1080
<210> 136
   <211> 1409
   <212> PRT
   <213> Wolinella succinogenes
<220>
   <223> CRISPR-associated endonuclease
<308> NP_907747
   <309> 2013-05-23
<400> 136
   Met Leu Val Ser Pro Ile Ser Val Asp Leu Gly Gly Lys Asn Thr Gly
    1               5                  10                  15
   Phe Phe Ser Phe Thr Asp Ser Leu Asp Asn Ser Gln Ser Gly Thr Val
               20                  25                  30
   Ile Tyr Asp Glu Ser Phe Val Leu Ser Gln Val Gly Arg Arg Ser Lys
           35                  40                  45
   Arg His Ser Lys Arg Asn Asn Leu Arg Asn Lys Leu Val Lys Arg Leu
       50                  55                  60
   Phe Leu Leu Ile Leu Gln Glu His His Gly Leu Ser Ile Asp Val Leu
   65                  70                  75                  80
   Pro Asp Glu Ile Arg Gly Leu Phe Asn Lys Arg Gly Tyr Thr Tyr Ala
                   85                  90                  95
   Gly Phe Glu Leu Asp Glu Lys Lys Lys Asp Ala Leu Glu Ser Asp Thr
               100                 105                 110
   Leu Lys Glu Phe Leu Ser Glu Lys Leu Gln Ser Ile Asp Arg Asp Ser
           115                 120                 125
   Asp Val Glu Asp Phe Leu Asn Gln Ile Ala Ser Asn Ala Glu Ser Phe
   130 135 140
   Lys Asp Tyr Lys Lys Gly Phe Glu Ala Val Phe Ala Ser Ala Thr His
   145 150 155 160
   Ser Pro Asn Lys Lys Leu Glu Leu Lys Asp Glu Leu Lys Ser Glu Tyr
   165 170 175
   Gly Glu Asn Ala Lys Glu Leu Leu Ala Gly Leu Arg Val Thr Lys Glu
   180 185 190
   Ile Leu Asp Glu Phe Asp Lys Gln Glu Asn Gln Gly Asn Leu Pro Arg
   195 200 205
   Ala Lys Tyr Phe Glu Glu Leu Gly Glu Tyr Ile Ala Thr Asn Glu Lys
   210 215 220
   Val Lys Ser Phe Phe Asp Ser Asn Ser Leu Lys Leu Thr Asp Met Thr
   225 230 235 240
   Lys Leu Ile Gly Asn Ile Ser Asn Tyr Gln Leu Lys Glu Leu Arg Arg
   245 250 255
   Tyr Phe Asn Asp Lys Glu Met Glu Lys Gly Asp Ile Trp Ile Pro Asn
   260 265 270
   Lys Leu His Lys Ile Thr Glu Arg Phe Val Arg Ser Trp His Pro Lys
   275 280 285
   Asn Asp Ala Asp Arg Gln Arg Arg Ala Glu Leu Met Lys Asp Leu Lys
   290 295 300
   Ser Lys Glu Ile Met Glu Leu Leu Thr Thr Thr Glu Pro Val Met Thr
   305 310 315 320
   Ile Pro Pro Tyr Asp Asp Met Asn Asn Arg Gly Ala Val Lys Cys Gln
   325 330 335
   Thr Leu Arg Leu Asn Glu Glu Tyr Leu Asp Lys His Leu Pro Asn Trp
   340 345 350
   Arg Asp Ile Ala Lys Arg Leu Asn His Gly Lys Phe Asn Asp Asp Leu
   355 360 365
   Ala Asp Ser Thr Val Lys Gly Tyr Ser Glu Asp Ser Thr Leu Leu His
   370 375 380
   Arg Leu Leu Asp Thr Ser Lys Glu Ile Asp Ile Tyr Glu Leu Arg Gly
   385 390 395 400
   Lys Lys Pro Asn Glu Leu Leu Val Lys Thr Leu Gly Gln Ser Asp Ala
   405 410 415
   Asn Arg Leu Tyr Gly Phe Ala Gln Asn Tyr Tyr Glu Leu Ile Arg Gln
   420 425 430
   Lys Val Arg Ala Gly Ile Trp Val Pro Val Lys Asn Lys Asp Asp Ser
   435 440 445
   Leu Asn Leu Glu Asp Asn Ser Asn Met Leu Lys Arg Cys Asn His Asn
   450 455 460
   Pro Pro His Lys Lys Asn Gln Ile His Asn Leu Val Ala Gly Ile Leu
   465 470 475 480
   Gly Val Lys Leu Asp Glu Ala Lys Phe Ala Glu Phe Glu Lys Glu Leu
   485 490 495
   Trp Ser Ala Lys Val Gly Asn Lys Lys Leu Ser Ala Tyr Cys Lys Asn
   500 505 510
   Ile Glu Glu Leu Arg Lys Thr His Gly Asn Thr Phe Lys Ile Asp Ile
   515 520 525
   Glu Glu Leu Arg Lys Lys Asp Pro Ala Glu Leu Ser Lys Glu Glu Lys
   530 535 540
   Ala Lys Leu Arg Leu Thr Asp Asp Val Ile Leu Asn Glu Trp Ser Gln
   545 550 555 560
   Lys Ile Ala Asn Phe Phe Asp Ile Asp Asp Lys His Arg Gln Arg Phe
   565 570 575
   Asn Asn Leu Phe Ser Met Ala Gln Leu His Thr Val Ile Asp Thr Pro
   580 585 590
   Arg Ser Gly Phe Ser Ser Thr Cys Lys Arg Cys Thr Ala Glu Asn Arg
   595 600 605
   Phe Arg Ser Glu Thr Ala Phe Tyr Asn Asp Glu Thr Gly Glu Phe His
   610 615 620
   Lys Lys Ala Thr Ala Thr Cys Gln Arg Leu Pro Ala Asp Thr Gln Arg
   625 630 635 640
   Pro Phe Ser Gly Lys Ile Glu Arg Tyr Ile Asp Lys Leu Gly Tyr Glu
   645 650 655
   Leu Ala Lys Ile Lys Ala Lys Glu Leu Glu Gly Met Glu Ala Lys Glu
   660 665 670
   Ile Lys Val Pro Ile Ile Leu Glu Gln Asn Ala Phe Glu Tyr Glu Glu
   675 680 685
   Ser Leu Arg Lys Ser Lys Thr Gly Ser Asn Asp Arg Val Ile Asn Ser
   690 695 700
   Lys Lys Asp Arg Asp Gly Lys Lys Leu Ala Lys Ala Lys Glu Asn Ala
   705 710 715 720
   Glu Asp Arg Leu Lys Asp Lys Asp Lys Arg Ile Lys Ala Phe Ser Ser
   725 730 735
   Gly Ile Cys Pro Tyr Cys Gly Asp Thr Ile Gly Asp Asp Gly Glu Ile
   740 745 750
   Asp His Ile Leu Pro Arg Ser His Thr Leu Lys Ile Tyr Gly Thr Val
   755 760 765
   Phe Asn Pro Glu Gly Asn Leu Ile Tyr Val His Gln Lys Cys Asn Gln
   770 775 780
   Ala Lys Ala Asp Ser Ile Tyr Lys Leu Ser Asp Ile Lys Ala Gly Val
   785 790 795 800
   Ser Ala Gln Trp Ile Glu Glu Gln Val Ala Asn Ile Lys Gly Tyr Lys
   805 810 815
   Thr Phe Ser Val Leu Ser Ala Glu Gln Gln Lys Ala Phe Arg Tyr Ala
   820 825 830
   Leu Phe Leu Gln Asn Asp Asn Glu Ala Tyr Lys Lys Val Val Asp Trp
   835 840 845
   Leu Arg Thr Asp Gln Ser Ala Arg Val Asn Gly Thr Gln Lys Tyr Leu
   850 855 860
   Ala Lys Lys Ile Gln Glu Lys Leu Thr Lys Met Leu Pro Asn Lys His
   865 870 875 880
   Leu Ser Phe Glu Phe Ile Leu Ala Asp Ala Thr Glu Val Ser Glu Leu
   885 890 895
   Arg Arg Gln Tyr Ala Arg Gln Asn Pro Leu Leu Ala Lys Ala Glu Lys
   900 905 910
   Gln Ala Pro Ser Ser His Ala Ile Asp Ala Val Met Ala Phe Val Ala
   915 920 925
   Arg Tyr Gln Lys Val Phe Lys Asp Gly Thr Pro Pro Asn Ala Asp Glu
   930 935 940
   Val Ala Lys Leu Ala Met Leu Asp Ser Trp Asn Pro Ala Ser Asn Glu
   945 950 955 960
   Pro Leu Thr Lys Gly Leu Ser Thr Asn Gln Lys Ile Glu Lys Met Ile
   965 970 975
   Lys Ser Gly Asp Tyr Gly Gln Lys Asn Met Arg Glu Val Phe Gly Lys
   980 985 990
   Ser Ile Phe Gly Glu Asn Ala Ile Gly Glu Arg Tyr Lys Pro Ile Val
   995 1000 1005
   Val Gln Glu Gly Gly Tyr Tyr Ile Gly Tyr Pro Ala Thr Val Lys Lys
   1010 1015 1020
   Gly Tyr Glu Leu Lys Asn Cys Lys Val Val Thr Ser Lys Asn Asp Ile
   1025 1030 1035 1040
   Ala Lys Leu Glu Lys Ile Ile Lys Asn Gln Asp Leu Ile Ser Leu Lys
   1045 1050 1055
   Glu Asn Gln Tyr Ile Lys Ile Phe Ser Ile Asn Lys Gln Thr Ile Ser
   1060 1065 1070
   Glu Leu Ser Asn Arg Tyr Phe Asn Met Asn Tyr Lys Asn Leu Val Glu
   1075 1080 1085
   Arg Asp Lys Glu Ile Val Gly Leu Leu Glu Phe Ile Val Glu Asn Cys
   1090 1095 1100
   Arg Tyr Tyr Thr Lys Lys Val Asp Val Lys Phe Ala Pro Lys Tyr Ile
   1105 1110 1115 1120
   His Glu Thr Lys Tyr Pro Phe Tyr Asp Asp Trp Arg Arg Phe Asp Glu
   1125 1130 1135
   Ala Trp Arg Tyr Leu Gln Glu Asn Gln Asn Lys Thr Ser Ser Lys Asp
                   1140                1145                1150
   Arg Phe Val Ile Asp Lys Ser Ser Leu Asn Glu Tyr Tyr Gln Pro Asp
               1155                1160                1165
   Lys Asn Glu Tyr Lys Leu Asp Val Asp Thr Gln Pro Ile Trp Asp Asp
           1170                1175                1180
   Phe Cys Arg Trp Tyr Phe Leu Asp Arg Tyr Lys Thr Ala Asn Asp Lys
   1185                1190                1195                1200
   Lys Ser Ile Arg Ile Lys Ala Arg Lys Thr Phe Ser Leu Leu Ala Glu
                   1205                1210                1215
   Ser Gly Val Gln Gly Lys Val Phe Arg Ala Lys Arg Lys Ile Pro Thr
               1220                1225                1230
   Gly Tyr Ala Tyr Gln Ala Leu Pro Met Asp Asn Asn Val Ile Ala Gly
           1235                1240                1245
   Asp Tyr Ala Asn Ile Leu Leu Glu Ala Asn Ser Lys Thr Leu Ser Leu
       1250                1255                1260
   Val Pro Lys Ser Gly Ile Ser Ile Glu Lys Gln Leu Asp Lys Lys Leu
   1265                1270                1275                1280
   Asp Val Ile Lys Lys Thr Asp Val Arg Gly Leu Ala Ile Asp Asn Asn
                   1285                1290                1295
   Ser Phe Phe Asn Ala Asp Phe Asp Thr His Gly Ile Arg Leu Ile Val
               1300                1305                1310
   Glu Asn Thr Ser Val Lys Val Gly Asn Phe Pro Ile Ser Ala Ile Asp
           1315                1320                1325
   Lys Ser Ala Lys Arg Met Ile Phe Arg Ala Leu Phe Glu Lys Glu Lys
       1330                1335                1340
   Gly Lys Arg Lys Lys Lys Thr Thr Ile Ser Phe Lys Glu Ser Gly Pro
   1345                1350                1355                1360
   Val Gln Asp Tyr Leu Lys Val Phe Leu Lys Lys Ile Val Lys Ile Gln
                   1365                1370                1375
   Leu Arg Thr Asp Gly Ser Ile Ser Asn Ile Val Val Arg Lys Asn Ala
               1380                1385                1390
   Ala Asp Phe Thr Leu Ser Phe Arg Ser Glu His Ile Gln Lys Leu Leu
           1395                1400                1405
   Lys
<210> 137
   <211> 1056
   <212> PRT
   <213> Pasteurella multocida
<220>
   <223> CRISPR-associated endonuclease
<308> NP_246064
   <309> 2013-06-27
<400> 137
   Met Gln Thr Thr Asn Leu Ser Tyr Ile Leu Gly Leu Asp Leu Gly Ile
    1               5                  10                  15
   Ala Ser Val Gly Trp Ala Val Val Glu Ile Asn Glu Asn Glu Asp Pro
               20                  25                  30
   Ile Gly Leu Ile Asp Val Gly Val Arg Ile Phe Glu Arg Ala Glu Val
           35                  40                  45
   Pro Lys Thr Gly Glu Ser Leu Ala Leu Ser Arg Arg Leu Ala Arg Ser
       50                  55                  60
   Thr Arg Arg Leu Ile Arg Arg Arg Ala His Arg Leu Leu Leu Ala Lys
   65                  70                  75                  80
   Arg Phe Leu Lys Arg Glu Gly Ile Leu Ser Thr Ile Asp Leu Glu Lys
                   85                  90                  95
   Gly Leu Pro Asn Gln Ala Trp Glu Leu Arg Val Ala Gly Leu Glu Arg
               100                 105                 110
   Arg Leu Ser Ala Ile Glu Trp Gly Ala Val Leu Leu His Leu Ile Lys
               115                 120                 125
   His Arg Gly Tyr Leu Ser Lys Arg Lys Asn Glu Ser Gln Thr Asn Asn
       130                 135                 140
   Lys Glu Leu Gly Ala Leu Leu Ser Gly Val Ala Gln Asn His Gln Leu
   145                 150                 155                 160
   Leu Gln Ser Asp Asp Tyr Arg Thr Pro Ala Glu Leu Ala Leu Lys Lys
                   165                 170                 175
   Phe Ala Lys Glu Glu Gly His Ile Arg Asn Gln Arg Gly Ala Tyr Thr
               180                 185                 190
   His Thr Phe Asn Arg Leu Asp Leu Leu Ala Glu Leu Asn Leu Leu Phe
           195                 200                 205
   Ala Gln Gln His Gln Phe Gly Asn Pro His Cys Lys Glu His Ile Gln
       210                 215                 220
   Gln Tyr Met Thr Glu Leu Leu Met Trp Gln Lys Pro Ala Leu Ser Gly
   225                 230                 235                 240
   Glu Ala Ile Leu Lys Met Leu Gly Lys Cys Thr His Glu Lys Asn Glu
                   245                 250                 255
   Phe Lys Ala Ala Lys His Thr Tyr Ser Ala Glu Arg Phe Val Trp Leu
               260                 265                 270
   Thr Lys Leu Asn Asn Leu Arg Ile Leu Glu Asp Gly Ala Glu Arg Ala
           275                 280                 285
   Leu Asn Glu Glu Glu Arg Gln Leu Leu Ile Asn His Pro Tyr Glu Lys
       290                 295                 300
   Ser Lys Leu Thr Tyr Ala Gln Val Arg Lys Leu Leu Gly Leu Ser Glu
   305                 310                 315                 320
   Gln Ala Ile Phe Lys His Leu Arg Tyr Ser Lys Glu Asn Ala Glu Ser
                   325                 330                 335
   Ala Thr Phe Met Glu Leu Lys Ala Trp His Ala Ile Arg Lys Ala Leu
               340                 345                 350
   Glu Asn Gln Gly Leu Lys Asp Thr Trp Gln Asp Leu Ala Lys Lys Pro
           355                 360                 365
   Asp Leu Leu Asp Glu Ile Gly Thr Ala Phe Ser Leu Tyr Lys Thr Asp
       370                 375                 380
   Glu Asp Ile Gln Gln Tyr Leu Thr Asn Lys Val Pro Asn Ser Val Ile
   385                 390                 395                 400
   Asn Ala Leu Leu Val Ser Leu Asn Phe Asp Lys Phe Ile Glu Leu Ser
                   405                 410                 415
   Leu Lys Ser Leu Arg Lys Ile Leu Pro Leu Met Glu Gln Gly Lys Arg
               420                 425                 430
   Tyr Asp Gln Ala Cys Arg Glu Ile Tyr Gly His His Tyr Gly Glu Ala
           435                 440                 445
   Asn Gln Lys Thr Ser Gln Leu Leu Pro Ala Ile Pro Ala Gln Glu Ile
       450                 455                 460
   Arg Asn Pro Val Val Leu Arg Thr Leu Ser Gln Ala Arg Lys Val Ile
   465                 470                 475                 480
   Asn Ala Ile Ile Arg Gln Tyr Gly Ser Pro Ala Arg Val His Ile Glu
                   485                 490                 495
   Thr Gly Arg Glu Leu Gly Lys Ser Phe Lys Glu Arg Arg Glu Ile Gln
               500                 505                 510
   Lys Gln Gln Glu Asp Asn Arg Thr Lys Arg Glu Ser Ala Val Gln Lys
           515                 520                 525
   Phe Lys Glu Leu Phe Ser Asp Phe Ser Ser Glu Pro Lys Ser Lys Asp
       530                 535                 540
   Ile Leu Lys Phe Arg Leu Tyr Glu Gln Gln His Gly Lys Cys Leu Tyr
   545                 550                 555                 560
   Ser Gly Lys Glu Ile Asn Ile His Arg Leu Asn Glu Lys Gly Tyr Val
                   565                 570                 575
   Glu Ile Asp His Ala Leu Pro Phe Ser Arg Thr Trp Asp Asp Ser Phe
               580                 585                 590
   Asn Asn Lys Val Leu Val Leu Ala Ser Glu Asn Gln Asn Lys Gly Asn
           595                 600                 605
   Gln Thr Pro Tyr Glu Trp Leu Gln Gly Lys Ile Asn Ser Glu Arg Trp
       610                 615                 620
   Lys Asn Phe Val Ala Leu Val Leu Gly Ser Gln Cys Ser Ala Ala Lys
   625                 630                 635                 640
   Lys Gln Arg Leu Leu Thr Gln Val Ile Asp Asp Asn Lys Phe Ile Asp
                   645                 650                 655
   Arg Asn Leu Asn Asp Thr Arg Tyr Ile Ala Arg Phe Leu Ser Asn Tyr
               660                 665                 670
   Ile Gln Glu Asn Leu Leu Leu Val Gly Lys Asn Lys Lys Asn Val Phe
           675                 680                 685
   Thr Pro Asn Gly Gln Ile Thr Ala Leu Leu Arg Ser Arg Trp Gly Leu
       690                 695                 700
   Ile Lys Ala Arg Glu Asn Asn Asn Arg His His Ala Leu Asp Ala Ile
   705                 710                 715                 720
   Val Val Ala Cys Ala Thr Pro Ser Met Gln Gln Lys Ile Thr Arg Phe
                   725                 730                 735
   Ile Arg Phe Lys Glu Val His Pro Tyr Lys Ile Glu Asn Arg Tyr Glu
               740                 745                 750
   Met Val Asp Gln Glu Ser Gly Glu Ile Ile Ser Pro His Phe Pro Glu
           755                 760                 765
   Pro Trp Ala Tyr Phe Arg Gln Glu Val Asn Ile Arg Val Phe Asp Asn
       770                 775                 780
   His Pro Asp Thr Val Leu Lys Glu Met Leu Pro Asp Arg Pro Gln Ala
   785                 790                 795                 800
   Asn His Gln Phe Val Gln Pro Leu Phe Val Ser Arg Ala Pro Thr Arg
                   805                 810                 815
   Lys Met Ser Gly Gln Gly His Met Glu Thr Ile Lys Ser Ala Lys Arg
               820                 825                 830
   Leu Ala Glu Gly Ile Ser Val Leu Arg Ile Pro Leu Thr Gln Leu Lys
           835                 840                 845
   Pro Asn Leu Leu Glu Asn Met Val Asn Lys Glu Arg Glu Pro Ala Leu
       850                 855                 860
   Tyr Ala Gly Leu Lys Ala Arg Leu Ala Glu Phe Asn Gln Asp Pro Ala
   865                 870                 875                 880
   Lys Ala Phe Ala Thr Pro Phe Tyr Lys Gln Gly Gly Gln Gln Val Lys
                   885                 890                 895
   Ala Ile Arg Val Glu Gln Val Gln Lys Ser Gly Val Leu Val Arg Glu
               900                 905                 910
   Asn Asn Gly Val Ala Asp Asn Ala Ser Ile Val Arg Thr Asp Val Phe
           915                 920                 925
   Ile Lys Asn Asn Lys Phe Phe Leu Val Pro Ile Tyr Thr Trp Gln Val
       930                 935                 940
   Ala Lys Gly Ile Leu Pro Asn Lys Ala Ile Val Ala His Lys Asn Glu
   945                 950                 955                 960
   Asp Glu Trp Glu Glu Met Asp Glu Gly Ala Lys Phe Lys Phe Ser Leu
                   965                 970                 975
   Phe Pro Asn Asp Leu Val Glu Leu Lys Thr Lys Lys Glu Tyr Phe Phe
               980                 985                 990
   Gly Tyr Tyr Ile Gly Leu Asp Arg Ala Thr Gly Asn Ile Ser Leu Lys
           995                 1000                1005
   Glu His Asp Gly Glu Ile Ser Lys Gly Lys Asp Gly Val Tyr Arg Val
       1010                1015                1020
   Gly Val Lys Leu Ala Leu Ser Phe Glu Lys Tyr Gln Val Asp Glu Leu
   1025                1030                1035                1040
   Gly Lys Asn Arg Gln Ile Cys Arg Pro Gln Gln Arg Gln Pro Val Arg
                   1045                1050                1055
<210> 138
   <211> 1368
   <212> PRT
   <213> Streptococcus pyogenes
<220>
   <223> CRISPR-associated endonuclease
<308> NP_802438
   <309> 2013-06-10
<400> 138
   Met Asp Lys Lys Tyr Ser Ile Gly Leu Asp Ile Gly Thr Asn Ser Val
   1               5                   10                  15
   Gly Trp Ala Val Ile Thr Asp Asp Tyr Lys Val Pro Ser Lys Lys Leu
               20                  25                  30
   Lys Gly Leu Gly Asn Thr Asp Arg His Gly Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Ala Asp
       130                 135                 140
   Ser Thr Asp Lys Val Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Arg Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Thr Leu Leu Ser Asp
       290                 295                 300
   Ile Leu Arg Val Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Ala Lys Leu Asn Arg Glu Asp Leu Leu Arg
   385                 390                 395                 400
   Lys Gln Arg Thr Phe Asp Asn Gly Ser Ile Pro Tyr Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Asp Ile Leu Lys Glu Tyr Pro
   785                 790                 795                 800
   Val Glu Asn Thr Gln Leu Gln Asn Glu Lys Leu Tyr Leu Tyr Tyr Leu
                   805                 810                 815
   Gln Asn Gly Arg Asp Met Tyr Val Asp Gln Glu Leu Asp Ile Asn Arg
               820                 825                 830
   Leu Ser Asp Tyr Asp Val Asp His Ile Val Pro Gln Ser Phe Leu Lys
           835                 840                 845
   Asp Asp Ser Ile Asp Asn Lys Val Leu Thr Arg Ser Asp Lys Asn Arg
       850                 855                 860
   Gly Lys Ser Asp Asn Val Pro Ser Glu Glu Val Val Lys Lys Met Lys
   865                 870                 875                 880
   Asn Tyr Trp Arg Gln Leu Leu Asn Ala Lys Leu Ile Thr Gln Arg Lys
                   885                 890                 895
   Phe Asp Asn Leu Thr Lys Ala Glu Arg Gly Gly Leu Ser Glu Leu Asp
               900                 905                 910
   Lys Val Gly Phe Ile Lys Arg Gln Leu Val Glu Thr Arg Gln Ile Thr
           915                 920                 925
   Lys His Val Ala Gln Ile Leu Asp Ser Arg Met Asn Thr Lys Tyr Asp
       930                 935                 940
   Glu Asn Asp Lys Leu Ile Arg Glu Val Arg Val Ile Thr Leu Lys Ser
   945                 950                 955                 960
   Lys Leu Val Ser Asp Phe Arg Lys Asp Phe Gln Phe Tyr Lys Val Arg
                   965                 970                 975
   Glu Ile Asn Asn Tyr His His Ala His Asp Ala Tyr Leu Asn Ala Val
               980                 985                 990
   Val Gly Thr Ala Leu Ile Lys Lys Tyr Pro Lys Leu Glu Ser Glu Phe
           995                 1000                1005
   Val Tyr Gly Asp Tyr Lys Val Tyr Asp Val Arg Lys Met Ile Ala Lys
       1010                1015                1020
   Ser Glu Gln Glu Ile Gly Lys Ala Thr Ala Lys Tyr Phe Phe Tyr Ser
   1025                1030                1035                1040
   Asn Ile Met Asn Phe Phe Lys Thr Glu Ile Thr Leu Ala Asn Gly Glu
                   1045                1050                1055
   Ile Arg Lys Arg Pro Leu Ile Glu Thr Asn Gly Glu Thr Gly Glu Ile
               1060                1065                1070
   Val Trp Asp Lys Gly Arg Asp Phe Ala Thr Val Arg Lys Val Leu Ser
           1075                1080                1085
   Met Pro Gln Val Asn Ile Val Lys Lys Thr Glu Val Gln Thr Gly Gly
       1090                1095                1100
   Phe Ser Lys Glu Ser Ile Leu Pro Lys Arg Asn Ser Asp Lys Leu Ile
   1105                1110                1115                1120
   Ala Arg Lys Lys Asp Trp Asp Pro Lys Lys Tyr Gly Gly Phe Asp Ser
                   1125                1130                1135
   Pro Thr Val Ala Tyr Ser Val Leu Val Val Ala Lys Val Glu Lys Gly
               1140                1145                1150
   Lys Ser Lys Lys Leu Lys Ser Val Lys Glu Leu Leu Gly Ile Thr Ile
           1155                1160                1165
   Met Glu Arg Ser Ser Phe Glu Lys Asp Pro Ile Asp Phe Leu Glu Ala
       1170                1175                1180
   Lys Gly Tyr Lys Glu Val Arg Lys Asp Leu Ile Ile Lys Leu Pro Lys
   1185                1190                1195                1200
   Tyr Ser Leu Phe Glu Leu Glu Asn Gly Arg Lys Arg Met Leu Ala Ser
                   1205                1210                1215
   Ala Gly Glu Leu Gln Lys Gly Asn Glu Leu Ala Leu Pro Ser Lys Tyr
               1220                1225                1230
   Val Asn Phe Leu Tyr Leu Ala Ser His Tyr Glu Lys Leu Lys Gly Ser
           1235                1240                1245
   Pro Glu Asp Asn Glu Gln Lys Gln Leu Phe Val Glu Gln His Lys His
       1250                1255                1260
   Tyr Leu Asp Glu Ile Ile Glu Gln Ile Ser Glu Phe Ser Lys Arg Val
   1265                1270                1275                1280
   Ile Leu Ala Asp Ala Asn Leu Asp Lys Val Leu Ser Ala Tyr Asn Lys
                   1285                1290                1295
   His Arg Asp Lys Pro Ile Arg Glu Gln Ala Glu Asn Ile Ile His Leu
               1300                1305                1310
   Phe Thr Leu Thr Asn Leu Gly Ala Pro Ala Ala Phe Lys Tyr Phe Asp
           1315                1320                1325
   Thr Thr Ile Asp Arg Lys Arg Tyr Thr Ser Thr Lys Glu Val Leu Asp
       1330                1335                1340
   Ala Thr Leu Ile His Gln Ser Ile Thr Gly Leu Tyr Glu Thr Arg Ile
   1345                1350                1355                1360
   Asp Leu Ser Gln Leu Gly Gly Asp
                   1365
<210> 139
   <211> 1345
   <212> PRT
   <213> Streptococcus mutans
<220>
   <223> CRISPR-associated endonuclease
<308> NP_721764
   <309> 2013-06-27
<400> 139
   Met Lys Lys Pro Tyr Ser Ile Gly Leu Asp Ile Gly Thr Asn Ser Val
   1               5                   10                  15
   Gly Trp Ala Val Val Thr Asp Asp Tyr Lys Val Pro Ala Lys Lys Met
               20                  25                  30
   Lys Val Leu Gly Asn Thr Asp Lys Ser His Ile Glu Lys Asn Leu Leu
           35                  40                  45
   Gly Ala Leu Leu Phe Asp Ser Gly Asn Thr Ala Glu Asp Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ser Glu Glu Met Gly Lys Val Asp Asp Ser
                   85                  90                  95
   Phe Phe His Arg Leu Glu Asp Ser Phe Leu Val Thr Glu Asp Lys Arg
               100                 105                 110
   Gly Glu Arg His Pro Ile Phe Gly Asn Leu Glu Glu Glu Val Lys Tyr
           115                 120                 125
   His Glu Asn Phe Pro Thr Ile Tyr His Leu Arg Gln Tyr Leu Ala Asp
       130                 135                 140
   Asn Pro Glu Lys Val Asp Leu Arg Leu Val Tyr Leu Ala Leu Ala His
   145                 150                 155                 160
   Ile Ile Lys Phe Arg Gly His Phe Leu Ile Glu Gly Lys Phe Asp Thr
                   165                 170                 175
   Arg Asn Asn Asp Val Gln Arg Leu Phe Gln Glu Phe Leu Ala Val Tyr
               180                 185                 190
   Asp Asn Thr Phe Glu Asn Ser Ser Leu Gln Glu Gln Asn Val Gln Val
           195                 200                 205
   Glu Glu Ile Leu Thr Asp Lys Ile Ser Lys Ser Ala Lys Lys Asp Arg
       210                 215                 220
   Val Leu Lys Leu Phe Pro Asn Glu Lys Ser Asn Gly Arg Phe Ala Glu
   225                 230                 235                 240
   Phe Leu Lys Leu Ile Val Gly Asn Gln Ala Asp Phe Lys Lys His Phe
                   245                 250                 255
   Glu Leu Glu Glu Lys Ala Pro Leu Gln Phe Ser Lys Asp Thr Tyr Glu
               260                 265                 270
   Glu Glu Leu Glu Val Leu Leu Ala Gln Ile Gly Asp Asn Tyr Ala Glu
           275                 280                 285
   Leu Phe Leu Ser Ala Lys Lys Leu Tyr Asp Ser Ile Leu Leu Ser Gly
       290                 295                 300
   Ile Leu Thr Val Thr Asp Val Gly Thr Lys Ala Pro Leu Ser Ala Ser
   305                 310                 315                 320
   Met Ile Gln Arg Tyr Asn Glu His Gln Met Asp Leu Ala Gln Leu Lys
                   325                 330                 335
   Gln Phe Ile Arg Gln Lys Leu Ser Asp Lys Tyr Asn Glu Val Phe Ser
               340                 345                 350
   Asp Val Ser Lys Asp Gly Tyr Ala Gly Tyr Ile Asp Gly Lys Thr Asn
           355                 360                 365
   Gln Glu Ala Phe Tyr Lys Tyr Leu Lys Gly Leu Leu Asn Lys Ile Glu
       370                 375                 380
   Gly Ser Gly Tyr Phe Leu Asp Lys Ile Glu Arg Glu Asp Phe Leu Arg
   385                 390                 395                 400
   Lys Gln Arg Thr Phe Asp Asn Gly Ser Ile Pro His Gln Ile His Leu
                   405                 410                 415
   Gln Glu Met Arg Ala Ile Ile Arg Arg Gln Ala Glu Phe Tyr Pro Phe
               420                 425                 430
   Leu Ala Asp Asn Gln Asp Arg Ile Glu Lys Leu Leu Thr Phe Arg Ile
           435                 440                 445
   Pro Tyr Tyr Val Gly Pro Leu Ala Arg Gly Lys Ser Asp Phe Ala Trp
       450                 455                 460
   Leu Ser Arg Lys Ser Ala Asp Lys Ile Thr Pro Trp Asn Phe Asp Glu
   465                 470                 475                 480
   Ile Val Asp Lys Glu Ser Ser Ala Glu Ala Phe Ile Asn Arg Met Thr
   485 490 495
   Asn Tyr Asp Leu Tyr Leu Pro Asn Gln Lys Val Leu Pro Lys His Ser
   500 505 510
   Leu Leu Tyr Glu Lys Phe Thr Val Tyr Asn Glu Leu Thr Lys Val Lys
   515 520 525
   Tyr Lys Thr Glu Gln Gly Lys Thr Ala Phe Phe Asp Ala Asn Met Lys
   530 535 540
   Gln Glu Ile Phe Asp Gly Val Phe Lys Val Tyr Arg Lys Val Thr Lys
   545 550 555 560
   Asp Lys Leu Met Asp Phe Leu Glu Lys Glu Phe Asp Glu Phe Arg Ile
   565 570 575
   Val Asp Leu Thr Gly Leu Asp Lys Glu Asn Lys Val Phe Asn Ala Ser
   580 585 590
   Tyr Gly Thr Tyr His Asp Leu Cys Lys Ile Leu Asp Lys Asp Phe Leu
   595 600 605
   Asp Asn Ser Lys Asn Glu Lys Ile Leu Glu Asp Ile Val Leu Thr Leu
   610 615 620
   Thr Leu Phe Glu Asp Arg Glu Met Ile Arg Lys Arg Leu Glu Asn Tyr
   625 630 635 640
   Ser Asp Leu Leu Thr Lys Glu Gln Val Lys Lys Leu Glu Arg Arg His
   645 650 655
   Tyr Thr Gly Trp Gly Arg Leu Ser Ala Glu Leu Ile His Gly Ile Arg
   660 665 670
   Asn Lys Glu Ser Arg Lys Thr Ile Leu Asp Tyr Leu Ile Asp Asp Gly
   675 680 685
   Asn Ser Asn Arg Asn Phe Met Gln Leu Ile Asn Asp Asp Ala Leu Ser
   690 695 700
   Phe Lys Glu Glu Ile Ala Lys Ala Gln Val Ile Gly Glu Thr Asp Asn
   705 710 715 720
   Leu Asn Gln Val Val Ser Asp Ile Ala Gly Ser Pro Ala Ile Lys Lys
   725 730 735
   Gly Ile Leu Gln Ser Leu Lys Ile Val Asp Glu Leu Val Lys Ile Met
   740 745 750
   Gly His Gln Pro Glu Asn Ile Val Val Glu Met Ala Arg Glu Asn Gln
   755 760 765
   Phe Thr Asn Gln Gly Arg Arg Asn Ser Gln Gln Arg Leu Lys Gly Leu
   770 775 780
   Thr Asp Ser Ile Lys Glu Phe Gly Ser Gln Ile Leu Lys Glu His Pro
   785 790 795 800
   Val Glu Asn Ser Gln Leu Gln Asn Asp Arg Leu Phe Leu Tyr Tyr Leu
   805 810 815
   Gln Asn Gly Arg Asp Met Tyr Thr Gly Glu Glu Leu Asp Ile Asp Tyr
   820 825 830
   Leu Ser Gln Tyr Asp Ile Asp His Ile Ile Pro Gln Ala Phe Ile Lys
   835 840 845
   Asp Asn Ser Ile Asp Asn Arg Val Leu Thr Ser Ser Lys Glu Asn Arg
   850 855 860
   Gly Lys Ser Asp Asp Val Pro Ser Lys Asp Val Val Arg Lys Met Lys
   865 870 875 880
   Ser Tyr Trp Ser Lys Leu Leu Ser Ala Lys Leu Ile Thr Gln Arg Lys
   885 890 895
   Phe Asp Asn Leu Thr Lys Ala Glu Arg Gly Gly Leu Thr Asp Asp Asp
   900 905 910
   Lys Ala Gly Phe Ile Lys Arg Gln Leu Val Glu Thr Arg Gln Ile Thr
   915 920 925
   Lys His Val Ala Arg Ile Leu Asp Glu Arg Phe Asn Thr Glu Thr Asp
   930 935 940
   Glu Asn Asn Lys Lys Ile Arg Gln Val Lys Ile Val Thr Leu Lys Ser
   945 950 955 960
   Asn Leu Val Ser Asn Phe Arg Lys Glu Phe Glu Leu Tyr Lys Val Arg
   965 970 975
   Glu Ile Asn Asp Tyr His His Ala His Asp Ala Tyr Leu Asn Ala Val
               980                 985                 990
   Ile Gly Lys Ala Leu Leu Gly Val Tyr Pro Gln Leu Glu Pro Glu Phe
           995                 1000                1005
   Val Tyr Gly Asp Tyr Pro His Phe His Gly His Lys Glu Asn Lys Ala
       1010                1015                1020
   Thr Ala Lys Lys Phe Phe Tyr Ser Asn Ile Met Asn Phe Phe Lys Lys
   1025                1030                1035                1040
   Asp Asp Val Arg Thr Asp Lys Asn Gly Glu Ile Ile Trp Lys Lys Asp
                   1045                1050                1055
   Glu His Ile Ser Asn Ile Lys Lys Val Leu Ser Tyr Pro Gln Val Asn
               1060                1065                1070
   Ile Val Lys Lys Val Glu Glu Gln Thr Gly Gly Phe Ser Lys Glu Ser
           1075                1080                1085
   Ile Leu Pro Lys Gly Asn Ser Asp Lys Leu Ile Pro Arg Lys Thr Lys
       1090                1095                1100
   Lys Phe Tyr Trp Asp Thr Lys Lys Tyr Gly Gly Phe Asp Ser Pro Ile
   1105                1110                1115                1120
   Val Ala Tyr Ser Ile Leu Val Ile Ala Asp Ile Glu Lys Gly Lys Ser
                   1125                1130                1135
   Lys Lys Leu Lys Thr Val Lys Ala Leu Val Gly Val Thr Ile Met Glu
               1140                1145                1150
   Lys Met Thr Phe Glu Arg Asp Pro Val Ala Phe Leu Glu Arg Lys Gly
           1155                1160                1165
   Tyr Arg Asn Val Gln Glu Glu Asn Ile Ile Lys Leu Pro Lys Tyr Ser
       1170                1175                1180
   Leu Phe Lys Leu Glu Asn Gly Arg Lys Arg Leu Leu Ala Ser Ala Arg
   1185                1190                1195                1200
   Glu Leu Gln Lys Gly Asn Glu Ile Val Leu Pro Asn His Leu Gly Thr
                   1205                1210                1215
   Leu Leu Tyr His Ala Lys Asn Ile His Lys Val Asp Glu Pro Lys His
               1220                1225                1230
   Leu Asp Tyr Val Asp Lys His Lys Asp Glu Phe Lys Glu Leu Leu Asp
           1235                1240                1245
   Val Val Ser Asn Phe Ser Lys Lys Tyr Thr Leu Ala Glu Gly Asn Leu
       1250                1255                1260
   Glu Lys Ile Lys Glu Leu Tyr Ala Gln Asn Asn Gly Glu Asp Leu Lys
   1265                1270                1275                1280
   Glu Leu Ala Ser Ser Phe Ile Asn Leu Leu Thr Phe Thr Ala Ile Gly
                   1285                1290                1295
   Ala Pro Ala Thr Phe Lys Phe Phe Asp Lys Asn Ile Asp Arg Lys Arg
               1300                1305                1310
   Tyr Thr Ser Thr Thr Glu Ile Leu Asn Ala Thr Leu Ile His Gln Ser
           1315                1320                1325
   Ile Thr Gly Leu Tyr Glu Thr Arg Ile Asp Leu Asn Lys Leu Gly Gly
       1330                1335                1340
   Asp
   1345
<210> 140
   <211> 1334
   <212> PRT
   <213> Listeria innocua
<220>
   <223> CRISPR-associated endonuclease
<308> NP_472073
   <309> 2013-06-27
<400> 140
   Met Lys Lys Pro Tyr Thr Ile Gly Leu Asp Ile Gly Thr Asn Ser Val
   1               5                   10                  15
   Gly Trp Ala Val Leu Thr Asp Gln Tyr Asp Leu Val Lys Arg Lys Met
               20                  25                  30
   Lys Ile Ala Gly Asp Ser Glu Lys Lys Gln Ile Lys Lys Asn Phe Trp
           35                  40                  45
   Gly Val Arg Leu Phe Asp Glu Gly Gln Thr Ala Ala Asp Arg Arg Met
       50                  55                  60
   Ala Arg Thr Ala Arg Arg Arg Ile Glu Arg Arg Arg Asn Arg Ile Ser
   65                  70                  75                  80
   Tyr Leu Gln Gly Ile Phe Ala Glu Glu Met Ser Lys Thr Asp Ala Asn
                   85                  90                  95
   Phe Phe Cys Arg Leu Ser Asp Ser Phe Tyr Val Asp Asn Glu Lys Arg
               100                 105                 110
   Asn Ser Arg His Pro Phe Phe Ala Thr Ile Glu Glu Glu Val Glu Tyr
           115                 120                 125
   His Lys Asn Tyr Pro Thr Ile Tyr His Leu Arg Glu Glu Leu Val Asn
       130                 135                 140
   Ser Ser Glu Lys Ala Asp Leu Arg Leu Val Tyr Leu Ala Leu Ala His
   145                 150                 155                 160
   Ile Ile Lys Tyr Arg Gly Asn Phe Leu Ile Glu Gly Ala Leu Asp Thr
                   165                 170                 175
   Gln Asn Thr Ser Val Asp Gly Ile Tyr Lys Gln Phe Ile Gln Thr Tyr
               180                 185                 190
   Asn Gln Val Phe Ala Ser Gly Ile Glu Asp Gly Ser Leu Lys Lys Leu
           195                 200                 205
   Glu Asp Asn Lys Asp Val Ala Lys Ile Leu Val Glu Lys Val Thr Arg
       210                 215                 220
   Lys Glu Lys Leu Glu Arg Ile Leu Lys Leu Tyr Pro Gly Glu Lys Ser
   225                 230                 235                 240
   Ala Gly Met Phe Ala Gln Phe Ile Ser Leu Ile Val Gly Ser Lys Gly
                   245                 250                 255
   Asn Phe Gln Lys Pro Phe Asp Leu Ile Glu Lys Ser Asp Ile Glu Cys
               260                 265                 270
   Ala Lys Asp Ser Tyr Glu Glu Asp Leu Glu Ser Leu Leu Ala Leu Ile
           275                 280                 285
   Gly Asp Glu Tyr Ala Glu Leu Phe Val Ala Ala Lys Asn Ala Tyr Ser
       290                 295                 300
   Ala Val Val Leu Ser Ser Ile Ile Thr Val Ala Glu Thr Glu Thr Asn
   305                 310                 315                 320
   Ala Lys Leu Ser Ala Ser Met Ile Glu Arg Phe Asp Thr His Glu Glu
                   325                 330                 335
   Asp Leu Gly Glu Leu Lys Ala Phe Ile Lys Leu His Leu Pro Lys His
               340                 345                 350
   Tyr Glu Glu Ile Phe Ser Asn Thr Glu Lys His Gly Tyr Ala Gly Tyr
           355                 360                 365
   Ile Asp Gly Lys Thr Lys Gln Ala Asp Phe Tyr Lys Tyr Met Lys Met
       370                 375                 380
   Thr Leu Glu Asn Ile Glu Gly Ala Asp Tyr Phe Ile Ala Lys Ile Glu
   385                 390                 395                 400
   Lys Glu Asn Phe Leu Arg Lys Gln Arg Thr Phe Asp Asn Gly Ala Ile
                   405                 410                 415
   Pro His Gln Leu His Leu Glu Glu Leu Glu Ala Ile Leu His Gln Gln
               420                 425                 430
   Ala Lys Tyr Tyr Pro Phe Leu Lys Glu Asn Tyr Asp Lys Ile Lys Ser
           435                 440                 445
   Leu Val Thr Phe Arg Ile Pro Tyr Phe Val Gly Pro Leu Ala Asn Gly
       450                 455                 460
   Gln Ser Glu Phe Ala Trp Leu Thr Arg Lys Ala Asp Gly Glu Ile Arg
   465                 470                 475                 480
   Pro Trp Asn Ile Glu Glu Lys Val Asp Phe Gly Lys Ser Ala Val Asp
                   485                 490                 495
   Phe Ile Glu Lys Met Thr Asn Lys Asp Thr Tyr Leu Pro Lys Glu Asn
               500                 505                 510
   Val Leu Pro Lys His Ser Leu Cys Tyr Gln Lys Tyr Leu Val Tyr Asn
               515                 520                 525
   Glu Leu Thr Lys Val Arg Tyr Ile Asn Asp Gln Gly Lys Thr Ser Tyr
       530                 535                 540
   Phe Ser Gly Gln Glu Lys Glu Gln Ile Phe Asn Asp Leu Phe Lys Gln
   545                 550                 555                 560
   Lys Arg Lys Val Lys Lys Lys Asp Leu Glu Leu Phe Leu Arg Asn Met
                   565                 570                 575
   Ser His Val Glu Ser Pro Thr Ile Glu Gly Leu Glu Asp Ser Phe Asn
               580                 585                 590
   Ser Ser Tyr Ser Thr Tyr His Asp Leu Leu Lys Val Gly Ile Lys Gln
           595                 600                 605
   Glu Ile Leu Asp Asn Pro Val Asn Thr Glu Met Leu Glu Asn Ile Val
       610                 615                 620
   Lys Ile Leu Thr Val Phe Glu Asp Lys Arg Met Ile Lys Glu Gln Leu
   625                 630                 635                 640
   Gln Gln Phe Ser Asp Val Leu Asp Gly Val Val Leu Lys Lys Leu Glu
                   645                 650                 655
   Arg Arg His Tyr Thr Gly Trp Gly Arg Leu Ser Ala Lys Leu Leu Met
               660                 665                 670
   Gly Ile Arg Asp Lys Gln Ser His Leu Thr Ile Leu Asp Tyr Leu Met
           675                 680                 685
   Asn Asp Asp Gly Leu Asn Arg Asn Leu Met Gln Leu Ile Asn Asp Ser
       690                 695                 700
   Asn Leu Ser Phe Lys Ser Ile Ile Glu Lys Glu Gln Val Thr Thr Ala
   705                 710                 715                 720
   Asp Lys Asp Ile Gln Ser Ile Val Ala Asp Leu Ala Gly Ser Pro Ala
                   725                 730                 735
   Ile Lys Lys Gly Ile Leu Gln Ser Leu Lys Ile Val Asp Glu Leu Val
               740                 745                 750
   Ser Val Met Gly Tyr Pro Pro Gln Thr Ile Val Val Glu Met Ala Arg
           755                 760                 765
   Glu Asn Gln Thr Thr Gly Lys Gly Lys Asn Asn Ser Arg Pro Arg Tyr
       770                 775                 780
   Lys Ser Leu Glu Lys Ala Ile Lys Glu Phe Gly Ser Gln Ile Leu Lys
   785                 790                 795                 800
   Glu His Pro Thr Asp Asn Gln Glu Leu Arg Asn Asn Arg Leu Tyr Leu
                   805                 810                 815
   Tyr Tyr Leu Gln Asn Gly Lys Asp Met Tyr Thr Gly Gln Asp Leu Asp
               820                 825                 830
   Ile His Asn Leu Ser Asn Tyr Asp Ile Asp His Ile Val Pro Gln Ser
           835                 840                 845
   Phe Ile Thr Asp Asn Ser Ile Asp Asn Leu Val Leu Thr Ser Ser Ala
       850                 855                 860
   Gly Asn Arg Glu Lys Gly Asp Asp Val Pro Pro Leu Glu Ile Val Arg
   865                 870                 875                 880
   Lys Arg Lys Val Phe Trp Glu Lys Leu Tyr Gln Gly Asn Leu Met Ser
                   885                 890                 895
   Lys Arg Lys Phe Asp Tyr Leu Thr Lys Ala Glu Arg Gly Gly Leu Thr
               900                 905                 910
   Glu Ala Asp Lys Ala Arg Phe Ile His Arg Gln Leu Val Glu Thr Arg
           915                 920                 925
   Gln Ile Thr Lys Asn Val Ala Asn Ile Leu His Gln Arg Phe Asn Tyr
       930                 935                 940
   Glu Lys Asp Asp His Gly Asn Thr Met Lys Gln Val Arg Ile Val Thr
   945                 950                 955                 960
   Leu Lys Ser Ala Leu Val Ser Gln Phe Arg Lys Gln Phe Gln Leu Tyr
                   965                 970                 975
   Lys Val Arg Asp Val Asn Asp Tyr His His Ala His Asp Ala Tyr Leu
               980                 985                 990
   Asn Gly Val Val Ala Asn Thr Leu Leu Lys Val Tyr Pro Gln Leu Glu
           995                 1000                1005
   Pro Glu Phe Val Tyr Gly Asp Tyr His Gln Phe Asp Trp Phe Lys Ala
       1010                1015                1020
   Asn Lys Ala Thr Ala Lys Lys Gln Phe Tyr Thr Asn Ile Met Leu Phe
   1025                1030                1035                1040
   Phe Ala Gln Lys Asp Arg Ile Ile Asp Glu Asn Gly Glu Ile Leu Trp
                   1045                1050                1055
   Asp Lys Lys Tyr Leu Asp Thr Val Lys Lys Val Met Ser Tyr Arg Gln
               1060                1065                1070
   Met Asn Ile Val Lys Lys Thr Glu Ile Gln Lys Gly Glu Phe Ser Lys
           1075                1080                1085
   Ala Thr Ile Lys Pro Lys Gly Asn Ser Ser Lys Leu Ile Pro Arg Lys
       1090                1095                1100
   Thr Asn Trp Asp Pro Met Lys Tyr Gly Gly Leu Asp Ser Pro Asn Met
   1105                1110                1115                1120
   Ala Tyr Ala Val Val Ile Glu Tyr Ala Lys Gly Lys Asn Lys Leu Val
                   1125                1130                1135
   Phe Glu Lys Lys Ile Ile Arg Val Thr Ile Met Glu Arg Lys Ala Phe
               1140                1145                1150
   Glu Lys Asp Glu Lys Ala Phe Leu Glu Glu Gln Gly Tyr Arg Gln Pro
           1155                1160                1165
   Lys Val Leu Ala Lys Leu Pro Lys Tyr Thr Leu Tyr Glu Cys Glu Glu
       1170                1175                1180
   Gly Arg Arg Arg Met Leu Ala Ser Ala Asn Glu Ala Gln Lys Gly Asn
   1185                1190                1195                1200
   Gln Gln Val Leu Pro Asn His Leu Val Thr Leu Leu His His Ala Ala
                   1205                1210                1215
   Asn Cys Glu Val Ser Asp Gly Lys Ser Leu Asp Tyr Ile Glu Ser Asn
               1220                1225                1230
   Arg Glu Met Phe Ala Glu Leu Leu Ala His Val Ser Glu Phe Ala Lys
           1235                1240                1245
   Arg Tyr Thr Leu Ala Glu Ala Asn Leu Asn Lys Ile Asn Gln Leu Phe
       1250                1255                1260
   Glu Gln Asn Lys Glu Gly Asp Ile Lys Ala Ile Ala Gln Ser Phe Val
   1265                1270                1275                1280
   Asp Leu Met Ala Phe Asn Ala Met Gly Ala Pro Ala Ser Phe Lys Phe
                   1285                1290                1295
   Phe Glu Thr Thr Ile Glu Arg Lys Arg Tyr Asn Asn Leu Lys Glu Leu
               1300                1305                1310
   Leu Asn Ser Thr Ile Ile Tyr Gln Ser Ile Thr Gly Leu Tyr Glu Ser
           1315                1320                1325
   Arg Lys Arg Leu Asp Asp
       1330
<210> 141
   <211> 1371
   <212> PRT
   <213> Streptococcus gallolyticus
<220>
   <223> CRISPR-associated endonuclease
<308> YP_006034260
   <309> 2013-06-11
<400> 141
   Met Thr Glu Lys Asn Tyr Ser Ile Gly Leu Asp Ile Gly Thr Asn Ser
    1               5                  10                  15
   Val Gly Trp Ala Val Ile Thr Asp Asp Tyr Lys Val Pro Ala Lys Lys
               20                  25                  30
   Met Lys Val Leu Gly Asn Thr Asp Lys Lys Tyr Ile Lys Lys Asn Leu
           35                  40                  45
   Leu Gly Ala Leu Leu Phe Asp Asn Gly Glu Thr Ala Glu Ala Thr Arg
       50                  55                  60
   Leu Lys Arg Thr Ala Arg Arg Arg Tyr Thr Arg Arg Lys Asn Arg Leu
   65                  70                  75                  80
   Arg Tyr Leu Gln Glu Ile Phe Ala Glu Glu Met Ala Lys Val Asp Glu
                   85                  90                  95
   Ser Phe Phe Tyr Arg Leu Asp Glu Ser Phe Leu Thr Thr Asp Asp Lys
               100                 105                 110
   Asp Phe Glu Arg His Pro Ile Phe Gly Asn Lys Ala Asp Glu Ile Lys
           115                 120                 125
   Tyr His Gln Glu Phe Pro Thr Ile Tyr His Leu Arg Lys His Leu Ala
       130                 135                 140
   Asp Ser His Glu Lys Ala Asp Leu Arg Leu Ile Tyr Leu Ala Leu Ala
   145                 150                 155                 160
   His Met Ile Lys Phe Arg Gly His Phe Leu Ile Glu Gly Glu Leu Asn
                   165                 170                 175
   Ala Glu Asn Thr Asp Val Gln Lys Leu Phe Glu Ala Phe Val Glu Val
               180                 185                 190
   Tyr Asp Arg Thr Phe Asp Asp Ser Asn Leu Ser Glu Ile Thr Val Asp
           195                 200                 205
   Ala Ser Ser Ile Leu Thr Glu Lys Phe Ser Lys Ser Arg Arg Leu Glu
       210                 215                 220
   Asn Leu Ile Lys His Tyr Pro Thr Glu Lys Lys Asn Thr Leu Phe Gly
   225                 230                 235                 240
   Asn Leu Val Ala Leu Ala Leu Gly Leu Gln Pro Asn Phe Lys Thr Ser
                   245                 250                 255
   Phe Lys Leu Ser Glu Asp Ala Lys Leu Gln Phe Ser Lys Asp Thr Tyr
               260                 265                 270
   Glu Glu Asp Leu Glu Glu Leu Ile Gly Lys Ile Gly Asp Glu Tyr Ala
           275                 280                 285
   Asp Leu Phe Thr Ser Ala Lys Asn Leu Tyr Asp Ala Ile Leu Leu Ser
       290                 295                 300
   Gly Ile Leu Thr Val Ala Asp Asn Thr Thr Lys Ala Pro Leu Ser Ala
   305                 310                 315                 320
   Ser Met Ile Lys Arg Tyr Asn Glu His Gln Val Asp Leu Lys Lys Leu
                   325                 330                 335
   Lys Glu Phe Ile Lys Asn Asn Ala Ser Asp Lys Tyr Asp Glu Ile Phe
               340                 345                 350
   Asn Asp Lys Asp Lys Asn Gly Tyr Ala Gly Tyr Ile Glu Asn Gly Val
           355                 360                 365
   Lys Gln Asp Glu Phe Tyr Lys Tyr Leu Lys Thr Thr Leu Ser Lys Ile
       370                 375                 380
   Asp Gly Ser Asp Tyr Phe Leu Asp Lys Ile Glu Arg Glu Asp Phe Leu
   385                 390                 395                 400
   Arg Lys Gln Arg Thr Phe Asp Asn Gly Ser Ile Pro His Gln Ile His
                   405                 410                 415
   Leu Gln Glu Met His Ala Ile Leu Arg Arg Gln Gly Glu His Tyr Ala
               420                 425                 430
   Phe Leu Lys Glu Asn Gln Ala Lys Ile Glu Lys Ile Leu Thr Phe Arg
           435                 440                 445
   Ile Pro Tyr Tyr Val Gly Pro Leu Ala Arg Lys Asn Ser Arg Phe Ala
       450                 455                 460
   Trp Ala Glu Tyr His Ser Asp Glu Lys Ile Thr Pro Trp Asn Phe Asp
   465                 470                 475                 480
   Glu Ile Ile Asp Lys Glu Lys Ser Ala Glu Lys Phe Ile Thr Arg Met
                   485                 490                 495
   Thr Leu Asn Asp Leu Tyr Leu Pro Glu Glu Lys Val Leu Pro Lys His
               500                 505                 510
   Ser Leu Val Tyr Glu Thr Tyr Thr Val Tyr Asn Glu Leu Thr Lys Val
           515                 520                 525
   Lys Tyr Val Asn Glu Gln Gly Lys Ser Asn Phe Phe Asp Ala Asn Met
       530                 535                 540
   Lys Gln Glu Ile Phe Glu His Val Phe Lys Glu Asn Arg Lys Val Thr
   545                 550                 555                 560
   Lys Asp Lys Phe Leu Asn Tyr Leu Asn Lys Glu Phe Pro Glu Tyr Arg
                   565                 570                 575
   Ile Gln Asp Leu Ile Gly Leu Asp Lys Glu Asn Lys Ser Phe Asn Ala
   580 585 590
   Ser Leu Gly Thr Tyr His Asp Leu Lys Lys Ile Leu Asp Lys Ser Phe
   595 600 605
   Leu Asp Asp Lys Thr Asn Glu Thr Ile Ile Glu Asp Ile Ile Gln Thr
   610 615 620
   Leu Thr Leu Phe Glu Asp Arg Asp Met Ile Arg Gln Arg Leu Gln Lys
   625 630 635 640
   Tyr Ser Asp Ile Phe Thr Pro Gln Gln Leu Lys Lys Leu Glu Arg Arg
   645 650 655
   His Tyr Thr Gly Trp Gly Arg Leu Ser Tyr Lys Leu Ile Asn Gly Ile
   660 665 670
   Arg Asn Lys Glu Asn Gly Lys Ser Ile Leu Asp Tyr Leu Ile Asp Asp
   675 680 685
   Gly Tyr Ala Asn Arg Asn Phe Met Gln Leu Ile Ser Asp Asp Thr Leu
   690 695 700
   Pro Phe Lys Gln Ile Ile Lys Asp Ala Gln Ile Ile Gly Asp Ile Asp
   705 710 715 720
   Asp Val Thr Ser Val Val Arg Glu Leu Pro Gly Ser Pro Ala Ile Lys
   725 730 735
   Lys Gly Ile Leu Gln Ser Val Lys Ile Val Asp Glu Leu Val Lys Val
   740 745 750
   Met Gly His Asn Pro Asp Asn Ile Val Ile Glu Met Ala Arg Glu Asn
   755 760 765
   Gln Thr Thr Asn Arg Gly Arg Asn Gln Ser Gln Gln Arg Leu Lys Lys
   770 775 780
   Leu Gln Asp Ser Leu Lys Glu Leu Gly Ser Asn Ile Leu Asn Glu Glu
   785 790 795 800
   Lys Pro Ser Tyr Ile Glu Gly Lys Val Glu Asn Asn His Leu Gln Asp
   805 810 815
   Asp Arg Leu Phe Leu Tyr Tyr Ile Gln Asn Gly Lys Asp Met Tyr Thr
   820 825 830
   Gly Asp Glu Leu Asp Ile Asp His Leu Ser Asp Tyr Asp Ile Asp His
   835 840 845
   Ile Ile Pro Gln Ala Phe Ile Lys Asp Asp Ser Ile Asp Asn Arg Val
   850 855 860
   Leu Thr Ser Ser Ala Lys Asn Arg Gly Lys Ser Asp Asp Val Pro Ser
   865 870 875 880
   Leu Asp Ile Val His Asp Arg Lys Ala Asp Trp Ile Arg Leu Tyr Lys
   885 890 895
   Ser Gly Leu Ile Ser Lys Arg Lys Phe Asp Asn Leu Thr Lys Ala Glu
   900 905 910
   Arg Gly Gly Leu Thr Glu Asn Asp Lys Ala Gly Phe Ile Lys Arg Gln
   915 920 925
   Leu Val Glu Thr Arg Gln Ile Thr Lys His Val Ala Gln Ile Leu Asp
   930 935 940
   Ser Arg Phe Asn Thr Glu Arg Asp Glu Asn Asp Lys Val Ile Arg Asn
   945 950 955 960
   Val Lys Val Ile Thr Leu Lys Ser Asn Leu Val Ser Gln Phe Arg Lys
   965 970 975
   Asp Phe Lys Phe Tyr Lys Val Arg Glu Ile Asn Asp Tyr His His Ala
   980 985 990
   His Asp Ala Tyr Leu Asn Ala Val Val Gly Thr Ala Leu Leu Lys Lys
   995 1000 1005
   Tyr Pro Lys Leu Ala Pro Glu Phe Val Tyr Gly Glu Tyr Lys Lys Tyr
   1010 1015 1020
   Asp Ile Arg Lys Phe Ile Thr Ser Ser Gly Asp Lys Ala Thr Ala Lys
   1025 1030 1035 1040
   Tyr Phe Phe Tyr Ser Asn Leu Met Asn Phe Phe Lys Arg Val Ile Arg
   1045 1050 1055
   Tyr Ser Asn Gly Lys Val Val Val Arg Pro Val Ile Glu Cys Ser Lys
   1060 1065 1070
   Asp Thr Gly Glu Ile Ala Trp Asn Lys Gln Thr Asp Phe Glu Lys Val
           1075                1080                1085
   Arg Arg Val Leu Ser Tyr Pro Gln Val Asn Ile Val Lys Lys Val Glu
       1090                1095                1100
   Thr Gln Thr Gly Gly Phe Ser Lys Glu Ser Ile Leu Pro Lys Gly Asn
   1105                1110                1115                1120
   Ser Asp Lys Leu Ile Pro Arg Lys Thr Lys Lys Phe Arg Trp Asp Thr
                   1125                1130                1135
   Pro Lys Tyr Gly Gly Phe Asp Ser Pro Asn Ile Ala Tyr Ser Val Phe
               1140                1145                1150
   Val Ile Ala Asp Val Glu Lys Gly Lys Ala Lys Lys Leu Lys Thr Val
           1155                1160                1165
   Lys Glu Leu Val Gly Ile Ser Ile Met Glu Arg Ser Ser Phe Glu Glu
       1170                1175                1180
   Asn Pro Val Val Phe Leu Glu Lys Lys Gly Tyr Gln Asn Val Gln Glu
   1185                1190                1195                1200
   Asp Asn Leu Ile Lys Leu Pro Lys Tyr Ser Leu Phe Glu Phe Glu Gly
                   1205                1210                1215
   Gly Arg Arg Arg Leu Leu Ala Ser Ala Ser Glu Leu Gln Lys Gly Asn
               1220                1225                1230
   Glu Val Val Leu Ser Arg His Leu Val Glu Leu Leu Tyr His Ala His
           1235                1240                1245
   Arg Val Asn Ser Phe Asn Asn Ser Glu His Leu Lys Tyr Val Ser Glu
       1250                1255                1260
   His Lys Lys Glu Phe Gly Glu Val Leu Ser Cys Val Glu Asn Phe Ala
   1265                1270                1275                1280
   Lys Ser Tyr Val Asp Val Glu Lys Asn Leu Gly Lys Ile Arg Ala Val
                   1285                1290                1295
   Ala Asp Lys Ile Asp Thr Phe Ser Ile Glu Asp Ile Ser Ile Ser Phe
               1300                1305                1310
   Val Asn Leu Leu Thr Leu Thr Ala Leu Gly Ala Pro Ala Asp Phe Asn
           1315                1320                1325
   Phe Leu Gly Glu Lys Ile Pro Arg Lys Arg Tyr Thr Ser Thr Lys Glu
       1330                1335                1340
   Cys Leu Asn Ala Thr Leu Ile His Gln Ser Ile Thr Gly Leu Tyr Glu
   1345                1350                1355                1360
   Thr Arg Ile Asp Leu Ser Lys Leu Gly Glu Glu
                   1365                1370
<210> 142
   <211> 1130
   <212> PRT
   <213> Streptococcus gallolyticus
<220>
   <223> CRISPR-associated endonuclease
<308> YP_006034250
   <309> 2013-06-11
<400> 142
   Met Thr Asn Gly Lys Ile Leu Gly Leu Asp Ile Gly Ile Ala Ser Val
   1               5                   10                  15
   Gly Val Gly Ile Ile Glu Ala Lys Thr Gly Lys Val Val His Ala Asn
               20                  25                  30
   Ser Arg Leu Phe Ser Ala Ala Asn Ala Glu Asn Asn Ala Glu Arg Arg
           35                  40                  45
   Gly Phe Arg Gly Ser Arg Arg Leu Asn Arg Arg Lys Lys His Arg Val
       50                  55                  60
   Lys Arg Val Arg Asp Leu Phe Glu Lys Tyr Glu Ile Val Thr Asp Phe
   65                  70                  75                  80
   Arg Asn Leu Asn Leu Asn Pro Tyr Glu Leu Arg Val Lys Gly Leu Thr
                   85                  90                  95
   Glu Gln Leu Thr Asn Glu Glu Leu Phe Ala Ala Leu Arg Thr Ile Ser
               100                 105                 110
   Lys Arg Arg Gly Ile Ser Tyr Leu Asp Asp Ala Glu Asp Asp Ser Thr
           115                 120                 125
   Gly Ser Thr Asp Tyr Ala Lys Ser Ile Asp Glu Asn Arg Arg Leu Leu
       130                 135                 140
   Lys Thr Lys Thr Pro Gly Gln Ile Gln Leu Glu Arg Leu Glu Lys Tyr
   145                 150                 155                 160
   Gly Gln Leu Arg Gly Asn Phe Thr Val Tyr Asp Glu Asn Gly Glu Ala
                   165                 170                 175
   His Arg Leu Ile Asn Val Phe Ser Thr Ser Asp Tyr Glu Lys Glu Ala
               180                 185                 190
   Arg Lys Ile Leu Glu Thr Gln Ala Asp Tyr Asn Lys Lys Ile Thr Ala
           195                 200                 205
   Glu Phe Ile Asp Asp Tyr Val Glu Ile Leu Thr Gln Lys Arg Lys Tyr
       210                 215                 220
   Tyr His Gly Pro Gly Asn Glu Lys Ser Arg Thr Asp Tyr Gly Arg Phe
   225                 230                 235                 240
   Arg Thr Asp Gly Thr Thr Leu Glu Asn Ile Phe Gly Ile Leu Ile Gly
                   245                 250                 255
   Lys Cys Ser Phe Tyr Pro Asp Glu Tyr Arg Ala Ser Lys Ala Ser Tyr
               260                 265                 270
   Thr Ala Gln Glu Tyr Asn Phe Leu Asn Asp Leu Asn Asn Leu Lys Val
           275                 280                 285
   Pro Thr Glu Thr Gly Lys Leu Ser Thr Glu Gln Lys Glu Ala Leu Val
       290                 295                 300
   Glu Phe Ala Lys Ser Thr Ala Thr Leu Gly Pro Ala Lys Leu Leu Lys
   305                 310                 315                 320
   Glu Ile Ala Lys Ile Leu Asp Cys Lys Val Asp Glu Ile Lys Gly Tyr
                   325                 330                 335
   Arg Glu Asp Asp Lys Gly Lys Pro Asp Leu His Thr Phe Glu Pro Tyr
               340                 345                 350
   Arg Lys Leu Lys Phe Asn Leu Asp Ser Val Asn Ile Asp Asp Leu Ser
           355                 360                 365
   Arg Glu Val Leu Asp Lys Leu Ala Asp Ile Leu Thr Leu Asn Thr Glu
       370                 375                 380
   Arg Glu Gly Ile Glu Asp Ala Ile Arg His Asn Leu Pro Asn Gln Phe
   385                 390                 395                 400
   Thr Glu Gly Gln Ile Ser Glu Ile Ile Lys Val Arg Lys Ser Gln Ser
                   405                 410                 415
   Thr Ala Phe Asn Lys Gly Trp His Ser Phe Ser Ala Lys Leu Met Asn
               420                 425                 430
   Glu Leu Ile Pro Glu Leu Tyr Ala Thr Ser Asp Glu Gln Met Thr Ile
           435                 440                 445
   Leu Thr Arg Leu Glu Lys Phe Lys Val Asn Lys Lys Ser Ser Lys Asn
       450                 455                 460
   Thr Lys Thr Ile Asp Glu Lys Glu Val Thr Asp Glu Ile Tyr Asn Pro
   465                 470                 475                 480
   Val Val Ala Lys Ser Val Arg Gln Thr Ile Lys Ile Ile Asn Ala Ala
                   485                 490                 495
   Val Lys Lys Tyr Gly Asp Phe Asp Lys Ile Val Ile Glu Met Pro Arg
               500                 505                 510
   Asp Lys Asn Ala Asp Asp Glu Lys Lys Phe Ile Asp Lys Arg Asn Lys
           515                 520                 525
   Glu Asn Lys Lys Glu Lys Asp Asp Ala Leu Lys Arg Ala Ala Tyr Leu
       530                 535                 540
   Tyr Asn Gly Thr Asp Lys Leu Pro Asp Glu Val Phe His Gly Asn Lys
   545                 550                 555                 560
   Gln Leu Glu Thr Lys Ile Arg Leu Trp Tyr Gln Gln Gly Glu Arg Cys
                   565                 570                 575
   Leu Tyr Ser Gly Lys Pro Ile Pro Ile Gln Glu Leu Val His Asn Ser
               580                 585                 590
   Asn Asn Phe Glu Ile Asp His Ile Leu Pro Leu Ser Leu Ser Phe Asp
   595 600 605
   Asp Ser Leu Ala Asn Lys Val Leu Val Tyr Ala Trp Thr Asn Gln Glu
   610 615 620
   Lys Gly Gln Lys Thr Pro Tyr Gln Val Ile Asp Ser Met Asp Ala Ala
   625 630 635 640
   Trp Ser Phe Arg Glu Met Lys Asp Tyr Val Leu Lys Gln Lys Gly Leu
   645 650 655
   Gly Lys Lys Lys Arg Asp Tyr Leu Leu Thr Thr Glu Asn Ile Asp Lys
   660 665 670
   Ile Glu Val Lys Lys Lys Phe Ile Glu Arg Asn Leu Val Asp Thr Arg
   675 680 685
   Tyr Ala Ser Arg Val Val Leu Asn Ser Leu Gln Ser Ala Leu Arg Glu
   690 695 700
   Leu Gly Lys Asp Thr Lys Ile Ser Val Ile Arg Gly Gln Phe Thr Ser
   705 710 715 720
   Gln Leu Arg Arg Lys Trp Lys Ile Asp Lys Ser Arg Glu Thr Tyr His
   725 730 735
   His His Ala Val Asp Ala Leu Ile Ile Ala Ala Ser Ser Gln Leu Lys
   740 745 750
   Leu Trp Glu Lys Gln Asp Asn Pro Met Phe Val Asp Tyr Gly Asn Asn
   755 760 765
   Gln Val Val Asp Lys Gln Thr Gly Glu Ile Leu Ser Val Ser Asp Asp
   770 775 780
   Glu Tyr Lys Glu Leu Val Phe Gln Pro Pro Tyr Gln Gly Phe Val Asn
   785 790 795 800
   Met Ile Ser Ser Lys Gly Phe Glu Asp Glu Ile Leu Phe Ser Tyr Gln
   805 810 815
   Val Asp Ser Lys Tyr Asn Arg Lys Val Ser Asp Ala Thr Ile Tyr Ser
   820 825 830
   Thr Arg Lys Ala Lys Ile Gly Lys Asp Lys Lys Glu Glu Thr Tyr Val
   835 840 845
   Leu Gly Lys Ile Lys Asp Ile Tyr Ser Gln Asn Gly Phe Asp Thr Phe
   850 855 860
   Ile Lys Lys Tyr Asn Lys Asp Lys Thr Gln Phe Leu Met Tyr Gln Lys
   865 870 875 880
   Asp Pro Leu Thr Trp Glu Asn Val Ile Glu Val Ile Leu Arg Asp Tyr
   885 890 895
   Pro Thr Thr Lys Lys Ser Glu Asp Gly Lys Asn Asp Val Lys Cys Asn
   900 905 910
   Pro Phe Glu Glu Tyr Arg Arg Glu Asn Gly Leu Val Cys Lys Tyr Ser
   915 920 925
   Lys Lys Gly Lys Gly Thr Pro Ile Lys Ser Leu Lys Tyr Tyr Asp Lys
   930 935 940
   Lys Leu Gly Asn Cys Ile Asp Ile Thr Pro Glu Gly Ser Lys Asn Glu
   945 950 955 960
   Val Val Leu Gln Ser Leu Asn Pro Trp Arg Ala Asp Val Tyr Phe Asn
   965 970 975
   Pro Glu Thr Leu Lys Tyr Glu Leu Leu Gly Leu Lys Tyr Ser Asp Leu
   980 985 990
   Ser Phe Glu Lys Gly Thr Gly Lys Tyr His Ile Ser Gln Glu Lys Tyr
   995 1000 1005
   Asp Val Ile Lys Glu Lys Glu Gly Ile Gly Lys Lys Ser Glu Phe Lys
   1010 1015 1020
   Phe Thr Leu Tyr Arg Asn Asp Leu Ile Leu Ile Lys Asp Thr Ala Ser
   1025 1030 1035 1040
   Gly Glu Gln Glu Ile Tyr Arg Phe Leu Ser Arg Thr Met Pro Asn Val
   1045 1050 1055
   Lys His Tyr Ala Glu Leu Lys Pro Tyr Asp Lys Glu Lys Phe Asp Asn
   1060 1065 1070
   Val Gln Glu Leu Val Glu Ala Leu Gly Glu Ala Asp Lys Val Gly Arg
   1075 1080 1085
   Cys Ile Lys Gly Leu Asn Lys Ser Asn Leu Ser Ile Tyr Lys Val Arg
   1090 1095 1100
   Thr Asp Val Leu Gly Asn Lys Tyr Phe Val Lys Lys Glu Gly Asp Lys
   1105 1110 1115 1120
   Pro Lys Leu Asp Phe Lys Asn Asn Lys Lys
   1125 1130
<210> 143
   <211> 897
   <212> PRT
   <213> Corynebacterium ulcerans
<220>
   <223> CRISPR-associated endonuclease
<308> YP_005709668
   <309> 2013-06-11
<400> 143
   Met Thr Asn Ala Gly Thr Asn His His Val Leu Trp Ala Lys Phe Asp
   1               5                   10                  15
   Asn Val Ser Glu Pro Tyr Pro Leu Leu Ala His Leu Leu Asp Thr Ala
               20                  25                  30
   Thr Ala Ala Thr Cys Leu Phe Asn His Trp Leu Arg Lys Gly Leu Arg
           35                  40                  45
   Asp Arg Leu Ser Thr Glu Leu Gly Pro Asp Ala Glu Lys Ile Leu Gly
       50                  55                  60
   Phe Val Ala Gly Ile His Asp Leu Gly Lys Ala Asn Pro Tyr Phe Gln
   65                  70                  75                  80
   Ala Gln Arg Arg Asn Lys Lys Glu Glu Trp Ile Thr Leu Arg Asp Ala
                   85                  90                  95
   Ile Gln Lys Ala Gly Phe Pro Leu Gly Asn Gly Thr Ser Ala Leu Phe
               100                 105                 110
   Glu Glu Thr Lys Glu Lys Arg Arg His Glu Asn Ile Thr Leu Ser Ile
           115                 120                 125
   Leu Gly Trp Glu Ile Thr Lys Phe Leu Gln Val Lys Asp Val Trp Pro
       130                 135                 140
   Gln Leu Ala Ile Ile Gly His His Gly Asn Phe Ser Ala Pro Gly Phe
   145                 150                 155                 160
   Leu Ser Asp Lys Asp Asp Leu Glu Asp Ile Glu Asp Ile Phe Asp Asp
                   165                 170                 175
   Asn Gly Trp Ser Pro Thr His Glu Leu Leu Val Ser Ser Leu Leu Gln
               180                 185                 190
   Ala Val Gly Leu Glu Lys Gln Pro Glu Ile Lys His Ile Ser Pro Ala
           195                 200                 205
   Ser Ala Ile Leu Ile Ser Gly Leu Val Val Leu Ala Asp Arg Ile Ala
       210                 215                 220
   Ser Gln Ser Glu Met Ala Ser Asp Gly Leu Gln Ala Leu Gln Lys Glu
   225                 230                 235                 240
   Glu Leu Phe Phe His Gln Pro Glu Lys Trp Ile Ala Asn Arg Lys Ala
                   245                 250                 255
   Phe Cys Arg Glu Ile Ile Glu Thr Thr Val Gly Thr Tyr His Pro Trp
               260                 265                 270
   Glu Ser Glu Ala Ala Gly Ile Arg Ala Val Leu Gly Asp Tyr Glu Pro
           275                 280                 285
   Arg Phe Thr Gln Lys Ala Ala Leu Asn Ala Gly Asp Gly Leu Phe Asn
       290                 295                 300
   Val Met Glu Thr Thr Gly Ala Gly Lys Thr Glu Ala Ala Leu Leu Arg
   305                 310                 315                 320
   His Val Lys Arg Lys Glu Arg Leu Leu Phe Phe Leu Pro Thr Gln Ala
                   325                 330                 335
   Thr Thr Asn Ala Ile Met Asp Arg Ile Gly Lys Ile Phe Asp Gly Thr
               340                 345                 350
   Pro Asn Val Ala Ser Leu Ala His Gly Leu Ala Val Thr Glu Asp Phe
           355                 360                 365
   Tyr Ala His Pro Ile Leu Pro Val Gln Gly Ser Ser Asp Asp Ala Asn
       370                 375                 380
   Tyr Lys Asp Asn Gly Gly Leu Tyr Pro Thr Glu Phe Val Arg Ser Ala
   385                 390                 395                 400
   Gly Thr Pro Arg Leu Leu Ala Pro Val Cys Val Gly Thr Ile Asp Gln
                   405                 410                 415
   Ala Leu Met Gly Ala Leu Pro Ser Lys Phe Asn His Leu Arg Leu Leu
               420                 425                 430
   Ala Leu Ala Asn Ala His Val Val Val Asp Glu Val His Thr Met Asp
           435                 440                 445
   Gln Tyr Gln Ser Glu Leu Met Ser Gly Leu Leu Glu Trp Trp Ser Ala
       450                 455                 460
   Thr Asp Thr Pro Val Thr Leu Leu Thr Ala Thr Met Pro Ala Trp Gln
   465                 470                 475                 480
   Arg Glu Lys Phe His Leu Ser Tyr Thr Gly Lys Glu Pro His Phe Lys
                   485                 490                 495
   Gly Val Phe Pro Ser Leu Glu Asp Trp Ser Thr Pro Asn Lys Asn Thr
               500                 505                 510
   Glu Thr Ser Gln Glu Asn Ile Pro Thr Glu Ala Phe Thr Ile Pro Ile
           515                 520                 525
   Arg Ile Asp Lys Ile Ala His Asn Glu Ile Val Asp Ser His Val Gln
       530                 535                 540
   Trp Val Ile Glu Gln Arg Lys Leu Phe Pro Gln Ala Arg Ile Gly Ile
   545                 550                 555                 560
   Ile Cys Asn Thr Val Gly Arg Ala Gln Ser Ile Ala Glu Ala Leu Ala
                   565                 570                 575
   His Glu Ser Pro Ile Val Leu His Ser Arg Met Thr Ala Gly His Arg
               580                 585                 590
   Lys Glu Ala Ala Thr Lys Leu Glu Gln Ala Ile Gly Lys Lys Gly Thr
           595                 600                 605
   Ala Thr Ala Thr Leu Val Ile Gly Thr Gln Ala Ile Glu Ala Ser Leu
       610                 615                 620
   Asp Ile Asp Leu Asp Leu Leu Arg Thr Glu Leu Cys Pro Ala Pro Ser
   625                 630                 635                 640
   Leu Ile Gln Arg Ala Gly Arg Leu Trp Arg Arg Leu Asp Pro Gln Arg
                   645                 650                 655
   Glu Val Arg Val Pro Gly Met Val Gly Lys Lys Leu Thr Ile Ala Val
               660                 665                 670
   Val Asp Ser Pro Ser Thr Gly Gln Thr Leu Pro Tyr Leu Arg Ser Gln
           675                 680                 685
   Leu Tyr Arg Val Glu Ser Trp Leu Lys Gln Arg Asp Arg Ile Glu Phe
       690                 695                 700
   Pro Ala Asp Ile Gln Asp Phe Ile Asp Ala Thr Thr Pro Gly Leu Gln
   705                 710                 715                 720
   Glu Leu Phe Gln Lys Val Ser Leu Pro Glu Asp Cys Gly Ser Ala Glu
                   725                 730                 735
   Glu Arg Glu Ala Leu Ala Asp Asp Tyr Leu Asn Glu Val Ala Ser Trp
               740                 745                 750
   Val Thr Lys Gln Arg Gln Ala Gly Thr Ser Arg Ile Asp Phe Ala Lys
           755                 760                 765
   His Gly Lys Pro Arg Gln Val Leu Ala Ser Asp Cys Val Val Glu Asp
       770                 775                 780
   Phe Leu Gln Ile Thr Ser Ala Lys Asp Leu Glu Glu Arg Ala Thr Arg
   785                 790                 795                 800
   Leu Ile Asp Tyr Pro Thr Ile Ser Ala Ile Leu Cys Asp Pro Thr Gly
                   805                 810                 815
   Thr Ile Pro Gly Ala Trp Thr Asp Ser Val Glu Lys Leu Ile Ala Ile
               820                 825                 830
   Pro Ala Lys Asp Arg Glu Ser Leu Arg Arg Ala Leu Ser Ala Ser Ile
           835                 840                 845
   Ser Ile Pro His Ser Lys Lys Phe Leu Pro Ile Thr Ser Arg Glu Ile
       850                 855                 860
   Pro Leu Ser Glu Ala Lys Thr Leu Leu Ser Gly Tyr Ser Ala Val His
   865                 870                 875                 880
   Ile Gln Pro Glu Glu Tyr Asp Leu Gln Ser Gly Leu Lys Gly Pro Gln
                   885                 890                 895
   Lys
<210> 144
   <211> 1050
   <212> PRT
   <213> Gluconacetobacter diazotrophicus
<220>
   <223> CRISPR-associated endonuclease
<308> YP_001602368
   <309> 2013-08-27
<400> 144
   Met Gly Glu Asn Met Ile Asp Glu Ser Leu Thr Phe Gly Ile Asp Leu
   1               5                   10                  15
   Gly Ile Gly Ser Cys Gly Trp Ala Val Leu Arg Arg Pro Ser Ala Phe
                   20                  25                  30
   Gly Arg Lys Gly Val Ile Glu Gly Met Gly Ser Trp Cys Phe Asp Val
           35                  40                  45
   Pro Glu Thr Ser Lys Glu Arg Thr Pro Thr Asn Gln Ile Arg Arg Ser
       50                  55                  60
   Asn Arg Leu Leu Arg Arg Val Ile Arg Arg Arg Arg Asn Arg Met Ala
   65                  70                  75                  80
   Ala Ile Arg Arg Leu Leu His Ala Ala Gly Leu Leu Pro Ser Thr Asp
                   85                  90                  95
   Ser Asp Ala Leu Lys Arg Pro Gly His Asp Pro Trp Glu Leu Arg Ala
               100                 105                 110
   Arg Gly Leu Asp Lys Pro Leu Lys Pro Val Glu Phe Ala Val Val Leu
           115                 120                 125
   Gly His Ile Ala Lys Arg Arg Gly Phe Lys Ser Ala Ala Lys Arg Lys
       130                 135                 140
   Ala Thr Asn Ile Ser Ser Asp Asp Lys Lys Met Leu Thr Ala Leu Glu
   145                 150                 155                 160
   Ala Thr Arg Glu Arg Leu Gly Arg Tyr Arg Thr Val Gly Glu Met Phe
                   165                 170                 175
   Ala Arg Asp Pro Asp Phe Ala Ser Arg Arg Arg Asn Arg Glu Gly Lys
               180                 185                 190
   Tyr Asp Arg Thr Thr Ala Arg Asp Asp Leu Glu His Glu Val His Ala
           195                 200                 205
   Leu Phe Ala Ala Gln Arg Arg Leu Gly Gln Gly Phe Ala Ser Pro Glu
       210                 215                 220
   Leu Glu Glu Ala Phe Thr Ala Ser Ala Phe His Gln Arg Pro Met Gln
   225                 230                 235                 240
   Asp Ser Glu Arg Leu Val Gly Phe Cys Pro Phe Glu Arg Thr Glu Lys
                   245                 250                 255
   Arg Ala Ala Lys Leu Thr Pro Ser Phe Glu Arg Phe Arg Leu Leu Ala
               260                 265                 270
   Arg Leu Leu Asn Leu Arg Ile Thr Thr Pro Asp Gly Glu Arg Pro Leu
           275                 280                 285
   Thr Val Asp Glu Ile Ala Leu Val Thr Arg Asp Leu Gly Lys Thr Ala
       290                 295                 300
   Lys Leu Ser Ile Lys Arg Val Arg Thr Leu Ile Gly Leu Glu Asp Asn
   305                 310                 315                 320
   Gln Arg Phe Thr Thr Ile Arg Pro Glu Asp Glu Asp Arg Asp Ile Val
                   325                 330                 335
   Ala Arg Thr Gly Gly Ala Met Thr Gly Thr Ala Thr Leu Arg Lys Ala
               340                 345                 350
   Leu Gly Glu Ala Leu Trp Thr Asp Met Gln Glu Arg Pro Glu Gln Leu
           355                 360                 365
   Asp Ala Ile Val Gln Val Leu Ser Phe Phe Glu Ala Asn Glu Thr Ile
       370                 375                 380
   Thr Glu Lys Leu Arg Glu Ile Gly Leu Thr Leu Ala Val Leu Asp Val
   385                 390                 395                 400
   Leu Leu Thr Ala Leu Asp Ala Gly Val Phe Ala Lys Phe Lys Gly Ala
                   405                 410                 415
   Ala His Ile Ser Thr Lys Ala Ala Arg Asn Leu Leu Pro His Leu Glu
               420                 425                 430
   Gln Gly Arg Arg Tyr Asp Glu Ala Cys Thr Met Ala Gly Tyr Asp His
           435                 440                 445
   Ala Ala Ser Arg Leu Ser His His Gly Gln Ile Val Ala Lys Thr Gln
       450                 455                 460
   Phe Asn Ala Leu Val Thr Glu Ile Gly Glu Ser Ile Ala Asn Pro Ile
   465                 470                 475                 480
   Ala Arg Lys Ala Leu Ile Glu Gly Leu Lys Gln Ile Trp Ala Met Arg
                   485                 490                 495
   Asn His Trp Gly Leu Pro Gly Ser Ile His Val Glu Leu Ala Arg Asp
               500                 505                 510
   Val Gly Asn Ser Ile Glu Lys Arg Arg Glu Ile Glu Lys His Ile Glu
           515                 520                 525
   Lys Asn Thr Ala Leu Arg Ala Arg Glu Arg Arg Glu Val His Asp Leu
       530                 535                 540
   Leu Asp Leu Glu Asp Val Asn Gly Asp Thr Leu Leu Arg Tyr Arg Leu
   545                 550                 555                 560
   Trp Lys Glu Gln Gly Gly Lys Cys Leu Tyr Thr Gly Lys Ala Ile His
                   565                 570                 575
   Ile Arg Gln Ile Ala Ala Thr Asp Asn Ser Val Gln Val Asp His Ile
               580                 585                 590
   Leu Pro Trp Ser Arg Phe Gly Asp Asp Ser Phe Asn Asn Lys Thr Leu
           595                 600                 605
   Cys Leu Ala Ser Ala Asn Gln Gln Lys Lys Arg Ser Thr Pro Tyr Glu
       610                 615                 620
   Trp Leu Ser Gly Gln Thr Gly Asp Ala Trp Asn Ala Phe Val Gln Arg
   625                 630                 635                 640
   Ile Glu Thr Asn Lys Glu Leu Arg Gly Phe Lys Lys Arg Asn Tyr Leu
                   645                 650                 655
   Leu Lys Asn Ala Lys Glu Ala Glu Glu Lys Phe Arg Ser Arg Asn Leu
               660                 665                 670
   Asn Asp Thr Arg Tyr Ala Ala Arg Leu Phe Ala Glu Ala Val Lys Leu
           675                 680                 685
   Leu Tyr Ala Phe Gly Glu Arg Gln Glu Lys Gly Gly Asn Arg Arg Val
       690                 695                 700
   Phe Thr Arg Pro Gly Ala Leu Thr Ala Ala Leu Arg Gln Ala Trp Gly
   705                 710                 715                 720
   Val Glu Ser Leu Lys Lys Gln Asp Gly Lys Arg Ile Asn Asp Asp Arg
                   725                 730                 735
   His His Ala Leu Asp Ala Leu Thr Val Ala Ala Val Asp Glu Ala Glu
               740                 745                 750
   Ile Gln Arg Leu Thr Lys Ser Phe His Glu Trp Glu Gln Gln Gly Leu
           755                 760                 765
   Gly Arg Pro Leu Arg Arg Val Glu Pro Pro Trp Glu Ser Phe Arg Ala
       770                 775                 780
   Asp Val Glu Ala Thr Tyr Pro Glu Val Phe Val Ala Arg Pro Glu Arg
   785                 790                 795                 800
   Arg Arg Ala Arg Gly Glu Gly His Ala Ala Thr Ile Arg Gln Val Lys
                   805                 810                 815
   Glu Arg Glu Cys Thr Pro Ile Val Phe Glu Arg Lys Ala Val Ser Ser
               820                 825                 830
   Leu Lys Glu Ala Asp Leu Glu Arg Ile Lys Asp Gly Glu Arg Asn Glu
           835                 840                 845
   Ala Ile Val Glu Ala Ile Arg Ser Trp Ile Ala Thr Gly Arg Pro Ala
       850                 855                 860
   Asp Ala Pro Pro Arg Ser Pro Arg Gly Asp Ile Ile Thr Lys Ile Arg
   865                 870                 875                 880
   Leu Ala Thr Thr Ile Lys Ala Ala Val Pro Val Arg Gly Gly Thr Ala
                   885                 890                 895
   Gly Arg Gly Glu Met Val Arg Ala Asp Val Phe Ser Lys Pro Asn Arg
               900                 905                 910
   Arg Gly Lys Asp Glu Trp Tyr Leu Val Pro Val Tyr Pro His Gln Ile
           915                 920                 925
   Met Asn Arg Lys Ala Trp Pro Lys Pro Pro Met Arg Ser Ile Val Ala
       930                 935                 940
   Asn Lys Asp Glu Asp Glu Trp Thr Glu Val Gly Pro Glu His Gln Phe
   945                 950                 955                 960
   Arg Phe Ser Leu Tyr Pro Arg Ser Asn Ile Glu Ile Ile Arg Pro Ser
                   965                 970                 975
   Gly Glu Val Ile Glu Gly Tyr Phe Val Gly Leu His Arg Asn Thr Gly
               980                 985                 990
   Ala Leu Thr Ile Ser Ala His Asn Asp Pro Lys Ser Ile His Ser Gly
           995                 1000                1005
   Ile Gly Thr Lys Thr Leu Leu Ala Ile Ser Lys Tyr Gln Val Asp Arg
       1010                1015                1020
   Phe Gly Arg Lys Ser Pro Val Arg Lys Glu Val Arg Thr Trp His Gly
   1025                1030                1035                1040
   Glu Ala Cys Ile Ser Pro Thr Pro Pro Gly
                   1045                1050
<210> 145
   <211> 1166
   <212> PRT
   <213> Nitrobacter hamburgensis
<220>
   <223> CRISPR-associated endonuclease
<308> YP_571550
   <309> 2013-07-30
<400> 145
   Met His Val Glu Ile Asp Phe Pro His Phe Ser Arg Gly Asp Ser His
   1 5 10 15
   Leu Ala Met Asn Lys Asn Glu Ile Leu Arg Gly Ser Ser Val Leu Tyr
   20 25 30
   Arg Leu Gly Leu Asp Leu Gly Ser Asn Ser Leu Gly Trp Phe Val Thr
   35 40 45
   His Leu Glu Lys Arg Gly Asp Arg His Glu Pro Val Ala Leu Gly Pro
   50 55 60
   Gly Gly Val Arg Ile Phe Pro Asp Gly Arg Asp Pro Gln Ser Gly Thr
   65 70 75 80
   Ser Asn Ala Val Asp Arg Arg Met Ala Arg Gly Ala Arg Lys Arg Arg
   85 90 95
   Asp Arg Phe Val Glu Arg Arg Lys Glu Leu Ile Ala Ala Leu Ile Lys
   100 105 110
   Tyr Asn Leu Leu Pro Asp Asp Ala Arg Glu Arg Arg Ala Leu Glu Val
   115 120 125
   Leu Asp Pro Tyr Ala Leu Arg Lys Thr Ala Leu Thr Asp Thr Leu Pro
   130 135 140
   Ala His His Val Gly Arg Ala Leu Phe His Leu Asn Gln Arg Arg Gly
   145 150 155 160
   Phe Gln Ser Asn Arg Lys Thr Asp Ser Lys Gln Ser Glu Asp Gly Ala
   165 170 175
   Ile Lys Gln Ala Ala Ser Arg Leu Ala Thr Asp Lys Gly Asn Glu Thr
           180                 185                 190
   Leu Gly Val Phe Phe Ala Asp Met His Leu Arg Lys Ser Tyr Glu Asp
       195                 200                 205
   Arg Gln Thr Ala Ile Arg Ala Glu Leu Val Arg Leu Gly Lys Asp His
       210                 215                 220
   Leu Thr Gly Asn Ala Arg Lys Lys Ile Trp Ala Lys Val Arg Lys Arg
   225                 230                 235                 240
   Leu Phe Gly Asp Glu Val Leu Pro Arg Ala Asp Ala Pro His Gly Val
                   245                 250                 255
   Arg Ala Arg Ala Thr Ile Thr Gly Thr Lys Ala Ser Tyr Asp Tyr Tyr
               260                 265                 270
   Pro Thr Arg Asp Met Leu Arg Asp Glu Phe Asn Ala Ile Trp Ala Gly
           275                 280                 285
   Gln Ser Ala His His Ala Thr Ile Thr Asp Glu Ala Arg Thr Glu Ile
       290                 295                 300
   Glu His Ile Ile Phe Tyr Gln Arg Pro Leu Lys Pro Ala Ile Val Gly
   305                 310                 315                 320
   Lys Cys Thr Leu Asp Pro Ala Thr Arg Pro Phe Lys Glu Asp Pro Glu
                   325                 330                 335
   Gly Tyr Arg Ala Pro Trp Ser His Pro Leu Ala Gln Arg Phe Arg Ile
               340                 345                 350
   Leu Ser Glu Ala Arg Asn Leu Glu Ile Arg Asp Thr Gly Lys Gly Ser
           355                 360                 365
   Arg Arg Leu Thr Lys Glu Gln Ser Asp Leu Val Val Ala Ala Leu Leu
       370                 375                 380
   Ala Asn Arg Glu Val Lys Phe Asp Lys Leu Arg Thr Leu Leu Lys Leu
   385                 390                 395                 400
   Pro Ala Glu Ala Arg Phe Asn Leu Glu Ser Asp Arg Arg Ala Ala Leu
                   405                 410                 415
   Asp Gly Asp Gln Thr Ala Ala Arg Leu Ser Asp Lys Lys Gly Phe Asn
               420                 425                 430
   Lys Ala Trp Arg Gly Phe Pro Pro Glu Arg Gln Ile Ala Ile Val Ala
           435                 440                 445
   Arg Leu Glu Glu Thr Glu Asp Glu Asn Glu Leu Ile Ala Trp Leu Glu
       450                 455                 460
   Lys Glu Cys Ala Leu Asp Gly Ala Ala Ala Ala Arg Val Ala Asn Thr
   465                 470                 475                 480
   Thr Leu Pro Asp Gly His Cys Arg Leu Gly Leu Arg Ala Ile Lys Lys
                   485                 490                 495
   Ile Val Pro Ile Met Gln Asp Gly Leu Asp Glu Asp Gly Val Ala Gly
               500                 505                 510
   Ala Gly Tyr His Ile Ala Ala Lys Arg Ala Gly Tyr Asp His Ala Lys
           515                 520                 525
   Leu Pro Thr Gly Glu Gln Leu Gly Arg Leu Pro Tyr Tyr Gly Gln Trp
       530                 535                 540
   Leu Gln Asp Ala Val Val Gly Ser Gly Asp Ala Arg Asp Gln Lys Glu
   545                 550                 555                 560
   Lys Gln Tyr Gly Gln Phe Pro Asn Pro Thr Val His Ile Gly Leu Gly
                   565                 570                 575
   Gln Leu Arg Arg Val Val Asn Asp Leu Ile Asp Lys Tyr Gly Pro Pro
               580                 585                 590
   Thr Glu Ile Ser Ile Glu Phe Thr Arg Ala Leu Lys Leu Ser Glu Gln
           595                 600                 605
   Gln Lys Ala Glu Arg Gln Arg Glu Gln Arg Arg Asn Gln Asp Lys Asn
       610                 615                 620
   Lys Ala Arg Ala Glu Glu Leu Ala Lys Phe Gly Arg Pro Ala Asn Pro
   625                 630                 635                 640
   Arg Asn Leu Leu Lys Met Arg Leu Trp Glu Glu Leu Ala His Asp Pro
                   645                 650                 655
   Leu Asp Arg Lys Cys Val Tyr Thr Gly Glu Gln Ile Ser Ile Glu Arg
               660                 665                 670
   Leu Leu Ser Asp Glu Val Asp Ile Asp His Ile Leu Pro Val Ala Met
           675                 680                 685
   Thr Leu Asp Asp Ser Pro Ala Asn Lys Ile Ile Cys Met Arg Tyr Ala
   690 695 700
   Asn Arg His Lys Arg Lys Gln Thr Pro Ser Glu Ala Phe Gly Ser Ser
   705 710 715 720
   Pro Thr Leu Gln Gly His Arg Tyr Asn Trp Asp Asp Ile Ala Ala Arg
   725 730 735
   Ala Thr Gly Leu Pro Arg Asn Lys Arg Trp Arg Phe Asp Ala Asn Ala
   740 745 750
   Arg Glu Glu Phe Asp Lys Arg Gly Gly Phe Leu Ala Arg Gln Leu Asn
   755 760 765
   Glu Thr Gly Trp Leu Ala Arg Leu Ala Lys Gln Tyr Leu Gly Ala Val
   770 775 780
   Thr Asp Pro Asn Gln Ile Trp Val Val Pro Gly Arg Leu Thr Ser Met
   785 790 795 800
   Leu Arg Gly Lys Trp Gly Leu Asn Gly Leu Leu Pro Ser Asp Asn Tyr
   805 810 815
   Ala Gly Val Gln Asp Lys Ala Glu Glu Phe Leu Ala Ser Thr Asp Asp
   820 825 830
   Met Glu Phe Ser Gly Val Lys Asn Arg Ala Asp His Arg His His Ala
   835 840 845
   Ile Asp Gly Leu Val Thr Ala Leu Thr Asp Arg Ser Leu Leu Trp Lys
   850 855 860
   Met Ala Asn Ala Tyr Asp Glu Glu His Glu Lys Phe Val Ile Glu Pro
   865 870 875 880
   Pro Trp Pro Thr Met Arg Asp Asp Leu Lys Ala Ala Leu Glu Lys Met
   885 890 895
   Val Val Ser His Lys Pro Asp His Gly Ile Glu Gly Lys Leu His Glu
   900 905 910
   Asp Ser Ala Tyr Gly Phe Val Lys Pro Leu Asp Ala Thr Gly Leu Lys
   915 920 925
   Glu Glu Glu Ala Gly Asn Leu Val Tyr Arg Lys Ala Ile Glu Ser Leu
   930 935 940
   Asn Glu Asn Glu Val Asp Arg Ile Arg Asp Ile Gln Leu Arg Thr Ile
   945 950 955 960
   Val Arg Asp His Val Asn Val Glu Lys Thr Lys Gly Val Ala Leu Ala
   965 970 975
   Asp Ala Leu Arg Gln Leu Gln Ala Pro Ser Asp Asp Tyr Pro Gln Phe
   980 985 990
   Lys His Gly Leu Arg His Val Arg Ile Leu Lys Lys Glu Lys Gly Asp
   995 1000 1005
   Tyr Leu Val Pro Ile Ala Asn Arg Ala Ser Gly Val Ala Tyr Lys Ala
   1010 1015 1020
   Tyr Ser Ala Gly Glu Asn Phe Cys Val Glu Val Phe Glu Thr Ala Gly
   1025 1030 1035 1040
   Gly Lys Trp Asp Gly Glu Ala Val Arg Arg Phe Asp Ala Asn Lys Lys
   1045 1050 1055
   Asn Ala Gly Pro Lys Ile Ala His Ala Pro Gln Trp Arg Asp Ala Asn
   1060 1065 1070
   Glu Gly Ala Lys Leu Val Met Arg Ile His Lys Gly Asp Leu Ile Arg
   1075 1080 1085
   Leu Asp His Glu Gly Arg Ala Arg Ile Met Val Val His Arg Leu Asp
   1090 1095 1100
   Ala Ala Ala Gly Arg Phe Lys Leu Ala Asp His Asn Glu Thr Gly Asn
   1105 1110 1115 1120
   Leu Asp Lys Arg His Ala Thr Asn Asn Asp Ile Asp Pro Phe Arg Trp
   1125 1130 1135
   Leu Met Ala Ser Tyr Asn Thr Leu Lys Lys Leu Ala Ala Val Pro Val
   1140 1145 1150
   Arg Val Asp Glu Leu Gly Arg Val Trp Arg Val Met Pro Asn
   1155 1160 1165
<210> 146
   <211> 1128
   <212> PRT
   <213> Streptococcus thermophilus
<220>
   <223> CRISPR-associated endonuclease
<308> YP_141068
   <309> 2013-08-27
<400> 146
   Met Ser Asp Leu Val Leu Gly Leu Asp Ile Gly Ile Gly Ser Val Gly
   1               5                   10                  15
   Val Gly Ile Leu Asn Lys Val Thr Gly Glu Ile Ile His Lys Asn Ser
               20                  25                  30
   Arg Ile Phe Pro Ala Ala Gln Ala Glu Asn Asn Leu Val Arg Arg Thr
           35                  40                  45
   Asn Arg Gln Gly Arg Arg Leu Thr Arg Arg Lys Lys His Arg Ile Val
       50                  55                  60
   Arg Leu Asn Arg Leu Phe Glu Glu Ser Gly Leu Ile Thr Asp Phe Thr
   65                  70                  75                  80
   Lys Ile Ser Ile Asn Leu Asn Pro Tyr Gln Leu Arg Val Lys Gly Leu
                   85                  90                  95
   Thr Asp Glu Leu Ser Asn Glu Glu Leu Phe Ile Ala Leu Lys Asn Met
               100                 105                 110
   Val Lys His Arg Gly Ile Ser Tyr Leu Asp Asp Ala Ser Asp Asp Gly
           115                 120                 125
   Asn Ser Ser Val Gly Asp Tyr Ala Gln Ile Val Lys Glu Asn Ser Lys
       130                 135                 140
   Gln Leu Glu Thr Lys Thr Pro Gly Gln Ile Gln Leu Glu Arg Tyr Gln
   145                 150                 155                 160
   Thr Tyr Gly Gln Leu Arg Gly Asp Phe Thr Val Glu Lys Asp Gly Lys
                   165                 170                 175
   Lys His Arg Leu Ile Asn Val Phe Pro Thr Ser Ala Tyr Arg Ser Glu
               180                 185                 190
   Ala Leu Arg Ile Leu Gln Thr Gln Gln Glu Phe Asn Pro Gln Ile Thr
           195                 200                 205
   Asp Glu Phe Ile Asn Arg Tyr Leu Glu Ile Leu Thr Gly Lys Arg Lys
       210                 215                 220
   Tyr Tyr His Gly Pro Gly Asn Glu Lys Ser Arg Thr Asp Tyr Gly Arg
   225                 230                 235                 240
   Tyr Arg Thr Ser Gly Glu Thr Leu Asp Asn Ile Phe Gly Ile Leu Ile
                   245                 250                 255
   Gly Lys Cys Thr Phe Tyr Pro Glu Glu Phe Arg Ala Ala Lys Ala Ser
               260                 265                 270
   Tyr Thr Ala Gln Glu Phe Asn Leu Leu Asn Asp Leu Asn Asn Leu Thr
           275                 280                 285
   Val Pro Thr Glu Thr Lys Lys Leu Ser Lys Glu Gln Lys Asn Gln Ile
       290                 295                 300
   Ile Asn Tyr Val Lys Asn Glu Lys Ala Met Gly Pro Ala Lys Leu Phe
   305                 310                 315                 320
   Lys Tyr Ile Ala Lys Leu Leu Ser Cys Asp Val Ala Asp Ile Lys Gly
                   325                 330                 335
   Tyr Arg Ile Asp Lys Ser Gly Lys Ala Glu Ile His Thr Phe Glu Ala
               340                 345                 350
   Tyr Arg Lys Met Lys Thr Leu Glu Thr Leu Asp Ile Glu Gln Met Asp
           355                 360                 365
   Arg Glu Thr Leu Asp Lys Leu Ala Tyr Val Leu Thr Leu Asn Thr Glu
       370                 375                 380
   Arg Glu Gly Ile Gln Glu Ala Leu Glu His Glu Phe Ala Asp Gly Ser
   385                 390                 395                 400
   Phe Ser Gln Lys Gln Val Asp Glu Leu Val Gln Phe Arg Lys Ala Asn
                   405                 410                 415
   Ser Ser Ile Phe Gly Lys Gly Trp His Asn Phe Ser Val Lys Leu Met
   420 425 430
   Met Glu Leu Ile Pro Glu Leu Tyr Glu Thr Ser Glu Glu Gln Met Thr
   435 440 445
   Ile Leu Thr Arg Leu Gly Lys Gln Lys Arg Leu Arg Leu Gln Ile Lys
   450 455 460
   Gln Asn Ile Ser Asn Lys Thr Lys Tyr Ile Asp Glu Lys Leu Leu Thr
   465 470 475 480
   Glu Glu Ile Tyr Asn Pro Val Val Ala Lys Ser Val Arg Gln Ala Ile
   485 490 495
   Lys Ile Val Asn Ala Ala Ile Lys Glu Tyr Gly Asp Phe Asp Asn Ile
   500 505 510
   Val Ile Glu Met Ala Arg Glu Thr Asn Glu Asp Asp Glu Lys Lys Ala
   515 520 525
   Ile Gln Lys Ile Gln Lys Ala Asn Lys Asp Glu Lys Asp Ala Ala Met
   530 535 540
   Leu Lys Ala Ala Asn Gln Tyr Asn Gly Lys Ala Glu Leu Pro His Ser
   545 550 555 560
   Val Phe His Gly His Lys Gln Leu Ala Thr Lys Ile Arg Leu Trp His
   565 570 575
   Gln Gln Gly Glu Arg Cys Leu Tyr Thr Gly Lys Thr Ile Ser Ile His
   580 585 590
   Asp Leu Ile Asn Asn Pro Asn Gln Phe Glu Val Asp His Ile Leu Pro
   595 600 605
   Leu Ser Ile Thr Phe Asp Asp Ser Leu Ala Asn Lys Val Leu Val Tyr
   610 615 620
   Ala Thr Ala Asn Gln Glu Lys Gly Gln Arg Thr Pro Tyr Gln Ala Leu
   625 630 635 640
   Asp Ser Met Asp Asp Ala Trp Ser Phe Arg Glu Leu Lys Ala Phe Val
   645 650 655
   Arg Glu Ser Lys Thr Leu Ser Asn Lys Lys Lys Glu Tyr Leu Leu Thr
   660 665 670
   Glu Glu Asp Ile Ser Lys Phe Asp Val Arg Lys Lys Phe Ile Glu Arg
   675 680 685
   Asn Leu Val Asp Thr Arg Tyr Ala Ser Arg Val Val Leu Asn Ala Leu
   690 695 700
   Gln Glu His Phe Arg Ala His Lys Ile Asp Thr Lys Val Ser Val Val
   705 710 715 720
   Arg Gly Gln Phe Thr Ser Gln Leu Arg Arg His Trp Gly Ile Glu Lys
   725 730 735
   Thr Arg Asp Thr Tyr His His His Ala Val Asp Ala Leu Ile Ile Ala
   740 745 750
   Ala Ser Ser Gln Leu Asn Leu Trp Lys Lys Gln Lys Asn Thr Leu Val
   755 760 765
   Ser Tyr Ser Glu Glu Gln Leu Leu Asp Ile Glu Thr Gly Glu Leu Ile
   770 775 780
   Ser Asp Asp Glu Tyr Lys Glu Ser Val Phe Lys Ala Pro Tyr Gln His
   785 790 795 800
   Phe Val Asp Thr Leu Lys Ser Lys Glu Phe Glu Asp Ser Ile Leu Phe
   805 810 815
   Ser Tyr Gln Val Asp Ser Lys Phe Asn Arg Lys Ile Ser Asp Ala Thr
   820 825 830
   Ile Tyr Ala Thr Arg Gln Ala Lys Val Gly Lys Asp Lys Lys Asp Glu
   835 840 845
   Thr Tyr Val Leu Gly Lys Ile Lys Asp Ile Tyr Thr Gln Asp Gly Tyr
   850 855 860
   Asp Ala Phe Met Lys Ile Tyr Lys Lys Asp Lys Ser Lys Phe Leu Met
   865 870 875 880
   Tyr Arg His Asp Pro Gln Thr Phe Glu Lys Val Ile Glu Pro Ile Leu
   885 890 895
   Glu Asn Tyr Pro Asn Lys Gln Met Asn Glu Lys Gly Lys Glu Val Pro
   900 905 910
   Cys Asn Pro Phe Leu Lys Tyr Lys Glu Glu His Gly Tyr Ile Arg Lys
               915                 920                 925
   Tyr Ser Lys Lys Gly Asn Gly Pro Glu Ile Lys Ser Leu Lys Tyr Tyr
           930                 935                 940
   Asp Ser Lys Leu Leu Gly Asn Pro Ile Asp Ile Thr Pro Glu Asn Ser
   945                 950                 955                 960
   Lys Asn Lys Val Val Leu Gln Ser Leu Lys Pro Trp Arg Thr Asp Val
                   965                 970                 975
   Tyr Phe Asn Lys Ala Thr Gly Lys Tyr Glu Ile Leu Gly Leu Lys Tyr
               980                 985                 990
   Ala Asp Leu Gln Phe Glu Lys Gly Thr Gly Thr Tyr Lys Ile Ser Gln
           995                 1000                1005
   Glu Lys Tyr Asn Asp Ile Lys Lys Lys Glu Gly Val Asp Ser Asp Ser
       1010                1015                1020
   Glu Phe Lys Phe Thr Leu Tyr Lys Asn Asp Leu Leu Leu Val Lys Asp
   1025                1030                1035                1040
   Thr Glu Thr Lys Glu Gln Gln Leu Phe Arg Phe Leu Ser Arg Thr Leu
                   1045                1050                1055
   Pro Lys Gln Lys His Tyr Val Glu Leu Lys Pro Tyr Asp Lys Gln Lys
               1060                1065                1070
   Phe Glu Gly Gly Glu Ala Leu Ile Lys Val Leu Gly Asn Val Ala Asn
           1075                1080                1085
   Gly Gly Gln Cys Ile Lys Gly Leu Ala Lys Ser Asn Ile Ser Ile Tyr
       1090                1095                1100
   Lys Val Arg Thr Asp Val Leu Gly Asn Gln His Ile Ile Lys Asn Glu
   1105                1110                1115                1120
   Gly Asp Lys Pro Lys Leu Asp Phe
                   1125
<210> 147
   <211> 1122
   <212> PRT
   <213> Streptococcus thermophilus
<220>
   <223> CRISPR-associated endonuclease
<308> YP_139177
   <309> 2013-06-10
<400> 147
   Met Ser Asp Leu Val Leu Gly Leu Asp Ile Gly Ile Gly Ser Val Gly
    1               5                  10                  15
   Val Gly Ile Leu Asn Lys Val Thr Gly Glu Ile Ile His Lys Asn Ser
               20                  25                  30
   Arg Ile Phe Pro Ala Ala Gln Ala Glu Asn Asn Leu Val Arg Arg Thr
           35                  40                  45
   Asn Arg Gln Gly Arg Arg Leu Thr Arg Arg Lys Lys His Arg Ile Val
       50                  55                  60
   Arg Leu Asn Arg Leu Phe Glu Glu Ser Gly Leu Ile Thr Asp Phe Thr
   65                  70                  75                  80
   Lys Ile Ser Ile Asn Leu Asn Pro Tyr Gln Leu Arg Val Lys Gly Leu
                   85                  90                  95
   Thr Asp Glu Leu Ser Asn Glu Glu Leu Phe Ile Ala Leu Lys Asn Met
               100                 105                 110
   Val Lys His Arg Gly Ile Ser Tyr Leu Asp Asp Ala Ser Asp Asp Gly
           115                 120                 125
   Asn Ser Ser Val Gly Asp Tyr Ala Gln Ile Val Lys Glu Asn Ser Lys
       130                 135                 140
   Gln Leu Glu Thr Lys Thr Pro Gly Gln Ile Gln Leu Glu Arg Tyr Gln
   145                 150                 155                 160
   Thr Tyr Gly Gln Leu Arg Gly Asp Phe Thr Val Glu Lys Asp Gly Lys
                   165                 170                 175
   Lys His Arg Leu Ile Asn Val Phe Pro Thr Ser Ala Tyr Arg Ser Glu
               180                 185                 190
   Ala Leu Arg Ile Leu Gln Thr Gln Gln Glu Phe Asn Ser Gln Ile Thr
           195                 200                 205
   Asp Glu Phe Ile Asn Arg Tyr Leu Glu Ile Leu Thr Gly Lys Arg Lys
       210                 215                 220
   Tyr Tyr His Gly Pro Gly Asn Glu Lys Ser Arg Thr Asp Tyr Gly Arg
   225                 230                 235                 240
   Tyr Arg Thr Asn Gly Glu Thr Leu Asp Asn Ile Phe Gly Ile Leu Ile
                   245                 250                 255
   Gly Lys Cys Thr Phe Tyr Pro Asp Glu Phe Arg Ala Ala Lys Ala Ser
               260                 265                 270
   Tyr Thr Ala Gln Glu Phe Asn Leu Leu Asn Asp Leu Asn Asn Leu Thr
           275                 280                 285
   Val Pro Thr Glu Thr Lys Lys Leu Ser Lys Glu Gln Lys Asn Gln Ile
       290                 295                 300
   Ile Asn Tyr Val Lys Asn Glu Lys Val Met Gly Pro Ala Lys Leu Phe
   305                 310                 315                 320
   Lys Tyr Ile Ala Lys Leu Leu Ser Cys Asp Val Ala Asp Ile Lys Gly
                   325                 330                 335
   His Arg Ile Asp Lys Ser Gly Lys Ala Glu Ile His Thr Phe Glu Ala
               340                 345                 350
   Tyr Arg Lys Met Lys Thr Leu Glu Thr Leu Asp Ile Glu Gln Met Asp
           355                 360                 365
   Arg Glu Thr Leu Asp Lys Leu Ala Tyr Val Leu Thr Leu Asn Thr Glu
       370                 375                 380
   Arg Glu Gly Ile Gln Glu Ala Leu Glu His Glu Phe Ala Asp Gly Ser
   385                 390                 395                 400
   Phe Ser Gln Lys Gln Val Asp Glu Leu Val Gln Phe Arg Lys Ala Asn
                   405                 410                 415
   Ser Ser Ile Phe Gly Lys Gly Trp His Asn Phe Ser Val Lys Leu Met
               420                 425                 430
   Met Glu Leu Ile Pro Glu Leu Tyr Glu Thr Ser Glu Glu Gln Met Thr
           435                 440                 445
   Ile Leu Thr Arg Leu Gly Lys Gln Lys Thr Thr Ser Ser Ser Asn Lys
       450                 455                 460
   Thr Lys Tyr Ile Asp Glu Lys Leu Leu Thr Glu Glu Ile Tyr Asn Pro
   465                 470                 475                 480
   Val Val Ala Lys Ser Val Arg Gln Ala Ile Lys Ile Val Asn Ala Ala
                   485                 490                 495
   Ile Lys Glu Tyr Gly Asp Phe Asp Asn Ile Val Ile Glu Met Ala Arg
               500                 505                 510
   Glu Thr Asn Glu Asp Asp Glu Lys Lys Ala Ile Gln Lys Ile Gln Lys
           515                 520                 525
   Ala Asn Lys Asp Glu Lys Asp Ala Ala Met Leu Lys Ala Ala Asn Gln
       530                 535                 540
   Tyr Asn Gly Lys Ala Glu Leu Pro His Ser Val Phe His Gly His Lys
   545                 550                 555                 560
   Gln Leu Ala Thr Lys Ile Arg Leu Trp His Gln Gln Gly Glu Arg Cys
                   565                 570                 575
   Leu Tyr Thr Gly Lys Thr Ile Ser Ile His Asp Leu Ile Asn Asn Pro
               580                 585                 590
   Asn Gln Phe Glu Val Asp His Ile Leu Pro Leu Ser Ile Thr Phe Asp
           595                 600                 605
   Asp Ser Leu Ala Asn Lys Val Leu Val Tyr Ala Thr Ala Asn Gln Glu
       610                 615                 620
   Lys Gly Gln Arg Thr Pro Tyr Gln Ala Leu Asp Ser Met Asp Asp Ala
   625                 630                 635                 640
   Trp Ser Phe Arg Glu Leu Lys Ala Phe Val Arg Glu Ser Lys Thr Leu
                   645                 650                 655
   Ser Asn Lys Lys Lys Glu Tyr Leu Leu Thr Glu Glu Asp Ile Ser Lys
               660                 665                 670
   Phe Asp Val Arg Lys Lys Phe Ile Glu Arg Asn Leu Val Asp Thr Arg
           675                 680                 685
   Tyr Ala Ser Arg Val Val Leu Asn Ala Leu Gln Glu His Phe Arg Ala
       690                 695                 700
   His Lys Ile Asp Thr Lys Val Ser Val Val Arg Gly Gln Phe Thr Ser
   705                 710                 715                 720
   Gln Leu Arg Arg His Trp Gly Ile Glu Lys Thr Arg Asp Thr Tyr His
                   725                 730                 735
   His His Ala Val Asp Ala Leu Ile Ile Ala Ala Ser Ser Gln Leu Asn
               740                 745                 750
   Leu Trp Lys Lys Gln Lys Asn Thr Leu Val Ser Tyr Ser Glu Glu Gln
           755                 760                 765
   Leu Leu Asp Ile Glu Thr Gly Glu Leu Ile Ser Asp Asp Glu Tyr Lys
       770                 775                 780
   Glu Ser Val Phe Lys Ala Pro Tyr Gln His Phe Val Asp Thr Leu Lys
   785                 790                 795                 800
   Ser Lys Glu Phe Glu Asp Ser Ile Leu Phe Ser Tyr Gln Val Asp Ser
                   805                 810                 815
   Lys Phe Asn Arg Lys Ile Ser Asp Ala Thr Ile Tyr Ala Thr Arg Gln
               820                 825                 830
   Ala Lys Val Gly Lys Asp Lys Lys Asp Glu Thr Tyr Val Leu Gly Lys
           835                 840                 845
   Ile Lys Asp Ile Tyr Thr Gln Asp Gly Tyr Asp Ala Phe Met Lys Ile
       850                 855                 860
   Tyr Lys Lys Asp Lys Ser Lys Phe Leu Met Tyr Arg His Asp Pro Gln
   865                 870                 875                 880
   Thr Phe Glu Lys Val Ile Glu Pro Ile Leu Glu Asn Tyr Pro Asn Lys
                   885                 890                 895
   Glu Met Asn Glu Lys Gly Lys Glu Val Pro Cys Asn Pro Phe Leu Lys
               900                 905                 910
   Tyr Lys Glu Glu His Gly Tyr Ile Arg Lys Tyr Ser Lys Lys Gly Asn
           915                 920                 925
   Gly Pro Glu Ile Lys Ser Leu Lys Tyr Tyr Asp Ser Lys Leu Leu Gly
       930                 935                 940
   Asn Pro Ile Asp Ile Thr Pro Glu Asn Ser Lys Asn Lys Val Val Leu
   945                 950                 955                 960
   Gln Ser Leu Lys Pro Trp Arg Thr Asp Val Tyr Phe Asn Lys Asn Thr
                   965                 970                 975
   Gly Lys Tyr Glu Ile Leu Gly Leu Lys Tyr Ala Asp Leu Gln Phe Glu
               980                 985                 990
   Lys Lys Thr Gly Thr Tyr Lys Ile Ser Gln Glu Lys Tyr Asn Gly Ile
           995                 1000                1005
   Met Lys Glu Glu Gly Val Asp Ser Asp Ser Glu Phe Lys Phe Thr Leu
       1010                1015                1020
   Tyr Lys Asn Asp Leu Leu Leu Val Lys Asp Thr Glu Thr Lys Glu Gln
   1025                1030                1035                1040
   Gln Leu Phe Arg Phe Leu Ser Arg Thr Met Pro Asn Val Lys Tyr Tyr
                   1045                1050                1055
   Val Glu Leu Lys Pro Tyr Ser Lys Asp Lys Phe Glu Lys Asn Glu Ser
               1060                1065                1070
   Leu Ile Glu Ile Leu Gly Ser Ala Asp Lys Ser Gly Arg Cys Ile Lys
           1075                1080                1085
   Gly Leu Gly Lys Ser Asn Ile Ser Ile Tyr Lys Val Arg Thr Asp Val
       1090                1095                1100
   Leu Gly Asn Gln His Ile Ile Lys Asn Glu Gly Asp Lys Pro Lys Leu
   1105                1110                1115                1120
   Asp Phe
<210> 148
   <211> 1368
   <212> PRT
   <213> Streptococcus pyogenes
<220>
   <223> CRISPR-associated endonuclease
<308> NP_664481
   <309> 2013-06-10
<400> 148
   Met Asp Lys Lys Tyr Ser Ile Gly Leu Asp Ile Gly Thr Asn Ser Val
   1               5                   10                  15
   Gly Trp Ala Val Ile Thr Asp Asp Tyr Lys Val Pro Ser Lys Lys Leu
               20                  25                  30
   Lys Gly Leu Gly Asn Thr Asp Arg His Gly Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Ala Asp
       130                 135                 140
   Ser Thr Asp Lys Val Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Arg Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Thr Leu Leu Ser Asp
       290                 295                 300
   Ile Leu Arg Val Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Ala Lys Leu Asn Arg Glu Asp Leu Leu Arg
   385                 390                 395                 400
   Lys Gln Arg Thr Phe Asp Asn Gly Ser Ile Pro Tyr Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
   435 440 445
   Pro Tyr Tyr Val Gly Pro Leu Ala Arg Gly Asn Ser Arg Phe Ala Trp
   450 455 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
   530 535 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Thr Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
   610 615 620
   Leu Phe Glu Asp Arg Glu Met Ile Glu Glu Arg Leu Lys Thr Tyr Ala
   625 630 635 640
   His Leu Phe Asp Asp Lys Val Met Lys Gln Leu Lys Arg Arg Arg Tyr
   645 650 655
   Thr Gly Trp Gly Arg Leu Ser Arg Lys Leu Ile Asn Gly Ile Arg Asp
   660 665 670
   Lys Gln Ser Gly Lys Thr Ile Leu Asp Phe Leu Lys Ser Asp Gly Phe
   675 680 685
   Ala Asn Arg Asn Phe Met Gln Leu Ile His Asp Asp Ser Leu Thr Phe
   690 695 700
   Lys Glu Asp Ile Gln Lys Ala Gln Val Ser Gly Gln Gly Asp Ser Leu
   705 710 715 720
   His Glu His Ile Ala Asn Leu Ala Gly Ser Pro Ala Ile Lys Lys Gly
   725 730 735
   Ile Leu Gln Thr Val Lys Val Val Asp Glu Leu Val Lys Val Met Gly
   740 745 750
   Arg His Lys Pro Glu Asn Ile Val Ile Glu Met Ala Arg Glu Asn Gln
   755 760 765
   Thr Thr Gln Lys Gly Gln Lys Asn Ser Arg Glu Arg Met Lys Arg Ile
   770 775 780
   Glu Glu Gly Ile Lys Glu Leu Gly Ser Asp Ile Leu Lys Glu Tyr Pro
   785 790 795 800
   Val Glu Asn Thr Gln Leu Gln Asn Glu Lys Leu Tyr Leu Tyr Tyr Leu
   805 810 815
   Gln Asn Gly Arg Asp Met Tyr Val Asp Gln Glu Leu Asp Ile Asn Arg
   820 825 830
   Leu Ser Asp Tyr Asp Val Asp His Ile Val Pro Gln Ser Phe Leu Lys
   835 840 845
   Asp Asp Ser Ile Asp Asn Lys Val Leu Thr Arg Ser Asp Lys Asn Arg
   850 855 860
   Gly Lys Ser Asp Asn Val Pro Ser Glu Glu Val Val Lys Lys Met Lys
   865 870 875 880
   Asn Tyr Trp Arg Gln Leu Leu Asn Ala Lys Leu Ile Thr Gln Arg Lys
   885 890 895
   Phe Asp Asn Leu Thr Lys Ala Glu Arg Gly Gly Leu Ser Glu Leu Asp
   900 905 910
   Lys Val Gly Phe Ile Lys Arg Gln Leu Val Glu Thr Arg Gln Ile Thr
   915 920 925
   Lys His Val Ala Gln Ile Leu Asp Ser Arg Met Asn Thr Lys Tyr Asp
   930 935 940
   Glu Asn Asp Lys Leu Ile Arg Glu Val Arg Val Ile Thr Leu Lys Ser
   945 950 955 960
   Lys Leu Val Ser Asp Phe Arg Lys Asp Phe Gln Phe Tyr Lys Val Arg
   965 970 975
   Glu Ile Asn Asn Tyr His His Ala His Asp Ala Tyr Leu Asn Ala Val
   980 985 990
   Val Gly Thr Ala Leu Ile Lys Lys Tyr Pro Lys Leu Glu Ser Glu Phe
   995 1000 1005
   Val Tyr Gly Asp Tyr Lys Val Tyr Asp Val Arg Lys Met Ile Ala Lys
   1010 1015 1020
   Ser Glu Gln Glu Ile Gly Lys Ala Thr Ala Lys Tyr Phe Phe Tyr Ser
   1025 1030 1035 1040
   Asn Ile Met Asn Phe Phe Lys Thr Glu Ile Thr Leu Ala Asn Gly Glu
   1045 1050 1055
   Ile Arg Lys Arg Pro Leu Ile Glu Thr Asn Gly Glu Thr Gly Glu Ile
   1060 1065 1070
   Val Trp Asp Lys Gly Arg Asp Phe Ala Thr Val Arg Lys Val Leu Ser
   1075 1080 1085
   Met Pro Gln Val Asn Ile Val Lys Lys Thr Glu Val Gln Thr Gly Gly
   1090 1095 1100
   Phe Ser Lys Glu Ser Ile Leu Pro Lys Arg Asn Ser Asp Lys Leu Ile
   1105 1110 1115 1120
   Ala Arg Lys Lys Asp Trp Asp Pro Lys Lys Tyr Gly Gly Phe Asp Ser
   1125 1130 1135
   Pro Thr Val Ala Tyr Ser Val Leu Val Val Ala Lys Val Glu Lys Gly
   1140 1145 1150
   Lys Ser Lys Lys Leu Lys Ser Val Lys Glu Leu Leu Gly Ile Thr Ile
   1155 1160 1165
   Met Glu Arg Ser Ser Phe Glu Lys Asp Pro Ile Asp Phe Leu Glu Ala
   1170 1175 1180
   Lys Gly Tyr Lys Glu Val Arg Lys Asp Leu Ile Ile Lys Leu Pro Lys
   1185 1190 1195 1200
   Tyr Ser Leu Phe Glu Leu Glu Asn Gly Arg Lys Arg Met Leu Ala Ser
   1205 1210 1215
   Ala Gly Glu Leu Gln Lys Gly Asn Glu Leu Ala Leu Pro Ser Lys Tyr
   1220 1225 1230
   Val Asn Phe Leu Tyr Leu Ala Ser His Tyr Glu Lys Leu Lys Gly Ser
   1235 1240 1245
   Pro Glu Asp Asn Glu Gln Lys Gln Leu Phe Val Glu Gln His Lys His
   1250 1255 1260
   Tyr Leu Asp Glu Ile Ile Glu Gln Ile Ser Glu Phe Ser Lys Arg Val
   1265 1270 1275 1280
   Ile Leu Ala Asp Ala Asn Leu Asp Lys Val Leu Ser Ala Tyr Asn Lys
   1285 1290 1295
   His Arg Asp Lys Pro Ile Arg Glu Gln Ala Glu Asn Ile Ile His Leu
   1300 1305 1310
   Phe Thr Leu Thr Asn Leu Gly Ala Pro Ala Ala Phe Lys Tyr Phe Asp
   1315 1320 1325
   Thr Thr Ile Asp Arg Lys Arg Tyr Thr Ser Thr Lys Glu Val Leu Asp
   1330 1335 1340
   Ala Thr Leu Ile His Gln Ser Ile Thr Gly Leu Tyr Glu Thr Arg Ile
   1345 1350 1355 1360
   Asp Leu Ser Gln Leu Gly Gly Asp
   1365
<210> 149
   <211> 1269
   <212> PRT
   <213> Mycoplasma gallisepticum
<220>
   <223> CRISPR-associated endonuclease
<308> YP_006586107
   <309> 2013-06-11
<400> 149
   Met Asn Asn Ser Ile Lys Ser Lys Pro Glu Val Thr Ile Gly Leu Asp
   1               5                   10                  15
   Leu Gly Val Gly Ser Val Gly Trp Ala Ile Val Asp Asn Glu Thr Asn
               20                  25                  30
   Ile Ile His His Leu Gly Ser Arg Leu Phe Ser Gln Ala Lys Thr Ala
           35                  40                  45
   Glu Asp Arg Arg Ser Phe Arg Gly Ala Arg Arg Leu Ile Arg Arg Arg
       50                  55                  60
   Lys Tyr Lys Leu Lys Arg Phe Ile Asn Leu Ile Trp Lys Tyr Asn Ser
   65                  70                  75                  80
   Tyr Phe Gly Phe Lys Asn Lys Glu Asp Ile Leu Asn Asn Tyr Gln Glu
                   85                  90                  95
   Gln Gln Lys Leu His Asn Thr Val Leu Asn Leu Lys Leu Glu Ala Leu
               100                 105                 110
   Asn Ala Lys Ile Asp Pro Lys Ala Leu Ser Trp Ile Leu His Asp Tyr
           115                 120                 125
   Leu Lys Asn Arg Gly His Phe Tyr Glu Asp Asn Arg Asp Phe Asn Val
       130                 135                 140
   Tyr Pro Thr Glu Glu Leu Ala Asn Tyr Phe Gly Glu Tyr Gly Tyr Tyr
   145                 150                 155                 160
   Lys Gly Ile Ile Asp Ser Lys Glu Asp Asn Asp Asp Lys Leu Glu Glu
                   165                 170                 175
   Gly Leu Thr Lys Tyr Lys Phe Ser Asn Gln His Trp Leu Glu Glu Ile
               180                 185                 190
   Lys Lys Val Leu Ser Asn Gln Thr Gly Leu Pro Glu Lys Phe Lys Glu
           195                 200                 205
   Glu Tyr Glu Ser Leu Phe Ser Tyr Val Arg Asn Tyr Ser Glu Gly Pro
       210                 215                 220
   Gly Ser Ile Asn Ser Val Ser Pro Tyr Gly Ile Tyr His Leu Asp Glu
   225                 230                 235                 240
   Lys Glu Gly Lys Val Val Gln Lys Tyr Asn Asn Ile Trp Asp Lys Thr
                   245                 250                 255
   Ile Gly Lys Cys Ser Ile Phe Pro Asp Glu Tyr Arg Ala Pro Lys Asn
               260                 265                 270
   Ser Pro Ile Ala Met Ile Phe Asn Glu Ile Asn Glu Leu Ser Thr Ile
           275                 280                 285
   Arg Ser Tyr Ser Ile Tyr Leu Thr Gly Trp Phe Ile Asn Gln Glu Phe
       290                 295                 300
   Lys Lys Ala Tyr Leu Asn Lys Leu Leu Asp Leu Leu Ile Lys Thr Asn
   305                 310                 315                 320
   Ser Glu Lys Pro Ile Asp Ala Arg Gln Phe Lys Lys Leu Arg Glu Glu
                   325                 330                 335
   Thr Ile Ala Glu Ser Ile Gly Lys Glu Thr Leu Lys Asp Val Glu Arg
               340                 345                 350
   Glu Glu Lys Leu Glu Lys Glu Asp His Lys Trp Lys Leu Lys Gly Leu
           355                 360                 365
   Lys Leu Asn Thr Asn Gly Lys Ile Gln Tyr Asn Asp Leu Ser Ser Leu
       370                 375                 380
   Ala Lys Phe Val His Lys Leu Lys Gln His Leu Lys Leu Asp Phe Leu
   385                 390                 395                 400
   Leu Glu Asp Gln Tyr Thr Thr Leu Asp Lys Ile Asn Phe Leu Gln Ser
                   405                 410                 415
   Leu Phe Val Tyr Leu Gly Lys His Leu Arg Tyr Ser Asn Arg Val Asp
               420                 425                 430
   Ser Ala Asn Leu Lys Glu Phe Ser Asp Ser Asn Lys Leu Phe Glu Arg
           435                 440                 445
   Val Leu Gln Glu Gln Lys Asp Asp Leu Phe Lys Leu Phe Glu Gln Thr
       450                 455                 460
   Asp Lys Asp Asp Glu Lys Ile Leu Ala Gln Thr His Ser Leu Ser Thr
   465 470 475 480
   Lys Ala Met Leu Leu Ala Ile Thr Arg Met Thr Asn Leu Asp Asn Asp
   485 490 495
   Glu Asp Asn Gln Lys Asn Asn Asp Lys Gly Trp Asn Phe Glu Ala Ile
   500 505 510
   Lys Asn Phe Asp Gln Lys Phe Ile Asp Ile Thr Lys Lys Asn Asn Asn
   515 520 525
   Leu Ser Leu Lys Gln Asp Lys Arg Tyr Leu Asp Asp Arg Phe Ile Asn
   530 535 540
   Asp Ala Ile Leu Ser Pro Gly Val Lys Arg Ile Leu Arg Glu Ala Thr
   545 550 555 560
   Lys Val Phe Asn Ala Ile Leu Lys Gln Phe Ser Glu Glu Tyr Asp Val
   565 570 575
   Thr Lys Val Val Ile Glu Leu Ala Arg Glu Leu Ser Glu Glu Lys Glu
   580 585 590
   Leu Glu Asn Thr Lys Asn Tyr Glu Lys Leu Ile Lys Lys Asn Ser Asp
   595 600 605
   Lys Ile Ser Glu Arg Leu Glu Ala Leu Asp Ile Ala Glu Asp Lys Ile
   610 615 620
   Glu Asp Ile Leu Lys Ser Pro Thr Lys Ser Tyr Lys Val Leu Leu Trp
   625 630 635 640
   Leu Gln Gln Asp His Ile Asp Pro Tyr Ser Gln Lys Glu Ile Ala Phe
   645 650 655
   Glu Asp Ile Leu Thr Lys Thr Glu Lys Thr Glu Ile Asp His Ile Ile
   660 665 670
   Pro Tyr Ser Ile Ser Phe Asp Asp Ser Ser Ser Asn Lys Leu Leu Val
   675 680 685
   Leu Ala Glu Ser Asn Gln Ala Lys Ser Asn Gln Thr Pro Tyr Glu Phe
   690 695 700
   Ile Thr Ser Gly Asn Ala Gly Ile Lys Trp Glu Asp Tyr Glu Ala Tyr
   705 710 715 720
   Cys Arg Lys Phe Lys Asp Gly Asp Thr Ser Leu Leu Asp Ser Thr Gln
   725 730 735
   Arg Ser Lys Lys Phe Ala Lys Met Met Lys Thr Asp Thr Ser Ser Lys
   740 745 750
   Tyr Asp Ile Gly Phe Leu Ala Arg Asn Leu Asn Asp Thr Arg Tyr Ala
   755 760 765
   Thr Ile Val Phe Arg Asp Ala Leu Lys Asp Tyr Ala Asn Asn His Leu
   770 775 780
   Val Glu Asp Lys Pro Met Phe Lys Val Val Cys Ile Asn Gly Gly Val
   785 790 795 800
   Thr Ser Phe Leu Arg Lys Asn Phe Asp Lys Ser Trp Tyr Ala Lys Lys
   805 810 815
   Asp Arg Asp Lys Asn Ile His His Ala Val Asp Ala Ser Ile Ile Ser
   820 825 830
   Ile Phe Ser Asn Glu Thr Lys Thr Leu Phe Asn Gln Leu Thr Gln Phe
   835 840 845
   Ala Asp Tyr Lys Leu Phe Lys Asn Thr Asp Gly Ser Trp Lys Lys Ile
   850 855 860
   Asp Pro Lys Thr Gly Val Val Thr Glu Val Thr Asp Glu Asn Trp Lys
   865 870 875 880
   Gln Ile Arg Val Arg Asn Gln Val Ser Glu Ile Ala Lys Val Ile Asn
   885 890 895
   Lys Cys Ile Gln Asp Ser Asn Ile Glu Arg Lys Ala Arg Tyr Ser Arg
   900 905 910
   Lys Ile Glu Asn Lys Thr Asn Ile Ser Leu Phe Asn Asp Thr Val Tyr
   915 920 925
   Ser Ala Lys Lys Val Gly Tyr Glu Asp Gln Ile Lys Arg Lys Asn Leu
   930 935 940
   Lys Thr Leu Asp Ile His Glu Ser Ala Glu Glu Asn Lys Asn Ser Lys
   945 950 955 960
   Val Lys Lys Gln Phe Val Tyr Arg Lys Leu Val Asn Val Ser Leu Leu
                   965                 970                 975
   Asn Asn Asp Lys Leu Ala Asp Leu Phe Ala Glu Lys Glu Asp Ile Leu
               980                 985                 990
   Met Tyr Arg Ala Asn Pro Trp Val Ile Asn Leu Ala Glu Gln Ile Phe
           995                 1000                1005
   Asn Glu Tyr Thr Glu Asn Lys Lys Ile Lys Ser Gln Asn Val Phe Glu
       1010                1015                1020
   Lys Tyr Met Leu Asp Leu Thr Lys Glu Phe Pro Glu Lys Phe Ser Glu
   1025                1030                1035                1040
   Ala Phe Val Lys Ser Met Leu Arg Asn Lys Thr Ala Ile Ile Tyr Asn
                   1045                1050                1055
   Val Glu Lys Lys Val Val His Arg Ile Lys Arg Leu Lys Ile Leu Ser
               1060                1065                1070
   Ser Glu Leu Lys Glu Asn Lys Leu Ser Asn Val Ile Ile Arg Ser Lys
           1075                1080                1085
   Asn Glu Ser Gly Thr Lys Leu Ser Tyr Gln Asp Thr Ile Asn Ser Ile
       1090                1095                1100
   Ala Leu Met Ile Met Arg Ser Ile Asp Pro Thr Ala Lys Lys Gln Tyr
   1105                1110                1115                1120
   Ile Arg Val Pro Leu Asn Thr Leu Asn Leu His Leu Gly Asp Gln Asp
                   1125                1130                1135
   Phe Asp Leu His Asn Ile Asp Ala Tyr Leu Lys Lys Pro Lys Phe Val
               1140                1145                1150
   Lys Tyr Leu Lys Ala Asn Glu Ile Gly Asp Glu Tyr Lys Pro Trp Arg
           1155                1160                1165
   Val Leu Thr Ser Gly Thr Leu Leu Ile His Lys Lys Asp Lys Lys Leu
       1170                1175                1180
   Met Tyr Ile Ser Ser Phe Gln Asn Leu Lys Asp Val Ile Glu Ile Lys
   1185                1190                1195                1200
   Asn Leu Ile Glu Thr Glu Tyr Lys Glu Asn Asp Asp Ser Asp Pro Lys
                   1205                1210                1215
   Lys Lys Lys Lys Ala Asn Arg Phe Leu Met Thr Leu Ser Thr Ile Leu
               1220                1225                1230
   Asn Asp Tyr Ile Leu Leu Asp Ala Lys Asp Asn Phe Asp Ile Leu Gly
           1235                1240                1245
   Leu Ser Lys Asn Arg Ile Asp Glu Ile Leu Asn Ser Lys Leu Gly Leu
       1250                1255                1260
   Asp Lys Ile Thr Lys
   1265
<210> 150
   <211> 1269
   <212> PRT
   <213> Mycoplasma gallisepticum
<220>
   <223> CRISPR-associated endonuclease
<308> YP_006584600
   <309> 2013-06-11
<400> 150
   Met Asn Asn Ser Ile Lys Ser Lys Pro Glu Val Thr Ile Gly Leu Asp
    1               5                  10                  15
   Leu Gly Val Gly Ser Val Gly Trp Ala Ile Val Asp Asn Glu Thr Asn
               20                  25                  30
   Ile Ile His His Leu Gly Ser Arg Leu Phe Ser Gln Ala Lys Thr Ala
           35                  40                  45
   Glu Asp Arg Arg Ser Phe Arg Gly Ala Arg Arg Leu Ile Arg Arg Arg
       50                  55                  60
   Lys Tyr Lys Leu Lys Arg Phe Ile Asn Leu Ile Trp Lys Tyr Asn Ser
   65                  70                  75                  80
   Tyr Phe Gly Phe Lys Asn Lys Glu Asp Ile Leu Asn Asn Tyr Gln Glu
   85 90 95
   Gln Gln Lys Leu His Asn Thr Val Leu Asn Leu Lys Leu Glu Ala Leu
   100 105 110
   Asn Ala Lys Ile Asp Pro Lys Ala Leu Ser Trp Ile Leu His Asp Tyr
   115 120 125
   Leu Lys Asn Arg Gly His Phe Tyr Glu Asp Asn Arg Asp Phe Asn Val
   130 135 140
   Tyr Pro Thr Glu Glu Leu Ala Asn Tyr Phe Gly Glu Tyr Gly Tyr Tyr
   145 150 155 160
   Lys Gly Ile Ile Asp Ser Lys Glu Asp Asn Asp Asp Lys Leu Glu Glu
   165 170 175
   Gly Leu Thr Lys Tyr Lys Phe Ser Asn Gln His Trp Leu Glu Glu Ile
   180 185 190
   Lys Lys Val Leu Ser Asn Gln Thr Gly Leu Pro Glu Lys Phe Lys Glu
   195 200 205
   Glu Tyr Glu Ser Leu Phe Ser Tyr Val Arg Asn Tyr Ser Glu Gly Pro
   210 215 220
   Gly Ser Ile Asn Ser Val Ser Pro Tyr Gly Ile Tyr His Leu Asp Glu
   225 230 235 240
   Lys Glu Gly Lys Val Val Gln Lys Tyr Asn Asn Ile Trp Asp Lys Thr
   245 250 255
   Ile Gly Lys Cys Ser Ile Phe Pro Asp Glu Tyr Arg Ala Pro Lys Asn
   260 265 270
   Ser Pro Ile Ala Met Ile Phe Asn Glu Ile Asn Glu Leu Ser Thr Ile
   275 280 285
   Arg Ser Tyr Ser Ile Tyr Leu Thr Gly Trp Phe Ile Asn Gln Glu Phe
   290 295 300
   Lys Lys Ala Tyr Leu Asn Lys Leu Leu Asp Leu Leu Ile Lys Thr Asn
   305 310 315 320
   Ser Glu Lys Pro Ile Asp Ala Arg Gln Phe Lys Lys Leu Arg Glu Glu
   325 330 335
   Thr Ile Ala Glu Ser Ile Gly Lys Glu Thr Leu Lys Asp Val Glu Arg
   340 345 350
   Glu Glu Lys Leu Glu Lys Glu Asp His Lys Trp Lys Leu Lys Gly Leu
   355 360 365
   Lys Leu Asn Thr Asn Gly Lys Ile Gln Tyr Asn Asp Leu Ser Ser Leu
   370 375 380
   Ala Lys Phe Val His Lys Leu Lys Gln His Leu Lys Leu Asp Phe Leu
   385 390 395 400
   Leu Glu Asp Gln Tyr Thr Thr Leu Asp Lys Ile Asn Phe Leu Gln Ser
   405 410 415
   Leu Phe Val Tyr Leu Gly Lys His Leu Arg Tyr Ser Asn Arg Val Asp
   420 425 430
   Ser Ala Asn Leu Lys Glu Phe Ser Asp Ser Asn Lys Leu Phe Glu Arg
   435 440 445
   Val Leu Gln Glu Gln Lys Asp Asp Leu Phe Lys Leu Phe Glu Gln Thr
   450 455 460
   Asp Lys Asp Asp Glu Lys Ile Leu Ala Gln Thr His Ser Leu Ser Thr
   465 470 475 480
   Lys Ala Met Leu Leu Ala Ile Thr Arg Met Thr Asn Leu Asp Asn Asp
   485 490 495
   Glu Asp Asn Gln Lys Asn Asn Asp Lys Gly Trp Asn Phe Glu Ala Ile
   500 505 510
   Lys Asn Phe Asp Gln Lys Phe Ile Asp Ile Thr Lys Lys Asn Asn Asn
   515 520 525
   Leu Ser Leu Lys Gln Asp Lys Arg Tyr Leu Asp Asp Arg Phe Ile Asn
   530 535 540
   Asp Ala Ile Leu Ser Pro Gly Val Lys Arg Ile Leu Arg Glu Ala Thr
   545 550 555 560
   Lys Val Phe Asn Ala Ile Leu Lys Gln Phe Ser Glu Glu Tyr Asp Val
   565 570 575
   Thr Lys Val Val Ile Glu Leu Ala Arg Glu Leu Ser Glu Glu Lys Glu
   580 585 590
   Leu Glu Asn Thr Lys Asn Tyr Glu Lys Leu Ile Lys Lys Asn Ser Asp
   595 600 605
   Lys Ile Ser Glu Arg Leu Glu Ala Leu Asp Ile Ala Glu Asp Lys Ile
   610 615 620
   Glu Asp Ile Leu Lys Ser Pro Thr Lys Ser Tyr Lys Val Leu Leu Trp
   625 630 635 640
   Leu Gln Gln Asp His Ile Asp Pro Tyr Ser Gln Lys Glu Ile Ala Phe
   645 650 655
   Glu Asp Ile Leu Thr Lys Thr Glu Lys Thr Glu Ile Asp His Ile Ile
   660 665 670
   Pro Tyr Ser Ile Ser Phe Asp Asp Ser Ser Ser Asn Lys Leu Leu Val
   675 680 685
   Leu Ala Glu Ser Asn Gln Ala Lys Ser Asn Gln Thr Pro Tyr Glu Phe
   690 695 700
   Ile Thr Ser Gly Asn Ala Gly Ile Lys Trp Glu Asp Tyr Glu Ala Tyr
   705 710 715 720
   Cys Arg Lys Phe Lys Asp Gly Asp Thr Ser Leu Leu Asp Ser Thr Gln
   725 730 735
   Arg Ser Lys Lys Phe Ala Lys Met Met Lys Thr Asp Thr Ser Ser Lys
   740 745 750
   Tyr Asp Ile Gly Phe Leu Ala Arg Asn Leu Asn Asp Thr Arg Tyr Ala
   755 760 765
   Thr Ile Val Phe Arg Asp Ala Leu Lys Asp Tyr Ala Asn Asn His Leu
   770 775 780
   Val Glu Asp Lys Pro Met Phe Lys Val Val Cys Ile Asn Gly Gly Val
   785 790 795 800
   Thr Ser Phe Leu Arg Lys Asn Phe Asp Lys Ser Trp Tyr Ala Lys Lys
   805 810 815
   Asp Arg Asp Lys Asn Ile His His Ala Val Asp Ala Ser Ile Ile Ser
   820 825 830
   Ile Phe Ser Asn Glu Thr Lys Thr Leu Phe Asn Gln Leu Thr Gln Phe
   835 840 845
   Ala Asp Tyr Lys Leu Phe Lys Asn Thr Asp Gly Ser Trp Lys Lys Ile
   850 855 860
   Asp Pro Lys Thr Gly Val Val Thr Glu Val Thr Asp Glu Asn Trp Lys
   865 870 875 880
   Gln Ile Arg Val Arg Asn Gln Val Ser Glu Ile Ala Lys Val Ile Asn
   885 890 895
   Lys Cys Ile Gln Asp Ser Asn Ile Glu Arg Lys Ala Arg Tyr Ser Arg
   900 905 910
   Lys Ile Glu Asn Lys Thr Asn Ile Ser Leu Phe Asn Asp Thr Val Tyr
   915 920 925
   Ser Ala Lys Lys Val Gly Tyr Glu Asp Gln Ile Lys Arg Lys Asn Leu
   930 935 940
   Lys Thr Leu Asp Ile His Glu Ser Ala Glu Glu Asn Lys Asn Ser Lys
   945 950 955 960
   Val Lys Lys Gln Phe Val Tyr Arg Lys Leu Val Asn Val Ser Leu Leu
   965 970 975
   Asn Asn Asp Lys Leu Ala Asp Leu Phe Ala Glu Lys Glu Asp Ile Leu
   980 985 990
   Met Tyr Arg Ala Asn Pro Trp Val Ile Asn Leu Ala Glu Gln Ile Phe
   995 1000 1005
   Asn Glu Tyr Thr Glu Asn Lys Lys Ile Lys Ser Gln Asn Val Phe Glu
   1010 1015 1020
   Lys Tyr Met Leu Asp Leu Thr Lys Glu Phe Pro Glu Lys Phe Ser Glu
   1025 1030 1035 1040
   Ala Phe Val Lys Ser Met Leu Arg Asn Lys Thr Ala Ile Ile Tyr Asn
   1045 1050 1055
   Val Glu Lys Lys Val Val His Arg Ile Lys Arg Leu Lys Ile Leu Ser
   1060 1065 1070
   Ser Glu Leu Lys Glu Asn Lys Leu Ser Asn Val Ile Ile Arg Ser Lys
   1075 1080 1085
   Asn Glu Ser Gly Thr Lys Leu Ser Tyr Gln Asp Thr Ile Asn Ser Ile
       1090                1095                1100
   Ala Leu Met Ile Met Arg Ser Ile Asp Pro Thr Ala Lys Lys Gln Tyr
   1105                1110                1115                1120
   Ile Arg Val Pro Leu Asn Thr Leu Asn Leu His Leu Gly Asp Gln Asp
                   1125                1130                1135
   Phe Asp Leu His Asn Ile Asp Ala Tyr Leu Lys Lys Pro Lys Phe Val
               1140                1145                1150
   Lys Tyr Leu Lys Ala Asn Glu Ile Gly Asp Glu Tyr Lys Pro Trp Arg
           1155                1160                1165
   Val Leu Thr Ser Gly Thr Leu Leu Ile His Lys Lys Asp Lys Lys Leu
       1170                1175                1180
   Met Tyr Ile Ser Ser Phe Gln Asn Leu Lys Asp Val Ile Glu Ile Lys
   1185                1190                1195                1200
   Asn Leu Ile Glu Thr Glu Tyr Lys Glu Asn Asp Asp Ser Asp Pro Lys
                   1205                1210                1215
   Lys Lys Lys Lys Ala Asn Arg Phe Leu Met Thr Leu Ser Thr Ile Leu
               1220                1225                1230
   Asn Asp Tyr Ile Leu Leu Asp Ala Lys Asp Asn Phe Asp Ile Leu Gly
           1235                1240                1245
   Leu Ser Lys Asn Arg Ile Asp Glu Ile Leu Asn Ser Lys Leu Gly Leu
       1250                1255                1260
   Asp Lys Ile Thr Lys
   1265
<210> 151
   <211> 1224
   <212> PRT
   <213> Mycoplasma gallisepticum
<220>
   <223> CRISPR-associated endonuclease
<308> YP_006583854
   <309> 2013-06-11
<400> 151
   Met Asn Asn Ser Ile Lys Ser Lys Pro Glu Val Thr Ile Gly Leu Asp
   1 5 10 15
   Leu Gly Val Gly Ser Val Gly Trp Ala Ile Val Asp Asn Glu Thr Asn
   20 25 30
   Ile Ile His His Leu Gly Ser Arg Leu Phe Ser Gln Ala Lys Thr Ala
   35 40 45
   Glu Asp Arg Arg Ser Phe Arg Gly Ala Arg Arg Leu Ile Arg Arg Arg
   50 55 60
   Lys Tyr Lys Leu Lys Arg Phe Ile Asn Leu Ile Trp Lys Tyr Asn Ser
   65 70 75 80
   Tyr Phe Gly Phe Lys Asn Lys Glu Asp Ile Leu Asn Asn Tyr Gln Glu
   85 90 95
   Gln Gln Lys Leu His Asn Thr Val Leu Asn Leu Lys Leu Glu Ala Leu
   100 105 110
   Asn Ala Lys Ile Asp Pro Lys Ala Leu Ser Trp Ile Leu His Asp Tyr
   115 120 125
   Leu Lys Asn Arg Gly His Phe Tyr Glu Asp Asn Arg Asp Phe Asn Val
   130 135 140
   Tyr Pro Thr Glu Glu Leu Ala Asn Tyr Phe Gly Glu Tyr Gly Tyr Tyr
   145 150 155 160
   Lys Gly Ile Ile Asp Ser Lys Glu Asp Asn Asp Asp Lys Leu Glu Glu
   165 170 175
   Gly Leu Thr Lys Tyr Lys Phe Ser Asn Gln His Trp Leu Glu Glu Ile
   180 185 190
   Lys Lys Val Leu Ser Asn Gln Thr Gly Leu Pro Glu Lys Phe Lys Glu
           195                 200                 205
   Glu Tyr Glu Ser Leu Phe Ser Tyr Val Arg Asn Tyr Ser Glu Gly Pro
       210                 215                 220
   Gly Ser Ile Asn Ser Val Ser Pro Tyr Gly Ile Tyr His Leu Asp Glu
   225                 230                 235                 240
   Lys Glu Gly Lys Val Val Gln Lys Tyr Asn Asn Ile Trp Asp Lys Thr
                   245                 250                 255
   Ile Gly Lys Cys Ser Ile Phe Pro Asp Glu Tyr Arg Ala Pro Lys Asn
               260                 265                 270
   Ser Pro Ile Ala Met Ile Phe Asn Glu Ile Asn Glu Leu Ser Thr Ile
           275                 280                 285
   Arg Ser Tyr Ser Ile Tyr Leu Thr Gly Trp Phe Ile Asn Gln Glu Phe
       290                 295                 300
   Lys Lys Ala Tyr Leu Asn Lys Leu Leu Asp Leu Leu Ile Lys Thr Asn
   305                 310                 315                 320
   Ser Glu Lys Pro Ile Asp Ala Arg Gln Phe Lys Lys Leu Arg Glu Glu
                   325                 330                 335
   Thr Ile Ala Glu Ser Ile Gly Lys Glu Thr Leu Lys Asp Val Glu Arg
               340                 345                 350
   Glu Glu Lys Leu Glu Lys Glu Asp His Lys Trp Lys Leu Lys Gly Leu
           355                 360                 365
   Lys Leu Asn Thr Asn Gly Lys Ile Gln Tyr Asn Asp Leu Ser Ser Leu
       370                 375                 380
   Ala Lys Phe Val His Lys Leu Lys Gln His Leu Lys Leu Asp Phe Leu
   385                 390                 395                 400
   Leu Glu Asp Gln Tyr Thr Thr Leu Asp Lys Ile Asn Phe Leu Gln Ser
                   405                 410                 415
   Leu Phe Val Tyr Leu Gly Lys His Leu Arg Tyr Ser Asn Arg Val Asp
               420                 425                 430
   Ser Ala Asn Leu Lys Glu Phe Ser Asp Ser Asn Lys Leu Phe Glu Arg
           435                 440                 445
   Val Leu Gln Glu Gln Lys Asp Asp Leu Phe Lys Leu Phe Glu Gln Thr
       450                 455                 460
   Asp Lys Asp Asp Glu Lys Ile Leu Ala Gln Thr His Ser Leu Ser Thr
   465                 470                 475                 480
   Lys Ala Met Leu Leu Ala Ile Thr Arg Met Thr Asn Leu Asp Asn Asp
                   485                 490                 495
   Glu Asp Asn Gln Lys Asn Asn Asp Lys Gly Trp Asn Phe Glu Ala Ile
               500                 505                 510
   Lys Asn Phe Asp Gln Lys Phe Ile Asp Ile Thr Lys Lys Asn Asn Asn
           515                 520                 525
   Leu Ser Leu Lys Gln Asp Lys Arg Tyr Leu Asp Asp Arg Phe Ile Asn
       530                 535                 540
   Asp Ala Ile Leu Ser Pro Gly Val Lys Arg Ile Leu Arg Glu Ala Thr
   545                 550                 555                 560
   Lys Val Phe Asn Ala Ile Leu Lys Gln Phe Ser Glu Glu Tyr Asp Val
                   565                 570                 575
   Thr Lys Val Val Ile Glu Leu Ala Arg Glu Leu Ser Glu Glu Lys Glu
               580                 585                 590
   Leu Glu Asn Thr Lys Asn Tyr Glu Lys Leu Ile Lys Lys Asn Ser Asp
           595                 600                 605
   Lys Ile Ser Glu Arg Leu Glu Ala Leu Asp Ile Ala Glu Asp Lys Ile
       610                 615                 620
   Glu Asp Ile Leu Lys Ser Pro Thr Lys Ser Tyr Lys Val Leu Leu Trp
   625                 630                 635                 640
   Leu Gln Gln Asp His Ile Asp Pro Tyr Ser Gln Lys Glu Ile Ala Phe
                   645                 650                 655
   Glu Asp Ile Leu Thr Lys Thr Glu Lys Thr Glu Ile Asp His Ile Ile
               660                 665                 670
   Pro Tyr Ser Ile Ser Phe Asp Asp Ser Ser Ser Asn Lys Leu Leu Val
           675                 680                 685
   Leu Ala Glu Ser Asn Gln Ala Lys Ser Asn Gln Thr Pro Tyr Glu Phe
       690                 695                 700
   Ile Thr Ser Gly Asn Ala Gly Ile Lys Trp Glu Asp Tyr Glu Ala Tyr
   705                 710                 715                 720
   Cys Arg Lys Phe Lys Asp Gly Asp Thr Ser Leu Leu Asp Ser Thr Gln
                   725                 730                 735
   Arg Ser Lys Lys Phe Ala Lys Met Met Lys Thr Asp Thr Ser Ser Lys
               740                 745                 750
   Tyr Asp Ile Gly Phe Leu Ala Arg Asn Leu Asn Asp Thr Arg Tyr Ala
           755                 760                 765
   Thr Ile Val Phe Arg Asp Ala Leu Lys Asp Tyr Ala Asn Asn His Leu
       770                 775                 780
   Val Glu Asp Lys Pro Met Phe Lys Val Val Cys Ile Asn Gly Gly Val
   785                 790                 795                 800
   Thr Ser Phe Leu Arg Lys Asn Phe Asp Lys Ser Trp Tyr Ala Lys Lys
                   805                 810                 815
   Asp Arg Asp Lys Asn Ile His His Ala Val Asp Ala Ser Ile Ile Ser
               820                 825                 830
   Ile Phe Ser Asn Glu Thr Lys Thr Leu Phe Asn Gln Leu Thr Gln Phe
           835                 840                 845
   Ala Asp Tyr Lys Leu Phe Lys Asn Thr Asp Gly Ser Trp Lys Lys Ile
       850                 855                 860
   Asp Pro Lys Thr Gly Val Val Thr Glu Val Thr Asp Glu Asn Trp Lys
   865                 870                 875                 880
   Gln Ile Arg Val Arg Asn Gln Val Ser Glu Ile Ala Lys Val Ile Asn
                   885                 890                 895
   Lys Cys Ile Gln Asp Ser Asn Ile Glu Arg Lys Ala Arg Tyr Ser Arg
               900                 905                 910
   Lys Ile Glu Asn Lys Thr Asn Ile Ser Leu Phe Asn Asp Thr Val Tyr
           915                 920                 925
   Ser Ala Lys Lys Val Gly Tyr Glu Asp Gln Ile Lys Arg Lys Asn Leu
       930                 935                 940
   Lys Thr Leu Asp Ile His Glu Ser Ala Glu Glu Asn Lys Asn Ser Lys
   945                 950                 955                 960
   Val Lys Lys Gln Phe Val Tyr Arg Lys Leu Val Asn Val Ser Leu Leu
                   965                 970                 975
   Asn Asn Asp Lys Leu Ala Asp Leu Phe Ala Glu Lys Glu Asp Ile Leu
               980                 985                 990
   Met Tyr Arg Ala Asn Pro Trp Val Ile Asn Leu Ala Glu Gln Ile Phe
           995                 1000                1005
   Asn Glu Tyr Thr Glu Asn Lys Lys Ile Lys Ser Gln Asn Val Phe Glu
       1010                1015                1020
   Lys Tyr Met Leu Asp Leu Thr Lys Glu Phe Pro Glu Lys Phe Ser Glu
   1025                1030                1035                1040
   Ala Phe Val Lys Ser Met Leu Arg Asn Lys Thr Ala Ile Ile Tyr Asn
                   1045                1050                1055
   Val Glu Lys Lys Val Val His Arg Ile Lys Arg Leu Lys Ile Leu Ser
               1060                1065                1070
   Ser Glu Leu Lys Glu Asn Lys Leu Ser Asn Val Ile Ile Arg Ser Lys
           1075                1080                1085
   Asn Glu Ser Gly Thr Lys Leu Ser Tyr Gln Asp Thr Ile Asn Ser Ile
       1090                1095                1100
   Ala Leu Met Ile Met Arg Ser Ile Asp Pro Thr Ala Lys Lys Gln Tyr
   1105                1110                1115                1120
   Ile Arg Val Pro Leu Asn Thr Leu Asn Leu His Leu Gly Asp Gln Asp
                   1125                1130                1135
   Phe Asp Leu His Asn Ile Asp Ala Tyr Leu Lys Lys Pro Lys Phe Val
               1140                1145                1150
   Lys Tyr Leu Lys Ala Asn Glu Ile Gly Asp Glu Tyr Lys Pro Trp Arg
           1155                1160                1165
   Val Leu Thr Ser Gly Thr Leu Leu Ile His Lys Lys Asp Lys Lys Leu
       1170                1175                1180
   Met Tyr Ile Ser Ser Phe Gln Asn Leu Lys Asp Val Ile Glu Ile Lys
   1185                1190                1195                1200
   Asn Leu Ile Glu Thr Glu Tyr Lys Glu Asn Asp Asp Ser Asp Pro Lys
   1205 1210 1215
   Lys Arg Arg Lys Gln Ile Gly Ser
   1220
<210> 152
   <211> 1269
   <212> PRT
   <213> Mycoplasma gallisepticum
<220>
   <223> CRISPR-associated endonuclease
<308> YP_006583094
   <309> 2013-06-11
<400> 152
   Met Asn Asn Ser Ile Lys Ser Lys Pro Glu Val Thr Ile Gly Leu Asp
   1               5                   10                  15
   Leu Gly Val Gly Ser Val Gly Trp Ala Ile Val Asp Asn Glu Thr Asn
               20                  25                  30
   Ile Ile His His Leu Gly Ser Arg Leu Phe Ser Gln Ala Lys Thr Ala
           35                  40                  45
   Glu Asp Arg Arg Ser Phe Arg Gly Ala Arg Arg Leu Ile Arg Arg Arg
       50                  55                  60
   Lys Tyr Lys Leu Lys Arg Phe Ile Asn Leu Ile Trp Lys Tyr Asn Ser
   65                  70                  75                  80
   Tyr Phe Gly Phe Lys Asn Lys Glu Asp Ile Leu Asn Asn Tyr Gln Glu
                   85                  90                  95
   Gln Gln Lys Leu His Asn Thr Val Leu Asn Leu Lys Leu Glu Ala Leu
               100                 105                 110
   Asn Ala Lys Ile Asp Pro Lys Ala Leu Ser Trp Ile Leu His Asp Tyr
           115                 120                 125
   Leu Lys Asn Arg Gly His Phe Tyr Glu Asp Asn Arg Asp Phe Asn Val
       130                 135                 140
   Tyr Pro Thr Glu Glu Leu Ala Asn Tyr Phe Gly Glu Tyr Gly Tyr Tyr
   145                 150                 155                 160
   Lys Gly Ile Ile Asp Ser Lys Glu Asp Asn Asp Asp Lys Leu Glu Glu
                   165                 170                 175
   Gly Leu Thr Lys Tyr Lys Phe Ser Asn Gln His Trp Leu Glu Glu Ile
               180                 185                 190
   Lys Lys Val Leu Ser Asn Gln Thr Gly Leu Pro Glu Lys Phe Lys Glu
           195                 200                 205
   Glu Tyr Glu Ser Leu Phe Ser Tyr Val Arg Asn Tyr Ser Glu Gly Pro
       210                 215                 220
   Gly Ser Ile Asn Ser Val Ser Pro Tyr Gly Ile Tyr His Leu Asp Glu
   225                 230                 235                 240
   Lys Glu Gly Lys Val Val Gln Lys Tyr Asn Asn Ile Trp Asp Lys Thr
                   245                 250                 255
   Ile Gly Lys Cys Ser Ile Phe Pro Asp Glu Tyr Arg Ala Pro Lys Asn
               260                 265                 270
   Ser Pro Ile Ala Met Ile Phe Asn Glu Ile Asn Glu Leu Ser Thr Ile
           275                 280                 285
   Arg Ser Tyr Ser Ile Tyr Leu Thr Gly Trp Phe Ile Asn Gln Glu Phe
       290                 295                 300
   Lys Lys Ala Tyr Leu Asn Lys Leu Leu Asp Leu Leu Ile Lys Thr Asn
   305                 310                 315                 320
   Ser Glu Lys Pro Ile Asp Ala Arg Gln Phe Lys Lys Leu Arg Glu Glu
                   325                 330                 335
   Thr Ile Ala Glu Ser Ile Gly Lys Glu Thr Leu Lys Asp Val Glu Arg
               340                 345                 350
   Glu Glu Lys Leu Glu Lys Glu Asp His Lys Trp Lys Leu Lys Gly Leu
           355                 360                 365
   Lys Leu Asn Thr Asn Gly Lys Ile Gln Tyr Asn Asp Leu Ser Ser Leu
   370 375 380
   Ala Lys Phe Val His Lys Leu Lys Gln His Leu Lys Leu Asp Phe Leu
   385 390 395 400
   Leu Glu Asp Gln Tyr Thr Thr Leu Asp Lys Ile Asn Phe Leu Gln Ser
   405 410 415
   Leu Phe Val Tyr Leu Gly Lys His Leu Arg Tyr Ser Asn Arg Val Asp
   420 425 430
   Ser Ala Asn Leu Lys Glu Phe Ser Asp Ser Asn Lys Leu Phe Glu Arg
   435 440 445
   Val Leu Gln Glu Gln Lys Asp Asp Leu Phe Lys Leu Phe Glu Gln Thr
   450 455 460
   Asp Lys Asp Asp Glu Lys Ile Leu Ala Gln Thr His Ser Leu Ser Thr
   465 470 475 480
   Lys Ala Met Leu Leu Ala Ile Thr Arg Met Thr Asn Leu Asp Asn Asp
   485 490 495
   Glu Asp Asn Gln Lys Asn Asn Asp Lys Gly Trp Asn Phe Glu Ala Ile
   500 505 510
   Lys Asn Phe Asp Gln Lys Phe Ile Asp Ile Thr Lys Lys Asn Asn Asn
   515 520 525
   Leu Ser Leu Lys Gln Asp Lys Arg Tyr Leu Asp Asp Arg Phe Ile Asn
   530 535 540
   Asp Ala Ile Leu Ser Pro Gly Val Lys Arg Ile Leu Arg Glu Ala Thr
   545 550 555 560
   Lys Val Phe Asn Ala Ile Leu Lys Gln Phe Ser Glu Glu Tyr Asp Val
   565 570 575
   Thr Lys Val Val Ile Glu Leu Ala Arg Glu Leu Ser Glu Glu Lys Glu
   580 585 590
   Leu Glu Asn Thr Lys Asn Tyr Glu Lys Leu Ile Lys Lys Asn Ser Asp
   595 600 605
   Lys Ile Ser Glu Arg Leu Glu Ala Leu Asp Ile Ala Glu Asp Lys Ile
   610 615 620
   Glu Asp Ile Leu Lys Ser Pro Thr Lys Ser Tyr Lys Val Leu Leu Trp
   625 630 635 640
   Leu Gln Gln Asp His Ile Asp Pro Tyr Ser Gln Lys Glu Ile Ala Phe
   645 650 655
   Glu Asp Ile Leu Thr Lys Thr Glu Lys Thr Glu Ile Asp His Ile Ile
   660 665 670
   Pro Tyr Ser Ile Ser Phe Asp Asp Ser Ser Ser Asn Lys Leu Leu Val
   675 680 685
   Leu Ala Glu Ser Asn Gln Ala Lys Ser Asn Gln Thr Pro Tyr Glu Phe
   690 695 700
   Ile Thr Ser Gly Asn Ala Gly Ile Lys Trp Glu Asp Tyr Glu Ala Tyr
   705 710 715 720
   Cys Arg Lys Phe Lys Asp Gly Asp Thr Ser Leu Leu Asp Ser Thr Gln
   725 730 735
   Arg Ser Lys Lys Phe Ala Lys Met Met Lys Thr Asp Thr Ser Ser Lys
   740 745 750
   Tyr Asp Ile Gly Phe Leu Ala Arg Asn Leu Asn Asp Thr Arg Tyr Ala
   755 760 765
   Thr Ile Val Phe Arg Asp Ala Leu Lys Asp Tyr Ala Asn Asn His Leu
   770 775 780
   Val Glu Asp Lys Pro Met Phe Lys Val Val Cys Ile Asn Gly Gly Val
   785 790 795 800
   Thr Ser Phe Leu Arg Lys Asn Phe Asp Lys Ser Trp Tyr Ala Lys Lys
   805 810 815
   Asp Arg Asp Lys Asn Ile His His Ala Val Asp Ala Ser Ile Ile Ser
   820 825 830
   Ile Phe Ser Asn Glu Thr Lys Thr Leu Phe Asn Gln Leu Thr Gln Phe
   835 840 845
   Ala Asp Tyr Lys Leu Phe Lys Asn Thr Asp Gly Ser Trp Lys Lys Ile
   850 855 860
   Asp Pro Lys Thr Gly Val Val Thr Glu Val Thr Asp Glu Asn Trp Lys
   865                 870                 875                 880
   Gln Ile Arg Val Arg Asn Gln Val Ser Glu Ile Ala Lys Val Ile Asn
                   885                 890                 895
   Lys Cys Ile Gln Asp Ser Asn Ile Glu Arg Lys Ala Arg Tyr Ser Arg
               900                 905                 910
   Lys Ile Glu Asn Lys Thr Asn Ile Ser Leu Phe Asn Asp Thr Val Tyr
           915                 920                 925
   Ser Ala Lys Lys Val Gly Tyr Glu Asp Gln Ile Lys Arg Lys Asn Leu
       930                 935                 940
   Lys Thr Leu Asp Ile His Glu Ser Ala Glu Glu Asn Lys Asn Ser Lys
   945                 950                 955                 960
   Val Lys Lys Gln Phe Val Tyr Arg Lys Leu Val Asn Val Ser Leu Leu
                   965                 970                 975
   Asn Asn Asp Lys Leu Ala Asp Leu Phe Ala Glu Lys Glu Asp Ile Leu
               980                 985                 990
   Met Tyr Arg Ala Asn Pro Trp Val Ile Asn Leu Ala Glu Gln Ile Phe
           995                 1000                1005
   Asn Glu Tyr Thr Glu Asn Lys Lys Ile Lys Ser Gln Asn Val Phe Glu
       1010                1015                1020
   Lys Tyr Met Leu Asp Leu Thr Lys Glu Phe Pro Glu Lys Phe Ser Glu
   1025                1030                1035                1040
   Ala Phe Val Lys Ser Met Leu Arg Asn Lys Thr Ala Ile Ile Tyr Asn
                   1045                1050                1055
   Val Glu Lys Lys Val Val His Arg Ile Lys Arg Leu Lys Ile Leu Ser
               1060                1065                1070
   Ser Glu Leu Lys Glu Asn Lys Leu Ser Asn Val Ile Ile Arg Ser Lys
           1075                1080                1085
   Asn Glu Ser Gly Thr Lys Leu Ser Tyr Gln Asp Thr Ile Asn Ser Ile
       1090                1095                1100
   Ala Leu Met Ile Met Arg Ser Ile Asp Pro Thr Ala Lys Lys Gln Tyr
   1105                1110                1115                1120
   Ile Arg Val Pro Leu Asn Thr Leu Asn Leu His Leu Gly Asp Gln Asp
                   1125                1130                1135
   Phe Asp Leu His Asn Ile Asp Ala Tyr Leu Lys Lys Pro Lys Phe Val
               1140                1145                1150
   Lys Tyr Leu Lys Ala Asn Glu Ile Gly Asp Glu Tyr Lys Pro Trp Arg
           1155                1160                1165
   Val Leu Thr Ser Gly Thr Leu Leu Ile His Lys Lys Asp Lys Lys Leu
       1170                1175                1180
   Met Tyr Ile Ser Ser Phe Gln Asn Leu Lys Asp Val Ile Glu Ile Lys
   1185                1190                1195                1200
   Asn Leu Ile Glu Thr Glu Tyr Lys Glu Asn Asp Asp Ser Asp Pro Lys
                   1205                1210                1215
   Lys Lys Lys Lys Ala Asn Arg Phe Leu Met Thr Leu Ser Thr Ile Leu
               1220                1225                1230
   Asn Asp Tyr Ile Leu Leu Asp Ala Lys Asp Asn Phe Asp Ile Leu Gly
           1235                1240                1245
   Leu Ser Lys Asn Arg Ile Asp Glu Ile Leu Asn Ser Lys Leu Gly Leu
       1250                1255                1260
   Asp Lys Ile Thr Lys
   1265
<210> 153
   <211> 1269
   <212> PRT
   <213> Mycoplasma gallisepticum
<220>
   <223> CRISPR-associated endonuclease
<308> YP_006582323
   <309> 2013-06-11
<400> 153
   Met Asn Asn Ser Ile Lys Ser Lys Pro Glu Val Thr Ile Gly Leu Asp
    1               5                  10                  15
   Leu Gly Val Gly Ser Val Gly Trp Ala Ile Val Asp Asn Glu Thr Asn
               20                  25                  30
   Ile Ile His His Leu Gly Ser Arg Leu Phe Ser Gln Ala Lys Thr Ala
           35                  40                  45
   Glu Asp Arg Arg Ser Phe Arg Gly Ala Arg Arg Leu Ile Arg Arg Arg
       50                  55                  60
   Lys Tyr Lys Leu Lys Arg Phe Ile Asn Leu Ile Trp Lys Tyr Asn Ser
   65                  70                  75                  80
   Tyr Phe Gly Phe Lys Asn Lys Glu Asp Ile Leu Asn Asn Tyr Gln Glu
                   85                  90                  95
   Gln Gln Lys Leu His Asn Thr Val Leu Asn Leu Lys Leu Glu Ala Leu
               100                 105                 110
   Asn Ala Lys Ile Asp Pro Lys Ala Leu Ser Trp Ile Leu His Asp Tyr
           115                 120                 125
   Leu Lys Asn Arg Gly His Phe Tyr Glu Asp Asn Arg Asp Phe Asn Val
       130                 135                 140
   Tyr Pro Thr Glu Glu Leu Ala Asn Tyr Phe Gly Glu Tyr Gly Tyr Tyr
   145                 150                 155                 160
   Lys Gly Ile Ile Asp Ser Lys Glu Asp Asn Asp Asp Lys Leu Glu Glu
                   165                 170                 175
   Gly Leu Thr Lys Tyr Lys Phe Ser Asn Gln His Trp Leu Glu Glu Ile
               180                 185                 190
   Lys Lys Val Leu Ser Asn Gln Thr Gly Leu Pro Glu Lys Phe Lys Glu
           195                 200                 205
   Glu Tyr Glu Ser Leu Phe Ser Tyr Val Arg Asn Tyr Ser Glu Gly Pro
       210                 215                 220
   Gly Ser Ile Asn Ser Val Ser Pro Tyr Gly Ile Tyr His Leu Asp Glu
   225                 230                 235                 240
   Lys Glu Gly Lys Val Val Gln Lys Tyr Asn Asn Ile Trp Asp Lys Thr
                   245                 250                 255
   Ile Gly Lys Cys Ser Ile Phe Pro Asp Glu Tyr Arg Ala Pro Lys Asn
               260                 265                 270
   Ser Pro Ile Ala Met Ile Phe Asn Glu Ile Asn Glu Leu Ser Thr Ile
           275                 280                 285
   Arg Ser Tyr Ser Ile Tyr Leu Thr Gly Trp Phe Ile Asn Gln Glu Phe
       290                 295                 300
   Lys Lys Ala Tyr Leu Asn Lys Leu Leu Asp Leu Leu Ile Lys Thr Asn
   305                 310                 315                 320
   Ser Glu Lys Pro Ile Asp Ala Arg Gln Phe Lys Lys Leu Arg Glu Glu
                   325                 330                 335
   Thr Ile Ala Glu Ser Ile Gly Lys Glu Thr Leu Lys Asp Val Glu Arg
               340                 345                 350
   Glu Glu Lys Leu Glu Lys Glu Asp His Lys Trp Lys Leu Lys Gly Leu
           355                 360                 365
   Lys Leu Asn Thr Asn Gly Lys Ile Gln Tyr Asn Asp Leu Ser Ser Leu
       370                 375                 380
   Ala Lys Phe Val His Lys Leu Lys Gln His Leu Lys Leu Asp Phe Leu
   385                 390                 395                 400
   Leu Glu Asp Gln Tyr Thr Thr Leu Asp Lys Ile Asn Phe Leu Gln Ser
                   405                 410                 415
   Leu Phe Val Tyr Leu Gly Lys His Leu Arg Tyr Ser Asn Arg Val Asp
               420                 425                 430
   Ser Ala Asn Leu Lys Glu Phe Ser Asp Ser Asn Lys Leu Phe Glu Arg
           435                 440                 445
   Val Leu Gln Glu Gln Lys Asp Asp Leu Phe Lys Leu Phe Glu Gln Thr
       450                 455                 460
   Asp Lys Asp Asp Glu Lys Ile Leu Ala Gln Thr His Ser Leu Ser Thr
   465                 470                 475                 480
   Lys Ala Met Leu Leu Ala Ile Thr Arg Met Thr Asn Leu Asp Asn Asp
                   485                 490                 495
   Glu Asp Asn Gln Lys Asn Asn Asp Lys Gly Trp Asn Phe Glu Ala Ile
               500                 505                 510
   Lys Asn Phe Asp Gln Lys Phe Ile Asp Ile Thr Lys Lys Asn Asn Asn
           515                 520                 525
   Leu Ser Leu Lys Gln Asp Lys Arg Tyr Leu Asp Asp Arg Phe Ile Asn
       530                 535                 540
   Asp Ala Ile Leu Ser Pro Gly Val Lys Arg Ile Leu Arg Glu Ala Thr
   545                 550                 555                 560
   Lys Val Phe Asn Ala Ile Leu Lys Gln Phe Ser Glu Glu Tyr Asp Val
                   565                 570                 575
   Thr Lys Val Val Ile Glu Leu Ala Arg Glu Leu Ser Glu Glu Lys Glu
               580                 585                 590
   Leu Glu Asn Thr Lys Asn Tyr Glu Lys Leu Ile Lys Lys Asn Ser Asp
           595                 600                 605
   Lys Ile Ser Glu Arg Leu Glu Ala Leu Asp Ile Ala Glu Asp Lys Ile
       610                 615                 620
   Glu Asp Ile Leu Lys Ser Pro Thr Lys Ser Tyr Lys Val Leu Leu Trp
   625                 630                 635                 640
   Leu Gln Gln Asp His Ile Asp Pro Tyr Ser Gln Lys Glu Ile Ala Phe
                   645                 650                 655
   Glu Asp Ile Leu Thr Lys Thr Glu Lys Thr Glu Ile Asp His Ile Ile
               660                 665                 670
   Pro Tyr Ser Ile Ser Phe Asp Asp Ser Ser Ser Asn Lys Leu Leu Val
           675                 680                 685
   Leu Ala Glu Ser Asn Gln Ala Lys Ser Asn Gln Thr Pro Tyr Glu Phe
       690                 695                 700
   Ile Thr Ser Gly Asn Ala Gly Ile Lys Trp Glu Asp Tyr Glu Ala Tyr
   705                 710                 715                 720
   Cys Arg Lys Phe Lys Asp Gly Asp Thr Ser Leu Leu Asp Ser Thr Gln
                   725                 730                 735
   Arg Ser Lys Lys Phe Ala Lys Met Met Lys Thr Asp Thr Ser Ser Lys
               740                 745                 750
   Tyr Asp Ile Gly Phe Leu Ala Arg Asn Leu Asn Asp Thr Arg Tyr Ala
           755                 760                 765
   Thr Ile Val Phe Arg Asp Ala Leu Lys Asp Tyr Ala Asn Asn His Leu
       770                 775                 780
   Val Glu Asp Lys Pro Met Phe Lys Val Val Cys Ile Asn Gly Gly Val
   785                 790                 795                 800
   Thr Ser Phe Leu Arg Lys Asn Phe Asp Lys Ser Trp Tyr Ala Lys Lys
                   805                 810                 815
   Asp Arg Asp Lys Asn Ile His His Ala Val Asp Ala Ser Ile Ile Ser
               820                 825                 830
   Ile Phe Ser Asn Glu Thr Lys Thr Leu Phe Asn Gln Leu Thr Gln Phe
           835                 840                 845
   Ala Asp Tyr Lys Leu Phe Lys Asn Thr Asp Gly Ser Trp Lys Lys Ile
       850                 855                 860
   Asp Pro Lys Thr Gly Val Val Thr Glu Val Thr Asp Glu Asn Trp Lys
   865                 870                 875                 880
   Gln Ile Arg Val Arg Asn Gln Val Ser Glu Ile Ala Lys Val Ile Asn
                   885                 890                 895
   Lys Cys Ile Gln Asp Ser Asn Ile Glu Arg Lys Ala Arg Tyr Ser Arg
               900                 905                 910
   Lys Ile Glu Asn Lys Thr Asn Ile Ser Leu Phe Asn Asp Thr Val Tyr
           915                 920                 925
   Ser Ala Lys Lys Val Gly Tyr Glu Asp Gln Ile Lys Arg Lys Asn Leu
       930                 935                 940
   Lys Thr Leu Asp Ile His Glu Ser Ala Glu Glu Asn Lys Asn Ser Lys
   945                 950                 955                 960
   Val Lys Lys Gln Phe Val Tyr Arg Lys Leu Val Asn Val Ser Leu Leu
                   965                 970                 975
   Asn Asn Asp Lys Leu Ala Asp Leu Phe Ala Glu Lys Glu Asp Ile Leu
               980                 985                 990
   Met Tyr Arg Ala Asn Pro Trp Val Ile Asn Leu Ala Glu Gln Ile Phe
   995 1000 1005
   Asn Glu Tyr Thr Glu Asn Lys Lys Ile Lys Ser Gln Asn Val Phe Glu
   1010 1015 1020
   Lys Tyr Met Leu Asp Leu Thr Lys Glu Phe Pro Glu Lys Phe Ser Glu
   1025 1030 1035 1040
   Ala Phe Val Lys Ser Met Leu Arg Asn Lys Thr Ala Ile Ile Tyr Asn
   1045 1050 1055
   Val Glu Lys Lys Val Val His Arg Ile Lys Arg Leu Lys Ile Leu Ser
   1060 1065 1070
   Ser Glu Leu Lys Glu Asn Lys Leu Ser Asn Val Ile Ile Arg Ser Lys
   1075 1080 1085
   Asn Glu Ser Gly Thr Lys Leu Ser Tyr Gln Asp Thr Ile Asn Ser Ile
   1090 1095 1100
   Ala Leu Met Ile Met Arg Ser Ile Asp Pro Thr Ala Lys Lys Gln Tyr
   1105 1110 1115 1120
   Ile Arg Val Pro Leu Asn Thr Leu Asn Leu His Leu Gly Asp Gln Asp
   1125 1130 1135
   Phe Asp Leu His Asn Ile Asp Ala Tyr Leu Lys Lys Pro Lys Phe Val
   1140 1145 1150
   Lys Tyr Leu Lys Ala Asn Glu Ile Gly Asp Glu Tyr Lys Pro Trp Arg
   1155 1160 1165
   Val Leu Thr Ser Gly Thr Leu Leu Ile His Lys Lys Asp Lys Lys Leu
   1170 1175 1180
   Met Tyr Ile Ser Ser Phe Gln Asn Leu Lys Asp Val Ile Glu Ile Lys
   1185 1190 1195 1200
   Asn Leu Ile Glu Thr Glu Tyr Lys Glu Asn Asp Asp Ser Asp Pro Lys
   1205 1210 1215
   Lys Lys Lys Lys Ala Asn Arg Phe Leu Met Thr Leu Ser Thr Ile Leu
   1220 1225 1230
   Asn Asp Tyr Ile Leu Leu Asp Ala Lys Asp Asn Phe Asp Ile Leu Gly
   1235 1240 1245
   Leu Ser Lys Asn Arg Ile Asp Glu Ile Leu Asn Ser Lys Leu Gly Leu
   1250 1255 1260
   Asp Lys Ile Thr Lys
   1265
<210> 154
   <211> 1371
   <212> PRT
   <213> Streptococcus pyogenes
<220>
   <223> CRISPR-associated endonuclease
<308> YP_602415
   <309> 2013-08-28
<400> 154
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
                   100                 105                 110
   His Glu Arg His Pro Ile Phe Gly Asn Ile Val Asp Glu Val Ala Tyr
               115                 120                 125
   His Glu Lys Tyr Pro Thr Ile Tyr His Leu Arg Lys Lys Leu Ala Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Arg Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Thr Leu Leu Ser Asp
       290                 295                 300
   Ile Leu Arg Val Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Ala Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Lys Glu Met Ile Glu Glu Arg Leu Lys Thr Tyr Ala
   625                 630                 635                 640
   His Leu Phe Asp Asp Lys Val Met Lys Gln Leu Lys Arg Arg His Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Ile Gln Leu Ile His Asp Asp Ser Leu Thr Phe
       690                 695                 700
   Lys Glu Ala Ile Gln Lys Ala Gln Val Ser Gly Gln Gly His Ser Leu
   705                 710                 715                 720
   His Glu Gln Ile Ala Asn Leu Ala Gly Ser Pro Ala Ile Lys Lys Gly
                   725                 730                 735
   Ile Leu Gln Ser Val Lys Val Val Asp Glu Leu Val Lys Val Met Gly
               740                 745                 750
   His Lys Pro Glu Asn Ile Val Ile Glu Met Ala Arg Glu Asn Gln Thr
           755                 760                 765
   Thr Gln Lys Gly Gln Lys Asn Ser Arg Glu Arg Met Lys Arg Ile Glu
       770                 775                 780
   Glu Gly Ile Lys Glu Leu Gly Ser Gln Ile Leu Lys Glu His Pro Val
   785                 790                 795                 800
   Glu Asn Thr Gln Leu Gln Asn Glu Lys Leu Tyr Leu Tyr Tyr Leu Gln
                   805                 810                 815
   Asn Gly Arg Asp Met Tyr Val Asp Gln Glu Leu Asp Ile Asn Arg Leu
               820                 825                 830
   Ser Asp Tyr Asp Val Asp His Ile Val Pro Gln Ser Phe Ile Lys Asp
           835                 840                 845
   Asp Ser Ile Asp Asn Lys Val Leu Thr Arg Ser Asp Lys Asn Arg Gly
       850                 855                 860
   Lys Ser Asp Asp Val Pro Ser Glu Glu Val Val Lys Lys Met Lys Asn
   865                 870                 875                 880
   Tyr Trp Arg Gln Leu Leu Asn Ala Lys Leu Ile Thr Gln Arg Lys Phe
                   885                 890                 895
   Asp Asn Leu Thr Lys Ala Glu Arg Gly Gly Leu Ser Glu Leu Asp Lys
               900                 905                 910
   Ala Gly Phe Ile Lys Arg Gln Leu Val Glu Thr Arg Gln Ile Thr Lys
           915                 920                 925
   His Val Ala Gln Ile Leu Asp Ser Arg Met Asn Thr Lys Tyr Asp Glu
       930                 935                 940
   Asn Asp Lys Leu Ile Arg Glu Val Lys Val Ile Thr Leu Lys Ser Lys
   945                 950                 955                 960
   Leu Val Ser Asp Phe Arg Lys Asp Phe Gln Phe Tyr Lys Val Arg Glu
                   965                 970                 975
   Ile Asn Asn Tyr His His Ala His Asp Ala Tyr Leu Asn Ala Val Val
               980                 985                 990
   Gly Thr Ala Leu Ile Lys Lys Tyr Pro Lys Leu Glu Ser Glu Phe Val
           995                 1000                1005
   Tyr Gly Asp Tyr Lys Val Tyr Asp Val Arg Lys Met Ile Ala Lys Ser
       1010                1015                1020
   Glu Gln Glu Ile Gly Lys Ala Thr Ala Lys Arg Phe Phe Tyr Ser Asn
   1025                1030                1035                1040
   Ile Met Asn Phe Phe Lys Thr Glu Ile Thr Leu Ala Asn Gly Glu Ile
                   1045                1050                1055
   Arg Lys Arg Pro Leu Ile Glu Thr Asn Glu Glu Thr Gly Glu Ile Val
               1060                1065                1070
   Trp Asp Lys Gly Arg Asp Phe Ala Thr Val Arg Lys Val Leu Ser Met
           1075                1080                1085
   Pro Gln Val Asn Ile Val Lys Lys Thr Glu Val Gln Thr Gly Ala Leu
       1090                1095                1100
   Thr Asn Glu Ser Ile Tyr Ala Arg Gly Ser Phe Asp Lys Leu Ile Ser
   1105                1110                1115                1120
   Arg Lys His Arg Phe Glu Ser Ser Lys Tyr Gly Gly Phe Gly Ser Pro
                   1125                1130                1135
   Thr Val Thr Tyr Ser Val Leu Val Val Ala Lys Ser Lys Val Gln Asp
               1140                1145                1150
   Gly Lys Val Lys Lys Ile Lys Thr Gly Lys Glu Leu Ile Gly Ile Thr
           1155                1160                1165
   Leu Leu Asp Lys Leu Val Phe Glu Lys Asn Pro Leu Lys Phe Ile Glu
       1170                1175                1180
   Asp Lys Gly Tyr Gly Asn Val Gln Ile Asp Lys Cys Ile Lys Leu Pro
   1185                1190                1195                1200
   Lys Tyr Ser Leu Phe Glu Phe Glu Asn Gly Thr Arg Arg Met Leu Ala
                   1205                1210                1215
   Ser Val Met Ala Asn Asn Asn Ser Arg Gly Asp Leu Gln Lys Ala Asn
               1220                1225                1230
   Glu Met Phe Leu Pro Ala Lys Leu Val Thr Leu Leu Tyr His Ala His
           1235                1240                1245
   Lys Ile Glu Ser Ser Lys Glu Leu Glu His Glu Ala Tyr Ile Leu Asp
       1250                1255                1260
   His Tyr Asn Asp Leu Tyr Gln Leu Leu Ser Tyr Ile Glu Arg Phe Ala
   1265                1270                1275                1280
   Ser Leu Tyr Val Asp Val Glu Lys Asn Ile Ser Lys Val Lys Glu Leu
                   1285                1290                1295
   Phe Ser Asn Ile Glu Ser Tyr Ser Ile Ser Glu Ile Cys Ser Ser Val
               1300                1305                1310
   Ile Asn Leu Leu Thr Leu Thr Ala Ser Gly Ala Pro Ala Asp Phe Lys
           1315                1320                1325
   Phe Leu Gly Thr Thr Ile Pro Arg Lys Arg Tyr Gly Ser Pro Gln Ser
       1330                1335                1340
   Ile Leu Ser Ser Thr Leu Ile His Gln Ser Ile Thr Gly Leu Tyr Glu
   1345                1350                1355                1360
   Thr Arg Ile Asp Leu Ser Gln Leu Gly Ser Asp
                   1365                1370
<210> 155
   <211> 151
   <212> PRT
   <213> Xanthomonas axonopodis pv. citri
<220>
   <223> CRISPR-associated endonuclease
<308> NP_643570
   <309> 2013-06-10
<400> 155
   Met Gly His Pro Pro Val Leu Cys Pro His Ser Gln Thr Val Pro Gly
    1               5                  10                  15
   Ala Arg Gly Pro Pro Asn Gly Gly Thr Gly Ile Leu Ser Ile Pro Glu
               20                  25                  30
   Leu Pro Ile Ser Lys Lys Arg Leu Lys Thr Phe Arg Leu Leu Ala Phe
           35                  40                  45
   His Ala Gln His Gly Arg Cys Phe Tyr Phe Thr Leu Pro Thr Trp Leu
       50                  55                  60
   Ala Ser Pro Asn Glu Leu Gly Leu Arg Pro Arg Ser Ala Arg Pro Tyr
   65                  70                  75                  80
   Gln Cys Thr Ala Lys His Leu Val Ala Cys Gln Asp Gly Gly Lys Asp
                   85                  90                  95
   Val Val Gly Asn Val Val Ala Ala His Ala Ala Cys Asn His Arg Arg
               100                 105                 110
   His Lys Arg Thr Ala Pro Val Pro Ser Ala Thr Asp Tyr Arg Thr Phe
           115                 120                 125
   Val Gln Arg Gln Leu Ala Lys Gly Lys Trp Cys Pro Gln Trp Ile Tyr
       130                 135                 140
   Gly Leu Ser Ile Gly Thr Pro
   145                 150
<210> 156
   <211> 1381
   <212> PRT
   <213> Streptococcus suis
<220>
   <223> CRISPR-associated endonuclease
<308> YP_006359179
   <309> 2013-06-11
<400> 156
   Met Val Lys Lys Lys Tyr Ala Ile Gly Ile Asp Ile Gly Thr Asn Ser
    1               5                  10                  15
   Val Gly Trp Ser Ile Val Thr Asp Asp Tyr Lys Val Pro Ser Lys Lys
               20                  25                  30
   Met Lys Val Phe Gly Asn Thr Glu Lys Arg Tyr Ile Lys Lys Asn Leu
           35                  40                  45
   Leu Gly Thr Leu Leu Phe Asp Glu Gly Asn Thr Ala Glu Asn Arg Arg
       50                  55                  60
   Leu Lys Arg Thr Ala Arg Arg Arg Tyr Thr Arg Arg Arg Asn Arg Ile
   65                  70                  75                  80
   Leu Tyr Leu Gln Glu Ile Phe Ala Glu Glu Ile Asn Lys Ile Asp Asp
                   85                  90                  95
   Ser Phe Phe Gln Arg Leu Asp Asp Ser Phe Leu Ile Val Glu Asp Lys
               100                 105                 110
   Gln Gly Ser Lys His Pro Ile Phe Gly Thr Leu Gln Glu Glu Lys Glu
           115                 120                 125
   Tyr His Lys Gln Phe Pro Thr Ile Tyr His Leu Arg Lys Gln Leu Ala
       130                 135                 140
   Asp Ser Ser Gln Lys Ala Asp Ile Arg Leu Ile Tyr Leu Ala Leu Ala
   145                 150                 155                 160
   His Ile Ile Lys Tyr Arg Gly His Phe Leu Phe Glu Gly Asp Leu Lys
                   165                 170                 175
   Ser Glu Asn Lys Asp Val Gln His Leu Phe Asn Asp Phe Val Glu Met
               180                 185                 190
   Phe Asp Lys Thr Val Glu Gly Ser Tyr Leu Ser Glu Asn Leu Pro Asn
           195                 200                 205
   Val Ala Asp Val Leu Val Glu Lys Val Ser Lys Ser Arg Arg Leu Glu
       210                 215                 220
   Asn Ile Leu His Tyr Phe Pro Asn Glu Lys Lys Asn Gly Leu Phe Gly
   225                 230                 235                 240
   Asn Phe Leu Ala Leu Ala Leu Gly Leu Gln Pro Asn Phe Lys Thr Asn
                   245                 250                 255
   Phe Glu Leu Ala Glu Asp Ala Lys Ile Gln Phe Ser Lys Glu Thr Tyr
               260                 265                 270
   Glu Glu Asp Leu Glu Glu Leu Leu Gly Lys Ile Gly Asp Asp Tyr Ala
           275                 280                 285
   Asp Leu Phe Ile Ala Thr Lys Ser Leu Tyr Asp Gly Ile Leu Leu Ala
       290                 295                 300
   Gly Ile Leu Ser Thr Thr Asp Ser Thr Thr Lys Ala Pro Leu Ser Ser
   305                 310                 315                 320
   Ser Met Val Asn Arg Tyr Glu Glu His Gln Lys Asp Leu Ala Leu Leu
                   325                 330                 335
   Lys Asn Phe Ile His Gln Asn Leu Ser Asp Ser Tyr Lys Glu Val Phe
               340                 345                 350
   Asn Asp Lys Leu Lys Asp Gly Tyr Ala Gly Tyr Ile Glu Gly Lys Thr
   355 360 365
   Thr Gln Glu Asn Phe Tyr Arg Phe Ile Lys Lys Ala Ile Glu Lys Ile
   370 375 380
   Glu Gly Ser Asn Tyr Phe Ile Asp Lys Ile Asp Arg Glu Asp Phe Leu
   385 390 395 400
   Arg Lys Gln Arg Thr Phe Asp Asn Gly Ser Ile Pro His Gln Ile His
   405 410 415
   Leu Gln Glu Met His Ala Ile Ile Arg Arg Gln Ala Glu Phe Tyr Pro
   420 425 430
   Phe Leu Val Glu Asn Gln Asp Lys Ile Glu Lys Ile Leu Thr Phe Arg
   435 440 445
   Ile Pro Tyr Tyr Val Gly Pro Leu Ala Arg Gly Lys Ser Glu Phe Ala
   450 455 460
   Trp Leu Asn Arg Lys Ser Asp Glu Lys Ile Arg Pro Trp Asn Phe Asp
   465 470 475 480
   Glu Met Val Asp Lys Glu Thr Ser Ala Glu Asn Phe Ile Thr Arg Met
   485 490 495
   Thr Asn Tyr Asp Arg Tyr Leu Pro Asp Gln Lys Val Leu Pro Lys His
   500 505 510
   Ser Leu Leu Tyr Glu Lys Phe Ala Val Tyr Asn Glu Leu Thr Lys Val
   515 520 525
   Lys Phe Ile Ala Glu Gly Met Arg Asp Tyr Gln Phe Leu Asp Ser Gly
   530 535 540
   Gln Lys Lys Asp Ile Val Lys Thr Leu Phe Lys Thr Lys Arg Lys Val
   545 550 555 560
   Thr Ala Lys Asp Ile Lys Ala Tyr Leu Glu Asn Ser Asn Gly Tyr Ala
   565 570 575
   Gly Val Glu Leu Lys Gly Leu Glu Glu Gln Phe Asn Ala Ser Leu Pro
   580 585 590
   Thr Tyr His Asp Leu Leu Lys Ile Leu Arg Asp Lys Ala Phe Ile Asp
   595 600 605
   Ala Glu Glu Asn Gln Glu Ile Leu Glu Asp Ile Val Leu Thr Leu Thr
   610 615 620
   Leu Phe Glu Asp Arg Glu Met Ile Arg Lys Arg Leu Glu Lys Tyr Lys
   625 630 635 640
   Asp Ile Leu Thr Glu Glu Gln Arg Lys Lys Leu Glu Arg Arg His Tyr
   645 650 655
   Thr Gly Trp Gly Arg Leu Ser Ala Lys Leu Ile Asn Gly Ile Leu Asp
   660 665 670
   Lys Val Thr Arg Lys Thr Ile Leu Gly Tyr Leu Ile Asp Asp Gly Thr
   675 680 685
   Ser Asn Arg Asn Phe Met Gln Leu Ile Asn Asp Asp Thr Leu Ser Phe
   690 695 700
   Val Asp Glu Ile Arg Leu Ala Gln Gly Ser Gly Glu Ala Glu Asp Tyr
   705 710 715 720
   Arg Ala Glu Val Gln Asn Leu Ala Gly Ser Pro Ala Ile Lys Lys Gly
   725 730 735
   Ile Leu Gln Ser Leu Lys Ile Val Asp Glu Leu Ile Glu Val Met Gly
   740 745 750
   Tyr Glu Pro Glu His Ile Val Val Glu Met Ala Arg Glu Asn Gln Phe
   755 760 765
   Thr Asn Gln Gly Arg Arg Asn Ser Gln Gln Arg Ile Asn Arg Leu Glu
   770 775 780
   Asn Ala Leu Lys Asp Phe Gly Ser Lys Ile Leu Glu Glu Gln Lys Pro
   785 790 795 800
   Tyr Gly Val Ser Lys Val Asn Asn Ile His Leu Gln Ser Asp Arg Leu
   805 810 815
   Phe Leu Tyr Tyr Leu Gln Asn Gly Lys Asp Met Tyr Thr Asp Glu Glu
   820 825 830
   Leu Asp Ile Asp His Leu Ser Glu Tyr Asp Ile Asp His Ile Ile Pro
   835 840 845
   Gln Ala Phe Ile Lys Asp Asp Ser Leu Asp Asn Lys Val Leu Thr Lys
   850 855 860
   Ser Ala Lys Asn Arg Gly Lys Ser Asp Asp Val Pro Ser Met Glu Ile
   865 870 875 880
   Val His Asn Arg Lys Asn Phe Trp Arg Gln Leu Leu Lys Ser Lys Leu
   885 890 895
   Ile Ser Gln Arg Lys Phe Asp Asn Leu Thr Lys Ala Glu Arg Gly Gly
   900 905 910
   Leu Thr Asn Glu Asp Lys Ala Arg Phe Ile Gln Arg Gln Leu Val Glu
   915 920 925
   Thr Arg Gln Ile Thr Lys His Val Ala Arg Met Leu Asp Thr Arg Phe
   930 935 940
   Asn Thr Lys Leu Asp Glu Ala Gly Asn Arg Ile Arg Asp Pro Lys Val
   945 950 955 960
   Asn Ile Ile Thr Leu Lys Ser Asn Leu Val Ser Gln Phe Arg Lys Asp
   965 970 975
   Tyr Gln Leu Tyr Lys Ile Arg Glu Ile Asn Asn Tyr His His Ala His
   980 985 990
   Asp Ala Tyr Ile Asn Ala Val Val Ala Thr Ala Leu Leu Lys Lys Tyr
   995 1000 1005
   Pro Gln Leu Ala Pro Glu Phe Val Tyr Gly Glu Tyr Pro Thr Tyr Asn
   1010 1015 1020
   Ser Tyr Asn Ser Arg Lys Ser Ala Thr Glu Lys Val Leu Phe Tyr Ser
   1025 1030 1035 1040
   Asn Ile Met Asn Phe Phe Lys Arg Ile Val Val His Ser Lys Thr Gly
   1045 1050 1055
   Glu Val Arg Ile Arg Pro Val Ile Glu Val Asn Lys Glu Thr Gly Glu
   1060 1065 1070
   Ile Val Trp Asp Lys Lys Ser Asp Phe Arg Thr Val Arg Lys Val Leu
   1075 1080 1085
   Ser Tyr Pro Gln Val Asn Val Val Lys Lys Thr Glu Val Gln Asn His
   1090 1095 1100
   Gly Leu Asp Arg Gly Lys Pro Lys Gly Phe Tyr Asn Ala Asn Leu Ser
   1105 1110 1115 1120
   Pro Arg Pro Lys Glu Gly Ser Val Glu Asn Leu Val Pro Ala Lys Gln
   1125 1130 1135
   Ser Phe Asp Thr Lys Arg Tyr Gly Gly Tyr Ala Gly Ile Ser Asn Ser
   1140 1145 1150
   Tyr Ala Val Leu Val Asn Gly Ile Ile Glu Lys Gly Lys Lys Lys Ser
   1155 1160 1165
   Lys Ala Glu Val Thr Glu Phe Gln Gly Ile Ser Ile Leu Ala Arg Lys
   1170 1175 1180
   Asp Phe Asp Lys Asn Pro Lys Gln Tyr Leu Leu Asn Leu Gly Tyr Lys
   1185 1190 1195 1200
   Asp Ile Lys Ser Ile Ile Lys Leu Pro Lys Tyr Ser Leu Phe Glu Leu
   1205 1210 1215
   Glu Asn Gly Ser Arg Arg Met Leu Ala Ser Ile Leu Ser Thr Asn Asn
   1220 1225 1230
   Lys Arg Gly Glu Ile His Lys Gly Asn Glu Met Tyr Leu Pro Asp Lys
   1235 1240 1245
   Phe Val Thr Leu Leu Tyr His Ala Met Arg Val Asn Arg Thr Leu Glu
   1250 1255 1260
   Pro Gly His Lys Lys Tyr Val Glu Thr His Arg His Val Phe Asp Glu
   1265 1270 1275 1280
   Leu Leu Ile Tyr Ile Leu Glu Tyr Asn Glu Lys Ile Val Gly Ala Lys
   1285 1290 1295
   Ala Asn Gly Lys Arg Ile Val Glu Ala Tyr Ser Ala Arg Lys Asp Thr
   1300 1305 1310
   Asp Asn Leu Glu Glu Leu Cys Asn Ser Phe Ile Asn Leu Leu Asn Leu
   1315 1320 1325
   Thr Ala Leu Gly Ser Ala Val Asp Phe Glu Phe Leu Gly Thr Lys Ile
   1330 1335 1340
   Pro Arg Tyr Arg Asp Tyr Thr Pro Ser Ser Leu Leu Lys Ala Thr Leu
   1345 1350 1355 1360
   Ile His Gln Ser Val Thr Gly Leu Tyr Glu Thr Arg Ile Asp Leu Ser
   1365 1370 1375
   Lys Leu Gly Gly Asp
   1380
<210> 157
   <211> 1122
   <212> PRT
   <213> Streptococcus suis
<220>
   <223> CRISPR-associated endonuclease
<308> YP_006080899
   <309> 2013-06-11
<400> 157
   Met Ser Asn Gly Lys Ile Leu Gly Leu Asp Ile Gly Ile Ala Ser Val
    1               5                  10                  15
   Gly Val Gly Val Ile Asp Ala Gln Thr Gly Glu Ile Ile His Ala Ser
               20                  25                  30
   Ser Arg Ile Phe Pro Ser Ala Asn Ala Ala Asn Asn Ala Glu Arg Arg
           35                  40                  45
   Thr Phe Arg Gly Ser Arg Arg Leu Ile Arg Arg Lys Lys His Arg Ile
       50                  55                  60
   Lys Arg Leu Asp Asp Leu Phe Asn Asp Phe His Ile Asn Leu Asp Gly
   65                  70                  75                  80
   Glu Met Ser Thr Asp Asn Pro Tyr Val Leu Arg Val Lys Gly Leu Ser
                   85                  90                  95
   Gln Lys Leu Thr Val Glu Glu Leu Tyr Ile Ser Ile Lys Asn Ile Met
               100                 105                 110
   Lys Arg Arg Gly Ile Ser Tyr Leu Asp Asp Ala Glu Ser Asp Asn Glu
           115                 120                 125
   Ala Gly Arg Ser Asp Tyr Ala Lys Ala Ile Glu Arg Asn Arg Gln Leu
       130                 135                 140
   Leu Thr Ser Lys Thr Pro Gly Glu Ile Gln Leu Glu Arg Leu Glu Lys
   145                 150                 155                 160
   Tyr Gly Gln Leu Arg Gly Asn Phe Thr Ile Ile Asp Glu Glu Gly Gln
                   165                 170                 175
   Ser Gln Gln Ile Ile Asn Val Phe Ser Thr Ser Asp Tyr Val Lys Glu
               180                 185                 190
   Val Glu Lys Ile Leu Asp Cys Gln Lys Met Tyr His Lys Phe Ile Ser
           195                 200                 205
   Asp Glu Phe Cys Asp Lys Leu Ile Glu Leu Leu Arg Glu Lys Arg Lys
       210                 215                 220
   Tyr Tyr Val Gly Pro Gly Asn Glu Lys Ser Arg Thr Asp Tyr Gly Ile
   225                 230                 235                 240
   Tyr Arg Thr Asp Gly Thr Thr Leu Glu Asn Leu Phe Gly Ile Leu Ile
                   245                 250                 255
   Gly Lys Cys Thr Phe Tyr Pro Asp Gln Tyr Arg Ser Ser Arg Ala Ser
               260                 265                 270
   Tyr Thr Ala Gln Glu Phe Asn Phe Leu Asn Asp Leu Asn Asn Leu Thr
           275                 280                 285
   Val Pro Thr Glu Thr Lys Lys Leu Ser Gln Glu Gln Lys Glu Phe Leu
       290                 295                 300
   Val Asn Tyr Ala Lys Glu Thr Ser Val Leu Gly Ala Gly Lys Ile Leu
   305                 310                 315                 320
   Gln Gln Ile Ala Lys Leu Ala Asp Cys Lys Val Glu Asp Ile Arg Gly
                   325                 330                 335
   Tyr Arg Leu Asp Asn Lys Asp Lys Pro Glu Leu His Thr Phe Glu Thr
               340                 345                 350
   Tyr Arg Ala Met Lys Gly Leu Val Pro Leu Val Asp Ile Gly Val Leu
           355                 360                 365
   Ser Arg Glu Gln Leu Asp Ile Leu Ala Asp Ile Leu Thr Leu Asn Thr
       370                 375                 380
   Asp Phe Glu Gly Ile Arg Glu Ala Leu Lys Lys Gln Leu Pro Asn Val
   385                 390                 395                 400
   Phe Asp Glu Lys Gln Val Lys Gly Leu Ala Ser Phe Arg Lys Ser Lys
                   405                 410                 415
   Ser Gln Leu Phe Ala Lys Gly Trp His Asn Leu Ser Gln Lys Ile Met
                   420                 425                 430
   Leu Glu Val Ile Pro Glu Leu Tyr Ala Thr Ser Asp Glu Gln Met Thr
           435                 440                 445
   Ile Leu Thr Arg Leu Gly Lys Phe Glu Lys Ser Ser Val Ala Glu Tyr
       450                 455                 460
   Pro Ser Ser Ile Asn Val Asp Glu Ile Thr Asp Glu Ile Tyr Asn Pro
   465                 470                 475                 480
   Val Val Ala Lys Ser Ile Arg Gln Thr Ile Lys Ile Ile Asn Ala Ser
                   485                 490                 495
   Ile Lys Lys Trp Asp Glu Phe Asp Gln Ile Val Ile Glu Met Pro Arg
               500                 505                 510
   Asp Arg Asn Glu Asp Glu Glu Lys Lys Arg Ile Ala Asp Gly Gln Lys
           515                 520                 525
   Ala Asn Ala Lys Glu Lys Ala Asp Ser Ile Leu Arg Ala Ala Glu Leu
       530                 535                 540
   Tyr Cys Ala Gly Lys Val Leu Pro Asp Tyr Val Tyr Asn Gly His Asn
   545                 550                 555                 560
   Gln Leu Ala Thr Lys Ile Arg Leu Trp Tyr Gln Gln Gly Glu Arg Cys
                   565                 570                 575
   Ile Tyr Thr Gly Gln Pro Ile Ser Ile His Asp Leu Ile His Asn Gln
               580                 585                 590
   Asn Gln Tyr Glu Ile Asp His Ile Leu Pro Leu Ser Leu Thr Phe Asp
           595                 600                 605
   Asp Ser Leu Ser Asn Lys Val Leu Val Leu Ala Thr Ala Asn Gln Glu
       610                 615                 620
   Lys Ala Gln Arg Thr Pro Tyr Asn Tyr Leu Lys Ser Ala Thr Ser Ala
   625                 630                 635                 640
   Trp Ser Tyr Arg Glu Phe Lys Asp Tyr Val Thr Lys Arg Lys Gly Ile
                   645                 650                 655
   Gly Lys Lys Lys Cys Glu Tyr Leu Thr Phe Glu Glu Asp Ile Asn Gly
               660                 665                 670
   Phe Glu Val Arg Ser Lys Phe Ile Gln Arg Asn Leu Val Asp Thr Arg
           675                 680                 685
   Tyr Ala Ser Lys Val Ile Leu Asn Ala Leu Gln Asp Tyr Phe Lys Ile
       690                 695                 700
   Ser Gly Ile Gln Thr Lys Val Ser Val Val Arg Gly Gln Phe Thr Ser
   705                 710                 715                 720
   Gln Leu Arg His Lys Trp Gly Ile Glu Lys Thr Arg Glu Thr Tyr His
                   725                 730                 735
   His His Ala Val Asp Ala Leu Ile Ile Ala Ala Ser Ser Gln Leu Arg
               740                 745                 750
   Leu Trp Lys Lys Gln Glu Ser Pro Leu Val Val Asp Tyr Gln Glu Gly
           755                 760                 765
   Arg Gln Val Asp Leu Glu Thr Gly Glu Ile Leu Glu Leu Thr Asp Glu
       770                 775                 780
   Gln Tyr Lys Glu Leu Val Tyr Gln Pro Pro Tyr Gln Gly Phe Val Asn
   785                 790                 795                 800
   Thr Ile Ser Ser Ser Ala Phe Asp Asn Glu Ile Leu Phe Ser Tyr Gln
                   805                 810                 815
   Val Asp Ser Lys Val Asn Arg Lys Ile Ser Asp Ala Thr Ile Tyr Ala
               820                 825                 830
   Thr Arg Asn Ala Gln Leu Gly Lys Asp Lys Thr Glu Gly Ile Tyr Val
           835                 840                 845
   Leu Gly Lys Ile Lys Asp Ile Tyr Thr Gln Ala Gly Tyr Glu Ala Phe
       850                 855                 860
   Leu Lys Arg Tyr Thr Lys Asp Lys Thr Ser Phe Leu Met Tyr His Lys
   865                 870                 875                 880
   Asp Leu Asp Thr Trp Glu Lys Val Ile Glu Ile Ile Leu Arg Asp Tyr
                   885                 890                 895
   Arg Glu Tyr Asp Glu Lys Gly Lys Glu Ile Gly Asn Pro Phe Glu Arg
               900                 905                 910
   Tyr Arg Arg Glu Asn Gly Tyr Val Lys Lys Tyr Ser Arg Lys Gly Asn
           915                 920                 925
   Gly Thr Ala Ile Lys Ser Leu Lys Tyr Tyr Asp Asn Lys Leu Gly Asn
       930                 935                 940
   His Ile Asp Ile Thr Pro Glu Asn Ser Arg Asn Ala Val Val Leu Gln
   945                 950                 955                 960
   Ser Leu Lys Pro Trp Arg Thr Asp Val Tyr Phe Asn Lys Glu Thr Gly
                   965                 970                 975
   Lys Tyr Glu Phe Leu Gly Ile Lys Tyr Ser Asp Leu Ser Phe Glu Lys
               980                 985                 990
   Gly Thr Gly Glu Tyr Gly Ile Ser Gln Glu Lys Tyr Asp Ser Ile Lys
           995                 1000                1005
   Ile Ala Glu Gly Val Ala Lys Lys Ser Ile Phe Lys Phe Thr Leu Tyr
       1010                1015                1020
   Lys Gln Asp Leu Leu Phe Ile Lys Asp Ile Glu Asn Asn Phe Gly Lys
   1025                1030                1035                1040
   Leu Leu Arg Phe Thr Ser Lys Asn Asp Thr Ser Lys His Tyr Val Glu
                   1045                1050                1055
   Leu Lys Pro Tyr Asp Lys Asn Lys Phe Gly Thr Glu Glu Pro Leu Leu
               1060                1065                1070
   Pro Val Leu Gly Asn Val Ala Lys Ser Gly Gln Cys Ile Lys Gly Leu
           1075                1080                1085
   Asn Lys Ser Asn Ile Ser Ile Tyr Lys Val Arg Thr Asp Ile Leu Gly
       1090                1095                1100
   Tyr Arg His Phe Ile Lys Gln Glu Gly Glu His Pro Gln Leu Lys Phe
   1105                1110                1115                1120
   Lys Lys
<210> 158
   <211> 1064
   <212> PRT
   <213> Bradyrhizobium sp.
<220>
   <223> CRISPR-associated endonuclease
<308> YP_001239928
   <309> 2013-06-10
<400> 158
   Met Lys Arg Thr Ser Leu Arg Ala Tyr Arg Leu Gly Val Asp Leu Gly
   1               5                   10                  15
   Ala Asn Ser Leu Gly Trp Phe Val Val Trp Leu Asp Asp His Gly Gln
               20                  25                  30
   Pro Glu Gly Leu Gly Pro Gly Gly Val Arg Ile Phe Pro Asp Gly Arg
           35                  40                  45
   Asn Pro Gln Ser Lys Gln Ser Asn Ala Ala Gly Arg Arg Leu Ala Arg
       50                  55                  60
   Ser Ala Arg Arg Arg Arg Asp Arg Tyr Leu Gln Arg Arg Gly Lys Leu
   65                  70                  75                  80
   Met Gly Leu Leu Val Lys His Gly Leu Met Pro Ala Asp Glu Pro Ala
                   85                  90                  95
   Arg Lys Arg Leu Glu Cys Leu Asp Pro Tyr Gly Leu Arg Ala Lys Ala
               100                 105                 110
   Leu Asp Glu Val Leu Pro Leu His His Val Gly Arg Ala Leu Phe His
           115                 120                 125
   Leu Asn Gln Arg Arg Gly Leu Phe Ala Asn Arg Ala Ile Glu Gln Gly
       130                 135                 140
   Asp Lys Asp Ala Ser Ala Ile Lys Ala Ala Ala Gly Arg Leu Gln Thr
   145                 150                 155                 160
   Ser Met Gln Ala Cys Gly Ala Arg Thr Leu Gly Glu Phe Leu Asn Arg
                   165                 170                 175
   Arg His Gln Leu Arg Ala Thr Val Arg Ala Arg Ser Pro Val Gly Gly
               180                 185                 190
   Asp Val Gln Ala Arg Tyr Glu Phe Tyr Pro Thr Arg Ala Met Val Asp
           195                 200                 205
   Ala Glu Phe Glu Ala Ile Trp Ala Ala Gln Ala Pro His His Pro Thr
       210                 215                 220
   Met Thr Ala Glu Ala His Asp Thr Ile Arg Glu Ala Ile Phe Ser Gln
   225                 230                 235                 240
   Arg Ala Met Lys Arg Pro Ser Ile Gly Lys Cys Ser Leu Asp Pro Ala
                   245                 250                 255
   Thr Ser Gln Asp Asp Val Asp Gly Phe Arg Cys Ala Trp Ser His Pro
               260                 265                 270
   Leu Ala Gln Arg Phe Arg Ile Trp Gln Asp Val Arg Asn Leu Ala Val
           275                 280                 285
   Val Glu Thr Gly Pro Thr Ser Ser Arg Leu Gly Lys Glu Asp Gln Asp
       290                 295                 300
   Lys Val Ala Arg Ala Leu Leu Gln Thr Asp Gln Leu Ser Phe Asp Glu
   305                 310                 315                 320
   Ile Arg Gly Leu Leu Gly Leu Pro Ser Asp Ala Arg Phe Asn Leu Glu
                   325                 330                 335
   Ser Asp Arg Arg Asp His Leu Lys Gly Asp Ala Thr Gly Ala Ile Leu
               340                 345                 350
   Ser Ala Arg Arg His Phe Gly Pro Ala Trp His Asp Arg Ser Leu Asp
           355                 360                 365
   Arg Gln Ile Asp Ile Val Ala Leu Leu Glu Ser Ala Leu Asp Glu Ala
       370                 375                 380
   Ala Ile Ile Ala Ser Leu Gly Thr Thr His Ser Leu Asp Glu Ala Ala
   385                 390                 395                 400
   Ala Gln Arg Ala Leu Ser Ala Leu Leu Pro Asp Gly Tyr Cys Arg Leu
                   405                 410                 415
   Gly Leu Arg Ala Ile Lys Arg Val Leu Pro Leu Met Glu Ala Gly Arg
               420                 425                 430
   Thr Tyr Ala Glu Ala Ala Ser Ala Ala Gly Tyr Asp His Ala Leu Leu
           435                 440                 445
   Pro Gly Gly Lys Leu Ser Pro Thr Gly Tyr Leu Pro Tyr Tyr Gly Gln
       450                 455                 460
   Trp Leu Gln Asn Asp Val Val Gly Ser Asp Asp Glu Arg Asp Thr Asn
   465                 470                 475                 480
   Glu Arg Arg Trp Gly Arg Leu Pro Asn Pro Thr Val His Ile Gly Ile
                   485                 490                 495
   Gly Gln Leu Arg Arg Val Val Asn Glu Leu Ile Arg Trp His Gly Pro
               500                 505                 510
   Pro Ala Glu Ile Thr Val Glu Leu Thr Arg Asp Leu Lys Leu Ser Pro
           515                 520                 525
   Arg Arg Leu Ala Glu Leu Glu Arg Glu Gln Ala Glu Asn Gln Arg Lys
       530                 535                 540
   Asn Asp Lys Arg Thr Ser Leu Leu Arg Lys Leu Gly Leu Pro Ala Ser
   545                 550                 555                 560
   Thr His Asn Leu Leu Lys Leu Arg Leu Trp Asp Glu Gln Gly Asp Val
                   565                 570                 575
   Ala Ser Glu Cys Pro Tyr Thr Gly Glu Ala Ile Gly Leu Glu Arg Leu
               580                 585                 590
   Val Ser Asp Asp Val Asp Ile Asp His Leu Ile Pro Phe Ser Ile Ser
           595                 600                 605
   Trp Asp Asp Ser Ala Ala Asn Lys Val Val Cys Met Arg Tyr Ala Asn
       610                 615                 620
   Arg Glu Lys Gly Asn Arg Thr Pro Phe Glu Ala Phe Gly His Arg Gln
   625                 630                 635                 640
   Gly Arg Pro Tyr Asp Trp Ala Asp Ile Ala Glu Arg Ala Ala Arg Leu
                   645                 650                 655
   Pro Arg Gly Lys Arg Trp Arg Phe Gly Pro Gly Ala Arg Ala Gln Phe
               660                 665                 670
   Glu Glu Leu Gly Asp Phe Gln Ala Arg Leu Leu Asn Glu Thr Ser Trp
           675                 680                 685
   Leu Ala Arg Val Ala Lys Gln Tyr Leu Ala Ala Val Thr His Pro His
       690                 695                 700
   Arg Ile His Val Leu Pro Gly Arg Leu Thr Ala Leu Leu Arg Ala Thr
   705                 710                 715                 720
   Trp Glu Leu Asn Asp Leu Leu Pro Gly Ser Asp Asp Arg Ala Ala Lys
                   725                 730                 735
   Ser Arg Lys Asp His Arg His His Ala Ile Asp Ala Leu Val Ala Ala
               740                 745                 750
   Leu Thr Asp Gln Ala Leu Leu Arg Arg Met Ala Asn Ala His Asp Asp
           755                 760                 765
   Thr Arg Arg Lys Ile Glu Val Leu Leu Pro Trp Pro Thr Phe Arg Ile
       770                 775                 780
   Asp Leu Glu Thr Arg Leu Lys Ala Met Leu Val Ser His Lys Pro Asp
   785                 790                 795                 800
   His Gly Leu Gln Ala Arg Leu His Glu Asp Thr Ala Tyr Gly Thr Val
                   805                 810                 815
   Glu His Pro Glu Thr Glu Asp Gly Ala Asn Leu Val Tyr Arg Lys Thr
               820                 825                 830
   Phe Val Asp Ile Ser Glu Lys Glu Ile Asp Arg Ile Arg Asp Arg Arg
           835                 840                 845
   Leu Arg Asp Leu Val Arg Ala His Val Ala Gly Glu Arg Gln Gln Gly
       850                 855                 860
   Lys Thr Leu Lys Ala Ala Val Leu Ser Phe Ala Gln Arg Arg Asp Ile
   865                 870                 875                 880
   Ala Gly His Pro Asn Gly Ile Arg His Val Arg Leu Thr Lys Ser Ile
                   885                 890                 895
   Lys Pro Asp Tyr Leu Val Pro Ile Arg Asp Lys Ala Gly Arg Ile Tyr
               900                 905                 910
   Lys Ser Tyr Asn Ala Gly Glu Asn Ala Phe Val Asp Ile Leu Gln Ala
           915                 920                 925
   Glu Ser Gly Arg Trp Ile Ala Arg Ala Thr Thr Val Phe Gln Ala Asn
       930                 935                 940
   Gln Ala Asn Glu Ser His Asp Ala Pro Ala Ala Gln Pro Ile Met Arg
   945                 950                 955                 960
   Val Phe Lys Gly Asp Met Leu Arg Ile Asp His Ala Gly Ala Glu Lys
                   965                 970                 975
   Phe Val Lys Ile Val Arg Leu Ser Pro Ser Asn Asn Leu Leu Tyr Leu
               980                 985                 990
   Val Glu His His Gln Ala Gly Val Phe Gln Thr Arg His Asp Asp Pro
           995                 1000                1005
   Glu Asp Ser Phe Arg Trp Leu Phe Ala Ser Phe Asp Lys Leu Arg Glu
       1010                1015                1020
   Trp Asn Ala Glu Leu Val Arg Ile Asp Thr Leu Gly Gln Pro Trp Arg
   1025                1030                1035                1040
   Arg Lys Arg Gly Leu Glu Thr Gly Ser Glu Asp Ala Thr Arg Ile Gly
                   1045                1050                1055
   Trp Thr Arg Pro Lys Lys Trp Pro
               1060
<210> 159
   <211> 765
   <212> PRT
   <213> Butyrivibrio fibrisolvens
<220>
   <223> CRISPR-associated endonuclease
<308> YP_007818384
   <309> 2013-08-27
<400> 159
   Met Gly Ile Thr Ile Gly Leu Asp Leu Gly Val Ala Ser Val Gly Trp
   1 5 10 15
   Ala Val Val Asn Asp Asp Tyr Glu Ile Leu Glu Ser Cys Ser Asn Ile
   20 25 30
   Phe Pro Ser Ala Asp Ala Ser Lys Asn Ser Glu Arg Arg Gly Phe Arg
   35 40 45
   Gln Gly Arg Arg Leu Thr Arg Arg Arg Lys Asn Arg Ile His Asp Phe
   50 55 60
   Gln Lys Leu Trp Glu Asp Lys Gly Phe Val Ile Pro Ser Gln Gly Thr
   65 70 75 80
   Glu Asp Val Leu Ala Ile Lys Ile Lys Gly Leu Ser Glu Lys Leu Ser
   85 90 95
   Val Asp Glu Val Tyr Trp Val Leu Leu Asn Ser Leu Lys His Arg Gly
   100 105 110
   Ile Ser Tyr Leu Asp Asp Ala Asp Ser Gly Asp Asn Ser Ser Asp Tyr
   115 120 125
   Ala Lys Ser Ile Ser Arg Asn Glu Glu Glu Leu Lys Glu Lys Leu Pro
   130 135 140
   Cys Glu Ile Gln Trp Glu Arg Leu Gln Lys Tyr Gly Ala Tyr Arg Gly
   145 150 155 160
   Asn Ile Ser Ile Val Glu Asp Gly Glu Pro Ile Thr Leu Arg Asn Val
   165 170 175
   Phe Thr Thr Ser Ala Tyr Lys Lys Glu Val Glu Gln Phe Ile Asp Thr
   180 185 190
   Gln Ala Lys Tyr Asn Ala Gln Tyr Ser Gly Asp Phe Lys Ala Asp Tyr
   195 200 205
   Leu Glu Ile Phe Asn Arg Lys Arg Glu Tyr Tyr Glu Gly Pro Gly Asn
   210 215 220
   Glu Leu Ser Arg Thr Asp Tyr Gly Lys Tyr Thr Thr Glu Ile Asn Ala
   225 230 235 240
   Asp Gly Glu Tyr Ile Thr Val Asp Asn Ile Phe Asp Lys Leu Val Gly
   245 250 255
   Lys Cys Ser Val Asn Pro Asp Glu Arg Arg Ala Ala Gly Ala Ser Tyr
   260 265 270
   Thr Ala Gln Glu Phe Asn Val Leu Asn Asp Leu Asn Asn Leu Thr Ile
   275 280 285
   Ser Ser Glu Ser Ser Phe Ile Glu Asp Gly Lys Leu Thr Glu Asp Ala
   290 295 300
   Lys Arg Lys Ile Ile Glu Thr Ile Lys Asn Ala Lys Thr Val Asn Val
   305 310 315 320
   Lys Lys Ile Ile Cys Asp Val Ile Gly Asp Lys Lys Cys Gln Ile Ser
   325 330 335
   Gly Ala Arg Ile Asp Lys Asn Glu Lys Glu Ile Phe His Ser Phe Glu
   340 345 350
   Ala Tyr Asn Lys Met Arg Arg Ala Leu Glu Glu Ile Gly Phe Asp Ile
   355 360 365
   Ser Ser Leu Ser Arg Glu Asn Leu Asp Leu Ile Gly Asp Ile Leu Thr
   370 375 380
   Leu Asn Thr Asp Arg Glu Ser Ile Leu Asn Ala Phe Asn Arg Lys Gly
   385 390 395 400
   Ile Glu Leu Ala Asp Glu Ala Lys Asp Ile Leu Val Lys Val Arg Lys
   405 410 415
   Thr Asn Gly Ser Leu Phe Asn Lys Trp Gln Ser Phe Gly Leu Ser Ile
   420 425 430
   Met Asn Glu Leu Ile Pro Glu Leu Tyr Ala Gln Pro Lys Asn Gln Met
   435 440 445
   Glu Leu Leu Thr Ala Met Gly Val Phe Lys Ser Arg Gly Asp Arg Phe
   450 455 460
   Leu Glu Cys Lys Glu Ile Pro Gly Asp Leu Ile Val Asp Asp Ile Tyr
   465 470 475 480
   Asn Pro Val Val Ser Lys Thr Val Arg Ile Thr Val Arg Ile Leu Asn
   485 490 495
   Ala Leu Ile Lys Lys Tyr Gly Tyr Pro Asp Arg Val Val Ile Glu Met
   500 505 510
   Pro Arg Asp Lys Asn Ser Asp Glu Glu Gln Gln Arg Leu Lys Lys Glu
   515 520 525
   Gln Arg Asp Asn Glu Asn Glu Ile Lys Asp Ile Lys Ala Arg Val Lys
   530 535 540
   Thr Glu Tyr Gly Arg Glu Ile Thr Glu Glu Asp Phe Arg Gln His Ser
   545 550 555 560
   Lys Leu Ser Leu Lys Leu Lys Leu Trp Asn Glu Gln Gln Gly Ile Cys
   565 570 575
   Pro Tyr Ser Gly Lys Ser Ile Lys Ile Asp Asp Leu Leu Asp Asn Pro
   580 585 590
   Asn Leu Phe Glu Val Asp His Ile Ile Pro Leu Ser Ile Ser Phe Asp
   595 600 605
   Asp Ser Arg Asn Asn Lys Val Leu Val Tyr Ser Ser Glu Asn Gln Asp
   610 615 620
   Lys Gly Asn Arg Thr Pro Leu Ala Tyr Leu Ala Ser Val Asn Arg Gln
   625 630 635 640
   Trp Asp Ile His Ser Phe Met Asp Tyr Val Leu Lys Thr Tyr Ala Gly
   645 650 655
   Ala Gln Lys Arg Lys Lys Arg Asp Asn Leu Leu Asn Glu Gln Asp Ile
   660 665 670
   Thr Lys Val Glu Val Leu Gln Gly Phe Val Asn Arg Asn Ile Asn Asp
   675 680 685
   Thr Arg Tyr Ala Ser Lys Val Val Leu Asn Ser Leu Gln Glu Tyr Phe
   690 695 700
   Ser Ser Lys Glu Cys Ser Thr Lys Val Lys Val Ile Arg Gly Ser Phe
   705 710 715 720
   Thr His Gln Met Arg Val Asn Leu Lys Leu Lys Lys Thr Val Met Asn
   725 730 735
   His Met Phe Ile Met Gln Leu Met Gln Cys Leu Leu Leu Phe His Arg
   740 745 750
   Trp Asp Met Thr Leu Ile Met Asn Leu Gln Ile Ser Ile
   755 760 765
<210> 160
   <211> 1027
   <212> PRT
   <213> Bdellovibrio exovorus
<220>
   <223> CRISPR-associated endonuclease
<308> YP_007644735
   <309> 2013-06-11
<400> 160
   Met Lys Tyr Thr Leu Gly Leu Asp Val Gly Thr Asn Ser Ile Gly Trp
    1               5                  10                  15
   Ala Val Leu Glu Leu Asn Thr Asn Gly Glu Pro Gln Arg Ile Glu Lys
               20                  25                  30
   Leu Gly Ser Arg Ile Phe Thr Asp Gly Arg His Pro Lys Asp Gly Thr
           35                  40                  45
   Thr Leu Ala Ser Lys Arg Arg Gln Lys Arg His Glu Arg Arg Arg Arg
       50                  55                  60
   Asp Arg Leu His Gln Arg Lys Lys Leu Val Leu Asn Gln Leu Val Ser
   65                  70                  75                  80
   Met Gly Leu Phe Pro Gln Ser Glu Gln Glu Gln Gln Asp Leu Lys Ser
                   85                  90                  95
   Leu Asp Val Leu Thr Leu Arg Ala Asn Gly Val Ser Lys Gln Leu Ser
               100                 105                 110
   Ala Tyr Glu Leu Gly Arg Val Phe Tyr Tyr Leu Asn Leu Lys Arg Gly
           115                 120                 125
   Phe Lys Ser Asn Arg Lys Gly Gly Ser Glu Glu Asp Lys Ala Asn Lys
       130                 135                 140
   Ile Ser Glu Arg Ile Lys Lys Leu Gln Glu Gln Leu Lys Glu Asn Asp
   145                 150                 155                 160
   Phe Lys Thr Val Gly Gln Tyr Leu His Asp Arg Tyr Ile Lys Gly Leu
                   165                 170                 175
   Ser Thr Lys Ala Thr Ile Glu Asn Asp Phe His Leu Leu Arg Gln Leu
               180                 185                 190
   Ile Glu Asp Glu Phe Asp Phe Ile Val Glu Thr Gln Ser Lys Phe Phe
           195                 200                 205
   Pro Glu Ile Ser Asp Glu Gln Trp Asn Thr Leu Arg Asn Arg Met Phe
       210                 215                 220
   His Gln Arg Pro Leu Lys Pro Val Glu Thr Gly Thr Cys Ser Leu Phe
   225                 230                 235                 240
   Glu Asp Lys Lys Arg Thr Tyr Lys Tyr Met Pro Ser Phe Glu Glu Tyr
                   245                 250                 255
   Arg Phe Leu Thr Glu Leu Phe Asn Leu Ser Tyr Gln Asp Glu Asn Phe
               260                 265                 270
   His Ser His Ser Leu Ser Ser Glu Gln Ile Phe Lys Val Phe His Glu
           275                 280                 285
   Leu Lys Asn Thr Arg Glu Ala Thr Tyr Thr Lys Ile Lys Lys Leu Leu
       290                 295                 300
   Lys Leu Pro Ser Asp Leu Ser Phe Ser Ile Glu Arg Ser Lys Ser Lys
   305                 310                 315                 320
   Asp Lys Ile Lys Ile Ser Thr Thr Asn His Phe Phe Ser Lys Ser Leu
                   325                 330                 335
   Leu Phe Ala Glu Glu Trp Ala Ser Leu Asp Leu Lys Leu Lys Asp Glu
               340                 345                 350
   Val Ala Glu Ile Tyr Phe Ser Asp Met Ser Val Glu Glu Ile Lys Lys
           355                 360                 365
   Gly Leu Leu Gln Leu Lys Ile Asp Ser Glu Ile Ile Glu Asn Leu Leu
       370                 375                 380
   Glu Glu Lys Val Pro Asn Val Gln Glu Ala Thr Cys Ser Tyr Ser Lys
   385                 390                 395                 400
   Glu Ala Leu Gln Lys Ile Val Ser Leu Ile Leu Glu Glu Glu Arg His
                   405                 410                 415
   Pro Thr Leu Ile Val Asp Asp Leu Phe Lys Asn Glu Lys Glu Glu Thr
               420                 425                 430
   Ser Leu Ile Asp Arg Leu Asp Tyr Tyr Gly Ala Ser Ile Pro Asp Ser
           435                 440                 445
   Met Gln Pro Ile Pro Gln His Ile Lys Asp Asn Asn Leu Thr Leu Asn
       450                 455                 460
   Glu Asp Glu Lys Arg Tyr Gly Lys Ile Ala Asn Pro Thr Val His Ala
   465                 470                 475                 480
   Ala Leu Asn Gln Ile Arg Gln Val Val Asn Asp Leu Ile Glu Gln Tyr
                   485                 490                 495
   Gly Lys Pro Thr Asn Ile His Ile Glu Phe Ala Arg Asp Leu Lys Lys
               500                 505                 510
   Ser Lys Glu Glu Arg Asp Phe Asp Asn Arg Lys Lys Lys Lys Asn Glu
           515                 520                 525
   Glu Leu Asn Glu Gln Val Arg Asp Phe Ile Glu Lys His Asn Gln Lys
       530                 535                 540
   Pro Ser Ala Phe Asn Phe Glu Arg Val Lys Leu Trp Phe Glu Leu Asp
   545                 550                 555                 560
   Ala Met Asn Asn Gln Val Cys Ala Tyr Ser Gly Lys Thr Ile Ser Ala
                   565                 570                 575
   Arg Met Val Leu Ser Asp Glu Val Gln Val Asp His Ile Leu Pro Phe
               580                 585                 590
   Ser Arg Thr Leu Asp Asp Ser Leu Ala Asn Lys Val Leu Val Leu Ala
           595                 600                 605
   Ser Glu Asn Val Lys Lys Arg Asn Arg Thr Pro Tyr Glu Ala Phe Gly
       610                 615                 620
   Gln Asp Thr Glu Lys Trp Ala Ser Ile Gln Glu Leu Ala Lys Arg Leu
   625                 630                 635                 640
   Pro Tyr His Lys Gln Trp Arg Phe Ala Glu Asp Ala Ile Lys Lys Phe
                   645                 650                 655
   Glu Glu Lys Asp Gln Phe Leu Gln Arg His Leu Asn Asp Thr Arg Tyr
               660                 665                 670
   Ile Ser Lys Ile Ala Lys Lys Tyr Leu Gly Ser Ile Thr Asp Pro Tyr
           675                 680                 685
   Lys Ile Val Ala Ser Arg Gly Gln Met Thr Ser Ile Ile Arg Gly Lys
       690                 695                 700
   Leu Gly Leu Asn Gln Phe Ile Gln Lys Pro Asp Gly Thr Lys Asn Arg
   705                 710                 715                 720
   Glu Asp His Arg His His Ala Ile Asp Ala Leu Thr Val Ala Leu Thr
                   725                 730                 735
   Ser Arg Ser Tyr Leu Lys Arg Ile Ser Asp Ala Ser Ala Arg Asn Gln
               740                 745                 750
   Asp Pro Asn Arg Ile Pro Ile Pro Glu Pro Trp Lys Gly Phe Ile Lys
           755                 760                 765
   Asp Ala Asp Val Lys Phe Asn Glu Ile Ile Val Ser His Lys Val Asp
       770                 775                 780
   His Gly Lys Asn Gly Pro Phe Met Glu Glu Thr Cys Phe Gly Leu Ile
   785                 790                 795                 800
   Lys Asn Pro Asn Lys Tyr Glu Glu Glu Asn Asp Phe Lys Leu Ile Thr
                   805                 810                 815
   Thr Lys Ser Leu Asp Ser Val Lys Asp Pro Ser Thr Ile Arg Ser Gln
               820                 825                 830
   Ser Leu Gln Asn Leu Ala Glu Lys Ser Gly Val Ile Ser Leu Ala Lys
           835                 840                 845
   Ser Gly Val Lys Lys Leu Arg Val Tyr Asp Val Thr Lys Glu Asn Pro
       850                 855                 860
   Ala Asp Ile Gly Leu Ile Glu Ser Gly Leu Ala Lys Ile Thr His Gly
   865                 870                 875                 880
   Lys Asp Lys Gln His Val Lys His Tyr Gln Lys Gly Asp Ile Asn Tyr
                   885                 890                 895
   Leu Ala Ile Trp Cys Leu Pro Gln Asn Ile Thr Leu Lys Ser Asp Asn
               900                 905                 910
   His Lys Lys Arg Lys Thr Asp Tyr Ile Phe Thr Ala Ile Lys Thr Phe
           915                 920                 925
   Asp Leu Asn Ser Lys Asn Pro Asn Asp Leu Arg Pro His Pro Ala Ala
       930                 935                 940
   Lys Leu Val Thr Lys Val Tyr Lys Gly Asp Thr Val Ala Leu Glu Val
   945                 950                 955                 960
   Asn Asn Gln Leu Asn Tyr Tyr Ile Val Lys Ser Ile Arg Ala Ala Asn
                   965                 970                 975
   Lys Lys Leu Tyr Phe Leu Lys Ile Asn Gln Ser Ser Glu Leu Ala Asn
               980                 985                 990
   Glu Thr Gln Phe Ile Pro Ala Tyr Thr Lys Leu Val Asp Tyr Lys Phe
           995                 1000                1005
   Arg Lys Val Ser Val Ser Pro Thr Gly Val Ile Thr Asp Asn Gly Pro
       1010                1015                1020
   Leu Leu Lys
   1025
<210> 161
   <211> 1239
   <212> PRT
   <213> Mycoplasma cynos
<220>
   <223> CRISPR-associated endonuclease
<308> YP_007258684
   <309> 2013-06-11
<400> 161
   Met Glu Glu Lys Arg Lys Val Thr Leu Gly Phe Asp Leu Gly Val Ala
   1 5 10 15
   Ser Val Gly Trp Ala Ile Val Asp Ser Glu Thr Asn Glu Val Tyr Lys
   20 25 30
   Leu Gly Ser Arg Leu Phe Asp Ile Pro Asp Thr Asn Ile Asp Arg Arg
   35 40 45
   Ala Lys Arg Gly Ser Arg Arg Leu Val Arg Arg Arg Ser Tyr Arg Asn
   50 55 60
   Gln Lys Phe Tyr Asn Leu Ile Lys Arg Thr Lys Thr Phe Gly Phe Leu
   65 70 75 80
   Asp Lys Glu Ala Ile Glu Lys Glu Phe Val Leu Leu Ser Ile Lys Tyr
   85 90 95
   Pro Asn Ile Ile Glu Leu Lys Val Lys Gly Leu Gln Glu Glu Val Ser
   100 105 110
   Lys Ser Glu Ala Val Trp Ile Leu His Asp Tyr Leu Lys Asn Arg Gly
   115 120 125
   Tyr Phe Tyr Asp Asp Lys Glu Glu Lys Asp Asp Leu Glu Asn Pro Ser
   130 135 140
   Ile Glu Ser Met Pro Ser Tyr Lys Leu Tyr Glu Phe Tyr Lys Glu Tyr
   145 150 155 160
   Gly Tyr Phe Lys Gly Ala Leu Ser Ser Pro Thr Glu Ser Glu Lys Asn
   165 170 175
   Lys Asp Ser Asp Leu Glu Lys Ala Ile Phe Phe Asn Phe Ser Asn Lys
   180 185 190
   Glu Trp Thr Lys Glu Ile Asn His Phe Phe Lys Ile Gln Lys Asn Ile
   195 200 205
   Phe Ser Glu Asp Phe Lys Ala Lys Phe Met Glu Phe Phe Ser Phe Ile
   210 215 220
   Arg Asp Ile Ser Lys Gly Pro Gly Ser Glu Asn Met Pro Ser Gln Tyr
   225 230 235 240
   Ser Ile Phe Gly Lys Tyr Gly Asp Asp Gly Met Gly Gly Lys Tyr Gln
   245 250 255
   His Ile Trp Asp Lys Asn Ile Gly Lys Cys Ser Ile Phe Lys Asp Lys
   260 265 270
   Val Arg Ala Pro Lys Tyr Leu Pro Ser Ala Leu Phe Phe Asn Leu Leu
   275 280 285
   Asn Glu Leu Ser Asn Ile Arg Leu Tyr Ser Val Asp Lys Arg Glu Lys
   290 295 300
   Ser Val Leu Trp Arg Leu Ser Ser Lys Asp Lys Ile Asn Ile Ile Leu
   305 310 315 320
   Asn Ile Leu Gln Ile Ile Leu Phe Lys Asn Asp Gly Lys Ile Ser Asn
   325 330 335
   Leu Asp Ile Asn Lys Ile Val Lys Asn Glu Ser Ile Lys Ser Ile Ile
   340 345 350
   Leu Asn Glu Ile Asp Leu Asp Leu Ile Lys Asp Met Trp Phe Glu Lys
   355 360 365
   Glu Pro Asn Val Tyr Gly Ile Gly Leu Ser Gly Leu Asn Ile Glu Glu
   370 375 380
   Asn Lys Lys Glu Asn Arg Phe Lys Phe Gln Asp Leu Lys Ile Phe Ser
   385 390 395 400
   Val Phe Ile Asp Leu Leu Lys Lys Ser Asn Ile Asn Ile Glu Phe Asn
   405 410 415
   Asn Glu Lys Asp Ile Ile His Asn Leu Lys Leu Leu Asp Glu Leu Tyr
   420 425 430
   Phe Phe Leu Ile Thr Gln Lys Tyr Ser Arg Asp Lys Tyr Gly Ser Ile
           435                 440                 445
   Lys Lys Phe Ile Leu Asn Asn Lys Ser Phe Asn Ile Asp Asn Leu Asp
       450                 455                 460
   Glu Lys Leu Asn Ile Phe Leu Ser Ile Ser Asp Asp Glu Phe Asp Asn
   465                 470                 475                 480
   His Asn Ser Lys Thr His Ser Leu Ser Lys Glu Ala Ile Tyr Phe Ile
                   485                 490                 495
   Phe Pro Lys Leu Leu His Asn Asn Glu Gly Trp Asn Leu Glu Ala Ile
               500                 505                 510
   Lys Asn Tyr Asp Ile Asp Val Arg Glu Glu Ile Ser Lys His Ser Phe
           515                 520                 525
   Gly Ile Lys Lys Gln Asp Lys Lys Tyr Leu Asp Asp Ala Phe Leu Asp
       530                 535                 540
   Asp Ala Ile Leu Pro Pro Ser Val Lys Val Thr Ile Lys Gln Ser Ile
   545                 550                 555                 560
   Leu Ile Phe Asn Lys Ile Ile Lys Lys Phe Ser Asn Lys Phe Gln Ile
                   565                 570                 575
   Asp Asn Val Val Ile Glu Leu Ala Arg Glu Met Thr Gln Glu Lys Glu
               580                 585                 590
   Met Asp Ala Gln Lys Ser Leu Asn Arg Leu Lys Lys Ser Arg Lys Lys
           595                 600                 605
   Ile Ile Glu Glu Arg Leu Asn Ala Asn Asn Ile Asp Ser Lys Met Phe
       610                 615                 620
   Arg Asp Lys Thr Gly Glu Ile Ser Pro Asn Tyr Ile Tyr Lys Ile Tyr
   625                 630                 635                 640
   Leu Trp Ile Ser Gln Asn Phe Lys Asp Pro Tyr Thr Gly Glu Asn Ile
                   645                 650                 655
   Ser Ala Asn Asp Ile Leu Lys Asn Asn Val Glu Ile Asp His Ile Ile
               660                 665                 670
   Pro Tyr Ser Leu Cys Phe Asp Asp Ser Ser Ser Asn Lys Val Leu Val
           675                 680                 685
   Phe Lys His Ser Asn Gln Ser Lys Gly Asn Phe Leu Pro Phe Asp Met
       690                 695                 700
   Ile Ser Thr Phe Ser Glu Asn Ser Val Trp Asn Trp Lys Glu Tyr Thr
   705                 710                 715                 720
   Gln Tyr Val Glu Lys Thr Phe Lys Thr Asn Leu Glu Ser Ile Leu Asp
                   725                 730                 735
   Lys Lys Glu Arg Val Lys Lys Ala Asp Asn Leu Leu Thr Ser Ser Tyr
               740                 745                 750
   Asp Gly Tyr Asp Lys Leu Gly Phe Leu Ala Arg Asn Leu Asn Asp Thr
           755                 760                 765
   Arg Tyr Ala Thr Ile Leu Phe Arg Asp Gln Leu Ile Asn Tyr Ser Glu
       770                 775                 780
   Asn His Leu Ile Asp Ser Lys Lys Met Phe Lys Val Ile Ala Met Asn
   785                 790                 795                 800
   Gly Gly Val Thr Ser Phe Ile Arg Lys Asn Met Ser Phe Asp Ser Gly
                   805                 810                 815
   Leu Lys Val Lys Asp Arg Ser Ile Phe Ser His His Ala Tyr Asp Ala
               820                 825                 830
   Ala Ile Ile Ala Leu Phe Ser Asn Lys Thr Lys Ile Leu Tyr Asn Leu
           835                 840                 845
   Ile Asn Pro Ser Leu Asp Gly Ile Ile Ser Lys Arg Ser Glu Gly Tyr
       850                 855                 860
   Trp Val Leu Glu Asp Arg Leu Thr Gly Glu Ile Arg Lys Leu Asn Tyr
   865                 870                 875                 880
   Asn Asp Trp Gln Ser Ile Lys Asn Asn Val Glu Val Lys Lys Ile Ala
                   885                 890                 895
   Arg Glu Ile Glu Ser His Leu Ser Asn Leu Asp Asn Val Val Met Phe
               900                 905                 910
   Ser Arg Lys Ser Lys Arg Lys Thr Asn Lys Glu Leu Tyr Asn Glu Thr
           915                 920                 925
   Leu Tyr Gly Ile Ala Thr Arg Ile Asp Glu Asn Gly Ile Lys Asn Tyr
       930                 935                 940
   Tyr Lys Lys Glu Lys Phe Asn Ile Leu Glu Asp Glu Asn Ile Tyr Leu
   945 950 955 960
   Arg Leu Leu Asn Glu Arg Glu Asn Phe Ile Ile Asn Arg Ser Asn Pro
   965 970 975
   Glu Val Ile Asp Ala Ile Ile Glu Ile Phe Glu Thr Ile Asp Lys Asn
   980 985 990
   Lys Ile Pro Ala Arg Asp Val Ala Lys Asn Ile Lys Tyr Thr Asp Asn
   995 1000 1005
   Lys Ile Lys Tyr Asn Leu Tyr Leu Lys Asp Tyr Met Arg Lys Leu Val
   1010 1015 1020
   Asp Glu Phe Pro Asp Lys Phe Arg Lys Asp Phe Ile Glu Gln Met Ile
   1025 1030 1035 1040
   Ala Lys Lys Ile Phe Ile Leu Phe Asn Pro Ile Lys Asn Thr Thr Arg
   1045 1050 1055
   Lys Ile Lys Ser Leu Arg Ser Ile Asn Asn Asn Lys Ile Glu Asp Ile
   1060 1065 1070
   Arg Lys Lys Gln Val Leu Glu Lys Phe Asn Thr Ala Lys Asn Glu Pro
   1075 1080 1085
   Arg Ser Phe Tyr Glu Ser Leu Asn Ser Leu Gly Ala Ile Ile Phe Lys
   1090 1095 1100
   Asn Asn Leu Asn Ile Tyr Lys Lys Met Ser Ile Ser Ser Gln Ile Ala
   1105 1110 1115 1120
   Thr Phe Gly Asp Lys Asn Trp Lys Ile Asp Asp Ile Asn Thr Tyr Asn
   1125 1130 1135
   Lys Glu Lys Leu Asn Lys Tyr Lys Glu Ile Tyr Gly Ile Asp Glu Thr
   1140 1145 1150
   Tyr Asp Phe Tyr Ser Phe Ile Phe Pro Gly Thr Ile Leu Leu Asp Glu
   1155 1160 1165
   Lys Asn Lys Glu Phe Leu Tyr Ile Ser Ser Ile Gln Thr Leu Lys Asp
   1170 1175 1180
   Lys Ile Glu Leu Lys Phe Leu Asp Arg Ile Glu Phe Glu Lys Lys Asn
   1185 1190 1195 1200
   Val Asn Ser Asn Leu Lys Pro Lys Ile Lys Arg Glu Ile Ile Ser Thr
   1205 1210 1215
   Lys Lys Leu Met Ser Asp Tyr Lys Ile Ile Lys Ile Ser Pro Leu Gly
   1220 1225 1230
   Ile Asn Lys Lys Ile Phe Glu
   1235
<210> 162
   <211> 1370
   <212> PRT
   <213> Streptococcus agalactiae
<220>
   <223> CRISPR-associated endonuclease
<308> YP_006951177
   <309> 2013-06-11
<400> 162
   Met Asn Lys Pro Tyr Ser Ile Gly Leu Asp Ile Gly Thr Asn Ser Val
    1               5                  10                  15
   Gly Trp Ser Ile Ile Thr Asp Asp Tyr Lys Val Pro Ala Lys Lys Met
               20                  25                  30
   Arg Val Leu Gly Asn Thr Asp Lys Glu Tyr Ile Lys Lys Asn Leu Ile
           35                  40                  45
   Gly Ala Leu Leu Phe Asp Gly Gly Asn Thr Ala Ala Asp Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ala Glu Glu Met Ser Lys Val Asp Asp Ser
                   85                  90                  95
   Phe Phe His Arg Leu Glu Asp Ser Phe Leu Val Glu Glu Asp Lys Arg
               100                 105                 110
   Gly Ser Lys Tyr Pro Ile Phe Ala Thr Met Gln Glu Glu Lys Asp Tyr
           115                 120                 125
   His Glu Lys Phe Pro Thr Ile Tyr His Leu Arg Lys Glu Leu Ala Asp
       130                 135                 140
   Lys Lys Glu Lys Ala Asp Leu Arg Leu Val Tyr Leu Ala Leu Ala His
   145                 150                 155                 160
   Ile Ile Lys Phe Arg Gly His Phe Leu Ile Glu Asp Asp Arg Phe Asp
                   165                 170                 175
   Val Arg Asn Thr Asp Ile Gln Lys Gln Tyr Gln Ala Phe Leu Glu Ile
               180                 185                 190
   Phe Asp Thr Ser Phe Glu Asn Asn His Leu Leu Ser Gln Asn Val Asp
           195                 200                 205
   Val Glu Ala Ile Leu Thr Asp Lys Ile Ser Lys Ser Ala Lys Lys Asp
       210                 215                 220
   Arg Ile Leu Ala Gln Tyr Pro Asn Gln Lys Ser Thr Gly Ile Phe Ala
   225                 230                 235                 240
   Glu Phe Leu Lys Leu Ile Val Gly Asn Gln Ala Asp Phe Lys Lys His
                   245                 250                 255
   Phe Asn Leu Glu Asp Lys Thr Pro Leu Gln Phe Ala Lys Asp Ser Tyr
               260                 265                 270
   Asp Glu Asp Leu Glu Asn Leu Leu Gly Gln Ile Gly Asp Glu Phe Ala
           275                 280                 285
   Asp Leu Phe Ser Ala Ala Lys Lys Leu Tyr Asp Ser Val Leu Leu Ser
       290                 295                 300
   Gly Ile Leu Thr Val Thr Asp Leu Ser Thr Lys Ala Pro Leu Ser Ala
   305                 310                 315                 320
   Ser Met Ile Gln Arg Tyr Asp Glu His Arg Glu Asp Leu Lys Gln Leu
                   325                 330                 335
   Lys Gln Phe Val Lys Ala Ser Leu Pro Glu Lys Tyr Gln Glu Ile Phe
               340                 345                 350
   Ala Asp Ser Ser Lys Asp Gly Tyr Ala Gly Tyr Ile Glu Gly Lys Thr
           355                 360                 365
   Asn Gln Glu Ala Phe Tyr Lys Tyr Leu Ser Lys Leu Leu Thr Lys Gln
       370                 375                 380
   Glu Asp Ser Glu Tyr Phe Leu Glu Lys Ile Lys Asn Glu Asp Phe Leu
   385                 390                 395                 400
   Arg Lys Gln Arg Thr Phe Asp Asn Gly Ser Ile Pro His Gln Val His
                   405                 410                 415
   Leu Thr Glu Leu Lys Ala Ile Ile Arg Arg Gln Ser Glu Tyr Tyr Pro
               420                 425                 430
   Phe Leu Lys Glu Asn Gln Asp Lys Ile Glu Lys Ile Leu Thr Phe Arg
           435                 440                 445
   Ile Pro Tyr Tyr Val Gly Pro Leu Ala Arg Gly Asn Ser Asp Phe Ala
       450                 455                 460
   Trp Met Thr Arg Lys Thr Asp Asp Ser Ile Arg Pro Trp Asn Phe Glu
   465                 470                 475                 480
   Asp Leu Val Asp Lys Glu Lys Ser Ala Glu Ala Phe Ile His Arg Met
                   485                 490                 495
   Thr Asn Asn Asp Leu Tyr Leu Pro Glu Glu Lys Val Leu Pro Lys His
               500                 505                 510
   Ser Leu Ile Tyr Glu Lys Phe Thr Val Tyr Asn Glu Leu Thr Lys Val
           515                 520                 525
   Arg Tyr Lys Asn Glu Gln Gly Glu Thr Tyr Phe Phe Asp Ser Asn Val
       530                 535                 540
   Lys Gln Glu Ile Phe Asp Gly Val Phe Lys Glu His Arg Lys Val Ser
   545                 550                 555                 560
   Lys Lys Gln Leu Leu Asp Phe Leu Ala Lys Glu Phe Glu Glu Phe Arg
                   565                 570                 575
   Ile Val Asp Val Thr Gly Leu Asp Lys Glu Asn Lys Ala Phe Asn Ala
               580                 585                 590
   Ser Leu Gly Thr Tyr His Asp Leu Lys Lys Ile Leu Asp Lys Asp Phe
   595 600 605
   Leu Asp Asn Pro Asp Asn Glu Ser Ile Leu Glu Asp Ile Val Gln Thr
   610 615 620
   Ile Thr Leu Phe Glu Asp Arg Glu Met Ile Lys Lys Arg Leu Glu Asn
   625 630 635 640
   Tyr Lys Asp Leu Phe Thr Glu Ser Gln Leu Lys Lys Leu Tyr Arg Arg
   645 650 655
   His Tyr Thr Gly Trp Gly Arg Leu Ser Ala Lys Leu Ile Asn Gly Ile
   660 665 670
   Arg Asp Arg Glu Ser Gln Lys Thr Ile Leu Asp Tyr Leu Ile Ser Asp
   675 680 685
   Gly Arg Ala Asn Arg Asn Phe Met Gln Leu Ile Asn Asp Asp Gly Leu
   690 695 700
   Ser Phe Lys Ser Ile Ile Ser Lys Ala Gln Ser Gly Ser His Ser Asp
   705 710 715 720
   Asn Leu Lys Glu Val Val Gly Glu Leu Ala Gly Ser Pro Ala Ile Lys
   725 730 735
   Lys Gly Ile Leu Gln Ser Leu Lys Ile Val Asp Glu Leu Val Lys Val
   740 745 750
   Met Gly Tyr Glu Pro Glu Gln Ile Val Val Glu Met Ala Arg Glu Asn
   755 760 765
   Gln Thr Thr Asn Gln Gly Arg Arg Asn Ser Arg Gln Arg Tyr Lys Leu
   770 775 780
   Leu Glu Asp Gly Val Lys Asn Leu Ala Ser Asp Leu Asn Gly Asp Ile
   785 790 795 800
   Leu Lys Glu Tyr Pro Thr Asp Asn Gln Ala Leu Gln Asn Glu Arg Leu
   805 810 815
   Phe Leu Tyr Tyr Leu Gln Asn Gly Arg Asp Met Tyr Thr Gly Glu Ala
   820 825 830
   Leu Asp Ile Asp Asn Leu Ser Gln Tyr Asp Ile Asp His Ile Val Pro
   835 840 845
   Gln Ala Phe Ile Lys Asp Asp Ser Ile Asp Asn Arg Val Leu Val Ser
   850 855 860
   Ser Ala Lys Asn Arg Gly Lys Ser Asp Asp Val Pro Ser Ile Asp Ile
   865 870 875 880
   Val Lys Ala Arg Lys Ala Phe Trp Lys Lys Leu Leu Asp Ala Lys Leu
   885 890 895
   Ile Ser Gln Arg Lys Tyr Asp Asn Leu Thr Lys Ala Glu Arg Gly Gly
   900 905 910
   Leu Thr Pro Asp Asp Lys Ala Gly Phe Ile Gln Arg Gln Leu Val Glu
   915 920 925
   Thr Arg Gln Ile Thr Lys His Val Ala Arg Ile Leu Asp Glu Arg Phe
   930 935 940
   Asn Asn Lys Val Asp Asp Asn Asn Lys Pro Ile Arg Lys Val Lys Ile
   945 950 955 960
   Val Thr Leu Lys Ser Asn Leu Val Ser Asn Phe Arg Lys Glu Phe Gly
   965 970 975
   Phe Tyr Lys Ile Arg Glu Val Asn Asn Tyr His His Ala His Asp Ala
   980 985 990
   Tyr Leu Asn Ala Val Val Ala Lys Ala Ile Leu Thr Lys Tyr Pro Gln
   995 1000 1005
   Leu Glu Pro Glu Phe Val Tyr Gly Asp Tyr Pro Lys Tyr Asn Ser Tyr
   1010 1015 1020
   Lys Thr Arg Lys Ser Ala Thr Glu Lys Leu Phe Phe Tyr Ser Asn Ile
   1025 1030 1035 1040
   Met Asn Phe Phe Lys Thr Lys Val Thr Leu Ala Asp Glu Thr Val Val
   1045 1050 1055
   Val Lys Asp Asp Ile Glu Val Asn Asn Glu Thr Gly Glu Ile Ala Trp
   1060 1065 1070
   Asp Lys Lys Lys His Phe Ala Thr Val Arg Lys Val Leu Ser Tyr Pro
   1075 1080 1085
   Gln Val Asn Ile Val Lys Lys Thr Glu Val Gln Thr Gly Gly Phe Ser
   1090 1095 1100
   Lys Glu Ser Ile Leu Ala His Ser Asn Ser Asp Lys Leu Ile Pro Arg
   1105 1110 1115 1120
   Lys Thr Lys Asp Ile Tyr Leu Asp Pro Lys Lys Tyr Gly Gly Phe Asp
   1125 1130 1135
   Ser Pro Ile Val Ala Tyr Ser Val Leu Val Leu Ala Asp Ile Lys Lys
   1140 1145 1150
   Gly Lys Ala Gln Lys Leu Lys Thr Val Lys Glu Leu Ile Gly Ile Thr
   1155 1160 1165
   Ile Met Glu Arg Glu Arg Phe Glu Lys Asn Pro Ser Ala Phe Leu Glu
   1170 1175 1180
   Ser Lys Gly Tyr Leu Asn Ile Arg Thr Asp Lys Leu Ile Ile Leu Pro
   1185 1190 1195 1200
   Lys Tyr Ser Leu Phe Glu Leu Glu Asn Gly Arg Arg Arg Leu Leu Ala
   1205 1210 1215
   Ser Ala Gly Glu Leu Gln Lys Gly Asn Glu Leu Ala Leu Pro Thr Gln
   1220 1225 1230
   Tyr Met Lys Phe Leu Tyr Leu Ala Ser Arg Tyr Asn Glu Ser Lys Gly
   1235 1240 1245
   Lys Pro Glu Glu Ile Glu Lys Lys Gln Glu Phe Val Asn Gln His Val
   1250 1255 1260
   Ser Tyr Phe Asp Asp Ile Leu Gln Leu Ile Asn Asp Phe Ser Lys Arg
   1265 1270 1275 1280
   Val Ile Leu Ala Asp Ala Asn Leu Glu Lys Ile Asn Lys Leu Tyr Ser
   1285 1290 1295
   Asp Asn Lys Asp Asn Thr Pro Val Asp Glu Leu Ala Lys Asn Ile Ile
   1300 1305 1310
   Asn Leu Phe Thr Phe Thr Ser Leu Gly Ala Pro Ala Ala Phe Lys Phe
   1315 1320 1325
   Phe Asp Lys Ser Val Asp Arg Lys Arg Tyr Thr Ser Thr Lys Glu Val
   1330 1335 1340
   Leu Asp Ser Thr Leu Ile His Gln Ser Ile Thr Gly Leu Tyr Glu Thr
   1345 1350 1355 1360
   Arg Ile Asp Leu Gly Lys Leu Gly Glu Asp
   1365 1370
<210> 163
   <211> 1368
   <212> PRT
   <213> Streptococcus pyogenes
<220>
   <223> CRISPR-associated endonuclease
<308> YP_006932845
   <309> 2013-06-11
<400> 163
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
   115 120 125
   His Glu Lys Tyr Pro Thr Ile Tyr His Leu Arg Lys Lys Leu Val Asp
   130 135 140
   Ser Thr Asp Lys Ala Asp Leu Arg Leu Ile Tyr Leu Ala Leu Ala His
   145 150 155 160
   Met Ile Lys Phe Arg Gly His Phe Leu Ile Glu Gly Asp Leu Asn Pro
   165 170 175
   Asp Asn Ser Asp Val Asp Lys Leu Phe Ile Gln Leu Val Gln Thr Tyr
   180 185 190
   Asn Gln Leu Phe Glu Glu Asn Pro Ile Asn Ala Ser Gly Val Asp Ala
   195 200 205
   Lys Ala Ile Leu Ser Ala Arg Leu Ser Lys Ser Arg Arg Leu Glu Asn
   210 215 220
   Leu Ile Ala Gln Leu Pro Gly Glu Lys Lys Asn Gly Leu Phe Gly Asn
   225 230 235 240
   Leu Ile Ala Leu Ser Leu Gly Leu Thr Pro Asn Phe Lys Ser Asn Phe
   245 250 255
   Asp Leu Ala Glu Asp Ala Lys Leu Gln Leu Ser Lys Asp Thr Tyr Asp
   260 265 270
   Asp Asp Leu Asp Asn Leu Leu Ala Gln Ile Gly Asp Gln Tyr Ala Asp
   275 280 285
   Leu Phe Leu Ala Ala Lys Asn Leu Ser Asp Ala Ile Leu Leu Ser Asp
   290 295 300
   Ile Leu Arg Val Asn Thr Glu Ile Thr Lys Ala Pro Leu Ser Ala Ser
   305 310 315 320
   Met Ile Lys Arg Tyr Asp Glu His His Gln Asp Leu Thr Leu Leu Lys
   325 330 335
   Ala Leu Val Arg Gln Gln Leu Pro Glu Lys Tyr Lys Glu Ile Phe Phe
   340 345 350
   Asp Gln Ser Lys Asn Gly Tyr Ala Gly Tyr Ile Asp Gly Gly Ala Ser
   355 360 365
   Gln Glu Glu Phe Tyr Lys Phe Ile Lys Pro Ile Leu Glu Lys Met Asp
   370 375 380
   Gly Thr Glu Glu Leu Leu Val Lys Leu Asn Arg Glu Asp Leu Leu Arg
   385 390 395 400
   Lys Gln Arg Thr Phe Asp Asn Gly Ser Ile Pro His Gln Ile His Leu
   405 410 415
   Gly Glu Leu His Ala Ile Leu Arg Arg Gln Glu Asp Phe Tyr Pro Phe
   420 425 430
   Leu Lys Asp Asn Arg Glu Lys Ile Glu Lys Ile Leu Thr Phe Arg Ile
   435 440 445
   Pro Tyr Tyr Val Gly Pro Leu Ala Arg Gly Asn Ser Arg Phe Ala Trp
   450 455 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
   530 535 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Thr Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
           610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Gln Ile Leu Lys Glu His Pro
   785                 790                 795                 800
   Val Glu Asn Thr Gln Leu Gln Asn Glu Lys Leu Tyr Leu Tyr Tyr Leu
                   805                 810                 815
   Gln Asn Gly Arg Asp Met Tyr Val Asp Gln Glu Leu Asp Ile Asn Arg
               820                 825                 830
   Leu Ser Asp Tyr Asp Val Asp His Ile Val Pro Gln Ser Phe Leu Lys
           835                 840                 845
   Asp Asp Ser Ile Asp Asn Lys Val Leu Thr Arg Ser Asp Lys Asn Arg
       850                 855                 860
   Gly Lys Ser Asp Asn Val Pro Ser Glu Glu Val Val Lys Lys Met Lys
   865                 870                 875                 880
   Asn Tyr Trp Arg Gln Leu Leu Asn Ala Lys Leu Ile Thr Gln Arg Lys
                   885                 890                 895
   Phe Asp Asn Leu Thr Lys Ala Glu Arg Gly Gly Leu Ser Glu Leu Asp
               900                 905                 910
   Lys Ala Gly Phe Ile Lys Arg Gln Leu Val Glu Thr Arg Gln Ile Thr
           915                 920                 925
   Lys His Val Ala Gln Ile Leu Asp Ser Arg Met Asn Thr Lys Tyr Asp
       930                 935                 940
   Glu Asn Asp Lys Leu Ile Arg Glu Val Lys Val Ile Thr Leu Lys Ser
   945                 950                 955                 960
   Lys Leu Val Ser Asp Phe Arg Lys Asp Phe Gln Phe Tyr Lys Val Arg
                   965                 970                 975
   Glu Ile Asn Asn Tyr His His Ala His Asp Ala Tyr Leu Asn Ala Val
               980                 985                 990
   Val Gly Thr Ala Leu Ile Lys Lys Tyr Pro Lys Leu Glu Ser Glu Phe
           995                 1000                1005
   Val Tyr Gly Asp Tyr Lys Val Tyr Asp Val Arg Lys Met Ile Ala Lys
       1010                1015                1020
   Ser Glu Gln Glu Ile Gly Lys Ala Thr Ala Lys Tyr Phe Phe Tyr Ser
   1025                1030                1035                1040
   Asn Ile Met Asn Phe Phe Lys Thr Glu Ile Thr Leu Ala Asn Gly Glu
                   1045                1050                1055
   Ile Arg Lys Arg Pro Leu Ile Glu Thr Asn Gly Glu Thr Gly Glu Ile
               1060                1065                1070
   Val Trp Asp Lys Gly Arg Asp Phe Ala Thr Val Arg Lys Val Leu Ser
           1075                1080                1085
   Met Pro Gln Val Asn Ile Val Lys Lys Thr Glu Val Gln Thr Gly Gly
       1090                1095                1100
   Phe Ser Lys Glu Ser Ile Leu Pro Lys Arg Asn Ser Asp Lys Leu Ile
   1105                1110                1115                1120
   Ala Arg Lys Lys Asp Trp Asp Pro Lys Lys Tyr Gly Gly Phe Asp Ser
                   1125                1130                1135
   Pro Thr Val Ala Tyr Ser Val Leu Val Val Ala Lys Val Glu Lys Gly
               1140                1145                1150
   Lys Ser Lys Lys Leu Lys Ser Val Lys Glu Leu Leu Gly Ile Thr Ile
           1155                1160                1165
   Met Glu Arg Ser Ser Phe Glu Lys Asn Pro Ile Asp Phe Leu Glu Ala
       1170                1175                1180
   Lys Gly Tyr Lys Glu Val Lys Lys Asp Leu Ile Ile Lys Leu Pro Lys
   1185                1190                1195                1200
   Tyr Ser Leu Phe Glu Leu Glu Asn Gly Arg Lys Arg Met Leu Ala Ser
                   1205                1210                1215
   Ala Gly Glu Leu Gln Lys Gly Asn Glu Leu Ala Leu Pro Ser Lys Tyr
               1220                1225                1230
   Val Asn Phe Leu Tyr Leu Ala Ser His Tyr Glu Lys Leu Lys Gly Ser
           1235                1240                1245
   Pro Glu Asp Asn Glu Gln Lys Gln Leu Phe Val Glu Gln His Lys His
       1250                1255                1260
   Tyr Leu Asp Glu Ile Ile Glu Gln Ile Ser Glu Phe Ser Lys Arg Val
   1265                1270                1275                1280
   Ile Leu Ala Asp Ala Asn Leu Asp Lys Val Leu Ser Ala Tyr Asn Lys
                   1285                1290                1295
   His Arg Asp Lys Pro Ile Arg Glu Gln Ala Glu Asn Ile Ile His Leu
               1300                1305                1310
   Phe Thr Leu Thr Asn Leu Gly Ala Pro Ala Ala Phe Lys Tyr Phe Asp
           1315                1320                1325
   Thr Thr Ile Asp Arg Lys Arg Tyr Thr Ser Thr Lys Glu Val Leu Asp
       1330                1335                1340
   Ala Thr Leu Ile His Gln Ser Ile Thr Gly Leu Tyr Glu Thr Arg Ile
   1345                1350                1355                1360
   Asp Leu Ser Gln Leu Gly Gly Asp
                   1365
<210> 164
   <211> 1509
   <212> PRT
   <213> Psychroflexus torquis
<220>
   <223> CRISPR-associated endonuclease
<308> YP_006866082
   <309> 2013-06-11
<400> 164
   Met Lys Arg Ile Leu Gly Leu Asp Leu Gly Thr Asn Ser Ile Gly Trp
    1               5                  10                  15
   Ser Leu Ile Glu His Asp Phe Lys Asn Lys Gln Gly Gln Ile Glu Gly
               20                  25                  30
   Leu Gly Val Arg Ile Ile Pro Met Ser Gln Glu Ile Leu Gly Lys Phe
           35                  40                  45
   Asp Ala Gly Gln Ser Ile Ser Gln Thr Ala Asp Arg Thr Lys Tyr Arg
       50                  55                  60
   Gly Val Arg Arg Leu Tyr Gln Arg Asp Asn Leu Arg Arg Glu Arg Leu
   65                  70                  75                  80
   His Arg Val Leu Lys Ile Leu Asp Phe Leu Pro Lys His Tyr Ser Glu
                   85                  90                  95
   Ser Ile Asp Phe Gln Asp Lys Val Gly Gln Phe Lys Pro Lys Gln Glu
               100                 105                 110
   Val Lys Leu Asn Tyr Arg Lys Asn Glu Lys Asn Lys His Glu Phe Val
           115                 120                 125
   Phe Met Asn Ser Phe Ile Glu Met Val Ser Glu Phe Lys Asn Ala Gln
   130 135 140
   Pro Glu Leu Phe Tyr Asn Lys Gly Asn Gly Glu Glu Thr Lys Ile Pro
   145 150 155 160
   Tyr Asp Trp Thr Leu Tyr Tyr Leu Arg Lys Lys Ala Leu Thr Gln Gln
   165 170 175
   Ile Thr Lys Glu Glu Leu Ala Trp Leu Ile Leu Asn Phe Asn Gln Lys
   180 185 190
   Arg Gly Tyr Tyr Gln Leu Arg Gly Glu Asp Ile Asp Glu Asp Lys Asn
   195 200 205
   Lys Lys Tyr Met Gln Leu Lys Val Asn Asn Leu Ile Asp Ser Gly Ala
   210 215 220
   Lys Val Lys Gly Lys Val Leu Tyr Asn Val Ile Phe Asp Asn Gly Trp
   225 230 235 240
   Lys Tyr Glu Lys Gln Ile Val Asn Lys Asp Glu Trp Glu Gly Arg Thr
   245 250 255
   Lys Glu Phe Ile Ile Thr Thr Lys Thr Leu Lys Asn Gly Asn Ile Lys
   260 265 270
   Arg Thr Tyr Lys Ala Val Asp Ser Glu Ile Asp Trp Ala Ala Ile Lys
   275 280 285
   Ala Lys Thr Glu Gln Asp Ile Asn Lys Ala Asn Lys Thr Val Gly Glu
   290 295 300
   Tyr Ile Tyr Glu Ser Leu Leu Asp Asn Pro Ser Gln Lys Ile Arg Gly
   305 310 315 320
   Lys Leu Val Lys Thr Ile Glu Arg Lys Phe Tyr Lys Glu Glu Phe Glu
   325 330 335
   Lys Leu Leu Ser Lys Gln Ile Glu Leu Gln Pro Glu Leu Phe Asn Glu
   340 345 350
   Ser Leu Tyr Lys Ala Cys Ile Lys Glu Leu Tyr Pro Arg Asn Glu Asn
   355 360 365
   His Gln Ser Asn Asn Lys Lys Gln Gly Phe Glu Tyr Leu Phe Thr Glu
   370 375 380
   Asp Ile Ile Phe Tyr Gln Arg Pro Leu Lys Ser Gln Lys Ser Asn Ile
   385 390 395 400
   Ser Gly Cys Gln Phe Glu His Lys Ile Tyr Lys Gln Lys Asn Lys Lys
   405 410 415
   Thr Gly Lys Leu Glu Leu Ile Lys Glu Pro Ile Lys Thr Ile Ser Arg
   420 425 430
   Ser His Pro Leu Phe Gln Glu Phe Arg Ile Trp Gln Trp Leu Gln Asn
   435 440 445
   Leu Lys Ile Tyr Asn Lys Glu Lys Ile Glu Asn Gly Lys Leu Glu Asp
   450 455 460
   Val Thr Thr Gln Leu Leu Pro Asn Asn Glu Ala Tyr Val Thr Leu Phe
   465 470 475 480
   Asp Phe Leu Asn Thr Lys Lys Glu Leu Glu Gln Lys Gln Phe Ile Glu
   485 490 495
   Tyr Phe Val Lys Lys Lys Leu Ile Asp Lys Lys Glu Lys Glu His Phe
   500 505 510
   Arg Trp Asn Phe Val Glu Asp Lys Lys Tyr Pro Phe Ser Glu Thr Arg
   515 520 525
   Ala Gln Phe Leu Ser Arg Leu Ala Lys Val Lys Gly Ile Lys Asn Thr
   530 535 540
   Glu Asp Phe Leu Asn Lys Asn Thr Gln Val Gly Ser Lys Glu Asn Ser
   545 550 555 560
   Pro Phe Ile Lys Arg Ile Glu Gln Leu Trp His Ile Ile Tyr Ser Val
   565 570 575
   Ser Asp Leu Lys Glu Tyr Glu Lys Ala Leu Glu Lys Phe Ala Glu Lys
   580 585 590
   His Asn Leu Glu Lys Asp Ser Phe Leu Lys Asn Phe Lys Lys Phe Pro
   595 600 605
   Pro Phe Val Ser Asp Tyr Ala Ser Tyr Ser Lys Lys Ala Ile Ser Lys
   610 615 620
   Leu Leu Pro Ile Met Arg Met Gly Lys Tyr Trp Ser Glu Ser Ala Val
   625 630 635 640
   Pro Thr Gln Val Lys Glu Arg Ser Leu Ser Ile Met Glu Arg Val Lys
   645 650 655
   Val Leu Pro Leu Lys Glu Gly Tyr Ser Asp Lys Asp Leu Ala Asp Leu
   660 665 670
   Leu Ser Arg Val Ser Asp Asp Asp Ile Pro Lys Gln Leu Ile Lys Ser
   675 680 685
   Phe Ile Ser Phe Lys Asp Lys Asn Pro Leu Lys Gly Leu Asn Thr Tyr
   690 695 700
   Gln Ala Asn Tyr Leu Val Tyr Gly Arg His Ser Glu Thr Gly Asp Ile
   705 710 715 720
   Gln His Trp Lys Thr Pro Glu Asp Ile Asp Arg Tyr Leu Asn Asn Phe
   725 730 735
   Lys Gln His Ser Leu Arg Asn Pro Ile Val Glu Gln Val Val Met Glu
   740 745 750
   Thr Leu Arg Val Val Arg Asp Ile Trp Glu His Tyr Gly Asn Asn Glu
   755 760 765
   Lys Asp Phe Phe Lys Glu Ile His Val Glu Leu Gly Arg Glu Met Lys
   770 775 780
   Ser Pro Ala Gly Lys Arg Glu Lys Leu Ser Gln Arg Asn Thr Glu Asn
   785 790 795 800
   Glu Asn Thr Asn His Arg Ile Arg Glu Val Leu Lys Glu Leu Met Asn
   805 810 815
   Asp Ala Ser Val Glu Gly Gly Val Arg Asp Tyr Ser Pro Ser Gln Gln
   820 825 830
   Glu Ile Leu Lys Leu Tyr Glu Glu Gly Ile Tyr Gln Asn Pro Asn Thr
   835 840 845
   Asn Tyr Leu Lys Val Asp Glu Asp Glu Ile Leu Lys Ile Arg Lys Lys
   850 855 860
   Asn Asn Pro Thr Gln Lys Glu Ile Gln Arg Tyr Lys Leu Trp Leu Glu
   865 870 875 880
   Gln Gly Tyr Ile Ser Pro Tyr Thr Gly Lys Ile Ile Pro Leu Thr Lys
   885 890 895
   Leu Phe Thr His Glu Tyr Gln Ile Glu His Ile Ile Pro Gln Ser Arg
   900 905 910
   Tyr Tyr Asp Asn Ser Leu Gly Asn Lys Ile Ile Cys Glu Ser Glu Val
   915 920 925
   Asn Glu Asp Lys Asp Asn Lys Thr Ala Tyr Glu Tyr Leu Lys Val Glu
   930 935 940
   Lys Gly Ser Ile Val Phe Gly His Lys Leu Leu Asn Leu Asp Glu Tyr
   945 950 955 960
   Glu Ala His Val Asn Lys Tyr Phe Lys Lys Asn Lys Thr Lys Leu Lys
   965 970 975
   Asn Leu Leu Ser Glu Asp Ile Pro Glu Gly Phe Ile Asn Arg Gln Leu
   980 985 990
   Asn Asp Ser Arg Tyr Ile Ser Lys Leu Val Lys Gly Leu Leu Ser Asn
   995 1000 1005
   Ile Val Arg Glu Asn Gly Glu Gln Glu Ala Thr Ser Lys Asn Leu Ile
   1010 1015 1020
   Pro Val Thr Gly Val Val Thr Ser Lys Leu Lys Gln Asp Trp Gly Leu
   1025 1030 1035 1040
   Asn Asp Lys Trp Asn Glu Ile Ile Ala Pro Arg Phe Lys Arg Leu Asn
   1045 1050 1055
   Lys Leu Thr Asn Ser Asn Asp Phe Gly Phe Trp Asp Asn Asp Ile Asn
   1060 1065 1070
   Ala Phe Arg Ile Gln Val Pro Asp Ser Leu Ile Lys Gly Phe Ser Lys
   1075 1080 1085
   Lys Arg Ile Asp His Arg His His Ala Leu Asp Ala Leu Val Val Ala
   1090 1095 1100
   Cys Thr Ser Arg Asn His Thr His Tyr Leu Ser Ala Leu Asn Ala Glu
   1105 1110 1115 1120
   Asn Lys Asn Tyr Ser Leu Arg Asp Lys Leu Val Ile Lys Asn Glu Asn
   1125 1130 1135
   Gly Asp Tyr Thr Lys Thr Phe Gln Ile Pro Trp Gln Gly Phe Thr Ile
               1140                1145                1150
   Glu Ala Lys Asn Asn Leu Glu Lys Thr Val Val Ser Phe Lys Lys Asn
           1155                1160                1165
   Leu Arg Val Ile Asn Lys Thr Asn Asn Lys Phe Trp Ser Tyr Lys Asp
       1170                1175                1180
   Glu Asn Gly Asn Leu Asn Leu Gly Lys Asp Gly Lys Pro Lys Lys Lys
   1185                1190                1195                1200
   Leu Arg Lys Gln Thr Lys Gly Tyr Asn Trp Ala Ile Arg Lys Pro Leu
                   1205                1210                1215
   His Lys Glu Thr Val Ser Gly Ile Tyr Asn Ile Asn Ala Pro Lys Asn
               1220                1225                1230
   Lys Ile Ala Thr Ser Val Arg Thr Leu Leu Thr Glu Ile Lys Asn Glu
           1235                1240                1245
   Lys His Leu Ala Lys Ile Thr Asp Leu Arg Ile Arg Glu Thr Ile Leu
       1250                1255                1260
   Pro Asn His Leu Lys His Tyr Leu Asn Asn Lys Gly Glu Ala Asn Phe
   1265                1270                1275                1280
   Ser Glu Ala Phe Ser Gln Gly Gly Ile Glu Asp Leu Asn Lys Lys Ile
                   1285                1290                1295
   Thr Thr Leu Asn Glu Gly Lys Lys His Gln Pro Ile Tyr Arg Val Lys
               1300                1305                1310
   Ile Phe Glu Val Gly Ser Lys Phe Ser Ile Ser Glu Asp Glu Asn Ser
           1315                1320                1325
   Ala Lys Ser Lys Lys Tyr Val Glu Ala Ala Lys Gly Thr Asn Leu Phe
       1330                1335                1340
   Phe Ala Ile Tyr Leu Asp Glu Glu Asn Lys Lys Arg Asn Tyr Glu Thr
   1345                1350                1355                1360
   Ile Pro Leu Asn Glu Val Ile Thr His Gln Lys Gln Val Ala Gly Phe
                   1365                1370                1375
   Pro Lys Ser Glu Arg Leu Ser Val Gln Pro Asp Ser Gln Lys Gly Thr
               1380                1385                1390
   Phe Leu Phe Thr Leu Ser Pro Asn Asp Leu Val Tyr Val Pro Asn Asn
           1395                1400                1405
   Glu Glu Leu Glu Asn Arg Asp Leu Phe Asn Leu Gly Asn Leu Asn Val
       1410                1415                1420
   Glu Gln Ile Ser Arg Ile Tyr Lys Phe Thr Asp Ser Ser Asp Lys Thr
   1425                1430                1435                1440
   Cys Asn Phe Ile Pro Phe Gln Val Ser Lys Leu Ile Phe Asn Leu Lys
                   1445                1450                1455
   Lys Lys Glu Gln Lys Lys Leu Asp Val Asp Phe Ile Ile Gln Asn Glu
               1460                1465                1470
   Phe Gly Leu Gly Ser Pro Gln Ser Lys Asn Gln Lys Ser Ile Asp Asp
           1475                1480                1485
   Val Met Ile Lys Glu Lys Cys Ile Lys Leu Lys Ile Asp Arg Leu Gly
       1490                1495                1500
   Asn Ile Ser Lys Ala
   1505
<210> 165
   <211> 987
   <212> PRT
   <213> Campylobacter jejuni
<220>
   <223> CRISPR-associated endonuclease
<308> YP_006858515
   <309> 2013-07-07
<400> 165
   Met Ala Arg Ile Leu Ala Phe Asp Ile Gly Ile Ser Ser Ile Gly Trp
    1               5                  10                  15
   Ala Phe Ser Glu Asn Asp Glu Leu Lys Asp Cys Gly Val Arg Ile Phe
   20 25 30
   Thr Lys Ala Glu Asn Pro Lys Thr Gly Glu Ser Leu Ala Leu Pro Arg
   35 40 45
   Arg Leu Ala Arg Ser Ala Arg Lys Arg Leu Ala Arg Arg Lys Ala Arg
   50 55 60
   Leu Asn His Leu Lys His Leu Ile Ala Asn Glu Phe Lys Leu Asn Tyr
   65 70 75 80
   Glu Asp Tyr Gln Ser Phe Asp Glu Ser Leu Ala Lys Ala Tyr Lys Gly
   85 90 95
   Ser Leu Ile Ser Pro Tyr Glu Leu Arg Phe Arg Ala Leu Asn Glu Leu
   100 105 110
   Leu Ser Lys Gln Asp Phe Ala Arg Val Ile Leu His Ile Ala Lys Arg
   115 120 125
   Arg Gly Tyr Asp Asp Ile Lys Asn Asn Gly Asp Glu Glu Lys Ser Glu
   130 135 140
   Ile Leu Lys Ala Ile Lys Gln Asn Glu Glu Lys Leu Val Asn Tyr Gln
   145 150 155 160
   Ser Val Gly Glu Tyr Leu Tyr Lys Glu Tyr Phe Gln Lys Phe Lys Glu
   165 170 175
   Asn Ser Lys Glu Phe Ile Asn Val Arg Asn Lys Lys Glu Ser Tyr Glu
   180 185 190
   Arg Cys Ile Ala Gln Ser Phe Leu Lys Asp Glu Leu Lys Leu Ile Phe
   195 200 205
   Gln Lys Gln Arg Glu Phe Gly Phe Ser Phe Ser Lys Lys Phe Glu Glu
   210 215 220
   Glu Val Leu Ser Val Ala Phe Tyr Lys Arg Ala Leu Lys Asp Phe Ser
   225 230 235 240
   His Leu Val Gly Asn Cys Ser Phe Phe Thr Asp Glu Lys Arg Ala Pro
   245 250 255
   Lys Asn Ser Pro Leu Ala Phe Met Phe Val Ala Leu Thr Arg Ile Ile
   260 265 270
   Asn Leu Leu Asn Asn Leu Lys Asn Thr Glu Gly Ile Leu Tyr Thr Lys
   275 280 285
   Asp Asp Leu Asn Thr Leu Leu Asn Glu Val Leu Lys Asn Gly Thr Leu
   290 295 300
   Thr Tyr Lys Gln Thr Lys Lys Leu Leu Gly Leu Ser Asp Asp Tyr Glu
   305 310 315 320
   Phe Lys Gly Glu Lys Gly Thr Tyr Phe Ile Glu Phe Lys Lys Tyr Lys
   325 330 335
   Glu Phe Ile Lys Ala Leu Gly Glu His Asn Leu Ser Gln Asp Asn Leu
   340 345 350
   Asn Glu Ile Ala Lys Asp Ile Thr Leu Ile Lys Asp Glu Ile Lys Leu
   355 360 365
   Lys Lys Ala Leu Ala Lys Tyr Asp Leu Asn Gln Asn Gln Ile Asp Ser
   370 375 380
   Leu Ser Lys Leu Glu Phe Lys Asp His Leu Asn Ile Ser Phe Lys Ala
   385 390 395 400
   Leu Lys Leu Ile Thr Pro Leu Met Leu Glu Gly Lys Lys Tyr Asp Glu
   405 410 415
   Ala Tyr Asn Glu Leu Asn Leu Lys Val Ala Ile Asn Glu Asp Lys Lys
   420 425 430
   Asp Phe Leu Pro Ala Phe Asn Glu Thr Tyr Tyr Lys Asp Glu Val Thr
   435 440 445
   Asn Pro Val Val Leu Arg Ala Ile Lys Glu Tyr Arg Lys Val Leu Asn
   450 455 460
   Ala Leu Leu Lys Lys Tyr Gly Lys Val His Lys Ile Asn Ile Glu Leu
   465 470 475 480
   Ala Arg Glu Val Gly Lys Asn His Ser Gln Arg Ala Lys Ile Glu Lys
   485 490 495
   Glu Gln Asn Glu Asn Tyr Lys Ala Lys Lys Asp Ala Glu Leu Glu Cys
   500 505 510
   Glu Lys Leu Gly Leu Lys Ile Asn Ser Lys Asn Ile Leu Lys Leu Arg
           515                 520                 525
   Leu Phe Lys Glu Gln Lys Glu Phe Cys Ala Tyr Ser Gly Glu Lys Ile
       530                 535                 540
   Lys Ile Ser Asp Leu Gln Asp Glu Lys Met Leu Glu Ile Asp His Ile
   545                 550                 555                 560
   Tyr Pro Tyr Ser Arg Ser Phe Asp Asp Ser Tyr Met Asn Lys Val Leu
                   565                 570                 575
   Val Phe Thr Lys Gln Asn Gln Glu Lys Leu Asn Gln Thr Pro Phe Glu
               580                 585                 590
   Ala Phe Gly Asn Asp Ser Ala Lys Trp Gln Lys Ile Glu Val Leu Ala
           595                 600                 605
   Lys Asn Leu Pro Thr Lys Lys Gln Lys Arg Ile Leu Asp Lys Asn Tyr
       610                 615                 620
   Lys Asp Lys Glu Gln Lys Asp Phe Lys Asp Arg Asn Leu Asn Asp Thr
   625                 630                 635                 640
   Arg Tyr Ile Ala Arg Leu Val Leu Asn Tyr Thr Lys Asp Tyr Leu Asp
                   645                 650                 655
   Phe Leu Pro Leu Ser Asp Asp Glu Asn Thr Lys Leu Asn Asp Thr Gln
               660                 665                 670
   Lys Gly Ser Lys Val His Val Glu Ala Lys Ser Gly Met Leu Thr Ser
           675                 680                 685
   Ala Leu Arg His Thr Trp Gly Phe Ser Ala Lys Asp Arg Asn Asn His
       690                 695                 700
   Leu His His Ala Ile Asp Ala Val Ile Ile Ala Tyr Ala Asn Asn Ser
   705                 710                 715                 720
   Ile Val Lys Ala Phe Ser Asp Phe Lys Lys Glu Gln Glu Ser Asn Ser
                   725                 730                 735
   Ala Glu Leu Tyr Ala Lys Lys Ile Ser Glu Leu Asp Tyr Lys Asn Lys
               740                 745                 750
   Arg Lys Phe Phe Glu Pro Phe Ser Gly Phe Arg Gln Lys Val Leu Asp
           755                 760                 765
   Lys Ile Asp Glu Ile Phe Val Ser Lys Pro Glu Arg Lys Lys Pro Ser
       770                 775                 780
   Gly Ala Leu His Glu Glu Thr Phe Arg Lys Glu Glu Glu Phe Tyr Gln
   785                 790                 795                 800
   Ser Tyr Gly Gly Lys Glu Gly Val Leu Lys Ala Leu Glu Leu Gly Lys
                   805                 810                 815
   Ile Arg Lys Val Asn Gly Lys Ile Val Lys Asn Gly Asp Met Phe Arg
               820                 825                 830
   Val Asp Ile Phe Lys His Lys Lys Thr Asn Lys Phe Tyr Pro Val Pro
           835                 840                 845
   Ile Tyr Thr Met Asp Phe Ala Leu Lys Val Leu Pro Asn Lys Ala Val
       850                 855                 860
   Val Gln Gly Lys Asp Lys Lys Ser Gly Leu Ile Lys Asp Trp Ile Leu
   865                 870                 875                 880
   Met Asp Glu Asn Tyr Glu Phe Cys Phe Ser Leu Tyr Lys Asp Ser Leu
                   885                 890                 895
   Ile Leu Ile Gln Thr Lys Asp Met Gln Glu Pro Glu Leu Val Tyr Phe
               900                 905                 910
   Asn Ala Phe Thr Ser Ser Thr Val Ser Leu Ile Val Ser Lys His Asp
           915                 920                 925
   Asn Lys Phe Glu Thr Leu Ser Lys Asn Gln Lys Ile Leu Phe Lys Asn
       930                 935                 940
   Ala Asn Glu Lys Glu Val Ile Ala Lys Ser Ile Gly Ile Gln Asn Leu
   945                 950                 955                 960
   Lys Val Phe Glu Lys Tyr Ile Val Ser Ala Leu Gly Glu Val Thr Lys
                   965                 970                 975
   Ala Glu Phe Arg Gln Arg Glu Asp Phe Lys Lys
               980                 985
<210> 166
   <211> 1054
   <212> PRT
   <213> Actinobacillus suis
<220>
   <223> CRISPR-associated endonuclease
<308> YP_006816880
   <309> 2013-08-28
<400> 166
   Met Lys Leu Thr Pro Leu Asn Tyr Ile Leu Gly Leu Asp Leu Gly Ile
   1               5                   10                  15
   Ala Ser Val Gly Trp Ala Val Val Glu Ile Asp Glu Gln Glu Asn Pro
               20                  25                  30
   Leu Gly Leu Ile Asp Val Gly Val Arg Thr Phe Asp Arg Ala Glu Val
           35                  40                  45
   Pro Lys Thr Gly Glu Ser Leu Ala Leu Ala Arg Arg Leu Ala Arg Ser
       50                  55                  60
   Ala Arg Arg Leu Val Lys Arg Arg Ala Asp Arg Ile Lys Lys Ala Lys
   65                  70                  75                  80
   Arg Leu Leu Lys Ala Glu Asn Ile Leu Leu Ser Ala Asp Glu His Leu
                   85                  90                  95
   Pro Asn Asp Val Trp Gln Leu Arg Val Lys Gly Leu Asp Gln Lys Leu
               100                 105                 110
   Glu Arg Gln Glu Trp Ala Ala Val Leu Leu His Leu Leu Lys His Arg
           115                 120                 125
   Gly Tyr Leu Ser Gln Arg Lys Asn Glu Ser Lys Ser Glu Asn Lys Glu
       130                 135                 140
   Leu Gly Ala Leu Leu Ser Gly Val Glu Thr Asn His Gln Ile Leu Gln
   145                 150                 155                 160
   Ser Ala Glu Tyr Arg Thr Pro Ala Glu Ile Ala Val Lys Lys Phe His
                   165                 170                 175
   Val Glu Asp Gly His Ile Arg Asn Gln Arg Gly Ala Tyr Thr His Thr
               180                 185                 190
   Phe Ser Arg Leu Asp Leu Leu Ala Glu Met Glu Leu Leu Phe Gln Arg
           195                 200                 205
   Gln Thr Asp Leu Gly Asn Pro His Thr Ser Ala Lys Leu Leu Glu Asn
       210                 215                 220
   Leu Thr Ala Leu Leu Met Trp Gln Lys Pro Ala Leu Ala Gly Glu Ala
   225                 230                 235                 240
   Ile Leu Lys Met Leu Gly Lys Cys Thr Phe Glu Pro Thr Glu Tyr Lys
                   245                 250                 255
   Ala Ala Lys Asn Ser Tyr Ser Ala Glu Arg Phe Val Trp Leu Thr Lys
               260                 265                 270
   Leu Asn Asn Leu Arg Ile Leu Glu Gln Gly Ala Glu Arg Ala Leu Thr
           275                 280                 285
   Asp Asn Glu Arg Phe Ala Leu Leu Asp Gln Pro Tyr Glu Lys Ala Lys
       290                 295                 300
   Phe Thr Tyr Ala Gln Ala Arg Thr Met Leu Ala Leu Pro Asp Glu Ala
   305                 310                 315                 320
   Ile Phe Lys Gly Val Arg Tyr Gln Gly Glu Asp Lys Lys Ala Val Glu
                   325                 330                 335
   Thr Lys Thr Ile Leu Met Glu Met Lys Ala Tyr His Gln Ile Arg Lys
               340                 345                 350
   Ala Leu Glu Asn Ala Asp Leu Lys Ala Glu Trp Asn Glu Leu Lys Asn
           355                 360                 365
   Asn Ser Glu Leu Leu Asp Asp Ile Gly Thr Ala Phe Ser Leu His Lys
       370                 375                 380
   Thr Asp Glu Asp Ile Cys Arg Tyr Leu Asp Gly Lys Leu Ser Glu Ser
   385                 390                 395                 400
   Ile Leu Asn Ala Leu Leu Glu Asn Leu Asn Phe Asp Lys Phe Ile Gln
                   405                 410                 415
   Leu Ser Leu Lys Ala Leu Gln Gln Ile Leu Pro Leu Met Leu Gln Gly
               420                     425                     430
   Gln Arg Tyr Asp Glu Ala Val Ser Ala Ile Tyr Gly Asp His Tyr Gly
           435                     440                     445
   Lys Lys Ser Ala Glu Ile Asn Arg Leu Leu Pro Thr Ile Pro Ala Asp
       450                     455                     460
   Glu Ile Arg Asn Pro Val Val Leu Arg Thr Leu Thr Gln Ala Arg Lys
   465                     470                     475                     480
   Val Ile Asn Ala Val Val Arg Leu Tyr Gly Ser Pro Ala Arg Ile His
                   485                     490                     495
   Ile Glu Thr Gly Arg Glu Val Gly Lys Ser Tyr Gln Asp Arg Lys Lys
               500                     505                     510
   Leu Glu Lys Gln Gln Glu Asp Asn Arg Lys Gln Arg Glu Ser Ala Val
           515                     520                     525
   Lys Lys Phe Lys Glu Tyr Phe Pro Asn Phe Val Gly Glu Pro Lys Gly
       530                     535                     540
   Lys Asp Ile Leu Lys Met Arg Leu Tyr Glu Leu Gln Gln Ala Lys Cys
   545                     550                     555                     560
   Leu Tyr Ser Gly Lys Ser Leu Glu Leu His Arg Leu Leu Glu Lys Gly
                   565                     570                     575
   Tyr Val Glu Val Asp His Ala Leu Pro Phe Ser Arg Thr Trp Asp Asp
               580                     585                     590
   Ser Phe Asn Asn Lys Val Leu Val Leu Ala Asn Glu Asn Gln Asn Lys
           595                     600                     605
   Gly Asn Leu Thr Pro Tyr Glu Trp Leu Asp Gly Lys Asn Asn Ser Glu
       610                     615                     620
   His Trp Gln Asn Phe Val Ala Arg Val Gln Thr Ser Gly Phe Ser His
   625                     630                     635                     640
   Thr Lys Lys Gln Arg Ile Leu Ser His Lys Leu Asp Glu Lys Gly Phe
                   645                     650                     655
   Ile Glu Arg Asn Leu Asn Asp Thr Arg Tyr Val Ala Arg Phe Leu Cys
               660                     665                     670
   Asn Phe Ile Ala Asp Asn Met Leu Leu Thr Gly Lys Gly Lys Arg Lys
           675                     680                     685
   Val Phe Ala Ser Asn Gly Gln Ile Thr Ala Leu Leu Arg Gly Arg Trp
       690                     695                     700
   Gly Leu Gln Lys Val Arg Asp Asp Asn Asp Arg His His Ala Leu Asp
   705                     710                     715                     720
   Ala Val Val Val Ala Cys Ser Thr Val Val Met Gln Gln Lys Ile Thr
                   725                     730                     735
   Arg Phe Val Arg Tyr Glu Glu Gly Asn Val Phe Ser Gly Glu Arg Ile
               740                     745                     750
   Asp Arg Glu Thr Gly Glu Ile Ile Pro Leu His Phe Pro Ser Pro Trp
           755                     760                     765
   Ala Phe Phe Arg Glu Asn Val Glu Ile Arg Ile Phe Ser Glu Asn Pro
       770                     775                     780
   Lys Leu Glu Leu Glu Asn Arg Leu Pro Asp Tyr Pro Gln Tyr Asn His
   785                     790                     795                     800
   Glu Phe Val Gln Pro Leu Phe Val Ser Arg Met Pro Thr Arg Lys Met
                   805                     810                     815
   Thr Gly Gln Gly His Met Glu Thr Val Lys Ser Ala Lys Arg Leu Asp
               820                     825                     830
   Glu Gly Leu Ser Val Leu Lys Val Pro Leu Thr Gln Leu Lys Leu Ser
           835                     840                     845
   Asp Leu Glu Arg Met Val Asn Arg Glu Arg Glu Val Thr Leu Tyr Glu
       850                     855                     860
   Ser Leu Lys Ala Arg Leu Glu Gln Phe Gly Asn Asp Pro Ala Lys Ala
   865                     870                     875                     880
   Phe Ala Glu Pro Phe His Lys Lys Gly Gly Ala Val Val Lys Ala Val
                   885                     890                     895
   Arg Val Glu Gln Thr Gln Lys Ser Gly Val Leu Val Arg Asp Gly Asn
               900                     905                     910
   Gly Val Ala Asp Asn Ala Ser Met Val Arg Val Asp Val Phe Thr Lys
           915                     920                     925
   Gly Gly Lys Tyr Phe Leu Val Pro Ile Tyr Thr Trp Gln Val Ala Lys
       930                 935                 940
   Gly Ile Leu Pro Asn Lys Ala Val Thr Ala Asn Val Asp Glu Ile Asp
   945                 950                 955                 960
   Trp Leu Glu Met Asp Glu Ser Tyr Gln Phe Ile Phe Thr Met Tyr Pro
                   965                 970                 975
   Asn Asp Leu Val Lys Val Lys Leu Lys Lys Glu Glu Phe Phe Gly Tyr
               980                 985                 990
   Tyr Gly Gly Leu Asp Arg Ala Thr Gly Ala Ile Val Ile Lys Glu His
           995                 1000                1005
   Asp Leu Glu Lys Ser Lys Gly Lys Gln Gly Ile Tyr Arg Ile Gly Val
       1010                1015                1020
   Lys Leu Ala Leu Ser Phe Glu Lys Tyr Gln Val Asp Glu Leu Gly Lys
   1025                1030                1035                1040
   Asn Ile Arg Pro Cys Arg Pro Thr Lys Arg Gln His Val Arg
                   1045                1050
<210> 167
   <211> 1334
   <212> PRT
   <213> Listeria monocytogenes
<220>
   <223> CRISPR-associated endonuclease
<308> YP_006680178
   <309> 2013-06-11
<400> 167
   Met Lys Asn Pro Tyr Thr Ile Gly Leu Asp Ile Gly Thr Asn Ser Val
   1               5                   10                  15
   Gly Trp Ala Val Leu Thr Asp Gln Tyr Asp Leu Val Lys Arg Lys Met
               20                  25                  30
   Lys Val Ala Gly Asn Ser Asp Lys Lys Gln Ile Lys Lys Asn Phe Trp
           35                  40                  45
   Gly Val Arg Leu Phe Asp Asp Gly Gln Thr Ala Val Asp Arg Arg Met
       50                  55                  60
   Asn Arg Thr Ala Arg Arg Arg Ile Glu Arg Arg Arg Asn Arg Ile Ser
   65                  70                  75                  80
   Tyr Leu Gln Glu Ile Phe Ala Val Glu Met Ala Asn Ile Asp Ala Asn
                   85                  90                  95
   Phe Phe Cys Arg Leu Asn Asp Ser Phe Tyr Val Asp Ser Glu Lys Arg
               100                 105                 110
   Asn Ser Arg His Pro Phe Phe Ala Thr Ile Glu Glu Glu Val Ala Tyr
           115                 120                 125
   His Lys Asn Tyr Arg Thr Ile Tyr His Leu Arg Glu Glu Leu Val Asn
       130                 135                 140
   Ser Ser Glu Lys Ala Asp Leu Arg Leu Val Tyr Leu Ala Leu Ala His
   145                 150                 155                 160
   Ile Ile Lys Tyr Arg Gly Asn Phe Leu Ile Glu Gly Ala Leu Asp Thr
                   165                 170                 175
   Lys Asn Thr Ser Val Asp Glu Val Tyr Lys Gln Phe Ile Gln Thr Tyr
               180                 185                 190
   Asn Gln Val Phe Met Ser Asn Ile Glu Glu Gly Ala Leu Ala Lys Val
           195                 200                 205
   Glu Glu Asn Thr Glu Val Ala Ser Ile Leu Ala Gly Lys Phe Thr Arg
       210                 215                 220
   Arg Glu Lys Phe Glu Arg Ile Leu Arg Leu Tyr Pro Gly Glu Lys Ser
   225                 230                 235                 240
   Thr Gly Met Phe Ala Gln Phe Ile Ser Leu Ile Val Gly Asn Lys Gly
                   245                 250                 255
   Asn Phe Gln Lys Val Phe Asn Leu Val Glu Lys Thr Asp Ile Glu Cys
                   260                 265                 270
   Ala Lys Asp Ser Tyr Glu Glu Asp Leu Glu Ala Leu Leu Ala Ile Ile
               275                 280                 285
   Gly Asp Glu Tyr Ala Glu Leu Phe Val Ala Ala Lys Asn Thr Tyr Asn
       290                 295                 300
   Ala Val Val Leu Ser Ser Ile Ile Thr Val Thr Ala Thr Glu Thr Asn
   305                 310                 315                 320
   Ala Lys Leu Ser Ala Ser Met Ile Glu Arg Phe Asp Ala His Glu Lys
                   325                 330                 335
   Glu Leu Gly Glu Leu Lys Ala Phe Ile Lys Leu His Leu Pro Lys Gln
               340                 345                 350
   Tyr Gln Glu Ile Phe Asn Asn Ala Glu Ile Asp Gly Tyr Ala Gly Tyr
           355                 360                 365
   Ile Asp Gly Lys Thr Lys Gln Val Asp Phe Tyr Lys Tyr Leu Lys Thr
       370                 375                 380
   Thr Leu Glu Asn Val Glu Gly Ala Asp Tyr Phe Ile Thr Lys Ile Glu
   385                 390                 395                 400
   Glu Glu Asn Phe Leu Arg Lys Gln Arg Thr Phe Asp Asn Gly Val Ile
                   405                 410                 415
   Pro His Gln Leu His Leu Glu Glu Leu Glu Ala Ile Leu His Gln Gln
               420                 425                 430
   Ala Lys Tyr Tyr Pro Phe Leu Arg Glu Gly Tyr Asp Lys Ile Lys Ser
           435                 440                 445
   Leu Val Thr Phe Arg Ile Pro Tyr Phe Val Gly Pro Leu Ala Asn Gly
       450                 455                 460
   Gln Ser Glu Phe Ala Trp Leu Thr Arg Lys Asp Asp Gly Glu Ile Arg
   465                 470                 475                 480
   Pro Trp Asn Ile Glu Glu Lys Val Asp Phe Gly Lys Ser Ala Val Asp
                   485                 490                 495
   Phe Ile Glu Lys Met Thr Asn Lys Asp Thr Tyr Leu Pro Lys Glu Asn
               500                 505                 510
   Val Leu Pro Lys His Ser Leu Cys Tyr Gln Lys Tyr Met Val Tyr Asn
           515                 520                 525
   Glu Leu Thr Lys Ile Arg Tyr Ile Asp Asp Gln Gly Lys Thr Asn Tyr
       530                 535                 540
   Phe Ser Gly Arg Glu Lys Gln Gln Ile Phe Asn Asp Leu Phe Lys Gln
   545                 550                 555                 560
   Lys Arg Lys Val Lys Lys Lys Asp Leu Glu Leu Phe Leu Arg Asn Ile
                   565                 570                 575
   Asn His Ile Glu Ser Pro Thr Ile Glu Gly Leu Glu Asp Ser Phe Asn
               580                 585                 590
   Ala Ser Tyr Ala Thr Tyr His Asp Leu Leu Lys Val Gly Leu Lys Gln
           595                 600                 605
   Glu Ile Leu Asp Asn Pro Leu Asn Thr Glu Ile Leu Glu Asp Ile Val
       610                 615                 620
   Lys Ile Leu Thr Val Phe Glu Asp Lys Arg Met Ile Lys Glu Gln Leu
   625                 630                 635                 640
   Glu Gln Phe Ser Asp Val Leu Asp Gly Val Val Leu Lys Lys Leu Glu
                   645                 650                 655
   Arg Arg His Tyr Thr Gly Trp Gly Arg Leu Ser Ala Lys Leu Leu Val
               660                 665                 670
   Gly Ile Arg Asp Lys Gln Ser His Leu Thr Ile Leu Asp Tyr Leu Met
           675                 680                 685
   Asn Asp Asp Gly Leu Asn Arg Asn Leu Met Gln Leu Ile Asn Asp Ser
       690                 695                 700
   Asn Leu Ser Phe Lys Ser Ile Ile Glu Lys Glu Gln Val Ser Thr Thr
   705                 710                 715                 720
   Asp Lys Asp Leu Gln Ser Ile Val Ala Asp Leu Ala Gly Ser Pro Ala
                   725                 730                 735
   Ile Lys Lys Gly Ile Leu Gln Ser Leu Lys Ile Val Asp Glu Leu Val
               740                 745                 750
   Ser Ile Met Gly Tyr Pro Pro Gln Thr Ile Val Val Glu Met Ala Arg
           755                 760                 765
   Glu Asn Gln Thr Thr Gly Lys Gly Lys Asn Asn Ser Lys Pro Arg Tyr
   770 775 780
   Lys Ser Leu Glu Lys Ala Ile Lys Asp Phe Gly Ser Gln Ile Leu Lys
   785 790 795 800
   Glu His Pro Thr Asp Asn Gln Glu Leu Lys Asn Asn Arg Leu Tyr Leu
   805 810 815
   Tyr Tyr Leu Gln Asn Gly Lys Asp Ile Tyr Thr Gly Gln Glu Leu Asp
   820 825 830
   Ile His Asn Leu Ser Asn Tyr Asp Ile Asp His Ile Val Pro Gln Ser
   835 840 845
   Phe Ile Thr Asp Asn Ser Ile Asp Asn Leu Val Leu Thr Ser Ser Ala
   850 855 860
   Gly Asn Arg Glu Lys Gly Gly Asp Val Pro Pro Leu Glu Ile Val Arg
   865 870 875 880
   Lys Arg Lys Val Phe Trp Glu Lys Leu Tyr Gln Gly Asn Leu Met Ser
   885 890 895
   Lys Arg Lys Phe Asp Tyr Leu Thr Lys Ala Glu Arg Gly Gly Leu Thr
   900 905 910
   Glu Ala Asp Lys Ala Arg Phe Ile His Arg Gln Leu Val Glu Thr Arg
   915 920 925
   Gln Ile Thr Lys Asn Val Ala Asn Ile Leu His Gln Arg Phe Asn Lys
   930 935 940
   Glu Thr Asp Asn His Gly Asn Thr Met Glu Gln Val Arg Ile Val Thr
   945 950 955 960
   Leu Lys Ser Ala Leu Val Ser Gln Phe Arg Lys Gln Phe Gln Leu Tyr
   965 970 975
   Lys Val Arg Glu Val Asn Asp Tyr His His Ala His Asp Ala Tyr Leu
   980 985 990
   Asn Gly Val Val Ala Asn Thr Leu Leu Lys Val Tyr Pro Gln Leu Glu
   995 1000 1005
   Pro Glu Phe Val Tyr Gly Glu Tyr His Gln Phe Asp Trp Phe Lys Ala
   1010 1015 1020
   Asn Lys Ala Thr Ala Lys Lys Gln Phe Tyr Thr Asn Ile Met Leu Phe
   1025 1030 1035 1040
   Phe Ala Gln Lys Glu Arg Ile Ile Asp Glu Asn Gly Glu Ile Leu Trp
   1045 1050 1055
   Asp Lys Lys Tyr Leu Glu Thr Ile Lys Lys Val Leu Asp Tyr Arg Gln
   1060 1065 1070
   Ile Asn Ile Val Lys Lys Thr Glu Ile Gln Lys Gly Glu Phe Ser Lys
   1075 1080 1085
   Ala Thr Ile Lys Pro Lys Gly Asn Ser Ser Lys Leu Ile Pro Arg Lys
   1090 1095 1100
   Glu Asn Trp Asp Pro Val Lys Tyr Gly Gly Leu Asp Ser Pro Asn Met
   1105 1110 1115 1120
   Ala Tyr Ala Val Ile Ile Glu His Ala Lys Gly Lys Lys Lys Ile Val
   1125 1130 1135
   Ile Glu Lys Lys Leu Ile Gln Ile Asn Ile Met Glu Arg Lys Met Phe
   1140 1145 1150
   Glu Lys Asp Glu Glu Ala Phe Leu Glu Glu Lys Gly Tyr His Gln Pro
   1155 1160 1165
   Lys Val Leu Thr Lys Leu Pro Lys Tyr Thr Leu Tyr Glu Cys Glu Lys
   1170 1175 1180
   Gly Arg Arg Arg Met Leu Ser Ser Ala Asn Glu Ala Gln Lys Gly Asn
   1185 1190 1195 1200
   Gln Leu Val Leu Ser Asn His Leu Val Ser Leu Leu Tyr His Ala Lys
   1205 1210 1215
   Asn Cys Glu Ala Ser Asp Gly Lys Ser Leu Lys Tyr Ile Glu Ala His
   1220 1225 1230
   Arg Glu Thr Phe Ser Glu Leu Leu Ala Gln Val Ser Glu Phe Ala Thr
   1235 1240 1245
   Lys Tyr Thr Leu Ala Asp Ala Asn Leu Ser Lys Ile Asn Asn Leu Phe
   1250 1255 1260
   Glu Gln Asn Lys Glu Gly Asp Ile Lys Ala Ile Ala Gln Ser Phe Val
   1265                1270                1275                1280
   Asp Leu Met Ala Phe Asn Ala Met Gly Ala Pro Ala Ser Phe Lys Phe
                   1285                1290                1295
   Phe Glu Ala Thr Ile Asp Arg Lys Arg Tyr Thr Asn Leu Lys Glu Leu
               1300                1305                1310
   Leu Ser Ser Thr Ile Ile Tyr Gln Ser Ile Thr Gly Leu Tyr Glu Ser
           1315                1320                1325
   Arg Lys Arg Leu Asp Asp
       1330
<210> 168
   <211> 1334
   <212> PRT
   <213> Listeria monocytogenes
<220>
   <223> CRISPR-associated endonuclease
<308> YP_006697432
   <309> 2013-08-28
<400> 168
   Met Lys Asn Pro Tyr Thr Ile Gly Leu Asp Ile Gly Thr Asn Ser Val
   1 5 10 15
   Gly Trp Ala Val Leu Thr Asn Gln Tyr Asp Leu Val Lys Arg Lys Met
   20 25 30
   Lys Val Ala Gly Asn Ser Asp Lys Lys Gln Ile Lys Lys Asn Phe Trp
   35 40 45
   Gly Val Arg Leu Phe Asp Asp Gly Gln Thr Ala Val Asp Arg Arg Met
   50 55 60
   Asn Arg Thr Ala Arg Arg Arg Ile Glu Arg Arg Arg Asn Arg Ile Ser
   65 70 75 80
   Tyr Leu Gln Glu Ile Phe Ala Val Glu Met Ala Asn Ile Asp Ala Asn
   85 90 95
   Phe Phe Cys Arg Leu Asn Asp Ser Phe Tyr Val Asp Ser Glu Lys Arg
   100 105 110
   Asn Ser Arg His Pro Phe Phe Ala Thr Ile Glu Glu Glu Val Ala Tyr
   115 120 125
   His Lys Asn Tyr Arg Thr Ile Tyr His Leu Arg Glu Glu Leu Val Asn
   130 135 140
   Ser Ser Glu Lys Ala Asp Leu Arg Leu Val Tyr Leu Ala Leu Ala His
   145 150 155 160
   Ile Ile Lys Tyr Arg Gly Asn Phe Leu Ile Glu Gly Ala Leu Asp Thr
   165 170 175
   Lys Asn Thr Ser Val Asp Gly Val Tyr Glu Gln Phe Ile Gln Thr Tyr
   180 185 190
   Asn Gln Val Phe Met Ser Asn Ile Glu Glu Gly Thr Leu Ala Lys Val
   195 200 205
   Glu Glu Asn Ile Glu Val Ala Asn Ile Leu Ala Gly Lys Phe Thr Arg
   210 215 220
   Arg Glu Lys Phe Glu Arg Ile Leu Gln Leu Tyr Pro Gly Glu Lys Ser
   225 230 235 240
   Thr Gly Met Phe Ala Gln Phe Ile Ser Leu Ile Val Gly Ser Lys Gly
   245 250 255
   Asn Phe Gln Lys Val Phe Asp Leu Ile Glu Lys Thr Asp Ile Glu Cys
   260 265 270
   Ala Lys Asp Ser Tyr Glu Glu Asp Leu Glu Ala Leu Leu Ala Ile Ile
   275 280 285
   Gly Asp Glu Tyr Ala Glu Leu Phe Val Ala Ala Lys Asn Thr Tyr Asn
   290 295 300
   Ala Val Val Leu Ser Ser Ile Ile Thr Val Thr Ala Thr Glu Thr Asn
   305 310 315 320
   Ala Lys Leu Ser Ala Ser Met Ile Glu Arg Phe Asp Ala His Glu Lys
                   325                 330                 335
   Asp Leu Gly Glu Leu Lys Ala Phe Ile Lys Leu His Leu Pro Lys Gln
               340                 345                 350
   Tyr Gln Glu Ile Phe Asn Asn Ala Ala Ile Asp Gly Tyr Ala Gly Tyr
           355                 360                 365
   Ile Asp Gly Lys Thr Lys Gln Val Asp Phe Tyr Lys Tyr Leu Lys Thr
       370                 375                 380
   Ile Leu Glu Asn Ile Glu Gly Ala Asp Tyr Phe Ile Ala Lys Ile Glu
   385                 390                 395                 400
   Glu Glu Asn Phe Leu Arg Lys Gln Arg Thr Phe Asp Asn Gly Ala Ile
                   405                 410                 415
   Pro His Gln Leu His Leu Glu Glu Leu Glu Ala Ile Ile His Gln Gln
               420                 425                 430
   Ala Lys Tyr Tyr Pro Phe Leu Arg Glu Asp Tyr Glu Lys Ile Lys Ser
           435                 440                 445
   Leu Val Thr Phe Arg Ile Pro Tyr Phe Val Gly Pro Leu Ala Lys Gly
       450                 455                 460
   Gln Ser Glu Phe Ala Trp Leu Thr Arg Lys Ala Asp Gly Glu Ile Arg
   465                 470                 475                 480
   Pro Trp Asn Ile Glu Glu Lys Val Asp Phe Gly Lys Ser Ala Val Asp
                   485                 490                 495
   Phe Ile Glu Lys Met Thr Asn Lys Asp Thr Tyr Leu Pro Lys Glu Asn
               500                 505                 510
   Val Leu Pro Lys His Ser Leu Cys Tyr Gln Lys Tyr Met Val Tyr Asn
           515                 520                 525
   Glu Leu Thr Lys Val Arg Tyr Ile Asp Asp Gln Gly Lys Thr Asn Tyr
       530                 535                 540
   Phe Ser Gly Gln Glu Lys Gln Gln Ile Phe Asn Asp Leu Phe Lys Gln
   545                 550                 555                 560
   Lys Arg Lys Val Lys Lys Lys Asp Leu Glu Leu Phe Leu Arg Asn Ile
                   565                 570                 575
   Asn His Ile Glu Ser Pro Thr Ile Glu Gly Leu Glu Asp Ser Phe Asn
               580                 585                 590
   Ala Ser Tyr Ala Thr Tyr His Asp Leu Leu Lys Val Gly Met Lys Gln
           595                 600                 605
   Glu Ile Leu Asp Asn Pro Leu Asn Thr Glu Met Leu Glu Asp Ile Val
       610                 615                 620
   Lys Ile Leu Thr Val Phe Glu Asp Lys Pro Met Ile Lys Glu Gln Leu
   625                 630                 635                 640
   Gln Gln Phe Ser Asp Val Leu Asp Gly Gly Val Leu Lys Lys Leu Glu
                   645                 650                 655
   Arg Arg His Tyr Thr Gly Trp Gly Arg Leu Ser Ala Lys Leu Leu Val
               660                 665                 670
   Gly Ile Arg Glu Lys Gln Ser His Leu Thr Ile Leu Asp Tyr Leu Met
           675                 680                 685
   Asn Asp Asp Gly Leu Asn Arg Asn Leu Met Gln Leu Ile Asn Asp Ser
       690                 695                 700
   Asn Leu Ser Phe Lys Ser Ile Ile Glu Lys Glu Gln Val Ser Thr Thr
   705                 710                 715                 720
   Asp Lys Asp Leu Gln Ser Ile Val Ala Asp Leu Ala Gly Ser Pro Ala
                   725                 730                 735
   Ile Lys Lys Gly Ile Leu Gln Ser Leu Lys Ile Val Asp Glu Leu Val
               740                 745                 750
   Ser Ile Met Gly Tyr Pro Pro Gln Thr Ile Val Val Glu Met Ala Arg
           755                 760                 765
   Glu Asn Gln Thr Thr Gly Lys Gly Lys Asn Asn Ser Lys Pro Arg Tyr
       770                 775                 780
   Lys Ser Leu Glu Lys Ala Ile Lys Glu Phe Gly Ser Lys Ile Leu Lys
   785                 790                 795                 800
   Glu His Pro Thr Asp Asn Gln Glu Leu Lys Asn Asn Arg Leu Tyr Leu
                   805                 810                 815
   Tyr Tyr Leu Gln Asn Gly Lys Asp Met Tyr Thr Gly Gln Glu Leu Asp
               820                 825                 830
   Ile His Asn Leu Ser Asn Tyr Asp Ile Asp His Ile Val Pro Gln Ser
           835                 840                 845
   Phe Ile Thr Asp Asn Ser Ile Asp Asn Leu Val Leu Thr Ser Ser Ala
       850                 855                 860
   Gly Asn Arg Glu Lys Gly Gly Asp Val Pro Pro Leu Glu Ile Val Arg
   865                 870                 875                 880
   Lys Arg Lys Val Phe Trp Glu Lys Leu Tyr Gln Gly Asn Leu Met Ser
                   885                 890                 895
   Lys Arg Lys Phe Asp Tyr Leu Thr Lys Ala Glu Arg Gly Gly Leu Thr
               900                 905                 910
   Asp Ala Asp Lys Ala Arg Phe Ile His Arg Gln Leu Val Glu Thr Arg
           915                 920                 925
   Gln Ile Thr Lys Asn Val Ala Asn Ile Leu His Gln Arg Phe Asn Asn
       930                 935                 940
   Glu Thr Asp Asn His Gly Asn Thr Met Glu Gln Val Arg Ile Val Thr
   945                 950                 955                 960
   Leu Lys Ser Ala Leu Val Ser Gln Phe Arg Lys Gln Phe Gln Leu Tyr
                   965                 970                 975
   Lys Val Arg Glu Val Asn Asp Tyr His His Ala His Asp Ala Tyr Leu
               980                 985                 990
   Asn Gly Val Val Ala Asn Thr Leu Leu Lys Val Tyr Pro Gln Leu Glu
           995                 1000                1005
   Pro Glu Phe Val Tyr Gly Glu Tyr His Gln Phe Asp Trp Phe Lys Ala
       1010                1015                1020
   Asn Lys Ala Thr Ala Lys Lys Gln Phe Tyr Thr Asn Ile Met Leu Phe
   1025                1030                1035                1040
   Phe Gly Gln Lys Glu Arg Ile Ile Asp Glu Asn Gly Glu Ile Leu Trp
                   1045                1050                1055
   Asp Lys Lys Tyr Leu Glu Thr Ile Lys Lys Val Leu Asp Tyr Arg Gln
               1060                1065                1070
   Met Asn Ile Val Lys Lys Thr Glu Ile Gln Lys Gly Glu Phe Ser Lys
           1075                1080                1085
   Ala Thr Ile Lys Pro Lys Gly Asn Ser Ser Lys Leu Ile Pro Arg Lys
       1090                1095                1100
   Glu Asn Trp Asp Pro Met Lys Tyr Gly Gly Leu Asp Ser Pro Asn Met
   1105                1110                1115                1120
   Ala Tyr Ala Val Ile Ile Glu His Ala Lys Gly Lys Lys Lys Leu Ile
                   1125                1130                1135
   Phe Glu Lys Lys Ile Ile Arg Ile Thr Ile Met Glu Arg Lys Met Phe
               1140                1145                1150
   Glu Lys Asp Glu Glu Ala Phe Leu Glu Glu Lys Gly Tyr Arg His Pro
           1155                1160                1165
   Lys Val Leu Thr Lys Leu Pro Lys Tyr Thr Leu Tyr Glu Cys Glu Lys
       1170                1175                1180
   Gly Arg Arg Arg Met Leu Ala Ser Ala Asn Glu Ala Gln Lys Gly Asn
   1185                1190                1195                1200
   Gln Leu Val Leu Ser Asn His Leu Val Ser Leu Leu Tyr His Ala Lys
                   1205                1210                1215
   Asn Cys Glu Ala Ser Asp Gly Lys Ser Leu Lys Tyr Ile Glu Ala His
               1220                1225                1230
   Arg Glu Thr Phe Ser Glu Leu Leu Ala Gln Val Ser Glu Phe Ala Thr
           1235                1240                1245
   Arg Tyr Thr Leu Ala Asp Ala Asn Leu Ser Lys Ile Asn Asn Leu Phe
       1250                1255                1260
   Glu Gln Asn Lys Glu Gly Asp Ile Gln Ala Ile Ala Gln Ser Phe Val
   1265                1270                1275                1280
   Asp Leu Met Ala Phe Asn Ala Met Gly Ala Pro Ala Ser Phe Lys Phe
                   1285                1290                1295
   Phe Glu Ala Thr Ile Asp Arg Lys Arg Tyr Thr Asn Leu Lys Glu Leu
               1300                1305                1310
   Leu Ser Ser Thr Ile Ile Tyr Gln Ser Ile Thr Gly Leu Tyr Glu Ser
           1315                1320                1325
   Arg Lys Arg Leu Asp Asp
   1330
<210> 169
   <211> 1334
   <212> PRT
   <213> Listeria monocytogenes serotype 7
<220>
   <223> CRISPR-associated endonuclease
<308> YP_006694559
   <309> 2013-06-11
<400> 169
   Met Lys Asn Pro Tyr Thr Ile Gly Leu Asp Ile Gly Thr Asn Ser Val
    1               5                  10                  15
   Gly Trp Ala Val Leu Thr Asp Gln Tyr Asp Leu Val Lys Arg Lys Met
               20                  25                  30
   Lys Val Ala Gly Asn Ser Asp Lys Lys Gln Ile Lys Lys Asn Phe Trp
           35                  40                  45
   Gly Val Arg Leu Phe Asp Asp Gly Gln Thr Ala Val Asp Arg Arg Met
       50                  55                  60
   Asn Arg Thr Ala Arg Arg Arg Ile Glu Arg Arg Arg Asn Arg Ile Ser
   65                  70                  75                  80
   Tyr Leu Gln Glu Ile Phe Ala Val Glu Met Ala Asn Ile Asp Ala Asn
                   85                  90                  95
   Phe Phe Cys Arg Leu Asn Asp Ser Phe Tyr Val Asp Ser Glu Lys Arg
               100                 105                 110
   Asn Ser Arg His Pro Phe Phe Ala Thr Ile Glu Glu Glu Val Ala Tyr
           115                 120                 125
   His Lys Asn Tyr Arg Thr Ile Tyr His Leu Arg Glu Glu Leu Val Asn
       130                 135                 140
   Ser Ser Glu Lys Ala Asp Leu Arg Leu Val Tyr Leu Ala Leu Ala His
   145                 150                 155                 160
   Ile Ile Lys Tyr Arg Gly Asn Phe Leu Ile Glu Gly Ala Leu Asp Thr
                   165                 170                 175
   Lys Asn Thr Ser Val Asp Glu Val Tyr Lys Gln Phe Ile Gln Thr Tyr
               180                 185                 190
   Asn Gln Val Phe Met Ser Asn Ile Glu Glu Gly Ala Leu Ala Lys Val
           195                 200                 205
   Glu Glu Asn Thr Glu Val Ala Ser Ile Leu Ala Gly Lys Phe Thr Arg
       210                 215                 220
   Arg Glu Lys Phe Glu Arg Ile Leu Arg Leu Tyr Pro Gly Glu Lys Ser
   225                 230                 235                 240
   Thr Gly Met Phe Ala Gln Phe Ile Ser Leu Ile Val Gly Asn Lys Gly
                   245                 250                 255
   Asn Phe Gln Lys Val Phe Asn Leu Val Glu Lys Thr Asp Ile Glu Cys
               260                 265                 270
   Ala Lys Asp Ser Tyr Glu Glu Asp Leu Glu Ala Leu Leu Ala Ile Ile
           275                 280                 285
   Gly Asp Glu Tyr Ala Glu Leu Phe Val Ala Ala Lys Asn Thr Tyr Asn
       290                 295                 300
   Ala Val Val Leu Ser Ser Ile Ile Thr Val Thr Ala Thr Glu Thr Asn
   305                 310                 315                 320
   Ala Lys Leu Ser Ala Ser Met Ile Glu Arg Phe Asp Ala His Glu Lys
                   325                 330                 335
   Glu Leu Gly Glu Leu Lys Ala Phe Ile Lys Leu His Leu Pro Lys Gln
               340                 345                 350
   Tyr Gln Glu Ile Phe Asn Asn Ala Glu Ile Asp Gly Tyr Ala Gly Tyr
           355                 360                 365
   Ile Asp Gly Lys Thr Lys Gln Val Asp Phe Tyr Lys Tyr Leu Lys Thr
   370 375 380
   Thr Leu Glu Asn Val Glu Gly Ala Asp Tyr Phe Ile Thr Lys Ile Glu
   385 390 395 400
   Glu Glu Asn Phe Leu Arg Lys Gln Arg Thr Phe Asp Asn Gly Val Ile
   405 410 415
   Pro His Gln Leu His Leu Glu Glu Leu Glu Ala Ile Leu His Gln Gln
   420 425 430
   Ala Lys Tyr Tyr Pro Phe Leu Arg Glu Gly Tyr Asp Lys Ile Lys Ser
   435 440 445
   Leu Val Thr Phe Arg Ile Pro Tyr Phe Val Gly Pro Leu Ala Asn Gly
   450 455 460
   Gln Ser Glu Phe Ala Trp Leu Thr Arg Lys Asp Asp Gly Glu Ile Arg
   465 470 475 480
   Pro Trp Asn Ile Glu Glu Lys Val Asp Phe Gly Lys Ser Ala Val Asp
   485 490 495
   Phe Ile Glu Lys Met Thr Asn Lys Asp Thr Tyr Leu Pro Lys Glu Asn
   500 505 510
   Val Leu Pro Lys His Ser Leu Cys Tyr Gln Lys Tyr Met Val Tyr Asn
   515 520 525
   Glu Leu Thr Lys Ile Arg Tyr Ile Asp Asp Gln Gly Lys Thr Asn Tyr
   530 535 540
   Phe Ser Gly Arg Glu Lys Gln Gln Ile Phe Asn Asp Leu Phe Lys Gln
   545 550 555 560
   Lys Arg Lys Val Lys Lys Lys Asp Leu Glu Leu Phe Leu Arg Asn Ile
   565 570 575
   Asn His Ile Glu Ser Pro Thr Ile Glu Gly Leu Glu Asp Ser Phe Asn
   580 585 590
   Ala Ser Tyr Ala Thr Tyr His Asp Leu Leu Lys Val Gly Leu Lys Gln
   595 600 605
   Glu Ile Leu Asp Asn Pro Leu Asn Thr Glu Ile Leu Glu Asp Ile Val
   610 615 620
   Lys Ile Leu Thr Val Phe Glu Asp Lys Arg Met Ile Lys Glu Gln Leu
   625 630 635 640
   Glu Gln Phe Ser Asp Val Leu Asp Gly Val Val Leu Lys Lys Leu Glu
   645 650 655
   Arg Arg His Tyr Thr Gly Trp Gly Arg Leu Ser Ala Lys Leu Leu Val
   660 665 670
   Gly Ile Arg Asp Lys Gln Ser His Leu Thr Ile Leu Asp Tyr Leu Met
   675 680 685
   Asn Asp Asp Gly Leu Asn Arg Asn Leu Met Gln Leu Ile Asn Asp Ser
   690 695 700
   Asn Leu Ser Phe Lys Ser Ile Ile Glu Lys Glu Gln Val Ser Thr Thr
   705 710 715 720
   Asp Lys Asp Leu Gln Ser Ile Val Ala Asp Leu Ala Gly Ser Pro Ala
   725 730 735
   Ile Lys Lys Gly Ile Leu Gln Ser Leu Lys Ile Val Asp Glu Leu Val
   740 745 750
   Ser Ile Met Gly Tyr Pro Pro Gln Thr Ile Val Val Glu Met Ala Arg
   755 760 765
   Glu Asn Gln Thr Thr Gly Lys Gly Lys Asn Asn Ser Lys Pro Arg Tyr
   770 775 780
   Lys Ser Leu Glu Lys Ala Ile Lys Asp Phe Gly Ser Gln Ile Leu Lys
   785 790 795 800
   Glu His Pro Thr Asp Asn Gln Glu Leu Lys Asn Asn Arg Leu Tyr Leu
   805 810 815
   Tyr Tyr Leu Gln Asn Gly Lys Asp Ile Tyr Thr Gly Gln Glu Leu Asp
   820 825 830
   Ile His Asn Leu Ser Asn Tyr Asp Ile Asp His Ile Val Pro Gln Ser
   835 840 845
   Phe Ile Thr Asp Asn Ser Ile Asp Asn Leu Val Leu Thr Ser Ser Ala
   850 855 860
   Gly Asn Arg Glu Lys Gly Gly Asp Val Pro Pro Leu Glu Ile Val Arg
   865 870 875 880
   Lys Arg Lys Val Phe Trp Glu Lys Leu Tyr Gln Gly Asn Leu Met Ser
   885 890 895
   Lys Arg Lys Phe Asp Tyr Leu Thr Lys Ala Glu Arg Gly Gly Leu Thr
   900 905 910
   Glu Ala Asp Lys Ala Arg Phe Ile His Arg Gln Leu Val Glu Thr Arg
   915 920 925
   Gln Ile Thr Lys Asn Val Ala Asn Ile Leu His Gln Arg Phe Asn Lys
   930 935 940
   Glu Thr Asp Asn His Gly Asn Thr Met Glu Gln Val Arg Ile Val Thr
   945 950 955 960
   Leu Lys Ser Ala Leu Val Ser Gln Phe Arg Lys Gln Phe Gln Leu Tyr
   965 970 975
   Lys Val Arg Glu Val Asn Asp Tyr His His Ala His Asp Ala Tyr Leu
   980 985 990
   Asn Gly Val Val Ala Asn Thr Leu Leu Lys Val Tyr Pro Gln Leu Glu
   995 1000 1005
   Pro Glu Phe Val Tyr Gly Glu Tyr His Gln Phe Asp Trp Phe Lys Ala
   1010 1015 1020
   Asn Lys Ala Thr Ala Lys Lys Gln Phe Tyr Thr Asn Ile Met Leu Phe
   1025 1030 1035 1040
   Phe Ala Gln Lys Glu Arg Ile Ile Asp Glu Asn Gly Glu Ile Leu Trp
   1045 1050 1055
   Asp Lys Lys Tyr Leu Glu Thr Ile Lys Lys Val Leu Asp Tyr Arg Gln
   1060 1065 1070
   Ile Asn Ile Val Lys Lys Thr Glu Ile Gln Lys Gly Glu Phe Ser Lys
   1075 1080 1085
   Ala Thr Ile Lys Pro Lys Gly Asn Ser Ser Lys Leu Ile Pro Arg Lys
   1090 1095 1100
   Glu Asn Trp Asp Pro Val Lys Tyr Gly Gly Leu Asp Ser Pro Asn Met
   1105 1110 1115 1120
   Ala Tyr Ala Val Ile Ile Glu His Ala Lys Gly Lys Lys Lys Ile Val
   1125 1130 1135
   Ile Glu Lys Lys Leu Ile Gln Ile Asn Ile Met Glu Arg Lys Met Phe
   1140 1145 1150
   Glu Lys Asp Glu Glu Ala Phe Leu Glu Glu Lys Gly Tyr His Gln Pro
   1155 1160 1165
   Lys Val Leu Thr Lys Leu Pro Lys Tyr Thr Leu Tyr Glu Cys Glu Lys
   1170 1175 1180
   Gly Arg Arg Arg Met Leu Ser Ser Ala Asn Glu Ala Gln Lys Gly Asn
   1185 1190 1195 1200
   Gln Leu Val Leu Ser Asn His Leu Val Ser Leu Leu Tyr His Ala Lys
   1205 1210 1215
   Asn Cys Glu Ala Ser Asp Gly Lys Ser Leu Lys Tyr Ile Glu Ala His
   1220 1225 1230
   Arg Glu Thr Phe Ser Glu Leu Leu Ala Gln Val Ser Glu Phe Ala Thr
   1235 1240 1245
   Lys Tyr Thr Leu Ala Asp Ala Asn Leu Ser Lys Ile Asn Asn Leu Phe
   1250 1255 1260
   Glu Gln Asn Lys Glu Gly Asp Ile Lys Ala Ile Ala Gln Ser Phe Val
   1265 1270 1275 1280
   Asp Leu Met Ala Phe Asn Ala Met Gly Ala Pro Ala Ser Phe Lys Phe
   1285 1290 1295
   Phe Glu Ala Thr Ile Asp Arg Lys Arg Tyr Thr Asn Leu Lys Glu Leu
   1300 1305 1310
   Leu Ser Ser Thr Ile Ile Tyr Gln Ser Ile Thr Gly Leu Tyr Glu Ser
   1315 1320 1325
   Arg Lys Arg Leu Asp Asp
   1330
<210> 170
   <211> 1334
   <212> PRT
   <213> Listeria monocytogenes
<220>
   <223> CRISPR-associated endonuclease
<308> YP_006683057
   <309> 2013-06-11
<400> 170
   Met Lys Asn Pro Tyr Thr Ile Gly Leu Asp Ile Gly Thr Asn Ser Val
   1 5 10 15
   Gly Trp Ala Val Leu Thr Asp Gln Tyr Asp Leu Val Lys Arg Lys Met
   20 25 30
   Lys Val Ala Gly Asn Ser Asp Lys Lys Gln Ile Lys Lys Asn Phe Trp
   35 40 45
   Gly Val Arg Leu Phe Asp Asp Gly Gln Thr Ala Val Asp Arg Arg Met
   50 55 60
   Asn Arg Thr Ala Arg Arg Arg Ile Glu Arg Arg Arg Asn Arg Ile Ser
   65 70 75 80
   Tyr Leu Gln Glu Ile Phe Ala Val Glu Met Ala Asn Ile Asp Ala Asn
   85 90 95
   Phe Phe Cys Arg Leu Asn Asp Ser Phe Tyr Val Asp Ser Glu Lys Arg
   100 105 110
   Asn Ser Arg His Pro Phe Phe Ala Thr Ile Glu Glu Glu Val Ala Tyr
   115 120 125
   His Lys Asn Tyr Arg Thr Ile Tyr His Leu Arg Glu Glu Leu Val Asn
   130 135 140
   Ser Ser Glu Lys Ala Asp Leu Arg Leu Val Tyr Leu Ala Leu Ala His
   145 150 155 160
   Ile Ile Lys Tyr Arg Gly Asn Phe Leu Ile Glu Gly Ala Leu Asp Thr
   165 170 175
   Lys Asn Thr Ser Val Asp Glu Val Tyr Lys Gln Phe Ile Gln Thr Tyr
   180 185 190
   Asn Gln Val Phe Met Ser Asn Ile Glu Glu Gly Ala Leu Ala Lys Val
   195 200 205
   Glu Glu Asn Thr Glu Val Ala Ser Ile Leu Ala Gly Lys Phe Thr Arg
   210 215 220
   Arg Glu Lys Phe Glu Arg Ile Leu Arg Leu Tyr Pro Gly Glu Lys Ser
   225 230 235 240
   Thr Gly Met Phe Ala Gln Phe Ile Ser Leu Ile Val Gly Asn Lys Gly
   245 250 255
   Asn Phe Gln Lys Val Phe Asn Leu Val Glu Lys Thr Asp Ile Glu Cys
   260 265 270
   Ala Lys Asp Ser Tyr Glu Glu Asp Leu Glu Ala Leu Leu Ala Ile Ile
   275 280 285
   Gly Asp Glu Tyr Ala Glu Leu Phe Val Ala Ala Lys Asn Thr Tyr Asn
   290 295 300
   Ala Val Val Leu Ser Ser Ile Ile Thr Val Thr Ala Thr Glu Thr Asn
   305 310 315 320
   Ala Lys Leu Ser Ala Ser Met Ile Glu Arg Phe Asp Ala His Glu Lys
   325 330 335
   Glu Leu Gly Glu Leu Lys Ala Phe Ile Lys Leu His Leu Pro Lys Gln
   340 345 350
   Tyr Gln Glu Ile Phe Asn Asn Ala Glu Ile Asp Gly Tyr Ala Gly Tyr
   355 360 365
   Ile Asp Gly Lys Thr Lys Gln Val Asp Phe Tyr Lys Tyr Leu Lys Thr
   370 375 380
   Thr Leu Glu Asn Val Glu Gly Ala Asp Tyr Phe Ile Thr Lys Ile Glu
   385 390 395 400
   Glu Glu Asn Phe Leu Arg Lys Gln Arg Thr Phe Asp Asn Gly Val Ile
   405 410 415
   Pro His Gln Leu His Leu Glu Glu Leu Glu Ala Ile Leu His Gln Gln
   420 425 430
   Ala Lys Tyr Tyr Pro Phe Leu Arg Glu Gly Tyr Asp Lys Ile Lys Ser
   435 440 445
   Leu Val Thr Phe Arg Ile Pro Tyr Phe Val Gly Pro Leu Ala Asn Gly
   450 455 460
   Gln Ser Glu Phe Ala Trp Leu Thr Arg Lys Asp Asp Gly Glu Ile Arg
   465 470 475 480
   Pro Trp Asn Ile Glu Glu Lys Val Asp Phe Gly Lys Ser Ala Val Asp
   485 490 495
   Phe Ile Glu Lys Met Thr Asn Lys Asp Thr Tyr Leu Pro Lys Glu Asn
   500 505 510
   Val Leu Pro Lys His Ser Leu Cys Tyr Gln Lys Tyr Met Val Tyr Asn
   515 520 525
   Glu Leu Thr Lys Ile Arg Tyr Ile Asp Asp Gln Gly Lys Thr Asn Tyr
   530 535 540
   Phe Ser Gly Arg Glu Lys Gln Gln Ile Phe Asn Asp Leu Phe Lys Gln
   545 550 555 560
   Lys Arg Lys Val Lys Lys Lys Asp Leu Glu Leu Phe Leu Arg Asn Ile
   565 570 575
   Asn His Ile Glu Ser Pro Thr Ile Glu Gly Leu Glu Asp Ser Phe Asn
   580 585 590
   Ala Ser Tyr Ala Thr Tyr His Asp Leu Leu Lys Val Gly Leu Lys Gln
   595 600 605
   Glu Ile Leu Asp Asn Pro Leu Asn Thr Glu Ile Leu Glu Asp Ile Val
   610 615 620
   Lys Ile Leu Thr Val Phe Glu Asp Lys Arg Met Ile Lys Glu Gln Leu
   625 630 635 640
   Glu Gln Phe Ser Asp Val Leu Asp Gly Val Val Leu Lys Lys Leu Glu
   645 650 655
   Arg Arg His Tyr Thr Gly Trp Gly Arg Leu Ser Ala Lys Leu Leu Val
   660 665 670
   Gly Ile Arg Asp Lys Gln Ser His Leu Thr Ile Leu Asp Tyr Leu Met
   675 680 685
   Asn Asp Asp Gly Leu Asn Arg Asn Leu Met Gln Leu Ile Asn Asp Ser
   690 695 700
   Asn Leu Ser Phe Lys Ser Ile Ile Glu Lys Glu Gln Val Ser Thr Thr
   705 710 715 720
   Asp Lys Asp Leu Gln Ser Ile Val Ala Asp Leu Ala Gly Ser Pro Ala
   725 730 735
   Ile Lys Lys Gly Ile Leu Gln Ser Leu Lys Ile Val Asp Glu Leu Val
   740 745 750
   Ser Ile Met Gly Tyr Pro Pro Gln Thr Ile Val Val Glu Met Ala Arg
   755 760 765
   Glu Asn Gln Thr Thr Gly Lys Gly Lys Asn Asn Ser Lys Pro Arg Tyr
   770 775 780
   Lys Ser Leu Glu Lys Ala Ile Lys Asp Phe Gly Ser Gln Ile Leu Lys
   785 790 795 800
   Glu His Pro Thr Asp Asn Gln Glu Leu Lys Asn Asn Arg Leu Tyr Leu
   805 810 815
   Tyr Tyr Leu Gln Asn Gly Lys Asp Ile Tyr Thr Gly Gln Glu Leu Asp
   820 825 830
   Ile His Asn Leu Ser Asn Tyr Asp Ile Asp His Ile Val Pro Gln Ser
   835 840 845
   Phe Ile Thr Asp Asn Ser Ile Asp Asn Leu Val Leu Thr Ser Ser Ala
   850 855 860
   Gly Asn Arg Glu Lys Gly Gly Asp Val Pro Pro Leu Glu Ile Val Arg
   865 870 875 880
   Lys Arg Lys Val Phe Trp Glu Lys Leu Tyr Gln Gly Asn Leu Met Ser
   885 890 895
   Lys Arg Lys Phe Asp Tyr Leu Thr Lys Ala Glu Arg Gly Gly Leu Thr
   900 905 910
   Glu Ala Asp Lys Ala Arg Phe Ile His Arg Gln Leu Val Glu Thr Arg
   915 920 925
   Gln Ile Thr Lys Asn Val Ala Asn Ile Leu His Gln Arg Phe Asn Lys
       930                 935                 940
   Glu Thr Asp Asn His Gly Asn Thr Met Glu Gln Val Arg Ile Val Thr
   945                 950                 955                 960
   Leu Lys Ser Ala Leu Val Ser Gln Phe Arg Lys Gln Phe Gln Leu Tyr
                   965                 970                 975
   Lys Val Arg Glu Val Asn Asp Tyr His His Ala His Asp Ala Tyr Leu
               980                 985                 990
   Asn Gly Val Val Ala Asn Thr Leu Leu Lys Val Tyr Pro Gln Leu Glu
           995                 1000                1005
   Pro Glu Phe Val Tyr Gly Glu Tyr His Gln Phe Asp Trp Phe Lys Ala
       1010                1015                1020
   Asn Lys Ala Thr Ala Lys Lys Gln Phe Tyr Thr Asn Ile Met Leu Phe
   1025                1030                1035                1040
   Phe Ala Gln Lys Glu Arg Ile Ile Asp Glu Asn Gly Glu Ile Leu Trp
                   1045                1050                1055
   Asp Lys Lys Tyr Leu Glu Thr Ile Lys Lys Val Leu Asp Tyr Arg Gln
               1060                1065                1070
   Ile Asn Ile Val Lys Lys Thr Glu Ile Gln Lys Gly Glu Phe Ser Lys
           1075                1080                1085
   Ala Thr Ile Lys Pro Lys Gly Asn Ser Ser Lys Leu Ile Pro Arg Lys
       1090                1095                1100
   Glu Asn Trp Asp Pro Val Lys Tyr Gly Gly Leu Asp Ser Pro Asn Met
   1105                1110                1115                1120
   Ala Tyr Ala Val Ile Ile Glu His Ala Lys Gly Lys Lys Lys Ile Val
                   1125                1130                1135
   Ile Glu Lys Lys Leu Ile Gln Ile Asn Ile Met Glu Arg Lys Met Phe
               1140                1145                1150
   Glu Lys Asp Glu Glu Ala Phe Leu Glu Glu Lys Gly Tyr His Gln Pro
           1155                1160                1165
   Lys Val Leu Thr Lys Leu Pro Lys Tyr Thr Leu Tyr Glu Cys Glu Lys
       1170                1175                1180
   Gly Arg Arg Arg Met Leu Ser Ser Ala Asn Glu Ala Gln Lys Gly Asn
   1185                1190                1195                1200
   Gln Leu Val Leu Ser Asn His Leu Val Ser Leu Leu Tyr His Ala Lys
                   1205                1210                1215
   Asn Cys Glu Ala Ser Asp Gly Lys Ser Leu Lys Tyr Ile Glu Ala His
               1220                1225                1230
   Arg Glu Thr Phe Ser Glu Leu Leu Ala Gln Val Ser Glu Phe Ala Thr
           1235                1240                1245
   Lys Tyr Thr Leu Ala Asp Ala Asn Leu Ser Lys Ile Asn Asn Leu Phe
       1250                1255                1260
   Glu Gln Asn Lys Glu Gly Asp Ile Lys Ala Ile Ala Gln Ser Phe Val
   1265                1270                1275                1280
   Asp Leu Met Ala Phe Asn Ala Met Gly Ala Pro Ala Ser Phe Lys Phe
                   1285                1290                1295
   Phe Glu Ala Thr Ile Asp Arg Lys Arg Tyr Thr Asn Leu Lys Glu Leu
               1300                1305                1310
   Leu Ser Ser Thr Ile Ile Tyr Gln Ser Ile Thr Gly Leu Tyr Glu Ser
           1315                1320                1325
   Arg Lys Arg Leu Asp Asp
       1330
<210> 171
   <211> 984
   <212> PRT
   <213> Campylobacter jejuni
<220>
   <223> CRISPR-associated endonuclease
<308> YP_006633648
   <309> 2013-08-28
<400> 171
   Met Ala Arg Ile Leu Ala Phe Asp Ile Gly Ile Ser Ser Ile Gly Trp
   1 5 10 15
   Ala Phe Ser Glu Asn Asp Glu Leu Lys Asp Cys Gly Val Arg Ile Phe
   20 25 30
   Thr Lys Val Glu Asn Pro Lys Thr Gly Glu Ser Leu Ala Leu Pro Arg
   35 40 45
   Arg Leu Ala Arg Ser Ala Arg Lys Arg Leu Ala Arg Arg Lys Ala Arg
   50 55 60
   Leu Asn His Leu Lys His Leu Ile Ala Asn Glu Phe Lys Leu Asn Tyr
   65 70 75 80
   Glu Asp Tyr Gln Ser Phe Asp Glu Ser Leu Ala Lys Ala Tyr Lys Gly
   85 90 95
   Ser Leu Ile Ser Pro Tyr Glu Leu Arg Phe Arg Ala Leu Asn Glu Leu
   100 105 110
   Leu Ser Lys Gln Asp Phe Ala Arg Val Ile Leu His Ile Ala Lys Arg
   115 120 125
   Arg Gly Tyr Asp Asp Ile Lys Asn Ser Asp Asp Lys Glu Lys Gly Ala
   130 135 140
   Ile Leu Lys Ala Ile Lys Gln Asn Glu Glu Lys Leu Ala Asn Tyr Gln
   145 150 155 160
   Ser Val Gly Glu Tyr Leu Tyr Lys Glu Tyr Phe Gln Lys Phe Lys Glu
   165 170 175
   Asn Ser Lys Glu Phe Thr Asn Val Arg Asn Lys Lys Glu Ser Tyr Glu
   180 185 190
   Arg Cys Ile Ala Gln Ser Phe Leu Lys Asp Glu Leu Lys Leu Ile Phe
   195 200 205
   Lys Lys Gln Arg Glu Phe Gly Phe Ser Phe Ser Lys Lys Phe Glu Glu
   210 215 220
   Glu Val Leu Ser Val Ala Phe Tyr Lys Arg Ala Leu Lys Asp Phe Ser
   225 230 235 240
   His Leu Val Gly Asn Cys Ser Phe Phe Thr Asp Glu Lys Arg Ala Pro
   245 250 255
   Lys Asn Ser Pro Leu Ala Phe Met Phe Val Ala Leu Thr Arg Ile Ile
   260 265 270
   Asn Leu Leu Asn Asn Leu Lys Asn Thr Glu Gly Ile Leu Tyr Thr Lys
   275 280 285
   Asp Asp Leu Asn Ala Leu Leu Asn Glu Val Leu Lys Asn Gly Thr Leu
   290 295 300
   Thr Tyr Lys Gln Thr Lys Lys Leu Leu Gly Leu Ser Asp Asp Tyr Glu
   305 310 315 320
   Phe Lys Gly Glu Lys Gly Thr Tyr Phe Ile Glu Phe Lys Lys Tyr Lys
   325 330 335
   Glu Phe Ile Lys Ala Leu Gly Glu His Asn Leu Ser Gln Asp Asp Leu
   340 345 350
   Asn Glu Ile Ala Lys Asp Ile Thr Leu Ile Lys Asp Glu Ile Lys Leu
   355 360 365
   Lys Lys Ala Leu Ala Lys Tyr Asp Leu Asn Gln Asn Gln Ile Asp Ser
   370 375 380
   Leu Ser Lys Leu Glu Phe Lys Asp His Leu Asn Ile Ser Phe Lys Ala
   385 390 395 400
   Leu Lys Leu Val Thr Pro Leu Met Leu Glu Gly Lys Lys Tyr Asp Glu
   405 410 415
   Ala Cys Asn Glu Leu Asn Leu Lys Val Ala Ile Asn Glu Asp Lys Lys
   420 425 430
   Asp Phe Leu Pro Ala Phe Asn Glu Thr Tyr Tyr Lys Asp Glu Val Thr
   435 440 445
   Asn Pro Val Val Leu Arg Ala Ile Lys Glu Tyr Arg Lys Val Leu Asn
   450 455 460
   Ala Leu Leu Lys Lys Tyr Gly Lys Val His Lys Ile Asn Ile Glu Leu
   465 470 475 480
   Ala Arg Glu Val Gly Lys Asn His Ser Gln Arg Ala Lys Ile Glu Lys
                   485                 490                 495
   Glu Gln Asn Glu Asn Tyr Lys Ala Lys Lys Asp Ala Glu Leu Glu Cys
               500                 505                 510
   Glu Lys Leu Gly Leu Lys Ile Asn Ser Lys Asn Ile Leu Lys Leu Arg
           515                 520                 525
   Leu Phe Lys Glu Gln Lys Glu Phe Cys Ala Tyr Ser Gly Glu Lys Ile
       530                 535                 540
   Lys Ile Ser Asp Leu Gln Asp Glu Lys Met Leu Glu Ile Asp His Ile
   545                 550                 555                 560
   Tyr Pro Tyr Ser Arg Ser Phe Asp Asp Ser Tyr Met Asn Lys Val Leu
                   565                 570                 575
   Val Phe Thr Lys Gln Asn Gln Glu Lys Leu Asn Gln Thr Pro Phe Glu
               580                 585                 590
   Ala Phe Gly Asn Asp Ser Ala Lys Trp Gln Lys Ile Glu Val Leu Ala
           595                 600                 605
   Lys Asn Leu Pro Thr Lys Lys Gln Lys Arg Ile Leu Asp Lys Asn Tyr
       610                 615                 620
   Lys Asp Lys Glu Gln Lys Asn Phe Lys Asp Arg Asn Leu Asn Asp Thr
   625                 630                 635                 640
   Arg Tyr Ile Ala Arg Leu Val Leu Asn Tyr Thr Lys Asp Tyr Leu Asp
                   645                 650                 655
   Phe Leu Pro Leu Ser Asp Asp Glu Asn Thr Lys Leu Asn Asp Thr Gln
               660                 665                 670
   Lys Gly Ser Lys Val His Val Glu Ala Lys Ser Gly Met Leu Thr Ser
           675                 680                 685
   Ala Leu Arg His Thr Trp Gly Phe Ser Ala Lys Asp Arg Asn Asn His
       690                 695                 700
   Leu His His Ala Ile Asp Ala Val Ile Ile Ala Tyr Ala Asn Asn Ser
   705                 710                 715                 720
   Ile Val Lys Ala Phe Ser Asp Phe Lys Lys Glu Gln Glu Ser Asn Ser
                   725                 730                 735
   Ala Glu Leu Tyr Ala Lys Lys Ile Ser Glu Leu Asp Tyr Lys Asn Lys
               740                 745                 750
   Arg Lys Phe Phe Glu Pro Phe Ser Gly Phe Arg Gln Lys Val Leu Asp
           755                 760                 765
   Lys Ile Asp Glu Ile Phe Val Ser Lys Pro Glu Arg Lys Lys Pro Ser
       770                 775                 780
   Gly Ala Leu His Glu Glu Thr Phe Arg Lys Glu Glu Glu Phe Tyr Gln
   785                 790                 795                 800
   Ser Tyr Gly Gly Lys Glu Gly Val Leu Lys Ala Leu Glu Leu Gly Lys
                   805                 810                 815
   Ile Arg Lys Val Asn Gly Lys Ile Val Lys Asn Gly Asp Met Phe Arg
               820                 825                 830
   Val Asp Ile Phe Lys His Lys Lys Thr Asn Lys Phe Tyr Ala Val Pro
           835                 840                 845
   Ile Tyr Thr Met Asp Phe Ala Leu Lys Val Leu Pro Asn Lys Ala Val
       850                 855                 860
   Ala Arg Ser Lys Lys Gly Glu Ile Lys Asp Trp Ile Leu Met Asp Glu
   865                 870                 875                 880
   Asn Tyr Glu Phe Cys Phe Ser Leu Tyr Lys Asp Ser Leu Ile Leu Ile
                   885                 890                 895
   Gln Thr Lys Asp Met Gln Glu Pro Glu Phe Val Tyr Tyr Asn Ala Phe
               900                 905                 910
   Thr Ser Ser Thr Val Ser Leu Ile Val Ser Lys His Asp Asn Lys Phe
           915                 920                 925
   Glu Thr Leu Ser Lys Asn Gln Lys Ile Leu Phe Lys Asn Ala Asn Glu
       930                 935                 940
   Lys Glu Val Ile Ala Lys Ser Ile Gly Ile Gln Asn Leu Lys Val Phe
   945                 950                 955                 960
   Glu Lys Tyr Ile Val Ser Ala Leu Gly Glu Val Thr Lys Ala Glu Phe
                   965                 970                 975
   Arg Gln Arg Glu Asp Phe Lys Lys
               980
<210> 172
   <211> 1352
   <212> PRT
   <213> Belliella baltica
<220>
   <223> CRISPR-associated endonuclease
<308> YP_006408468
   <309> 2013-06-11
<400> 172
   Met Lys Lys Ile Leu Gly Leu Asp Leu Gly Thr Thr Ser Ile Gly Trp
   1               5                   10                  15
   Ala Phe Ile Lys Glu Pro Glu Lys Asp Val Val Gly Ser Glu Ile Val
               20                  25                  30
   Asp Met Gly Val Arg Ile Val Pro Leu Ser Ser Asp Glu Glu Asn Asp
           35                  40                  45
   Phe Ala Lys Gly Asn Thr Ile Ser Ile Asn Ala Asp Arg Thr Leu Lys
       50                  55                  60
   Arg Gly Ala Arg Arg Asn Leu Gln Arg Phe Lys Gln Arg Arg Asn Ala
   65                  70                  75                  80
   Leu Leu Glu Ile Phe Lys Glu Lys Lys Leu Ile Ser Thr Asn Phe Lys
                   85                  90                  95
   Tyr Ala Glu Asp Gly Pro Ser Ser Thr Phe Ser Thr Leu Asn Leu Arg
               100                 105                 110
   Ala Lys Ala Ala Lys Glu Lys Ile Glu Leu Gln Asp Leu Val Lys Val
           115                 120                 125
   Leu Leu Gln Ile Asn Lys Lys Arg Gly Tyr Lys Ser Ser Arg Lys Ala
       130                 135                 140
   Lys Ser Glu Glu Asp Asp Gly Ser Ala Ile Asp Ser Met Gly Ile Ala
   145                 150                 155                 160
   Lys Glu Leu Tyr Glu Asn Asp Leu Thr Pro Gly Gln Trp Val Tyr Glu
                   165                 170                 175
   Ala Leu Gln Lys Gly Arg Lys Asn Val Pro Asp Phe Tyr Arg Ser Asp
               180                 185                 190
   Leu Gln Glu Glu Phe Lys Lys Ile Val Asn Tyr Gln Ser Glu Phe Phe
           195                 200                 205
   Pro Asp Ile Phe Asn Ala Ser Phe Val Glu Asp Trp Met Gly Lys Ala
       210                 215                 220
   Ser Thr Pro Thr Lys Gln Tyr Phe Asn Lys Lys Gly Val Gln Leu Ala
   225                 230                 235                 240
   Glu Asn Lys Gly Lys Arg Glu Glu Arg Arg Leu Gln Glu Tyr Lys Trp
                   245                 250                 255
   Arg Ala Glu Ala Val Asn Phe Lys Ile Asp Leu Ser Glu Ile Ala Leu
               260                 265                 270
   Ile Leu Ser Gln Ile Asn Ser Gln Ile Ser Asn Ser Ser Gly Tyr Leu
           275                 280                 285
   Gly Ala Ile Ser Asp Arg Ser Lys Glu Leu Tyr Phe Lys Asn Leu Thr
       290                 295                 300
   Val Gly Gln Tyr Leu Tyr Gln Gln Ile Lys Lys Asn Pro His Thr Arg
   305                 310                 315                 320
   Leu Lys Gly Gln Val Phe Tyr Arg Gln Asp Tyr Leu Asp Glu Phe Glu
                   325                 330                 335
   Arg Ile Trp Ser Val Gln Ser Ser Phe Tyr Pro Gln Leu Asn Asp Ala
               340                 345                 350
   Leu Lys Arg Glu Val Arg Asp Ile Thr Ile Phe Phe Gln Arg Arg Leu
           355                 360                 365
   Lys Ser Gln Lys His Leu Ile Ser Asn Cys Glu Phe Glu Asp His His
       370                 375                 380
   Lys Val Val Pro Lys Ser His Pro Val Phe Gln Glu Phe Arg Ile Trp
   385 390 395 400
   Gln Asn Leu Asn Asn Leu Leu Leu Ile Lys Lys Asp Asn Leu Asn Glu
   405 410 415
   Lys Phe Asp Leu Glu Leu Glu Ser Lys Ile Ala Leu Ala Asn Glu Leu
   420 425 430
   Ala Phe Lys Arg Glu Leu Asn Val Lys Asp Ala Leu Lys Ile Leu Gly
   435 440 445
   Leu Lys Pro Asn Glu Trp Glu Phe Asn Phe Thr Lys Ile Glu Gly Asn
   450 455 460
   Arg Thr Asn Gln Ala Phe Phe Asp Ala Phe Ala Lys Ile Ile Glu Leu
   465 470 475 480
   Glu Asp Gly Glu Pro Ile Asp Leu Gly Asp Leu Lys Ala Asp Asp Ile
   485 490 495
   Leu Asp Gln Phe Ser Glu Ala Phe Leu Arg Ile Gly Ile Asp Thr Glu
   500 505 510
   Leu Leu Gln Val Asn Ser Asp Ile Glu Gly Ala Glu Tyr Glu Lys Gln
   515 520 525
   Ser Tyr Ile Gln Phe Trp His Leu Leu Tyr Ser Ser Glu Asp Asp Gln
   530 535 540
   Lys Leu Lys Leu Asn Leu Ile Arg Lys Phe Gly Phe Lys Pro Glu His
   545 550 555 560
   Ala Lys Ile Leu Ala Ser Ile Ser Leu Gln Asp Asp His Ala Ser Leu
   565 570 575
   Ser Ser Arg Ala Ile Lys Lys Ile Leu Pro His Leu Gln Ser Gly Leu
   580 585 590
   Ile Tyr Asp Lys Ala Cys Thr Tyr Ala Gly Tyr Asn His Ser Ser Ser
   595 600 605
   Phe Thr Lys Asp Glu Asn Glu Lys Arg Glu Leu Arg Ala Glu Leu Glu
   610 615 620
   Leu Leu Lys Lys Asn Ser Leu Arg Asn Pro Val Val Glu Lys Ile Leu
   625 630 635 640
   Asn Gln Met Ile Asn Val Val Asn Ala Ile Leu Lys Asp Pro Glu Leu
   645 650 655
   Gly Arg Pro Asp Glu Ile Arg Val Glu Met Ala Arg Glu Leu Lys Ala
   660 665 670
   Asn Ala Glu Gln Arg Lys Asn Met Thr Ser Asn Ile Ala Ser Ala Thr
   675 680 685
   Arg Asp His Asp Lys Tyr Arg Glu Ile Leu Lys Ser Glu Phe Gly Leu
   690 695 700
   Lys Arg Val Thr Lys Asn Asp Leu Leu Arg Tyr Lys Leu Trp Leu Glu
   705 710 715 720
   Thr Asp Gly Ile Ser Leu Tyr Thr Gly Lys Pro Ile Glu Ala Ser Lys
   725 730 735
   Leu Phe Ser Lys Glu Tyr Asp Ile Glu His Ile Ile Pro Lys Ala Arg
   740 745 750
   Leu Phe Asp Asp Ser Phe Ser Asn Lys Thr Ile Cys Glu Arg Gln Leu
   755 760 765
   Asn Ile Asp Lys Ala Asn Val Thr Ala Phe Ser Phe Leu Gln Asn Lys
   770 775 780
   Leu Ser Ala Asp Glu Phe Glu Gln Tyr Gln Ser Arg Val Lys Ser Leu
   785 790 795 800
   Tyr Gly Lys Leu Ser Lys Ala Lys Ile Gln Lys Leu Leu Met Ala Asn
   805 810 815
   Asp Lys Ile Pro Glu Asp Phe Ile Ala Arg Gln Leu Gln Glu Thr Arg
   820 825 830
   Tyr Ile Ser Lys Lys Ala Lys Glu Ile Leu Phe Glu Ile Ser Arg Arg
   835 840 845
   Val Ser Val Thr Thr Gly Thr Ile Thr Asp Lys Leu Arg Glu Asp Trp
   850 855 860
   Gly Leu Val Glu Ile Met Lys Glu Leu Asn Trp Glu Lys Tyr Asp Lys
   865 870 875 880
   Leu Gly Leu Thr Tyr Thr Ile Glu Gly Lys His Gly Glu Arg Leu Asn
   885 890 895
   Lys Ile Lys Asp Trp Ser Lys Arg Asn Asp His Arg His His Ala Met
               900                 905                 910
   Asp Ala Leu Thr Val Ala Leu Thr Lys Pro Ala Tyr Ile Gln Tyr Leu
           915                 920                 925
   Asn Asn Leu Asn Ala Lys Gly Leu Asn Asn Lys Lys Gly Thr Glu Val
       930                 935                 940
   Phe Ala Ile Glu Gln Lys Tyr Leu Lys Arg Glu Asn Gly Lys Leu Cys
   945                 950                 955                 960
   Phe Ile Pro Pro Ile Glu Asn Ile Arg Ser Glu Ala Lys Lys His Leu
                   965                 970                 975
   Ser Arg Ile Leu Val Ser Tyr Lys Ala Lys Asn Lys Val Val Thr Ile
               980                 985                 990
   Asn Lys Asn Lys Thr Lys Ser Lys Ala Gly Leu Asn Glu Gln Ile Ala
           995                 1000                1005
   Leu Thr Pro Arg Gly Gln Leu His Lys Glu Thr Val Tyr Gly Lys Ser
       1010                1015                1020
   Phe His Tyr Ser Thr Lys Phe Glu Lys Ile Gly Ala Ser Phe Asn Val
   1025                1030                1035                1040
   Gln Lys Ile Asn Thr Val Ala Lys Lys Glu Glu Arg Glu Ala Leu Leu
                   1045                1050                1055
   Lys Arg Leu Ala Glu Asn Gly Asn Asp Pro Lys Lys Ala Phe Thr Gly
               1060                1065                1070
   Lys Asn Thr Leu Asn Lys Met Pro Ile Tyr Leu Asp Leu Gly Lys Asn
           1075                1080                1085
   Ile Lys Leu Ser Glu Lys Val Lys Thr Val Val Leu Glu Gln Asn Tyr
       1090                1095                1100
   Thr Ile Arg Lys Asn Ile Asp Pro Asp Leu Lys Val Asp Lys Val Ile
   1105                1110                1115                1120
   Asp Val Gly Ile Lys Arg Ile Leu Glu Ser Arg Leu Glu Glu Phe Gly
                   1125                1130                1135
   Gly Asn Ala Lys Leu Ala Phe Ser Asn Leu Glu Glu Asn Pro Ile Trp
               1140                1145                1150
   Leu Asn Lys Glu Lys Gly Ile Ser Ile Lys Arg Val Lys Ile Ser Gly
           1155                1160                1165
   Val Ser Asn Val Glu Ser Leu His Val Lys Lys Asp His Phe Gly Glu
       1170                1175                1180
   Pro Ile Leu Asp Gln Glu Gly Asn Glu Ile Pro Val Asp Phe Val Ser
   1185                1190                1195                1200
   Thr Gly Asn Asn His His Val Ala Ile Tyr Glu Asp Glu Asn Gly Asn
                   1205                1210                1215
   Leu Gln Glu Glu Val Val Ser Phe Phe Glu Ala Val Val Arg Gln Asn
               1220                1225                1230
   Gln Gly Leu Pro Ile Ile Lys Lys Asn His Thr Leu Gly Trp Lys Phe
           1235                1240                1245
   Leu Phe Thr Leu Lys Gln Asn Glu Tyr Phe Val Phe Pro Ser Asp Asp
       1250                1255                1260
   Phe Val Pro Ala Asp Val Asp Leu Met Asp Glu Gln Asn Tyr His Leu
   1265                1270                1275                1280
   Ile Ser Pro Asn Leu Phe Arg Val Gln Lys Ile Ala Arg Lys Asn Tyr
                   1285                1290                1295
   Val Phe Asn Asn His Leu Glu Thr Lys Ala Val Asp Asn Asp Leu Leu
               1300                1305                1310
   Lys Ser Lys Lys Glu Leu Ser Lys Ile Thr Tyr His Phe Tyr Gln Thr
           1315                1320                1325
   Pro Glu His Leu Arg Gly Ile Ile Lys Ile Arg Ile Asn His Leu Gly
       1330                1335                1340
   Lys Ile Ile Gln Ile Gly Glu Tyr
   1345                1350
<210> 173
   <211> 1646
   <212> PRT
   <213> Francisella cf. novicida
<220>
   <223> CRISPR-associated endonuclease
<308> YP_005824175
   <309> 2013-06-11
<400> 173
   Met Asn Ile Lys Ile Leu Pro Ile Ala Ile Asp Leu Gly Ala Lys Asn
   1               5                   10                  15
   Thr Gly Val Phe Ser Ala Phe Tyr Gln Lys Gly Thr Ser Leu Glu Ser
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Arg Arg His Gln Arg Arg Gly
       50                  55                  60
   Ile Asp Arg Lys Gln Leu Val Lys Arg Leu Phe Lys Leu Ile Trp Thr
   65                  70                  75                  80
   Lys Gln Leu Asn Leu Glu Trp Asn Lys Asp Thr Gln Gln Thr Ile Ser
                   85                  90                  95
   Phe Leu Leu Asn Arg Arg Gly Phe Ser Phe Ile Thr Asp Gly Tyr Ser
               100                 105                 110
   Pro Glu Tyr Leu Asn Ile Ala Pro Glu Ala Val Lys Ala Ile Leu Met
           115                 120                 125
   Ser Ile Leu Asp Asp Tyr Asn Gly Glu Asp Asp Leu Asp Ser Tyr Leu
       130                 135                 140
   Gln Ser Ala Thr Glu Asn Asp Ser Lys Ile Asp Glu Leu Tyr Asn Lys
   145                 150                 155                 160
   Leu Leu Gln Lys Ala Leu Glu Phe Lys Leu Arg Lys Leu Cys Ile Asp
                   165                 170                 175
   Ile Lys Glu Asp Lys Val Thr Thr Lys Thr Leu Lys Glu Leu Ser Asn
               180                 185                 190
   Thr Glu Phe Lys Leu Leu Ala Asn Tyr Leu Val Asp Tyr Asp Arg Ile
           195                 200                 205
   Leu Arg Thr Gln Lys Phe Ser Tyr Thr Asp Lys Gln Gly Asn Leu Arg
       210                 215                 220
   Glu Leu Asn Tyr Tyr His His Asp Lys Tyr Asn Ile Gln Glu Phe Leu
   225                 230                 235                 240
   Lys Arg Asn Ile Ile Ile Asn Asp Val Ile Leu Glu Lys Leu Thr Asp
                   245                 250                 255
   Asp Leu Asp Ile Trp Asn Leu Asn Phe Asp Lys Phe Asp Phe Glu Lys
               260                 265                 270
   Asn Leu Glu Lys Leu Glu Asn Gln Glu Asp Lys Asp Tyr Leu Gln Thr
           275                 280                 285
   His Leu His His Phe Val Phe Ala Val Asn Lys Ile Lys Ser Glu Met
       290                 295                 300
   Ala Ser Gly Gly Arg His Arg Ser Gln Tyr Phe Gln Glu Ile Thr Asn
   305                 310                 315                 320
   Val Leu Val Glu Asn Asn His Gln Glu Gly Tyr Leu Lys Asn Phe Cys
                   325                 330                 335
   Glu Asn Leu His Asn Lys Lys Tyr Ser Asn Leu Ser Val Lys Asn Leu
               340                 345                 350
   Val Asn Leu Ile Gly Asn Leu Ser Asn Leu Glu Leu Lys Pro Leu Arg
           355                 360                 365
   Lys Tyr Phe Asn Asp Lys Ile His Ala Lys Ala Asp Tyr Trp Asp Glu
       370                 375                 380
   Gln Lys Phe Ala Glu Thr Tyr Ser Asp Trp Ile Leu Gly Glu Trp Arg
   385                 390                 395                 400
   Val Gly Ala Lys Asp Lys Asp Lys Lys Asp Gly Ala Lys Tyr Ser Tyr
                   405                 410                 415
   Lys Asn Leu Cys Asp Glu Leu Lys Gln Lys Val Gly Ile Asn Gln Asp
               420                 425                 430
   Gly Ile Ile Asn Gln Thr Lys Ser Asp Phe Val Asp Phe Leu Leu Glu
   435 440 445
   Leu Asp Pro Cys Arg Thr Ile Pro Pro Tyr Leu Asp Asn Asn Asn Arg
   450 455 460
   Lys Pro Pro Lys Cys Gln Ser Leu Ile Leu Asn Pro Lys Phe Leu Asp
   465 470 475 480
   Asn Lys Tyr Pro Asn Trp Gln Gln Tyr Leu Gln Glu Leu Lys Lys Leu
   485 490 495
   Gln Thr Val Gln Asn Tyr Leu Gly Asn Phe Glu Ile Asp Leu Lys Asp
   500 505 510
   Leu Lys Ser Ser Lys Glu Gln Pro Tyr Phe Val Lys Tyr Lys Ser Leu
   515 520 525
   Asn Gln Gln Ile Ala Ser Gly Gln Arg Asp Tyr Lys Asp Leu Asp Ala
   530 535 540
   Arg Val Leu Gln Phe Ile Phe Asp Arg Val Lys Ala Ser Asp Glu Leu
   545 550 555 560
   Leu Leu Asn Glu Ile Tyr Ser His Ala Lys Lys Leu Lys Gln Asn Thr
   565 570 575
   Ser Ser Glu Leu Glu Lys Leu Glu Leu Cys Lys Lys Leu Asp Glu Val
   580 585 590
   Ile Ala Asn Ser Gln Leu Ser Gln Ile Leu Lys Ser Gln His Val Asn
   595 600 605
   Gly Ile Phe Glu Gln Gly Thr Phe Leu His Leu Val Cys Lys Tyr Tyr
   610 615 620
   Lys Gln Arg Gln Arg Ala Arg Asp Ser Arg Leu Tyr Ile Met Pro Glu
   625 630 635 640
   Tyr Arg Tyr Asp Lys Lys Leu Asp Lys Tyr Asn Asn Thr Gly Arg Phe
   645 650 655
   Asp Asp Asp Asn Gln Leu Leu Thr Tyr Cys Asn His Lys Pro Arg Gln
   660 665 670
   Lys Arg Tyr Gln Leu Leu Asn Asp Leu Ala Gly Val Leu Gln Val Ser
   675 680 685
   Pro Asn Phe Leu Lys Asp Lys Ile Gly Ser Asp Asp Asp Leu Phe Ile
   690 695 700
   Ser Lys Trp Leu Val Glu His Ile Arg Gly Phe Lys Lys Ala Cys Glu
   705 710 715 720
   Asp Ser Leu Lys Ile Gln Lys Asp Asn Arg Gly Leu Leu Asn His Lys
   725 730 735
   Ile Asn Ile Ala Arg Asn Thr Lys Gly Lys Cys Glu Lys Glu Ile Phe
   740 745 750
   Asn Leu Ile Cys Lys Ile Glu Gly Ser Glu Asp Lys Lys Gly Asn Tyr
   755 760 765
   Lys His Gly Leu Ala Tyr Glu Leu Gly Val Leu Leu Phe Gly Glu Pro
   770 775 780
   Asn Gln Ala Ser Lys Leu Glu Phe Asp Arg Lys Ile Lys Lys Phe Asn
   785 790 795 800
   Ser Ile Tyr Ser Phe Ala Gln Ile Gln Gln Ile Ala Phe Ala Glu Arg
   805 810 815
   Lys Gly Asn Ala Asn Thr Cys Ala Val Cys Ser Ala Asp Asn Ala His
   820 825 830
   Arg Met Gln Gln Ile Lys Val Ala Lys Pro Val Glu Gly Asn Lys Asp
   835 840 845
   Asn Ile Ile Leu Ser Ala Lys Ala Gln Arg Leu Pro Ala Ile Pro Thr
   850 855 860
   Arg Ile Val Asp Gly Ala Val Lys Lys Ile Ala Thr Ile Leu Ala Arg
   865 870 875 880
   Asn Ile Val Asp Asp Asn Trp Asp Asn Ile Lys Gln Ala Leu Ser Asn
   885 890 895
   Asn Gln Gln Leu His Val Pro Ile Ile Thr Glu Ser Asn Ala Phe Glu
   900 905 910
   Phe Glu Pro Ala Leu Ala Asp Ile Lys Gly Lys Ser Leu Lys Asp Lys
   915 920 925
   Arg Lys Lys Ala Leu Glu Arg Ile Asp Pro Glu Asn Val Phe Lys Asp
   930 935 940
   Lys Asn Asn Arg Ile Lys Glu Phe Ala Lys Gly Ile Ser Ala Tyr Ser
   945 950 955 960
   Asp Ala Asn Leu Ala Asn Gly Asp Phe Asp Gly Ala Lys Glu Glu Leu
   965 970 975
   Asp His Ile Ile Pro Arg Ser His Lys Lys Tyr Gly Thr Leu Asn Asp
   980 985 990
   Glu Ala Asn Leu Ile Cys Val Thr Arg Glu Asp Asn Gln Asn Arg Gly
   995 1000 1005
   Asn Lys Ala Val Phe Leu Tyr Asp Leu Lys Pro Asn Tyr Lys Leu Lys
   1010 1015 1020
   Gln Phe Asn Thr Thr Asp Asp Leu Glu Ile Glu Lys Lys Ile Ala Asp
   1025 1030 1035 1040
   Thr Ile Trp Asp Ala Ser Lys Gln Asp Phe Lys Phe Gly Asn Tyr Arg
   1045 1050 1055
   Ser Phe Ile Asn Leu Thr Pro Gln Glu Gln Ile Ala Phe Arg His Ala
   1060 1065 1070
   Leu Phe Leu Ala Asp Lys Asn Pro Ile Lys Lys Ala Val Ile Arg Ala
   1075 1080 1085
   Ile Asp Asn Arg Asn Arg Thr Phe Val Asn Gly Thr Gln Arg Tyr Phe
   1090 1095 1100
   Ala Glu Val Leu Ala Asn Asn Ile Tyr Leu Arg Ala Lys Lys Glu Asn
   1105 1110 1115 1120
   Leu Ala Thr Asn Arg Ile Thr Phe Asp Tyr Phe Gly Ile Glu Thr Thr
   1125 1130 1135
   Asn Ser Asn Gly Arg Gly Ile Ala Asp Val Arg Lys Leu Tyr Glu Lys
   1140 1145 1150
   Val Asp Ser Asp Ile Gln Ala Tyr Ala Lys Gly Asp Lys Pro Gln Asp
   1155 1160 1165
   Ser Tyr Ser His Leu Ile Asp Ala Met Ile Ala Phe Cys Val Ala Ala
   1170 1175 1180
   Asp Glu His Lys Asn Glu Gly Ser Ile Gly Leu Lys Met Gly Asn Gln
   1185 1190 1195 1200
   Tyr Gly Leu Phe Pro Thr Pro Asp Lys Tyr Asp Glu Asn Thr Gly Glu
   1205 1210 1215
   Ile Ile Ser Trp His Asn Asp Asp Ile Phe Arg Lys Ile Lys Val Ser
   1220 1225 1230
   Asp Asn Gln Phe Ser Asp Lys Lys Leu Val Arg Lys Lys Ala Thr Glu
   1235 1240 1245
   Gly Phe Asn Thr His Arg Gln Met Thr Arg Asp Gly Ile Tyr Gly Glu
   1250 1255 1260
   Ser Tyr Leu Pro Ile Leu Ile His Lys Asn Leu Asn Glu Val Arg Lys
   1265 1270 1275 1280
   Gly Tyr Asn Trp Glu Asn Ser Glu Glu Ile Lys Ile Phe Lys Gly Lys
   1285 1290 1295
   Lys Tyr Asp Ile Gln Gln Leu Asn Asn Leu Val Tyr Cys Leu Lys Phe
   1300 1305 1310
   Val Asp Lys Pro Ile Ser Ile Asp Ile Gln Ile Thr Thr Leu Glu Glu
   1315 1320 1325
   Leu Arg Asn Ile Leu Glu Thr Asn Asn Ile Ser Thr Thr Ala Glu Tyr
   1330 1335 1340
   Tyr Tyr Ile Asn Leu Lys Thr Gln Lys Leu His Glu Tyr Tyr Ile Glu
   1345 1350 1355 1360
   Asn Tyr Asn Thr Ala Leu Gly Tyr Lys Lys Tyr Thr Lys Glu Met Glu
   1365 1370 1375
   Phe Leu Arg Ser Leu Ala Tyr Arg Thr Glu Arg Val Lys Ile Lys Ser
   1380 1385 1390
   Ile Asp Asp Val Ser Met Ile Leu Ala Lys Asp Ser Asn Phe Lys Ala
   1395 1400 1405
   Gly Lys Ile Glu Leu Pro Phe Lys Thr Glu Trp Gln Arg Leu Tyr Leu
   1410 1415 1420
   Glu Trp Gln Asn Thr Thr Ile Lys Asp Asn His Glu Phe Leu Lys Ser
   1425 1430 1435 1440
   Tyr Phe Ser Val Lys Asn Ala Thr Lys Gln His Lys Lys Val Arg Lys
   1445 1450 1455
   Asp Phe Ser Leu Pro Ile Ser Thr Asn Glu Gly Lys Leu Leu Val Lys
   1460 1465 1470
   Arg Lys Thr Trp Asp Asn Asn Phe Ile Tyr Gln Ile Leu Asn Asp Ser
   1475 1480 1485
   Asp Ser Arg Ala Asp Gly Thr Lys Pro Phe Ile Pro Ala Phe Asp Ile
   1490 1495 1500
   Ser Lys Asn Glu Ile Val Glu Thr Ile Ile Lys Ser Phe Thr Ser Lys
   1505 1510 1515 1520
   Asn Ile Phe Trp Leu Pro Lys Asn Ile Glu Leu Gln Lys Val Asp Asn
   1525 1530 1535
   Lys Ser Ile Phe Ala Ile Asp Thr Ser Arg Trp Phe Glu Val Glu Thr
   1540 1545 1550
   Pro Lys Asp Leu Ile Glu Ile Gly Val Ser Thr Ile Gln Tyr Lys Ile
   1555 1560 1565
   Asp Asn Asn Ser Arg Pro Lys Val Arg Val Lys Leu Asp Tyr Val Met
   1570 1575 1580
   Asp Asp Asp Ser Lys Ile Asn Tyr Phe Met Asn His Ser Leu Leu Lys
   1585 1590 1595 1600
   Ser Arg Tyr Pro Asp Lys Val Leu Glu Ile Leu Lys Gln Ser Thr Ile
   1605 1610 1615
   Ile Glu Phe Glu Ser Ser Gly Phe Asn Lys Thr Ile Lys Glu Met Leu
   1620 1625 1630
   Gly Met Thr Leu Ala Gly Ile Tyr Asn Glu Thr Ser Asn Asn
   1635 1640 1645
<210> 174
   <211> 1380
   <212> PRT
   <213> Prevotella intermedia
<220>
   <223> CRISPR-associated endonuclease
<308> YP_006298249
   <309> 2013-06-11
<400> 174
   Met Lys Arg Ile Leu Gly Leu Asp Leu Gly Thr Thr Ser Ile Gly Trp
    1               5                  10                  15
   Ala Leu Val Asn Glu Ala Glu Asn Asn Asn Glu Ala Ser Ser Ile Val
               20                  25                  30
   Arg Leu Gly Val Arg Val Asn Pro Leu Thr Val Asp Glu Lys Ser Asn
           35                  40                  45
   Phe Glu Lys Gly Lys Ala Ile Thr Thr Asn Ala Asp Arg Gln Leu Arg
       50                  55                  60
   His Gly Ala Arg Ile Asn Leu Gln Arg Tyr Lys Leu Arg Arg Gln Asn
   65                  70                  75                  80
   Leu His Asp Cys Leu Gln Lys Gln Gly Trp Leu Gly Thr Glu Ala Met
                   85                  90                  95
   Tyr Glu Glu Gly Lys Ala Ser Thr Phe Glu Thr Tyr Lys Leu Arg Ala
               100                 105                 110
   Lys Ala Ala Glu Glu Glu Ile Ser Leu His Glu Phe Ala Arg Val Leu
           115                 120                 125
   Phe Met Leu Asn Lys Lys Arg Gly Tyr Lys Ser Asn Arg Lys Ala Asn
       130                 135                 140
   Asn Lys Glu Asp Gly Gln Leu Phe Asp Gly Met Thr Ile Ala Lys Lys
   145                 150                 155                 160
   Leu Tyr Glu Glu His Leu Thr Pro Ala Glu Tyr Ser Leu Gln Leu Leu
                   165                 170                 175
   Asn Lys Gly Lys Lys Phe Thr Gln Gly Tyr Tyr Arg Ser Asp Leu Asn
                           180                     185                     190
   Ala Glu Leu Glu Arg Ile Trp Asp Glu Gln Lys Lys Tyr Tyr Pro Glu
               195                     200                     205
   Ile Leu Thr Asp Glu Phe Lys Gln Gln Leu Glu Gly Lys Thr Lys Thr
       210                     215                     220
   Asn Thr Ser Lys Ile Phe Leu Ala Lys Tyr Gly Ile Tyr Ser Ala Asp
   225                     230                     235                     240
   Leu Lys Gly Leu Asp Arg Lys Phe Gln Pro Leu Lys Trp Arg Val Glu
                   245                     250                     255
   Ala Leu Gln Gln Gln Val Asp Lys Glu Val Leu Ala Phe Val Ile Ser
               260                     265                     270
   Asp Leu Lys Gly Gln Ile Ala Asn Thr Ser Gly Leu Leu Gly Ala Ile
           275                     280                     285
   Ser Asp Arg Ser Lys Glu Leu Tyr Phe Asn Lys Gln Thr Val Gly Gln
       290                     295                     300
   Tyr Leu Trp Ala Ser Leu Glu Glu Asn Pro His Ile Ser Ile Lys Asn
   305                     310                     315                     320
   Lys Pro Phe Tyr Arg Gln Asp Tyr Leu Asp Glu Phe Glu Lys Ile Trp
                   325                     330                     335
   Glu Thr Gln Ala Ala Phe His Lys Gln Leu Thr Pro Glu Leu Lys Gln
               340                     345                     350
   Glu Ile Arg Asp Ile Ile Ile Phe Tyr Gln Arg Pro Leu Lys Ser Lys
           355                     360                     365
   Lys Ser Leu Ile Ser Val Cys Glu Leu Glu Gln Arg Lys Val Lys Ala
       370                     375                     380
   Thr Ile Asp Gly Lys Glu Lys Glu Ile Thr Ile Gly Pro Lys Val Ala
   385                     390                     395                     400
   Pro Lys Ser Ser Pro Val Phe Gln Glu Phe Arg Ile Trp Gln Asn Leu
                   405                     410                     415
   Asn Asn Val Leu Leu Ile Asp Asn Asp Thr Asn Glu Lys Arg Pro Leu
               420                     425                     430
   Asp Glu Val Glu Arg Asn Leu Leu Tyr Lys Glu Leu Ser Ile Lys Ala
           435                     440                     445
   Lys Leu Ser Lys Thr Glu Ala Leu Lys Ile Leu Asn Lys Lys Gly Lys
       450                     455                     460
   Gln Trp Asp Leu Asn Tyr Arg Glu Leu Glu Gly Asn Arg Thr Gln Ala
   465                     470                     475                     480
   Ile Leu Phe Asp Cys Tyr Asn Arg Ile Ile Thr Leu Thr Gly His Glu
                   485                     490                     495
   Glu Cys Asp Phe Lys Lys Ile Lys Ala Ser Glu Ile Arg His Tyr Val
               500                     505                     510
   Ser Thr Ile Phe Lys Asn Leu Gly Phe Ser Thr Glu Ile Leu Asp Phe
           515                     520                     525
   Asp Pro Ser Leu Lys Lys His Glu Leu Glu Lys Gln Pro Met Tyr Gln
       530                     535                     540
   Leu Trp His Leu Leu Tyr Ser Tyr Glu Ser Asp Asn Ser Arg Thr Gly
   545                     550                     555                     560
   Asn Glu Ser Leu Leu Arg Lys Leu Glu Thr Thr Phe Gly Phe Pro Glu
                   565                     570                     575
   Glu Tyr Ala Thr Val Leu Cys Asp Val Val Phe Glu Glu Asp Tyr Gly
               580                     585                     590
   Asn Leu Ser Val Lys Ala Met Arg Glu Ile Leu Pro Tyr Leu Gln Ala
           595                     600                     605
   Gly Asn Asp Tyr Ser Gln Ala Cys Ala Tyr Ala Gly Tyr Asn His Ser
       610                     615                     620
   Arg His Ser Leu Thr Lys Glu Glu Leu Asp Gln Lys Val Tyr Lys Glu
   625                     630                     635                     640
   Arg Leu Glu Leu Leu Pro Lys Asn Ser Leu Arg Asn Pro Val Val Glu
                   645                     650                     655
   Lys Ile Leu Asn Gln Met Ile Asn Val Ile Asn Ala Ile Ile Asp Glu
               660                     665                     670
   Tyr Gly Lys Pro Asp Glu Ile Arg Ile Glu Met Ala Arg Glu Leu Lys
           675                     680                     685
   Ser Ser Ala Ala Asp Arg Lys Lys Thr Thr His Ala Ile Ser Gln Gly
       690                 695                 700
   Asn Ala Glu Asn Gln Arg Ile Arg Glu Ile Leu Glu Lys Glu Phe Ser
   705                 710                 715                 720
   Leu Ser Tyr Ile Ser Arg Asn Asp Ile Ile Lys Tyr Lys Leu Tyr Glu
                   725                 730                 735
   Glu Leu Glu Pro Asn Tyr Tyr Lys Thr Leu Tyr Ser Asp Thr Tyr Ile
               740                 745                 750
   Thr Lys Asp Lys Leu Phe Ser Lys Asp Phe Asp Ile Glu His Ile Ile
           755                 760                 765
   Pro Lys Ala Arg Leu Phe Asp Asp Ser Phe Ser Asn Lys Thr Leu Glu
       770                 775                 780
   Ala Arg Asn Ile Asn Leu Glu Lys Ser Asn Lys Thr Ala Phe Asp Phe
   785                 790                 795                 800
   Ile Lys Glu Lys Tyr Gly Glu Asp Gly Ala Glu Ala Tyr Lys Lys Lys
                   805                 810                 815
   Leu Asp Met Leu Leu Glu Asn Asp Ala Ile Ser Arg Pro Lys Tyr Asn
               820                 825                 830
   Asn Leu Leu Arg Ala Glu Ala Asp Ile Pro Ser Asp Phe Ile Asn Arg
           835                 840                 845
   Asp Leu Arg Asn Thr Gln Tyr Ile Ala Lys Lys Ala Cys Glu Ile Leu
       850                 855                 860
   Gly Glu Leu Val Lys Thr Val Thr Pro Thr Thr Gly Lys Ile Thr Asn
   865                 870                 875                 880
   Arg Leu Arg Glu Asp Trp Gln Leu Val Asp Val Met Lys Glu Leu Asn
                   885                 890                 895
   Phe Glu Lys Tyr Glu Lys Leu Gly Leu Thr Glu Ile Val Glu Asp Arg
               900                 905                 910
   Asp Gly Arg Lys Ile Lys Arg Ile Lys Asp Trp Thr Lys Arg Asn Asp
           915                 920                 925
   His Arg His His Ala Met Asp Ala Leu Ala Ile Ala Phe Thr Lys Pro
       930                 935                 940
   Ser Phe Ile Gln Tyr Leu Asn Asn Leu Asn Ala Arg Ser Asn Lys Gly
   945                 950                 955                 960
   Asp Ser Ile Tyr Ala Ile Glu Asn Lys Glu Leu His Tyr Glu Glu Gly
                   965                 970                 975
   Lys Leu Arg Phe Asn Ala Pro Ile Pro Val Asn Glu Phe Arg Ala Glu
               980                 985                 990
   Ala Lys Arg His Leu Ser Ala Ile Leu Val Ser Ile Lys Ala Lys Asn
           995                 1000                1005
   Lys Val Met Thr Gln Asn Val Asn Lys Ile Lys Thr Lys His Gly Ile
       1010                1015                1020
   Ile Lys Lys Ile Gln Leu Thr Pro Arg Gly Pro Leu His Asn Glu Thr
   1025                1030                1035                1040
   Ile Tyr Gly Thr Lys Met Arg Pro Ile Ile Lys Met Val Lys Val Gly
                   1045                1050                1055
   Ala Ala Leu Asp Glu Ala Thr Ile Asn Lys Val Ser Ser Pro Ala Ile
               1060                1065                1070
   Arg Glu Ala Leu Leu Lys Arg Leu Asn Glu Tyr Ser Gly Asn Ala Lys
           1075                1080                1085
   Lys Ala Phe Thr Gly Lys Asn Thr Leu Glu Lys Asn Pro Ile Tyr Leu
       1090                1095                1100
   Asn Ala Gly Arg Thr Lys Thr Val Pro Ser Leu Val Lys Thr Val Glu
   1105                1110                1115                1120
   Trp Glu Ser Phe His Pro Thr Arg Lys Leu Ile Asp Lys Asp Leu Asn
                   1125                1130                1135
   Val Asp Lys Val Val Asp Lys Gly Ile Arg Glu Ile Leu Lys Ala Arg
               1140                1145                1150
   Leu Glu Glu Phe Asn Gly Asp Ala Lys Lys Ala Phe Ser Asn Leu Glu
           1155                1160                1165
   Glu Asn Pro Ile Tyr Leu Asp Glu Ala Lys Lys Ile Ala Leu Lys Arg
       1170                1175                1180
   Val Ser Ile Glu Gly Val Leu Ser Ala Ile Pro Leu His Thr Leu Lys
   1185 1190 1195 1200
   Asn Gln Ala Gly Lys Pro Ile Thr Gly Lys Asp Gly Lys Pro Val Leu
   1205 1210 1215
   Gly Asn Tyr Val Gln Thr Ser Asn Asn His His Ile Ala Phe Tyr Tyr
   1220 1225 1230
   Asp Glu Asp Gly Asn Leu Gln Asp Asn Ala Val Ser Phe Phe Glu Ala
   1235 1240 1245
   Ala Glu Arg Lys Ser Gln Gly Ile Pro Val Ile Asp Lys Asp Tyr Asn
   1250 1255 1260
   Arg Asp Lys Gly Trp Arg Phe Leu Phe Thr Met Lys Gln Asn Glu Tyr
   1265 1270 1275 1280
   Phe Val Phe Pro Asn Glu Ala Thr Gly Phe Ile Pro Ser Glu Val Asp
   1285 1290 1295
   Leu Thr Asp Glu Ala Asn Tyr Gly Ile Ile Ser Pro Asn Leu Tyr Arg
   1300 1305 1310
   Val Gln Lys Val Ser Arg Ile Asp Lys Gly Thr Ser Ala Ser Arg Asp
   1315 1320 1325
   Tyr Trp Phe Arg His His Leu Glu Thr Ile Leu Asn Asp Asp Ala Lys
   1330 1335 1340
   Leu Lys Asn Leu Ala Phe Lys Arg Ile Arg Gly Leu Leu Glu Leu Lys
   1345 1350 1355 1360
   Asp Ile Ile Lys Val Arg Ile Asn Ser Thr Gly Lys Ile Val Ala Val
   1365 1370 1375
   Gly Glu Tyr Asp
   1380
<210> 175
   <211> 1121
   <212> PRT
   <213> Streptococcus thermophilus
<220>
   <223> CRISPR-associated endonuclease
<308> YP_006040517
   <309> 2013-06-11
<400> 175
   Met Ser Asp Leu Val Leu Gly Leu Asp Ile Gly Ile Gly Ser Val Gly
    1               5                  10                  15
   Val Gly Ile Leu Asn Lys Val Thr Gly Glu Ile Ile His Lys Asn Ser
               20                  25                  30
   Arg Ile Phe Pro Ala Ala Gln Ala Glu Asn Asn Leu Val Arg Arg Thr
           35                  40                  45
   Asn Arg Gln Gly Arg Arg Leu Thr Arg Arg Lys Lys His Arg Ile Val
       50                  55                  60
   Arg Leu Asn Arg Leu Phe Glu Glu Ser Gly Leu Ile Thr Asp Phe Thr
   65                  70                  75                  80
   Lys Ile Ser Ile Asn Leu Asn Pro Tyr Gln Leu Arg Val Lys Gly Leu
                   85                  90                  95
   Thr Asp Glu Leu Ser Asn Glu Glu Leu Phe Ile Ala Leu Lys Asn Met
               100                 105                 110
   Val Lys His Arg Gly Ile Ser Tyr Leu Asp Asp Ala Ser Asp Asp Gly
           115                 120                 125
   Asn Ser Ser Val Gly Asp Tyr Ala Gln Ile Val Lys Glu Asn Ser Lys
       130                 135                 140
   Gln Leu Glu Thr Lys Thr Pro Gly Gln Ile Gln Leu Glu Arg Tyr Gln
   145                 150                 155                 160
   Thr Tyr Gly Gln Leu Arg Gly Asp Phe Thr Val Glu Lys Asp Gly Lys
                   165                 170                 175
   Lys His Arg Leu Ile Asn Val Phe Pro Thr Ser Ala Tyr Arg Ser Glu
               180                 185                 190
   Ala Leu Arg Ile Leu Gln Thr Gln Gln Glu Phe Asn Pro Gln Ile Thr
           195                 200                 205
   Asp Glu Phe Ile Asn Arg Tyr Leu Glu Ile Leu Thr Gly Lys Arg Lys
       210                 215                 220
   Tyr Tyr His Gly Pro Gly Asn Glu Lys Ser Arg Thr Asp Tyr Gly Arg
   225                 230                 235                 240
   Tyr Arg Thr Ser Gly Glu Thr Leu Asp Asn Ile Phe Gly Ile Leu Ile
                   245                 250                 255
   Gly Lys Cys Thr Phe Tyr Pro Asp Glu Phe Arg Ala Ala Lys Ala Ser
               260                 265                 270
   Tyr Thr Ala Gln Glu Phe Asn Leu Leu Asn Asp Leu Asn Asn Leu Thr
           275                 280                 285
   Val Pro Thr Glu Thr Lys Lys Leu Ser Lys Glu Gln Lys Asn Gln Ile
       290                 295                 300
   Ile Asn Tyr Val Lys Asn Glu Lys Ala Met Gly Pro Ala Lys Leu Phe
   305                 310                 315                 320
   Lys Tyr Ile Ala Lys Leu Leu Ser Cys Asp Val Ala Asp Ile Lys Glu
                   325                 330                 335
   Tyr Arg Ile Asp Lys Ser Gly Lys Ala Glu Ile His Thr Phe Glu Ala
               340                 345                 350
   Tyr Arg Lys Met Lys Thr Leu Glu Thr Leu Asp Ile Glu Gln Met Asp
           355                 360                 365
   Arg Glu Thr Leu Asp Lys Leu Ala Tyr Val Leu Thr Leu Asn Thr Glu
       370                 375                 380
   Arg Glu Gly Ile Gln Glu Ala Leu Glu His Glu Phe Ala Asp Gly Ser
   385                 390                 395                 400
   Phe Ser Gln Lys Gln Val Asp Glu Leu Val Gln Phe Arg Lys Ala Asn
                   405                 410                 415
   Ser Ser Ile Phe Gly Lys Gly Trp His Asn Phe Ser Val Lys Leu Met
               420                 425                 430
   Met Glu Leu Ile Pro Glu Leu Tyr Glu Thr Ser Glu Glu Gln Met Thr
           435                 440                 445
   Ile Leu Thr Arg Leu Gly Lys Gln Lys Thr Thr Ser Ser Ser Asn Lys
       450                 455                 460
   Thr Lys Tyr Ile Asp Glu Lys Leu Leu Thr Glu Glu Ile Tyr Asn Pro
   465                 470                 475                 480
   Val Val Ala Lys Ser Val Arg Gln Ala Ile Lys Ile Val Asn Ala Ala
                   485                 490                 495
   Ile Lys Glu Tyr Gly Asp Phe Asp Asn Ile Val Ile Glu Met Ala Arg
               500                 505                 510
   Glu Thr Asn Glu Asp Asp Glu Lys Lys Ala Ile Gln Lys Ile Gln Lys
           515                 520                 525
   Ala Asn Lys Asp Glu Lys Asp Ala Ala Met Leu Lys Ala Ala Asn Gln
       530                 535                 540
   Tyr Asn Gly Lys Ala Glu Leu Pro His Ser Val Phe His Gly His Lys
   545                 550                 555                 560
   Gln Leu Ala Thr Lys Ile Arg Leu Trp His Gln Gln Gly Glu Arg Cys
                   565                 570                 575
   Leu Tyr Thr Gly Lys Thr Ile Ser Ile His Asp Leu Ile Asn Asn Ser
               580                 585                 590
   Asn Gln Phe Glu Val Asp His Ile Leu Pro Leu Ser Ile Thr Phe Asp
           595                 600                 605
   Asp Ser Leu Ala Asn Lys Val Leu Val Tyr Ala Thr Ala Asn Gln Glu
       610                 615                 620
   Lys Gly Gln Arg Thr Pro Tyr Gln Ala Leu Asp Ser Met Asp Asp Ala
   625                 630                 635                 640
   Trp Ser Phe Arg Glu Leu Lys Ala Phe Val Arg Glu Ser Lys Thr Leu
                   645                 650                 655
   Ser Asn Lys Lys Lys Glu Tyr Leu Leu Thr Glu Glu Asp Ile Ser Lys
               660                 665                 670
   Phe Asp Val Arg Lys Lys Phe Ile Glu Arg Asn Leu Val Asp Thr Arg
           675                 680                 685
   Tyr Ala Ser Arg Val Val Leu Asn Ala Leu Gln Glu His Phe Arg Ala
       690                 695                 700
   His Lys Ile Asp Thr Lys Val Ser Val Val Arg Gly Gln Phe Thr Ser
   705                 710                 715                 720
   Gln Leu Arg Arg His Trp Gly Ile Glu Lys Thr Arg Asp Thr Tyr His
                   725                 730                 735
   His His Ala Val Asp Ala Leu Ile Ile Ala Ala Ser Ile Gln Leu Asn
               740                 745                 750
   Leu Trp Lys Lys Gln Lys Asn Thr Leu Val Ser Tyr Ser Glu Asp Gln
           755                 760                 765
   Leu Leu Asp Ile Glu Thr Gly Glu Leu Ile Ser Asp Asp Glu Tyr Lys
       770                 775                 780
   Glu Ser Val Phe Lys Ala Pro Tyr Gln His Phe Val Asp Thr Leu Lys
   785                 790                 795                 800
   Ser Lys Glu Phe Glu Asp Ser Ile Leu Phe Ser Tyr Gln Val Asp Ser
                   805                 810                 815
   Lys Phe Asn Arg Lys Ile Ser Asp Ala Thr Ile Tyr Ala Thr Arg Gln
               820                 825                 830
   Ala Lys Val Gly Lys Asp Lys Ala Asp Glu Thr Tyr Val Leu Gly Lys
           835                 840                 845
   Ile Lys Asp Ile Tyr Thr Gln Asp Gly Tyr Asp Ala Phe Met Lys Ile
       850                 855                 860
   Tyr Lys Lys Asp Lys Ser Lys Phe Leu Met Tyr Arg His Asp Pro Gln
   865                 870                 875                 880
   Thr Phe Glu Lys Val Ile Glu Pro Ile Leu Glu Asn Tyr Pro Asn Lys
                   885                 890                 895
   Gln Ile Asn Glu Lys Gly Lys Glu Val Pro Val Asn Pro Phe Leu Lys
               900                 905                 910
   Tyr Lys Glu Glu His Gly Tyr Ile Arg Lys Tyr Ser Lys Lys Gly Asn
           915                 920                 925
   Gly Pro Glu Ile Lys Ser Leu Lys Tyr Tyr Asp Ser Lys Leu Gly Asn
       930                 935                 940
   His Ile Asp Ile Thr Pro Lys Asp Ser Asn Asn Lys Val Val Leu Gln
   945                 950                 955                 960
   Ser Val Ser Pro Trp Arg Ala Asp Val Tyr Phe Asn Lys Thr Thr Gly
                   965                 970                 975
   Lys Tyr Glu Ile Leu Gly Leu Lys Tyr Ala Asp Leu Gln Phe Glu Lys
               980                 985                 990
   Gly Thr Gly Thr Tyr Lys Ile Ser Gln Glu Lys Tyr Asn Asp Ile Lys
           995                 1000                1005
   Lys Lys Glu Gly Val Asp Ser Asp Ser Glu Phe Lys Phe Thr Leu Tyr
       1010                1015                1020
   Lys Asn Asp Leu Leu Leu Val Lys Asp Thr Glu Thr Lys Glu Gln Gln
   1025                1030                1035                1040
   Leu Phe Arg Phe Leu Ser Arg Thr Met Pro Lys Gln Lys His Tyr Val
                   1045                1050                1055
   Glu Leu Lys Pro Tyr Asp Lys Gln Lys Phe Glu Gly Gly Glu Ala Leu
               1060                1065                1070
   Ile Lys Val Leu Gly Asn Val Ala Asn Ser Gly Gln Cys Lys Lys Gly
           1075                1080                1085
   Leu Gly Lys Ser Asn Ile Ser Ile Tyr Lys Val Arg Thr Asp Val Leu
       1090                1095                1100
   Gly Asn Gln His Ile Ile Lys Asn Glu Gly Asp Lys Pro Lys Leu Asp
   1105                1110                1115                1120
   Phe
<210> 176
   <211> 1334
   <212> PRT
   <213> Listeria monocytogenes
<220>
   <223> CRISPR-associated endonuclease
<308> YP_005966656
   <309> 2013-06-11
<400> 176
   Met Lys Asn Pro Tyr Thr Ile Gly Leu Asp Ile Gly Thr Asn Ser Val
    1               5                  10                  15
   Gly Trp Ala Val Leu Thr Asn Gln Tyr Asp Leu Val Lys Arg Lys Met
               20                  25                  30
   Lys Val Ala Gly Asn Ser Asp Lys Lys Gln Ile Lys Lys Asn Phe Trp
           35                  40                  45
   Gly Val Arg Leu Phe Asp Asp Gly Gln Thr Ala Val Asp Arg Arg Met
       50                  55                  60
   Asn Arg Thr Ala Arg Arg Arg Ile Glu Arg Arg Arg Asn Arg Ile Ser
   65                  70                  75                  80
   Tyr Leu Gln Glu Ile Phe Ala Val Glu Met Ala Asn Ile Asp Ala Asn
                   85                  90                  95
   Phe Phe Cys Arg Leu Asn Asp Ser Phe Tyr Val Asp Ser Glu Lys Arg
               100                 105                 110
   Asn Ser Arg His Pro Phe Phe Ala Thr Ile Glu Glu Glu Val Ala Tyr
           115                 120                 125
   His Asp Asn Tyr Arg Thr Ile Tyr His Leu Arg Glu Lys Leu Val Asn
       130                 135                 140
   Ser Ser Glu Lys Ala Asp Leu Arg Leu Val Tyr Leu Ala Leu Ala His
   145                 150                 155                 160
   Ile Ile Lys Tyr Arg Gly Asn Phe Leu Ile Glu Gly Ala Leu Asp Thr
                   165                 170                 175
   Lys Asn Thr Ser Val Asp Glu Val Tyr Lys Gln Phe Ile Glu Thr Tyr
               180                 185                 190
   Asn Gln Val Phe Met Ser Asn Ile Glu Glu Gly Ala Leu Ala Lys Val
           195                 200                 205
   Glu Glu Asn Ile Glu Val Ala Asn Ile Leu Ala Gly Lys Phe Thr Arg
       210                 215                 220
   Arg Glu Lys Phe Glu Arg Ile Leu Gln Leu Tyr Pro Gly Glu Lys Ser
   225                 230                 235                 240
   Thr Gly Met Phe Ala Gln Phe Ile Ser Leu Ile Val Gly Ser Lys Gly
                   245                 250                 255
   Asn Phe Gln Lys Val Phe Asp Leu Ile Glu Lys Thr Asp Ile Glu Cys
               260                 265                 270
   Ala Lys Asp Ser Tyr Glu Glu Asp Leu Glu Thr Leu Leu Ala Ile Ile
           275                 280                 285
   Gly Asp Glu Tyr Ala Glu Leu Phe Val Ala Ala Lys Asn Thr Tyr Asn
       290                 295                 300
   Ala Val Val Leu Ser Ser Ile Ile Thr Val Thr Asp Thr Glu Thr Asn
   305                 310                 315                 320
   Ala Lys Leu Ser Ala Ser Met Ile Glu Arg Phe Asp Ala His Glu Lys
                   325                 330                 335
   Asp Leu Val Glu Leu Lys Ala Phe Ile Lys Leu Asn Leu Pro Lys Gln
               340                 345                 350
   Tyr Glu Glu Ile Phe Ser Asn Ala Ala Ile Asp Gly Tyr Ala Gly Tyr
           355                 360                 365
   Ile Asp Gly Lys Thr Lys Gln Val Asp Phe Tyr Lys Tyr Leu Lys Thr
       370                 375                 380
   Ile Leu Glu Asn Ile Glu Gly Ser Asp Tyr Phe Ile Ala Lys Ile Glu
   385                 390                 395                 400
   Glu Glu Asn Phe Leu Arg Lys Gln Arg Thr Phe Asp Asn Gly Ala Ile
                   405                 410                 415
   Pro His Gln Leu His Leu Glu Glu Leu Glu Ala Ile Ile His Gln Gln
               420                 425                 430
   Ala Lys Tyr Tyr Pro Phe Leu Lys Glu Asp Tyr Asp Lys Ile Lys Ser
           435                 440                 445
   Leu Val Thr Phe Arg Ile Pro Tyr Phe Val Gly Pro Leu Ala Asn Gly
       450                 455                 460
   Gln Ser Glu Phe Ala Trp Leu Thr Arg Lys Ala Asp Gly Glu Ile Arg
   465                 470                 475                 480
   Pro Trp Asn Ile Glu Glu Lys Val Asp Phe Gly Lys Ser Ala Val Asp
                   485                 490                 495
   Phe Ile Glu Lys Met Thr Asn Lys Asp Thr Tyr Leu Pro Lys Glu Asn
               500                 505                 510
   Val Leu Pro Lys His Ser Leu Cys Tyr Gln Lys Tyr Met Val Tyr Asn
           515                 520                 525
   Glu Leu Thr Lys Ile Arg Tyr Ile Asp Asp Gln Gly Lys Thr Asn Tyr
       530                 535                 540
   Phe Ser Gly Arg Glu Lys Gln Gln Val Phe Asn Asp Leu Phe Lys Gln
   545                 550                 555                 560
   Lys Arg Lys Val Lys Lys Lys Asp Leu Glu Leu Phe Leu Arg Asn Ile
                   565                 570                 575
   Asn His Ile Glu Ser Pro Thr Ile Glu Gly Leu Glu Asp Ser Phe Asn
               580                 585                 590
   Ala Ser Tyr Ala Thr Tyr His Asp Leu Leu Lys Val Gly Met Lys Gln
           595                 600                 605
   Glu Ile Leu Asp Asn Pro Leu Asn Thr Glu Met Leu Glu Asp Ile Val
       610                 615                 620
   Lys Ile Leu Thr Val Phe Glu Asp Lys Pro Met Ile Lys Glu Gln Leu
   625                 630                 635                 640
   Gln Gln Phe Ser Asp Val Leu Asp Gly Gly Val Leu Lys Lys Leu Glu
                   645                 650                 655
   Arg Arg His Tyr Thr Gly Trp Gly Arg Leu Ser Ala Lys Leu Leu Val
               660                 665                 670
   Gly Ile Arg Glu Lys Gln Ser His Leu Thr Ile Leu Asp Tyr Leu Met
           675                 680                 685
   Asn Asp Asp Gly Leu Asn Arg Asn Leu Met Gln Leu Ile Asn Asp Ser
       690                 695                 700
   Asn Leu Ser Phe Lys Ser Ile Ile Glu Lys Glu Gln Val Ser Thr Thr
   705                 710                 715                 720
   Asp Lys Asp Leu Gln Ser Ile Val Ala Glu Leu Ala Gly Ser Pro Ala
                   725                 730                 735
   Ile Lys Lys Gly Ile Leu Gln Ser Leu Lys Ile Val Asp Glu Leu Val
               740                 745                 750
   Ser Ile Met Gly Tyr Pro Pro Gln Thr Ile Val Val Glu Met Ala Arg
           755                 760                 765
   Glu Asn Gln Thr Thr Gly Lys Gly Lys Asn Asn Ser Lys Pro Arg Tyr
       770                 775                 780
   Lys Ser Leu Glu Lys Ala Ile Lys Glu Phe Gly Ser Gln Ile Leu Lys
   785                 790                 795                 800
   Glu His Pro Thr Asp Asn Gln Glu Leu Lys Asn Asn Arg Leu Tyr Leu
                   805                 810                 815
   Tyr Tyr Leu Gln Asn Gly Lys Asp Met Tyr Thr Gly Gln Glu Leu Asp
               820                 825                 830
   Ile His Asn Leu Ser Asn Tyr Asp Ile Asp His Ile Val Pro Gln Ser
           835                 840                 845
   Phe Ile Thr Asp Asn Ser Ile Asp Asn Leu Val Leu Thr Ser Ser Ala
       850                 855                 860
   Gly Asn Arg Glu Lys Gly Gly Asp Val Pro Pro Leu Glu Ile Val Arg
   865                 870                 875                 880
   Lys Arg Lys Val Phe Trp Glu Lys Leu Tyr Gln Gly Asn Leu Met Ser
                   885                 890                 895
   Lys Arg Lys Phe Asp Tyr Leu Thr Lys Ala Glu Arg Gly Gly Leu Thr
               900                 905                 910
   Glu Ala Asp Lys Ala Arg Phe Ile His Arg Gln Leu Val Glu Thr Arg
           915                 920                 925
   Gln Ile Thr Lys Asn Val Ala Asn Ile Leu Tyr Gln Arg Phe Asn Lys
       930                 935                 940
   Glu Thr Asp Asn His Gly Asn Thr Met Glu Gln Val Arg Ile Val Thr
   945                 950                 955                 960
   Leu Lys Ser Ala Leu Val Ser Gln Phe Arg Lys Gln Phe Gln Leu Tyr
   965 970 975
   Lys Val Arg Glu Val Asn Gly Tyr His His Ala His Asp Ala Tyr Leu
   980 985 990
   Asn Gly Val Val Ala Asn Thr Leu Leu Lys Val Tyr Pro Gln Leu Glu
   995 1000 1005
   Pro Glu Phe Val Tyr Gly Glu Tyr His Gln Phe Asp Trp Phe Lys Ala
   1010 1015 1020
   Asn Lys Ala Thr Ala Lys Lys Gln Phe Tyr Thr Asn Ile Met Leu Phe
   1025 1030 1035 1040
   Phe Ala Gln Lys Glu Arg Ile Ile Asp Glu Asn Gly Glu Ile Leu Trp
   1045 1050 1055
   Asp Lys Lys Tyr Leu Glu Thr Ile Lys Lys Val Leu Asp Tyr Arg Gln
   1060 1065 1070
   Met Asn Ile Val Lys Lys Thr Glu Ile Gln Lys Gly Glu Phe Ser Lys
   1075 1080 1085
   Ala Thr Ile Lys Pro Lys Gly Asn Ser Ser Lys Leu Ile Pro Arg Lys
   1090 1095 1100
   Glu Asn Trp Asp Pro Met Lys Tyr Gly Gly Leu Asp Ser Pro Asn Met
   1105 1110 1115 1120
   Ala Tyr Ala Val Ile Ile Glu His Ala Lys Gly Lys Lys Lys Ile Val
   1125 1130 1135
   Ile Glu Lys Lys Leu Ile Gln Ile Asn Ile Met Glu Arg Lys Met Phe
   1140 1145 1150
   Glu Lys Asp Glu Glu Ala Phe Leu Glu Glu Lys Gly Tyr Arg His Pro
   1155 1160 1165
   Lys Val Leu Thr Lys Leu Pro Lys Tyr Thr Leu Tyr Glu Cys Glu Lys
   1170 1175 1180
   Gly Arg Arg Arg Met Leu Ala Ser Ala Asn Glu Ala Gln Lys Gly Asn
   1185 1190 1195 1200
   Gln Leu Val Leu Ser Asn His Leu Val Ser Leu Leu Tyr His Ala Lys
   1205 1210 1215
   Asn Cys Glu Ala Ser Asp Gly Lys Ser Leu Lys Tyr Ile Glu Ala His
   1220 1225 1230
   Arg Glu Thr Phe Ser Glu Leu Leu Ala Gln Val Ser Glu Phe Ala Thr
   1235 1240 1245
   Arg Tyr Thr Leu Ala Asp Ala Asn Leu Ser Lys Ile Asn Asn Leu Phe
   1250 1255 1260
   Glu Gln Asn Lys Glu Gly Asp Ile Lys Ala Ile Ala Gln Ser Phe Val
   1265 1270 1275 1280
   Asp Leu Met Ala Phe Asn Ala Met Gly Ala Pro Ala Ser Phe Lys Phe
   1285 1290 1295
   Phe Glu Ala Thr Ile Asp Arg Lys Arg Tyr Thr Asn Leu Lys Glu Leu
   1300 1305 1310
   Leu Ser Ser Thr Ile Ile Tyr Gln Ser Ile Thr Gly Leu Tyr Glu Ser
   1315 1320 1325
   Arg Lys Arg Leu Asp Asp
   1330
<210> 177
   <211> 1334
   <212> PRT
   <213> Listeria monocytogenes
<220>
   <223> CRISPR-associated endonuclease
<308> YP_005963707
   <309> 2013-06-11
<400> 177
   Met Lys Asn Pro Tyr Thr Ile Gly Leu Asp Ile Gly Thr Asn Ser Val
   1               5                   10                  15
   Gly Trp Ala Val Leu Thr Asn Gln Tyr Asp Leu Val Lys Arg Lys Met
               20                  25                  30
   Lys Val Ala Gly Asn Ser Asp Lys Lys Gln Ile Lys Lys Asn Phe Trp
           35                  40                  45
   Gly Val Arg Leu Phe Asp Asp Gly Gln Thr Ala Val Asp Arg Arg Met
       50                  55                  60
   Asn Arg Thr Ala Arg Arg Arg Ile Glu Arg Arg Arg Asn Arg Ile Ser
   65                  70                  75                  80
   Tyr Leu Gln Glu Ile Phe Ala Val Glu Met Ala Asn Ile Asp Ala Asn
                   85                  90                  95
   Phe Phe Cys Arg Leu Asn Asp Ser Phe Tyr Val Asp Ser Glu Lys Arg
               100                 105                 110
   Asn Ser Arg His Pro Phe Phe Ala Thr Ile Glu Glu Glu Val Ala Tyr
           115                 120                 125
   His Lys Asn Tyr Arg Thr Ile Tyr His Leu Arg Glu Glu Leu Val Asn
       130                 135                 140
   Ser Ser Glu Lys Ala Asp Leu Arg Leu Val Tyr Leu Ala Leu Ala His
   145                 150                 155                 160
   Ile Ile Lys Tyr Arg Gly Asn Phe Leu Ile Glu Gly Ala Leu Asp Thr
                   165                 170                 175
   Lys Asn Thr Ser Val Asp Gly Val Tyr Glu Gln Phe Ile Gln Thr Tyr
               180                 185                 190
   Asn Gln Val Phe Met Ser Asn Ile Glu Glu Gly Thr Leu Ala Lys Val
           195                 200                 205
   Glu Glu Asn Ile Glu Val Ala Asn Ile Leu Ala Gly Lys Phe Thr Arg
       210                 215                 220
   Arg Glu Lys Phe Glu Arg Ile Leu Gln Leu Tyr Pro Gly Glu Lys Ser
   225                 230                 235                 240
   Thr Gly Met Phe Ala Gln Phe Ile Ser Leu Ile Val Gly Ser Lys Gly
                   245                 250                 255
   Asn Phe Gln Lys Val Phe Asp Leu Ile Glu Lys Thr Asp Ile Glu Cys
               260                 265                 270
   Ala Lys Asp Ser Tyr Glu Glu Asp Leu Glu Ala Leu Leu Ala Ile Ile
           275                 280                 285
   Gly Asp Glu Tyr Ala Glu Leu Phe Val Ala Ala Lys Asn Thr Tyr Asn
       290                 295                 300
   Ala Val Val Leu Ser Ser Ile Ile Thr Val Thr Ala Thr Glu Thr Asn
   305                 310                 315                 320
   Ala Lys Leu Ser Ala Ser Met Ile Glu Arg Phe Asp Ala His Glu Lys
                   325                 330                 335
   Asp Leu Gly Glu Leu Lys Ala Phe Ile Lys Leu His Leu Pro Lys Gln
               340                 345                 350
   Tyr Gln Glu Ile Phe Asn Asn Ala Ala Ile Asp Gly Tyr Ala Gly Tyr
           355                 360                 365
   Ile Asp Gly Lys Thr Lys Gln Val Asp Phe Tyr Lys Tyr Leu Lys Thr
       370                 375                 380
   Ile Leu Glu Asn Ile Glu Gly Ala Asp Tyr Phe Ile Ala Lys Ile Glu
   385                 390                 395                 400
   Glu Glu Asn Phe Leu Arg Lys Gln Arg Thr Phe Asp Asn Gly Ala Ile
                   405                 410                 415
   Pro His Gln Leu His Leu Glu Glu Leu Glu Ala Ile Ile His Gln Gln
               420                 425                 430
   Ala Lys Tyr Tyr Pro Phe Leu Arg Glu Asp Tyr Glu Lys Ile Lys Ser
           435                 440                 445
   Leu Val Thr Phe Arg Ile Pro Tyr Phe Val Gly Pro Leu Ala Lys Gly
       450                 455                 460
   Gln Ser Glu Phe Ala Trp Leu Thr Arg Lys Ala Asp Gly Glu Ile Arg
   465                 470                 475                 480
   Pro Trp Asn Ile Glu Glu Lys Val Asp Phe Gly Lys Ser Ala Val Asp
                   485                 490                 495
   Phe Ile Glu Lys Met Thr Asn Lys Asp Thr Tyr Leu Pro Lys Glu Asn
               500                 505                 510
   Val Leu Pro Lys His Ser Leu Cys Tyr Gln Lys Tyr Met Val Tyr Asn
           515                 520                 525
   Glu Leu Thr Lys Val Arg Tyr Ile Asp Asp Gln Gly Lys Thr Asn Tyr
       530                 535                 540
   Phe Ser Gly Gln Glu Lys Gln Gln Ile Phe Asn Asp Leu Phe Lys Gln
   545                 550                 555                 560
   Lys Arg Lys Val Lys Lys Lys Asp Leu Glu Leu Phe Leu Arg Asn Ile
                   565                 570                 575
   Asn His Ile Glu Ser Pro Thr Ile Glu Gly Leu Glu Asp Ser Phe Asn
               580                 585                 590
   Ala Ser Tyr Ala Thr Tyr His Asp Leu Leu Lys Val Gly Met Lys Gln
           595                 600                 605
   Glu Ile Leu Asp Asn Pro Leu Asn Thr Glu Met Leu Glu Asp Ile Val
       610                 615                 620
   Lys Ile Leu Thr Val Phe Glu Asp Lys Pro Met Ile Lys Glu Gln Leu
   625                 630                 635                 640
   Gln Gln Phe Ser Asp Val Leu Asp Gly Gly Val Leu Lys Lys Leu Glu
                   645                 650                 655
   Arg Arg His Tyr Thr Gly Trp Gly Arg Leu Ser Ala Lys Leu Leu Val
               660                 665                 670
   Gly Ile Arg Glu Lys Gln Ser His Leu Thr Ile Leu Asp Tyr Leu Met
           675                 680                 685
   Asn Asp Asp Gly Leu Asn Arg Asn Leu Met Gln Leu Ile Asn Asp Ser
       690                 695                 700
   Asn Leu Ser Phe Lys Ser Ile Ile Glu Lys Glu Gln Val Ser Thr Thr
   705                 710                 715                 720
   Asp Lys Asp Leu Gln Ser Ile Val Ala Asp Leu Ala Gly Ser Pro Ala
                   725                 730                 735
   Ile Lys Lys Gly Ile Leu Gln Ser Leu Lys Ile Val Asp Glu Leu Val
               740                 745                 750
   Ser Ile Met Gly Tyr Pro Pro Gln Thr Ile Val Val Glu Met Ala Arg
           755                 760                 765
   Glu Asn Gln Thr Thr Gly Lys Gly Lys Asn Asn Ser Lys Pro Arg Tyr
       770                 775                 780
   Lys Ser Leu Glu Lys Ala Ile Lys Glu Phe Gly Ser Lys Ile Leu Lys
   785                 790                 795                 800
   Glu His Pro Thr Asp Asn Gln Glu Leu Lys Asn Asn Arg Leu Tyr Leu
                   805                 810                 815
   Tyr Tyr Leu Gln Asn Gly Lys Asp Met Tyr Thr Gly Gln Glu Leu Asp
               820                 825                 830
   Ile His Asn Leu Ser Asn Tyr Asp Ile Asp His Ile Val Pro Gln Ser
           835                 840                 845
   Phe Ile Thr Asp Asn Ser Ile Asp Asn Leu Val Leu Thr Ser Ser Ala
       850                 855                 860
   Gly Asn Arg Glu Lys Gly Gly Asp Val Pro Pro Leu Glu Ile Val Arg
   865                 870                 875                 880
   Lys Arg Lys Val Phe Trp Glu Lys Leu Tyr Gln Gly Asn Leu Met Ser
                   885                 890                 895
   Lys Arg Lys Phe Asp Tyr Leu Thr Lys Ala Glu Arg Gly Gly Leu Thr
               900                 905                 910
   Asp Ala Asp Lys Ala Arg Phe Ile His Arg Gln Leu Val Glu Thr Arg
           915                 920                 925
   Gln Ile Thr Lys Asn Val Ala Asn Ile Leu His Gln Arg Phe Asn Asn
       930                 935                 940
   Glu Thr Asp Asn His Gly Asn Thr Met Glu Gln Val Arg Ile Val Thr
   945                 950                 955                 960
   Leu Lys Ser Ala Leu Val Ser Gln Phe Arg Lys Gln Phe Gln Leu Tyr
                   965                 970                 975
   Lys Val Arg Glu Val Asn Asp Tyr His His Ala His Asp Ala Tyr Leu
               980                 985                 990
   Asn Gly Val Val Ala Asn Thr Leu Leu Lys Val Tyr Pro Gln Leu Glu
           995                 1000                1005
   Pro Glu Phe Val Tyr Gly Glu Tyr His Gln Phe Asp Trp Phe Lys Ala
       1010                1015                1020
   Asn Lys Ala Thr Ala Lys Lys Gln Phe Tyr Thr Asn Ile Met Leu Phe
   1025                1030                1035                1040
   Phe Gly Gln Lys Glu Arg Ile Ile Asp Glu Asn Gly Glu Ile Leu Trp
                   1045                1050                1055
   Asp Lys Lys Tyr Leu Glu Thr Ile Lys Lys Val Leu Asp Tyr Arg Gln
               1060                1065                1070
   Met Asn Ile Val Lys Lys Thr Glu Ile Gln Lys Gly Glu Phe Ser Lys
           1075                1080                1085
   Ala Thr Ile Lys Pro Lys Gly Asn Ser Ser Lys Leu Ile Pro Arg Lys
       1090                1095                1100
   Glu Asn Trp Asp Pro Met Lys Tyr Gly Gly Leu Asp Ser Pro Asn Met
   1105                1110                1115                1120
   Ala Tyr Ala Val Ile Ile Glu His Ala Lys Gly Lys Lys Lys Leu Ile
                   1125                1130                1135
   Phe Glu Lys Lys Ile Ile Arg Ile Thr Ile Met Glu Arg Lys Met Phe
               1140                1145                1150
   Glu Lys Asp Glu Glu Ala Phe Leu Glu Glu Lys Gly Tyr Arg His Pro
           1155                1160                1165
   Lys Val Leu Thr Lys Leu Pro Lys Tyr Thr Leu Tyr Glu Cys Glu Lys
       1170                1175                1180
   Gly Arg Arg Arg Met Leu Ala Ser Ala Asn Glu Ala Gln Lys Gly Asn
   1185                1190                1195                1200
   Gln Leu Val Leu Ser Asn His Leu Val Ser Leu Leu Tyr His Ala Lys
                   1205                1210                1215
   Asn Cys Glu Ala Ser Asp Gly Lys Ser Leu Lys Tyr Ile Glu Ala His
               1220                1225                1230
   Arg Glu Thr Phe Ser Glu Leu Leu Ala Gln Val Ser Glu Phe Ala Thr
           1235                1240                1245
   Arg Tyr Thr Leu Ala Asp Ala Asn Leu Ser Lys Ile Asn Asn Leu Phe
       1250                1255                1260
   Glu Gln Asn Lys Glu Gly Asp Ile Gln Ala Ile Ala Gln Ser Phe Val
   1265                1270                1275                1280
   Asp Leu Met Ala Phe Asn Ala Met Gly Ala Pro Ala Ser Phe Lys Phe
                   1285                1290                1295
   Phe Glu Ala Thr Ile Asp Arg Lys Arg Tyr Thr Asn Leu Lys Glu Leu
               1300                1305                1310
   Leu Ser Ser Thr Ile Ile Tyr Gln Ser Ile Thr Gly Leu Tyr Glu Ser
           1315                1320                1325
   Arg Lys Arg Leu Asp Asp
       1330
<210> 178
   <211> 984
   <212> PRT
   <213> Campylobacter jejuni
<220>
   <223> CRISPR-associated endonuclease
<308> YP_005656797
   <309> 2013-06-11
<400> 178
   Met Val Arg Ile Leu Ala Phe Asp Ile Gly Ile Ser Ser Ile Gly Trp
    1               5                  10                  15
   Ala Phe Ser Glu Asn Asp Glu Leu Lys Asp Cys Gly Val Arg Ile Phe
               20                  25                  30
   Thr Lys Val Glu Asn Pro Lys Thr Gly Glu Ser Leu Ala Leu Pro Arg
           35                  40                  45
   Arg Leu Ala Arg Ser Ala Arg Lys Arg Leu Ala Arg Arg Lys Ala Arg
       50                  55                  60
   Leu Asn His Leu Lys His Leu Ile Ala Asn Glu Phe Lys Leu Asn Tyr
   65 70 75 80
   Glu Asp Tyr Gln Ser Phe Asp Glu Ser Leu Ala Lys Ala Tyr Lys Gly
   85 90 95
   Ser Leu Ile Ser Pro Tyr Glu Leu Arg Phe Arg Ala Leu Asn Glu Leu
   100 105 110
   Leu Ser Lys Gln Asp Phe Ala Arg Val Ile Leu His Ile Ala Lys Arg
   115 120 125
   Arg Gly Tyr Asp Asp Ile Lys Asn Ser Asp Asp Lys Glu Lys Gly Ala
   130 135 140
   Ile Leu Lys Ala Ile Lys Gln Asn Glu Glu Lys Leu Ala Asn Tyr Gln
   145 150 155 160
   Ser Val Gly Glu Tyr Leu Tyr Lys Glu Tyr Phe Gln Lys Phe Lys Glu
   165 170 175
   Asn Ser Lys Glu Phe Thr Asn Val Arg Asn Lys Lys Glu Ser Tyr Glu
   180 185 190
   Arg Cys Ile Ala Gln Ser Phe Leu Lys Asp Glu Leu Lys Leu Ile Phe
   195 200 205
   Lys Lys Gln Arg Glu Phe Gly Phe Ser Phe Ser Lys Lys Phe Glu Glu
   210 215 220
   Glu Val Leu Ser Val Ala Phe Tyr Lys Arg Ala Leu Lys Asp Phe Ser
   225 230 235 240
   His Leu Val Gly Asn Cys Ser Phe Phe Thr Asp Glu Lys Arg Ala Pro
   245 250 255
   Lys Asn Ser Pro Leu Ala Phe Met Phe Val Ala Leu Thr Arg Ile Ile
   260 265 270
   Asn Leu Leu Asn Asn Leu Lys Asn Thr Glu Gly Ile Leu Tyr Thr Lys
   275 280 285
   Asp Asp Leu Asn Ala Leu Leu Asn Glu Val Leu Lys Asn Gly Thr Leu
   290 295 300
   Thr Tyr Lys Gln Thr Lys Lys Leu Leu Gly Leu Ser Asp Asp Tyr Glu
   305 310 315 320
   Phe Lys Gly Glu Lys Gly Thr Tyr Phe Ile Glu Phe Lys Lys Tyr Lys
   325 330 335
   Glu Phe Ile Lys Ala Leu Gly Glu His Asn Leu Ser Gln Asp Asp Leu
   340 345 350
   Asn Glu Ile Ala Lys Asp Ile Thr Leu Ile Lys Asp Glu Ile Lys Leu
   355 360 365
   Lys Lys Ala Leu Ala Lys Tyr Asp Leu Asn Gln Asn Gln Ile Asp Ser
   370 375 380
   Leu Ser Lys Leu Glu Phe Lys Asp His Leu Asn Ile Ser Phe Lys Ala
   385 390 395 400
   Leu Lys Leu Ile Thr Pro Leu Met Leu Glu Gly Lys Lys Tyr Asp Glu
   405 410 415
   Ala Cys Asn Glu Leu Asn Leu Lys Val Ala Ile Asn Glu Asp Lys Lys
   420 425 430
   Asp Phe Leu Pro Ala Phe Asn Glu Thr Tyr Tyr Lys Asp Glu Val Thr
   435 440 445
   Asn Pro Val Val Leu Arg Ala Ile Lys Glu Tyr Arg Lys Val Leu Asn
   450 455 460
   Ala Leu Leu Lys Lys Tyr Gly Lys Val His Lys Ile Asn Ile Glu Leu
   465 470 475 480
   Ala Arg Glu Val Gly Lys Asn His Ser Gln Arg Ala Lys Ile Glu Lys
   485 490 495
   Glu Gln Asn Glu Asn Tyr Lys Ala Lys Lys Asp Ala Glu Leu Glu Cys
   500 505 510
   Glu Lys Leu Gly Leu Lys Ile Asn Ser Lys Asn Ile Leu Lys Leu Arg
   515 520 525
   Leu Phe Lys Glu Gln Lys Glu Phe Cys Ala Tyr Ser Gly Glu Lys Ile
   530 535 540
   Lys Ile Ser Asp Leu Gln Asp Glu Lys Met Leu Glu Ile Asp His Ile
   545 550 555 560
   Tyr Pro Tyr Ser Arg Ser Phe Asp Asp Ser Tyr Met Asn Lys Val Leu
                           565                 570                 575
   Val Phe Thr Lys Gln Asn Gln Glu Lys Leu Asn Lys Thr Pro Phe Glu
                   580                 585                 590
   Ala Phe Gly Asn Asp Ser Ala Lys Trp Gln Lys Ile Glu Val Leu Ala
               595                 600                 605
   Lys Asn Leu Pro Thr Lys Lys Gln Lys Arg Ile Leu Asp Lys Asn Tyr
       610                 615                 620
   Lys Asp Lys Glu Gln Lys Asp Phe Lys Asp Arg Asn Leu Asn Asp Thr
   625                 630                 635                 640
   Arg Tyr Ile Ala Arg Leu Val Leu Asn Tyr Thr Lys Asp Tyr Leu Asp
                   645                 650                 655
   Phe Leu Pro Leu Ser Asp Asp Glu Asn Thr Lys Leu Asn Asp Thr Gln
               660                 665                 670
   Lys Gly Ser Lys Val His Val Glu Ala Lys Ser Gly Met Leu Thr Ser
           675                 680                 685
   Ala Leu Arg His Thr Trp Gly Phe Ser Thr Lys Asp Arg Asn Asn His
       690                 695                 700
   Leu His His Ala Ile Asp Ala Val Ile Ile Ala Tyr Ala Asn Asn Ser
   705                 710                 715                 720
   Ile Val Lys Ala Phe Ser Asp Phe Lys Lys Glu Gln Glu Ser Asn Ser
                   725                 730                 735
   Ala Glu Leu Tyr Ala Lys Lys Ile Ser Glu Leu Asp Tyr Lys Asn Lys
               740                 745                 750
   Arg Lys Phe Phe Glu Pro Phe Ser Gly Phe Arg Gln Lys Val Leu Asp
           755                 760                 765
   Lys Ile Asp Glu Ile Phe Val Ser Lys Pro Glu Arg Lys Lys Pro Ser
       770                 775                 780
   Gly Ala Leu His Glu Glu Thr Phe Arg Lys Glu Glu Glu Phe Tyr Gln
   785                 790                 795                 800
   Ser Tyr Gly Gly Lys Glu Gly Val Leu Lys Ala Leu Glu Leu Gly Lys
                   805                 810                 815
   Ile Arg Lys Val Asn Gly Lys Ile Val Lys Asn Gly Asp Met Phe Arg
               820                 825                 830
   Val Asp Ile Phe Lys His Lys Lys Thr Asn Lys Phe Tyr Ala Val Pro
           835                 840                 845
   Ile Tyr Thr Met Asp Phe Ala Leu Lys Val Leu Pro Asn Lys Ala Val
       850                 855                 860
   Ala Arg Ser Lys Lys Gly Glu Ile Lys Asp Trp Ile Leu Met Asp Glu
   865                 870                 875                 880
   Asn Tyr Glu Phe Cys Phe Ser Leu Tyr Lys Asp Ser Leu Ile Leu Ile
                   885                 890                 895
   Gln Thr Lys Asp Met Gln Glu Pro Glu Phe Val Tyr Tyr Asn Ala Phe
               900                 905                 910
   Thr Ser Ser Thr Val Ser Leu Ile Val Ser Lys His Asp Asn Lys Phe
           915                 920                 925
   Glu Thr Leu Ser Lys Asn Gln Lys Ile Leu Phe Lys Asn Ala Asn Glu
       930                 935                 940
   Lys Glu Val Ile Ala Lys Ser Ile Gly Ile Gln Asn Leu Lys Val Phe
   945                 950                 955                 960
   Glu Lys Tyr Ile Val Ser Ala Leu Gly Glu Val Thr Lys Ala Glu Phe
                   965                 970                 975
   Arg Gln Arg Glu Asp Phe Lys Lys
               980
<210> 179
   <211> 1367
   <212> PRT
   <213> Streptococcus pyogenes
<220>
   <223> CRISPR-associated endonuclease
<308> YP_005411537
   <309> 2013-07-06
<400> 179
   Met Asp Lys Lys Tyr Ser Ile Gly Leu Asp Ile Gly Thr Asn Ser Val
   1               5                   10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Gly Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Ala Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Ile Tyr
               180                 185                 190
   Asn Gln Leu Phe Glu Glu Asn Pro Ile Asn Ala Ser Arg Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Arg Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
   530 535 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Ala Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
   610 615 620
   Leu Phe Glu Asp Arg Gly Met Ile Glu Glu Arg Leu Lys Thr Tyr Ala
   625 630 635 640
   His Leu Phe Asp Asp Lys Val Met Lys Gln Leu Lys Arg Arg Arg Tyr
   645 650 655
   Thr Gly Trp Gly Arg Leu Ser Arg Lys Leu Ile Asn Gly Ile Arg Asp
   660 665 670
   Lys Gln Ser Gly Lys Thr Ile Leu Asp Phe Leu Lys Ser Asp Gly Phe
   675 680 685
   Ala Asn Arg Asn Phe Met Gln Leu Ile His Asp Asp Ser Leu Thr Phe
   690 695 700
   Lys Glu Asp Ile Gln Lys Ala Gln Val Ser Gly Gln Gly His Ser Leu
   705 710 715 720
   His Glu Gln Ile Ala Asn Leu Ala Gly Ser Pro Ala Ile Lys Lys Gly
   725 730 735
   Ile Leu Gln Thr Val Lys Ile Val Asp Glu Leu Val Lys Val Met Gly
   740 745 750
   His Lys Pro Glu Asn Ile Val Ile Glu Met Ala Arg Glu Asn Gln Thr
   755 760 765
   Thr Gln Lys Gly Gln Lys Asn Ser Arg Glu Arg Met Lys Arg Ile Glu
   770 775 780
   Glu Gly Ile Lys Glu Leu Gly Ser Gln Ile Leu Lys Glu His Pro Val
   785 790 795 800
   Glu Asn Thr Gln Leu Gln Asn Glu Lys Leu Tyr Leu Tyr Tyr Leu Gln
   805 810 815
   Asn Gly Arg Asp Met Tyr Val Asp Gln Glu Leu Asp Ile Asn Arg Leu
   820 825 830
   Ser Asp Tyr Asp Val Asp His Ile Val Pro Gln Ser Phe Ile Lys Asp
   835 840 845
   Asp Ser Ile Asp Asn Lys Val Leu Thr Arg Ser Asp Lys Asn Arg Gly
   850 855 860
   Lys Ser Asp Asn Val Pro Ser Glu Glu Val Val Lys Lys Met Lys Asn
   865 870 875 880
   Tyr Trp Arg Gln Leu Leu Asn Ala Lys Leu Ile Thr Gln Arg Lys Phe
   885 890 895
   Asp Asn Leu Thr Lys Ala Glu Arg Gly Gly Leu Ser Glu Leu Asp Lys
   900 905 910
   Ala Gly Phe Ile Lys Arg Gln Leu Val Glu Thr Arg Gln Ile Thr Lys
   915 920 925
   His Val Ala Gln Ile Leu Asp Ser Arg Met Asn Thr Lys Tyr Asp Glu
   930 935 940
   Asn Asp Lys Leu Ile Arg Glu Val Lys Val Ile Thr Leu Lys Ser Lys
   945 950 955 960
   Leu Val Ser Asp Phe Arg Lys Asp Phe Gln Phe Tyr Lys Val Arg Glu
   965 970 975
   Ile Asn Asn Tyr His His Ala His Asp Ala Tyr Leu Asn Ala Val Val
               980                 985                 990
   Gly Thr Ala Leu Ile Lys Lys Tyr Pro Lys Leu Glu Ser Glu Phe Val
           995                 1000                1005
   Tyr Gly Asp Tyr Lys Val Tyr Asp Val Arg Lys Met Ile Ala Lys Ser
       1010                1015                1020
   Glu Gln Glu Ile Gly Lys Ala Thr Ala Lys Tyr Phe Phe Tyr Ser Asn
   1025                1030                1035                1040
   Ile Met Asn Phe Phe Lys Thr Glu Ile Thr Leu Ala Asn Gly Glu Ile
                   1045                1050                1055
   Arg Lys Arg Pro Leu Ile Glu Thr Asn Gly Glu Thr Gly Glu Ile Val
               1060                1065                1070
   Trp Asp Lys Gly Arg Asp Phe Ala Thr Val Arg Lys Val Leu Ser Met
           1075                1080                1085
   Pro Gln Val Asn Ile Val Lys Lys Thr Glu Val Gln Thr Gly Gly Phe
       1090                1095                1100
   Ser Lys Glu Ser Ile Leu Pro Lys Arg Asn Ser Asp Lys Leu Ile Ala
   1105                1110                1115                1120
   Arg Lys Lys Asp Trp Asp Pro Lys Lys Tyr Gly Gly Phe Asp Ser Pro
                   1125                1130                1135
   Thr Val Ala Tyr Ser Val Leu Val Val Ala Lys Val Glu Lys Gly Lys
               1140                1145                1150
   Ser Lys Lys Leu Lys Ser Val Lys Glu Leu Leu Gly Ile Thr Ile Met
           1155                1160                1165
   Glu Arg Ser Ser Phe Glu Lys Asn Pro Ile Asp Phe Leu Glu Ala Lys
       1170                1175                1180
   Gly Tyr Lys Glu Val Lys Lys Asp Leu Ile Ile Lys Leu Pro Lys Tyr
   1185                1190                1195                1200
   Ser Leu Phe Glu Leu Glu Asn Gly Arg Lys Arg Met Leu Ala Ser Ala
                   1205                1210                1215
   Gly Glu Leu Gln Lys Gly Asn Glu Leu Ala Leu Pro Ser Lys Tyr Val
               1220                1225                1230
   Asn Phe Leu Tyr Leu Ala Ser His Tyr Glu Lys Leu Lys Gly Ser Pro
           1235                1240                1245
   Glu Asp Asn Glu Gln Lys Gln Leu Phe Val Glu Gln His Lys His Tyr
       1250                1255                1260
   Leu Asp Glu Ile Ile Glu Gln Ile Ser Glu Phe Ser Lys Arg Val Ile
   1265                1270                1275                1280
   Leu Ala Asp Ala Asn Leu Asp Lys Val Leu Ser Ala Tyr Asn Lys His
                   1285                1290                1295
   Arg Asp Lys Pro Ile Arg Glu Gln Ala Glu Asn Ile Ile His Leu Phe
               1300                1305                1310
   Thr Leu Thr Asn Leu Gly Ala Pro Ala Ala Phe Lys Tyr Phe Asp Thr
           1315                1320                1325
   Thr Ile Asp Arg Lys Arg Tyr Thr Ser Thr Lys Glu Val Leu Asp Ala
       1330                1335                1340
   Thr Leu Ile His Gln Ser Ile Thr Gly Leu Tyr Glu Thr Arg Ile Asp
   1345                1350                1355                1360
   Leu Ser Gln Leu Gly Gly Asp
                   1365
<210> 180
   <211> 1367
   <212> PRT
   <213> Streptococcus pyogenes
<220>
   <223> CRISPR-associated endonuclease
<308> YP_005388840
   <309> 2013-07-06
<400> 180
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Gly Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Ala Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Ile Tyr
               180                 185                 190
   Asn Gln Leu Phe Glu Glu Asn Pro Ile Asn Ala Ser Arg Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Arg Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
   530 535 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Ala Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
   610 615 620
   Leu Phe Glu Asp Arg Gly Met Ile Glu Glu Arg Leu Lys Thr Tyr Ala
   625 630 635 640
   His Leu Phe Asp Asp Lys Val Met Lys Gln Leu Lys Arg Arg Arg Tyr
   645 650 655
   Thr Gly Trp Gly Arg Leu Ser Arg Lys Leu Ile Asn Gly Ile Arg Asp
   660 665 670
   Lys Gln Ser Gly Lys Thr Ile Leu Asp Phe Leu Lys Ser Asp Gly Phe
   675 680 685
   Ala Asn Arg Asn Phe Met Gln Leu Ile His Asp Asp Ser Leu Thr Phe
   690 695 700
   Lys Glu Asp Ile Gln Lys Ala Gln Val Ser Gly Gln Gly His Ser Leu
   705 710 715 720
   His Glu Gln Ile Ala Asn Leu Ala Gly Ser Pro Ala Ile Lys Lys Gly
   725 730 735
   Ile Leu Gln Thr Val Lys Ile Val Asp Glu Leu Val Lys Val Met Gly
   740 745 750
   His Lys Pro Glu Asn Ile Val Ile Glu Met Ala Arg Glu Asn Gln Thr
   755 760 765
   Thr Gln Lys Gly Gln Lys Asn Ser Arg Glu Arg Met Lys Arg Ile Glu
   770 775 780
   Glu Gly Ile Lys Glu Leu Gly Ser Gln Ile Leu Lys Glu His Pro Val
   785 790 795 800
   Glu Asn Thr Gln Leu Gln Asn Glu Lys Leu Tyr Leu Tyr Tyr Leu Gln
   805 810 815
   Asn Gly Arg Asp Met Tyr Val Asp Gln Glu Leu Asp Ile Asn Arg Leu
   820 825 830
   Ser Asp Tyr Asp Val Asp His Ile Val Pro Gln Ser Phe Ile Lys Asp
   835 840 845
   Asp Ser Ile Asp Asn Lys Val Leu Thr Arg Ser Asp Lys Asn Arg Gly
   850 855 860
   Lys Ser Asp Asn Val Pro Ser Glu Glu Val Val Lys Lys Met Lys Asn
   865 870 875 880
   Tyr Trp Arg Gln Leu Leu Asn Ala Lys Leu Ile Thr Gln Arg Lys Phe
   885 890 895
   Asp Asn Leu Thr Lys Ala Glu Arg Gly Gly Leu Ser Glu Leu Asp Lys
   900 905 910
   Ala Gly Phe Ile Lys Arg Gln Leu Val Glu Thr Arg Gln Ile Thr Lys
   915 920 925
   His Val Ala Gln Ile Leu Asp Ser Arg Met Asn Thr Lys Tyr Asp Glu
   930 935 940
   Asn Asp Lys Leu Ile Arg Glu Val Lys Val Ile Thr Leu Lys Ser Lys
   945 950 955 960
   Leu Val Ser Asp Phe Arg Lys Asp Phe Gln Phe Tyr Lys Val Arg Glu
   965 970 975
   Ile Asn Asn Tyr His His Ala His Asp Ala Tyr Leu Asn Ala Val Val
   980 985 990
   Gly Thr Ala Leu Ile Lys Lys Tyr Pro Lys Leu Glu Ser Glu Phe Val
           995                 1000                1005
   Tyr Gly Asp Tyr Lys Val Tyr Asp Val Arg Lys Met Ile Ala Lys Ser
       1010                1015                1020
   Glu Gln Glu Ile Gly Lys Ala Thr Ala Lys Tyr Phe Phe Tyr Ser Asn
   1025                1030                1035                1040
   Ile Met Asn Phe Phe Lys Thr Glu Ile Thr Leu Ala Asn Gly Glu Ile
                   1045                1050                1055
   Arg Lys Arg Pro Leu Ile Glu Thr Asn Gly Glu Thr Gly Glu Ile Val
               1060                1065                1070
   Trp Asp Lys Gly Arg Asp Phe Ala Thr Val Arg Lys Val Leu Ser Met
           1075                1080                1085
   Pro Gln Val Asn Ile Val Lys Lys Thr Glu Val Gln Thr Gly Gly Phe
       1090                1095                1100
   Ser Lys Glu Ser Ile Leu Pro Lys Arg Asn Ser Asp Lys Leu Ile Ala
   1105                1110                1115                1120
   Arg Lys Lys Asp Trp Asp Pro Lys Lys Tyr Gly Gly Phe Asp Ser Pro
                   1125                1130                1135
   Thr Val Ala Tyr Ser Val Leu Val Val Ala Lys Val Glu Lys Gly Lys
               1140                1145                1150
   Ser Lys Lys Leu Lys Ser Val Lys Glu Leu Leu Gly Ile Thr Ile Met
           1155                1160                1165
   Glu Arg Ser Ser Phe Glu Lys Asn Pro Ile Asp Phe Leu Glu Ala Lys
       1170                1175                1180
   Gly Tyr Lys Glu Val Lys Lys Asp Leu Ile Ile Lys Leu Pro Lys Tyr
   1185                1190                1195                1200
   Ser Leu Phe Glu Leu Glu Asn Gly Arg Lys Arg Met Leu Ala Ser Ala
                   1205                1210                1215
   Gly Glu Leu Gln Lys Gly Asn Glu Leu Ala Leu Pro Ser Lys Tyr Val
               1220                1225                1230
   Asn Phe Leu Tyr Leu Ala Ser His Tyr Glu Lys Leu Lys Gly Ser Pro
           1235                1240                1245
   Glu Asp Asn Glu Gln Lys Gln Leu Phe Val Glu Gln His Lys His Tyr
       1250                1255                1260
   Leu Asp Glu Ile Ile Glu Gln Ile Ser Glu Phe Ser Lys Arg Val Ile
   1265                1270                1275                1280
   Leu Ala Asp Ala Asn Leu Asp Lys Val Leu Ser Ala Tyr Asn Lys His
                   1285                1290                1295
   Arg Asp Lys Pro Ile Arg Glu Gln Ala Glu Asn Ile Ile His Leu Phe
               1300                1305                1310
   Thr Leu Thr Asn Leu Gly Ala Pro Ala Ala Phe Lys Tyr Phe Asp Thr
           1315                1320                1325
   Thr Ile Asp Arg Lys Arg Tyr Thr Ser Thr Lys Glu Val Leu Asp Ala
       1330                1335                1340
   Thr Leu Ile His Gln Ser Ile Thr Gly Leu Tyr Glu Thr Arg Ile Asp
   1345                1350                1355                1360
   Leu Ser Gln Leu Gly Gly Asp
                   1365
<210> 181
   <211> 1084
   <212> PRT
   <213> Corynebacterium diphtheriae
<220>
   <223> CRISPR-associated endonuclease
<308> YP_005163828
   <309> 2013-06-11
<400> 181
   Met Lys Tyr His Val Gly Ile Asp Val Gly Thr Phe Ser Val Gly Leu
   1               5                   10                  15
   Ala Ala Ile Glu Val Asp Asp Ala Gly Met Pro Ile Lys Thr Leu Ser
   20 25 30
   Leu Val Ser His Ile His Asp Ser Gly Leu Asp Pro Asp Glu Ile Lys
   35 40 45
   Ser Ala Val Thr Arg Leu Ala Ser Ser Gly Ile Ala Arg Arg Thr Arg
   50 55 60
   Arg Leu Tyr Arg Arg Lys Arg Arg Arg Leu Gln Gln Leu Asp Lys Phe
   65 70 75 80
   Ile Gln Arg Gln Gly Trp Pro Val Ile Glu Leu Glu Asp Tyr Ser Asp
   85 90 95
   Pro Leu Tyr Pro Trp Lys Val Arg Ala Glu Leu Ala Ala Ser Tyr Ile
   100 105 110
   Ala Asp Glu Lys Glu Arg Gly Glu Lys Leu Ser Val Ala Leu Arg His
   115 120 125
   Ile Ala Arg His Arg Gly Trp Arg Asn Pro Tyr Ala Lys Val Ser Ser
   130 135 140
   Leu Tyr Leu Pro Asp Gly Pro Ser Asp Ala Phe Lys Ala Ile Arg Glu
   145 150 155 160
   Glu Ile Lys Arg Ala Ser Gly Gln Pro Val Pro Glu Thr Ala Thr Val
   165 170 175
   Gly Gln Met Val Thr Leu Cys Glu Leu Gly Thr Leu Lys Leu Arg Gly
   180 185 190
   Glu Gly Gly Val Leu Ser Ala Arg Leu Gln Gln Ser Asp His Ala Arg
   195 200 205
   Glu Ile Gln Glu Ile Cys Arg Met Gln Glu Ile Gly Gln Glu Leu Tyr
   210 215 220
   Arg Lys Ile Ile Asp Val Val Phe Ala Ala Glu Ser Pro Lys Gly Ser
   225 230 235 240
   Ala Ser Ser Arg Val Gly Lys Asp Pro Leu Gln Pro Gly Lys Asn Arg
   245 250 255
   Ala Leu Lys Ala Ser Asp Ala Phe Gln Arg Tyr Arg Ile Ala Ala Leu
   260 265 270
   Ile Gly Asn Leu Arg Val Trp Val Asp Gly Glu Lys Arg Ile Leu Ser
   275 280 285
   Val Glu Glu Lys Asn Leu Val Phe Asp His Leu Val Asn Leu Thr Pro
   290 295 300
   Lys Lys Glu Pro Glu Trp Val Thr Ile Ala Glu Ile Leu Gly Ile Asp
   305 310 315 320
   Arg Gly Gln Leu Ile Gly Thr Ala Thr Met Thr Asp Asp Gly Glu Arg
   325 330 335
   Ala Gly Ala Arg Pro Pro Thr His Asp Thr Asn Arg Ser Ile Val Asn
   340 345 350
   Ser Arg Ile Ala Pro Leu Val Asp Trp Trp Lys Thr Ala Ser Ala Leu
   355 360 365
   Glu Gln His Ala Met Val Lys Ala Leu Ser Asn Ala Glu Val Asp Asp
   370 375 380
   Phe Asp Ser Pro Glu Arg Ala Lys Val Gln Ala Phe Phe Ala Asp Leu
   385 390 395 400
   Asp Asp Asp Val His Ala Lys Leu Asp Ser Leu His Leu Pro Val Gly
   405 410 415
   Arg Ala Ala Tyr Ser Glu Asp Thr Leu Val Arg Leu Thr Arg Arg Met
   420 425 430
   Leu Ser Asp Gly Val Asp Leu Tyr Thr Ala Arg Leu Gln Glu Phe Gly
   435 440 445
   Ile Glu Pro Ser Trp Thr Pro Pro Thr Pro Arg Ile Gly Glu Pro Val
   450 455 460
   Gly Asn Pro Ala Val Asp Arg Val Leu Lys Thr Val Ser Arg Trp Leu
   465 470 475 480
   Glu Ser Ala Thr Lys Thr Trp Gly Ala Pro Glu Arg Val Ile Ile Glu
   485 490 495
   His Val Arg Glu Gly Phe Val Thr Glu Lys Arg Ala Arg Glu Met Asp
   500 505 510
   Gly Asp Met Arg Arg Arg Ala Ala Arg Asn Ala Lys Leu Phe Gln Glu
               515                 520                 525
   Met Gln Glu Lys Leu Asn Val Gln Gly Lys Pro Ser Arg Ala Asp Leu
           530                 535                 540
   Trp Arg Tyr Gln Ser Val Gln Arg Gln Asn Cys Gln Cys Ala Tyr Cys
   545                 550                 555                 560
   Gly Ser Pro Ile Thr Phe Ser Asn Ser Glu Met Asp His Ile Val Pro
                   565                 570                 575
   Arg Ala Gly Gln Gly Ser Thr Asn Thr Arg Glu Asn Leu Val Ala Val
               580                 585                 590
   Cys His Arg Cys Asn Gln Ser Lys Gly Asn Thr Pro Phe Ala Ile Trp
           595                 600                 605
   Ala Lys Asn Thr Ser Ile Glu Gly Val Ser Val Lys Glu Ala Val Glu
       610                 615                 620
   Arg Thr Arg His Trp Val Thr Asp Thr Gly Met Arg Ser Thr Asp Phe
   625                 630                 635                 640
   Lys Lys Phe Thr Lys Ala Val Val Glu Arg Phe Gln Arg Ala Thr Met
                   645                 650                 655
   Asp Glu Glu Ile Asp Ala Arg Ser Met Glu Ser Val Ala Trp Met Ala
               660                 665                 670
   Asn Glu Leu Arg Ser Arg Val Ala Gln His Phe Ala Ser His Gly Thr
           675                 680                 685
   Thr Val Arg Val Tyr Arg Gly Ser Leu Thr Ala Glu Ala Arg Arg Ala
       690                 695                 700
   Ser Gly Ile Ser Gly Lys Leu Glu Phe Leu Asp Gly Val Gly Lys Ser
   705                 710                 715                 720
   Arg Leu Asp Arg Arg His His Ala Ile Asp Ala Ala Val Ile Ala Phe
                   725                 730                 735
   Thr Ser Asp Tyr Val Ala Glu Thr Leu Ala Val Arg Ser Asn Leu Lys
               740                 745                 750
   Gln Ser Gln Ala His Arg Gln Glu Ala Pro Gln Trp Arg Glu Phe Thr
           755                 760                 765
   Gly Lys Asp Ala Glu His Arg Ala Thr Trp Arg Val Trp Cys Gln Lys
       770                 775                 780
   Met Glu Lys Leu Ser Ala Leu Leu Thr Glu Asp Leu Arg Asp Asp Arg
   785                 790                 795                 800
   Val Val Val Met Ser Asn Val Arg Leu Arg Leu Gly Asn Gly Ser Ala
                   805                 810                 815
   His Glu Glu Thr Ile Gly Lys Leu Ser Lys Val Lys Leu Gly Ser Gln
               820                 825                 830
   Leu Ser Val Ser Asp Ile Asp Lys Ala Ser Ser Glu Ala Leu Trp Cys
           835                 840                 845
   Ala Leu Thr Arg Glu Pro Asp Phe Asp Pro Lys Asp Gly Leu Pro Ala
       850                 855                 860
   Asn Pro Glu Arg His Ile Arg Val Asn Gly Thr His Val Tyr Ala Gly
   865                 870                 875                 880
   Asp Asn Ile Gly Leu Phe Pro Val Ser Ala Gly Ser Ile Ala Leu Arg
                   885                 890                 895
   Gly Gly Tyr Ala Glu Leu Gly Ser Ser Phe His His Ala Arg Val Tyr
               900                 905                 910
   Lys Ile Thr Ser Gly Lys Lys Pro Ala Phe Ala Met Leu Arg Val Tyr
           915                 920                 925
   Thr Ile Asp Leu Leu Pro Tyr Arg Asn Gln Asp Leu Phe Ser Val Glu
       930                 935                 940
   Leu Lys Pro Gln Thr Met Ser Met Arg Gln Ala Glu Lys Lys Leu Arg
   945                 950                 955                 960
   Asp Ala Leu Ala Thr Gly Asn Ala Glu Tyr Leu Gly Trp Leu Val Val
                   965                 970                 975
   Asp Asp Glu Leu Val Val Asp Thr Ser Lys Ile Ala Thr Asp Gln Val
               980                 985                 990
   Lys Ala Val Glu Ala Glu Leu Gly Thr Ile Arg Arg Trp Arg Val Asp
           995                 1000                1005
   Gly Phe Phe Gly Asp Thr Arg Leu Arg Leu Arg Pro Leu Gln Met Ser
       1010                1015                1020
   Lys Glu Gly Ile Lys Lys Glu Ser Ala Pro Glu Leu Ser Lys Ile Ile
   1025                1030                1035                1040
   Asp Arg Pro Gly Trp Leu Pro Ala Val Asn Lys Leu Phe Ser Glu Gly
                   1045                1050                1055
   Asn Val Thr Val Val Arg Arg Asp Ser Leu Gly Arg Val Arg Leu Glu
               1060                1065                1070
   Ser Thr Ala His Leu Pro Val Thr Trp Lys Val Gln
           1075                1080
<210> 182
   <211> 1084
   <212> PRT
   <213> Corynebacterium diphtheriae
<220>
   <223> CRISPR-associated endonuclease
<308> YP_005161490
   <309> 2013-06-11
<400> 182
   Met Lys Tyr His Val Gly Ile Asp Val Gly Thr Phe Ser Val Gly Leu
   1               5                   10                  15
   Ala Ala Ile Glu Val Asp Asp Ala Gly Met Pro Ile Lys Thr Leu Ser
               20                  25                  30
   Leu Val Ser His Ile His Asp Ser Gly Leu Asp Pro Asp Lys Ile Lys
           35                  40                  45
   Ser Ala Val Thr Arg Leu Ala Ser Ser Gly Ile Ala Arg Arg Thr Arg
       50                  55                  60
   Arg Leu Tyr Arg Arg Lys Arg Arg Arg Leu Gln Gln Leu Asp Lys Phe
   65                  70                  75                  80
   Ile Gln Arg Gln Gly Trp Pro Val Ile Glu Leu Glu Asp Tyr Ser Asp
                   85                  90                  95
   Pro Leu Tyr Pro Trp Lys Val Arg Ala Glu Leu Ala Ala Ser Tyr Ile
               100                 105                 110
   Ala Asp Glu Lys Glu Arg Gly Glu Lys Leu Ser Val Ala Leu Arg His
           115                 120                 125
   Ile Ala Arg His Arg Gly Trp Arg Asn Pro Tyr Ala Lys Val Ser Ser
       130                 135                 140
   Leu Tyr Leu Pro Asp Glu Pro Ser Asp Ala Phe Lys Ala Ile Arg Glu
   145                 150                 155                 160
   Glu Ile Lys Arg Ala Ser Gly Gln Pro Val Pro Glu Thr Ala Thr Val
                   165                 170                 175
   Gly Gln Met Val Thr Leu Cys Glu Leu Gly Thr Leu Lys Leu Arg Gly
               180                 185                 190
   Glu Gly Gly Val Leu Ser Ala Arg Leu Gln Gln Ser Asp His Ala Arg
           195                 200                 205
   Glu Ile Gln Glu Ile Cys Arg Met Gln Glu Ile Gly Gln Glu Leu Tyr
       210                 215                 220
   Arg Lys Ile Ile Asp Val Val Phe Ala Ala Glu Ser Pro Lys Gly Ser
   225                 230                 235                 240
   Ala Ser Ser Arg Val Gly Lys Asp Pro Leu Gln Pro Gly Lys Asn Arg
                   245                 250                 255
   Ala Leu Lys Ala Ser Asp Ala Phe Gln Arg Tyr Arg Ile Ala Ala Leu
               260                 265                 270
   Ile Gly Asn Leu Arg Val Arg Val Asp Gly Glu Lys Arg Ile Leu Ser
           275                 280                 285
   Val Glu Glu Lys Asn Leu Val Phe Asp His Leu Val Asn Leu Ala Pro
       290                 295                 300
   Lys Lys Glu Pro Glu Trp Val Thr Ile Ala Glu Ile Leu Gly Ile Asp
   305                 310                 315                 320
   Arg Gly Gln Leu Ile Gly Thr Ala Thr Met Thr Asp Asp Gly Glu Arg
                   325                 330                 335
   Ala Gly Ala Arg Pro Pro Thr His Asp Thr Asn Arg Ser Ile Val Asn
               340                 345                 350
   Ser Arg Ile Ala Pro Leu Val Asp Trp Trp Lys Thr Ala Ser Ala Leu
           355                 360                 365
   Glu Gln His Ala Met Val Lys Ala Leu Ser Asn Ala Glu Val Asp Asp
       370                 375                 380
   Phe Asp Ser Pro Glu Gly Ala Lys Val Gln Ala Phe Phe Ala Asp Leu
   385                 390                 395                 400
   Asp Asp Asp Val His Ala Lys Leu Asp Ser Leu His Leu Pro Val Gly
                   405                 410                 415
   Arg Ala Ala Tyr Ser Glu Asp Thr Leu Val Arg Leu Thr Arg Arg Met
               420                 425                 430
   Leu Ala Asp Gly Val Asp Leu Tyr Thr Ala Arg Leu Gln Glu Phe Gly
           435                 440                 445
   Ile Glu Pro Ser Trp Thr Pro Pro Ala Pro Arg Ile Gly Glu Pro Val
       450                 455                 460
   Gly Asn Pro Ala Val Asp Arg Val Leu Lys Thr Val Ser Arg Trp Leu
   465                 470                 475                 480
   Glu Ser Ala Thr Lys Thr Trp Gly Ala Pro Glu Arg Val Ile Ile Glu
                   485                 490                 495
   His Val Arg Glu Gly Phe Val Thr Glu Lys Arg Ala Arg Glu Met Asp
               500                 505                 510
   Gly Asp Met Arg Arg Arg Ala Ala Arg Asn Ala Lys Leu Phe Gln Glu
           515                 520                 525
   Met Gln Glu Lys Leu Asn Val Gln Gly Lys Pro Ser Arg Ala Asp Leu
       530                 535                 540
   Trp Arg Tyr Gln Ser Val Gln Arg Gln Asn Cys Gln Cys Ala Tyr Cys
   545                 550                 555                 560
   Gly Ser Pro Ile Thr Phe Ser Asn Ser Glu Met Asp His Ile Val Pro
                   565                 570                 575
   Arg Ala Gly Gln Gly Ser Thr Asn Thr Arg Glu Asn Leu Val Ala Val
               580                 585                 590
   Cys His Arg Cys Asn Gln Ser Lys Gly Asn Thr Pro Phe Ala Ile Trp
           595                 600                 605
   Ala Lys Asn Thr Ser Ile Glu Gly Val Ser Val Lys Glu Ala Val Glu
       610                 615                 620
   Arg Thr Arg His Trp Val Thr Asp Thr Gly Met Arg Ser Thr Asp Phe
   625                 630                 635                 640
   Lys Lys Phe Thr Lys Ala Val Val Glu Arg Phe Gln Arg Ala Thr Met
                   645                 650                 655
   Asp Glu Glu Ile Asp Ala Arg Ser Met Glu Ser Val Ala Trp Met Ala
               660                 665                 670
   Asn Glu Leu Arg Ser Arg Val Ala Gln His Phe Ala Ser His Gly Thr
           675                 680                 685
   Thr Val Arg Val Tyr Arg Gly Ser Leu Thr Ala Glu Ala Arg Arg Ala
       690                 695                 700
   Ser Gly Ile Ser Gly Lys Leu Glu Phe Leu Asp Gly Val Gly Lys Ser
   705                 710                 715                 720
   Arg Leu Asp Arg Arg His His Ala Ile Asp Ala Ala Val Ile Ala Phe
                   725                 730                 735
   Thr Ser Asp Tyr Val Ala Glu Thr Leu Ala Val Arg Ser Asn Leu Lys
               740                 745                 750
   Gln Ser Gln Ala His Arg Gln Glu Ala Pro Gln Trp Arg Glu Phe Thr
           755                 760                 765
   Gly Lys Asp Ala Glu His Arg Ala Ala Trp Arg Val Trp Cys Gln Lys
       770                 775                 780
   Met Glu Lys Leu Ser Ala Leu Leu Thr Glu Asp Leu Arg Asp Asp Arg
   785                 790                 795                 800
   Val Val Val Met Ser Asn Val Arg Leu Arg Leu Gly Asn Gly Ser Ala
                   805                 810                 815
   His Glu Glu Thr Ile Gly Lys Leu Ser Lys Val Lys Leu Gly Ser Gln
               820                 825                 830
   Leu Ser Val Ser Asp Ile Asp Lys Ala Ser Ser Glu Ala Leu Trp Cys
           835                 840                 845
   Ala Leu Thr Arg Glu Pro Asp Phe Asp Pro Lys Asp Gly Leu Pro Ala
       850                 855                 860
   Asn Pro Glu Arg His Ile Arg Val Asn Gly Thr His Val Tyr Ala Gly
   865                 870                 875                 880
   Asp Asn Ile Gly Leu Phe Pro Val Ser Ala Gly Ser Ile Ala Leu Arg
                   885                 890                 895
   Gly Gly Tyr Ala Glu Leu Gly Ser Ser Phe His His Ala Arg Val Tyr
               900                 905                 910
   Lys Ile Thr Ser Gly Lys Lys Pro Ala Phe Ala Met Leu Arg Val Tyr
           915                 920                 925
   Thr Ile Asp Leu Leu Pro Tyr Arg Asn Gln Asp Leu Phe Ser Val Glu
       930                 935                 940
   Leu Lys Pro Gln Thr Met Ser Met Arg Gln Ala Glu Lys Lys Leu Arg
   945                 950                 955                 960
   Asp Ala Leu Ala Thr Gly Asn Ala Glu Tyr Leu Gly Trp Leu Val Val
                   965                 970                 975
   Asp Asp Glu Leu Val Val Asp Thr Ser Lys Ile Ala Thr Asp Gln Val
               980                 985                 990
   Lys Ala Val Glu Ala Glu Leu Gly Thr Ile Arg Arg Trp Arg Val Asp
           995                 1000                1005
   Gly Phe Phe Gly Asp Thr Arg Leu Arg Leu Arg Pro Leu Gln Met Ser
       1010                1015                1020
   Lys Glu Gly Ile Lys Lys Glu Ser Ala Pro Glu Leu Ser Lys Ile Ile
   1025                1030                1035                1040
   Asp Arg Pro Gly Trp Leu Pro Ala Val Asn Lys Leu Phe Ser Glu Gly
                   1045                1050                1055
   Asn Val Thr Val Val Arg Arg Asp Ser Leu Gly Arg Val Arg Leu Glu
               1060                1065                1070
   Ser Thr Ala His Leu Pro Val Thr Trp Lys Val Gln
           1075                1080
<210> 183
   <211> 1084
   <212> PRT
   <213> Corynebacterium diphtheriae
<220>
   <223> CRISPR-associated endonuclease
<308> YP_005159132
   <309> 2013-06-11
<400> 183
   Met Lys Tyr His Val Gly Ile Asp Val Gly Thr Phe Ser Val Gly Leu
    1               5                   10                  15
   Ala Ala Ile Glu Val Asp Asp Ala Gly Met Pro Ile Lys Thr Leu Ser
               20                  25                  30
   Leu Val Ser His Ile His Asp Ser Arg Leu Asp Pro Asp Lys Ile Lys
           35                  40                  45
   Ser Ala Val Thr Arg Leu Ala Ser Ser Gly Ile Ala Arg Arg Thr Arg
       50                  55                  60
   Arg Leu Tyr Arg Arg Lys Arg Arg Arg Leu Gln Gln Leu Asp Lys Phe
   65                  70                  75                  80
   Ile Gln Arg Gln Gly Trp Pro Val Ile Glu Leu Glu Asp Tyr Ser Asp
                   85                  90                  95
   Pro Leu Tyr Pro Trp Lys Val Arg Ala Glu Leu Ala Ala Ser Tyr Ile
               100                 105                 110
   Ala Asp Glu Lys Glu Arg Gly Glu Lys Leu Ser Val Ala Leu Arg His
           115                 120                 125
   Ile Ala Arg His Arg Gly Trp Arg Asn Pro Tyr Ala Lys Val Ser Ser
           130                 135                 140
   Leu Tyr Leu Pro Asp Glu Pro Ser Asp Ala Phe Lys Ala Ile Arg Glu
   145                 150                 155                 160
   Glu Ile Lys Arg Ala Ser Gly Gln Pro Val Pro Glu Thr Ala Thr Val
                   165                 170                 175
   Gly Gln Met Val Thr Leu Cys Glu Leu Gly Thr Leu Lys Leu Arg Gly
               180                 185                 190
   Glu Gly Gly Val Leu Ser Ala Arg Leu Gln Gln Ser Asp His Ala Arg
           195                 200                 205
   Glu Ile Gln Glu Ile Cys Arg Met Gln Glu Ile Gly Gln Glu Leu Tyr
       210                 215                 220
   Arg Lys Ile Ile Asp Val Val Phe Ala Ala Glu Ser Pro Lys Gly Ser
   225                 230                 235                 240
   Ala Ser Ser Arg Val Gly Lys Asp Pro Leu Gln Pro Gly Lys Asn Arg
                   245                 250                 255
   Ala Leu Lys Ala Ser Asp Ala Phe Gln Arg Tyr Arg Ile Ala Ala Leu
               260                 265                 270
   Ile Gly Asn Leu Arg Val Arg Val Asp Gly Glu Lys Arg Ile Leu Ser
           275                 280                 285
   Val Glu Glu Lys Asn Leu Val Phe Asp His Leu Val Asn Leu Ala Pro
       290                 295                 300
   Lys Lys Glu Pro Glu Trp Val Thr Ile Ala Glu Ile Leu Gly Ile Asp
   305                 310                 315                 320
   Arg Gly Gln Leu Ile Gly Thr Ala Thr Met Thr Asp Asp Gly Glu Arg
                   325                 330                 335
   Ala Gly Ala Arg Pro Pro Thr His Asp Thr Asn Arg Ser Ile Val Asn
               340                 345                 350
   Ser Arg Ile Ala Pro Leu Val Asp Trp Trp Lys Thr Ala Ser Ala Leu
           355                 360                 365
   Glu Gln His Ala Met Val Lys Ala Leu Ser Asn Ala Glu Val Asp Asp
       370                 375                 380
   Phe Asp Ser Pro Glu Gly Ala Lys Val Gln Ala Phe Phe Ala Asp Leu
   385                 390                 395                 400
   Asp Asp Asp Val His Ala Lys Leu Asp Ser Leu His Leu Pro Val Gly
                   405                 410                 415
   Arg Ala Ala Tyr Ser Glu Asp Thr Leu Val Arg Leu Thr Arg Arg Met
               420                 425                 430
   Leu Ala Asp Gly Val Asp Leu Tyr Thr Ala Arg Leu Gln Glu Phe Gly
           435                 440                 445
   Ile Glu Pro Ser Trp Thr Pro Pro Ala Pro Arg Ile Gly Glu Pro Val
       450                 455                 460
   Gly Asn Pro Ala Val Asp Arg Val Leu Lys Thr Val Ser Arg Trp Leu
   465                 470                 475                 480
   Glu Ser Ala Thr Lys Thr Trp Gly Ala Pro Glu Arg Val Ile Ile Glu
                   485                 490                 495
   His Val Arg Glu Gly Phe Val Thr Glu Lys Arg Ala Arg Glu Met Asp
               500                 505                 510
   Gly Asp Met Arg Arg Arg Ala Ala Arg Asn Ala Lys Leu Phe Gln Glu
           515                 520                 525
   Met Gln Glu Lys Leu Asn Ile Gln Gly Lys Pro Ser Arg Ala Asp Leu
       530                 535                 540
   Trp Arg Tyr Gln Ser Val Gln Arg Gln Asn Cys Gln Cys Ala Tyr Cys
   545                 550                 555                 560
   Gly Ser Pro Ile Thr Phe Ser Asn Ser Glu Met Asp His Ile Val Pro
                   565                 570                 575
   Arg Ala Gly Gln Gly Ser Thr Asn Thr Arg Glu Asn Leu Val Ala Val
               580                 585                 590
   Cys His Arg Cys Asn Gln Ser Lys Gly Asn Thr Pro Phe Ala Ile Trp
           595                 600                 605
   Ala Lys Asn Thr Ser Ile Glu Gly Val Ser Val Lys Glu Ala Val Glu
       610                 615                 620
   Arg Thr Arg His Trp Val Thr Asp Thr Gly Met Arg Ser Thr Asp Phe
   625                 630                 635                 640
   Lys Lys Phe Thr Lys Ala Val Val Glu Arg Phe Gln Arg Val Thr Met
   645 650 655
   Asp Glu Glu Ile Asp Ala Arg Ser Met Glu Ser Val Ala Trp Met Ala
   660 665 670
   Asn Glu Leu Arg Ser Arg Val Ala Gln His Phe Ala Ser Arg Gly Thr
   675 680 685
   Thr Val Arg Val Tyr Arg Gly Ser Leu Thr Ala Glu Ala Arg Arg Ala
   690 695 700
   Ser Gly Ile Ser Gly Lys Leu Glu Phe Leu Asp Gly Val Gly Lys Ser
   705 710 715 720
   Arg Leu Asp Arg Arg His His Ala Ile Asp Ala Ala Val Ile Ala Phe
   725 730 735
   Thr Ser Asp Tyr Val Ala Glu Thr Leu Ala Val Arg Ser Asn Leu Lys
   740 745 750
   Gln Ser Gln Ala His Arg Gln Glu Ala Pro Gln Trp Arg Glu Phe Thr
   755 760 765
   Gly Lys Asp Ala Glu His Arg Ala Ala Trp Arg Val Trp Cys Gln Lys
   770 775 780
   Met Glu Lys Leu Ser Ala Leu Leu Thr Glu Asp Leu Arg Asp Asp Arg
   785 790 795 800
   Val Val Val Met Ser Asn Val Arg Leu Arg Leu Gly Asn Gly Ser Ala
   805 810 815
   His Glu Glu Thr Ile Gly Lys Leu Ser Lys Val Lys Leu Gly Ser Gln
   820 825 830
   Leu Ser Val Ser Asp Ile Asp Lys Ala Ser Ser Glu Ala Leu Trp Cys
   835 840 845
   Ala Leu Thr Arg Glu Pro Asp Phe Asp Pro Lys Asp Gly Leu Pro Ala
   850 855 860
   Asn Pro Glu Arg His Ile Arg Val Asn Gly Thr His Val Tyr Ala Gly
   865 870 875 880
   Asp Asn Ile Gly Leu Phe Pro Val Ser Ala Gly Ser Ile Ala Leu Arg
   885 890 895
   Gly Gly Tyr Ala Glu Leu Gly Ser Ser Phe His His Ala Arg Val Tyr
   900 905 910
   Lys Ile Thr Ser Gly Lys Lys Pro Ala Phe Ala Met Leu Arg Val Tyr
   915 920 925
   Thr Ile Asp Leu Leu Pro Tyr Arg Asn Gln Asp Leu Phe Ser Val Glu
   930 935 940
   Leu Lys Pro Gln Thr Met Ser Met Arg Gln Ala Glu Lys Lys Leu Arg
   945 950 955 960
   Asp Ala Leu Ala Thr Gly Asn Ala Glu Tyr Leu Gly Trp Leu Val Val
   965 970 975
   Asp Asp Glu Leu Val Val Asp Thr Ser Lys Ile Ala Thr Asp Gln Val
   980 985 990
   Lys Ala Val Glu Ala Glu Leu Gly Thr Ile Arg Arg Trp Arg Val Asp
   995 1000 1005
   Gly Phe Phe Gly Asp Thr Arg Leu Arg Leu Arg Pro Leu Gln Met Ser
   1010 1015 1020
   Lys Glu Gly Ile Lys Lys Glu Ser Ala Pro Glu Leu Ser Lys Ile Ile
   1025 1030 1035 1040
   Asp Arg Pro Gly Trp Leu Pro Ala Val Asn Lys Leu Phe Ser Glu Gly
   1045 1050 1055
   Asn Val Thr Val Val Arg Arg Asp Ser Leu Gly Arg Val Arg Leu Glu
   1060 1065 1070
   Ser Thr Ala His Leu Pro Val Thr Trp Lys Val Gln
   1075 1080
<210> 184
   <211> 1084
   <212> PRT
   <213> Corynebacterium diphtheriae
<220>
   <223> CRISPR-associated endonuclease
<308> YP_005156749
   <309> 2013-08-28
<400> 184
   Met Lys Tyr His Val Gly Ile Asp Val Gly Thr Phe Ser Val Gly Leu
   1               5                   10                  15
   Ala Ala Ile Glu Val Asp Asp Ala Gly Met Pro Ile Lys Thr Leu Ser
               20                  25                  30
   Leu Val Ser His Ile His Asp Ser Gly Leu Asp Pro Asp Lys Met Lys
           35                  40                  45
   Ser Ala Val Thr Arg Leu Ala Ser Ser Gly Ile Ala Arg Arg Thr Arg
       50                  55                  60
   Arg Leu Tyr Arg Arg Lys Arg Arg Arg Leu Gln Gln Leu Asp Lys Phe
   65                  70                  75                  80
   Ile Gln Arg Gln Gly Trp Pro Val Ile Glu Leu Glu Asp Tyr Ser Asp
                   85                  90                  95
   Pro Leu Tyr Pro Trp Lys Val Arg Ala Glu Leu Ala Ala Ser Tyr Ile
               100                 105                 110
   Ala Asp Glu Lys Glu Arg Gly Glu Lys Leu Ser Val Ala Leu Arg His
           115                 120                 125
   Ile Ala Arg His Arg Gly Trp Arg Asn Pro Tyr Ala Lys Val Ser Ser
       130                 135                 140
   Leu Tyr Leu Pro Asp Glu Pro Ser Asp Ala Phe Lys Ser Ile Arg Glu
   145                 150                 155                 160
   Glu Ile Lys Arg Ala Ser Gly Gln Pro Val Pro Glu Thr Ala Thr Val
                   165                 170                 175
   Gly Gln Met Val Thr Leu Cys Glu Leu Gly Thr Leu Lys Leu Arg Gly
               180                 185                 190
   Glu Gly Gly Val Leu Ser Ala Arg Leu Gln Gln Ser Asp His Ala Arg
           195                 200                 205
   Glu Ile Gln Glu Ile Cys Arg Met Gln Glu Ile Gly Gln Glu Leu Tyr
       210                 215                 220
   Arg Lys Ile Ile Asp Val Val Phe Ala Ala Glu Ser Pro Lys Gly Ser
   225                 230                 235                 240
   Ala Ser Ser Arg Val Gly Lys Asp Pro Leu Gln Pro Gly Lys Asn Arg
                   245                 250                 255
   Ala Leu Lys Ala Ser Asp Ala Phe Gln Arg Tyr Arg Ile Ala Ala Leu
               260                 265                 270
   Ile Gly Asn Leu Arg Val Arg Val Asp Gly Glu Lys Arg Ile Leu Ser
           275                 280                 285
   Val Glu Glu Lys Asn Leu Val Phe Asp His Leu Val Asn Leu Ala Pro
       290                 295                 300
   Lys Lys Glu Pro Glu Trp Val Thr Ile Ala Glu Ile Leu Gly Ile Asp
   305                 310                 315                 320
   Arg Gly Gln Leu Ile Gly Thr Ala Thr Met Thr Asp Asp Gly Glu Arg
                   325                 330                 335
   Ala Gly Ala Arg Pro Pro Thr His Asp Thr Asn Arg Ser Ile Val Asn
               340                 345                 350
   Ser Arg Ile Ala Pro Leu Val Asp Trp Trp Lys Thr Ala Ser Ala Leu
           355                 360                 365
   Glu Gln His Ala Met Val Lys Ala Leu Ser Asn Ala Glu Val Asp Asp
       370                 375                 380
   Phe Asp Ser Pro Glu Gly Ala Lys Val Gln Ala Phe Phe Ala Asp Leu
   385                 390                 395                 400
   Asp Asp Asp Val His Ala Lys Leu Asp Ser Leu His Leu Pro Val Gly
                   405                 410                 415
   Arg Ala Ala Tyr Ser Glu Asp Thr Leu Val Arg Leu Thr Arg Arg Met
               420                 425                 430
   Leu Ala Asp Gly Val Asp Leu Tyr Thr Ala Arg Leu Gln Glu Phe Gly
           435                 440                 445
   Ile Glu Pro Ser Trp Thr Pro Pro Ala Pro Arg Ile Gly Glu Pro Val
   450 455 460
   Gly Asn Pro Ala Val Asp Arg Val Leu Lys Thr Val Ser Arg Trp Leu
   465 470 475 480
   Glu Ser Ala Thr Lys Thr Trp Gly Ala Pro Glu Arg Val Ile Ile Glu
   485 490 495
   His Val Arg Glu Gly Phe Val Thr Glu Lys Arg Ala Arg Glu Met Asp
   500 505 510
   Gly Asp Met Arg Arg Arg Ala Ala Arg Asn Ala Lys Leu Phe Gln Glu
   515 520 525
   Met Gln Glu Lys Leu Asn Val Gln Gly Lys Pro Ser Arg Ala Asp Leu
   530 535 540
   Trp Arg Tyr Gln Ser Val Gln Arg Gln Asn Cys Gln Cys Ala Tyr Cys
   545 550 555 560
   Gly Ser Pro Ile Thr Phe Ser Asn Ser Glu Met Asp His Ile Val Pro
   565 570 575
   Arg Ala Gly Gln Gly Ser Thr Asn Thr Arg Glu Asn Leu Val Ala Val
   580 585 590
   Cys His Arg Cys Asn Gln Ser Lys Gly Asn Thr Pro Phe Ala Ile Trp
   595 600 605
   Ala Lys Asn Thr Ser Ile Glu Gly Val Ser Val Lys Glu Ala Val Glu
   610 615 620
   Arg Thr Arg His Trp Val Thr Asp Thr Gly Met Arg Ser Thr Asp Phe
   625 630 635 640
   Lys Lys Phe Thr Lys Ala Val Val Glu Arg Phe Gln Arg Ala Thr Met
   645 650 655
   Asp Glu Glu Ile Asp Ala Arg Ser Met Glu Ser Val Ala Trp Met Ala
   660 665 670
   Asn Glu Leu Arg Ser Arg Val Ala Gln His Phe Ala Ser His Gly Thr
   675 680 685
   Thr Val Arg Val Tyr Arg Gly Ser Leu Thr Ala Glu Ala Arg Arg Ala
   690 695 700
   Ser Gly Ile Ser Gly Lys Leu Glu Phe Leu Asp Gly Val Gly Lys Ser
   705 710 715 720
   Arg Leu Asp Arg Arg His His Ala Ile Asp Ala Ala Val Ile Ala Phe
   725 730 735
   Thr Ser Asp Tyr Val Ala Glu Thr Leu Ala Val Arg Ser Asn Leu Lys
   740 745 750
   Gln Ser Gln Ala His Arg Gln Glu Ala Pro Gln Trp Arg Glu Phe Thr
   755 760 765
   Gly Lys Asp Ala Glu His Arg Ala Ala Trp Arg Val Trp Cys Gln Lys
   770 775 780
   Met Glu Lys Leu Ser Ala Leu Leu Thr Glu Asp Leu Arg Asp Asp Arg
   785 790 795 800
   Val Val Val Met Ser Asn Val Arg Leu Arg Leu Gly Asn Gly Ser Ala
   805 810 815
   His Glu Glu Thr Ile Gly Lys Leu Ser Lys Val Lys Leu Gly Ser Gln
   820 825 830
   Leu Ser Val Ser Asp Ile Asp Lys Ala Ser Ser Glu Ala Leu Trp Cys
   835 840 845
   Ala Leu Thr Arg Glu Pro Asp Phe Asp Pro Lys Asp Gly Leu Pro Ala
   850 855 860
   Asn Pro Glu Arg His Ile Arg Val Asn Gly Thr His Val Tyr Ala Gly
   865 870 875 880
   Asp Asn Ile Gly Leu Phe Pro Val Ser Ala Gly Ser Ile Ala Leu Arg
   885 890 895
   Gly Gly Tyr Ala Glu Leu Gly Ser Ser Phe His His Ala Arg Val Tyr
   900 905 910
   Lys Ile Thr Ser Gly Lys Lys Pro Ala Phe Ala Met Leu Arg Val Tyr
   915 920 925
   Thr Ile Asp Leu Leu Pro Tyr Arg Asn Gln Asp Leu Phe Ser Val Glu
   930 935 940
   Leu Lys Pro Gln Thr Met Ser Met Arg Gln Ala Glu Lys Lys Leu Arg
   945                 950                 955                 960
   Asp Ala Leu Ala Thr Gly Asn Ala Glu Tyr Leu Gly Trp Leu Val Val
                   965                 970                 975
   Asp Asp Glu Leu Val Val Asp Thr Ser Lys Ile Ala Thr Asp Gln Val
               980                 985                 990
   Lys Ala Val Glu Ala Glu Leu Gly Thr Ile Arg Arg Trp Arg Val Asp
           995                 1000                1005
   Gly Phe Phe Gly Asp Thr Arg Leu Arg Leu Arg Pro Leu Gln Met Ser
       1010                1015                1020
   Lys Glu Gly Ile Lys Lys Glu Ser Ala Pro Glu Leu Ser Lys Ile Ile
   1025                1030                1035                1040
   Asp Arg Pro Gly Trp Leu Pro Ala Val Asn Lys Leu Phe Ser Glu Gly
                   1045                1050                1055
   Asn Val Thr Val Val Arg Arg Asp Ser Leu Gly Arg Val Arg Leu Glu
               1060                1065                1070
   Ser Thr Ala His Leu Pro Val Thr Trp Lys Val Gln
           1075                1080
<210> 185
   <211> 1084
   <212> PRT
   <213> Corynebacterium diphtheriae
<220>
   <223> CRISPR-associated endonuclease
<308> YP_005143942
   <309> 2013-06-11
<400> 185
   Met Lys Tyr His Val Gly Ile Asp Val Gly Thr Phe Ser Val Gly Leu
    1               5                  10                  15
   Ala Ala Ile Glu Val Asp Asp Ala Gly Met Pro Ile Lys Thr Leu Ser
               20                  25                  30
   Leu Val Ser His Ile His Asp Ser Gly Leu Asp Pro Asp Lys Met Lys
           35                  40                  45
   Ser Ala Val Thr Arg Leu Ala Ser Ser Gly Ile Ala Arg Arg Thr Arg
       50                  55                  60
   Arg Leu Tyr Arg Arg Lys Arg Arg Arg Leu Gln Gln Leu Asp Lys Phe
   65                  70                  75                  80
   Ile Gln Arg Gln Gly Trp Pro Val Ile Glu Leu Glu Asp Tyr Ser Asp
                   85                  90                  95
   Pro Leu Tyr Pro Trp Lys Val Arg Ala Glu Leu Ala Ala Ser Tyr Ile
               100                 105                 110
   Ala Asp Glu Lys Glu Arg Gly Glu Lys Leu Ser Val Ala Leu Arg His
           115                 120                 125
   Ile Ala Arg His Arg Gly Trp Arg Asn Pro Tyr Ala Lys Val Ser Ser
       130                 135                 140
   Leu Tyr Leu Pro Asp Glu Pro Ser Asp Ala Phe Lys Ser Ile Arg Glu
   145                 150                 155                 160
   Glu Ile Lys Arg Ala Ser Gly Gln Pro Val Pro Glu Thr Ala Thr Val
                   165                 170                 175
   Gly Gln Met Val Thr Leu Cys Glu Leu Gly Thr Leu Lys Leu Arg Gly
               180                 185                 190
   Glu Gly Gly Val Leu Ser Ala Arg Leu Gln Gln Ser Asp His Ala Arg
           195                 200                 205
   Glu Ile Gln Glu Ile Cys Arg Met Gln Glu Ile Gly Gln Glu Leu Tyr
       210                 215                 220
   Arg Lys Ile Ile Asp Val Val Phe Ala Ala Glu Ser Pro Lys Gly Ser
   225                 230                 235                 240
   Ala Ser Ser Arg Val Gly Lys Asp Pro Leu Gln Pro Gly Lys Asn Arg
                   245                 250                 255
   Ala Leu Lys Ala Ser Asp Ser Phe Gln Arg Tyr Arg Ile Ala Ala Leu
   260 265 270
   Ile Ser Asn Leu Arg Val Arg Val Asp Gly Glu Lys Arg Ile Leu Ser
   275 280 285
   Val Glu Glu Lys Asn Leu Val Phe Asp His Leu Val Asn Leu Ala Pro
   290 295 300
   Lys Lys Glu Pro Glu Trp Val Thr Ile Ala Glu Ile Leu Gly Ile Asp
   305 310 315 320
   Arg Gly Gln Leu Ile Gly Thr Ala Thr Met Thr Asp Asp Gly Glu Arg
   325 330 335
   Ala Gly Ala Arg Pro Pro Thr His Asp Thr Asn Arg Ser Ile Val Asn
   340 345 350
   Ser Arg Ile Ala Pro Leu Val Asp Trp Trp Lys Thr Ala Ser Ala Leu
   355 360 365
   Glu Gln His Ala Met Val Lys Ala Leu Ser Asn Ala Glu Val Asp Asp
   370 375 380
   Phe Asp Ser Pro Glu Gly Ala Lys Val Gln Ala Phe Phe Ala Asp Leu
   385 390 395 400
   Asp Asp Asp Val His Ala Lys Leu Asp Ser Leu His Leu Pro Val Gly
   405 410 415
   Arg Ala Ala Tyr Ser Glu Asp Thr Leu Val Arg Leu Thr Arg Arg Met
   420 425 430
   Leu Ala Asp Gly Val Asp Leu Tyr Thr Ala Arg Leu Gln Glu Phe Gly
   435 440 445
   Ile Glu Pro Ser Trp Thr Pro Pro Ala Pro Arg Ile Gly Glu Pro Val
   450 455 460
   Gly Asn Pro Ala Val Asp Arg Val Leu Lys Thr Val Ser Arg Trp Leu
   465 470 475 480
   Glu Ser Ala Thr Lys Thr Trp Gly Ala Pro Glu Arg Val Ile Ile Glu
   485 490 495
   His Val Arg Asp Gly Phe Val Thr Glu Lys Arg Ala Arg Glu Met Asp
   500 505 510
   Gly Asp Met Arg Arg Arg Ala Ala Arg Asn Ala Lys Leu Phe Gln Glu
   515 520 525
   Met Gln Glu Lys Leu Asn Val Gln Gly Lys Pro Ser Arg Ala Asp Leu
   530 535 540
   Trp Arg Tyr Gln Ser Val Gln Arg Gln Asn Cys Gln Cys Ala Tyr Cys
   545 550 555 560
   Gly Ser Pro Ile Thr Phe Ser Asn Ser Glu Met Asp His Ile Val Pro
   565 570 575
   Arg Ala Gly Gln Gly Ser Thr Asn Thr Arg Glu Asn Leu Val Ala Val
   580 585 590
   Cys His Arg Cys Asn Gln Ser Lys Gly Asn Thr Pro Phe Ala Ile Trp
   595 600 605
   Ala Lys Asn Thr Ser Ile Glu Gly Val Ser Val Lys Glu Ala Val Glu
   610 615 620
   Arg Thr Arg His Trp Val Thr Asp Thr Gly Met Arg Ser Thr Asp Phe
   625 630 635 640
   Lys Lys Phe Thr Lys Ala Val Val Glu Arg Phe Gln Arg Ala Thr Met
   645 650 655
   Asp Glu Glu Ile Asp Ala Arg Ser Met Glu Ser Val Ala Trp Met Ala
   660 665 670
   Asn Glu Leu Arg Ser Arg Val Ala Gln His Phe Ala Ser His Gly Thr
   675 680 685
   Thr Val Arg Val Tyr Arg Gly Ser Leu Thr Ala Glu Ala Arg Arg Ala
   690 695 700
   Ser Gly Ile Ser Gly Lys Leu Glu Phe Leu Asp Gly Val Gly Lys Ser
   705 710 715 720
   Arg Leu Asp Arg Arg His His Ala Ile Asp Ala Ala Val Ile Ala Phe
   725 730 735
   Thr Ser Asp Tyr Val Ala Glu Thr Leu Ala Val Arg Ser Asn Leu Lys
   740 745 750
   Gln Ser Gln Ala His Arg Gln Glu Ala Pro Gln Trp Arg Glu Phe Thr
           755                 760                 765
   Gly Lys Asp Ala Glu His Arg Ala Ala Trp Arg Val Trp Cys Gln Lys
       770                 775                 780
   Met Glu Lys Leu Ser Ala Leu Leu Thr Glu Asp Leu Arg Asp Asp Arg
   785                 790                 795                 800
   Val Val Val Met Ser Asn Val Arg Leu Arg Leu Gly Asn Gly Ser Ala
                   805                 810                 815
   His Glu Glu Thr Ile Gly Lys Leu Ser Lys Val Lys Leu Gly Ser Gln
               820                 825                 830
   Leu Ser Val Ser Asp Ile Asp Lys Ala Ser Ser Glu Ala Leu Trp Cys
           835                 840                 845
   Ala Leu Thr Arg Glu Pro Asp Phe Asp Pro Lys Asp Gly Leu Pro Ala
       850                 855                 860
   Asn Pro Glu Arg His Ile Arg Val Asn Gly Thr His Val Tyr Ala Gly
   865                 870                 875                 880
   Asp Asn Ile Gly Leu Phe Pro Val Ser Ala Gly Ser Ile Ala Leu Arg
                   885                 890                 895
   Gly Gly Tyr Ala Glu Leu Gly Ser Ser Phe His His Ala Arg Val Tyr
               900                 905                 910
   Lys Ile Thr Ser Gly Lys Lys Pro Ala Phe Ala Met Leu Arg Val Tyr
           915                 920                 925
   Thr Ile Asp Leu Leu Pro Tyr Arg Asn Gln Asp Leu Phe Ser Val Glu
       930                 935                 940
   Leu Lys Pro Gln Thr Met Ser Met Arg Gln Ala Glu Lys Lys Leu Arg
   945                 950                 955                 960
   Asp Ala Leu Ala Thr Gly Asn Ala Glu Tyr Leu Gly Trp Leu Val Val
                   965                 970                 975
   Asp Asp Glu Leu Val Val Asp Thr Ser Lys Ile Ala Thr Asp Gln Val
               980                 985                 990
   Lys Ala Val Glu Ala Glu Leu Gly Thr Ile Arg Arg Trp Arg Val Asp
           995                 1000                1005
   Gly Phe Phe Gly Asp Thr Arg Leu Arg Leu Arg Pro Leu Gln Met Ser
       1010                1015                1020
   Lys Glu Gly Ile Lys Lys Glu Leu Ala Pro Glu Leu Ser Lys Ile Ile
   1025                1030                1035                1040
   Asp Arg Pro Gly Trp Leu Pro Ala Val Asn Lys Leu Phe Ser Glu Gly
                   1045                1050                1055
   Asn Val Thr Val Val Arg Arg Asp Ser Leu Gly Arg Val Arg Leu Glu
               1060                1065                1070
   Ser Thr Ala His Leu Pro Val Thr Trp Lys Val Gln
           1075                1080
<210> 186
   <211> 1084
   <212> PRT
   <213> Corynebacterium diphtheriae
<220>
   <223> CRISPR-associated endonuclease
<308> YP_005134835
   <309> 2013-08-28
<400> 186
   Met Lys Tyr His Val Gly Ile Asp Val Gly Thr Phe Ser Val Gly Leu
   1               5                   10                  15
   Ala Ala Ile Glu Val Asp Asp Ala Gly Met Pro Ile Lys Thr Leu Ser
               20                  25                  30
   Leu Val Ser His Ile His Asp Ser Gly Leu Asp Pro Asp Lys Ile Lys
           35                  40                  45
   Ser Ala Val Thr Arg Leu Ala Ser Ser Gly Ile Ala Arg Arg Thr Arg
       50                  55                  60
   Arg Leu Tyr Arg Arg Lys Arg Arg Arg Leu Gln Gln Leu Asp Lys Phe
   65                  70                  75                  80
   Ile Gln Arg Gln Gly Trp Pro Val Ile Glu Leu Glu Asp Tyr Ser Asp
                   85                  90                  95
   Pro Leu Tyr Pro Trp Lys Val Arg Ala Glu Leu Ala Ala Ser Tyr Ile
               100                 105                 110
   Ala Asp Glu Lys Glu Arg Gly Glu Lys Leu Ser Val Ala Leu Arg His
           115                 120                 125
   Ile Ala Arg His Arg Gly Trp Arg Asn Pro Tyr Ala Lys Val Ser Ser
       130                 135                 140
   Leu Tyr Leu Pro Asp Glu Pro Ser Asp Ala Phe Lys Ala Ile Arg Glu
   145                 150                 155                 160
   Glu Ile Lys Arg Ala Ser Gly Gln Pro Val Pro Glu Thr Ala Thr Val
                   165                 170                 175
   Gly Gln Met Val Thr Leu Cys Glu Leu Gly Thr Leu Lys Leu Arg Gly
               180                 185                 190
   Glu Gly Gly Val Leu Ser Ala Arg Leu Gln Gln Ser Asp His Ala Arg
           195                 200                 205
   Glu Ile Gln Glu Ile Cys Arg Met Gln Glu Ile Gly Gln Glu Leu Tyr
       210                 215                 220
   Arg Lys Ile Ile Asp Val Val Phe Ala Ala Glu Ser Pro Lys Gly Ser
   225                 230                 235                 240
   Ala Ser Ser Arg Val Gly Lys Asp Pro Leu Gln Pro Gly Lys Asn Arg
                   245                 250                 255
   Ala Leu Lys Ala Ser Asp Ala Phe Gln Arg Tyr Arg Ile Ala Ala Leu
               260                 265                 270
   Ile Gly Asn Leu Arg Val Arg Val Asp Gly Glu Lys Arg Ile Leu Ser
           275                 280                 285
   Val Glu Glu Lys Asn Leu Val Phe Asp His Leu Val Asn Leu Ala Pro
       290                 295                 300
   Lys Lys Glu Pro Glu Trp Val Thr Ile Ala Glu Ile Leu Gly Ile Asp
   305                 310                 315                 320
   Arg Gly Gln Leu Ile Gly Thr Ala Thr Met Thr Asp Asp Gly Glu Arg
                   325                 330                 335
   Ala Gly Ala Arg Pro Pro Thr His Asp Thr Asn Arg Ser Ile Val Asn
               340                 345                 350
   Ser Arg Ile Ala Pro Leu Val Asp Trp Trp Lys Thr Ala Ser Ala Leu
           355                 360                 365
   Glu Gln His Ala Met Val Lys Ala Leu Ser Asn Ala Glu Val Asp Asp
       370                 375                 380
   Phe Asp Ser Pro Glu Gly Ala Lys Val Gln Ala Phe Phe Ala Asp Leu
   385                 390                 395                 400
   Asp Asp Asp Val His Ala Lys Leu Asp Ser Leu His Leu Pro Val Gly
                   405                 410                 415
   Arg Ala Ala Tyr Ser Glu Asp Thr Leu Val Arg Leu Thr Arg Arg Met
               420                 425                 430
   Leu Ala Asp Gly Val Asp Leu Tyr Thr Ala Arg Leu Gln Glu Phe Gly
           435                 440                 445
   Ile Glu Pro Ser Trp Thr Pro Pro Ala Pro Arg Ile Gly Glu Pro Val
       450                 455                 460
   Gly Asn Pro Ala Val Asp Arg Val Leu Lys Thr Val Ser Arg Trp Leu
   465                 470                 475                 480
   Lys Ser Ala Thr Lys Thr Trp Gly Ala Pro Glu Arg Val Ile Ile Glu
                   485                 490                 495
   His Val Arg Glu Gly Phe Val Thr Glu Lys Arg Ala Arg Glu Met Asp
               500                 505                 510
   Gly Asp Met Arg Arg Arg Ala Ala Arg Asn Ala Lys Leu Phe Gln Glu
           515                 520                 525
   Met Gln Glu Lys Leu Asn Val Gln Gly Lys Pro Ser Arg Ala Asp Leu
       530                 535                 540
   Trp Arg Tyr Gln Ser Val Gln Arg Gln Asn Cys Gln Cys Ala Tyr Cys
   545                 550                 555                 560
   Gly Ser Pro Ile Thr Phe Ser Asn Ser Glu Met Asp His Ile Val Pro
   565 570 575
   Arg Ala Gly Gln Gly Ser Thr Asn Thr Arg Glu Asn Leu Val Ala Val
   580 585 590
   Cys His Arg Cys Asn Gln Ser Lys Gly Asn Thr Pro Phe Ala Ile Trp
   595 600 605
   Ala Lys Asn Thr Ser Ile Glu Gly Val Ser Val Lys Glu Ala Val Glu
   610 615 620
   Arg Thr Arg His Trp Val Thr Asp Thr Gly Met Arg Ser Thr Asp Phe
   625 630 635 640
   Lys Lys Phe Thr Lys Ala Val Val Glu Arg Phe Gln Arg Ala Thr Met
   645 650 655
   Asp Glu Glu Ile Asp Ala Arg Ser Met Glu Ser Val Ala Trp Met Ala
   660 665 670
   Asn Glu Leu Arg Ser Arg Val Ala Gln His Phe Ala Ser His Gly Thr
   675 680 685
   Thr Val Arg Val Tyr Arg Gly Ser Leu Thr Ala Glu Ala Arg Arg Ala
   690 695 700
   Ser Gly Ile Ser Gly Lys Leu Glu Phe Leu Asp Gly Val Gly Lys Ser
   705 710 715 720
   Arg Leu Asp Arg Arg His His Ala Ile Asp Ala Ala Val Ile Ala Phe
   725 730 735
   Thr Ser Asp Tyr Val Ala Glu Thr Leu Ala Val Arg Ser Asn Leu Lys
   740 745 750
   Gln Ser Gln Ala His Arg Gln Glu Ala Pro Gln Trp Arg Glu Phe Thr
   755 760 765
   Gly Lys Asp Ala Glu His Arg Ala Ala Trp Arg Val Trp Cys Gln Lys
   770 775 780
   Met Glu Lys Leu Ser Ala Leu Leu Thr Glu Asp Leu Arg Asp Asp Arg
   785 790 795 800
   Val Val Val Met Ser Asn Val Arg Leu Arg Leu Gly Asn Gly Ser Ala
   805 810 815
   His Glu Glu Thr Ile Gly Lys Leu Ser Lys Val Lys Leu Gly Ser Gln
   820 825 830
   Leu Ser Val Ser Asp Ile Asp Lys Ala Ser Ser Glu Ala Leu Trp Cys
   835 840 845
   Ala Leu Thr Arg Glu Pro Asp Phe Asp Pro Lys Asp Gly Leu Pro Ala
   850 855 860
   Asn Pro Glu Arg His Ile Arg Val Asn Gly Thr His Val Tyr Ala Gly
   865 870 875 880
   Asp Asn Ile Gly Leu Phe Pro Val Ser Ala Gly Ser Ile Ala Leu Arg
   885 890 895
   Gly Gly Tyr Ala Glu Leu Gly Ser Ser Phe His His Ala Arg Val Tyr
   900 905 910
   Lys Ile Thr Ser Gly Lys Lys Pro Ala Phe Ala Met Leu Arg Val Tyr
   915 920 925
   Thr Ile Asp Leu Leu Pro Tyr Arg Asn Gln Asp Leu Phe Ser Val Glu
   930 935 940
   Leu Lys Pro Gln Thr Met Ser Met Arg Gln Ala Glu Lys Lys Leu Arg
   945 950 955 960
   Asp Ala Leu Ala Thr Gly Asn Ala Glu Tyr Leu Gly Trp Leu Val Val
   965 970 975
   Asp Asp Glu Leu Val Val Asp Thr Ser Lys Ile Ala Thr Asp Gln Val
   980 985 990
   Lys Ala Val Glu Ala Glu Leu Gly Thr Ile Arg Arg Trp Arg Val Asp
   995 1000 1005
   Gly Phe Phe Gly Asp Thr Arg Leu Arg Leu Arg Pro Leu Gln Met Ser
   1010 1015 1020
   Lys Glu Gly Ile Lys Lys Glu Ser Ala Pro Glu Leu Ser Lys Ile Ile
   1025 1030 1035 1040
   Asp Arg Pro Gly Trp Leu Pro Ala Val Asn Lys Leu Phe Ser Glu Gly
   1045 1050 1055
   Asn Val Thr Val Val Arg Arg Asp Ser Leu Gly Arg Val Arg Leu Glu
   1060 1065 1070
   Ser Thr Ala His Leu Pro Val Thr Trp Lys Val Gln
   1075 1080
<210> 187
   <211> 876
   <212> PRT
   <213> Corynebacterium diphtheriae
<220>
   <223> CRISPR-associated endonuclease
<308> YP_005136892
   <309> 2013-08-28
<400> 187
   Met Asn Pro His Glu Glu Leu Trp Ala Lys Gln Lys Gly Leu Ala Lys
   1               5                   10                  15
   Pro Tyr Pro Leu Leu Ala His Leu Leu Asp Ser Ala Ala Val Ala Gly
               20                  25                  30
   Ala Leu Trp Asp His Trp Leu Arg Gln Asp Leu Arg Gln Met Phe Ile
           35                  40                  45
   Glu Glu Leu Gly Ser Asn Ala Arg Glu Ile Ile Gln Phe Val Val Gly
       50                  55                  60
   Ser His Asp Ile Gly Lys Ala Thr Pro Leu Phe Gln Tyr Gln Lys Ala
   65                  70                  75                  80
   Gln Lys Gly Glu Val Trp Asp Ser Ile Arg Tyr Ala Ile Asp Arg Thr
                   85                  90                  95
   Gly Arg Tyr Gln Lys Pro Leu Pro Ser Ser Tyr Leu Val Lys Lys Thr
               100                 105                 110
   Ser Gly Gly Pro Asn Arg His Glu Gln Trp Ser Ser Phe Ala Ser Lys
           115                 120                 125
   Asn Glu Tyr Leu Lys Pro Ser Ala Ala Ala Lys Glu Asn Trp Ile Gly
       130                 135                 140
   Leu Ala Ile Gly Gly His His Gly Arg Phe Glu Pro Val Gly Tyr Gly
   145                 150                 155                 160
   Arg His Gln Arg Lys Ala Ala Glu Asp Leu Ala Lys Ser Gly Trp Ser
                   165                 170                 175
   Ala Ala Gln Gln Asp Leu Leu Arg Ala Leu Glu Lys Ala Ser Gly Ile
               180                 185                 190
   Thr Arg Ala Ser Leu Pro Ser Glu Leu Ser Pro Glu Leu Thr Leu Val
           195                 200                 205
   Leu Ser Gly Leu Thr Ile Leu Ala Asp Arg Ile Ser Ser Thr Glu Ser
       210                 215                 220
   Phe Val Ile Thr Gly Ala Arg Met Ile Asp Asp Gly Thr Leu His Leu
   225                 230                 235                 240
   Ala Thr Pro Ile Asp Trp Leu Lys Thr Arg Lys Leu Asp Ser Glu Lys
                   245                 250                 255
   His Val Ala Lys Thr Val Gly Ile Tyr His Gly Trp Asn Asn His Glu
               260                 265                 270
   Ser Ala Ile His Ser Ile Leu Lys Gly Tyr Asp Pro Arg Pro Leu Gln
           275                 280                 285
   Thr Ile Ala Leu Gln Asn Gln Val Gly Leu Leu Asn Leu Met Ala Pro
       290                 295                 300
   Thr Gly Asn Gly Lys Thr Glu Ala Ala Ile Leu Arg His Ser Leu Lys
   305                 310                 315                 320
   Glu Asn Asp Arg Leu Ile Phe Leu Leu Pro Thr Gln Ala Thr Ser Asn
                   325                 330                 335
   Ala Ile Met Arg Arg Val Gln Gly Ile Tyr Ser Asp Thr Pro Asn Ala
               340                 345                 350
   Ala Ala Leu Ala His Ser Leu Ala Ser Val Glu Asp Phe Tyr Gln Thr
           355                 360                 365
   Pro Leu Ser Val Phe Asp Asp His Tyr Asp Pro Ser Lys Glu Gln Phe
       370                 375                 380
   Glu Ser Ser Met Ser Gly Gly Leu Tyr Pro Ser Ser Phe Val Cys Ser
   385                 390                 395                 400
   Gly Ala Ala Arg Leu Leu Ala Pro Ile Cys Ile Gly Thr Val Asp Gln
                   405                 410                 415
   Ala Leu Ala Thr Ala Leu Pro Gly Lys Trp Ile His Leu Arg Ile Leu
               420                 425                 430
   Ala Leu Ala Asn Ala His Ile Val Ile Asp Glu Val His Thr Leu Asp
           435                 440                 445
   His Tyr Gln Thr Ala Leu Leu Glu Asn Ile Leu Pro Ile Leu Ala Lys
       450                 455                 460
   Leu Lys Thr Lys Ile Thr Phe Leu Thr Ala Thr Met Pro Ser Trp Gln
   465                 470                 475                 480
   Arg Thr Lys Leu Leu Thr Ala Tyr Gly Gly Glu Asp Leu Gln Ile Pro
                   485                 490                 495
   Pro Thr Val Phe Pro Ala Ala Glu Thr Val Leu Pro Gly Gln Phe Asn
               500                 505                 510
   Arg Thr Leu Ile Asp Ser Asp Ser Thr Thr Ile Asp Phe Thr Met Glu
           515                 520                 525
   Glu Thr Ser Tyr Asp His Leu Val Glu Ser His Val Lys Trp His Gln
       530                 535                 540
   Thr Thr Arg Leu Asn Ala Pro His Ala Arg Ile Gly Leu Ile Cys Asn
   545                 550                 555                 560
   Thr Val Lys Arg Ala Gln Glu Ile Ala Ala Ala Leu Glu Lys Thr Asn
                   565                 570                 575
   Asp Arg Ile Val Leu Leu His Ser Arg Met Thr Thr Glu His Arg Arg
               580                 585                 590
   Arg Ser Ala Glu Leu Leu Glu Ser Leu Leu Gly Pro Asn Gly Asn Arg
           595                 600                 605
   Lys Thr Ile Thr Val Val Gly Thr Gln Ala Ile Glu Ala Ser Leu Asp
       610                 615                 620
   Ile Asp Leu Asp Ile Leu Arg Thr Glu Leu Cys Pro Ala Pro Ser Leu
   625                 630                 635                 640
   Val Gln Arg Ala Gly Arg Val Trp Arg Arg Asn Asp Pro Tyr Arg Ser
                   645                 650                 655
   Ser Arg Ile Thr Ala Asp His Lys Pro Ile Ser Val Val Phe Ile Ala
               660                 665                 670
   Glu Ala Lys Asp Trp Gln Val Leu Pro Tyr Leu Arg Ala Glu Thr Ser
           675                 680                 685
   Arg Thr Gln Arg Trp Leu Glu Lys His Asn Gln Met Phe Leu Pro Gln
       690                 695                 700
   Met Ala Gln Glu Phe Ile Asp Ala Ala Thr Val Asp Leu Asp Thr Ala
   705                 710                 715                 720
   Thr Ser Glu Met Asp Leu Asp Ala Leu Ala Leu Met Gly Ile His Leu
                   725                 730                 735
   Met Lys Ala Asp Gly Ala Lys Ala Arg Ile Gln Asp Val Leu Asn Ser
               740                 745                 750
   Asp Ser Lys Val Ser Asp Phe Ala Leu Leu Thr Ser Lys Asn Glu Ile
           755                 760                 765
   Asp Glu Ala Gln Thr Arg Leu Ile Glu Glu Gly Thr His Leu Arg Ile
       770                 775                 780
   Ile Leu Gly Asp Glu Asn Glu Ser Ile Pro Gly Gly Trp Lys His Gly
   785                 790                 795                 800
   Leu Ser Ser Leu Leu Lys Leu Lys Ala Ser Asp Arg Glu Ser Leu Arg
                   805                 810                 815
   Thr Ala Leu Leu Ala Ser Ile Pro Leu Leu Val Ser Glu Lys Gln Lys
               820                 825                 830
   Gln Leu Leu Tyr Gln His Asn Leu Val Pro Leu Ser Ser Ser Lys Thr
           835                 840                 845
   Val Leu Ala Gly Phe Tyr Phe Leu Pro Lys Ala Gln Asn Phe Tyr Ser
       850                 855                 860
   Lys Asn Leu Gly Phe Ile Trp Pro Glu Glu Lys Asp
   865                 870                 875
<210> 188
   <211> 1052
   <212> PRT
   <213> Haemophilus parainfluenzae
<220>
   <223> CRISPR-associated endonuclease
<308> YP_004823543
   <309> 2013-06-11
<400> 188
   Met Glu Asn Lys Asn Leu His Tyr Ile Leu Gly Leu Asp Leu Gly Ile
   1               5                   10                  15
   Ala Ser Val Gly Trp Ala Val Val Glu Ile Asp Glu Lys Glu Asn Pro
               20                  25                  30
   Leu Arg Leu Ile Asp Val Gly Val Arg Thr Phe Glu Arg Ala Glu Thr
           35                  40                  45
   Gln Lys Gly Glu Ser Leu Ala Leu Ser Arg Arg Ser Ala Arg Ser Ala
       50                  55                  60
   Arg Arg Leu Thr Gln Arg Arg Val Ala Arg Leu Lys Lys Ala Lys Arg
   65                  70                  75                  80
   Leu Leu Lys Ser Glu Asn Ile Leu Leu Ser Thr Asp Glu Arg Leu Pro
                   85                  90                  95
   His Gln Val Trp Gln Leu Arg Val Glu Gly Leu Asp Arg Lys Leu Glu
               100                 105                 110
   Arg Gln Glu Trp Ala Ala Val Leu Leu His Leu Ile Lys His Arg Gly
           115                 120                 125
   Tyr Leu Ser Gln Arg Lys Asn Glu Ser Lys Ser Glu Asn Lys Glu Leu
       130                 135                 140
   Gly Ala Leu Leu Ser Gly Val Ala Ser Asn His Glu Leu Leu Gln Gln
   145                 150                 155                 160
   Ala Thr Tyr Arg Thr Pro Ala Glu Leu Ala Val Lys Lys Phe Glu Val
                   165                 170                 175
   Glu Glu Gly His Ile Arg Asn Gln Gln Gly Ala Tyr Thr His Thr Phe
               180                 185                 190
   Ser Arg Leu Asp Leu Leu Ala Glu Met Glu Leu Leu Phe Ser Arg Gln
           195                 200                 205
   Gln His Phe Gly Asn Pro Phe Ala Ser Glu Lys Leu Leu Glu Asn Leu
       210                 215                 220
   Thr Ala Leu Leu Met Trp Gln Lys Pro Ala Leu Ser Gly Glu Asp Ile
   225                 230                 235                 240
   Leu Lys Met Leu Gly Lys Cys Thr Phe Glu Asp Glu Tyr Lys Ala Ala
                   245                 250                 255
   Lys Asn Thr Tyr Thr Ala Glu Arg Phe Val Trp Ile Thr Lys Leu Asn
               260                 265                 270
   Asn Leu Arg Ile Gln Glu Asn Gly Leu Glu Arg Ala Leu Ser Asp Asn
           275                 280                 285
   Glu Arg Leu Met Leu Ile Glu Gln Pro Tyr Glu Lys Ala Lys Leu Thr
       290                 295                 300
   Tyr Ala Gln Val Arg Ser Ile Leu Asn Leu Ser Asp Asp Ala Ile Phe
   305                 310                 315                 320
   Lys Gly Val Arg Tyr Ser Gly Glu Asp Lys Lys Ala Ile Glu Thr Lys
                   325                 330                 335
   Thr Thr Leu Met Glu Met Lys Ala Tyr His Gln Ile Arg Lys Val Leu
               340                 345                 350
   Glu Gly Asn Asn Leu Lys Ala Glu Trp Val Ala Leu Lys Ala Asn Pro
           355                 360                 365
   Thr Leu Leu Asp Glu Ile Gly Thr Ala Phe Ser Leu Tyr Lys Thr Asp
       370                 375                 380
   Glu Asp Ile Ser Ala Tyr Leu Ala Gly Lys Leu Ser Gln Pro Val Leu
   385 390 395 400
   Asn Ala Leu Leu Glu Asn Leu Ser Phe Asp Lys Phe Ile Gln Leu Ser
   405 410 415
   Leu Lys Ala Leu Tyr Lys Leu Leu Pro Leu Met Gln Gln Gly Leu Arg
   420 425 430
   Tyr Asp Glu Ala Cys Arg Glu Ile Tyr Gly Asp His Tyr Gly Lys Lys
   435 440 445
   Thr Glu Glu Thr His His Phe Leu Pro Gln Ile Pro Ala Asp Glu Ile
   450 455 460
   Arg Asn Pro Val Val Leu Arg Thr Leu Thr Gln Ala Arg Lys Val Ile
   465 470 475 480
   Asn Gly Val Val Arg Leu Tyr Gly Ser Pro Ala Arg Ile His Ile Glu
   485 490 495
   Thr Gly Arg Glu Val Gly Lys Ser Tyr Lys Asp Arg His Glu Leu Lys
   500 505 510
   Lys Arg Gln Glu Glu Asn Arg Lys Gln Arg Glu Lys Thr Ile Ser Glu
   515 520 525
   Ile Lys Thr Leu Phe Pro Asn Phe Ser Gly Glu Pro Lys Gly Lys Asp
   530 535 540
   Ile Leu Lys Met Arg Leu Tyr Tyr Gln Gln Asn Ala Lys Cys Leu Tyr
   545 550 555 560
   Ser Gly Lys Pro Leu Glu Leu His Arg Leu Phe Glu Gln Lys Tyr Val
   565 570 575
   Glu Val Asp His Ala Leu Pro Leu Ser Arg Thr Trp Asp Asp Ser Phe
   580 585 590
   Asn Asn Lys Val Leu Val Leu Ala Asn Glu Asn Gln Asn Lys Gly Asn
   595 600 605
   Leu Thr Pro Phe Glu Trp Leu Asp Gly Lys Asn Asn Ser Glu Arg Trp
   610 615 620
   Arg Thr Phe Lys Ala Leu Val Glu Thr Ser Ala Phe Pro Tyr Ala Lys
   625 630 635 640
   Lys Gln Arg Ile Leu Ser Gln Lys Leu Asp Glu Lys Gly Phe Ile Glu
   645 650 655
   Arg Asn Leu Asn Asp Thr Arg Tyr Val Ala Arg Phe Leu Cys Asn Phe
   660 665 670
   Ile Ala Asp Asn Met His Leu Thr Gly Glu Gly Lys Arg Lys Val Phe
   675 680 685
   Ala Ser Asn Gly Gln Ile Thr Ala Leu Leu Arg Arg Arg Trp Gly Leu
   690 695 700
   Ala Lys Ser Arg Glu Asp Asn Asp Arg His His Ala Leu Asp Ala Val
   705 710 715 720
   Leu Val Ala Cys Ser Thr Val Ala Met Gln Gln Lys Ile Thr Arg Phe
   725 730 735
   Val Arg Phe Lys Ala Gly Asp Val Phe Thr Gly Glu Arg Ile Asp Arg
   740 745 750
   Glu Thr Gly Glu Ile Ile Pro Leu His Phe Pro Thr Pro Trp Gln Phe
   755 760 765
   Phe Lys Gln Glu Val Glu Ile Arg Ile Phe Ser Asp Asn Pro Lys Leu
   770 775 780
   Glu Leu Glu Asn Arg Leu Pro Asp Arg Pro Gln Ala Asn His Glu Phe
   785 790 795 800
   Val Gln Pro Leu Phe Val Ser Arg Met Pro Thr Arg Lys Met Thr Gly
   805 810 815
   Gln Gly His Met Glu Thr Val Lys Ser Ala Lys Arg Leu Asn Glu Gly
   820 825 830
   Ile Ser Met Ile Lys Met Pro Leu Thr Lys Leu Lys Leu Lys Asp Leu
   835 840 845
   Glu Leu Met Val Asn Arg Glu Arg Glu Lys Asp Leu Tyr Asp Ala Leu
   850 855 860
   Lys Thr Arg Leu Glu Ala Phe Asn Asp Asp Pro Ala Lys Ala Phe Ala
   865 870 875 880
   Glu Pro Phe Met Lys Lys Gly Gly Ala Ile Val Lys Ser Val Arg Val
   885 890 895
   Glu Gln Val Gln Lys Ser Gly Val Leu Val Arg Gln Gly Asn Gly Val
   900 905 910
   Ala Asp Asn Ala Ser Met Val Arg Val Asp Val Phe Thr Lys Asp Gly
   915 920 925
   Lys Tyr Phe Leu Val Pro Ile Tyr Thr Trp Gln Val Ala Lys Gly Ile
   930 935 940
   Leu Pro Asn Lys Ala Val Ile Gln Gly Lys Asp Glu Glu Asp Trp Glu
   945 950 955 960
   Asp Ile Asp Asp Ala Thr Phe Gln Phe Ser Leu His Pro Asn Asp Leu
   965 970 975
   Ile Ser Val Lys Thr Lys Lys Asp Glu Phe Leu Gly Tyr Phe Asn Gly
   980 985 990
   Leu Asn Arg His Thr Gly Gly Ile Asn Ile Arg Thr His Asp Leu Glu
   995 1000 1005
   Lys Ser Lys Gly Lys Gln Gly Ile Phe Glu Gly Ile Gly Val Lys Ile
   1010 1015 1020
   Ala Leu Ser Phe Glu Lys Tyr Gln Ile Asp Glu Leu Gly Lys Asn Ile
   1025 1030 1035 1040
   Arg Leu Cys Lys Pro Ser Lys Arg Gln Pro Val Arg
   1045 1050
<210> 189
   <211> 1025
   <212> PRT
   <213> gamma proteobacterium
<220>
   <223> CRISPR-associated endonuclease
<308> YP_003812627
   <309> 2013-06-10
<400> 189
   Met Gly Gly Tyr Met Pro Gln Leu Met Arg Tyr Arg Leu Ala Leu Asp
   1               5                   10                  15
   Ile Gly Ser Thr Ser Ile Gly Trp Ala Met Ile Arg Leu Ser Met Gln
               20                  25                  30
   Asn Glu Pro Ile Ala Ile Ile Arg Met Gly Val Arg Ile Phe Ser Asp
           35                  40                  45
   Gly Arg Asn Pro Lys Asp Gly Ser Ser Leu Ala Val Thr Arg Arg Val
       50                  55                  60
   Ala Arg Gln Met Arg Arg Arg Arg Asp Arg Leu Leu Lys Arg Lys Thr
   65                  70                  75                  80
   Arg Leu Leu Asn Ala Leu Val Gln Lys Lys Phe Phe Pro Glu Asp Ala
                   85                  90                  95
   Asp Ala Arg Lys Ala Leu Val Val Leu Asn Pro Tyr Glu Leu Arg Lys
               100                 105                 110
   Glu Gly Leu Tyr Arg Gln Leu Ser Gly Pro Glu Phe Gly Arg Ala Leu
           115                 120                 125
   Phe His Ile Ser Gln Arg Arg Gly Phe Leu Ser Asn Arg Lys Thr Asp
       130                 135                 140
   Lys Lys Asp Asn Asp Ser Ser Val Leu Lys Lys Ala Ile Val Asp Leu
   145                 150                 155                 160
   Arg Gln Gln Leu Glu Val Glu Asn Cys Gln Thr Ile Gly Glu Trp Leu
                   165                 170                 175
   Phe Lys Gln Gln Gln Ser Gly Lys Ser Val Arg Ala Arg Leu Arg Gly
               180                 185                 190
   Lys Thr Gln Lys Asp Lys Ala Tyr Asp Phe Tyr Ile Asp Arg Ala Met
           195                 200                 205
   Ile Ala His Glu Phe Asp Thr Leu Trp Val Ser Gln Ala Lys Tyr Asp
       210                 215                 220
   Pro Leu Leu Phe Asn Glu Asp Ala Arg Ala Ile Leu Arg Asp Ile Leu
   225 230 235 240
   Leu Tyr Gln Arg Pro Leu Lys Pro Val Lys Pro Gly Arg Cys Thr Leu
   245 250 255
   Ile Pro Asp Glu Ser Arg Ala Pro Leu Ala Leu Pro Leu Thr Gln Gln
   260 265 270
   Ala Arg Ile Tyr Gln Glu Leu Asn His Leu Arg Ile Thr Thr Pro Ala
   275 280 285
   Leu Gly Ala Gln Ala Leu Thr Val Ala Gln Arg Asn Thr Ile Ala Ala
   290 295 300
   Leu Leu Glu Lys Ser Asp Val Thr Phe Val Lys Ile Ala Lys Ala Leu
   305 310 315 320
   Gly Leu Thr Ser Thr Thr Glu Phe Asn Leu Gln Asp Ile Lys Arg Asp
   325 330 335
   Arg Leu Lys Gly Asn Ala Thr Ala Ile Ile Leu Ala Arg Asn Gln Tyr
   340 345 350
   Phe Gly Arg Arg Trp His Asp Phe Asn Ala Asp Leu Gln Asn Leu Ile
   355 360 365
   Val Asp Lys Leu Val Asn Glu Gly Ser Glu Thr Ala Leu Ile Glu Trp
   370 375 380
   Leu Lys Ala Asn Thr Asp Val Asp Glu Ser Ala Ala Glu Arg Val Ser
   385 390 395 400
   Gly Val Thr Leu Pro Glu Gly Tyr Gly Ser Leu Ser Val Ala Ala Leu
   405 410 415
   Arg Arg Leu Leu Pro Ala Leu Lys Glu Asp Val Val Val Tyr Ser Glu
   420 425 430
   Ala Val Lys Arg Ala Gly Phe Asp Ser His Ser Ala Leu Ser Leu Ile
   435 440 445
   Glu Gln Thr Gly Glu Leu Tyr Gln Glu Leu Pro Tyr Tyr Gly Glu Val
   450 455 460
   Leu Gln Arg His Val Gly Phe Gly Ser Gly Asp Pro Asn Asp Ser Pro
   465 470 475 480
   Glu Arg Arg Phe Gly Lys Ile Ala Asn Pro Thr Val His Ile Gly Leu
   485 490 495
   Asn Glu Leu Arg Lys Ile Ile Asn Ala Leu Ile Lys Arg Tyr Gly Asn
   500 505 510
   Pro Ala Glu Val Val Val Glu Leu Ala Arg Asp Leu Lys Leu Ser Arg
   515 520 525
   Glu Lys Arg Lys Glu Ile Gln Glu Glu Gln Lys Gln Arg Gln Asp Arg
   530 535 540
   Asn Glu Lys Arg Ile Lys Asp Ala Cys Glu Ile Leu Gly Ile Asp Pro
   545 550 555 560
   Leu Val Ile Asp Arg Gly Lys Arg Arg Glu Ile Ser Gln Lys Met Gln
   565 570 575
   Leu Trp Asp Glu Leu Asn Pro Lys Asp Ala Ala Asn Arg Arg Cys Pro
   580 585 590
   Tyr Thr Gly Asp Gln Ile Ser Ile Glu Lys Leu Leu Ser Ala Glu Val
   595 600 605
   Glu Ile Glu His Ile Leu Pro Phe Ser Lys Thr Leu Asp Asp Ser Leu
   610 615 620
   Asn Asn Lys Thr Val Ala Met Arg Arg Ala Asn Arg Asp Lys Gly Asn
   625 630 635 640
   Arg Thr Pro Tyr Glu Ala Phe Gly Glu Ser Leu Asn Pro Asp Tyr Asp
   645 650 655
   Tyr Asp Ala Ile Leu Gln Arg Ala Ser Leu Met Pro Arg Glu Lys Arg
   660 665 670
   Lys Arg Phe Ala Ser Asp Gly Tyr Gln Gln Trp Leu Arg Asp Asp Lys
   675 680 685
   Asp Phe Leu Ser Arg Ala Leu Asn Asp Thr Ala Tyr Leu Ser Arg Ile
   690 695 700
   Ala Lys Glu Tyr Leu Thr Leu Ile Cys Pro Pro Asn Ser Val Arg Ala
   705 710 715 720
   Ile Pro Gly Arg Met Thr Ala Gln Leu Arg Ala Met Phe Gly Leu Asn
   725 730 735
   Arg Leu Leu Ser Glu Gly Gly Ile Lys Asn Arg Glu Asp His Arg His
               740                 745                 750
   His Ala Ile Asp Ala Ala Val Ile Gly Val Thr Asp Gln Gly Leu Leu
           755                 760                 765
   Gln Lys Phe Ser Arg Ala Ser Ala Arg Thr Arg Glu Leu Gln Leu Ser
       770                 775                 780
   Arg Leu Val Asp Asp Met Pro Leu Pro Trp Asp Thr Tyr Arg Glu His
   785                 790                 795                 800
   Val Thr Arg Ala Leu Glu His Leu Val Val Ser His Lys Pro Asp His
                   805                 810                 815
   Gly Tyr Gln Gly Lys Met His Glu Asp Thr Ala Trp Gly Leu Arg Asp
               820                 825                 830
   Asn Gly Gln Val Thr Arg Arg Val Arg Ser Glu Asp Gly Gly Pro Arg
           835                 840                 845
   Gln Arg Leu Leu Glu Asn Lys Asn Val Val Lys Ile Asn Ser Thr Arg
       850                 855                 860
   Asp Phe Ser Arg His Gly Ile Asp Glu Gln Gly Gln Val Asn Ala Tyr
   865                 870                 875                 880
   Lys Gly Tyr Val Gly Gly Ser Asn Tyr Cys Ile Glu Ile Trp Ile Asp
                   885                 890                 895
   Glu Lys Gly Lys Trp Asn Ser Asp Val Val Ser Thr Phe Gln Ala Tyr
               900                 905                 910
   Gln Ile Val Arg Glu Leu Gly Glu Lys Ala Ala Trp Gln Lys Leu Arg
           915                 920                 925
   Asn Pro Leu Leu Ser Leu Ser Ala Lys Pro Leu Val Met Arg Leu Ile
       930                 935                 940
   Ile Asp Asp Val Val Lys Met Gln Ile Ala Gly Gln Asn Lys Ile Met
   945                 950                 955                 960
   Arg Val Val Lys Ile Ala Gln Asn Gly Gln Ile Phe Phe Ala Glu His
                   965                 970                 975
   Asn Glu Ala Asn Val Asp Ala Arg Asp Lys Asp Lys Asn Asn Pro Phe
               980                 985                 990
   Lys Tyr Ile Ser Lys Thr Ala Gly Ser Leu Gln Lys Ala Gly Gly Ile
           995                 1000                1005
   His Met Ala Ile Ser Pro Ile Gly Asp Ala Arg Pro Val Arg Tyr Gly
       1010                1015                1020
   Arg
   1025
<210> 190
   <211> 1368
   <212> PRT
   <213> Streptococcus pyogenes
<220>
   <223> CRISPR-associated endonuclease
<308> NP_269215
   <309> 2013-06-27
<400> 190
<210> 191
   <211> 1370
   <212> PRT
   <213> Streptococcus agalactiae
<220>
   <223> CRISPR-associated endonuclease
<308> YP_007968536
   <309> 2013-06-11
<400> 191
   Met Asn Lys Pro Tyr Ser Ile Gly Leu Asp Ile Gly Thr Asn Ser Val
    1               5                  10                  15
   Gly Trp Ser Ile Ile Thr Asp Asp Tyr Lys Val Pro Ala Lys Lys Met
               20                  25                  30
   Arg Val Leu Gly Asn Thr Asp Lys Glu Tyr Ile Lys Lys Asn Leu Ile
           35                  40                  45
   Gly Ala Leu Leu Phe Asp Gly Gly Asn Thr Ala Ala Asp Arg Arg Leu
       50                  55                  60
   Lys Arg Thr Ala Arg Arg Arg Tyr Thr Arg Arg Arg Asn Arg Ile Leu
   65                  70                  75                  80
   Tyr Leu Gln Glu Ile Phe Ala Glu Glu Met Ser Lys Val Asp Asp Ser
                   85                  90                  95
   Phe Phe His Arg Leu Glu Asp Ser Phe Leu Val Glu Glu Asp Lys Arg
               100                 105                 110
   Gly Ser Lys Tyr Pro Ile Phe Ala Thr Met Gln Glu Glu Lys Asp Tyr
           115                 120                 125
   His Glu Lys Phe Pro Thr Ile Tyr His Leu Arg Lys Glu Leu Ala Asp
       130                 135                 140
   Lys Lys Glu Lys Ala Asp Leu Arg Leu Val Tyr Leu Ala Leu Ala His
   145                 150                 155                 160
   Ile Ile Lys Phe Arg Gly His Phe Leu Ile Glu Asp Asp Arg Phe Asp
                   165                 170                 175
   Val Arg Asn Thr Asp Ile Gln Lys Gln Tyr Gln Ala Phe Leu Glu Ile
               180                 185                 190
   Phe Asp Thr Ser Phe Glu Asn Asn His Leu Leu Ser Gln Asn Val Asp
           195                 200                 205
   Val Glu Ala Ile Leu Thr Asp Lys Ile Ser Lys Ser Ala Lys Lys Asp
       210                 215                 220
   Arg Ile Leu Ala Gln Tyr Pro Asn Gln Lys Ser Thr Gly Ile Phe Ala
   225                 230                 235                 240
   Glu Phe Leu Lys Leu Ile Val Gly Asn Gln Ala Asp Phe Lys Lys His
                   245                 250                 255
   Phe Asn Leu Glu Asp Lys Thr Pro Leu Gln Phe Ala Lys Asp Ser Tyr
               260                 265                 270
   Asp Glu Asp Leu Glu Asn Leu Leu Gly Gln Ile Gly Asp Glu Phe Ala
           275                 280                 285
   Asp Leu Phe Ser Ala Ala Lys Lys Leu Tyr Asp Ser Val Leu Leu Ser
       290                 295                 300
   Gly Ile Leu Thr Val Thr Asp Leu Ser Thr Lys Ala Pro Leu Ser Ala
   305                 310                 315                 320
   Ser Met Ile Gln Arg Tyr Asp Glu His Arg Glu Asp Leu Lys Gln Leu
                   325                 330                 335
   Lys Gln Phe Val Lys Ala Ser Leu Pro Glu Lys Tyr Gln Glu Ile Phe
               340                 345                 350
   Ala Asp Ser Ser Lys Asp Gly Tyr Ala Gly Tyr Ile Glu Gly Lys Thr
           355                 360                 365
   Asn Gln Glu Ala Phe Tyr Lys Tyr Leu Ser Lys Leu Leu Thr Lys Gln
       370                 375                 380
   Glu Asp Ser Glu Tyr Phe Leu Glu Lys Ile Lys Asn Glu Asp Phe Leu
   385                 390                 395                 400
   Arg Lys Gln Arg Thr Phe Asp Asn Gly Ser Ile Pro His Gln Val His
                   405                 410                 415
   Leu Thr Glu Leu Lys Ala Ile Ile Arg Arg Gln Ser Glu Tyr Tyr Pro
               420                 425                 430
   Phe Leu Lys Glu Asn Gln Asp Lys Ile Glu Lys Ile Leu Thr Phe Arg
           435                 440                 445
   Ile Pro Tyr Tyr Val Gly Pro Leu Ala Arg Gly Asn Ser Asp Phe Ala
       450                 455                 460
   Trp Met Thr Arg Lys Thr Asp Asp Ser Ile Arg Pro Trp Asn Phe Glu
   465                 470                 475                 480
   Asp Leu Val Asp Lys Glu Lys Ser Ala Glu Ala Phe Ile His Arg Met
                   485                 490                 495
   Thr Asn Asn Asp Leu Tyr Leu Pro Glu Glu Lys Val Leu Pro Lys His
               500                 505                 510
   Ser Leu Ile Tyr Glu Lys Phe Thr Val Tyr Asn Glu Leu Thr Lys Val
           515                 520                 525
   Arg Tyr Lys Asn Glu Gln Gly Glu Thr Tyr Phe Phe Asp Ser Asn Val
       530                 535                 540
   Lys Gln Glu Ile Phe Asp Gly Val Phe Lys Glu His Arg Lys Val Ser
   545                 550                 555                 560
   Lys Lys Gln Leu Leu Asp Phe Leu Ala Lys Glu Phe Glu Glu Phe Arg
                   565                 570                 575
   Ile Val Asp Val Thr Gly Leu Asp Lys Glu Asn Lys Ala Phe Asn Ala
               580                 585                 590
   Ser Leu Gly Thr Tyr His Asp Leu Lys Lys Ile Leu Asp Lys Asp Phe
           595                 600                 605
   Leu Asp Asn Pro Asp Asn Glu Ser Ile Leu Glu Asp Ile Val Gln Thr
       610                 615                 620
   Ile Thr Leu Phe Glu Asp Arg Glu Met Ile Lys Lys Arg Leu Glu Asn
   625                 630                 635                 640
   Tyr Lys Asp Leu Phe Thr Glu Ser Gln Leu Lys Lys Leu Tyr Arg Arg
                   645                 650                 655
   His Tyr Thr Gly Trp Gly Arg Leu Ser Ala Lys Leu Ile Asn Gly Ile
               660                 665                 670
   Arg Asp Arg Glu Ser Gln Lys Ile Ile Leu Asp Tyr Leu Ile Ser Asp
           675                 680                 685
   Gly Arg Ala Asn Arg Asn Phe Met Gln Leu Ile Asn Asp Asp Gly Leu
       690                 695                 700
   Ser Phe Lys Ser Ile Ile Ser Lys Ala Gln Ser Gly Ser His Ser Asp
   705                 710                 715                 720
   Asn Leu Lys Glu Val Val Gly Glu Leu Ala Gly Ser Pro Ala Ile Lys
                   725                 730                 735
   Lys Gly Ile Leu Gln Ser Leu Lys Ile Val Asp Glu Leu Val Lys Val
               740                 745                 750
   Met Gly Tyr Glu Pro Glu Gln Ile Val Val Glu Met Ala Arg Glu Asn
           755                 760                 765
   Gln Thr Thr Asn Gln Gly Arg Arg Asn Ser Arg Gln Arg Tyr Lys Leu
       770                 775                 780
   Leu Glu Asp Gly Val Lys Asn Leu Ala Ser Asp Leu Asn Gly Asp Ile
   785                 790                 795                 800
   Leu Lys Glu Tyr Pro Thr Asp Asn Gln Ala Leu Gln Asn Glu Arg Leu
                   805                 810                 815
   Phe Leu Tyr Tyr Leu Gln Asn Gly Arg Asp Met Tyr Thr Gly Glu Ala
               820                 825                 830
   Leu Asp Ile Asp Ser Leu Ser Gln Tyr Asp Ile Asp His Ile Val Pro
           835                 840                 845
   Gln Ala Phe Ile Lys Asp Asp Ser Ile Asp Asn Arg Val Leu Val Ser
       850                 855                 860
   Ser Ala Lys Asn Arg Gly Lys Ser Asp Asp Val Pro Ser Ile Asp Ile
   865                 870                 875                 880
   Val Lys Ala Arg Lys Ala Phe Trp Lys Lys Leu Leu Asp Ala Lys Leu
                   885                 890                 895
   Ile Ser Gln Arg Lys Tyr Asp Asn Leu Thr Lys Ala Glu Arg Gly Gly
               900                 905                 910
   Leu Thr Pro Asp Asp Lys Ala Gly Phe Ile Gln Arg Gln Leu Val Glu
           915                 920                 925
   Thr Arg Gln Ile Thr Lys His Val Ala Arg Ile Leu Asp Glu Arg Phe
       930                 935                 940
   Asn Asn Lys Val Asp Asp Asn Asn Lys Pro Ile Arg Lys Val Lys Ile
   945                 950                 955                 960
   Val Thr Leu Lys Ser Asn Leu Val Ser Asn Phe Arg Lys Glu Phe Gly
                   965                 970                 975
   Phe Tyr Lys Ile Arg Glu Val Asn Asn Tyr His His Ala His Asp Ala
               980                 985                 990
   Tyr Leu Asn Ala Val Val Ala Lys Ala Ile Leu Thr Lys Tyr Pro Gln
           995                 1000                1005
   Leu Glu Pro Glu Phe Val Tyr Gly Asp Tyr Pro Lys Tyr Asn Ser Tyr
       1010                1015                1020
   Lys Thr Arg Lys Ser Ala Thr Glu Lys Leu Phe Phe Tyr Ser Asn Ile
   1025                1030                1035                1040
   Met Asn Phe Phe Lys Thr Lys Val Thr Leu Ala Asp Glu Thr Val Val
                   1045                1050                1055
   Val Lys Asp Asp Ile Glu Val Asn Asn Glu Thr Gly Glu Ile Ala Trp
               1060                1065                1070
   Asp Lys Lys Lys His Phe Ala Thr Val Arg Lys Val Leu Ser Tyr Pro
           1075                1080                1085
   Gln Val Asn Ile Val Lys Lys Thr Glu Val Gln Thr Gly Gly Phe Ser
       1090                1095                1100
   Lys Glu Ser Ile Leu Ala His Ser Asn Ser Asp Lys Leu Ile Pro Arg
   1105                1110                1115                1120
   Lys Thr Lys Asp Ile Tyr Leu Asp Pro Lys Lys Tyr Gly Gly Phe Asp
                   1125                1130                1135
   Ser Pro Ile Val Ala Tyr Ser Val Leu Val Leu Ala Asp Ile Lys Lys
               1140                1145                1150
   Gly Lys Ala Gln Lys Leu Lys Thr Val Lys Glu Leu Ile Gly Ile Thr
           1155                1160                1165
   Ile Met Glu Arg Glu Arg Phe Glu Lys Asn Pro Ser Ala Phe Leu Glu
       1170                1175                1180
   Ser Lys Gly Tyr Leu Asn Ile Arg Thr Asp Lys Leu Ile Ile Leu Pro
   1185                1190                1195                1200
   Lys Tyr Ser Leu Phe Glu Leu Glu Asn Gly Arg Arg Arg Leu Leu Ala
                   1205                1210                1215
   Ser Ala Gly Glu Leu Gln Lys Gly Asn Glu Leu Ala Leu Pro Thr Gln
               1220                1225                1230
   Tyr Met Lys Phe Leu Tyr Leu Ala Ser Arg Tyr Asn Glu Ser Lys Gly
           1235                1240                1245
   Lys Pro Glu Glu Ile Glu Lys Lys Gln Glu Phe Val Asn Gln His Val
       1250                1255                1260
   Ser Tyr Phe Asp Asp Ile Leu Gln Leu Ile Asn Asp Phe Ser Lys Arg
   1265                1270                1275                1280
   Val Ile Leu Ala Asp Ala Asn Leu Glu Lys Ile Asn Lys Leu Tyr Ser
                   1285                1290                1295
   Asp Asn Lys Asp Asn Thr Pro Val Asp Glu Leu Ala Lys Asn Ile Ile
               1300                1305                1310
   Asn Leu Phe Thr Phe Thr Ser Leu Gly Ala Pro Ala Ala Phe Lys Phe
           1315                1320                1325
   Phe Asp Lys Ser Val Asp Arg Lys Arg Tyr Thr Ser Thr Lys Glu Val
       1330                1335                1340
   Leu Asp Ser Thr Leu Ile His Gln Ser Ile Thr Gly Leu Tyr Glu Thr
   1345                1350                1355                1360
   Arg Ile Asp Leu Gly Lys Leu Gly Glu Asp
                   1365                1370
<210> 192
   <211> 1123
   <212> PRT
   <213> Francisella tularensis subsp. tularensis
<220>
   <223> CRISPR-associated endonuclease
<308> YP_005830750
   <309> 2013-06-11
<400> 192
   Met Asn Val Lys Ile Leu Pro Ile Ala Ile Asp Leu Asp Val Lys Asn
    1               5                  10                  15
   Thr Gly Val Phe Ser Ala Phe Tyr Gln Lys Gly Thr Ser Leu Glu Lys
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Arg Arg His Lys Arg Arg Gly
       50                  55                  60
   Ile Asp Arg Lys Gln Leu Val Lys Arg Leu Phe Lys Leu Val Trp Thr
   65                  70                  75                  80
   Glu Gln Leu Asn Leu Glu Trp Asp Lys Asp Thr Gln Gln Ala Ile Ser
                   85                  90                  95
   Phe Leu Phe Asn Arg Arg Gly Phe Ser Phe Ile Thr Asp Gly Tyr Ser
               100                 105                 110
   Thr Glu Tyr Leu Asn Ile Val Pro Glu Gln Val Lys Ala Ile Leu Met
           115                 120                 125
   Asp Ile Phe Asp Asp Tyr Asn Gly Glu Asp Asp Leu Asp Ser Tyr Leu
       130                 135                 140
   Lys Leu Ala Thr Glu Gln Glu Ser Lys Ile Ser Glu Ile Tyr Asn Lys
   145                 150                 155                 160
   Leu Met Gln Lys Ile Leu Glu Phe Lys Leu Arg Lys Leu Cys Thr Asp
                   165                 170                 175
   Ile Lys Asp Asp Lys Val Ser Thr Lys Thr Leu Lys Glu Ile Thr Ser
               180                 185                 190
   Tyr Glu Phe Glu Leu Leu Ala Asp Tyr Leu Ala Asn Tyr Ser Glu Ser
           195                 200                 205
   Leu Lys Thr Gln Lys Phe Ser Tyr Thr Asp Lys Gln Gly Asn Leu Lys
       210                 215                 220
   Glu Leu Ser Tyr Tyr His His Asp Lys Tyr Asn Ile Gln Glu Phe Leu
   225                 230                 235                 240
   Lys Arg His Ala Thr Ile Asn Asp Glu Ile Leu Gly Thr Leu Leu Thr
                   245                 250                 255
   Asp Asp Phe Asp Ile Trp Asn Phe Asn Phe Glu Lys Phe Asp Phe Asp
               260                 265                 270
   Lys Asn Glu Glu Lys Leu Gln Asn Gln Glu Asp Lys Asp His Thr Gln
           275                 280                 285
   Ala His Leu His His Phe Val Phe Val Val Asn Lys Ile Lys Ser Glu
       290                 295                 300
   Met Ala Ser Gly Gly Arg His Arg Ser Gln Tyr Phe Gln Glu Ile Thr
   305                 310                 315                 320
   Asn Val Leu Asp Glu Asn Asn His Gln Glu Gly Tyr Leu Lys Asn Phe
                   325                 330                 335
   Cys Glu Asn Leu His Asn Lys Lys Tyr Ser Asn Leu Ser Val Lys Asn
               340                 345                 350
   Leu Val Asn Leu Val Gly Asn Leu Ser Asn Leu Glu Leu Lys Pro Leu
           355                 360                 365
   Arg Lys Tyr Phe Asn Asp Lys Ile His Ala Lys Ala Asp His Trp Asp
       370                 375                 380
   Glu Gln Lys Phe Thr Glu Thr Tyr Cys His Trp Ile Leu Gly Glu Trp
   385                 390                 395                 400
   Arg Val Gly Val Lys Asp Gln Asp Lys Lys Asp Gly Ala Lys Tyr Ser
                   405                 410                 415
   Tyr Lys Asp Leu Cys Asn Glu Leu Lys Gln Lys Val Thr Lys Ala Gly
               420                 425                 430
   Leu Ile Asp Phe Leu Leu Glu Leu Asp Pro Cys Arg Thr Ile Pro Pro
           435                 440                 445
   Tyr Leu Asp Asn Asn Asn Arg Lys Pro Pro Lys Cys Gln Ser Leu Ile
       450                 455                 460
   Leu Asn Pro Lys Phe Leu Asp Asn Gln Tyr Pro Asn Trp Gln Gln Tyr
   465                 470                 475                 480
   Leu Gln Glu Leu Lys Lys Leu Gln Ser Ile Gln Asp Tyr Leu Asp Ser
                   485                 490                 495
   Phe Glu Thr Asp Leu Lys Asp Leu Lys Ser Ser Lys Asp Gln Pro Tyr
               500                 505                 510
   Phe Val Glu Tyr Lys Ser Ser Asn Gln Gln Met Ala Ser Gly Gln Arg
           515                 520                 525
   Asp Tyr Lys Asp Leu Asp Ala Arg Ile Leu Gln Phe Ile Phe Asp Arg
       530                 535                 540
   Val Lys Ala Ser Asp Glu Leu Leu Leu Asn Glu Ile Tyr Phe Gln Ala
   545                 550                 555                 560
   Lys Lys Leu Lys Gln Lys Ala Ser Ser Glu Leu Glu Lys Leu Glu Ser
                   565                 570                 575
   Ser Lys Lys Leu Asp Glu Val Ile Ala Asn Ser Gln Leu Ser Gln Ile
               580                 585                 590
   Leu Lys Ser Gln His Thr Asn Gly Ile Phe Glu Gln Gly Thr Phe Leu
           595                 600                 605
   His Leu Val Cys Lys Tyr Tyr Lys Gln Arg Gln Arg Ala Arg Asp Ser
       610                 615                 620
   Arg Leu Tyr Ile Met Pro Glu Tyr Arg Tyr Asp Lys Lys Leu Asp Lys
   625                 630                 635                 640
   Tyr Asn Asn Thr Gly Arg Phe Asp Asp Asn Asn Gln Leu Leu Thr Tyr
   645 650 655
   Cys Asn His Lys Pro Arg Gln Lys Arg Tyr Gln Leu Leu Asn Asp Leu
   660 665 670
   Ala Gly Val Leu Gln Val Ser Arg Asn Gln Leu Leu Ser Ser Val Glu
   675 680 685
   Glu Trp Phe Gln Gln Ala Gln Arg Val Gly Glu Ile Ser Lys Ser Gln
   690 695 700
   Asp Glu Gln Ile Phe Glu Trp Leu Lys Ser Phe Lys Ile Ala Ser Tyr
   705 710 715 720
   Cys Lys Ala Ala Val Glu Met Gln Lys Gln Tyr Arg Gly Thr Leu Lys
   725 730 735
   Asn Ala Ile Gln Thr Ala Ile Phe Arg Gln Ser Glu Asn Ile Asn Lys
   740 745 750
   Asn Lys Asn Thr Gly Asn Gln Gln Gln Ala Leu Ser Glu Asn Ser Lys
   755 760 765
   Asp Val Lys Ser Leu Thr Ala Asp Glu Lys Lys Leu Leu Lys Leu Ile
   770 775 780
   Glu Asn Ile Ala Lys Ala Ser Gln Lys Ile Gly Glu Ser Leu Gly Leu
   785 790 795 800
   Asn Asp Lys Gln Ile Lys Lys Phe Asn Ser Ile Tyr Ser Phe Ala Gln
   805 810 815
   Ile Gln Gln Ile Ala Phe Ala Lys Arg Lys Gly Asn Ala Asn Thr Cys
   820 825 830
   Ala Val Cys Ser Ala Asp Asn Ala His Arg Met Gln Gln Ile Lys Ile
   835 840 845
   Thr Glu Leu Val Glu Asp Asn Lys Asp Asn Ile Ile Leu Ser Ala Lys
   850 855 860
   Ala Gln Arg Leu Pro Ala Ile Pro Thr Arg Ile Val Asp Gly Ala Val
   865 870 875 880
   Lys Lys Met Ala Thr Ile Leu Ala Lys Asn Ile Val Asp Asp Asn Trp
   885 890 895
   Gln Asn Ile Lys Gln Val Leu Ser Ala Lys His Gln Leu His Ile Pro
   900 905 910
   Ile Ile Thr Glu Ser Asn Ala Phe Glu Phe Glu Pro Ala Leu Ala Asp
   915 920 925
   Val Lys Gly Lys Ser Leu Lys Asp Arg Arg Lys Lys Ala Leu Glu Arg
   930 935 940
   Ile Ser Pro Glu Asn Ile Phe Lys Asp Lys Asn Asn Arg Ile Lys Glu
   945 950 955 960
   Phe Ala Lys Gly Ile Ser Ala Tyr Ser Gly Ala Asn Leu Thr Asp Gly
   965 970 975
   Asp Phe Asp Gly Ala Lys Glu Glu Leu Asp His Ile Ile Pro Arg Ser
   980 985 990
   His Lys Lys Tyr Gly Thr Leu Asn Asp Glu Ala Asn Leu Ile Cys Val
   995 1000 1005
   Thr Arg Asp Asp Asn Lys Asn Ile Phe Ala Ile Asp Thr Ser Lys Trp
   1010 1015 1020
   Phe Glu Ile Glu Thr Pro Ser Asp Leu Arg Asp Ile Gly Val Ala Thr
   1025 1030 1035 1040
   Ile Gln Tyr Lys Ile Asp Asn Asn Ser Arg Pro Lys Val Arg Val Lys
   1045 1050 1055
   Leu Asp Tyr Val Ile Asp Asp Asp Ser Lys Ile Asn Tyr Phe Met Asn
   1060 1065 1070
   His Ser Leu Leu Lys Ser Arg Tyr Pro Asp Lys Val Leu Glu Ile Leu
   1075 1080 1085
   Lys Gln Ser Thr Ile Ile Glu Phe Glu Ser Ser Gly Phe Asn Lys Thr
   1090 1095 1100
   Ile Lys Glu Met Leu Gly Met Thr Leu Ala Gly Ile Tyr Asn Glu Thr
   1105 1110 1115 1120
   Ser Asn Asn
<210> 193
   <211> 1082
   <212> PRT
   <213> Neisseria meningitidis
<220>
   <223> CRISPR-associated endonuclease
<308> YP_005879219
   <309> 2013-06-11
<400> 193
   Met Ala Ala Phe Lys Pro Asn Ser Ile Asn Tyr Ile Leu Gly Leu Asp
    1               5                  10                  15
   Ile Gly Ile Ala Ser Val Gly Trp Ala Met Val Glu Ile Asp Glu Glu
               20                  25                  30
   Glu Asn Pro Ile Arg Leu Ile Asp Leu Gly Val Arg Val Phe Glu Arg
           35                  40                  45
   Ala Glu Val Pro Lys Thr Gly Asp Ser Leu Ala Met Ala Arg Arg Leu
       50                  55                  60
   Ala Arg Ser Val Arg Arg Leu Thr Arg Arg Arg Ala His Arg Leu Leu
   65                  70                  75                  80
   Arg Thr Arg Arg Leu Leu Lys Arg Glu Gly Val Leu Gln Ala Ala Asn
                   85                  90                  95
   Phe Asp Glu Asn Gly Leu Ile Lys Ser Leu Pro Asn Thr Pro Trp Gln
               100                 105                 110
   Leu Arg Ala Ala Ala Leu Asp Arg Lys Leu Thr Pro Leu Glu Trp Ser
           115                 120                 125
   Ala Val Leu Leu His Leu Ile Lys His Arg Gly Tyr Leu Ser Gln Arg
       130                 135                 140
   Lys Asn Glu Gly Glu Thr Ala Asp Lys Glu Leu Gly Ala Leu Leu Lys
   145                 150                 155                 160
   Gly Val Ala Gly Asn Ala His Ala Leu Gln Thr Gly Asp Phe Arg Thr
                   165                 170                 175
   Pro Ala Glu Leu Ala Leu Asn Lys Phe Glu Lys Glu Ser Gly His Ile
               180                 185                 190
   Arg Asn Gln Arg Ser Asp Tyr Ser His Thr Phe Ser Arg Lys Asp Leu
           195                 200                 205
   Gln Ala Glu Leu Ile Leu Leu Phe Glu Lys Gln Lys Glu Phe Gly Asn
       210                 215                 220
   Pro His Val Ser Gly Gly Leu Lys Glu Gly Ile Glu Thr Leu Leu Met
   225                 230                 235                 240
   Thr Gln Arg Pro Ala Leu Ser Gly Asp Ala Val Gln Lys Met Leu Gly
                   245                 250                 255
   His Cys Thr Phe Glu Pro Ala Glu Pro Lys Ala Ala Lys Asn Thr Tyr
               260                 265                 270
   Thr Ala Glu Arg Phe Ile Trp Leu Thr Lys Leu Asn Asn Leu Arg Ile
           275                 280                 285
   Leu Glu Gln Gly Ser Glu Arg Pro Leu Thr Asp Thr Glu Arg Ala Thr
       290                 295                 300
   Leu Met Asp Glu Pro Tyr Arg Lys Ser Lys Leu Thr Tyr Ala Gln Ala
   305                 310                 315                 320
   Arg Lys Leu Leu Gly Leu Glu Asp Thr Ala Phe Phe Lys Gly Leu Arg
                   325                 330                 335
   Tyr Gly Lys Asp Asn Ala Glu Ala Ser Thr Leu Met Glu Met Lys Ala
               340                 345                 350
   Tyr His Ala Ile Ser Arg Ala Leu Glu Lys Glu Gly Leu Lys Asp Lys
           355                 360                 365
   Lys Ser Pro Leu Asn Leu Ser Pro Glu Leu Gln Asp Glu Ile Gly Thr
       370                 375                 380
   Ala Phe Ser Leu Phe Lys Thr Asp Glu Asp Ile Thr Gly Arg Leu Lys
   385                 390                 395                 400
   Asp Arg Ile Gln Pro Glu Ile Leu Glu Ala Leu Leu Lys His Ile Ser
   405 410 415
   Phe Asp Lys Phe Val Gln Ile Ser Leu Lys Ala Leu Arg Arg Ile Val
   420 425 430
   Pro Leu Met Glu Gln Gly Lys Arg Tyr Asp Glu Ala Cys Ala Glu Ile
   435 440 445
   Tyr Gly Asp His Tyr Gly Lys Lys Asn Thr Glu Glu Lys Ile Tyr Leu
   450 455 460
   Pro Pro Ile Pro Ala Asp Glu Ile Arg Asn Pro Val Val Leu Arg Ala
   465 470 475 480
   Leu Ser Gln Ala Arg Lys Val Ile Asn Gly Val Val Arg Arg Tyr Gly
   485 490 495
   Ser Pro Ala Arg Ile His Ile Glu Thr Ala Arg Glu Val Gly Lys Ser
   500 505 510
   Phe Lys Asp Arg Lys Glu Ile Glu Lys Arg Gln Glu Glu Asn Arg Lys
   515 520 525
   Asp Arg Glu Lys Ala Ala Ala Lys Phe Arg Glu Tyr Phe Pro Asn Phe
   530 535 540
   Val Gly Glu Pro Lys Ser Lys Asp Ile Leu Lys Leu Arg Leu Tyr Glu
   545 550 555 560
   Gln Gln His Gly Lys Cys Leu Tyr Ser Gly Lys Glu Ile Asn Leu Gly
   565 570 575
   Arg Leu Asn Glu Lys Gly Tyr Val Glu Ile Asp His Ala Leu Pro Phe
   580 585 590
   Ser Arg Thr Trp Asp Asp Ser Phe Asn Asn Lys Val Leu Val Leu Gly
   595 600 605
   Ser Glu Asn Gln Asn Lys Gly Asn Gln Thr Pro Tyr Glu Tyr Phe Asn
   610 615 620
   Gly Lys Asp Asn Ser Arg Glu Trp Gln Glu Phe Lys Ala Arg Val Glu
   625 630 635 640
   Thr Ser Arg Phe Pro Arg Ser Lys Lys Gln Arg Ile Leu Leu Gln Lys
   645 650 655
   Phe Asp Glu Asp Gly Phe Lys Glu Arg Asn Leu Asn Asp Thr Arg Tyr
   660 665 670
   Val Asn Arg Phe Leu Cys Gln Phe Val Ala Asp Arg Met Arg Leu Thr
   675 680 685
   Gly Lys Gly Lys Lys Arg Val Phe Ala Ser Asn Gly Gln Ile Thr Asn
   690 695 700
   Leu Leu Arg Gly Phe Trp Gly Leu Arg Lys Val Arg Ala Glu Asn Asp
   705 710 715 720
   Arg His His Ala Leu Asp Ala Val Val Val Ala Cys Ser Thr Val Ala
   725 730 735
   Met Gln Gln Lys Ile Thr Arg Phe Val Arg Tyr Lys Glu Met Asn Ala
   740 745 750
   Phe Asp Gly Lys Thr Ile Asp Lys Glu Thr Gly Glu Val Leu His Gln
   755 760 765
   Lys Thr His Phe Pro Gln Pro Trp Glu Phe Phe Ala Gln Glu Val Met
   770 775 780
   Ile Arg Val Phe Gly Lys Pro Asp Gly Lys Pro Glu Phe Glu Glu Ala
   785 790 795 800
   Asp Thr Leu Glu Lys Leu Arg Thr Leu Leu Ala Glu Lys Leu Ser Ser
   805 810 815
   Arg Pro Glu Ala Val His Glu Tyr Val Thr Pro Leu Phe Val Ser Arg
   820 825 830
   Ala Pro Asn Arg Lys Met Ser Gly Gln Gly His Met Glu Thr Val Lys
   835 840 845
   Ser Ala Lys Arg Leu Asp Glu Gly Val Ser Val Leu Arg Val Pro Leu
   850 855 860
   Thr Gln Leu Lys Leu Lys Asp Leu Glu Lys Met Val Asn Arg Glu Arg
   865 870 875 880
   Glu Pro Lys Leu Tyr Glu Ala Leu Lys Ala Arg Leu Glu Ala His Lys
   885 890 895
   Asp Asp Pro Ala Lys Ala Phe Ala Glu Pro Phe Tyr Lys Tyr Asp Lys
                   900                 905                 910
   Ala Gly Asn Arg Thr Gln Gln Val Lys Ala Val Arg Val Glu Gln Val
               915                 920                 925
   Gln Lys Thr Gly Val Trp Val Arg Asn His Asn Gly Ile Ala Asp Asn
       930                 935                 940
   Ala Thr Met Val Arg Val Asp Val Phe Glu Lys Gly Asp Lys Tyr Tyr
   945                 950                 955                 960
   Leu Val Pro Ile Tyr Ser Trp Gln Val Ala Lys Gly Ile Leu Pro Asp
                   965                 970                 975
   Arg Ala Val Val Gln Gly Lys Asp Glu Glu Asp Trp Gln Leu Ile Asp
               980                 985                 990
   Asp Ser Phe Asn Phe Lys Phe Ser Leu His Pro Asn Asp Leu Val Glu
           995                 1000                1005
   Val Ile Thr Lys Lys Ala Arg Met Phe Gly Tyr Phe Ala Ser Cys His
       1010                1015                1020
   Arg Gly Thr Gly Asn Ile Asn Ile Arg Ile His Asp Leu Asp His Lys
   1025                1030                1035                1040
   Ile Gly Lys Asn Gly Ile Leu Glu Gly Ile Gly Val Lys Thr Ala Leu
                   1045                1050                1055
   Ser Phe Gln Lys Tyr Gln Ile Asp Glu Leu Gly Lys Glu Ile Arg Pro
               1060                1065                1070
   Cys Arg Leu Lys Lys Arg Pro Pro Val Arg
           1075                1080
<210> 194
   <211> 861
   <212> PRT
   <213> Campylobacter jejuni
<220>
   <223> CRISPR-associated endonuclease
<308> YP_005658406
   <309> 2013-06-11
<400> 194
   Met Ala Arg Ile Leu Ala Phe Asp Ile Gly Ile Ser Ser Ile Gly Trp
   1               5                   10                  15
   Ala Phe Ser Glu Asn Asp Glu Leu Lys Asp Cys Gly Val Arg Ile Phe
               20                  25                  30
   Thr Lys Ala Glu Asn Pro Lys Thr Gly Glu Ser Leu Ala Leu Pro Arg
           35                  40                  45
   Arg Leu Ala Arg Ser Ala Arg Lys Arg Leu Ala Arg Arg Lys Ala Arg
       50                  55                  60
   Leu Asn His Leu Lys His Leu Ile Ala Asn Glu Phe Lys Leu Asn Tyr
   65                  70                  75                  80
   Glu Asp Tyr Gln Ser Phe Asp Glu Ser Leu Ala Lys Ala Tyr Lys Gly
                   85                  90                  95
   Ser Leu Ile Ser Pro Tyr Glu Leu Arg Phe Arg Ala Leu Asn Glu Leu
               100                 105                 110
   Leu Ser Lys Gln Asp Phe Ala Arg Val Ile Leu His Ile Ala Lys Arg
           115                 120                 125
   Arg Gly Tyr Asp Asp Ile Lys Asn Asn Gly Asp Glu Glu Lys Ser Glu
       130                 135                 140
   Ile Leu Lys Ala Ile Lys Gln Asn Glu Glu Lys Leu Val Asn Tyr Gln
   145                 150                 155                 160
   Ser Val Gly Glu Tyr Leu Tyr Lys Glu Tyr Phe Gln Lys Phe Lys Glu
                   165                 170                 175
   Asn Ser Lys Glu Phe Ile Asn Val Arg Asn Lys Lys Glu Ser Tyr Glu
               180                 185                 190
   Arg Cys Ile Thr Gln Ser Phe Leu Lys Asp Glu Leu Lys Leu Ile Phe
           195                 200                 205
   Gln Lys Gln Arg Glu Phe Gly Phe Ser Phe Ser Lys Lys Phe Glu Glu
       210                 215                 220
   Glu Val Leu Ser Val Ala Phe Tyr Lys Arg Ala Leu Lys Asp Phe Ser
   225                 230                 235                 240
   His Leu Val Gly Asn Cys Ser Phe Phe Thr Asp Glu Lys Arg Ala Pro
                   245                 250                 255
   Lys Asn Ser Pro Leu Ala Phe Met Phe Val Ala Leu Thr Arg Ile Ile
               260                 265                 270
   Asn Ile Leu Asn Asn Leu Lys Asn Thr Glu Gly Ile Leu Tyr Thr Lys
           275                 280                 285
   Asp Asp Leu Asn Ala Leu Leu Asn Glu Val Leu Lys Asn Gly Thr Leu
       290                 295                 300
   Thr Tyr Lys Gln Thr Lys Lys Leu Leu Gly Leu Ser Asp Asp Tyr Glu
   305                 310                 315                 320
   Phe Lys Gly Glu Lys Gly Thr Tyr Phe Ile Glu Phe Lys Lys Tyr Lys
                   325                 330                 335
   Glu Phe Ile Lys Ala Leu Gly Glu His Asn Leu Ser Gln Asp Asp Leu
               340                 345                 350
   Asn Glu Ile Ala Lys Asp Ile Thr Leu Ile Lys Asp Glu Ile Lys Leu
           355                 360                 365
   Lys Lys Ala Leu Ala Lys Tyr Asp Leu Asn Gln Asn Gln Ile Asp Ser
       370                 375                 380
   Leu Ser Lys Leu Glu Phe Lys Asp His Leu Asn Ile Ser Phe Lys Ala
   385                 390                 395                 400
   Leu Lys Leu Ile Thr Pro Leu Met Leu Glu Gly Lys Lys Tyr Asp Glu
                   405                 410                 415
   Ala Cys Asn Glu Leu Asn Leu Lys Val Ala Ile Asn Glu Asp Lys Lys
               420                 425                 430
   Asp Phe Leu Pro Ala Phe Asn Glu Thr Tyr Tyr Lys Asp Glu Val Thr
           435                 440                 445
   Asn Pro Val Val Leu Arg Ala Ile Lys Glu Tyr Arg Lys Ile Leu Asn
       450                 455                 460
   Ala Leu Leu Lys Lys Tyr Gly Lys Val His Lys Ile Asn Ile Glu Leu
   465                 470                 475                 480
   Ala Arg Glu Val Gly Lys Asn His Ser Gln Arg Ala Lys Ile Glu Lys
                   485                 490                 495
   Glu Gln Asn Glu Asn Tyr Lys Ala Lys Lys Asp Ala Glu Ile Glu Cys
               500                 505                 510
   Glu Lys Leu Gly Leu Lys Ile Asn Ser Lys Asn Ile Leu Lys Leu Arg
           515                 520                 525
   Leu Phe Lys Glu Gln Lys Glu Phe Cys Ala Tyr Ser Gly Glu Lys Ile
       530                 535                 540
   Lys Leu Ser Asp Leu Gln Asp Glu Lys Met Leu Glu Ile Asp His Ile
   545                 550                 555                 560
   Tyr Pro Tyr Ser Arg Ser Phe Asp Asp Ser Tyr Met Asn Lys Val Leu
                   565                 570                 575
   Val Phe Thr Lys Gln Asn Gln Glu Lys Leu Asn Lys Thr Pro Phe Glu
               580                 585                 590
   Ala Phe Gly Asn Asp Ser Thr Lys Trp Gln Lys Ile Glu Val Leu Ala
           595                 600                 605
   Lys Asn Leu Pro Glu Lys Lys Gln Lys Arg Ile Leu Asp Lys Asn Tyr
       610                 615                 620
   Lys Asp Lys Glu Gln Lys Asp Phe Lys Asp Arg Asn Leu Asn Asp Thr
   625                 630                 635                 640
   Arg Tyr Ile Ala Arg Leu Val Leu Asn Tyr Thr Lys Asp Tyr Leu Asp
                   645                 650                 655
   Phe Leu Pro Leu Ser Asp Asp Glu Asn Thr Lys Leu Asn Asp Thr Gln
               660                 665                 670
   Lys Gly Ser Lys Val His Val Glu Ala Lys Ser Gly Met Leu Thr Ser
           675                 680                 685
   Ala Leu Arg His Thr Trp Gly Phe Ser Ala Lys Asp Arg Asn Asn His
       690                 695                 700
   Leu His His Ala Ile Asp Ala Ala Ile Ile Ala Tyr Ala Asn Asn Ser
   705                 710                 715                 720
   Ile Val Lys Ala Phe Ser Asp Phe Lys Lys Glu Gln Glu Ser Asn Ser
                   725                 730                 735
   Val Glu Leu Tyr Ala Lys Lys Ile Ser Glu Leu Asp Tyr Lys Asn Lys
               740                 745                 750
   Arg Lys Phe Phe Glu Pro Phe Ser Gly Phe Arg Gln Lys Val Leu Asp
           755                 760                 765
   Lys Ile Asp Glu Ile Phe Val Ser Lys Pro Glu Arg Lys Lys Pro Ser
       770                 775                 780
   Gly Ala Leu His Glu Glu Thr Phe Arg Lys Glu Glu Glu Phe Tyr Gln
   785                 790                 795                 800
   Ser Tyr Gly Gly Lys Glu Gly Val Leu Lys Ala Leu Glu Leu Gly Lys
                   805                 810                 815
   Ile Arg Lys Val Asn Gly Lys Ile Val Lys Asn Gly Asp Met Phe Arg
               820                 825                 830
   Val Asp Ile Phe Lys His Lys Lys Thr Asn Lys Phe Leu Cys Cys Ala
           835                 840                 845
   Asn Leu Tyr Tyr Gly Phe Cys Phe Lys Ser Ile Thr Lys
       850                 855                 860
<210> 195
   <211> 1123
   <212> PRT
   <213> Francisella tularensis
<220>
   <223> CRISPR-associated endonuclease
<308> YP_005305300
   <309> 2013-07-06
<400> 195
   Met Asn Val Lys Ile Leu Pro Ile Ala Ile Asp Leu Asp Val Lys Asn
    1               5                  10                  15
   Thr Gly Val Phe Ser Ala Phe Tyr Gln Lys Gly Thr Ser Leu Glu Lys
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Arg Arg His Lys Arg Arg Gly
       50                  55                  60
   Ile Asp Arg Lys Gln Leu Val Lys Arg Leu Phe Lys Leu Val Trp Thr
   65                  70                  75                  80
   Glu Gln Leu Asn Leu Glu Trp Asp Lys Asp Thr Gln Gln Ala Ile Ser
                   85                  90                  95
   Phe Leu Phe Asn Arg Arg Gly Phe Ser Phe Ile Thr Asp Gly Tyr Ser
               100                 105                 110
   Thr Glu Tyr Leu Asn Ile Val Pro Glu Gln Val Lys Ala Ile Leu Met
           115                 120                 125
   Asp Ile Phe Asp Asp Tyr Asn Gly Glu Asp Asp Leu Asp Ser Tyr Leu
       130                 135                 140
   Lys Leu Ala Thr Glu Gln Glu Ser Lys Ile Ser Glu Ile Tyr Asn Lys
   145                 150                 155                 160
   Leu Met Gln Lys Ile Leu Glu Phe Lys Leu Arg Lys Leu Cys Thr Asp
                   165                 170                 175
   Ile Lys Asp Asp Lys Val Ser Thr Lys Thr Leu Lys Glu Ile Thr Ser
               180                 185                 190
   Tyr Glu Phe Glu Leu Leu Ala Asp Tyr Leu Ala Asn Tyr Ser Glu Ser
           195                 200                 205
   Leu Lys Thr Gln Lys Phe Ser Tyr Thr Asp Lys Gln Gly Asn Leu Lys
       210                 215                 220
   Glu Leu Ser Tyr Tyr His His Asp Lys Tyr Asn Ile Gln Glu Phe Leu
   225                 230                 235                 240
   Lys Arg His Ala Thr Ile Asn Asp Glu Ile Leu Gly Thr Leu Leu Thr
                   245                 250                 255
   Asp Asp Phe Asp Ile Trp Asn Phe Asn Phe Glu Lys Phe Asp Phe Asp
               260                 265                 270
   Lys Asn Glu Glu Lys Leu Gln Asn Gln Glu Asp Lys Asp His Thr Gln
           275                 280                 285
   Ala His Leu His His Phe Val Phe Val Val Asn Lys Ile Lys Ser Glu
       290                 295                 300
   Met Ala Ser Gly Gly Arg His Arg Ser Gln Tyr Phe Gln Glu Ile Thr
   305                 310                 315                 320
   Asn Val Leu Asp Glu Asn Asn His Gln Glu Gly Tyr Leu Lys Asn Phe
                   325                 330                 335
   Cys Glu Asn Leu His Asn Lys Lys Tyr Ser Asn Leu Ser Val Lys Asn
               340                 345                 350
   Leu Val Asn Leu Val Gly Asn Leu Ser Asn Leu Glu Leu Lys Pro Leu
           355                 360                 365
   Arg Lys Tyr Phe Asn Asp Lys Ile His Ala Lys Ala Asp His Trp Asp
       370                 375                 380
   Glu Gln Lys Phe Thr Glu Thr Tyr Cys His Trp Ile Leu Gly Glu Trp
   385                 390                 395                 400
   Arg Val Gly Val Lys Asp Gln Asp Lys Lys Asp Gly Ala Lys Tyr Ser
                   405                 410                 415
   Tyr Lys Asp Leu Cys Asn Glu Leu Lys Gln Lys Val Thr Lys Ala Gly
               420                 425                 430
   Leu Ile Asp Phe Leu Leu Glu Leu Asp Pro Cys Arg Thr Ile Pro Pro
           435                 440                 445
   Tyr Leu Asp Asn Asn Asn Arg Lys Pro Pro Lys Cys Gln Ser Leu Ile
       450                 455                 460
   Leu Asn Pro Lys Phe Leu Asp Asn Gln Tyr Pro Asn Trp Gln Gln Tyr
   465                 470                 475                 480
   Leu Gln Glu Leu Lys Lys Leu Gln Ser Ile Gln Asp Tyr Leu Asp Ser
                   485                 490                 495
   Phe Glu Thr Asp Leu Lys Asp Leu Lys Ser Ser Lys Asp Gln Pro Tyr
               500                 505                 510
   Phe Val Glu Tyr Lys Ser Ser Asn Gln Gln Met Ala Ser Gly Gln Arg
           515                 520                 525
   Asp Tyr Lys Asp Leu Asp Ala Arg Ile Leu Gln Phe Ile Phe Asp Arg
       530                 535                 540
   Val Lys Ala Ser Asp Glu Leu Leu Leu Asn Glu Ile Tyr Phe Gln Ala
   545                 550                 555                 560
   Lys Lys Leu Lys Gln Lys Ala Ser Ser Glu Leu Glu Lys Leu Glu Ser
                   565                 570                 575
   Ser Lys Lys Leu Asp Glu Val Ile Ala Asn Ser Gln Leu Ser Gln Ile
               580                 585                 590
   Leu Lys Ser Gln His Thr Asn Gly Ile Phe Glu Gln Gly Thr Phe Leu
           595                 600                 605
   His Leu Val Cys Lys Tyr Tyr Lys Gln Arg Gln Arg Ala Arg Asp Ser
       610                 615                 620
   Arg Leu Tyr Ile Met Pro Glu Tyr Arg Tyr Asp Lys Lys Leu Asp Lys
   625                 630                 635                 640
   Tyr Asn Asn Thr Gly Arg Phe Asp Asp Asn Asn Gln Leu Leu Thr Tyr
                   645                 650                 655
   Cys Asn His Lys Pro Arg Gln Lys Arg Tyr Gln Leu Leu Asn Asp Leu
               660                 665                 670
   Ala Gly Val Leu Gln Val Ser Arg Asn Gln Leu Leu Ser Ser Val Glu
           675                 680                 685
   Glu Trp Phe Gln Gln Ala Gln Arg Val Gly Glu Ile Ser Lys Ser Gln
       690                 695                 700
   Asp Glu Gln Ile Phe Glu Trp Leu Lys Ser Phe Lys Ile Ala Ser Tyr
   705                 710                 715                 720
   Cys Lys Ala Ala Val Glu Met Gln Lys Gln Tyr Arg Gly Thr Leu Lys
                   725                 730                 735
   Asn Ala Ile Gln Thr Ala Ile Phe Arg Gln Ser Glu Asn Ile Asn Lys
   740 745 750
   Asn Lys Asn Thr Gly Asn Gln Gln Gln Ala Leu Ser Glu Asn Ser Lys
   755 760 765
   Asp Val Lys Ser Leu Thr Ala Asp Glu Lys Lys Leu Leu Lys Leu Ile
   770 775 780
   Glu Asn Ile Ala Lys Ala Ser Gln Lys Ile Gly Glu Ser Leu Gly Leu
   785 790 795 800
   Asn Asp Lys Gln Ile Lys Lys Phe Asn Ser Ile Tyr Ser Phe Ala Gln
   805 810 815
   Ile Gln Gln Ile Ala Phe Ala Lys Arg Lys Gly Asn Ala Asn Thr Cys
   820 825 830
   Ala Val Cys Ser Ala Asp Asn Ala His Arg Met Gln Gln Ile Lys Ile
   835 840 845
   Thr Glu Leu Val Glu Asp Asn Lys Asp Asn Ile Ile Leu Ser Ala Lys
   850 855 860
   Ala Gln Arg Leu Pro Ala Ile Pro Thr Arg Ile Val Asp Gly Ala Val
   865 870 875 880
   Lys Lys Met Ala Thr Ile Leu Ala Lys Asn Ile Val Asp Asp Asn Trp
   885 890 895
   Gln Asn Ile Lys Gln Val Leu Ser Ala Lys His Gln Leu His Ile Pro
   900 905 910
   Ile Ile Thr Glu Ser Asn Ala Phe Glu Phe Glu Pro Ala Leu Ala Asp
   915 920 925
   Val Lys Gly Lys Ser Leu Lys Asp Arg Arg Lys Lys Ala Leu Glu Arg
   930 935 940
   Ile Ser Pro Glu Asn Ile Phe Lys Asp Lys Asn Asn Arg Ile Lys Glu
   945 950 955 960
   Phe Ala Lys Gly Ile Ser Ala Tyr Ser Gly Ala Asn Leu Thr Asp Gly
   965 970 975
   Asp Phe Asp Gly Ala Lys Glu Glu Leu Asp His Ile Ile Pro Arg Ser
   980 985 990
   His Lys Lys Tyr Gly Thr Leu Asn Asp Glu Ala Asn Leu Ile Cys Val
   995 1000 1005
   Thr Arg Asp Asp Asn Lys Asn Ile Phe Ala Ile Asp Thr Ser Lys Trp
   1010 1015 1020
   Phe Glu Ile Glu Thr Pro Ser Asp Leu Arg Asp Ile Gly Val Ala Thr
   1025 1030 1035 1040
   Ile Gln Tyr Lys Ile Asp Asn Asn Ser Arg Pro Lys Val Arg Val Lys
   1045 1050 1055
   Leu Asp Tyr Val Ile Asp Asp Asp Ser Lys Ile Asn Tyr Phe Met Asn
   1060 1065 1070
   His Ser Leu Leu Lys Ser Arg Tyr Pro Asp Lys Val Leu Glu Ile Leu
   1075 1080 1085
   Lys Gln Ser Thr Ile Ile Glu Phe Glu Ser Ser Gly Phe Asn Lys Thr
   1090 1095 1100
   Ile Lys Glu Met Leu Gly Met Thr Leu Ala Gly Ile Tyr Asn Glu Thr
   1105 1110 1115 1120
   Ser Asn Asn
<210> 196
   <211> 1082
   <212> PRT
   <213> Neisseria meningitidis
<220>
   <223> CRISPR-associated endonuclease
<308> YP_005891308
   <309> 2013-06-11
<400> 196
   Met Ala Ala Phe Lys Pro Asn Pro Ile Asn Tyr Ile Leu Gly Leu Asp
   1               5                   10                  15
   Ile Gly Ile Ala Ser Val Gly Trp Ala Met Val Glu Ile Asp Glu Asp
               20                  25                  30
   Glu Asn Pro Ile Cys Leu Ile Asp Leu Gly Val Arg Val Phe Glu Arg
           35                  40                  45
   Ala Glu Val Pro Lys Thr Gly Asp Ser Leu Ala Met Ala Arg Arg Leu
       50                  55                  60
   Ala Arg Ser Val Arg Arg Leu Thr Arg Arg Arg Ala His Arg Leu Leu
   65                  70                  75                  80
   Arg Ala Arg Arg Leu Leu Lys Arg Glu Gly Val Leu Gln Ala Ala Asp
                   85                  90                  95
   Phe Asp Glu Asn Gly Leu Ile Lys Ser Leu Pro Asn Thr Pro Trp Gln
               100                 105                 110
   Leu Arg Ala Ala Ala Leu Asp Arg Lys Leu Thr Pro Leu Glu Trp Ser
           115                 120                 125
   Ala Val Leu Leu His Leu Ile Lys His Arg Gly Tyr Leu Ser Gln Arg
       130                 135                 140
   Lys Asn Glu Gly Glu Thr Ala Asp Lys Glu Leu Gly Ala Leu Leu Lys
   145                 150                 155                 160
   Gly Val Ala Asp Asn Ala His Ala Leu Gln Thr Gly Asp Phe Arg Thr
                   165                 170                 175
   Pro Ala Glu Leu Ala Leu Asn Lys Phe Glu Lys Glu Ser Gly His Ile
               180                 185                 190
   Arg Asn Gln Arg Gly Asp Tyr Ser His Thr Phe Ser Arg Lys Asp Leu
           195                 200                 205
   Gln Ala Glu Leu Ile Leu Leu Phe Glu Lys Gln Lys Glu Phe Gly Asn
       210                 215                 220
   Pro His Val Ser Gly Gly Leu Lys Glu Gly Ile Glu Thr Leu Leu Met
   225                 230                 235                 240
   Thr Gln Arg Pro Ala Leu Ser Ser Asp Ala Val Gln Lys Met Leu Gly
                   245                 250                 255
   His Cys Thr Phe Glu Pro Ala Glu Pro Lys Ala Ala Lys Asn Thr Tyr
               260                 265                 270
   Thr Ala Glu Arg Phe Ile Trp Leu Thr Lys Leu Asn Asn Leu Arg Ile
           275                 280                 285
   Leu Glu Gln Gly Ser Glu Arg Pro Leu Thr Asp Thr Glu Arg Ala Thr
       290                 295                 300
   Leu Met Asp Glu Pro Tyr Arg Lys Ser Lys Leu Thr Tyr Ala Gln Ala
   305                 310                 315                 320
   Arg Lys Leu Leu Gly Leu Glu Asp Thr Ala Phe Phe Lys Gly Leu Arg
                   325                 330                 335
   Tyr Gly Lys Asp Asn Ala Glu Ala Ser Thr Leu Met Glu Met Lys Ala
               340                 345                 350
   Tyr His Ala Ile Ser Arg Ala Leu Glu Lys Glu Gly Leu Lys Asp Lys
           355                 360                 365
   Lys Ser Pro Leu Asn Leu Ser Pro Glu Leu Gln Asp Glu Ile Gly Thr
       370                 375                 380
   Ala Phe Ser Leu Phe Lys Thr Asp Glu Asp Ile Thr Gly Arg Leu Lys
   385                 390                 395                 400
   Asp Arg Ile Gln Pro Glu Ile Leu Glu Ala Leu Leu Lys His Ile Ser
                   405                 410                 415
   Phe Asp Lys Phe Val Gln Ile Ser Leu Lys Ala Leu Arg Arg Ile Val
               420                 425                 430
   Pro Leu Met Glu Gln Gly Lys Arg Tyr Asp Glu Ala Cys Ala Glu Ile
           435                 440                 445
   Tyr Gly Asp His Tyr Gly Lys Lys Asn Thr Glu Glu Lys Ile Tyr Leu
       450                 455                 460
   Pro Pro Ile Pro Ala Asp Glu Ile Arg Asn Pro Val Val Leu Arg Ala
   465                 470                 475                 480
   Leu Ser Gln Ala Arg Lys Val Ile Asn Gly Val Val Arg Arg Tyr Gly
                   485                 490                 495
   Ser Pro Ala Arg Ile His Ile Glu Thr Ala Arg Glu Val Gly Lys Ser
                       500                 505                 510
   Phe Lys Asp Arg Lys Glu Ile Glu Lys Arg Gln Glu Glu Asn Arg Lys
                   515                 520                 525
   Asp Arg Glu Lys Ala Ala Ala Lys Phe Arg Glu Tyr Phe Pro Asn Phe
               530                 535                 540
   Val Gly Glu Pro Lys Ser Lys Asp Ile Leu Lys Leu Arg Leu Tyr Glu
   545                 550                 555                 560
   Gln Gln His Gly Lys Cys Leu Tyr Ser Gly Lys Glu Ile Asn Leu Gly
                   565                 570                 575
   Arg Leu Asn Glu Lys Gly Tyr Val Glu Ile Asp His Ala Leu Pro Phe
               580                 585                 590
   Ser Arg Thr Trp Asp Asp Ser Phe Asn Asn Lys Val Leu Val Leu Gly
           595                 600                 605
   Ser Glu Asn Gln Asn Lys Gly Asn Gln Thr Pro Tyr Glu Tyr Phe Asn
       610                 615                 620
   Gly Lys Asp Asn Ser Arg Glu Trp Gln Glu Phe Lys Ala Arg Val Glu
   625                 630                 635                 640
   Thr Ser Arg Phe Pro Arg Ser Lys Lys Gln Arg Ile Leu Leu Gln Lys
                   645                 650                 655
   Phe Asp Glu Asp Gly Phe Lys Glu Arg Asn Leu Asn Asp Thr Arg Tyr
               660                 665                 670
   Val Asn Arg Phe Leu Cys Gln Phe Val Ala Asp Arg Met Arg Leu Thr
           675                 680                 685
   Gly Lys Gly Lys Lys Arg Val Phe Ala Ser Asn Gly Gln Ile Thr Asn
       690                 695                 700
   Leu Leu Arg Gly Phe Trp Gly Leu Arg Lys Val Arg Ala Glu Asn Asp
   705                 710                 715                 720
   Arg His His Ala Leu Asp Ala Val Val Val Ala Cys Ser Thr Val Ala
                   725                 730                 735
   Met Gln Gln Lys Ile Thr Arg Phe Val Arg Tyr Lys Glu Met Asn Ala
               740                 745                 750
   Phe Asp Gly Lys Thr Ile Asp Lys Glu Thr Gly Glu Val Leu His Gln
           755                 760                 765
   Lys Thr His Phe Pro Gln Pro Trp Glu Phe Phe Ala Gln Glu Val Met
       770                 775                 780
   Ile Arg Val Phe Gly Lys Pro Asp Gly Lys Pro Glu Phe Glu Glu Ala
   785                 790                 795                 800
   Asp Thr Pro Glu Lys Leu Arg Thr Leu Leu Ala Glu Lys Leu Ser Ser
                   805                 810                 815
   Arg Pro Glu Ala Val His Glu Tyr Val Thr Pro Leu Phe Val Ser Arg
               820                 825                 830
   Ala Pro Asn Arg Lys Met Ser Gly Gln Gly His Met Glu Thr Val Lys
           835                 840                 845
   Ser Ala Lys Arg Leu Asp Glu Gly Val Ser Val Leu Arg Val Pro Leu
       850                 855                 860
   Thr Gln Leu Lys Leu Lys Asp Leu Glu Lys Met Val Asn Arg Glu Arg
   865                 870                 875                 880
   Glu Pro Lys Leu Tyr Glu Ala Leu Lys Ala Arg Leu Glu Ala His Lys
                   885                 890                 895
   Asp Asp Pro Ala Lys Ala Phe Ala Glu Pro Phe Tyr Lys Tyr Asp Lys
               900                 905                 910
   Ala Gly Asn Arg Thr Gln Gln Val Lys Ala Val Arg Val Glu Gln Val
           915                 920                 925
   Gln Lys Thr Gly Val Trp Val Arg Asn His Asn Gly Ile Ala Asp Asn
       930                 935                 940
   Ala Thr Met Val Arg Val Asp Val Phe Glu Lys Gly Asp Lys Tyr Tyr
   945                 950                 955                 960
   Leu Val Pro Ile Tyr Ser Trp Gln Val Ala Lys Gly Ile Leu Pro Asp
                   965                 970                 975
   Arg Ala Val Val Gln Gly Lys Asp Glu Glu Asp Trp Gln Leu Ile Asp
               980                 985                 990
   Asp Ser Phe Asn Phe Lys Phe Ser Leu His Pro Asn Asp Leu Val Glu
           995                 1000                1005
   Val Ile Thr Lys Lys Ala Arg Met Phe Gly Tyr Phe Ala Ser Cys His
       1010                1015                1020
   Arg Gly Thr Gly Asn Ile Asn Ile Arg Ile His Asp Leu Asp His Lys
   1025                1030                1035                1040
   Ile Gly Lys Asn Gly Ile Leu Glu Gly Ile Gly Val Lys Thr Ala Leu
                   1045                1050                1055
   Ser Phe Gln Lys Tyr Gln Ile Asp Glu Leu Gly Lys Glu Ile Arg Pro
               1060                1065                1070
   Cys Arg Leu Lys Lys Arg Pro Pro Val Arg
           1075                1080
<210> 197
   <211> 1082
   <212> PRT
   <213> Wolinella succinogenes
<220>
   <223> CRISPR-associated endonuclease
<308> YP_005891308
   <309> 2013-06-11
<400> 197
   Met Ala Ala Phe Lys Pro Asn Pro Ile Asn Tyr Ile Leu Gly Leu Asp
    1               5                  10                  15
   Ile Gly Ile Ala Ser Val Gly Trp Ala Met Val Glu Ile Asp Glu Asp
               20                  25                  30
   Glu Asn Pro Ile Cys Leu Ile Asp Leu Gly Val Arg Val Phe Glu Arg
           35                  40                  45
   Ala Glu Val Pro Lys Thr Gly Asp Ser Leu Ala Met Ala Arg Arg Leu
       50                  55                  60
   Ala Arg Ser Val Arg Arg Leu Thr Arg Arg Arg Ala His Arg Leu Leu
   65                  70                  75                  80
   Arg Ala Arg Arg Leu Leu Lys Arg Glu Gly Val Leu Gln Ala Ala Asp
                   85                  90                  95
   Phe Asp Glu Asn Gly Leu Ile Lys Ser Leu Pro Asn Thr Pro Trp Gln
               100                 105                 110
   Leu Arg Ala Ala Ala Leu Asp Arg Lys Leu Thr Pro Leu Glu Trp Ser
           115                 120                 125
   Ala Val Leu Leu His Leu Ile Lys His Arg Gly Tyr Leu Ser Gln Arg
       130                 135                 140
   Lys Asn Glu Gly Glu Thr Ala Asp Lys Glu Leu Gly Ala Leu Leu Lys
   145                 150                 155                 160
   Gly Val Ala Asp Asn Ala His Ala Leu Gln Thr Gly Asp Phe Arg Thr
                   165                 170                 175
   Pro Ala Glu Leu Ala Leu Asn Lys Phe Glu Lys Glu Ser Gly His Ile
               180                 185                 190
   Arg Asn Gln Arg Gly Asp Tyr Ser His Thr Phe Ser Arg Lys Asp Leu
           195                 200                 205
   Gln Ala Glu Leu Ile Leu Leu Phe Glu Lys Gln Lys Glu Phe Gly Asn
       210                 215                 220
   Pro His Val Ser Gly Gly Leu Lys Glu Gly Ile Glu Thr Leu Leu Met
   225                 230                 235                 240
   Thr Gln Arg Pro Ala Leu Ser Ser Asp Ala Val Gln Lys Met Leu Gly
                   245                 250                 255
   His Cys Thr Phe Glu Pro Ala Glu Pro Lys Ala Ala Lys Asn Thr Tyr
               260                 265                 270
   Thr Ala Glu Arg Phe Ile Trp Leu Thr Lys Leu Asn Asn Leu Arg Ile
           275                 280                 285
   Leu Glu Gln Gly Ser Glu Arg Pro Leu Thr Asp Thr Glu Arg Ala Thr
       290                 295                 300
   Leu Met Asp Glu Pro Tyr Arg Lys Ser Lys Leu Thr Tyr Ala Gln Ala
   305                 310                 315                 320
   Arg Lys Leu Leu Gly Leu Glu Asp Thr Ala Phe Phe Lys Gly Leu Arg
                   325                 330                 335
   Tyr Gly Lys Asp Asn Ala Glu Ala Ser Thr Leu Met Glu Met Lys Ala
               340                 345                 350
   Tyr His Ala Ile Ser Arg Ala Leu Glu Lys Glu Gly Leu Lys Asp Lys
           355                 360                 365
   Lys Ser Pro Leu Asn Leu Ser Pro Glu Leu Gln Asp Glu Ile Gly Thr
       370                 375                 380
   Ala Phe Ser Leu Phe Lys Thr Asp Glu Asp Ile Thr Gly Arg Leu Lys
   385                 390                 395                 400
   Asp Arg Ile Gln Pro Glu Ile Leu Glu Ala Leu Leu Lys His Ile Ser
                   405                 410                 415
   Phe Asp Lys Phe Val Gln Ile Ser Leu Lys Ala Leu Arg Arg Ile Val
               420                 425                 430
   Pro Leu Met Glu Gln Gly Lys Arg Tyr Asp Glu Ala Cys Ala Glu Ile
           435                 440                 445
   Tyr Gly Asp His Tyr Gly Lys Lys Asn Thr Glu Glu Lys Ile Tyr Leu
       450                 455                 460
   Pro Pro Ile Pro Ala Asp Glu Ile Arg Asn Pro Val Val Leu Arg Ala
   465                 470                 475                 480
   Leu Ser Gln Ala Arg Lys Val Ile Asn Gly Val Val Arg Arg Tyr Gly
                   485                 490                 495
   Ser Pro Ala Arg Ile His Ile Glu Thr Ala Arg Glu Val Gly Lys Ser
               500                 505                 510
   Phe Lys Asp Arg Lys Glu Ile Glu Lys Arg Gln Glu Glu Asn Arg Lys
           515                 520                 525
   Asp Arg Glu Lys Ala Ala Ala Lys Phe Arg Glu Tyr Phe Pro Asn Phe
       530                 535                 540
   Val Gly Glu Pro Lys Ser Lys Asp Ile Leu Lys Leu Arg Leu Tyr Glu
   545                 550                 555                 560
   Gln Gln His Gly Lys Cys Leu Tyr Ser Gly Lys Glu Ile Asn Leu Gly
                   565                 570                 575
   Arg Leu Asn Glu Lys Gly Tyr Val Glu Ile Asp His Ala Leu Pro Phe
               580                 585                 590
   Ser Arg Thr Trp Asp Asp Ser Phe Asn Asn Lys Val Leu Val Leu Gly
           595                 600                 605
   Ser Glu Asn Gln Asn Lys Gly Asn Gln Thr Pro Tyr Glu Tyr Phe Asn
       610                 615                 620
   Gly Lys Asp Asn Ser Arg Glu Trp Gln Glu Phe Lys Ala Arg Val Glu
   625                 630                 635                 640
   Thr Ser Arg Phe Pro Arg Ser Lys Lys Gln Arg Ile Leu Leu Gln Lys
                   645                 650                 655
   Phe Asp Glu Asp Gly Phe Lys Glu Arg Asn Leu Asn Asp Thr Arg Tyr
               660                 665                 670
   Val Asn Arg Phe Leu Cys Gln Phe Val Ala Asp Arg Met Arg Leu Thr
           675                 680                 685
   Gly Lys Gly Lys Lys Arg Val Phe Ala Ser Asn Gly Gln Ile Thr Asn
       690                 695                 700
   Leu Leu Arg Gly Phe Trp Gly Leu Arg Lys Val Arg Ala Glu Asn Asp
   705                 710                 715                 720
   Arg His His Ala Leu Asp Ala Val Val Val Ala Cys Ser Thr Val Ala
                   725                 730                 735
   Met Gln Gln Lys Ile Thr Arg Phe Val Arg Tyr Lys Glu Met Asn Ala
               740                 745                 750
   Phe Asp Gly Lys Thr Ile Asp Lys Glu Thr Gly Glu Val Leu His Gln
           755                 760                 765
   Lys Thr His Phe Pro Gln Pro Trp Glu Phe Phe Ala Gln Glu Val Met
       770                 775                 780
   Ile Arg Val Phe Gly Lys Pro Asp Gly Lys Pro Glu Phe Glu Glu Ala
   785                 790                 795                 800
   Asp Thr Pro Glu Lys Leu Arg Thr Leu Leu Ala Glu Lys Leu Ser Ser
                   805                 810                 815
   Arg Pro Glu Ala Val His Glu Tyr Val Thr Pro Leu Phe Val Ser Arg
   820 825 830
   Ala Pro Asn Arg Lys Met Ser Gly Gln Gly His Met Glu Thr Val Lys
   835 840 845
   Ser Ala Lys Arg Leu Asp Glu Gly Val Ser Val Leu Arg Val Pro Leu
   850 855 860
   Thr Gln Leu Lys Leu Lys Asp Leu Glu Lys Met Val Asn Arg Glu Arg
   865 870 875 880
   Glu Pro Lys Leu Tyr Glu Ala Leu Lys Ala Arg Leu Glu Ala His Lys
   885 890 895
   Asp Asp Pro Ala Lys Ala Phe Ala Glu Pro Phe Tyr Lys Tyr Asp Lys
   900 905 910
   Ala Gly Asn Arg Thr Gln Gln Val Lys Ala Val Arg Val Glu Gln Val
   915 920 925
   Gln Lys Thr Gly Val Trp Val Arg Asn His Asn Gly Ile Ala Asp Asn
   930 935 940
   Ala Thr Met Val Arg Val Asp Val Phe Glu Lys Gly Asp Lys Tyr Tyr
   945 950 955 960
   Leu Val Pro Ile Tyr Ser Trp Gln Val Ala Lys Gly Ile Leu Pro Asp
   965 970 975
   Arg Ala Val Val Gln Gly Lys Asp Glu Glu Asp Trp Gln Leu Ile Asp
   980 985 990
   Asp Ser Phe Asn Phe Lys Phe Ser Leu His Pro Asn Asp Leu Val Glu
   995 1000 1005
   Val Ile Thr Lys Lys Ala Arg Met Phe Gly Tyr Phe Ala Ser Cys His
   1010 1015 1020
   Arg Gly Thr Gly Asn Ile Asn Ile Arg Ile His Asp Leu Asp His Lys
   1025 1030 1035 1040
   Ile Gly Lys Asn Gly Ile Leu Glu Gly Ile Gly Val Lys Thr Ala Leu
   1045 1050 1055
   Ser Phe Gln Lys Tyr Gln Ile Asp Glu Leu Gly Lys Glu Ile Arg Pro
   1060 1065 1070
   Cys Arg Leu Lys Lys Arg Pro Pro Val Arg
   1075 1080
<210> 198
   <211> 58
   <212> PRT
   <213> Thielavia terrestris
<220>
   <223> CRISPR-associated endonuclease
<308> XP_003657788
   <309> 2012-01-04
<400> 198
   Met Ala Ala Leu Asp Asp Gly Phe Glu Ala Leu Leu Glu Pro Phe Tyr
   1 5 10 15
   Asn Gly Lys Lys Leu Thr Asp Pro Ile Ser Thr Lys Glu Asp Lys Tyr
   20 25 30
   Gln Leu Leu Pro Ala Phe Leu Lys Val Lys Gly Gly Ser Thr Ser Asn
   35 40 45
   Arg Arg Gly Ala Ala Leu Ile His Pro Arg
   50 55
<210> 199
   <211> 151
   <212> PRT
   <213> Xanthomonas axonopodis Xac29-1
<308> YP_007637705
   <309> 2013-08-28
<400> 199
   Met Gly His Pro Pro Val Leu Cys Pro His Ser Gln Thr Val Pro Gly
    1               5                   10                  15
   Ala Arg Gly Pro Pro Asn Gly Gly Thr Gly Ile Leu Ser Ile Pro Glu
               20                  25                  30
   Leu Pro Ile Ser Lys Lys Arg Leu Lys Thr Phe Arg Leu Leu Ala Phe
           35                  40                  45
   His Ala Gln His Gly Arg Cys Phe Tyr Phe Thr Leu Pro Thr Trp Leu
       50                  55                  60
   Ala Ser Pro Asn Glu Leu Gly Leu Arg Pro Arg Ser Ala Arg Pro Tyr
   65                  70                  75                  80
   Gln Cys Thr Ala Lys His Leu Val Ala Cys Gln Asp Gly Gly Lys Asp
                   85                  90                  95
   Val Val Gly Asn Val Val Ala Ala His Ala Ala Cys Asn His Arg Arg
               100                 105                 110
   His Lys Arg Thr Ala Pro Val Pro Ser Ala Thr Asp Tyr Arg Thr Phe
           115                 120                 125
   Val Gln Arg Gln Leu Ala Lys Gly Lys Trp Cys Pro Gln Trp Ile Tyr
       130                 135                 140
   Gly Leu Ser Ile Gly Thr Pro
   145                 150
<210> 200
   <211> 1368
   <212> PRT
   <213> Streptococcus pyogenes
<220>
   <223> CRISPR-associated endonuclease
<308> YP_007587356
   <309> 2013-09-25
<400> 200
   Met Asp Lys Lys Tyr Ser Ile Gly Leu Asp Ile Gly Thr Asn Ser Val
    1               5                  10                  15
   Gly Trp Ala Val Ile Thr Asp Asp Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705 710 715 720
   His Glu His Ile Ala Asn Leu Ala Gly Ser Pro Ala Ile Lys Lys Gly
   725 730 735
   Ile Leu Gln Thr Val Lys Val Val Asp Glu Leu Val Lys Val Met Gly
   740 745 750
   Arg His Lys Pro Glu Asn Ile Val Ile Glu Met Ala Arg Glu Asn Gln
   755 760 765
   Thr Thr Gln Lys Gly Gln Lys Asn Ser Arg Glu Arg Met Lys Arg Ile
   770 775 780
   Glu Glu Gly Ile Lys Glu Leu Gly Ser Gln Ile Leu Lys Glu His Pro
   785 790 795 800
   Val Glu Asn Thr Gln Leu Gln Asn Glu Lys Leu Tyr Leu Tyr Tyr Leu
   805 810 815
   Gln Asn Gly Arg Asp Met Tyr Val Asp Gln Glu Leu Asp Ile Asn Arg
   820 825 830
   Leu Ser Asp Tyr Asp Val Asp His Ile Val Pro Gln Ser Phe Leu Lys
   835 840 845
   Asp Asp Ser Ile Asp Asn Lys Val Leu Thr Arg Ser Asp Lys Asn Arg
   850 855 860
   Gly Lys Ser Asp Asn Val Pro Ser Glu Glu Val Val Lys Lys Met Lys
   865 870 875 880
   Asn Tyr Trp Arg Gln Leu Leu Asn Ala Lys Leu Ile Thr Gln Arg Lys
   885 890 895
   Phe Asp Asn Leu Thr Lys Ala Glu Arg Gly Gly Leu Ser Glu Leu Asp
   900 905 910
   Lys Ala Gly Phe Ile Lys Arg Gln Leu Val Glu Thr Arg Gln Ile Thr
   915 920 925
   Lys His Val Ala Gln Ile Leu Asp Ser Arg Met Asn Thr Lys Tyr Asp
   930 935 940
   Glu Asn Asp Lys Leu Ile Arg Glu Val Lys Val Ile Thr Leu Lys Ser
   945 950 955 960
   Lys Leu Val Ser Asp Phe Arg Lys Asp Phe Gln Phe Tyr Lys Val Arg
   965 970 975
   Glu Ile Asn Asn Tyr His His Ala His Asp Ala Tyr Leu Asn Ala Val
   980 985 990
   Val Gly Thr Ala Leu Ile Lys Lys Tyr Pro Lys Leu Glu Ser Glu Phe
   995 1000 1005
   Val Tyr Gly Asp Tyr Lys Val Tyr Asp Val Arg Lys Met Ile Ala Lys
   1010 1015 1020
   Ser Glu Gln Glu Ile Gly Lys Ala Thr Ala Lys Tyr Phe Phe Tyr Ser
   1025 1030 1035 1040
   Asn Ile Met Asn Phe Phe Lys Thr Glu Ile Thr Leu Ala Asn Gly Glu
   1045 1050 1055
   Ile Arg Lys Arg Pro Leu Ile Glu Thr Asn Gly Glu Thr Gly Glu Ile
   1060 1065 1070
   Val Trp Asp Lys Gly Arg Asp Phe Ala Thr Val Arg Lys Val Leu Ser
   1075 1080 1085
   Met Pro Gln Val Asn Ile Val Lys Lys Thr Glu Val Gln Thr Gly Gly
   1090 1095 1100
   Phe Ser Lys Glu Ser Ile Leu Pro Lys Arg Asn Ser Asp Lys Leu Ile
   1105 1110 1115 1120
   Ala Arg Lys Lys Asp Trp Asp Pro Lys Lys Tyr Gly Gly Phe Asp Ser
   1125 1130 1135
   Pro Thr Val Ala Tyr Ser Val Leu Val Val Ala Lys Val Glu Lys Gly
   1140 1145 1150
   Lys Ser Lys Lys Leu Lys Ser Val Lys Glu Leu Leu Gly Ile Thr Ile
   1155 1160 1165
   Met Glu Arg Ser Ser Phe Glu Lys Asn Pro Ile Asp Phe Leu Glu Ala
   1170 1175 1180
   Lys Gly Tyr Lys Glu Val Lys Lys Asp Leu Ile Ile Lys Leu Pro Lys
   1185 1190 1195 1200
   Tyr Ser Leu Phe Glu Leu Glu Asn Gly Arg Lys Arg Met Leu Ala Ser
                   1205                1210                1215
   Ala Gly Glu Leu Gln Lys Gly Asn Glu Leu Ala Leu Pro Ser Lys Tyr
               1220                1225                1230
   Val Asn Phe Leu Tyr Leu Ala Ser His Tyr Glu Lys Leu Lys Gly Ser
           1235                1240                1245
   Pro Glu Asp Asn Glu Gln Lys Gln Leu Phe Val Glu Gln His Lys His
       1250                1255                1260
   Tyr Leu Asp Glu Ile Ile Glu Gln Ile Ser Glu Phe Ser Lys Arg Val
   1265                1270                1275                1280
   Ile Leu Ala Asp Ala Asn Leu Asp Lys Val Leu Ser Ala Tyr Asn Lys
                   1285                1290                1295
   His Arg Asp Lys Pro Ile Arg Glu Gln Ala Glu Asn Ile Ile His Leu
               1300                1305                1310
   Phe Thr Leu Thr Asn Leu Gly Ala Pro Ala Ala Phe Lys Tyr Phe Asp
           1315                1320                1325
   Thr Thr Ile Asp Arg Lys Arg Tyr Thr Ser Thr Lys Glu Val Leu Asp
       1330                1335                1340
   Ala Thr Leu Ile His Gln Ser Ile Thr Gly Leu Tyr Glu Thr Arg Ile
   1345                1350                1355                1360
   Asp Leu Ser Gln Leu Gly Gly Asp
                   1365
<210> 201
   <211> 73
   <212> PRT
   <213> Millerozyma farinosa
<220>
   <223> CRISPR-associated endonuclease
<308> XP_004204917
   <309> 2013-02-06
<400> 201
   Met Ala Arg Gly Asn Gln Arg Glu Leu Ala Arg Gln Lys Asn Leu Lys
   1               5                   10                  15
   Lys Gln Gln Glu Ala Ala Lys Ser Lys Arg Lys Glu Gly Ser Ala Ala
               20                  25                  30
   Lys Arg Met Glu Ser Asp Ala Glu Lys Met Arg Gln Lys Gln Ala Ala
           35                  40                  45
   Gly Met Trp Ile Ser Tyr Val Leu Ser Asp Asp Asp Ser Ser Leu Glu
       50                  55                  60
   Lys Asn Asn Phe Thr Asn Cys Thr Ser
   65                  70
<210> 202
   <211> 1400
   <212> PRT
   <213> Riemerella anatipestifer
<220>
   <223> CRISPR-associated endonuclease
<308> YP_007355826
   <309> 2013-06-11
<400> 202
   Met Lys Thr Ile Leu Gly Leu Asp Leu Gly Thr Asn Ser Ile Gly Trp
    1              5                  10                  15
   Ala Leu Val Lys Glu Thr Glu Asn Ser Asn Glu Lys Ser Glu Ile Ile
               20                  25                  30
   Lys Leu Gly Val Arg Val Asn Pro Leu Thr Val Asp Glu Lys Thr Asn
           35                  40                  45
   Phe Glu Ala Gly Arg Pro Leu Ser Thr Asn Ala Asp Arg Thr Ala Lys
       50                  55                  60
   Arg Ser Ala Arg Arg Asn Leu Gln Arg Tyr Lys Leu Arg Arg Lys Asn
   65                  70                  75                  80
   Leu Ile Asp Leu Leu Ile Lys His Arg Leu Ile Asp Lys Asp Thr Pro
                   85                  90                  95
   Leu Thr Glu Ile Gly Lys Asn Thr Thr His Gln Thr Leu Glu Leu Arg
               100                 105                 110
   Ala Lys Ala Ala Arg Glu Arg Ile Glu Leu Glu Asp Leu Ala Arg Val
           115                 120                 125
   Phe Leu Ala Ile Asn Lys Lys Arg Gly Tyr Arg Ser Ser Arg Lys Val
       130                 135                 140
   Asn Asn Glu Glu Glu Gly Gln Val Val Asp Gly Met Ala Val Ala Lys
   145                 150                 155                 160
   Lys Leu Tyr Asp Glu Asn Leu Thr Pro Gly Gln Tyr Ala Tyr Glu Leu
                   165                 170                 175
   Leu Ser Lys Gly Lys Lys Tyr Val Pro Asp Phe Tyr Arg Ser Asp Leu
               180                 185                 190
   Gln Ala Glu Phe Asp Ser Ile Trp Glu Tyr Gln Lys Gln Phe Tyr Ala
           195                 200                 205
   Asp Ile Leu Asp Asp Glu Leu Tyr Asn Ala Leu Lys Gly Gln Gly Gln
       210                 215                 220
   Gln Asn Ser Arg Lys Arg Phe Leu Ala Ile Lys Gly Val Tyr Thr Ala
   225                 230                 235                 240
   Glu Asn Lys Gly Lys Arg Asp Glu Val Lys Leu Gln His Tyr Lys Trp
                   245                 250                 255
   Arg Ser Glu Ala Ile Thr Gln Lys Leu Ser Ile Glu Glu Val Ala Tyr
               260                 265                 270
   Val Leu Val Glu Ile Asn Asn Asp Leu Asn Lys Ser Ser Gly Tyr Leu
           275                 280                 285
   Gly Ala Ile Ser Asp Arg Ser Lys Glu Leu Tyr Phe Asn Lys Glu Thr
       290                 295                 300
   Val Gly Glu Asn Leu Trp Lys Gln Ile Gln Lys Asn Pro His Thr Ser
   305                 310                 315                 320
   Leu Lys Asn Gln Val Phe Tyr Arg Gln Asp Tyr Leu Asp Glu Phe Glu
                   325                 330                 335
   Gln Ile Trp Glu Thr Gln Ala Gln Phe His Pro Gln Leu Thr Leu Ala
               340                 345                 350
   Leu Lys Glu Gln Ile Arg Asp Val Val Ile Phe Tyr Gln Arg Lys Leu
           355                 360                 365
   Lys Ser Gln Lys Gly Leu Leu Ser Phe Cys Gln Phe Glu Ser Trp Glu
       370                 375                 380
   Ile Glu Arg Lys Asp Glu Asn Gly Asn Val Ile Leu Asn Lys Thr Thr
   385                 390                 395                 400
   Gln Leu Pro Lys Arg Gln Thr Val Gly Arg Arg Val Ala Pro Lys Ser
                   405                 410                 415
   Ser Pro Leu Phe Gln Glu Phe Lys Ile Trp Gln Asn Ile Asn Asn Leu
               420                 425                 430
   Glu Ile Ala Lys Ile Ser Asp Gly Asn Ser Lys Asn Lys Lys Glu Thr
           435                 440                 445
   Glu Ala Leu Ser Asp Asp Glu Arg Lys Leu Leu Phe Glu Glu Leu Asn
       450                 455                 460
   Leu Arg Gly Asn Leu Ser Gln Lys Glu Val Leu Gln Ile Leu Gly Leu
   465                 470                 475                 480
   Lys Asp Lys Glu Tyr Lys Thr Asn Phe Pro Glu Gly Leu Glu Gly Asn
                   485                 490                 495
   Arg Thr Asn Ala Ala Leu Phe Asn Ile Tyr Gln Gln Ile Ala Glu Asn
               500                 505                 510
   Glu Gly Tyr Gly Asp Trp Thr Lys Lys Ser Ala Gln Glu Ile Lys Glu
           515                 520                 525
   Glu Leu Lys Ala Val Phe Pro Gln Ile Gly Ile Gln Ala Asp Ile Leu
   530 535 540
   Asp Phe Asn Ala Glu Leu Asp Gly Lys Glu Phe Glu Asn Gln Ala Ser
   545 550 555 560
   Tyr Gln Leu Trp His Leu Leu Tyr Ser Ala Glu Glu Asp Asp Lys Ile
   565 570 575
   Asn Glu Glu Asp Gln Ile Ile Tyr Gly Asn Ser Ala Val Ser Leu Lys
   580 585 590
   Lys Lys Leu Cys Glu Lys Phe Gly Phe Thr Pro Glu Tyr Ala Lys Trp
   595 600 605
   Ile Ala Asn Val Ser Leu Gln Asp Asp Tyr Gly Asn Leu Ser Thr Lys
   610 615 620
   Ala Met Arg Lys Ile Ile Pro Tyr Leu Ile Asp Gly Asn Asp Tyr Ser
   625 630 635 640
   Glu Ala Cys Ala Leu Ala Gly Tyr Asn His Ser Asn Ser Leu Thr Lys
   645 650 655
   Glu Glu Asn Asp Asn Arg Glu Arg Leu Asn Lys Leu Glu Leu Leu Pro
   660 665 670
   Lys Asn Ser Leu Arg Asn Pro Val Val Glu Lys Ile Leu Asn Gln Met
   675 680 685
   Val Asn Val Val Asn Gln Val Ile Glu Thr Tyr Gly Lys Pro Asp Glu
   690 695 700
   Val Arg Ile Glu Leu Ala Arg Glu Leu Lys Lys Ser Ala Glu Glu Arg
   705 710 715 720
   Ala Glu Met Thr Lys Gly Ile Asn Glu Ala Thr Arg Arg Asn Glu Asp
   725 730 735
   Ile Lys Lys Leu Ile Thr Lys Asn Phe Gly Ile Pro Asn Pro Thr Lys
   740 745 750
   Asn Asp Val Val Arg Tyr Arg Leu Trp Glu Glu Leu Ala Pro Leu Ala
   755 760 765
   Tyr Lys Asp Val Phe Thr Gly Arg Gln Ile Lys Lys Glu Asp Leu Phe
   770 775 780
   Ser Ser Lys Ile Asp Ile Glu His Ile Ile Pro Lys Ala Leu Leu Phe
   785 790 795 800
   Asp Asp Ser Phe Ser Asn Lys Thr Leu Ala Phe Arg Glu Thr Asn Leu
   805 810 815
   Lys Lys Ala Asp Arg Thr Ala Tyr Gly Phe Ile Glu Ser Asp Tyr Asn
   820 825 830
   Ala Thr Leu Asp Asp Tyr Ile Gln Arg Val Glu Thr Leu Tyr Asn Asn
   835 840 845
   Ala Lys Gly Thr Ile Ser Lys Gly Lys Arg Asn Lys Leu Leu Met Ala
   850 855 860
   Gln Lys Asn Leu Pro Asp Gly Phe Ile Glu Arg Asp Leu Arg Asn Ser
   865 870 875 880
   Gln Tyr Ile Ala Lys Lys Ala Lys Ser Met Ile Glu Lys Val Phe Asn
   885 890 895
   Asp Val Asn Val Thr Ser Gly Ser Ile Thr Asp Arg Leu Arg Glu Asp
   900 905 910
   Trp Asp Leu Ile Asn Val Met Lys Glu Leu Asn Phe Pro Lys Tyr Lys
   915 920 925
   Ala Leu Gly Leu Thr Glu Ile Glu Glu Arg Tyr Asp Ile Gly Gln Glu
   930 935 940
   Lys Thr Lys Lys Val Glu Val Ile Thr Asp Trp Thr Lys Arg Asn Asp
   945 950 955 960
   His Arg His His Ala Met Asp Ala Leu Thr Val Ala Phe Thr Thr Lys
   965 970 975
   Ser His Val Gln Tyr Leu Asn Asn Leu Asn Thr Ile His Ser Leu Lys
   980 985 990
   Gly Asp Glu Val Asp Leu Glu Leu Ser Lys Leu Tyr Gly Leu Lys Asn
   995 1000 1005
   Thr Ile Thr Glu Val Val Asn Lys Lys Arg Lys Phe Ile Pro Pro Met
   1010 1015 1020
   Pro Asn Phe Arg Glu Glu Ala Lys Lys His Ile Glu Thr Ile Leu Val
   1025 1030 1035 1040
   Ser Ile Lys Asn Lys Asn Lys Val Tyr Thr Lys Asn Ile Asn Lys Thr
   1045 1050 1055
   Lys Lys Glu Ser Gly Tyr His Thr Lys Glu Gln Leu Thr Pro Arg Gly
   1060 1065 1070
   Gln Leu His Lys Glu Thr Val Tyr Gly Lys Ser Lys Arg Pro Met Asn
   1075 1080 1085
   Lys Pro Thr Lys Ile Asn Lys Asn Phe Ser Leu Glu Gln Ala Gln Leu
   1090 1095 1100
   Ile Ile Asn Val Gln Glu Lys Glu Leu Val Leu Arg His Leu Ala Gln
   1105 1110 1115 1120
   Phe Asp Asn Asn Pro Ala Val Ala Phe Asp Thr Lys Thr Leu Lys Lys
   1125 1130 1135
   Met Pro Leu Leu Lys Asn Gly Glu Pro Leu Lys Glu Val Leu Cys Phe
   1140 1145 1150
   Glu Glu Ile Phe Thr Ile Arg Lys Asp Ile Thr Pro Asp Leu Lys Ile
   1155 1160 1165
   Glu Lys Val Val Asp Glu Lys Ile Lys Ala Ile Leu Glu Gln Arg Lys
   1170 1175 1180
   Lys Asp Phe Gly Gly Asn Ala Lys Glu Ala Phe Ser Asp Leu Asp Lys
   1185 1190 1195 1200
   Asn Pro Ile Trp Leu Asn Lys Glu Lys Gly Ile Cys Ile Lys Arg Val
   1205 1210 1215
   Thr Ile Thr Gly Val Ser Asn Ala Glu Ala Leu His His Gln Lys Asp
   1220 1225 1230
   His Leu Gly Arg Glu Ile Leu Asp Asp Asn Gly Asn Pro Ile Pro Ala
   1235 1240 1245
   Asp Phe Val Ser Thr Gly Asn Asn His His Val Ala Ile Tyr Glu Asp
   1250 1255 1260
   Glu Lys Gly Lys Leu Gln Glu Lys Val Val Ser Leu Tyr Glu Ala Val
   1265 1270 1275 1280
   Ala Arg Ala Asn Gln Gly Leu Pro Val Val Asp Lys Gly Leu Asn Glu
   1285 1290 1295
   Asp Leu Gly Trp Lys Phe Leu Phe Thr Met Lys Gln Asn Glu Met Phe
   1300 1305 1310
   Val Phe Pro Asn Asp Asp Phe Asp Val Asn Asp Ile Asp Leu Leu Asp
   1315 1320 1325
   Glu Asn Asn Ala Lys Ile Ile Ser Gln His Leu Phe Arg Val Gln Lys
   1330 1335 1340
   Leu Ala Thr Lys Asn Tyr Met Phe Arg His His Leu Glu Thr Asn Val
   1345 1350 1355 1360
   Gln Glu Val Lys Glu Leu Asn Asp Ile Ala Tyr Lys Ser Ile Arg Ser
   1365 1370 1375
   Thr Glu Pro Leu Lys Asn Ile Lys Lys Val Arg Ile Asn His Ile Gly
   1380 1385 1390
   Lys Ile Val Gln Val Gly Glu Tyr
   1395 1400
<210> 203
   <211> 1371
   <212> PRT
   <213> Streptococcus dysgalactiae
<220>
   <223> CRISPR-associated endonuclease
<308> YP_006904759
   <309> 2013-08-28
<400> 203
   Met Asp Lys Lys Tyr Ser Ile Gly Leu Asp Ile Gly Thr Asn Ser Val
   1               5                   10                  15
   Gly Trp Ala Val Ile Thr Asp Asp Tyr Lys Val Pro Ser Lys Lys Phe
                   20                  25                  30
   Lys Val Leu Gly Asn Thr Asp Arg His Ser Ile Lys Lys Asn Leu Ile
               35                  40                  45
   Gly Ala Leu Leu Phe Asp Ser Gly Glu Thr Ala Glu Ala Thr Arg Leu
           50                  55                  60
   Lys Arg Thr Ala Arg Arg Arg Tyr Thr Arg Arg Lys Asn Arg Ile Arg
   65                  70                  75                  80
   Tyr Leu Gln Glu Ile Phe Ser Ser Glu Met Ser Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Ala Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Met Asp Lys Leu Phe Ile Gln Leu Val Gln Thr Tyr
               180                 185                 190
   Asn Gln Leu Phe Glu Glu Asn Pro Ile Asn Ala Ser Arg Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Arg Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Ser Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Ala Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Glu Phe Leu Ser Gly Lys Gln
       530                 535                 540
   Lys Glu Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Lys Glu Met Ile Glu Glu Arg Leu Lys Lys Tyr Ala
   625                 630                 635                 640
   Asn Leu Phe Asp Asp Lys Val Met Lys Gln Leu Lys Arg Arg His Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile Asn Asp Asp Ser Leu Thr Phe
       690                 695                 700
   Lys Glu Ala Ile Gln Lys Ala Gln Val Ser Gly Gln Gly His Ser Leu
   705                 710                 715                 720
   His Glu Gln Ile Ala Asn Leu Ala Gly Ser Pro Ala Ile Lys Lys Gly
                   725                 730                 735
   Ile Leu Gln Ser Val Lys Val Val Asp Glu Leu Val Lys Val Met Gly
               740                 745                 750
   His Lys Pro Glu Asn Ile Val Ile Glu Met Ala Arg Glu Asn Gln Thr
           755                 760                 765
   Thr Gln Lys Gly Gln Lys Asn Ser Arg Glu Arg Met Lys Arg Ile Glu
       770                 775                 780
   Glu Gly Ile Lys Glu Leu Gly Ser Gln Ile Leu Lys Glu His Pro Val
   785                 790                 795                 800
   Glu Asn Thr Gln Leu Gln Asn Glu Lys Leu Tyr Leu Tyr Tyr Leu Gln
                   805                 810                 815
   Asn Gly Arg Asp Met Tyr Val Asp Gln Glu Leu Asp Ile Asn Arg Leu
               820                 825                 830
   Ser Asp Tyr Asp Val Asp His Ile Val Pro Gln Ser Phe Ile Lys Asp
           835                 840                 845
   Asp Ser Ile Asp Asn Lys Val Leu Thr Arg Ser Asp Lys Asn Arg Gly
       850                 855                 860
   Lys Ser Asp Asn Val Pro Ser Glu Glu Val Val Lys Lys Met Lys Asn
   865                 870                 875                 880
   Tyr Trp Arg Gln Leu Leu Asn Ala Lys Leu Ile Thr Gln Arg Lys Phe
                   885                 890                 895
   Asp Asn Leu Thr Lys Ala Glu Arg Gly Gly Leu Ser Glu Leu Asp Lys
               900                 905                 910
   Ala Gly Phe Ile Lys Arg Gln Leu Val Glu Thr Arg Gln Ile Thr Lys
           915                 920                 925
   His Val Ala Gln Ile Leu Asp Ser Arg Met Asn Thr Lys Tyr Asp Glu
       930                 935                 940
   Asn Asp Lys Leu Ile Arg Glu Val Lys Val Ile Thr Leu Lys Ser Lys
   945                 950                 955                 960
   Leu Val Ser Asp Phe Arg Lys Asp Phe Gln Phe Tyr Lys Val Arg Glu
                   965                 970                 975
   Ile Asn Asn Tyr His His Ala His Asp Ala Tyr Leu Asn Ala Val Val
               980                 985                 990
   Gly Thr Ala Leu Ile Lys Lys Tyr Pro Lys Leu Glu Ser Glu Phe Val
           995                 1000                1005
   Tyr Gly Asp Tyr Lys Val Tyr Asp Val Arg Lys Met Ile Ala Lys Ser
       1010                1015                1020
   Glu Gln Glu Ile Gly Lys Ala Thr Ala Lys Arg Phe Phe Tyr Ser Asn
   1025 1030 1035 1040
   Ile Met Asn Phe Phe Lys Thr Glu Ile Thr Leu Ala Asn Gly Glu Ile
   1045 1050 1055
   Arg Lys Arg Pro Leu Ile Glu Thr Asn Glu Glu Thr Gly Glu Ile Val
   1060 1065 1070
   Trp Asp Lys Gly Arg Asp Phe Ala Thr Val Arg Lys Val Leu Ser Met
   1075 1080 1085
   Pro Gln Val Asn Ile Val Lys Lys Thr Glu Val Gln Thr Gly Ala Leu
   1090 1095 1100
   Thr Asn Glu Ser Ile Tyr Ala Arg Gly Ser Phe Asp Lys Leu Ile Ser
   1105 1110 1115 1120
   Arg Lys His Arg Phe Glu Ser Ser Lys Tyr Gly Gly Phe Gly Ser Pro
   1125 1130 1135
   Thr Val Thr Tyr Ser Val Leu Val Val Ala Lys Ser Lys Val Gln Asp
   1140 1145 1150
   Gly Lys Val Lys Lys Ile Lys Thr Gly Lys Glu Leu Ile Gly Ile Thr
   1155 1160 1165
   Leu Leu Asp Lys Leu Val Phe Glu Lys Asn Pro Leu Lys Phe Ile Glu
   1170 1175 1180
   Asp Lys Gly Tyr Gly Asn Val Gln Ile Asp Lys Cys Ile Lys Leu Pro
   1185 1190 1195 1200
   Lys Tyr Ser Leu Phe Glu Phe Glu Asn Gly Thr Arg Arg Met Leu Ala
   1205 1210 1215
   Ser Val Met Ala Asn Asn Asn Ser Arg Gly Asp Leu Gln Lys Ala Asn
   1220 1225 1230
   Glu Met Phe Leu Pro Ala Lys Leu Val Thr Leu Leu Tyr His Ala His
   1235 1240 1245
   Lys Ile Glu Ser Ser Lys Glu Leu Glu His Glu Ala Tyr Ile Leu Asp
   1250 1255 1260
   His Tyr Asn Asp Leu Tyr Gln Leu Leu Ser Tyr Ile Glu Arg Phe Ala
   1265 1270 1275 1280
   Ser Leu Tyr Val Asp Val Glu Lys Asn Ile Ser Lys Val Lys Glu Leu
   1285 1290 1295
   Phe Ser Asn Ile Glu Ser Tyr Ser Ile Ser Glu Ile Cys Ser Ser Val
   1300 1305 1310
   Ile Asn Leu Leu Thr Leu Thr Ala Ser Gly Ala Pro Ala Asp Phe Lys
   1315 1320 1325
   Phe Leu Gly Thr Thr Ile Pro Arg Lys Arg Tyr Gly Ser Pro Gln Ser
   1330 1335 1340
   Ile Leu Ser Ser Thr Leu Ile His Gln Ser Ile Thr Gly Leu Tyr Glu
   1345 1350 1355 1360
   Thr Arg Ile Asp Leu Ser Gln Leu Gly Gly Asp
   1365 1370
<210> 204
   <211> 1370
   <212> PRT
   <213> Streptococcus agalactiae
<220>
   <223> CRISPR-associated endonuclease
<308> YP_006764109
   <309> 2013-06-11
<400> 204
   Met Asn Lys Pro Tyr Ser Ile Gly Leu Asp Ile Gly Thr Asn Ser Val
   1               5                   10                  15
   Gly Trp Ser Ile Ile Thr Asp Asp Tyr Lys Val Pro Ala Lys Lys Met
               20                  25                  30
   Arg Val Leu Gly Asn Thr Asp Lys Glu Tyr Ile Lys Lys Asn Leu Ile
           35                  40                  45
   Gly Ala Leu Leu Phe Asp Gly Gly Asn Thr Ala Ala Asp Arg Arg Leu
   50 55 60
   Lys Arg Thr Ala Arg Arg Arg Tyr Thr Arg Arg Arg Asn Arg Ile Leu
   65 70 75 80
   Tyr Leu Gln Glu Ile Phe Ala Glu Glu Met Ser Lys Val Asp Asp Ser
   85 90 95
   Phe Phe His Arg Leu Glu Asp Ser Phe Leu Val Glu Glu Asp Lys Arg
   100 105 110
   Gly Ser Lys Tyr Pro Ile Phe Ala Thr Leu Gln Glu Glu Lys Asp Tyr
   115 120 125
   His Glu Lys Phe Ser Thr Ile Tyr His Leu Arg Lys Glu Leu Ala Asp
   130 135 140
   Lys Lys Glu Lys Ala Asp Leu Arg Leu Ile Tyr Ile Ala Leu Ala His
   145 150 155 160
   Ile Ile Lys Phe Arg Gly His Phe Leu Ile Glu Asp Asp Ser Phe Asp
   165 170 175
   Val Arg Asn Thr Asp Ile Ser Lys Gln Tyr Gln Asp Phe Leu Glu Ile
   180 185 190
   Phe Asn Thr Thr Phe Glu Asn Asn Asp Leu Leu Ser Gln Asn Val Asp
   195 200 205
   Val Glu Ala Ile Leu Thr Asp Lys Ile Ser Lys Ser Ala Lys Lys Asp
   210 215 220
   Arg Ile Leu Ala Gln Tyr Pro Asn Gln Lys Ser Thr Gly Ile Phe Ala
   225 230 235 240
   Glu Phe Leu Lys Leu Ile Val Gly Asn Gln Ala Asp Phe Lys Lys Tyr
   245 250 255
   Phe Asn Leu Glu Asp Lys Thr Pro Leu Gln Phe Ala Lys Asp Ser Tyr
   260 265 270
   Asp Glu Asp Leu Glu Asn Leu Leu Gly Gln Ile Gly Asp Glu Phe Ala
   275 280 285
   Asp Leu Phe Ser Ala Ala Lys Lys Leu Tyr Asp Ser Val Leu Leu Ser
   290 295 300
   Gly Ile Leu Thr Val Ile Asp Leu Ser Thr Lys Ala Pro Leu Ser Ala
   305 310 315 320
   Ser Met Ile Gln Arg Tyr Asp Glu His Arg Glu Asp Leu Lys Gln Leu
   325 330 335
   Lys Gln Phe Val Lys Ala Ser Leu Pro Glu Lys Tyr Gln Glu Ile Phe
   340 345 350
   Ala Asp Ser Ser Lys Asp Gly Tyr Ala Gly Tyr Ile Glu Gly Lys Thr
   355 360 365
   Asn Gln Glu Ala Phe Tyr Lys Tyr Leu Ser Lys Leu Leu Thr Lys Gln
   370 375 380
   Glu Asp Ser Glu Asn Phe Leu Glu Lys Ile Lys Asn Glu Asp Phe Leu
   385 390 395 400
   Arg Lys Gln Arg Thr Phe Asp Asn Gly Ser Ile Pro His Gln Val His
   405 410 415
   Leu Thr Glu Leu Lys Ala Ile Ile Arg Arg Gln Ser Glu Tyr Tyr Pro
   420 425 430
   Phe Leu Lys Glu Asn Gln Asp Arg Ile Glu Lys Ile Leu Thr Phe Arg
   435 440 445
   Ile Pro Tyr Tyr Ile Gly Pro Leu Ala Arg Glu Lys Ser Asp Phe Ala
   450 455 460
   Trp Met Thr Arg Lys Thr Asp Asp Ser Ile Arg Pro Trp Asn Phe Glu
   465 470 475 480
   Asp Leu Val Asp Lys Glu Lys Ser Ala Glu Ala Phe Ile His Arg Met
   485 490 495
   Thr Asn Asn Asp Phe Tyr Leu Pro Glu Glu Lys Val Leu Pro Lys His
   500 505 510
   Ser Leu Ile Tyr Glu Lys Phe Thr Val Tyr Asn Glu Leu Thr Lys Val
   515 520 525
   Arg Tyr Lys Asn Glu Gln Gly Glu Thr Tyr Phe Phe Asp Ser Asn Ile
   530 535 540
   Lys Gln Glu Ile Phe Asp Gly Val Phe Lys Glu His Arg Lys Val Ser
   545 550 555 560
   Lys Lys Lys Leu Leu Asp Phe Leu Ala Lys Glu Tyr Glu Glu Phe Arg
   565 570 575
   Ile Val Asp Val Ile Gly Leu Asp Lys Glu Asn Lys Ala Phe Asn Ala
   580 585 590
   Ser Leu Gly Thr Tyr His Asp Leu Glu Lys Ile Leu Asp Lys Asp Phe
   595 600 605
   Leu Asp Asn Pro Asp Asn Glu Ser Ile Leu Glu Asp Ile Val Gln Thr
   610 615 620
   Leu Thr Leu Phe Glu Asp Arg Glu Met Ile Lys Lys Arg Leu Glu Asn
   625 630 635 640
   Tyr Lys Asp Leu Phe Thr Glu Ser Gln Leu Lys Lys Leu Tyr Arg Arg
   645 650 655
   His Tyr Thr Gly Trp Gly Arg Leu Ser Ala Lys Leu Ile Asn Gly Ile
   660 665 670
   Arg Asp Lys Glu Ser Gln Lys Thr Ile Leu Asp Tyr Leu Ile Asp Asp
   675 680 685
   Gly Arg Ser Asn Arg Asn Phe Met Gln Leu Ile Asn Asp Asp Gly Leu
   690 695 700
   Ser Phe Lys Ser Ile Ile Ser Lys Ala Gln Ala Gly Ser His Ser Asp
   705 710 715 720
   Asn Leu Lys Glu Val Val Gly Glu Leu Ala Gly Ser Pro Ala Ile Lys
   725 730 735
   Lys Gly Ile Leu Gln Ser Leu Lys Ile Val Asp Glu Leu Val Lys Val
   740 745 750
   Met Gly Tyr Glu Pro Glu Gln Ile Val Val Glu Met Ala Arg Glu Asn
   755 760 765
   Gln Thr Thr Asn Gln Gly Arg Arg Asn Ser Arg Gln Arg Tyr Lys Leu
   770 775 780
   Leu Asp Asp Gly Val Lys Asn Leu Ala Ser Asp Leu Asn Gly Asn Ile
   785 790 795 800
   Leu Lys Glu Tyr Pro Thr Asp Asn Gln Ala Leu Gln Asn Glu Arg Leu
   805 810 815
   Phe Leu Tyr Tyr Leu Gln Asn Gly Arg Asp Met Tyr Thr Gly Glu Ala
   820 825 830
   Leu Asp Ile Asp Asn Leu Ser Gln Tyr Asp Ile Asp His Ile Ile Pro
   835 840 845
   Gln Ala Phe Ile Lys Asp Asp Ser Ile Asp Asn Arg Val Leu Val Ser
   850 855 860
   Ser Ala Lys Asn Arg Gly Lys Ser Asp Asp Val Pro Ser Leu Glu Ile
   865 870 875 880
   Val Lys Asp Cys Lys Val Phe Trp Lys Lys Leu Leu Asp Ala Lys Leu
   885 890 895
   Met Ser Gln Arg Lys Tyr Asp Asn Leu Thr Lys Ala Glu Arg Gly Gly
   900 905 910
   Leu Thr Ser Asp Asp Lys Ala Arg Phe Ile Gln Arg Gln Leu Val Glu
   915 920 925
   Thr Arg Gln Ile Thr Lys His Val Ala Arg Ile Leu Asp Glu Arg Phe
   930 935 940
   Asn Asn Glu Leu Asp Ser Lys Gly Arg Arg Ile Arg Lys Val Lys Ile
   945 950 955 960
   Val Thr Leu Lys Ser Asn Leu Val Ser Asn Phe Arg Lys Glu Phe Gly
   965 970 975
   Phe Tyr Lys Ile Arg Glu Val Asn Asn Tyr His His Ala His Asp Ala
   980 985 990
   Tyr Leu Asn Ala Val Val Ala Lys Ala Ile Leu Thr Lys Tyr Pro Gln
   995 1000 1005
   Leu Glu Pro Glu Phe Val Tyr Gly Asp Tyr Pro Lys Tyr Asn Ser Tyr
   1010 1015 1020
   Lys Thr Arg Lys Ser Ala Thr Glu Lys Leu Phe Phe Tyr Ser Asn Ile
   1025 1030 1035 1040
   Met Asn Phe Phe Lys Thr Lys Val Thr Leu Ala Asp Gly Thr Val Val
   1045 1050 1055
   Val Lys Asp Asp Ile Glu Val Asn Asn Asp Thr Gly Glu Ile Val Trp
               1060                1065                1070
   Asp Lys Lys Lys His Phe Ala Thr Val Arg Lys Val Leu Ser Tyr Pro
           1075                1080                1085
   Gln Asn Asn Ile Val Lys Lys Thr Glu Ile Gln Thr Gly Gly Phe Ser
       1090                1095                1100
   Lys Glu Ser Ile Leu Ala His Gly Asn Ser Asp Lys Leu Ile Pro Arg
   1105                1110                1115                1120
   Lys Thr Lys Asp Ile Tyr Leu Asp Pro Lys Lys Tyr Gly Gly Phe Asp
                   1125                1130                1135
   Ser Pro Ile Val Ala Tyr Ser Val Leu Val Val Ala Asp Ile Lys Lys
               1140                1145                1150
   Gly Lys Ala Gln Lys Leu Lys Thr Val Thr Glu Leu Leu Gly Ile Thr
           1155                1160                1165
   Ile Met Glu Arg Ser Arg Phe Glu Lys Asn Pro Ser Ala Phe Leu Glu
       1170                1175                1180
   Ser Lys Gly Tyr Leu Asn Ile Arg Ala Asp Lys Leu Ile Ile Leu Pro
   1185                1190                1195                1200
   Lys Tyr Ser Leu Phe Glu Leu Glu Asn Gly Arg Arg Arg Leu Leu Ala
                   1205                1210                1215
   Ser Ala Gly Glu Leu Gln Lys Gly Asn Glu Leu Ala Leu Pro Thr Gln
               1220                1225                1230
   Phe Met Lys Phe Leu Tyr Leu Ala Ser Arg Tyr Asn Glu Ser Lys Gly
           1235                1240                1245
   Lys Pro Glu Glu Ile Glu Lys Lys Gln Glu Phe Val Asn Gln His Val
       1250                1255                1260
   Ser Tyr Phe Asp Asp Ile Leu Gln Leu Ile Asn Asp Phe Ser Lys Arg
   1265                1270                1275                1280
   Val Ile Leu Ala Asp Ala Asn Leu Glu Lys Ile Asn Lys Leu Tyr Gln
                   1285                1290                1295
   Asp Asn Lys Glu Asn Ile Ser Val Asp Glu Leu Ala Asn Asn Ile Ile
               1300                1305                1310
   Asn Leu Phe Thr Phe Thr Ser Leu Gly Ala Pro Ala Ala Phe Lys Phe
           1315                1320                1325
   Phe Asp Lys Ile Val Asp Arg Lys Arg Tyr Thr Ser Thr Lys Glu Val
       1330                1335                1340
   Leu Asn Ser Thr Leu Ile His Gln Ser Ile Thr Gly Leu Tyr Glu Thr
   1345                1350                1355                1360
   Arg Ile Asp Leu Gly Lys Leu Gly Glu Asp
                   1365                1370
<210> 205
   <211> 1629
   <212> PRT
   <213> Francisella cf. novicida
<220>
   <223> CRISPR-associated endonuclease
<308> YP_005825670
   <309> 2013-06-11
<400> 205
   Met Asn Phe Lys Ile Leu Pro Ile Ala Ile Asp Leu Gly Val Lys Asn
    1               5                  10                  15
   Thr Gly Val Phe Ser Ala Phe Tyr Gln Lys Gly Thr Ser Leu Glu Arg
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Arg Arg His Gln Arg Arg Gly
       50                  55                  60
   Ile Asp Arg Lys Gln Leu Val Lys Arg Leu Phe Lys Leu Ile Trp Thr
   65 70 75 80
   Glu Gln Leu Asn Leu Glu Trp Asp Lys Asp Thr Gln Gln Ala Ile Ser
   85 90 95
   Phe Leu Phe Asn Arg Arg Gly Phe Ser Phe Ile Thr Asp Gly Tyr Ser
   100 105 110
   Pro Glu Tyr Leu Asn Ile Val Pro Glu Gln Val Lys Ala Ile Leu Met
   115 120 125
   Asp Ile Phe Asp Asp Tyr Asn Gly Glu Asp Asp Leu Asp Ser Tyr Leu
   130 135 140
   Lys Leu Ala Thr Glu Gln Glu Ser Lys Ile Ser Glu Ile Tyr Asn Lys
   145 150 155 160
   Leu Met Gln Lys Ile Leu Glu Phe Lys Leu Arg Lys Leu Cys Thr Asp
   165 170 175
   Ile Lys Asp Asp Lys Val Ser Thr Lys Thr Leu Lys Glu Ile Thr Ser
   180 185 190
   Tyr Glu Phe Glu Leu Leu Ala Asp Tyr Leu Ala Asn Tyr Ser Glu Ile
   195 200 205
   Leu Lys Thr Gln Lys Phe Ser Tyr Thr Asp Lys Gln Gly Asn Leu Lys
   210 215 220
   Glu Leu Ser Tyr Tyr His His Asp Lys Tyr Asn Ile Gln Glu Phe Leu
   225 230 235 240
   Lys Arg His Ala Thr Ile Asn Ile Gln Ile Leu Asp Thr Leu Leu Thr
   245 250 255
   Asp Asp Leu Asp Ile Trp Asn Phe Asn Phe Glu Lys Phe Asp Phe Asp
   260 265 270
   Lys Asn Glu Glu Lys Leu Gln Asn Gln Glu Asp Lys Asp His Ile Gln
   275 280 285
   Ala His Leu His His Phe Val Phe Ala Val Asn Lys Ile Lys Ser Glu
   290 295 300
   Met Ala Ser Gly Gly Arg His Arg Ser Gln Tyr Phe Gln Glu Ile Thr
   305 310 315 320
   Asn Val Leu Asp Glu Asn Asn His Gln Glu Gly Tyr Leu Lys Asn Phe
   325 330 335
   Cys Glu Asn Leu His Asn Lys Lys Tyr Ser Asn Leu Ser Val Lys Asn
   340 345 350
   Leu Val Asn Leu Ile Gly Asn Leu Ser Asn Leu Glu Leu Lys Pro Leu
   355 360 365
   Arg Lys Tyr Phe Asn Asp Lys Ile His Ala Lys Ala Asp His Trp Asp
   370 375 380
   Glu Gln Lys Phe Thr Glu Thr Tyr Cys His Trp Ile Leu Gly Glu Trp
   385 390 395 400
   Arg Val Gly Val Lys Asp Gln Asp Lys Lys Asp Gly Ala Lys Tyr Ser
   405 410 415
   Tyr Lys Asp Leu Cys Asn Glu Leu Lys Gln Lys Val Thr Lys Ala Gly
   420 425 430
   Leu Val Asp Phe Leu Leu Glu Leu Asp Pro Cys Arg Thr Ile Pro Pro
   435 440 445
   Tyr Leu Asp Asn Asn Asn Arg Lys Pro Pro Lys Cys Gln Ser Leu Ile
   450 455 460
   Leu Asn Pro Lys Phe Leu Asp Asn Gln Tyr Pro Asn Trp Gln Gln Tyr
   465 470 475 480
   Leu Gln Glu Leu Lys Lys Leu Gln Ser Ile Gln Asn Tyr Leu Glu Ser
   485 490 495
   Phe Glu Thr Asp Leu Lys Val Leu Lys Ser Ser Lys Asp Gln Pro Tyr
   500 505 510
   Phe Val Glu Tyr Arg Ser Ser Asn Gln Gln Ile Ala Ser Gly Gln Arg
   515 520 525
   Asp Tyr Lys Asp Leu Asp Ala Arg Ile Leu Gln Phe Ile Phe Asp Arg
   530 535 540
   Val Lys Ala Ser Asp Glu Leu Leu Leu Asn Glu Ile Tyr Phe Gln Ala
   545 550 555 560
   Lys Lys Leu Lys Gln Lys Ala Ser Ser Glu Leu Glu Lys Leu Glu Ser
   565 570 575
   Ser Lys Lys Leu Asp Glu Val Ile Ala Asn Ser Gln Leu Ser Gln Ile
   580 585 590
   Leu Lys Ser Gln His Thr Asn Gly Ile Phe Glu Gln Gly Thr Phe Leu
   595 600 605
   His Leu Val Cys Lys Tyr Tyr Lys Gln Arg Gln Arg Ala Arg Asp Ser
   610 615 620
   Arg Leu Tyr Ile Met Pro Glu Tyr Arg Tyr Asp Lys Lys Leu His Lys
   625 630 635 640
   Tyr Asn Asn Thr Gly Arg Phe Asp Asp Asp Asn Gln Leu Leu Thr Tyr
   645 650 655
   Cys Asn His Lys Pro Arg Gln Lys Arg Tyr Gln Leu Leu Asn Asp Leu
   660 665 670
   Ala Gly Val Leu Gln Val Ser Pro Asn Phe Leu Lys Asp Lys Ile Gly
   675 680 685
   Ser Asp Asp Asp Leu Phe Ile Ser Lys Trp Leu Val Glu His Ile Arg
   690 695 700
   Gly Phe Lys Lys Ala Cys Glu Asp Ser Leu Lys Ile Gln Lys Asp Asn
   705 710 715 720
   Arg Gly Leu Leu Asn His Lys Ile Asn Ile Ala Arg Asn Thr Lys Gly
   725 730 735
   Lys Cys Glu Lys Glu Ile Phe Asn Leu Ile Cys Lys Ile Glu Gly Ser
   740 745 750
   Glu Asp Lys Lys Gly Asn Tyr Lys His Gly Leu Ala Tyr Glu Leu Gly
   755 760 765
   Val Leu Leu Phe Gly Glu Pro Asn Glu Ala Ser Lys Pro Glu Phe Asp
   770 775 780
   Arg Lys Ile Lys Lys Phe Asn Ser Ile Tyr Ser Phe Ala Gln Ile Gln
   785 790 795 800
   Gln Ile Ala Phe Ala Glu Arg Lys Gly Asn Ala Asn Thr Cys Ala Val
   805 810 815
   Cys Ser Ala Asp Asn Ala His Arg Met Gln Gln Ile Lys Ile Thr Glu
   820 825 830
   Pro Val Glu Asp Asn Lys Asp Lys Ile Ile Leu Ser Ala Lys Ala Gln
   835 840 845
   Arg Leu Pro Ala Ile Thr Thr Arg Ile Val Asp Gly Ala Val Lys Lys
   850 855 860
   Met Ala Thr Ile Leu Ala Lys Asn Ile Val Asp Asp Asn Trp Gln Asn
   865 870 875 880
   Ile Lys Gln Val Leu Ser Ala Lys His Gln Leu His Ile Pro Ile Ile
   885 890 895
   Thr Glu Ser Asn Ala Phe Glu Phe Glu Pro Ala Leu Ala Asp Val Lys
   900 905 910
   Gly Lys Ser Leu Lys Asp Arg Arg Lys Lys Ala Leu Glu Arg Ile Ser
   915 920 925
   Pro Glu Asn Ile Phe Lys Asp Lys Asn Asn Arg Ile Lys Glu Phe Ala
   930 935 940
   Lys Gly Ile Ser Ala Tyr Ser Gly Ala Asn Leu Thr Asp Gly Asp Phe
   945 950 955 960
   Asp Gly Ala Lys Glu Glu Leu Asp His Ile Ile Pro Arg Ser His Lys
   965 970 975
   Lys Tyr Gly Thr Leu Asn Asp Glu Ala Asn Leu Ile Cys Val Thr Arg
   980 985 990
   Gly Asp Asn Lys Asn Lys Gly Asn Arg Ile Phe Cys Leu Arg Asp Leu
   995 1000 1005
   Ala Asn Asn Tyr Lys Leu Lys Gln Phe Glu Thr Thr Asp Asp Leu Glu
   1010 1015 1020
   Ile Glu Lys Lys Ile Ala Asp Thr Ile Trp Asp Ala Asn Lys Lys Asp
   1025 1030 1035 1040
   Phe Lys Phe Gly Asn Tyr Arg Ser Phe Ile Asn Leu Thr Pro Gln Glu
   1045 1050 1055
   Gln Lys Ala Phe Arg His Ala Leu Phe Leu Ala Asp Glu Asn Pro Ile
   1060 1065 1070
   Lys Gln Ala Val Ile Arg Ala Ile Asn Asn Arg Asn Arg Thr Phe Val
               1075                1080                1085
   Asn Gly Thr Gln Arg Tyr Phe Ala Glu Val Leu Ala Asn Asn Ile Tyr
           1090                1095                1100
   Leu Arg Ala Lys Lys Glu Asn Leu Asn Thr Asp Lys Ile Ser Phe Asp
   1105                1110                1115                1120
   Tyr Phe Gly Ile Pro Thr Ile Gly Asn Gly Arg Gly Ile Ala Glu Ile
                   1125                1130                1135
   Arg Gln Leu Tyr Glu Lys Val Asp Ser Asp Ile Gln Ala Tyr Ala Lys
               1140                1145                1150
   Gly Asp Lys Pro Gln Ala Ser Tyr Ser His Leu Ile Asp Ala Met Leu
           1155                1160                1165
   Ala Phe Cys Ile Ala Ala Asp Glu His Arg Asn Asp Gly Ser Ile Gly
       1170                1175                1180
   Leu Glu Ile Asp Lys Asn Tyr Ser Leu Tyr Pro Leu Asp Lys Asn Thr
   1185                1190                1195                1200
   Gly Glu Val Phe Thr Lys Asp Ile Phe Ser Gln Ile Lys Ile Thr Asp
                   1205                1210                1215
   Asn Glu Phe Ser Asp Lys Lys Leu Val Arg Lys Lys Ala Thr Glu Gly
               1220                1225                1230
   Phe Asn Thr His Arg Gln Met Thr Arg Asp Gly Ile Tyr Ala Glu Asn
           1235                1240                1245
   Tyr Leu Pro Ile Leu Ile His Lys Glu Leu Asp Glu Val Arg Lys Gly
       1250                1255                1260
   Tyr Thr Trp Lys Asn Ser Glu Glu Ile Lys Ile Phe Lys Gly Lys Lys
   1265                1270                1275                1280
   Tyr Asp Ile Gln Gln Leu Asn Asn Leu Leu Tyr Cys Leu Lys Phe Val
                   1285                1290                1295
   Asp Lys Pro Ile Ser Ile Asp Ile Gln Ile Ser Thr Leu Glu Glu Leu
               1300                1305                1310
   Arg Asn Ile Leu Thr Thr Asn Asn Ile Ala Ala Thr Ala Glu Tyr Tyr
           1315                1320                1325
   Tyr Ile Asn Leu Lys Thr Gln Lys Leu His Glu Tyr Tyr Ile Glu Asn
       1330                1335                1340
   Tyr Asn Thr Ala Leu Gly Tyr Lys Lys Tyr Thr Lys Glu Met Glu Phe
   1345                1350                1355                1360
   Leu Arg Ser Leu Ala Tyr Arg Ser Glu Arg Val Lys Ile Lys Ser Ile
                   1365                1370                1375
   Asp Asp Val Lys Gln Val Leu Asp Lys Asp Ser Asn Phe Ile Ile Gly
               1380                1385                1390
   Lys Ile Thr Leu Pro Phe Lys Lys Glu Trp Gln Arg Leu Tyr Arg Glu
           1395                1400                1405
   Trp Gln Asn Ile Thr Ile Lys Asp Asp Tyr Glu Phe Leu Lys Ser Phe
       1410                1415                1420
   Phe Asn Val Lys Ser Ile Thr Lys Leu His Lys Lys Val Arg Lys Asp
   1425                1430                1435                1440
   Phe Ser Leu Pro Ile Ser Thr Asn Glu Gly Lys Phe Leu Val Lys Arg
                   1445                1450                1455
   Lys Thr Trp Asp Asn Asn Phe Ile Tyr Gln Ile Leu Asn Asp Ser Asp
               1460                1465                1470
   Ser Arg Val Asp Gly Thr Lys Pro Phe Ile Pro Ala Phe Asp Ile Ser
           1475                1480                1485
   Lys Asn Glu Ile Val Glu Ala Ile Ile Asp Ser Phe Thr Ser Lys Asn
       1490                1495                1500
   Ile Phe Trp Leu Pro Lys Asn Ile Glu Leu Gln Lys Val Asp Asn Lys
   1505                1510                1515                1520
   Asn Ile Phe Ala Ile Asp Thr Ser Lys Trp Phe Glu Val Glu Thr Pro
                   1525                1530                1535
   Ser Asp Leu Arg Asp Ile Gly Val Ala Thr Ile Gln Tyr Lys Ile Asp
               1540                1545                1550
   Asn Asn Ser Arg Pro Lys Val Arg Val Lys Leu Asp Tyr Val Ile Asp
           1555                1560                1565
   Asp Asp Ser Lys Ile Asn Tyr Phe Thr Asn His Ser Leu Leu Lys Ser
       1570                1575                1580
   Arg Tyr Pro Asp Lys Val Leu Glu Ile Leu Lys Gln Ser Thr Ile Ile
   1585                1590                1595                1600
   Glu Phe Glu Ser Ser Gly Phe Asn Lys Thr Ile Lys Glu Met Leu Gly
                   1605                1610                1615
   Met Thr Leu Ala Gly Ile Tyr Asn Glu Thr Ser Asn Asn
               1620                1625
<210> 206
   <211> 1123
   <212> PRT
   <213> Francisella tularensis
<220>
   <223> CRISPR-associated endonuclease
<308> YP_005317754
   <309> 2013-07-06
<400> 206
   Met Asn Val Lys Ile Leu Pro Ile Ala Ile Asp Leu Asp Val Lys Asn
   1               5                   10                  15
   Thr Gly Val Phe Ser Ala Phe Tyr Gln Lys Gly Thr Ser Leu Glu Lys
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Arg Arg His Lys Arg Arg Gly
       50                  55                  60
   Ile Asp Arg Lys Gln Leu Val Lys Arg Leu Phe Lys Leu Val Trp Thr
   65                  70                  75                  80
   Glu Gln Leu Asn Leu Glu Trp Asp Lys Asp Thr Gln Gln Ala Ile Ser
                   85                  90                  95
   Phe Leu Phe Asn Arg Arg Gly Phe Ser Phe Ile Thr Asp Gly Tyr Ser
               100                 105                 110
   Thr Glu Tyr Leu Asn Ile Val Pro Glu Gln Val Lys Ala Ile Leu Met
           115                 120                 125
   Asp Ile Phe Asp Asp Tyr Asn Gly Glu Asp Asp Leu Asp Ser Tyr Leu
       130                 135                 140
   Lys Leu Ala Thr Glu Gln Glu Ser Lys Ile Ser Glu Ile Tyr Asn Lys
   145                 150                 155                 160
   Leu Met Gln Lys Ile Leu Glu Phe Lys Leu Arg Lys Leu Cys Thr Asp
                   165                 170                 175
   Ile Lys Asp Asp Lys Val Ser Thr Lys Thr Leu Lys Glu Ile Thr Ser
               180                 185                 190
   Tyr Glu Phe Glu Leu Leu Ala Asp Tyr Leu Ala Asn Tyr Ser Glu Ser
           195                 200                 205
   Leu Lys Thr Gln Lys Phe Ser Tyr Thr Asp Lys Gln Gly Asn Leu Lys
       210                 215                 220
   Glu Leu Ser Tyr Tyr His His Asp Lys Tyr Asn Ile Gln Glu Phe Leu
   225                 230                 235                 240
   Lys Arg His Ala Thr Ile Asn Asp Glu Ile Leu Gly Thr Leu Leu Thr
                   245                 250                 255
   Asp Asp Phe Asp Ile Trp Asn Phe Asn Phe Glu Lys Phe Asp Phe Asp
               260                 265                 270
   Lys Asn Glu Glu Lys Leu Gln Asn Gln Glu Asp Lys Asp His Thr Gln
           275                 280                 285
   Ala His Leu His His Phe Val Phe Val Val Asn Lys Ile Lys Ser Glu
       290                 295                 300
   Met Ala Ser Gly Gly Arg His Arg Ser Gln Tyr Phe Gln Glu Ile Thr
   305                 310                 315                 320
   Asn Val Leu Asp Glu Asn Asn His Gln Glu Gly Tyr Leu Lys Asn Phe
                   325                 330                 335
   Cys Glu Asn Leu His Asn Lys Lys Tyr Ser Asn Leu Ser Val Lys Asn
                   340                 345                 350
   Leu Val Asn Leu Val Gly Asn Leu Ser Asn Leu Glu Leu Lys Pro Leu
               355                 360                 365
   Arg Lys Tyr Phe Asn Asp Lys Ile His Ala Lys Ala Asp His Trp Asp
       370                 375                 380
   Glu Gln Lys Phe Thr Glu Thr Tyr Cys His Trp Ile Leu Gly Glu Trp
   385                 390                 395                 400
   Arg Val Gly Val Lys Asp Gln Asp Lys Lys Asp Gly Ala Lys Tyr Ser
                   405                 410                 415
   Tyr Lys Asp Leu Cys Asn Glu Leu Lys Gln Lys Val Thr Lys Ala Gly
               420                 425                 430
   Leu Ile Asp Phe Leu Leu Glu Leu Asp Pro Cys Arg Thr Ile Pro Pro
           435                 440                 445
   Tyr Leu Asp Asn Asn Asn Arg Lys Pro Pro Lys Cys Gln Ser Leu Ile
       450                 455                 460
   Leu Asn Pro Lys Phe Leu Asp Asn Gln Tyr Pro Asn Trp Gln Gln Tyr
   465                 470                 475                 480
   Leu Gln Glu Leu Lys Lys Leu Gln Ser Ile Gln Asp Tyr Leu Asp Ser
                   485                 490                 495
   Phe Glu Thr Asp Leu Lys Asp Leu Lys Ser Ser Lys Asp Gln Pro Tyr
               500                 505                 510
   Phe Val Glu Tyr Lys Ser Ser Asn Gln Gln Met Ala Ser Gly Gln Arg
           515                 520                 525
   Asp Tyr Lys Asp Leu Asp Ala Arg Ile Leu Gln Phe Ile Phe Asp Arg
       530                 535                 540
   Val Lys Ala Ser Asp Glu Leu Leu Leu Asn Glu Ile Tyr Phe Gln Ala
   545                 550                 555                 560
   Lys Lys Leu Lys Gln Lys Ala Ser Ser Glu Leu Glu Lys Leu Glu Ser
                   565                 570                 575
   Ser Lys Lys Leu Asp Glu Val Ile Ala Asn Ser Gln Leu Ser Gln Ile
               580                 585                 590
   Leu Lys Ser Gln His Thr Asn Gly Ile Phe Glu Gln Gly Thr Phe Leu
           595                 600                 605
   His Leu Val Cys Lys Tyr Tyr Lys Gln Arg Gln Arg Ala Arg Asp Ser
       610                 615                 620
   Arg Leu Tyr Ile Met Pro Glu Tyr Arg Tyr Asp Lys Lys Leu Asp Lys
   625                 630                 635                 640
   Tyr Asn Asn Thr Gly Arg Phe Asp Asp Asn Asn Gln Leu Leu Thr Tyr
                   645                 650                 655
   Cys Asn His Lys Pro Arg Gln Lys Arg Tyr Gln Leu Leu Asn Asp Leu
               660                 665                 670
   Ala Gly Val Leu Gln Val Ser Arg Asn Gln Leu Leu Ser Ser Val Glu
           675                 680                 685
   Glu Trp Phe Gln Gln Ala Gln Arg Val Gly Glu Ile Ser Lys Ser Gln
       690                 695                 700
   Asp Glu Gln Ile Phe Glu Trp Leu Lys Ser Phe Lys Ile Ala Ser Tyr
   705                 710                 715                 720
   Cys Lys Ala Ala Val Glu Met Gln Lys Gln Tyr Arg Gly Thr Leu Lys
                   725                 730                 735
   Asn Ala Ile Gln Thr Ala Ile Phe Arg Gln Ser Glu Asn Ile Asn Lys
               740                 745                 750
   Asn Lys Asn Thr Gly Asn Gln Gln Gln Ala Leu Ser Glu Asn Ser Lys
           755                 760                 765
   Asp Val Lys Ser Leu Thr Ala Asp Glu Lys Lys Leu Leu Lys Leu Ile
       770                 775                 780
   Glu Asn Ile Ala Lys Ala Ser Gln Lys Ile Gly Glu Ser Leu Gly Leu
   785                 790                 795                 800
   Asn Asp Lys Gln Ile Lys Lys Phe Asn Ser Ile Tyr Ser Phe Ala Gln
                   805                 810                 815
   Ile Gln Gln Ile Ala Phe Ala Lys Arg Lys Gly Asn Ala Asn Thr Cys
               820                 825                 830
   Ala Val Cys Ser Ala Asp Asn Ala His Arg Met Gln Gln Ile Lys Ile
           835                 840                 845
   Thr Glu Leu Val Glu Asp Asn Lys Asp Asn Ile Ile Leu Ser Ala Lys
       850                 855                 860
   Ala Gln Arg Leu Pro Ala Ile Pro Thr Arg Ile Val Asp Gly Ala Val
   865                 870                 875                 880
   Lys Lys Met Ala Thr Ile Leu Ala Lys Asn Ile Val Asp Asp Asn Trp
                   885                 890                 895
   Gln Asn Ile Lys Gln Val Leu Ser Ala Lys His Gln Leu His Ile Pro
               900                 905                 910
   Ile Ile Thr Glu Ser Asn Ala Phe Glu Phe Glu Pro Ala Leu Ala Asp
           915                 920                 925
   Val Lys Gly Lys Ser Leu Lys Asp Arg Arg Lys Lys Ala Leu Glu Arg
       930                 935                 940
   Ile Ser Pro Glu Asn Ile Phe Lys Asp Lys Asn Asn Arg Ile Lys Glu
   945                 950                 955                 960
   Phe Ala Lys Gly Ile Ser Ala Tyr Ser Gly Ala Asn Leu Thr Asp Gly
                   965                 970                 975
   Asp Phe Asp Gly Ala Lys Glu Glu Leu Asp His Ile Ile Pro Arg Ser
               980                 985                 990
   His Lys Lys Tyr Gly Thr Leu Asn Asp Glu Ala Asn Leu Ile Cys Val
           995                 1000                1005
   Thr Arg Asp Asp Asn Lys Asn Ile Phe Ala Ile Asp Thr Ser Lys Trp
       1010                1015                1020
   Phe Glu Ile Glu Thr Pro Ser Asp Leu Arg Asp Ile Gly Val Ala Thr
   1025                1030                1035                1040
   Ile Gln Tyr Lys Ile Asp Asn Asn Ser Arg Pro Lys Val Arg Val Lys
                   1045                1050                1055
   Leu Asp Tyr Val Ile Asp Asp Asp Ser Lys Ile Asn Tyr Phe Met Asn
               1060                1065                1070
   His Ser Leu Leu Lys Ser Arg Tyr Pro Asp Lys Val Leu Glu Ile Leu
           1075                1080                1085
   Lys Gln Ser Thr Ile Ile Glu Phe Glu Ser Ser Gly Phe Asn Lys Thr
       1090                1095                1100
   Ile Lys Glu Met Leu Gly Met Thr Leu Ala Gly Ile Tyr Asn Glu Thr
   1105                1110                1115                1120
   Ser Asn Asn
<210> 207
   <211> 393
   <212> PRT
   <213> Francisella tularensis
<220>
   <223> CRISPR-associated endonuclease
<308> YP_513993
   <309> 2013-06-10
<400> 207
   Met Asn Val Lys Ile Leu Pro Ile Ala Ile Asp Leu Asp Val Lys Asn
   1               5                   10                  15
   Thr Gly Val Phe Ser Ala Phe Tyr Gln Lys Gly Thr Ser Leu Glu Lys
               20                  25                  30
   Leu Asp Asn Lys Asn Gly Lys Val Tyr Glu Leu Ser Lys Asp Ser Tyr
           35                  40                  45
   Thr Leu Leu Met Asn Asn Arg Thr Ala Gln Arg His Gln Arg Arg Gly
       50                  55                  60
   Ile Asp Arg Lys Gln Leu Val Lys Arg Leu Phe Lys Leu Val Trp Thr
   65                  70                  75                  80
   Glu Gln Leu Asn Leu Glu Trp Asp Lys Asp Thr Gln Gln Ala Ile Ser
                   85                  90                  95
   Phe Leu Phe Asn Arg Arg Gly Phe Ser Phe Ile Thr Asp Gly Tyr Ser
               100                 105                 110
   Thr Glu Tyr Leu Asn Ile Val Pro Glu Gln Val Lys Ala Ile Leu Met
           115                 120                 125
   Asp Ile Phe Asp Asp Tyr Asn Gly Glu Asp Asp Leu Asp Ser Tyr Leu
       130                 135                 140
   Lys Leu Ala Thr Glu Gln Glu Ser Lys Ile Ser Glu Ile Tyr Asn Lys
   145                 150                 155                 160
   Leu Met Gln Lys Ile Leu Glu Phe Lys Leu Arg Lys Leu Cys Thr Asp
                   165                 170                 175
   Ile Lys Asp Asp Lys Val Ser Thr Lys Thr Leu Lys Glu Ile Thr Ser
               180                 185                 190
   Tyr Glu Phe Glu Leu Leu Ala Asp Tyr Leu Ala Asn Tyr Ser Glu Ser
           195                 200                 205
   Leu Lys Thr Gln Lys Phe Ser Tyr Thr Asp Lys Gln Gly Asn Leu Lys
       210                 215                 220
   Glu Leu Ser Tyr Tyr His His Asp Lys Tyr Asn Ile Gln Glu Phe Leu
   225                 230                 235                 240
   Lys Arg His Ala Thr Ile Asn Asp Glu Ile Leu Asp Thr Leu Leu Thr
                   245                 250                 255
   Asp Asp Phe Asp Ile Trp Asn Phe Asn Phe Glu Lys Phe Asp Phe Asp
               260                 265                 270
   Lys Asn Glu Glu Lys Leu Gln Ser Gln Glu Asp Lys Asp His Thr Gln
           275                 280                 285
   Ala Tyr Phe His His Phe Val Phe Ala Val Asn Lys Ile Lys Ser Glu
       290                 295                 300
   Met Ala Ser Gly Gly Arg His Arg Ser Gln Tyr Phe Gln Glu Ile Thr
   305                 310                 315                 320
   Asn Val Leu Asp Glu Asn Asn His Gln Glu Gly Tyr Leu Lys Asn Phe
                   325                 330                 335
   Cys Glu Asn Leu His Asn Lys Lys Tyr Ser Asn Leu Ser Val Lys Asn
               340                 345                 350
   Leu Val Asn Leu Val Gly Asn Leu Ser Asn Leu Glu Leu Lys Pro Leu
           355                 360                 365
   Arg Lys Tyr Phe Asn Asp Lys Asn Leu Ile Ile Gly Met Ser Lys Ser
       370                 375                 380
   Leu Gln Lys Leu Ile Ala Thr Gly Tyr
   385                 390
<210> 208
   <211> 158
   <212> PRT
   <213> Francisella tularensis
<220>
   <223> CRISPR-associated endonuclease
<308> YP_513992
   <309> 2013-06-10
<400> 208
   Met Ile Asp Phe Leu Leu Glu Leu Asp Pro Cys Ile Thr Ile Pro Pro
    1               5                  10                  15
   Tyr Leu Asp Asn Asn Asn Arg Lys Pro Pro Lys Cys Gln Ser Leu Ile
               20                  25                  30
   Leu Asn Pro Lys Phe Leu Asp Asn Gln Tyr Pro Asn Trp Gln Gln Tyr
           35                  40                  45
   Leu Gln Glu Leu Lys Lys Leu Gln Ser Ile Gln Asp Tyr Leu Asp Ser
       50                  55                  60
   Phe Glu Thr Asp Leu Lys Asp Leu Lys Ser Ser Lys Asp Gln Pro Tyr
   65                  70                  75                  80
   Phe Val Glu Tyr Lys Ser Ser Asn Gln Gln Met Ala Ser Gly Gln Arg
                   85                  90                  95
   Asp Tyr Lys Asp Leu Asp Ala Arg Ile Leu Gln Phe Ile Phe Asp Arg
               100                 105                 110
   Val Lys Ala Ser Asp Glu Leu Leu Leu Ser Glu Ile Tyr Phe Gln Ala
           115                 120                 125
   Lys Lys Leu Lys Gln Lys Ala Ser Ser Glu Leu Glu Lys Leu Glu Ser
       130                 135                 140
   Ser Lys Lys Leu Asp Glu Val Ile Ala Asn Ser Gln Leu Ser
   145                 150                 155

## Description

The inventors have devised an approach for introducing one or more desired insertions and/or deletions of known sizes into one or more predefined locations in a nucleic acid (e.g., in a cell or organism genome). They developed techniques to do this either in a sequential fashion or by inserting a discrete DNA fragment of defined size into the genome precisely in a predefined location or carrying out a discrete deletion of a defined size at a precise location. The technique is based on the observation that DNA single-stranded breaks are preferentially repaired through the HDR pathway, and this reduces the chances of indels (e.g., produced by NHEJ) in the present invention and disclosure, and thus is more efficient than prior art techniques.

The inventors have also devised new techniques termed sequential endonuclease-mediated homology directed recombination (sEHDR) and sequential Cas-mediated homology directed recombination (sCHDR).

### BACKGROUND

Certain bacterial and archaea strains have been shown to contain highly evolved adaptive immune defence systems, CRISPR/Cas systems, which continually undergo reprogramming to direct degradation of complementary sequences present within invading viral or plasmid DNA. Short segments of foreign DNA, called spacers, are incorporated into the genome between CRISPR repeats, and serve as a 'memory' of past exposures. CRISPR spacers are then used to recognize and silence exogenous genetic elements in a manner analogous to RNAi in eukaryotic organisms.

The Clustered Regularly Interspaced Short Palindromic Repeats (CRISPR) system including the CRISPR associated (Cas) protein has been reconstituted *in vitro* by a number of research groups allowing for the DNA cleavage of almost any DNA template without the caveat of searching for the right restriction enzyme cutter. The CRISPR/Cas system also offers a blunt end cleavage creating a dsDNA or, using mutated Cas versions, a selective single strand-specific cleavage (see Cong *et al.,* Wang *et al.* & Mali *et al.* cited below).

Through *in vitro* studies using *Streptococcus pyogenes* type II CRISPR/Cas system it has been shown that the only components required for efficient CRISPR/Cas-mediated target DNA or genome modification are a Cas nuclease (e.g., a Cas9 nuclease), CRISPR RNA (crRNA) and transactivating crRNA (tracrRNA). The wild-type mechanism of CRISPR/Cas-mediated DNA cleavage occurs via several steps. Transcription of the CRISPR array, containing small fragments (20-30 base-pairs) of the encountered (or target) DNA, into pre-crRNA, which undergoes maturation through the hybridisation with tracrRNA via direct repeats of pre-crRNA. The hybridisation of the pre-crRNA and tracrRNA, known as guide RNA (gRNA or sgRNA), associates with the Cas nuclease forming a ribonucleoprotein complex, which mediates conversion of pre-crRNA into mature crRNA. Mature crRNA:tracrRNA duplex directs Cas9 to the DNA target consisting of the protospacer and the requisite protospacer adjacent motif (CRISPR/cas protospacer-adjacent motif; PAM) via heteroduplex formation between the spacer region of the crRNA and the protospacer DNA on the host genome. The Cas9 nuclease mediates cleavage of the target DNA upstream of PAM to create a double-stranded break within the protospacer or a strand-specific nick using mutated Cas9 nuclease whereby one DNA strand-specific cleavage motif is mutated (for example, Cas9 nickase contains a D10A substitution) (Cong *et al*.).

It is worth noting that different strains of *Streptococcus* have been isolated which use PAM sequences that are different from that used by *Streptococcus pyogenesCas9.* The latter requires a NGG PAM sequence. CRISPR/Cas systems (for example, the Csy4 endoribonulcease in *Pseudomonas aeroginosa* (see Shah *et al*.) have been described in other prokaryotic species, which recognise a different PAM sequence (e.g., CCN, TCN, TTC, AWG, CC, NNAGNN, NGG, NGGNG). It is noteworthy that the Csy4 (also known as Cas6f) has no sequence homology to Cas9 but the DNA cleavage occurs through a similar mechanism involving the assembly of a Cas-protein-crRNA complex that facilitates target DNA recognition leading to specific DNA cleavage (Haurwitz *et al*.).

*In vitro*-reconstituted type II CRISPR/Cas system has been adapted and applied in a number of different settings. These include creating selective gene disruption in single or multiple genes in ES cells and also single or multiple gene disruption using a one-step approach using zygotes to generate biallelic mutations in mice. The speed, accuracy and the efficiency at which this system could be applied to genome editing in addition to its multiplexing capability makes this system vastly superior to its predecessor genome editing technologies namely zinc finger nucleases (ZFNs), transcription activator-like effector nucleases (TALENs) and engineered homing meganucleases (Gaj *et al.* & Perez-Pinera *et al*.). These have been successfully used in various eukaryotic hosts but they all suffer from important limitations; notably off-target mutagenesis leading to nuclease-related toxicity, and also the time and cost of developing such engineered proteins. The CRISPR/Cas system on the other hand is a superior genome editing system by which mutations can be introduced with relative ease, simply by designing a single guided RNA complementary to the protospacer sequence on the target DNA.

The dsDNA break induced by an endonuclease, such as Cas9, is subsequently repaired through non-homologous end joining mechanism (NHEJ), whereby the subsequent DNA repair at the breakpoint junction is stitched together with different and unpredictable inserted or deletions (indels) of varying size. This is highly undesirable when precise nucleic acid or genome editing is required. However a predefined precise mutation can be generated using homology directed repair (HDR), e.g., with the inclusion of a donor oligo or donor DNA fragment. This approach with Cas9 nuclease has been shown to generate precise predefined mutations but the efficiency at which this occurs in both alleles is low and mutation is seen in one of the strands of the dsDNA target (Wang *et al*.).

The CRISPR/Cas system does therefore have some limitations in its current form. While it may be possible to modify a desired sequence in one strand of dsDNA, the sequence in the other strand is often mutated through undesirable NHEJ.

### SUMMARY

The invention is as set out in the claims.

### A first configuration provides:-

A method of nucleic acid recombination, the method comprising providing dsDNA comprising first and second strands and
(a) using nucleic acid cleavage to create 5' and 3' cut ends in the first strand;
(b) using homologous recombination to insert a nucleotide sequence between the ends, thereby producing a modified first strand; thereby producing DNA wherein the first strand has been modified by said recombination but the second strand has not been modified; and
(c) optionally replicating the modified first strand to produce a progeny dsDNA wherein each strand thereof comprises a copy of the inserted nucleotide sequence; and isolating the progeny dsDNA.

### A second configuration provides:-

A method of nucleic acid recombination, the method comprising
(a) using nucleic acid cleavage to create 5' and 3' cut ends in a single nucleic acid strand;
(b) using homologous recombination to insert a nucleotide sequence between the ends, wherein the insert sequence comprises a regulatory element or encodes all or part of a protein; and
(c) optionally obtaining the nucleic acid strand modified in step (b) or a progeny nucleic strand comprising the inserted nucleotide sequence.

### A third configuration provides:-

A method of nucleic acid recombination, the method comprising
(a) using nucleic acid cleavage to create first and second breaks in a nucleic acid strand, thereby creating 5' and 3' cut ends and a nucleotide sequence between the ends;
(b) using homologous recombination to delete the nucleotide sequence; and
(c) optionally obtaining the nucleic acid strand modified in step (b) or a progeny nucleic strand comprising the deletion.

### A fourth configuration provides:-

A method of nucleic acid recombination, the method comprising providing dsDNA comprising first and second strands and
(a) using Cas endonuclease-mediated nucleic acid cleavage to create a cut end in the first strand 3' of a PAM motif;
(b) using Cas endonuclease-mediated nucleic acid cleavage to create a cut in the second strand at a position which corresponds to a position 3' of the cut end of the strand of part (a), which cut is 3' of the PAM motif;
(c) providing a first gRNA which hybridises with a sequence 5' to the PAM motif in the strand of part (a)
(d) providing a second gRNA which hybridises with a sequence 5' to the PAM motif in the strand of part (b)
wherein the nucleic acid strands of part (a) and part (b) are repaired to produce a deletion of nucleic acid between the cuts.

In aspects of the configurations herein there is provided a method of sequential endonuclease-mediated homology directed recombination (sEHDR) comprising carrying out the method of any preceding configuration a first time and carrying out the method of any preceding configuration a second time. In this way, the disclosure enables serial nucleic acid modifications, e.g., genome modifications, to be carried out, which may comprise precise sequence deletions, insertions or combinations of these two or more times. For example, it is possible to use this aspect to "walk along" nucleic acids (e.g., chromosomes in cells) to make relatively large and precise nucleotide sequence deletions or insertions. In an embodiment, one or more Cas endonucleases (e.g., a Cas9 and/or Cys4) is used in a method of sequential Cas-mediated homology directed recombination (sCHDR).

### BRIEF DESCRIPTION OF THE FIGURES

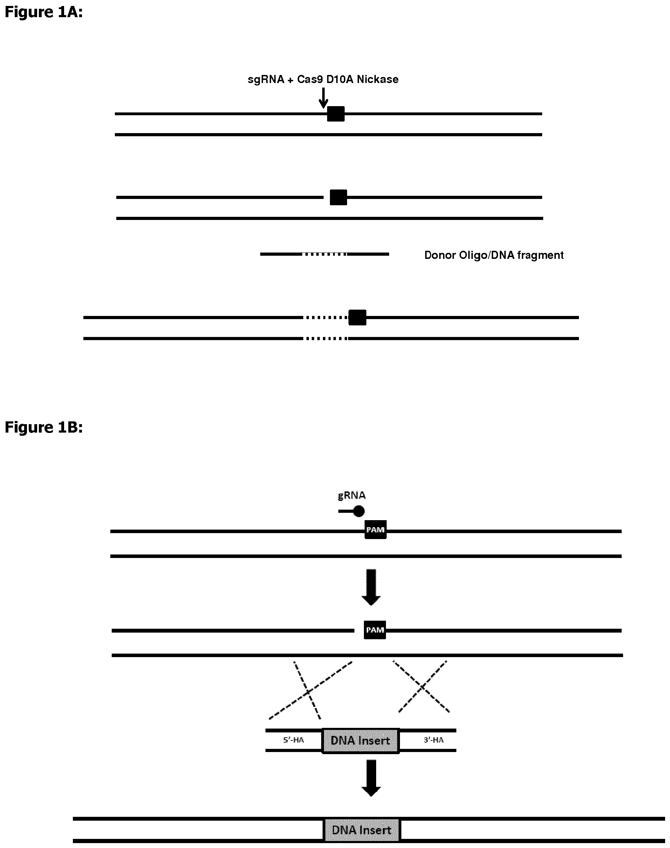

**Figure 1A****. Precise DNA Insertion in a Predefined Location (KI):** gRNA designed against a predefined location can induce DNA nick using Cas9 D10A nickase 5' of the PAM sequence (shown as solid black box). Alternatively, gRNA can be used together with Cas9 wild-type nuclease to induce double-stranded DNA breaks 5' of the PAM sequence. The addition of a donor oligo or a donor DNA fragment (single or double stranded) with homology around the breakpoint region containing any form of DNA alterations including addition of endogenous or exogenous DNA can be precisely inserted at the breakpoint junction where the DNA is repaired through HDR.
**Figure 1B****. Precise DNA Insertion in a Predefined Location (KI):** This figure shows a more detailed description of the mechanism described in Figure 1A. sgRNA designed against a predefined location can induce DNA nick using Cas9 D10A nickase 5' of the PAM sequence (shown as solid black box). Alternatively, sgRNA can be used together with Cas9 wild-type nuclease to induce double-stranded DNA breaks 5' of the PAM sequence. The addition of a donor oligo or a donor DNA fragment (single or double stranded) with homology arms (HA) around the breakpoint region containing any form of DNA alterations including addition of endogenous or exogenous DNA, can be precisely inserted at the breakpoint junction where the DNA is repaired through HDR.

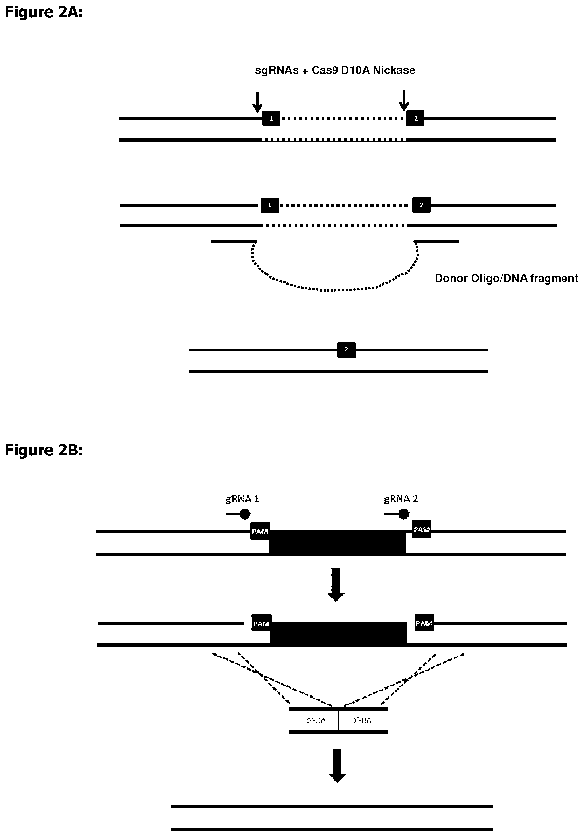

**Figure 2A****. Precise DNA Deletion (KO):** gRNAs targeting flanking region of interest can induce two DNA nicks using Cas9 D10A nickase in predefine locations containing the desired PAM sequences (shown as solid black box). Alternatively, gRNAs can be used with Cas9 wild-type nuclease to induce two DSB flanking the region of interest. Addition of a donor oligo or a donor DNA fragment (single or double stranded) with homology to region 5' of PAM 1 and 3' of PAM 2 sequence will guide DNA repair in a precise manner via HDR. DNA repair via HDR will reduce the risk of indel formation at the breakpoint junctions and avoid DNA repair through NHEJ and in doing so, it will delete out the region flanked by the PAM sequence and carry out DNA repair in a pre-determined and pre-defined manner.
**Figure 2B****. Precise DNA Deletion (KO):** This figure shows a more detailed description of the mechanism described in Figure 2A. sgRNAs targeting flanking region of interest can induce two DNA nicks using Cas9 D10A nickase in predefine locations containing the desired PAM sequences (shown as solid black box). Note. The PAMs can be located in opposite DNA strands as suppose to the example depicted in the figure where both PAMs are on the same DNA strand. Alternatively, sgRNAs can be used with Cas9 wild-type nuclease to induce two DSB flanking the region of interest. Addition of a donor oligo or a donor DNA fragment (single or double stranded) with homology to region 5' of PAM 1 and 3' of PAM 2 sequence will guide DNA repair in a precise manner via HDR. DNA repair via HDR will reduce the risk of indel formation at the breakpoint junctions and avoid DNA repair through NHEJ and in doing so, it will delete out the region flanked by the PAM sequence and carry out DNA repair in a pre-determined and pre-defined manner.

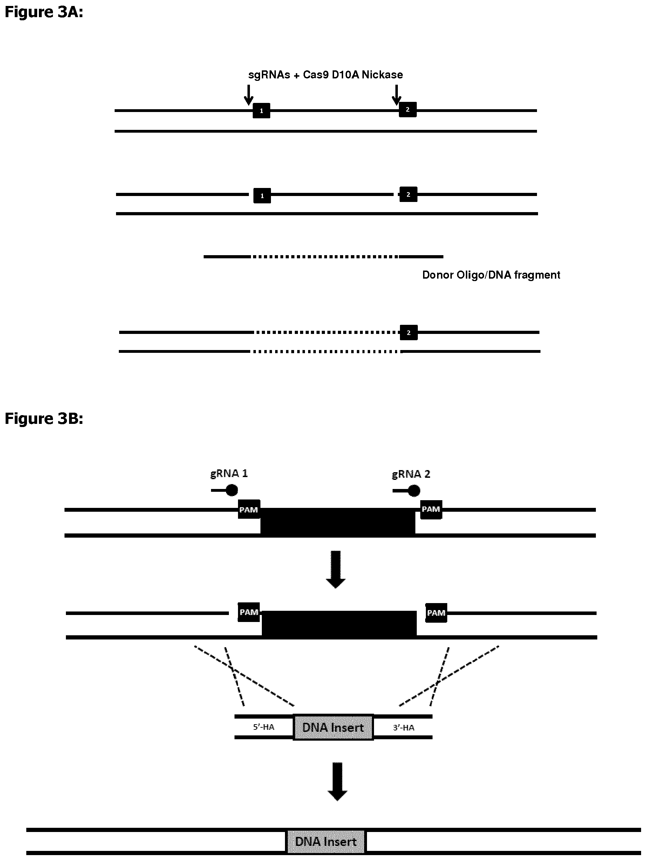

**Figure 3A****: Precise DNA Deletion and Insertion (KO → KI):** gRNAs targeting flanking region of interest can induce two DNA nicks using Cas9 D10A nickase in predefine locations containing the desired PAM sequences (shown as solid black box). Alternatively, gRNAs can be used with Cas9 wild-type nuclease to induce two DSB flanking the region of interest. Addition of a donor oligo or a donor DNA fragment (single or double stranded) with homology to region 5' of PAM 1 and 3' to PAM 2 with inclusion of additional endogenous or exogenous DNA, will guide DNA repair in a precise manner via HDR with the concomitant deletion of the region flanked by DSB or nick and the insertion of DNA of interest.
**Figure 3B****: Precise DNA Deletion and Insertion (KO → KI):** s This figure shows a more detailed description of the mechanism described in Figure 3A. gRNAs targeting flanking region of interest can induce two DNA nicks using Cas9 D10A nickase in predefine locations containing the desired PAM sequences (shown as solid black box). Alternatively, sgRNAs can be used with Cas9 wild-type nuclease to induce two DSB flanking the region of interest. Addition of a donor oligo or a donor DNA fragment (single or double stranded) with homology to region 5' of PAM 1 and 3' to PAM 2 with inclusion of additional endogenous or exogenous DNA (DNA insert), will guide DNA repair in a precise manner via HDR with the concomitant deletion of the region flanked by DSB or nick and the insertion of DNA of interest. Note. Once again, the PAMs can be located in opposite DNA strands as suppose to the example depicted in the figure where both PAMs are on the same DNA strand

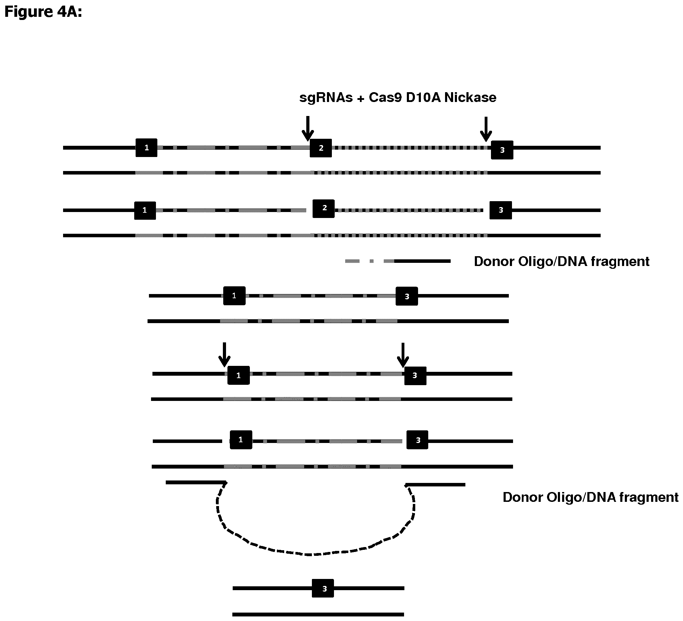

**Figure 4A****: Recycling PAM For Sequential Genome Editing (Deletions):** gRNAs targeting flanking region of interest can induce two DNA nicks using Cas9 D10A nickase in predefine locations containing the desired PAM sequences (shown as solid black box). Alternatively, gRNAs can be used with Cas9 wild-type nuclease to induce two DSB flanking the region of interest. Addition of a donor oligo or a donor DNA fragment (single or double stranded) with homology to region 5' of PAM 2 and 3' of PAM 3 will guide DNA repair in a precise manner via HDR and in doing so, it will delete out the region between PAM 2 and PAM3. This deletion will retain PAM 3 and thus acts as a site for carrying out another round of CRISPR/Cas mediated genome editing. Another PAM site (e.g.. PAM 1) can be used in conjunction with PAM 3 sequence to carry out another round of deletion as described above. Using this PAM recycling approach, many rounds of deletions can be performed in a stepwise deletion fashion, where PAM 3 is recycled after each round. This approach can be used also for the stepwise addition of endogenous or exogenous DNA.

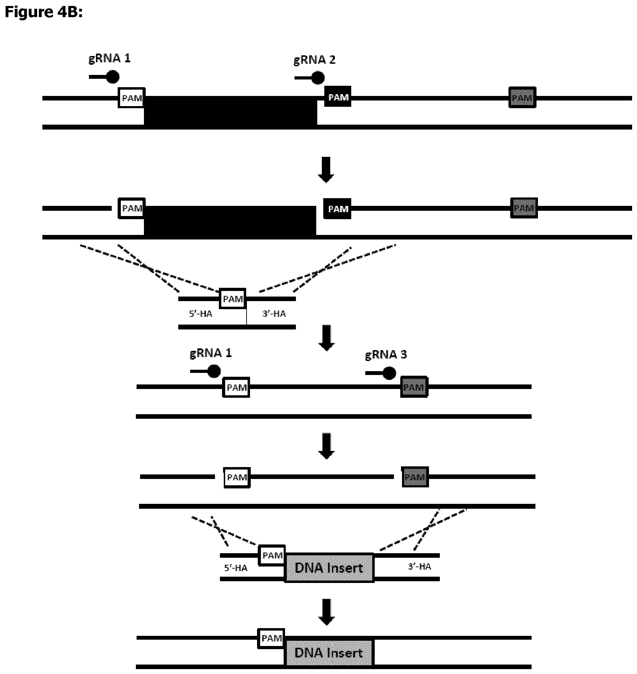

**Figure 4B****: Recycling PAM For Sequential Genome Editing (Deletions):** This figure shows a more detailed description of the mechanism described in Figure 4B. sgRNAs targeting flanking region of interest can induce two DNA nicks using Cas9 D10A nickase in predefine locations containing the desired PAM sequences. Alternatively, sgRNAs can be used with Cas9 wild-type nuclease to induce two DSB flanking the region of interest. Addition of a donor oligo or a donor DNA fragment (single or double stranded) with homology to region 5' of PAM 1 (clear PAM box) and 3' of PAM 2 (black PAM box) will guide DNA repair in a precise manner via HDR and in doing so, it will delete out the region between PAM 1 and PAM 2. PAM sequence together with unique gRNA can be included in the intruding DNA and targeted back into the site of editing. In this, PAM 1 sequence for example can be recycled and thus acts as a site for carrying out another round of CRISPR/Cas mediated genome editing. Another PAM site (eg. PAM 3, grey PAM box) can be used in conjunction with the recycled PAM 1 sequence to carry out another round of editing (i.e. Insertion) as described above. Using this PAM recycling approach, many rounds of genome editing can be performed in a stepwise fashion, where PAM 1 is recycled after each round. This approach can be used also for the stepwise addition of endogenous or exogenous DNA.

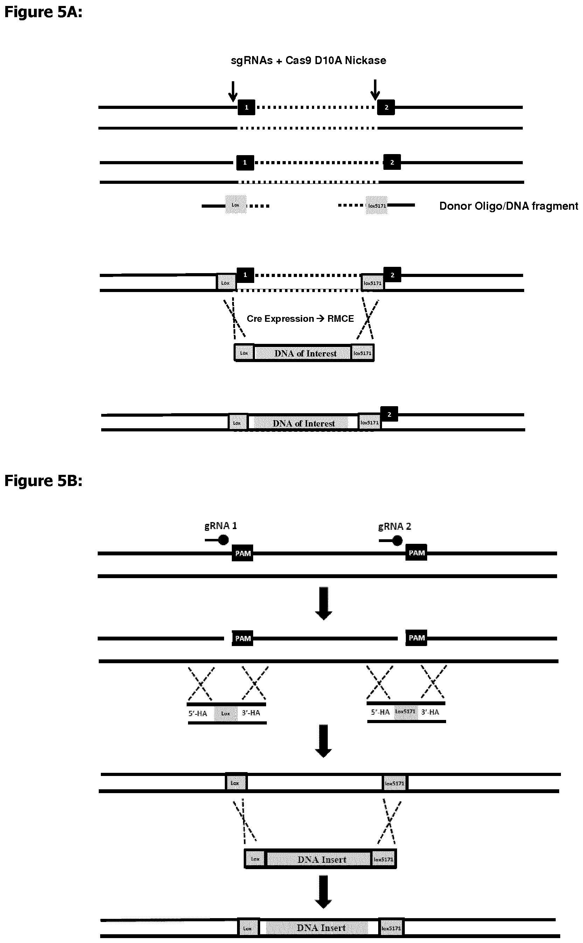

**Figure 5A****: CRISPR/Cas mediated Lox Insertion to facilitate RMCE:** gRNAs targeting flanking region of interest can induce two DNA nicks using Cas9 D10A nickase in predefine locations containing the desired PAM sequences (shown as solid black box). Alternatively, gRNAs can be used with Cas9 wild-type nuclease to induce two DSB flanking the region of interest. Addition of two donor oligos or donor DNA fragments (single or double stranded) with homology to regions 5' and 3' of each PAM sequence where the donor DNA contains recombinase recognition sequence (RRS) such as loxP and lox5171 will guide DNA repair in a precise manner via HDR with the inclusion of these RRS. The introduced RRS can be used as a landing pad for inserting any DNA of interest with high efficiency and precisely using recombinase mediated cassette exchange (RMCE). The retained PAM 2 site can be recycled for another round of CRISPR/Cas mediated genome editing for deleting or inserting DNA of interest. Note, the inserted DNA of interest could contain selection marker such as PGK-Puro flanked by PiggyBac LTR to allow for the initial selection and upon successful integration into DNA of interest, the selection marker can be removed conveniently by expressing hyperPbase transposase.
**Figure 5B****: CRISPR/Cas mediated Lox Insertion to facilitate RMCE:** This figure shows a more detailed description of the mechanism described in Figure 5A. sgRNAs targeting flanking region of interest can induce two DNA nicks using Cas9 D10A nickase in predefine locations containing the desired PAM sequences (shown as solid black box). Alternatively, sgRNAs can be used with Cas9 wild-type nuclease to induce two DSB flanking the region of interest. Addition of two donor oligos or donor DNA fragments (single or double stranded) with homology to regions 5' and 3' of each PAM sequences where the donor DNA contains recombinase recognition sequence (RRS) such as loxP and lox5171 will guide DNA repair in a precise manner via HDR with the inclusion of these RRS. Note. The targeting of the lox sites can be done sequentially or as a pool in a single step process. The introduced RRS can be used as a landing pad for inserting any DNA of interest with high efficiency and precisely using recombinase mediated cassette exchange (RMCE). As detailed in Figure 4, the PAM sequence can be recycled for another round of CRISPR/Cas mediated genome editing for deleting or inserting DNA of interest. As an option, the inserted DNA of interest could contain selection marker such as PGK-Puro flanked by PiggyBac LTR to allow for the initial selection and upon successful integration into DNA of interest, the selection marker can be removed conveniently by expressing hyperPbase transposase.

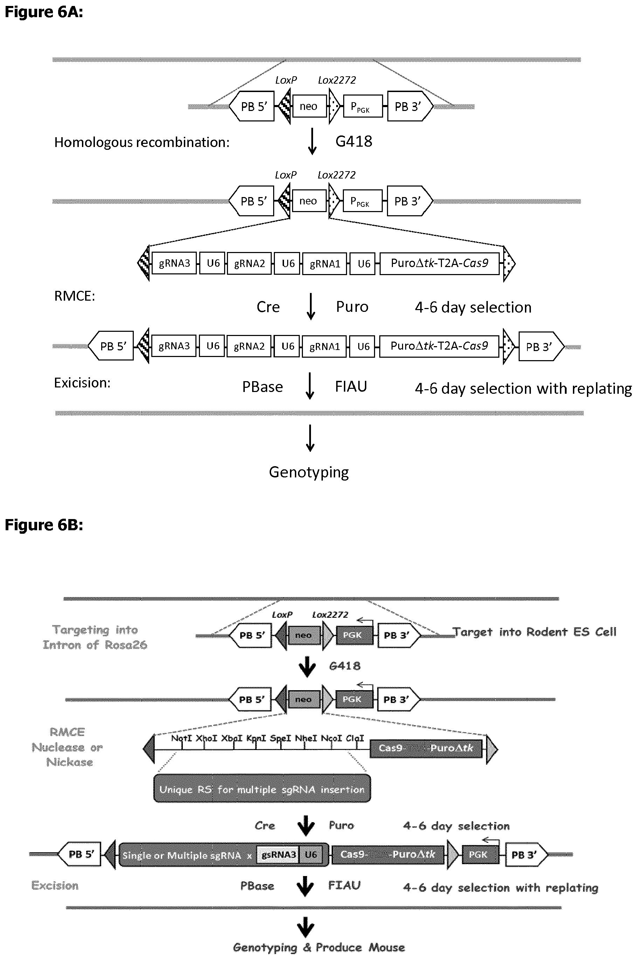

**Figure 6A and 6B****: Genome modification to produce transposon-excisable Cas9 and gRNA**

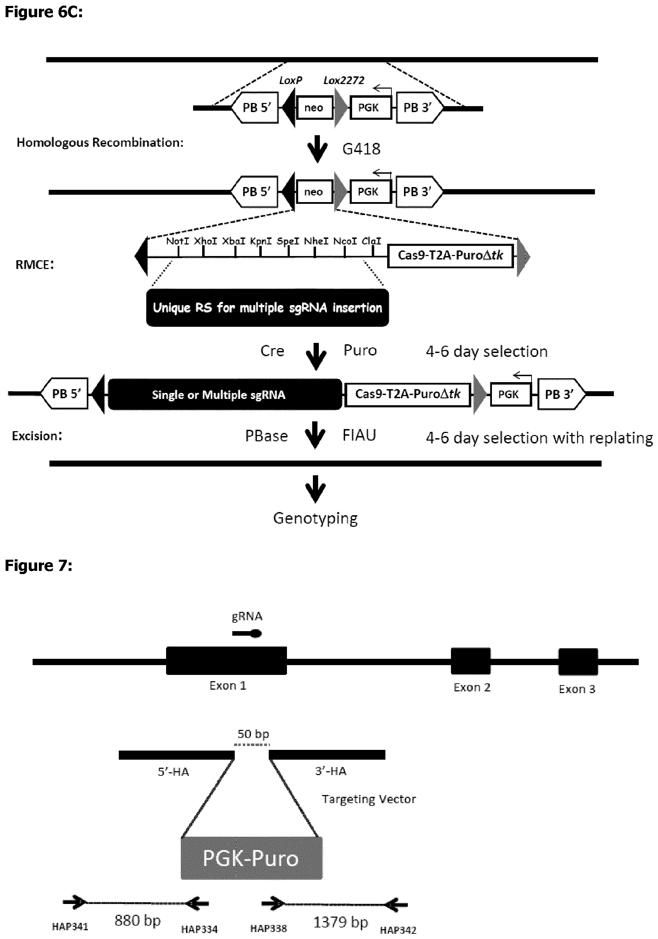

**Figure 6C****: Single copy Cas9 Expression:** A landing pad initially can be targeted into any locus of choice in mouse ES cells or any other eukaryotic cell line. The landing pad will typically contain PiggyBac 5' and 3' LTR, selection marker such as neo for example floxed and a gene less promoter such as PGK in the general configuration shown. Targeting is done by homologous recombination and clones are selected on G418. The next step will involve RMCE to insert Cas9 linked via a T2A sequence to Puro-delta-tk with the option to insert single or multiple guide RNA using the unique restriction sites (RS). The orientation of the lox sites are positioned in a manner that only once the intruding DNA containing the Cas9 is inserted into the landing pad, the PGK promoter on the landing pad can activate the transcription and thus the expression of the puromycin and via the T2A transcribe and expression Cas9 production. Using this approach a single stable expression of Cas9 can be achieved. Following 4-6 days of selection on puromycin, the entire Cas9 and guide RNA floxed cassette can be excised using PiggyBac transposase (Pbase) and individual clones can be analysed for genome editing resulting from the introduced guide RNA. As an option, a stable bank cell line expressing Cas9 can be generated from which multiple engineered cell lines can be generated. To do this, only Cas9-T2A-Puro-delta-tk will be inserted and no gRNA at the stage of RMCE. This will produce a general single copy Cas9 expressing cell line where its genome can be edited by transfecting single or multiple gRNA.
**Figure 7****: Schematic representing the gRNA position with respect to gene X, the structure of the targeting vector and the oligo pair used for genotyping the resulting targeted clones.**

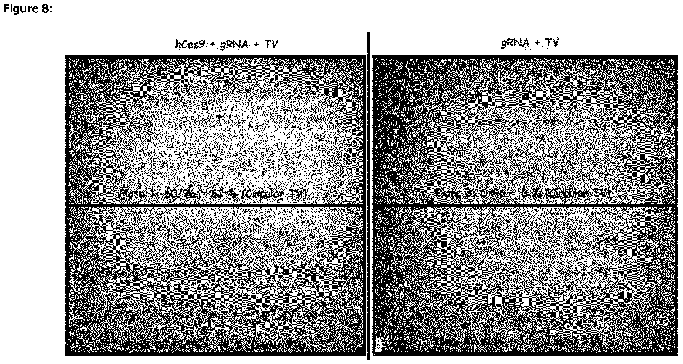

**Figure 8****: A gel image showing the genotyping results following Cas9 nuclease mediated double stranded DNA break and the subsequent DNA targeting.** The genotyping shows PCR product (880 bp) specific for the 5'targeted homology arm using oligo pair HAP341/HAP334. The left hand gels show genotyping data from 96 ES cell clones transfected with gRNA, human Cas9 nuclease and either a circular targeting vector (plate 1) or a linear targeting vector (Plate 2). The right hand side gels shows 96 ES cell clones transfected with gRNA and either a circular targeting vector (plate 3) or a linear targeting vector (Plate 4) but with no human Cas9 nuclease. The percentage of the clones correctly targeted is shown for each transfection.

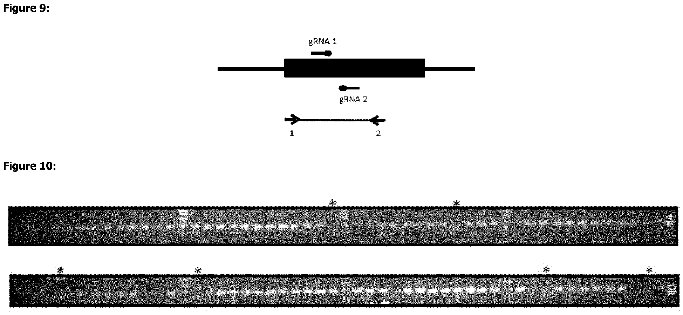

**Figure 9****: Schematic showing the position of the gRNAs on a gene to allow for a define deletion of the region in between the two gRNA.** The oligo pair primer 1 and 2 was used to detect ES clones containing the specific 55 bp deletion.
**Figure 10****: A 3% agarose gel containing PCR products amplified from 96 ES clones transfected with gRNA 1 and 2.** Primers 1 and 2 was used to amplify around the two gRNA and any clones containing the define deletion can be seen as a smaller PCR product, which are highlighted by an asterix.

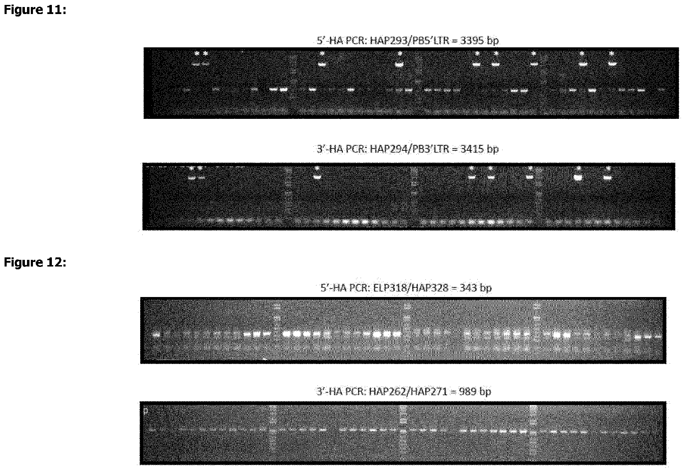

**Figure 11****: PCR genotyping by amplifying the 5' (top gel) and 3' (bottom gel) targeted homology arms within the Rosa26 gene located on chromosome 6.** Correctly targeted clones yielding PCR product for both 5' and 3' junctions are marked with an asterix.
**Figure 12****: Genotyping for the correct insertion of the Cas9 DNA cassette by PCR amplifying the 5' (top gel) and 3' (bottom gel) arm of the inserted DNA cassette.**

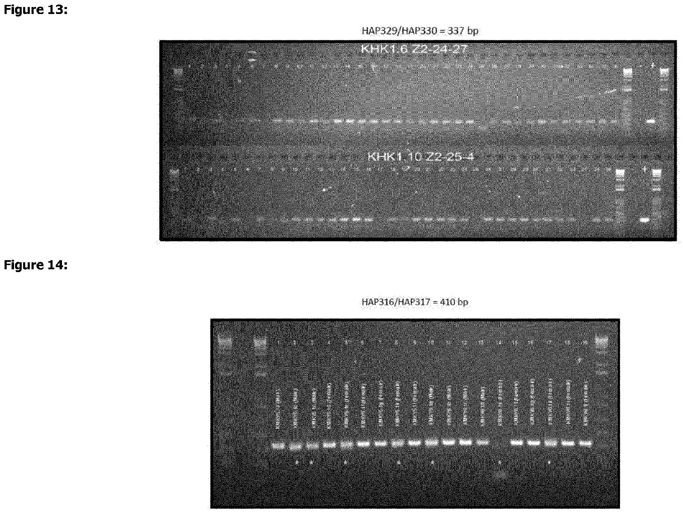

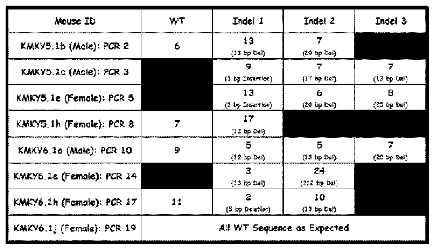

**Figure 13****: PCR genotyping by amplifying the region around the guide RNA and assessing the PCR product for the presence of indels.** Larger indels can be seen directly from the gel as they yielded PCR product shorter than the expected WT DNA suggesting significant deletion. For the positive control, genomic DNA from mouse AB2.1 was used to size the corresponding WT PCR product. The negative control was a no DNA water control.
**Figure 14****: PCR amplification of the region flanking the guide RNA using DNA extracted from pups following zygote Cas9/guide mRNA injection for analysing indel formation.** Lane 14 shows a gross deletion in that mouse and those lanes marked with an asterix indicate these mice contain smaller indels.

**Table 3:** Summary of the sequencing data from the 8 mice analysed and the details of the indels detected are shown. The number refers to the frequency of that particular indel identified in the clones analysed and the description of the indels are shown in brackets.

### DETAILED DESCRIPTION

The inventors addressed the need for improved nucleic acid modification techniques. An example of a technique for nucleic acid modification is the application of the CRISPR/Cas system. This system has been shown thus far to be the most advanced genome editing system available due, *inter alia,* to its broad application, the relative speed at which genomes can be edited to create mutations and its ease of use. The inventors, however, believed that this technology can be advanced for even broader applications than are apparent from the state of the art.

The inventors realised that an important aspect to achieve this would be to find a way of improving the fidelity of nucleic acid modifications beyond that contemplated by the CRISPR/Cas methods known in the art.

Additionally, the inventors realised that only modest nucleic acid modifications had been reported to date. It would be desirable to effect relatively large predefined and precise DNA deletions or insertions using the CRISPR/Cas system.

The inventors have devised an approach for introducing one or more desired insertions and/or deletions of known sizes into one or more predefined locations in a nucleic acid (e.g., in a cell or organism genome). They developed techniques to do this either in a sequential fashion or by inserting a discrete DNA fragment of defined size into the genome precisely in a predefined location or carrying out a discrete deletion of a defined size at a precise location. The technique is based on the observation that DNA single-stranded breaks are preferentially repaired through the HDR pathway, and this reduces the chances of indels (e.g., produced by NHEJ), and thus is more efficient than prior art techniques.

To this end, there is provided:-
A method of nucleic acid recombination, the method comprising providing double stranded DNA (dsDNA) comprising first and second strands and
(a) using nucleic acid cleavage to create 5' and 3' cut ends in the first strand; and
(b) using homologous recombination to insert a nucleotide sequence between the ends, thereby producing a modified first strand; thereby producing DNA wherein the first strand has been modified by said recombination but the second strand has not been modified.

Optionally, the method further comprises replicating the modified first strand to produce a progeny dsDNA wherein each strand thereof comprises a copy of the insert nucleotide sequence. Optionally, the method comprises (c) isolating the progeny dsDNA, e.g., by obtaining a cell containing said progeny dsDNA. Replication can be effected, for example in a cell. For example, steps (a) and (b) are carried out in a cell and the cell is replicated, wherein the machinery of the cell replicates the modified first strand, e.g., to produce a dsDNA progeny in which each strand comprises the modification.

Optionally, in any configuration, aspect, example or embodiment, the modified DNA strand resulting from step (b) is isolated.

Optionally, in any configuration, aspect, example or embodiment, the method is carried out *in vitro.* For example, the method is carried out in a cell or cell population *in vitro.*

Alternatively, optionally, in any configuration, aspect, example or embodiment, the method is carried out to modify the genome of a virus.

Alternatively, optionally, in any configuration, aspect, example or embodiment, the method is carried out *in vivo* in an organism. In an example, the organism is a non-human organism. In an example, it is a plant or an animal or an insect or a bacterium or a yeast. For example, the method is practised on a vertebrate (e.g., a human patient or a non-human vertebrate (e.g., a bird, e.g., a chicken) or non-human mammal such as a mouse, a rat or a rabbit).

Optionally, in any configuration, aspect, example or embodiment, the method is a method of cosmetic treatment of a human or a non-therapeutic, non-surgical, non-diagnostic method, e.g., practised on a human or a non-human vertebrate or mammal (e.g., a mouse or a rat).

There isalso provided:-
A method of nucleic acid recombination, the method comprising
(a) using nucleic acid cleavage to create 5' and 3' cut ends in a single nucleic acid strand;
(b) using homologous recombination to insert a nucleotide sequence between the ends, wherein the insert sequence comprises a regulatory element or encodes all or part of a protein; and
(c) Optionally obtaining the nucleic acid strand modified in step (b) or a progeny nucleic strand comprising the inserted nucleotide sequence, e.g., by obtaining a cell containing said progeny nucleic acid strand.

In an example the progeny strand is a product of the replication of the strand produced by step (b). The progeny strand is, for example, produced by nucleic acid replication in a cell. For example, steps (a) and (b) are carried out in a cell and the cell is replicated, wherein the machinery of the cell replicates the modified strand produced in step (b), e.g., to produce a dsDNA progeny in which each strand comprises the modification.

In an example, the single nucleic acid strand is a DNA or RNA strand.

In an example, the regulatory element is a promoter or enhancer.

Optionally, in any configuration, aspect, example or embodiment, the inserted nucleotide sequence is a plant, animal, vertebrate or mammalian sequence, e.g., a human sequence. For example, the sequence encodes a complete protein, polypeptide, peptide, domain or a plurality (e.g. one, two or more) of any one of these. In an example, the inserted sequence confers a resistance property to a cell comprising the modified nucleic acid produced by the method (e.g., herbicide, viral or bacterial resistance). In an example, the inserted sequence encodes an interleukin, receptor (e.g., a cell surface receptor), growth factor, hormone, antibody (or variable domain or binding site thereof), antagonist, agonist; e.g., a human version of any of these. In an example, the inserted sequence is an exon.

Optionally, in any configuration, aspect, example or embodiment, the inserted nucleotide sequence replaces an orthologous or homologous sequence of the strand (e.g., the insert is a human sequence that replaces a plant, human or mouse sequence). For example, the method is carried out in a mouse or mouse cell (such as an ES cell) and the insert replaces an orthologous or homologous mouse sequence (e.g., a mouse biological target protein implicated in disease). For example, the method is carried out (e.g., *in* vitro) in a human cell and the insert replaces an orthologous or homologous human sequence (e.g., a human biological target protein implicated in disease, e.g., a mutated form of a sequence is replaced with a different (e.g., wild-type) human sequence, which may be useful for correcting a gene defect in the cell.

Optionally, in any configuration, aspect, example or embodiment, the inserted nucleotide sequence is at least 10 nucleotides long, e.g., at least 20, 30, 40, 50, 60, 70, 80, 90, 100, 150, 200, 300, 400, 500, 600, 700, 800 or 900 nucleotides, or at least 1, 2, 3, 5, 10, 20, 50 or 100kb long.

Optionally, in any configuration, aspect, example or embodiment, the insert sequence comprises a site specific recombination site, e.g., a lox, frt or rox site. For example, the site can be a loxP, lox511 or lox2272 site.

There is also provided:-
A method of nucleic acid recombination, the method comprising
(a) using nucleic acid cleavage to create first and second breaks in a nucleic acid strand, thereby creating 5' and 3' cut ends and a nucleotide sequence between the ends;
(b) using homologous recombination to delete the nucleotide sequence; and
(c) optionally obtaining the nucleic acid strand modified in step (b) or a progeny nucleic strand comprising the deletion.

In an example, the progeny strand is a product of the replication of the strand produced by step (b). The progeny strand is, for example, produced by nucleic acid replication in a cell. For example, steps (a) and (b) are carried out in a cell and the cell is replicated, wherein the machinery of the cell replicates the modified strand produced in step (b), e.g., to produce a dsDNA progeny in which each strand comprises the modification.

In an example, the single nucleic acid strand is a DNA or RNA strand.

In an example, the deleted sequence comprises a regulatory element or encodes all or part of a protein. In an embodiment, the deleted regulatory element is a promoter or enhancer.

Optionally, in any configuration, aspect, example or embodiment, the deleted nucleotide sequence is a plant, animal, vertebrate or mammalian sequence, e.g., a human sequence. For example, the sequence encodes a complete protein, polypeptide, peptide, domain or a plurality (e.g. one, two or more) of any one of these. In an example, the deleted sequence encodes an interleukin, receptor (e.g., a cell surface receptor), growth factor, hormone, antibody (or variable domain or binding site thereof), antagonist, agonist; e.g., a non-human version of any of these. In an example, the deleted sequence is an exon.

Optionally, in any configuration, aspect, example or embodiment, the deleted nucleotide sequence is replaced by an orthologous or homologous sequence of a different species or strain (e.g., a human sequence replaces an orthologous or homologous plant, human or mouse sequence). For example, the method is carried out in a mouse or mouse cell and the insert replaces an orthologous or homologous mouse sequence (e.g., a mouse biological target protein implicated in disease). For example, the method is carried out (e.g., *in* vitro) in a human cell and the insert replaces an orthologous or homologous human sequence (e.g., a human biological target protein implicated in disease, e.g., a mutated form of a sequence is replaced with a different (e.g., wild-type) human sequence, which may be useful for correcting a gene defect in the cell. In this embodiment, the cell may be a human ES or iPS or totipotent or pluripotent stem cell and may be subsequently introduced into a human patient in a method of gene therapy to treat and/or prevent a medical disease or condition in the patient).

Optionally, in any configuration, aspect, example or embodiment, the deleted nucleotide sequence is at least 10 nucleotides long, e.g., at least 20, 30, 40, 50, 60, 70, 80, 90, 100, 150, 200, 300, 400, 500, 600, 700, 800 or 900 nucleotides, or at least 1, 2, 3, 5, 10, 20, 50 or 100kb long.

Optionally, in any configuration, aspect, example or embodiment, step (c) is performed by isolating a cell comprising the modified first strand, or by obtaining a non-human vertebrate in which the method has been performed or a progeny thereof.

Optionally, in any configuration, aspect, example or embodiment, the product of the method comprises a nucleic acid strand comprising a PAM motif 3' of the insertion or deletion. In an example, the PAM motif is within 10, 9, 8, 7 6, 5, 4 or 3 nucleotides of the insertion or deletion. This is useful to enable serial insertions and/or deletions according to the method as explained further below.

Optionally, in any configuration, aspect, example or embodiment, the product of the method comprises a nucleic acid strand comprising a PAM motif 5' of the insertion or deletion. In an example, the PAM motif is within 10, 9, 8, 7 6, 5, 4 or 3 nucleotides of the insertion or deletion. This is useful to enable serial insertions and/or deletions according to the method as explained further below.

Optionally, in any configuration, aspect, example or embodiment, step (b) is performed by carrying out homologous recombination between an incoming nucleic acid comprising first and second homology arms, wherein the homology arms are substantially homologous respectively to a sequence extending 5' from the 5' end and a sequence extending 3' from the 3' end. The skilled person will be familiar with constructing vectors and DNA molecules for use in homologous recombination, including considerations such as homology arm size and sequence and the inclusion of selection markers between the arms. For example, the incoming nucleic acid comprises first and second homology arms, and the insert sequence and an optional selection marker sequence (e.g., neo nucleotide sequence). The arms may be at least 20, 30, 40, 50, 100 or 150 nucleotides in length, for example. Where deletion is required, the insert is omitted (although an optional selection marker sequence may or may not be included between the arms).

Thus, in an embodiment, step (b) is performed by carrying out homologous recombination between an incoming nucleic acid comprising an insert nucleotide sequence flanked by the first and second homology arms, wherein the insert nucleotide sequence is inserted between the 5' and 3' ends.

In another embodiment, the insert is between the homology arms and there is no further sequence between the arms.

In an example, each homology arm is at least 20, 30, 40, 50, 100 or 150 nucleotides long.

Optionally, in any configuration, aspect, example or embodiment, step (a) is carried out using an endonuclease, e.g., a nickase. Nickases cut in a single strand of dsDNA only. For example, the endonuclease is an endonuclease of a CRISPR/Cas system, e.g., a Cas9 or Cys4 endonuclease (e.g., a Cas9 or Cys4 nickase). In an example, the endonuclease recognises a PAM listed in Table 1 below, for example, the endonuclease is a Cas endonuclease that recognises a PAM selected from CCN, TCN, TTC, AWG, CC, NNAGNN, NGGNG GG, NGG, WGG, CWT, CTT and GAA. In an example, the Cas endonuclease is a *S pyogenes* endonuclease, e.g., a *S pyogenes* Cas9 endonuclease. In an example, a *S. pyogenes* PAM sequence or *Streptococcus thermophilus* LMD-9 PAM sequence is used.

In an example, the endonuclease is a Group 1 Cas endonuclease. In an example, the endonuclease is a Group 2 Cas endonuclease. In an example, the endonuclease is a Group 3 Cas endonuclease. In an example, the endonuclease is a Group 4 Cas endonuclease. In an example, the endonuclease is a Group 7 Cas endonuclease. In an example, the endonuclease is a Group 10 Cas endonuclease.

In an example, the endonuclease recognises a CRISPR/Cas Group 1 PAM. In an example, the endonuclease recognises a CRISPR/Cas Group 2 PAM. In an example, the endonuclease recognises a CRISPR/Cas Group 3 PAM. In an example, the endonuclease recognises a CRISPR/Cas Group 4 PAM. In an example, the endonuclease recognises a CRISPR/Cas Group 7 PAM. In an example, the endonuclease recognises a CRISPR/Cas Group 10 PAM.

In an example, Cas endonuclease-mediated cleavage is used in step (a); optionally by recognition of a GG or NGG PAM motif.

In an example, the first and/or second homology arm comprises a PAM motif. This is useful to enable serial insertions and/or deletions according to the method as explained further below.

An example of a suitable nickase is *Spyogenes* Cas9 D10A nickase (see Cong *et al.* and the Examples section below).

Optionally, in any configuration, aspect, example or embodiment, steps (a) and (b) of the method is carried out in a cell, e.g. a bacterial, yeast, eukaryotic cell, plant, animal, mammal, vertebrate, non-human animal, rodent, rat, mouse, rabbit, fish, bird or chicken cell. For example, the cell is an *E coli* cell or CHO or HEK293 or *Picchia* or *Saccharomyces* cell*.* In an example, the cell is a human cell *in vitro.* In one embodiment, the cell is an embryonic stem cell (ES cell, e.g., a human or non-human ES cell, such as a mouse ES cell) or an induced pluripotent stem cell (iPS cell; e.g., a human, rodent, rat or mouse iPS cell) or a pluripotent or totipotent cell. Optionally, the cell is not an embryonic cell, e.g. wherein the cell is not a human embryonic cell. Optionally, the cell is not a pluripotent or totipotent cell. In an example, the method is used to produce a human stem cell for human therapy (e.g., an iPS cell generated from a cell of a patient for reintroduction into the patient after the method has been performed on the cell), wherein the stem cell comprises a nucleotide sequence or gene sequence inserted by the method. The features of the examples in this paragraph can be combined.

In an example, the method is carried out in a mammalian cell. For example, the cell is a human cell in vitro or a non-human mammalian cell. For example, a non-human (e.g., rodent, rat or mouse) zygote. For example, a single-cell non-human zygote.

In an example, the method is carried out in a plant or non-human mammal, e.g. a rodent, mouse or rat or rabbit, or a tissue or organ thereof (e.g., *in vitro*).

In an example, the 3' or each cleavage site is flanked 3' by PAM motif (e.g., a motif disclosed herein, such as NGG or NGGNG sequence, wherein N is any base and G is a guanine). For example, one or more or all cleavage sites are flanked 3' by the sequence 5'-TGGTG-3'. Unlike dsDNA, the PAM is not absolutely required for ssDNA binding and cleavage: A single-stranded oligodeoxynucleotide containing a protospacer with or without a PAM sequence is bound nearly as well as dsDNA and may be used in the method wherein a single strand of DNA is modified. Moreover, in the presence of Mg²⁺ ions, Cas9 cuts ssDNA bound to the crRNA using its HNH active site independently of PAM.

Optionally, in any configuration, aspect, example or embodiment, step (a) is carried out by cleavage in one single strand of dsDNA or in ssDNA.

Optionally, in any configuration, aspect, example or embodiment, step (a) is carried out by combining in a cell the nucleic acid strand, a Cas endonuclease, a crRNA and a tracrRNA (e.g., provided by one or more gRNAs) for targeting the endonuclease to carry out the cleavage, and optionally an insert sequence for homologous recombination with the nucleic acid strand. Instead of an insert sequence, one can use an incoming sequence containing homology arms but no insert sequence, to effect deletion as described above. In an example, the Cas endonuclease is encoded by a nucleotide sequence that has been introduced into the cell. In an example, the gRNA is encoded by a DNA sequence that has been introduced into the cell.

In an example, the method is carried out in the presence of Mg²⁺.

Optionally, in any configuration, aspect, example or embodiment, step (b) is performed by carrying out homologous recombination with an incoming nucleic acid comprising first and second homology arms, wherein the homology arms are substantially homologous respectively to a sequence extending 5' from the 5' end and a sequence extending 3' from the 3' end, wherein the second homology arm comprises a PAM sequence such that homologous recombination between the second homology arm and the sequence extending 3' from the 3' end produces a sequence comprising a PAM motif in the product of the method. The PAM can be any PAM sequence disclosed herein, for example. Thus, the method produces a modified nucleic acid strand comprising a PAM that can be used for a subsequent nucleic acid modification according to any configuration, aspect, example or embodiment, wherein a Cas endonuclease is used to cut the nucleic acid. This is useful, for example, for performing sequential endonuclease-mediated homology directed recombination (sEHDR), more particularly sCHDR described below.

### Sequential Endonuclease-Mediated Homology Directed Recombination (sEHDR)

There is further provided:-
A method of sequential endonuclease-mediated homology directed recombination (sEHDR) comprising carrying out the method of any preceding configuration, aspect, example or embodiment a first time and a second time, wherein endonuclease-mediated cleavage is used in each step (a); wherein the product of the first time is used for endonuclease-mediated cleavage the second time, whereby either (i) first and second nucleotide sequences are deleted the first time and the second times respectively; (ii) a first nucleotide sequence is deleted the first time and a second nucleotide sequence is inserted the second time; (iii) a first nucleotide sequence is inserted the first time and a second nucleotide sequence is deleted the second time; or (iv) first and second nucleotide sequences are inserted the first and second times respectively; optionally wherein the nucleic acid strand modification the second time is within 20, 10, 5, 4, 3, 2 or 1 or less nucleotides of the nucleic acid strand modification the first time or directly adjacent to the nucleic acid strand modification the first time.

For example, the first and second nucleotide sequences are inserted so that they are contiguous after the insertion the second time. Alternatively, the first and second deletions are such that a contiguous sequence has been deleted after the first and second deletions have been performed.

In an embodiment of sEHDR, the method uses a Cas endonuclease. Thus, there is provided:-
A method of sequential Cas-mediated homology directed recombination (sCHDR) comprising carrying out the method of any preceding sentence a first time and a second time, wherein Cas endonuclease-mediated cleavage is used in each step (a); wherein step (b) of the first time is carried out performing homologous recombination with an incoming nucleic acid comprising first and second homology arms, wherein the homology arms are substantially homologous respectively to a sequence extending 5' from the 5' end and a sequence extending 3' from the 3' end, wherein the second homology arm comprises a PAM sequence such that homologous recombination between the second homology arm and the sequence extending 3' from the 3' end produces a sequence comprising a PAM motif in the product of the method; wherein the PAM motif of the product of the first time is used for Cas endonuclease-mediated cleavage the second time, whereby either (i) first and second nucleotide sequences are deleted the first time and the second times respectively; (ii) a first nucleotide sequence is deleted the first time and a second nucleotide sequence is inserted the second time; (iii) a first nucleotide sequence is inserted the first time and a second nucleotide sequence is deleted the second time; or (iv) first and second nucleotide sequences are inserted the first and second times respectively; optionally wherein the nucleic acid strand modification the second time is within 20, 10, 5, 4, 3, 2 or 1 or less nucleotides of the nucleic acid strand modification the first time or directly adjacent to the nucleic acid strand modification the first time.

For example, the first and second nucleotide sequences are inserted so that they are contiguous after the insertion the second time. Alternatively, the first and second deletions are such that a contiguous sequence has been deleted after the first and second deletions have been performed.

In an embodiment (First Embodiment), the first time is carried out according to the third configuration, wherein the incoming nucleic acid comprises no sequence between the first and second homology arms, wherein sequence between the 5' and 3' ends is deleted by homologous recombination; and/or the second time is carried out according to the third configuration, wherein step (b) is performed by carrying out homologous recombination between an incoming nucleic acid comprising first and second homology arms, wherein the homology arms are substantially homologous respectively to a sequence extending 5' from the 5' end and a sequence extending 3' from the 3' end, wherein the incoming nucleic acid comprises no sequence between the first and second homology arms such that sequence between the 5' and 3' ends is deleted by homologous recombination; optionally wherein the second arm comprises a PAM motif such that the product of the second time comprises a PAM motif for use in a subsequent Cas endonuclease-mediated method according to any configuration, aspect, example or embodiment.

In an embodiment (Second Embodiment), the first time is carried out according to the first or second configuration, wherein the incoming nucleic acid comprises the insert sequence between the first and second homology arms, wherein the insert sequence is inserted between the 5' and 3' ends by homologous recombination; and/or the second time is carried out according to the first or second configuration, wherein step (b) is performed by carrying out homologous recombination between an incoming nucleic acid comprising first and second homology arms, wherein the homology arms are substantially homologous respectively to a sequence extending 5' from the 5' end and a sequence extending 3' from the 3' end, wherein the insert sequence is inserted between the 5' and 3' ends by homologous recombination; optionally wherein the second arm comprises a PAM motif such that the product of the second time comprises a PAM motif for use in a subsequent Cas endonuclease-mediated method according to any configuration, aspect, example or embodiment.

In an example, one of said first and second times is carried out as specified in the First Embodiment and the other time is carried out as specified in the Second Embodiment, wherein at least one sequence deletion and at least one sequence insertion is performed.

Optionally, in any configuration, aspect, example or embodiment, step (a) is carried out by Cas endonuclease-mediated cleavage using a Cas endonuclease, one or more crRNAs and a tracrRNA. For example, the method is carried out in a cell and the crRNA and tracrRNA is introduced into the cell as RNA molecules. For example, the method is carried out in a zygote (e.g., a non-human zygote, e.g., a rodent, rat or mouse zygote) and the crRNA and tracrRNA is injected into zygote. In another embodiment, the crRNA and tracrRNA are encoded by DNA within a cell or organism and are transcribed inside the cell (e.g., an ES cell, e.g., a non-human ES cell, e.g., a rodent, rat or mouse ES cell) or organism to produce the crRNA and tracrRNA. The organism is, for example, a non-human animal or plant or bacterium or yeast or insect. In an embodiment, the tracrRNA is in this way encoded by DNA but one or more crRNAs are introduced as RNA nucleic acid into the cell or organism to effect the method.

Additionally or alternatively to these examples, the endonuclease may be introduced as a protein or a vector encoding the endonuclease may be introduced into the cell or organism to effect the method. In another example, the endonuclease is encoded by DNA that is genomically integrated into the cell or organism and is transcribed and translated inside the cell or organism.

In an example, the method is carried out in an ES cell (e.g., a non-human ES cell, e.g., a rodent, rat or mouse ES cell) that has been pre-engineered to comprise an expressible genomically-integrated Cas endonuclease sequence (or a vector carrying this has been include in the cell). It would be possible to introduce (or encode) a tracrRNA. By introducing a crRNA with a guiding oligo sequence to target the desired area of the cell genome, one can then carry out modifications in the cell genome. In an example, a gRNA as described herein is introduced into the ES cell. The genomically-integrated expressible Cas endonuclease sequence can, for example, be constitutively expressed or inducibly expressible. Alternatively or additionally, the sequence may be expressible in a tissue-specific manner in a progeny organism (e.g., a rodent) developed using the ES cell.

The initial ES cell comprising a genomically-integrated expressible Cas endonuclease sequence can be used, via standard techniques, to produce a progeny non-human animal that contains the expressible Cas endonuclease sequence. Thus, there is provided:-
A non-human animal (e.g., a vertebrate, mammal, fish or bird), animal cell, insect, insect cell, plant or plant cell comprising a genomically-integrated expressible Cas endonuclease nucleotide sequence and optionally a tracrRNA and/or a nucleotide sequence encoding a tracrRNA. The Cas endonuclease is, for example, Cas9 or Cys4. In an example, the animal, insect or plant genome comprises a chromosomal DNA sequence flanked by site-specific recombination sites and/or transposon elements (e.g., piggyBac transposon repeat elements), wherein the sequence encodes the endonuclease and optionally one or more gRNAs. As described in the Examples below, recombinase-mediated cassette exchange (RMCE) can be used to insert such a sequence. The transposon elements can be used to excise the sequence from the genome once the endonuclease has been used to perform recombination. The RMCE and/or transposon-mediated excision can be performed in a cell (e.g., an ES cell) that later is used to derive a progeny animal or plant comprising the desired genomic modification.

There is also provided an ES cell derived or derivable from such an animal, wherein the ES cell comprises a genomically-integrated expressible Cas endonuclease nucleotide sequence. In an example, the ES cell is a rodent, e.g., a mouse or rat ES cell, or is a rabbit, dog, pig, cat, cow, non-human primate, fish, amphibian or bird ES cell.

There is also provided a method of isolating an ES cell, the method comprising deriving an ES cell from an animal (e.g., a non-human animal, e.g., a rodent, e.g., a rat or a mouse), wherein the animal comprises a genomically-integrated expressible Cas endonuclease nucleotide sequence, as described herein.

In any of these aspects, instead of an ES cell, the cell may be an iPS cell or a totipotent or pluripotent cell. Thus, an iPS or stem cell can be derived from (e.g., a somatic cell of) a human, engineered *in vitro* to comprise a genomically-integrated expressible Cas endonuclease nucleotide sequence and optionally one or more DNA sequences encoding a tracrRNA or gRNA. The disclosure, thus, also relates to such a method and to a human iPS or stem cell comprising a genomically-integrated expressible Cas endonuclease nucleotide sequence and optionally one or more DNA sequences encoding a tracrRNA or gRNA. This cell can be used in a method of the disclosure to carry out genome modification (e.g., to correct a genetic defect, e.g., by replacement of defective sequence with a desired sequence, optionally with subsequent transposon-mediated excision of the endonuclease-encoding sequence). After optional excision of the Cas endonuclease sequence, the iPS cell or stem cell can be introduced into the donor human (or a different human, e.g., a genetic relative thereof) to carry out genetic therapy or prophylaxis. In the alternative, a totipotent or pluripotent human cell is used and then subsequently developed into human tissue or an organ or part thereof. This is useful for providing material for human therapy or prophylaxis or for producing assay materials (e.g., for implantation into model non-human animals) or for use in *in vitro* testing (e.g., of drugs).

In an example, the method uses a single guided RNA (gRNA or sgRNA) comprising a crRNA and a tracrRNA. The crRNA comprises an oligonucleotide sequence ("X" in the structure 5'-X-Y-3' mentioned below) that is chosen to target a desired part of the nucleic acid or genome to be modified. The skilled person will be able readily to select appropriate oligo sequence(s). In an example, the sequence is from 3 to 100 nucleotides long, e.g., from 3 to 50, 40, 30, 25, 20, 15 or 10 nucleotides long, e.g., from or 5, 10, 15 or 20 to 100 nucleotides long, e.g., from 5, 10, 15 or 20 to 50 nucleotides long.

For example, the gRNA is a single nucleic acid comprising both the crRNA and the tracrRNA. An example of a gRNA comprises the sequence 5'-[oligo]-[UUUUAGAGCUA (S N1UUUUAN2N3GCUA)]-[LINKER]-[UAGCAAGUUAAAA (SEQ ID NO:2)]-3', wherein the LINKER comprises a plurality (e.g., 4 or more, e.g., 4, 5 or 6) nucleotides (e.g., 5'-GAAA-3').

For example, the crRNA has the structure 5'-X-Y-3', wherein X is an RNA nucleotide sequence (optionally, at least 5 nucleotides long) and Y is a crRNA sequence comprising a nucleotide motif that hybridises with a motif comprised by the tracrRNA, wherein X is capable of hybridising with a nucleotide sequence 5' of the desired site of the 5' cut end, e.g., extending 5' from the desired site of the 5' cut.

In an example, Y is 5'-N1UUUUAN2N3GCUA-3' (SEQ ID NO:3), wherein each of N1-3 is a A, U, C or G and/or the tracrRNA comprises the sequence (in 5' to 3' orientation) UAGCM1UUAAAAM2 (SEQ ID NO:4), wherein M1 is spacer nucleotide sequence and M2 is a nucleotide; e.g., N1-G, N2=G and N3=A. The spacer sequence is, e.g., 5, 4, 3, 2 or 1 RNA nucleotides in length (e.g., AAG in 5' to 3' orientation). M2 is, for example, an A, U, C or G (e.g., M2 is a G). In an embodiment, a chimaeric gRNA is used which comprises a sequence 5'-X-Y-Z-3', wherein X and Y are as defined above and Z is a tracrRNA comprising the sequence (in 5' to 3' orientation) UAGCM1UUAAAAM2 (SEQ ID NO:4), wherein M1 is spacer nucleotide sequence and M2 is a nucleotide. In an example, Z comprises the sequence 5'- UAGCAAGUUAAAA-3' (SEQ ID NO:2), e.g., Z is 5'- UAGCAAGUUAAAAUAAGGCUAGUCCG-3' (SEQ ID NO:5). In an example, the gRNA has the sequence:

When it is desired to use the present method to insert an exogenous sequence into the nucleic acid to be modified, the exogenous sequence can be provided on linear or circular nucleic acid (e.g., DNA). Typically, the exogenous sequence is flanked by homology arms that can undergo homologous recombination with sequences 5' and 3' respectively of the site where the exogenous sequence is to be inserted. The skilled person is familiar with choosing homology arms for homologous recombination.

The methods can be used in a method of producing a transgenic organism, e.g., any organism recited herein. For example, the organism can be a non-human organism used as an assay model to test a pharmaceutical drug or to express an exogenous protein or a part thereof (e.g., a human protein target knocked-in into a non-human animal assay organism). In another example, the methods have been used to knock-out an endogenous sequence (e.g., a target protein) in an organism, such as a non-human organism. This can be useful to assess the effect (phenotype) of the knock-out and thus to assess potential drug targets or proteins implicated in disease. In one example, the organism is a non-human animal (e.g., a vertebrate, mammal, bird, fish, rodent, mouse, rat or rabbit) in which a human target protein has been knocked-in using the methods described herein. Optionally, the methods have been used to knock out an orthologous or homologous endogenous target of the organism (e.g., an endogenous target sequence has been replaced at the endogenous position by an orthologous or homologous human target sequence). In this way, an assay model can be produced for testing pharmaceutical drugs that act via the human target.

In an embodiment, the organism is a non-human vertebrate that expresses human antibody variable regions whose genome comprises a replacement of an endogenous target with an orthologous or homologous human sequence. In an example, the method is used to produce an Antibody-Generating Vertebrate or Assay Vertebrate as disclosed in WO2013061078.

In an example, an exogenous regulatory element is knocked-in using the method. For example, it is knocked-in to replace an endogenous regulatory element.

In one aspect, there is provided a method of producing a cell or a transgenic non-human organism (e.g., any non-human organism recited herein), the method comprising:
(a) carrying out the method of any in any configuration, aspect, example or embodiment to (i) knock out a target nucleotide sequence in the genome of a first cell and/or (ii) knock in an insert nucleotide sequence into the genome of a first cell, optionally wherein the insert sequence replaces a target sequence in whole or in part at the endogenous location of the target sequence in the genome; wherein the cell or a progeny thereof can develop into a non-human organism or cell; and
(b) developing the cell or progeny into a non-human organism or a non-human cell.

In an example, the organism or cell is homozygous for the modification (i) and/or (ii).

In an example, the cell is an ES cell (such as a mouse ES cell), iPS cell, totipotent cell or pluripotent cell. In an example, the cell is a non-human vertebrate cell or a human cell *in vitro.* In an example, the cell is a plant, yeast, insect or bacterial cell.

In an example, the cell or organism is a rodent (e.g., a mouse or rat) cell or a rabbit, bird, fish, chicken, non-human primate, monkey, pig, dog, Camelid, shark, sheep, cow or cat cell.

In an example, the target sequence is an endogenous sequence comprising all or part of a regulatory element or encoding all or part of a protein.

In an example, the insert sequence is a synthetic sequence; or comprises a sequence encoding all or part of a protein from a species other than the species from which the first cell is derived; or comprises a regulatory element from said first species. This is useful to combine genes with new regulatory elements.

In an example, the insert sequence encodes all or part of a human protein or a human protein subunit or domain. For example, the insert sequence encodes a cell membrane protein, secreted protein, intracellular protein, cytokine, receptor protein (e.g., Fc receptor protein, such as FcRn or a FcY receptor protein), protein of the human immune system or domain thereof (e.g., an Ig protein or domain, such as an antibody or TCR protein or domain, or a MHC protein), a hormone or growth factor.

There is also provided:-
A cell (e.g., an isolated or purified cell, e.g., a cell *in vitro,* or any cell disclosed herein) or a non-human organism (e.g., any organism disclosed herein, such as a mouse) whose genome comprises a modification comprising a non-endogenous nucleotide sequence flanked by endogenous nucleotide sequences, wherein the cell or organism is obtainable by the method of any configuration, aspect, example or embodiment, and wherein the non-endogenous sequence is flanked 3' and/or 5' by (e.g., within 20, 10, 5, 4, 3, 2 or 1 or less nucleotides of, or directly adjacent to) a Cas PAM motif; wherein the cell is not comprised by a human; and one, more or all of (a) to (d) applies (for example, (a); (b); (c); (d); (a) and (b); (a) and (c); (a) and (d); (b) and (c); (b) and (d); (c) and (d); (a), (b) and (c); (a), (b) and (d); (a), (c) and (d); (b), (c) and (d) or all of (a), (b), (c) and (d)).
(a) the genome is homozygous for the modification; or comprises the modification at one allele and is unmodified by Cas-mediated homologous recombination at the other allele;
(b) the non-endogenous sequence comprises all or part of a regulatory element or encodes all or part of a protein;
(c) the non-endogenous sequence is at least 20, 30, 40, 50, 60, 70, 80, 90, 100, 150, 200, 300, 400, 500, 600, 700, 800 or 900 nucleotides, or at least 1, 2, 3, 5, 10, 20, 50 or 100kb long;
(d) the non-endogenous sequence replaces an orthologous or homologous sequence in the genome.

The cell can be a human cell, or included in human tissue but not part of a human being. For example, the cell is a human cell *in vitro.*

In an example, the non-endogenous sequence is a human sequence.

In an example, the PAM motif is any PAM disclosed herein or comprises a sequence selected from CCN, TCN, TTC, AWG, CC, NNAGNN, NGGNG GG, NGG, WGG, CWT, CTT and GAA. For example, the motif is a Cas9 PAM motif. For example, the PAM is NGG. In another example, the PAM is GG.

In an example, there is a PAM motif no more than 10 nucleotides (e.g., 3 nucleotides) 3' and/or 5' of the non-endogenous sequence.

In an example, the PAM motif is recognised by a *Streptococcus*Cas9.

In an example, the cell or organism is a non-human vertebrate cell or a non-human vertebrate that expresses one or more human antibody heavy chain variable domains (and optionally no heavy chain variable domains of a non-human vertebrate species). For example, the organism is an Antibody-Generating Vertebrate or Assay Vertebrate disclosed in WO2013061078.

In an example, the cell or organism is a non-human vertebrate cell or a non-human vertebrate that expresses one or more human antibody kappa light chain variable domains (and optionally no kappa light chain variable domains of a non-human vertebrate species).

In an example, the cell or organism is a non-human vertebrate cell or a non-human vertebrate that expresses one or more human antibody lambda light chain variable domains (and optionally no kappa light chain variable domains of a non-human vertebrate species).

In an example, the non-endogenous sequence encodes a human Fc receptor protein or subunit or domain thereof (e.g., a human FcRn or FcY receptor protein, subunit or domain).

In an example, the non-endogenous sequence comprises one or more human antibody gene segments, an antibody variable region or an antibody constant region.

In an example, the insert sequence is a human sequence that replaces or supplements an orthologous non-human sequence.

There is also provided:-
A monoclonal or polyclonal antibody prepared by immunisation of a vertebrate (e.g., mouse or rat) described herein (or produced by a method described herein) with an antigen.

There is also provided:-
A method of isolating an antibody that binds a predetermined antigen, the method comprising:
(a) providing a vertebrate (optionally a mouse or rat) described herein (or produced by a method described herein);
(b) immunising said vertebrate with said antigen;
(c) removing B lymphocytes from the vertebrate and selecting one or more B lymphocytes expressing antibodies that bind to the antigen;
(d) optionally immortalising said selected B lymphocytes or progeny thereof, optionally by producing hybridomas therefrom; and
(e) isolating an antibody (e.g., an IgG-type antibody) expressed by the B lymphocytes.

In an example, the method comprises the step of isolating from said B lymphocytes nucleic acid encoding said antibody that binds said antigen; optionally exchanging the heavy chain constant region nucleotide sequence of the antibody with a nucleotide sequence encoding a human or humanised heavy chain constant region and optionally affinity maturing the variable region of said antibody; and optionally inserting said nucleic acid into an expression vector and optionally a host.

In an example, the method comprises making a mutant or derivative of the antibody produced by the method.

There is provided the use of an isolated, monoclonal or polyclonal antibody described herein, or a mutant or derivative antibody thereof that binds said antigen, in the manufacture of a composition for use as a medicament.

There is provided the use of an isolated, monoclonal or polyclonal antibody described herein, or a mutant or derivative antibody thereof that binds said antigen for use in medicine.

There is provided a method of treating a patient in need thereof (e.g., a human patient), comprising administering a therapeutically effective amount of an isolated, monoclonal or polyclonal antibody described herein, or a mutant or derivative antibody thereof which binds an antigen.

There is provided a nucleotide sequence encoding an antibody described herein, optionally wherein the nucleotide sequence is part of a vector. There is also provided a host cell comprising said nucleotide sequence.

There isprovided a pharmaceutical composition comprising the antibody or antibodies described herein and a diluent, excipient or carrier.

There is provided an ES cell, a non-human animal or a non-human blastocyst comprising an expressible genomically-integrated nucleotide sequence encoding a Cas endonuclease (e.g., a Cas9 or Cys4) and optionally an expressible genomically-integrated nucleotide sequence encoding a tracrRNA or a gRNA. For example, the ES cell is any ES cell type described herein.

In an example of the cell, animal or blastocyst, the endonuclease sequence is constitutively expressible.

In an example of the cell, animal or blastocyst, the endonuclease sequence is inducibly expressible.

In an example of the cell, animal or blastocyst, the endonuclease sequence is expressible in a tissue-specific manner in the animal or a progeny thereof, or in a non-human animal that is a progeny of the cell or blastocyst.

In an example, the cell, animal or blastocyst comprises one or more gRNAs or an expressible nucleotide sequence encoding a gRNA or a plurality of expressible nucleotide sequences each encoding a different gRNA.

There is provided the use of the cell, animal or blastocyst in a method according to any configuration, aspect, embodiment or example.

An aspect provides an antibody produced by the method of the disclosure, optionally for use in medicine, e.g., for treating and/or preventing (such as in a method of treating and/or preventing) a medical condition or disease in a patient, e.g., a human.

An aspect provides a nucleotide sequence encoding the antibody of the disclosure, optionally wherein the nucleotide sequence is part of a vector. Suitable vectors will be readily apparent to the skilled person, e.g., a conventional antibody expression vector comprising the nucleotide sequence together in operable linkage with one or more expression control elements.

An aspect provides a pharmaceutical composition comprising the antibody of the disclosure and a diluent, excipient or carrier, optionally wherein the composition is contained in an intravenous (IV) container (e.g., and IV bag) or a container connected to an IV syringe.

An aspect provides the use of the antibody of the disclosure in the manufacture of a medicament for the treatment and/or prophylaxis of a disease or condition in a patient, e.g. a human.

In a further aspect, the disclosure relates to humanised antibodies and antibody chains produced according to the present disclosure, both in chimaeric and fully humanised form, and use of said antibodies in medicine. The disclosure also relates to a pharmaceutical composition comprising such an antibody and a pharmaceutically acceptable carrier or other excipient.

Antibody chains containing human sequences, such as chimaeric human-non human antibody chains, are considered humanised herein by virtue of the presence of the human protein coding regions region. Fully human antibodies may be produced starting from DNA encoding a chimaeric antibody chain of the disclosure using standard techniques.

Methods for the generation of both monoclonal and polyclonal antibodies are well known in the art, and the present disclosure relates to both polyclonal and monoclonal antibodies of chimaeric or fully humanised antibodies produced in response to antigen challenge in non-human vertebrates of the present disclosure.

In a yet further aspect, chimaeric antibodies or antibody chains generated in the present disclosure may be manipulated, suitably at the DNA level, to generate molecules with antibody-like properties or structure, such as a human variable region from a heavy or light chain absent a constant region, for example a domain antibody; or a human variable region with any constant region from either heavy or light chain from the same or different species; or a human variable region with a non-naturally occurring constant region; or human variable region together with any other fusion partner. The disclosure relates to all such chimaeric antibody derivatives derived from chimaeric antibodies identified according to the present disclosure

In a further aspect, the disclosure relates to use of animals described hereinin the analysis of the likely effects of drugs and vaccines in the context of a quasi-human antibody repertoire.

The disclosure also relates to a method for identification or validation of a drug or vaccine, the method comprising delivering the vaccine or drug to a mammal described herein and monitoring one or more of: the immune response, the safety profile; the effect on disease.

The disclosure also relates to a kit comprising an antibody or antibody derivative as disclosed herein and either instructions for use of such antibody or a suitable laboratory reagent, such as a buffer, antibody detection reagent.

It will be understood that particular embodiments described herein are shown by way of illustration and not as limitations of the invention. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or openended and do not exclude additional, unrecited elements or method steps

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

Any part of this disclosure may be read in combination with any other part of the disclosure, unless otherwise apparent from the context.

All of the compositions and/or methods disclosed and claimed herein can be made and executed without undue experimentation in light of the present disclosure.

### References

1. Cong L, Ran FA, Cox D, Lin S, Barretto R, Habib N, Hsu PD, Wu X, Jiang W, Marraffini LA et al: Multiplex genome engineering using CRISPR/Cas systems. Science 2013, 339(6121):819-823.
2. Wang H, Yang H, Shivalila CS, Dawlaty MM, Cheng AW, Zhang F, Jaenisch R: One-step generation of mice carrying mutations in multiple genes by CRISPR/Cas-mediated genome engineering. Cell 2013, 153(4):910-918.
3. Mali P, Yang L, Esvelt KM, Aach J, Guell M, DiCarlo JE, Norville JE, Church GM: RNA-guided human genome engineering via Cas9. Science 2013, 339(6121):823-826.
4. Gaj T, Gersbach CA, Barbas CF, 3rd: ZFN, TALEN, and CRISPR/Cas-based methods for genome engineering. Trends Biotechnol 2013, 31(7):397-405.
5. Perez-Pinera P, Ousterout DG, Gersbach CA: Advances in targeted genome editing. Curr Opin Chem Biol 2012, 16(3-4):268-277.
6. Shah SA, Erdmann S, Mojica FJ, Garrett RA: Protospacer recognition motifs: Mixed identities and functional diversity. RNA Biol 2013, 10(5).
7. Haurwitz RE, Sternberg SH, Doudna JA: Csy4 relies on an unusual catalytic dyad to position and cleave CRISPR RNA. EMBO J 2012, 31(12):2824-2832.
8. Yusa K, Zhou L, Li MA, Bradley A, Craig NL: A hyperactive piggyBac transposase for mammalian applications. Proc Natl Acad Sci U S A 2011, 108(4): 1531-1536.
9. Qiao J, Oumard A, Wegloehner W, Bode J: Novel tag-and-exchange (RMCE) strategies generate master cell clones with predictable and stable transgene expression properties. J Mol Biol 2009, 390(4):579-594.
10. Oumard A, Qiao J, Jostock T, Li J, Bode J: Recommended Method for Chromosome Exploitation: RMCE-based Cassette-exchange Systems in Animal Cell Biotechnology. Cytotechnology 2006, 50(1-3):93-108.

### EXAMPLES

### Example 1: Precise DNA Modifications

### (a) Use of Nickase for HDR

It has been reported that the Cas9 nuclease can be converted into a nickase through the substitution of an aspartate to alanine (D10A) in the RuvCI domain of SpCas9 (Cong *et al*.). It is noteworthy that DNA single-stranded breaks are preferentially repaired through the HDR pathway. The Cas9 D10A nickase, when in a complex with mature crRNA:tracrRNA, can specifically induce DNA nicking at a precise location. With this in mind, we propose extending the application of the CRISPR/Cas system by creating a nick in a given location in a genome using Cas9 D10A nickase and then exploiting the HDR pathway for inserting a single-stranded DNA fragment (endogenous or exogenous) which will contain DNA homology (typically for recombineering, 50 bp is enough for efficient recombination) flanking the nicked DNA junction to bring in and insert a given DNA in a precision location; similar size homology will be used with the present example (Figure 1A). Guide RNA (gRNA) will be design individually per target protospacer sequence or incorporated into a single CRISPR array encoding for 2 or more spacer sequences allowing multiplex genome editing from a single CRSPR array.

### (b) Example of Precise DNA Deletion

To demonstrate precise deletion using Cas9 in association with gRNA and no targeting vector or donor DNA, we designed two gRNA within a gene, which were 55 bp apart. The two gRNA were on opposite DNA strands as shown in Figure 9.

Mouse ES cells were transfected with human Cas9 nuclease and the two gRNAs. The transfection procedure was carried out as detailed above but the resulting clones were not selected. The transfected ES clones were genotyped using oligos pair spanning the two gRNA (Primer 1 & 2) to detect specific 55 bp deletion (Figure 10).

Most of the clones did not show the specific 55 bp deletion, however, clones were clearly identified which contained the defined deletion. Out of the 384 clones analysed, approximately 4% of the clones were found to contain the specific 55 bp deletion. Note: Not all the genotyping data is shown. The clones containing the specific 55 bp deletion were further analysed by sequencing the PCR products as a final confirmation (data not shown). Furthermore, where we saw the specific deletion, we observed both alleles to contain the specific deletion. These data confirmed that when two gRNAs are used, a precise and specific deletion can be made without the requirement for a targeting vector. However we can assume the efficiency of the define deletion can be greatly enhance using the two gRNA combination together with a targeting vector or a donor DNA fragment containing homology arms flanking the intended deletion region.

### (c) Alternative methodology for deletion of DNA

In a separate setting, two gRNA or a single CRISPR array encoding multiple spacer sequence can be designed flanking a gene or a region of interest and with the association of Cas9 D10A nickase, two separate single-stranded breaks can be induced. This, in association with a single-stranded DNA fragment containing DNA homology to the 5' breakpoint junction of the first DNA nick, and DNA homology to the 3' breakpoint junction of the second nick, the region in between the two single stranded DNA nick can be precisely deleted (Figure 2A).

### (d) Alternative methodology for replacement of DNA

In an another setting, two separate gRNA or a multiplex single CRISPR array can be designed flanking a gene or a region of interest and with the association of Cas9 D10A nickase two separate single-stranded breaks can be induced. In this case the intruding single stranded DNA fragment (or double stranded DNA) can contain DNA sequence from either endogenous or exogenous source containing sequence for a known gene, regulatory element promoter etc. This single-stranded DNA fragment (or double stranded DNA) can be brought together to replace the DNA region of interest flanked by DNA nick by arming it with DNA homology from the 5' region of the first nick and 3' region from the second nick (Figure 3A). Due to the high efficiency of the CRISPR/Cas system to cleave DNA, the above proposed strategy will not require introduction of any selection marker, thus creating exact seamless genome editing in a precise and defined manner. As an option, a selection marker can be included flanked by PiggyBac LTRs to allow for the direct selection of correctly modified clones. Once the correct clones have been identified, the selection marker can be removed conveniently through the expression of hyperactive piggyBac transposase (Yusa K., Zhou L., Li M.A., Bradley A., Craig N.L.: A hyperactive piggyBac transposase for mammalian applications., Proc. Natl. Acad. Sci. USA, 2011, 108(4):1531-1536). Furthermore, the above approaches can be applied to ES cells, mammalian cells, yeast cells, bacterial cells, plant cells as well as directly performing in zygotes to expedite the process of homozygeous genome engineering in record time. It would be also possible to multiplex this system to generate multiple simultaneous DNA insertions (KI), deletions (KO) and the sequential deletion and insertion (KO → KI).

### (e) Example of DNA Deletion and Insertion in a predefined location (KO → KI)

To demonstrate a desired DNA region can be manipulated using Cas9, a single guide RNA (gRNA) was selected at a desired region (Exon 1 of gene *X*) Figure 7. A targeting vector was also constructed, which contained approximately 300 bp homology arms (5' and 3' HA) flanking the gRNA. The homology arms will hybridise exactly in the defined region and thus delete a 50 bp region, which is intended for deletion. The targeting vector also allows for the insertion of any DNA sequence of interest. In this proof of concept experiment, we included an approximate 1.6 kb PGK-puromycin cassette. The guide RNA (0.5 ug) together with the targeting vector (1 ug) and Cas9 nuclease vector (1 ug) was transfected into ES cells and 96 clones were picked after selection on puromycin using the protocol described above. Note. As a test for targeting efficiency, we compared linear verses circular targeting vector. Also as a negative control, we did the same experiment using no Cas9 vector to compare targeting efficiency via homologous recombination with and without Cas9 expression.

All the selected clones were puromycin resistant and the 96 clones picked from each of the four transfections were genotyped using the oligo pair HAP341/HAP334. Correctly targeted clones yielded an 880 bp PCR product. The resulting genotyping data is shown in Figure 8.

From the genotyping data of this experiment, it can be seen that Cas9 mediated double stranded DNA break greatly improves homologous recombination efficiency of the targeting vector as 62% and 49% of the clones using circular or linear targeting vector respectively were correctly targeted verse only a single targeted clone using circular targeting vector when no Cas9 was used. Also it can be seen from this data that the circular targeting vector yielded slightly better targeting efficiency than when linear vector was used but a general conclusion cannot be drawn from this single experiment but to say, both circular and linear targeting vector yielded greatly improved targeting efficiency when associated with Cas9 and a specific guide RNA. This experiment also demonstrated that using Cas9 to create a define DNA breakage can be used to delete out a defined DNA region and subsequently insert any DNA fragment of interest

### Reference Example 2: Recycling PAM for Sequential Insertions or Deletions

In certain settings it may be useful to edit a genome by chromosome walking. Using any of the three examples outlined above, it could be possible to carry out sequential genome editing in a stepwise fashion whereby the PAM sequence used in a previous round of CRISPR/Cas mediated genome editing, can be re-used to carry out multiple rounds of genome editing such as deletions, insertions or the simultaneous deletion and insertion. An example of sequential deletion whereby the PAM sequence from the previous genome editing step is recycled is shown in Figure 4A. Using the PAM recycling approach, it is possible to carry out sequential insertions as well as sequential simultaneous deletion and insertion.

The PAM sequence us recycled through reintroducing it via homologous recombination and as part of the homology arm. The PAM sequence can be optionally accompanied by a unique guide-RNA sequence creating a novel site within the host genome for further round of genome editing

### Reference Example 3: Rapid Insertion of Lox Sites Using CRISPR/Cas System

Targeting efficiency using conventional homologous recombination methods in ES cells is low. In a different setting, the CRISPR/Cas system can be used to rapidly and efficiently introduce lox sites or other recombinase recognition sequence such as Frt in a defined location to act as a landing pad for genome editing using recombinase mediated cassette exchange (RMCE) (Qiao J., Oumard A., Wegloehner W., Bode J.: Novel tag-and-exchange (RMCE) strategies generate master cell clones with predictable and stable transgene expression properties., J. Mol. Biol., 2009, 390(4):579-594; and Oumard A., Qiao J., Jostock T., Li J., Bode J.: Recommended Method for Chromosome Exploitation: RMCE-based Cassette-exchange Systems in Animal Cell Biotechnology., Cytotechnology, 2006, 50(1-3):93-108). Once the lox sites are introduced into the genome, inversion, deletion or cassette exchange to delete and introduce DNA fragment varying in size at this site can be efficiently conducted via expression of Cre recombinase. An example of CRISPR/Cas mediated lox insertion followed by RMCE is shown in Figure 5A. The RMCE step can be used to invert the region flanked by lox site or to delete this region as well as to simultaneously delete and insert DNA of interest in this region. Furthermore, the RMCE step can be adapted for carrying out multiple sequential rounds of RMCE (sRMCE).

### Reference Example 4A:

Reference is made to Figure 6A. A piggyBac transposon harbouring a PGK promoter-driven loxP/mutant lox-flanked *neo*^{R} gene is targeted into an ES cell genome by standard homologous recombination. The targeted clones can be selected by G418. This provides a landing pad for the following recombinase-mediated cassette exchange (RMCE). Such an ES clone can be used a parental cells for any modification further. A cassette containing the loxP/mutant lox-flanked promoterless *Puro*Δ *TK*-T2A-*Cas9* and U6 polymerase III promoter-driven guide RNA (gRNA) genes are inserted into the landing pad through transient *cre* expression. The gRNA genes can be one or more than one which target to the same gene or different genes. The inserted clones can be selected with puromycin and confirmed by junction PCRs. During the selection, the expression of Cas9 and gRNAs from the inserted cassette results in more efficient gene targeting or modification than transient expression of the Cas9 and gRNA can achieve. Following 4-6 day selection, the whole modified cassette is excised by the transient expression of *piggyBac transposase (PBase).* The final ES cell clones would not contain any *Cas9* or gRNA sequence. The clones with homozygous modified genes would be confirmed by PCR and sequence.

The main feature of thismethod is to control the *Cas9and* gRNA expression in certain time to be sufficient to generate efficient targeting rates.

### Reference Example 4B: Single Copy Cas9 expression

As detailed in example 6, to demonstrate the single and stable expression of Cas9 from within the chromosome of a cell, we targeted a landing pad vector into Rosa26 allele on chromosome 6. DNA homology arms were used to target the landing pad vector in between exons 2 and 3 of Rosa26. The landing pad vector was targeted into ES cells using procedure described above. The transfected ES clones were selected on G418 and genotyped for correct targeting (Figure 11) by PCR amplifying the 5' and 3' homology arm junctions.

Targeting of the landing pad yielded many targeted ES clones. A selection of the targeted clones were used to insert a DNA cassette containing Cas9 nuclease linked to Puro-delta-tk via a T2A sequence into the targeted landing pad via RMCE, which involved the expression of Cre recombinase. The corresponding loxP and lo2272 sites within both the landing pad and the incoming vector ensured correct orientation of insertion. Since the landing pad contained a geneless PGK promoter, correct insertion of the incoming vector DNA containing Cas9, activated expression of puromycin and thus clones were positively selected on puromycin. Non-specific targeting of this DNA cassette will not yield puromycin resistant clones due to the absence of a promoter driving the transcription of the promoterless puromycin gene in the inserted DNA cassette. The initial Cas9 vector inserted into the landing pad did not contain any guide RNA sequence. The puromycin resistant ES clones were genotyped by PCR for the correct insertion of Cas9 (Figure 12).

As expected owing to the positive selection, most of the clones genotyped for insertion of the Cas9 vector were correctly targeted via RMCE based on the PCR genotyping results. Two of the correct clones (KHK1.6 Z2-24-27 and KHK1.10Z2-25-4 referred to as positive Z clones) which now contain the single copy Cas9 integrated into the Rosa26 gene as a single copy were used to test whether the Cas9 expression was sufficient enough to induce Cas9 mediated genome editing. Into the two positive Z clones, guide RNA against a gene referred to as gene Y was transfected using procedure described above. Following transfection and expansion of the resulting ES clones, 36 individual clones were isolated from each transfection and analysed initially by PCR using oligo flanking the guide RNA (Figure 13).

Most of the clones yielded a PCR product of size equivalent to the positive control PCR where DNA from mouse AB2.1 ES cells was used. However, it can be seen clearly that some clones yielded a PCR product distinctively smaller than that of the positive control suggesting these clones contain a significant deletion via indel. To verify this and to check whether the rest of the PCR products though similar in size to the positive control did not contain indels, all the PCR products were purified using Qiagen gel extraction kit and analysed by sequencing. The sequencing data confirmed significant deletion for those PCR products that yielded shorter products than the positive control. It also highlighted, some of the other clones with similar PCR product size to the positive control to contain indels, which included various combinations of insertion and deletion (Sequencing data not shown). Out of the clones analysed, 18% of them contained an indel. These data clearly demonstrated that a single copy expression of Cas9 can be used to carry out genome editing and these clones can now be used as a Cas9 host cells for carrying out a multitude of genome editing. These ES clones are now being used to generate transgenic mouse lines whereby we can carry out a one-step genome editing by injecting only guide mRNA directly into zygotes without the requirement for transcribing Cas9 mRNA to simplify the one-step genome editing protocol.

### Reference Example 5(A): Methodology

### A: Reconstructing CRISPR/Cas Vector System (Nuclease)

The CRISPR/Cas genome editing system has been reconstructed *in vitro* and exemplified in mouse embryonic stem cells using vector pX330 containing humanised *S. pyogenes* (hSpCsn1) (Cong *et al*.). The CRISPR/Cas system can be reconstructed as described in Cong *et al* using synthetic DNA strings and DNA assembly. In the present example, the entire DNA assembly would constitute a 6006 bp fragment containing 45 bp homology to pBlueScript KS+ vector 5' to the EcoRV cutting site, Human U6 promoter, two BbsI restriction sites for cloning in the spacer sequence which fuses to a chimeric guided RNA sequence, chicken beta-actin promoter with 3 FLAG, nuclear localisation signal (NLS) followed by hSpCsn1 sequence and another NLS, bGH polyA, inverted terminal repeat sequence and finally another 45 bp homology to pBlueScript KS+ 3' to the EcoRV cutting site. This 6006 bp stretch of DNA will be synthetized as 7 individual DNA fragments where each fragment will have a 45 bp overlap to the adjacent DNA fragment to allow DNA assembly. The DNA sequence of these fragments is shown below in the order of assembly.

### Fragment 1A (1340 bp)

### Fragment 2 (852 bp)

### Fragment 3 (920 bp)

### Fragment 4 (920 bp)

### Fragment 5 (920 bp)

### Fragment 6 (789 bp)

### Fragment 7 (535 bp)

To reconstruct the CRISPR/Cas system described in Cong *et al* the above DNA fragments in addition to EcoRV linearised pBlueScript KS+ vector will be assembled using Gibson Assembly kit (NEB Cat No. E5510S). As an alternative approach, the 6006 bp fragment can be assembled by assembly PCR by mixing molar ratio of the individual DNA fragments together and using the DNA mixture as PCR template. The assembled PCR product can then be cloned directly into pBlueScript vector or a standard cloning vector system such as a TOPO TA cloning kit (Invitrogen).

### B: Reconstructing CRISPR/Cas Vector System (D10A Nickase)

The D10A nickase version of the CRISPR/Cas system can be conveniently reconstructed by assembling the above fragments where fragment 2 is replaced with fragment 2A which contains the D10A substitution (See sequence below).

### Fragment 2A (852 bp)

The substituted aspartate to alanine is highlighted in bold and underlined.

### C: Target (Spacer) Sequence Cloning

The target spacer sequence can be cloned into the above CRISPR/Cas vector system via the BbsI restriction sites located upstream of the chimeric guided RNA sequence. The spacer sequence can be ordered as oligo pairs and annealed together with overhangs as shown below to allow direct cloning into BbsI linearised CRISPR/Cas vector using standard molecular biology protocols.

Sequence of an example oligo pair with spacer sequence:
5'- CACCGNNNNNNNNNNNNNNNNNNN - 3' (SEQ ID NO: 15)
3' - CNNNNNNNNNNNNNNNNNNNCAAA - 5' (SEQ ID NO: 16)

The 4 bp overhang sequence underlined is required to be included in the spacer oligos to facilitate cloning into the BbsI restriction site in the CRISPR/Cas vector. Using this approach, any spacer sequence can be conveniently cloned into the CRISPR/Cas vector.

### D: Reconstructing CRISPR/Cas System for One-step Generation of Transgenic Animals

In order to reconstitute a CRISPR/Cas system for one-step generation of transgenic animal as described in Wang *et al.* (Wang H., Yang H., Shivalila C.S., Dawlaty M.M., Cheng A.W., Zhang F., Jaenisch R.: One-step generation of mice carrying mutations in multiple genes by CRISPR/Cas-mediated genome engineering., Cell, 2013, 153(4):910-918) where direct embryo injection is used, the above detailed CRISPR/Cas vector system needs to be modified to incorporate a T7 polymerase promoter to the Cas9 coding sequence. In addition, the gRNA needs to be removed and synthetised separately by annealing oligos or produced synthetically (See below for an example T7-spacer sequence fused to chimeric guided RNA sequence - T7-gRNA). Note, ideally the spacer sequence will be designed in a unique region of a given chromosome to minimise off-target effect and also the respective protospacer genomic sequence needs to have a PAM at the 3'-end.

### Example T7-gRNA Sequence

The underlined 20 bp of N's depicts the spacer sequence for a given target DNA.

To reconstruct the one-step CRISPR/Cas system, the above detailed DNA fragments (Fragments 2, 3, 4, 5, 6 & 7) can be assembled together where fragment 1A (containing 45 bp homology to pBlueScript KS+ vector 5' to the EcoRV restriction site, human U6 promoter, BbsI restriction sites, chimeric guided RNA sequence and chicken b-actin promoter) is replaced with fragment 1, which only contains 45 bp homology to pBlueScript KS+ vector and the DNA sequence for T7 polymerase promoter with 45 bp homology to fragment 2. This will create the nuclease version of the CRISPR/Cas system for one-step generation of transgenic animals. To create the nickase version, fragment 2 can be replaced with fragment 2A as detailed above and then fragments 1, 2A, 3, 4, 5, 6 and 7 can be assembled together either by Gibson assembly or by assembly PCR.

### Fragment 1 (111 bp)

### E: Preparation of Oligo/DNA fragments for HDR-mediated repair

DNA oligos ranging from 15 bp and upwards in excess of >125 bp will be synthetised through Sigma Custom Oligo synthesis Service. The oligos can contain any sequence such as a defined mutation, introduced restriction sites or a sequence of interest including recombination recognition sequence such as loxP or derivatives thereof, Frt and derivatives thereof or PiggyBac LTR or any other transposon elements or regulatory elements including enhancers, promoter sequence, reporter gene, selection markets and tags. The oligo design will incorporate DNA homology to the region where Cas9 mediates double-stranded DNA break or DNA nick. The size of the homology will range from a few base pairs (2-5 bp) to upwards and in excess of 80 bp. Larger DNA fragments (>100 bp ranging up to several kilobases) will be prepared either synthetically (GeneArt) or by PCR. The DNA fragment will be synthetised either with or without flanked NLS or only with a single NLS and either with or without a promoter (e,g, T7 polymerase promoter). The DNA can be prepared as a single stranded DNA fragment using either the capture biotinylated target DNA sequence method (Invitrogen: Dynabeads M-270 Streptavidin) or any other standard and established single stranded DNA preparation methodology. The single stranded DNA can be prepared for microinjection by IVT as described above and the mRNA co-injected with Cas9 mRNA and gRNA. The DNA fragment can also be co-injected as a double stranded DNA fragment. The DNA fragment will be flanked by DNA homology to the site where Cas9 mediates double-stranded DNA break or DNA nick. The DNA homology can range from a few base pairs (2-5 bp) and up to or in excess of several kilobases. The DNA fragment can be used to introduce any endogenous or exogenous DNA.

HDR-mediated repair can also be done in ES cells following CRISPR/Cas-mediated DNA cleavage. The above mentioned donor oligo or DNA fragment can be co-transfected into ES cells along with the CRISPR/Cas expression vector.

### F: Production of Cas9 mRNA and gRNA

Vector containing the T7 polymerase promoter with the coding region of humanised Cas9 will be PCR amplified using oligos Cas9-F and Cas9-R. The T7-Cas9 PCR product can be gel extracted and the DNA purified using Qiagen gel extraction kit. The purified T7-Cas9 DNA will be used for in vitro transcription (IVT) using mMESSAGE mMACHINE T7 Ultra Kit (Life Technologies Cat No. AM1345). The vector containing the T7-gRNA can be PCR amplified using oligos gRNA-F and gRNA-R and once again the PCR products gel purified. IVT of the T7-gRNA will be carried out using MEGAshortscript T7 Kit (Life Technologies Cat No. AM1354) and the gRNA purified using MEGAclear Kit (Life Technologies Cat No. AM1908) and eluted in RNase-free water.

| | |
|---|---|
| Cas9-F: | TTAATACGACTCACTATAGG (SEQ ID NO:19) |
| Cas9-R: | GCGAGCTCTAGGAATTCTTAC (SEQ ID NO:20) |
| gRNA-F: | TTAATACGACTCACTATAGG (SEQ ID NO:21) |
| gRNA-R: | AAAAAAGCACCGACTCGGTGCCAC (SEQ ID NO:22) |

### Reference Example 5B: One step generation of Transgenic animals

### A: ES Cell Transfection Procedure

Mouse embryonic stem cells AB2.1 and derivatives of this line will be used for transfecting the mammalian codon optimised Cas9 and sgRNA from a single expression vector or from separate vectors if desired. AB2.1 ES cells will be cultured on a PSNL76/7/4 MEF feeder layer in M-15: Knockout DMEM (Gibco, no pyruvate, high glucose, 15% FBS, 1xGPS, 1xBME) with standard ES cell culturing techniques. Transfection of CRISPR/Cas expression vector along with the optional addition of a donor oligo or DNA fragment will be done by electroporation using the Amaxa 4D-Nucleofector® Protocol (Lonza). A plasmid expressing PGK-Puro will also be optionally co-transfected to promote transfection efficiency.

In one method, after transfection ES cells will be plated back onto feeder plates and Puromycin (2µg/ml) will be added 72 hours post transfection for 7 days after which colonies will be picked and genotyped by PCR. Positive colonies will be further cultured and expanded on feeder layer and selection markers where necessary will be excised using a PiggyBac transposon system. This will be done by electroporation of ES cells with a plasmid containing HyPbase using the Amaxa 4D-Nucleofector® Protocol (Lonza). The ES cell will be plated back onto feeder plates. ES cells will be passaged 2-3 days post transfection and after a further 2-3 days the ES cells will be plated out at different cells densities (1:10, 1:20, 1:100 and 1:300) and FIAU (2µg/ml) selection will be added 24 hours after replating. Colonies will be picked and analysed by PCR genotyping after 7-10 days on selection media. Positive clones will be further cultured and expanded on feeder layer and sent for zygote (blastocyst) microinjection.

In an alternative method, 8 hours prior to transfection ES cells are seeded at a density of 0.5x106 cells using antibiotic free M-15 Knockout DMEM (Gibco, no pyruvate, high glucose, 15% FBS, 1xL-Glutamine, 1xBME) onto 6w feeder plates. Transient transfection is performed using Lipofectamine® LTX Reagent with PLUS™ Reagent (Invitrogen™) by standard protocol. After incubation time transfection reagents are transferred to feeder plates (cultured in antibiotic free media), media (M-15) will not be changed on these plates for at least 24 hours post transfection. 48 hours post transfection ES cells are trypsinized into a single cell suspension and a cell count is carried out and cells are plated out at different cell densities ranging for 100-5000 cells per 10cm feeder plate. 24 hours after replating Puro selection at 2µg/ml (Puromycin dihydrochloride from Streptomyces alboniger powder, P8833 Sigma) is applied to the cells for 4 days, after which cells are cultured again in M-15. Colonies are picked 10-13 days post transfection.

### Method 5C: Microinjection of Mouse Zygotes - Method 1

### Materials and Reagents:

- M2 (Sigma M7167)
- Embryo Max KSOM (Speciality media MR-020P-F)
- Hyaluronidase (Sigma H4272)
- Mineral Oil (Sigma, M-8410)

Possible Donor Strains:
- S3F/S3F;KF3/KF3
- S3F/S3F;K4/K4
- S7F/S7F
- K5F/K5F

### Preparation of Zygotes and Microinjection:

The protocol is as described in: A.Nagy Et al. Manipulating the Mouse Embryo 3rd Edition. Chapter 7, Protocols 7-1, 7-6, 7-10, 7-11.Cold Spring Harbor Laboratory Press.

In brief:
1. Zygotes are harvested from E0.5dpc (day post-coitum) superovulated female mice.
2. The zygotes are incubated in hyaluronidase to disperse cumulus cells.
3. Zygotes are collected and transferred to several drops of M2 medium to rinse off the hyaluronidase solution and debris. Zygotes are placed into KSOM Media and incubated at 37°C, 5% CO₂ until required.
4. Zygote quality is assessed and zygotes with normal morphology are selected for injection, these are placed in KSOM media and incubated at 37°C, 5% CO₂ until required.

### Microinjection set up:

Injection procedures are performed on a Nikon Eclipse Ti inverted microscope with Eppendorf micromanipulators and an Eppendorf femtojet injection system. A slide is prepared by adding a large drop ∼200 microlitres of M2 into the centre.

### Microinjection:

Place an appropriate number of zygotes onto the slide. Examine the zygotes and select only those with normal morphology (2 distinct pronuclei are visible). Whilst holding a zygote with a male pronucleus closest to the injection pipette, carefully push the injection pipette through the zona pellucida into the pronucleus, apply injection pressure, the pronucleus should visibly swell, remove the injection pipette quickly. The injected zygote can be placed down while the rest are injected.

At the end of the injection session all viable injected zygotes should be placed into prepared dishes containing drops of KSOM and incubated until ready to surgically implant. They are incubated for 2-3 hours before surgically implanting into pseudo pregnant females. Pups will be born 21 days later.

### Method 5C: Microinjection of Mouse Zygotes - Method 2

### Materials And Reagents

- PMSG
- hCG
- M2 (Sigma M7167)
- Embryo Max KSOM (Specialty media MR-020P-F)
- Mineral Oil (Sigma, M-8410)
- Hyluronidase (Sigma H 4272)
- 35mm Falcon Petri dishes(Fisher 08-757-100A)
- Sharp scissors
- Sharp watchmakers forceps

### Preparation of Oocytes:

1. Day 0: Give PMSG (5 I.U.) to the females by I. P. injection.
2. Day 2: Give hCG (5 I.U.) to the females 48 Hours later by I. P. injection. Mate the females to stud males.
3. Day 3: Check plugs, sacrifice plugged female mice by CO2 asphyxiation or cervical dislocation at 0.5dpc at 8.00 am.
4. Dissect open the abdomen, locate the ovary and fat pad, dissect out the oviduct leaving the ovary and fat, trimming the uterine horn to ∼1cm, place into a 35mm Petri dish containing M2 at room temp.
5. Place one ovary at a time into a dish containing hyaluronidase solution in M2 (∼0.3mg/ml) at room temp. View through a stereoscope at 20x or 40x magnification.
6. Use one pair of forceps to grasp the oviduct and hold it on the bottom of the dish. Use the second pair of forceps or a 26g needle to tear the oviduct close to where the zygotes are located (the ampulla), releasing the clutch of cumulus cells.
7. The zygotes should be left in the hyaluronidase for a few minutes only, after which time the zygotes may become damaged. If necessary pipette them up and down a few times to help the release of the zygotes from the cumulus cells.
8. Use a mouth pipette to pick up the zygotes and transfer them to a fresh dish of M2, then transfer through several drops of M2 to rinse off the hyaluronidase, cumulus cells and debris. Sort through the zygotes removing any obviously bad ones (fragmented, misshapen, not fertilized), and place the good ones (two polar bodies should be visible and any with polar bodies) into equilibrated drops of KSOM+ AA at 37°c and 5% CO₂, keep incubated until needed. Place about 50 eggs per drop.

### Pronuclear Microinjection

1. **Microinjection set up:** Injection procedures are performed on a Nikon Eclipse Ti inverted microscope with Eppendorf micromanipulators. Prepare a 60mm petri dish to place injected zygotes into. Pipette four - six 40µl drops of KSOM+AA, cover with oil and place in a 5% CO₂ incubator to equilibrate. Prepare a cavity slide by making a large (∼200µl) drop of M2 media onto the center of the well, add a small drop of medium on the left side of the slide, for the holding pipette.
2. **Microinjection:** Ensure that the pressurized injector has been switched on and is ready to use. Place an appropriate number of zygotes onto the slide, do not add more zygotes than can be injected within 20-30mins. Place the holding pipette into the drop of M2 on the left of the slide; it will fill using capillary action, once filled to about the shoulder attach to the manipulator. Carefully examine the zygotes, making sure that two pronuclei are visible and morphology is good, discard any that appear abnormal. To test if the injection needle is open, place the tip near to but not touching a zygote in the same focal plane. Apply pressure using the pressurized system, if the zygote moves the needle is open, if it doesn't the needle is closed. In this case apply pressure using the "clear' feature, if the tip is still not open manually break the tip. Carefully "knock" the tip on the holding pipette and repeat the above test, make sure the tip does not become too large, if this happens replace the needle and start again. Place the tip of the holding pipette next to a zygote and suck it onto the end of the pipette by applying negative pressure. Focus the microscope to locate the pronuclei, the zygote should be positioned in such a way that allows injection into the zygote without hitting the pronuclei, preferably with a gap between the zona pellucida and the oolema. Bring the tip of the injection needle into the same focal plane as the zona pellucida. Bring the injection pipette to the same y-axis position as the zona pellucida, adjust the height of the needle so the tip appears completely sharp, without changing the focus. This ensures the needle will target the zygote exactly. Push the injection pipette through the zona pellucida, through the cytoplasm towards the back of the zygote. The needle will create a "bubble" through the oolema, this needs to be broken, you will see it snap back at which point remove the needle quickly, you will see the cytoplasm moving to indicate RNA is flowing from the needle. Cytoplasmic granules flowing out of the oocytes after removal of the injection pipette is a clear sign that the zygote will soon lyse. In this case, or if nuclear/cytoplasmic components are sticking to the injection pipette, the oocytes should be discarded after injection. If the zygote appears to be intact and successfully injected, sort this into a good group. Pick a new zygote for injection. The same injection pipette can be used as long as it continues to inject successfully. Switch to a new injection pipette if (a) you cannot see any cytoplasmic distortion (b) zygotes are lysing one after the other; (c) the tip of the pipette becomes visibly "dirty" or nuclear contents stick to the pipette. Once all the zygotes have been injected, remove them and place them into the equilibrated KSOM +AA and place them into the incubator at 37°C overnight. Only transfer those zygotes that have survived injection, and cultured to the 2 cell stage. Leave any lysed ones, and zygotes that have not developed.
3. Count the total number injected and record the numbers transferred per recipient

### Results

To demonstrate the efficient of the one-step generation of transgenic mice, we used our T7-Cas9 nuclease vector to generate mRNA via in vitro transcription detailed above. mRNA from the guide RNA was also produced using in vitro transcription described above. Before injecting the mRNA mixture into the cytoplasm, oocytes were prepared from female mice using the protocol detailed above. An mRNA mixture containing 100ng/ul Cas9 nuclease mRNA and 50ng/ul guide mRNA was injected by microinjection into the cytoplasm as detailed above. The microinjection is done at the single-cell stage. Zygotes that survived the injection were cultured to 2 cell stage, which were then transferred to recipient mice.

In total, 107 zygotes were injected from which 49 survived and went to 2 cell stage. These were then transferred to two recipient female mice. This resulted in 19 pups from 2 litters. Litter 1 yielded 3 males and 6 females. Litter 2 yielded 4 males and 6 females. The pups were ear clipped 3 weeks after birth and DNA was extracted. PCR was carried out using oligos flanking the gRNA to detect possible indels (Figure 14).

PCR amplifying around the guide RNA and separating out the PCR products on an agarose gel highlighted at least one mouse contained a large indel in the form of a deletion, whereas other mice appeared to have smaller indels judging by the sharpness of the PCR product on the gel. As an initial crude analysis, all the PCR products were sent for sequencing and those marked with an asterix (7 mice in total, Figure 14) yielded mix sequences around the gRNA further confirming they contain indels. To confirm this, the PCR products from these 7 mice together with the PCR product from another mouse which did not yield a mix sequence (PCR product from lane 19, Figure 14) were individually cloned into a general cloning vector. From each individual cloning, 28 clones were picked and analysed by sequencing. The sequencing confirmed all 7 mice contain indels and the mice that did not contain any mix sequence contained no indels. The sequencing data is summarised in Table 3.

The sequencing data confirmed all of the mice analysed contained indels. It also suggests that using our zygote injection protocol detailed above and our method for preparing mRNA for Cas9 and guide RNA, Cas9 works efficiently at an early stage and until the point where cells starts to divide beyond the 2 cell stage judging by the fact that in all of the mice analysed, no more than 3 types of indels were identified. Out of the 7 mice containing indels, 3 of them had no detectable WT sequence. The female mouse (KMKY6.1j) that did not show mix sequence from the initial sequencing analysis indeed did not contain any indels so it validates our initial sequencing analysis of the PCR products.

The male mouse (KMKY5.1c) that showed no WT sequence was used as a mating partner for the two female mice (KMKY5.1e & KMKY6.1e) that showed no WT sequence too. The resulting pups from the two matings yielded 14 pups in total from the first litter. Following similar sequencing analysis whereby PCR products amplified from the region around the guide RNA were cloned individual and several clones were then analysed for the presence of indels. For each mouse, 24 clones were analysed by sequencing. The sequencing data from all 14 pups confirmed only two indel sequences reflecting the two alleles arising from the parental male and female mouse. This data unequivocally demonstrates that our one-step genome editing protocol works very efficiently at an early stage and not beyond the 2 cell stage thus avoiding complex mosaic indel formation. Using our established protocol, we can carry out define deletions directly in zygotes or carry out define deletion followed by insertion to expedite the process of generating transgenic mice to homozygosity in record time.

### Reference Example 6:

### Single Copy Cas9 Expression in ES Cells

Reference is made to figure 6B.
1. A landing pad consisting of a PiggyBac transposon element with the following features will be targeted into mouse ES cells (e.g., 129-derived ES cells, such as AB2.1 ES cells; Baylor College of Medicine, Texas, USA) and selected for on G418. The PiggyBac transposon element will contain neomycin resistance gene flanked by loxP and lox2272. It will also have a geneless PGK promoter. In this example, the landing pad will be targeted into the introgenic region of Rosa26 gene located on chromosome 6, but it could be targeted elsewhere. Targeting this landing pad in the Rosa26 gene will provide a universal ES cell line for precisely inserting any desired DNA fragment including DNA fragments containing Cas9, mutant Cas9 or any other gene of interest via RMCE with high efficiency. Targeting Rosa26is beneficial since the targeted construct will be inserted as a single copy (unlike random integration elsewhere) and is unlikely to produce an unwanted phenotypic effect.
   Note. This landing pad can be inserted into any gene in any chromosome or indeed in any eukaryotic or mammalian cell line, e.g., a human, insect, plant, yeast, mouse, rat, rabbit, rodent, pig, dog, cat, fish, chicken or bird cell line, followed by generation of the respective transgenic organism expressing Cas9.

### Rosa 26 Locus

Ubiquitous expression of transgene in mouse embryonic stem cell can be achieved by gene targeting to the ROSA26 locus (also known as: gene trap ROSA 26 or Gt(ROSA)26) by homologous recombination (Ref. (a) and (b) below). The genomic coordinates for mouse C57BL/6J Rosa26 gene based on Ensemble release 73 - September 2013 is: Chromosome 6: 113,067,428 - 113,077,333; reverse strand.

The Rosa26 locus can also be used to as a recipient location to knock-in a transgene. In our example we have use the Rosa26 locus to knock-in the landing pad vector by targeting through homologous recombination into the intronic region located between exons 2 and 3 of mouse strain 129-derived embryonic stem cells using approx. 3.1 kb homology arms. The homology arms were retrieved by recombineering from a BAC Clone generated from mouse strain 129. The sequence of the Rosa26 homology arms used for targeting is given below.

### Sequence of Rosa26 5' homology arm

### Sequence of Rosa26 3' homology arm

### Reference:

a) Pablo Perez-Pinera, David G. Ousterout, Matthew T. Brown and Charles A. Gersbach (2012) Gene targeting to the ROSA26 locus directed by engineered zinc finger nucleases. Nucleic Acids Research, 2012, Vol. 40, No. 8 3741-3752
b) Peter Hohenstein, Joan Slight, Derya Deniz Ozdemir, Sally F Burn, Rachel Berry and Nicholas D Hastie (2008) High-efficiency Rosa26 knock-in vector construction for Cre-regulated overexpression and RNAi. PathoGenetics 2008, 1:3

2. A recombinase mediated cassette exchange (RMCE)-enabled vector containing a promoterless puromycin-delta-tk with in-frame fusion of T2A at the C-terminus following by either Cas9 or mutant Cas9 nucleotide sequence and a series of unique restriction sites flanked by loxP and lox2272 will allow for the direct targeting of this vector into the landing pad by Cre-mediated RMCE. As is known, T2A allows ribosomal skipping during translation. The insertion of the coding sequence of T2A between two genes results in two products (one gene, one transcript but two proteins expressed, in this case the Cas9 and selection marker). ES clones containing the correctly inserted DNA fragment can be directly selected on puromycin. This approach also advantageously ensures single copy expression of Cas9 as suppose to a random integration or transient expression approach. Insertion of the RMCE enabled vector into the desired locus containing the landing pad can be selected directly as the PGK promoter in the landing pad will drive the transcription of the promoterless Puro-Delta-Tk and Cas9. Since the Puro-delta-Tk is in the same transcriptional unit as Cas9, ES clones selected on puromycin will ensure expression of Cas9.
3. The above strategy allows for three separate approaches to express the sgRNA designed for disrupting (mutation through indel formation, deletion or deletion followed by insertion) gene of interest.
   a. The above ES cell line containing Cas9 can be used for generating transgenic mice with either constitutively expressed Cas9 or modified for inducible Cas9 expression or indeed tissue specific Cas9 expression for example expression of Cas9 at an embryo stage using Nanog-, Pou5f1- or SoxB promoter-specifc Cas9 expression. Such derived mouse line expressing Cas9 can be used for genome editing in a streamline fashion whereby *in vitro* transcribed sgRNA can be easily injected into embryos obtained from such transgenic mice. This will enhance the efficiency of generating mouse lines with the desired homozygous genotype and thus will dramatically reduce the number of animals required.
   b. sgRNA can be transfected directly into the ES cells expressing Cas9 and thus avoids the requirement for cloning into the RMCE enabled vector single or multiple sgRNA. This approach will allow multiple sgRNA to be inserted into the ES cells simultaneously very rapidly.
   c. Multiple sgRNA can be cloned directly into the multiple cloning site of the RMCE enabled vector (ie, using a plurality of different restriction endonuclease sites) to allow single copy expression of the guide-RNA. This approach may be useful for limiting off-target effects particularly relevant for those genes with high sequence homology within the genome.
4. ES cells expressing Cas9 and sgRNA can be selected for directly on medium containing puromycin. Selection on puromycin for 4-6 days will allow for the desired location to be mutated or disrupted and the advantage of manipulating ES cells is that individual clones can be analysed by PCR followed by sequencing for the desired mutation. Only correctly mutated ES cell clones can be processed further whereby inserted DNA element introduced through insertion of the landing pad and the subsequent insertion of the RMCE vector can be completely removed leaving the ES cell devoid of any alteration other than the intended mutation induced by the action of Cas9 and the sgRNA. This can be done through transiently expressing PBase transposon followed by selection on FIAU. Removal of the constitutively expressed Cas9 with only the minimal length of time required to induce mutation in the presence of sgRNA will reduce or eliminate the possibility of Cas9 inducing unwanted mutations.
5. ES Clones containing the desired mutation can be injected into blastocyst to generate transgenic mice.

**Table 1: PAM conservation in repeats and leaders for various CRISPR types**

| | | | | |
|---|---|---|---|---|
| (reproduced from Short motif sequences determine the targets of the prokaryotic CRISPR defence system F. J. M. Mojica, C. Díez-Villaseñor, J. García-Martínez, C. Almendros Microbiology(2009), 155, 733-740) | | | | |

| | **Genomes*** | **PAM** | **CRISPR Consensus^{†}** | **Leaders^{‡}** |
|---|---|---|---|---|
| **Group 1** | Mth | NGG | ATTTCAATCCCATTTTGGTCTGATTTTAAC | |
| | Lmo | WGG | ATTTACATTTCAHAATAAGTARYTAAAAC | |
| **Group 2** | Eco | CWT | CGGTTTATCCCCGCTGGCGCGGGGAACWC | |
| | Pae | CTT | CGGTTCATCCCCACRCMYGTGGGGAACAC | |
| **Group 3** | Spy | GAA | ATTTCAATCCACTCACCCATGAAGGGTGAGAC | TGCGCCAAAT |
| | Xan | GAA | GTTTCAATCCACGCGCCCGTGAGGRCGCGAC | |
| **Group 4** | She | GG | TITCTAAGCCGCCTGTGCGGCGGTGAAC | |
| | Pae | GG | TTTCTTAGCTGCCTATACGGCAGTGAAC | |
| | Ype | GG | TTTCTAAGCTGCCTGTGCGGCAGTGAAC | GTAAGATAAT |
| **Group 7** | Sso | NGG | CTTTCAATTCTATAAGAGATTATC | TGAGGGTTTA |
| | Mse | NGG | CTTTCAACTCTATAGGAGATTAAC | |
| **Group 10** | Str | NGG | GTTTTAGAGCTATGCTGTTTTGAATGGTCCCAAAAC | |
| | Lis | NGG | GTTTTAGAGCTATGTTATTTTGAATGCTAMCAAAAC | |

| | | | | |
|---|---|---|---|---|
| * Genomes are abbreviated according to the denominations of the species or genera carrying the corresponding CRISPR arrays: Mth, *M. thermautotrophicus;* Lmo, *L*. *monocytogenes;* Eco, *E coli;* Pae, *P. aeruginosa;* Spy, *S. pyogenes;* Xan, *Xanthomonas* spp.; She, *Shewanella* spp.; Ype, *Y. pestis;* Sso, *S*. *solfataricus;* Mse, *M. sedula,* Str, *Streptococcus* spp.; Lis, *Listeria* spp. † Sequences matching the PAM are underlined. ‡ Representative CRISPR array proximal Leader sequences. Nucleotides matching the PAM are underlined. | | | | |

SEQ ID NOs for the sequences in Table 1 are set out in the table below.

**Table 2: CRISPR-Associated Endonucleases**

| | **Genomes*** | **PA M** | **CRISPR Consens^{†}** | **SEQ ID NO.** | **Leaders^{‡}** | **SEQ ID NO** |
|---|---|---|---|---|---|---|
| Group 1 | Mth | NG G | | 25 | | 38 |
| | | | | | | 39 |
| | Lmo | WG G | | 26 | | 40 |
| | | | | | | 41 |
| Group 2 | Eco | CW T | | 27 | | 42 |
| | | | | | | 43 |
| | Pae | CT T | | 28 | | 44 |
| | | | | | | 45 |
| Group 3 | Spy | GA A | | 29 | | 46 |
| | Xan | GA A | | 30 | | 47 |
| | | | | | | 48 |
| Group 4 | She | GG | | 31 | | 49 |
| | | | | | | 50 |
| | Pae | GG | | 32 | | 51 |
| | | | | | | 52 |
| | Ype | GG | | 33 | | 53 |
| Grou p 7 | Sso | NG G | | 34 | | 54 |
| | Mse | NG G | | 35 | | 55 |
| | | | | | | 56 |
| | | | | | | 57 |
| Grou p 10 | Str | NG G | | 36 | | 58 |
| | | | | | | 59 |
| | Lis | NG G | | 37 | | 60 |
| | | | | | | 61 |

[Gene ID numbers refer to genes in the NCBI Gene Database as at September 2013; all sequence information relating to the gene IDs below is incorporated herein by reference for possible use in the present invention]

### 1. Plav_0099

CRISPR-associated endonuclease Csn1 family protein [Parvibaculum lavamentivorans DS-1]
Other Aliases: Plav_0099
Genomic context: Chromosome
Annotation: NC_009719.1 (105795..108908, complement)
ID: 5454634
SEQ ID NO: 62

### 2. FTN_0757

membrane protein [Francisella novicida U112]
Other Aliases: FTN_0757
Genomic context: Chromosome
Annotation: NC_008601.1 (810052..814941)
ID: 4548251
SEQ ID NO: 63

### 3. Cj1523c

CRISPR-associated protein [Campylobacter jejuni subsp. jejuni NCTC 11168 = ATCC 700819]
Other Aliases: Cj1523c
Genomic context: Chromosome
Annotation: NC_002163.1 (1456880..1459834, complement)
ID: 905809
SEQ ID NO: 64

### 4. mcrA

restriction endonuclease [Bifidobacterium longum DJO10A]
Other Aliases: BLD_1902
Genomic context: Chromosome
Annotation: NC_010816.1 (2257993..2261556)
ID: 6362834
SEQ ID NO: 65

### 5. MGA_0519

Csn1 family CRISPR-associated protein [Mycoplasma gallisepticum str. R(low)]
Other Aliases: MGA_0519
Genomic context: Chromosome
Annotation: NC_004829.2 (919248..923060)
ID: 1089911
SEQ ID NO: 66

### 6. Emin_0243

CRISPR-associated endonuclease Csn1 family protein [Elusimicrobium minutum Pei191]
Other Aliases: Emin_0243
Genomic context: Chromosome
Annotation: NC_010644.1 (261119..264706)
ID: 6263045
SEQ ID NO: 67

### 7. FTW_1427

CRISPR-associated large protein [Francisella tularensis subsp. tularensis WY96-3418]
Other Aliases: FTW_1427
Genomic context: Chromosome
Annotation: NC_009257.1 (1332426..1335803, complement)
ID: 4958852
SEQ ID NO: 68

### 8. SMA_1444

CRISPR-associated protein, Csn1 family [Streptococcus macedonicus ACA-DC 198]
Other Aliases: SMA_1444
Annotation: NC_016749.1 (1418337..1421729, complement)
ID: 11601419
SEQ ID NO: 69

### 9. SSUST3_1318

CRISPR-associated protein, Csn1 family [Streptococcus suis ST3]
Other Aliases: SSUST3_1318
Genomic context: Chromosome
Annotation: NC_015433.1 (1323872..1327240, complement)
ID: 10491484
SEQ ID NO: 70

### 10. cas5

CRISPR-associated protein, Csn1 family [Streptococcus gallolyticus UCN34]
Other Aliases: GALLO_1439
Genomic context: Chromosome
Annotation: NC_013798.1 (1511433..1514825, complement)
ID: 8776949
SEQ ID NO: 71

### 11. GALLO_1446

CRISPR-associated protein [Streptococcus gallolyticus UCN34]
Other Aliases: GALLO_1446
Genomic context: Chromosome
Annotation: NC_013798.1 (1518984..1523110, complement)
ID: 8776185
SEQ ID NO: 72

### 12. csn1

CRISPR-associated endonuclease Csn1 [Bifidobacterium dentium Bd1]
Other Aliases: BDP_1254
Genomic context: Chromosome
Annotation: NC_013714.1 (1400576..1403992, complement)
ID: 8692053
SEQ ID NO: 73

### 13. NMO_0348

putative CRISPR-associated protein [Neisseria meningitidis alpha14]
Other Aliases: NMO_0348
Genomic context: Chromosome
Annotation: NC_013016.1 (369547..372795, complement)
ID: 8221228
SEQ ID NO: 74

### 14. csn1

CRISPR-Associated Protein Csn1 [Streptococcus equi subsp. zooepidemicus MGCS10565]
Other Aliases: Sez_1330
Genomic context: Chromosome
Annotation: NC_011134.1 (1369339..1373385, complement)
ID: 6762114
SEQ ID NO: 75

### 15. csn1

CRISPR-associated endonuclease Csn1 family protein [Streptococcus gordonii str. Challis substr. CH1]
Other Aliases: SGO_1381
Genomic context: Chromosome
Annotation: NC_009785.1 (1426750..1430160, complement)
ID: 5599802
SEQ ID NO: 76

### 16. M28_Spy0748

cytoplasmic protein [Streptococcus pyogenes MGAS6180]
Other Aliases: M28_Spy0748
Genomic context: Chromosome
Annotation: NC_007296.1 (771231..775337)
ID: 3573516
SEQ ID NO: 77

### 17. SGGBAA2069_c14690

CRISPR-associated protein [Streptococcus gallolyticus subsp. gallolyticus ATCC BAA-2069]
Other Aliases: SGGBAA2069_c14690
Genomic context: Chromosome
Annotation: NC_015215.1 (1520905..1525017, complement)
ID: 10295470
SEQ ID NO: 78

### 18. SAR116_2544

CRISPR-associated protein, Csn1 family [Candidatus Puniceispirillum marinum IMCC1322]
Other Aliases: SAR116_2544
Genomic context: Chromosome
Annotation: NC_014010.1 (2748992..2752099)
ID: 8962895
SEQ ID NO: 79

### 19. TDE0327

CRISPR-associated Cas5e [Treponema denticola ATCC 35405]
Other Aliases: TDE0327
Genomic context: Chromosome
Annotation: NC_002967.9 (361021..365208)
ID: 2741543
SEQ ID NO: 80

### 20. csn1

CRISPR-associated protein [Streptococcus pasteurianus ATCC 43144]
Other Aliases: SGPB_1342
Genomic context: Chromosome
Annotation: NC_015600.1 (1400035..1403427, complement)
ID: 10753339
SEQ ID NO: 81

### 21. cas9

CRISPR-associated protein [Corynebacterium ulcerans BR-AD22]
Other Aliases: CULC22_00031
Genomic context: Chromosome
Annotation: NC_015683.1 (30419..33112, complement)
ID: 10842578
SEQ ID NO: 82

### 22. MGAS2096_Spy0843

putative cytoplasmic protein [Streptococcus pyogenes MGAS2096]
Other Aliases: MGAS2096_Spy0843
Genomic context: Chromosome
Annotation: NC_008023.1 (813084..817190)
ID: 4066021
SEQ ID NO: 83

### 23. MGAS9429_Spy0885

cytoplasmic protein [Streptococcus pyogenes MGAS9429]
Other Aliases: MGAS9429_Spy0885
Genomic context: Chromosome
Annotation: NC_008021.1 (852508..856614)
ID: 4061575
SEQ ID NO: 84

### 24. AZL_009000

CRISPR-associated protein, Csn1 family [Azospirillum sp. B510]
Other Aliases: AZL_009000
Genomic context: Chromosome
Annotation: NC_013854.1 (1019522..1023028, complement)
ID: 8789261
SEQ ID NO: 85

### 25. EUBREC_1713

contains RuvC-like nuclease and HNH-nuclease domains [Eubacterium rectale ATCC 33656]
Other Aliases: EUBREC_1713
Other Designations: CRISPR-system related protein
Genomic context: Chromosome
Annotation: NC_012781.1 (1591112..1594456)
ID: 7963668
SEQ ID NO: 86

### 26. Alide2_0194

CRISPR-associated protein, Csn1 family [Alicycliphilus denitrificans K601]
Other Aliases: Alide2_0194
Genomic context: Chromosome
Annotation: NC_015422.1 (218107..221196)
ID: 10481210
SEQ ID NO: 87

### 27. Alide_0205

crispr-associated protein, csn1 family [Alicycliphilus denitrificans BC]
Other Aliases: Alide_0205
Genomic context: Chromosome
Annotation: NC_014910.1 (228371..231460)
ID: 10102228
SEQ ID NO: 88

### 28. STER_1477

CRISPR-system-like protein [Streptococcus thermophilus LMD-9]
Other Aliases: STER_1477
Genomic context: Chromosome
Annotation: NC_008532.1 (1379975..1384141, complement)
ID: 4437923
SEQ ID NO: 89

### 29. STER_0709

CRISPR-system-like protein [Streptococcus thermophilus LMD-9]
Other Aliases: STER_0709
Genomic context: Chromosome
Annotation: NC_008532.1 (643235..646600)
ID: 4437391
SEQ ID NO: 90

### 30. cas9

CRISPR-associated protein [Corynebacterium diphtheriae 241]
Other Aliases: CD241_2102
Genomic context: Chromosome
Annotation: NC_016782.1 (2245769..2248399)
ID: 11674395
SEQ ID NO: 91

### 31. cas3

CRISPR-associated endonuclease [Corynebacterium diphtheriae 241]
Other Aliases: CD241_0034
Genomic context: Chromosome
Annotation: NC_016782.1 (35063..38317)
ID: 11672999
SEQ ID NO: 92

### 32. Corgl_1738

CRISPR-associated protein, Csn1 family [Coriobacterium glomerans PW2]
Other Aliases: Corgl_1738
Genomic context: Chromosome
Annotation: NC_015389.1 (2036091..2040245)
ID: 10439994
SEQ ID NO: 93

### 33. Fluta_3147

CRISPR-associated protein, Csn1 family [Fluviicola taffensis DSM 16823]
Other Aliases: Fluta_3147
Genomic context: Chromosome
Annotation: NC_015321.1 (3610221..3614597, complement)
ID: 10398516
SEQ ID NO: 94

### 34. Acav_0267

CRISPR-associated protein, Csn1 family [Acidovorax avenae subsp. avenae ATCC 19860]
Other Aliases: Acav_0267
Genomic context: Chromosome
Annotation: NC_015138.1 (295839..298976)
ID: 10305168
SEQ ID NO: 95

### 35. NAL212_2952

CRISPR-associated protein, Csn1 family [Nitrosomonas sp. AL212]
Other Aliases: NAL212_2952
Genomic context: Chromosome
Annotation: NC_015222.1 (2941806..2944940, complement)
ID: 10299493
SEQ ID NO: 96

### 36. SpiBuddy_2181

CRISPR-associated protein, Csn1 family [Sphaerochaeta globosa str. Buddy]
Other Aliases: SpiBuddy_2181
Genomic context: Chromosome
Annotation: NC_015152.1 (2367952..2371491, complement)
ID: 10292274
SEQ ID NO: 97

### 37. Tmz1t_2411

HNH endonuclease [Thauera sp. MZ1T]
Other Aliases: Tmzlt_2411
Genomic context: Plasmid pTha01
Annotation: NC_011667.1 (75253..76200, complement)
ID: 7094333
SEQ ID NO: 98

### 38. Gdia_0342

Csn1 family CRISPR-associated protein [Gluconacetobacter diazotrophicus PAI 5]
Other Aliases: Gdia_0342
Genomic context: Chromosome
Annotation: NC_011365.1 (382737..385748)
ID: 6973736
SEQ ID NO: 99

### 39. JJD26997_1875

CRISPR-associated Cas5e family protein [Campylobacter jejuni subsp. doylei 269.97]
Other Aliases: JJD26997_1875
Genomic context: Chromosome
Annotation: NC_009707.1 (1656109..1659063, complement)
ID: 5389688
SEQ ID NO: 100

### 40. Asuc_0376

CRISPR-associated endonuclease Csn1 family protein [Actinobacillus succinogenes 130Z]
Other Aliases: Asuc_0376
Genomic context: Chromosome
Annotation: NC_009655.1 (431928..435116)
ID: 5348478
SEQ ID NO: 101

### 41. Veis_1230

CRISPR-associated endonuclease Csn1 family protein [Verminephrobacter eiseniae EF01-2]
Other Aliases: Veis_1230
Genomic context: Chromosome
Annotation: NC_008786.1 (1365979..1369185)
ID: 4695198
SEQ ID NO: 102

### 42. MGAS10270_Spy0886

putative cytoplasmic protein [Streptococcus pyogenes MGAS10270]
Other Aliases: MGAS10270_Spy0886
Genomic context: Chromosome
Annotation: NC_008022.1 (844446..848552)
ID: 4063984
SEQ ID NO: 103

### 43. gbs0911

hypothetical protein [Streptococcus agalactiae NEM316]
Other Aliases: gbs0911
Genomic context: Chromosome
Annotation: NC_004368.1 (945801..949946)
ID: 1029893
SEQ ID NO: 104

### 44. NMA0631

hypothetical protein [Neisseria meningitidis Z2491]
Other Aliases: NMA0631
Genomic context: Chromosome
Annotation: NC_003116.1 (610868..614116, complement)
ID: 906626
SEQ ID NO: 105

### 45. Ccan_14650

hypothetical protein [Capnocytophaga canimorsus Cc5]
Other Aliases: Ccan_14650
Genomic context: Chromosome
Annotation: NC_015846.1 (1579873..1584165, complement)
ID: 10980451
SEQ ID NO: 106

### 46. Ipp0160

hypothetical protein [Legionella pneumophila str. Paris]
Other Aliases: Ipp0160
Genomic context: Chromosome
Annotation: NC_006368.1 (183831..187949)
ID: 3118625
SEQ ID NO: 107

### 47. Cbei_2080

hypothetical protein [Clostridium beijerinckii NCIMB 8052]
Other Aliases: Cbei_2080
Genomic context: Chromosome
Annotation: NC_009617.1 (2422056..2423096)
ID: 5296367
SEQ ID NO: 108

### 48. MMOB0330

hypothetical protein [Mycoplasma mobile 163K]
Other Aliases: MMOB0330
Genomic context: Chromosome
Annotation: NC_006908.1 (45652..49362, complement)
ID: 2807677
SEQ ID NO: 109

### 49. MGF_5203

Csn1 family CRISPR-associated protein [Mycoplasma gallisepticum str. F]
Other Aliases: MGF_5203
Genomic context: Chromosome
Annotation: NC_017503.1 (888602..892411)
ID: 12397088
SEQ ID NO: 110

### 50. MGAH_0519

Csn1 family CRISPR-associated protein [Mycoplasma gallisepticum str. R(high)]
Other Aliases: MGAH_0519
Genomic context: Chromosome
Annotation: NC_017502.1 (918476..922288)
ID: 12395725
SEQ ID NO: 111

### 51. Smon_1063

CRISPR-associated protein, Csn1 family [Streptobacillus moniliformis DSM 12112]
Other Aliases: Smon_1063
Genomic context: Chromosome
Annotation: NC_013515.1 (1159048..1162827, complement)
ID: 8600791
SEQ ID NO: 112

### 52. Spy49_0823

hypothetical protein [Streptococcus pyogenes NZ131]
Other Aliases: Spy49_0823
Genomic context: Chromosome
Annotation: NC_011375.1 (821210..825316)
ID: 6985827
SEQ ID NO: 113

### 53. C8J_1425

hypothetical protein [Campylobacter jejuni subsp. jejuni 81116]
Other Aliases: C8J_1425
Genomic context: Chromosome
Annotation: NC_009839.1 (1442672..1445626, complement)
ID: 5618449
SEQ ID NO: 114

### 54. FTF0584

hypothetical protein [Francisella tularensis subsp. tularensis FSC198]
Other Aliases: FTF0584
Genomic context: Chromosome
Annotation: NC_008245.1 (601115..604486)
ID: 4200457
SEQ ID NO: 115

### 55. FTT_0584

hypothetical protein [Francisella tularensis subsp. tularensis SCHU S4]
Other Aliases: FTT_0584
Genomic context: Chromosome
Annotation: NC_006570.2 (601162..604533)
ID: 3191177
SEQ ID NO: 116

### 56. csn1

CRISPR-associated protein [Streptococcus dysgalactiae subsp. equisimilis RE378]
Other Aliases: GGS_1116
Annotation: NC_018712.1 (1169559..1173674, complement)
ID: 13799322
SEQ ID NO: 117

### 57. SMUGS5_06270

CRISPR-associated protein csn1 [Streptococcus mutans GS-5]
Other Aliases: SMUGS5_06270
Genomic context: Chromosome
Annotation: NC_018089.1 (1320641..1324678, complement)
ID: 13299050
SEQ ID NO: 118

### 58. Y1U_C1412

Csn1 [Streptococcus thermophilus MN-ZLW-002]
Other Aliases: Y1U_C1412
Genomic context: Chromosome
Annotation: NC_017927.1 (1376653..1380819, complement)
ID: 12977193
SEQ ID NO: 119

### 59. Y1U_C0633

CRISPR-system-like protein [Streptococcus thermophilus MN-ZLW-002]
Other Aliases: Y1U_C0633
Genomic context: Chromosome
Annotation: NC_017927.1 (624274..627639)
ID: 12975630
SEQ ID NO: 120

### 60. SALIVA_0715

CRISPR-associated endonuclease, Csn1 family [Streptococcus salivarius JIM8777]
Other Aliases: SALIVA_0715
Annotation: NC_017595.1 (708034..711417)
ID: 12910728
SEQ ID NO: 121

### 61. csn1

CRISPR-associated protein csn1 [Streptococcus mutans LJ23]
Other Aliases: SMULJ23_0701
Annotation: NC_017768.1 (751695..755732)
ID: 12898085
SEQ ID NO: 122

### 62. RIA_1455

CRISPR-associated protein, SAG0894 [Riemerella anatipestifer RA-GD]
Other Aliases: RIA_1455
Genomic context: Chromosome
Annotation: NC_017569.1 (1443996..1448198)
ID: 12613647
SEQ ID NO: 123

### 63. STND_0658

CRISPR-associated endonuclease, Csn1 family [Streptococcus thermophilus ND03]
Other Aliases: STND_0658
Genomic context: Chromosome
Annotation: NC_017563.1 (633621..636986)
ID: 12590813
SEQ ID NO: 124

### 64. RA0C_1034

putative BCR [Riemerella anatipestifer ATCC 11845 = DSM 15868]
Other Aliases: RA0C_1034
Genomic context: Chromosome
Annotation: NC_017045.1 (1023494..1026931, complement)
ID: 11996006
SEQ ID NO: 125

### 65. Sinf_1255

CRISPR-associated protein, SAG0894 family [Streptococcus infantarius subsp. infantarius CJ18]
Other Aliases: Sinf_1255
Genomic context: Chromosome
Annotation: NC_016826.1 (1276484..1280611, complement)
ID: 11877786
SEQ ID NO: 126

### 66. Nitsa_1472

CRISPR-associated protein, csn1 family [Nitratifractor salsuginis DSM 16511]
Other Aliases: Nitsa_1472
Genomic context: Chromosome
Annotation: NC_014935.1 (1477331..1480729)
ID: 10148263
SEQ ID NO: 127

### 67. NLA_17660

hypothetical protein [Neisseria lactamica 020-06]
Other Aliases: NLA_17660
Genomic context: Chromosome
Annotation: NC_014752.1 (1890078..1893326)
ID: 10006697
SEQ ID NO: 128

### 68. SmuNN2025_0694

hypothetical protein [Streptococcus mutans NN2025]
Other Aliases: SmuNN2025_0694
Genomic context: Chromosome
Annotation: NC_013928.1 (737258..741295)
ID: 8834629
SEQ ID NO: 129

### 69. SDEG_1231

hypothetical protein [Streptococcus dysgalactiae subsp. equisimilis GGS_124]
Other Aliases: SDEG_1231
Chromosome: 1
Annotation: Chromosome 1NC_012891.1 (1176755..1180870, complement)
ID: 8111553
SEQ ID NO: 130

### 70. NMCC_0397

hypothetical protein [Neisseria meningitidis 053442]
Other Aliases: NMCC_0397
Genomic context: Chromosome
Annotation: NC_010120.1 (402733..405981, complement)
ID: 5796426
SEQ ID NO: 131

### 71. SAK_1017

hypothetical protein [Streptococcus agalactiae A909]
Other Aliases: SAK_1
Genomic context: Chromosome
Annotation: NC_007432.1 (980303..984415)
ID: 3686185
SEQ ID NO: 132

### 72. M5005_Spy_0769

hypothetical protein [Streptococcus pyogenes MGAS5005]
Other Aliases: M5005_Spy_0769
Genomic context: Chromosome
Annotation: NC_007297.1 (773340..777446)
ID: 3572134
SEQ ID NO: 133

### 73. MS53_0582

hypothetical protein [Mycoplasma synoviae 53]
Other Aliases: MS53_0582
Genomic context: Chromosome
Annotation: NC_007294.1 (684155..688099)
ID: 3564051
SEQ ID NO: 134

### 74. DIP0036

hypothetical protein [Corynebacterium diphtheriae NCTC 13129]
Other Aliases: DIP0036
Genomic context: Chromosome
Annotation: NC_002935.2 (34478..37732)
ID: 2650188
SEQ ID NO: 135

### 75. WS1613

hypothetical protein [Wolinella succinogenes DSM 1740]
Other Aliases: WS1613
Genomic context: Chromosome
Annotation: NC_005090.1 (1525628..1529857)
ID: 2553552
SEQ ID NO: 136

### 76. PM1127

hypothetical protein [Pasteurella multocida subsp. multocida str. Pm70]
Other Aliases: PM1127
Genomic context: Chromosome
Annotation: NC_002663.1 (1324015..1327185, complement)
ID: 1244474
SEQ ID NO: 137

### 77. SPs1176

hypothetical protein [Streptococcus pyogenes SSI-1]
Other Aliases: SPs1176
Genomic context: Chromosome
Annotation: NC_004606.1 (1149610..1153716, complement)
ID: 1065374
SEQ ID NO: 138

### 78. SMU_1405c

hypothetical protein [Streptococcus mutans UA159]
Other Aliases: SMU_1405c, SMU.1405c
Genomic context: Chromosome
Annotation: NC_004350.2 (1330942..1334979, complement)
ID: 1028661
SEQ ID NO: 139

### 79. lin2744

hypothetical protein [Listeria innocua Clip11262]
Other Aliases: lin2744
Genomic context: Chromosome
Annotation: NC_003212.1 (2770707..2774711, complement)
ID: 1131597
SEQ ID NO: 140

### 80. csn1B

CRISPR-associated protein [Streptococcus gallolyticus subsp. gallolyticus ATCC 43143]
Other Aliases: SGGB_1441
Annotation: NC_017576.1 (1489111..1493226, complement)
ID: 12630646
SEQ ID NO: 141

### 81. csn1A

CRISPR-associated protein [Streptococcus gallolyticus subsp. gallolyticus ATCC 43143]
Other Aliases: SGGB_1431
Annotation: NC_017576.1 (1480439..1483831, complement)
ID: 12630636
SEQ ID NO: 142

### 82. cas9

CRISPR-associated protein [Corynebacterium ulcerans 809]
Other Aliases: CULC809_00033
Genomic context: Chromosome
Annotation: NC_017317.1 (30370..33063, complement)
ID: 12286148
SEQ ID NO: 143

### 83. GDI_2123

hypothetical protein [Gluconacetobacter diazotrophicus PAI 5]
Other Aliases: GDI_2123
Genomic context: Chromosome
Annotation: NC_010125.1 (2177083..2180235)
ID: 5792482
SEQ ID NO: 144

### 84. Nham_4054

hypothetical protein [Nitrobacter hamburgensis X14]
Other Aliases: Nham_4054
Genomic context: Plasmid 1
Annotation: NC_007959.1 (13284..16784, complement)
ID: 4025380
SEQ ID NO: 145

### 85. str0657

hypothetical protein [Streptococcus thermophilus CNRZ1066]
Other Aliases: str0657
Genomic context: Chromosome
Annotation: NC_006449.1 (619189..622575)
ID: 3165636
SEQ ID NO: 146

### 86. stu0657

hypothetical protein [Streptococcus thermophilus LMG 18311]
Other Aliases: stu0657
Genomic context: Chromosome
Annotation: NC_006448.1 (624007..627375)
ID: 3165000
SEQ ID NO: 147

### 87. SpyM3_0677

hypothetical protein [Streptococcus pyogenes MGAS315]
Other Aliases: SpyM3_0677
Genomic context: Chromosome
Annotation: NC_004070.1 (743040..747146)
ID: 1008991
SEQ ID NO: 148

### 88. HFMG06CAA_5227

Csn1 family CRISPR-associated protein [Mycoplasma gallisepticum CA06_2006.052-5-2P]
Other Aliases: HFMG06CAA_5227
Genomic context: Chromosome
Annotation: NC_018412.1 (895338..899147)
ID: 13464859
SEQ ID NO: 149

### 89. HFMG01WIA_5025

Csn1 family CRISPR-associated protein [Mycoplasma gallisepticum WI01_2001.043-13-2P]
Other Aliases: HFMG01WIA_5025
Genomic context: Chromosome
Annotation: NC_018410.1 (857648..861457)
ID: 13463863
SEQ ID NO: 150

### 90. HFMG01NYA_5169

Csn1 family CRISPR-associated protein [Mycoplasma gallisepticum NY01_2001.047-5-1P]
Other Aliases: HFMG01NYA_5169
Genomic context: Chromosome
Annotation: NC_018409.1 (883511..887185)
ID: 13462600
SEQ ID NO: 151

### 91. HFMG96NC SEQ ID NO: 127

### A_5295

Csn1 family CRISPR-associated protein [Mycoplasma gallisepticum NC96_1596-4-2P]
Other Aliases: HFMG96NCA_5295
Genomic context: Chromosome
Annotation: NC_018408.1 (904664..908473)
ID: 13462279
SEQ ID NO: 152

### 92. HFMG95NCA_5107

Csn1 family CRISPR-associated protein [Mycoplasma gallisepticum NC95_13295-2-2P]
Other Aliases: HFMG95NCA_5107
Genomic context: Chromosome
Annotation: NC_018407.1 (871783..875592)
ID: 13461469
SEQ ID NO: 153

### 93. MGAS10750_Spy0921

hypothetical protein [Streptococcus pyogenes MGAS10750]
Other Aliases: MGAS10750_Spy0921
Genomic context: Chromosome
Annotation: NC_008024.1 (875719..879834)
ID: 4066656
SEQ ID NO: 154

### 94. XAC3262

hypothetical protein [Xanthomonas axonopodis pv. citri str. 306]
Other Aliases: XAC3262
Genomic context: Chromosome
Annotation: NC_003919.1 (3842310..3842765)
ID: 1157333
SEQ ID NO: 155

### 95. SSUST1_1305

CRISPR-system-like protein [Streptococcus suis ST1]
Other Aliases: SSUST1_1305
Genomic context: Chromosome
Annotation: NC_017950.1 (1293105..1297250, complement)
ID: 13017849
SEQ ID NO: 156

### 96. SSUD9_1467

CRISPR-associated protein, Csn1 family [Streptococcus suis D9]
Other Aliases: SSUD9_1467
Genomic context: Chromosome
Annotation: NC_017620.1 (1456318..1459686, complement)
ID: 12718289
SEQ ID NO: 157

### 97. BBta_3952

hypothetical protein [Bradyrhizobium sp. BTAi1]
Other Aliases: BBta_3952
Genomic context: Chromosome
Annotation: NC_009485.1 (4149455..4152649, complement)
ID: 5151538
SEQ ID NO: 158

### 98. CIY_03670

CRISPR-associated protein, Csn1 family [Butyrivibrio fibrisolvens 16/4]
Other Aliases: CIY_03670
Annotation: NC_021031.1 (309663..311960, complement)
ID: 15213189
SEQ ID NO: 159

### 99. A11Q_912

CRISPR-associated protein, Csn1 family [Bdellovibrio exovorus JSS]
Other Aliases: A11Q_912
Genomic context: Chromosome
Annotation: NC_020813.1 (904781..907864, complement)
ID: 14861475
SEQ ID NO: 160

### 100. MCYN0850

Csn1 family CRISPR-associated protein [Mycoplasma cynos C142]
Other Aliases: MCYN_0850
Annotation: NC_019949.1 (951497..955216, complement)
ID: 14356531
SEQ ID NO: 161

### 101. SaSA20_0769

CRISPR-associated protein [Streptococcus agalactiae SA20-06]
Other Aliases: SaSA20_0769
Genomic context: Chromosome
Annotation: NC_019048.1 (803597..807709)
ID: 13908026
SEQ ID NO: 162

### 102. csn1

CRISPR-associated protein, Csn1 family [Streptococcus pyogenes A20]
Other Aliases: A20_0810
Genomic context: Chromosome
Annotation: NC_018936.1 (772038..776144)
ID: 13864445
SEQ ID NO: 163

### 103. P700755_000291

CRISPR-associated protein Cas9/Csn1, subtype II [Psychroflexus torquis ATCC 700755]
Other Aliases: P700755_000291
Genomic context: Chromosome
Annotation: NC_018721.1 (312899..317428)
ID: 13804571
SEQ ID NO: 164

### 104. A911_07335

CRISPR-associated protein [Campylobacter jejuni subsp. jejuni PT14]
Other Aliases: A911_07335
Genomic context: Chromosome
Annotation: NC_018709.2 (1450217..1453180, complement)
ID: 13791138
SEQ ID NO: 165

### 105. ASU2_02495

CRISPR-associated endonuclease Csn1 family protein [Actinobacillus suis H91-0380]
Other Aliases: ASU2_02495
Genomic context: Chromosome
Annotation: NC_018690.1 (552318..555482)
ID: 13751600
SEQ ID NO: 166

### 106. csn1

CRISPR-associated protein [Listeria monocytogenes SLCC2540]
Other Aliases: LMOSLCC2540_2635
Annotation: NC_018586.1 (2700744..2704748, complement)
ID: 13647248
SEQ ID NO: 167

### 107. csn1

CRISPR-associated protein [Listeria monocytogenes SLCC5850]
Other Aliases: LMOSLCC5850_2605
Annotation: NC_018592.1 (2646023..2650027, complement)
ID: 13626042
SEQ ID NO: 168

### 108. csn1

CRISPR-associated protein [Listeria monocytogenes serotype 7 str. SLCC2482]
Other Aliases: LMOSLCC2482_2606
Annotation: NC_018591.1 (2665393..2669397, complement)
ID: 13605045
SEQ ID NO: 169

### 109. csn1

CRISPR-associated protein [Listeria monocytogenes SLCC2755]
Other Aliases: LMOSLCC2755_2607
Annotation: NC_018587.1 (2694850..2698854, complement)
ID: 13599053
SEQ ID NO: 170

### 110. BN148_1523c

CRISPR-associated protein [Campylobacter jejuni subsp. jejuni NCTC 11168-BN148]
Other Aliases: BN148_1523c
Annotation: NC_018521.1 (1456880..1459834, complement)
ID: 13530688
SEQ ID NO: 171

### 111. Belba_3201

CRISPR-associated protein Cas9/Csn1, subtype II/NMEMI [Belliella baltica DSM 15883]
Other Aliases: Belba_3201
Genomic context: Chromosome
Annotation: NC_018010.1 (3445311..3449369, complement)
ID: 13056967
SEQ ID NO: 172

### 112. FN3523_1121

membrane protein [Francisella cf. novicida 3523]
Other Aliases: FN3523_1121
Genomic context: Chromosome
Annotation: NC_017449.1 (1129528..1134468, complement)
ID: 12924881
SEQ ID NO: 173

### 113.cas9

CRISPR-associated protein Cas9/Csn1, subtype II/NMEMI [Prevotella intermedia 17]
Other Aliases: PIN17_A0201
Chromosome: II
Annotation: Chromosome IINC_017861.1 (240722..244864)
ID: 12849954
SEQ ID NO: 174

### 114. csn1

CRISPR-associated protein, Csn1 family [Streptococcus thermophilus JIM 8232]
Other Aliases: STH8232_0853
Annotation: NC_017581.1 (706443..709808)
ID: 12637306
SEQ ID NO: 175

### 115. LMOG_01918

CRISPR-associated protein [Listeria monocytogenes J0161]
Other Aliases: LMOG_01918
Genomic context: Chromosome
Annotation: NC_017545.1 (2735374..2739378, complement)
ID: 12557915
SEQ ID NO: 176

### 116. LMRG_02138

CRISPR-associated protein [Listeria monocytogenes 10403S]
Other Aliases: LMRG_02138
Genomic context: Chromosome
Annotation: NC_017544.1 (2641981..2645985, complement)
ID: 12554876
SEQ ID NO: 177

### 117. CJSA_1443

putative CRISPR-associated protein [Campylobacter jejuni subsp. jejuni IA3902]
Other Aliases: CJSA_1443
Genomic context: Chromosome
Annotation: NC_017279.1 (1454273..1457227, complement)
ID: 12250720
SEQ ID NO: 178

### 118. csn1

CRISPR-associated protein Csn1 [Streptococcus pyogenes MGAS1882]
Other Aliases: MGAS1882_0792
Genomic context: Chromosome
Annotation: NC_017053.1 (775696..779799)
ID: 12014080
SEQ ID NO: 179

### 119. csn1

CRISPR-associated protein Csn1 [Streptococcus pyogenes MGAS15252]
Other Aliases: MGAS15252_0796
Genomic context: Chromosome
Annotation: NC_017040.1 (778271..782374)
ID: 11991096
SEQ ID NO: 180

### 120. cas3

CRISPR-associated endonuclease [Corynebacterium diphtheriae HC02]
Other Aliases: CDHC02_0036
Genomic context: Chromosome
Annotation: NC_016802.1 (37125..40379)
ID: 11739116
SEQ ID NO: 181

### 121. cas3

CRISPR-associated endonuclease [Corynebacterium diphtheriae C7 (beta)]
Other Aliases: CDC7B_0035
Genomic context: Chromosome
Annotation: NC_016801.1 (36309..39563)
ID: 11737358
SEQ ID NO: 182

### 122. cas3

CRISPR-associated endonuclease [Corynebacterium diphtheriae BH8]
Other Aliases: CDBH8_0038
Genomic context: Chromosome
Annotation: NC_016800.1 (37261..40515)
ID: 11735325
SEQ ID NO: 183

### 123. cas3

CRISPR-associated endonuclease [Corynebacterium diphtheriae 31A]
Other Aliases: CD31A_0036
Genomic context: Chromosome
Annotation: NC_016799.1 (34597..37851)
ID: 11731168
SEQ ID NO: 184

### 124. cas3

CRISPR-associated endonuclease [Corynebacterium diphtheriae VA01]
Other Aliases: CDVA01_0033
Genomic context: Chromosome
Annotation: NC_016790.1 (34795..38049)
ID: 11717708
SEQ ID NO: 185

### 125. cas3

CRISPR-associated endonuclease [Corynebacterium diphtheriae HC01]
Other Aliases: CDHC01_0034
Genomic context: Chromosome
Annotation: NC_016786.1 (35060..38314)
ID: 11708318
SEQ ID NO: 186

### 126. cas9

CRISPR-associated protein [Corynebacterium diphtheriae HC01]
Other Aliases: CDHC01_2103
Genomic context: Chromosome
Annotation: NC_016786.1 (2246368..2248998)
ID: 11708126
SEQ ID NO: 187

### 127. PARA_18570

hypothetical protein [Haemophilus parainfluenzae T3T1]
Other Aliases: PARA_18570
Genomic context: Chromosome
Annotation: NC_015964.1 (1913335..1916493)
ID: 11115627
SEQ ID NO: 188

### 128. HDN1F_34120

hypothetical protein [gamma proteobacterium HdN1]
Other Aliases: HDN1F_34120
Genomic context: Chromosome
Annotation: NC_014366.1 (4143336..4146413, complement)
ID: 9702142
SEQ ID NO: 189

### 129. SPy_1046

hypothetical protein [Streptococcus pyogenes M1 GAS]
Other Aliases: SPy_1046
Genomic context: Chromosome
Annotation: NC_002737.1 (854757..858863)
ID: 901176
SEQ ID NO: 190

### 130. GBS222_0765

Hypothetical protein [Streptococcus agalactiae]
Other Aliases: GBS222_0765
Annotation: NC_021195.1 (810875..814987)
ID: 15484689
SEQ ID NO: 191

### 131. NE061598_03330

hypothetical protein [Francisella tularensis subsp. tularensis NE061598]
Other Aliases: NE061598_03330
Genomic context: Chromosome
Annotation: NC_017453.1 (601219..604590)
ID: 12437259
SEQ ID NO: 192

### 132. NMV_1993

hypothetical protein [Neisseria meningitidis 8013]
Other Aliases: NMV_1993
Annotation: NC_017501.1 (1917073..1920321)
ID: 12393700
SEQ ID NO: 193

### 133. csn1

hypothetical protein [Campylobacter jejuni subsp. jejuni M1]
Other Aliases: CJM1_1467
Genomic context: Chromosome
Annotation: NC_017280.1 (1433667..1436252, complement)
ID: 12249021
SEQ ID NO: 194

### 134. FTU_0629

hypothetical protein [Francisella tularensis subsp. tularensis TIGB03]
Other Aliases: FTU_0629
Genomic context: Chromosome
Annotation: NC_016933.1 (677092..680463)
ID: 11890131
SEQ ID NO: 195

### 135. NMAA_0315

hypothetical protein [Neisseria meningitidis WUE 2594]
Other Aliases: NMAA_0315
Annotation: NC_017512.1 (377010..380258, complement)
ID: 12407849
SEQ ID NO: 196

### 136. WS1445

hypothetical protein [Wolinella succinogenes DSM 1740]
Other Aliases: WS1445
Genomic context: Chromosome
Annotation: NC_005090.1 (1388202..1391381, complement)
ID: 2554690
SEQ ID NO: 197

### 137. THITE_2123823

hypothetical protein [Thielavia terrestris NRRL 8126]
Other Aliases: THITE_2123823
Chromosome: 6
Annotation: Chromosome 6NC_016462.1 (1725696..1725928)
ID: 11523019
SEQ ID NO: 198

### 138. XAC29_16635

hypothetical protein [Xanthomonas axonopodis Xac29-1]
Other Aliases: XAC29_16635
Genomic context: Chromosome
Annotation: NC_020800.1 (3849847..3850302)
ID: 14853997
SEQ ID NO: 199

### 139. M1GAS476_0830

hypothetical protein [Streptococcus pyogenes M1 476]
Other Aliases: M1GAS476_0830
Chromosome: 1
Annotation: NC_020540.1 (792119..796225)
ID: 14819166
SEQ ID NO: 200

### 140. Piso0_000203

Piso0_000203[Millerozyma farinosa CBS 7064]
Other Aliases: GNLVRS01_PISO0A04202g
Other Designations: hypothetical protein
Chromosome: A
Annotation: NC_020226.1 (343553..343774, complement)
ID: 14528449
SEQ ID NO: 201

### 141. G148_0828

hypothetical protein [Riemerella anatipestifer RA-CH-2]
Other Aliases: G148_0828
Genomic context: Chromosome
Annotation: NC_020125.1 (865673..869875)
ID: 14447195
SEQ ID NO: 202

### 142. csn1

hypothetical protein [Streptococcus dysgalactiae subsp. equisimilis AC-2713]
Other Aliases: SDSE_1207
Annotation: NC_019042.1 (1134173..1138288, complement)
ID: 13901498
SEQ ID NO: 203

### 143. A964_0899

hypothetical protein [Streptococcus agalactiae GD201008-001]
Other Aliases: A964_0899
Genomic context: Chromosome
Annotation: NC_018646.1 (935164..939276)
ID: 13681619
SEQ ID NO: 204

### 144. FNFX1_0762

hypothetical protein [Francisella cf. novicida Fx1]
Other Aliases: FNFX1_0762
Genomic context: Chromosome
Annotation: NC_017450.1 (781484..786373)
ID: 12435564
SEQ ID NO: 205

### 145. FTV_0545

hypothetical protein [Francisella tularensis subsp. tularensis TI0902]
Other Aliases: FTV_0545
Genomic context: Chromosome
Annotation: NC_016937.1 (601185..604556)
ID: 11880693
SEQ ID NO: 206

### 146. FTL_1327

hypothetical protein [Francisella tularensis subsp. holarctica LVS]
Other Aliases: FTL_1327
Genomic context: Chromosome
Annotation: NC_007880.1 (1262508..1263689, complement)
ID: 3952607
SEQ ID NO: 207

### 147. FTL_1326

hypothetical protein [Francisella tularensis subsp. holarctica LVS]
Other Aliases: FTL_1326
Genomic context: Chromosome
Annotation: NC_007880.1 (1261927..1262403, complement)
ID: 3952606
SEQ ID NO: 208

## Claims

1. A method of producing a mammalian cell or a transgenic non-human mammalian organism, the method comprising
(a) carrying out a method to
(i) knock out a target nucleotide sequence by deletion of a nucleotide sequence that is at least 20 kb in the genome of a first cell; and/or
(ii) knock in an insert nucleotide sequence that is at least 20 kb into the genome of a first cell;
wherein the cell or a progeny thereof can develop into a non-human organism or cell; wherein the method comprises:
(Ia) using nucleic acid cleavage to create first and second breaks in a nucleic acid strand, thereby creating 5' and 3' cut ends and a nucleotide sequence between the ends, wherein the cleavage is carried out by combining in a cell the nucleic acid strand, a Cas9 endonuclease, a crRNA and a tracrRNA for targeting the endonuclease;
(Ib) using homologous recombination to delete the nucleotide sequence, wherein the homologous recombination is carried out:
(A) between an incoming nucleic acid comprising first and second homology arms, wherein the homology arms are substantially homologous respectively to a sequence extending 5' from the 5' end and a sequence extending 3' from the 3' end; or
(B) between an incoming nucleic acid comprising an insert nucleotide sequence flanked by the first and second homology arms, wherein the homology arms are substantially homologous respectively to a sequence extending 5' from the 5' end and a sequence extending 3' from the 3' end, wherein the insert nucleotide sequence is inserted between the 5' and 3' ends;
and/or
(IIa) using nucleic acid cleavage to create 5' and 3' cut ends in a single nucleic acid strand, wherein the cleavage is carried out by combining in a cell the nucleic acid strand, a Cas9 endonuclease, a crRNA and a tracrRNA for targeting the endonuclease;
(IIb) using homologous recombination to insert a nucleotide sequence between the ends, wherein the insert sequence comprises a regulatory element or encodes all or part of a protein, wherein the homologous recombination is carried out between an incoming nucleic acid comprising an insert nucleotide sequence flanked by the first and second homology arms, wherein the homology arms are substantially homologous respectively to a sequence extending 5' from the 5' end and a sequence extending 3' from the 3' end, wherein the insert nucleotide sequence is inserted between the 5' and 3' ends;
(b) developing the cell or progeny into a non-human organism or a non-human cell.

2. The method of claim 1, wherein the insert nucleotide sequence is at least 50 kb or 100 kb.

3. The method of claims 1 or 2, wherein the deleted nucleotide sequence is at least 50 kb or 100 kb.

4. The method of any preceding claim, wherein step (Ia) and/or step (IIa) is carried out using a guide RNA, e.g., a single guide RNA (sgRNA), comprising a crRNA and a tracrRNA, optionally wherein step (Ia) uses a first and second gRNA to target a desired part of the nucleic acid, defining the region to be deleted.

5. The method of any preceding claim, wherein
i. the crRNA and tracrRNA is introduced into the cell as RNA molecules; or
ii. the crRNA and tracrRNA are encoded by DNA within the cell or organism and are transcribed inside the cell or organism to produce the crRNA and tracrRNA; or
iii. the tracrRNA is encoded by DNA within the cell or organism and is transcribed inside the cell or organism to produce the tracrRNA, but one or more crRNAs are introduced as RNA nucleic acid into the cell or organism; or
iv. a gRNA comprising a crRNA and a tracrRNA is encoded by a DNA sequence that has been introduced into the cell.

6. The method of any preceding claim, wherein
i. the Cas9 endonuclease is encoded by a nucleotide sequence that has been introduced into the cell; or
ii. the Cas9 endonuclease is introduced as a protein into the cell or organism; or
iii. the Cas9 endonuclease is introduced as a vector encoding the Cas9 endonuclease into the cell or organism; or
iv. the Cas9 endonuclease is encoded by DNA that is genomically integrated into the cell or organism and is transcribed and translated inside the cell or organism.

7. The method of any preceding claim, wherein the method is carried out in a non-human mammalian cell or a rodent ES cell or rodent zygote or a mouse ES cell or a mouse zygote or a mouse or rat or rabbit.

8. The method of any preceding claim, wherein the organism or cell is homozygous for the modification (i) and/or modification (ii).

9. The method of any preceding claim, wherein the insert sequence is a synthetic sequence; or comprises a sequence encoding all or part of a protein from a species other than the species from which the first cell is derived; or comprises a regulatory element from said first species, e.g. the insert sequence encodes all or part of a human protein or a human protein subunit or domain; or is a human sequence that replaces or supplements an orthologous non-human sequence.

10. The method of any preceding claim, wherein
i. the cell or organism is a non-human mammal cell or a non-human mammal that expresses one or more human antibody heavy chain variable domains, and optionally no heavy chain variable domains of a non-human mammal species; and/or
ii. the cell or organism is a non-human mammal cell or a non-human mammal that expresses one or more human antibody kappa light chain variable domains; and/or
iii. the cell or organism is a non-human mammal cell or a non-human mammal that expresses one or more human antibody lambda light chain variable domains; and/or
iv. the organism is a non-human mammal that expresses human antibody variable regions whose genome comprises a replacement of an endogenous target with an orthologous or homologous human sequence.

11. A non-human mammalian cell or a non-human mammalian organism, e.g., a mouse, whose genome comprises a modification comprising a non-endogenous nucleotide sequence of at least 20 kb flanked by endogenous nucleotide sequences, wherein the cell or organism is obtainable by the method of any preceding claim, and wherein the non-endogenous sequence is flanked 3' and/or 5' by a Cas9 PAM motif; wherein the cell is not comprised by a human and
i. the non-endogenous sequence comprises all or part of a regulatory element or encodes all or part of a protein; and/or
ii. the non-endogenous sequence replaces an orthologous or homologous sequence in the genome.

12. The cell or non-human organism of claim 11, wherein
i. the non-endogenous sequence is flanked 3' and/or 5' within 20, 10, 5, 4, 3, 2 or 1 or less nucleotides of, or directly adjacent to a Cas9 PAM motif; and/or
ii. the PAM motif is recognised by a *Streptococcus* Cas9; and/or
iii. the PAM motif comprises a sequence selected from CCN, TCN, TTC, AWG, CC, NNAGNN, NGGNG, GG, NGG, WGG, CWT, CTT and GAA.

13. The cell or non-human organism of claims 11 or 12, wherein the non-endogenous sequence
i. comprises one or more human antibody gene segments; and/or
ii. an antibody variable region; and/or
iii. an antibody constant region; and/or
iv. encodes a human Fc receptor protein or subunit or domain thereof; and/or
v. is a human sequence.

## Patentansprüche

1. Verfahren zum Herstellen einer Säugetierzelle oder eines transgenen nicht-humanen Säugetierorganismus, wobei das Verfahren umfasst
(a) Durchführen eines Verfahrens zum
(i) Knock-out einer Target-Nukleotid-Sequenz durch Deletion einer Nukleotid-Sequenz, die mindestens 20 kb im Genom einer ersten Zelle ist; und/oder
(ii) Knock-in einer Insert-Nukleotid-Sequenz, die mindestens 20kb im Genom einer ersten Zelle ist;
wobei die Zelle oder ein Nachkommen davon sich in einen nicht-humanen Organismus oder Zelle entwickeln kann;
wobei das Verfahren umfasst:
(la) Verwenden von Nukleinsäurespaltung, um erste und zweite Brüche in einem Nukleinsäurestrang zu bilden, wodurch 5'- und 3'-Schnittenden und eine Nukleotid-Sequenz zwischen den Enden erzeugt werden, wobei die Spaltung durch Kombinieren, in einer Zelle, des Nukleinsäurestrangs, einer Cas9-Endonuklease, einer crRNA und einer tracrRNA zum Targeting der Endonuklease durchgeführt wird;
(Ib) Verwenden von homologer Rekombination, um die Nukleotid-Sequenz zu deletieren, wobei die homologe Rekombination durchgeführt wird:
(A) zwischen einer hereinkommenden Nukleinsäure, umfassend erste und zweite Homologiearme, wobei die Homologiearme im Wesentlichen hinsichtlich einer Sequenz, die sich 5' von dem 5'-Ende erstreckt bzw. einer Sequenz, die sich 3' von dem 3'-Ende erstreckt, homolog sind; oder
(B) zwischen einer hereinkommenden Nukleinsäure, umfassend eine Insert-Nukleotid-Sequenz, die von dem ersten und zweiten Homologiearm flankiert ist, wobei die Homologiearme im Wesentlichen hinsichtlich einer Sequenz, die sich 5' von dem 5'-Ende erstreckt bzw. einer Sequenz, die sich 3' von dem 3'-Ende erstreckt, homolog sind, wobei die Insert-Nukleotid-Sequenz zwischen dem 5'- und 3'-Ende eingefügt ist; und/oder
(IIa) Verwenden von Nukleinsäurespaltung, um 5'- und 3'-Schnittenden in einem einzigen Nukleinsäurestrang zu bilden, wobei die Spaltung durch Kombinieren, in einer Zelle, des Nukleinsäurestrangs, einer Cas9-Endonuklease, einer crRNA und einer tracrRNA zum Targeting der Endonuklease durchgeführt wird;
(IIb) Verwenden von homologer Rekombination, um eine Nukleotid-Sequenz zwischen den Enden einzufügen, wobei die Insert-Sequenz ein regulatorisches Element umfasst oder für alles oder einen Teil eines Proteins kodiert; wobei die homologe Rekombination zwischen einer hereinkommenden Nukleinsäure, umfassend eine Insert-Nukleotid-Sequenz, die von dem ersten und zweiten Homologiearm flankiert ist, durchgeführt wird, wobei die Homologiearme im Wesentlichen hinsichtlich einer Sequenz, die sich 5' von dem 5'-Ende erstreckt bzw. einer Sequenz, die sich 3' von dem 3'-Ende erstreckt, homolog sind, wobei die Insert-Nukleinsäure-Sequenz zwischen dem 5'- und 3'-Ende eingefügt ist;
(b) Entwickeln der Zelle oder des Nachkommens zu einem nicht-humanen Organismus oder einer nicht-humanen Zelle.

2. Verfahren nach Anspruch 1, wobei die Insert-Nukleotid-Sequenz mindestens 50 kb oder 100 kb ist.

3. Verfahren nach Anspruch 1 oder 2, wobei die deletierte Nukleotid-Sequenz mindestens 50 kb oder 100 kb ist.

4. Verfahren nach einem der vorstehenden Ansprüche, wobei Schritt (la) und/oder Schritt (IIa) unter Verwendung einer Führungs-RNA (guide RNA), z. B. einer einzigen Führungs-RNA (single guide RNA) (sgRNA), umfassend eine crRNA und eine tracrRNA, durchgeführt wird, wobei gegebenenfalls Schritt (la) eine erste und zweite gRNA verwendet, um einen gewünschten Teil der Nukleinsäure zu targeten, die zu deletierende Region definierend.

5. Verfahren nach einem der vorstehenden Ansprüche, wobei
i. die crRNA und tracrRNA als RNA-Moleküle in die Zelle eingeführt werden; oder
ii. die crRNA und tracrRNA durch DNA in der Zelle oder dem Organismus kodiert werden und in der Zelle oder dem Organismus transkribiert werden, um die crRNA und tracrRNA zu erzeugen; oder
iii. die tracrRNA durch DNA in der Zelle oder dem Organismus kodiert wird, und in der Zelle oder dem Organismus transkribiert wird, um die tracrRNA zu erzeugen, jedoch eine oder mehrere crRNAs als RNA-Nukleinsäure in die Zelle oder den Organismus eingeführt werden; oder
iv. eine gRNA, umfassend eine crRNA und eine tracrRNA, durch eine DNA-Sequenz kodiert wird, die in die Zelle eingeführt wurde.

6. Verfahren nach einem der vorstehenden Ansprüche, wobei
i. die Cas9-Endonuklease durch eine Nukleotid-Sequenz kodiert wird, die in die Zelle eingeführt wurde; oder
ii. die Cas9-Endonuklease als ein Protein in die Zelle oder den Organismus eingeführt wird; oder
iii. die Cas9-Endonuklease als ein Vektor, der für die Cas9-Endonuklease kodiert, in die Zelle oder den Organismus eingeführt wird; oder
iv. die Cas9-Endonuklease durch DNA kodiert wird, die genomisch in die Zelle oder den Organismus integriert ist, und in der Zelle oder dem Organismus transkribiert und übersetzt wird.

7. Verfahren nach einem der vorstehenden Ansprüche, wobei das Verfahren in einer nicht-humanen Säugetierzelle oder eine Nager-ES-Zelle oder Nager-Zygote oder einer Maus-ES-Zelle oder einer Maus-Zygote oder einer Maus oder Ratte oder einem Hasen durchgeführt wird.

8. Verfahren nach einem der vorstehenden Ansprüche, wobei der Organismus oder die Zelle homozygot für die Modifikation (i) und/oder Modifikation (ii) ist.

9. Verfahren nach einem der vorstehenden Ansprüche, wobei die Insert-Sequenz eine synthetische Sequenz ist; oder eine Sequenz umfasst, die für alles oder einen Teil eines Proteins von einer Spezies kodiert, die nicht die Spezies ist, von der die erste Zelle abgeleitet ist; oder ein regulatorisches Element von der ersten Spezies umfasst, z. B. kodiert die Insert-Sequenz für alles oder einen Teil eines humanen Proteins oder einer humanen Proteinteileinheit oder Domäne; oder ist eine humane Sequenz, die eine orthologe nicht-humane Sequenz ersetzt oder ergänzt.

10. Verfahren nach einem der vorstehenden Ansprüche, wobei
i. die Zelle oder der Organismus eine nicht-humane Säugetierzelle oder ein nicht-humanes Säugetier ist, das einen oder mehrere humane Antikörper mit schwerer Kette mit variabler Domänen exprimiert, und gegebenenfalls keine schwere Kette mit variablen Domänen einer nicht-humanen Säugetierspezies; und/oder
ii. die Zelle oder der Organismus eine nicht-humane Säugetierzelle oder ein nicht-humanes Säugetier ist, das einen oder mehrere humane Antikörper mit leichter Kappa-Kette mit variablen Domänen exprimiert; und/oder
iii. die Zelle oder der Organismus eine nicht-humane Säugetierzelle oder ein nicht-humanes Säugetier ist, das einen oder mehrere humane Antikörper mit leichter Lambda-Kette mit variablen Domänen exprimiert; und/oder
iv. der Organismus ein nicht-humanes Säugetier ist, das einen oder mehrere humane Antikörper mit variabler Regionen exprimiert, deren Genom einen Ersatz eines endogenen Targets mit einer orthologen oder homologen humanen Sequenz umfasst.

11. Nicht-humane Säugetierzelle oder nicht-humaner Säugetierorganismus, z.B. eine Maus, deren Genom eine Modifikation umfasst, die eine nicht-endogene Nukleotid-Sequenz von mindestens 20 kb, flankiert von endogenen Nukleotid-Sequenzen, umfasst, wobei die Zelle oder der Organismus durch ein Verfahren nach einem der vorstehenden Ansprüche erhalten werden kann, und wobei die nicht-endogene Sequenz an 3' und/oder 5' von einem Cas9-PAM-Motiv flankiert ist; wobei die Zelle nicht von einem Menschen umfasst ist; und
i. die nicht-endogene Sequenz ein gesamtes oder einen Teil eines regulatorischen Elements umfasst oder für alles oder einen Teil eines Proteins kodiert; und/oder
ii. die nicht-endogene Sequenz ein orthologe oder homologe Sequenz im Genom ersetzt.

12. Zelle oder nicht-humaner Organismus nach Anspruch 11, wobei
i. die nicht-endogene Sequenz an 3' und/oder 5' innerhalb von 20, 10, 5, 4, 3, 2 oder 1 oder weniger Nukleotiden von, oder unmittelbar benachbart zu einem Cas9-PAM-Motiv flankiert ist; und/oder
ii. das PAM-Motiv von einem *Streptococcus Cas9* erkannt wird; und/oder
iii. das PAM-Motiv eine Sequenz umfasst, die ausgewählt ist aus CCN, TCN, TTC, AWG, CC, NNAGNN, NGGNG, GG, NGG, WGG, CWT, CTT und GAA.

13. Zelle oder nicht-humaner Organismus nach Anspruch 11 oder 12, wobei die nicht-endogene Sequenz
i. einen oder mehrere humane Antikörpergensegmente umfasst; und/oder
ii. einen Antikörper mit variabler Region; und/oder
iii. einen Antikörper mit konstanter Region; und/oder
iv. für ein humanes Fc-Rezeptorprotein oder Teileinheit oder Domäne davon kodiert; und/oder
v. eine humane Sequenz ist.

## Revendications

1. Procédé de production d'une cellule de mammifère ou d'un organisme de mammifère non humain transgénique, le procédé comprenant les étapes consistant à
(a) effectuer un procédé pour
(i) supprimer une séquence nucléotidique cible par délétion d'une séquence nucléotidique qui est au moins 20 kb dans le génome d'une première cellule ; et/ou
(ii) insérer une séquence nucléotidique d'insertion qui est au moins 20 kb dans le génome d'une première cellule ;
dans lequel la cellule ou une progéniture de celle-ci peut se développer dans un organisme ou une cellule non humain(e) ;
dans lequel le procédé comprend les étapes consistant à :
(la) utiliser un clivage d'acide nucléique pour créer des première et seconde coupures dans un brin d'acide nucléique, créant ainsi des extrémités coupées 5' et 3' et une séquence nucléotidique entre les extrémités, dans lequel le clivage est réalisé en combinant, dans une cellule, le brin d'acide nucléique, une endonucléase Cas9, un ARNcr et un ARNtracr pour cibler l'endonucléase ;
(Ib) utiliser une recombinaison homologue pour supprimer la séquence nucléotidique, dans lequel la recombinaison homologue est réalisée :
(A) entre un acide nucléique entrant comprenant des premier et second bras d'homologie, dans lequel les bras d'homologie sont substantiellement homologues respectivement à une séquence s'étendant en 5' de l'extrémité 5' et à une séquence s'étendant en 3' de l'extrémité 3' ; ou
(B) entre un acide nucléique entrant comprenant une séquence nucléotidique d'insertion flanquée par les premier et second bras d'homologie, dans lequel les bras d'homologie sont substantiellement homologues respectivement à une séquence s'étendant en 5' de l'extrémité 5' et à une séquence s'étendant en 3' de l'extrémité 3', dans lequel la séquence nucléotidique d'insertion est insérée entre les extrémités 5' et 3' ;
et/ou
(IIa) utiliser un clivage d'acide nucléique pour créer des extrémités coupées 5' et 3' dans un seul brin d'acide nucléique, dans lequel le clivage est réalisé en combinant, dans une cellule, le brin d'acide nucléique, une endonucléase Cas9, un ARNcr et un ARNtracr pour cibler l'endonucléase ;
(IIb) utiliser une recombinaison homologue pour insérer une séquence nucléotidique entre les extrémités, dans lequel la séquence d'insertion comprend un élément de régulation ou code pour tout ou partie d'une protéine, dans lequel la recombinaison homologue est réalisée entre un acide nucléique entrant comprenant une séquence nucléotidique d'insertion flanquée par les premier et second bras d'homologie, dans lequel les bras d'homologie sont substantiellement homologues respectivement à une séquence s'étendant en 5' de l'extrémité 5' et à une séquence s'étendant en 3' de l'extrémité 3', dans lequel la séquence nucléotidique d'insertion est insérée entre les extrémités 5' et 3' ;
(b) développer la cellule ou la progéniture dans un organisme non humain ou une cellule non humaine.

2. Procédé selon la revendication 1, dans lequel la séquence nucléotidique d'insertion est au moins 50 kb ou 100 kb.

3. Procédé selon les revendications 1 ou 2, dans lequel la séquence nucléotidique supprimée est au moins 50 kb ou 100 kb.

4. Procédé selon l'une quelconque des revendications précédentes, dans lequel l'étape (la) et/ou l'étape (IIa) est/sont effectuée(s) en utilisant un ARN guide, par ex. un ARN guide simple (ARNsg), comprenant un ARNcr et un ARNtracr, facultativement dans lequel l'étape (la) utilise un premier et un second ARNg pour cibler une partie souhaitée de l'acide nucléique, définissant la région à supprimer.

5. Procédé selon l'une quelconque des revendications précédentes, dans lequel
i. l'ARNcr et l'ARNtracr sont introduits dans la cellule en tant que molécules d'ARN ; ou
ii. l'ARNcr et l'ARNtracr sont codés par l'ADN dans la cellule ou l'organisme et sont transcrits à l'intérieur de la cellule ou l'organisme pour produire l'ARNcr et l'ARNtracr ; ou
iii. l'ARNtracr est codé par l'ADN dans la cellule ou l'organisme et est transcrit à l'intérieur de la cellule ou l'organisme pour produire l'ARNtracr, mais un ou plusieurs ARNcr sont introduits en tant qu'acide nucléique ARN dans la cellule ou l'organisme ; ou
iv. un ARNg comprenant un ARNcr et un ARNtracr est codé par une séquence ADN qui a été introduite dans la cellule.

6. Procédé selon l'une quelconque des revendications précédentes, dans lequel
i. l'endonucléase Cas9 est codée par une séquence nucléotidique qui a été introduite dans la cellule ; ou
ii. l'endonucléase Cas9 est introduite en tant que protéine dans la cellule ou l'organisme ; ou
iii. l'endonucléase Cas9 est introduite en tant que vecteur codant pour l'endonucléase Cas9 dans la cellule ou l'organisme ; ou
iv. l'endonucléase Cas9 est codée par l'ADN qui est intégré génomiquement dans la cellule ou l'organisme et est transcrite et traduite à l'intérieur de la cellule ou l'organisme.

7. Procédé selon l'une quelconque des revendications précédentes, dans lequel le procédé est réalisé dans une cellule de mammifère non humain ou une cellule ES de rongeur ou un zygote de rongeur ou une cellule ES de souris ou un zygote de souris ou une souris ou un rat ou un lapin.

8. Procédé selon l'une quelconque des revendications précédentes, dans lequel l'organisme ou la cellule est homozygote pour la modification (i) et/ou la modification (ii).

9. Procédé selon l'une quelconque des revendications précédentes, dans lequel la séquence d'insertion est une séquence synthétique ; ou comprend une séquence codant pour tout ou partie d'une protéine provenant d'une espèce autre que l'espèce de laquelle la première cellule est dérivée ; ou comprend un élément de régulation provenant de ladite première espèce, par ex. la séquence d'insertion code pour tout ou partie d'une protéine humaine ou d'une sous-unité ou un domaine de protéine humaine ; ou est une séquence humaine qui remplace ou complète une séquence orthologue non humaine.

10. Procédé selon l'une quelconque des revendications précédentes, dans lequel
i. la cellule ou l'organisme est une cellule de mammifère non humain ou un mammifère non humain qui exprime un ou plusieurs domaines variables de chaîne lourde d'anticorps humain, et facultativement aucun domaine variable de chaîne lourde d'une espèce de mammifère non humain ; et/ou
ii. la cellule ou l'organisme est une cellule de mammifère non humain ou un mammifère non humain qui exprime un ou plusieurs domaines variables de chaîne légère kappa d'anticorps humain ; et/ou
iii. la cellule ou l'organisme est une cellule de mammifère non humain ou un mammifère non humain qui exprime un ou plusieurs domaines variables de chaîne légère lambda d'anticorps humain ; et/ou
iv. l'organisme est un mammifère non humain qui exprime des régions variables d'anticorps humain dont le génome comprend un remplacement d'une cible endogène par une séquence humaine orthologue ou homologue.

11. Cellule de mammifère non humain ou organisme de mammifère non humain, par ex. une souris, dont le génome comprend une modification comprenant une séquence nucléotidique non endogène d'au moins 20 kb flanquée par des séquences nucléotidiques endogènes, dans lequel la cellule ou l'organisme peut être obtenu par le procédé selon l'une quelconque des revendications précédentes, et dans lequel la séquence non endogène est flanquée en 3' et/ou 5' par un motif PAM Cas9 ; dans lequel la cellule n'est pas comprise par un humain et
i. la séquence non endogène comprend tout ou partie d'un élément de régulation ou code pour tout ou partie d'une protéine ; et/ou
ii. la séquence non endogène remplace une séquence orthologue ou homologue dans le génome.

12. Cellule ou organisme non humain selon la revendication 11, dans lequel
i. la séquence non endogène est flanquée en 3' et/ou 5' dans 20, 10, 5, 4, 3, 2 ou 1 nucléotides ou moins de, ou directement adjacents à, un motif PAM Cas9 ; et/ou
ii. le motif PAM est reconnu par un Cas9 *Streptococcus ;* et/ou
iii. le motif PAM comprend une séquence sélectionnée parmi CCN, TCN, TTC, AWG, CC, NNAGNN, NGGNG, GG, NGG, WGG, CWT, CTT et GAA.

13. Cellule ou organisme non humain selon les revendications 11 ou 12, dans lequel la séquence non endogène
i. comprend un ou plusieurs segments de gène d'anticorps humains ; et/ou
ii. une région variable d'anticorps ; et/ou
iii. une région constante d'anticorps ; et/ou
iv. code pour une protéine de récepteur Fc humain ou une sous-unité ou un domaine de celle-ci ; et/ou
v. est une séquence humaine.
